# EUROPEAN PATENT APPLICATION

(11) **EP 4 775 582 A1**
(43) Date of publication of application: **15.07.2026**
(21) Application number: 24862079.1
(22) Date of filing: 06.09.2024
(51) Int. Cl.: C07D 471/22, C07D 487/12, C07D 513/22, A61K 31/429, A61K 31/5025, A61P 35/00

(54) **MACROCYCLIC COMPOUND AND ANTIBODY-DRUG CONJUGATE THEREOF**

(30) Priority: 06.09.2023 CN 202311147514; 22.11.2023 CN 202311569777; 13.12.2023 CN 202311720542; 05.01.2024 CN 202410021309; 08.02.2024 CN 202410177985; 27.03.2024 CN 202410365458; 29.05.2024 CN 202410687961; 16.07.2024 CN 202410956050
(71) Applicant: Genfleet Therapeutics (Shanghai) Inc., Shanghai 201203 (CN)
(72) Inventor: ZHAO, Jichen, Shanghai 201203 (CN); ZHOU, Fusheng, Shanghai 201203 (CN); YAN, Feng, Shanghai 201203 (CN); HE, Xiangyu, Shanghai 201203 (CN); YANG, Hongxing, Shanghai 201203 (CN); JIANG, Tao, Shanghai 201203 (CN); ZHANG, Leitao, Shanghai 201203 (CN); HE, Wan, Shanghai 201203 (CN); LAN, Jiong, Shanghai 201203 (CN); LU, Qiang, Shanghai 201203 (CN)
(74) Representative: Zanoli, Enrico
(86) International application number: PCT/CN2024/117436
(87) International publication number: WO 2025/051241

(57) **Abstract**

Provided are a macrocyclic compound and an antibody-drug conjugate thereof. Specifically, the macrocyclic compound is an inhibitor targeting RAS proteins. Further provided are a method for preparing the above-mentioned macrocyclic compound and antibody-drug conjugate thereof, and the pharmaceutical use thereof.

## Description

The present application is based on and claims priority to Chinese Patent Application No. CN202311147514.5 filed on Sep. 6, 2023, Chinese Patent Application No. CN202410956050.0 filed on Jul. 16, 2024, Chinese Patent Application No. CN202311569777.5 filed on Nov. 22, 2023, Chinese Patent Application No. CN202311720542.1 filed on Dec. 13, 2023, Chinese Patent Application No. CN202410021309.2 filed on Jan. 5, 2024, Chinese Patent Application No. CN202410177985.9 filed on Feb. 8, 2024, Chinese Patent Application No. CN202410365458.0 filed on Mar. 27, 2024, and Chinese Patent Application No. CN202410687961.8 filed on May 29, 2024, the disclosure of each of which is incorporated herein by reference in its entirety.

### TECHNICAL FIELD

The present disclosure pertains to the field of biomedicine and particularly relates to a macrocyclic compound and an antibody-drug conjugate thereof. The macrocyclic compound is an RAS protein-targeting inhibitor. The present disclosure further relates to use of the macrocyclic compound and the antibody-drug conjugate thereof in the treatment of diseases.

### BACKGROUND

An antibody-drug conjugate is formed by linking an antibody or an antibody fragment to an active toxic drug via a chemical linker, which can fully utilize the binding specificity of the antibody for surface antigens of tumor cells and the high efficiency of the active toxic drug in killing the tumor cells, and meanwhile solve the defects of low efficacy of the antibody alone and serious toxic and side effects of the toxic drug. Currently, a variety of antibody-drug conjugates have been approved at home and abroad, such as gemtuzumab ozogamicin approved by the FDA for marketing, and Disitamab vedotin (trade name: Aidixi^{®}) approved for marketing in China. Currently, there are also a large number of antibody-drug conjugates in clinical development.

The RAS gene (rat sarcoma viral oncogene homolog) encodes a class of small GTPase family proteins with a molecular weight of about 21 kDa. The mammalian RAS family comprises three members: HRAS, KRAS, and NRAS, which serve as molecular switches in a variety of cell pathway signaling processes. RAS is in an inactivated state (dormant or turned off) when bound to GDP. When cells are exposed to certain growthpromoting stimuli, RAS is induced to exchange its bound GDP for GTP, thereby becoming activated. Subsequently, it recruits and activates other downstream target proteins (e.g., Raf and PI3K), thereby promoting cell proliferation. The RAS protein endogenously possesses the activity of hydrolyzing GTP to GDP (thereby converting itself into an inactivated state), but the conversion rate is low. However, once it binds to a GTPase-activating protein (GAP), the interaction between the two greatly accelerates the rate at which the RAS protein converts GTP to GDP, so that RAS can revert to an inactivated state rapidly upon cessation of cell stimulation, thereby shutting down the signaling pathway transduction.

Approximately 30% of human tumors harbor RAS gene mutations, in which KRAS mutations are the most common and account for about 85%, while NRAS and HRAS mutations account for 12% and 3%, respectively. KRAS mutations are predominantly found in pancreatic cancer (86%), colorectal cancer (41%), and lung cancer (32%); NRAS mutations are common in melanoma and acute myeloid leukemia; and HRAS mutations are common in bladder cancer and head and neck cancer. In tumors, RAS gene mutations occur primarily via point mutations. More than 150 distinct RAS point mutations have been identified, with mutations at positions G12, G13, and Q61 being the most common. Generally, these pathogenic mutations affect the interaction between RAS and GAPs and the endogenous GTPase activity of RAS. Consequently, the mutated RAS protein exists mainly in a GTP-bound activated form within cells, such that the signaling pathway mediated by this protein is in a continuously activated state, which in turn gives rise to uncontrolled cell proliferation and ultimately promotes tumor formation.

Currently, for KRAS G12C mutation, targeted drugs AMG510 and MRTX849 have been approved for marketing for the treatment of non-small cell lung cancer with KRAS G12C mutation. However, for KRAS G12D and KRAS G12V mutations that commonly occur in pancreatic cancer and colorectal cancer, as well as NRAS and HRAS mutations commonly found in melanoma and head and neck cancer, no corresponding targeted drugs have been approved. This represents a significant unmet clinical need.

Currently, the pan-RAS inhibitor, RMC-6236, for various RAS mutations and RAS wild-type tumors is undergoing phase I clinical trials. Preliminary clinical trial results show that RMC-6236 has certain efficacy on lung cancer and pancreatic cancer patients with KRAS G12D, KRAS G12V, and other mutations, but also shows significant skin toxicity. This toxicity presumably results from the inhibitory effect of pan-RAS inhibitors on normal skin tissue cells. If a pan-RAS inhibitor is conjugated to an antibody via a linker, the toxic and side effects of the RAS inhibitor on normal tissue cells can be reduced or avoided by fully utilizing the specificity of the antibody for targeting tumor cells and the high efficiency of the RAS inhibitor. This means that compared with pan-RAS inhibitors, the antibody-conjugated pan-RAS inhibitor can accurately target tumor cells and reduce the effect on non-tumor cells, and is expected to improve the therapeutic window and enhance the safety of the pan-RAS inhibitor.

Therefore, the development of highly effective inhibitors targeting various RAS mutations and antibody conjugates thereof is of great clinical significance, and also represents both a focus and a challenge in the field of new drug research and development.

### SUMMARY

The present application relates to an antibody-drug conjugate, a linker-drug, and a macrocyclic compound. Experiments show that the antibody-drug conjugate of the present application has excellent cytostatic effect and anti-tumor effect; the compound of the present application has excellent protein binding effect, cytostatic effect, liver microsome stability, solubility, and hydrophilicity. The present application further relates to a pharmaceutical composition comprising the antibody-drug conjugate or the compound, and use of the antibody-drug conjugate, the compound, or the pharmaceutical composition in the preparation of a medicament for treating, preventing, and/or treating a disease or disorder associated with RAS protein activity.

### Antibody-Drug Conjugate

A first aspect of the present disclosure provides an antibody-drug conjugate represented by formula (I): 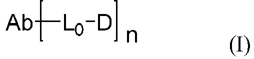
wherein Ab is an antibody or a fragment thereof that binds to a target;
n is 1-20 and may be an integer or non-integer;
D is an RAS protein-targeting inhibitor fragment;
L₀ is a linker linking Ab and D.

In one embodiment, 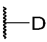 is a structure represented by formula (VI-1), formula (VI-2), formula (VI-3), formula (VI-4), formula (VI-5), or formula (VI-6), or a stable deuterated derivative thereof, or a stereoisomer thereof, 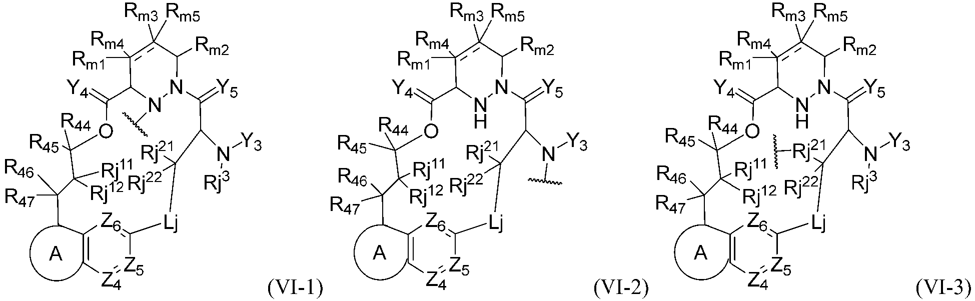 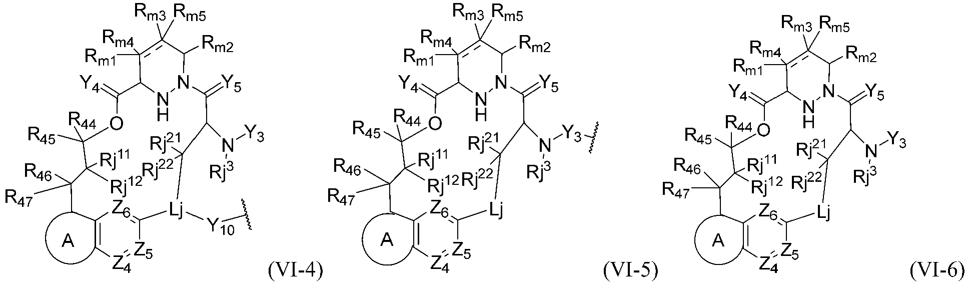 wherein:
in formula (VI-1), formula (VI-2), formula (VI-3), formula (VI-4), and formula (VI-5), the structural unit 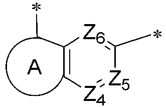 is 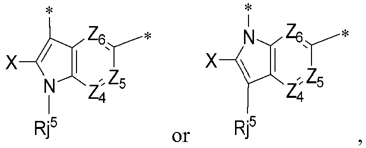 and X is 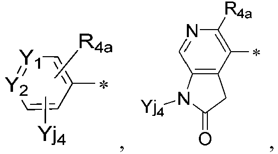 or 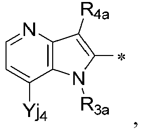
in formula (VI-6), the structural unit 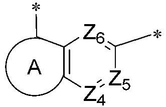 is 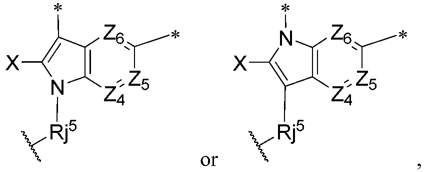
and X is 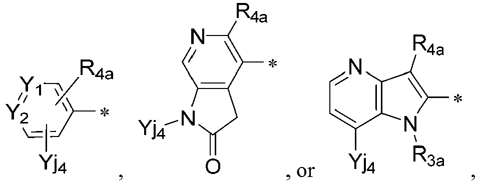 or
the structural unit 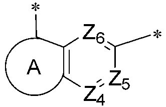 is 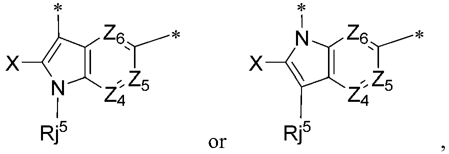 and X is 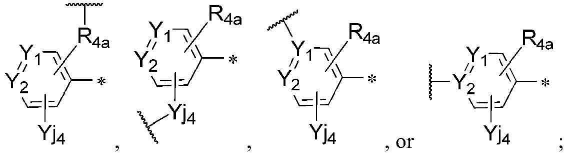
the 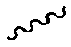 end is attached to L₀, and the * end is attached to another fragment in D;
when L₀ is attached to D, the hydrogen atom on D is replaced by L₀, or the N atom on D is quaternized;
Y₁ and Y₂ are each independently N, CH, CR₄ₐ, CR_{4b}, or CYj₄, wherein N may be oxidized (N⁺-O⁻);
Z₄, Z₅, and Z₆ are each independently N, CR₁₃, CR₁₄, CR₄₈, CR₄₉, or CR₅₀;
R₁₃ and R₁₄ are each independently hydrogen, halogen, C₁₋₆ alkyl optionally substituted with one or more (e.g., 1, 2, 3, 4, 5, or 6) S1, or -P(=O)-(C₁₋₆ alkyl)₂, or
R₁₃ and R₁₄, together with the carbon atom to which they are attached, form C₃₋₆ cycloalkyl optionally substituted with one or more (e.g., 1, 2, 3, 4, 5, or 6) S1 or 3- to 8-membered heterocyclyl optionally substituted with one or more (e.g., 1, 2, 3, 4, 5, or 6) S1;
R₄₈, R₄₉, and R₅₀ are each independently hydrogen, deuterium, halogen, C₁₋₄ alkyl, C₁₋₄ haloalkyl, or C₁₋₄ alkoxy;
R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, 8- to 10-membered bicyclic heteroaryl, or - P(=O)-(C₁₋₆ alkyl)₂, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
R₄ₐ is 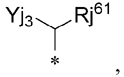 C₁₋₆ alkyl, C₃₋₆ cycloalkyl, or 3- to 8-membered heterocyclyl, wherein the C₁₋₆ alkyl, C₃₋₆ cycloalkyl, or 3- to 8-membered heterocyclyl is optionally substituted with one or more (e.g., 1, 2, 3, 4, 5, or 6) S1;
Yj₃ is NH-Rj⁶, O-Rj⁶, NRj⁶²Rj⁶, -C₁₋₄ alkylene-NH-Rj⁶, -C₁₋₄ alkylene-NRj⁶²Rj⁶, or -C₁₋₄ alkylene-O-Rj⁶;
Rj⁶ is hydrogen or C₁₋₆ alkyl;
Rj⁶² is hydrogen or C₁₋₆ alkyl;
Rj⁶¹ is C₁₋₆ alkyl;
R_{4b} is deuterium, halogen, C₁₋₆ alkyl optionally substituted with one or more (e.g., 1, 2, 3, 4, 5, or 6) S1, or C₁₋₆ heteroalkyl optionally substituted with one or more (e.g., 1, 2, 3, 4, 5, or 6) S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, - C₂₋₄ alkenylene-NR₀₁R₀₂, -C₂₋₄ alkenylene-C(R₀₅)R₀₃R₀₄, -C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂ (-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 3- to 8-membered monocyclic heterocyclyl, 7- to 12-membered bicyclic heterocyclyl, C₆₋₁₀ aryl, 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
R₀₁ and R₀₂ are each independently hydrogen, C₁₋₆ alkyl, C₁₋₆ alkoxy, 3- to 6-membered cycloalkyl, 3- to 8-membered monocyclic heteroalkyl, C₂₋₆ alkenyl, C₂₋₆ alkynyl, or (C₁₋₆ alkyl)₃-Si-, wherein the C₁₋₆ alkyl, C₁₋₆ alkoxy, 3- to 6-membered cycloalkyl, 3- to 8-membered monocyclic heteroalkyl, C₂₋₆ alkenyl, or C₂₋₆ alkynyl is optionally substituted with one or more (e.g., 1, 2, 3, 4, 5, or 6) S1, or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with one or more (e.g., 1, 2, 3, 4, 5, or 6) S1, or
R₀₁ and R₀₂ together form 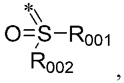 wherein R₀₀₁ and R₀₀₂, together with the sulfur atom to which they are attached, form 3- to 8-membered monocyclic heterocyclyl optionally substituted with one or more (e.g., 1, 2, 3, 4, 5, or 6) S1;
R₀₃ and R₀₄ are each independently hydrogen, C₁₋₆ alkyl, C₁₋₆ alkoxy, C₂₋₆ alkenyl, or C₂₋₆ alkynyl, wherein the C₁₋₆ alkyl, C₁₋₆ alkoxy, C₂₋₆ alkenyl, or C₂₋₆ alkynyl is optionally substituted with one or more (e.g., 1, 2, 3, 4, 5, or 6) S1, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6, or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5-or 6-membered monocyclic heteroaryl, or 8- to 10-membered bicyclic heteroaryl is optionally substituted with one or more (e.g., 1, 2, 3, 4, 5, or 6) S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₄ is O or S;
Y₅ is O or S;
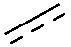 is a single bond or a double bond;
Rₘ₁ and Rₘ₃ are absent or are hydrogen, Rₘ₂ is hydrogen, and Rₘ₄ and Rₘ₅ are each independently hydrogen, halogen, or C₁₋₆ alkyl; or
Rₘ₁ is attached to Rₘ₂ to form -CRₘ₇Rₘ₆- or -CRₘ₇Rₘ₆-CRₘ₉Rₘ₈-; or
Rₘ₁ is attached to Rₘ₃ to form -CRₘ₇Rₘ₆- or -CRₘ₇Rₘ₆-CRₘ₉Rₘ₈-; or
Rₘ₁ is attached to Rₘ₄ to form -CRₘ₇Rₘ₆- or -CRₘ₇Rₘ₆-CRₘ₉Rₘ₈-;
Rₘ₆, Rₘ₇, Rₘ₈, and Rₘ₉ are each independently hydrogen, halogen, or C₁₋₆ alkyl;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
Y₁₀ is a bond, O, NH, or N(C₁₋₄ alkyl);
R₀₀ is hydrogen or C₁₋₆ alkyl;
Rj²¹ is hydrogen, deuterium, halogen, hydroxy, amino, C₁₋₆ alkyl, C₁₋₆ heteroalkyl optionally substituted with one or more (e.g., 1, 2, 3, 4, 5, or 6) S1, -NRj^{f}Rj^{g}, or -O-C₁₋₄ alkylene-NRj^{f}Rj^{g};
Rj²² is hydrogen, deuterium, halogen, C₁₋₆ alkyl, C₁₋₆ heteroalkyl optionally substituted with one or more (e.g., 1, 2, 3, 4, 5, or 6) S1, -NRj^{f}Rj^{g}, or -O-C₁₋₄ alkylene-NRj^{f}Rj^{g};
Rj^{f} is hydrogen or C₁₋₆ alkyl;
Rj^{g} is hydrogen or C₁₋₆ alkyl;
Rj¹¹ and Rj¹² are each independently hydrogen, halogen, or C₁₋₆ alkyl optionally substituted with one or more (e.g., 1, 2, 3, 4, 5, or 6) S1; or
Rj¹¹ and Rj¹², together with the carbon atom to which they are attached, form C₃₋₆ cycloalkyl optionally substituted with one or more (e.g., 1, 2, 3, 4, 5, or 6) S1 or 3- to 8-membered heterocyclyl optionally substituted with one or more (e.g., 1, 2, 3, 4, 5, or 6) S1;
R₄₄, R₄₅, R₄₆, and R₄₇ are each independently hydrogen, deuterium, halogen, C₁₋₄ alkyl, C₁₋₄ haloalkyl, or C₁₋₄ alkoxy;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁;
Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with one or more (e.g., 1, 2, 3, 4, 5 or 6) S1; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, or heteroaryl, wherein the C₁₋₆ alkyl, C₁₋₆ heteroalkyl, C₃₋₆ cycloalkyl, 3- to 8-membered heterocyclyl, C₆₋₁₀ aryl, or heteroaryl is optionally substituted with one or more (e.g., 1, 2, 3, 4, 5, or 6) S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with one or more (e.g., 1, 2, 3, 4, 5, or 6) S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form 3- to 8-membered heterocyclyl optionally substituted with one or more (e.g., 1, 2, 3, 4, 5, or 6) S1;
in the groups described above, each S1 is independently selected from the group consisting of: deuterium, oxo (=O), thio (=S), =CR^{e}R^{f}, =NR^{e}, SF₅, halogen, cyano, hydroxy, carboxyl, nitro, C₁₋₆ alkyl, C₂₋₆ alkenyl, C₂₋₆ alkynyl, C₁₋₆ alkoxy, C₁₋₆ heteroalkyl, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ heteroalkylene-C₁₋₄ heteroalkylene-C₁₋₆ heteroalkyl, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, - C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, - C₁₋₄ alkylene-O-5- or 6-membered monocyclic heteroaryl, -C₁₋₄ alkylene-8- to 10-membered bicyclic heteroaryl, -C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₄ alkylene-C(=O)O-C₁₋₆ alkyl, -C₁₄ alkylene-C(=O)O-C₃₋₂₀ cycloalkyl, -C₁₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, -N=S(=O)(R^{g1})(R^{h1}), and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with one or more (e.g., 1, 2, or 3) groups selected from halogen, deuterium, cyano, hydroxy, and amino; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with one or more (e.g., 1, 2, 3, or 4) groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(O)H, -C(O)-C₁₋₄ alkylene-OH, -C(=O)-C₃₋₆ monocyclic cycloalkyl, -C(=O)-3- to 6-membered monocyclic heterocyclyl, or -C(=O)-5- to 8-membered heterocyclyl, wherein the C₃₋₆ monocyclic cycloalkyl, 3-to 6-membered monocyclic heterocyclyl, 5- to 8-membered heterocyclyl, phenyl, 5- or 6-membered monocyclic heteroaryl, or 8- to 10-membered bicyclic heteroaryl is optionally substituted with one or more (e.g., 1 or 2) groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, -C(O)C₁₋₆ alkyl, -C(O)OC₁₋₆ alkyl, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -(C=O)OC₁₋₆alkyl, -C₁₋₄ alkylene-P(=O)-(C₁₋₆ alkyl)₂, - P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, C₃₋₆ cycloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, 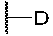 is a structure represented by formula (VI-1), formula (VI-2), formula (VI-3), formula (VI-4), formula (VI-5), or formula (VI-6), or a stable deuterated derivative thereof, or a stereoisomer thereof, 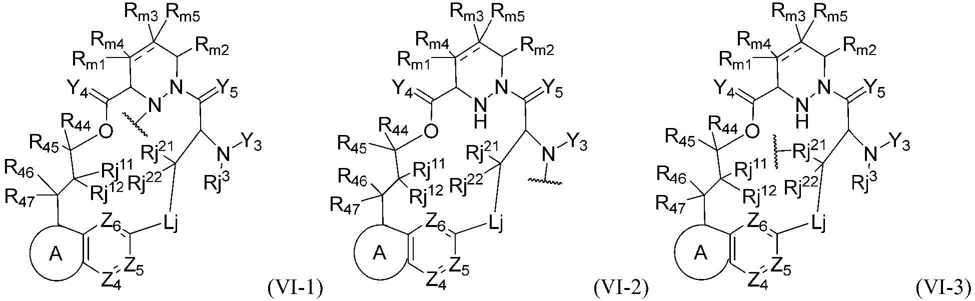 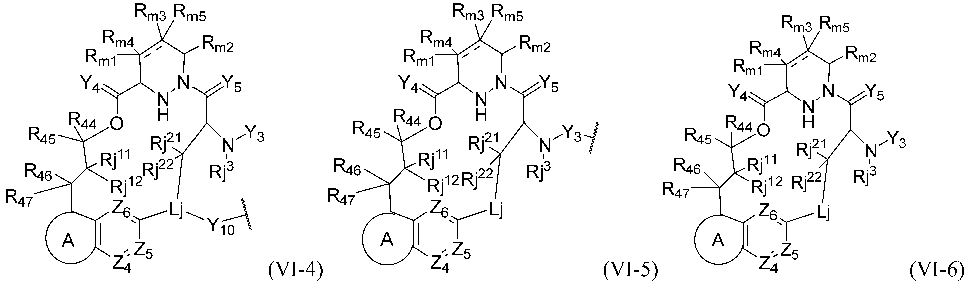 wherein:
the structural unit 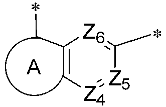 is 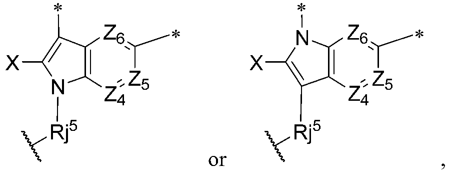 and X is 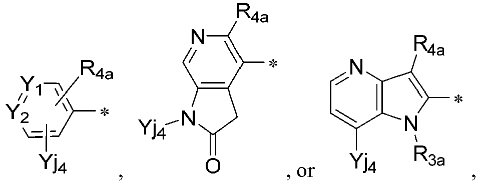 or
the structural unit 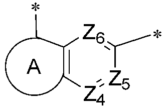 is 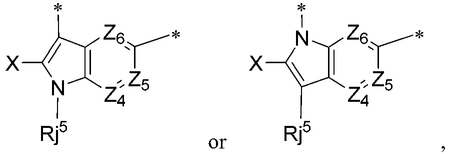 and X is 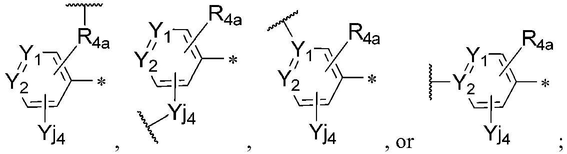
the 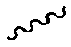 end is attached to L₀, and the * end is attached to another fragment in D;
when L₀ is attached to D, the hydrogen atom on D is replaced by L₀, or the N atom on D is quaternized;
Y₁ and Y₂ are each independently N, CH, CR₄ₐ, CR_{4b}, or CYj₄, wherein N may be oxidized (N⁺-O⁻);
Z₄, Z₅, and Z₆ are each independently N, CR₁₃, CR₁₄, CR₄₈, CR₄₉, or CR₅₀;
R₁₃ and R₁₄ are each independently hydrogen, halogen, C₁₋₆ alkyl optionally substituted with one or more (e.g., 1, 2, 3, 4, 5, or 6) S1, or -P(=O)-(C₁₋₆ alkyl)₂, or
R₁₃ and R₁₄, together with the carbon atom to which they are attached, form C₃₋₆ cycloalkyl optionally substituted with one or more (e.g., 1, 2, 3, 4, 5, or 6) S1 or 3- to 8-membered heterocyclyl optionally substituted with one or more (e.g., 1, 2, 3, 4, 5, or 6) S1;
R₄₈, R₄₉, and R₅₀ are each independently hydrogen, deuterium, halogen, C₁₋₄ alkyl, C₁₋₄ haloalkyl, or C₁₋₄ alkoxy; R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, 8- to 10-membered bicyclic heteroaryl, or - P(=O)-(C₁₋₆ alkyl)₂, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
R₄ₐ is 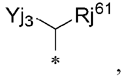 C₁₋₆ alkyl, C₃₋₆ cycloalkyl, or 3- to 8-membered heterocyclyl, wherein the C₁₋₆ alkyl, C₃₋₆ cycloalkyl, or 3- to 8-membered heterocyclyl is optionally substituted with one or more (e.g., 1, 2, 3, 4, 5, or 6) S1;
Yj₃ is NH-Rj⁶, O-Rj⁶, NRj⁶²Rj⁶, -C₁₋₄ alkylene-NH-Rj⁶, -C₁₋₄ alkylene-NRj⁶²Rj⁶, or -C₁₋₄ alkylene-O-Rj⁶;
Rj⁶ is hydrogen or C₁₋₆ alkyl;
Rj⁶² is hydrogen or C₁₋₆ alkyl;
Rj⁶¹ is C₁₋₆ alkyl;
R_{4b} is deuterium, halogen, C₁₋₆ alkyl optionally substituted with one or more (e.g., 1, 2, 3, 4, 5, or 6) S1, or C₁₋₆ heteroalkyl optionally substituted with one or more (e.g., 1, 2, 3, 4, 5, or 6) S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, - C₂₋₄ alkenylene-NR₀₁R₀₂, -C₂₋₄ alkenylene-C(R₀₅)R₀₃R₀₄, -C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂ (-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄, -S(=O)(-C₁₋₄ alkylene-C(R₀₅)R₀₃R₀₄, 3- to 8-membered monocyclic heterocyclyl, 7- to 12-membered bicyclic heterocyclyl, C₆₋₁₀ aryl, 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
R₀₁ and R₀₂ are each independently hydrogen, C₁₋₆ alkyl, C₁₋₆ alkoxy, 3- to 6-membered cycloalkyl, 3- to 8-membered monocyclic heteroalkyl, C₂₋₆ alkenyl, C₂₋₆ alkynyl, or (C₁₋₆ alkyl)₃-Si-, wherein the C₁₋₆ alkyl, C₁₋₆ alkoxy, 3- to 6-membered cycloalkyl, 3- to 8-membered monocyclic heteroalkyl, C₂₋₆ alkenyl, or C₂₋₆ alkynyl is optionally substituted with one or more (e.g., 1, 2, 3, 4, 5, or 6) S1, or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with one or more (e.g., 1, 2, 3, 4, 5, or 6) S1, or
R₀₁ and R₀₂ together form 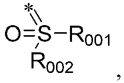 wherein R₀₀₁ and R₀₀₂, together with the sulfur atom to which they are attached, form 3- to 8-membered monocyclic heterocyclyl optionally substituted with one or more (e.g., 1, 2, 3, 4, 5, or 6) S1;
R₀₃ and R₀₄ are each independently hydrogen, C₁₋₆ alkyl, C₁₋₆ alkoxy, C₂₋₆ alkenyl, or C₂₋₆ alkynyl, wherein the C₁₋₆ alkyl, C₁₋₆ alkoxy, C₂₋₆ alkenyl, or C₂₋₆ alkynyl is optionally substituted with one or more (e.g., 1, 2, 3, 4, 5, or 6) S1, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6, or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5-or 6-membered monocyclic heteroaryl, or 8- to 10-membered bicyclic heteroaryl is optionally substituted with one or more (e.g., 1, 2, 3, 4, 5, or 6) S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₄ is O or S;
Y₅ is O or S;
is a single bond or a double bond;
Rₘ₁ and Rₘ₃ are absent or are hydrogen, Rₘ₂ is hydrogen, and Rₘ₄ and Rₘ₅ are each independently hydrogen, halogen, or C₁₋₆ alkyl; or
Rₘ₁ is attached to Rₘ₂ to form -CRₘ₇Rₘ₆- or -CRₘ₇Rₘ₆-CRₘ₉Rₘ₈-; or
Rₘ₁ is attached to Rₘ₃ to form -CRₘ₇Rₘ₆- or -CRₘ₇Rₘ₆-CRₘ₉Rₘ₈-; or
Rₘ₁ is attached to Rₘ₄ to form -CRₘ₇Rₘ₆- or -CRₘ₇Rₘ₆-CRₘ₉Rₘ₈-;
Rₘ₆, Rₘ₇, Rₘ₈, and Rₘ₉ are each independently hydrogen, halogen, or C₁₋₆ alkyl;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more (e.g., 1, 2, 3, 4, 5, or 6) S1;
Y₁₀ is a bond, O, NH, or N(C₁₋₄ alkyl);
R₀₀ is hydrogen or C₁₋₆ alkyl;
Rj²¹ is hydrogen, deuterium, halogen, hydroxy, amino, C₁₋₆ alkyl, C₁₋₆ heteroalkyl optionally substituted with one or more (e.g., 1, 2, 3, 4, 5, or 6) S1, -NRj^{f}Rj^{g}, or -O-C₁₋₄ alkylene-NRj^{f}Rj^{g};
Rj²² is hydrogen, deuterium, halogen, C₁₋₆ alkyl, C₁₋₆ heteroalkyl optionally substituted with one or more (e.g., 1, 2, 3, 4, 5, or 6) S1, -NRj^{f}Rj^{g}, or -O-C₁₋₄ alkylene-NRj^{f}Rj^{g};
Rj^{f} is hydrogen or C₁₋₆ alkyl;
Rj^{g} is hydrogen or C₁₋₆ alkyl;
Rj¹¹ and Rj¹² are each independently hydrogen, halogen, or C₁₋₆ alkyl optionally substituted with one or more (e.g., 1, 2, 3, 4, 5, or 6) S1; or
Rj¹¹ and Rj¹², together with the carbon atom to which they are attached, form C₃₋₆ cycloalkyl optionally substituted with one or more (e.g., 1, 2, 3, 4, 5, or 6) S1 or 3- to 8-membered heterocyclyl optionally substituted with one or more (e.g., 1, 2, 3, 4, 5, or 6) S1;
R₄₄, R₄₅, R₄₆, and R₄₇ are each independently hydrogen, deuterium, halogen, C₁₋₄ alkyl, C₁₋₄ haloalkyl, or C₁₋₄ alkoxy;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁;
Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with one or more (e.g., 1, 2, 3, 4, 5 or 6) S1; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, or heteroaryl, wherein the C₁₋₆ alkyl, C₁₋₆ heteroalkyl, C₃₋₆ cycloalkyl, 3- to 8-membered heterocyclyl, C₆₋₁₀ aryl, or heteroaryl is optionally substituted with one or more (e.g., 1, 2, 3, 4, 5, or 6) S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with one or more (e.g., 1, 2, 3, 4, 5, or 6) S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form 3- to 8-membered heterocyclyl optionally substituted with one or more (e.g., 1, 2, 3, 4, 5, or 6) S1;
in the groups described above, each S1 is independently selected from the group consisting of: deuterium, oxo (=O), thio (=S), =CR^{e}R^{f}, =NR^{e}, SF₅, halogen, cyano, hydroxy, carboxyl, nitro, C₁₋₆ alkyl, C₂₋₆ alkenyl, C₂₋₆ alkynyl, C₁₋₆ alkoxy, C₁₋₆ heteroalkyl, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C=C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ heteroalkylene-C₁₋₄ heteroalkylene-C₁₋₆ heteroalkyl, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, - C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, - C₁₋₄ alkylene-O-5- or 6-membered monocyclic heteroaryl, -C₁₋₄ alkylene-8- to 10-membered bicyclic heteroaryl, -C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NRₐ₁R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)2-R^{c1}, -S(=O)₂-NR^{a1}R^{b1}, -NR^{d1}-S(=O)₂-NR^{a1}R^{b1}, - N=S(=O)(R^{g1})(R^{h1}), and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with one or more (e.g., 1, 2, or 3) groups selected from halogen, deuterium, cyano, hydroxy, and amino; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with one or more (e.g., 1, 2, 3, or 4) groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(O)H, -C(O)-C₁₋₄ alkylene-OH, -C(=O)-C₃₋₆ monocyclic cycloalkyl, -C(=O)-3- to 6-membered monocyclic heterocyclyl, or -C(=O)-5- to 8-membered heterocyclyl, wherein the C₃₋₆ monocyclic cycloalkyl, 3-to 6-membered monocyclic heterocyclyl, 5- to 8-membered heterocyclyl, phenyl, 5- or 6-membered monocyclic heteroaryl, or 8- to 10-membered bicyclic heteroaryl is optionally substituted with one or more (e.g., 1 or 2) groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, -C(O)C₁₋₆ alkyl, -C(O)OC₁₋₆ alkyl, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -(C=O)OC₁₋₆alkyl, -C₁₋₄ alkylene-P(=O)-(C₁₋₆ alkyl)₂, - P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, Rₘ₁ is hydrogen; Rₘ₂ is hydrogen; Rₘ₃ is hydrogen; Rₘ₄ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₅ is hydrogen, halogen (e.g., fluorine, chlorine, or bromine), or C₁₋₆ alkyl (e.g., C₁₋₄ alkyl, such as methyl, ethyl, n-propyl, or isopropyl); and 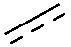 is a single bond.

In one embodiment, Rₘ₁ and Rₘ₃ are both absent; Rₘ₂ is hydrogen; Rₘ₄ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₅ is hydrogen, halogen (e.g., fluorine, chlorine, or bromine), or C₁₋₆ alkyl (e.g., C₁₋₄ alkyl, such as methyl, ethyl, n-propyl, or isopropyl); and 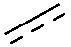 is a double bond.

In one embodiment, Rₘ₁ is attached to Rₘ₂ to form -CRₘ₇Rₘ₆- or -CRₘ₇Rₘ₆-CRₘ₉Rₘ₈-; Rₘ₃ is hydrogen; Rₘ₄ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₅ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₆, Rₘ₇, Rₘ₈, and Rₘ₉ are each independently hydrogen, halogen, or C₁₋₆ alkyl; and 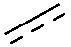 is a single bond.

In one embodiment, Rₘ₁ is attached to Rₘ₃ to form -CRₘ₇Rₘ₆- or -CRₘ₇Rₘ₆-CRₘ₉Rₘ₈-; Rₘ₂ is hydrogen; Rₘ₄ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₅ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₆, Rₘ₇, Rₘ₈, and Rₘ₉ are each independently hydrogen, halogen (e.g., fluorine, chlorine, or bromine), or C₁₋₆ alkyl (e.g., C₁₋₄ alkyl, such as methyl, ethyl, n-propyl, or isopropyl); and 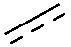 is a single bond.

In one embodiment, Rₘ₁ is attached to Rₘ₄ to form -CRₘ₇Rₘ₆- or -CRₘ₇Rₘ₆-CRₘ₉Rₘ₈-; Rₘ₂ is hydrogen; Rₘ₃ is hydrogen; Rₘ₅ is hydrogen, halogen (e.g., fluorine, chlorine, or bromine), or C₁₋₆ alkyl (e.g., C₁₋₄ alkyl, such as methyl, ethyl, n-propyl, or isopropyl); Rₘ₆, Rₘ₇, Rₘ₈, and Rₘ₉ are each independently hydrogen, halogen, or C₁₋₆ alkyl (e.g., C₁₋₄ alkyl, such as methyl, ethyl, n-propyl, or isopropyl); and 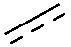 is a single bond.

In one embodiment, the structural unit 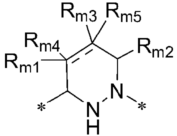 is 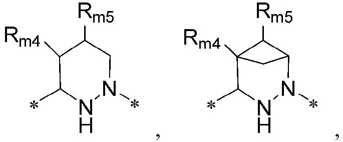 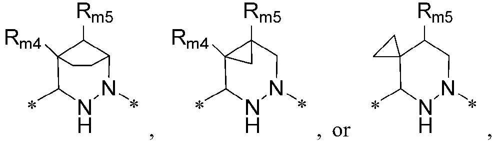 and Rₘ₄ and Rₘ₅ are as defined in any one of the embodiments.

In one embodiment, the structural unit 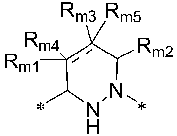 is

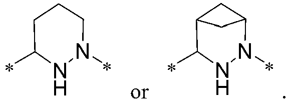

In one embodiment, the structural unit 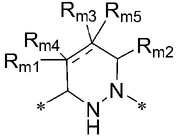 is

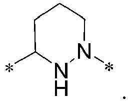

In one embodiment, the structural unit 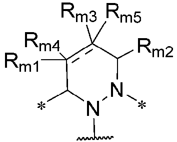 is 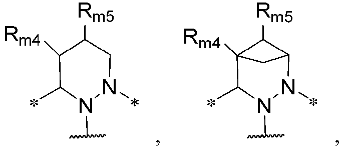 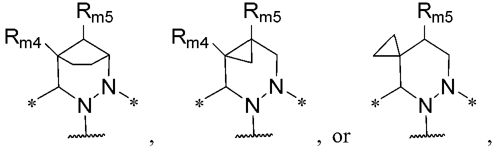 and Rₘ₄ and Rₘ₅ are as defined in any one of the embodiments.

In one embodiment, the structural unit 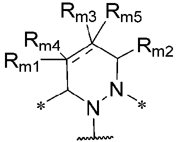 is 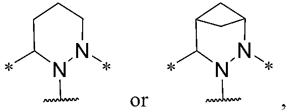 wherein the 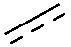 end is attached to L₀, and the * end is attached to another fragment in D.

In one embodiment, the structural unit 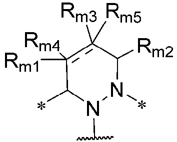 is 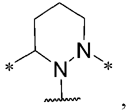 wherein the 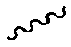 end is attached to L₀, and the * end is attached to another fragment in D.

In one embodiment, 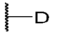 is a structure represented by formula (II-1), formula (II-2), formula (II-3), or formula (II-4), or a stable deuterated derivative thereof, or a stereoisomer thereof: 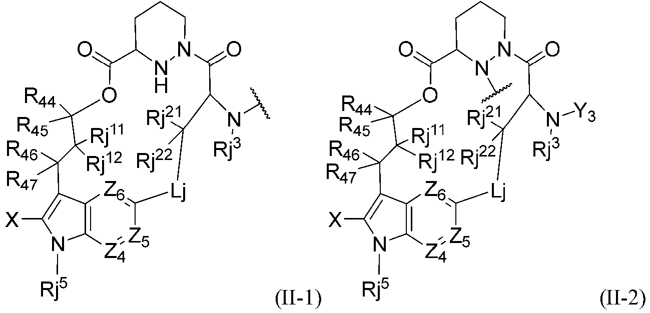 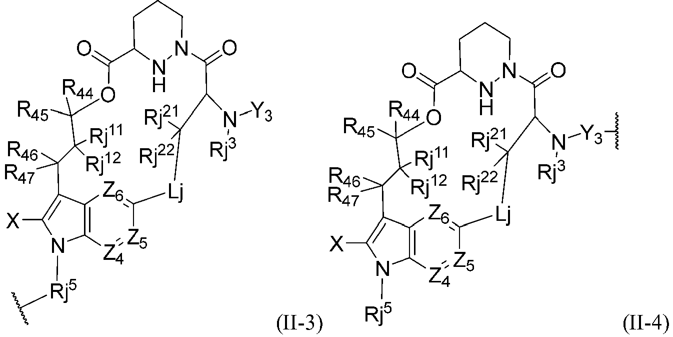 wherein in the formulas,
X is 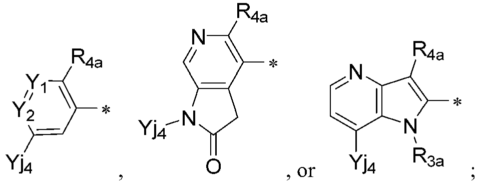
the 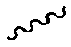 end is attached to L₀, and the * end is attached to another fragment in D;
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₀₀ is hydrogen or C₁₋₆ alkyl;
Rj²¹ is hydrogen, deuterium, halogen, hydroxy, C₁₋₆ alkyl, or C₁₋₆ heteroalkyl optionally substituted with 1, 2, 3, 4, 5, or 6 S1;
Rj²² is hydrogen, deuterium, halogen, C₁₋₆ alkyl, or C₁₋₆ heteroalkyl optionally substituted with 1, 2, 3, 4, 5, or 6 S1;
R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₃₋₆ cycloalkyl, or 3-to 8-membered heterocyclyl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₀₆)C(-O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5- or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁; Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with 1, 2, 3, 4, 5, or 6 S1; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with 1, 2, 3, 4, 5, or 6 S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, 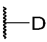 is a structure represented by formula (II-5), or a stable deuterated derivative thereof, or a stereoisomer thereof: 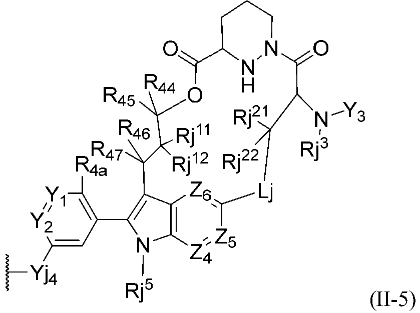 wherein in the formula,
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, *Z₅,* ₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₀₀ is hydrogen or C₁₋₆ alkyl;
Rj²¹ is hydrogen, deuterium, halogen, hydroxy, C₁₋₆ alkyl, or C₁₋₆ heteroalkyl optionally substituted with 1, 2, 3, 4, 5, or 6 S1;
Rj²² is hydrogen, deuterium, halogen, C₁₋₆ alkyl, or C₁₋₆ heteroalkyl optionally substituted with 1, 2, 3, 4, 5, or 6 S1;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₃₋₆ cycloalkyl, or 3-to 8-membered heterocyclyl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(-O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5- or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁; Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with 1, 2, 3, 4, 5, or 6 S1; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with 1, 2, 3, 4, 5, or 6 S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)2-R^{c1}, -S(=O)₂-NR^{a1}R^{b1}, -NR^{d1}-S(=O)2-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, 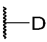 is a structure represented by formula (II-6), or a stable deuterated derivative thereof, or a stereoisomer thereof: 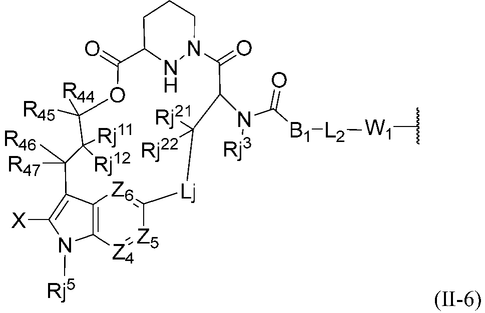 wherein in the formula,
X is 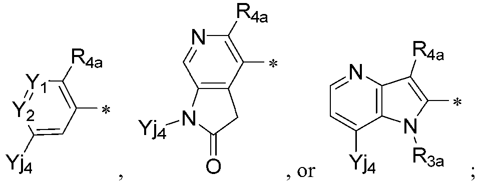
the 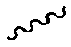 end is attached to L₀, and the * end is attached to another fragment in D;
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₀₀ is hydrogen or C₁₋₆ alkyl;
Rj²¹ is hydrogen, deuterium, halogen, hydroxy, C₁₋₆ alkyl, or C₁₋₆ heteroalkyl optionally substituted with 1, 2, 3, 4, 5, or 6 S1;
Rj²² is hydrogen, deuterium, halogen, C₁₋₆ alkyl, or C₁₋₆ heteroalkyl optionally substituted with 1, 2, 3, 4, 5, or 6 S1;
R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5- or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Rj³ is hydrogen, C₁₋₆ alkyl, or deuterated C₁₋₆ alkyl;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above;
B₁ is absent or is -CH(R₉)- or >C=CR₉R_{9'}, optionally substituted 3- to 6-membered cycloalkylene, optionally substituted 3- to 6-membered heterocycloalkylene, optionally substituted 6-membered arylene, or 5- to 6-membered heteroarylene;
L₂ is absent or is a linker;
W₁ is absent or is hydrogen, cyano, optionally substituted amino, optionally substituted C₁₋₄ alkoxy, optionally substituted C₁₋₄ hydroxyalkyl, optionally substituted C₁₋₄ aminoalkyl, optionally substituted C₁₋₄ haloalkyl, optionally substituted C₁₋₄ alkyl, optionally substituted C₁₋₄ guanidinoalkyl, 3- to 11-membered heterocycloalkyl optionally substituted with C₀₋₄ alkyl, optionally substituted 3- to 8-membered cycloalkyl, optionally substituted 6- to 10-membered aryl, or optionally substituted 3- to 8-membered heteroaryl;
R₉ is hydrogen, F, optionally substituted C₁₋₆ alkyl, optionally substituted C₁₋₆ heteroalkyl, optionally substituted 3- to 6-membered cycloalkyl, or optionally substituted 3- to 7-membered heterocycloalkyl; or
R₉ and L₂, together with the atom(s) to which they are attached, form optionally substituted 3- to 14-membered heterocycloalkyl;
R_{9'} is hydrogen or optionally substituted C₁₋₆ alkyl.

In one embodiment, 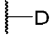 is a structure represented by formula (II-6a), or a stable deuterated derivative thereof, or a stereoisomer thereof: 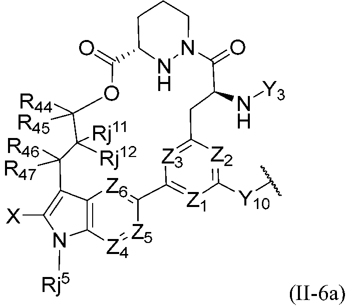 wherein in the formula,
Z₁ is C(H), C(deuterium), or N;
Z₂ is C(H), C(deuterium), or N;
Z₃ is C(H), C(deuterium), or N;
Z₄, *Z₅,* ₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Y₁₀ is a bond, O, NH, or N(C₁₋₄ alkyl);
X is 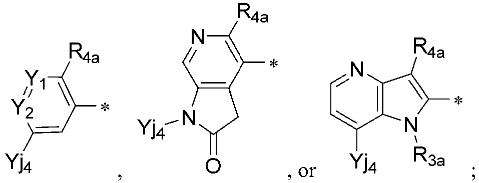
the 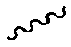 end is attached to L₀, and the * end is attached to another fragment in D;
Y₁ is N or CH, wherein N may be oxidized (N⁺-O⁻);
Y₂ is N or CH, wherein N may be oxidized (N⁺-O⁻);
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
R₄₄, R₄₅, R₄₆, and R₄₇ are each independently H, deuterium, halogen, C₁₋₄ alkyl, C₁₋₄ haloalkyl, or C₁₋₄ alkoxy; R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₃₋₆ cycloalkyl, or 3-to 8-membered heterocyclyl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(-O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5-or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5-or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁;
Rj⁴ is C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with 1, 2, 3, 4, 5, or 6 S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

In one embodiment, Z₄, Z₅, and Z₆ are each independently CR₁₃, CR₁₄, CR₄₈, CR₄₉, or CR₅₀, wherein R₁₃, R₁₄, R₄₈, R₄₉, or R₅₀ is as defined in any one of the embodiments.

In one embodiment, R₁₃ and R₁₄ are each independently hydrogen, halogen, or C₁₋₄ alkyl optionally substituted with 1, 2, 3, 4, 5, or 6 S1. In one embodiment, R₁₃ and R₁₄ are each independently hydrogen, fluorine, chlorine, bromine, methyl, ethyl, or halomethyl. In one embodiment, R₁₃ and R₁₄ are each independently hydrogen.

In one embodiment, R₁₃ and R₁₄, together with the carbon atom to which they are attached, form C₃₋₆ cycloalkyl or 3- to 7-membered heterocyclyl.

In one embodiment, R₄₈, R₄₉, and R₅₀ are each independently hydrogen, deuterium, halogen, C₁₋₄ alkyl, or C₁₋₄ haloalkyl. In one embodiment, R₄₈, R₄₉, and R₅₀ are each independently hydrogen, deuterium, fluorine, chlorine, bromine, methyl, ethyl, or halomethyl. In one embodiment, R₄₈, R₄₉, and R₅₀ are each independently hydrogen. In one embodiment, Z₄, Z₅, and Z₆ are each independently CH.

In one embodiment, R₃ₐ is C₁₋₄ alkyl or deuterated C₁₋₄ alkyl. In one embodiment, R₃ₐ is methyl or deuterated methyl.

In one embodiment, Rj⁵ is hydrogen, C₁₋₆ alkyl, C₁₋₆ heteroalkyl, C₂₋₆ alkenyl, C₂₋₆ alkynyl, C₃₋₆ cycloalkyl, or 3- to 8-membered heterocyclyl, wherein the C₁₋₆ alkyl, C₁₋₆ heteroalkyl, C₂₋₆ alkenyl, C₂₋₆ alkynyl, C₃₋₆ cycloalkyl, or 3- to 8-membered heterocyclyl is optionally substituted with 1, 2, 3, 4, 5, or 6 S1, and S1 is as defined in any one of the embodiments. In one embodiment, Rj⁵ is hydrogen, C₁₋₆ alkyl, C₁₋₆ heteroalkyl, or C₃₋₆ cycloalkyl, wherein the C₁₋₆ alkyl, C₁₋₆ heteroalkyl, or C₃₋₆ cycloalkyl is optionally substituted with 1, 2, 3, 4, 5, or 6 S1, and S1 is as defined in any one of the embodiments. In one embodiment, each S1 is independently hydrogen, hydroxy, cyano, amino, -C₁₋₄ alkyl, NH(C₁₋₄ alkyl), or N(C₁₋₄ alkyl)₂, wherein the C₁₋₄ alkyl is optionally substituted with 1, 2, or 3 substituents selected from OH and NH₂.

In one embodiment, Rj⁵ is hydrogen, C₁₋₆ alkyl, C₃₋₆ cycloalkyl, -C₁₋₄ alkylene-OH, -C₁₋₄ alkylene-NHC₁₋₆ alkyl, or -C₁₋₄ alkylene-C₁₋₆ alkoxy. In one embodiment, Rj⁵ is hydrogen, C₁₋₄ alkyl, -C₁₋₄ alkylene-OH, -C₁₋₄ alkylene-NHC₁₋₄ alkyl, or -C₁₋₄ alkylene-C₁₋₄ alkoxy. In one embodiment, Rj⁵ is hydrogen or -C₁₋₄ alkylene-OH.

In one embodiment, Rj⁵ is ethyl, cyclopropyl, -(CH₂)₂OH, -(CH₂)₃OH, -(CH₂)₂-NH(CH₃), or -(CH₂)₂-OCH₃. In one embodiment, Rj⁵ is ethyl, -(CH₂)₂OH, -(CH₂)₃OH, -(CH₂)₂-NH(CH₃), or -(CH₂)₂-OCH₃. In one embodiment, Rj⁵ is -CH₂CH₃ or -CH₂CH₂OCH₃.

In one embodiment, when L₀ is attached to Rj⁵, any hydrogen atom on Rj⁵ is replaced by L₀, wherein Rj⁵ is as defined in any one of the embodiments.

In one embodiment, when L₀ is attached to Rj⁵, Rj⁵ is 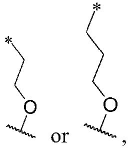 wherein the 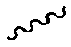 end is attached to L₀, and the * end is attached to another fragment in D. In one embodiment, when L₀ is attached to Rj⁵, Rj⁵ is 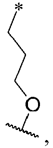 wherein the 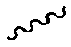 end is attached to L₀, and the * end is attached to another fragment in D.

In one embodiment, Y₁ and Y₂ are each independently N, N⁺-O⁻, CH, or CR_{4b}, wherein R_{4b} is deuterium, halogen, C₁₋₆ alkyl, or C₁₋₆ heteroalkyl. In one embodiment, R_{4b} is deuterium, halogen, C₁₋₆ alkyl, or C₁₋₆ alkoxy. In one embodiment, R_{4b} is halogen, C₁₋₄ alkyl, or C₁₋₄ alkoxy. In one embodiment, R_{4b} is chlorine, methyl, or methoxy. In one embodiment, Y₁ and Y₂ are each independently N, N⁺-O⁻, or CH. In one embodiment, Y₁ is N or N⁺-O⁻. In one embodiment, Y₂ is CH.

In one embodiment, when L₀ is attached to Y₁, any hydrogen atom on Y₁ is replaced by L₀, or any N atom on Y₁ is quaternized, wherein Y₁ is as defined in any one of the embodiments. In one embodiment, when L₀ is attached to Y₁, Y₁ is N⁺.

In one embodiment, R₄ₐ is 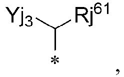 and Yj₃ is NH-Rj⁶, O-Rj⁶, NRj⁶²Rj⁶, -C₁₋₄ alkylene-NH-Rj⁶, -C₁₋₄ alkylene-NRj⁶²Rj⁶, or -C₁₋₄ alkylene-O-Rj⁶, wherein Rj⁶, Rj⁶², and Rj⁶¹ are as defined in any one of the embodiments, and the * end is attached to another fragment in D.

In one embodiment, Rj⁶¹ is C₁₋₆ alkyl. In one embodiment, Rj⁶¹ is C₁₋₄ alkyl. In one embodiment, Rj⁶¹ is methyl, ethyl, n-propyl, or isopropyl. In one embodiment, Rj⁶¹ is methyl.

In one embodiment, Rj⁶¹ is hydrogen.

In one embodiment, Rj⁶ is C₁₋₆ alkyl. In one embodiment, Rj⁶ is C₁₋₄ alkyl. In one embodiment, Rj⁶ is methyl, ethyl, n-propyl, or isopropyl. In one embodiment, Rj⁶ is methyl.

In one embodiment, Rj⁶ is hydrogen.

In one embodiment, Rj⁶² is hydrogen or C₁₋₄ alkyl. In one embodiment, Rj⁶² is hydrogen, methyl, ethyl, n-propyl, or isopropyl. In one embodiment, Rj⁶² is hydrogen.

In one embodiment, Yj₃ is NH-Rj⁶, O-Rj⁶, -C₁₋₄ alkylene-NH-Rj⁶, or -C₁₋₄ alkylene-O-Rj⁶, wherein Rj⁶ is as defined in any one of the embodiments. In one embodiment, Yj₃ is NH-Rj⁶ or O-Rj⁶, wherein Rj⁶ is as defined in any one of the embodiments. In one embodiment, Yj₃ is NHCH₃ or OCH₃. In one embodiment, Yj₃ is NHCH₃. In one embodiment, Yj₃ is OCH₃.

In one embodiment, R₄ₐ is C₁₋₆ alkyl optionally substituted with 1, 2, 3, 4, 5, or 6 S1. In one embodiment, R₄ₐ is C₁₋₆ alkyl substituted with hydroxy or C₁₋₄ heteroalkyl (e.g., methoxy or methylamino). In one embodiment, R₄ₐ is -C₁₋₄ alkylene-OH, -C₁₋₄ alkylene-O-C₁₋₄ alkyl, -C₁₋₄ alkylene-NH-C₁₋₄ alkyl, or -C₁₋₄ alkylene-N(C₁₋₄ alkyl)₂. In one embodiment, R₄ₐ is -CH(CH₃)OCH₃ or -CH(CH₃)NHCH₃.

In one embodiment, when L₀ is attached to R₄ₐ, any hydrogen atom on R₄ₐ is replaced by L₀, or any N atom on R₄ₐ is quaternized, wherein R₄ₐ is as defined in any one of the embodiments. In one embodiment, when L₀ is attached to R₄ₐ, R₄ₐ is 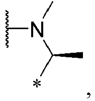 wherein the 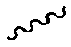 end is attached to L₀, and the * end is attached to another fragment in D.

In one embodiment, Yj₄ is -C₂₋₄ alkynylene-C₁₋₄ alkylene-NR₀₁R₀₂, -C₂₋₄ alkynylene-C₁₋₄ alkylene-3- to 8-membered monocyclic heterocyclyl, -O-C₁₋₄ alkylene-3- to 8-membered monocyclic heterocyclyl, 3- to 8-membered monocyclic heterocyclyl, 7- to 12-membered bicyclic heterocyclyl, or C₆₋₁₀ aryl, wherein the 3- to 8-membered monocyclic heterocyclyl, 7- to 12-membered bicyclic heterocyclyl, or C₆₋₁₀ aryl is optionally substituted with 1, 2, 3, 4, 5, or 6 S1, and R₀₁ and R₀₂ are each independently hydrogen, C₁₋₆ alkyl, or 3- to 8-membered monocyclic heteroalkyl, wherein the C₁₋₆ alkyl or 3- to 8-membered monocyclic heteroalkyl is optionally substituted with 1, 2, 3, 4, 5, or 6 S1, and S1 is as defined in any one of the embodiments.

In one embodiment, Yj₄ is -C₂₋₄ alkynylene-C₁₋₄ alkylene-NR₀₁R₀₂, -C₂₋₄ alkynylene-C₁₋₄ alkylene-3- to 8-membered monocyclic heterocyclyl, or -O-C₁₋₄ alkylene-3- to 8-membered monocyclic heterocyclyl, wherein the 3- to 8-membered monocyclic heterocyclyl is optionally substituted with 1, 2, 3, 4, 5, or 6 S1, and R₀₁ and R₀₂ are each independently hydrogen, C₁₋₆ alkyl, or 3- to 8-membered monocyclic heteroalkyl, wherein the C₁₋₆ alkyl or 3- to 8-membered monocyclic heteroalkyl is optionally substituted with 1, 2, 3, 4, 5, or 6 S1, and S1 is as defined in any one of the embodiments.

In one embodiment, Yj₄ is -C₂₋₄ alkynylene-C₁₋₄ alkylene-3- to 8-membered monocyclic heterocyclyl, or -O-C₁₋₄ alkylene-3- to 8-membered monocyclic heterocyclyl, wherein the 3- to 8-membered monocyclic heterocyclyl is optionally substituted with 1, 2, 3, 4, 5, or 6 S1, and S1 is as defined in any one of the embodiments. In one embodiment, the 3- to 8-membered monocyclic heterocyclyl contains 1, 2, or 3 heteroatoms selected from N, O, and S. In one embodiment, the 3- to 8-membered monocyclic heterocyclyl is azetidinyl, tetrahydropyrrolyl, piperidinyl, piperazinyl, thiomorpholinyl, homomorpholinyl, or homopiperazinyl. In one embodiment, the 3- to 8-membered monocyclic heterocyclyl is piperidinyl, piperazinyl, thiomorpholinyl, homomorpholinyl, or homopiperazinyl. In one embodiment, the 3- to 8-membered monocyclic heterocyclyl is piperidinyl or piperazinyl.

In one embodiment, each S1 is independently hydrogen, hydroxy, cyano, amino, sulfhydryl, halogen, carboxyl, nitro, -C₁₋₆ alkyl, -C₂₋₆ alkenyl, -C₂₋₆ alkynyl, -C₁₋₆ heteroalkyl, =O, =NH, -(CH₂CH₂U)pCH₃, -C(O)-C₁₋₄ alkyl, -C(O)-NH₂, -C(O)-NHC₁₋₆ alkyl, or -C(O)-N(C₁₋₆ alkyl)₂, wherein the -C₁₋₆ alkyl, -C₂₋₆ alkenyl, -C₂₋₆ alkynyl, or -C₁₋₆ heteroalkyl is optionally substituted with 1, 2, 3, 4, or 5 substituents selected from OH, NH₂, CN, SH, halogen, carboxyl, nitro, NH(C₁₋₆ alkyl), and N(C₁₋₆ alkyl)₂; each U is independently O or N; and p is 1, 2, 3, 4, or 5.

In one embodiment, each S1 is independently hydrogen, hydroxy, cyano, amino, -C₁₋₄ alkyl, =O, =NH, - (CH₂CH₂U)pCH₃, -C(O)-C₁₋₄ alkyl, -C(O)-NH₂, -C(O)-NHC₁₋₄ alkyl, or -C(O)-N(C₁₋₄ alkyl)₂, wherein the C₁₋₄ alkyl is optionally substituted with 1, 2, 3, 4, or 5 substituents selected from OH, NH₂, NH(C₁₋₄ alkyl), and N(C₁₋₄ alkyl)₂; each U is independently O or N; and p is 1, 2, 3, 4, or 5.

In one embodiment, each S1 is independently hydrogen, hydroxy, cyano, amino, methyl, =O, =NH, -CH₂OH, - CH₂CH₂OCH₂CH₂NHCH₂CH₂OCH₃, -CH₂NH₂, -CH(CH₃)NH₂, -CH₂NHCH₃, -C(O)CH₃, -C(O)CH₂OH, - C(O)CH₂NH₂, -C(O)CH₂NH₂CH₃, or -C(O)NHCH₂CH₂OH.

In one embodiment, each S1 is independently hydroxy, cyano, -C₁₋₄ alkylene-OH, -C₁₋₄ alkylene-NH₂, =O, =NH, -(CH₂CH₂U)pCH₃, -C(O)-C₁₋₄ alkylene-OH, or -C(O)-C₁₋₄ alkylene-NH₂, wherein each U is independently O or N, and p is 1, 2, 3, 4, or 5.

In one embodiment, each S1 is independently hydroxy, cyano, =O, =NH, -CH₂OH, - CH₂CH₂OCH₂CH₂NHCH₂CH₂OCH₃, -CH₂NH₂, -C(O)CH₂OH, or -C(O)CH₂NH₂.

In one embodiment, each S1 is independently cyano, =O, -CH₂CH₂OCH₂CH₂NHCH₂CH₂OCH₃, -CH₂NH₂, - C(O)CH₂OH, or -C(O)CH₂NH₂.

In one embodiment, Yj₄ is 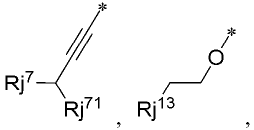 3- to 8-membered monocyclic heterocyclyl, 7- to 12-membered bicyclic heterocyclyl, or phenyl, wherein the 3- to 8-membered monocyclic heterocyclyl, 7- to 12-membered bicyclic heterocyclyl, or phenyl is optionally substituted with one or more substituents selected from C₁₋₆ alkyl, -C₁₋₄ alkylene-hydroxy, -C(=O)-C₁₋₄ alkylene-hydroxy, and -C₁₋₄ alkylene-3- to 8-membered monocyclic heterocyclyl;
each Rj⁷ or Rj¹³ is independently 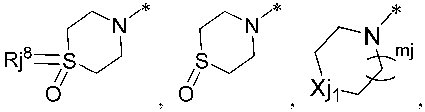 NR₀₁R₀₂, 4- to 5-membered monocyclic heterocyclyl, oxo-5-membered heterocyclyl-Lj₁-Lj₂-Rj¹⁴, oxo-6-membered heterocyclyl-Lj₁-Lj₂-Rj¹⁴, or oxo-7-membered heterocyclyl-Lj₁-Lj₂-Rj¹⁴, wherein the 4- to 5-membered monocyclic heterocyclyl is optionally substituted with hydroxy, cyano, or amino, and Rj⁷ is optionally substituted with -C₁₋₄ alkylene-OH or -C₁₋₄ alkylene-NH-Rj⁶;
Rj⁷¹ is hydrogen, C₁₋₆ alkyl, -C₁₋₄ alkylene-OH, or -C₁₋₄ alkylene-NH-Rj⁶;
Rj⁸ is O or NH;
Xj₁ is -O-, -CRj⁹¹Rj⁹²-, or -NRj¹⁰-;
mj is 1 or 2;
Lj₁ and Lj₂ are each independently -C₁₋₄ alkylene-O- or -C₁₋₄ alkylene-NH-;
Rj¹⁴ is C₁₋₆ heteroalkyl;
Rj⁶ is hydrogen or C₁₋₆ alkyl;
Rj⁹¹ and Rj⁹² are each independently hydrogen, hydroxy, cyano, -NRj^{c}Rj^{d}, -C₁₋₄ alkylene-NRj^{c}Rj^{d}, -C₁₋₄ alkylene-OH, -C(O)-NRj^{a}Rj^{b}, or -C(O)-NRj^{c}Rj^{d}, wherein the -C₁₋₄ alkylene- is optionally substituted with methyl;
Rj^{l0} is hydrogen, C₁₋₆ alkyl, -C(O)-C₁₋₆ alkyl, -C(O)-C₁₋₄ alkylene-NRj^{f}Rj^{g}, or -C(O)-C₁₋₄ alkylene-OH;
Rj^{a} and Rj^{b} are each independently hydrogen, C₁₋₆ alkyl, or C₃₋₆ cycloalkyl;
Rj^{c} and Rj^{d} are each independently hydrogen, -C(O)H, C₁₋₆ alkyl, -C₁₋₄ alkylene-OH, 5- to 8-membered heterocyclyl, -C(O)-5- to 8-membered heterocyclyl, or -C(O)-C₁₋₄ alkylene-OH, wherein the 5- to 8-membered heterocyclyl is optionally substituted with one or more substituents selected from Rj⁴²;
Rj⁴² is methyl, -NRj^{f}Rj^{g}, -C(O)Rj^{e}, or -C(O)ORj^{e};
Rj^{e} is C₁₋₆ alkyl;
Rj^{f} and Rj^{g} are each independently hydrogen or C₁₋₆ alkyl.

In one embodiment, Yj₄ is 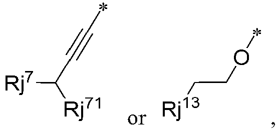 wherein Rj⁷, Rj⁷¹, and Rj¹³ are as defined in any one of the embodiments.

In one embodiment, Rj⁷¹ is hydrogen, C₁₋₄ alkyl, -C₁₋₄ alkylene-OH, or -C₁₋₄ alkylene-NH₂. In one embodiment, Rj⁷¹ is hydrogen, methyl, ethyl, -CH₂OH, or -CH₂NH₂. In one embodiment, Rj⁷¹ is hydrogen, -CH₂OH, or - CH₂NH₂. In one embodiment, Rj⁷¹ is hydrogen.

In one embodiment, Rj⁷¹ is -C₁₋₄ alkylene-OH. In one embodiment, Rj⁷¹ is -(CH₂)₃OH, -(CH₂)₂OH, or -CH₂OH. In one embodiment, Rj⁷¹ is hydrogen, C₁₋₆ alkyl, or -C₁₋₄ alkylene-OH.

In one embodiment, each Rj⁷ or Rj¹³ is independently 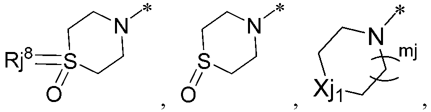 NR₀₁R₀₂, or oxo-6-membered heterocyclyl-Lj₁-Lj₂-Rj¹⁴, wherein the Rj⁷ is optionally substituted with -C₁₋₄ alkylene-OH or -C₁₋₄ alkylene-NH-Rj⁶; Rj⁸, Xj₁, mⱼ, R₀₁, R₀₂, Lj₁, Lj₂, and Rj¹⁴ are as defined in any one of the embodiments; the * end is attached to another fragment in D.

In one embodiment, Rj⁷ is 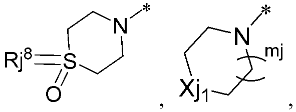 or NR₀₁R₀₂, wherein Rj⁷ is optionally substituted with -C₁₋₄ alkylene-OH; Rj⁸, Xj₁, mj, R₀₁, and R₀₂ are as defined in any one of the embodiments; the * end is attached to another fragment in D. In one embodiment, Rj⁷ is 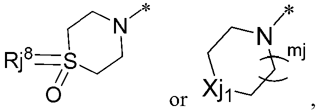 wherein Rj⁸, Xj₁, and mj are as defined in any one of the embodiments.

In one embodiment, Xj₁ is -CRj⁹¹Rj⁹²- or -NRj¹⁰-, wherein Rj⁹¹, Rj⁹², and Rj¹⁰ are as defined in any one of the embodiments, and the * end is attached to another fragment in D.

**In** one embodiment, Rj⁷ is 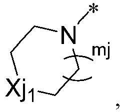 and Xj₁ is -CRj⁹¹Rj⁹²- or -NRj¹⁰-, wherein Rj⁹¹, Rj⁹², Rj¹⁰, and mj are as defined in any one of the embodiments, and the * end is attached to another fragment in D.

In one embodiment, R₀₁ and R₀₂ are each independently hydrogen, C₁₋₄ alkyl, or 4- to 7-membered monocyclic heteroalkyl. In one embodiment, R₀₁ and R₀₂ are each independently hydrogen, methyl, ethyl, n-propyl, isopropyl, oxetanyl, tetrahydrofuranyl, tetrahydropyranyl, azetidinyl, pyrrolidinyl, piperidinyl, piperazinyl, or morpholinyl. In one embodiment, R₀₁ and R₀₂ are each independently methyl, isopropyl, or tetrahydropyranyl.

In one embodiment, R₀₁ is methyl.

In one embodiment, R₀₂ is isopropyl or tetrahydropyranyl.

In one embodiment, Rj⁶ is hydrogen or C₁₋₄ alkyl. In one embodiment, Rj⁶ is hydrogen, methyl, ethyl, n-propyl, or isopropyl. In one embodiment, Rj⁶ is hydrogen.

In one embodiment, Rj⁹¹ and Rj⁹² are each independently hydrogen, hydroxy, cyano, -NRj^{c}Rj^{d}, -C₁₋₄ alkylene-NRj^{c}Rj^{d}, -C₁₋₄ alkylene-OH, or -C(O)-NRj^{c}Rj^{d}, wherein Rj^{c} and Rj^{d} are as defined in any one of the embodiments. In one embodiment, Rj⁹¹ and Rj⁹² are each independently hydrogen, hydroxy, cyano, -C₁₋₄ alkylene-OH, or-C(O)-NRj^{c}Rj^{d}, wherein Rj^{c} and Rj^{d} are as defined in any one of the embodiments.

In one embodiment, Rj⁹¹ and Rj⁹² are each independently hydrogen, hydroxy, cyano, amino, -NHC₁₋₄ alkyl, - N(C₁₋₄ alkyl)₂, -C₁₋₄ alkylene-OH, -C₁₋₄ alkylene-NH₂, -C₁₋₄ alkylene-NHC₁₋₄ alkyl, -C₁₋₄ alkylene-N(C₁₋₄ alkyl)₂, -C(O)-NH₂, -C(O)-NHC₁₋₄ alkyl, -C(O)-NHC₁₋₄ alkylene-OH, or -C(O)-N(C₁₋₄ alkyl)₂. In one embodiment, Rj⁹¹ and Rj⁹² are each independently hydrogen, hydroxy, cyano, amino, -C₁₋₄ alkylene-OH, -C₁₋₄ alkylene-NH₂, -C₁₋₄ alkylene-NHC₁₋₄ alkyl, or -C(O)-NHC₁₋₄ alkylene-OH. In one embodiment, Rj⁹¹ and Rj⁹² are each independently hydrogen, hydroxy, cyano, amino, -CH₂OH, -CH₂NH₂, -CH(CH₃)NH₂, -CH₂NHCH₃, or - C(O)NHCH₂CH₂OH.

In one embodiment, Rj⁹¹ and Rj⁹² are each independently hydrogen, hydroxy, cyano, -C(O)-C₁₋₄ alkyl, or -C(O)-C₁₋₄ alkylene-NH₂. In one embodiment, Rj⁹¹ and Rj⁹² are each independently hydrogen, hydroxy, cyano, -CH₂OH, or -CH₂NH₂.

In one embodiment, Rj⁹¹ is hydrogen, hydroxy, or cyano. In one embodiment, Rj⁹¹ is cyano.

In one embodiment, Rj⁹² is hydrogen, amino, -CH₂OH, -CH₂NH₂, or -C(O)NHCH₂CH₂OH.

In one embodiment, Rj⁹¹ and Rj⁹² are not both hydrogen.

In one embodiment, Rj¹⁰ is hydrogen, C₁₋₄ alkyl, -C(O)-C₁₋₄ alkyl, -C(O)-C₁₋₄ alkylene-NH₂, -C(O)-C₁₋₄ alkylene-NHC₁₋₄ alkyl, -C(O)-C₁₋₄ alkylene-N(C₁₋₄ alkyl)₂, or -C(O)-C₁₋₄ alkylene-OH.

In one embodiment, Rj¹⁰ is hydrogen, methyl, ethyl, n-propyl, isopropyl, -C(O)CH₃, -C(O)CH₂CH₃, - C(O)CH(CH₃)₂, -C(O)CH₂NH₂, -C(O)CH₂CH₂NH₂, -C(O)CH₂NHCH₃, -C(O)CH₂N(CH₃)₂, - C(O)CH₂CH₂NHCH₃, -C(O)CH₂OH, or -C(O)CH₂CH₂OH. In one embodiment, Rj¹⁰ is hydrogen, methyl, - C(O)CH₃, -C(O)CH₂OH, or -C(O)CH₂NH₂.

In one embodiment, Rj¹⁰ is hydrogen, -C(O)-C₁₋₄ alkylene-NH₂, or -C(O)-C₁₋₄ alkylene-OH. In one embodiment, Rj¹⁰ is hydrogen, -C(O)CH₂OH, or -C(O)CH₂NH₂. In one embodiment, Rj¹⁰ is -C(O)CH₂OH or -C(O)CH₂NH₂. In one embodiment, Rj^{a} and Rj^{b} are each independently hydrogen or C₁₋₄ alkyl. In one embodiment, Rj^{a} and Rj^{b} are each independently hydrogen, methyl, ethyl, n-propyl, or isopropyl. In one embodiment, Rj^{a} and Rj^{b} are each independently hydrogen.

In one embodiment, Rj^{c} and Rj^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from Rj⁴², and Rj⁴² is as defined in any one of the embodiments. In one embodiment, Rj^{c} and Rj^{d} are each independently hydrogen, -C(O)H, C₁₋₄ alkyl, -C₁₋₄ alkylene-OH, or - C(O)-C₁₋₄ alkylene-OH.

In one embodiment, Rj^{c} and Rj^{d} are each independently hydrogen, C₁₋₆ alkyl, -C₁₋₄ alkylene-OH, or -C(O)-C₁₋₄ alkylene-OH. In one embodiment, Rj^{c} and Rj^{d} are each independently hydrogen, C₁₋₄ alkyl, -C₁₋₄ alkylene-OH, or -C(O)-C₁₋₄ alkylene-OH. In one embodiment, Rj^{c} and Rj^{d} are each independently hydrogen, methyl, ethyl, n-propyl, isopropyl, -C(O)CH₃, -C(O)CH₂CH₃, -C(O)CH(CH₃)₂, -C(O)CH₂OH, or -C(O)CH₂CH₂OH.

In one embodiment, Rj⁴² is methyl, -NRj^{f}Rj^{g}, -C(O)Rj^{e}, or -C(O)ORj^{e}, wherein Rj^{f}, Rj^{g}, and Rj^{e} are as defined in any one of the embodiments. In one embodiment, Rj⁴² is methyl, -C(O)Rj^{e}, or -C(O)ORj^{e}, wherein Rj^{e} is as defined in any one of the embodiments.

In one embodiment, Rj^{e} is C₁₋₄ alkyl. In one embodiment, Rj^{e} is methyl, ethyl, n-propyl, or isopropyl. In one embodiment, Rj^{a} is methyl.

In one embodiment, Rj^{f} and Rj^{g} are each independently hydrogen or C₁₋₄ alkyl. In one embodiment, Rj^{f} and Rj^{g} are each independently hydrogen, methyl, ethyl, n-propyl, or isopropyl. In one embodiment, Rj^{f} and Rj^{g} are each independently hydrogen or methyl.

**In** one embodiment, Rj⁷ is 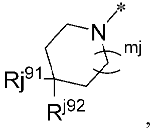 wherein Rj⁹¹ and Rj⁹² are each independently hydrogen, hydroxy, cyano, -NH₂, -CH₂NH₂, -CH₂NH(CH₃), -CH₂OH, or -C(O)-NH(CH₂CH₂OH); Rj⁹¹ and Rj⁹² are not both hydrogen; mj is 1 or 2; and the * end is attached to another fragment in D. In one embodiment, Rj⁹¹ is cyano.

In one embodiment, Rj⁷ is 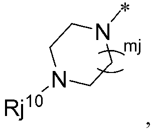 wherein Rj¹⁰ is hydrogen or -C(O)CH₂OH; mj is 1 or 2; and the * end is attached to another fragment in D.

In one embodiment, Rj⁷ is 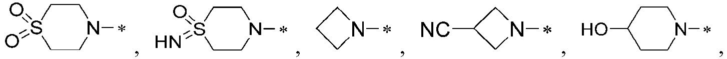 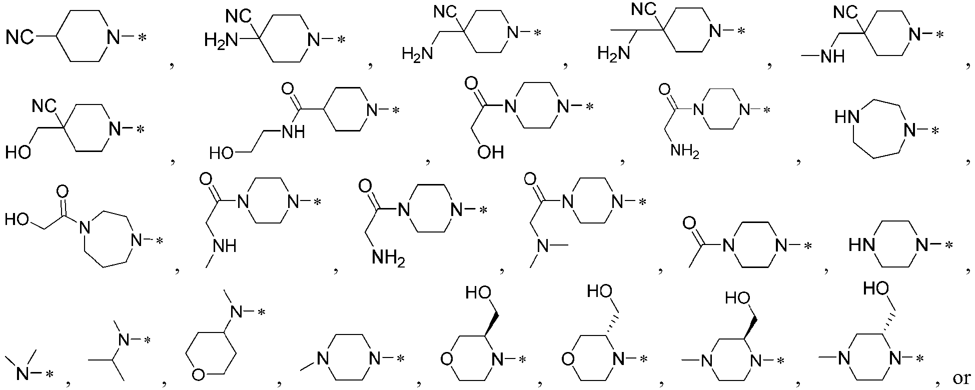 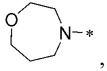 wherein the * end is attached to another fragment in D.

**In** one embodiment, Rj⁷ is 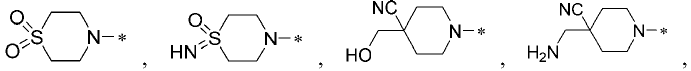 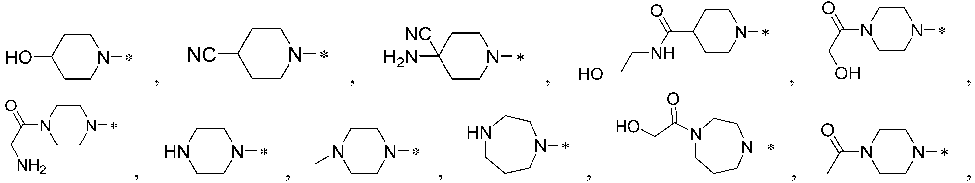 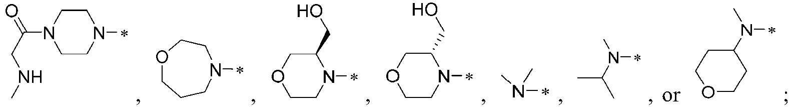 in one embodiment, Rj⁷ is 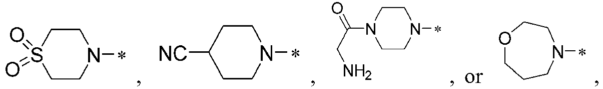 wherein the * end is attached to another fragment in D.

In one embodiment, Rj⁷ is 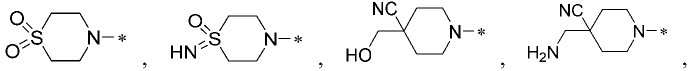 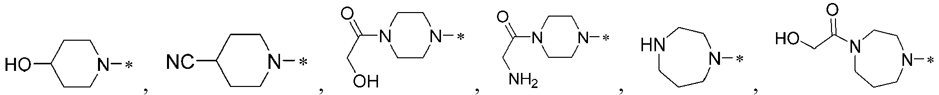 , or 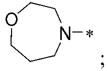 in one embodiment, Rj⁷ is 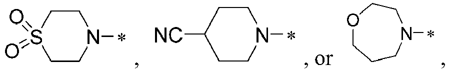 wherein the * end is attached to another fragment in D.

In one embodiment, Rj⁷ is 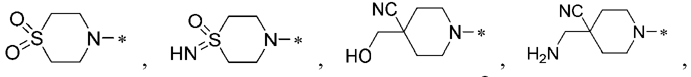 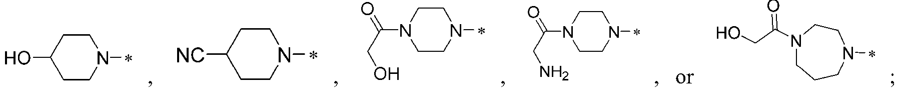 in one embodiment, Rj⁷ is 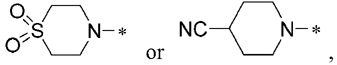 wherein the * end is attached to another fragment in D.

In one embodiment, Rj⁷ is 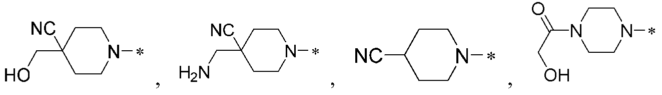 , or 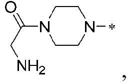 wherein the * end is attached to another fragment in D.

In one embodiment, Rj¹³ is 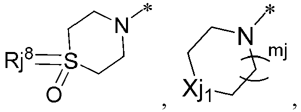 or oxo-6-membered heterocyclyl-Lj₁-Lj₂-Rj¹⁴, wherein Rj⁸, Xj₁, mj, Lj₁, Lj₂, and Rj¹⁴ are as defined in any one of the embodiments, and the * end is attached to another fragment in D.

In one embodiment, Xj₁ is -CRj⁹¹Rj⁹²-, wherein Rj⁹¹ and Rj⁹² are as defined in any one of the embodiments.

In one embodiment, Rj⁹¹ and Rj⁹² are each independently hydrogen, hydroxy, cyano, amino, -C₁₋₄ alkylene-NH₂, -C₁₋₄ alkylene-NHC₁₋₄ alkyl, or -C₁₋₄ alkylene-N(C₁₋₄ alkyl)₂, and Rj⁹¹ and Rj⁹² are not both hydrogen.

In one embodiment, Rj⁹¹ is cyano, and Rj⁹² is -CH₂NH₂.

In one embodiment, mj is 1.

In one embodiment, Lj₁ is -C₁₋₄ alkylene-O-. In one embodiment, Lj₁ is -CH₂-O-, -CH₂CH₂-O-, or -CH₂(CH₂)₂-O-. In one embodiment, Lj₁ is -CH₂CH₂-O-.

In one embodiment, Lj₂ is -C₁₋₄ alkylene-NH-. In one embodiment, Lj₂ is -CH₂-NH-, -CH₂CH₂-NH-, or - CH₂(CH₂)₂-NH-. In one embodiment, Lj₂ is -CH₂CH₂-NH-.

In one embodiment, Rj¹⁴ is C₁₋₄ heteroalkyl. In one embodiment, Rj¹⁴ is -CH₂OCH₃, -CH₂CH₂OCH₃, - CH₂NHCH₃, -CH₂N(CH₃)₂, -CH₂CH₂NHCH₃, or -CH₂CH₂N(CH₃)₂. In one embodiment, Rj¹⁴ is -CH₂OCH₃ or -CH₂CH₂OCH₃. In one embodiment, Rj¹⁴ is -CH₂CH₂OCH₃.

In one embodiment, Rj¹³ is 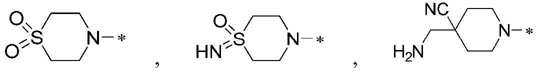 , or 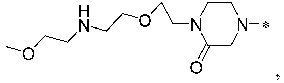 wherein the * end is attached to another fragment in D.

In one embodiment, Rj¹³ is 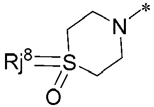 or oxo-6-membered heterocyclyl-Lj₁-Lj₂-Rj¹⁴, wherein Rj⁸, Lj₁, Lj₂, and Rj¹⁴ are as defined in any one of the embodiments, and the * end is attached to another fragment in D.

In one embodiment, Rj¹³ is 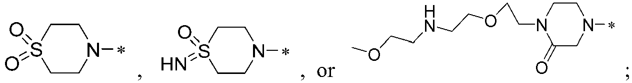 in one embodiment, Rj¹³ is 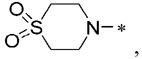 wherein the * end is attached to another fragment in D.

In one embodiment, Rj¹³ is oxo-6-membered heterocyclyl-Lj₁-Lj₂-Rj¹⁴, wherein Rj⁸, Lj₁, Lj₂, and Rj¹⁴ are as defined in any one of the embodiments. In one embodiment, Rj¹³ is 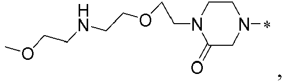 wherein the * end is attached to another fragment in D.

In one embodiment, Yj₄ is 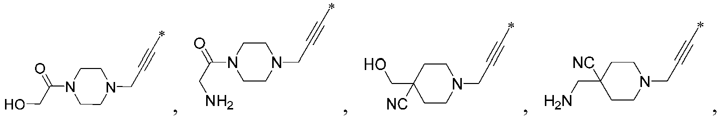 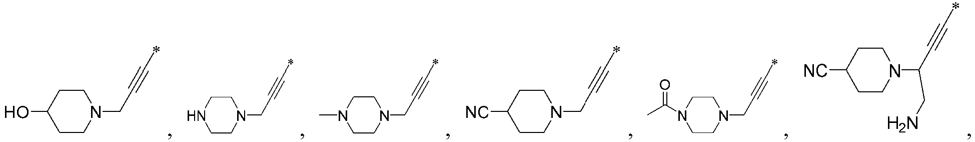 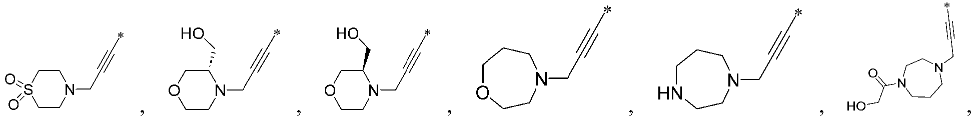 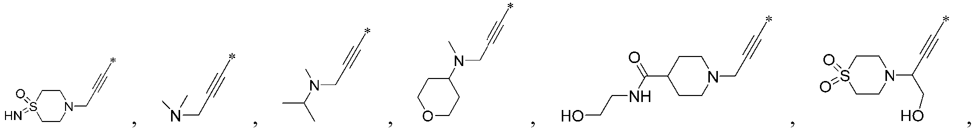 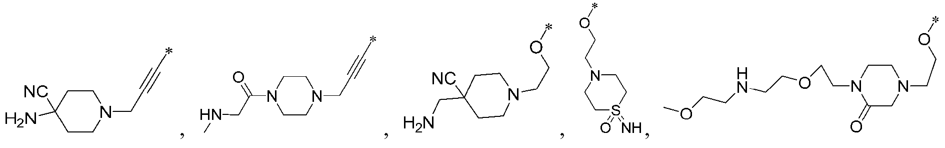 , or 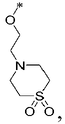 wherein the * end is attached to another fragment in D.

In one embodiment, Yj₄ is 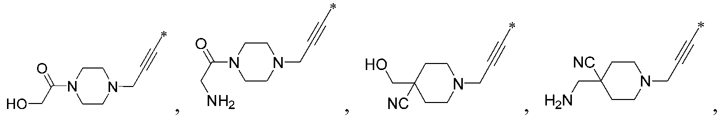 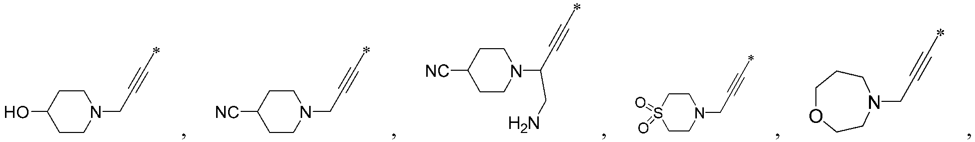 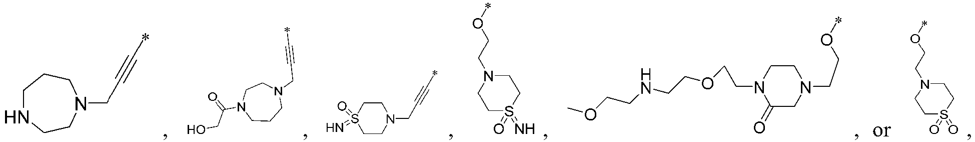 wherein the * end is attached to another fragment in D.

In one embodiment, Yj₄ is 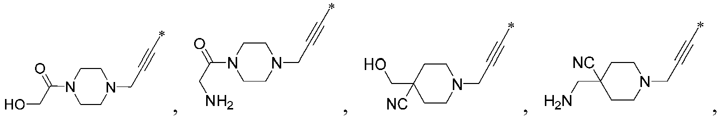 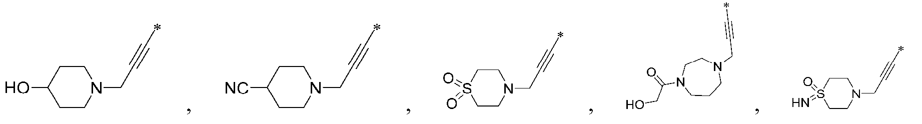 , or 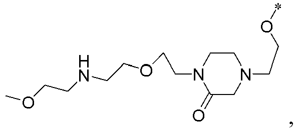 wherein the * end is attached to another fragment in D.

In one embodiment, Yj₄ is 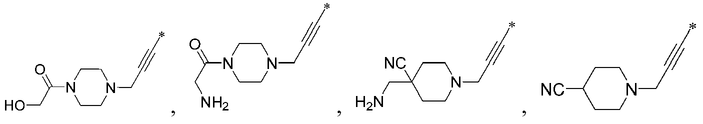 , or 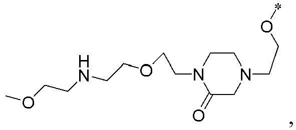 wherein the * end is attached to another fragment in D.

In one embodiment, Yj₄ is 5- to 7-membered nitrogen-containing monocyclic heterocyclyl, 9- to 10-membered nitrogen-containing bicyclic heterocyclyl, or phenyl, wherein the 5- to 7-membered nitrogen-containing monocyclic heterocyclyl, 9- to 10-membered nitrogen-containing bicyclic heterocyclyl, or phenyl is optionally substituted with one or more substituents selected from C₁₋₄ alkyl, -methylene-hydroxy, -C(=O)C₁₋₄ alkylene-OH, and -methylene-5- to 7-membered nitrogen-containing monocyclic heterocyclyl.

In one embodiment, Yj₄ is piperidinyl, piperazinyl, 9- to 10-membered nitrogen-containing bicyclic heterocyclyl, or phenyl, wherein the piperidinyl, piperazinyl, 9- to 10-membered nitrogen-containing bicyclic heterocyclyl, or phenyl is optionally substituted with one or more substituents selected from methyl, -methylene-hydroxy, - methylene-morpholinyl, -C(=O)CH₂OH, and -methylene-piperazinyl.

In one embodiment, Yj₄ is 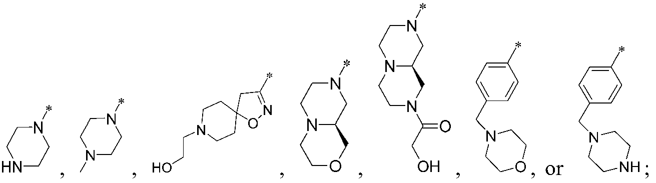 in one embodiment, Yj₄ is 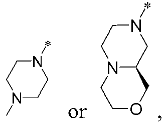 wherein the * end is attached to another fragment in D.

In one embodiment, Yj₄ is piperazinyl or 9- to 10-membered nitrogen-containing bicyclic heterocyclyl, wherein the piperazinyl or 9- to 10-membered nitrogen-containing bicyclic heterocyclyl is optionally substituted with one or more substituents selected from methyl or C(=O)CH₂OH.

In one embodiment, Yj₄ is 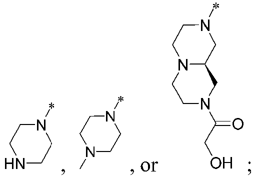 in one embodiment, Yj₄ is 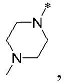 wherein the * end is attached to another fragment in D.

In one embodiment, when L₀ is attached to Yj₄, any hydrogen atom on Yj₄ is replaced by L₀, or any N atom on Yj₄ is quaternized, wherein Yj₄ is as defined in any one of the embodiments.

In one embodiment, when L₀ is attached to Yj₄, Yj₄ is 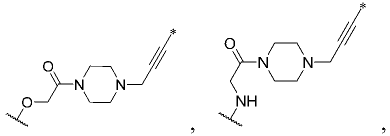 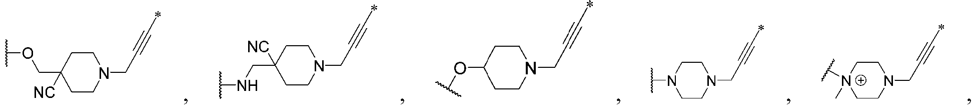 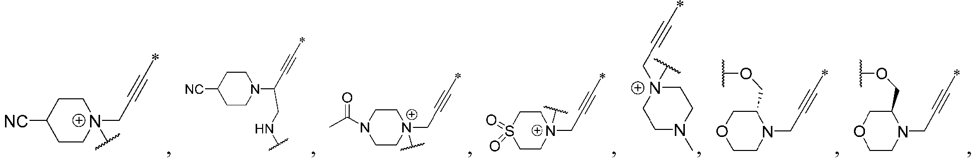 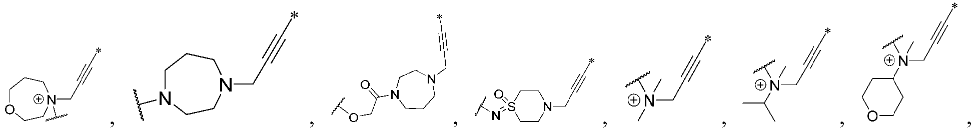 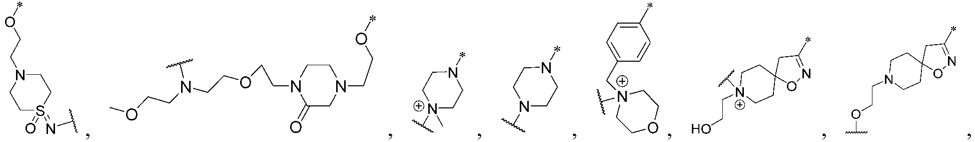 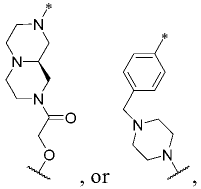 wherein the 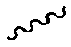 end is attached to L₀, and the * end is attached to another fragment in D.

In one embodiment, when L₀ is attached to Yj₄, Yj₄ is 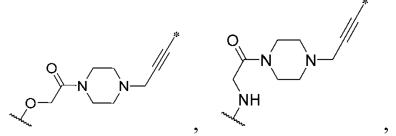 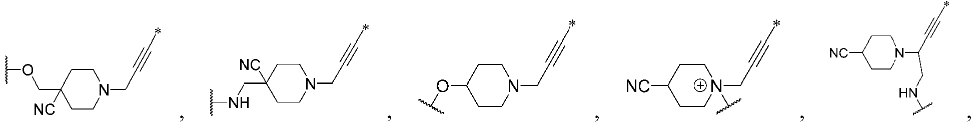 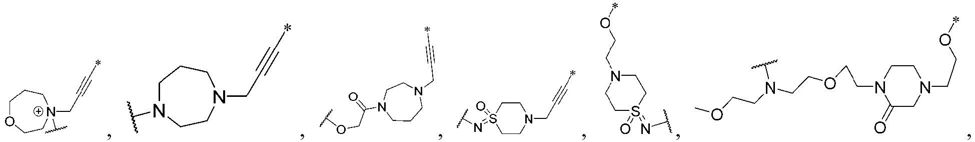 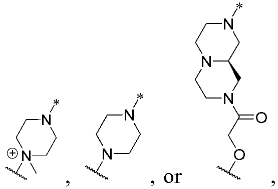 wherein the 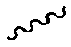 end is attached to L₀, and the * end is attached to another fragment in D.

In one embodiment, when L₀ is attached to Yj₄, Yj₄ is 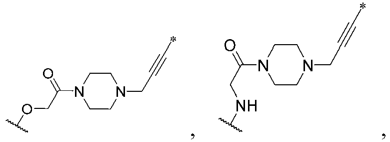 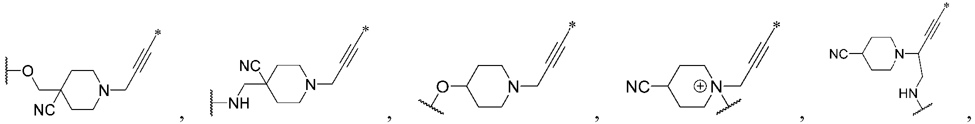 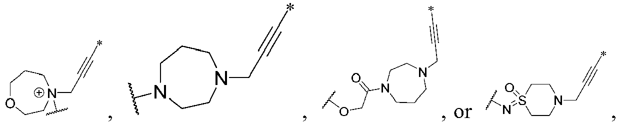 wherein the 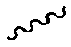 end is attached to L₀, and the * end is attached to another fragment in D.

In one embodiment, when L₀ is attached to Yj₄, Yj₄ is 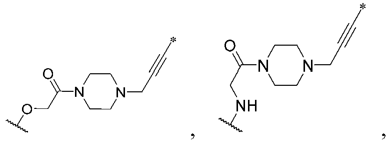 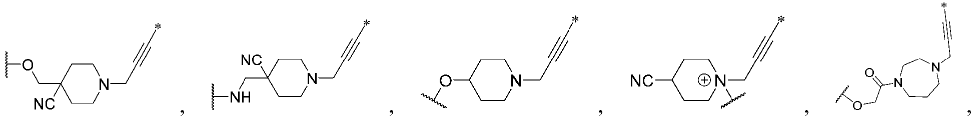 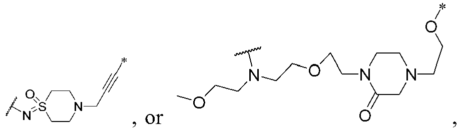 wherein the 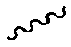 end is attached to L₀, and the * end is attached to another fragment in D.

In one embodiment, when L₀ is attached to Yj₄, Yj₄ is 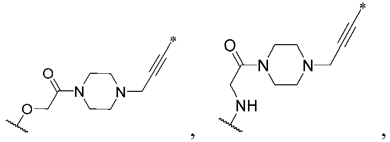 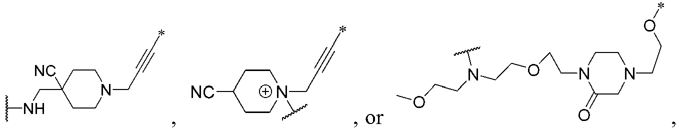 wherein the 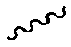 end is attached to L₀, and the * end is attached to another fragment in D.

In one embodiment, when L₀ is attached to Yj₄, Yj₄ is 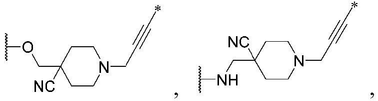 or 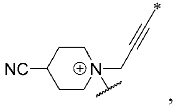 wherein the 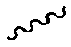 end is attached to L₀, and the * end is attached to another fragment in D.

In one embodiment, when L₀ is attached to Yj₄, Yj₄ is 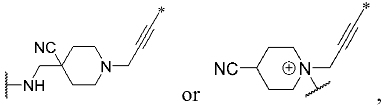 wherein the 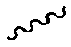 end is attached to L₀, and the * end is attached to another fragment in D.

In one embodiment, when L₀ is attached to Yj₄, Yj₄ is 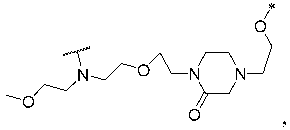 wherein the 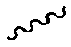 end is attached to L₀, and the * end is attached to another fragment in D.

In one embodiment, the structural unit 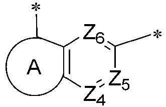 is 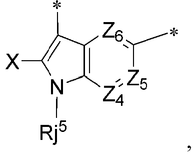 and X is 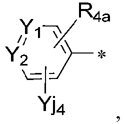 wherein Z₄, Z₅, Z₆, Y₁, Y₂, R₄ₐ, Yj₄, and Rj⁵ are as defined in any one of the embodiments.

In one embodiment, the structural unit 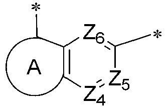 is 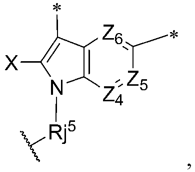 and X is 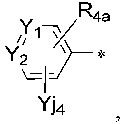 wherein Z₄, Z₅, Z₆, Y₁, Y₂, R₄ₐ, Yj₄, and Rj⁵ are as defined in any one of the embodiments, the 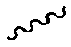 end is attached to L₀, and the * end is attached to another fragment in D.

In one embodiment, the structural unit 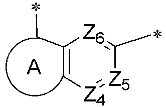 is 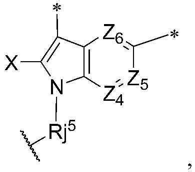 and X is 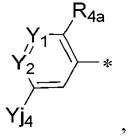 wherein Z₄, Z₅, Z₆, Y₁, Y₂, R₄ₐ, Yj₄, and Rj⁵ are as defined in any one of the embodiments, the 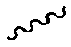 end is attached to L₀, and the * end is attached to another fragment in D.

In one embodiment, the structural unit 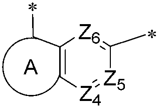 is 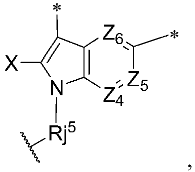 and X is 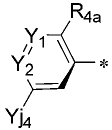 or 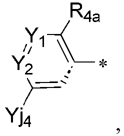 wherein Z₄, Z₅, Z₆, Y₁, Y₂, R₄ₐ, Yj₄, and Rj⁵ are as defined in any one of the embodiments, the 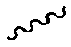 end is attached to L₀, and the * end is attached to another fragment in D.

In one embodiment, the structural unit 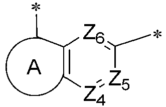 is 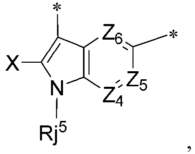 and X is 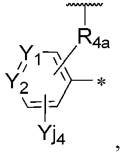 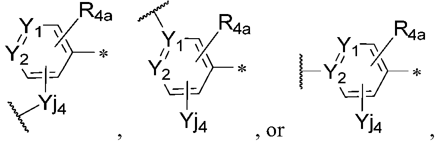 wherein Z₄, Z₅, Z₆, Y₁, Y₂, R₄ₐ, Yj₄, and Rj⁵ are as defined in any one of the embodiments, the 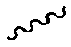 end is attached to L₀, and the * end is attached to another fragment in D.

In one embodiment, the structural unit 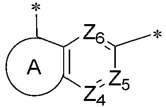 is 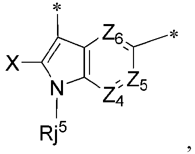 and X is 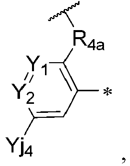 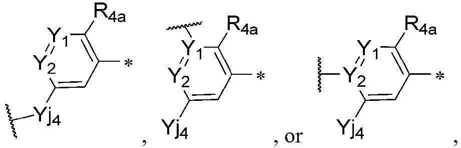 wherein Z₄, Z₅, Z₆, Y₁, Y₂, R₄ₐ, Yj₄, and Rj⁵ are as defined in any one of the embodiments, the 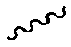 end is attached to L₀, and the * end is attached to another fragment in D.

In one embodiment, the structural unit 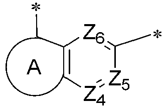 is 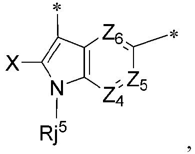 **and** X is 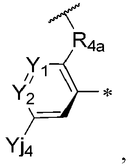 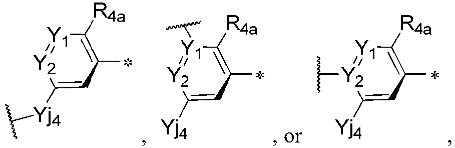 wherein Z₄, Z₅, Z₆, Y₁, Y₂, R₄ₐ, Yj₄, and Rj⁵ are as defined in any one of the embodiments, the 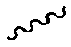 end is attached to L₀, and the * end is attached to another fragment in D.

In one embodiment, the structural unit 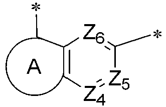 is 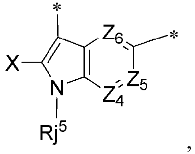 **and** X is 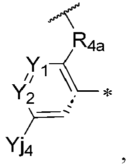 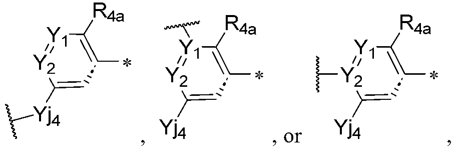 wherein Z₄, Z₅, Z₆, Y₁, Y₂, R₄ₐ, Yj₄, and Rj⁵ are as defined in any one of the embodiments, the 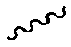 end is attached to L₀, and the * end is attached to another fragment in D.

In one embodiment, the structural unit 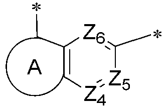 is 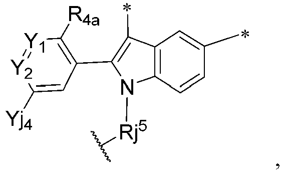 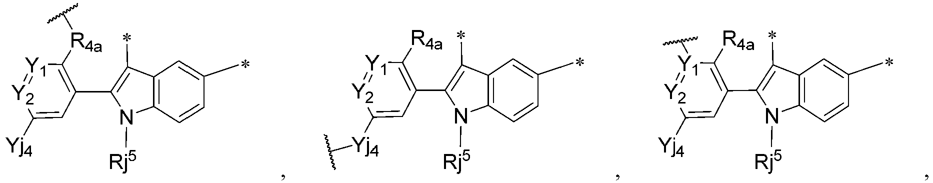 or 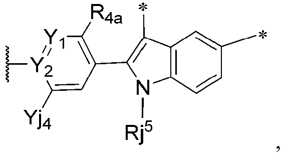 wherein Y₁, Y₂, R₄ₐ, Yj₄, and Rj⁵ are as defined in any one of the embodiments, the 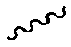 end is attached to L₀, and the * end is attached to another fragment in D.

In one embodiment, the structural unit 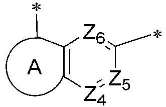 is 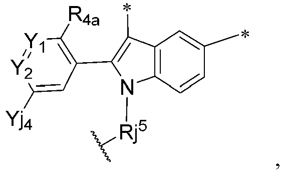 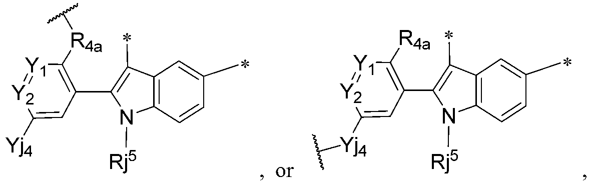 wherein Y₁, Y₂, R₄ₐ, Yj₄, and Rj⁵ are as defined in any one of the embodiments, the 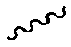 end is attached to L₀, and the * end is attached to another fragment in D.

In one embodiment, the structural unit 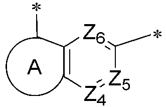 is 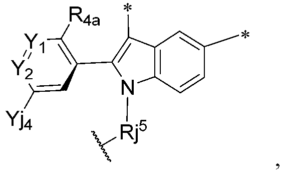 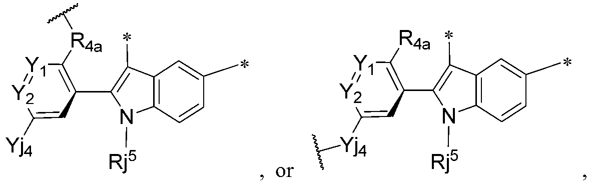 wherein Y₁, Y₂, R₄ₐ, Yj₄, and Rj⁵ are as defined in any one of the embodiments, the 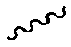 end is attached to L₀, and the * end is attached to another fragment in D.

In one embodiment, the structural unit 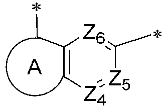 is 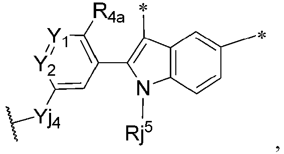 wherein Y₁, Y₂, R₄ₐ, Yj₄, and Rj⁵ are as defined in any one of the embodiments, the 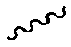 end is attached to L₀, and the * end is attached to another fragment in D.

In one embodiment, the structural unit 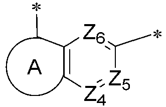 is 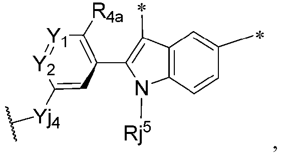 wherein Y₁, Y₂, R₄ₐ, Yj₄, and Rj⁵ are as defined in any one of the embodiments, the 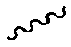 end is attached to L₀, and the * end is attached to another fragment in D.

In one embodiment, Lj is -NHC(=O)-, -N(C₁₋₆ alkyl)C(=O)-, C₆₋₁₀ aryl, 5- or 6-membered monocyclic heteroaryl, or 8- to 10-membered bicyclic heteroaryl, wherein the C₆₋₁₀ aryl, 5- or 6-membered monocyclic heteroaryl, or 8- to 10-membered bicyclic heteroaryl is optionally substituted with 1, 2, 3, 4, 5, or 6 S1, and S1 is as defined in any one of the embodiments.

In one embodiment, each S1 is independently selected from deuterium, oxo (=O), thio (=S), halogen, cyano, hydroxy, carboxyl, nitro, amino, and C₁₋₆ alkyl. In one embodiment, each S1 is independently selected from deuterium, halogen, cyano, hydroxy, amino, and C₁₋₄ alkyl.

In one embodiment, Lj is C₆₋₁₀ aryl or 5- or 6-membered monocyclic heteroaryl, wherein the C₆₋₁₀ aryl is optionally substituted with hydroxy or amino. In one embodiment, Lj is phenyl, thiazolyl, oxazolyl, 1,3,4-oxadiazolyl, or 1,2,4-oxadiazolyl, wherein the phenyl is optionally substituted with hydroxy or amino. In one embodiment, Lj is phenyl, oxazolyl, or thiazolyl, wherein the phenyl is optionally substituted with hydroxy or amino.

In one embodiment, Lj is phenyl, 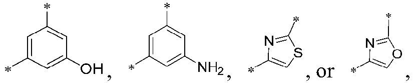 wherein the * end is attached to another fragment in D. In one embodiment, Lj is 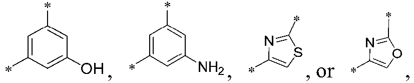 wherein the * end is attached to another fragment in D. In one embodiment, Lj is 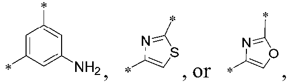 wherein the * end is attached to another fragment in D.

In one embodiment, Lj is oxazolyl or thiazolyl. In one embodiment, Lj is 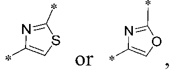 wherein the * end is attached to another fragment in D.

In one embodiment, Lj is thiazolyl. In one embodiment, Lj is 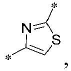 wherein the * end is attached to another fragment in D.

In one embodiment, Y₁₀ is O, NH, NCH₃, NCH₂CH₃, or NCH(CH₃)₂. In one embodiment, Y₁₀ is O or NH.

In one embodiment, when L₀ is attached to Lj, any hydrogen atom on Lj is replaced by L₀, or any N atom on Lj is quaternized, wherein Lj is as defined in any one of the embodiments.

In one embodiment, when L₀ is attached to Lj, Lj is 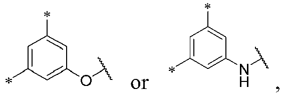 and further, Lj is 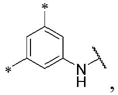 wherein the 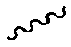 end is attached to L₀, and the * end is attached to another fragment in D.

In one embodiment, Rj²¹ and Rj²² are each independently hydrogen, C₁₋₆ alkyl, -NRj^{f}Rj^{g}, or -O-C₁₋₄ alkylene-NRj^{f}Rj^{g}, wherein Rj^{f} and Rj^{g} are as defined in any one of the embodiments. In one embodiment, Rj²¹ and Rj²² are each independently hydrogen, C₁₋₄ alkyl, -NRj^{f}Rj^{g}, or -O-C₁₋₄ alkylene-NRj^{f}Rj^{g}, wherein Rj^{f} and Rj^{g} are as defined in any one of the embodiments.

In one embodiment, Rj^{f} and Rj^{g} are each independently hydrogen or C₁₋₄ alkyl. In one embodiment, Rj^{f} and Rj^{g} are each independently hydrogen, methyl, ethyl, n-propyl, or isopropyl.

In one embodiment, Rj²¹ and Rj²² are each independently hydrogen, amino, -NH(C₁₋₄ alkyl), -N(C₁₋₄ alkyl)₂, - O-C₁₋₄ alkylene-NH(C₁₋₄ alkyl), or -O-C₁₋₄ alkylene-N(C₁₋₄ alkyl)₂.

In one embodiment, Rj²¹ is hydrogen, amino, -NH(CH₃), -OCH₂CH₂NHCH₃, or -OCH₂CH₂N(CH₃)₂. In one embodiment, Rj²¹ is hydrogen. In one embodiment, Rj²² is hydrogen.

In one embodiment, when L₀ is attached to Rj²¹, any hydrogen atom on Rj²¹ is replaced by L₀, or any N atom on Rj²¹ is quaternized, wherein Rj²¹ is as defined in any one of the embodiments.

In one embodiment, when L₀ is attached to Rj²¹, Rj²¹ is -NH-, -N(CH₃)-, 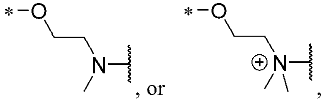 wherein the 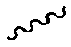 end is attached to L₀, and the * end is attached to another fragment in D.

In one embodiment, when L₀ is attached to Rj²¹, Rj²¹ is -NH-, -N(CH₃)-, or 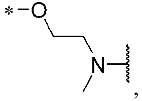 wherein the 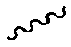 end is attached to L₀, and the * end is attached to another fragment in D.

In one embodiment, Rj¹¹ and Rj¹² are each independently hydrogen, deuterated C₁₋₆ alkyl, or C₁₋₆ alkyl. In one embodiment, Rj¹¹ and Rj¹² are each independently hydrogen, deuterated C₁₋₄ alkyl, or C₁₋₄ alkyl. In one embodiment, Rj¹¹ and Rj¹² are each independently methyl or deuterated methyl. In one embodiment, Rj¹¹ and Rj¹² are methyl.

In one embodiment, Rj¹¹ and Rj¹², together with the carbon atom to which they are attached, form C₃₋₆ cycloalkyl or 3- to 7-membered heterocyclyl. In one embodiment, Rj¹¹ and Rj¹², together with the carbon atom to which they are attached, form cyclopropyl, cyclobutyl, azetidinyl, or oxetanyl. In one embodiment, Rj¹¹ and Rj¹², together with the carbon atom to which they are attached, form cyclopropyl.

In one embodiment, R₄₄, R₄₅, R₄₆, and R₄₇ are each independently hydrogen, deuterium, halogen, C₁₋₄ alkyl, or C₁₋₄ haloalkyl. In one embodiment, R₄₄, R₄₅, R₄₆, and R₄₇ are each independently hydrogen, deuterium, fluorine, chlorine, bromine, methyl, ethyl, or halomethyl. In one embodiment, R₄₄, R₄₅, R₄₆, and R₄₇ are hydrogen.

In one embodiment, Y₃ is -C(=O)Rj⁴ or R₁₁;

Rj⁴ is C₆₋₁₀ aryl, heteroaryl, C₃₋₆ cycloalkyl, 3- to 8-membered heterocyclyl, or -CRj^{a}Rj^{b} -NRj^{c}Rj^{d}, wherein the C₆₋₁₀ aryl, heteroaryl, C₃₋₆ cycloalkyl, or 3- to 8-membered heterocyclyl is optionally substituted with one or more Rj⁴¹;

R₁₁ is 5- or 6-membered monocyclic heteroaryl or 8- to 10-membered bicyclic heteroaryl, wherein the 5- or 6-membered monocyclic heteroaryl or 8- to 10-membered bicyclic heteroaryl is optionally substituted with one or more Rj⁴¹;

Rj⁴¹ is C₁₋₆ alkyl, hydroxy, SF₅, NRj^{c}Rj^{d}, -P(-O)(C₁₋₆ alkyl)₂, -C₁₋₄ alkylene-OH, or -C(O)-NRj^{c}Rj^{d};

Rj^{a} and Rj^{b} are each independently hydrogen, C₁₋₆ alkyl, or C₃₋₆ cycloalkyl;

Rj^{c} and Rj^{d} are each independently hydrogen, -C(O)H, C₁₋₆ alkyl, C₁₋₆ haloalkyl, phenyl, 5- or 6-membered monocyclic heteroaryl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more Rj⁴²;

Rj⁴² is C₁₋₆ alkyl, C₁₋₆ haloalkyl, -NH₂, -NHC₁₋₆ alkyl, -N(C₁₋₆ alkyl)₂, -C(O)C₁₋₆ alkyl, or -C(O)OC₁₋₆ alkyl. In one embodiment, Y₃ is -C(=O)Rj⁴, wherein Rj⁴ is as defined in any one of the embodiments.

In one embodiment, Rj⁴ is C₆₋₁₀ aryl, 5- or 6-membered monocyclic heteroaryl, C₃₋₆ cycloalkyl, 3- to 8-membered heterocyclyl, or -CRj^{a}Rj^{b}-NRj^{c}Rj^{d}, wherein the C₆₋₁₀ aryl, 5- or 6-membered monocyclic heteroaryl, C₃₋₆ cycloalkyl, or 3- to 8-membered heterocyclyl is optionally substituted with one or more Rj⁴¹, and Rj^{a}, Rj^{b}, Rj^{c}, Rj^{d}, and Rj⁴¹ are as defined in any one of the embodiments.

In one embodiment, Rj⁴ is C₃₋₆ cycloalkyl, 3- to 8-membered heterocyclyl, or -CRj^{a}Rj^{b}-NRj^{c}Rj^{d}, wherein the C₃₋₆ cycloalkyl or 3- to 8-membered heterocyclyl is optionally substituted with one or more substituents Rj⁴¹, and Rj^{a}, Rj^{b}, Rj^{c}, Rj^{d}, and Rj⁴¹ are as defined in any one of the embodiments.

In one embodiment, Rj⁴ is C₃₋₆ cycloalkyl, 5- to 8-membered heterocyclyl, or -CRj^{a}Rj^{b}-NRj^{c}Rj^{d}, wherein the C₃₋₆ cycloalkyl is optionally substituted with one or more Rj⁴¹, and Rj^{a}, Rj^{b}, Rj^{c}, Rj^{d}, and Rj⁴¹ are as defined in any one of the embodiments.

In one embodiment, Rj⁴ is cyclopropyl, cyclobutyl, cyclopentyl, 5- to 8-membered bicyclic heterocyclyl, or - CHRj^{b}-NRj^{c}Rj^{d}, wherein the cyclopropyl, cyclobutyl, cyclopentyl, or 5- to 8-membered bicyclic heterocyclyl is optionally substituted with 1, 2, 3, 4, or 5 Rj⁴¹, and Rj^{b}, Rj^{c}, Rj^{d}, and Rj⁴¹ are as defined in any one of the embodiments.

In one embodiment, Rj⁴ is 5- to 8-membered oxacyclyl, preferably 5- to 8-membered oxa-fused heterocyclyl.

In one embodiment, Rj⁴ is C₃₋₆ cycloalkyl, wherein the C₃₋₆ cycloalkyl is optionally substituted with 1, 2, 3, 4, or 5 Rj⁴¹, and Rj⁴¹ is as defined in any one of the embodiments.

In one embodiment, Rj⁴¹ is hydroxy, methyl, -C₁₋₄ alkylene-OH, or -C(O)-NH(C₁₋₄ alkylene-OH). In one embodiment, Rj⁴¹ is hydroxy, methyl, -CH₂OH, or -C(O)-NH(CH₂CH₂OH).

In one embodiment, Rj⁴ is -CRj^{a}Rj^{b}-NRj^{c}Rj^{d}, wherein Rj^{a}, Rj^{b}, Rj^{c}, and Rj^{d} are as defined in any one of the embodiments. In one embodiment, Rj^{a} and Rj^{b} are each independently hydrogen, C₁₋₆ alkyl, or C₃₋₆ cycloalkyl, and Rj^{c} and Rj^{d} are each independently hydrogen, C₁₋₆ alkyl, or -C(O)-C₁₋₄ alkylene-OH.

In one embodiment, Rj⁴ is -CRj^{a}Rj^{b}-NRj^{c}Rj^{d}, wherein Rj^{a} and Rj^{b} are each independently hydrogen or C₁₋₆ alkyl, and Rj^{c} and Rj^{d} are each independently hydrogen, C₁₋₆ alkyl, or -C(O)-pyrrolidinyl, wherein the pyrrolidinyl is optionally substituted with 1, 2, 3, 4, or 5 Rj⁴², and Rj⁴² is methyl or -C(O)OCH₃.

In one embodiment, Rj⁴¹ is C₁₋₄ alkyl, hydroxy, NRj^{c}Rj^{d}, -C₁₋₄ alkylene-OH, or -C(O)-NRj^{c}Rj^{d}, wherein Rj^{c} and Rj^{d} are as defined in any one of the embodiments. In one embodiment, Rj⁴¹ is methyl, ethyl, hydroxy, amino, CH₂OH, CH₂CH₂OH, or -C(O)-NRj^{c}Rj^{d}, wherein Rj^{c} and Rj^{d} are as defined in any one of the embodiments. In one embodiment, Rj⁴¹ is methyl, hydroxy, CH₂OH, or -C(O)NHCH₂CH₂OH.

In one embodiment, Rj⁴¹ is C₁₋₄ alkyl, hydroxy, or -C₁₋₄ alkylene-OH. In one embodiment, Rj⁴¹ is methyl, hydroxy, or CH₂OH.

In one embodiment, Rj⁴¹ is C₁₋₄ alkyl. In one embodiment, Rj⁴¹ is methyl.

In one embodiment, Rj⁴¹ is amino.

In one embodiment, Rj⁴¹ is methyl, -C₁₋₄ alkylene-OH, or -C(O)-NRj^{c}Rj^{d}, wherein Rj^{c} and Rj^{d} are as defined in any one of the embodiments.

In one embodiment, Rj^{a} and Rj^{b} are each independently hydrogen, C₁₋₄ alkyl, or C₃₋₅ cycloalkyl. In one embodiment, Rj^{a} and Rj^{b} are each independently hydrogen, methyl, ethyl, n-propyl, isopropyl, cyclopropyl, cyclobutyl, or cyclopentyl. In one embodiment, Rj^{a} and Rj^{b} are each independently hydrogen, isopropyl, cyclobutyl, or cyclopentyl.

In one embodiment, Rj^{a} is hydrogen.

In one embodiment, Rj^{b} is isopropyl, cyclobutyl, or cyclopentyl.

In one embodiment, Rj^{b} is C₁₋₄ alkyl. In one embodiment, Rj^{b} is isopropyl or cyclobutyl.

In one embodiment, Rj^{c} and Rj^{d} are each independently hydrogen, -C(O)H, C₁₋₄ alkyl, C₁₋₄ haloalkyl, phenyl, pyridinyl, -C₁₋₄ alkylene-OH, -C(O)-5- to 6-membered heterocyclyl, or -C(O)-C₁₋₄ alkylene-OH, wherein the 5-to 6-membered heterocyclyl is optionally substituted with one or more Rj⁴², and Rj⁴² is as defined in any one of the embodiments.

In one embodiment, Rj^{c} and Rj^{d} are each independently hydrogen, -C(O)H, C₁₋₄ alkyl, C₁₋₄ haloalkyl, -C₁₋₄ alkylene-OH, -C(O)-5- to 6-membered monocyclic heterocyclyl, or -C(O)-C₁₋₄ alkylene-OH, wherein the 5- to 6-membered monocyclic heterocyclyl is optionally substituted with one or more Rj⁴², and Rj⁴² is as defined in any one of the embodiments.

In one embodiment, Rj^{c} and Rj^{d} are each independently hydrogen, methyl, ethyl, fluoromethyl, fluoroethyl, - C(O)H, -C(=O)CH₂OH, -C(=O)CH₂CH₂OH, -C(=O)-azetidinyl, or -C(=O)-pyrrolidinyl, wherein the azetidinyl or pyrrolidinyl is optionally substituted with 1, 2, or 3 Rj⁴², and Rj⁴² is as defined in any one of the embodiments. In one embodiment, Rj^{c} and Rj^{d} are each independently hydrogen, methyl, -C(=O)CH₂OH, -C(=O)CH₂CH₂OH, -C(=O)-azetidinyl-NHCH₃, or -C(=O)-pyrrolidinyl(methyl)-C(=O)OCH₃-

In one embodiment, Rj^{c} and Rj^{d} are each independently hydrogen or C₁₋₄ alkyl. In one embodiment, Rj^{c} and Rj^{d} are each independently hydrogen or methyl.

In one embodiment, Rj⁴² is C₁₋₄ alkyl, C₁₋₄ haloalkyl, -NHC₁₋₄ alkyl, -N(C₁₋₄ alkyl)₂, -C(O)C₁₋₄ alkyl, or - C(O)OC₁₋₄ alkyl. In one embodiment, Rj⁴² is methyl, ethyl, halomethyl, haloethyl, -NHCH₃, -N(CH₃)₂, - C(O)CH₃, or -C(O)OCH₃. In one embodiment, Rj⁴² is methyl, -NHCH₃, or -C(O)OCH₃.

In one embodiment, Rj⁴ is 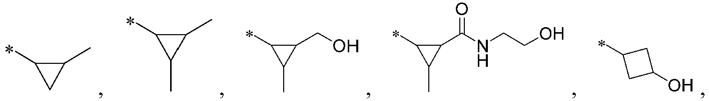 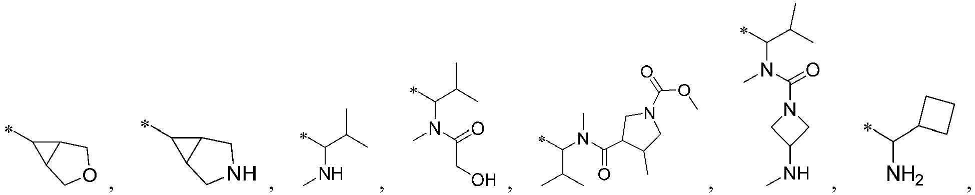 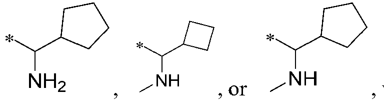 wherein the * end is attached to another fragment in D.

In one embodiment, Rj⁴ is 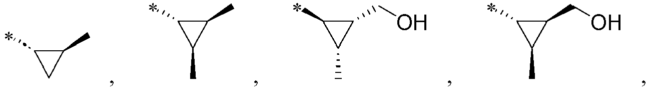 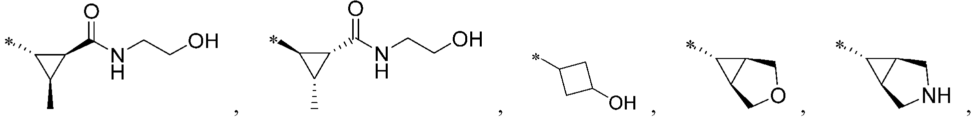 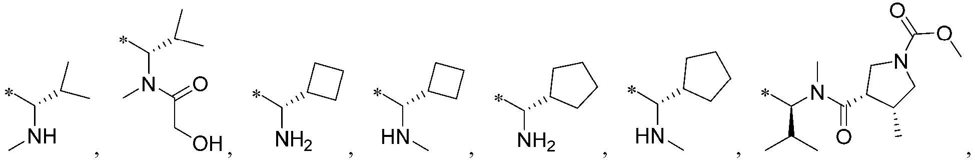 or 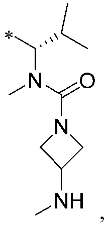 wherein the * end is attached to another fragment in D.

In one embodiment, Rj⁴ is 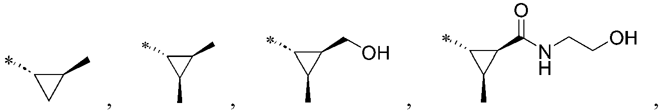 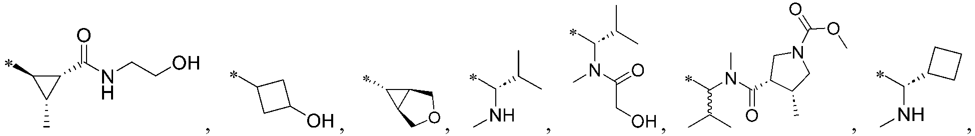 or 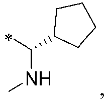 wherein the * end is attached to another fragment in D.

In one embodiment, Rj⁴ is 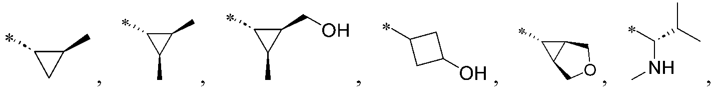 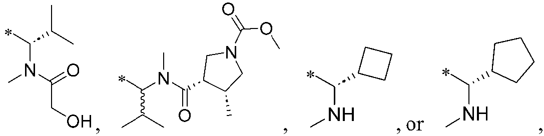 wherein the * end is attached to another fragment in D.

In one embodiment, Rj⁴ is 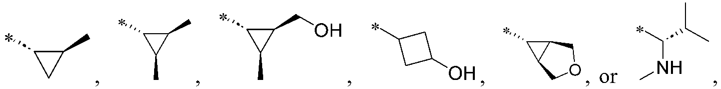 wherein the * end is attached to another fragment in D.

In one embodiment, Rj⁴ is 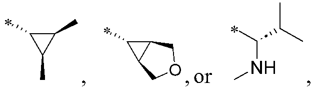 wherein the * end is attached to another fragment in D.

In one embodiment, Rj⁴ is 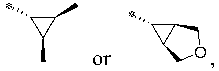 wherein the * end is attached to another fragment in D.

In one embodiment, Rj⁴ is 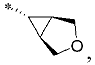 wherein the * end is attached to another fragment in D.

In one embodiment, R₁₁ is 8- to 10-membered bicyclic heteroaryl, wherein the 8- to 10-membered bicyclic heteroaryl is optionally substituted with one or more Rj⁴¹, and Rj⁴¹ is as defined in any one of the embodiments. In one embodiment, R₁₁ is 8- to 10-membered bicyclic heteroaryl, wherein the 8- to 10-membered bicyclic heteroaryl contains one or more nitrogen heteroatoms; the 8- to 10-membered bicyclic heteroaryl is optionally substituted with one or more Rj⁴¹; Rj⁴¹ is as defined in any one of the embodiments.

In one embodiment, R₁₁ is indolyl, quinolyl, or triazolopyridinyl, wherein the indolyl, quinolyl, or triazolopyridinyl is optionally substituted with 1, 2, 3, 4, or 5 Rj⁴¹, and Rj⁴¹ is as defined in any one of the embodiments.

In one embodiment, R₁₁ is 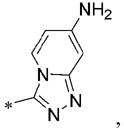 wherein the * end is attached to another fragment in D.

**In** one embodiment, Y₃ is 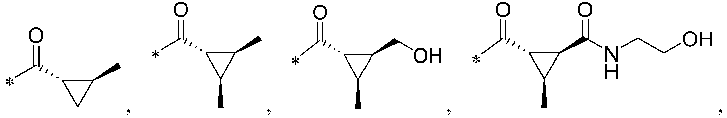 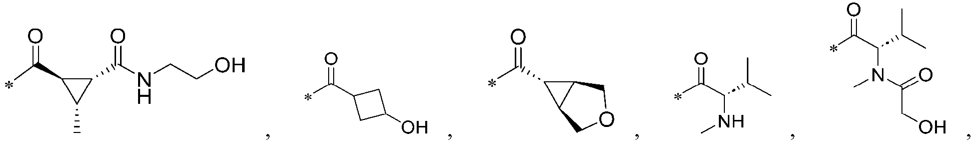 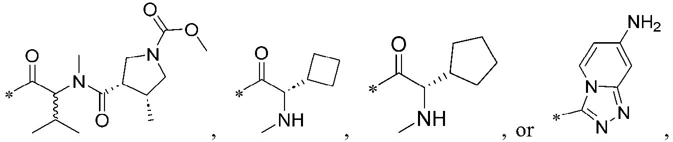 wherein the * end is attached to another fragment in D.

In one embodiment, when L₀ is attached to Y₃, any hydrogen atom on Y₃ is replaced by L₀, or any N atom on Y₃ is quaternized, wherein Y₃ is as defined in any one of the embodiments.

In one embodiment, when L₀ is attached to Y₃, Y₃ is 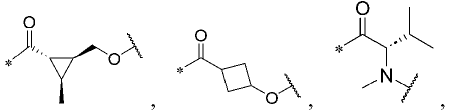 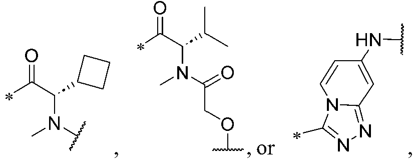 wherein the 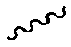 end is attached to L₀, and the * end is attached to another fragment in D.

In one embodiment, when L₀ is attached to Y₃, Y₃ is 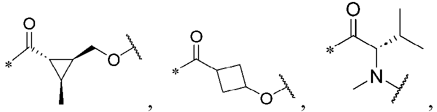 or 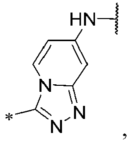 wherein the 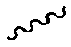 end is attached to L₀, and the * end is attached to another fragment in D.

In one embodiment, when L₀ is attached to Y₃, Y₃ is 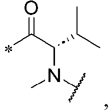 wherein the 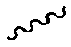 end is attached to L₀, and the * end is attached to another fragment in D.

In one embodiment, when L₀ is attached to Rj⁴, any hydrogen atom on Rj⁴ is replaced by L₀, or any N atom on Rj⁴ is quaternized, wherein Rj⁴ is as defined in any one of the embodiments.

In one embodiment, when L₀ is attached to Rj⁴, Rj⁴ is 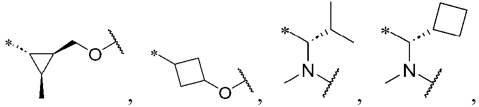 or 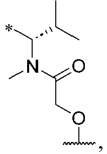 wherein the 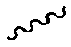 end is attached to L₀, and the * end is attached to another fragment in D.

In one embodiment, when L₀ is attached to Rj⁴, Rj⁴ is 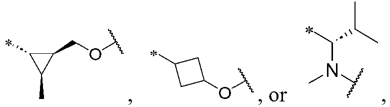 wherein the 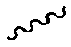 end is attached to L₀, and the * end is attached to another fragment in D.

In one embodiment, when L₀ is attached to Rj⁴, Rj⁴ is 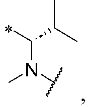 wherein the 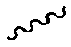 end is attached to L₀, and the * end is attached to another fragment in D.

In one embodiment, Rj³ is hydrogen or C¹⁻⁶ alkyl. In one embodiment, Rj³ is hydrogen or C₁₋₄ alkyl. In one embodiment, Rj³ is hydrogen, methyl, or ethyl. In one embodiment, Rj³ is hydrogen or methyl. In one embodiment, Rj³ is hydrogen.

In one embodiment, -D is a structure represented by formula (II-1), formula (II-2), formula (II-3), or formula (II-4), or a stereoisomer thereof: 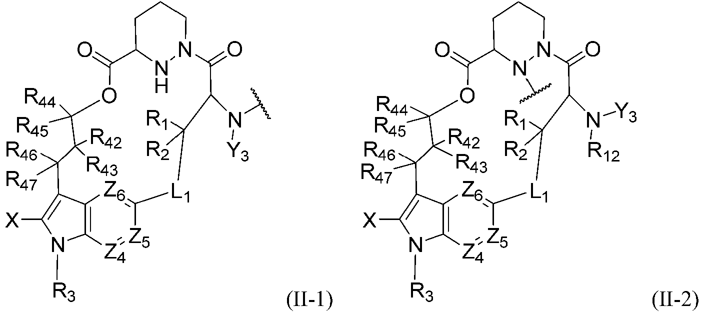 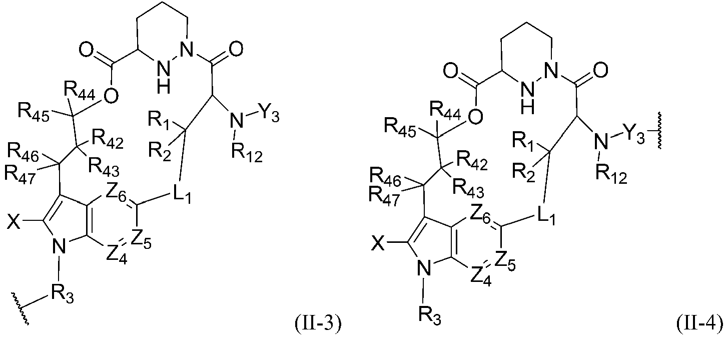 wherein in the formulas,
X is 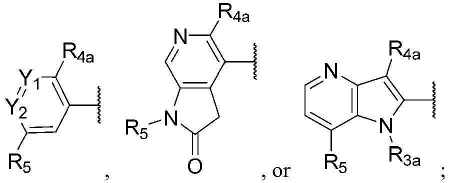
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C¹⁻⁶ alkyl;
R₁ is hydrogen, deuterium, halogen, hydroxy, C¹⁻⁶ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₀, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, - O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), or -S(=O)(-C₁₋₄ alkylene-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NR₆-S(=O)(R₀₇)(=N-R₀₈), -C(=O)-C₁₋₄ alkylene-N=S(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C¹⁻⁶ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl;
the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
in the groups described above, the substitutions each independently mean that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3-to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

In one embodiment, -D is a structure represented by formula (II-5) or a stereoisomer thereof: 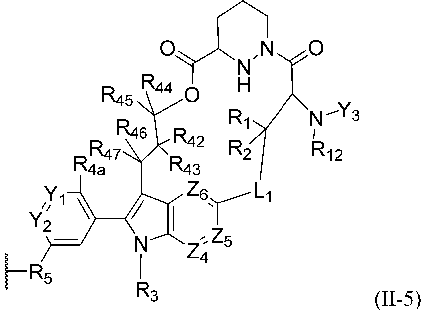 wherein in the formula,
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, - O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), or -S(=O)(-C₁₋₄ alkylene-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NR₆-S(=O)(R₀₇)(=N-R₀₈), -C(=O)-C₁₋₄ alkylene-N=S(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
in the groups described above, the substitutions each independently mean that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C=C-5- or 6-membered monocyclic heteroaryl, -C=C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1} -S(-O)₂-NR^{a1}R^{b1}, -NR^{d1}-S(O)₂-NR^{a1}R^{b1}, and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3-to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.

In one embodiment, -D is a structure represented by formula (II-6) or a stereoisomer thereof: 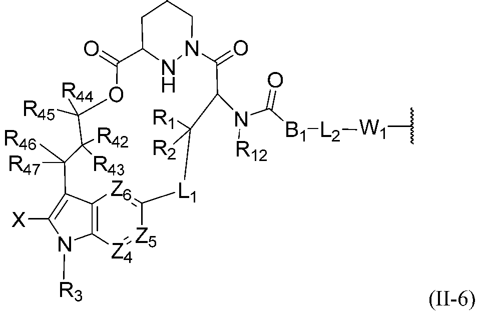 wherein in the formula,
X is 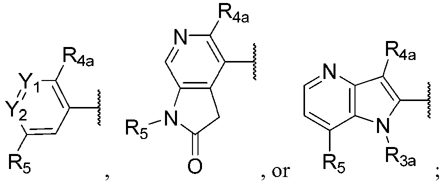
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, - O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), or -S(=O)(-C₁₋₄ alkylene-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
R₁₂ is hydrogen, C₁₋₆ alkyl, or deuterated C₁₋₆ alkyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
in the groups described above, the substitutions each independently mean that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3-to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above;
B₁ is absent or is -CH(R₉)- or >C=CR₉R_{9'}, optionally substituted 3- to 6-membered cycloalkylene, optionally substituted 3- to 6-membered heterocycloalkylene, optionally substituted 6-membered arylene, or 5- to 6-membered heteroarylene;
L₂ is absent or is a linker;
W₁ is absent or is hydrogen, cyano, optionally substituted amino, optionally substituted C₁₋₄ alkoxy, optionally substituted C₁₋₄ hydroxyalkyl, optionally substituted C₁₋₄ aminoalkyl, optionally substituted C₁₋₄ haloalkyl, optionally substituted C₁₋₄ alkyl, optionally substituted C₁₋₄ guanidinoalkyl, 3- to 11-membered heterocycloalkyl optionally substituted with C₀₋₄ alkyl, optionally substituted 3- to 8-membered cycloalkyl, optionally substituted 6- to 10-membered aryl, or optionally substituted 3- to 8-membered heteroaryl;
R₉ is hydrogen, F, optionally substituted C₁₋₆ alkyl, optionally substituted C₁₋₆ heteroalkyl, optionally substituted 3- to 6-membered cycloalkyl, or optionally substituted 3- to 7-membered heterocycloalkyl; or
R₉ and L₂, together with the atom(s) to which they are attached, form optionally substituted 3- to 14-membered heterocycloalkyl;
R_{9'} is hydrogen or optionally substituted C₁₋₆ alkyl.

In one embodiment, -D is a structure represented by formula (II-6a) or a stereoisomer thereof: 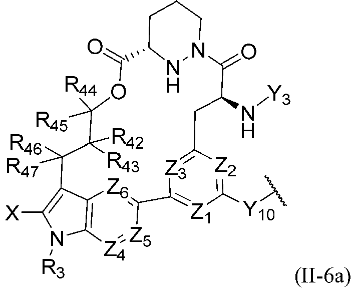 wherein in the formula,
Z₁ is C(H), C(deuterium), or N;
Z₂ is C(H), C(deuterium), or N;
Z₃ is C(H), C(deuterium), or N;
Z₄, Z₅*,* and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Y₁₀ is H, O, or NR₂₀; R₂₀ is H or C₁₋₄ alkyl;
X is 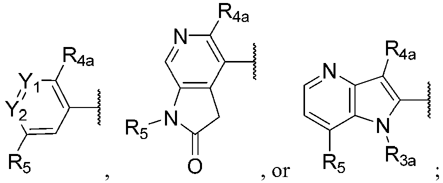
Y₁ is N or CH, wherein N may be oxidized (N⁺-O⁻);
Y₂ is N or CH, wherein N may be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
R₄₄, R₄₅, R₄₆, and R₄₇ are each independently H, deuterium, halogen, C₁₋₄ alkyl, C₁₋₄ haloalkyl, or C₁₋₄ alkoxy; R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ₐ is substituted or unsubstituted C₁₋₆ alkyl, substituted or unsubstituted C₃₋₆ cycloalkyl, or substituted or unsubstituted 3- to 8-membered heterocyclyl;
R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃Rₒ₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃Rₒ₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃Rₒ₄, -O-C₁₋₄ alkylene-NR₀₁R₀₂, -O-C₁₋₄ alkylene-C(R₀₅)R₀₃Rₒ₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃Rₒ₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, - O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), or -S(=O)(-C₁₋₄ alkylene-C(R₀₅)R₀₃R₀₄);
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, and substituted or unsubstituted C₂₋₆ alkynyl; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NR₆-S(=O)(R₀₇)(=N-R₀₈), -C(=O)-C₁₋₄ alkylene-N=S(=O)(R₀₇)(R₀₈), or R₁₁;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
in the groups described above, the substitutions each independently mean that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-(S=O)-NR^{a1}R^{b1}, and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3-to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the sulfur atom is optionally substituted with one or two groups selected from oxo and =NR^{e}; R^{e} is as defined above.
In formula (II-1), formula (II-2), formula (II-3), formula (II-4), formula (II-5), formula (II-6), and formula (II-6a),
in one embodiment, B₁ is -CH(R₉)-, optionally substituted 3- to 6-membered cycloalkylene, optionally substituted 3- to 6-membered heterocycloalkylene, optionally substituted 6-membered arylene, or 5- to 6-membered heteroarylene.

In one embodiment, B₁ is -CH(R₉)-.

In one embodiment, R₉ is

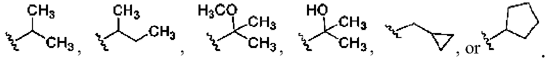

In one embodiment, B₁ is optionally substituted 6-membered arylene.

In one embodiment, B₁ is optionally substituted phenyl.

In one embodiment, L₂ is A¹-(B¹)_{f}-(C¹)_{g}-(B²)ₕ-(D¹)-(B³)ᵢ-(C²)ⱼ-(B⁴)ₖ-A², wherein A¹ is a bond between the linker and B₁; A² is a bond between W₁ and the linker; B¹, B², B³, and B⁴ are each independently selected from optionally substituted C₁₋₂ alkylene, optionally substituted C₁₋₃ heteroalkylene, O, S, and NR^{N}; R^{N} is hydrogen, optionally substituted C₁₋₄ alkyl, optionally substituted C₁₋₃ cycloalkyl, optionally substituted C₂₋₄ alkenyl, optionally substituted C₂₋₄ alkynyl, optionally substituted 3- to 14-membered heterocycloalkyl, optionally substituted 6- to 10-membered aryl, or optionally substituted C₁₋₇ heteroalkyl; C¹ and C² are each independently selected from carbonyl, thiocarbonyl, sulfonyl, and phosphoryl; f, g, h, i, j, and k are each independently 0 or 1; and D¹ is optionally substituted C₁₋₁₀ alkylene, optionally substituted C₂₋₁₀ alkenylene, optionally substituted C₂₋₁₀ alkynylene, optionally substituted 3- to 14-membered heterocycloalkylene, optionally substituted 5- to 10-membered heteroarylene, optionally substituted 3- to 8-membered cycloalkylene, optionally substituted 6- to 10-membered arylene, optionally substituted C₂₋₁₀ polyethylene glycol, optionally substituted C₁₋₁₀ heteroalkylene, or a chemical bond linking A¹-(B¹)_{f}-(C¹)_{g}-(B²)ₕ- to -(B³)ᵢ-(C²)ⱼ-(B⁴)ₖ-A². In one embodiment, L₂ is 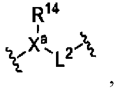 wherein X^{a} is absent or is N; R¹⁴ is absent or is hydrogen, optionally substituted C₁₋₆ alkyl, or optionally substituted C₁₋₃ cycloalkyl; and L₂ is absent or is -C(=O)-, -S(=O)₂-, optionally substituted C₁₋₄ alkylene, or optionally substituted C₁₋₄ heteroalkylene, wherein at least one of X^{a}, R¹⁴, and L² is absent.

In one embodiment, L₂ is 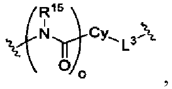 wherein o is 0 or 1; R¹⁵ is hydrogen or optionally substituted C₁₋₆ alkyl; Cy is optionally substituted 3- to 8-membered cycloalkylene, optionally substituted 3- to 8-membered heterocycloalkylene, optionally substituted 6- to 10-membered arylene, or optionally substituted 5- to 10-membered heteroarylene; and L³ is absent or is -C(=O)-, -S(=O)₂-, optionally substituted C₁₋₄ alkylene, or optionally substituted C₁₋₄ heteroalkylene.

In one embodiment, L₂ is selected from the group consisting of:

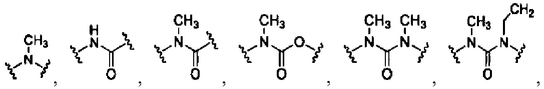

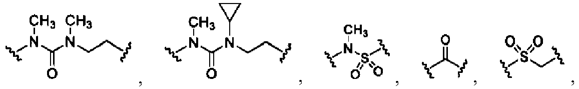

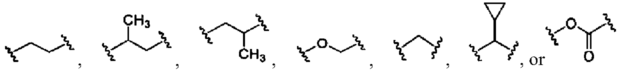

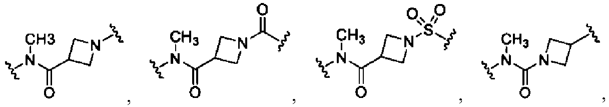

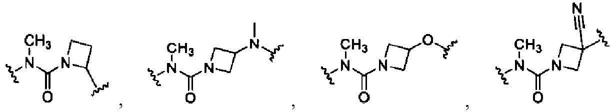

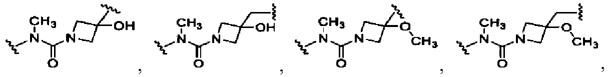

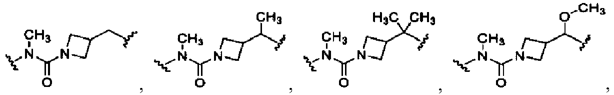

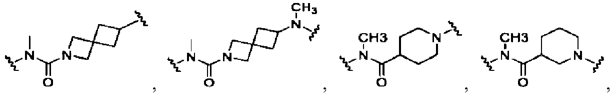

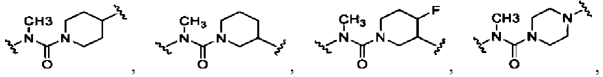

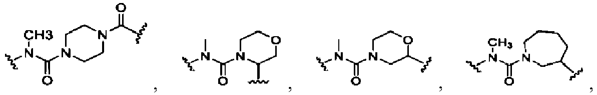

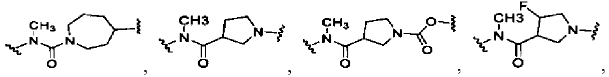

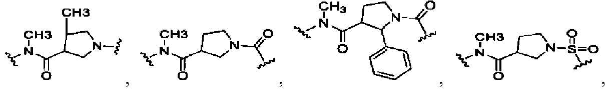

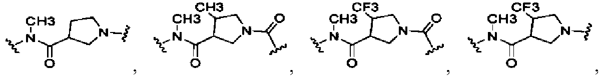

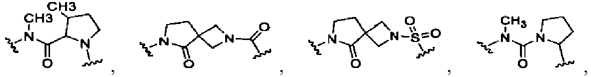

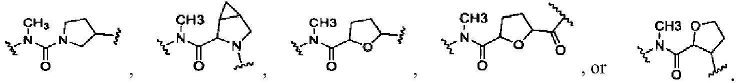

In one embodiment, W₁ is hydrogen, optionally substituted amino, optionally substituted C₁₋₄ alkoxy, optionally substituted C₁₋₄ hydroxyalkyl, optionally substituted C₁₋₄ aminoalkyl, optionally substituted C₁₋₄ haloalkyl, optionally substituted C₁₋₄ alkyl, optionally substituted C₁₋₄ guanidinoalkyl, 3- to 8-membered heterocycloalkyl optionally substituted with C₀₋₄ alkyl, optionally substituted 3- to 8-membered cycloalkyl, or optionally substituted 3- to 8-membered heteroaryl.

In one embodiment, W₁ is hydrogen. In one embodiment, W₁ is optionally substituted amino. In one embodiment, W₁ is -NHCH3 or -N(CH3)2. In one embodiment, W₁ is optionally substituted C₁₋₄ alkoxy. In one embodiment, W₁ is methoxy or isopropoxy. In one embodiment, W₁ is optionally substituted C₁₋₄ alkyl. In one embodiment, W₁ is methyl, ethyl, isopropyl, tert-butyl, or benzyl. In one embodiment, W₁ is optionally substituted amido. In one embodiment, W₁ is optionally substituted C₁₋₄ hydroxyalkyl. In one embodiment, W₁ is optionally substituted C₁₋₄ aminoalkyl.

In one embodiment, W₁ is selected from the group consisting of:

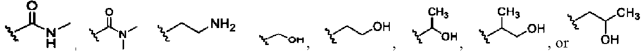

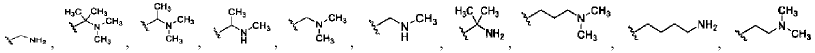

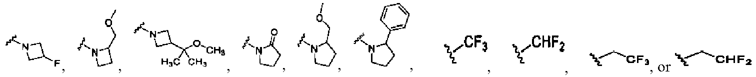

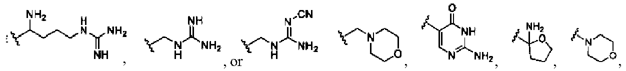

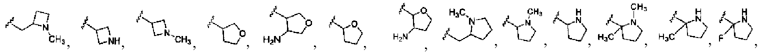

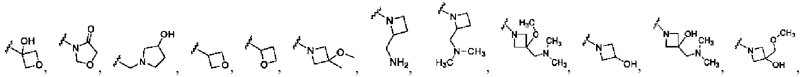

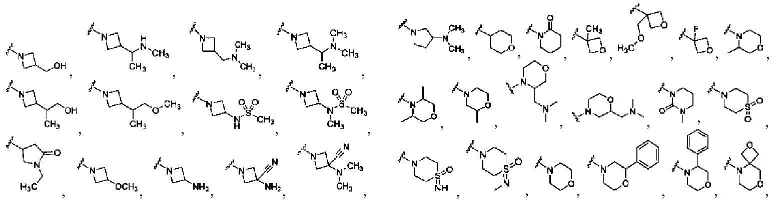

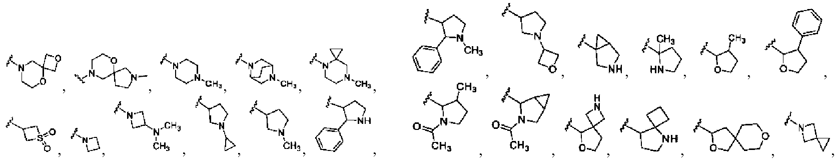

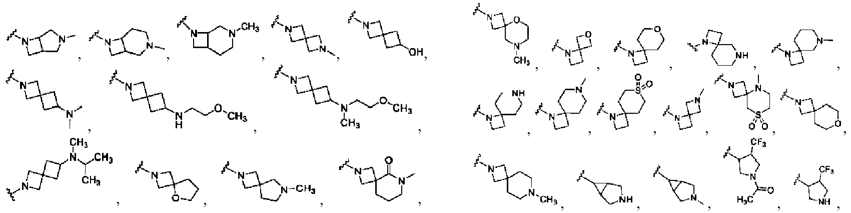

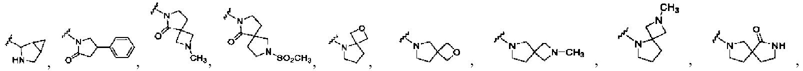

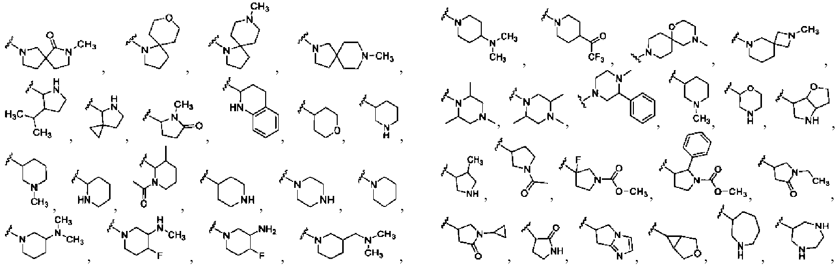

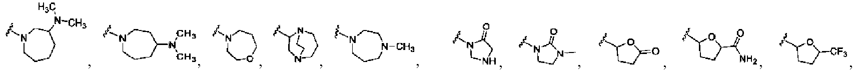

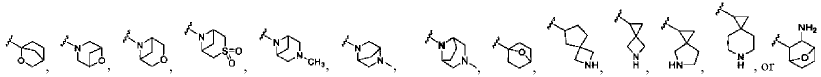

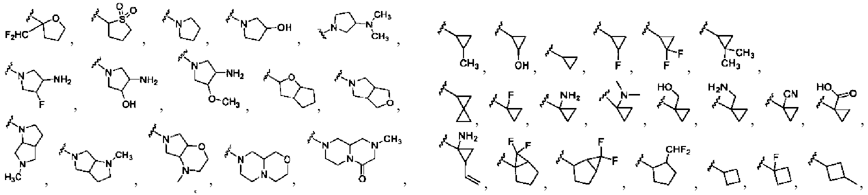

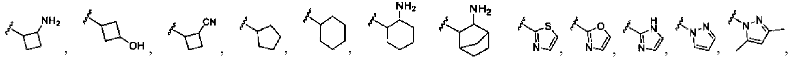

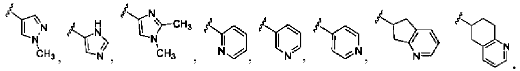

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, R₁ is hydrogen; R₂ is hydrogen.

In one embodiment, R₁ is ethoxy; R₂ is hydrogen.

In one embodiment, R₁ is hydrogen or C₁₋₆ alkoxy; R₂ is hydrogen.

In one embodiment, R₁ is hydrogen; R₂ is hydrogen.

In one embodiment, R₃ is hydrogen, C₁₋₆ alkyl, C₁₋₆ haloalkyl, or deuterated C₁₋₆ alkyl.

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₃ is hydrogen, C₁₋₆ alkyl, or C₁₋₆ haloalkyl.

In one embodiment, R₃ is hydrogen or C₁₋₆ alkyl.

In one embodiment, R₃ is trifluoromethyl, trifluoroethyl, or trifluoropropyl.

In one embodiment, R₃ is ethyl.

In one embodiment, R₃ is haloethyl. In one embodiment, R₃ is trifluoroethyl.

In one embodiment, R₄₂ is methyl, and R₄₃ is trifluoromethyl.

In one embodiment, R₄₂ is hydrogen, and R₄₃ is trifluoromethyl.

In one embodiment, R₄₂ is fluorine, and R₄₃ is trifluoromethyl.

In one embodiment, R₄₂ is hydrogen, and R₄₃ is fluorine.

In one embodiment, R₄₂ is hydrogen, and R₄₃ is methyl.

In one embodiment, R₄₂ and R₄₃, together with the carbon atom to which they are attached, form cyclopropyl or cyclobutyl.

In one embodiment, R₄₂ and R₄₃ are both methyl.

In one embodiment, R₄₂ and R₄₃ are not both methyl.

In one embodiment, R₄₂ is hydrogen; R₄₃ is hydrogen, halogen, or substituted or unsubstituted C₁₋₆ alkyl.

In one embodiment, R₄₂ and R₄₃, together with the carbon atom to which they are attached, form substituted or unsubstituted cyclopropyl, substituted or unsubstituted cyclobutyl, or substituted or unsubstituted cyclopentyl. In one embodiment, R₄₂ and R₄₃, together with the carbon atom to which they are attached, form unsubstituted cyclopropyl.

In one embodiment, R₄₄ is hydrogen.

In one embodiment, R₄₅ is hydrogen.

In one embodiment, R₄₆ is hydrogen.

In one embodiment, R₄₇ is hydrogen.

In one embodiment, Z₄ is N or CH.

In one embodiment, Z₅ is N or CH.

In one embodiment, Z₆ is N or CH.

In one embodiment, R₅ is 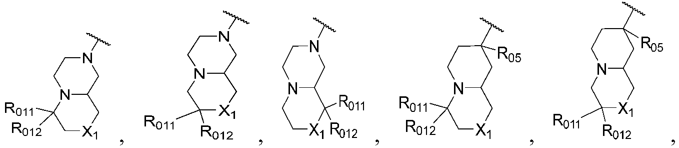 or 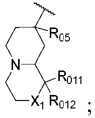
R₀₁₁ and R₀₁₂ are each independently hydrogen, C₁₋₆ alkyl, C₁₋₆ haloalkyl, or deuterated C₁₋₆ alkyl; or R₀₁₁ and R₀₁₂, together with the carbon atom to which they are attached, form substituted or unsubstituted C₃₋₆ cycloalkyl; the substitution is as defined herein;
X₁ is a bond, S, O, or N(R₀₁₃); R₀₁₃ is hydrogen, C₁₋₆ alkyl, or deuterated C₁₋₆ alkyl;
R₀₅ is hydrogen, deuterium, or C₁₋₆ alkyl.

In one embodiment, R₅ is hydrogen,

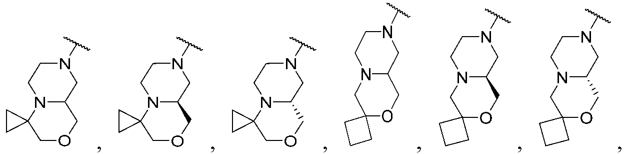

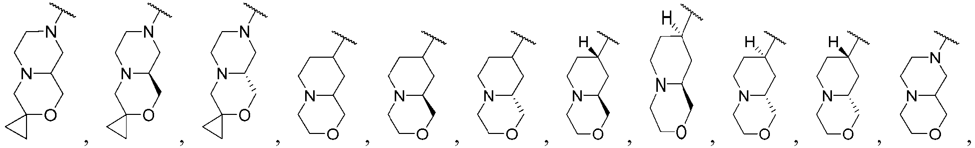

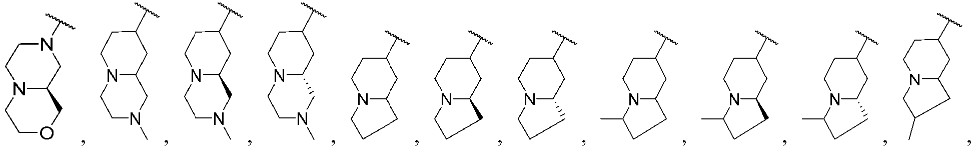

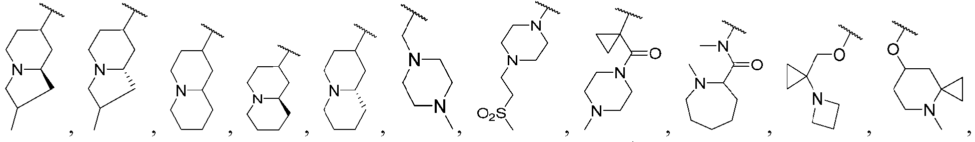

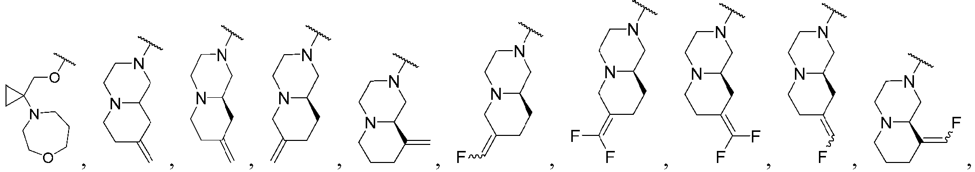

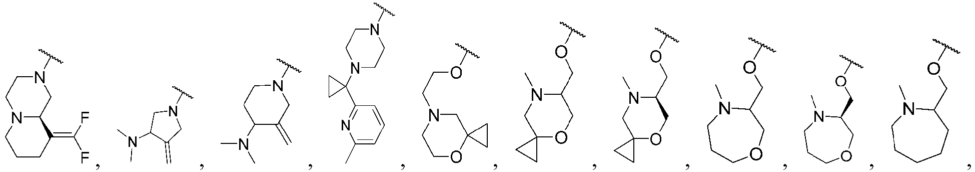

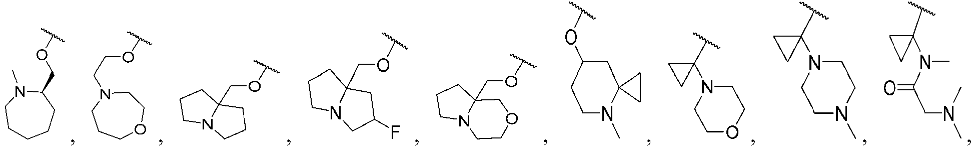

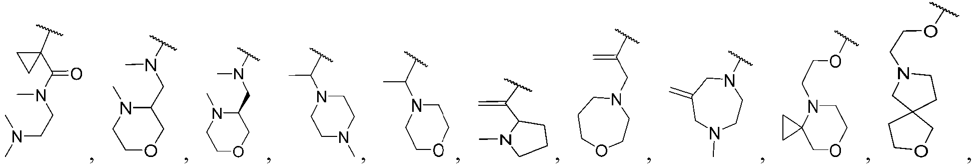

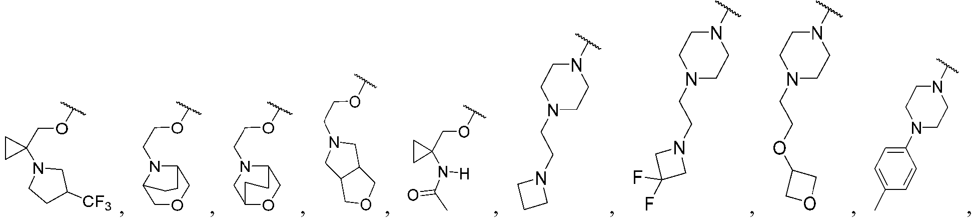

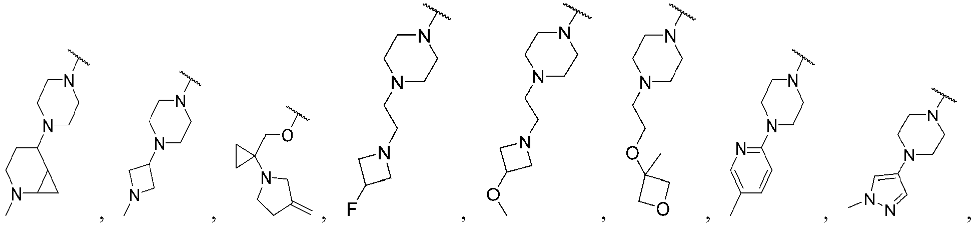

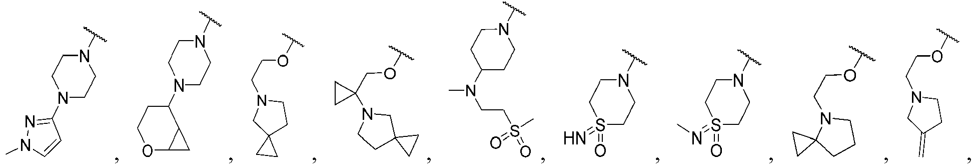

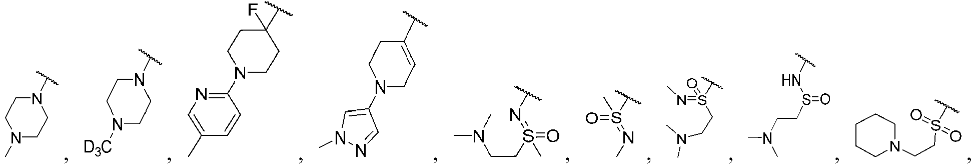

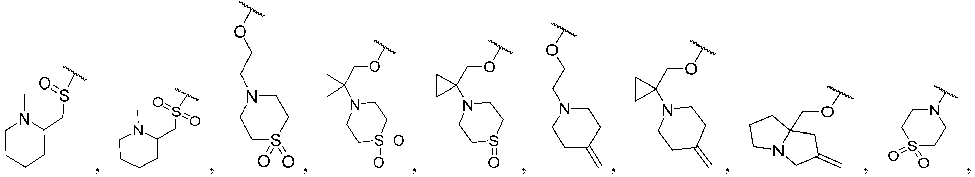

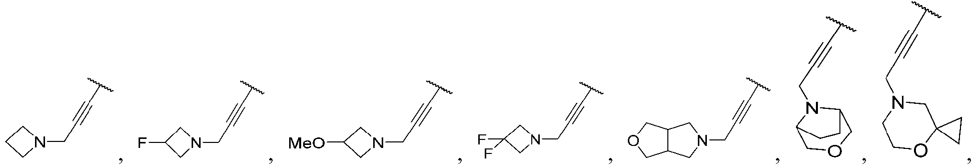

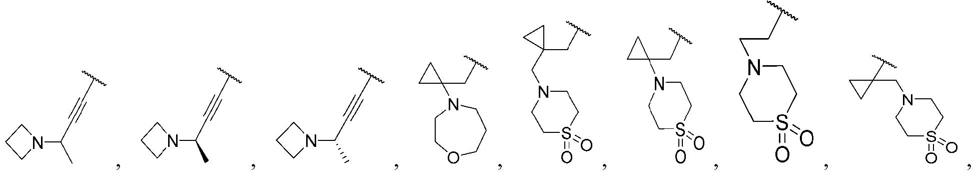

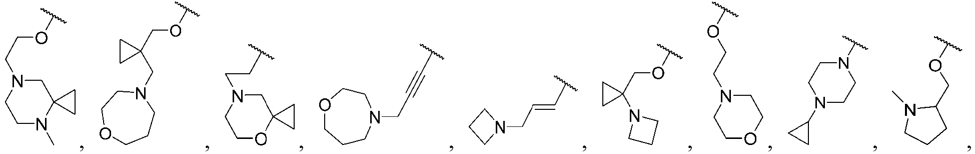

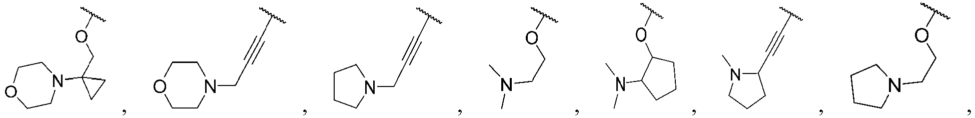

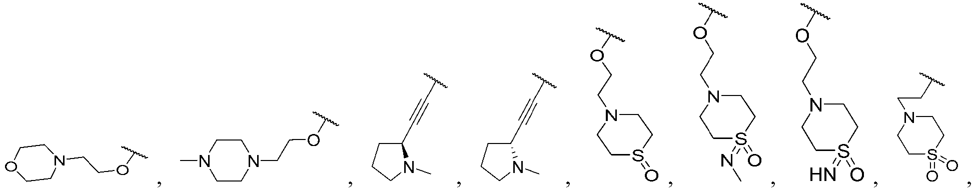

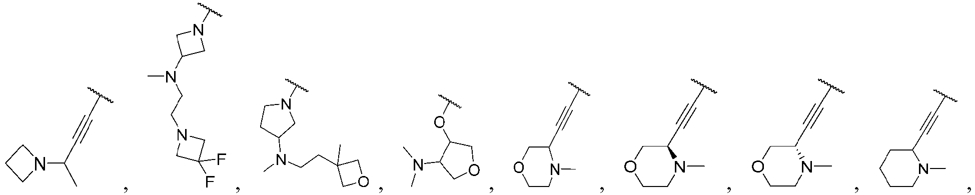

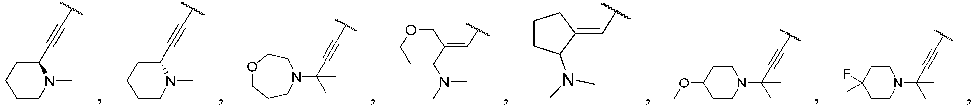

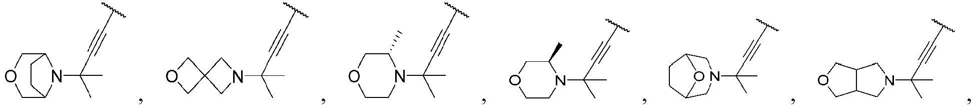

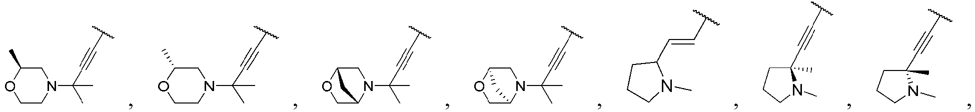

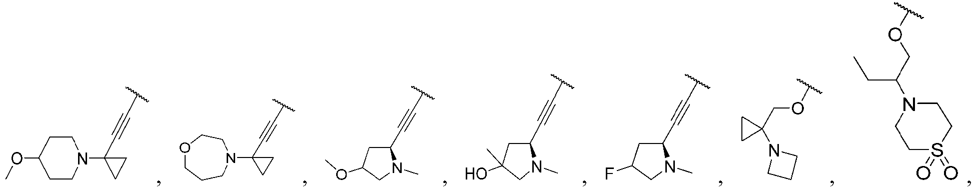

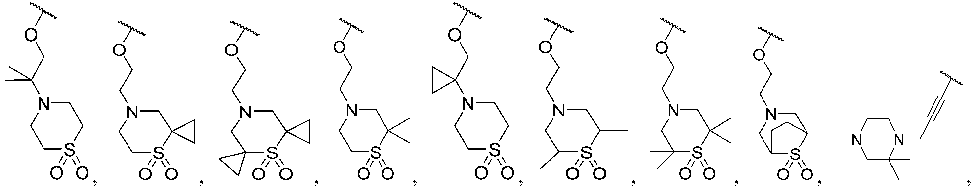

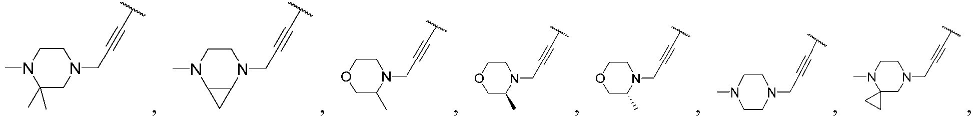

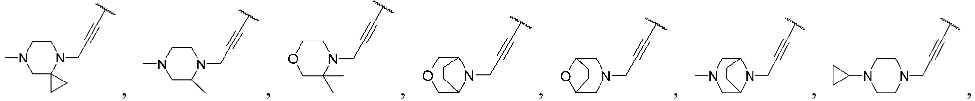

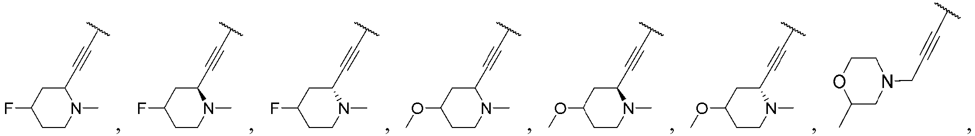

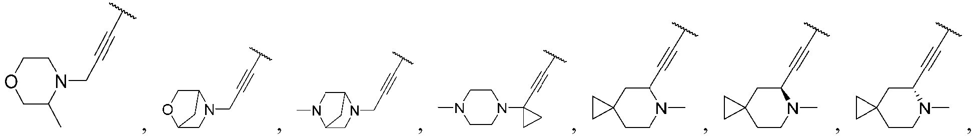

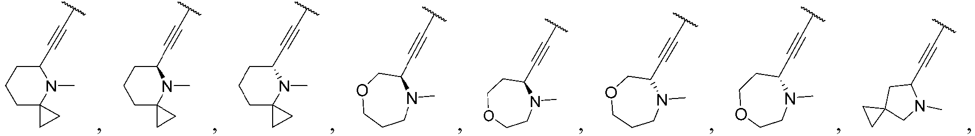

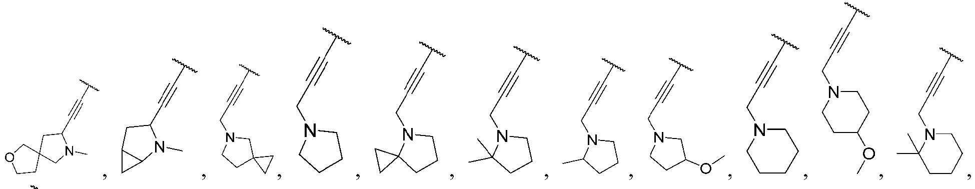

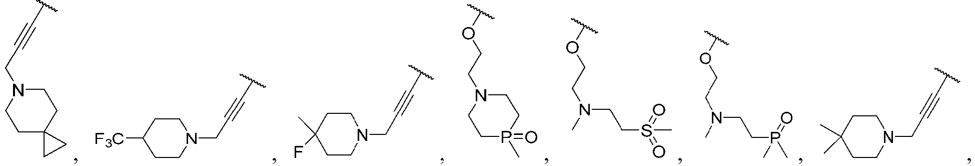

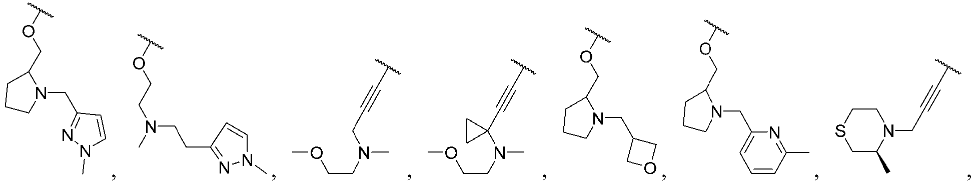

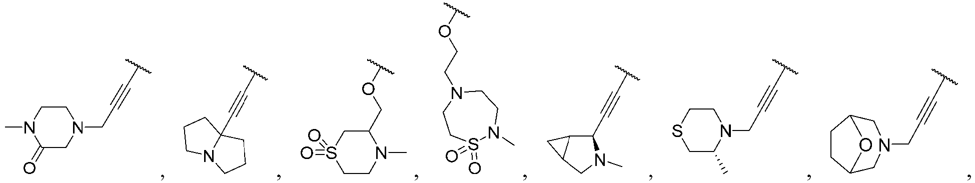

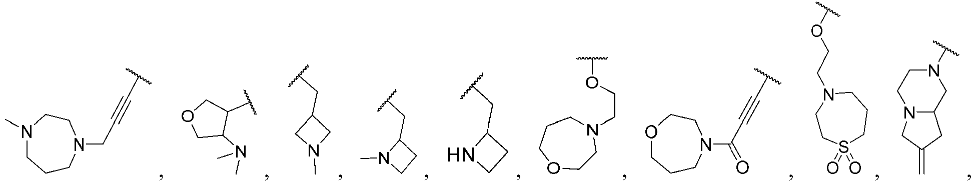

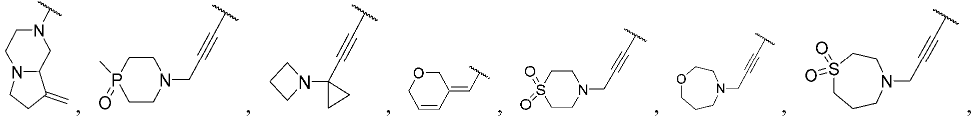

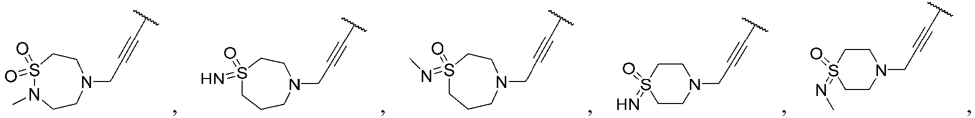

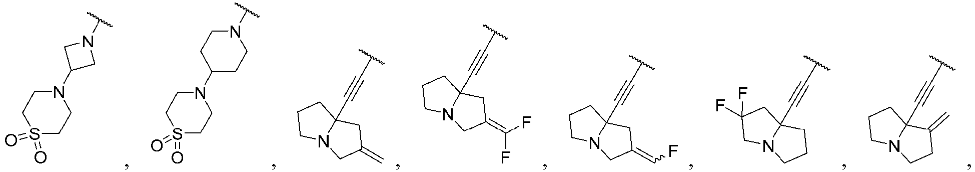

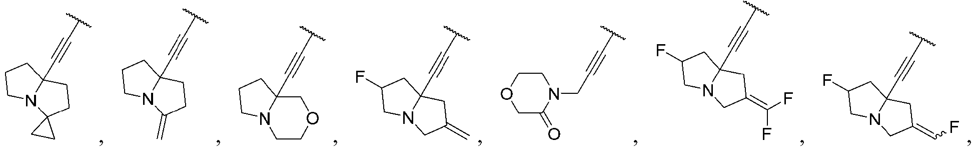

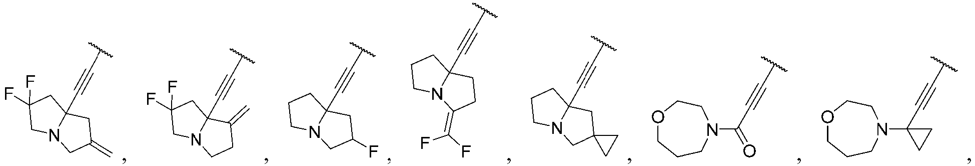

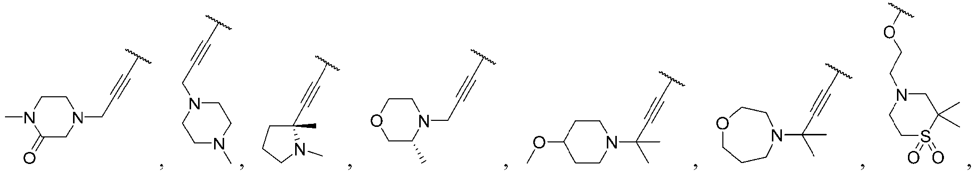

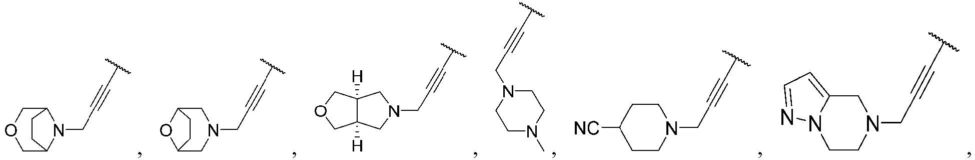

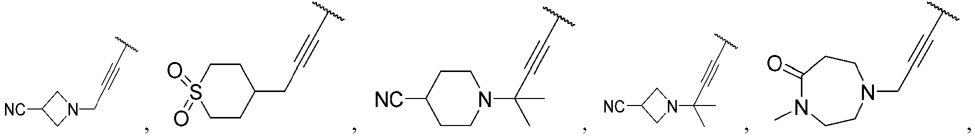

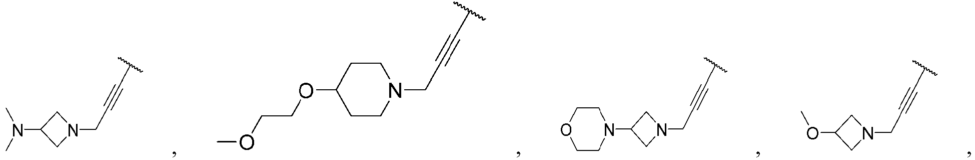

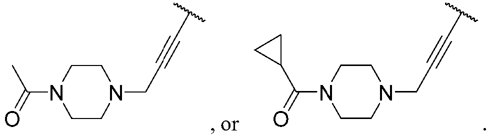

In one embodiment, R₅ is hydrogen or methylpiperazinyl.

In one embodiment, R₆ is substituted or unsubstituted C₃₋₆ cycloalkyl; the substitution is as defined herein.

In one embodiment, R₆ is cyclopropyl substituted with C₁₋₆ alkyl. Preferably, R₆ is cyclopropyl substituted with methyl. Preferably, R₆ is cyclopropyl substituted with two methyl groups. For example, R₆ is

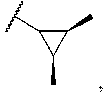

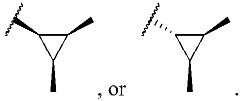

In one embodiment, R₆ is halogen-substituted or unsubstituted C₁₋₆ alkyl.

In one embodiment, R₆ is substituted or unsubstituted C₁₋₆ alkyl, or substituted or unsubstituted C₃₋₆ cycloalkyl.

In one embodiment, R₆ is C₁₋₆ alkyl substituted with halogen, preferably C₁₋₆ alkyl substituted with F.

In one embodiment, R₆ is substituted or unsubstituted cyclopropyl.

In one embodiment, R₆ is cyclopropyl substituted with C₁₋₆ alkyl, preferably cyclopropyl substituted with one or two methyl groups.

**In** one embodiment, R₆ is

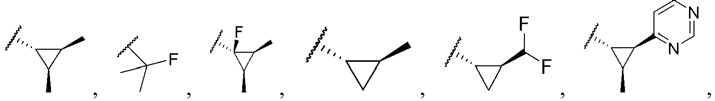

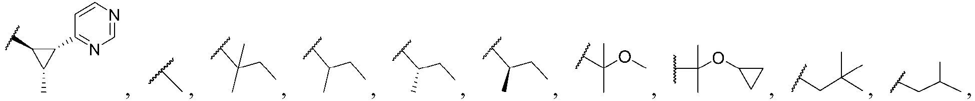

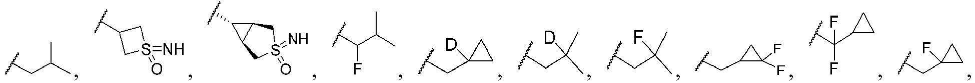

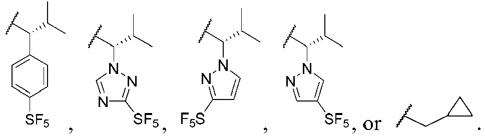

In one embodiment, R₁₂ is hydrogen.

In one embodiment, L₁ is 5- or 6-membered monocyclic heteroaryl.

In one embodiment, L₁ is thiazolyl.

In one embodiment, R₁₁ is 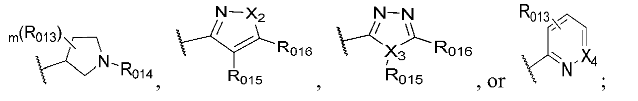 ;
wherein X₂ is NH, O, or CR₀₁₃; X₃ and X₄ are each independently N or CR₀₁₃;
R₀₁₃ and R₀₁₄ are each independently hydrogen, halogen, oxo, C₁₋₆ alkyl, or C₁₋₆ alkoxy; m is 1, 2, or 3;
R₀₁₅ and R₀₁₆ are each independently hydrogen, C₁₋₆ alkyl, C₃₋₆ cycloalkyl, or -C₁₋₆ alkyl-5- or 6-membered monocyclic heteroaryl; or R₀₁₅ and R₀₁₆, together with the carbon atom to which they are attached, form substituted or unsubstituted 3- to 8-membered monocyclic heterocyclyl; the substitution is as defined herein. In one embodiment, R₁₁ is pyrrolidinyl, isoxazolyl, triazolyl, pyridazinyl, or triazolopyridinyl, wherein the pyrrolidinyl, isoxazolyl, triazolyl, pyridazinyl, or triazolopyridinyl is optionally substituted with a substituent selected from oxo, halogen, C₁₋₆ alkyl, C₁₋₆ alkoxy, C₃₋₆ cycloalkyl, and -C₁₋₄ alkylene-5- or 6-membered heteroaryl.

In one embodiment, R₁₁ is selected from the group consisting of:

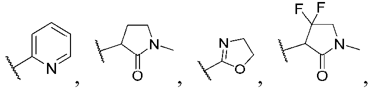

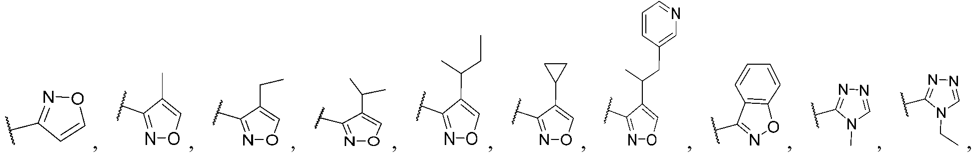

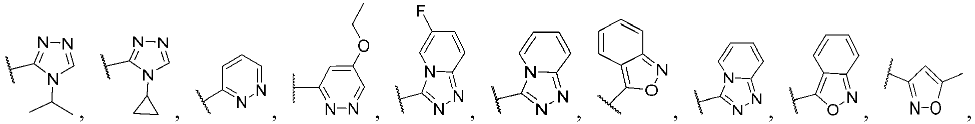

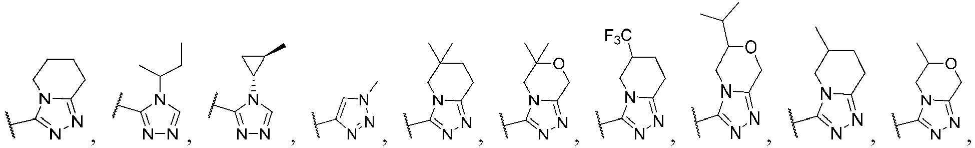

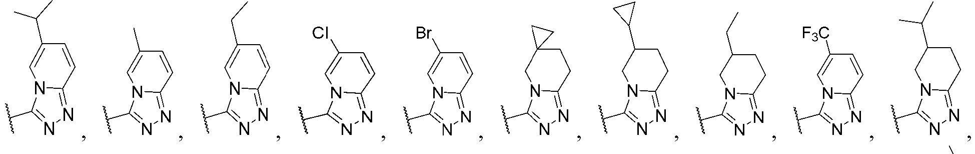

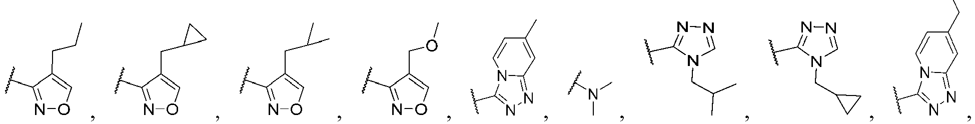

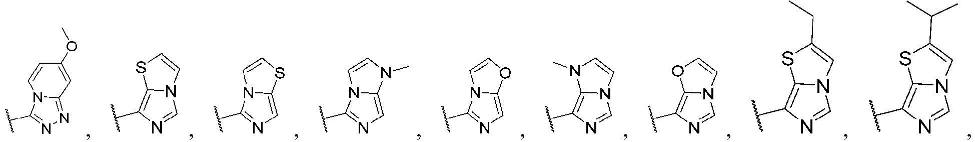

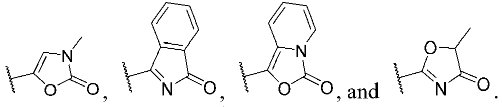

In one embodiment, 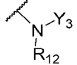 is selected from the group consisting of:

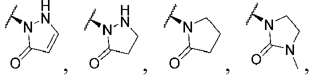

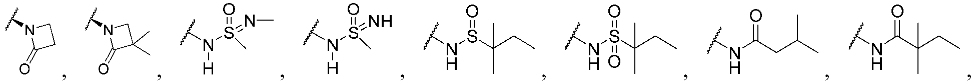

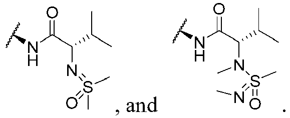

In one embodiment, Y₁ is N; Y₂ is CH.

In one embodiment, Y₁ is CH; Y₂ is N.

In one embodiment, Y₁ is N; Y₂ is N.

In one embodiment, Y₁ is N⁺-O⁻; Y₂ is CH.

In one embodiment, Y₁ is CH; Y₂ is N⁺-O⁻.

In one embodiment, 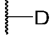 is a compound represented by any one of formula (AIII-I) to formula (AIII-10), or a stable deuterated derivative thereof, a stereoisomer thereof, or a pharmaceutically acceptable salt thereof: 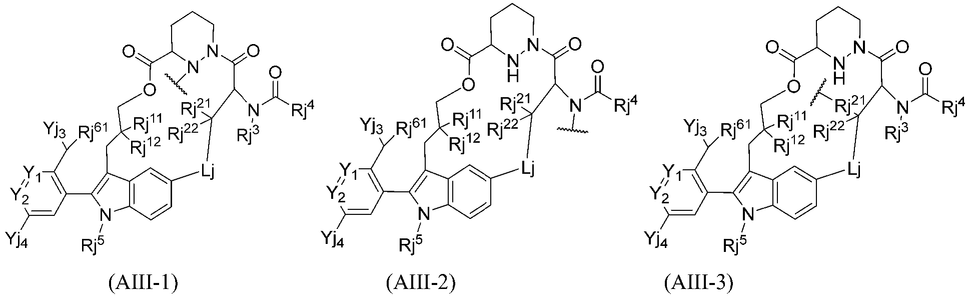 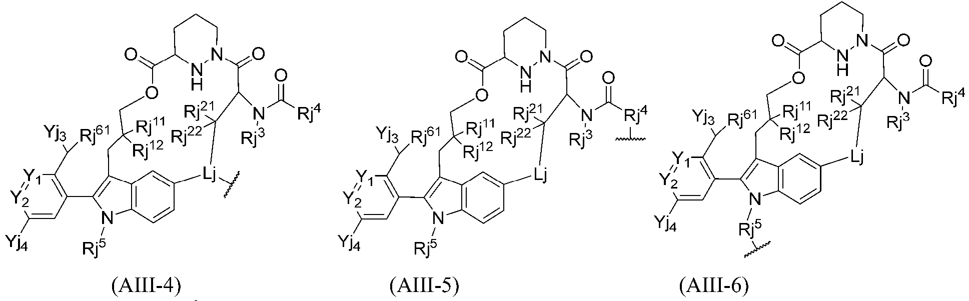 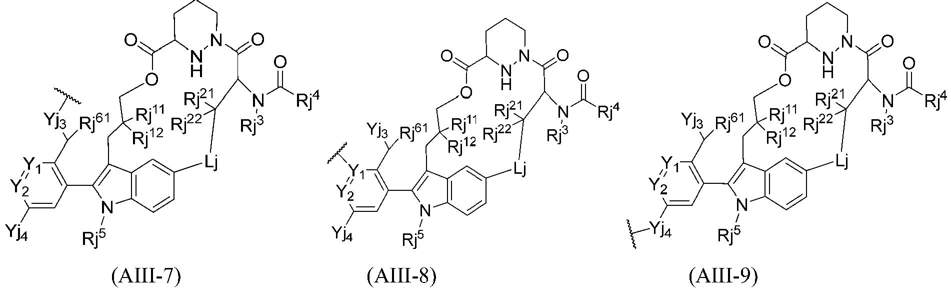 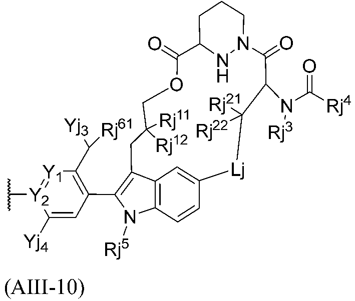 wherein in the formulas, Rj¹¹, Rj¹², Rj²¹, Rj²², Rj³, Rj⁴, Rj⁵, Rj⁶¹, Y₁, Y₂, Yj₃, Yj₄, and Lj are as defined in any one of the embodiments, and the 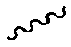 end is attached to L₀.

In one embodiment, the structural unit 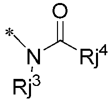 is selected from the group consisting of: 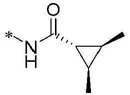 (preferably

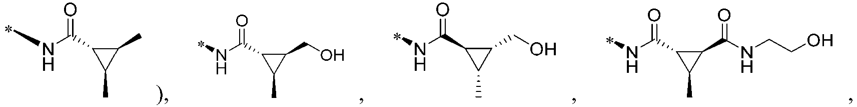

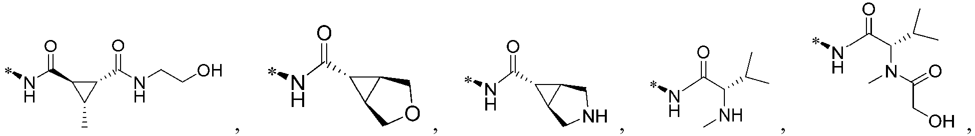

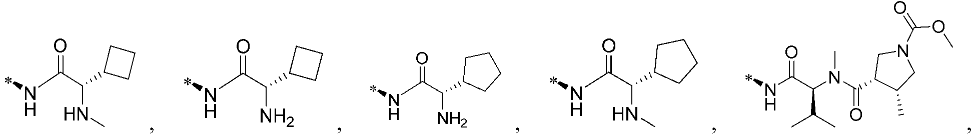

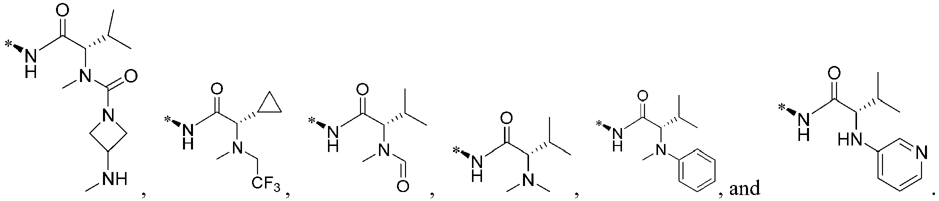

In one embodiment, the structural unit 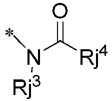 is selected from the group consisting of: 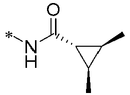 (preferably

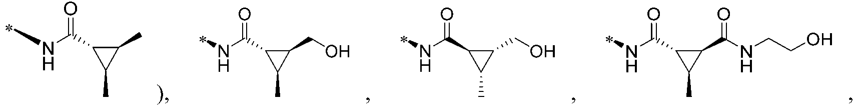

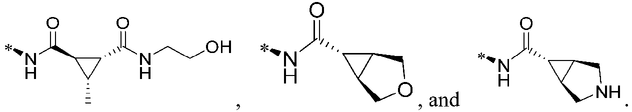

In one embodiment, Yj₄ is 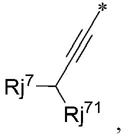 wherein Rj⁷, Rj⁷¹, Rj¹¹, Rj¹², Rj²¹, Rj²², Rj³, Rj⁴, Rj⁵, Rj⁶¹, Y₁, Y₂, Yj₃, and Lj are as defined in any one of the embodiments, and the * end is attached to another fragment in D.

In one embodiment, Yj₄ is 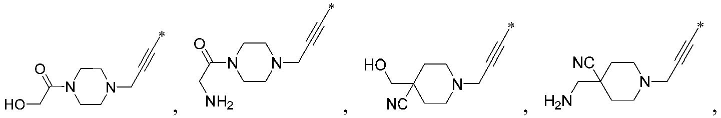 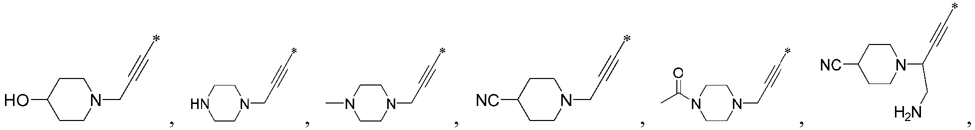 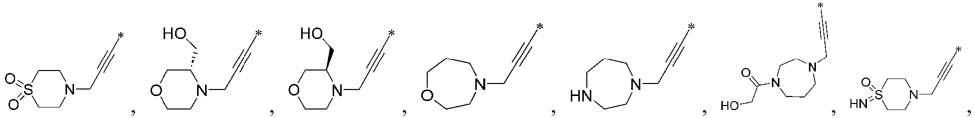 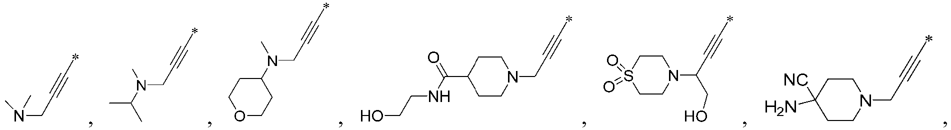 or 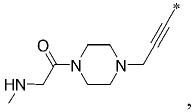 wherein the * end is attached to another fragment in D .

In one embodiment, 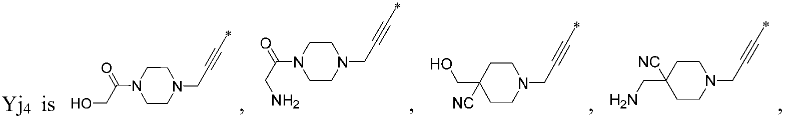 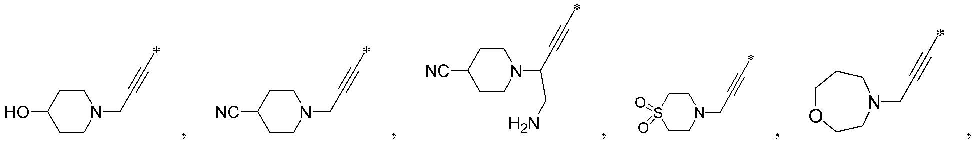 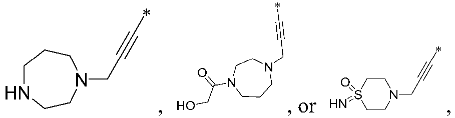 wherein the * end is attached to another fragment in D.

In one embodiment, Yj₄ is

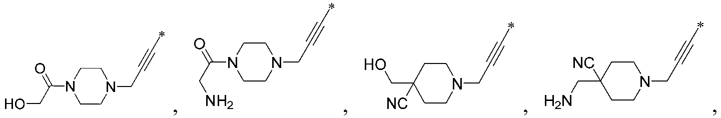

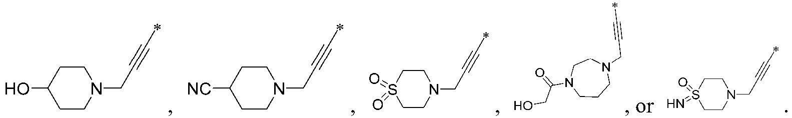

In one embodiment, Yj₄ is 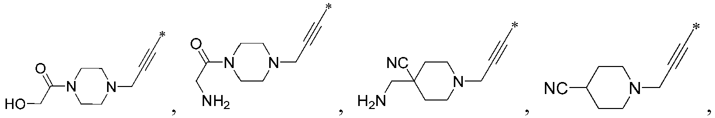 or 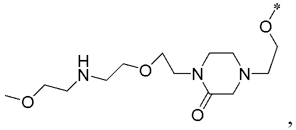 wherein the * end is attached to another fragment in D.

In one embodiment, when L₀ is attached to Yj₄, Yj₄ is 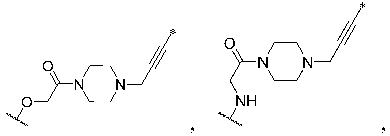 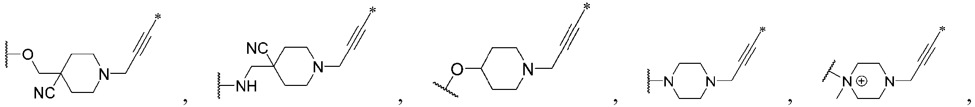 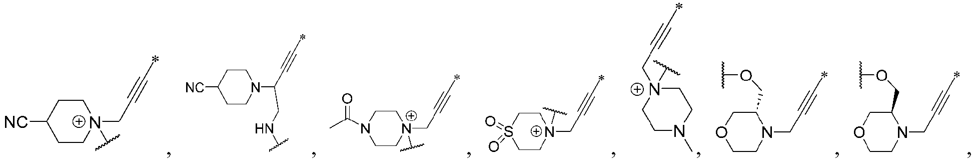 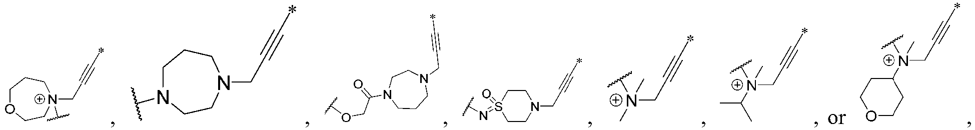 wherein the 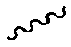 end is attached to L₀, and the * end is attached to another fragment in D.

In one embodiment, when L₀ is attached to Yj₄, Yj₄ is 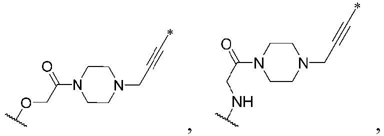 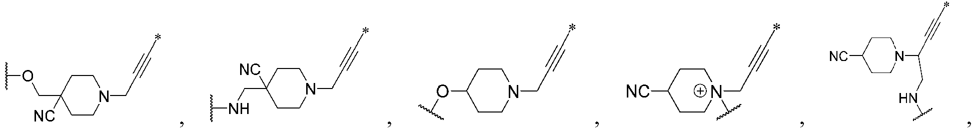 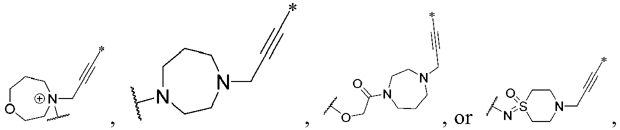 wherein the 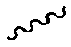 end is attached to L₀, and the * end is attached to another fragment in D.

In one embodiment, when L₀ is attached to Yj₄, Yj₄ is 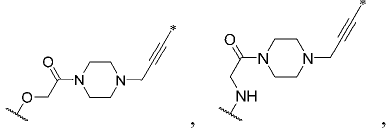 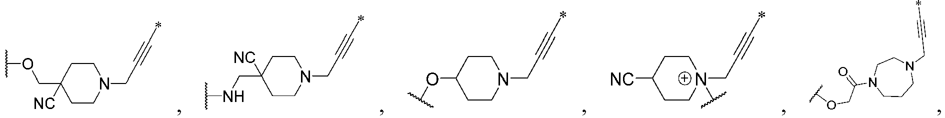 or 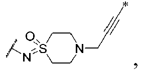 wherein the 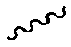 end is attached to L₀, and the * end is attached to another fragment in D.

In one embodiment, when L₀ is attached to Yj₄, Yj₄ is 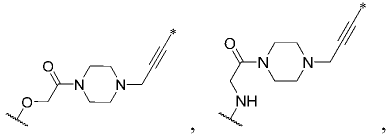 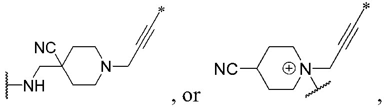 wherein the 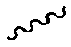 end is attached to L₀, and the * end is attached to another fragment in D.

In one embodiment, Yj₄ is 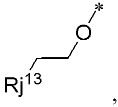 wherein Rj¹³, Rj¹¹, Rj¹², Rj²¹, Rj²², Rj³, Rj⁴, Rj⁵, Rj⁶¹, Y₁, Y₂, Yj₃, and Lj are as defined in any one of the embodiments, and the * end is attached to another fragment in D.

In one embodiment, Yj₄ is 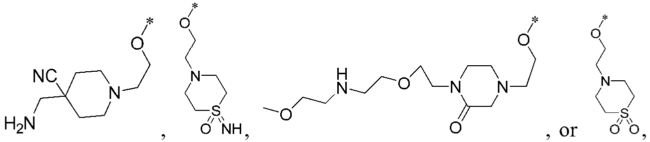 wherein the * end is attached to another fragment in D.

In one embodiment, Yj₄ is 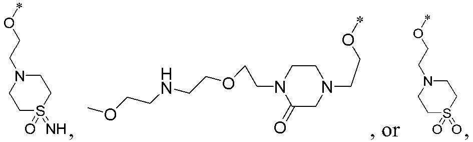 wherein the * end is attached to another fragment in D.

In one embodiment, Yj₄ is 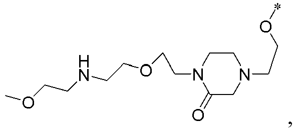 wherein the * end is attached to another fragment in D.

In one embodiment, Rj⁴ is C₃₋₆ cycloalkyl or 3- to 8-membered heterocyclyl, wherein the C₃₋₆ cycloalkyl or 3-to 8-membered heterocyclyl is optionally substituted with one or more substituents selected from hydroxy, methyl, -C₁₋₄ alkylene-OH, and -C(O)-NH(C₁₋₄ alkylene-OH).

In one embodiment, Rj⁴ is cyclopropyl, cyclobutyl, cyclopentyl, or 5- to 8-membered bicyclic heterocyclyl, wherein the cyclopropyl, cyclobutyl, cyclopentyl, or 5- to 8-membered bicyclic heterocyclyl is optionally substituted with 1, 2, or 3 substituents selected from hydroxy, methyl, -CH₂OH, or -C(O)-NH(CH₂CH₂OH).

In one embodiment, Rj⁴ is 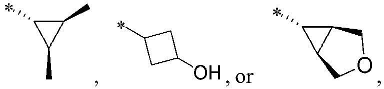 wherein the * end is attached to another fragment in D.

In one embodiment, Lj is 5- or 6-membered heteroaryl.

In one embodiment, Lj is oxazolyl.

In one embodiment, Rj₅ is C₁₋₄ alkyl.

In one embodiment, Rj₅ is ethyl.

In one embodiment, when L₀ is attached to Yj₄ or Rj⁴, any hydrogen atom on Yj₄ or Rj⁴ is replaced by L₀, or any N atom on Yj₄ or Rj⁴ is quaternized, wherein Yj₄ and Rj⁴ are as defined in any one of the embodiments.

In one embodiment, when L₀ is attached to Yj₄ or Rj⁴, Yj₄ is 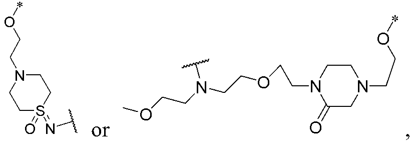 wherein the 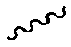 end is attached to L₀, and the * end is attached to another fragment in D.

In one embodiment, when L₀ is attached to Yj₄ or Rj⁴, Rj⁴ is 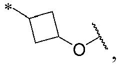 wherein the 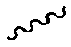 end is attached to L₀, and the * end is attached to another fragment in D.

In one embodiment, Yj₄ is 3- to 8-membered monocyclic heterocyclyl, 7- to 12-membered bicyclic heterocyclyl, or phenyl, wherein the 3- to 8-membered monocyclic heterocyclyl, 7- to 12-membered bicyclic heterocyclyl, or phenyl is optionally substituted with one or more substituents selected from C₁₋₆ alkyl, -C₁₋₄ alkylene-hydroxy, and -C₁₋₄ alkylene-3- to 8-membered monocyclic heterocyclyl, and Rj¹¹, Rj¹², Rj²¹, Rj²², Rj³, Rj⁴, Rj⁵, Rj⁶¹, Y₁, Y₂, Yj₃, and Lj are as defined in any one of the embodiments.

In one embodiment, Yj₄ is as defined in any one of the embodiments.

In one embodiment, Rj⁴ is C₃₋₆ cycloalkyl or 3- to 8-membered heterocyclyl, wherein the C₃₋₆ cycloalkyl or 3-to 8-membered heterocyclyl is optionally substituted with one or more substituents selected from hydroxy, methyl, -C₁₋₄ alkylene-OH, and -C(O)-NH(C₁₋₄ alkylene-OH).

In one embodiment, Rj⁴ is cyclopropyl, cyclobutyl, or cyclopentyl, wherein the cyclopropyl, cyclobutyl, or cyclopentyl is optionally substituted with 1, 2, or 3 substituents selected from hydroxy, methyl, and -CH₂OH.

In one embodiment, Rj⁴ is 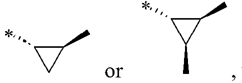 wherein the * end is attached to another fragment in D.

In one embodiment, when L₀ is attached to Yj₄, Rj⁵, Lj, or Rj²¹, any hydrogen atom on Yj₄, Rj⁵, Lj, or Rj²¹ is replaced by L₀, or any N atom on Yj₄, Rj⁵, Lj, or Rj²¹ is quaternized, wherein Yj₄, Rj⁵, Lj, and Rj²¹ are as defined in any one of the embodiments.

In one embodiment, when L₀ is attached to Yj₄, Yj₄ is 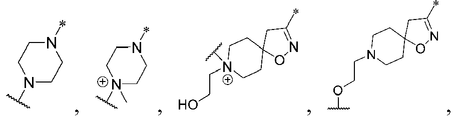 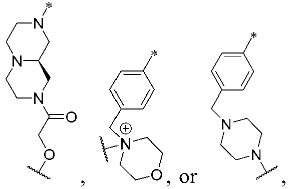 wherein the 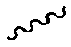 end is attached to L₀, and the * end is attached to another fragment in D.

In one embodiment, when L₀ is attached to Yj₄, Yj₄ is 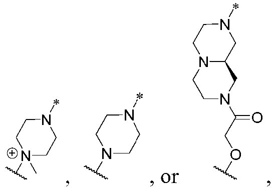 wherein the 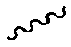 end is attached to L₀, and the * end is attached to another fragment in D.

In one embodiment, when L₀ is attached to Rj⁵, Rj⁵ is 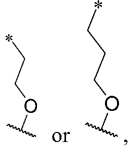 wherein the 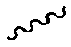 end is attached to L₀, and the * end is attached to another fragment in D.

In one embodiment, when L₀ is attached to Lj, Lj is 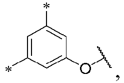 wherein the 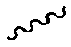 end is attached to L₀, and the * end is attached to another fragment in D.

In one embodiment, when L₀ is attached to Rj²¹, Rj²¹ is 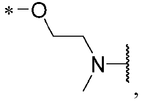 wherein the 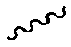 end is attached to L₀, and the * end is attached to another fragment in D.

In one embodiment, 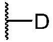 is a compound represented by any one of formula (AIA-1) to formula (AIA-8), or a stable deuterated derivative thereof, a stereoisomer thereof, or a pharmaceutically acceptable salt thereof: 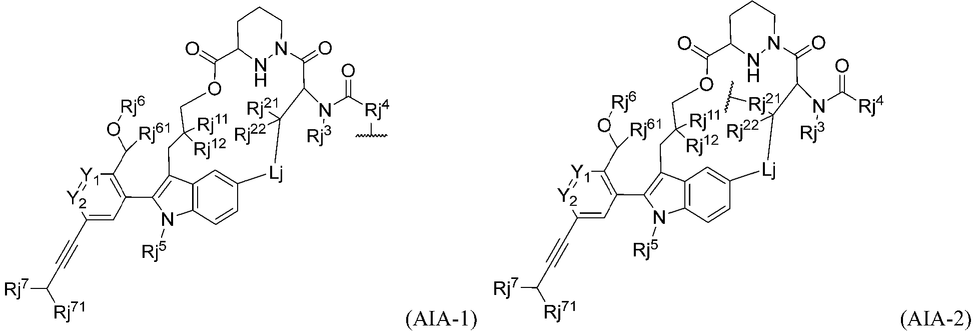 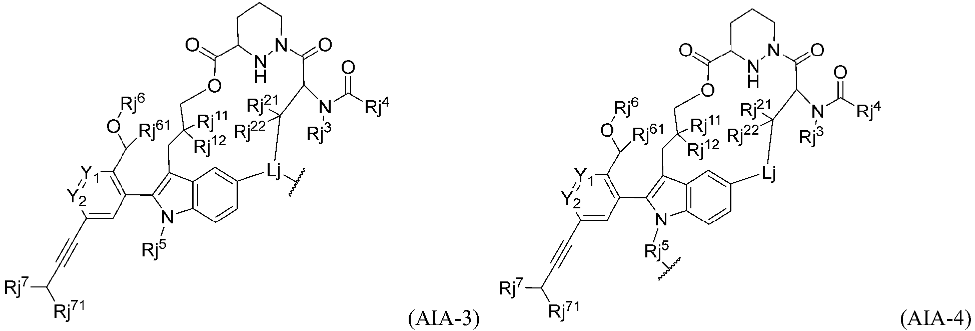 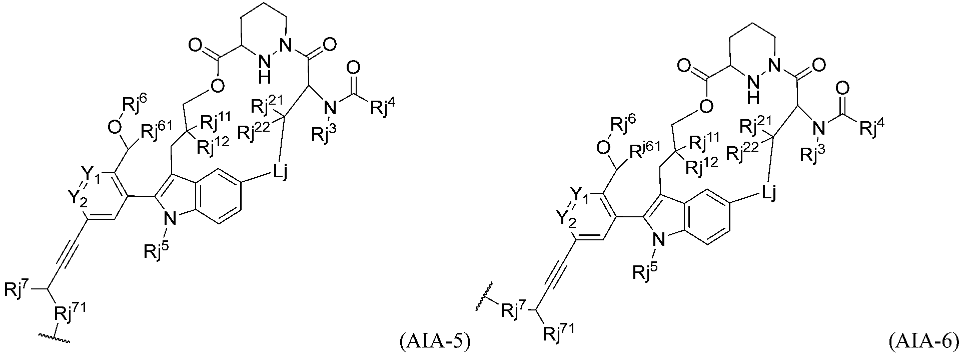 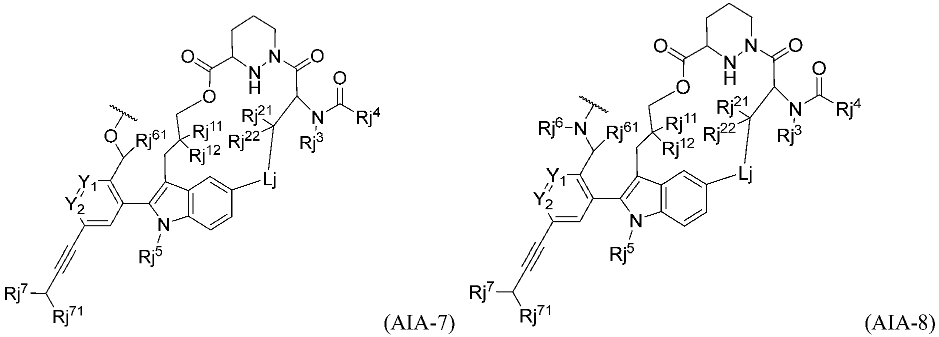 wherein in the formulas, Rj¹¹, Rj¹², Rj²¹, Rj²², Y₁, Y₂, Rj⁵, Rj⁴, Rj³, Rj⁶, Rj⁶¹, Rj⁷, Rj⁷¹, and Lj are as defined in any one of the embodiments, and the 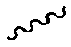 end is attached to L₀.

In one embodiment, 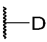 is a compound represented by any one of formula (AIA-1-1) to formula (AIA-1-7), formula (AIA-2-1) to formula (AIA-2-8), formula (AIA-3-1) to formula (AIA-3-5), and formula (AIA-4-1) to formula (AIA-4-6), or a stable deuterated derivative thereof, a stereoisomer thereof, or a pharmaceutically acceptable salt thereof: 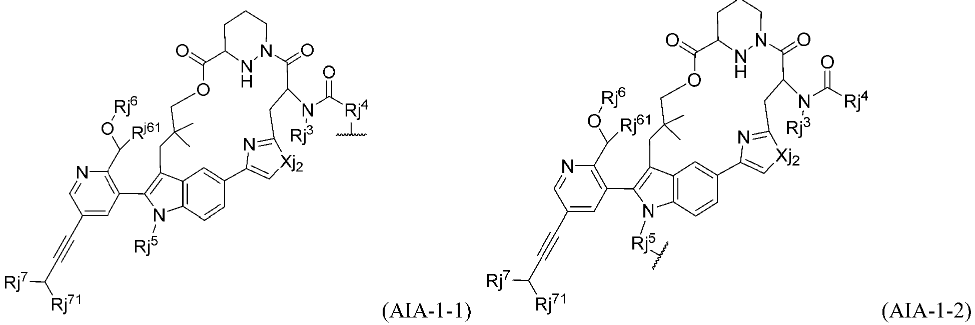 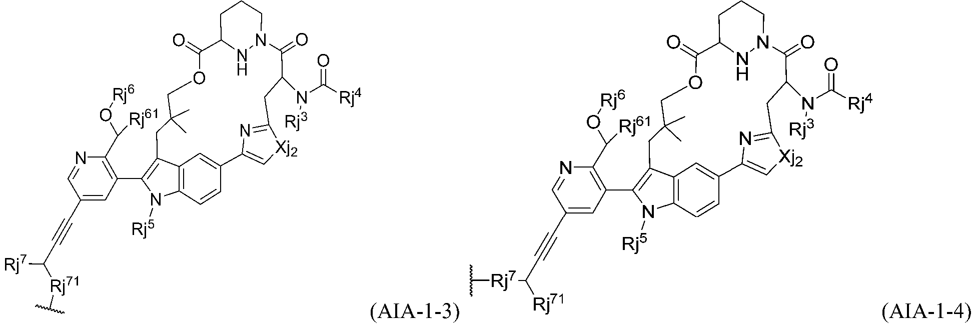 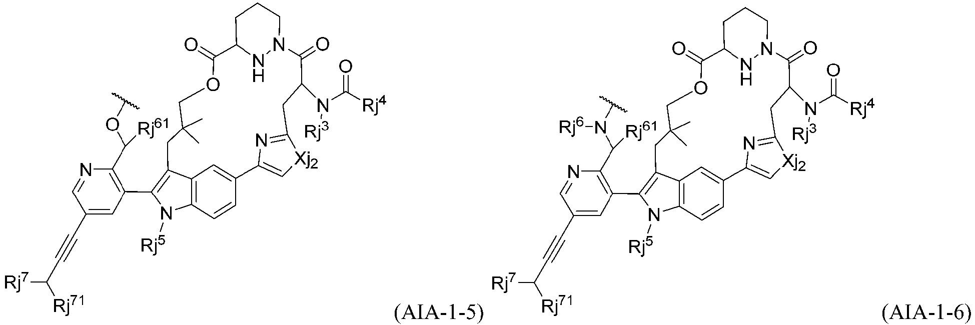 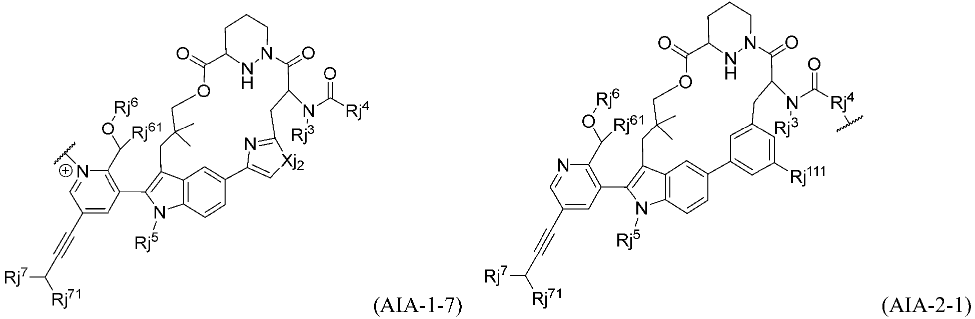 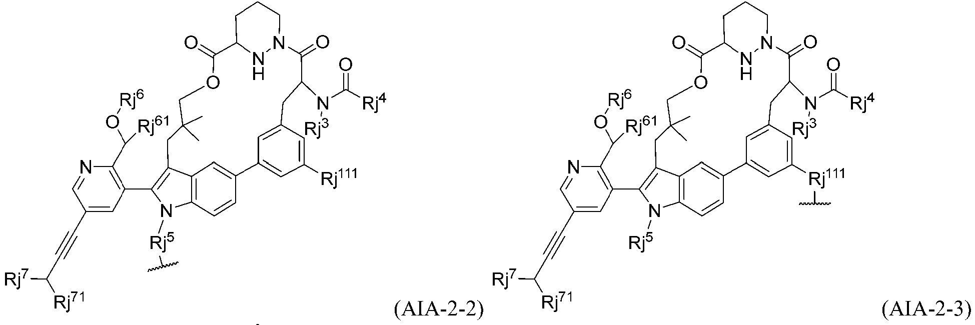 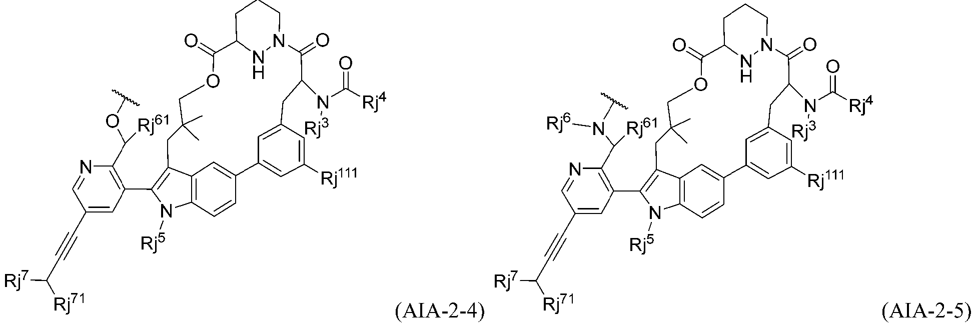 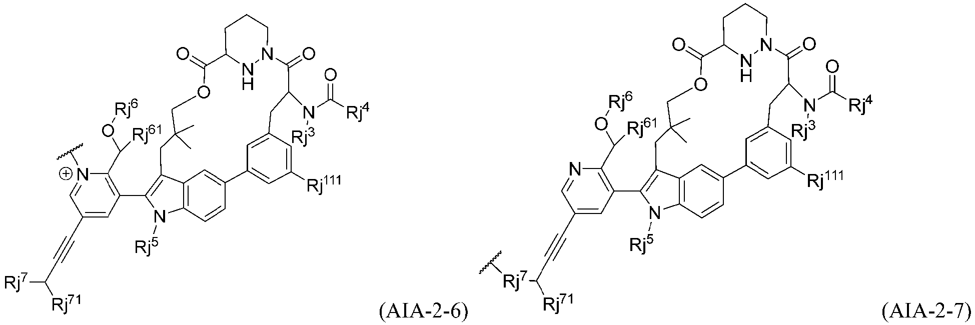 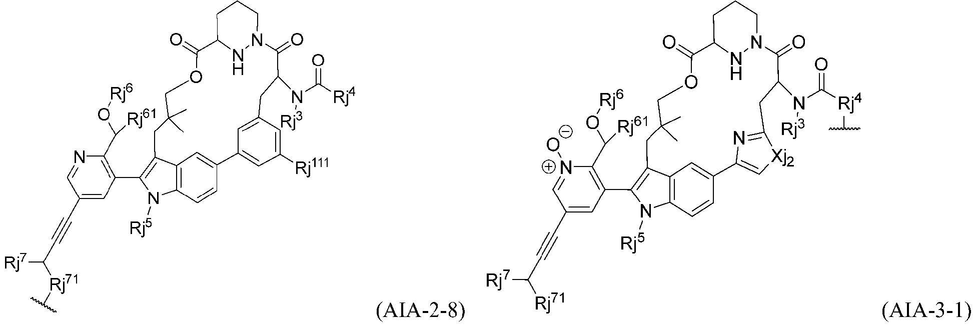 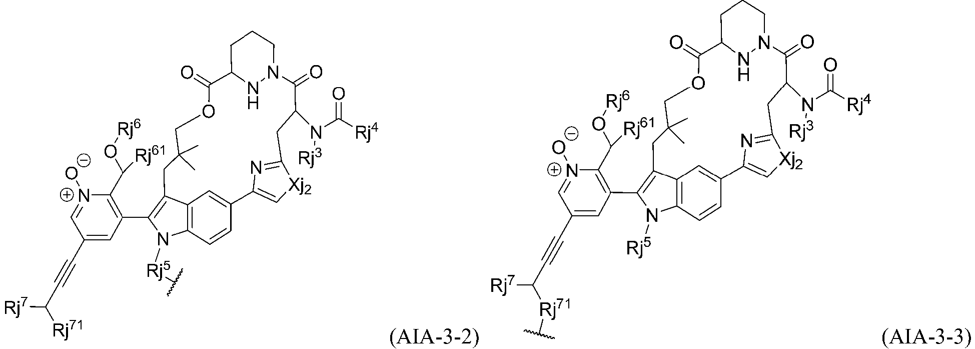 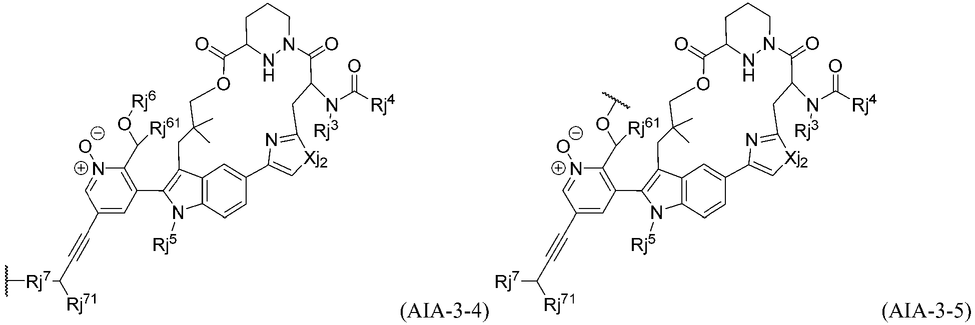 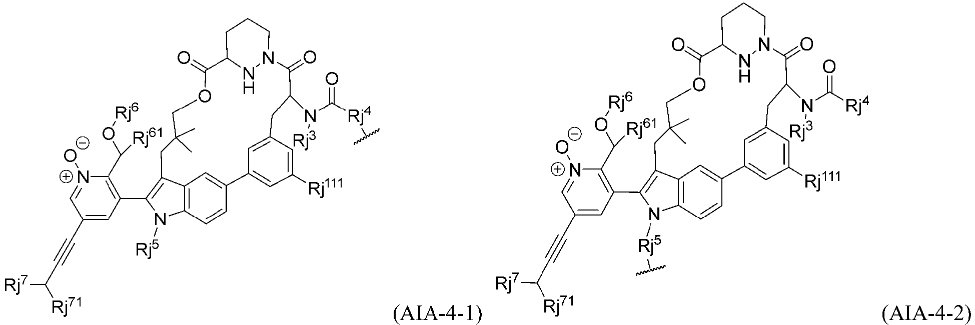 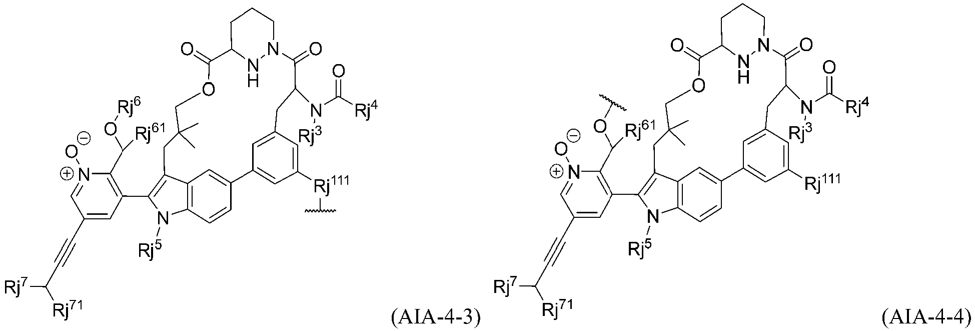 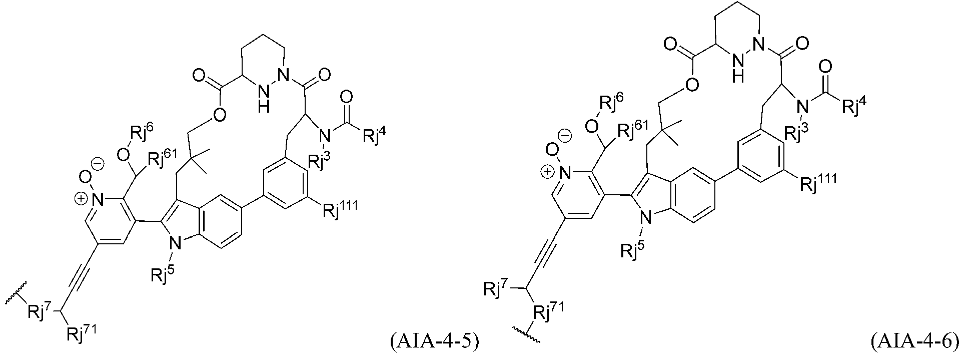
wherein in the formulas, Rj³, Rj⁴, Rj⁵, Rj⁶, Rj⁶¹, Rj⁷, and Rj⁷¹ are as defined in any one of the embodiments; Xj₂ is S or O; Rj¹¹¹ is amino, hydroxy, or hydrogen;
the 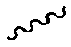 end is attached to L₀.

In one embodiment, 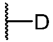 is a compound represented by any one of formula (AIA-1a-1) to formula (AIA-1a-4) and formula (AIA-1b-1) to formula (AIA-1b-4), or a stable deuterated derivative thereof, a stereoisomer thereof, or a pharmaceutically acceptable salt thereof: 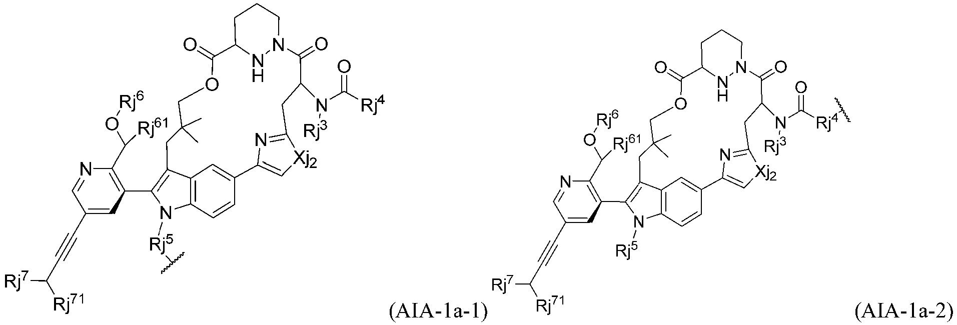 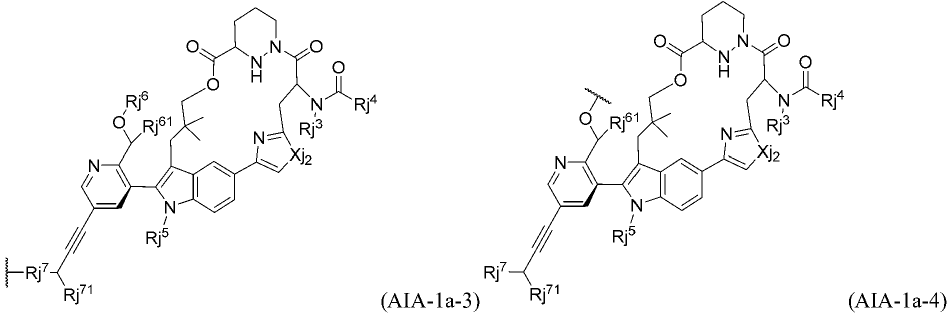 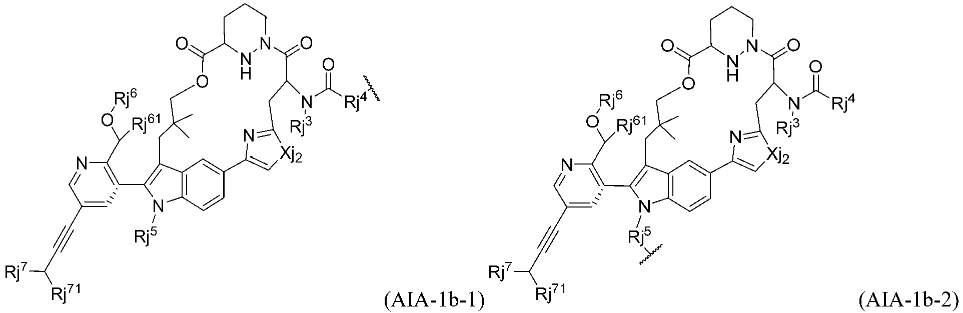 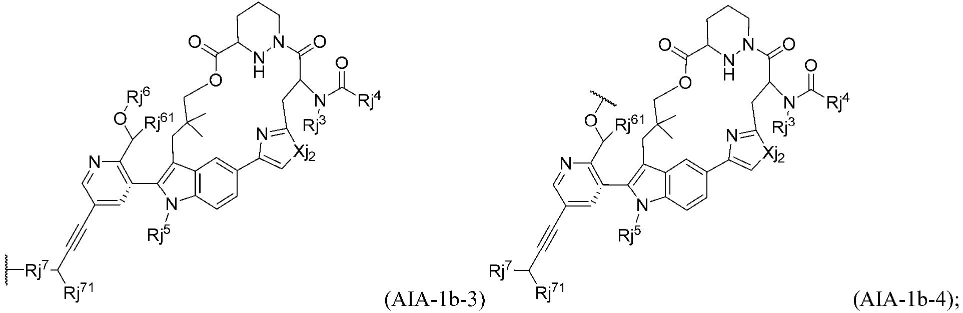 wherein in the formulas,
Rj³, Rj⁴, Rj⁵, Rj⁶, Rj⁶¹, Rj⁷, and Rj⁷¹ are as defined in any one of the embodiments;
Xj₂ is S or O;
the 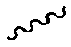 end is attached to L₀.

In one embodiment, 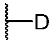 is a compound represented by formula (AIA-1a1-1) to formula (AIA-1a1-4) or formula (AIA-1b1-1) to formula (AIA-1b1-4), or a stable deuterated derivative thereof, a stereoisomer thereof, or a pharmaceutically acceptable salt thereof: 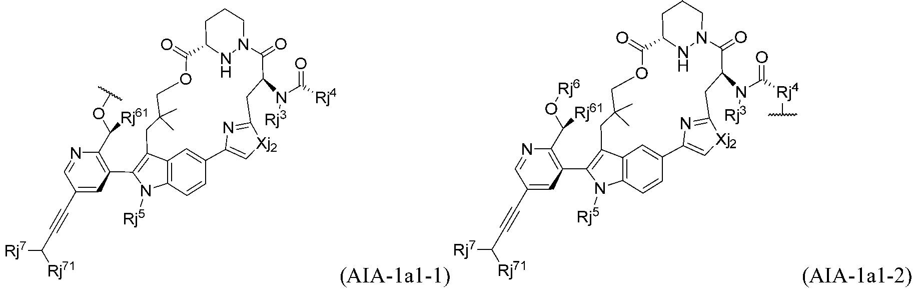 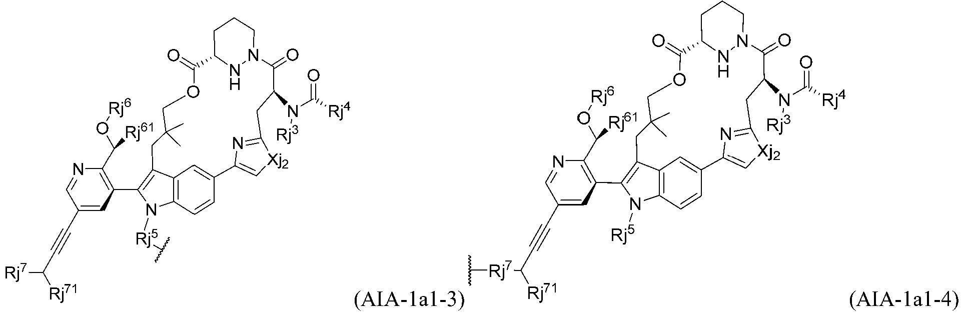 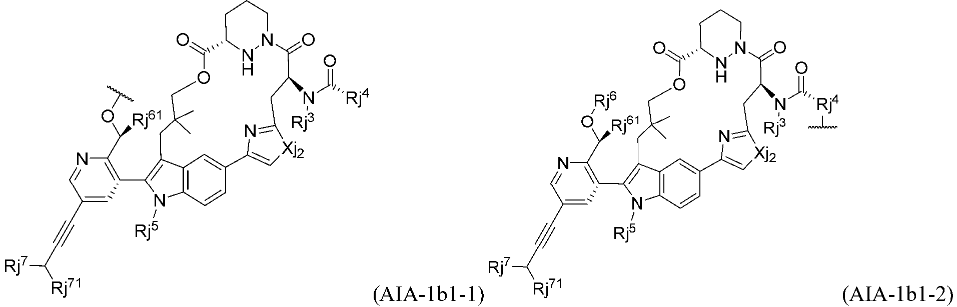 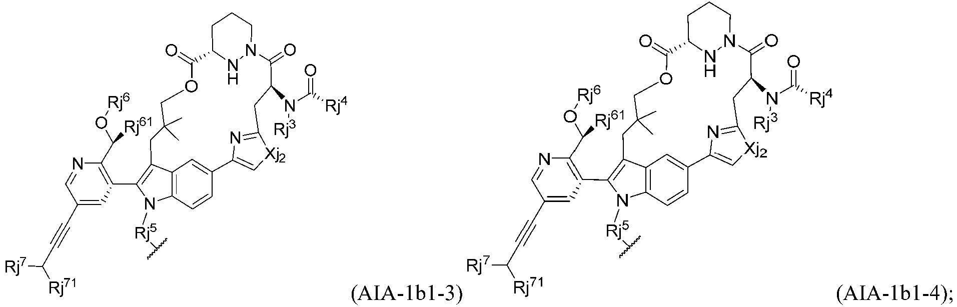 wherein in the formulas,
Rj³, Rj⁴, Rj⁵, Rj⁶, Rj⁶¹, Rj⁷, and Rj⁷¹ are as defined in any one of the embodiments;
Xj₂ is S or O;
the 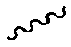 end is attached to L₀.

In one embodiment, 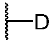 is a compound represented by any one of formula (AIA-2a-1) to formula (AIA-2a-4) and formula (AIA-2b-1) to formula (AIA-2b-4), or a stable deuterated derivative thereof, a stereoisomer thereof, or a pharmaceutically acceptable salt thereof: 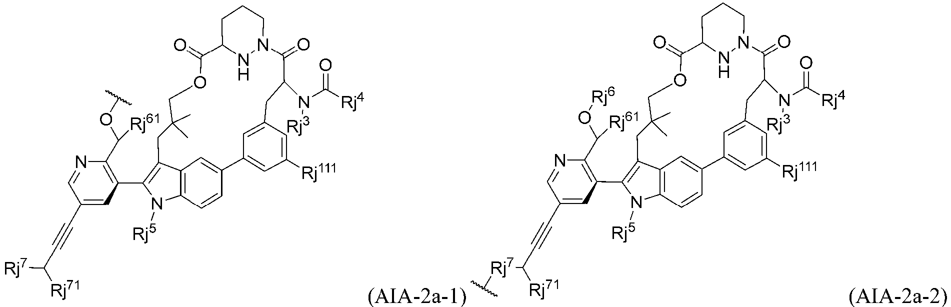 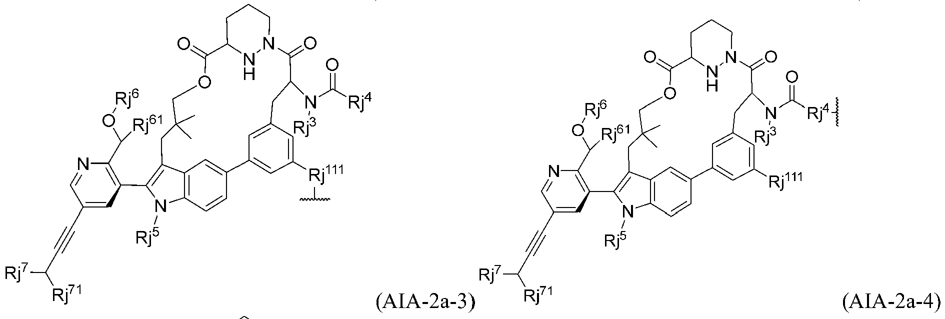 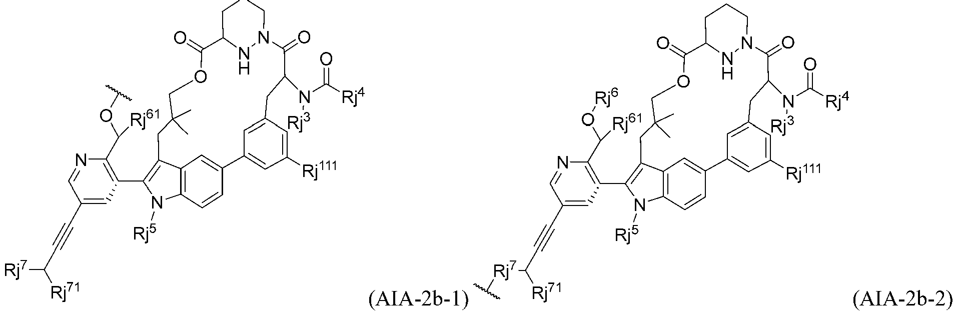 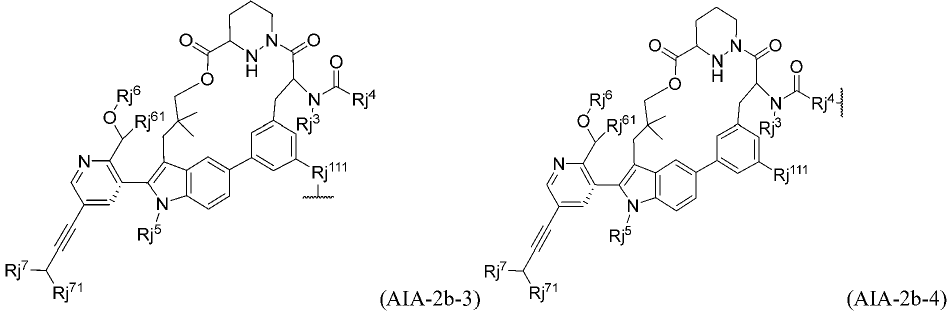 wherein in the formulas,
Rj³, Rj⁴, Rj⁵, Rj⁶, Rj⁶¹, Rj⁷, and Rj⁷¹ are as defined in any one of the embodiments;
Rj¹¹¹ is amino or hydroxy;
the 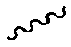 end is attached to L₀.

In one embodiment, 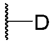 is a compound represented by any one of formula (AIA-2a1-1) to formula (AIA-2a1-5) and formula (AIA-2b1-1) to formula (AIA-2b1-3), or a stable deuterated derivative thereof, a stereoisomer thereof, or a pharmaceutically acceptable salt thereof: 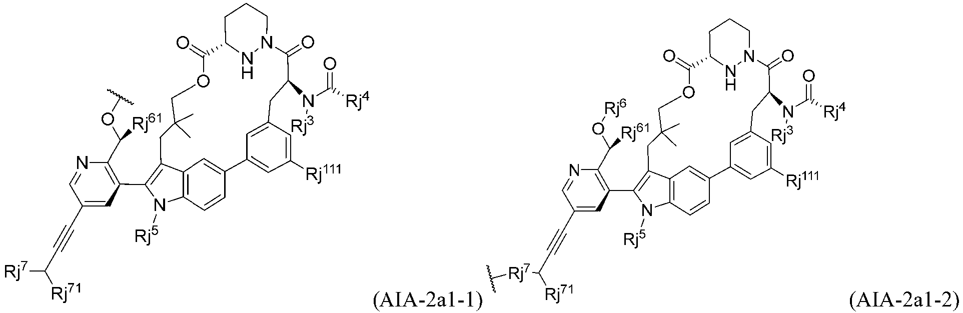 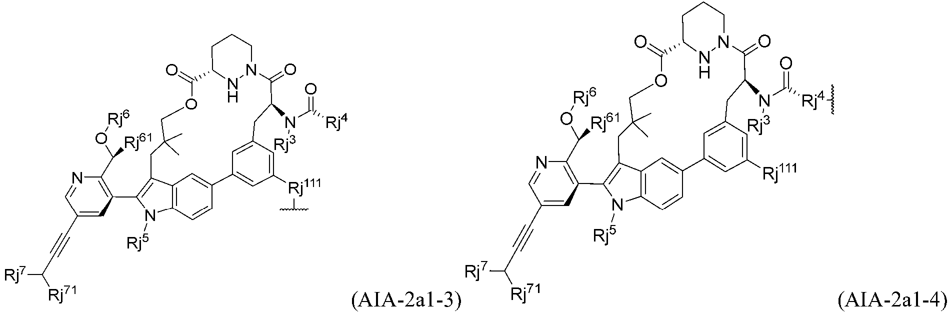 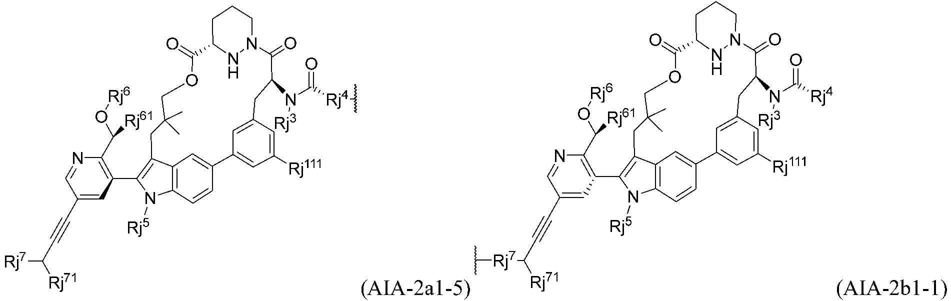 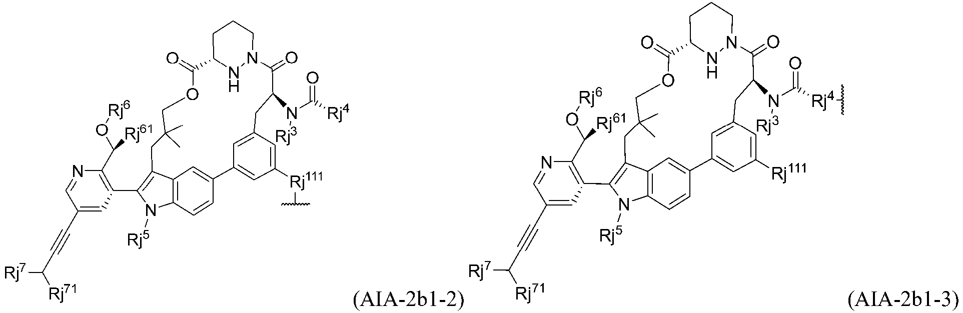 wherein in the formulas,
Rj³, Rj⁴, Rj⁵, Rj⁶, Rj⁶¹, Rj⁷, and Rj⁷¹ are as defined in any one of the embodiments;
Rj¹¹¹ is amino or hydroxy;
the 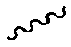 end is attached to L₀.

In one embodiment, 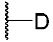 is a compound represented by formula (AIA-3a-1) to formula (AIA-3a-4) or formula (AIA-3b-1) to formula (AIA-3b-4), or a stable deuterated derivative thereof, a stereoisomer thereof, or a pharmaceutically acceptable salt thereof: 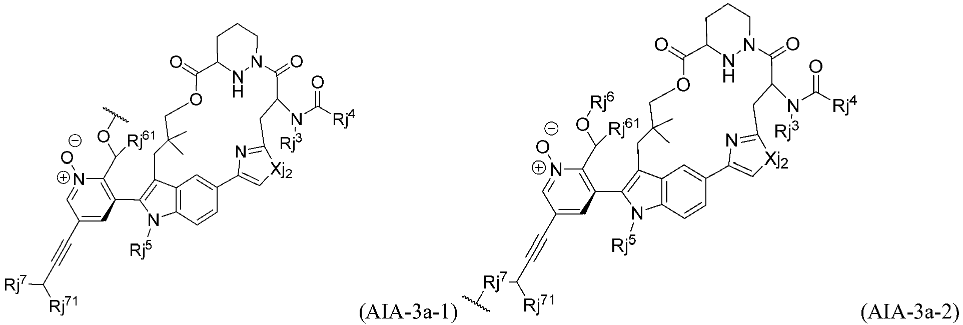 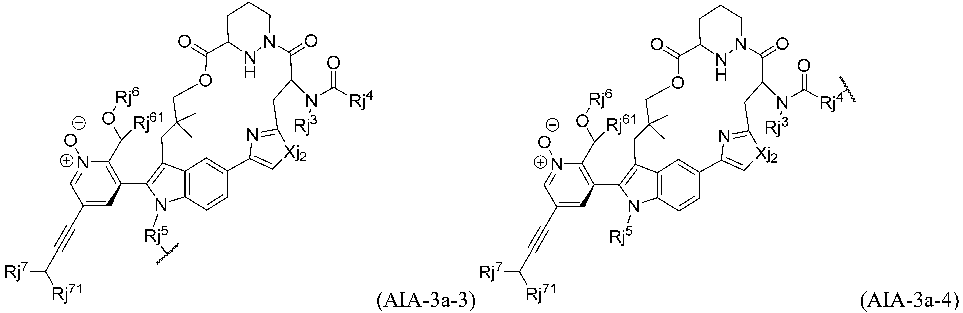 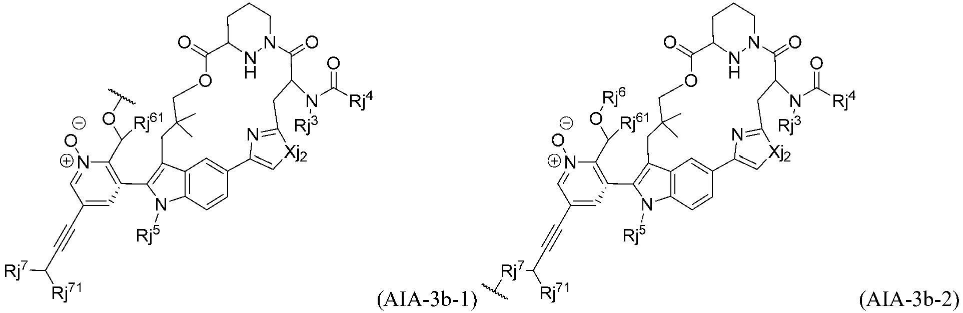 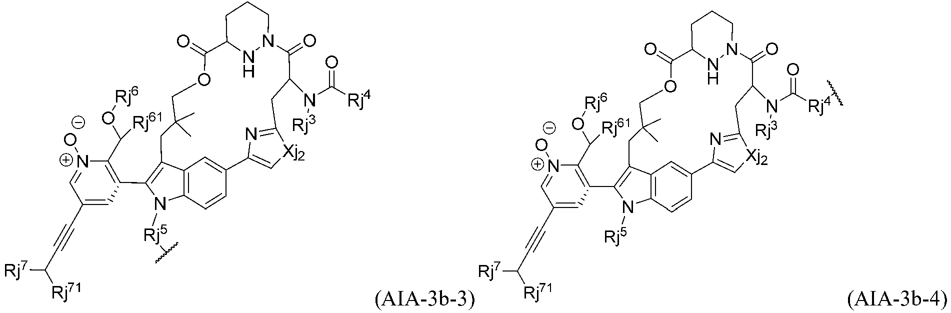 wherein in the formulas,
Rj³, Rj⁴, Rj⁵, Rj⁶, Rj⁶¹, Rj⁷, and Rj⁷¹ are as defined in any one of the embodiments;
Xj₂ is S or O;
the 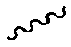 end is attached to Lo.

In one embodiment, 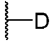 is a compound represented by formula (AIA-3a1-1) to formula (AIA-3a1-4) or formula (AIA-3b1-1) to formula (AIA-3b1-4), or a stable deuterated derivative thereof, a stereoisomer thereof, or a pharmaceutically acceptable salt thereof: 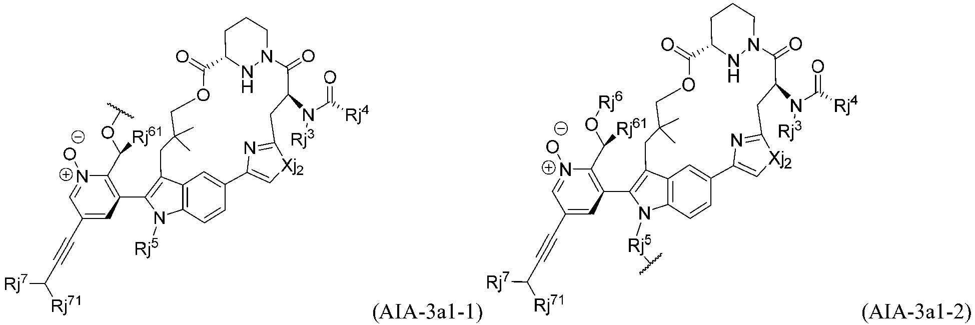 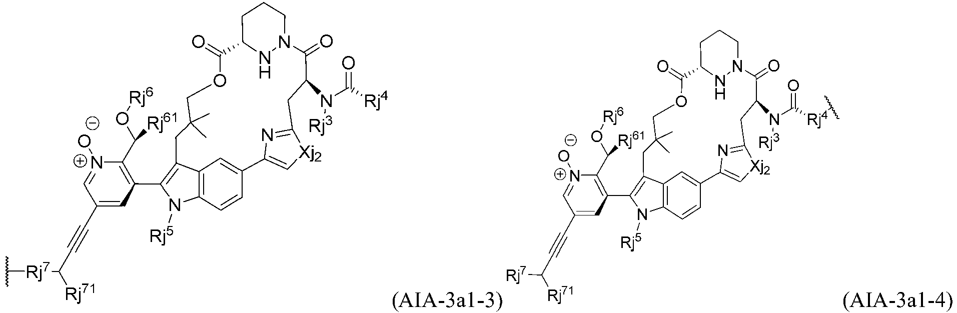 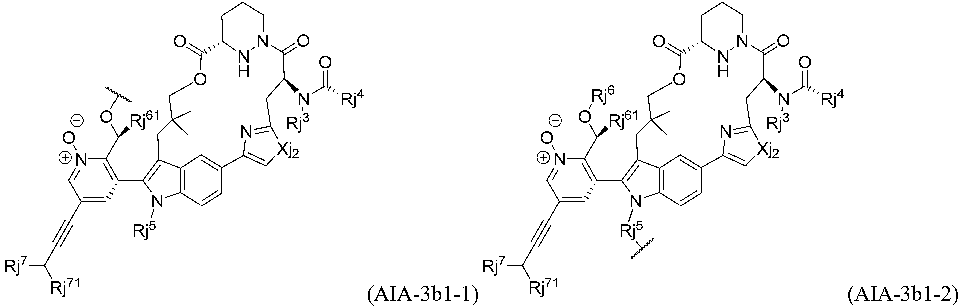 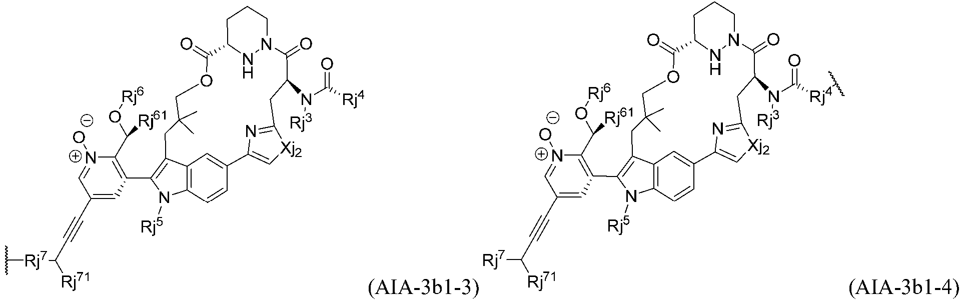 wherein in the formulas,
Rj³, Rj⁴, Rj⁵, Rj⁶, Rj⁶¹, Rj⁷, and Rj⁷¹ are as defined in any one of the embodiments;
Xj₂ is S or O;
the 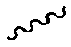 end is attached to L₀.

In one embodiment, 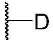 is a compound represented by formula (AIA-4a-1) to formula (AIA-4a-4) or formula (AIA-4b-1) to formula (AIA-4b-4), or a stable deuterated derivative thereof, a stereoisomer thereof, or a pharmaceutically acceptable salt thereof: 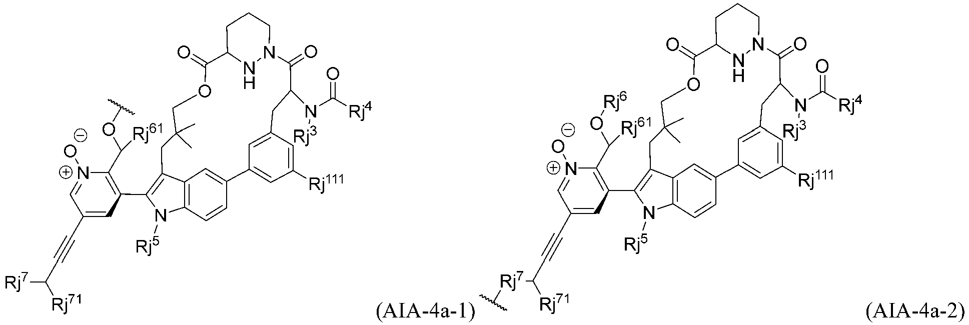 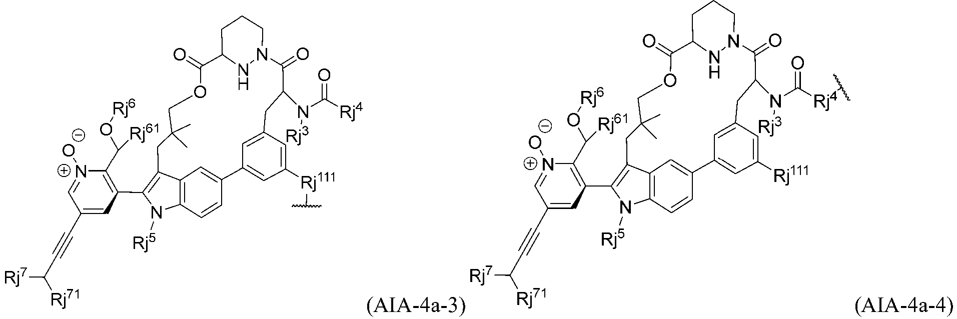 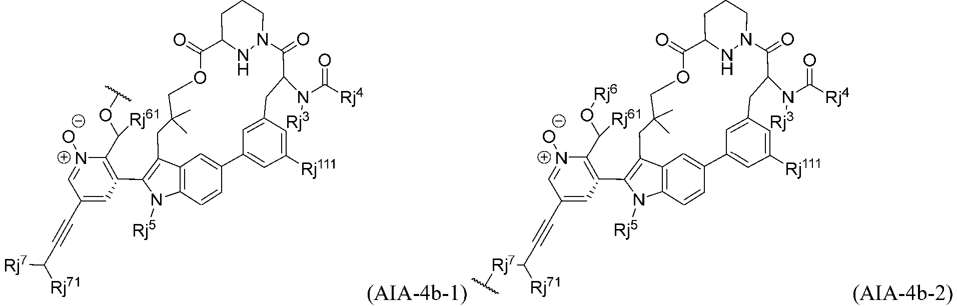 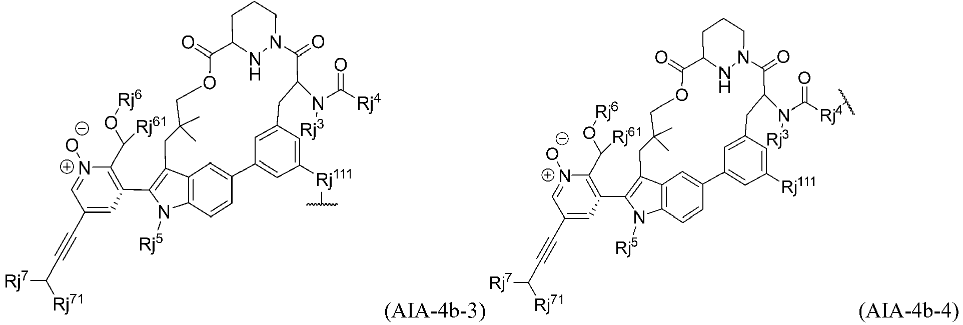 wherein in the formulas,
Rj³, Rj⁴, Rj⁵, Rj⁶, Rj⁶¹, Rj⁷, and Rj⁷¹ are as defined in any one of the embodiments;
Rj¹¹¹ is amino or hydroxy;
the 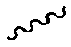 end is attached to L₀.

In one embodiment, 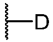 is a compound represented by formula (AIA-4a1-1) to formula (AIA-4a1-4) or formula (AIA-4b1-1) to formula (AIA-4b1-4), or a stable deuterated derivative thereof, a stereoisomer thereof, or a pharmaceutically acceptable salt thereof: 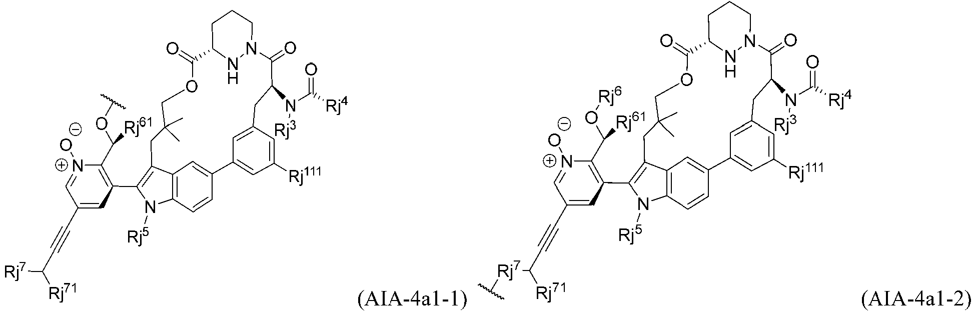 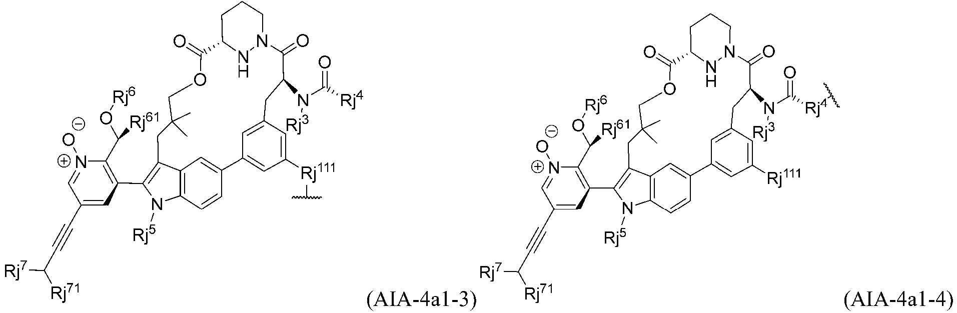 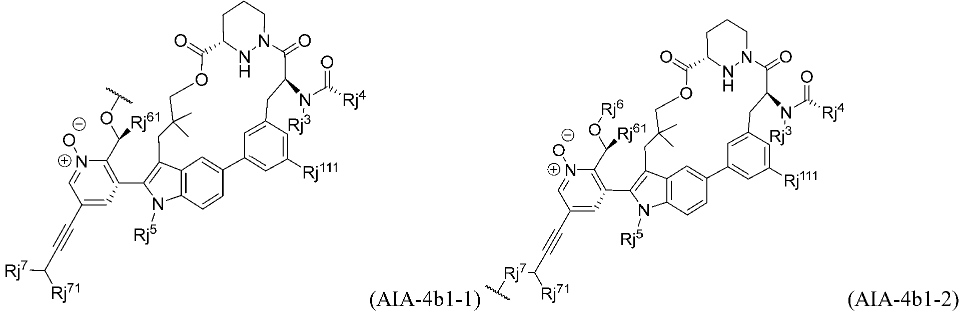 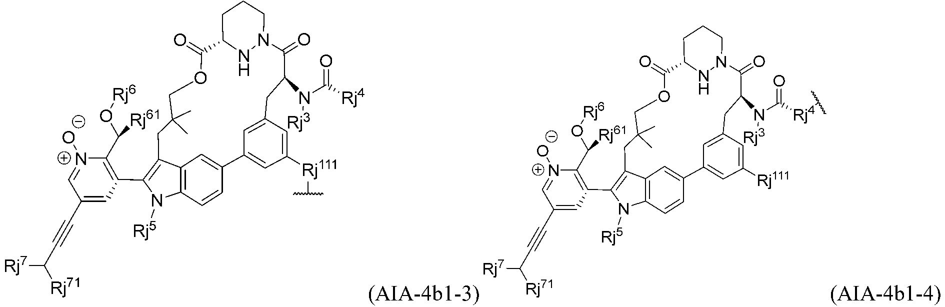 wherein in the formulas,
Rj³, Rj⁴, Rj⁵, Rj⁶, Rj⁶¹, Rj⁷, and Rj⁷¹ are as defined in any one of the embodiments;
Rj¹¹¹ is amino or hydroxy;
the 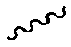 end is attached to L₀.

In one embodiment, 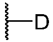 is a compound represented by any one of formula (AI-1) to formula (AI-6), or a stable deuterated derivative thereof, a stereoisomer thereof, or a pharmaceutically acceptable salt thereof: 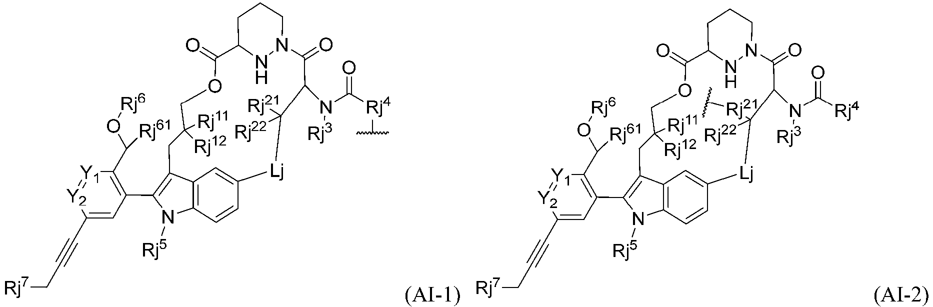 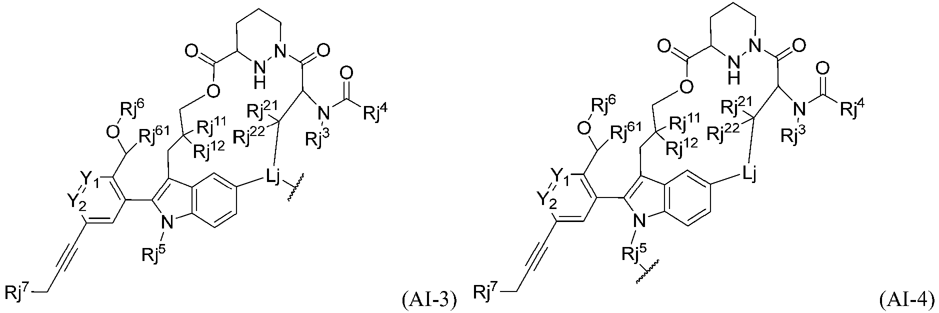 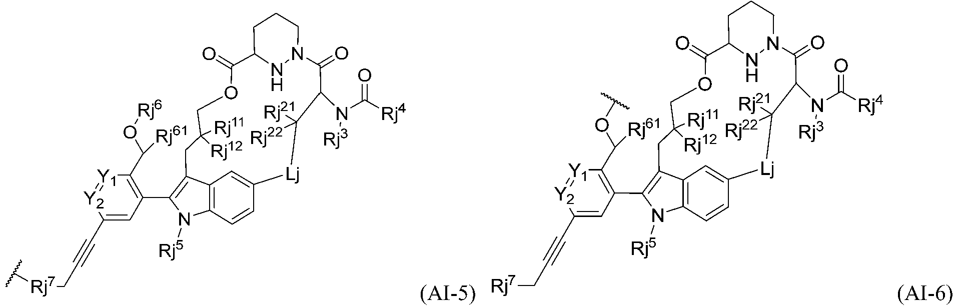 wherein in the formulas, Rj¹¹, Rj¹², Rj²¹, Rj²², Rj³, Rj⁴, Rj⁵, Rj⁶, Rj⁶¹, Rj⁷, Y₁, Y₂, and Lj are as defined in any one of the embodiments, and the 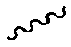 end is attached to L₀.

In one embodiment, 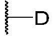 is a compound represented by any one of formula (AI-1-1) to formula (AI-1-4), formula (AI-2-1) to formula (AI-2-4), formula (AI-3-1) to formula (AI-3-4), and formula (AI-4-1) to formula (AI-4-4), or a stable deuterated derivative thereof, a stereoisomer thereof, or a pharmaceutically acceptable salt thereof: 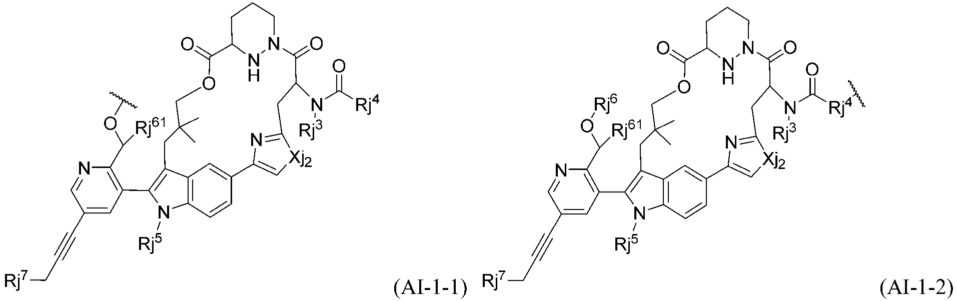 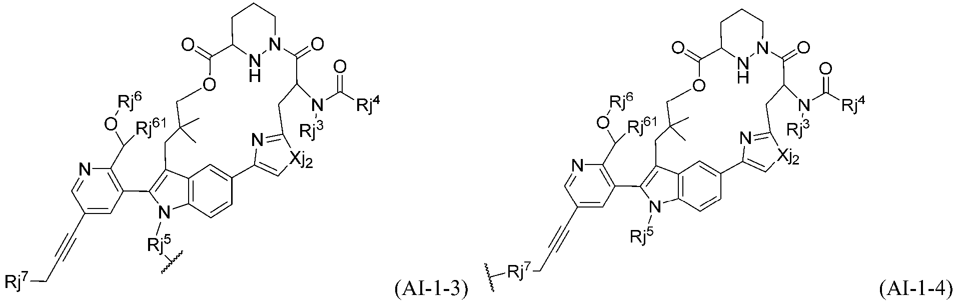 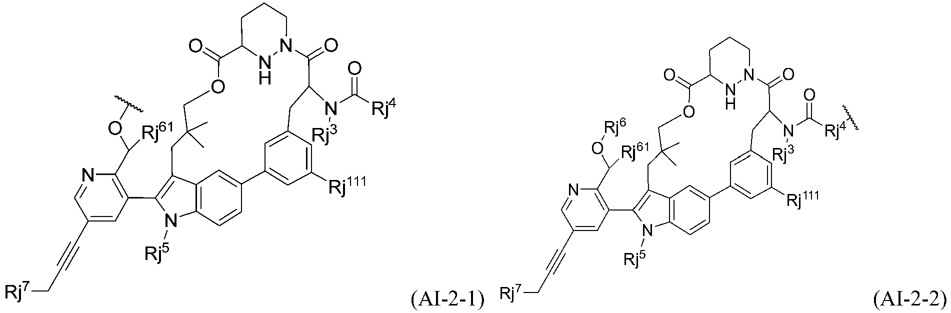 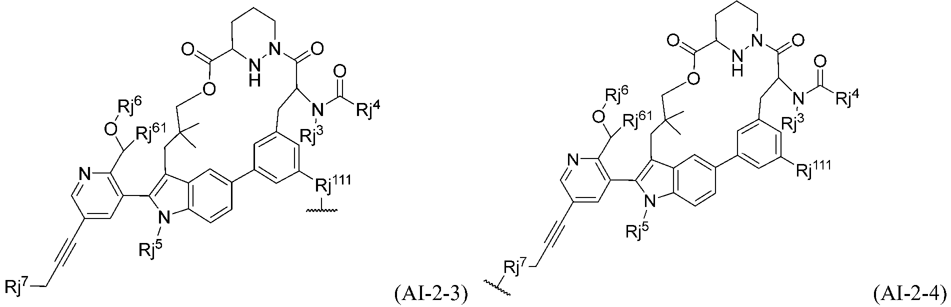 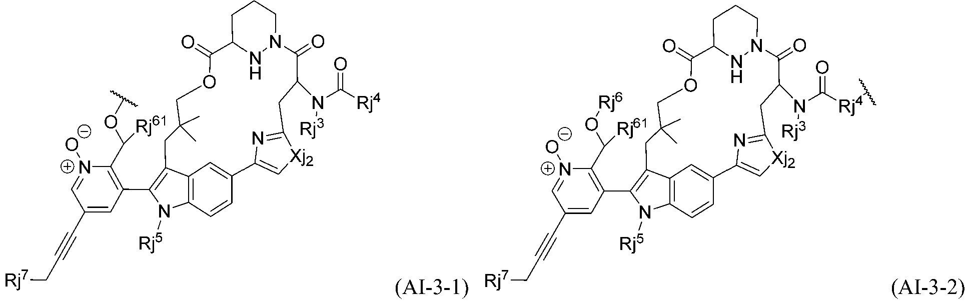 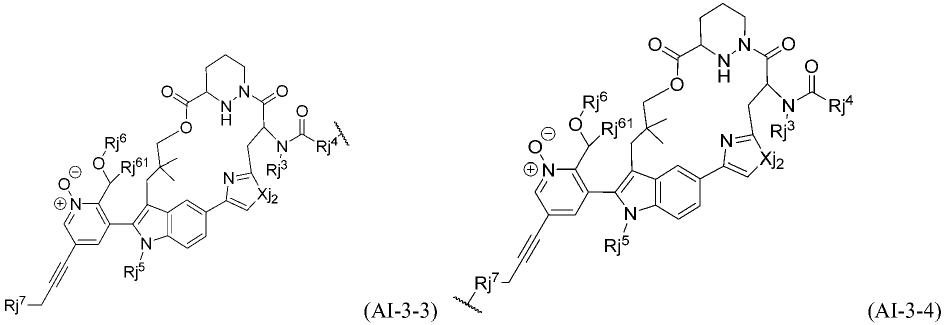 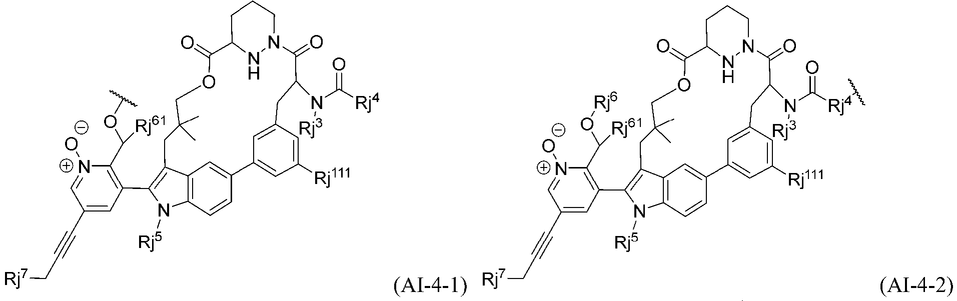 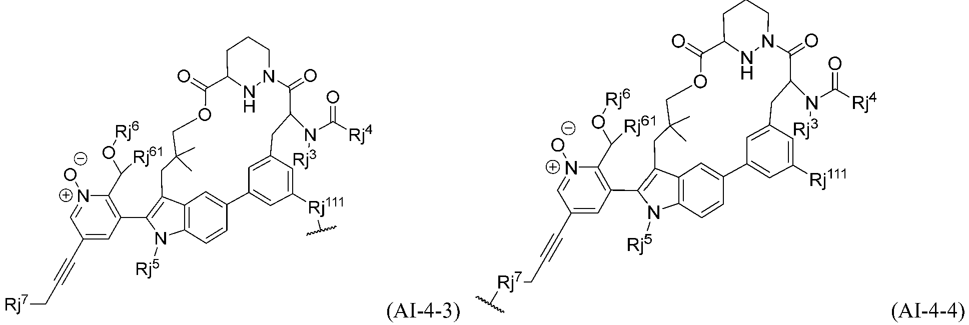
wherein in the formulas, Rj³, Rj⁴, Rj⁵, Rj⁶, Rj⁶¹, and Rj⁷ are as defined in any one of the embodiments;
Xj₂ is S or O; Rj¹¹¹ is amino or hydroxy;
the 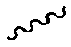 end is attached to L₀.

In one embodiment, 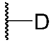 is a compound represented by any one of formula (AIIA-1-1) to formula (AIIA-1-4), or a stable deuterated derivative thereof, a stereoisomer thereof, or a pharmaceutically acceptable salt thereof: 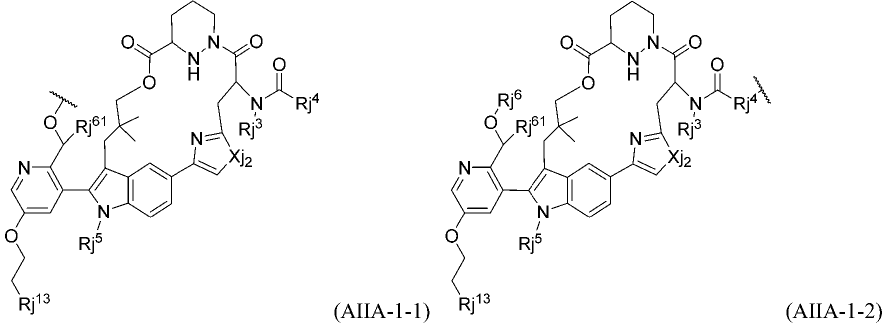 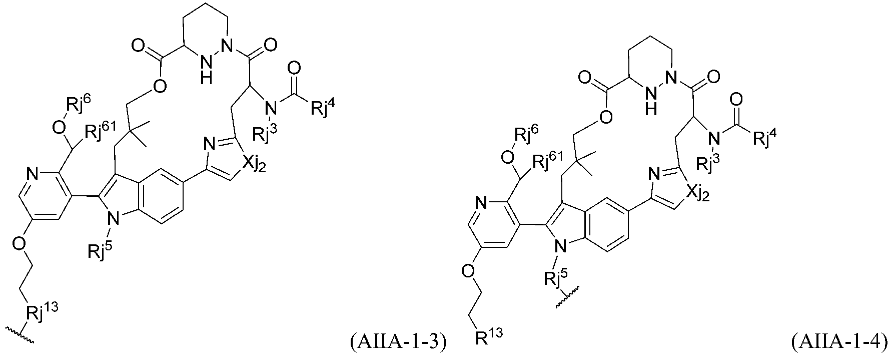
wherein in the formulas, Rj³, Rj⁴, Rj⁵, Rj⁶, Rj⁶¹, and Rj¹³ are as defined in any one of the embodiments;
Xj₂ is S or O;
the 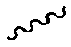 end is attached to L₀.

In one embodiment, 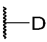 is a compound represented by any one of formula (AIIA-1a-1) to formula (AIIA-1a-4) and formula (AIIA-1b-1) to formula (AIIA-1b-4), or a stable deuterated derivative thereof, a stereoisomer thereof, or a pharmaceutically acceptable salt thereof: 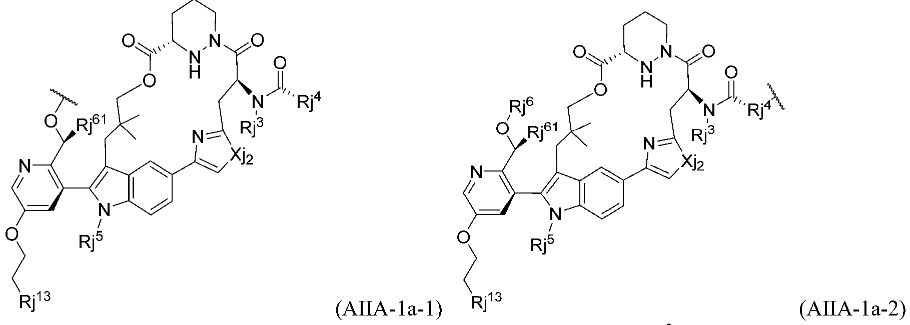 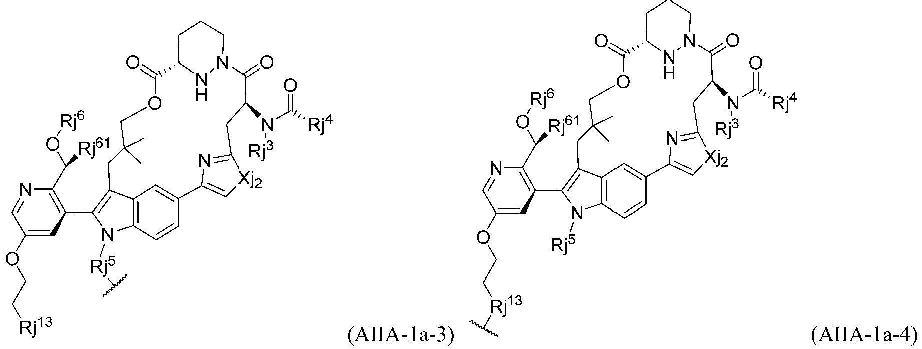 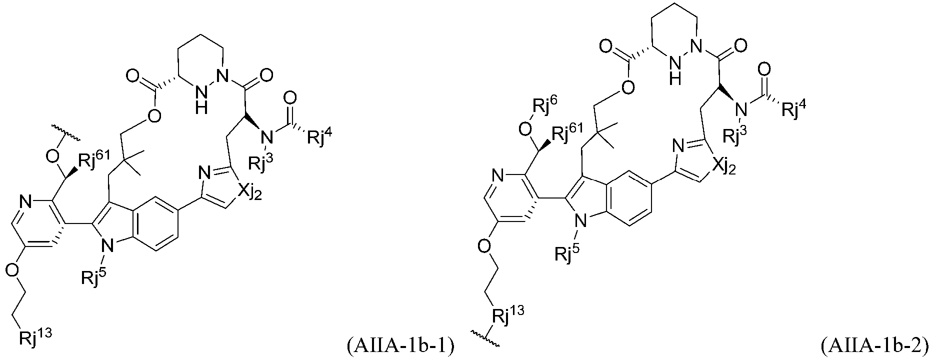 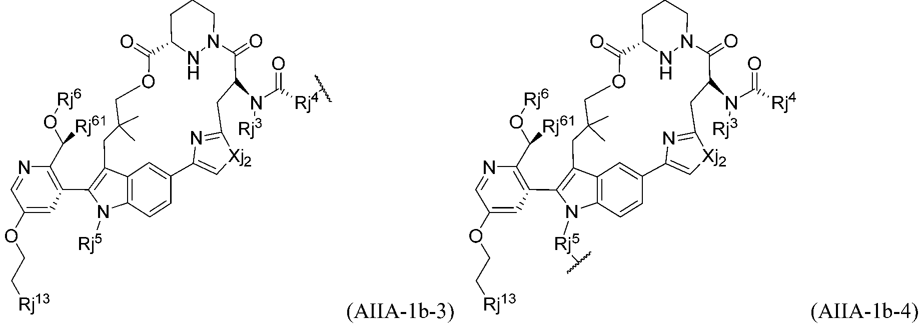
wherein in the formulas, Rj³, Rj⁴, Rj⁵, Rj⁶, Rj⁶¹, and Rj¹³ are as defined in any one of the embodiments; Xj₂ is S or O;
the 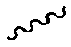 end is attached to L₀.

In one embodiment, 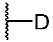 is selected from structures shown in Table 8,

**Table 8**

| Number | Structure | Number | Structure |
|---|---|---|---|
| D6 | 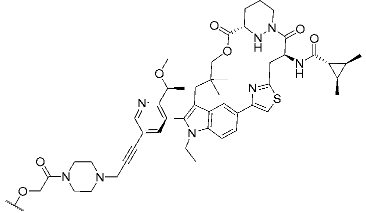 | D31 | 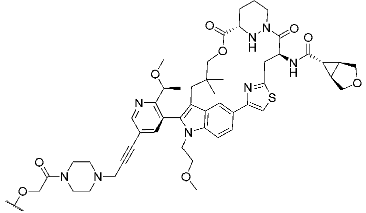 |
| D38-1 | 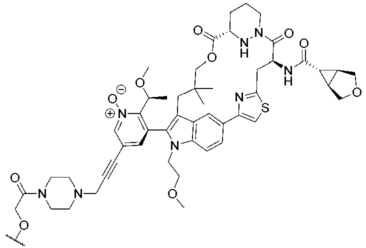 | D35-A | 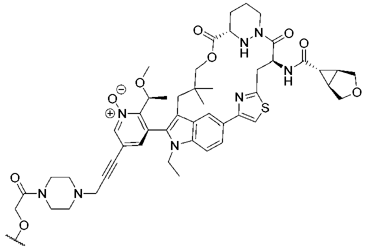 |
| D63 | 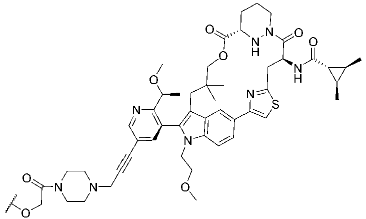 | D47 | 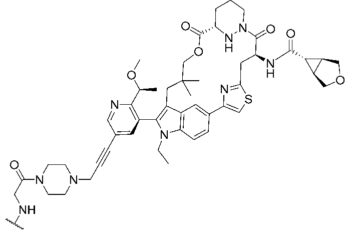 |
| D48 | 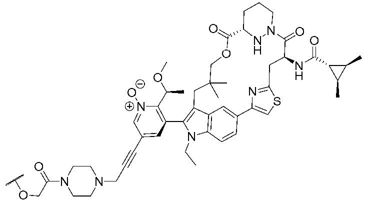 | D34 | 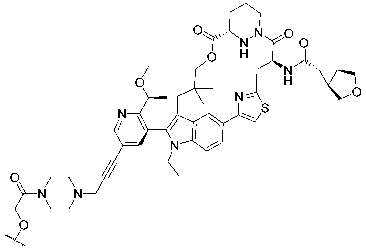 |
| D54 | 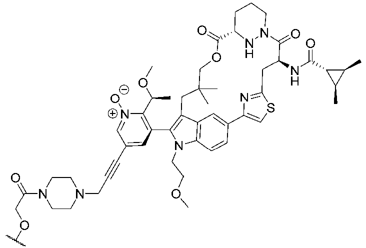 | D10 | 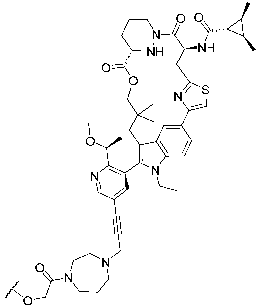 |
| D52 | 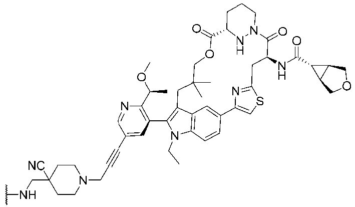 | D166 | 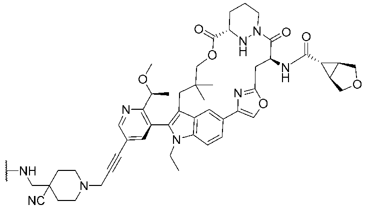 |
| D57-A | 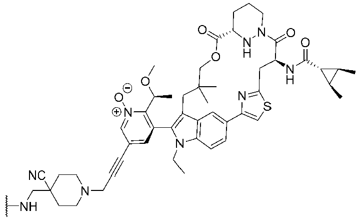 | D45-1 | 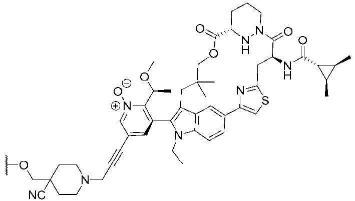 |
| D64-1 | 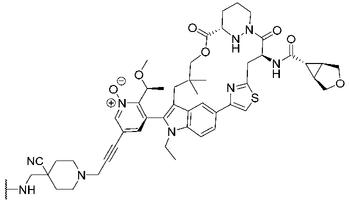 | D30-A | 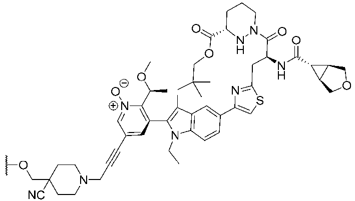 |
| D231 | 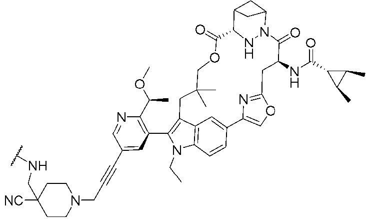 | D23 | 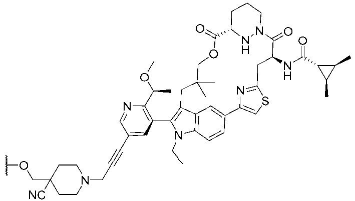 |
| D182 | 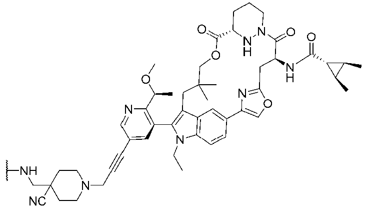 | D243 | 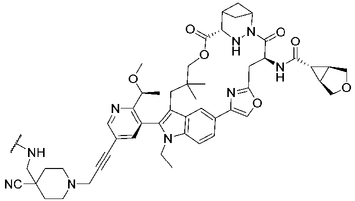 |
| D170 | 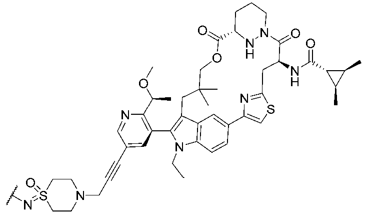 | D27 | 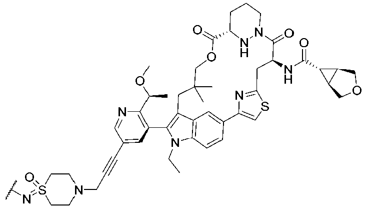 |
| D11 | 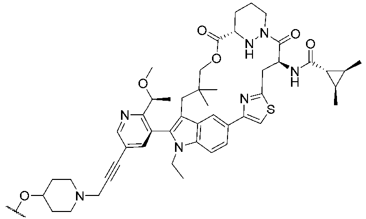 | D289 | 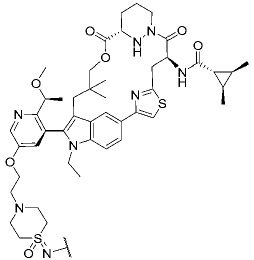 |
| D56 | 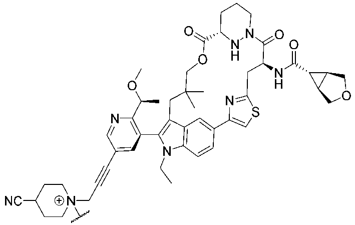 | D61 | 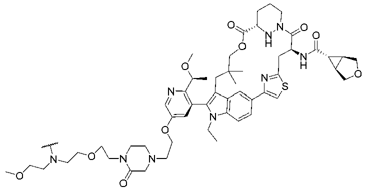 |
| D155 | 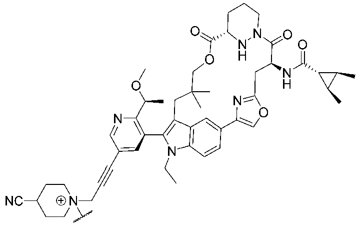 | D168 | 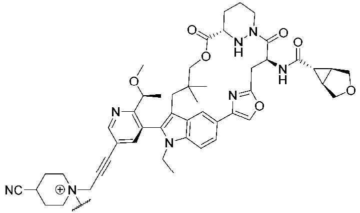 |
| D245 | 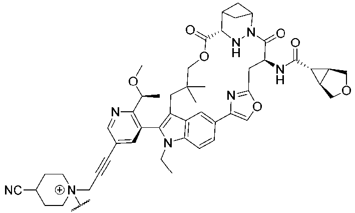 | D83 | 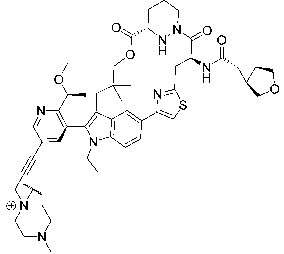 |
| D312 | 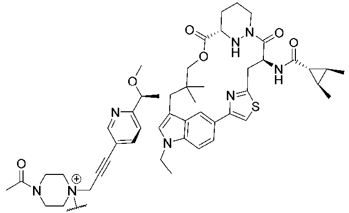 | D233 | 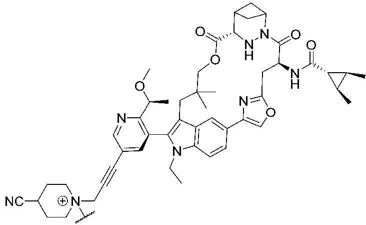 |
| D192 | 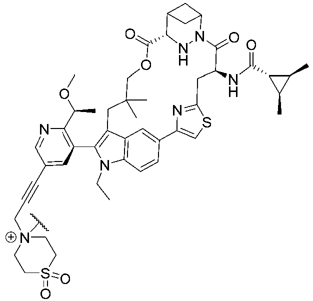 | D313 | 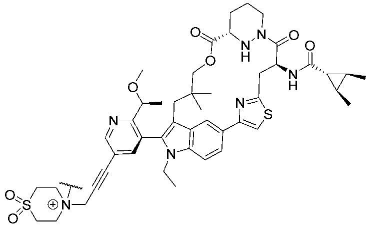 |
| D315 | 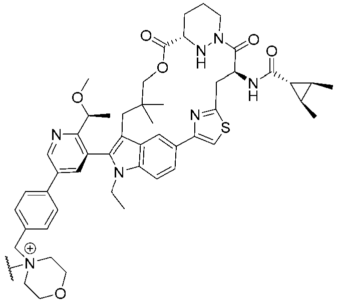 | D314 | 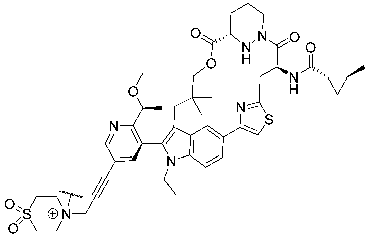 |
| D83-A | 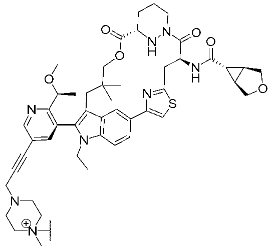 | D316 | 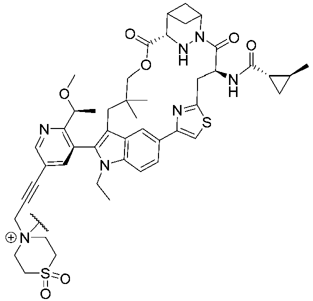 |
| D190 | 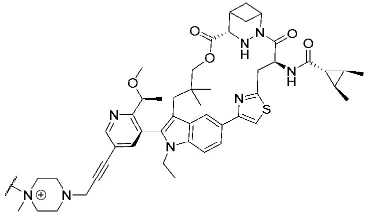 | D312-A | 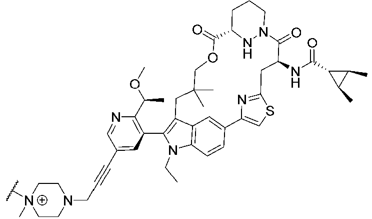 |
| D300 | 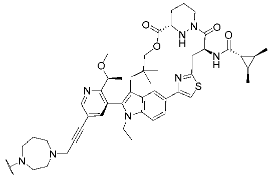 | D317 | 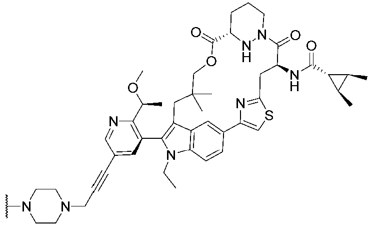 |
| D318 | 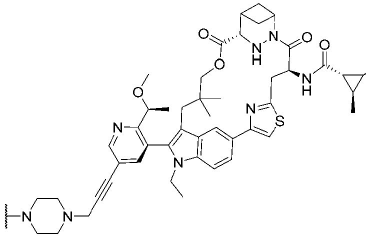 | D301 | 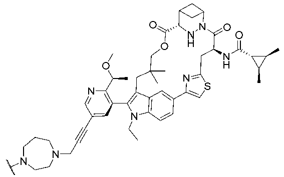 |
| D299-A | 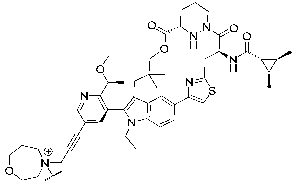 | D319 | 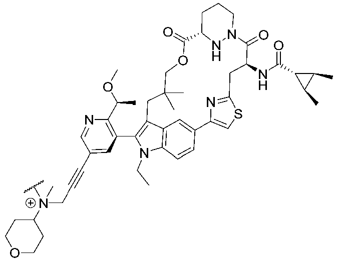 |
| D320 | 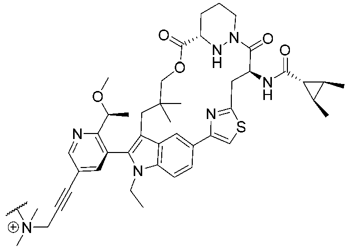 | D187 | 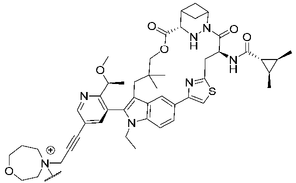 |
| D193 | 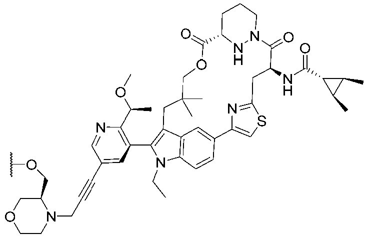 | D321 | 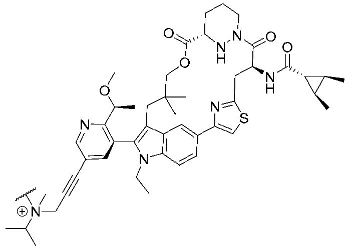 |
| D335 | 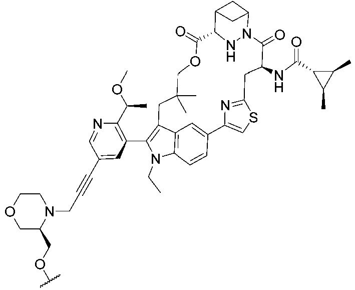 | D194 | 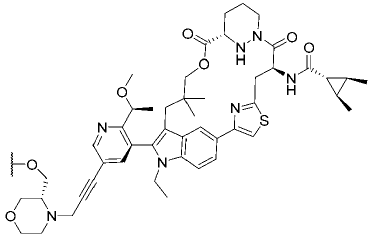 |
| D322 | 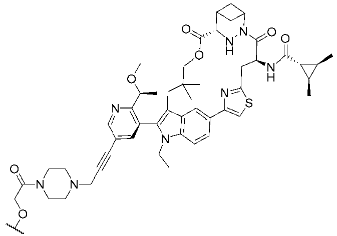 | D336 | 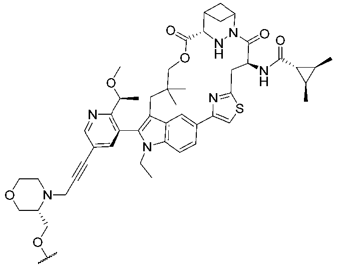 |
| D323 | 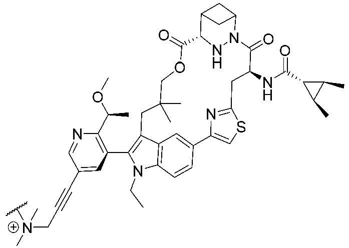 | D191 | 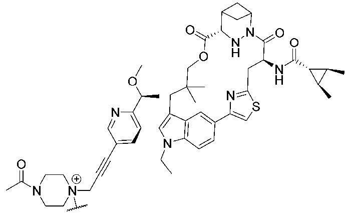 |
| D302 | 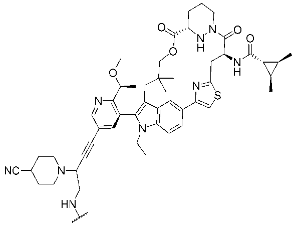 | D324 | 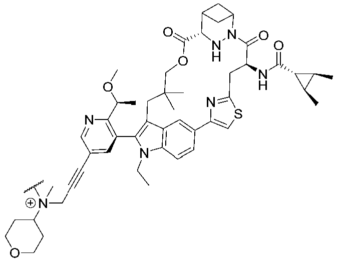 |
| D325 | 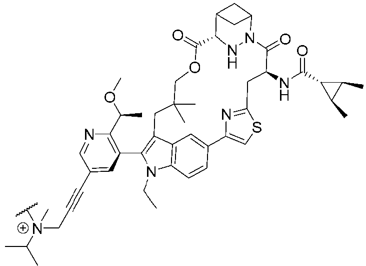 | D303 | 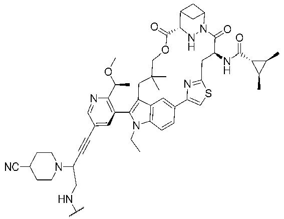 |
| D288-A | 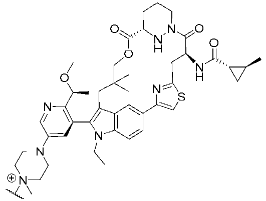 | D290 | 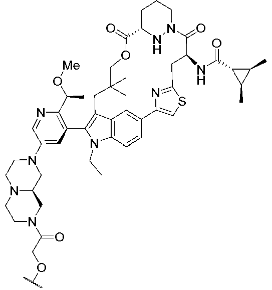 |
| D326 | 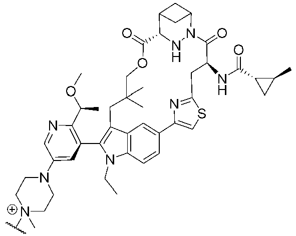 | D286-1a | 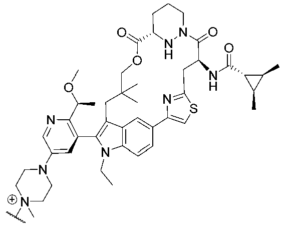 |
| D288 | 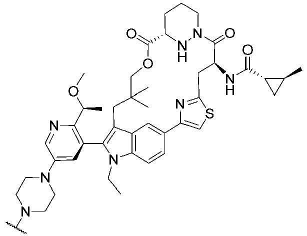 | D285-1b | 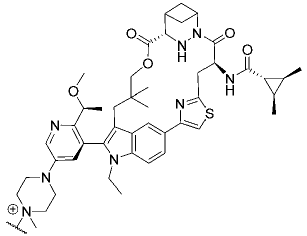 |
| D327 | 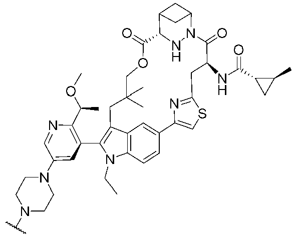 | D286-B | 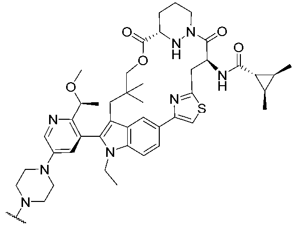 |
| D328 | 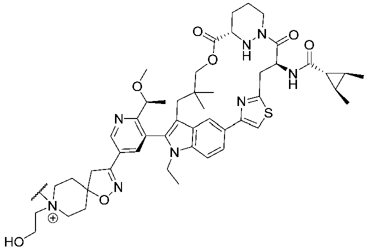 | D285-B | 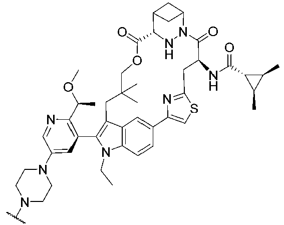 |
| D330 | 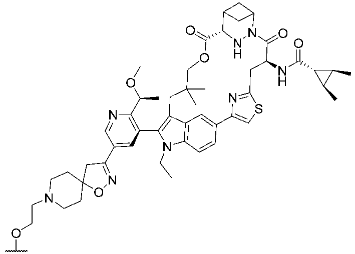 | D329 | 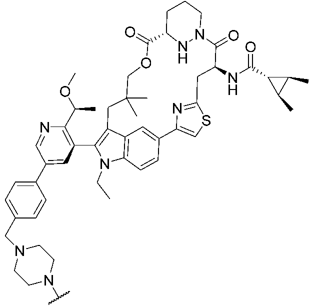 |
| D56-A | 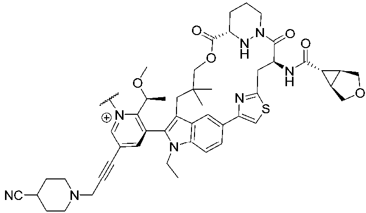 | D331 | 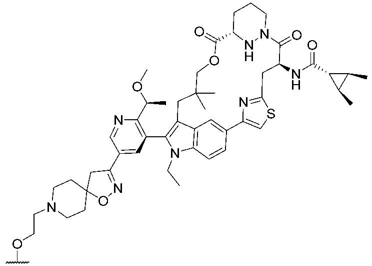 |
| D169-1 | 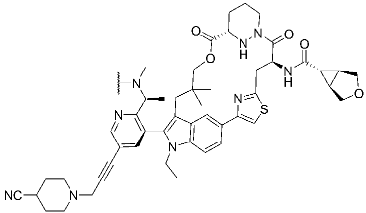 | D168-A | 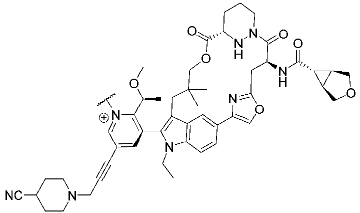 |
| D309 | 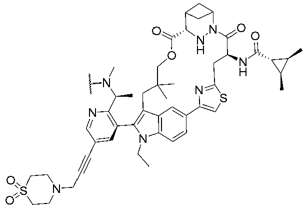 | D67-A | 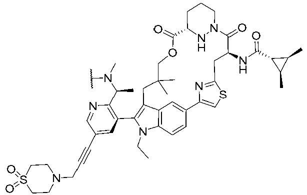 |
| D292 | 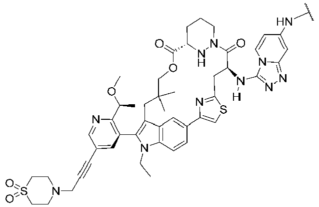 | D192-C | 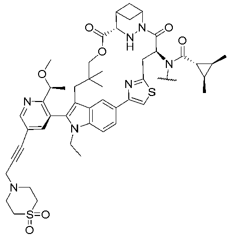 |
| D285 | 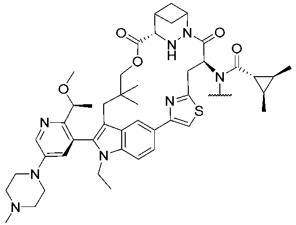 | D284 | 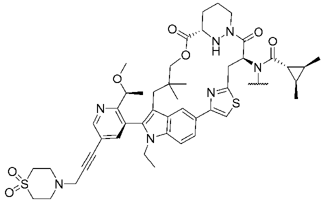 |
| D332 | 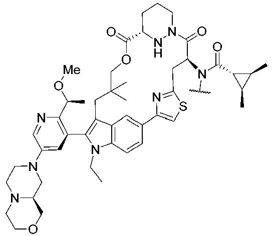 | D283 | 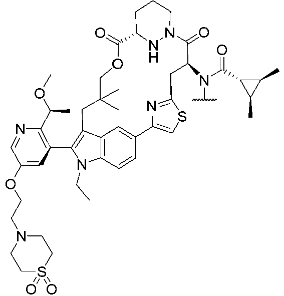 |
| D4 | 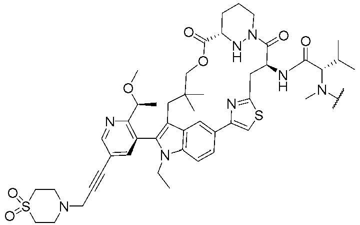 | D17 | 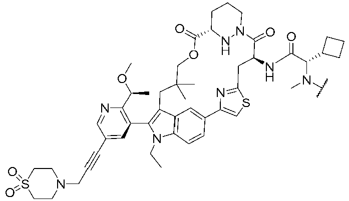 |
| D7-2 | 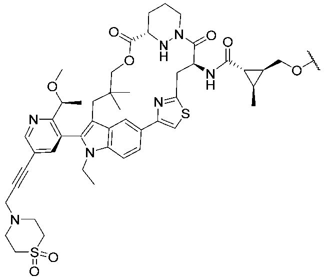 | D5 | 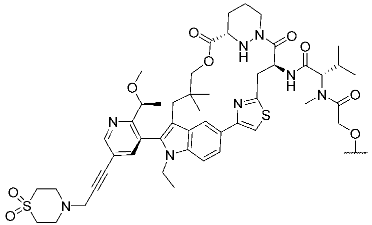 |
| D192-A | 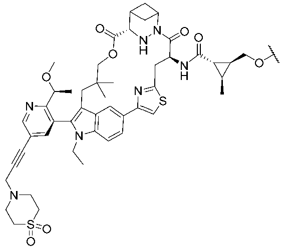 | D291 | 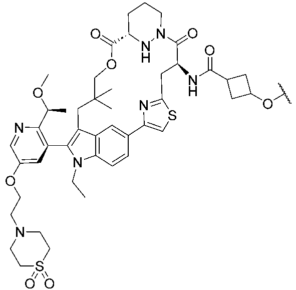 |
| D65 | 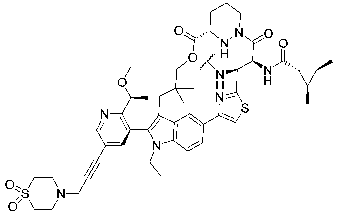 | D310 | 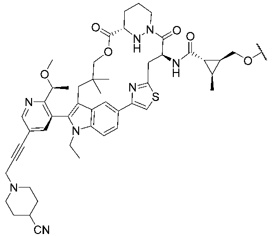 |
| D192-B | 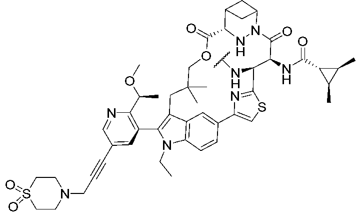 | D88 | 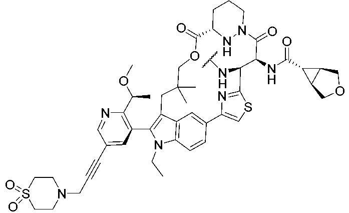 |
| D297 | 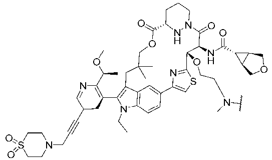 | D204-A | 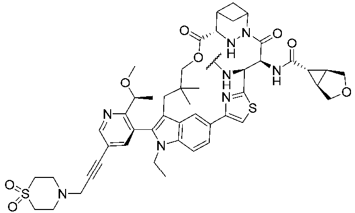 |
| D307 | 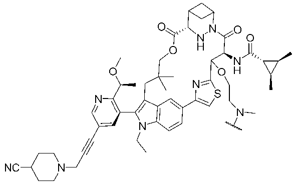 | D306 | 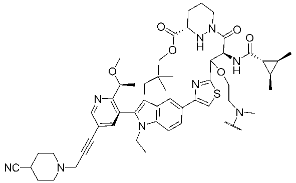 |
| D308 | 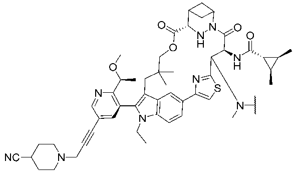 | D282-A | 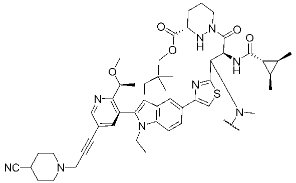 |
| D85 | 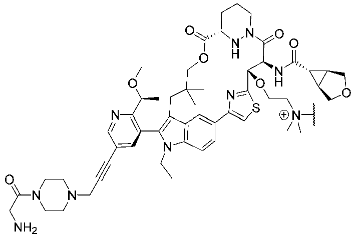 | D85-A | 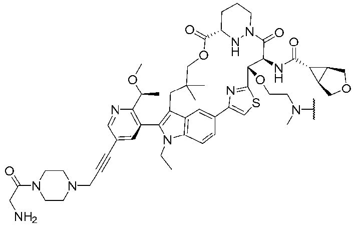 |
| D333 | 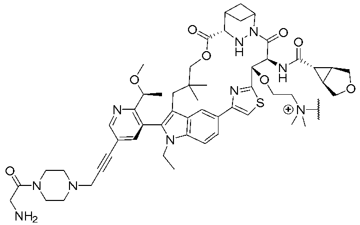 | D333 | 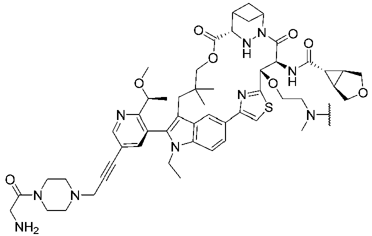 |
| D297-A | 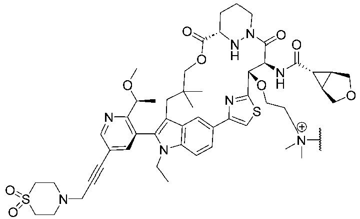 | D286-C | 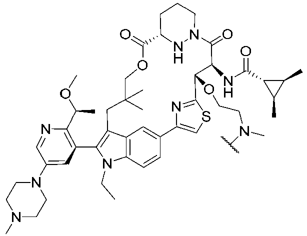 |
| D296 | 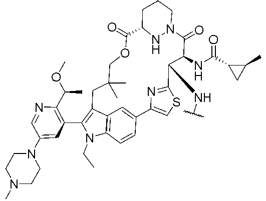 | D295 | 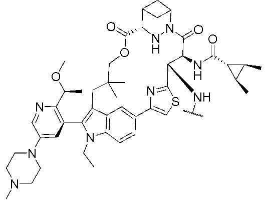 |
| D22 | 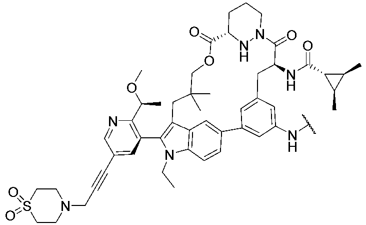 | D293 | 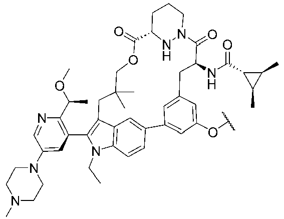 |
| D287 | 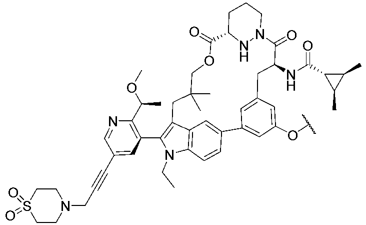 | D62 | 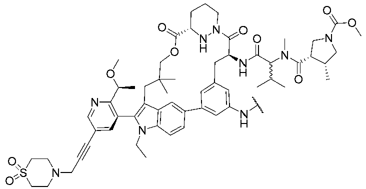 |
| D311 | 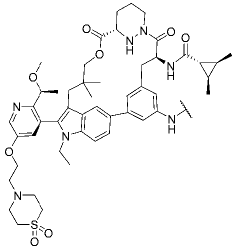 | D294 | 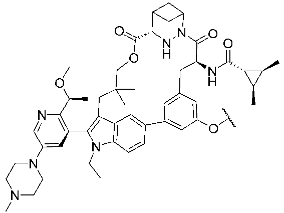 |
| D305-A | 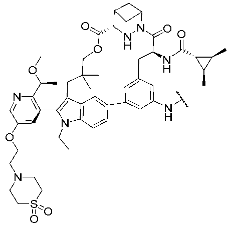 | D304 | 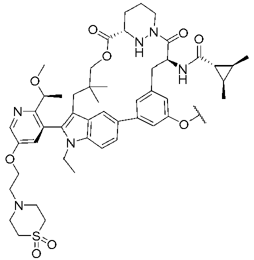 |
| D3 | 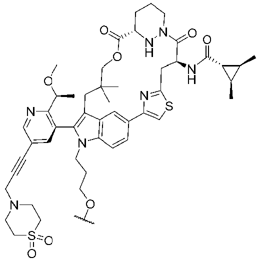 | D305 | 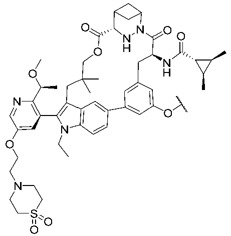 |
| D299 | 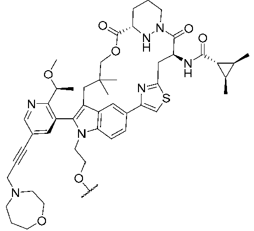 | D284-A | 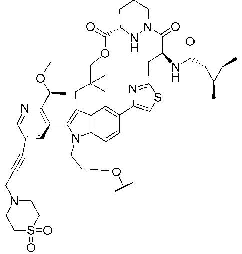 |
| D286-A | 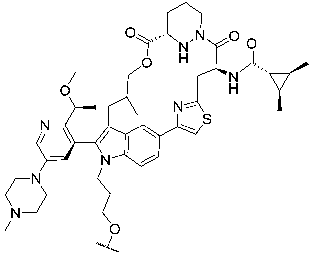 | D334 | 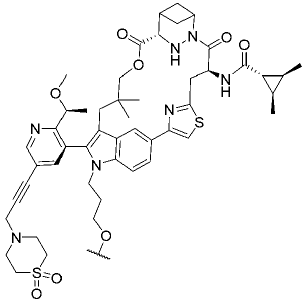 |
| D286-D | 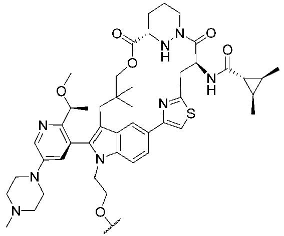 | D187-A | 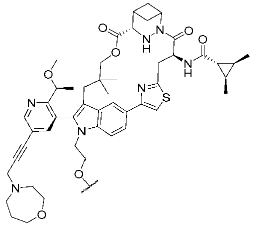 |
| D284-1a | 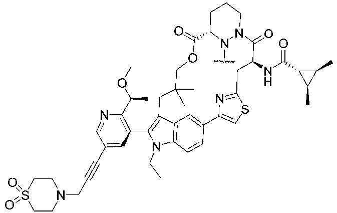 | D285-A | 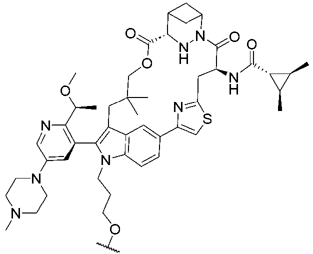 |
| D285-1a | 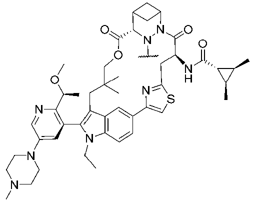 | D298 | 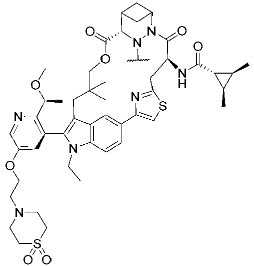 |
| D283-1a | 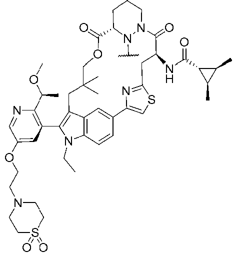 | D286 | 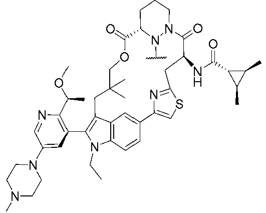 |

In one embodiment, 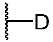 is selected from D10, D11, D166, D168, D169-1, D170, D187, D187-A, D192-A, D192-C, D22, D282-A, D283, D284, D284-A, D7-2, D285, D285-A, D285-B, D286, D286-A, D286-B, D287, D288, D288-A, D289, D290, D291, D292, D293, D294, D295, D296, D297, D298, D299, D299-A, D3, D300, D301, D302, D303, D304, D305, D305-A, D306, D307, D308, D309, D31, D310, D311, D34, D35-A, D38-1, D4, D45-1, D47, D48, D52, D54, D56, D57-A, D6, D61, D63, D64-1, D67-A, and D88.

In one embodiment, 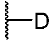 is selected from D10, D11, D166, D168, D169-1, D170, D22, D3, D31, D34, D35-A, D38-1, D4, D45-1, D47, D48, D52, D54, D56, D57-A, D6, D61, D63, and D64-1.

In one embodiment, 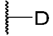 is selected from D31, D34, D35-A, D38-1, D47, D52, D56, D61, and D64-1.

In one embodiment, L₀ is a hydrophilic linker or a non-hydrophilic linker.

In one embodiment, 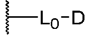 is selected from the structure represented by formula (III-1) or formula (III-2):

-H₃-L₁ₐ-L_{1b}-L_{1c}-L₁₄-D (III-1)

-H₂-L₁ₐ-L_{1b}-L_{1d}(-L_{1c}-C(=O)-CH₃)-D (III-2)

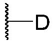

is as defined in any one of the embodiments;
H₂ is an antibody-linking unit attached to Ab;
L₁ₐ is a bond or a derivative unit;
L_{1b} is a bond or a hydrophilic derivative unit;
L_{1c} is a bond or an amino acid unit;
L_{1d} is a bond or a drug-linking unit;
at least one of L₁ₐ, L_{1b}, L_{1c}, and L_{1d} is not a bond;
H₂, L₁ₐ, L_{1b}, L_{1c}, and L_{1d} are each independently and optionally substituted with a hydrophilic group.

In one embodiment, 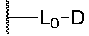 is selected from the structure represented by formula (III-1).

In one embodiment, the hydrophilic group is -1- to 300-membered linear or branched heteroalkyl-R_{end}, wherein the 1- to 300-membered linear or branched heteroalkyl means that 1-100 carbon atoms at any position in the 1-to 300-membered linear or branched alkyl may be replaced by a heteroatom selected from N, O, and S, and the remaining carbon atoms may be optionally substituted with a group selected from deuterium, C₁₋₆ alkyl, oxo (=O), and thio (=S); R_{end} is H, deuterium, hydroxy, carboxyl, amino, an ester group, amido, alkoxy, or substituted amino.

In one embodiment, the hydrophilic group consists of one or more groups selected from: N, NH, N(CH₃), O, C(O), NHC(O), N(CH₃)C(O), C₁₋₆ alkyl, polyethylene glycol, polysarcosine, glucose, glucuronic acid,

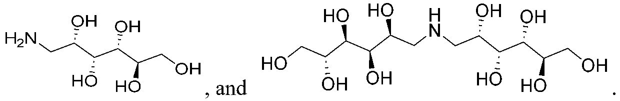

In one embodiment, the hydrophilic group is selected from: 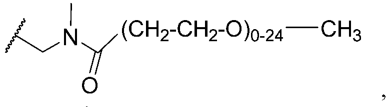 polysarcosine, 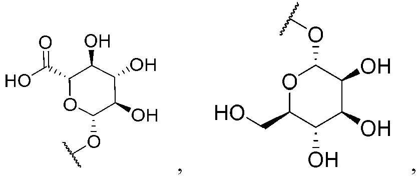 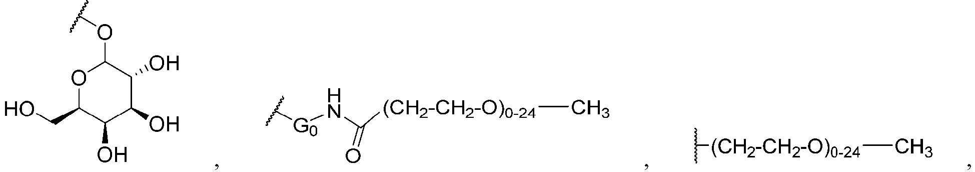 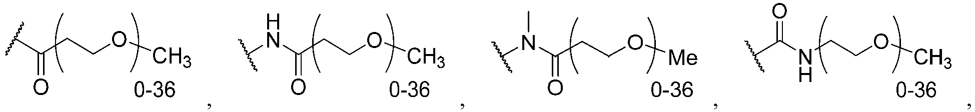 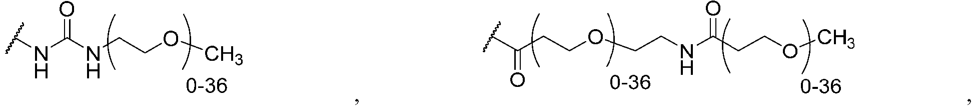 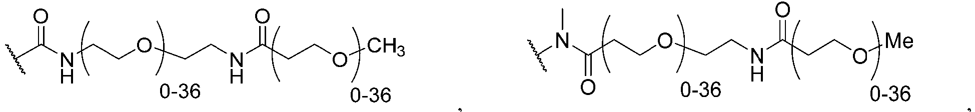 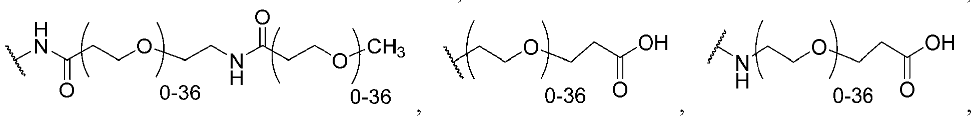 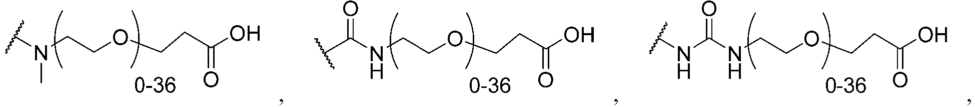 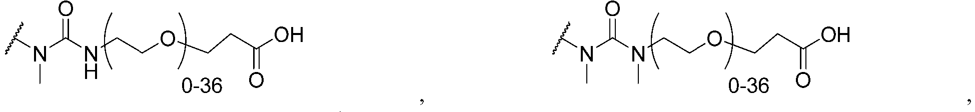 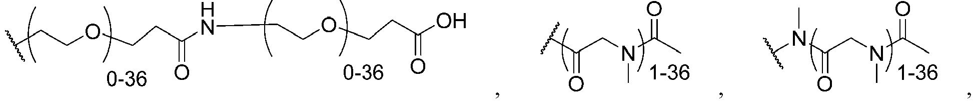 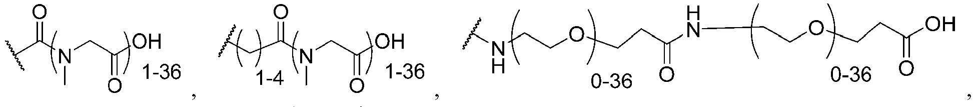 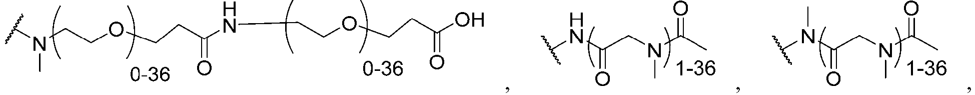 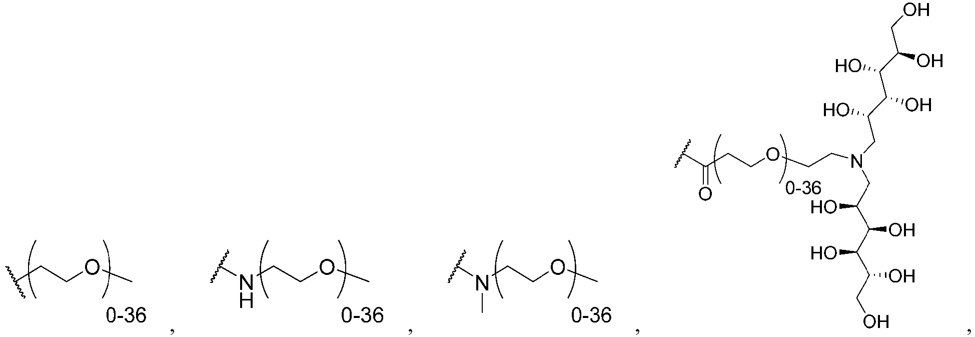 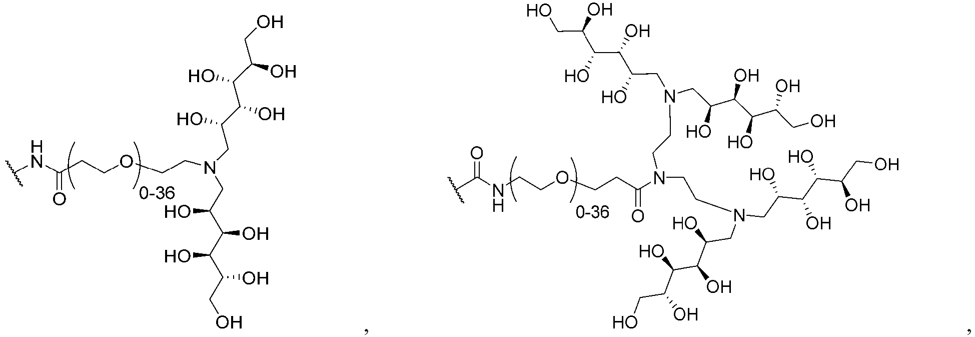 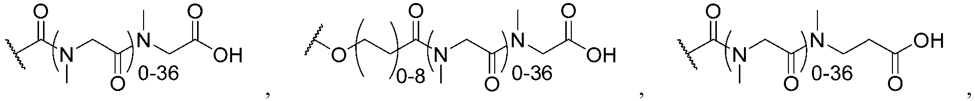 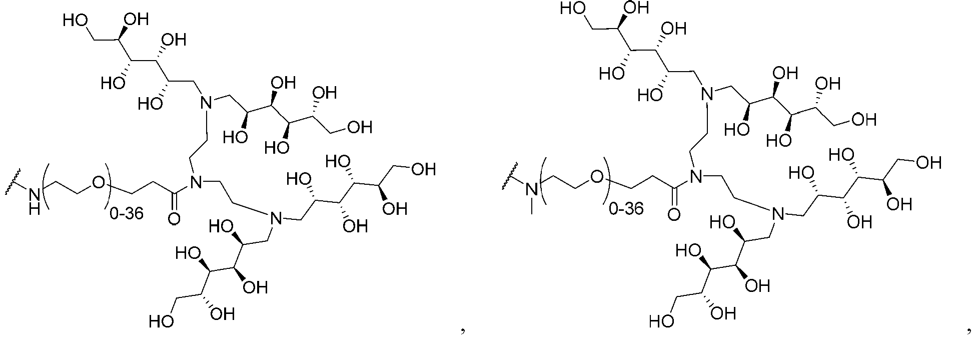 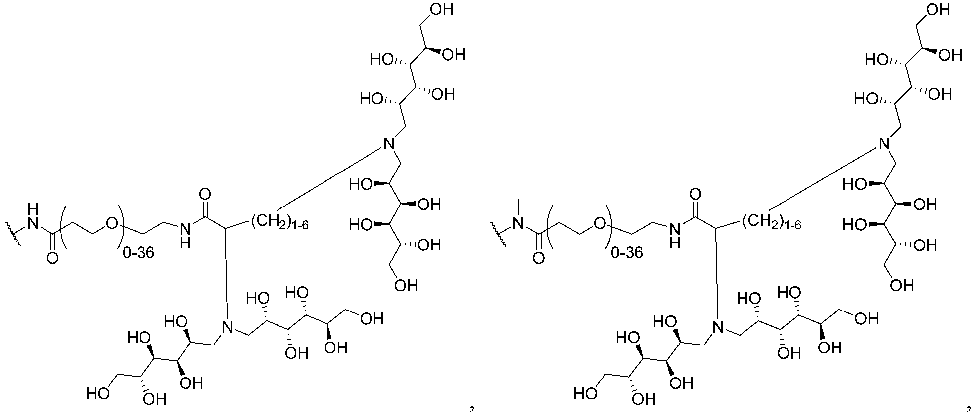 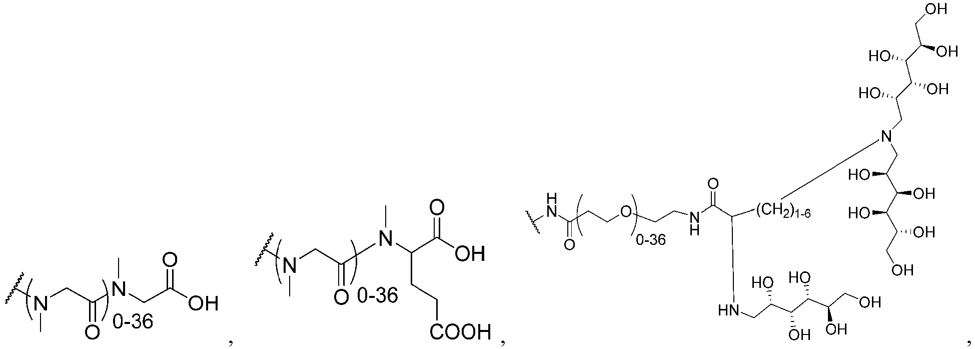 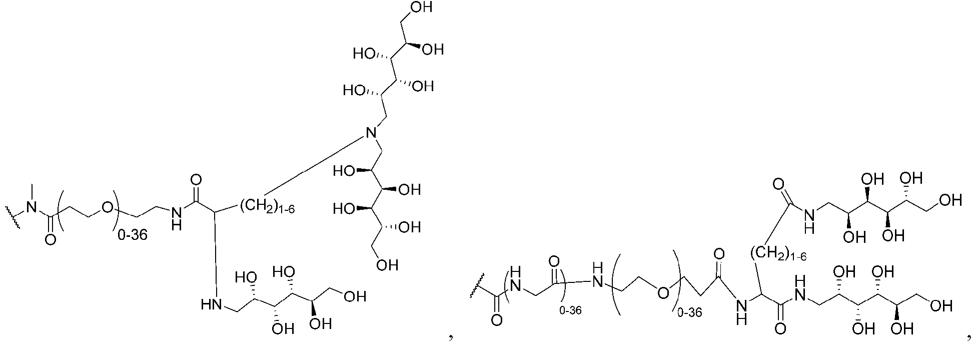 and 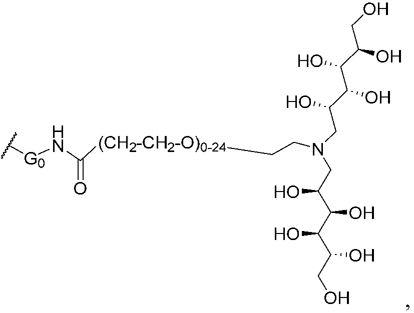 wherein G₀ is C₀₋₈ hydrocarbyl, C₀₋₈ heteroalkyl, -NH-, O, or S.

In one embodiment, G₀ is C₀₋₈ hydrocarbyl or C₀₋₈ heteroalkyl.

In one embodiment, G₀ is a bond, C₁₋₄ alkyl, or C₁₋₄ heteroalkyl.

In one embodiment, G₀ is a bond or n-butyl.

In one embodiment, the hydrophilic group is selected from: 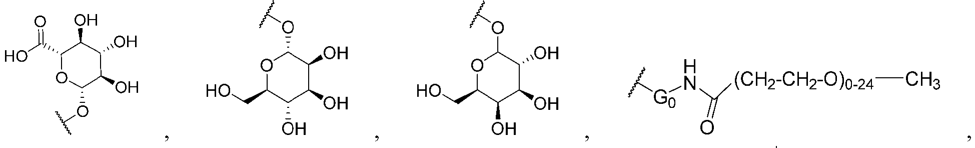 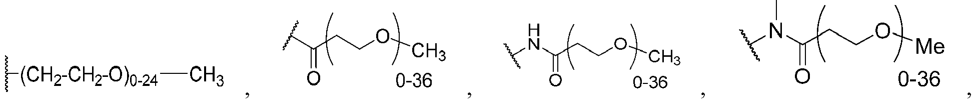 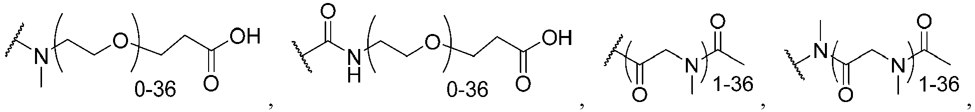 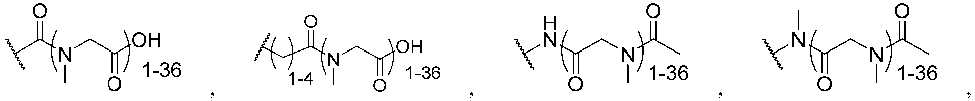 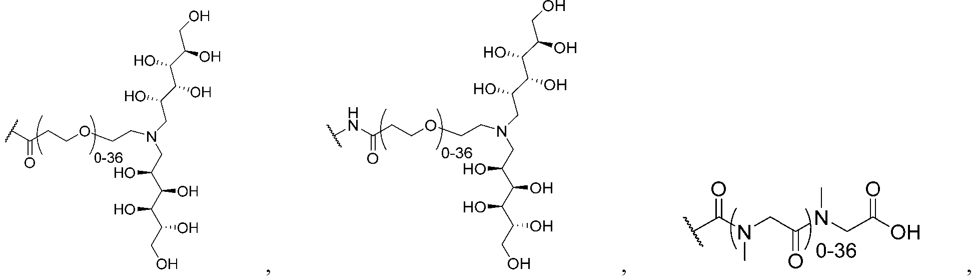 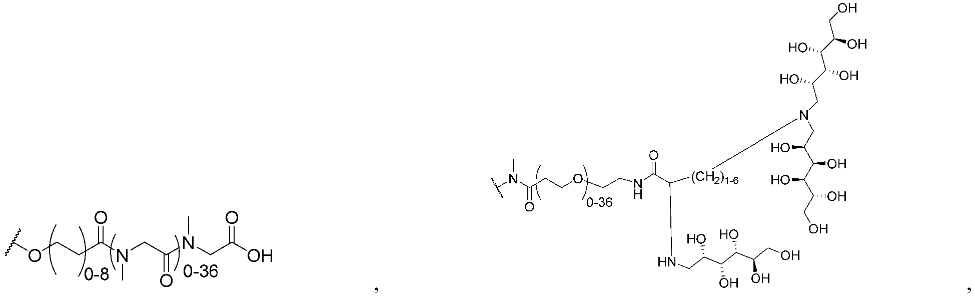 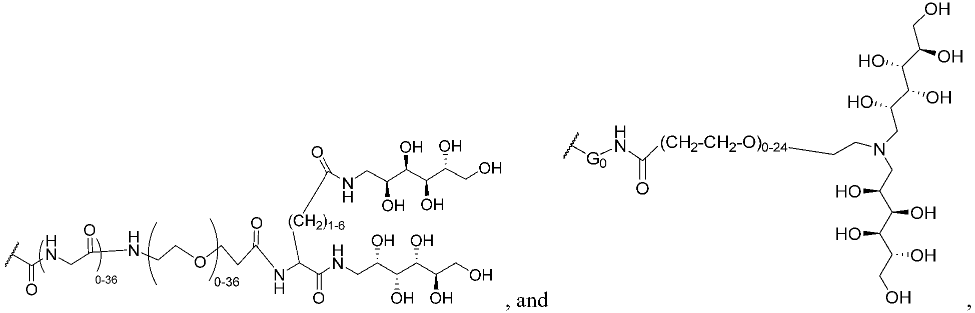 wherein G₀ is as defined in any one of the embodiments.

In one embodiment, the hydrophilic group is selected from: 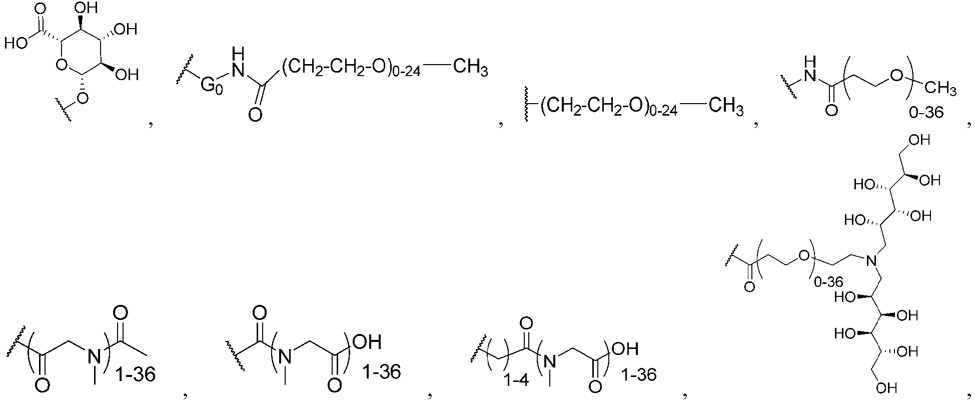 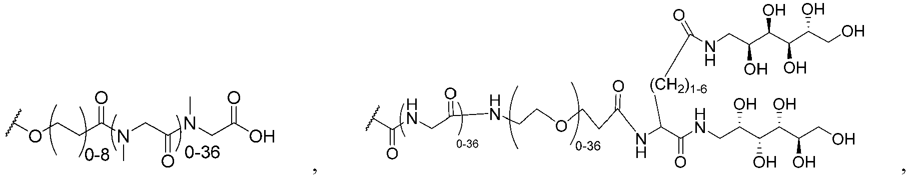 and 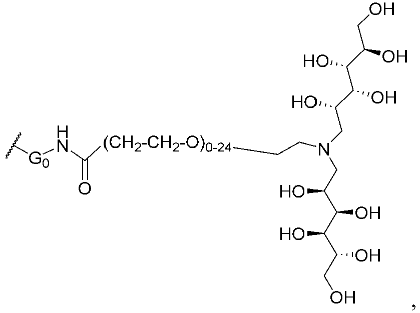 wherein G₀ is as defined in any one of the embodiments.

In one embodiment, the hydrophilic group is selected from: 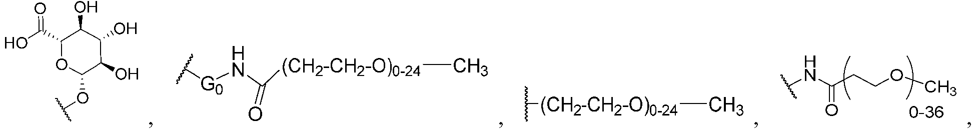 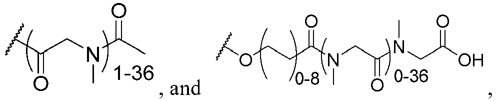 wherein G₀ is as defined in any one of the embodiments.

In one embodiment, the hydrophilic group is selected from:

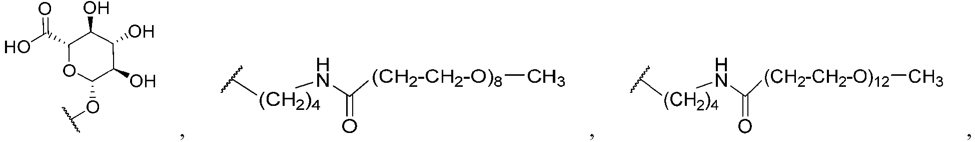

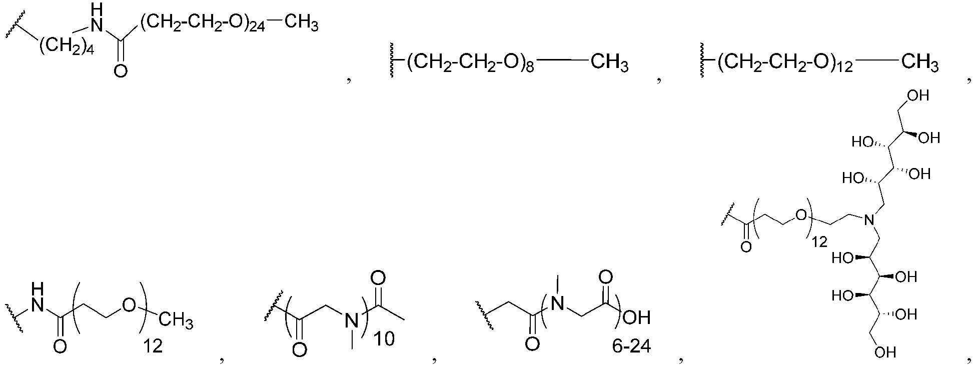

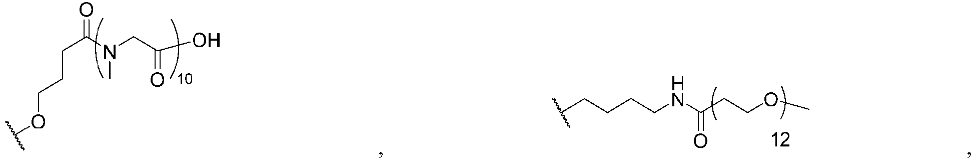

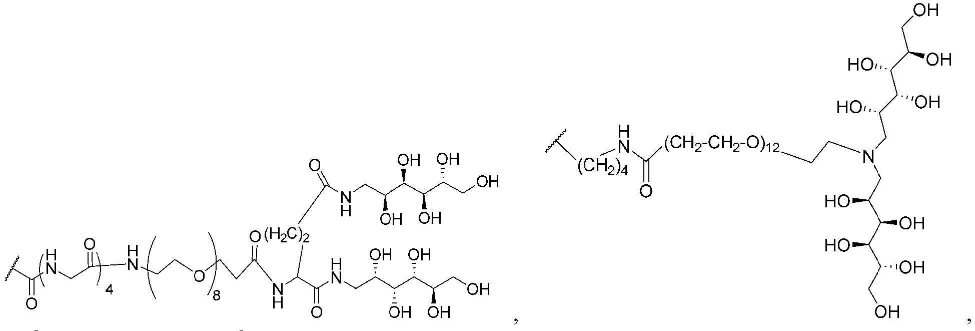

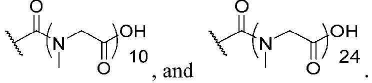

In one embodiment, the hydrophilic group is selected from: 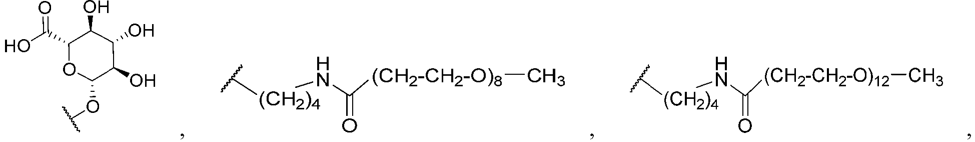 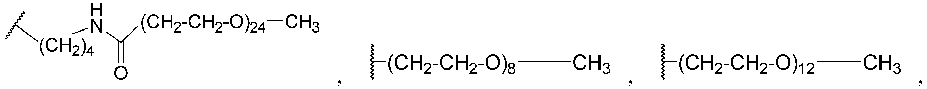 and

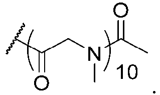

In one embodiment, H₂ is selected from the group consisting of: 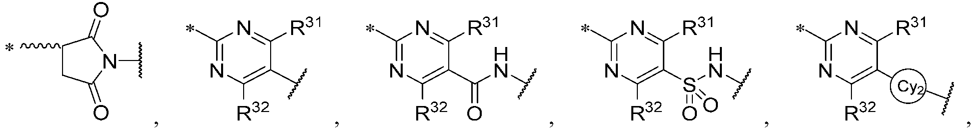 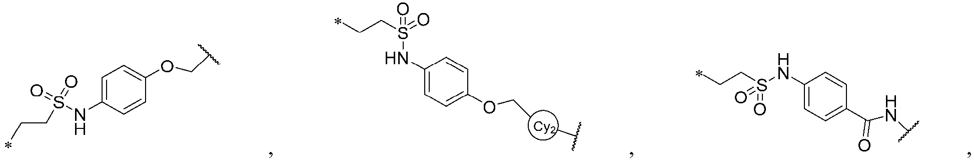 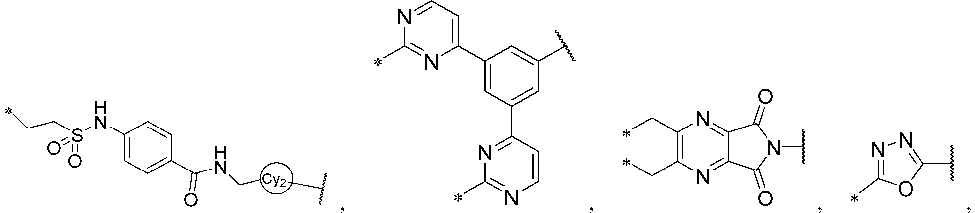 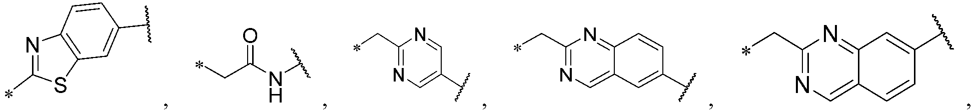 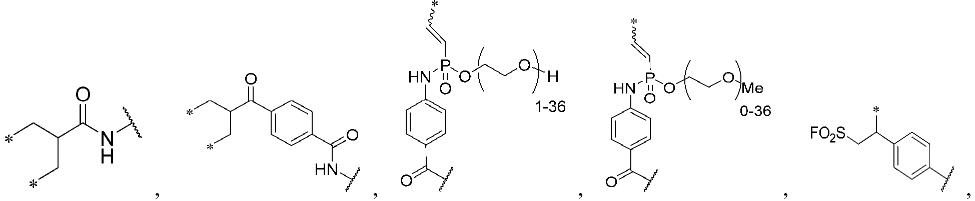 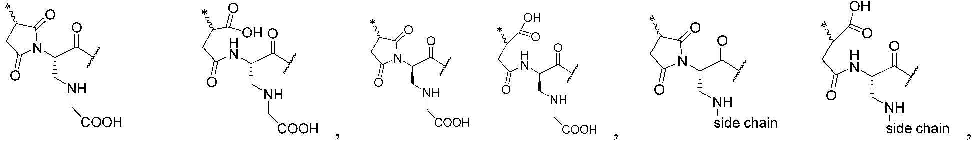 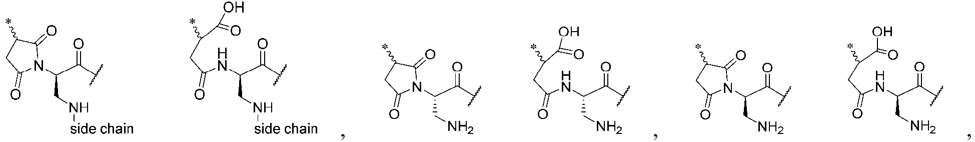 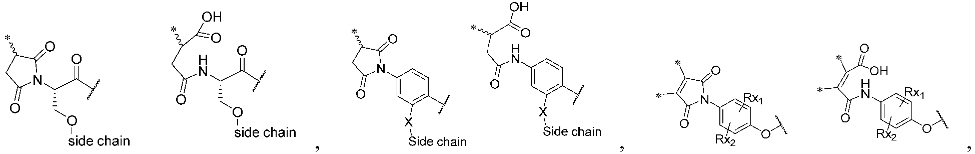 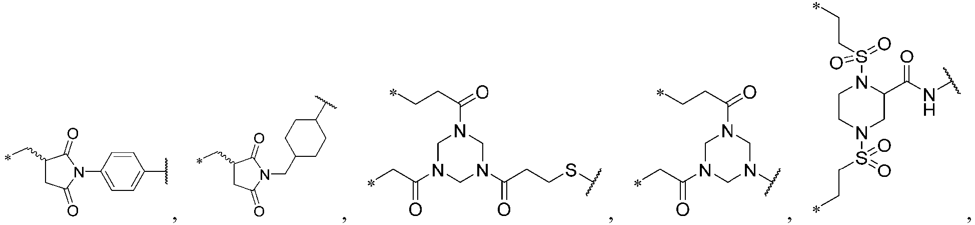 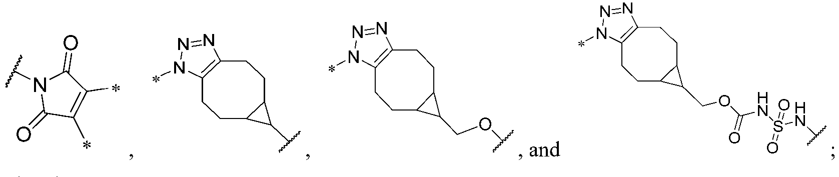 wherein:
the * end is attached to the antibody, and the 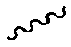 end is attached to L₁ₐ;
X is -O-(CH₂)₀₋₆-, -N(CH₃)-, -O-, -CONH-, or -CON(CH₃)-;
Rₓ₁ and Rₓ₂ are each independently hydrogen, C₁₋₆ alkyl, halogen, deuterium, C₁₋₆ alkoxy, C₁₋₆ alkylene-NH-, or (C₁₋₆ alkyl)₂-N-;
R³¹ is hydrogen, deuterium, halogen, cyano, or C₁₋₄ alkyl;
R³² is hydrogen, deuterium, halogen, cyano, or C₁₋₄ alkyl;
Cy2 is 5- to 10-membered heteroaryl, C₆₋₁₀ aryl, 3- to 10-membered heterocyclyl, or C₃₋₁₀ cycloalkyl;
the side chain is a hydrophilic group, wherein the hydrophilic group is as defined in any one of the embodiments.

In one embodiment, Rₓ₁ and Rₓ₂ are each independently hydrogen, C₁₋₄ alkyl, halogen, deuterium, C₁₋₄ alkoxy, C₁₋₄ alkylene-NH-, or (C₁₋₄ alkyl)₂-N-. In one embodiment, Rₓ₁ and Rₓ₂ are each independently hydrogen, methyl, halogen, deuterium, methoxy, methyl-NH-, or (methyl)₂-N-.

In one embodiment, R³¹ is hydrogen, deuterium, halogen, cyano, or methyl. In one embodiment, R³¹ is hydrogen. In one embodiment, R³² is hydrogen, deuterium, halogen, cyano, or methyl. In one embodiment, R³² is hydrogen. In one embodiment, Cy2 is 5- to 10-membered heteroaryl, C₆₋₁₀ aryl, 4- to 8-membered heterocyclyl, or C₃₋₆ cycloalkyl. In one embodiment, Cy2 is pyrrolyl, imidazolyl, pyridinyl, pyrimidinyl, phenyl, pyrrolidinyl, piperidinyl, piperazinyl, cyclopropyl, cyclobutyl, or cyclopentyl.

In one embodiment, H₂ is selected from the group consisting of: 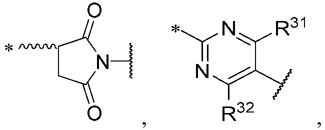 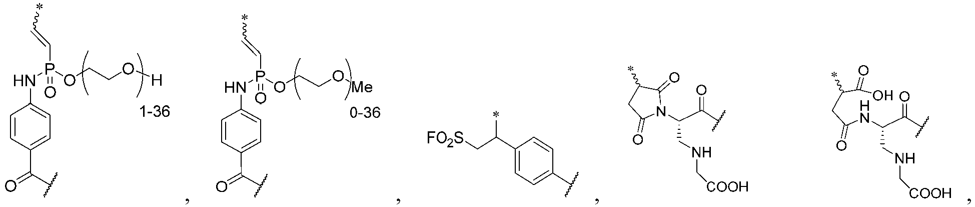 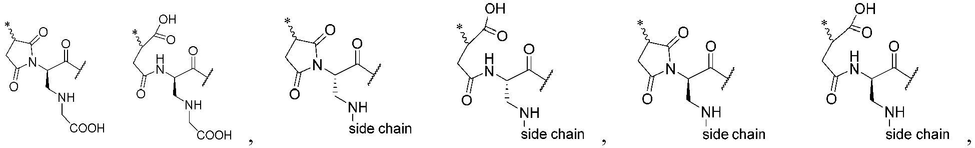 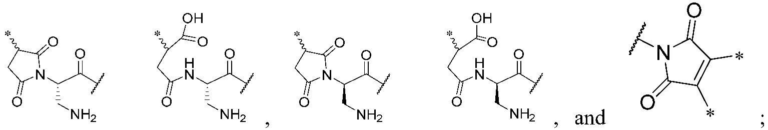 the side chain is a hydrophilic group, wherein the hydrophilic group is as defined in any one of the embodiments; R³¹ and R³² are as defined in any one of the embodiments; the * end is attached to the antibody, and the 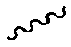 end is attached to L₁ₐ.

In one embodiment, H₂ is selected from the group consisting of: 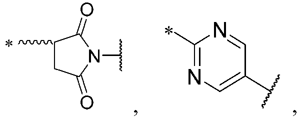 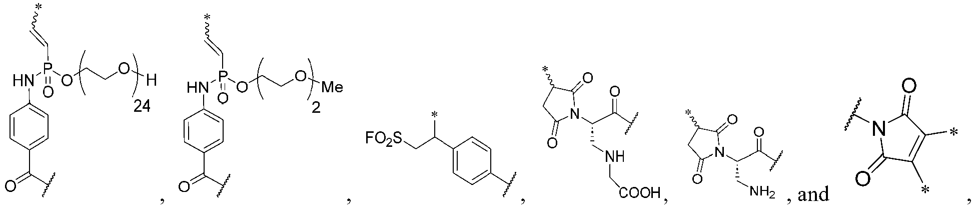 wherein the * end is attached to the antibody, and the 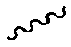 end is attached to L₁ₐ.

In one embodiment, H₂ is selected from the group consisting of: 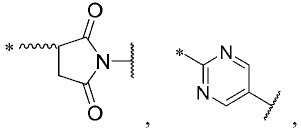 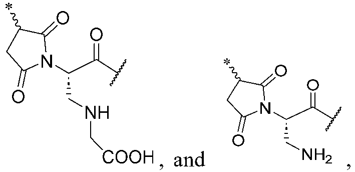 wherein the * end is attached to the antibody, and the 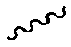 end is attached to L₁ₐ.

In one embodiment, H₂ is selected from the group consisting of: 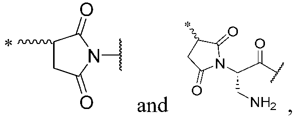 wherein the * end is attached to the antibody, and the 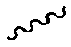 end is attached to L₁ₐ.

**In** one embodiment, H₂ is 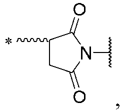 wherein the * end is attached to the antibody, and the 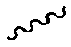 end is attached to L₁ₐ.

In one embodiment, L₁ₐ is a bond or selected from C₁₋₆ alkyl, C₁₋₆ alkylene-C(=O), C₁₋₆ alkylene-C(=O)NH, C₁₋₆ heteroalkyl, C₂₋₆ alkenyl, C₂₋₆ alkynyl, C₃₋₈ cycloalkyl, 4- to 10-membered heterocyclyl, C₆₋₁₀ aryl, 5- to 10-membered heteroaryl, C₁₋₄ alkylene-C₃₋₈ cycloalkyl, C₁₋₄ alkylene-4- to 10-membered heterocyclyl, C₁₋₄ alkylene-C₆₋₁₀ aryl, C₁₋₄ alkylene-5- to 10-membered heteroaryl, C₁₋₆ heteroalkylene-C(=O), C₂₋₆ alkenylene-C(=O), C₂₋₆ alkynylene-C(=O), C₃₋₈ cycloalkyl-C(=O), 4- to 10-membered heterocyclyl-C(=O), C₆₋₁₀ aryl-C(=O), 5- to 10-membered heteroaryl-C(=O), C₁₋₄ alkylene-C₃₋₈ cycloalkyl-C(=O), C₁₋₄ alkylene-4- to 10-membered heterocyclyl-C(=O), C₁₋₄ alkylene-C₆₋₁₀ aryl-C(=O), C₁₋₄ alkylene-5- to 10-membered heteroaryl-C(=O), C₁₋₆ heteroalkylene-C(=O)NH, C₂₋₆ alkenylene-C(=O)NH, C₂₋₆ alkynylene-C(=O)NH, C₃₋₈ cycloalkyl-C(=O)NH, 4-to 10-membered heterocyclyl-C(=O)NH, C₆₋₁₀ aryl-C(=O)NH, 5- to 10-membered heteroaryl-C(=O)NH, C₁₋₄ alkylene-C₃₋₈ cycloalkyl-C(=O)NH, C₁₋₄ alkylene-4- to 10-membered heterocyclyl-C(=O)NH, C₁₋₄ alkylene-C₆₋₁₀ aryl-C(=O)NH, and C₁₋₄ alkylene-5- to 10-membered heteroaryl-C(=O)NH, wherein the C₁₋₆ alkyl, C₁₋₆ alkylene, or C₁₋₄ alkylene is optionally substituted with -C₁₋₄ alkylene-NH₂ or -C₁₋₄ alkylene-NH-C₁₋₄ alkylene-COOH; L₁ₐ is optionally substituted with a hydrophilic group; the hydrophilic group is as defined in any one of the embodiments.

In one embodiment, L₁ₐ is a bond or selected from C₃₋₈ cycloalkyl, 4- to 10-membered heterocyclyl, C₆₋₁₀ aryl, 5- to 10-membered heteroaryl, C₁₋₄ alkylene-C₃₋₈ cycloalkyl-C(=O), C₁₋₄ alkylene-4- to 10-membered heterocyclyl-C(=O), C₁₋₄ alkylene-C₆₋₁₀ aryl-C(=O), and C₁₋₄ alkylene-5- to 10-membered heteroaryl-C(=O), wherein the C₁₋₄ alkylene is optionally substituted with -C₁₋₄ alkylene-NH₂ or -C₁₋₄ alkylene-NH-C₁₋₄ alkylene-COOH; L₁ₐ is optionally substituted with a hydrophilic group; the hydrophilic group is as defined in any one of the embodiments.

In one embodiment, L₁ₐ is a bond or selected from C₆₋₁₀ aryl and C₁₋₄ alkylene-C₃₋₈ cycloalkyl-C(=O), wherein L₁ₐ is optionally substituted with a hydrophilic group, and the hydrophilic group is as defined in any one of the embodiments.

In one embodiment, L₁ₐ is a bond or selected from phenyl, -CH₂-cyclohexyl-C(=O), and -CH₂-piperidinyl-C(=O), wherein L₁ₐ is optionally substituted with a hydrophilic group, and the hydrophilic group is as defined in any one of the embodiments.

In one embodiment, L₁ₐ is a bond, phenyl, 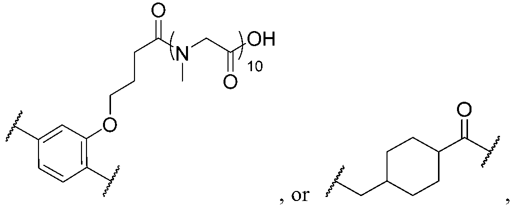 wherein the left side of 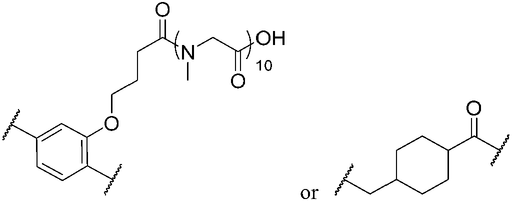 is attached to H₂, and the right side is attached to L_{1b}.

In one embodiment, L₁ₐ is a bond or 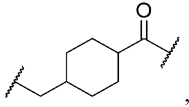 wherein the left side of the 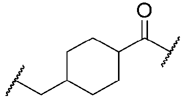 is attached to H₂, and the right side is attached to L_{1b}.

In one embodiment, L₁ₐ is a bond.

In one embodiment, L_{1b} is a bond or selected from: 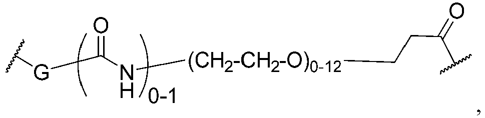 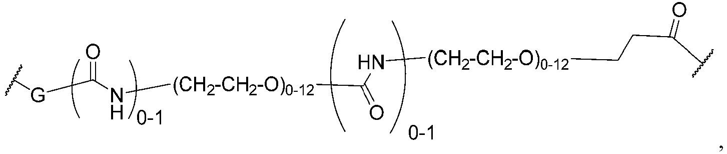 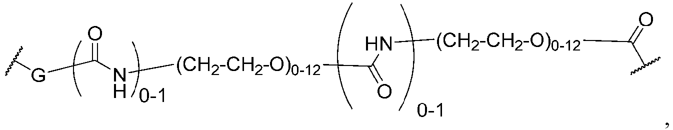 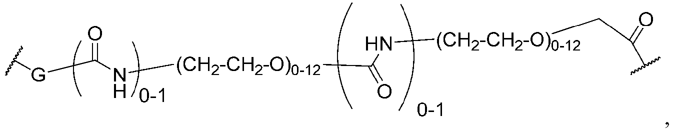 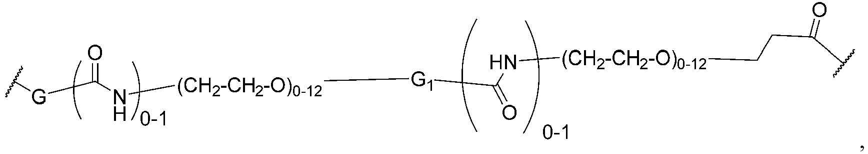 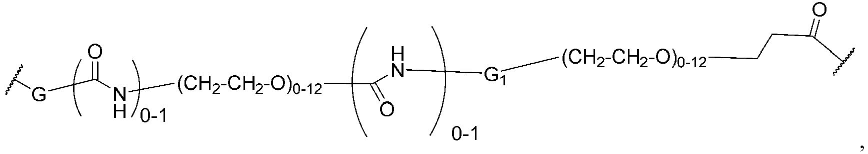 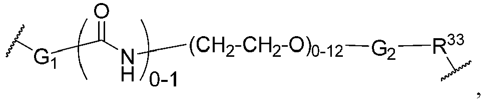 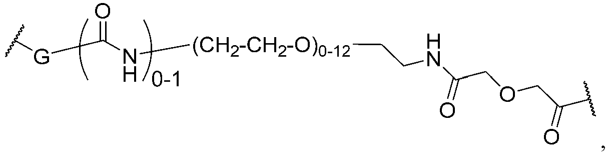 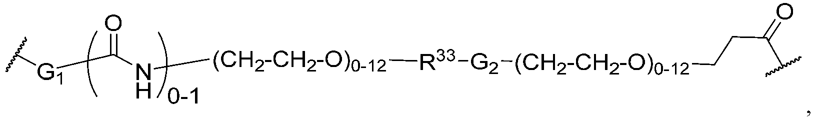 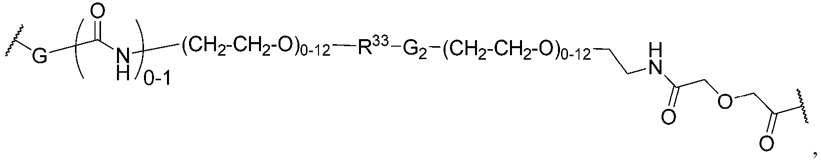 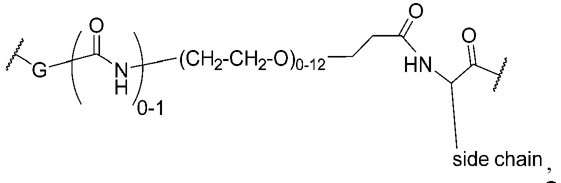 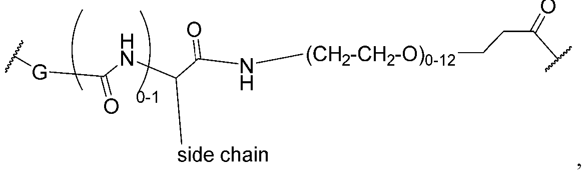 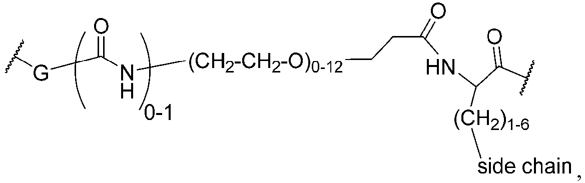 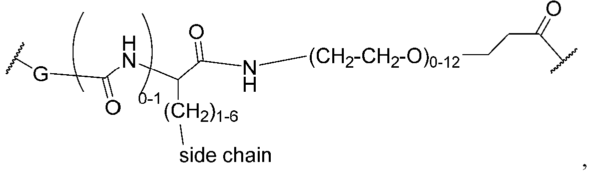 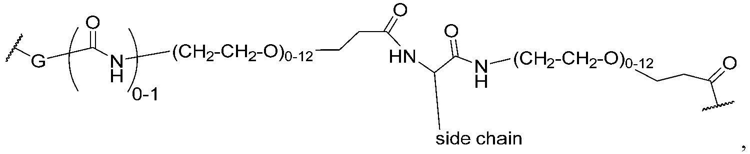 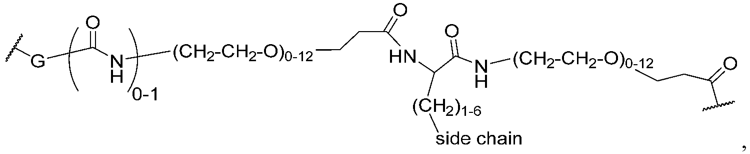 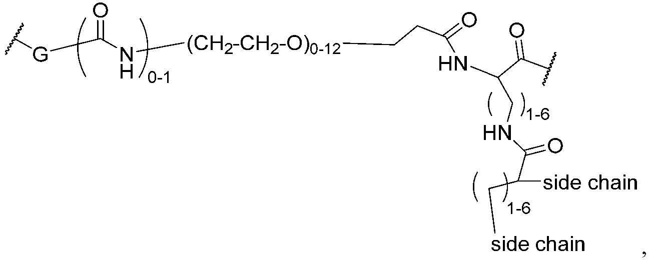 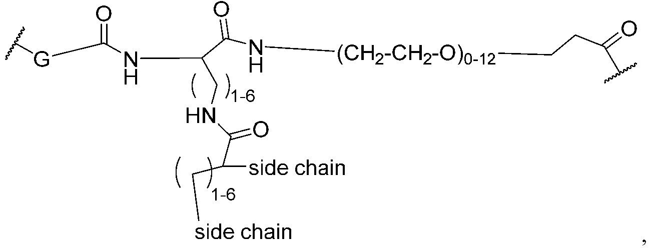 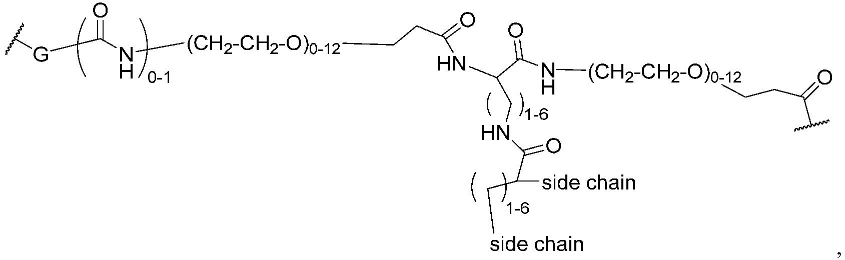 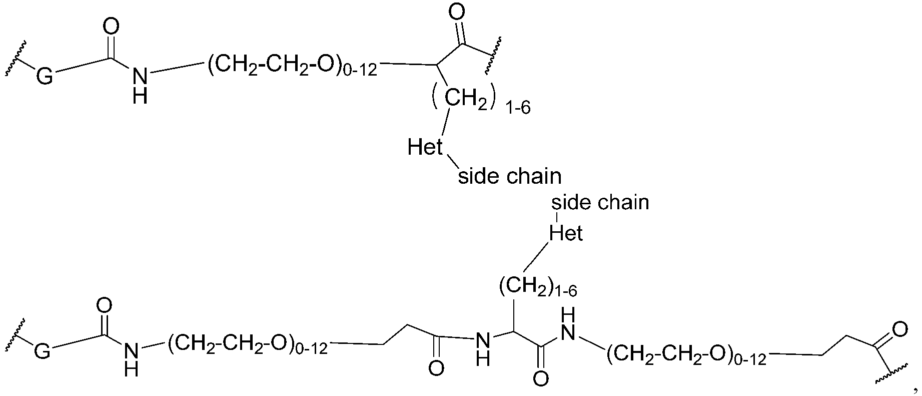 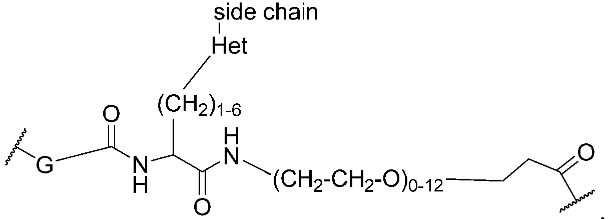 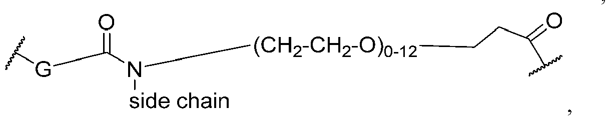 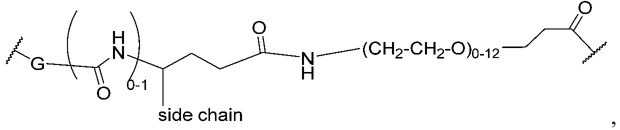 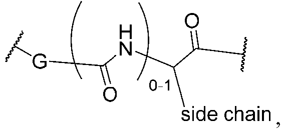 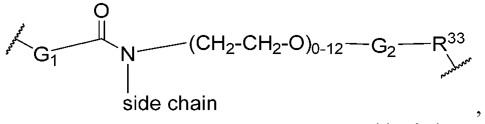 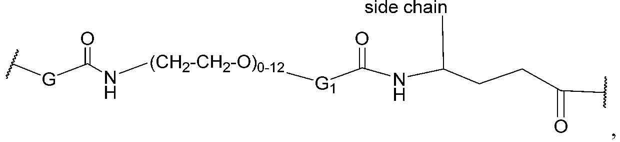
wherein the left side of the fragment described above is attached to L₁ₐ, and the right side is attached to L_{1c};
G is C₀₋₈ hydrocarbylene, C₀₋₈ heteroalkylene, -O-, -NH-, or a bond;
G₁ and G₂ are each independently C₀₋₈ hydrocarbylene, C₀₋₈ heteroalkylene, -O-, -NH-, or a bond;
R³³ is a 3- to 12-membered heterocyclic ring, a 5- to 10-membered heteroaromatic ring, NR³⁴, -CONR³⁴-, or - NR³⁴CO-;
R³⁴ is hydrogen, -(CH₂CH₂O)₀₋₁₂-H, -(CH₂CH₂O)₀₋₁₂-CH₃, -CO-(CH₂CH₂O)₀₋₁₂-H, -CO-(CH₂CH₂O)₀₋₁₂-CH₃, or CH₂-CO-(N(Me)-CH₂-CO)₀₋₂₄-OH;
Het is a 5- to 6-membered heteroaromatic ring;
the side chain is a hydrophilic group, wherein the hydrophilic group is as defined in any one of the embodiments. In one embodiment, G is C₁₋₆ alkylene, C₂₋₆ alkenylene, C₂₋₆ alkynylene, C₁₋₆ heteroalkylene, -O-, -NH-, or a bond. In one embodiment, G is C₁₋₄ alkylene, C₂₋₆ alkynylene, -NHC₁₋₄ alkyl, -O-, -NH-, or a bond. In one embodiment, G is methylene, ethylene, n-propylene, 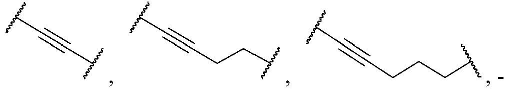 N-CH₂-, -O-, -NH-, or a bond.

In one embodiment, G₁ and G₂ are each independently C₁₋₄ alkylene, C₂₋₆ alkynylene, -NHC₁₋₄ alkylene, -O-, - NH-, or a bond. In one embodiment, G₁ and G₂ are each independently methylene, ethylene, n-propylene, -O-, - NH-, or a bond. In one embodiment, G₁ is methylene. In one embodiment, G₂ is ethylene.

In one embodiment, R³³ is a 5- to 10-membered heteroaromatic ring. In one embodiment, R³³ is a 5- to 6-membered heteroaromatic ring. In one embodiment, R³³ is pyrrolyl, imidazolyl, pyrazolyl, triazolyl, pyridinyl, or pyrimidinyl. In one embodiment, R³³ is 1,2,3-triazolyl.

In one embodiment, L_{1b} is a bond or selected from: 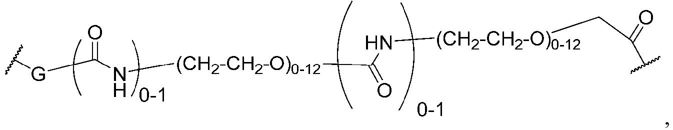 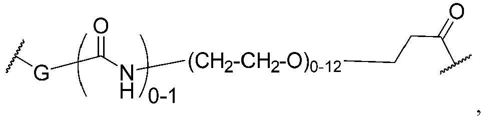 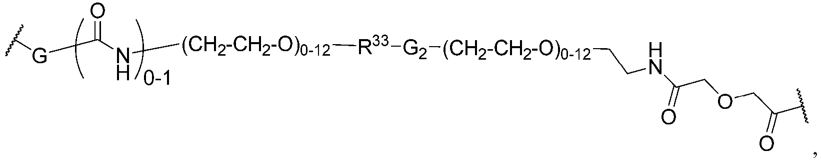 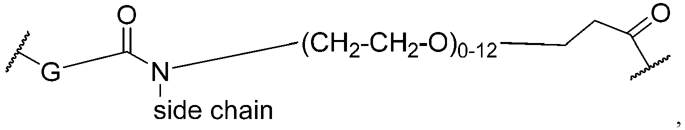 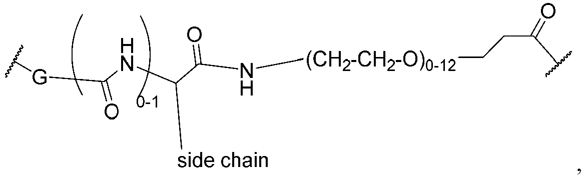 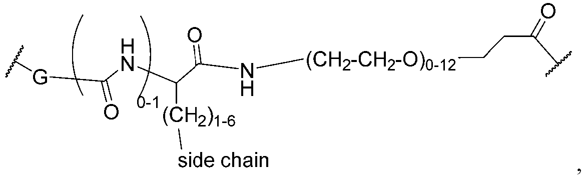 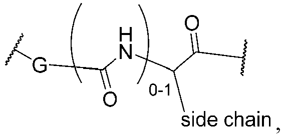 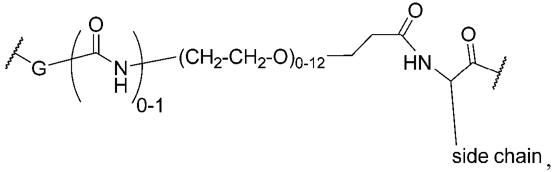 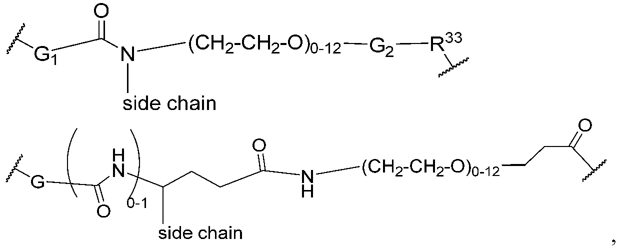 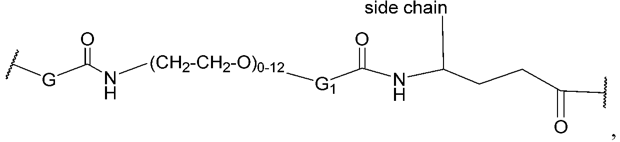 the side chain is a hydrophilic group, wherein the hydrophilic group is as defined in any one of the embodiments; the left side of the fragment is attached to L₁ₐ, and the right side is attached to L_{1c}; G, G₁, G₂, and R³³ are as defined in any one of the embodiments.

In one embodiment, L_{1b} is a bond or selected from: 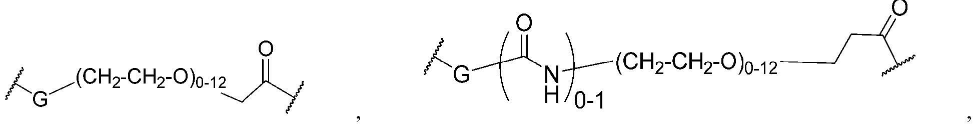 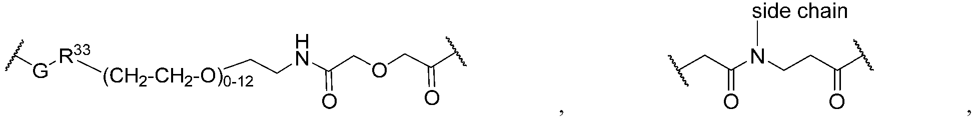 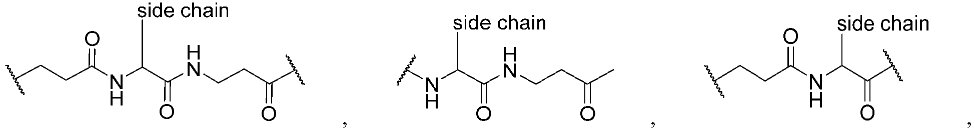 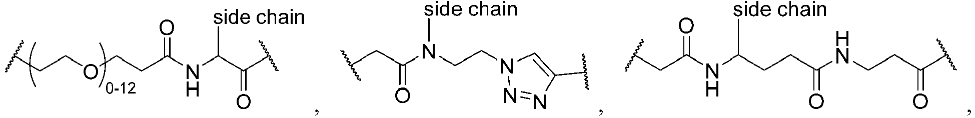 and 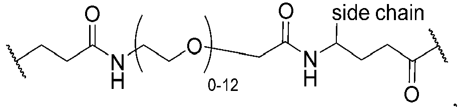
the side chain is a hydrophilic group, wherein the hydrophilic group is defined in any one of the embodiments;
G and R³³ are as defined in any one of the embodiments; the left side of the fragment is attached to L₁ₐ, and the right side is attached to L_{1c}.

In one embodiment, L_{1b} is a bond or selected from: 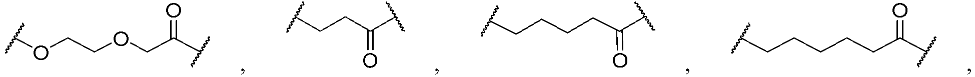 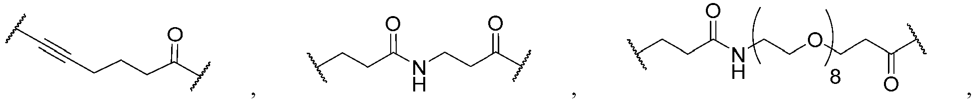 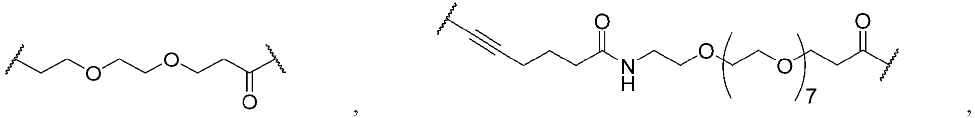 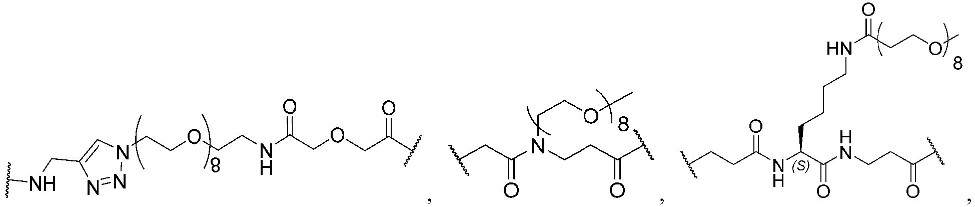 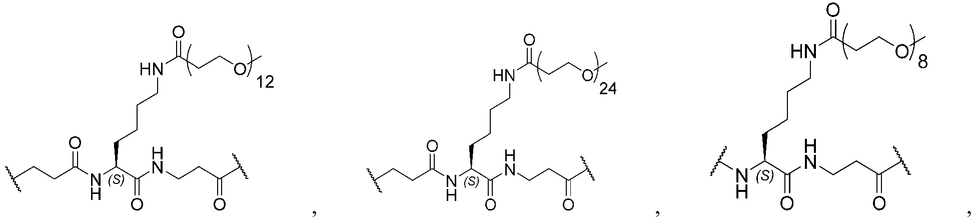 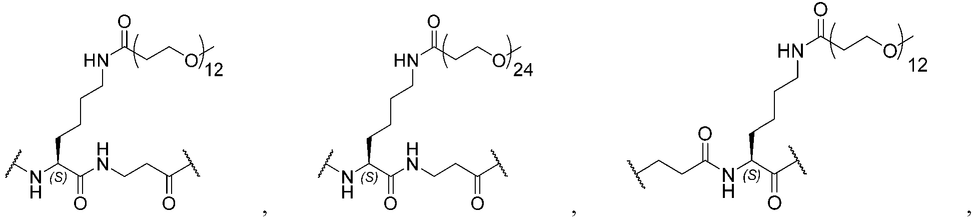 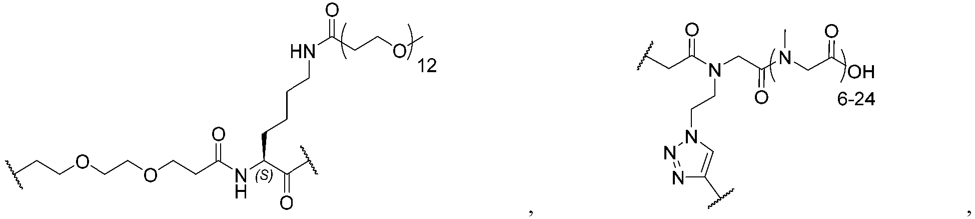 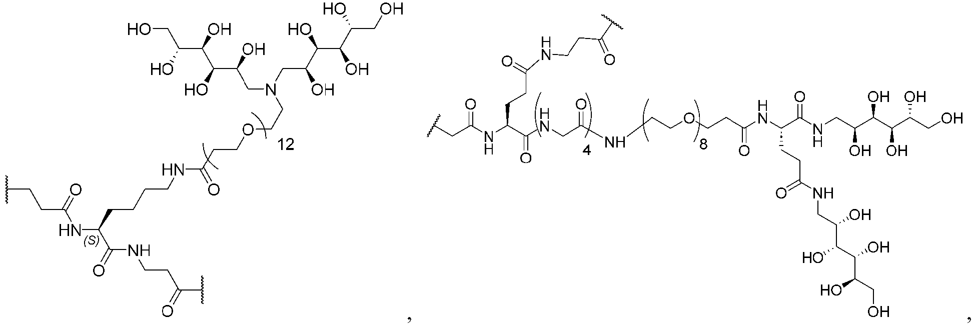 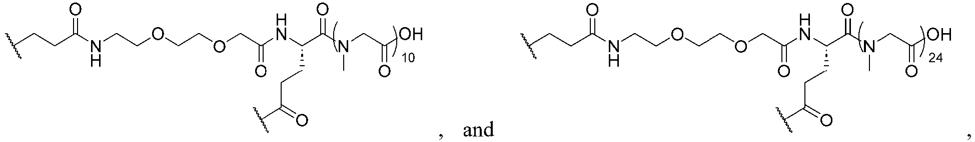 wherein the left side of the fragment is attached to L₁ₐ, and the right side is attached to L_{1c}.

In one embodiment, L_{1b} is 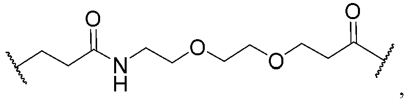 wherein the left side of the fragment is attached to L₁ₐ, and the right side is attached to L_{1c}.

In one embodiment, L_{1d} is a bond or selected from: 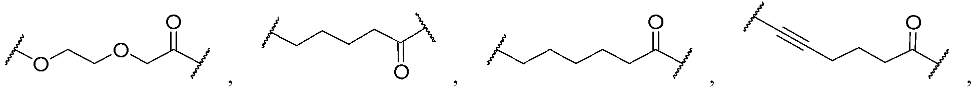 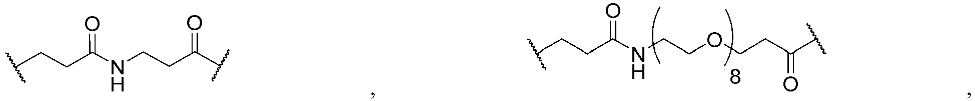 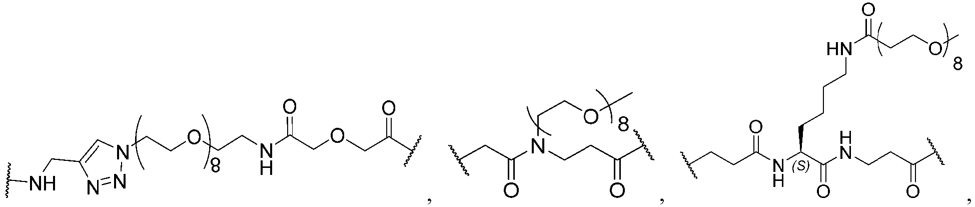 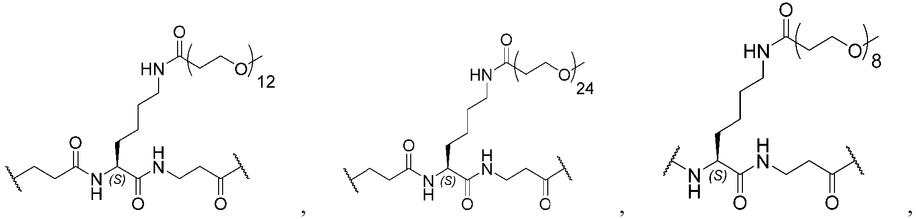 and 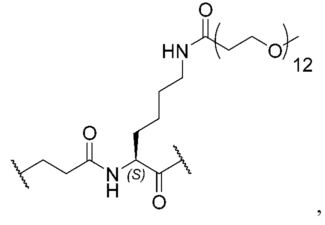 wherein the left side of the fragment is attached to L₁ₐ, and the right side is attached to L_{1c}.

In one embodiment, L_{1b} is a bond or selected from: 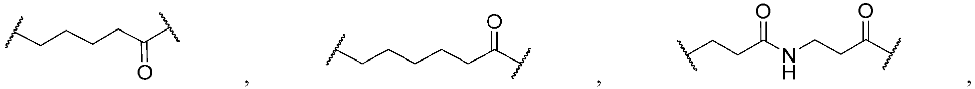 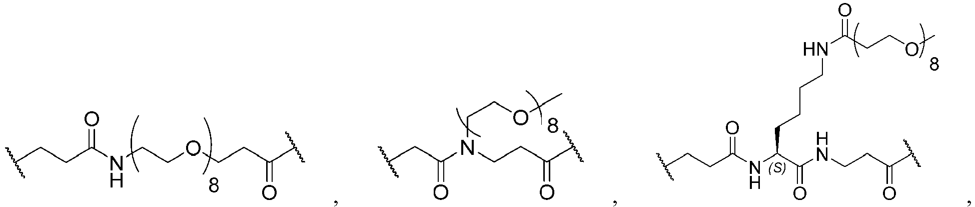 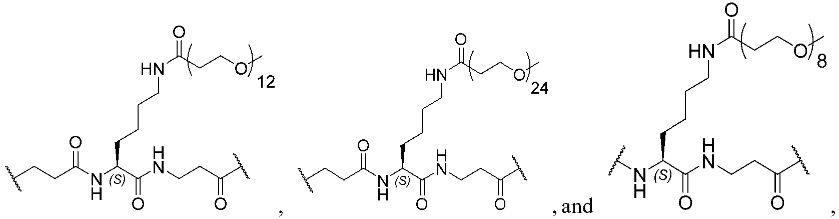 wherein the left side of the fragment is attached to L₁ₐ, and the right side is attached to L_{1c}.

In one embodiment, L_{1b} is a bond or selected from: 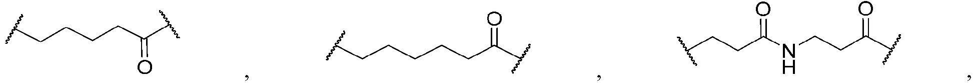 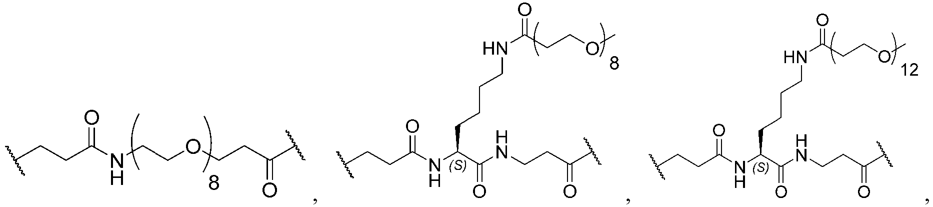 and 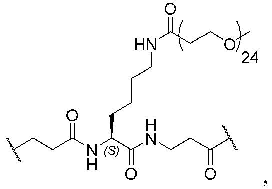 wherein the left side of the fragment is attached to L₁ₐ, and the right side is attached to L_{1c}.

In one embodiment, L_{1c} is a bond or an amino acid unit, wherein the amino acid unit is an amino acid residue or a short peptide consisting of 2-10 amino acid residues; the amino acid residue is optionally substituted with a hydrophilic group or C₁₋₆ alkyl; the hydrophilic group is as defined in any one of the embodiments; the amino acid residue is selected from a natural amino acid residue, a non-natural amino acid residue, and an amino acid residue represented by formula (1a) or a stereoisomer thereof,

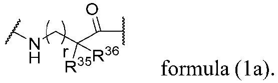

In one embodiment, L_{1c} is an amino acid residue or a short peptide consisting of 2-10 amino acid residues; the amino acid residue is selected from a natural amino acid residue, a non-natural amino acid residue, and an amino acid residue represented by formula (1a) or a stereoisomer thereof. 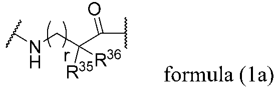 wherein:
R³⁵ and R³⁶ are each independently selected from: hydrogen, deuterium, 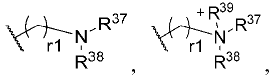 and 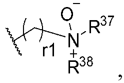 and R³⁵ and R³⁶ are not both H; or
R³⁵ and R³⁶, together with the carbon atom to which they are both attached, form 3- to 8-membered cycloalkyl or 4- to 10-membered heterocyclyl, wherein the 3- to 8-membered cycloalkyl or the 4- to 10-membered heterocyclyl is optionally substituted with one or more R⁴⁰;
r and r1 are each independently an integer from 0 to 20;
R³⁷ and R³⁸ are each independently H, C₁₋₆ alkyl, C₃₋₆ cycloalkyl, or -COO-C₁₋₆ alkyl; or
R³⁷ and R³⁸, together with the nitrogen atom to which they are attached, form 4- to 10-membered heterocyclyl, wherein the 4- to 10-membered heterocyclyl is optionally substituted with one or more R⁴⁰;
R³⁹ is C₁₋₆ alkyl;
R⁴⁰ is C₁₋₆ alkyl, C₁₋₆ haloalkyl, C₁₋₆ heteroalkyl, C₃₋₆ cycloalkyl, 4- to 10-membered heterocyclyl, C₁₋₆ alkylene-C₃₋₆ cycloalkyl, C₁₋₆ alkylene-4- to 10-membered heterocyclyl, -NR⁴⁰¹R⁴⁰², and 4- to 10-membered heterocyclyl optionally substituted with C₁₋₆ alkyl, wherein R⁴⁰¹ and R⁴⁰² are each independently H or C₁₋₆ alkyl.

In one embodiment, r and r1 are each independently 0, 1, 2, 3, 4, or 5. In one embodiment, r is 0. In one embodiment, r1 is 4.

In one embodiment, R³⁷ and R³⁸ are each independently H, C₁₋₄ alkyl, C₃₋₆ cycloalkyl, or -COO-C₁₋₄ alkyl. In one embodiment, R³⁷ and R³⁸ are each independently H or C₁₋₄ alkyl. In one embodiment, R³⁷ and R³⁸ are each independently ethyl.

In one embodiment, R³⁹ is C₁₋₄ alkyl. In one embodiment, R³⁹ is methyl or ethyl.

In one embodiment, R⁴⁰ is C₁₋₄ alkyl, C₁₋₄ haloalkyl, C₃₋₆ cycloalkyl, C₁₋₄ alkylene-C₃₋₆ cycloalkyl, or -NR⁴⁰¹R⁴⁰², wherein R⁴⁰¹ and R⁴⁰² are each independently H or C₁₋₄ alkyl. In one embodiment, R⁴⁰ is methyl, halomethyl, cyclopropyl, amino, NHCH₃, or N(CH₃)₂.

In one embodiment, R³⁵ and R³⁶ are each independently selected from: hydrogen, deuterium, or 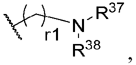 wherein r1, R³⁷, and R³⁸ are as defined in any one of the embodiments. In one embodiment, R³⁵ is hydrogen. In one embodiment, R³⁶ is -(CH₂)₄N(CH₃)₂.

In one embodiment, L_{1c} is a bond or selected from an amino acid residue and a short peptide consisting of 2-10 amino acid residues, wherein the amino acid residue is selected from: Lys, Val, Ala, Cit, Gly, Phe, Arg, Asn, Asp, Cys, Gln, Glu, His, Ile, Leu, Met, Pro, Ser, Thr, Trp, Tyr, and the amino acid residue represented by formula (1a); the amino acid residue is optionally substituted with one or more substituents selected from a hydrophilic group and C₁₋₄ alkyl; the hydrophilic group is as defined in any one of the embodiments.

In one embodiment, the short peptide is selected from: Val-Ala, Val-Cit, Ala-Ala, Ala-Lys, Gly-Gly, Gly-Lys, Val-Lys, Val-Lys-Gly, Val-Ala-Gly, Val-Ser-Gly, Lys-Val-Cit, Val-Gly-Gly, Gly-Val-Cit, Ala-Ala-Ala, Ala-Ala-Gly, Gly-Gly-Gly, Gly-Gly-Phe-Gly (SEQ ID NO: 39), and Gly-Gly-Val-Ala (SEQ ID NO: 40); the short peptide is optionally substituted with one or more substituents selected from a hydrophilic group and C₁₋₄ alkyl; the hydrophilic group is as defined in any one of the embodiments.

In one embodiment, L_{1c} is a bond or selected from: Lys, Val-Ala, Val-Cit, Val-Lys-Gly, Val-Ala-Gly, Lys-Val-Cit, and Gly-Gly-Phe-Gly, wherein the Lys, Val-Ala, Val-Cit, Val-Lys-Gly, Val-Ala-Gly, Lys-Val-Cit, or Gly-Gly-Phe-Gly is optionally substituted with 1, 2, 3, 4, or 5 substituents selected from a hydrophilic group, methyl, and ethyl; the hydrophilic group is as defined in any one of the embodiments.

In one embodiment, in formula (III-1), L_{1c} is a bond or selected from:

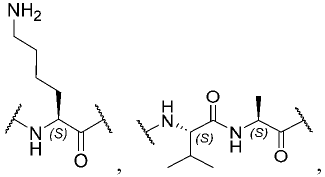

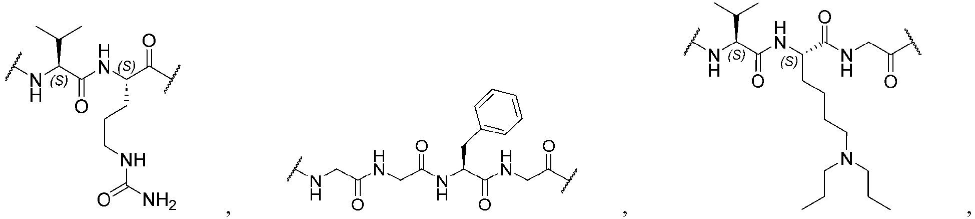

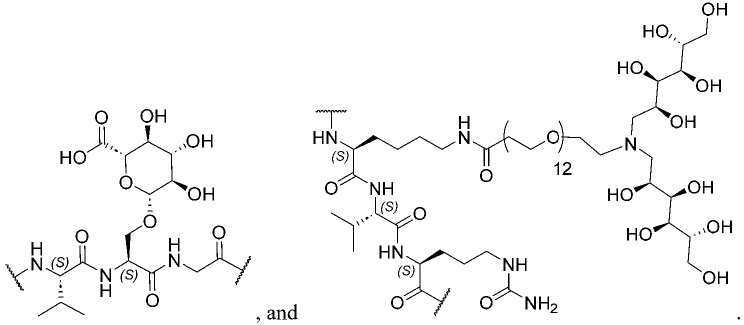

In one embodiment, in formula (III-1), L_{1c} is selected from: 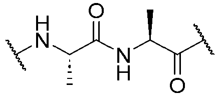 and

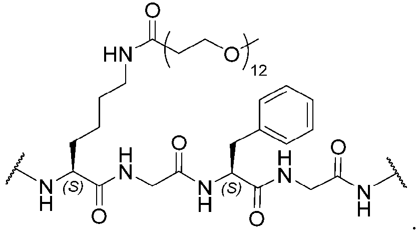

In one embodiment, in formula (III-1), L_{1c} is a bond or selected from: 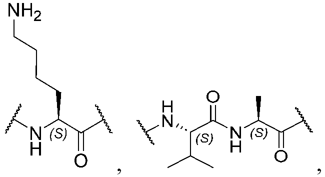 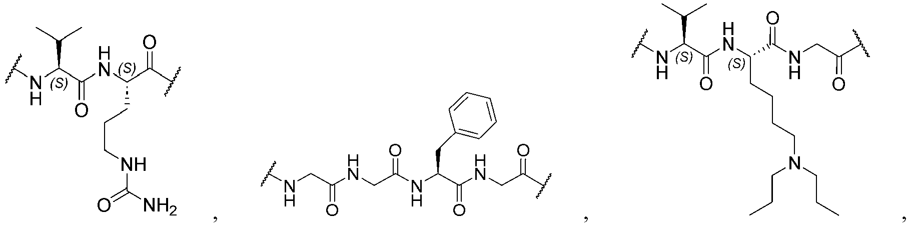 and

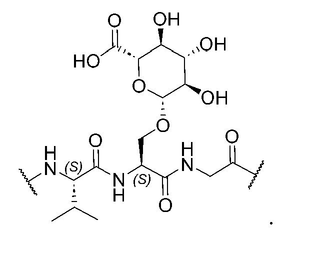

In one embodiment, in formula (III-1), L_{1c} is a bond or selected from:

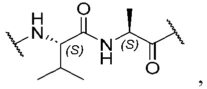

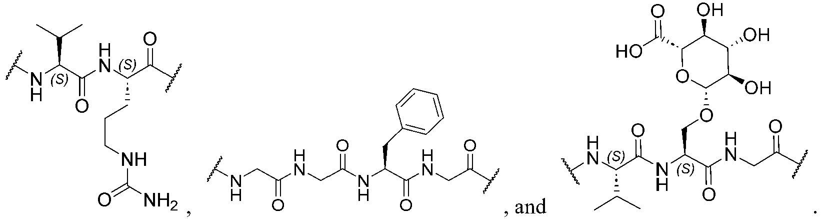

In one embodiment, in formula (III-1), the left side of the fragment is attached to L_{1c}, and the right side is attached to L_{1d}.

In one embodiment, in formula (III-2), L_{1c} is a bond or selected from:

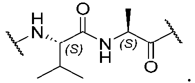

In one embodiment, L_{1c} is selected from: the amino acid residues Val, D-Val, Cit, Phe, Lys, Lys(Ac), Leu, Gly, Ala, Asn, Asp, and Arg, the amino acid residue represented by formula (1a), and a short peptide consisting of 2-10 amino acid residues selected from Val, Cit, Phe, Lys, D-Val, Leu, Gly, Ala, Asn, Asp, and the amino acid residue represented by formula (1a).

In one embodiment, one of R³⁵ and R³⁶ is hydrogen, and the other is selected from: 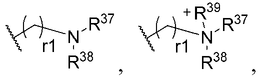 and

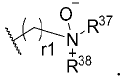

In one embodiment, R³⁵ and R³⁶, together with the carbon atom to which they are both attached, form 5- to 6-membered heterocyclyl, wherein the 5- to 6-membered heterocyclyl is substituted with any one or more R⁴⁰.

In one embodiment, r and r1 are each independently selected from 0, 1, 2, 3, 4, and 5.

In one embodiment, R³⁷ and R³⁸ are each independently selected from: hydrogen, methyl, ethyl, n-propyl, n-butyl, -COOCH3, -COOCH2CH3, -COOCH2CH2CH3, -COOCH(CH3)2, -COOC(CH3)3, and - COOCH2CH2CH2CH3; or R³⁷ and R³⁸, together with the nitrogen atom to which they are attached, form 5- to 6-membered heterocyclyl, which is substituted with any one or more R⁴⁰.

In one embodiment, R³⁹ is methyl.

In one embodiment, R⁴⁰ is C₁₋₆ alkyl, -NR⁴⁰¹R⁴⁰², or 5- to 6-membered heterocyclyl optionally substituted with C₁₋₆ alkyl.

In one embodiment, L_{1c} is selected from Val, Cit, Phe, Lys, D-Val, Leu, Gly, Ala, Asn, the amino acid residue represented by formula (1a), Val-Cit, Cit-Val, Cit-Ala, Val-Ala, Val-Arg, Val-Ala-Gly, Lys-Val, Val-Lys(Ac), Phe-Lys, Phe-Lys(Ac), Ala-Ala, Val-the amino acid residue represented by formula (1a), Ala-the amino acid residue represented by formula (1a), Gly-the amino acid residue represented by formula (1a), the amino acid residue represented by formula (1a)-Gly, Ala-Ala-Ala, Ala-Ala-Asn, Ala-Ala-Asp, Val-the amino acid residue represented by formula (1a)-Gly, Ala-the amino acid residue represented by formula (1a)-Gly, Gly-the amino acid residue represented by formula (1a)-Gly, Lys-Ala-Ala-Asn (SEQ ID NO: 41), Lys-Ala-Ala-Asp (SEQ ID NO: 42), Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly (SEQ ID NO: 43), Val-Lys, and Lys-Ala-Asn.

In one embodiment, L_{1c} is selected from Val-Ala, Val-Arg, Val-Ala-Gly, and Gly-Gly-Phe-Gly.

In one embodiment, L_{1c} is selected from: the amino acid residue represented by formula (1a), the amino acid residue represented by formula (1a)-Gly, Val-Cit, Val-the amino acid residue represented by formula (1a)-Gly, the amino acid residue represented by formula (1a)-Ala-Asn, and Gly-Gly-Phe-Gly.

In one embodiment, L_{1c} is selected from: the amino acid residue represented by formula (1a) and Val-the amino acid residue represented by formula (1a)-Gly.

In one embodiment, L_{1c} is selected from Val-the amino acid residue represented by formula (1a)-Gly.

In one embodiment, in formula (III-2), the left side of the fragment is attached to -C(=O)CH₃, and the right side is attached to L_{1d}.

In one embodiment, in formula (III-1), L_{1d} is a bond, or L_{1d} is selected from the group consisting of: 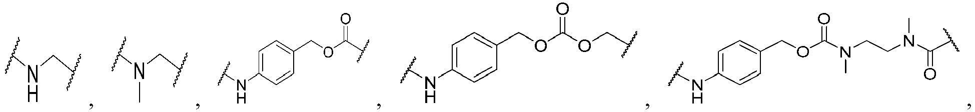 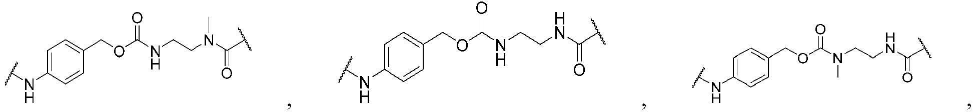 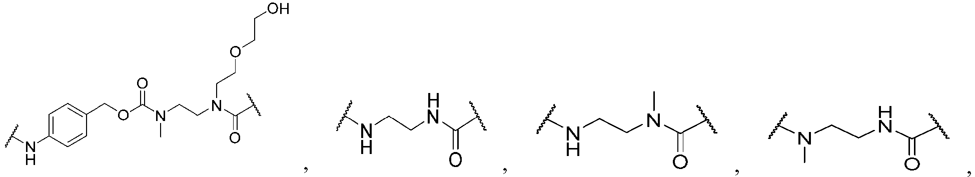 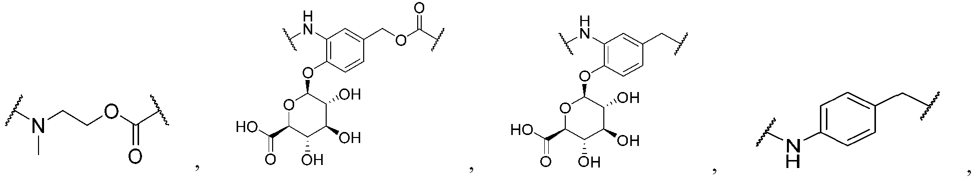 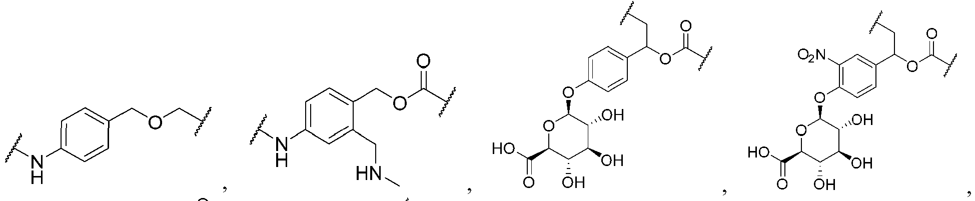 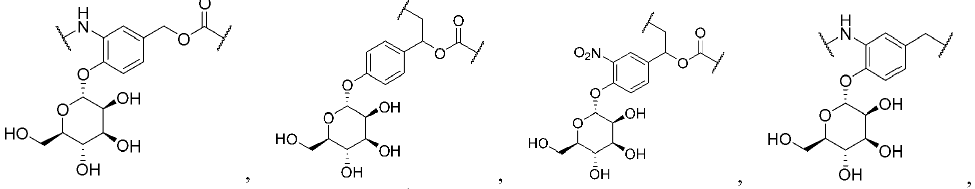 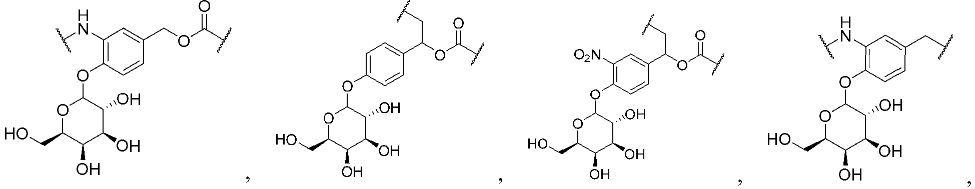 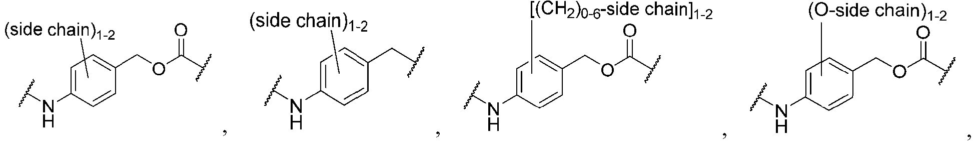 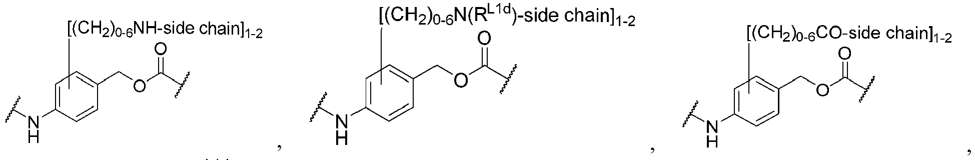 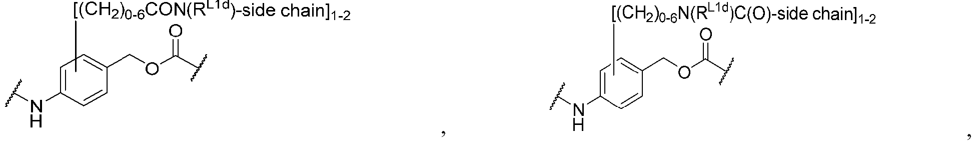 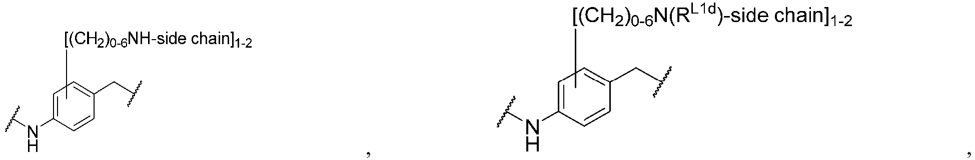 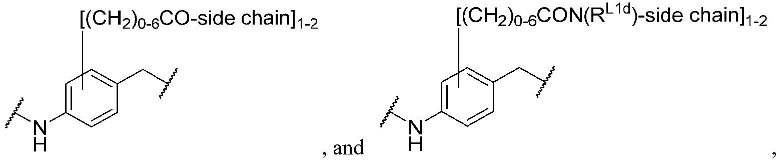
the benzene ring in the fragment described above is optionally substituted with one or more of the following groups: -C₁₋₄ alkylene-NH₂, -C₁₋₄ alkylene-NHC₁₋₄ alkyl, -C₁₋₄ alkylene-N(C₁₋₄ alkyl)₂, and -C₁₋₄ alkylene-NH-C₁₋₄ alkylene-COOH;
R^{L1d} in the fragment described above is hydrogen or C₁₋₄ alkyl;
the side chain in the fragment described above is a hydrophilic group, wherein the hydrophilic group is as defined in any one of the embodiments;
the left side of the fragment described above is attached to L_{1c}, and the right side is attached to D.

In one embodiment, in formula (III-1), the benzene ring in the L_{1d} group is optionally substituted with one or more of the following groups: -C₁₋₄ alkylene-NHC₁₋₄ alkyl and -C₁₋₄ alkylene-N(C₁₋₄ alkyl)₂.

In one embodiment, in formula (III-1), the benzene ring in the L_{1d} group is optionally substituted with 1, 2, or 3 of the following groups: -CH₂NHCH₃ and -CH₂NH(CH₃)₂.

In one embodiment, in formula (III-1), R^{L1d} is hydrogen, methyl, ethyl, or isopropyl.

In one embodiment, in formula (III-1), R^{L1d} is methyl.

In one embodiment, in formula (III-1), L_{1d} is a bond, or L_{1d} is selected from the group consisting of: 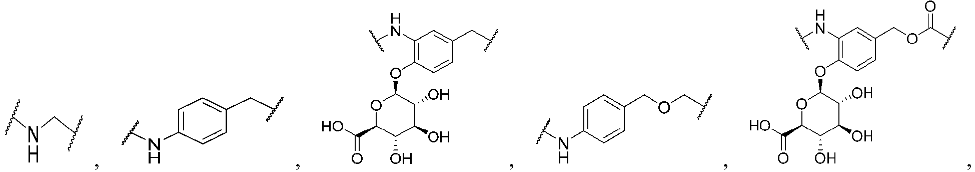 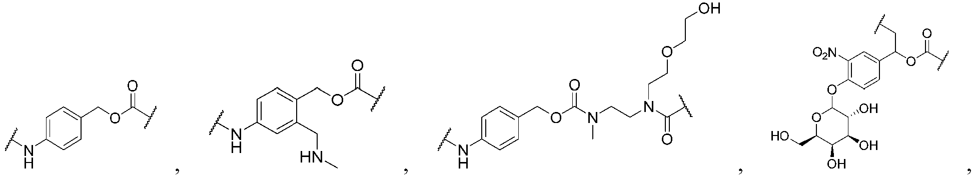 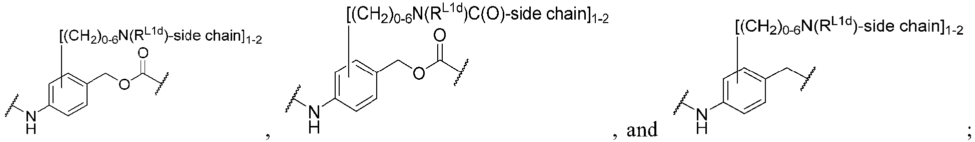 the side chain is a hydrophilic group, wherein the hydrophilic group is as defined in any one of the embodiments; the left side of the fragment is attached to L_{1c}, and the right side is attached to D; R^{L1d} is as defined in any one of the embodiments.

In one embodiment, in formula (III-1), L_{1d} is a bond or selected from the group consisting of: 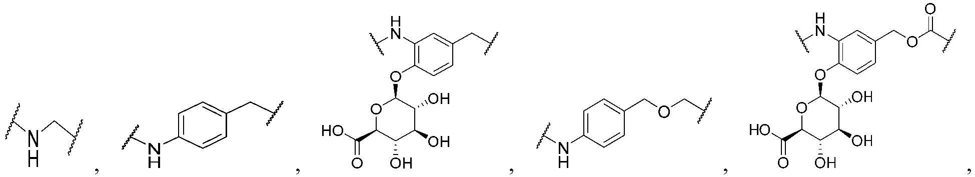 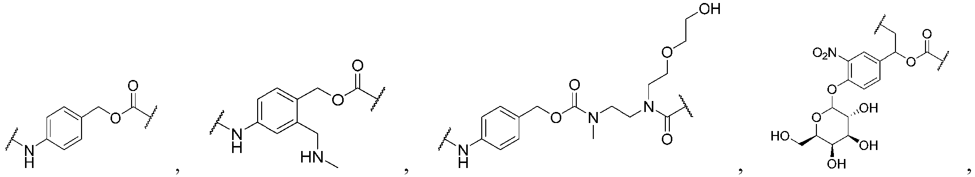 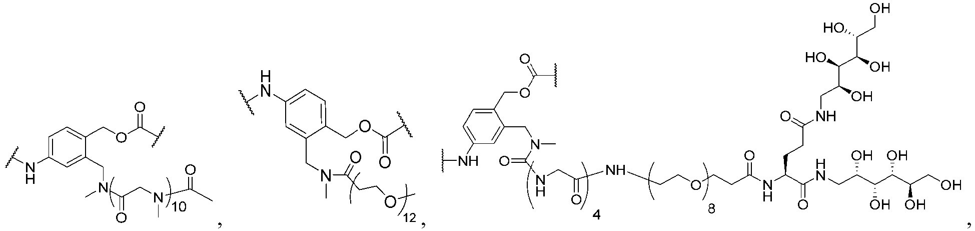 and 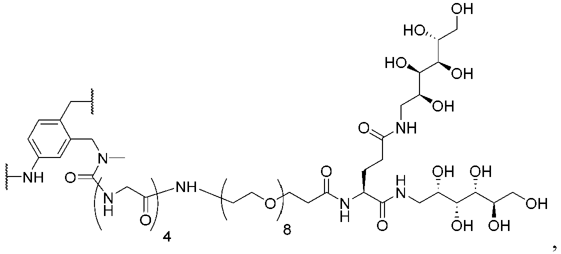 wherein the left side of the fragment is attached to L_{1c}, and the right side is attached to D.

In one embodiment, in formula (III-1), L_{1d} is a bond or selected from the group consisting of: 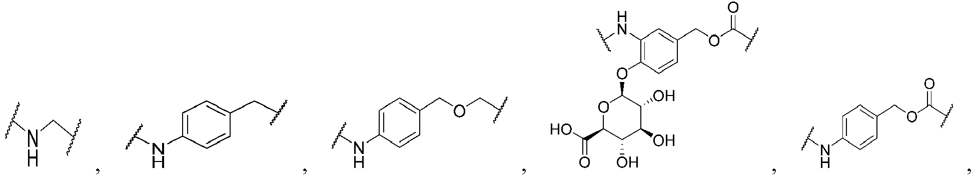 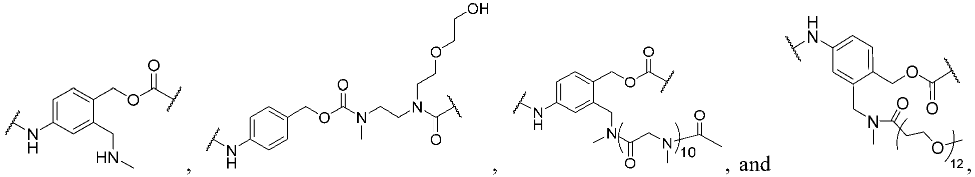 wherein the left side of the fragment is attached to L_{1c}, and the right side is attached to D. In one embodiment, in formula (III-1), L_{1d} is:

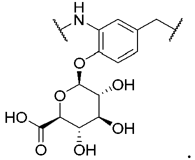

In one embodiment, in formula (III-1), L_{1d} is a bond or selected from the group consisting of: 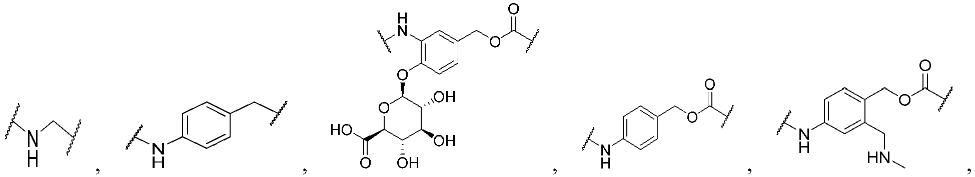 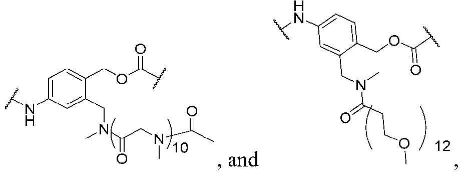 wherein the left side of the fragment is attached to L_{1c}, and the right side is attached to D.

In one embodiment, in formula (III-2), L_{1d} is a bond, or L_{1d} is selected from the group consisting of: 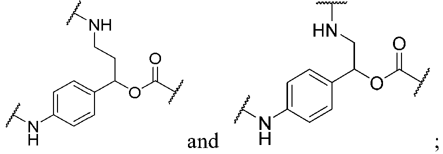
the benzene ring in the group described above is optionally substituted with one or more of the following groups: -C₁₋₄ alkylene-NH₂, -C₁₋₄ alkylene-NHC₁₋₄ alkyl, -C₁₋₄ alkylene-N(C₁₋₄ alkyl)₂, and -C₁₋₄ alkylene-NH-C₁₋₄ alkylene-COOH;
the upper side of the fragment described above is attached to L_{1b}, the left side is attached to L_{1c}, and the right side is attached to D.

In one embodiment, in formula (III-2), the benzene ring in the L_{1d} group is optionally substituted with one or more of the following groups: -C₁₋₄ alkylene-NHC₁₋₄ alkyl and -C₁₋₄ alkylene-N(C₁₋₄ alkyl)₂.

In one embodiment, in formula (III-2), the benzene ring in the L_{1d} group is optionally substituted with 1, 2, or 3 of the following groups: -CH₂NHCH₃ and -CH₂NH(CH₃)₂.

In one embodiment, in formula (III-2), L_{1d} is

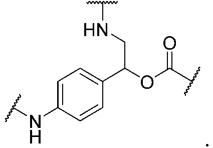

In one embodiment, the antibody-drug conjugate represented by formula (I) has a structure represented by formula (IV-1): wherein: 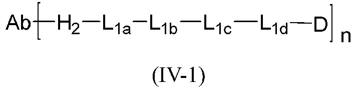
n is 1-20 and may be an integer or non-integer;
-D is as defined in any one of the embodiments;
wherein Ab is an antibody or a fragment thereof that binds to a target;
H₂ is selected from the group consisting of: 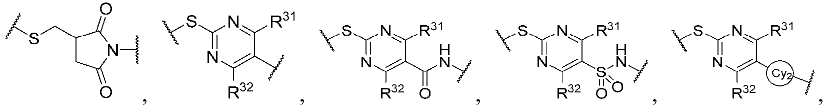 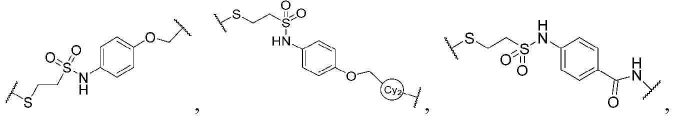 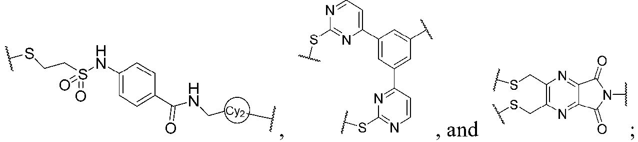 wherein:
   R³¹ is hydrogen, deuterium, halogen, cyano, or C₁₋₄ alkyl;
   R³² is hydrogen, deuterium, halogen, cyano, or C₁₋₄ alkyl;
   Cy2 is 5- to 10-membered heteroaryl, C₆₋₁₀ aryl, 3- to 10-membered heterocyclyl, or C₃₋₁₀ cycloalkyl;
   L₁ₐ is a bond or a fragment linking Y to L_{1b}, wherein the fragment is selected from C₁₋₆ alkyl, C₁₋₆ heteroalkyl, C₂₋₆ alkenyl, C₂₋₆ alkynyl, C₃₋₈ cycloalkyl, 4- to 10-membered heterocyclyl, C₆₋₁₀ aryl, 5- to 10-membered heteroaryl, C₁₋₄ alkyl-C₃₋₈ cycloalkyl, C₁₋₄ alkyl-4- to 10-membered heterocyclyl, C₁₋₄ alkyl-C₆₋₁₀ aryl, and C₁₋₄ alkyl-5- to 10-membered heteroaryl;
   L_{1b} is a bond or a fragment linking L₁ₐ to L_{1c}, wherein the fragment is selected from: 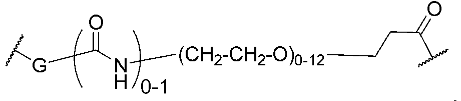 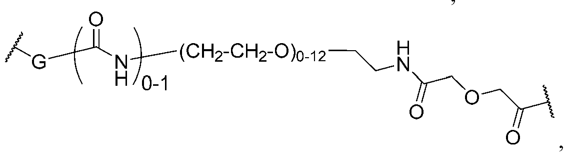 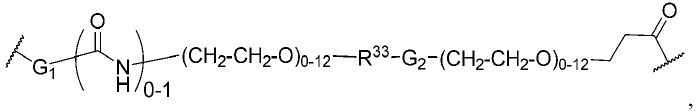 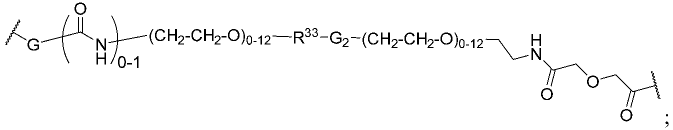
   wherein the left side of the fragment described above is attached to L₁ₐ, and the right side is attached to L_{1c};
   G is C₀₋₈ hydrocarbyl or C₀₋₈ heteroalkyl;
   G₁ and G₂ are each independently C₀₋₈ hydrocarbyl or C₀₋₈ heteroalkyl;
   R³³ is a 3- to 12-membered heterocyclic ring, a 5- to 10-membered heteroaromatic ring, NR³⁴, -CONR³⁴-, or - NR³⁴CO-;
   R³⁴ is hydrogen, -(CH₂CH₂O)₀₋₁₂-H, -(CH₂CH₂O)₀₋₁₂-CH₃, -CO-(CH₂CH₂O)₀₋₁₂-H, -CO-(CH₂CH₂O)₀₋₁₂-CH₃, or CH₂-CO-(N(Me)-CH₂-CO)₀₋₂₄-OH;
   L_{1c} is a bond or a fragment linking L_{1b} to L_{1d}, wherein the fragment is an amino acid residue or a short peptide consisting of 2-10 amino acid residues;
   L_{1d} is a bond or a fragment linking L_{1c} to D, and L_{1d} is selected from the group consisting of: 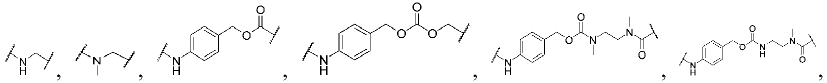 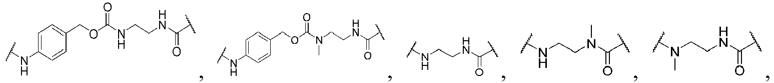 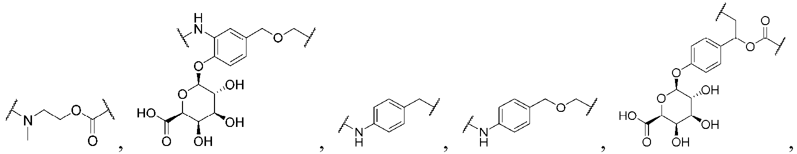 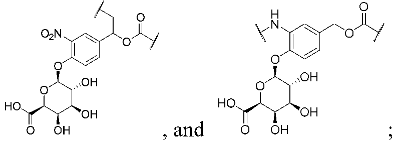
   the left side of the fragment described above is attached to L_{1c}, and the right side is attached to D.

In one embodiment, -H₂-L₁ₐ-L_{1b}-L_{1c}-L_{1d}- is selected from structures shown in Table 9,

**Table 9**

| Number | Structure |
|---|---|
| L1 | 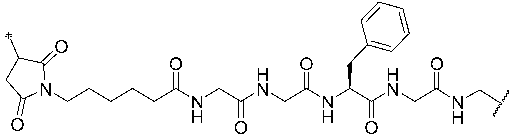 |
| L2 | 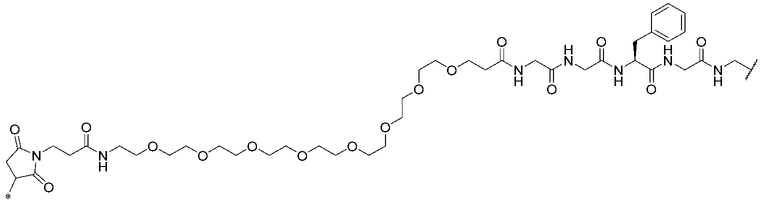 |
| L3 | 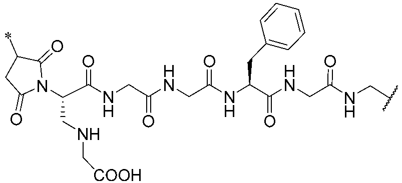 |
| L4 | 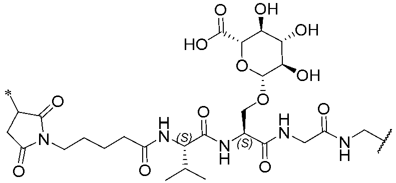 |
| L5 | 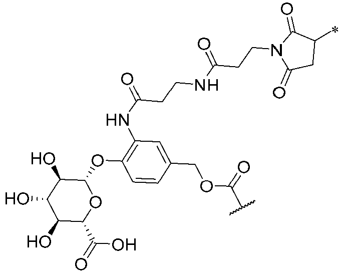 |
| L6 | 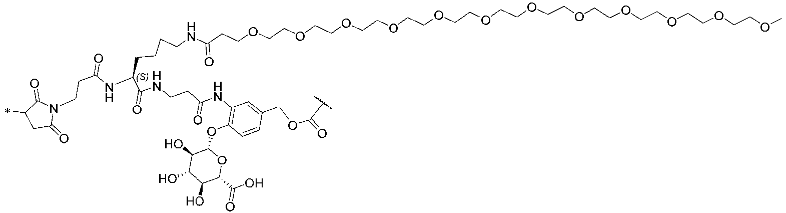 |
| L7 | 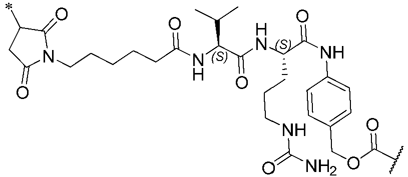 |
| L8 | 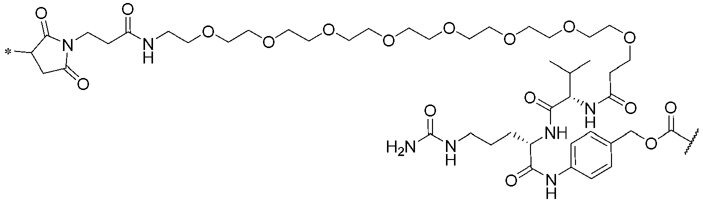 |
| L9 | 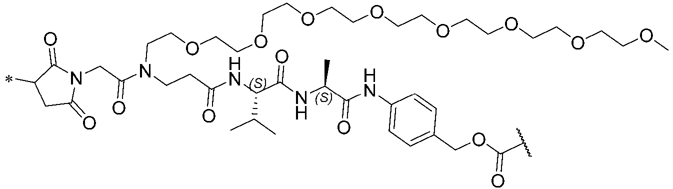 |
| L10 | 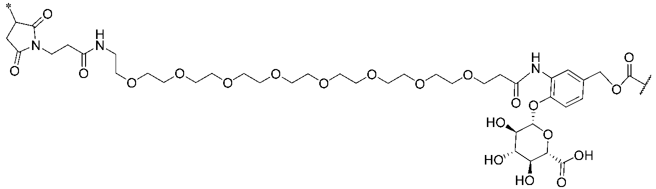 |
| L11 | 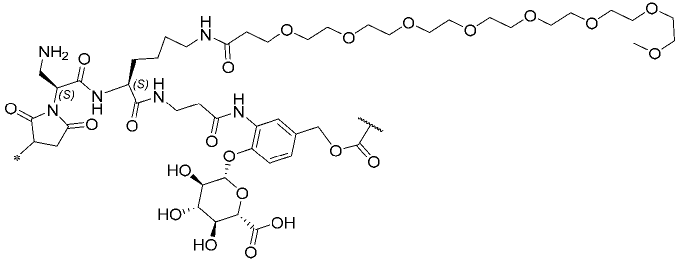 |
| L12 | 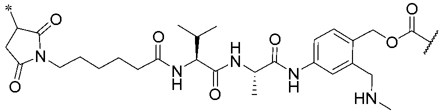 |
| L13 | 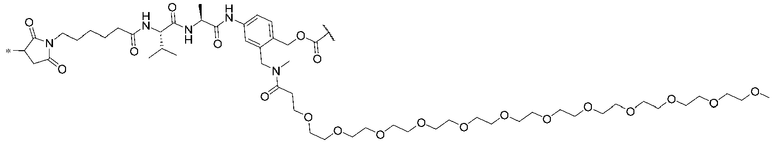 |
| L14 | 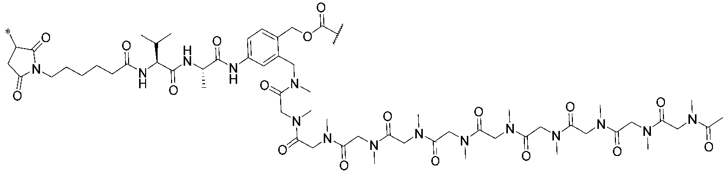 |
| L15 | 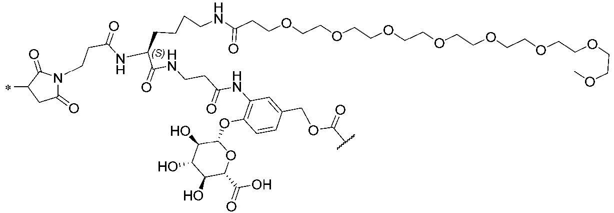 |
| L16 | 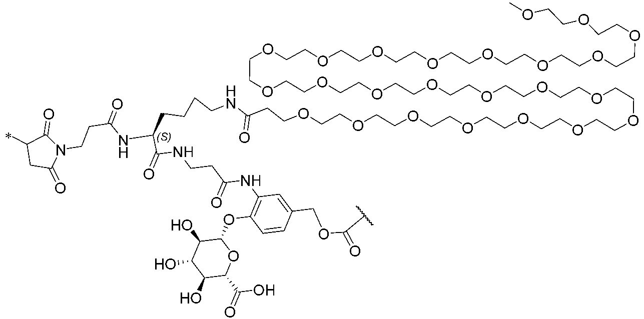 |
| L17 | 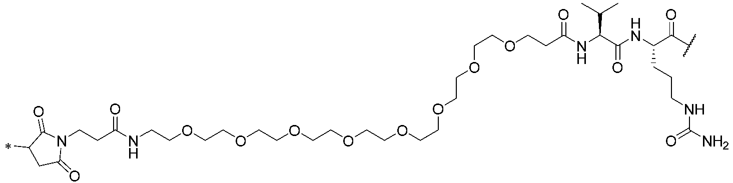 |
| L18 | 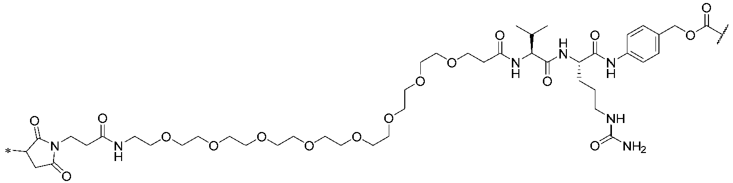 |
| L19 | 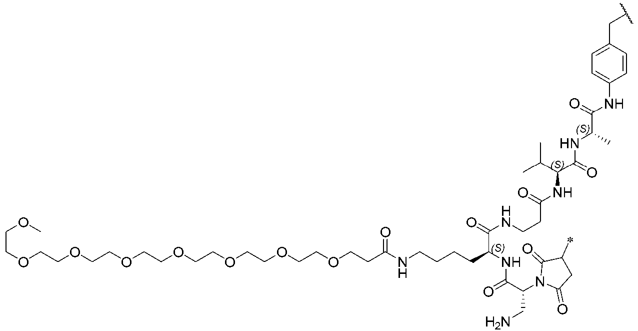 |
| L20 | 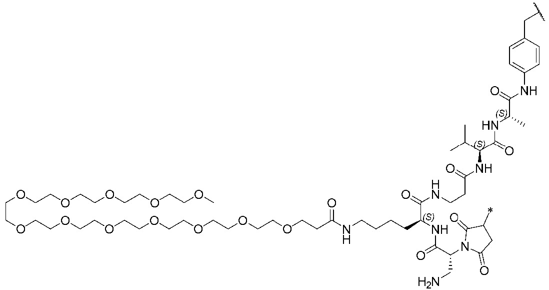 |
| L21 | 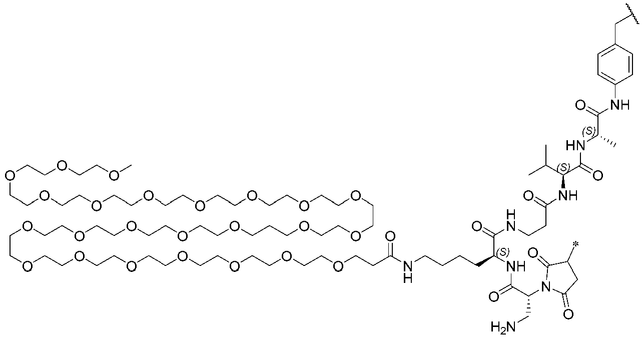 |
| L22 | 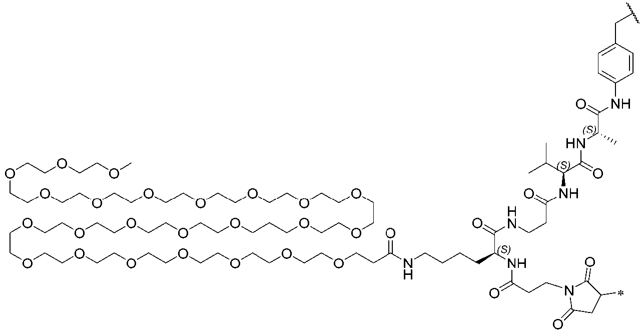 |
| L23 | 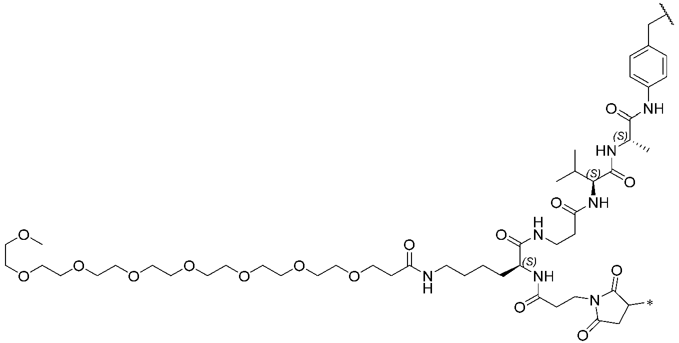 |
| L24 | 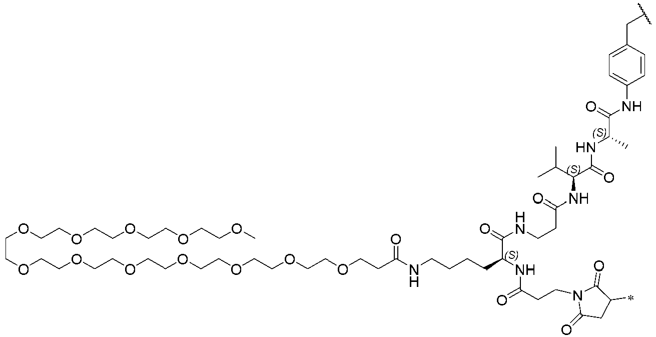 |
| L25 | 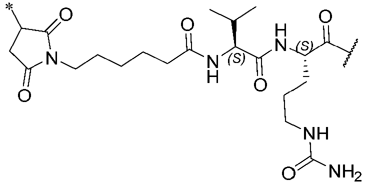 |
| L26 | 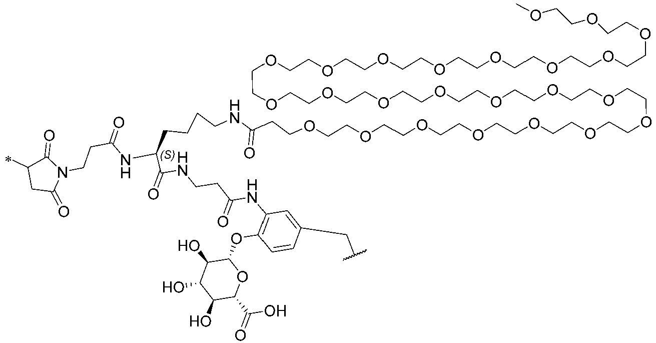 |
| L27 | 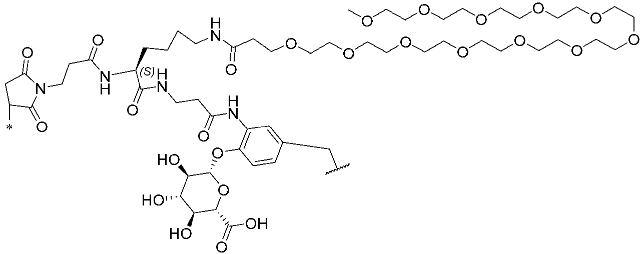 |
| L28 | 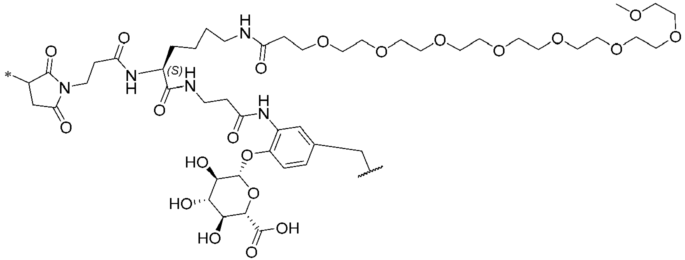 |
| L29 | 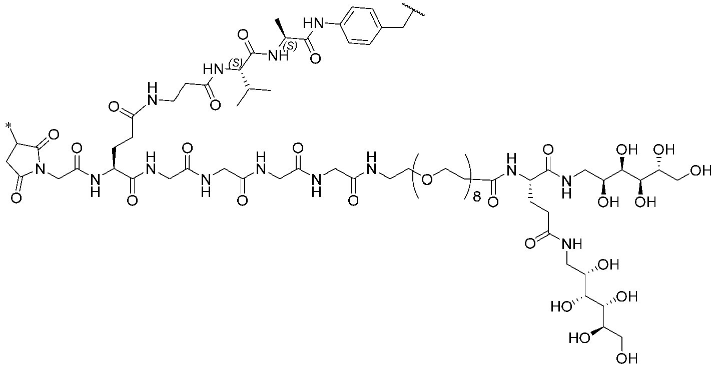 |
| L30 | 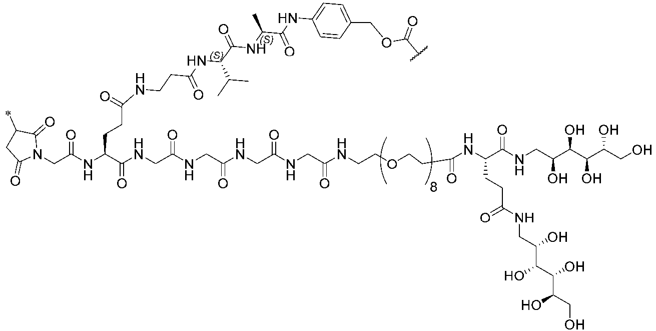 |
| L31 | 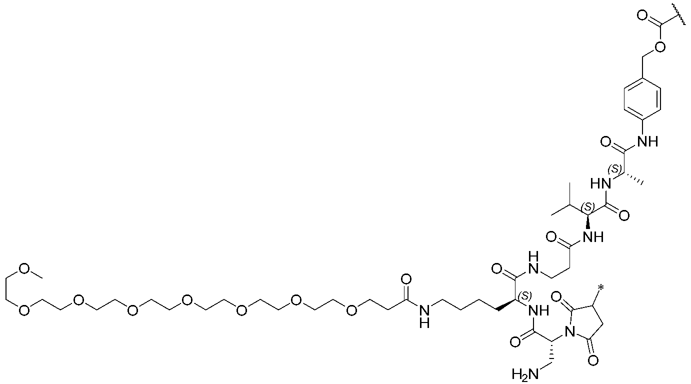 |
| L32 | 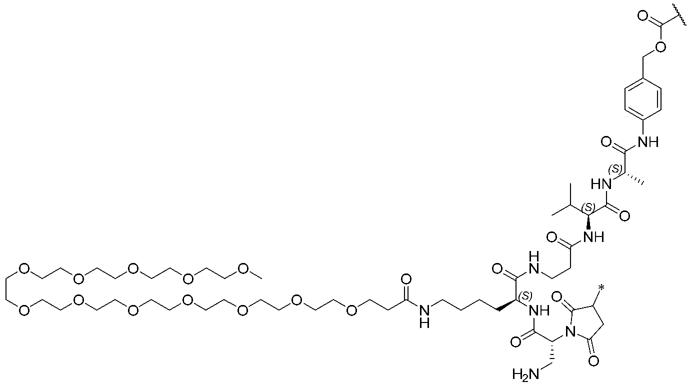 |
| L33 | 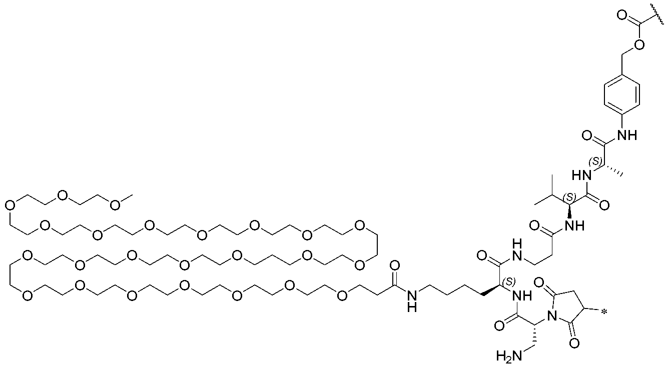 |
| L34 | 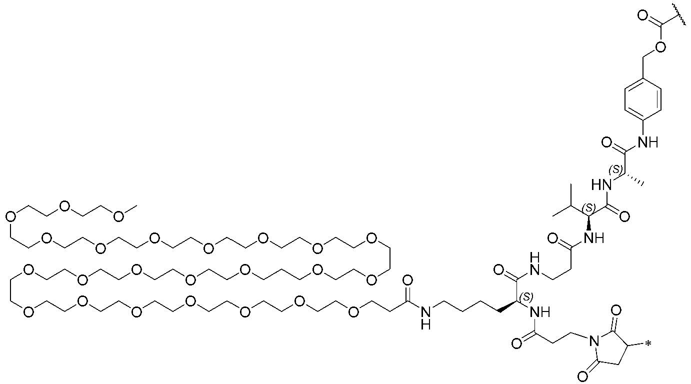 |
| L35 | 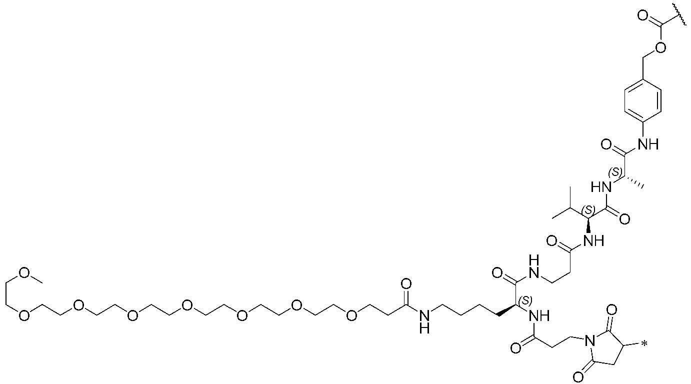 |
| L36 | 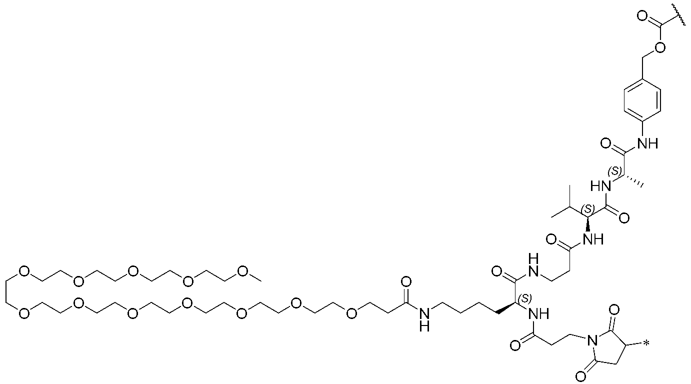 |
| L37 | 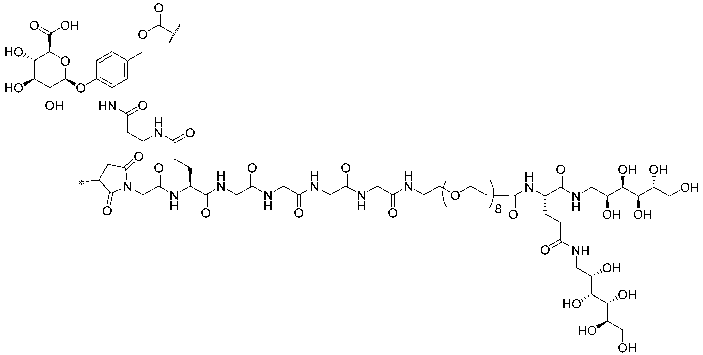 |
| L38 | 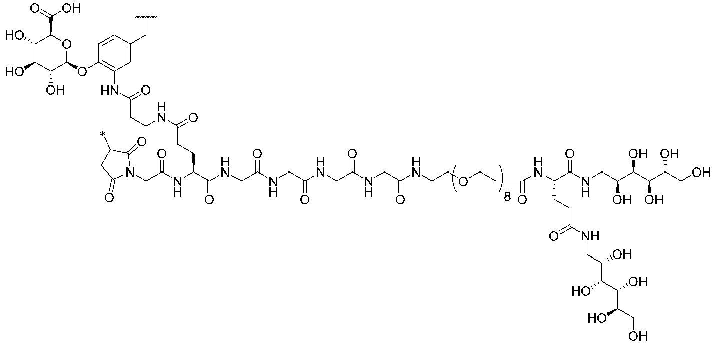 |
| L39 | 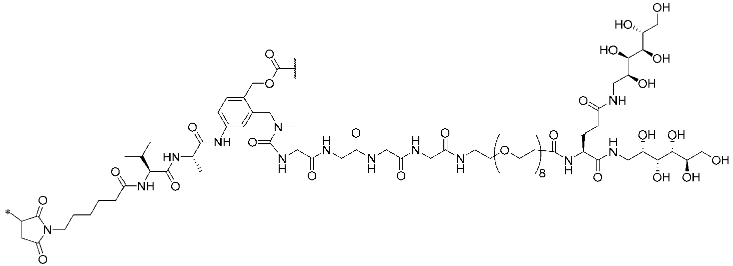 |
| L40 | 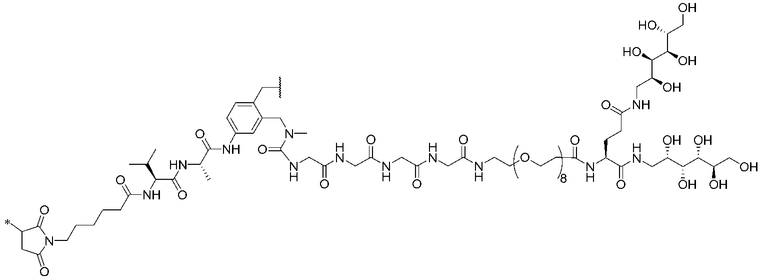 |
| L45 | 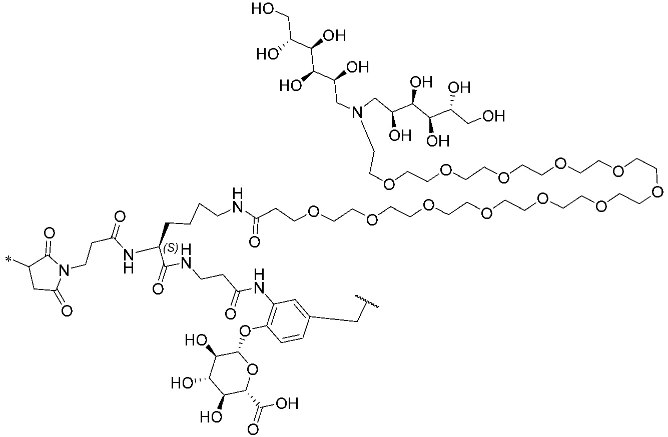 |
| L46 | 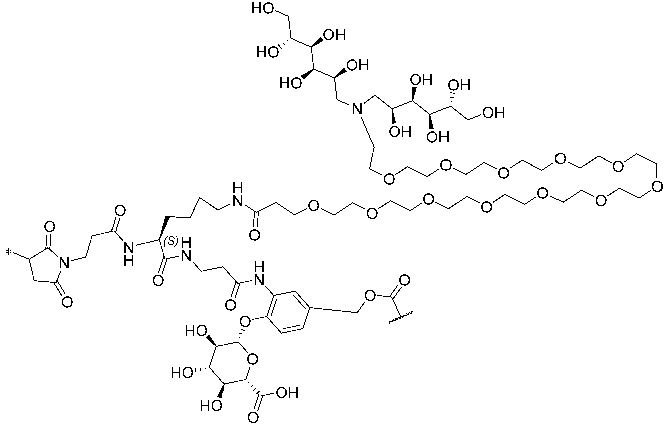 |
| L49 | 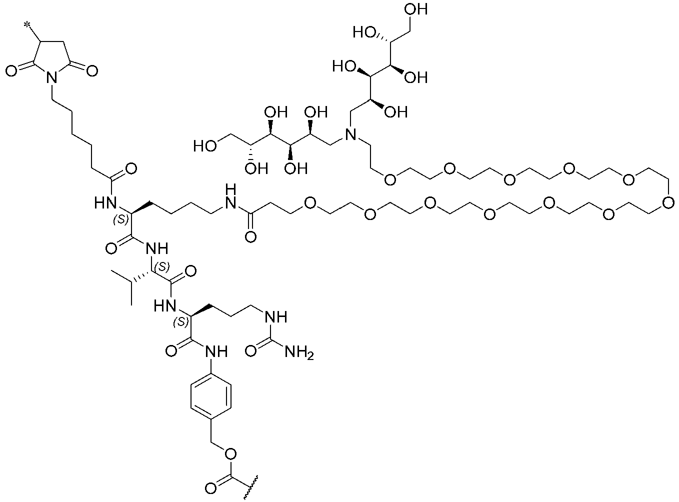 |
| L50 | 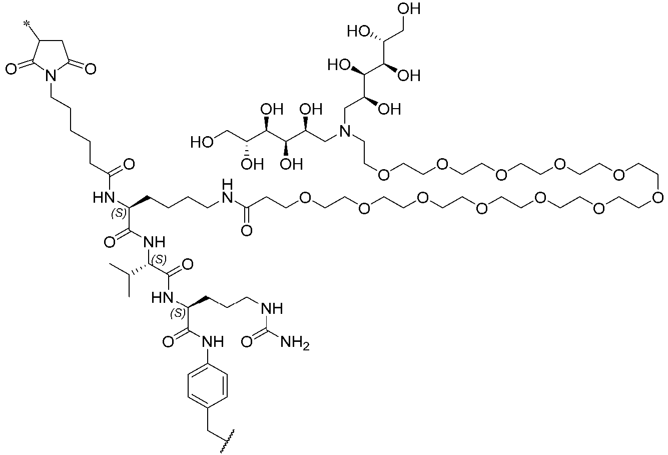 |
| L51 | 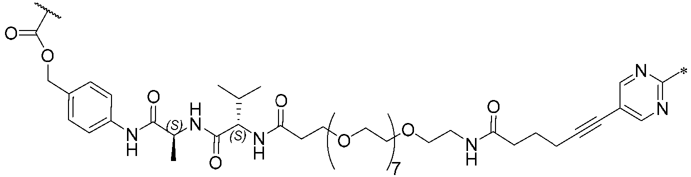 |
| L52 | 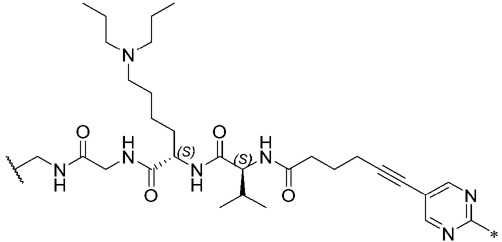 |
| L53 | 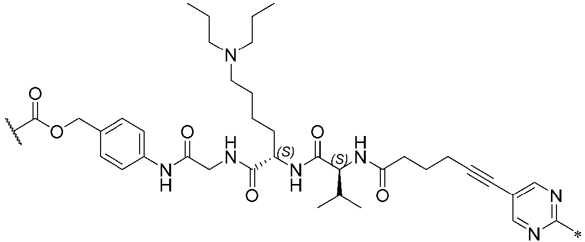 |
| L54 | 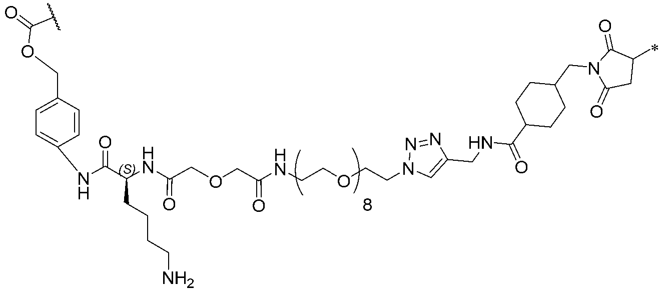 |
| L54-2 | 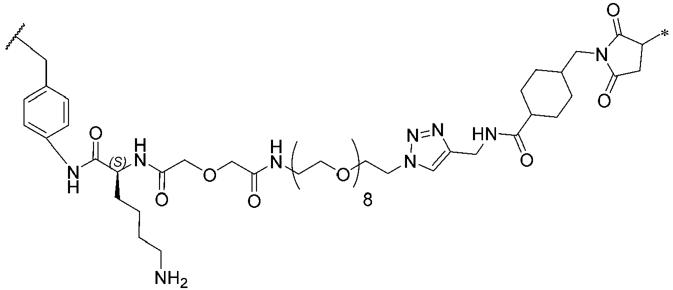 |
| L55 | 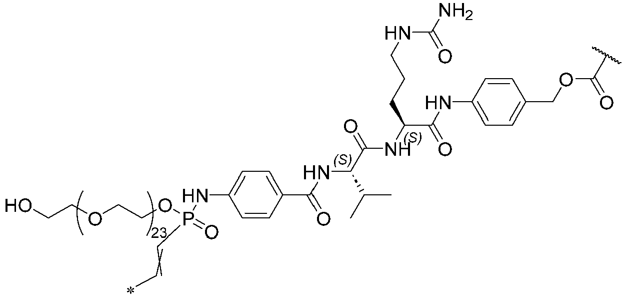 |
| L56 | 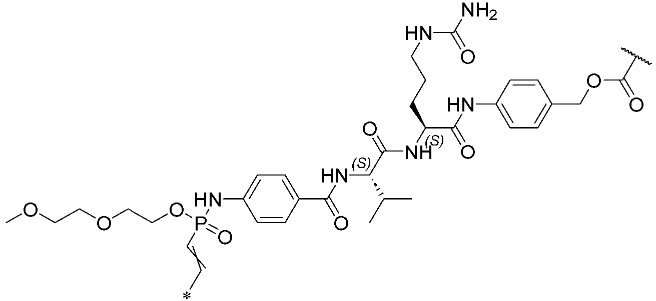 |
| L57 | 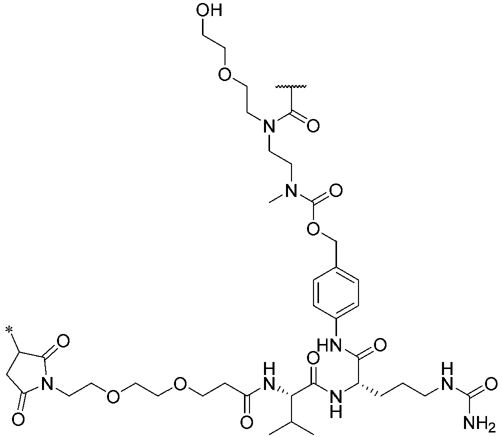 |
| L58 | 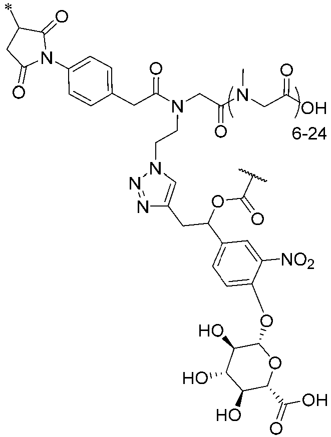 |
| L61 | 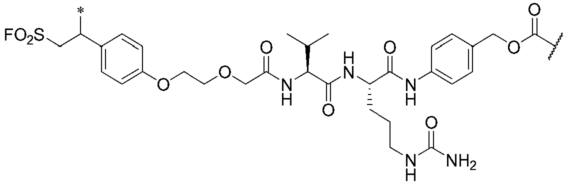 |
| L62 | 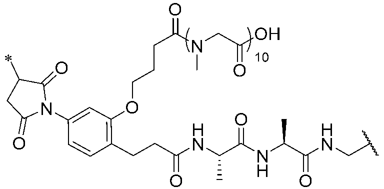 |
| L63 | 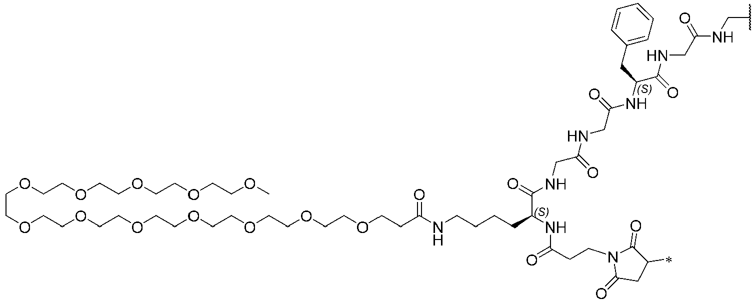 |
| L64 | 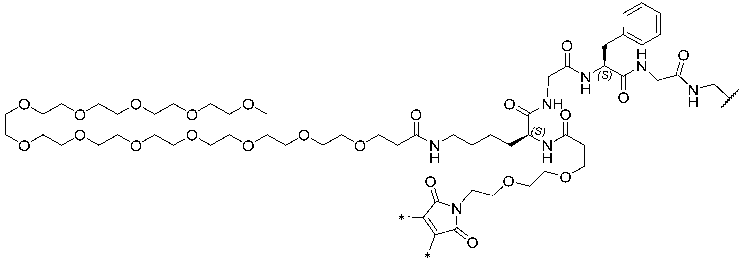 |
| L65 | 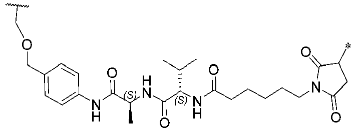 |
| L66 | 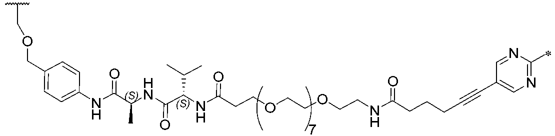 |
| L67 | 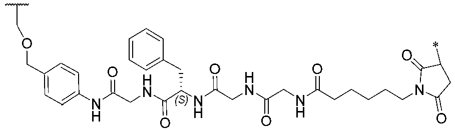 |
| L68 | 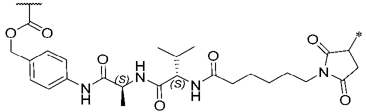 |
| L69 | 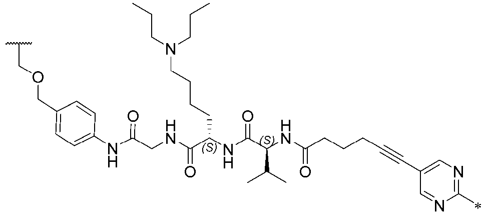 |
| L70 | 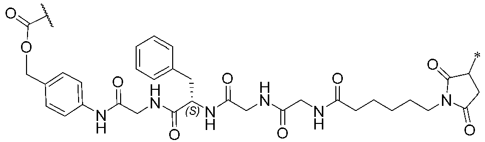 |
| L71 | 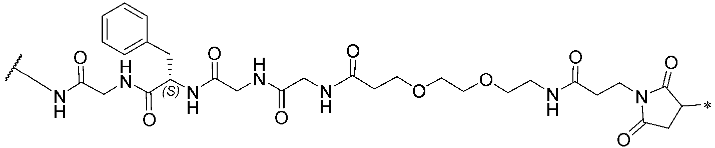 |
| L72 | 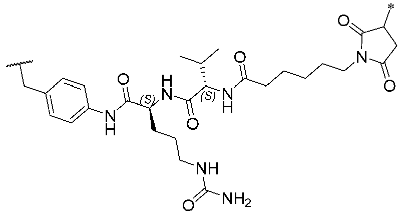 |
| L73 | 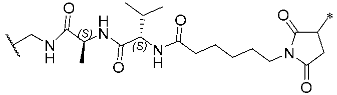 |
| L74 | 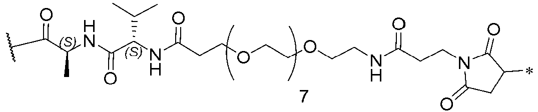 |

In one embodiment, -H₂-L₁ₐ-L_{1d}-L_{1c}-L_{1d}- is selected from L1, L2, L3, L4, L5, L6, L7, L8, L9, L10, L11, L12, L13, L14, L15, L16, L17, L19, L22, L23, L24, L25, L26, L34, L52, L53, L54, L54-2, L57, L58, L63, L64, L65, L66, L67, L68, L69, L70, L72, L73, and L74.

In one embodiment, -H₂-L₁ₐ-L_{1d}-L_{1c}-L_{1d}- is selected from L1, L2, L3, L4, L5, L6, L7, L8, L9, L10, L11, L12, L13, L14, L15, L16, L17, L19, L22, L23, L24, L25, L26, L34, L52, L53, L54, L57, L63, L64, L65, L66, L67, L68, L69, L70, L72, L73, and L74.

In one embodiment, -H₂-L₁ₐ-L_{1b}-L_{1c}-L_{1d}- is selected from L1, L2, L4, L5, L6, L7, L8, L9, L10, L11, L12, L13, L14, L15, L16, L17, L23, L24, and L25.

In one embodiment, -H₂-L₁ₐ-L_{1d}-L_{1c}-L_{1d}- is selected from L1, L4, L5, L6, L7, L10, L12, L13, L14, L15, L16, and L23.

In one embodiment, -H₂-L₁ₐ-L_{1d}-L_{1d}(-L_{1c}-C(=O)-CH₃)- is selected from structures shown in Table 10,

**Table 10**

| Number | Structure | Number | Structure |
|---|---|---|---|
| L59 | 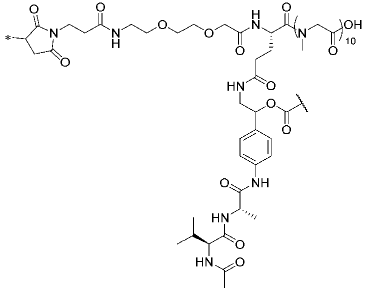 | L60 | 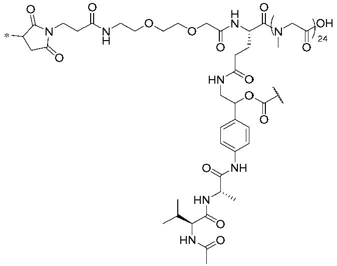 |

In one embodiment, the antibody-drug conjugate represented by formula (I) has a structure represented by formula (IV-1) or formula (IV-2): 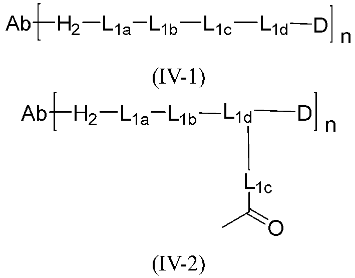 wherein:
Ab, n, H₂, L₁ₐ, L_{1b}, L_{1c}, L_{1d}, and D are as defined in any one of the embodiments.

In one embodiment, the antibody-drug conjugate represented by formula (I) has a structure represented by formula (IV-1).

In one embodiment, Ab is an antibody or an antigen-binding fragment thereof that binds to one, two, or more targets selected from the group consisting of: HER2, Trop2, EGFR, Claudin18.2, Nectin-4, FRa, TF, CD49B, Claudin7, HER3, Notch1, MUC5AC, IGF1R, cMET, PD-L1, B7-H3, B7-H4, CDCP1, MUC16, MUC1, MSLN, FAP, 5T4, CEACAM5, LIV, NaPi2b, AXL, LY6E, CDH6, CDH17, ITGB6, Claudin6, CEACAM6, LY6G6D, LGR5, PTK7, CD228, and SLC34A2.

In one embodiment, Ab is an antibody that binds to a target selected from the group consisting of: HER2, Trop2, EGFR, Claudin18.2, Nectin-4, FRa, TF, CD49B, Claudin7, HER3, Notch1, MUC5AC, IGF1R, cMET, PD-L1, B7-H3, B7-H4, CDCP1, MUC16, MUC1, MSLN, FAP, 5T4, CEACAM5, LIV, NaPi2b, AXL, and LY6E.

In one embodiment, Ab is selected from the group consisting of: anti-HER2 antibody Trastuzumab, anti-Trop2 antibody hRS7, anti-EGFR antibody Panitumumab, anti-Claudin18.2 antibody Claudiximab, anti-Nectin-4 antibody Enfortumab, anti-FRα antibody Milvetuximab, anti-TF antibody Tisotumab, anti-CD49B antibody Vatelizumab, anti-Claudin7 antibody 6-G3, anti-HER3 antibody Patritumab, anti-Notch1 antibody Brontictuzumab, anti-MUC5AC antibody Clivatuzumab, anti-IGF1R antibody Cixutumumab, anti-cMET antibody Telisotuzumab, anti-PD-L1 antibody Atezolizumab, anti-B7-H3 antibody Vobramitamab, anti-B7-H4 antibody FPA-150, anti-CDCP1 antibody CUB4, anti-MUC16 antibody Sofituzumab, anti-MUC1 antibody Pankomab, anti-MSLN antibody Anetumab, anti-FAP antibody Sibrotuzumab, anti-5T4 antibody Naptumomab, anti-CEACAM5 antibody Labetuzumab, anti-LIV antibody Ladiratuzumab, anti-NaPi2b antibody Lifastuzumab, anti-AXL antibody Enapotamab, and anti-LY6E antibody 9B12.

In one embodiment, Ab is selected from the group consisting of: an anti-EGFR antibody or an antigen-binding fragment thereof, an anti-HER3 antibody or an antigen-binding fragment thereof, and a bispecific antibody that specifically binds to EGFR and HER3 or an antigen-binding fragment thereof.

In one embodiment, the anti-EGFR antibody or the antigen-binding fragment thereof comprises:
(a) CDR1, CDR2, and CDR3 sequences contained in a VH set forth in SEQ ID NO: 3 or a variant thereof, and/or
(b) CDR1, CDR2, and CDR3 sequences contained in a VL set forth in SEQ ID NO: 7 or a variant thereof,
wherein the variant has one or several amino acid substitutions, deletions, or additions (e.g., 1, 2, or 3 amino acid substitutions, deletions, or additions) compared to the sequence from which the variant is derived.

In one embodiment, the substitution is a conservative substitution.

In one embodiment, the anti-EGFR antibody or the antigen-binding fragment thereof comprises:
(a) VH CDRs of a CDR1 set forth in SEQ ID NO: 4 or a variant thereof, a CDR2 set forth in SEQ ID NO: 5 or a variant thereof, and a CDR3 set forth in SEQ ID NO: 6 or a variant thereof, and/or
(b) VL CDRs of a CDR1 set forth in SEQ ID NO: 8 or a variant thereof, a CDR2 set forth in SEQ ID NO: 9 or a variant thereof, and a CDR3 set forth in SEQ ID NO: 10 or a variant thereof,
wherein the variant has one or several amino acid substitutions, deletions, or additions (e.g., 1, 2, or 3 amino acid substitutions, deletions, or additions) compared to the sequence from which the variant is derived.

In one embodiment, the substitution is a conservative substitution.

In one embodiment, the VH of the anti-EGFR antibody or the antigen-binding fragment thereof comprises: a VH CDR1 set forth in SEQ ID NO: 4, a VH CDR2 set forth in SEQ ID NO: 5, and a VH CDR3 set forth in SEQ ID NO: 6; and/or the VL of the anti-EGFR antibody or the antigen-binding fragment thereof comprises: a VL CDR1 set forth in SEQ ID NO: 8, a VL CDR2 set forth in SEQ ID NO: 9, and a VL CDR3 set forth in SEQ ID NO: 10.

In one embodiment, the VH of the anti-EGFR antibody or the antigen-binding fragment thereof comprises: a VH CDR1 set forth in SEQ ID NO: 4, a VH CDR2 set forth in SEQ ID NO: 5, and a VH CDR3 set forth in SEQ ID NO: 6; and the VL of the anti-EGFR antibody or the antigen-binding fragment thereof comprises: a VL CDR1 set forth in SEQ ID NO: 8, a VL CDR2 set forth in SEQ ID NO: 9, and a VL CDR3 set forth in SEQ ID NO: 10. In one embodiment, the amino acid sequence of the heavy chain variable region of the anti-EGFR antibody or the antigen-binding fragment thereof is set forth in SEQ ID NO: 3, and the amino acid sequence of the light chain variable region of the anti-EGFR antibody or the antigen-binding fragment thereof is set forth in SEQ ID NO: 7.

In one embodiment, the amino acid sequence of the heavy chain of the anti-EGFR antibody is set forth in SEQ ID NO: 1, and the amino acid sequence of the light chain of the anti-EGFR antibody is set forth in SEQ ID NO: 2.

In one embodiment, the anti-EGFR antibody or the antigen-binding fragment thereof is Panitumumab, Necitumumab, Nimotuzumab, EG-007, Laprituximab, Cetuximab, or JMT101.

In one embodiment, the anti-HER3 antibody or the antigen-binding fragment thereof comprises:
(a) CDR1, CDR2, and CDR3 sequences contained in a VH set forth in SEQ ID NO: 13 or a variant thereof, and/or
(b) CDR1, CDR2, and CDR3 sequences contained in a VL set forth in SEQ ID NO: 17 or a variant thereof, wherein the variant has one or several amino acid substitutions, deletions, or additions (e.g., 1, 2, or 3 amino acid substitutions, deletions, or additions) compared to the sequence from which the variant is derived.

In one embodiment, the substitution is a conservative substitution.

In one embodiment, the anti-HER3 antibody or the antigen-binding fragment thereof comprises:
(a) VH CDRs of a CDR1 set forth in SEQ ID NO: 14 or a variant thereof, a CDR2 set forth in SEQ ID NO: 15 or a variant thereof, and a CDR3 set forth in SEQ ID NO: 16 or a variant thereof, and/or
(b) VL CDRs of a CDR1 set forth in SEQ ID NO: 18 or a variant thereof, a CDR2 set forth in SEQ ID NO: 19 or a variant thereof, and a CDR3 set forth in SEQ ID NO: 20 or a variant thereof,
wherein the variant has one or several amino acid substitutions, deletions, or additions (e.g., 1, 2, or 3 amino acid substitutions, deletions, or additions) compared to the sequence from which the variant is derived.

In one embodiment, the substitution is a conservative substitution.

In one embodiment, the VH of the anti-HER3 antibody or the antigen-binding fragment thereof comprises: a VH CDR1 set forth in SEQ ID NO: 14, a VH CDR2 set forth in SEQ ID NO: 15, and a VH CDR3 set forth in SEQ ID NO: 16; and/or the VL of the anti-HER3 antibody or the antigen-binding fragment thereof comprises: a VL CDR1 set forth in SEQ ID NO: 18, a VL CDR2 set forth in SEQ ID NO: 19, and a VL CDR3 set forth in SEQ ID NO: 20.

In one embodiment, the VH of the anti-HER3 antibody or the antigen-binding fragment thereof comprises: a VH CDR1 set forth in SEQ ID NO: 14, a VH CDR2 set forth in SEQ ID NO: 15, and a VH CDR3 set forth in SEQ ID NO: 16; and the VL of the anti-HER3 antibody or the antigen-binding fragment thereof comprises: a VL CDR1 set forth in SEQ ID NO: 18, a VL CDR2 set forth in SEQ ID NO: 19, and a VL CDR3 set forth in SEQ ID NO: 20.

In one embodiment, the amino acid sequence of the heavy chain variable region of the anti-HER3 antibody or the antigen-binding fragment thereof is set forth in SEQ ID NO: 13, and the amino acid sequence of the light chain variable region of the anti-HER3 antibody or the antigen-binding fragment thereof is set forth in SEQ ID NO: 17.

In one embodiment, the amino acid sequence of the heavy chain of the anti-HER3 antibody is set forth in SEQ ID NO: 11, and the amino acid sequence of the light chain of the anti-HER3 antibody is set forth in SEQ ID NO: 12.

In one embodiment, the anti-HER3 antibody or the antigen-binding fragment thereof is Seribantumab, Elgemtumab, Lumretuzumab, AV203, Barecetamab, CDX-3379, GSK2849330, HMBD-001, REGN1400, or Patritumab.

In one embodiment, the bispecific antibody or the antigen-binding fragment thereof that specifically binds to EGFR and HER3 comprises a first antigen-binding domain that specifically binds to EGFR and a second antigen-binding domain that specifically binds to HER3.

In one embodiment, the first antigen-binding domain comprises a first heavy chain variable region and a first light chain variable region, wherein the first heavy chain variable region and the first light chain variable region together form a domain capable of specifically binding to EGFR.

In one embodiment, the first heavy chain variable region comprises VH CDRs of a CDR1 set forth in SEQ ID NO: 24, a CDR2 set forth in SEQ ID NO: 25, and a CDR3 set forth in SEQ ID NO: 26.

In one embodiment, the first light chain variable region comprises VL CDRs of a CDR1 set forth in SEQ ID NO: 28, a CDR2 set forth in SEQ ID NO: 29, and a CDR3 set forth in SEQ ID NO: 30.

In one embodiment, the second antigen-binding domain comprises a second heavy chain variable region and a second light chain variable region, wherein the second heavy chain variable region and the second light chain variable region together form a domain capable of specifically binding to HER3.

In one embodiment, the second heavy chain variable region comprises VH CDRs of a CDR1 set forth in SEQ ID NO: 32, a CDR2 set forth in SEQ ID NO: 33, and a CDR3 set forth in SEQ ID NO: 34.

In one embodiment, the second light chain variable region comprises VL CDRs of a CDR1 set forth in SEQ ID NO: 36, a CDR2 set forth in SEQ ID NO: 37, and a CDR3 set forth in SEQ ID NO: 38.

In one embodiment, the first heavy chain variable region comprises the amino acid sequence set forth in SEQ ID NO: 23, and/or the first light chain variable region comprises the amino acid sequence set forth in SEQ ID NO: 27.

In one embodiment, the second heavy chain variable region comprises the amino acid sequence set forth in SEQ ID NO: 31, and/or the second light chain variable region comprises the amino acid sequence set forth in SEQ ID NO: 35.

In one embodiment, the second antigen-binding domain is an scFv.

In one embodiment, the bispecific antibody comprises a heavy chain and a light chain, wherein the heavy chain comprises the first heavy chain variable region, a heavy chain constant region, the second heavy chain variable region, and the second light chain variable region; the light chain comprises the first light chain variable region and a light chain constant region.

In one embodiment, the heavy chain comprises, from N-terminus to C-terminus: the first heavy chain variable region, a heavy chain constant region, the second heavy chain variable region, and the second light chain variable region; the light chain comprises, from N-terminus to C-terminus: the first light chain variable region and a light chain constant region.

In one embodiment, adjacent domains of the heavy chain are optionally attached to each other with or without a linker, and adjacent domains of the light chain are optionally attached to each other with or without a linker.

In one embodiment, the linkers are each independently identical or different peptide linkers (e.g., a rigid peptide linker or a flexible peptide linker). In one embodiment, the peptide linkers are each independently selected from a peptide linker comprising one or more glycines (G) and/or serines (S), e.g., having a structure represented by (GGGGS)ₙ, wherein n is selected from 1, 2, 3, 4, 5, 6, 7, 8, 9, and 10. In one embodiment, n is 2 or 3.

In one embodiment, the heavy chain comprises, from N-terminus to C-terminus: the first heavy chain variable region, a heavy chain constant region, a linker, the second heavy chain variable region, a linker, and the second light chain variable region.

In one embodiment, the heavy chain comprises the amino acid sequence set forth in SEQ ID NO: 21, and/or the light chain comprises the amino acid sequence set forth in SEQ ID NO: 22.

In one embodiment, the bispecific antibody is a tetravalent antibody comprising two heavy chains and two light chains.

In one embodiment, the bispecific antibody that specifically binds to EGFR and HER3 or the antigen-binding fragment thereof is SI-B001 or Duligotuzumab.

In one embodiment, Ab is attached to L₀ or H₂ via a sulfhydryl group thereof.

In one embodiment, n is a number from 1 to 15. In one embodiment, n is a number from 2 to 10. In one embodiment, n is a number from 3 to 9. In one embodiment, n is 3-4.5, 4.5-6, 6-7, 7-8, or 8-9. In one embodiment, n is about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, about 7, about 7.1, about 7.2, about 7.3, about 7.4, about 7.5, about 7.6, about 7.7, about 7.8, about 7.9, about 8, about 8.1, about 8.2, about 8.3, about 8.4, about 8.5, about 8.6, about 8.7, about 8.8, about 8.9, or about 9.

In one embodiment, the antibody-drug conjugate is represented by formula (V-1) or formula (V-1-A): 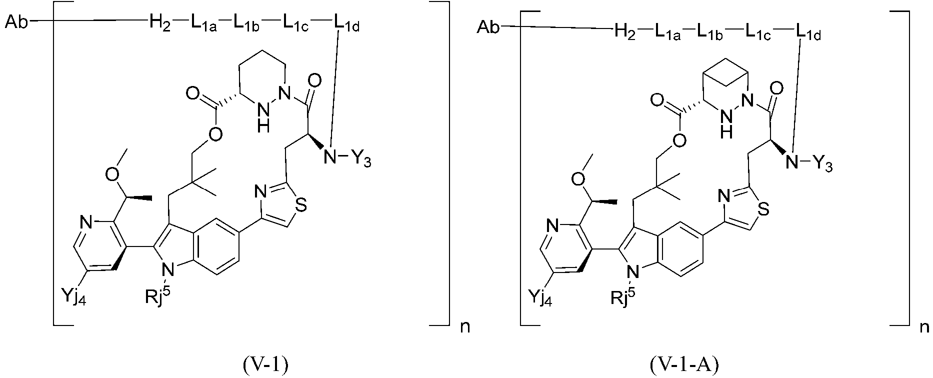 wherein H₂, L₁ₐ, L_{1b}, L_{1c}, L_{1d}, Rj⁵, Yj₄, Y₃, n, and Ab are as defined in any one of the embodiments.

In one embodiment, the antibody-drug conjugate is represented by formula (V-1a) or formula (V-1a-A): 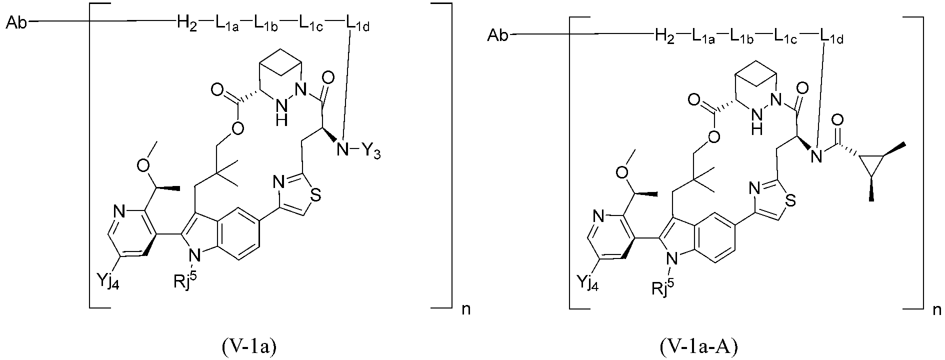 wherein H₂, L₁ₐ, L_{1b}, L_{1c}, L_{1d}, Rj⁵, Yj₄, Y₃, n, and Ab are as defined in any one of the embodiments.

In one embodiment, the antibody-drug conjugate is represented by formula (V-2) or formula (V-2-A): 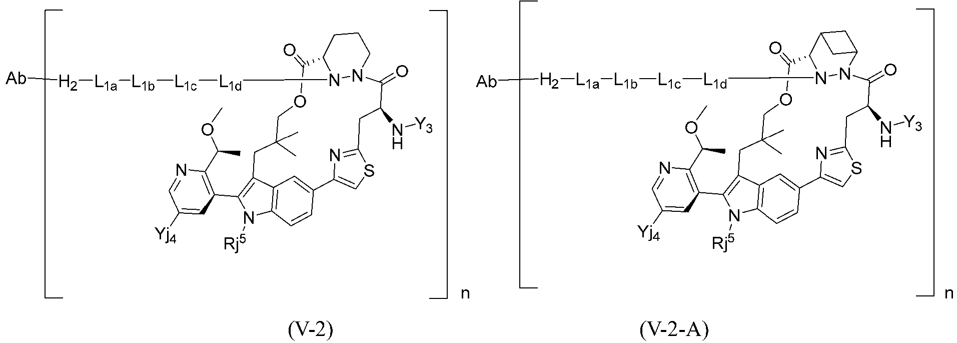 wherein H₂, L₁ₐ, L_{1b}, L_{1c}, L_{1d}, Rj⁵, Yj₄, Y₃, n, and Ab are as defined in any one of the embodiments.

In one embodiment, the antibody-drug conjugate is represented by formula (V-3) or formula (V-3-A): 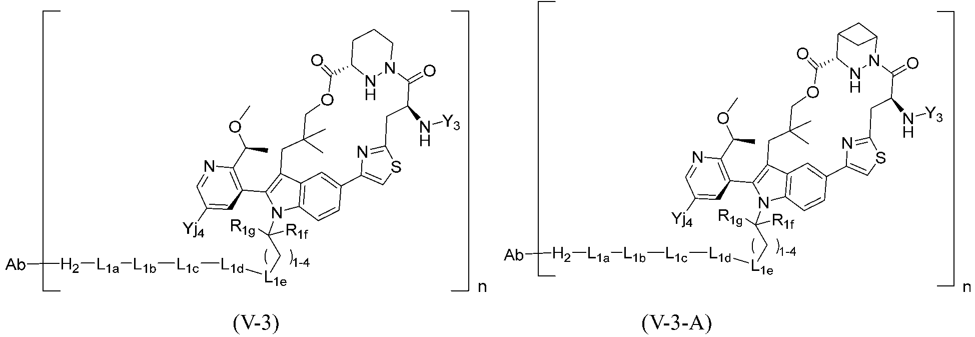
wherein H₂, L₁ₐ, L_{1b}, L_{1c}, L_{1d}, Yj₄, Y₃, n, and Ab are as defined in any one of the embodiments;
L_{1c} is NR₁ₑ or O, and R₁ₑ is H, C₁₋₄ alkyl, or C₁₋₄ alkoxy-C₁₋₄ alkyl;
R_{1f} is H or C₁₋₄ alkyl;
R_{1g} is H or C₁₋₄ alkyl.

In one embodiment, R₁ₑ is H or C₁₋₄ alkyl. In one embodiment, R₁ₑ is H, methyl, or ethyl. In one embodiment, R₁ₑ is H. In one embodiment, L₁ₑ is NH.

In one embodiment, R_{1f} is H, methyl, or ethyl. In one embodiment, R_{1f} is H.

In one embodiment, R_{1g} is H, methyl, or ethyl. In one embodiment, R_{1g} is H.

In one embodiment, the antibody-drug conjugate is represented by formula (V-4) or formula (V-4-A): 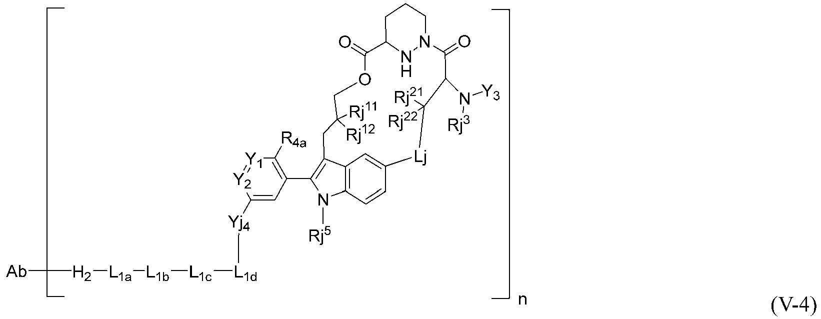 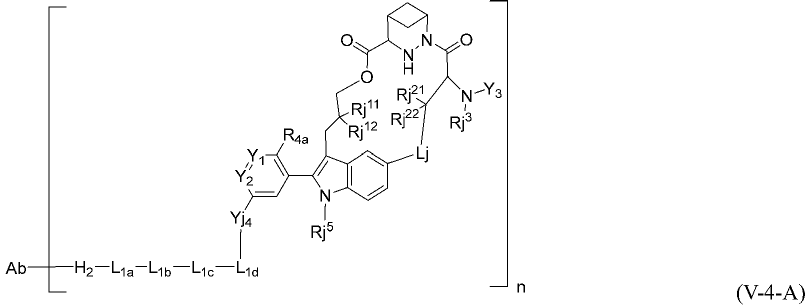 wherein H₂, L₁ₐ, L_{1b}, L_{1c}, L_{1d}, Rj⁵, Rj¹¹, Rj¹², Rj²¹, Rj²², Rj³, R₄ₐ, Lj, Yj₄, Y₁, Y₂, Y₃, n, and Ab are as defined in any one of the embodiments.

In one embodiment, the antibody-drug conjugate is represented by formula (V-5) or formula (V-5-A): 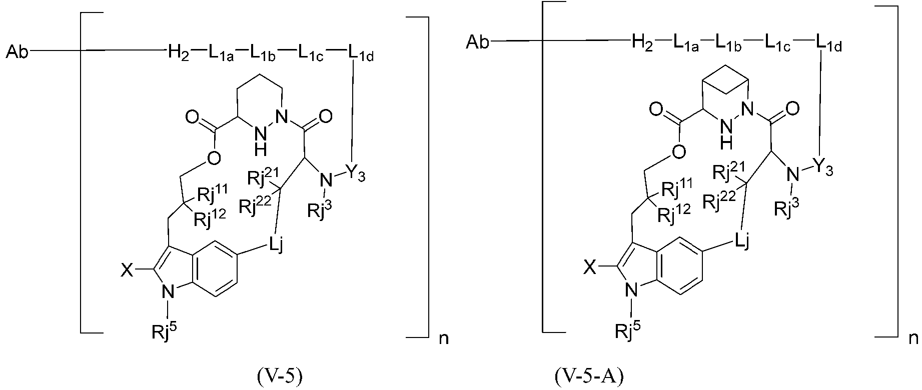 wherein H₂, L₁ₐ, L_{1b}, L_{1c}, L_{1d}, Rj¹¹, Rj¹², Rj²¹, Rj²², Rj³, Rj⁵, X, Lj, Y₃, n, and Ab are as defined in any one of the embodiments.

In one embodiment, the antibody-drug conjugate is represented by formula (V-6) or formula (V-6-A): 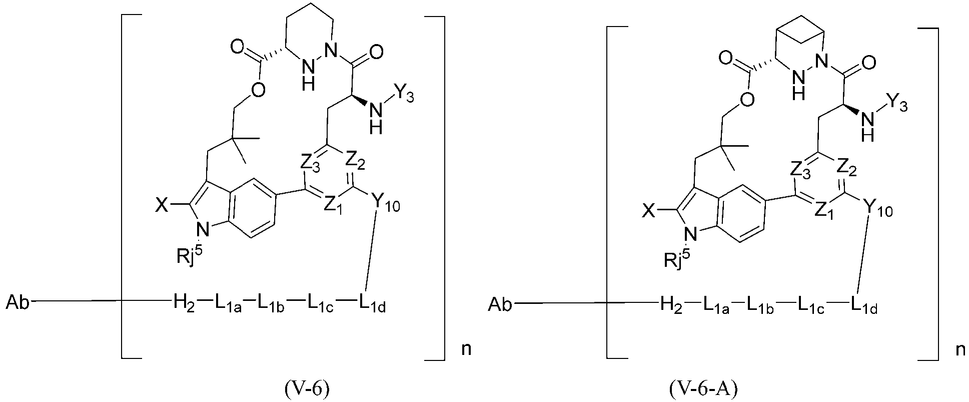 wherein H₂, L₁ₐ, L_{1b}, L_{1c}, L_{1d}, Rj⁵, X, Z₁, Z₂, Z₃, Y₃, Y₁₀, n, and Ab are as defined in any one of the embodiments.

In one embodiment, the antibody-drug conjugate is represented by formula (V-7) or formula (V-7-A): 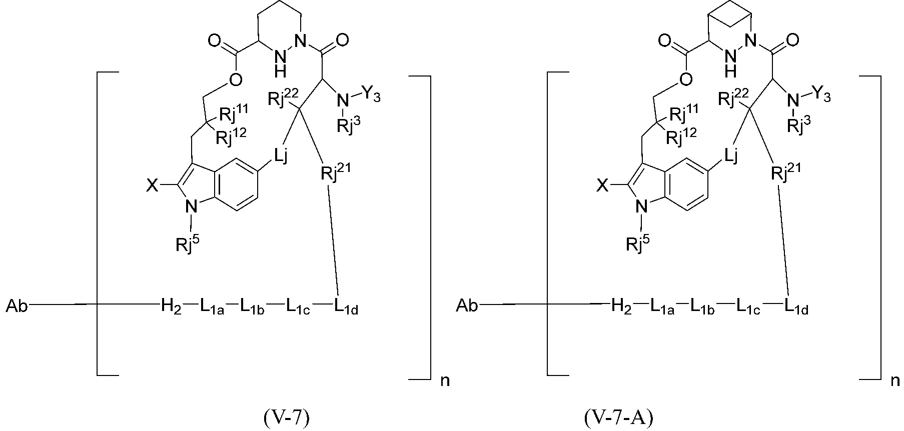 wherein H₂, L₁ₐ, L_{1b}, L_{1c}, L_{1d}, Rj¹¹, Rj¹², Rj²¹, Rj²², Rj³, Rj⁵, X, Lj, Y₃, n, and Ab are as defined in any one of the embodiments.

In one embodiment, the antibody-drug conjugate is represented by formula (V-8) or formula (V-8-A): 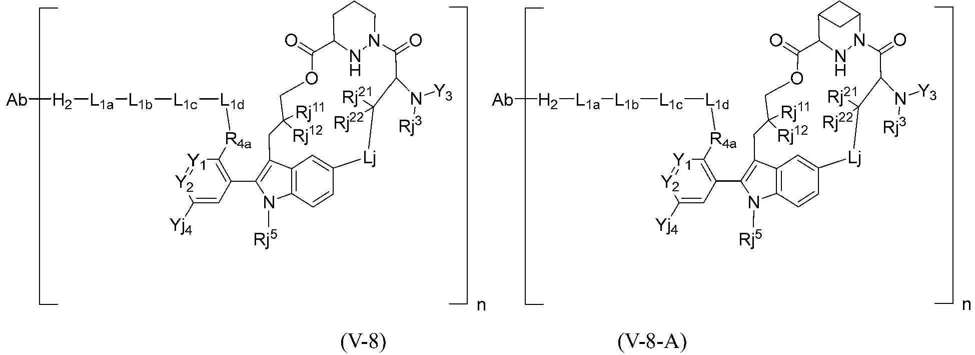 wherein H₂, L₁ₐ, L_{1b}, L_{1c}, L_{1d}, Rj¹¹, Rj¹², Rj²¹, Rj²², Rj³, Rj⁵, R₄ₐ, Lj, Yj₄, Y₁, Y₂, Y₃, n, and Ab are as defined in any one of the embodiments.

In one embodiment, for the antibody-drug conjugate, the antibody-drug conjugate is selected from structures shown in Table 11,

**Table 11**

| Number | Structure |
|---|---|
| Z6-L1-Ab | 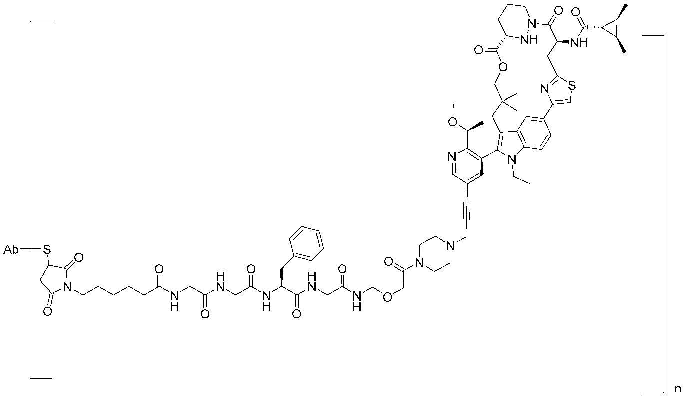 |
| Z6-L2-Ab | 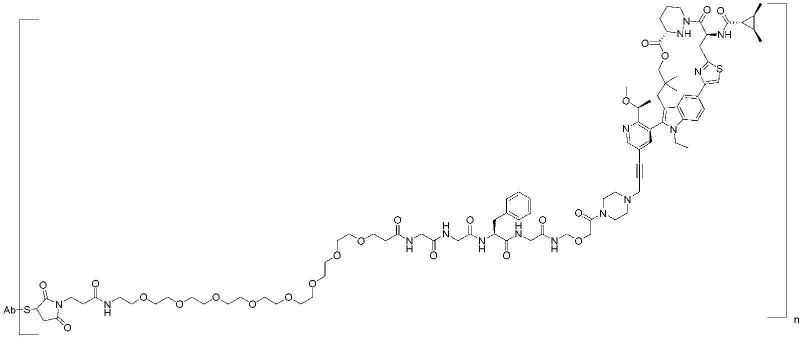 |
| Z34-L1-Ab | 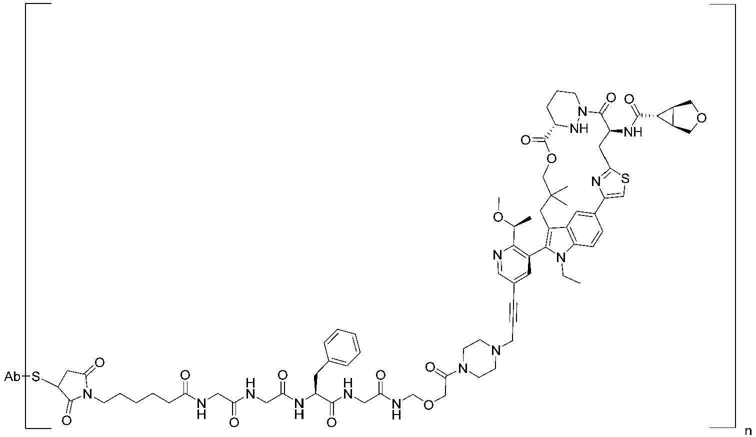 |
| Z34-L2-Ab | 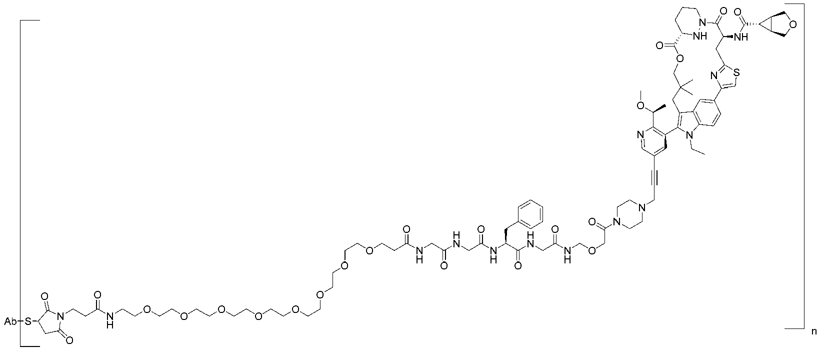 |
| Z34-L3-Ab | 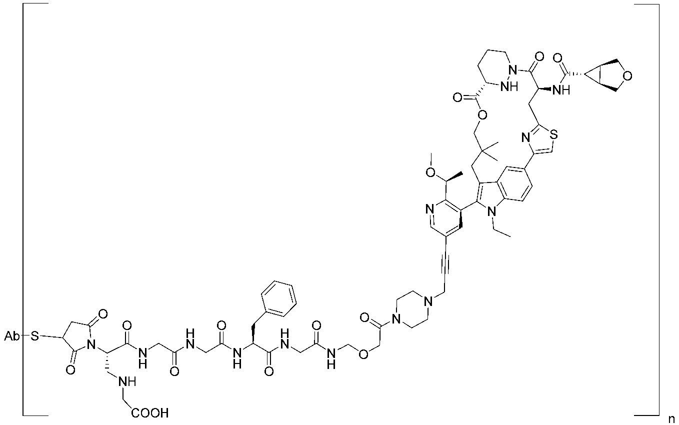 |
| Z34-L4-Ab | 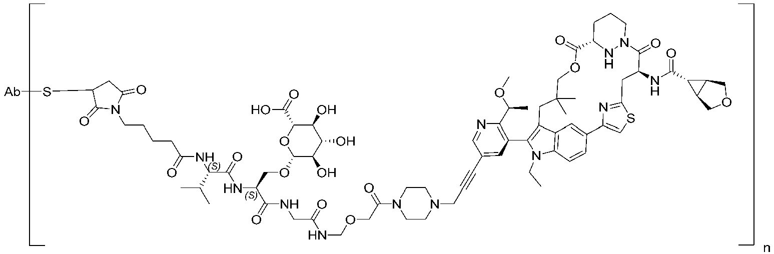 |
| Z63-L1-Ab | 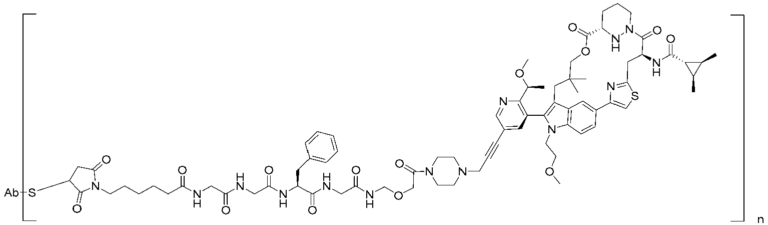 |
| Z48-L1-Ab | 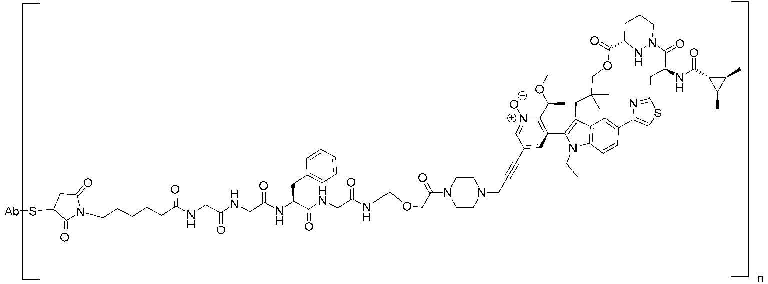 |
| Z54-L1-Ab | 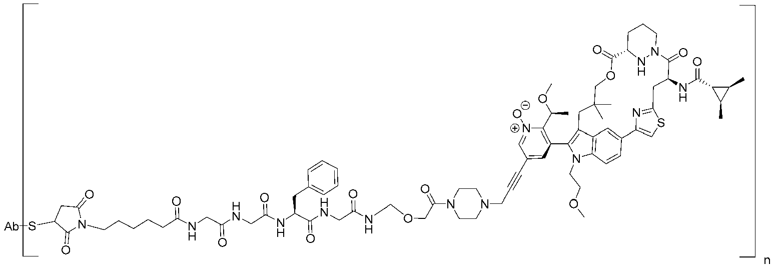 |
| Z54-L2-Ab | 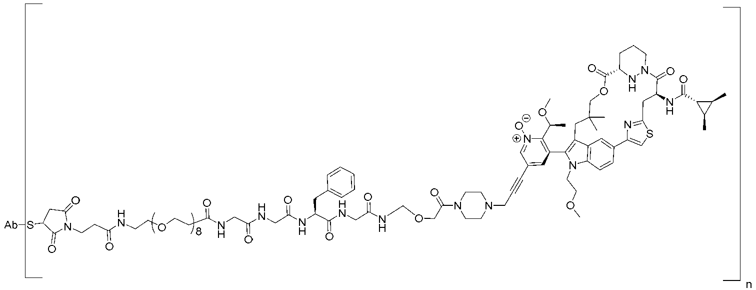 |
| Z31-L1-Ab | 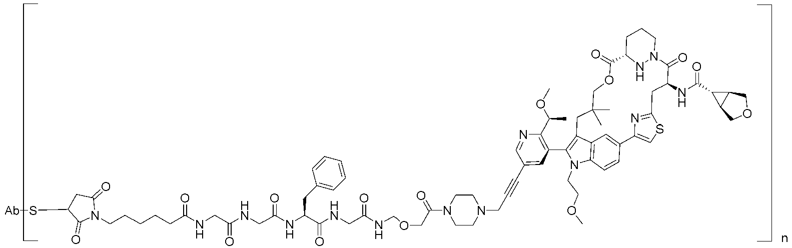 |
| Z35-L1-A-Ab | 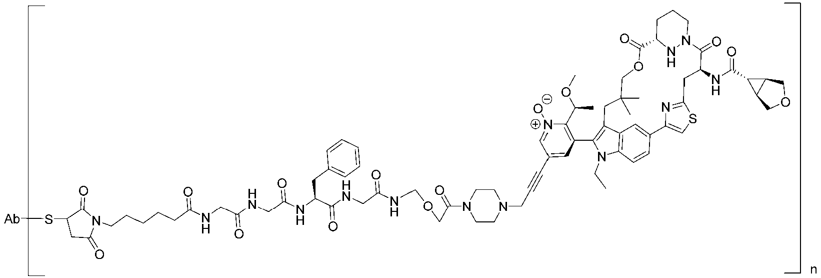 |
| Z38-L1-A-Ab | 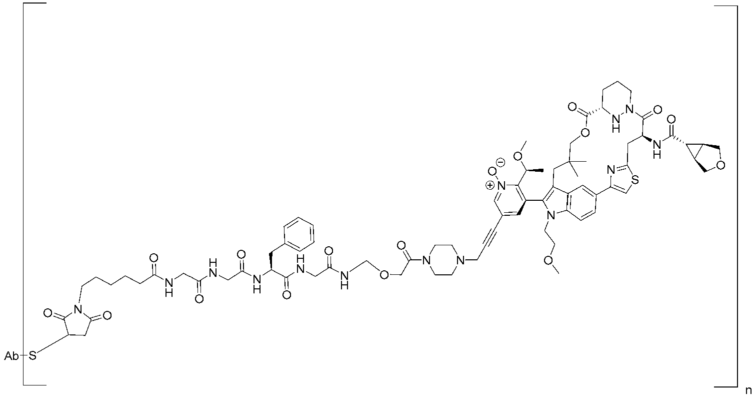 |
| Z38-L2-A-Ab | 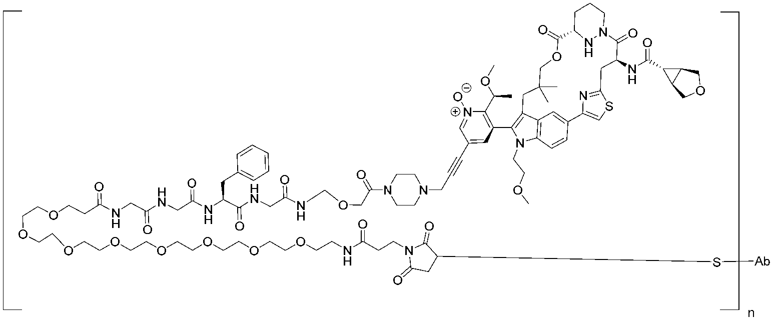 |
| Z47-L5-Ab | 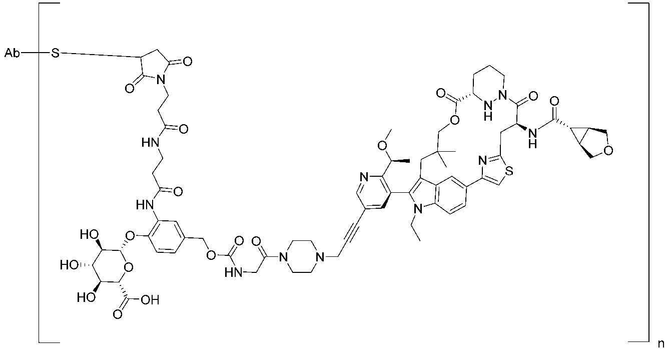 |
| Z47-L6-Ab | 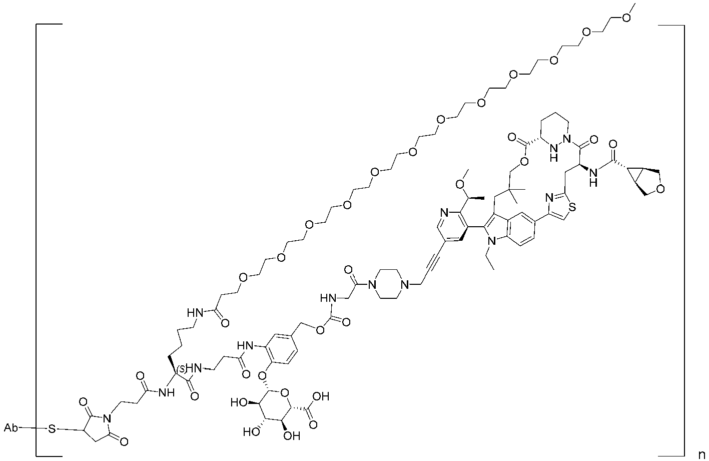 |
| Z52-L7-Ab | 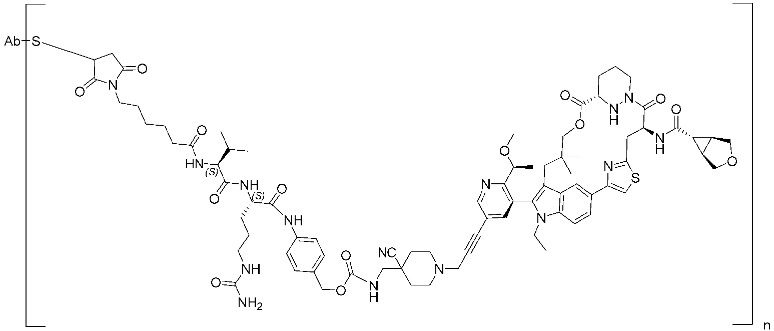 |
| Z52-L8-Ab | 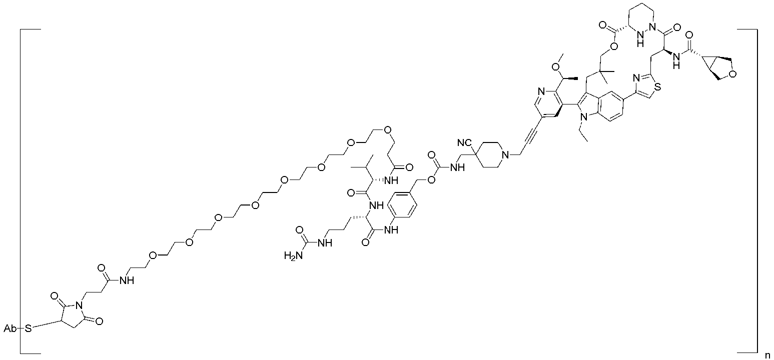 |
| Z52-L9-Ab | 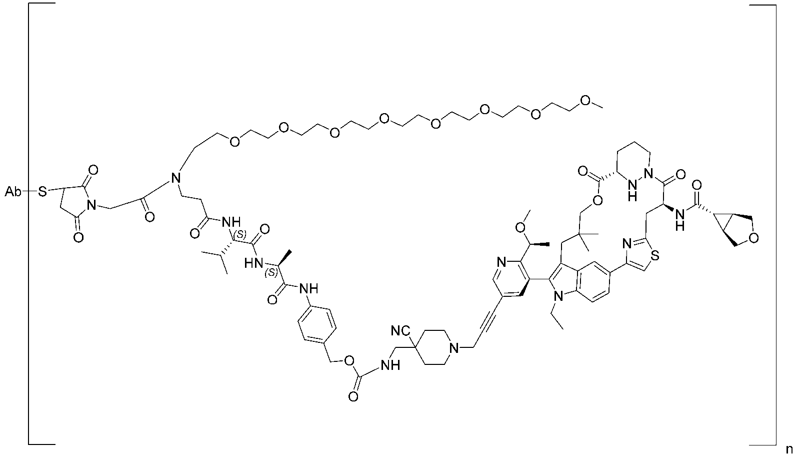 |
| Z52-L10-Ab | 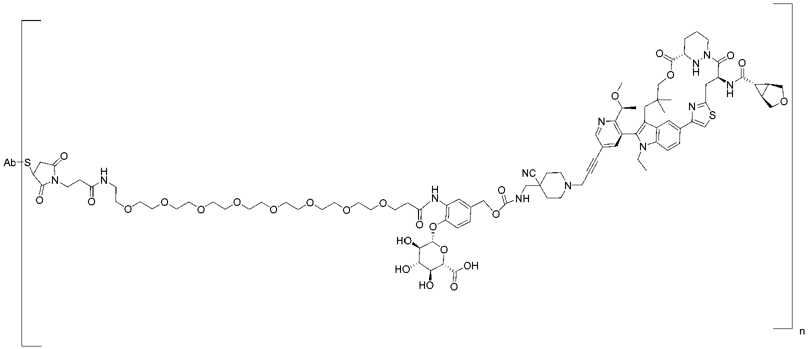 |
| Z52-L12-Ab | 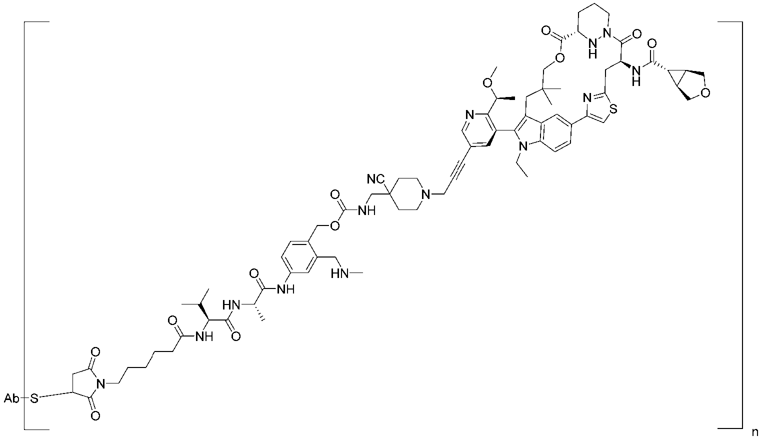 |
| Z52-L13-Ab | 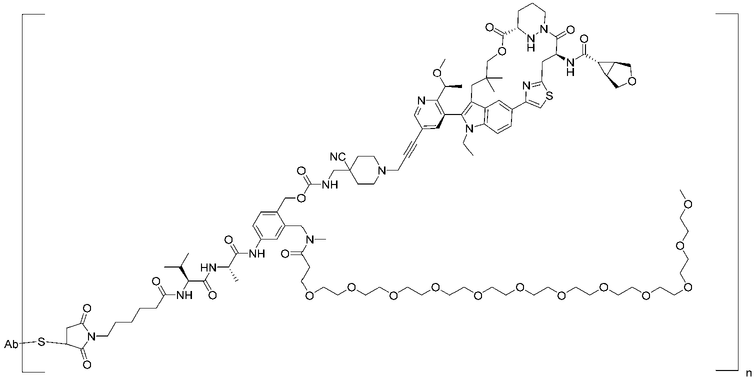 |
| Z52-L14-Ab | 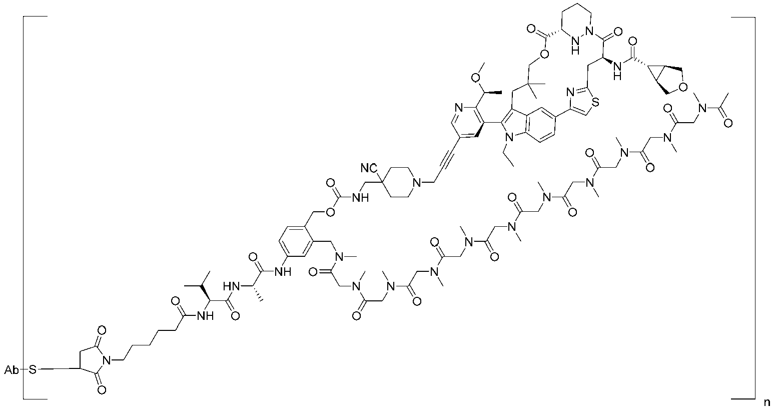 |
| Z166-L14-Ab | 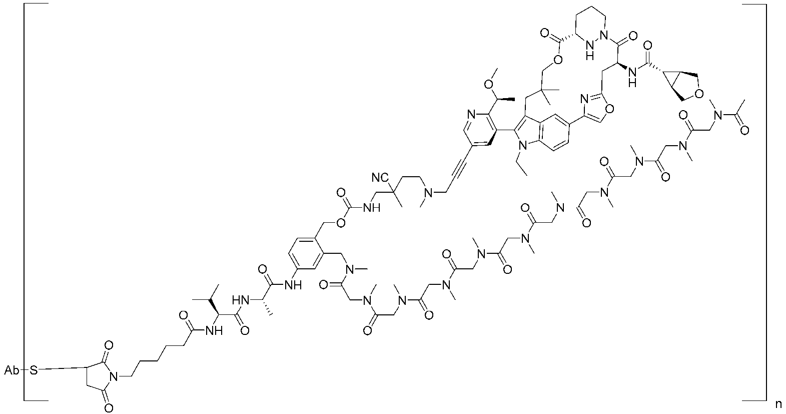 |
| Z52-L15-Ab | 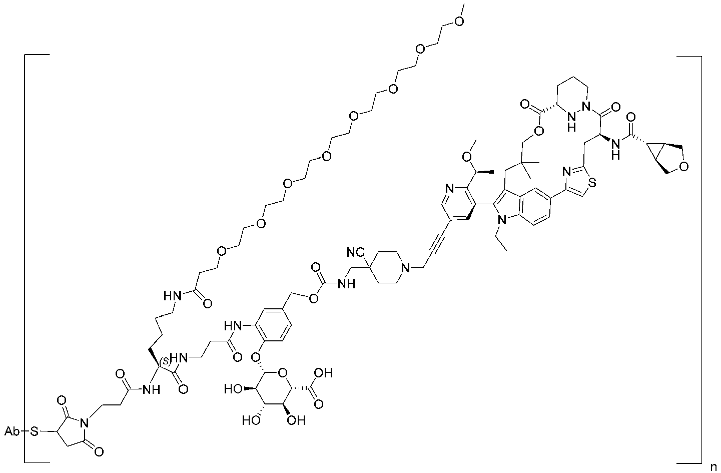 |
| Z52-L16-Ab | 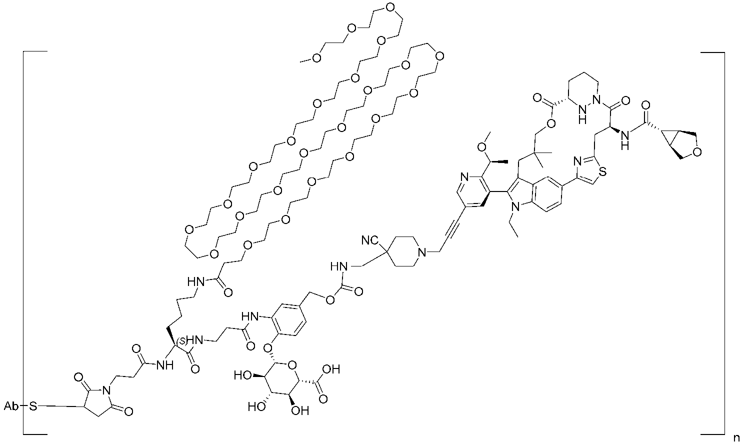 |
| Z57-L7-A-Ab | 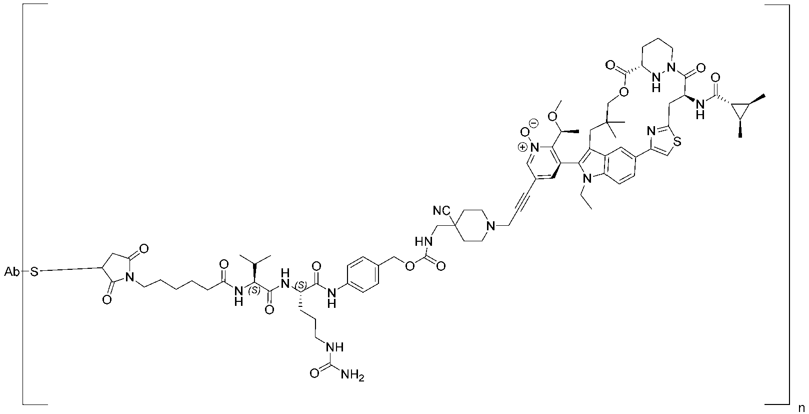 |
| Z64-L7-A-Ab | 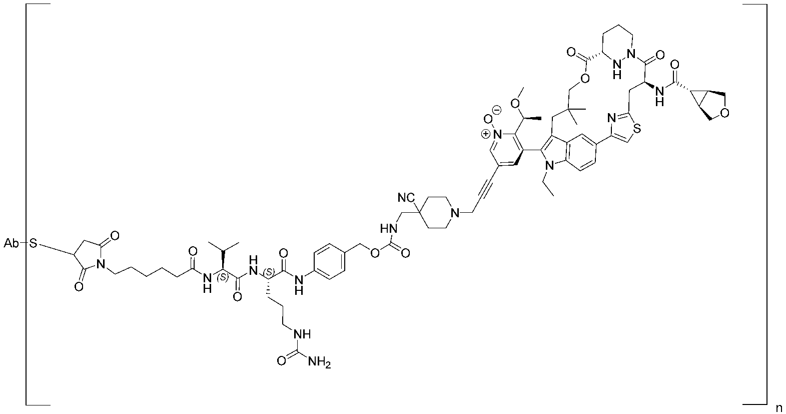 |
| Z64-1-L16-Ab | 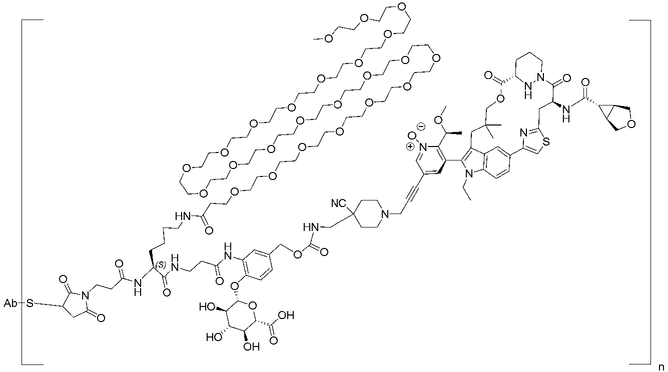 |
| Z61-L7-Ab | 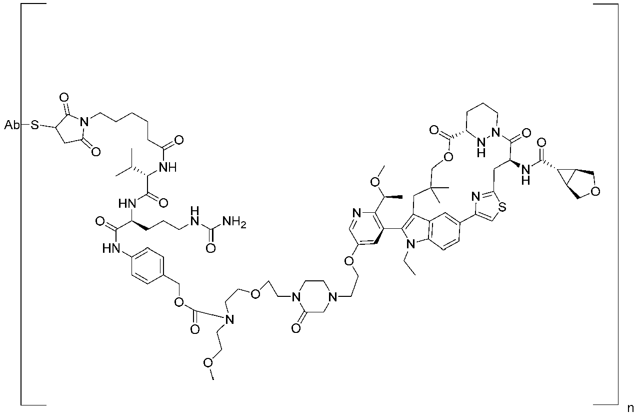 |
| Z45-L2-A-Ab | 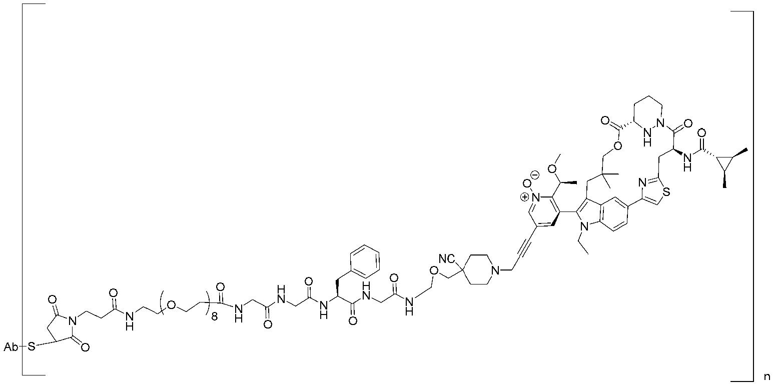 |
| Z11-L2-Ab | 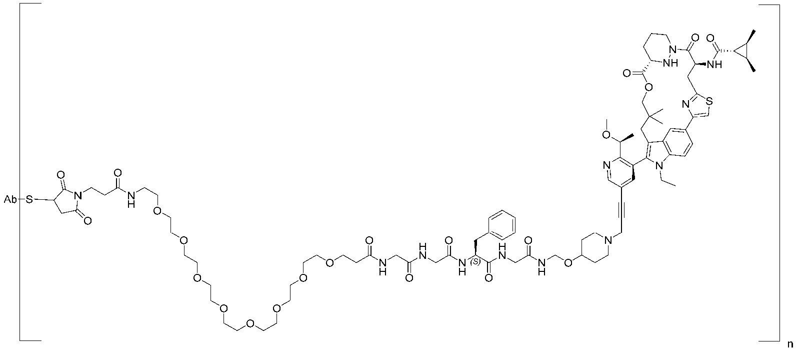 |
| Z170-L17-Ab | 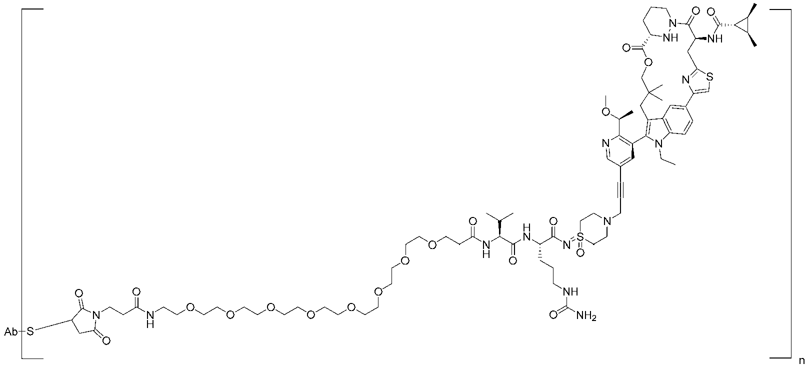 |
| Z56-L19-Ab | 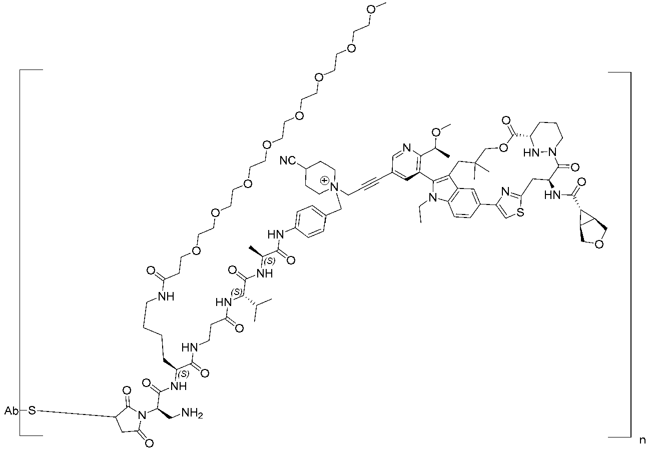 |
| Z56-L23-Ab | 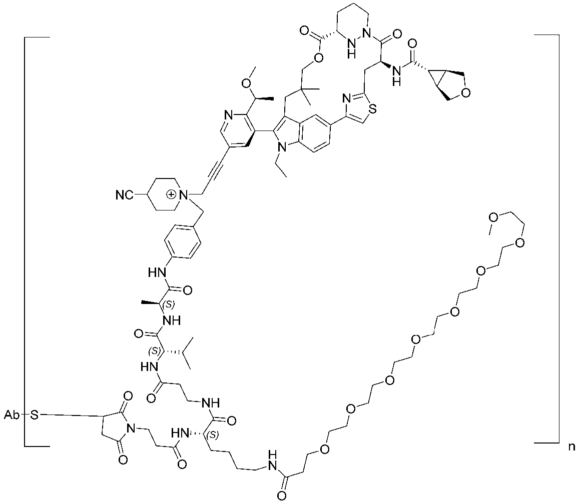 |
| Z10-L1-Ab | 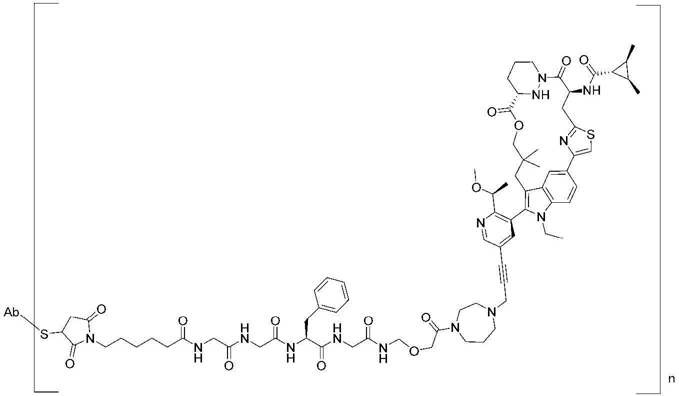 |
| Z52-L11-Ab | 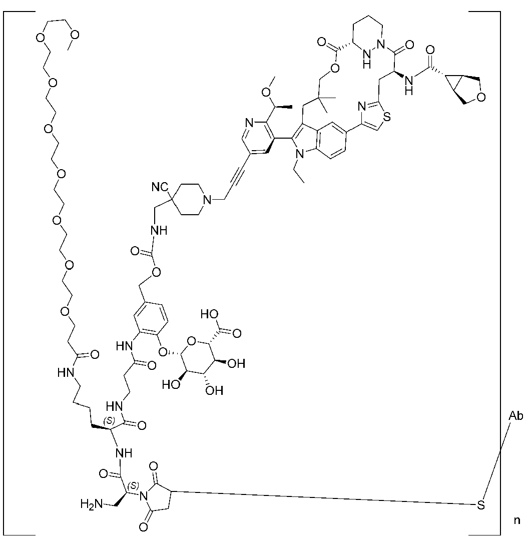 |
| Z56-L26-Ab | 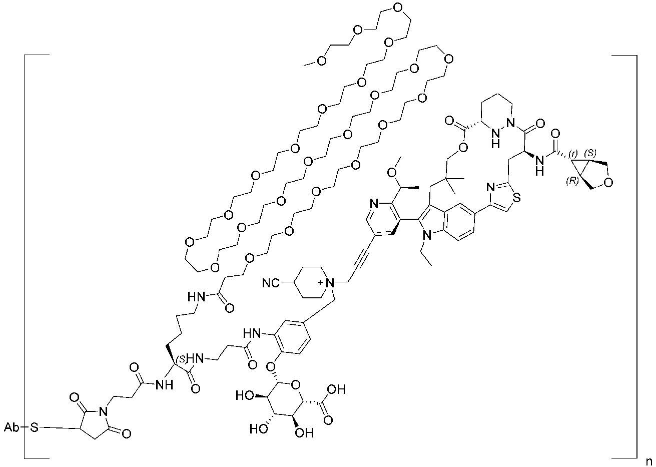 |
| Z168-L22-Ab | 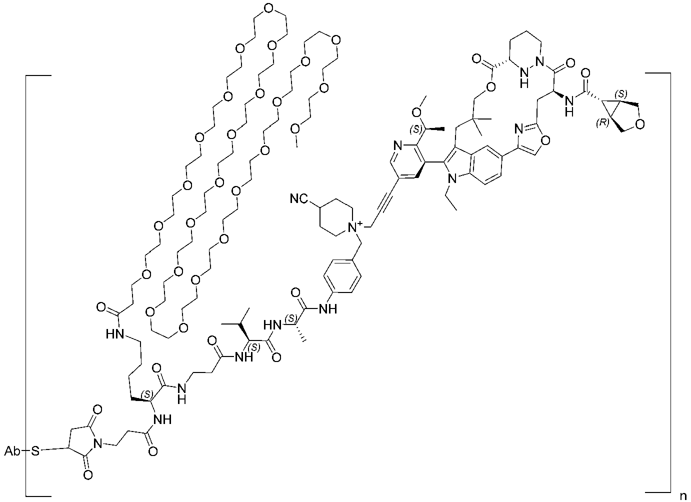 |
| Z168-L24-Ab | 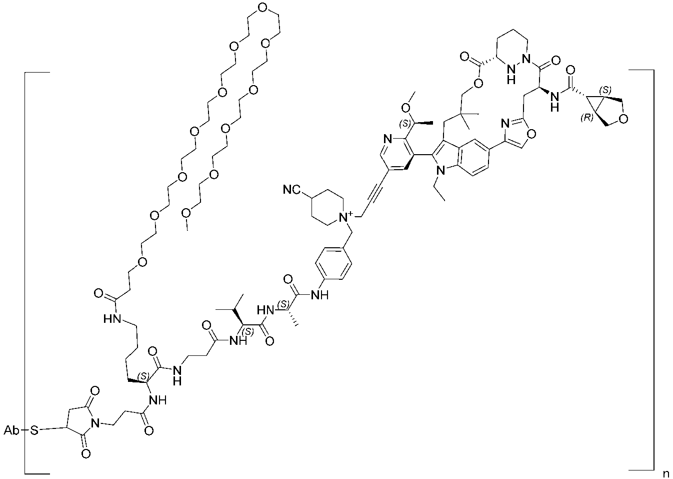 |
| Z168-L23-Ab | 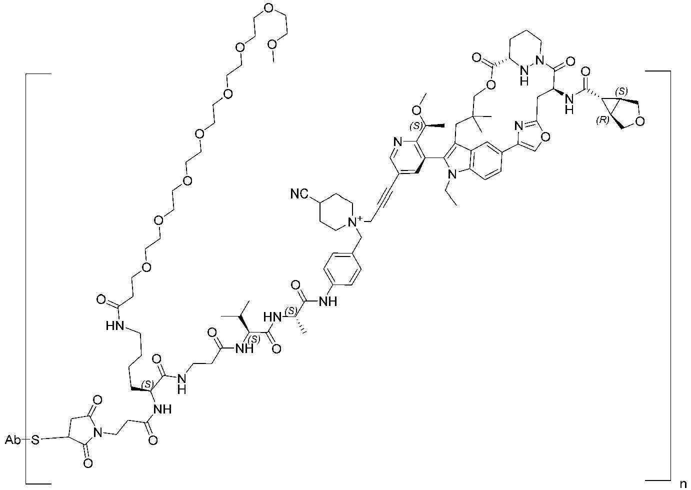 |
| Z4-L7-Ab | 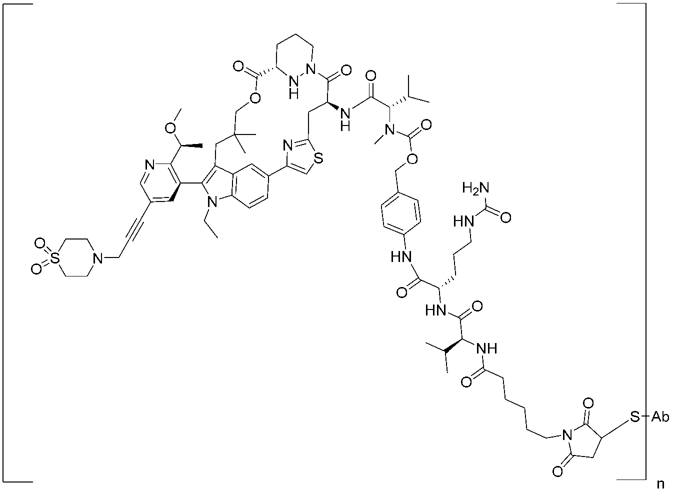 |
| Z4-L8-Ab | 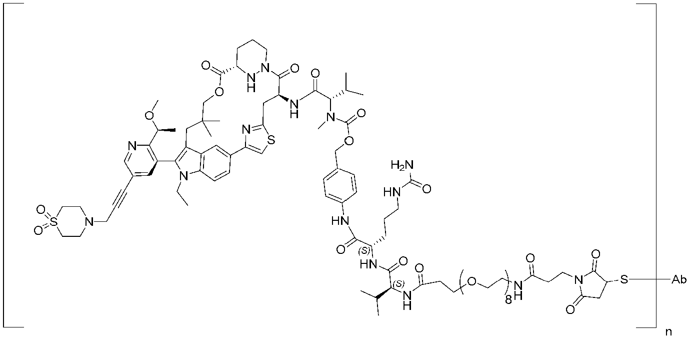 |
| Z22-L17-Ab | 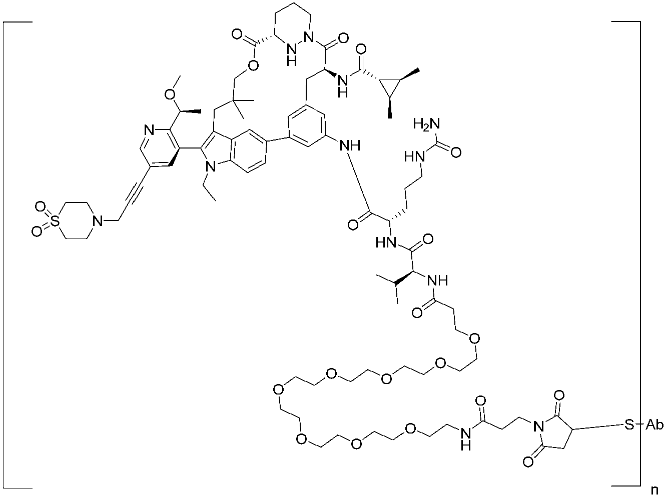 |
| Z22-L25-Ab | 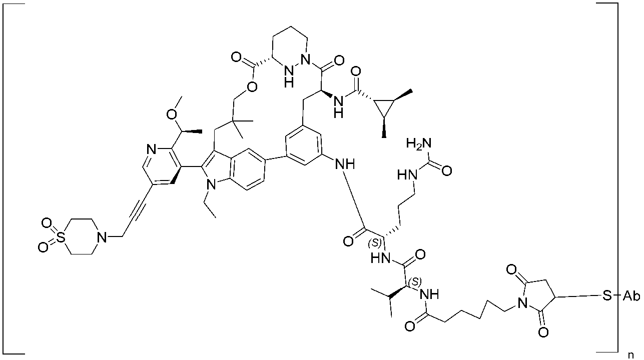 |
| Z3-L1-Ab | 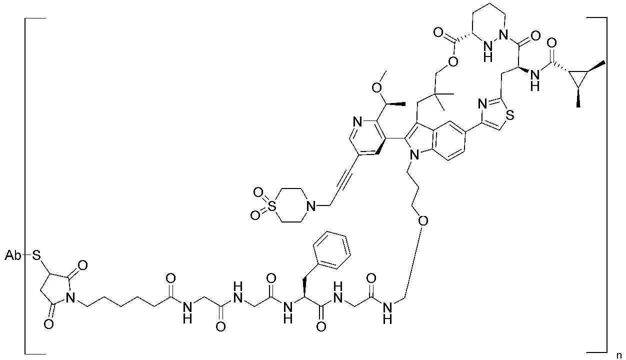 |
| Z169-1-L16-Ab | 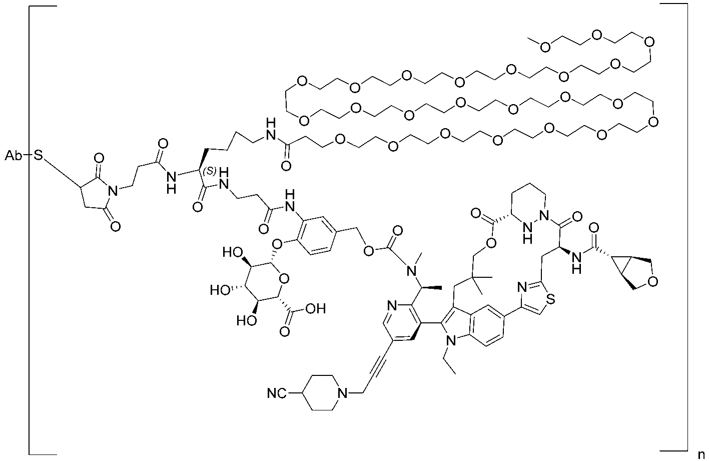 |
| Z283-L65-Ab | 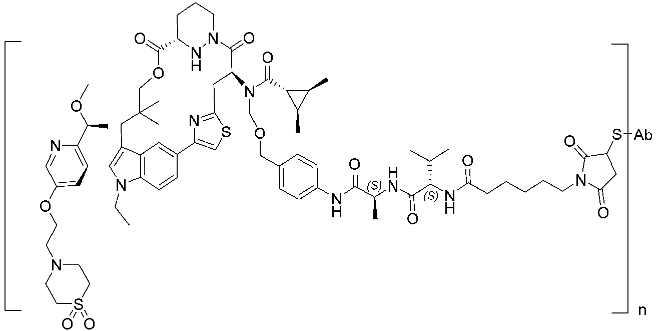 |
| Z284-L66-Ab | 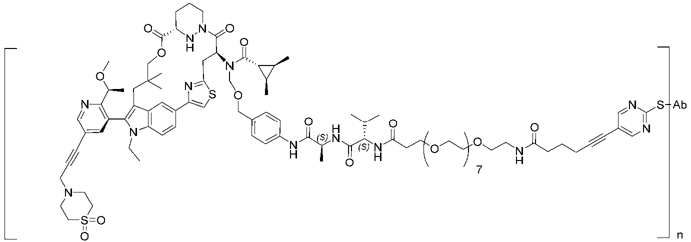 |
| Z285-L67-Ab | 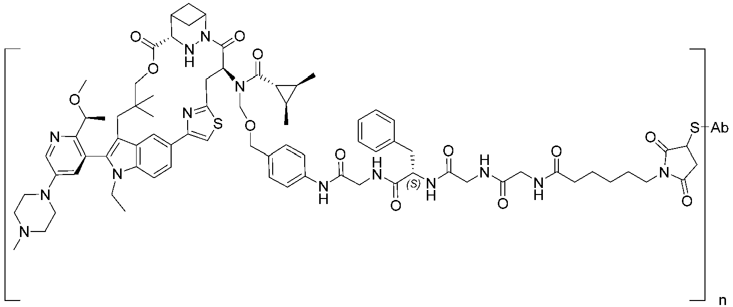 |
| Z284-L68-Ab | 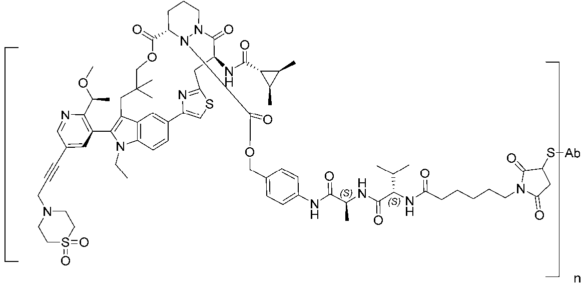 |
| Z286-L68-Ab | 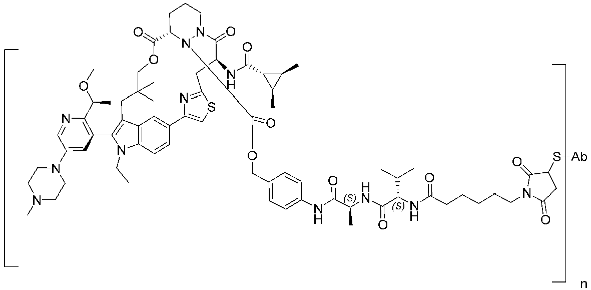 |
| Z285-L68-Ab | 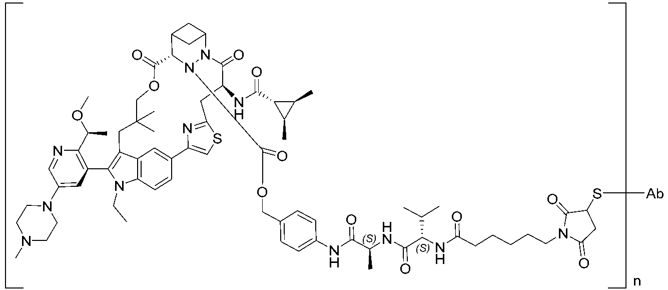 |
| Z287-L57-Ab | 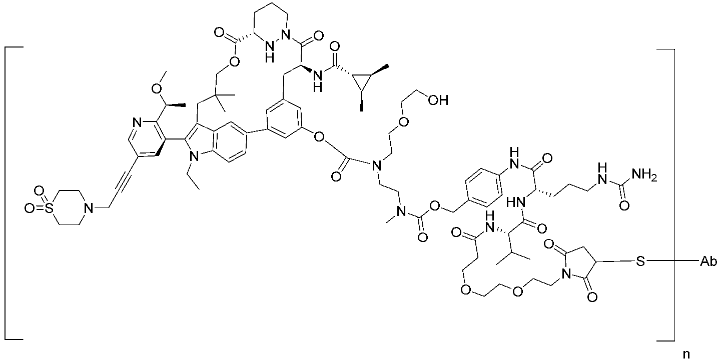 |
| Z283-L69-Ab | 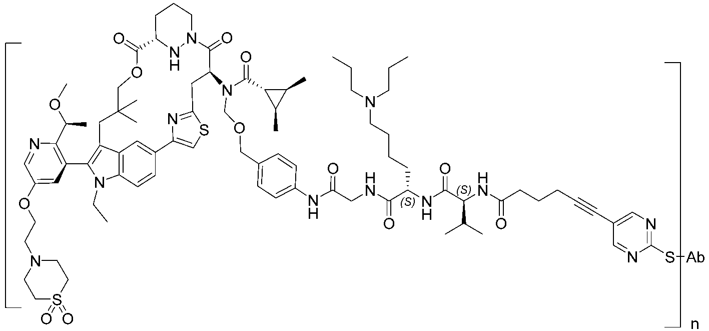 |
| Z284-A-L53-Ab | 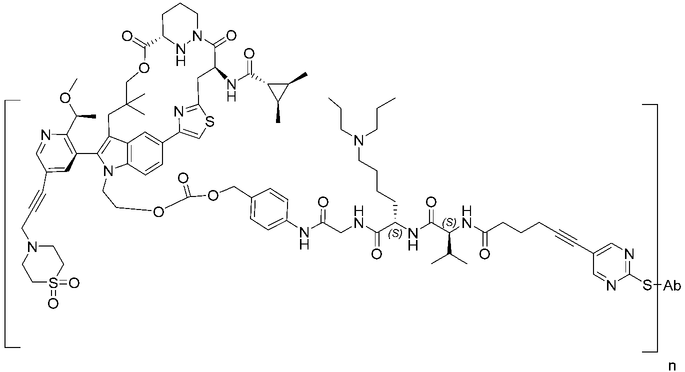 |
| Z286-A-L1-Ab | 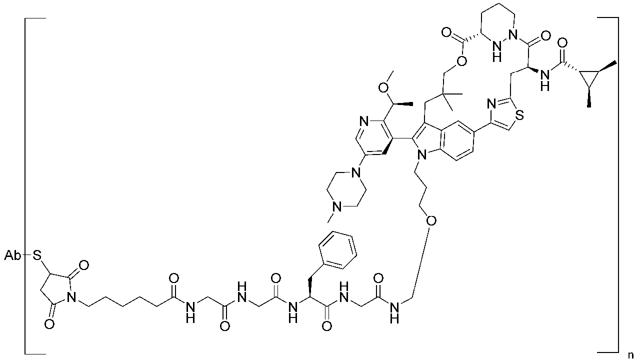 |
| Z285-A-L1-Ab | 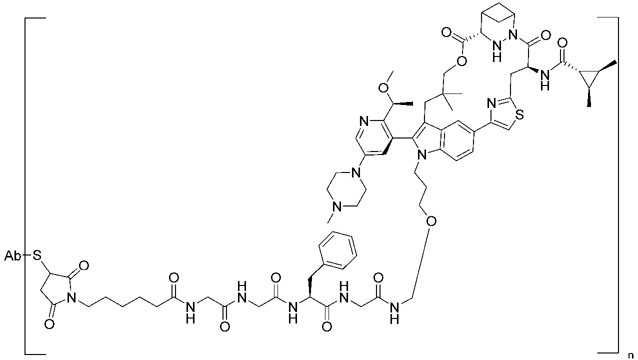 |
| Z285-A-L63-Ab | 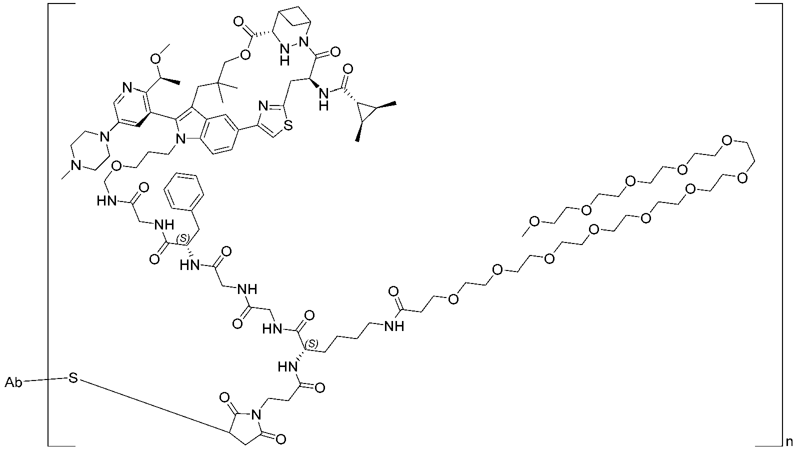 |
| Z289-L53-Ab | 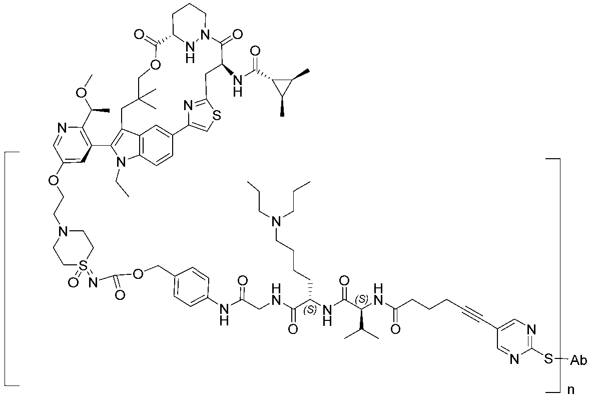 |
| Z286-B-L70-Ab | 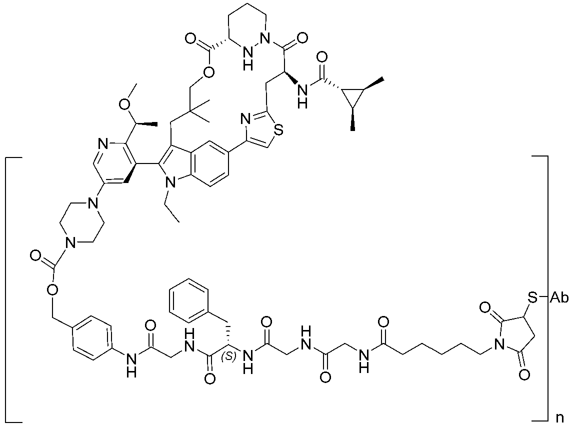 |
| Z290-L2-Ab | 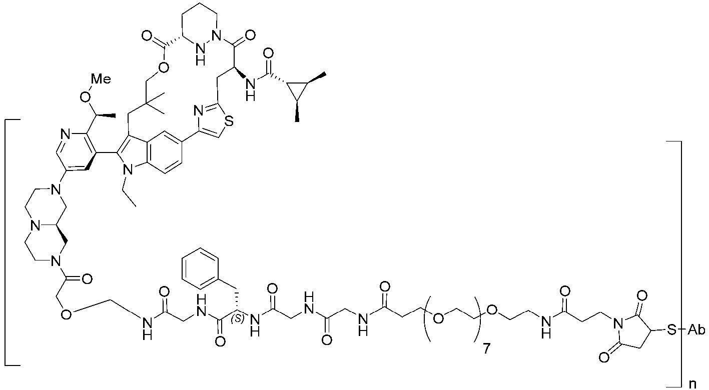 |
| Z285-B-L70-Ab | 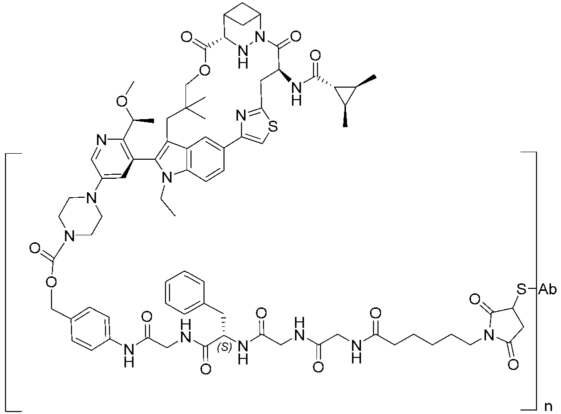 |
| Z286-B-L7-Ab | 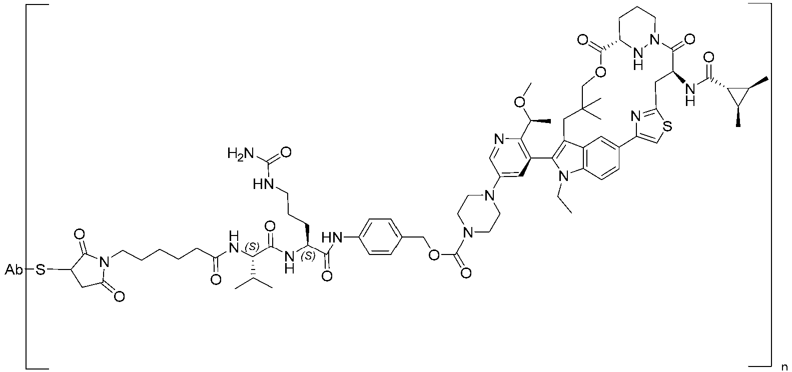 |
| Z288-L7-Ab | 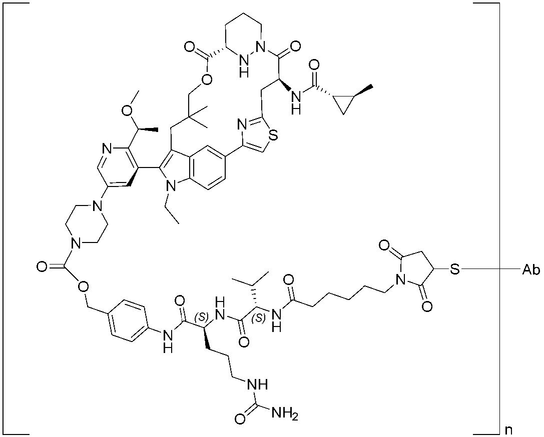 |
| Z285-B-L7-Ab | 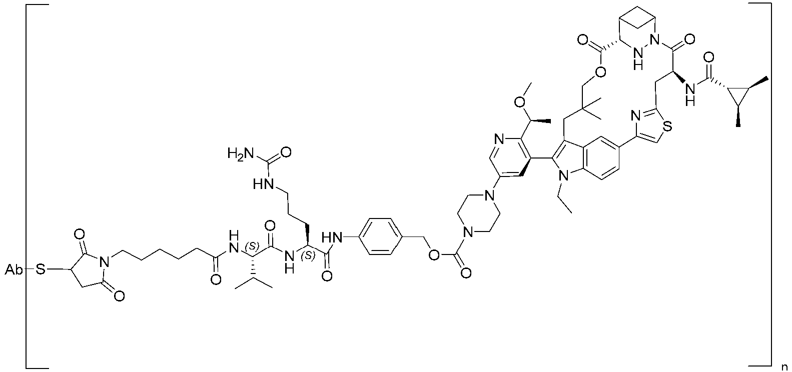 |
| Z288-L7-Ab | 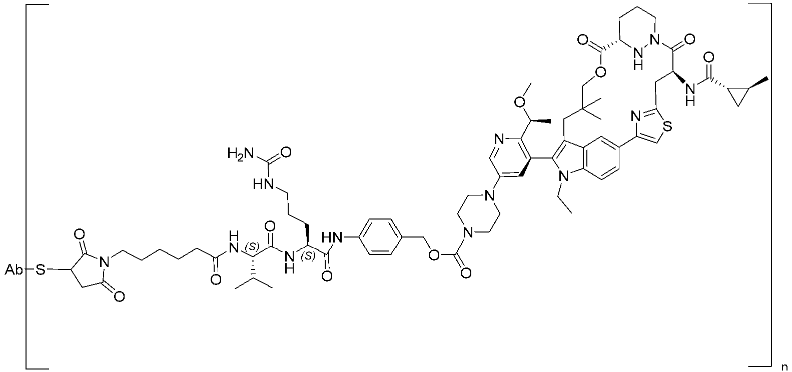 |
| Z286-L72-Ab | 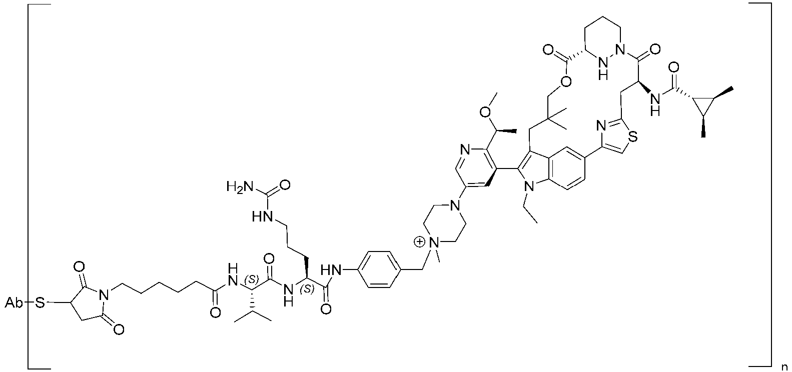 |
| Z288-A-L72-Ab | 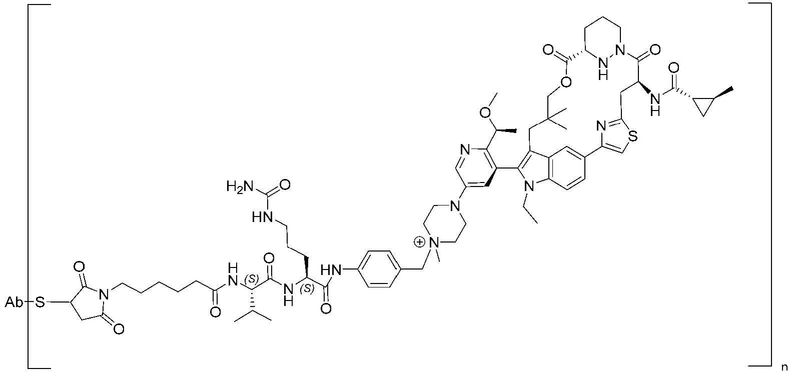 |
| Z285-L72-Ab | 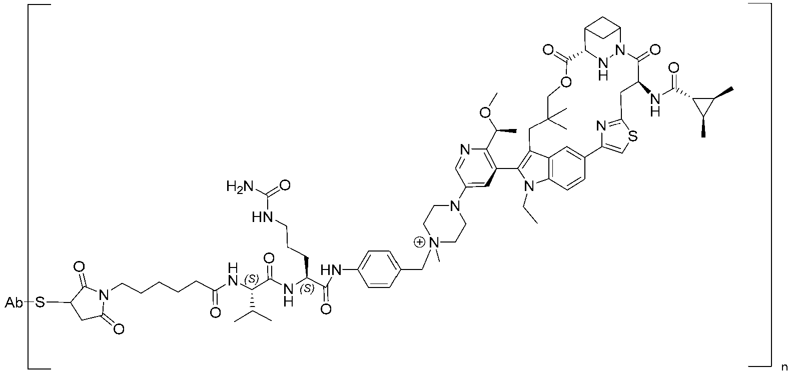 |
| Z285-B-L16-Ab | 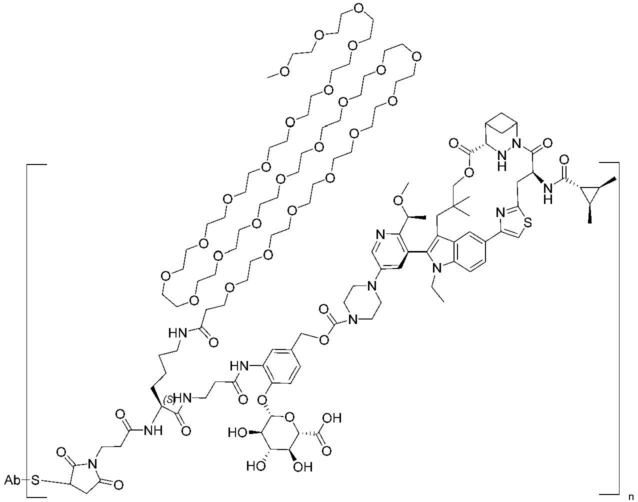 |
| Z286-B-L16-Ab | 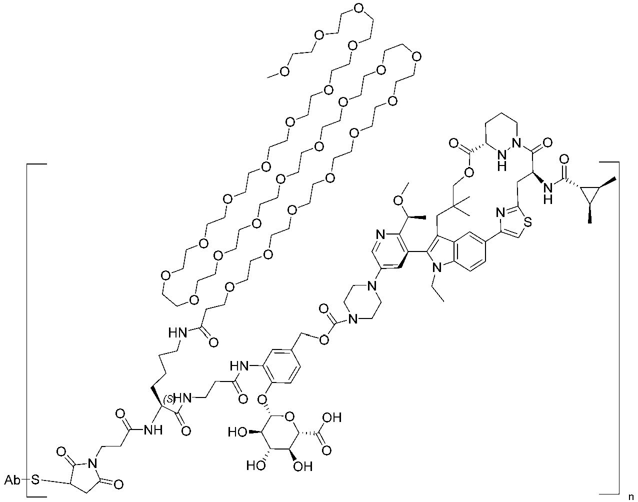 |
| Z288-L16-Ab | 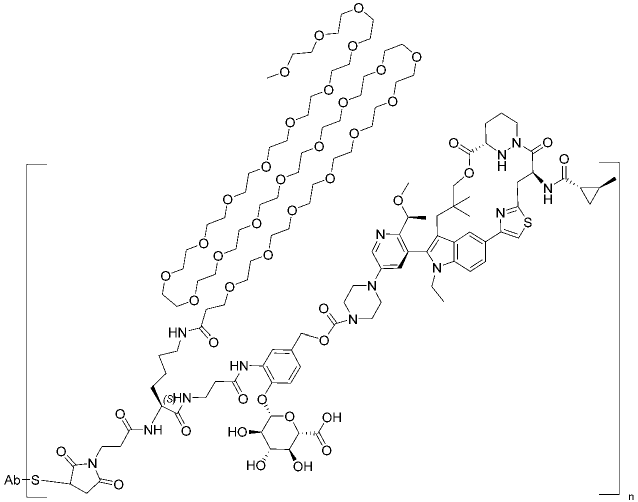 |
| Z285-L26-Ab | 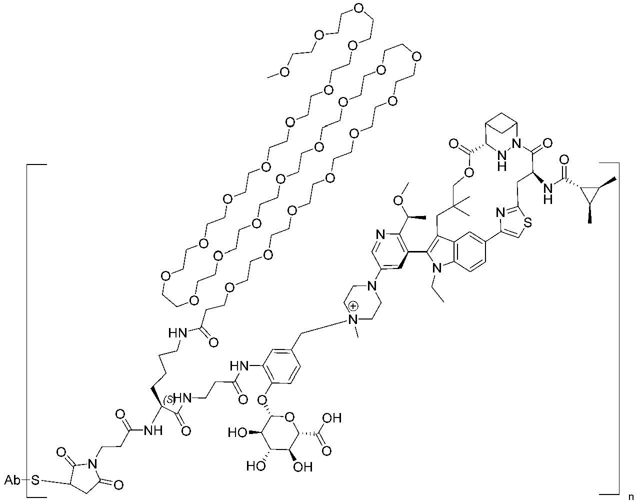 |
| Z286-L26-Ab | 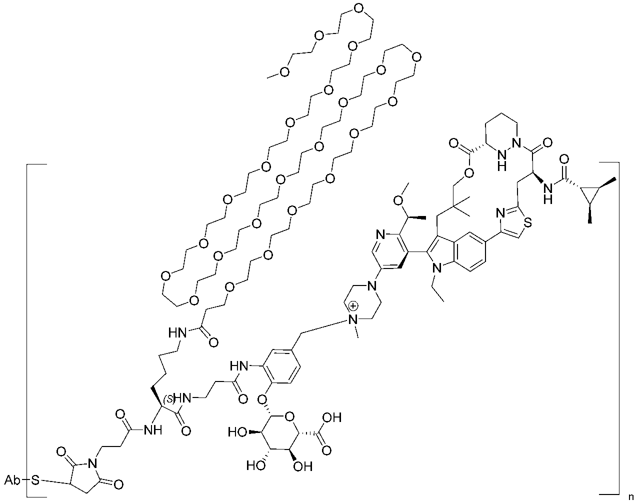 |
| Z288-A-L26-Ab | 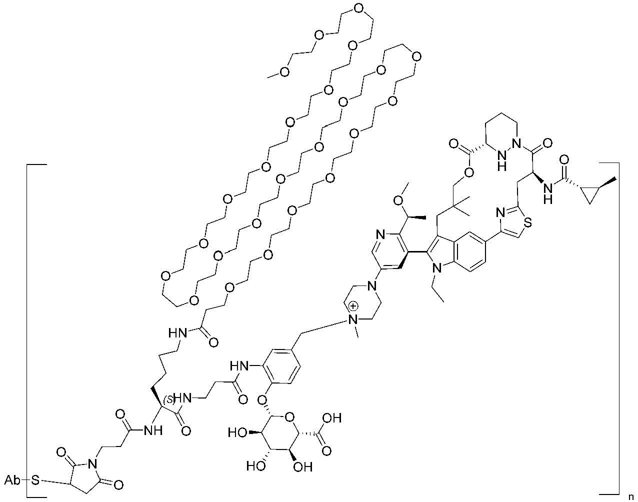 |
| Z285-B-L34-Ab | 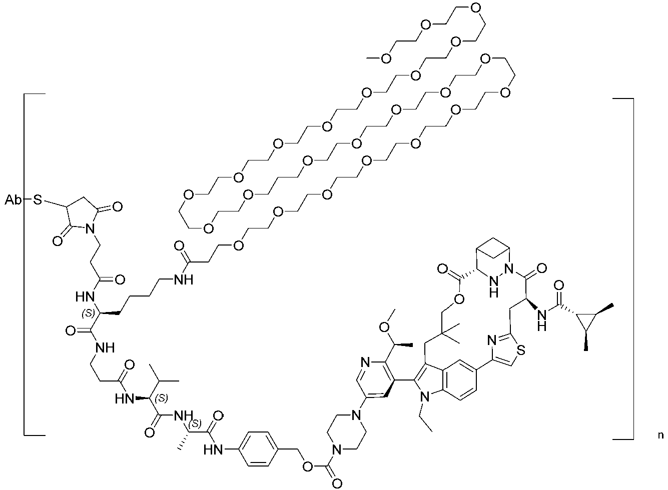 |
| Z286-B-L34-Ab | 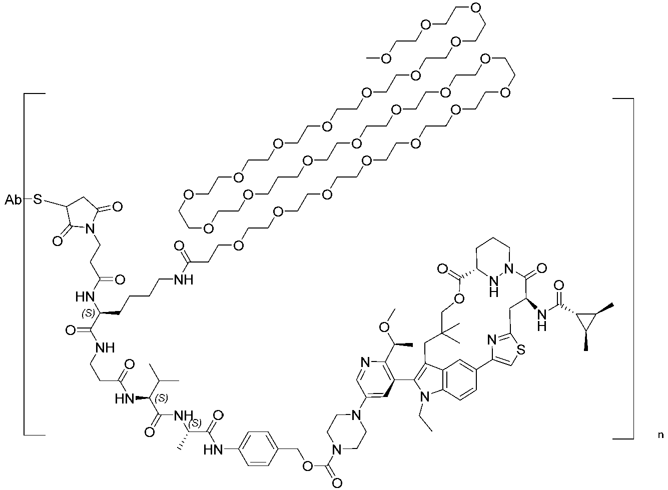 |
| Z285-L22-Ab | 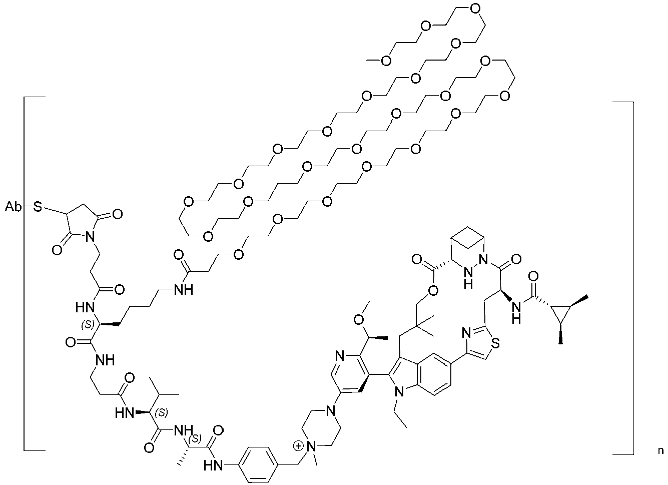 |
| Z286-L22-Ab | 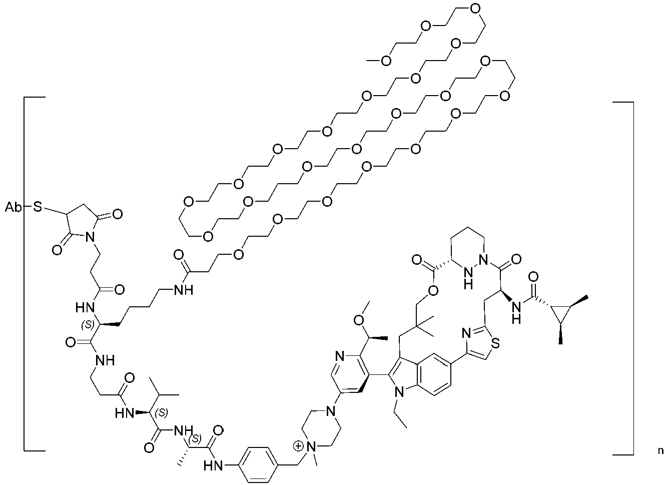 |
| Z288-A-L22-Ab | 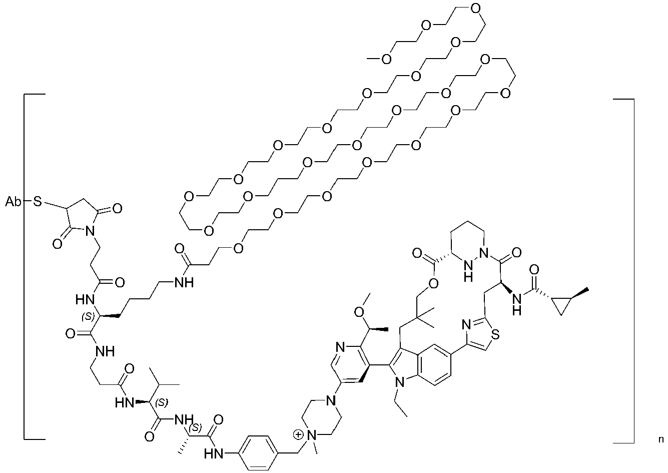 |
| Z7-2-L1-Ab | 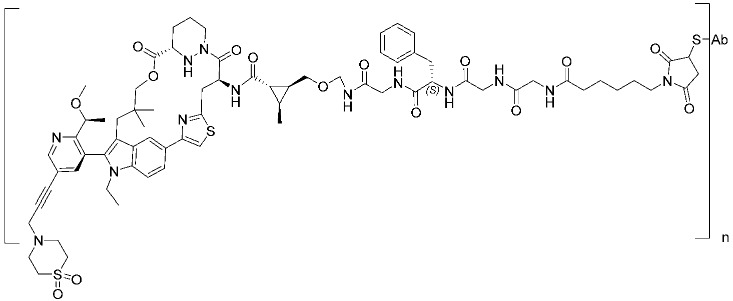 |
| Z291-L73-Ab | 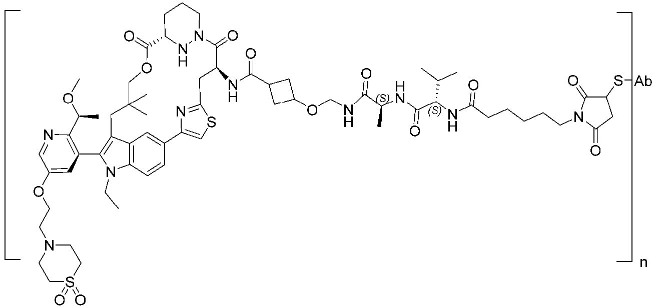 |
| Z310-L2 | 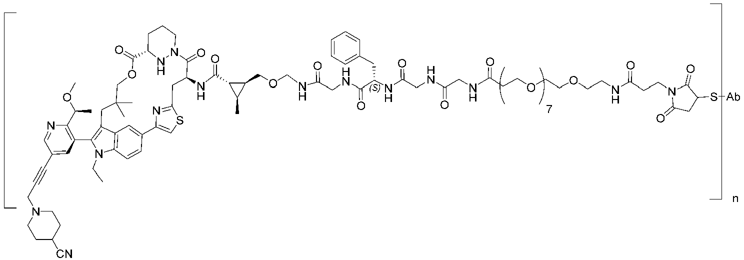 |
| Z292-L74-Ab | 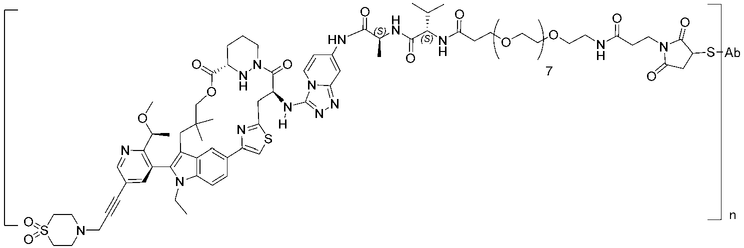 |
| Z7-2-L64-Ab | 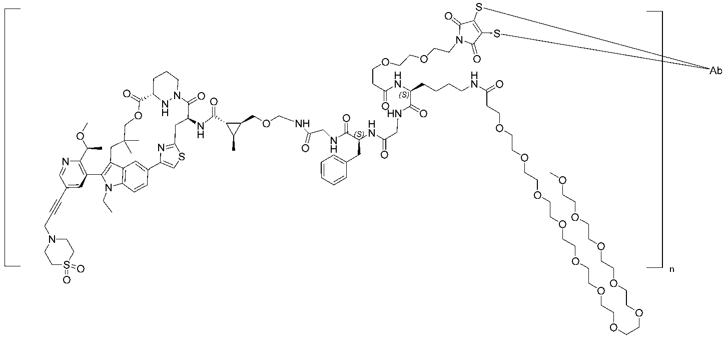 |
| Z7-2-L63-Ab | 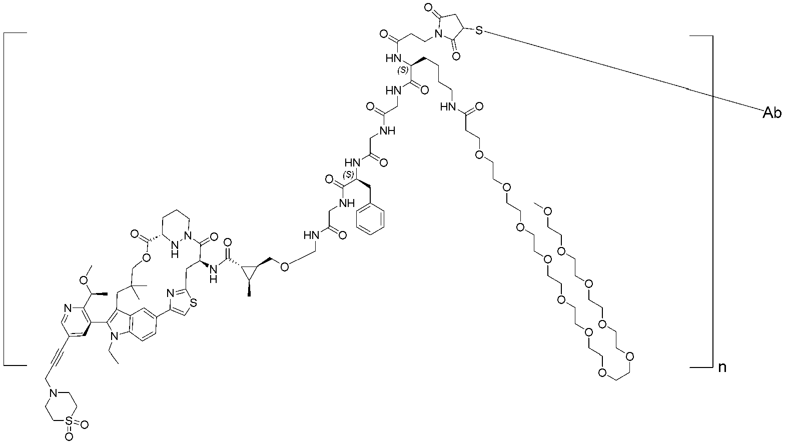 |
| Z293-L57-Ab | 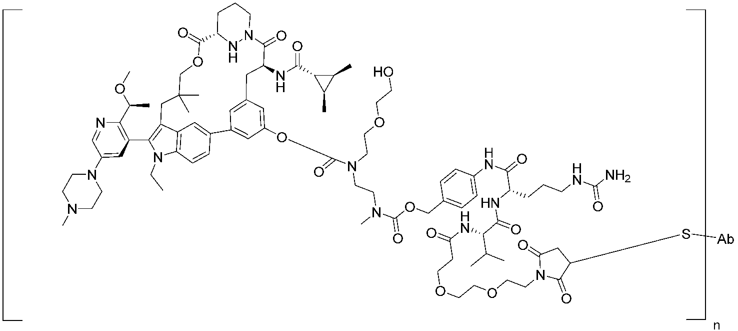 |
| Z294-L57-Ab | 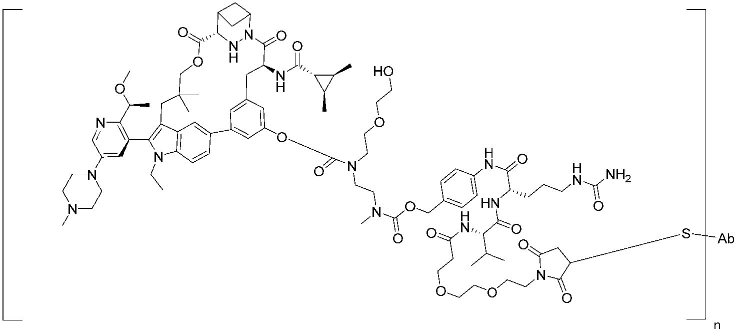 |
| Z88-L7-Ab | 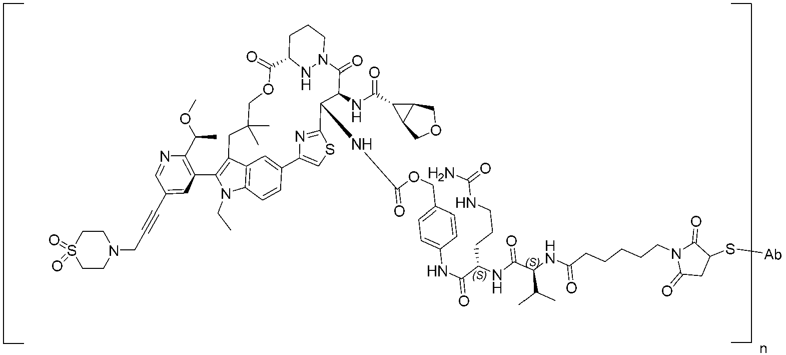 |
| Z295-L7-Ab | 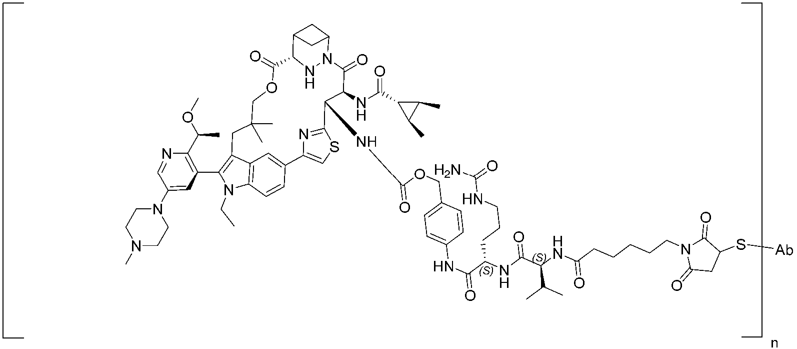 |
| Z296-L7-Ab | 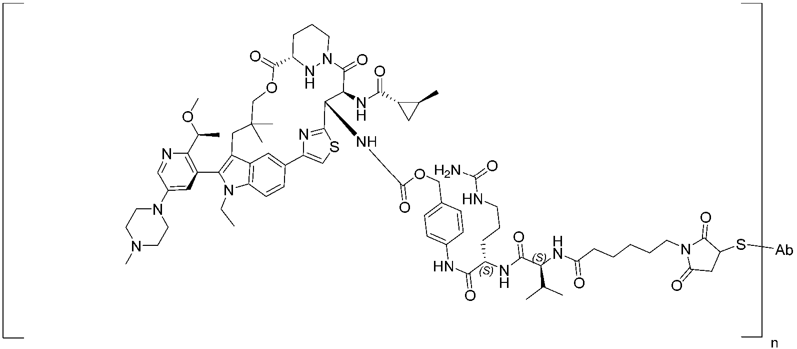 |
| Z297-L7-Ab | 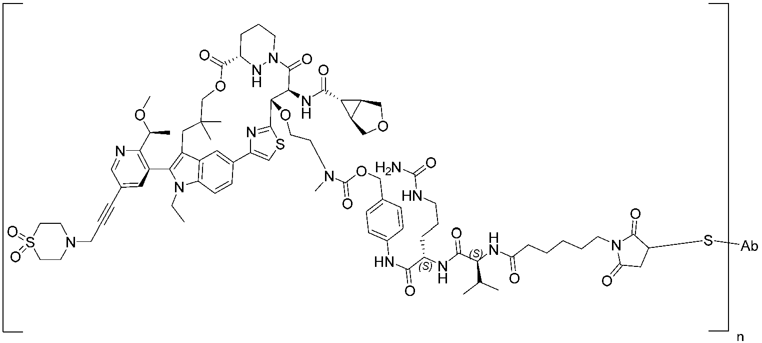 |
| Z297-A-L72-Ab | 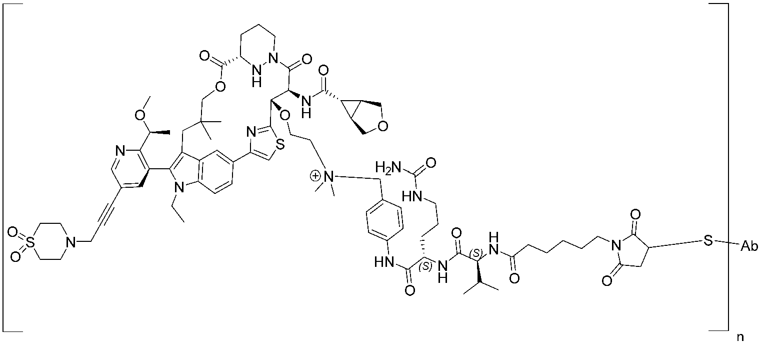 |
| Z284-L72-Ab | 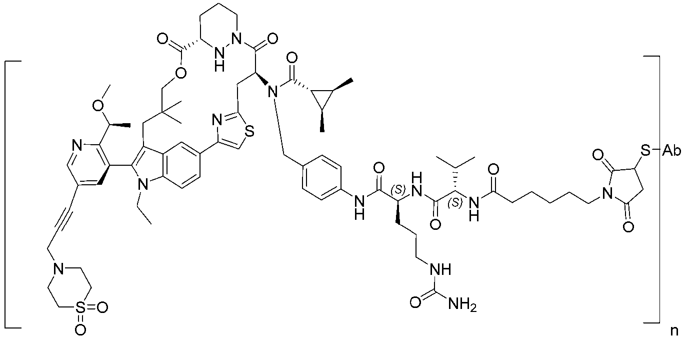 |
| Z192-C-L72-Ab | 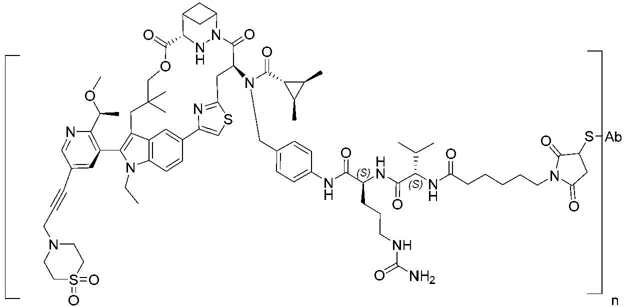 |
| Z283-L7-Ab | 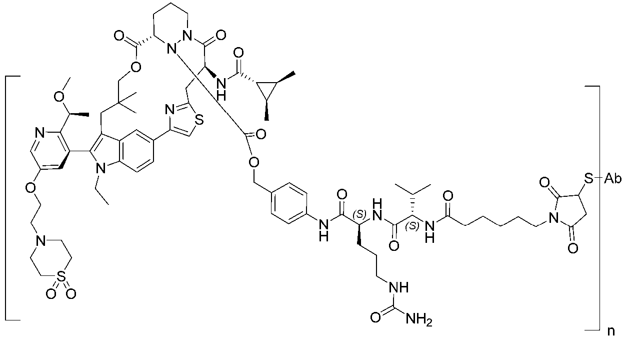 |
| Z298-L7-Ab | 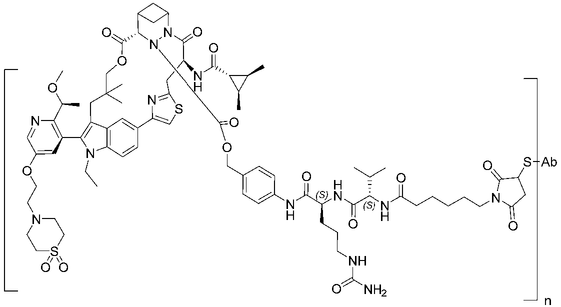 |
| Z299-L52-Ab | 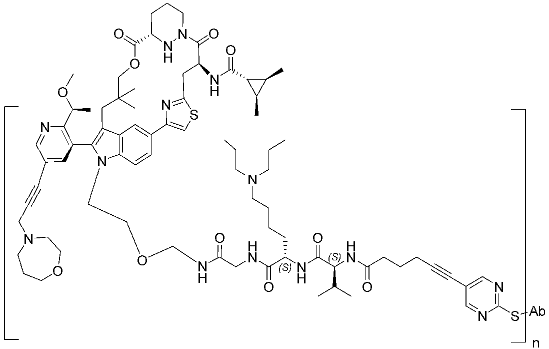 |
| Z187-A-L52-Ab | 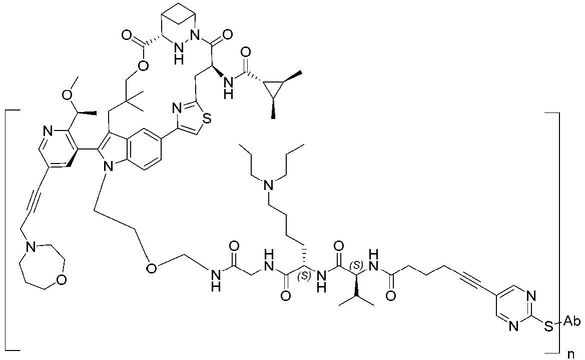 |
| Z300-L7-Ab | 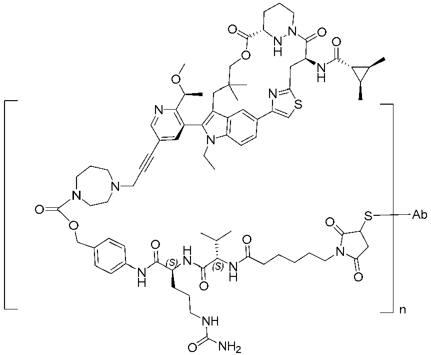 |
| Z301-L7-Ab | 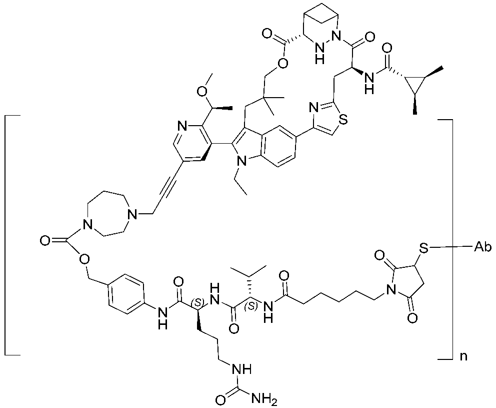 |
| Z299-A-L72-Ab | 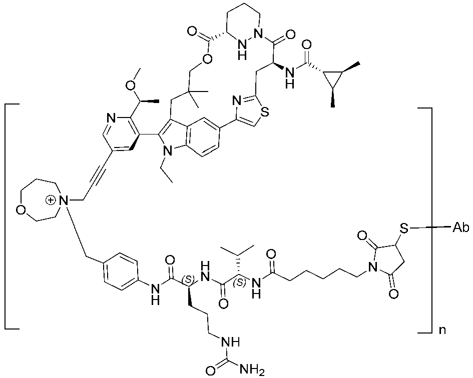 |
| Z187-L72-Ab | 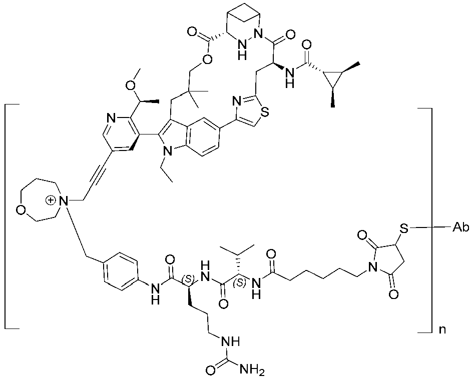 |
| Z302-L7-Ab | 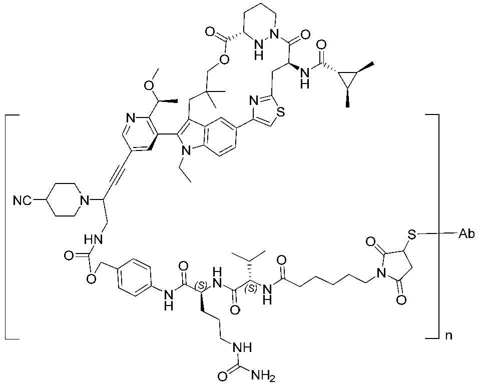 |
| Z303-L7-Ab | 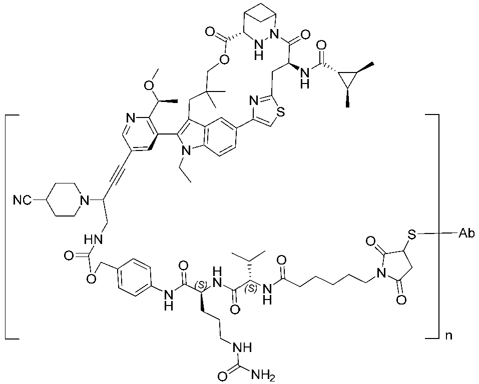 |
| Z7-2-L2-Ab | 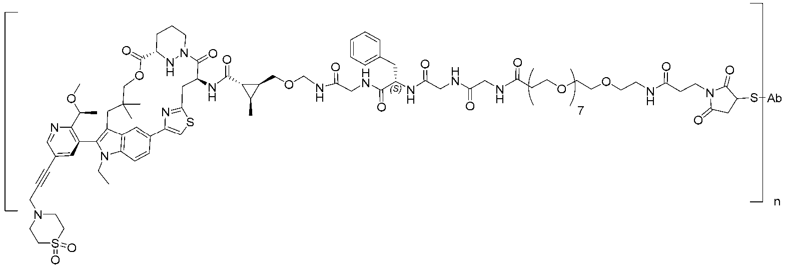 |
| Z192-A-L2-Ab | 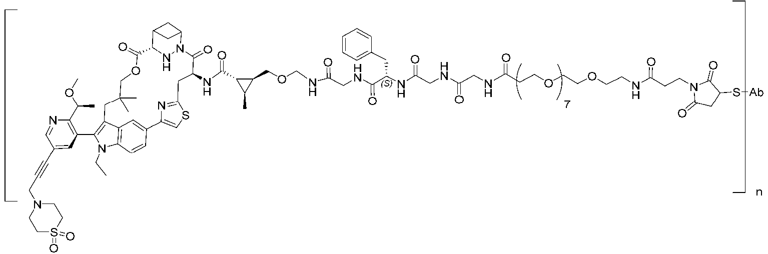 |
| Z304-L54-2-Ab | 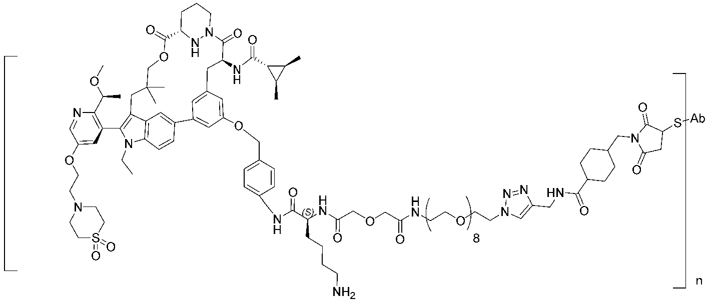 |
| Z311-L74-Ab | 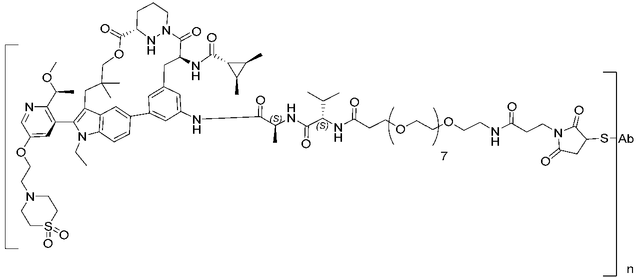 |
| Z305-L54-2-Ab | 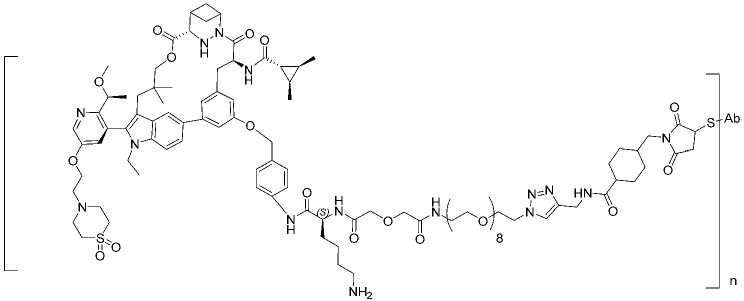 |
| Z305-A-L74-Ab | 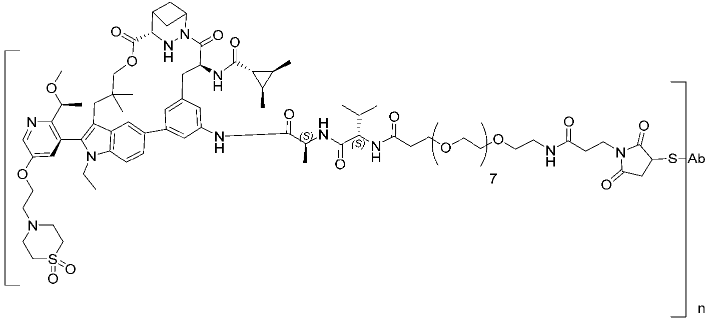 |
| Z306-L7-Ab | 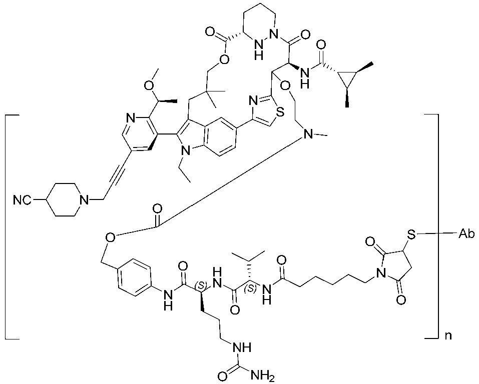 |
| Z307-L7-Ab | 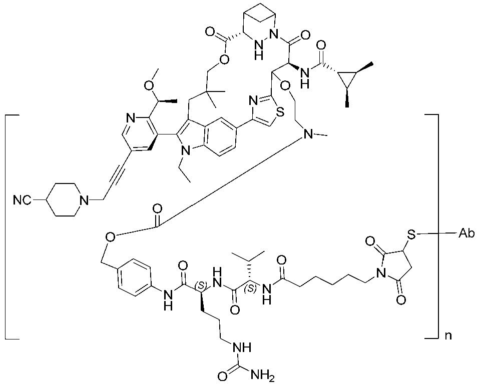 |
| Z282-A-L7-Ab | 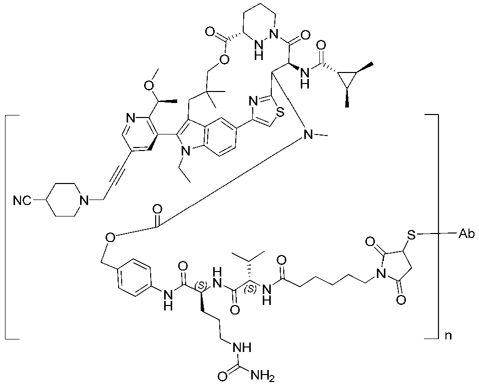 |
| Z308-L7-Ab | 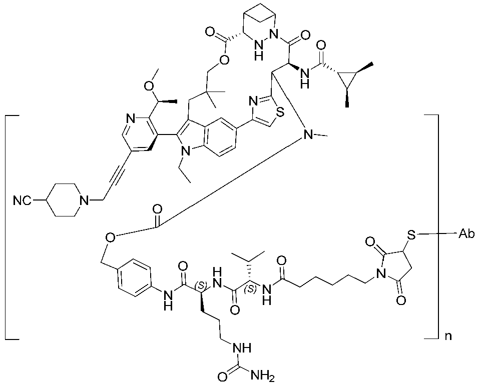 |
| Z67-A-L7-Ab | 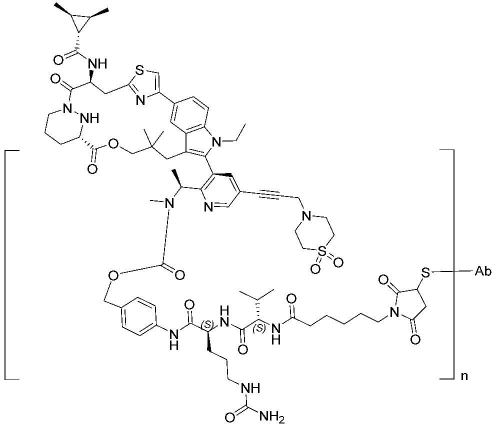 |
| Z309-L7-Ab | 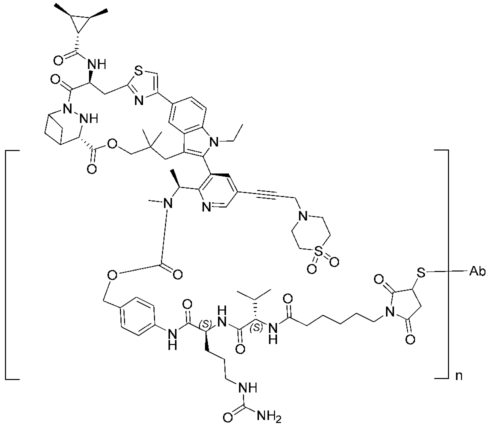 |
| Z52-L58-Ab | 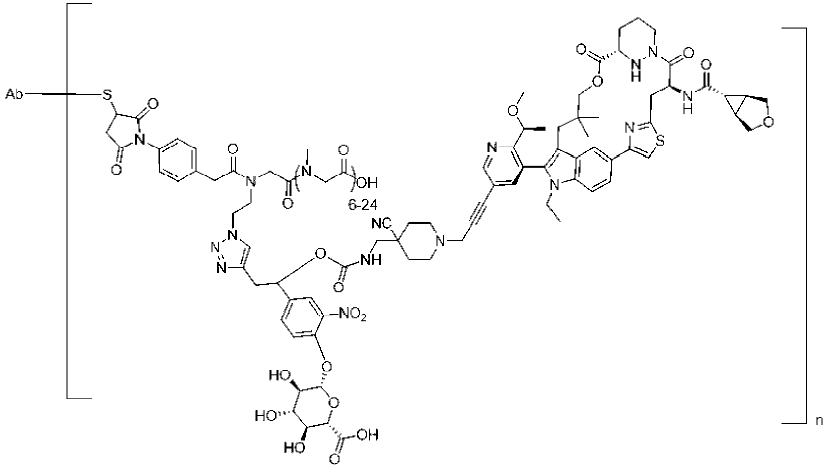 |
| Z52-L59-Ab | 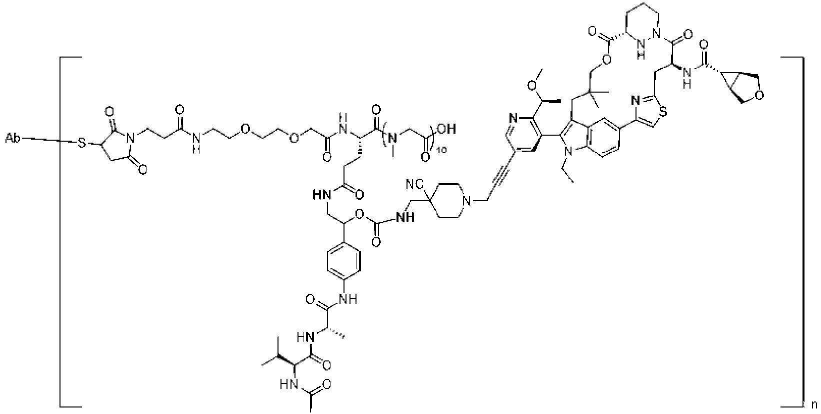 |
| Z52-L60-Ab | 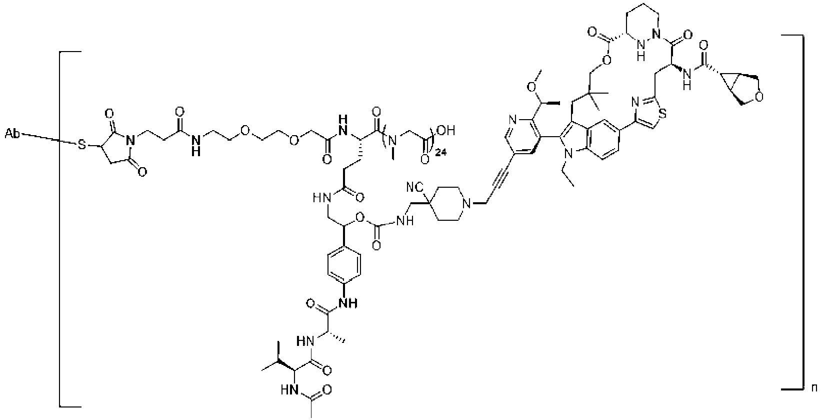 |

wherein n and Ab are as defined in any one of the embodiments.

In one embodiment, 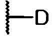 is a compound as shown below, or a stable deuterated derivative thereof, a stereoisomer thereof, or a pharmaceutically acceptable salt thereof: 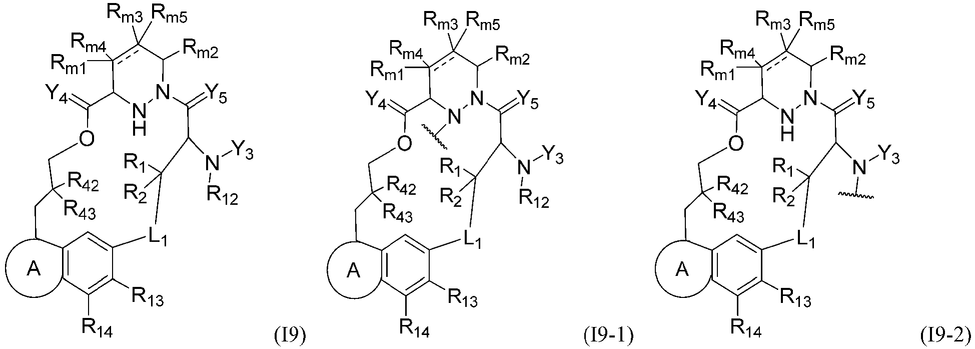 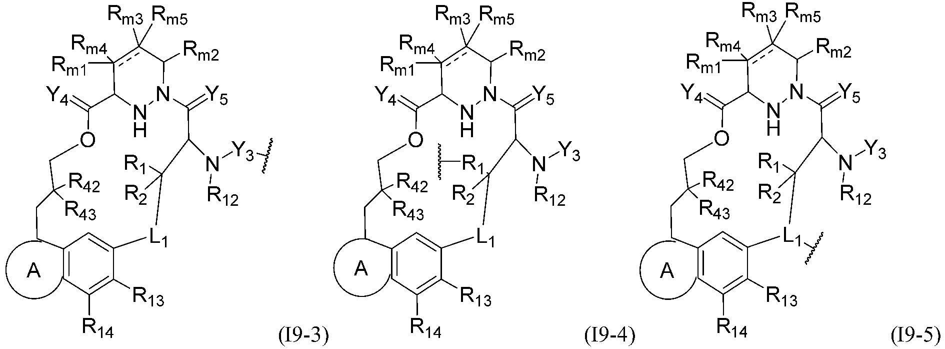 wherein in the formulas,
Rₘ₁ is hydrogen; Rₘ₂ is hydrogen; Rₘ₃ is hydrogen; Rₘ₄ is hydrogen, halogen, or C₁₋₆ alkyl; Rₘ₅ is hydrogen, halogen, or C₁₋₆ alkyl; and 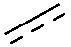 is a single bond; or
Rₘ₁ and Rₘ₃ are both absent; Rₘ₂ is hydrogen; Rₘ₄ is hydrogen, halogen, or C₁₋₆ alkyl; Rₘ₅ is hydrogen, halogen, or C₁₋₆ alkyl; and 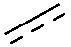 is a double bond; or
Rₘ₁ is attached to Rₘ₂ to jointly form -CRₘ₇Rₘ₆- or -CRₘ₇Rₘ₆-CRₘ₉Rₘ₈-; Rₘ₃ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 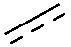 is a single bond; or
Rₘ₁ is attached to Rₘ₃ to jointly form -CRₘ₇Rₘ₆- or -CRₘ₇Rₘ₆-CRₘ₉Rₘ₈-; Rₘ₂ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 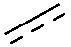 is a single bond; or
Rₘ₁ is attached to Rₘ₄ to jointly form -CRₘ₇Rₘ₆- or -CRₘ₇Rₘ₆-CRₘ₉Rₘ₈-; Rₘ₂ is hydrogen; Rₘ₃ is hydrogen;
Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 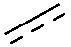 is a single bond;
in formula (I9), 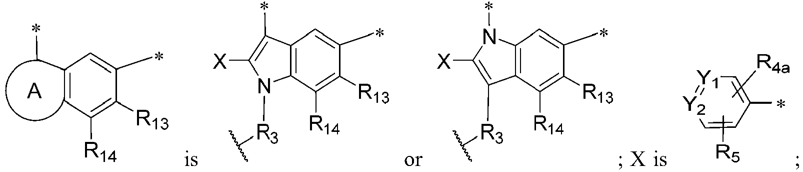 or 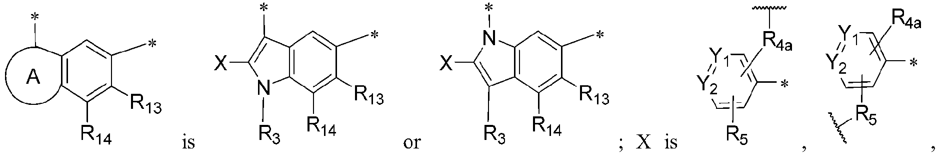 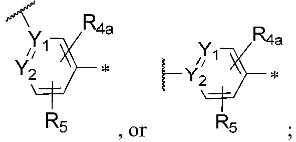
in formula (I9-1), formula (I9-2), formula (I9-3), formula (I9-4), and formula (I9-5), 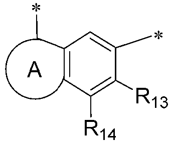 is 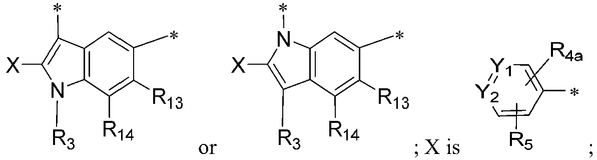
Y₁ is N, CH, CR₄ₐ, CR_{4b}, or CR₅, wherein N may be oxidized (N⁺-O⁻);
Y₂ is N, CH, CR₄ₐ, CR_{4b}, or CR₅, wherein N may be oxidized (N⁺-O⁻);
R₄₂ is hydrogen, halogen, or substituted or unsubstituted C₁₋₆ alkyl; R₄₃ is hydrogen, halogen, or substituted or unsubstituted C₁₋₆ alkyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ₐ is C₁₋₆ alkyl or deuterated C₁₋₆ alkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl or substituted or unsubstituted 8- to 10-membered bicyclic heteroaryl, or -P(=O)-(C₁₋₆ alkyl)₂;
R₄ₐ is substituted or unsubstituted C₁₋₆ alkyl, substituted or unsubstituted C₃₋₆ cycloalkyl, or substituted or unsubstituted 3- to 8-membered heterocyclyl;
R_{4b} is deuterium, halogen, substituted or unsubstituted C₁₋₆ alkyl, or substituted or unsubstituted C₁₋₆ heteroalkyl; R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, - O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄, or -S(=O)(-C₁₋₄ alkylene-C(R₀₅)R₀₃R₀₄,
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 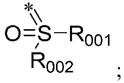 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NR₆-S(=O)(R₀₇)(=N-R₀₈), -C(=O)-C₁₋₄ alkylene-N=S(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl;
R₀₇ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
R₀₈ is hydrogen, deuterium, substituted or unsubstituted C₁₋₆ alkyl, or substituted or unsubstituted C₃₋₆ cycloalkyl; the substitutions refer to substitutions with one or more (e.g., 1, 2, 3, or 4) groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form substituted or unsubstituted 3- to 8-membered heterocyclyl;
Y₅ is O or S;
Y₄ is O or S;
R₁₃ is hydrogen, halogen, substituted or unsubstituted C₁₋₆ alkyl, or -P(=O)-(C₁₋₆ alkyl)₂; R₁₄ is hydrogen, halogen, substituted or unsubstituted C₁₋₆ alkyl, or -P(=O)-(C₁₋₆ alkyl)₂; or
R₁₃ and R₁₄, together with the carbon atom to which they are attached, form substituted or unsubstituted C₃₋₆ cycloalkyl or substituted or unsubstituted 3- to 8-membered heterocyclyl;
in the groups described above, the substitutions each independently mean that the groups described above are each independently substituted with 1, 2, 3, 4, 5, or 6 groups selected from group S1; the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, -N=S(=O)(R^{g1})(R^{h1}), and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(-O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more (e.g., 1, 2, 3, or 4) atoms of the C₁₋₆ heteroalkyl are heteroatoms selected from nitrogen, oxygen, sulfur, and phosphorus; one or more (e.g., 1, 2, 3, 4, or 5) ring atoms of the 7- to 12-membered bicyclic heterocyclyl are heteroatoms selected from nitrogen, oxygen, sulfur, and phosphorus; one or more (e.g., 1, 2, 3, 4, or 5) ring atoms of the 3- to 20-membered heterocyclyl are heteroatoms selected from nitrogen, oxygen, sulfur, and phosphorus; one or more (e.g., 1, 2, 3, 4, or 5) ring atoms of the 5- to 20-membered heteroaryl are heteroatoms selected from nitrogen, oxygen, sulfur, and phosphorus; one or more (e.g., 1, 2, 3, or 4) ring atoms of the 5- or 6-membered monocyclic heteroaryl are heteroatoms selected from nitrogen, oxygen, sulfur, and phosphorus; one or more (e.g., 1, 2, 3, 4, or 5) ring atoms of the 8- to 10-membered bicyclic heteroaryl are heteroatoms selected from nitrogen, oxygen, sulfur, and phosphorus; one or more (e.g., 1, 2, 3, or 4) ring atoms of the 3- to 6-membered monocyclic heterocyclyl are heteroatoms selected from nitrogen, oxygen, sulfur, and phosphorus; one or more (e.g., 1, 2, 3, 4, or 5) ring atoms of the 5- to 15-membered tricyclic heterocyclyl are heteroatoms selected from nitrogen, oxygen, sulfur, and phosphorus; one or more (e.g., 1, 2, 3, 4, or 5) ring atoms of the 6- to 12-membered heteroaryl-fused cycloalkyl are heteroatoms selected from nitrogen, oxygen, sulfur, and phosphorus; one or more (e.g., 1, 2, 3, 4, or 5) ring atoms of the 6- to 12-membered heteroaryl-fused heterocyclyl are heteroatoms selected from nitrogen, oxygen, sulfur, and phosphorus;
wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above;
the 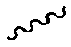 end is attached to L₀, and the * end is attached to another fragment in D.
In one embodiment, Rₘ₁ is hydrogen; Rₘ₂ is hydrogen; Rₘ₃ is hydrogen; Rₘ₄ is hydrogen; Rₘ₅ is hydrogen; and 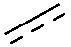 is a single bond.
In one embodiment, Y₄ is O; Y₅ is O.
In one embodiment, when the following conditions are satisfied, R₅ is not methylpiperazinyl: R₆ is dimethylcyclopropyl; Y₄ is O; Y₅ is O; R₁ and R₂ are hydrogen; L₁ is thiazole; R₄₂ and R₄₃ are methyl; R₁₃ and R₁₄ are hydrogen; and R₃ is ethyl.
In one embodiment, R₃ is substituted or unsubstituted C₁₋₆ alkyl.
In one embodiment, R₃ is unsubstituted C₁₋₆ alkyl or C₁₋₆ haloalkyl.
In one embodiment, R₅ is -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, or -C₁₋₄ alkylene-C₂₋₄ alkynylene-C₁₋₄ alkylene-NR₀₁R₀₂.
In one embodiment, R₅ is -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, or -C₁₋₄ alkylene-C₂₋₄ alkynylene-C₁₋₄ alkylene-NR₀₁R₀₂;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 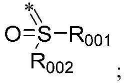 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl.
In one embodiment, R₅ is -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, or -C₁₋₄ alkylene-C₂₋₄ alkynylene-C₁₋₄ alkylene-NR₀₁R₀₂;
R₀₁ and R₀₂ are each independently selected from hydrogen and substituted or unsubstituted C₁₋₆ alkyl; or
R₀₁ and R₀₂, together with the nitrogen atom to which they are attached, form substituted or unsubstituted azetidinyl, substituted or unsubstituted oxazepanyl, substituted or unsubstituted dioxepanyl, substituted or unsubstituted morpholinyl, substituted or unsubstituted pyrrolidinyl, substituted or unsubstituted piperidinyl, substituted or unsubstituted piperazinyl, substituted or unsubstituted 3-oxa-8-azabicyclo[3.2.1]octyl, substituted or unsubstituted tetrahydro-1H-pyrrolizinyl, substituted or unsubstituted thiomorpholinyl, substituted or unsubstituted tetrahydro-5H-pyrazolo[4,3-c]pyridinyl, substituted or unsubstituted 1,2,5-thiadiazepanyl, substituted or unsubstituted 1,4-thiazepanyl, substituted or unsubstituted 3,8-diazabicyclo[3.2.1]octyl, or substituted or unsubstituted 4-oxa-7-azaspiro[2.5]octyl;
R₀₃ and R₀₄, together with the carbon atom to which they are attached, form substituted or unsubstituted azetidinyl, substituted or unsubstituted oxazepanyl, substituted or unsubstituted dioxepanyl, substituted or unsubstituted morpholinyl, substituted or unsubstituted pyrrolidinyl, substituted or unsubstituted piperidinyl, substituted or unsubstituted piperazinyl, substituted or unsubstituted 3-oxa-8-azabicyclo[3.2.1]octyl, substituted or unsubstituted tetrahydro-1H-pyrrolizinyl, substituted or unsubstituted thiomorpholinyl, substituted or unsubstituted tetrahydro-5H-pyrazolo[4,3-c]pyridinyl, substituted or unsubstituted 1,2,5-thiadiazepanyl, substituted or unsubstituted 1,4-thiazepanyl, substituted or unsubstituted 3,8-diazabicyclo[3.2.1]octyl, substituted or unsubstituted 4-oxa-7-azaspiro[2.5]octyl, or substituted or unsubstituted cyclobutyl;
R₀₅ is hydrogen, deuterium, halogen, deuterated C₁₋₆ alkyl, or C₁₋₆ alkyl.
In one embodiment, R₅ is -C₂₋₄ alkynylene-NR₀₁R₀₂, -C₁₋₄ alkylene-C₂₋₄ alkynylene-NR₀₁R₀₂, -C₂₋₄ alkynylene-C₁₋₄ alkylene-NR₀₁R₀₂, -C₂₋₄ alkynylene-C(=O)-NR₀₁R₀₂, -C₁₋₄ alkylene-C₂₋₄ alkynylene-C(R₀₅)R₀₃R₀₄, -C₂₋₄ alkynylene-C₁₋₄ alkylene-C(R₀₅)R₀₃R₀₄, or -C₂₋₄ alkynylene-C(R₀₅)R₀₃R₀₄, R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -C₂₋₄ alkynylene-NR₀₁R₀₂, -C₁₋₄ alkylene-C₂₋₄ alkynylene-NR₀₁R₀₂, -C₂₋₄ alkynylene-C₁₋₄ alkylene-NR₀₁R₀₂, or -C₁₋₄ alkylene-C₂₋₄ alkynylene-C₁₋₄ alkylene-NR₀₁R₀₂; R₀₁ and R₀₂, together with the nitrogen atom to which they are attached, form substituted or unsubstituted 5- to 15-membered tricyclic heterocyclyl.

In one embodiment, R₅ is -C₂₋₄ alkynylene-NR₀₁R₀₂, -C₁₋₄ alkylene-C₂₋₄ alkynylene-NR₀₁R₀₂, -C₂₋₄ alkynylene-C₁₋₄ alkylene-NR₀₁R₀₂, or -C₁₋₄ alkylene-C₂₋₄ alkynylene-C₁₋₄ alkylene-NR₀₁R₀₂; R₀₁ and R₀₂, together with the nitrogen atom to which they are attached, form substituted or unsubstituted 7- to 12-membered bicyclic heterocyclyl.

In one embodiment, R₅ is -C₂₋₄ alkynylene-NR₀₁R₀₂, -C₁₋₄ alkylene-C₂₋₄ alkynylene-NR₀₁R₀₂, -C₂₋₄ alkynylene-C₁₋₄ alkylene-NR₀₁R₀₂, or -C₁₋₄ alkylene-C₂₋₄ alkynylene-C₁₋₄ alkylene-NR₀₁R₀₂; R₀₁ and R₀₂, together with the nitrogen atom to which they are attached, form substituted or unsubstituted 3- to 8-membered monocyclic heterocyclyl.

In one embodiment, R₅ is -C₂₋₄ alkynylene-C(R₀₅)R₀₃R₀₄; R₀₃ and R₀₄, together with the carbon atom to which they are attached, form substituted or unsubstituted 7- to 12-membered bicyclic heterocyclyl; R₀₅ is hydrogen, deuterium, halogen, deuterated C₁₋₆ alkyl, or C₁₋₆ alkyl.

In one embodiment, R₅ is -C₂₋₄ alkynylene-C(R₀₅)R₀₃R₀₄; R₀₃ and R₀₄, together with the carbon atom to which they are attached, form substituted or unsubstituted 5- to 15-membered tricyclic heterocyclyl; R₀₅ is hydrogen, deuterium, halogen, deuterated C₁₋₆ alkyl, or C₁₋₆ alkyl.

In one embodiment, R₅ is -C₂₋₄ alkynylene-C(R₀₅)R₀₃R₀₄; R₀₃ and R₀₄, together with the carbon atom to which they are attached, form substituted or unsubstituted 3- to 8-membered monocyclic heterocyclyl; R₀₅ is hydrogen, deuterium, halogen, deuterated C₁₋₆ alkyl, or C₁₋₆ alkyl.

In one embodiment, R₅ is -C₂₋₄ alkynylene-C(R₀₅)R₀₃R₀₄; R₀₃ and R₀₄, together with the carbon atom to which they are attached, form substituted or unsubstituted 5- or 6-membered monocyclic heteroaryl or substituted or unsubstituted 8- to 10-membered bicyclic heteroaryl; R₀₅ is hydrogen, deuterium, halogen, deuterated C₁₋₆ alkyl, or C₁₋₆ alkyl.

In one embodiment, R₅ is -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, or -O-substituted or unsubstituted 7- to 12-membered bicyclic heterocyclyl;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 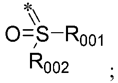 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -O-C₁₋₄ alkylene-NR₀₁R₀₂; R₀₁ and R₀₂, together with the nitrogen atom to which they are attached, form substituted or unsubstituted 5- to 15-membered tricyclic heterocyclyl.

In one embodiment, R₅ is -O-C₁₋₄ alkylene-NR₀₁R₀₂; R₀₁ and R₀₂, together with the nitrogen atom to which they are attached, form substituted or unsubstituted 7- to 12-membered bicyclic heterocyclyl.

In one embodiment, R₅ is -O-C₁₋₄ alkylene-NR₀₁R₀₂; R₀₁ and R₀₂, together with the nitrogen atom to which they are attached, form substituted or unsubstituted 3- to 8-membered monocyclic heterocyclyl.

In one embodiment, R₅ is -O-C₁₋₄ alkylene-substituted or unsubstituted 3- to 8-membered monocyclic heterocyclyl.

In one embodiment, R₅ is -O-C₁₋₄ alkylene-substituted or unsubstituted 5- to 15-membered tricyclic heterocyclyl. In one embodiment, R₅ is -O-C₁₋₄ alkylene-substituted or unsubstituted 7- to 12-membered bicyclic heterocyclyl. In one embodiment, R₅ is -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -C₁₋₄ alkylene-NR₀₁R₀₂, -C₁₋₄ alkylene-C(R₀₅)R₀₃R₀₄, -CH=CR₀₁R₀₂, -C₂₋₄ alkenylene-NR₀₁R₀₂, -C₂₋₄ alkenylene-C(R₀₅)R₀₃R₀₄, -C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), - S(=O)(-C₁₋₄ alkylene-NR₀₁R₀₂), -S(=O)₂(-C₁₋₄ alkylene-C(R₀₅)R₀₃R₀₄, or -S(=O)(-C₁₋₄ alkylene-C(R₀₅)R₀₃R₀₄; R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 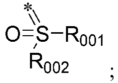 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₅ is -NR₀₁R₀₂, -C₁₋₄ alkylene-NR₀₁R₀₂, -C₁₋₄ alkylene-C(=O)-NR₀₁R₀₂, -C₁₋₄ alkylene-N(R₀₆)C(=O)-C₁₋₄ alkylene-NR₀₁R₀₂, -O-C₁₋₄ alkylene-NR₀₁R₀₂, -O-C₁₋₄ alkylene-C(R₀₅)R₀₃R₀₄, -O-C(R₀₅)R₀₃R₀₄, -C₁₋₄ alkylene-NR₀₁R₀₂, -N(R₀₆)C(=O)-C(R₀₅)R₀₃R₀₄, or -CH=CR₀₁R₀₂; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl;
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
R₀₆ is hydrogen, deuterium, or C₁₋₆ alkyl.

In one embodiment, R₀₁ and R₀₂, together with the nitrogen atom to which they are attached, form substituted or unsubstituted 5- to 15-membered tricyclic heterocyclyl.

In one embodiment, R₀₁ and R₀₂, together with the nitrogen atom to which they are attached, form substituted or unsubstituted 7- to 12-membered bicyclic heterocyclyl.

In one embodiment, R₀₁ and R₀₂, together with the nitrogen atom to which they are attached, form substituted or unsubstituted 3- to 8-membered monocyclic heterocyclyl.

In one embodiment, Y₃ is -C(=O)R₆; R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl.

In one embodiment, Y₃ is R₁₁; R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, substituted or unsubstituted 6- to 12-membered heteroaryl-fused heterocyclyl, substituted or unsubstituted 7- to 12-membered bicyclic heterocyclyl, or substituted or unsubstituted 5- to 15-membered tricyclic heterocyclyl.

In one embodiment, R₁₂ is hydrogen.

In one embodiment, in the groups described herein, the substitutions each independently mean that the groups described above are each independently substituted with 1, 2, 3, 4, 5, or 6 groups selected from group S1.

In one embodiment, the groups of group S1 are each independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₈ cycloalkyl, 3- to 8-membered heterocyclyl, phenyl, 5- or 6-membered monocyclic heteroaryl, 8- to 10-membered bicyclic heteroaryl, -O-C₃₋₈ cycloalkyl, -O-3- to 8-membered heterocyclyl, -O-phenyl, -O-5- or 6-membered monocyclic heteroaryl, -O-8- to 10-membered bicyclic heteroaryl, -C≡C-C₃₋₈ cycloalkyl, -C≡C-3- to 8-membered heterocyclyl, -C≡C-phenyl, -C≡C-5- or 6-membered monocyclic heteroaryl, -C≡C-8- to 10-membered bicyclic heteroaryl, -C≡C-C₁₋₄ alkylene-C₃₋₈ cycloalkyl, -C≡C-C₁₋₄ alkylene-3- to 8-membered heterocyclyl, -C≡C-C₁₋₄ alkylene-phenyl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₈ cycloalkyl, -C₁₋₄ alkylene-O-C₃₋₈ cycloalkyl, -C₁₋₄ alkylene-3- to 8-membered heterocyclyl, -C₁₋₄ alkylene-O-3- to 8-membered heterocyclyl, - C₁₋₄ alkylene-phenyl, -C₁₋₄ alkylene-O-phenyl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₈ cycloalkyl, -O-C₁₋₄ alkylene-O-C₃₋₈ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-phenyl, -O-C₁₋₄ alkylene-O-phenyl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₈ cycloalkyl, -S(=O)₂-3- to 20-membered heterocyclyl, -C(=O)O-C₁₋₆ alkyl, -C(=O)O-C₃₋₈ cycloalkyl, - C(=O)-C₁₋₆ alkyl, -C(=O)-C₃₋₈ cycloalkyl, -C(=O)-phenyl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₈ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₈ cycloalkyl, -C₁₋₄ alkylene-C(=O)-C₁₋₆ alkyl, -C₁₋₄ alkylene-C(=O)-C₃₋₈ cycloalkyl, -C₁₋₄ alkylene-C(=O)-phenyl, -C(=O)-5- or 6-membered monocyclic heteroaryl, -C(=O)-8- to 10-membered bicyclic heteroaryl, -C(=O)-C₁₋₆ alkyl-C₃₋₈ cycloalkyl, -C(=O)-C₁₋₆ alkyl-3- to 8-membered heterocyclyl, -C(=O)-C₁₋₆ alkyl-phenyl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, - C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)2-NR^{a1}R^{b1}, -NR^{d1}-C(=O)-R^{c1}, -NR^{d1}-C(=O)-C₁₋₄ alkylene-R^{c1}, -NR^{d1}-C(=O)-NR^{a1}R^{b1}, - NR^{d1}-S(=O)₂-R^{c1}, -S(=O)₂-NR^{a1}R^{b1}, -NR^{d1}-S(=O)₂-NR^{a1}R^{b1}, -N=S(=O)(R^{g1})(R^{h1}), and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₈ cycloalkyl, 3-to 8-membered heterocyclyl, phenyl, 5- or 6-membered monocyclic heteroaryl, and 8- to 10-membered bicyclic heteroaryl are each independently and optionally substituted with 1, 2, 3, or 4 groups selected from group S2; the nitrogen atom(s) in the 5- or 6-membered monocyclic heteroaryl may optionally be oxidized (N⁺-O⁻).

In one embodiment, the groups of group S1 are each independently selected from the group consisting of: oxo (=O), thio (=S), =CR^{e}R^{f}, =NR^{e}, halogen, cyano, hydroxy, C₁₋₆ alkyl, C₁₋₆ alkoxy, phenyl, 5- or 6-membered monocyclic heteroaryl, -C₁₋₄ alkylene-hydroxy, -C₁₋₄ alkylene-cyano, -C₁₋₄ alkylene-5- or 6-membered monocyclic heteroaryl, -S(=O)₂-C₁₋₆ alkyl, -C(=O)O-C₁₋₆ alkyl, -C(=O)-C₁₋₆ alkyl, -C(=O)-NR^{a1}R^{b1}, - N=S(=O)(R^{g1})(R^{h1}), -P(=O)-(C₁₋₆ alkyl)₂, -C(=O)-C₁₋₄ alkylene-hydroxy, and -C(=O)-C₁₋₄ alkylene-cyano, wherein the C₁₋₆ alkyl is optionally substituted with 1, 2, or 3 groups selected from halogen, deuterium, cyano, and hydroxy; the phenyl or the 5- or 6-membered monocyclic heteroaryl is optionally substituted with 1, 2, 3, or 4 groups selected from group S2; the nitrogen atom(s) in the 5- or 6-membered monocyclic heteroaryl may optionally be oxidized (N⁺-O⁻).

In one embodiment, in the groups described herein, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, - S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkylene-S(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, - N(C₁₋₆ alkyl)₂, -C₁₋₄ alkylene-P(=O)-(C₁₋₆ alkyl)₂, and -P(=O)-(C₁₋₆ alkyl)₂.

In one embodiment, in the groups described herein, each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂.

In one embodiment, in the groups described herein, each R^{d1} is independently H, C₁₋₆ alkyl, or deuterated C₁₋₆ alkyl.

In one embodiment, in the groups described herein, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂.

In one embodiment, in the groups described herein, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl.

In one embodiment, in the groups described herein, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅.

In one embodiment, in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f}.

In one embodiment, in the groups described above, the -C₁₋₄ alkylene- is unsubstituted.

In one embodiment, in the groups described above, 1, 2, 3, or 4 hydrogen atoms on the -C₁₋₄ alkylene- are each independently substituted with a group selected from C₁₋₄ alkyl, C₁₋₄ haloalkyl, and deuterated C₁₋₄ alkyl.

In one embodiment, in the groups described above, 2 hydrogen atoms on the same carbon atom of the -C₁₋₄ alkylene- are both substituted with -(CH₂)ⱼ- to form cycloalkyl, wherein j is 2, 3, 4, 5, or 6.

In one embodiment, in the groups described above, 2 hydrogen atoms on the same carbon atom of the -C₁₋₄ alkylene- are both substituted with =CR^{e}R^{f}.

In one embodiment, in the groups described herein, each R^{e} is independently H, halogen, C₁₋₆ alkyl, C₁₋₆ haloalkyl, or deuterated C₁₋₆ alkyl. Further, each R^{e} is independently H or halogen.

In one embodiment, in the groups described herein, each R^{f} is independently H, halogen, C₁₋₆ alkyl, C₁₋₆ haloalkyl, or deuterated C₁₋₆ alkyl. Further, each R^{f} is independently H or halogen.

In one embodiment, in the groups described herein, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus.

In one embodiment, in the groups described herein, one or more (e.g., 1, 2, 3, or 4) atoms of the C₁₋₆ heteroalkyl are heteroatoms selected from nitrogen, oxygen, sulfur, and phosphorus.

In one embodiment, in the groups described herein, one or more (e.g., 1, 2, 3, 4, or 5) ring atoms of the 7- to 12-membered bicyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 3- to 20-membered heterocyclyl are heteroatoms selected from nitrogen, oxygen, sulfur, and phosphorus.

In one embodiment, in the groups described herein, one or more (e.g., 1, 2, 3, 4, or 5) ring atoms of the 5- to 20-membered heteroaryl are heteroatoms selected from nitrogen, oxygen, sulfur, and phosphorus.

In one embodiment, in the groups described herein, one or more (e.g., 1, 2, 3, or 4) ring atoms of the 5- or 6-membered monocyclic heteroaryl are heteroatoms selected from nitrogen, oxygen, sulfur, and phosphorus.

In one embodiment, in the groups described herein, one or more (e.g., 1, 2, 3, 4, or 5) ring atoms of the 8- to 10-membered bicyclic heteroaryl are heteroatoms selected from nitrogen, oxygen, sulfur, and phosphorus.

In one embodiment, in the groups described herein, one or more (e.g., 1, 2, 3, or 4) ring atoms of the 3- to 6-membered monocyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 5- to 15-membered tricyclic heterocyclyl are heteroatoms selected from nitrogen, oxygen, sulfur, and phosphorus.

In one embodiment, in the groups described herein, one or more (e.g., 1, 2, 3, 4, or 5) ring atoms of the 6- to 12-membered heteroaryl-fused cycloalkyl are heteroatoms selected from nitrogen, oxygen, sulfur, and phosphorus. In one embodiment, in the groups described herein, one or more (e.g., 1, 2, 3, 4, or 5) ring atoms of the 6- to 12-membered heteroaryl-fused heterocyclyl are heteroatoms selected from nitrogen, oxygen, sulfur, and phosphorus. In one embodiment, in the groups described herein, when the ring atom is a sulfur atom, the sulfur atom is optionally substituted with one or two groups selected from oxo and =NR^{e}; R^{e} is as defined above.

In one embodiment, in the groups described herein, when the ring atom is a phosphorus atom, the phosphorus atom is optionally substituted with one or two groups selected from oxo and =NR^{e}; R^{e} is as defined above.

In one embodiment, 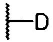 is a compound as shown below, or a stable deuterated derivative thereof, a stereoisomer thereof, or a pharmaceutically acceptable salt thereof: 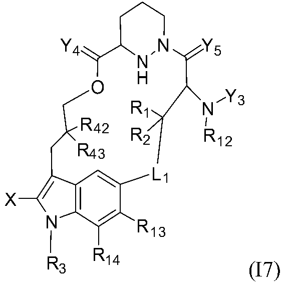 wherein in the formula,
X is 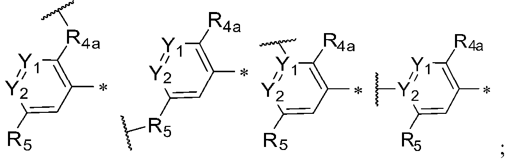
Y₄, Y₅, Y₃, R₁, R₂, R₄₂, R₄₃, R₁₂, L₁, R₃, R₁₃, R₁₄, X, R₄ₐ, R₅, Y₁, and Y₂ are as defined above; the 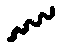 end is attached to L₀, and the * end is attached to another fragment in D.

In one embodiment, in formula (I7-1), formula (I7-2), formula (I7-3), formula (I7-4), formula (I7-5), formula (I7-6), and formula (17),
Y₁ is N or CH;
Y₂ is N or CH;
R₄ₐ is substituted or unsubstituted C₁₋₆ alkyl, or substituted or unsubstituted C₃₋₆ cycloalkyl;
Y₃ is -C(=O)R₆, -C(=S)R₆, -S(=O)(R₀₇)(=N-R₀₈), -S(=O)(R₀₇)(R₀₈), -S(=O)₂(R₀₇)(R₀₈), -P(=O)(R₀₇)(R₀₈), or R₁₁; R₁₂ is hydrogen, C₁₋₆ alkyl, or deuterated C₁₋₆ alkyl;
or Y₃ and R₁₂, together with the nitrogen atom to which they are attached, form substituted or unsubstituted nitrogen-containing 3- to 8-membered heterocyclyl; in the nitrogen-containing 3- to 8-membered heterocyclyl, one ring atom is a nitrogen atom, and optionally one or more (e.g., 1, 2, or 3) ring atoms are heteroatoms selected from nitrogen, oxygen, sulfur, and phosphorus;
in the groups described above, the substitutions each independently mean that 1, 2, 3, 4, 5, or 6 hydrogen atoms on the groups described above are substituted with a group selected from group S1; the groups of group S1 are as defined above.

In one embodiment,
in formula (I7), X is 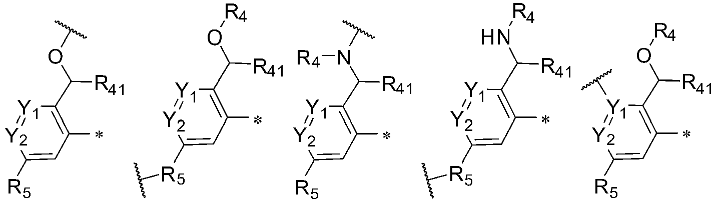 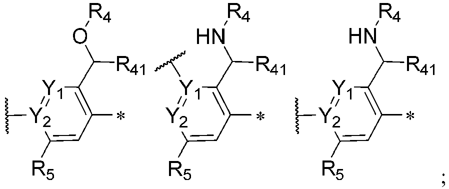
Y₁, Y₂, and R₅ are as defined above;
R₄ is C₁₋₆ alkyl or deuterated C₁₋₆ alkyl;
R₄₁ is C₁₋₆ alkyl or deuterated C₁₋₆ alkyl.

In one embodiment,
R₅ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, - O-C₁₋₄ alkylene-substituted or unsubstituted 3- to 8-membered monocyclic heterocyclyl, -O-C₁₋₄ alkylene-substituted or unsubstituted 5- to 15-membered tricyclic heterocyclyl, -O-C₁₋₄ alkylene-substituted or unsubstituted 7- to 12-membered bicyclic heterocyclyl, -O-substituted or unsubstituted 3- to 8-membered monocyclic heterocyclyl, -O-substituted or unsubstituted 5- to 15-membered tricyclic heterocyclyl, -O-substituted or unsubstituted 7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), - S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), or - S(=O)(-C₁₋₄ alkylene-C(R₀₅)R₀₃R₀₄);
Y₃ is -C(=O)R₆ or R₁₁;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₁ is substituted or unsubstituted 3- to 10-membered heterocyclyl, substituted or unsubstituted 5- or 6-membered monocyclic heteroaryl, substituted or unsubstituted 8- to 10-membered bicyclic heteroaryl, substituted or unsubstituted 6- to 12-membered heteroaryl-fused cycloalkyl, or substituted or unsubstituted 6- to 12-membered heteroaryl-fused heterocyclyl;
R₁₂ is hydrogen, C₁₋₆ alkyl, or deuterated C₁₋₆ alkyl;
in the groups described above, one or more (e.g., 1, 2, 3, or 4) ring atoms of the 3- to 8-membered heterocyclyl, 3- to 10-membered heterocyclyl, or 3- to 8-membered monocyclic heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 7- to 12-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur;
one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur; one or more (e.g., 1, 2, 3, 4, or 5) ring atoms of the 5- to 15-membered tricyclic heterocyclyl are heteroatoms selected from nitrogen, oxygen, and sulfur; one or more (e.g., 1, 2, 3, 4, or 5) ring atoms of the 6- to 12-membered heteroaryl-fused cycloalkyl are heteroatoms selected from nitrogen, oxygen, and sulfur; one or more (e.g., 1, 2, 3, 4, or 5) ring atoms of the 6- to 12-membered heteroaryl-fused heterocyclyl are heteroatoms selected from nitrogen, oxygen, and sulfur, wherein when the ring atom is a sulfur atom, the sulfur atom is optionally substituted with one or two groups selected from oxo and =NR^{e} (for example, when the ring atom is a sulfur atom, the sulfur atom may be substituted to form S(=O), S(=O)₂, or S(=O)(=NR^{e}).); R^{e} is as defined above.

In one embodiment, R₁₃ and R₁₄ are hydrogen.

In one embodiment, R^{4a} is

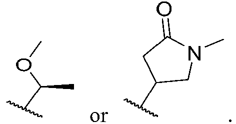

In one embodiment, R^{4a} is

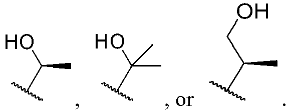

In one embodiment, R^{4a} is C₁₋₆ alkyl, preferably isopropyl.

In one embodiment, R_{4b} is deuterium, halogen, C₁₋₆ alkyl, or C₁₋₆ heteroalkyl.

In one embodiment, R_{4b} is deuterium, halogen, C₁₋₆ alkyl, or C₁₋₆ alkoxy.

In one embodiment, R_{4b} is chlorine, methyl, or methoxy.

In one embodiment, 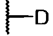 is a compound as shown below, or a stable deuterated derivative thereof, a stereoisomer thereof, or a pharmaceutically acceptable salt thereof: 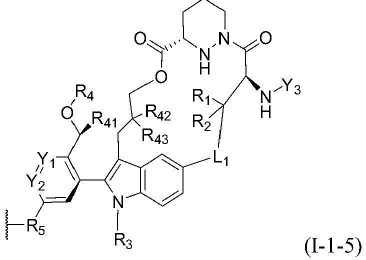 wherein in the formula, R₁, R₂, R₃, R₄, R₅, R₄₁, R₄₂, R₄₃, Y₁, Y₂, Y₃, and L₁ are each as defined herein.

In one embodiment, 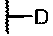 is a compound as shown below, or a stable deuterated derivative thereof, a stereoisomer thereof, or a pharmaceutically acceptable salt thereof: 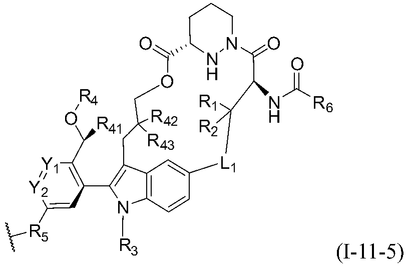 wherein in the formula, R₁, R₂, R₃, R₄, R₅, R₄₁, R₄₂, R₄₃, Y₁, Y₂, R₆, and L₁ are each as defined herein.

In one embodiment, 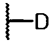 is a compound as shown below, or a stable deuterated derivative thereof, a stereoisomer thereof, or a pharmaceutically acceptable salt thereof: 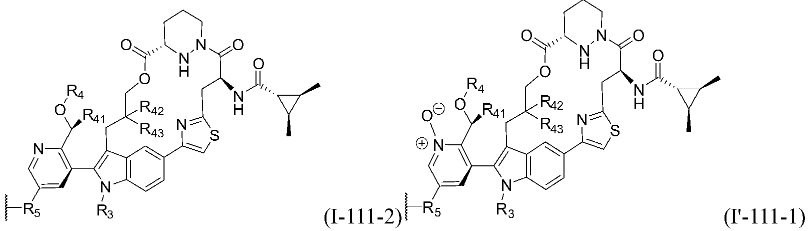 wherein in the formulas, R₃, R₄, R₅, R₄₁, R₄₂, and R₄₃ are each as defined herein.

In one embodiment, 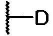 is a compound as shown below, or a stable deuterated derivative thereof, a stereoisomer thereof, or a pharmaceutically acceptable salt thereof: 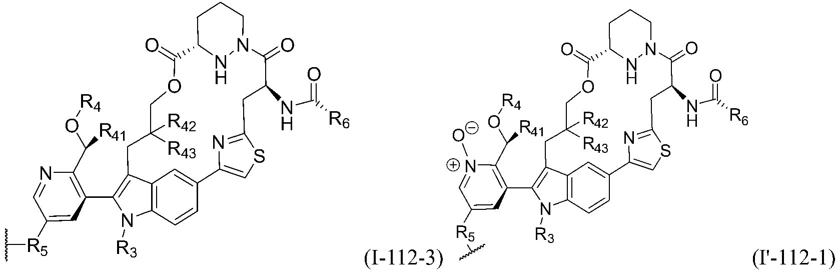 wherein in the formulas, R₃, R₄, R₅, R₄₁, R₄₂, R₄₃, and R₄₆ are each as defined herein.

In one embodiment, 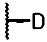 is a compound as shown below, or a stable deuterated derivative thereof, a stereoisomer thereof, or a pharmaceutically acceptable salt thereof: 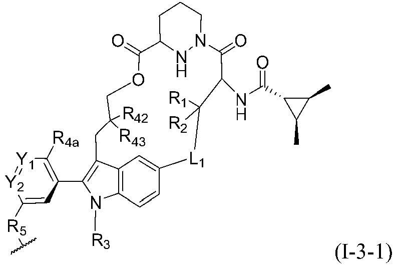 wherein in the formula, Y₁, Y₂, R₁, R₂, R₃, R₄ₐ, R₅, R₄₂, R₄₃, and L₁ are each as defined above.

In one embodiment, 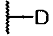 is a compound as shown below, or a stable deuterated derivative thereof, a stereoisomer thereof, or a pharmaceutically acceptable salt thereof: 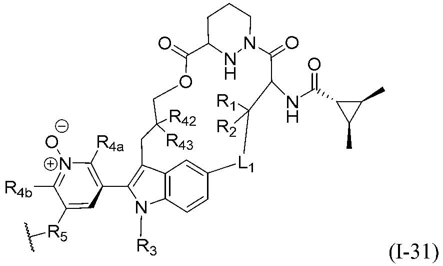 wherein in the formula, R₁, R₂, R₃, R₄ₐ, R₅, R₄₂, R₄₃, and L₁ are each as defined above.

In one embodiment, R_{4b} is halogen, C₁₋₆ alkyl, or C₁₋₆ alkoxy.

In one embodiment, R_{4b} is chlorine, methyl, or methoxy.

In one embodiment, R₄ₐ is C₁₋₆ alkyl, preferably isopropyl.

In one embodiment, R₃ is ethyl.

In one embodiment, R₁ and R₂ are hydrogen.

In one embodiment, R₅ is -NR₀₁R₀₂; R₀₁ and R₀₂, together with the nitrogen atom to which they are attached, form substituted or unsubstituted 7- to 12-membered bicyclic heterocyclyl; one or more (e.g., 1, 2, 3, 4, or 5) ring atoms of the 7- to 12-membered bicyclic heterocyclyl are heteroatoms selected from nitrogen, oxygen, and sulfur; the substitutions each independently mean that 1, 2, 3, 4, 5, or 6 hydrogen atoms on the groups described above are substituted with a group selected from group S1; the groups of group S1 are selected from =CR^{e}R^{f}; each R^{e} is independently H, halogen, C₁₋₆ alkyl, C₁₋₆ haloalkyl, or deuterated C₁₋₆ alkyl; each R^{f} is independently H, halogen, C₁₋₆ alkyl, C₁₋₆ haloalkyl, or deuterated C ₁₋₆ alkyl.

In one embodiment, R₅ is -O-C₁₋₄alkylene-NR₀₁R₀₂; R₀₁ and R₀₂, together with the nitrogen atom to which they are attached, form substituted or unsubstituted 3- to 8-membered monocyclic heterocyclyl; one or more (e.g., 1, 2, 3, or 4) ring atoms of the 3- to 8-membered monocyclic heterocyclyl are heteroatoms selected from nitrogen, oxygen, and sulfur; the substitutions each independently mean that 1, 2, 3, 4, 5, or 6 hydrogen atoms on the groups described above are substituted with a group selected from group S1, wherein when the ring atom is a sulfur atom, the sulfur atom is optionally substituted with one or two groups selected from oxo and =NR^{e}; each R^{e} is independently H, halogen, C₁₋₆ alkyl, C₁₋₆ haloalkyl, or deuterated C₁₋₆ alkyl.

In one embodiment, R₅ is -C₂₋₄alkynylene-C₁₋₄alkylene-NR₀₁R₀₂; R₀₁ and R₀₂, together with the nitrogen atom to which they are attached, form substituted or unsubstituted 3- to 8-membered monocyclic heterocyclyl; one or more (e.g., 1, 2, 3, or 4) ring atoms of the 3- to 8-membered monocyclic heterocyclyl are heteroatoms selected from nitrogen, oxygen, and sulfur; the substitutions each independently mean that 1, 2, 3, 4, 5, or 6 hydrogen atoms on the groups described above are substituted with a group selected from group S1, wherein when the ring atom is a sulfur atom, the sulfur atom is optionally substituted with one or two groups selected from oxo and =NR^{e}; each R^{e} is independently H, halogen, C₁₋₆ alkyl, C₁₋₆ haloalkyl, or deuterated C₁₋₆ alkyl.

In one embodiment, the 7- to 12-membered bicyclic heterocyclyl is selected from the group consisting of:

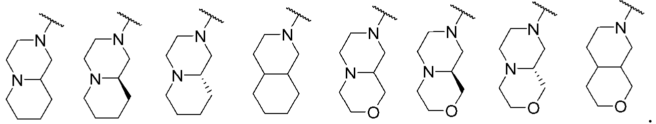

In one embodiment, the 3- to 8-membered monocyclic heterocyclyl is selected from the group consisting of:

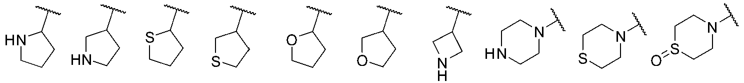

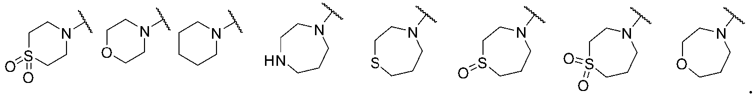

In one embodiment, the 3- to 8-membered monocyclic heterocyclyl is selected from the group consisting of:

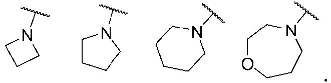

In one embodiment, the 3- to 8-membered monocyclic heterocyclyl is optionally substituted with a substituent selected from cyano, hydroxy, 5- or 6-membered monocyclic heteroaryl, -NR^{a1}R^{b1}, and -N=S(=O)(R^{g1})(R^{h1}). In one embodiment, the 5- or 6-membered monocyclic heteroaryl is pyridine, and the nitrogen atom in the pyridine may optionally be oxidized (N⁺-O⁻).

In one embodiment, R^{a1} and R^{b1} are each independently H or phenyl; the phenyl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂.

In one embodiment, R^{a1} and R^{b1} are each independently H or phenyl; the phenyl is optionally substituted with - P(=O)-(CH₃)₂.

In one embodiment, R^{g1} and R^{h1} are each independently H, C₁₋₆ alkyl, or deuterated C₁₋₆ alkyl, preferably C₁₋₆ alkyl.

In one embodiment, 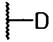 is a compound as shown below, or a stable deuterated derivative thereof, a stereoisomer thereof, or a pharmaceutically acceptable salt thereof: 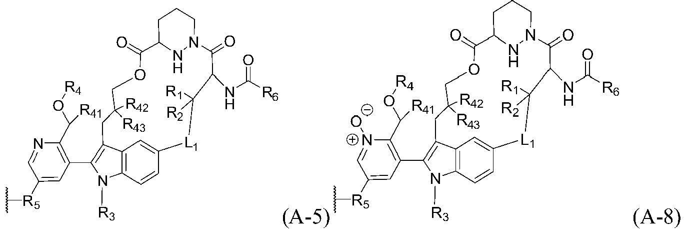 wherein in the formulas,
R₄₂ is hydrogen, halogen, or substituted or unsubstituted C₁₋₆ alkyl; R₄₃ is hydrogen, halogen, or substituted or unsubstituted C₁₋₆ alkyl; and R₄₂ and R₄₃ are not both methyl; or
R₄₂ and R₄₃, together with the carbon atom to which they are attached, form substituted or unsubstituted C₃₋₆ cycloalkyl or substituted or unsubstituted 3- to 8-membered heterocyclyl;
L₁ is -N(R₀₀)C(=O)-, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₄ is C₁₋₆ alkyl or deuterated C₁₋₆ alkyl;
R₄₁ is C₁₋₆ alkyl or deuterated C₁₋₆ alkyl;
R₅ is -NR₀₁R₀₂ or -C(R₀₅)R₀₃R₀₄;
R₀₁ and R₀₂, together with the nitrogen atom to which they are attached, form substituted or unsubstituted 5- to 15-membered tricyclic heterocyclyl; the 5- to 15-membered tricyclic heterocyclyl has, in addition to the existing nitrogen atom, 0 or more (e.g., 0, 1, 2, 3, 4, or 5) ring atoms that are heteroatoms selected from nitrogen, oxygen, and sulfur;
R₀₃ and R₀₄, together with the carbon atom to which they are attached, form substituted or unsubstituted 9- to 11-membered bicyclic heterocyclyl, substituted or unsubstituted 5- to 15-membered tricyclic heterocyclyl, substituted or unsubstituted 5- or 6-membered monocyclic heteroaryl, or substituted or unsubstituted 8- to 10-membered bicyclic heteroaryl; one or more (e.g., 1, 2, 3, 4, or 5) ring atoms of the 5- to 15-membered tricyclic heterocyclyl are heteroatoms selected from nitrogen, oxygen, and sulfur;
R₀₅ is hydrogen, deuterium, or C₁₋₆ alkyl;
R₆ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
the substitutions each independently mean that 1, 2, 3, or 4 hydrogen atoms on the groups described above are substituted with a group selected from group S1; the groups of group S1 are each independently selected from the group consisting of: deuterium, oxo (=O), thio (=S), =CR^{e}R^{f}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, -C₁₋₄ alkylene-O-8- to 10-membered bicyclic heteroaryl, - S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, - C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, -C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, - C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, -S(=O)₂-NR^{a1}R^{b1}, and - NR^{d1}-S(=O)₂-NR^{a1}R^{b1}, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3-to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, and -(C=O)C₁₋₆ alkyl;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl, or 2 hydrogen atoms on the same carbon atom of the -C₁₋₄ alkylene- are both substituted with -(CH₂)ⱼ- to form cycloalkyl, wherein j is 2, 3, 4, 5, or 6;
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 9- to 11-membered bicyclic heterocyclyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur.

In one embodiment, 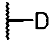 is a compound as shown below, or a stable deuterated derivative thereof, a stereoisomer thereof, or a pharmaceutically acceptable salt thereof: 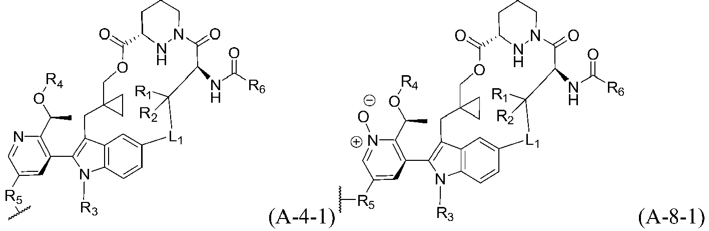 wherein in the formulas, R₁, R₂, R₃, R₄, R₅, R₆, and L₁ are each as defined herein.

In one embodiment, 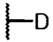 is a compound as shown below, or a stable deuterated derivative thereof, a stereoisomer thereof, or a pharmaceutically acceptable salt thereof: 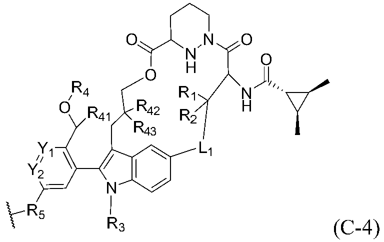 wherein in the formula, Y₁, Y₂, R₁, R₂, R₃, R₄, R₅, R₄₁, R₄₂, R₄₃, and L₁ are each as defined herein.

In one embodiment, R₅ is -NR₀₁R₀₂, -CR₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, - C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C(R₀₅)R₀₃R₀₄, -C₁₋₄ alkylene-NR₀₁R₀₂, -C₁₋₄ alkylene-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(R₀₅)R₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl;
R₀₁ and R₀₂ are each independently selected from C₁₋₆ alkyl; or
R₀₁ and R₀₂, together with the nitrogen atom to which they are attached, form 5- to 8-membered monocyclic heterocyclyl;
the C₁₋₆ alkyl or 5- to 8-membered monocyclic heterocyclyl is optionally substituted with a substituent selected from the aforementioned group S1;
further, the C₁₋₆ alkyl is optionally substituted with -C₁₋₄ alkylene-3- to 20-membered heterocyclyl or -C(=O)-3-to 20-membered heterocyclyl; the 3- to 20-membered heterocyclyl is optionally substituted with a substituent selected from the aforementioned group S2.
R₀₃ and R₀₄ are each independently selected from substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6.

The C₁₋₆ alkyl, C₁₋₆ alkoxy, C₂₋₆ alkenyl, or C₂₋₆ alkynyl is optionally substituted with a substituent selected from the aforementioned group S1.

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, 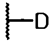 is a compound as shown below, or a stable deuterated derivative thereof, a stereoisomer thereof, or a pharmaceutically acceptable salt thereof: 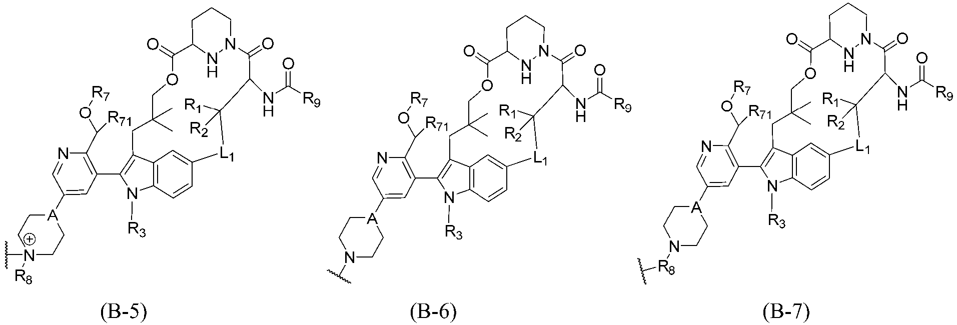 wherein in the formulas,
A is N, C(H), or C(D);
L₁ is -N(R₀₀)C(=O)-, substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₀₀ is hydrogen or C₁₋₆ alkyl;
R₁ is hydrogen, deuterium, halogen, hydroxy, C₁₋₆ alkyl, or substituted or unsubstituted C₁₋₆ heteroalkyl;
R₂ is hydrogen, deuterium, halogen, C₁₋₆ alkyl, or substituted or unsubstituted C₁₋₆ heteroalkyl;
R₃ is hydrogen, substituted or unsubstituted C₁₋₆ alkyl, substituted or unsubstituted C₁₋₆ heteroalkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₇ is C₁₋₆ alkyl or deuterated C₁₋₆ alkyl;
R₇₁ is C₁₋₆ alkyl or deuterated C₁₋₆ alkyl;
R₈ is C₁₋₆ alkyl or deuterated C₁₋₆ alkyl;
and when R₈ does not form a ring with R₁₀, at least one of R₇, R₇₁, and R₈ is deuterated C₁₋₆ alkyl;
R₉ is substituted or unsubstituted C₁₋₆ alkyl, substituted or unsubstituted C₁₋₆ heteroalkyl, substituted or unsubstituted C₃₋₆ cycloalkyl, substituted or unsubstituted 3- to 8-membered heterocyclyl, substituted or unsubstituted C₆₋₁₀ aryl, substituted or unsubstituted 5- or 6-membered monocyclic heteroaryl, or substituted or unsubstituted 8- to 10-membered bicyclic heteroaryl;
R₁₀ is hydrogen, substituted or unsubstituted C₁₋₆ alkyl, or substituted or unsubstituted C₁₋₆ heteroalkyl;
the substitutions described above each independently mean that 1, 2, 3, or 4 hydrogen atoms on the groups described above are substituted with a group selected from group S1; the groups of group S1 are each independently selected from the group consisting of: deuterium, oxo (=O), thio (=S), =CR^{e}R^{f}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C=C-8- to 10-membered bicyclic heteroaryl, -C=C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, -C₁₋₄ alkylene-O-8- to 10-membered bicyclic heteroaryl, -S(=O)₂-C₁₋₆ alkyl, -S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, - C(=O)-NR^{a1}R^{b1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkyleneS(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, -C(=O)-C₁₋₆ alkyl-C₃₋₂₀ cycloalkyl, -C(=O)-C₁₋₆ alkyl-3- to 20-membered heterocyclyl, -C(=O)-C₁₋₆ alkyl-C₆₋₁₄ aryl, - C(=O)-C₁₋₆ alkyl-5- or 6-membered monocyclic heteroaryl, -C(=O)-C₁₋₆ alkyl-8- to 10-membered bicyclic heteroaryl, -C₁₋₄ alkylene-NR^{a1}R^{b1}, -C₁₋₄ alkylene-C(=O)-NR^{a1}R^{b1}, -C=C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-NR^{a1}R^{b1}, -NR^{d1}-S(=O)₂-R^{c1}, - S(=O)₂-NR^{a1}R^{b1}, and -NR^{d1}-S(=O)₂-NR^{a1}R^{b1}, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3-to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
or R₈ and R₁₀, together with the atom(s) to which they are attached, form 3- to 20-membered heterocyclyl unsubstituted or substituted with 1, 2, 3, or 4 groups selected from group S2, 5- or 6-membered monocyclic heteroaryl unsubstituted or substituted with 1, 2, 3, or 4 groups selected from group S2, or 8- to 10-membered bicyclic heteroaryl unsubstituted or substituted with 1, 2, 3, or 4 groups selected from group S2;
in the groups described above, the groups of group S2 are each independently selected from the group consisting of: oxo (C=O), thio (=S), =CR^{e}R^{f}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, and -(C=O)C₁₋₆ alkyl;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl, or 2 hydrogen atoms on the same carbon atom of the C₁₋₄ alkylene are both substituted with -(CH₂)ⱼ- to form cycloalkyl, wherein j is 2, 3, 4, 5, or 6;
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more (e.g., 1, 2, 3, or 4) ring atoms of the 3- to 8-membered heterocyclyl are heteroatoms selected from nitrogen, oxygen, and sulfur; one or more (e.g., 1, 2, 3, or 4) atoms of the C₁₋₆ heteroalkyl are heteroatoms selected from nitrogen, oxygen, and sulfur; one or more (e.g., 1, 2, 3, 4, or 5) ring atoms of the 3- to 20-membered heterocyclyl are heteroatoms selected from nitrogen, oxygen, and sulfur; one or more (e.g., 1, 2, 3, 4, or 5) ring atoms of the 5- to 20-membered heteroaryl are heteroatoms selected from nitrogen, oxygen, and sulfur; one or more (e.g., 1, 2, 3, or 4) ring atoms of the 5- or 6-membered monocyclic heteroaryl are heteroatoms selected from nitrogen, oxygen, and sulfur; one or more (e.g., 1, 2, 3, 4, or 5) ring atoms of the 8- to 10-membered bicyclic heteroaryl are heteroatoms selected from nitrogen, oxygen, and sulfur; one or more (e.g., 1, 2, 3, or 4) ring atoms of the 3- to 6-membered monocyclic heterocyclyl are heteroatoms selected from nitrogen, oxygen, and sulfur.

In one embodiment, R₇ is methyl or deuterated methyl; A is N.

In one embodiment, 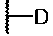 is a compound as shown below, or a stable deuterated derivative thereof, a stereoisomer thereof, or a pharmaceutically acceptable salt thereof: 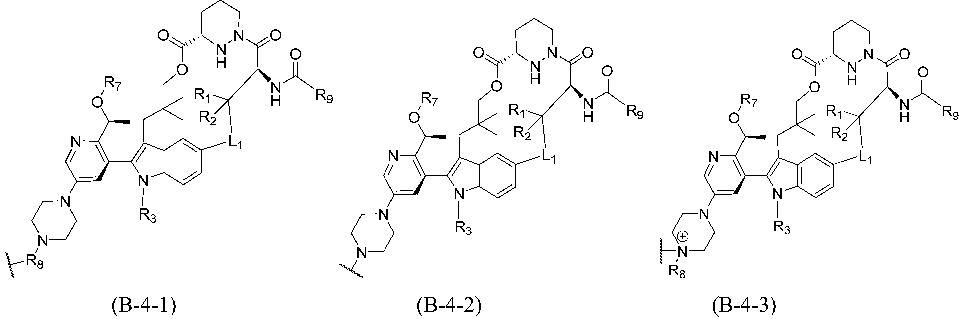 wherein in the formulas, R₁, R₂, R₃, R₇, R₈, R₉, and L₁ are each as defined herein.

In one embodiment, R₇₁ is methyl or deuterated methyl.

In one embodiment, R₇ is methyl or deuterated methyl.

In one embodiment, R₈ is methyl or deuterated methyl.

In one embodiment, R₈ and R₁₀, together with the atom(s) to which they are attached, form 5-membered heterocyclyl unsubstituted or substituted with 1, 2, 3, or 4 groups selected from group S2, 6-membered heterocyclyl unsubstituted or substituted with 1, 2, 3, or 4 groups selected from group S2, or 5- or 6-membered monocyclic heteroaryl unsubstituted or substituted with 1, 2, 3, or 4 groups selected from group S2.

In one embodiment, R₉ is C₁₋₆ haloalkyl. Preferably, R₉ is fluoromethyl, fluoroethyl, fluoropropyl, or fluorobutyl. In one embodiment, R₉ is substituted or unsubstituted C₃₋₆ cycloalkyl.

In one embodiment, R₉ is substituted or unsubstituted cyclopropyl.

In one embodiment, R₉ is cyclopropyl substituted with C₁₋₆ alkyl, preferably cyclopropyl substituted with one or two methyl groups.

In one embodiment, R₉ is

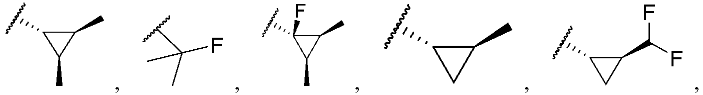

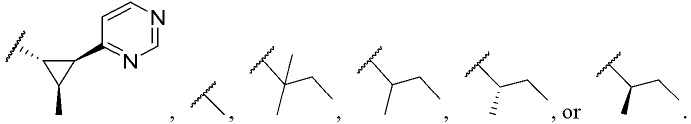

In one embodiment, R₃ is hydrogen or C₁₋₆ alkyl.

In one embodiment, R₃ is methyl, ethyl, or propyl (including isopropyl or n-propyl).

In one embodiment, R₁ is hydrogen or C₁₋₆ alkoxy; R₂ is hydrogen.

In one embodiment, R₁ is hydrogen, methoxy, ethoxy, propoxy, or butoxy; R₂ is hydrogen.

In one embodiment, the substituents of group S2 are selected from the group consisting of: halogen, oxo, methyl, ethyl, halomethyl, haloethyl, deuterated methyl, deuterated ethyl, and =CR^{e}R^{f}.

In one embodiment, each R^{e} is independently H, fluorine, methyl, ethyl, halomethyl, haloethyl, deuterated methyl, or deuterated ethyl; each R^{f} is independently H, fluorine, methyl, ethyl, halomethyl, haloethyl, deuterated methyl, or deuterated ethyl.

In one embodiment, the compound is selected from the group consisting of Z63 and Z66.

In one embodiment, the compound is selected from the group consisting of Z64, Z65, Z67, and Z68.

In one embodiment, R₃ is hydrogen or C₁₋₆ alkyl.

In one embodiment, R₃ is hydrogen, C₁₋₆ haloalkyl, or C₁₋₆ alkyl.

In one embodiment, R₃ is methyl, ethyl, or propyl (including isopropyl or n-propyl).

In one embodiment, R₃ is ethyl.

In one embodiment, R₃ is haloethyl.

In one embodiment, R₃ is trifluoroethyl.

In one embodiment, R₁ is hydrogen or C₁₋₆ alkoxy; R₂ is hydrogen.

In one embodiment, R₁ is hydrogen, methoxy, ethoxy, propoxy, or butoxy; R₂ is hydrogen.

In one embodiment, R₁ is hydrogen; R₂ is hydrogen.

In one embodiment, R₅ is -NR₀₁R₀₂, -CR₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, - C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C(R₀₅)R₀₃R₀₄, -C₁₋₄ alkylene-NR₀₁R₀₂, -C₁₋₄ alkylene-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(R₀₅)R₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl;
R₀₁ and R₀₂ are each independently selected from C₁₋₆ alkyl; or
R₀₁ and R₀₂, together with the nitrogen atom to which they are attached, form 5- to 8-membered monocyclic heterocyclyl;
the C₁₋₆ alkyl or 5- to 8-membered monocyclic heterocyclyl is optionally substituted with a substituent selected from the aforementioned group S1;
further, the C₁₋₆ alkyl is optionally substituted with -C₁₋₄ alkylene-3- to 20-membered heterocyclyl or -C(=O)-3-to 20-membered heterocyclyl; the 3- to 20-membered heterocyclyl is optionally substituted with a substituent selected from the aforementioned group S2;
R₀₃ and R₀₄ are each independently selected from substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6;

The C₁₋₆ alkyl, C₁₋₆ alkoxy, C₂₋₆ alkenyl, or C₂₋₆ alkynyl is optionally substituted with a substituent selected from the aforementioned group S1.

In one embodiment, R₅ is -NR₀₁R₀₂, -C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, - C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C(R₀₅)R₀₃R₀₄, -C₁₋₄ alkylene-NR₀₁R₀₂, -C₁₋₄ alkylene-C(R₀₅)R₀₃R₀₄, - N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(R₀₅)R₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, or -O-7- to 12-membered bicyclic heterocyclyl, wherein each group is as defined herein.

In one embodiment, R₃ is hydrogen, C₁₋₆ alkyl, or deuterated C₁₋₆ alkyl.

In one embodiment, R₃ is methyl, ethyl, or propyl (including isopropyl or n-propyl).

In one embodiment, R₁ is hydrogen, C₁₋₆ alkoxy, or deuterated C₁₋₆ alkoxy; R₂ is hydrogen.

In one embodiment, R₁ is hydrogen, methoxy, ethoxy, propoxy, or butoxy; R₂ is hydrogen.

In one embodiment, L₁ is substituted or unsubstituted 5- or 6-membered monocyclic heteroaryl.

In one embodiment, L₁ is thiazolyl, oxazolyl, or substituted or unsubstituted phenyl.

In one embodiment, Y₁ is N; Y₂ is CH.

In one embodiment, Y₁ is CH; Y₂ is N.

In one embodiment, Y₁ is N; Y₂ is N.

In one embodiment, Y₁ is 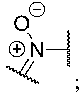 Y₂ is CH.

In one embodiment, Y₁ is CH; Y₂ is

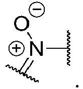

In one embodiment, R₄₃ and R₄₂ are methyl.

In one embodiment, 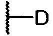 is a compound as shown below, or a stable deuterated derivative thereof, a stereoisomer thereof, or a pharmaceutically acceptable salt thereof: 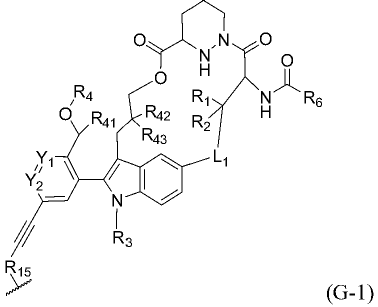
wherein in the formula, R₁, R₂, R₃, R₄, R₆, R₄₁, R₄₂, R₄₃, R₁₁, Y₁, Y₂, and L₁ are each as defined above;
R₁₅ is selected from -NR₀₁R₀₂, -C(=O)-NR₀₁R₀₂, -C(R₀₅)R₀₃R₀₄, -C₁₋₄ alkylene-NR₀₁R₀₂, and -C₁₋₄alkylene-C(R₀₅)R₀₃R₀₄;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 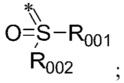 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
the substitutions each independently mean that 1, 2, 3, or 4 hydrogen atoms on the groups described above are substituted with a group selected from group S1; the groups of group S1 are as defined above.

In one embodiment, 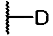 is a compound as shown below, or a stable deuterated derivative thereof, a stereoisomer thereof, or a pharmaceutically acceptable salt thereof: 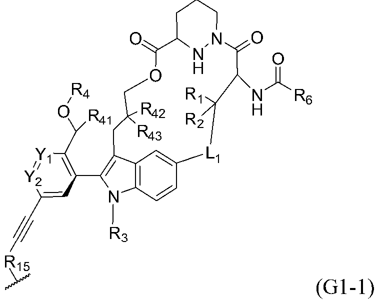
wherein in the formula, R₁, R₂, R₃, R₄, R₆, R₄₁, R₄₂, R₄₃, R₁₁, Y₁, Y₂, and L₁ are each as defined above;
R₁₅ is selected from -NR₀₁R₀₂, -C(=O)-NR₀₁R₀₂, -C(R₀₅)R₀₃R₀₄, -C₁₋₄ alkylene-NR₀₁R₀₂, and -C₁₋₄alkylene-C(R₀₅)R₀₃R₀₄;
R₀₁ and R₀₂ are each independently selected from hydrogen, substituted or unsubstituted C₁₋₆ alkyl, substituted or unsubstituted C₁₋₆ alkoxy, substituted or unsubstituted 3- to 6-membered cycloalkyl, substituted or unsubstituted C₂₋₆ alkenyl, substituted or unsubstituted C₂₋₆ alkynyl, and (substituted or unsubstituted C₁₋₆ alkyl)₃-Si-; or
R₀₁ and R₀₂, together with the nitrogen atom to which they are attached, form substituted or unsubstituted 5- to 15-membered tricyclic heterocyclyl, substituted or unsubstituted 7- to 12-membered bicyclic heterocyclyl, or substituted or unsubstituted 3- to 8-membered monocyclic heterocyclyl; or
R₀₁ and R₀₂ together form 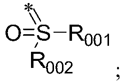 R₀₀₁ and R₀₀₂, together with the sulfur atom to which they are attached, form substituted or unsubstituted 3- to 8-membered monocyclic heterocyclyl;
R₀₃ and R₀₄ are each independently selected from hydrogen, substituted or unsubstituted C₁₋₆ alkyl, substituted or unsubstituted C₁₋₆ alkoxy, substituted or unsubstituted C₂₋₆ alkenyl, and substituted or unsubstituted C₂₋₆ alkynyl, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form substituted or unsubstituted 7- to 12-membered bicyclic heterocyclyl, substituted or unsubstituted 5- to 15-membered tricyclic heterocyclyl, substituted or unsubstituted 3- to 8-membered monocyclic heterocyclyl, substituted or unsubstituted 5- or 6-membered monocyclic heteroaryl, or substituted or unsubstituted 8- to 10-membered bicyclic heteroaryl;
R₀₅ is hydrogen, deuterium, halogen, deuterated C₁₋₆ alkyl, or C₁₋₆ alkyl;
the substitutions each independently mean that 1, 2, 3, or 4 hydrogen atoms on the groups described above are substituted with a group selected from group S1; the groups of group S1 are as defined above.

In one embodiment, R₅ is 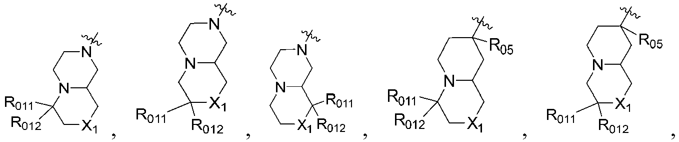 or 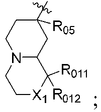 R₀₁₁ and R₀₁₂ are each independently hydrogen, C₁₋₆ alkyl, C₁₋₆ haloalkyl, or deuterated C₁₋₆ alkyl; or R₀₁₁ and R₀₁₂, together with the carbon atom to which they are attached, form substituted or unsubstituted C₃₋₆ cycloalkyl; the substitutions are as defined herein; X₁ is a bond, S, O, or N(R₀₁₃); R₀₁₃ is hydrogen, C₁₋₆ alkyl, or deuterated C₁₋₆ alkyl; R₀₅ is hydrogen, deuterium, or C₁₋₆ alkyl.

In one embodiment, R₅ is hydrogen, 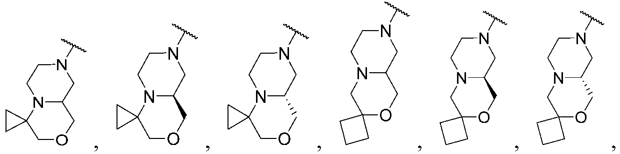 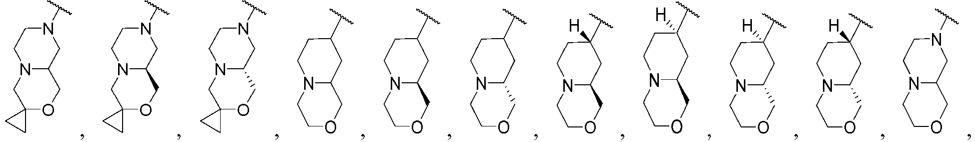 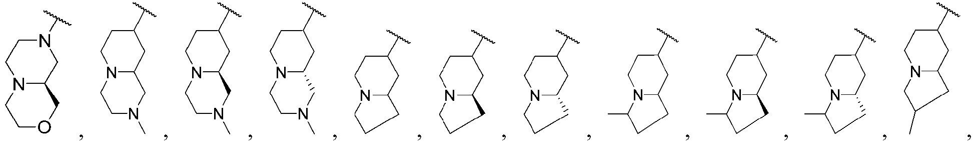 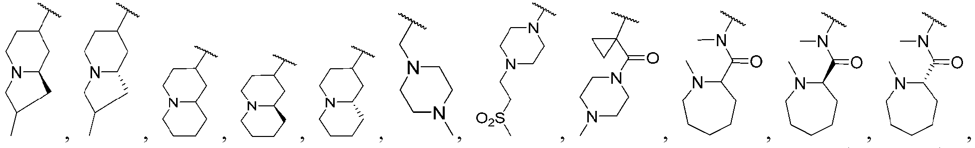 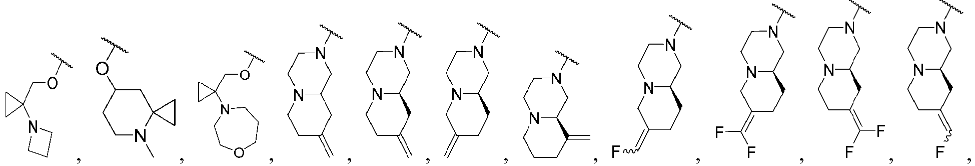 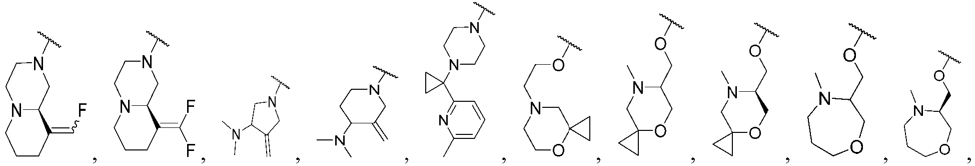 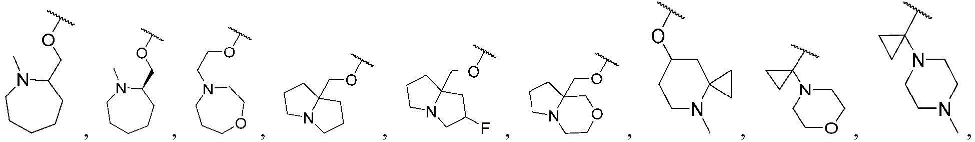 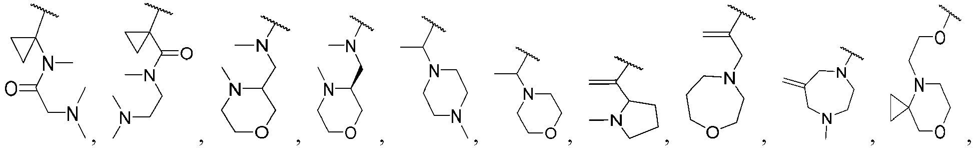 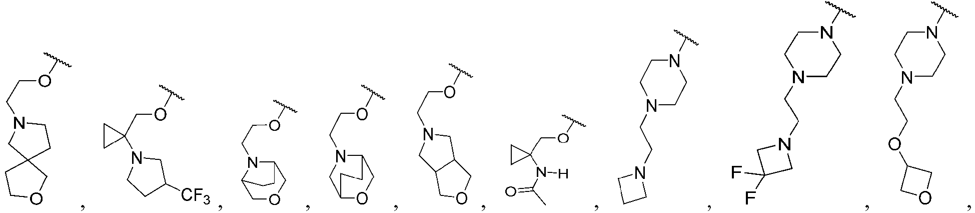 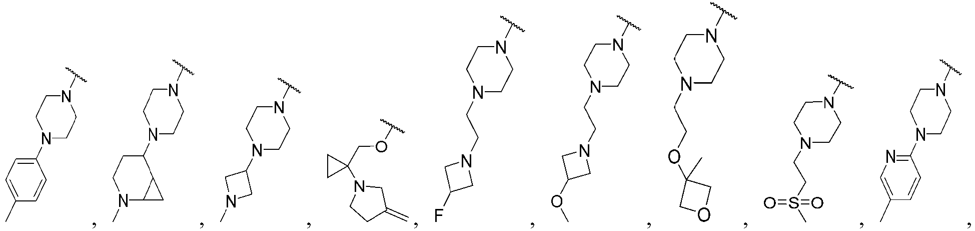 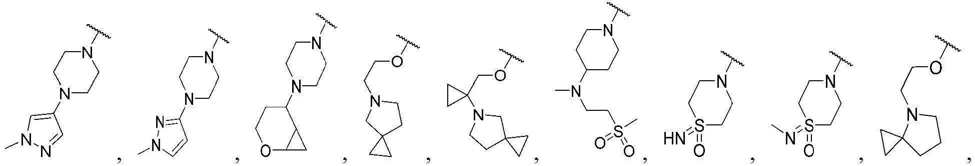 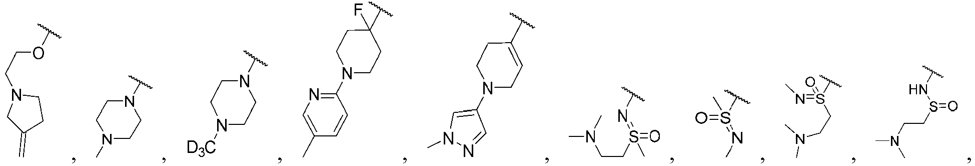 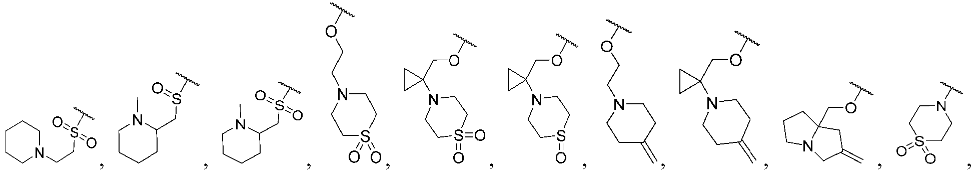 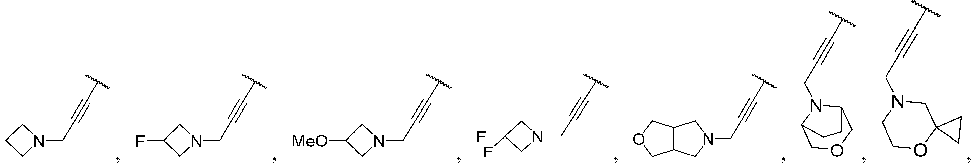 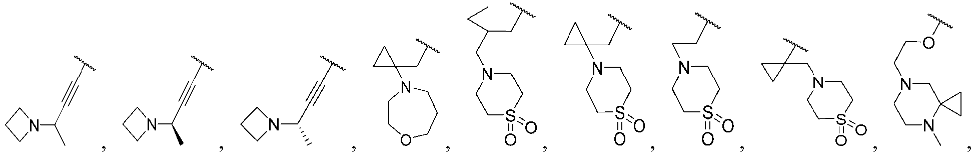 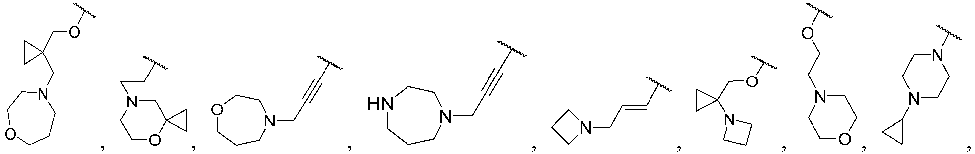 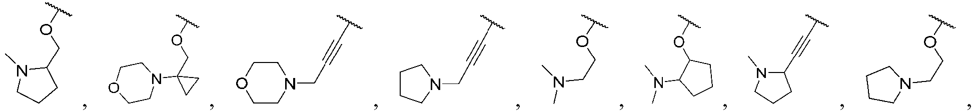 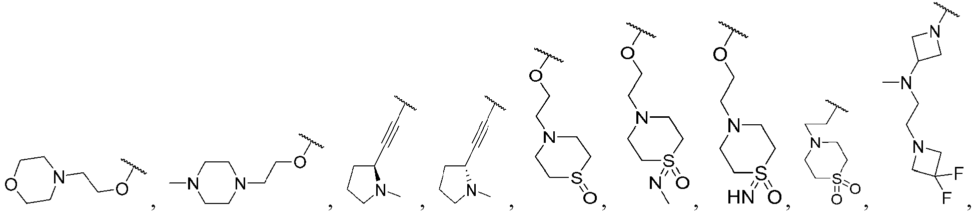 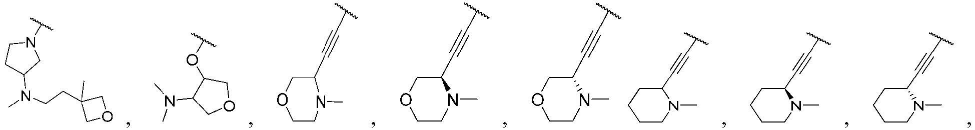 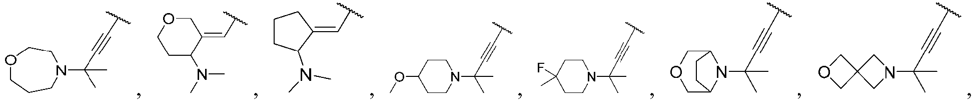 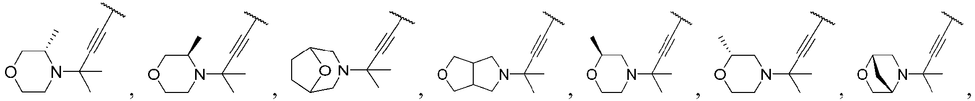 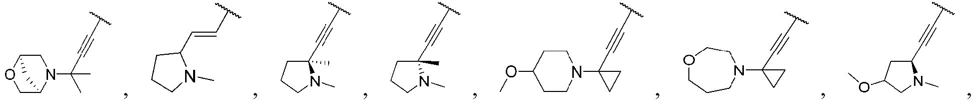 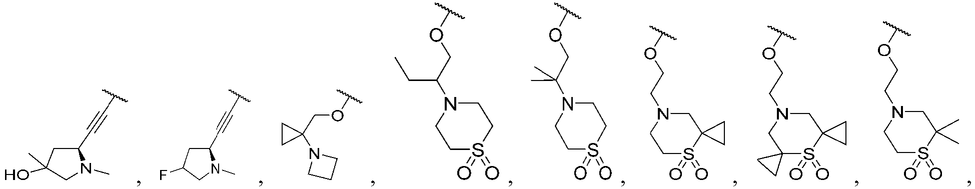 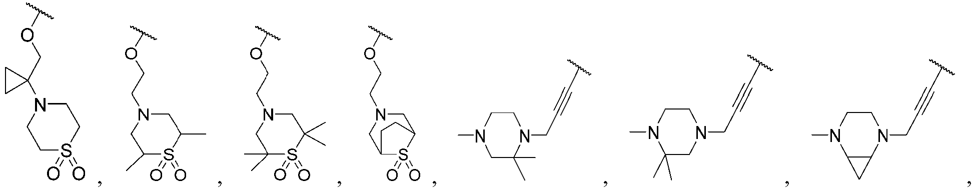 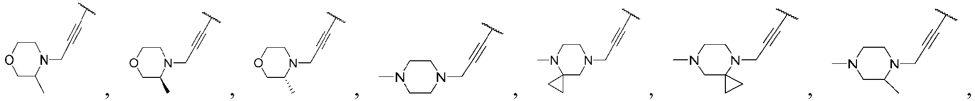 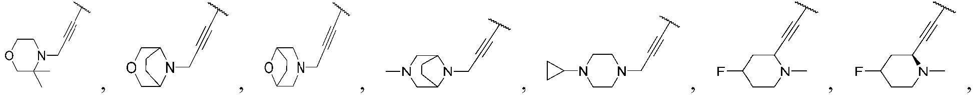 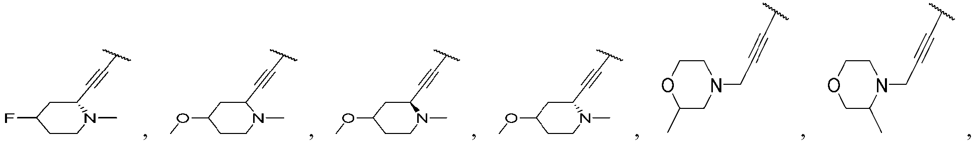 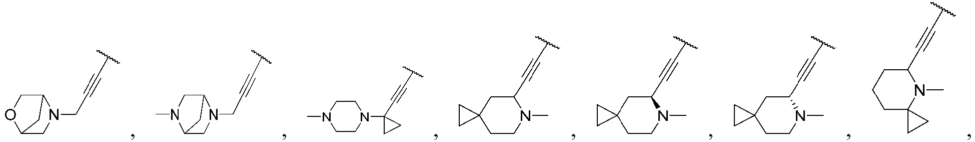 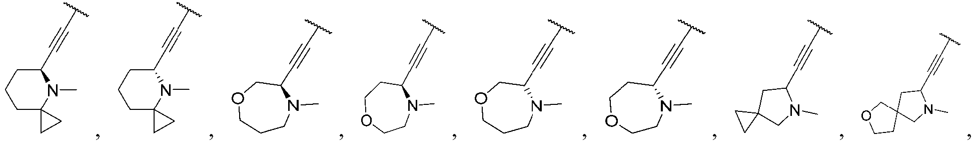 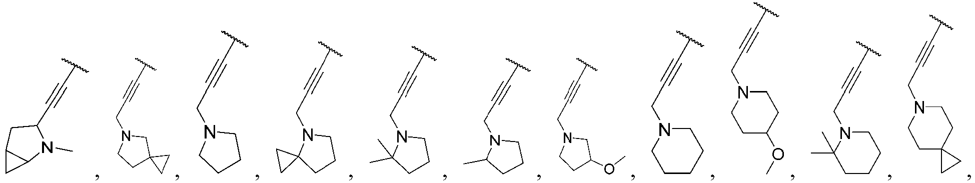 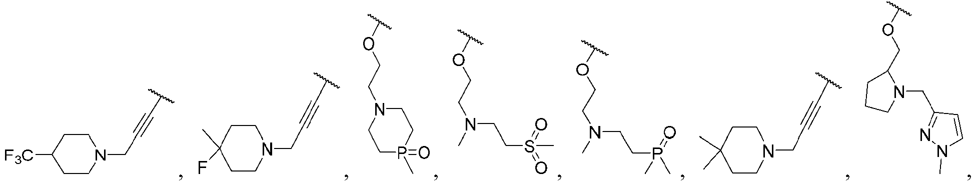 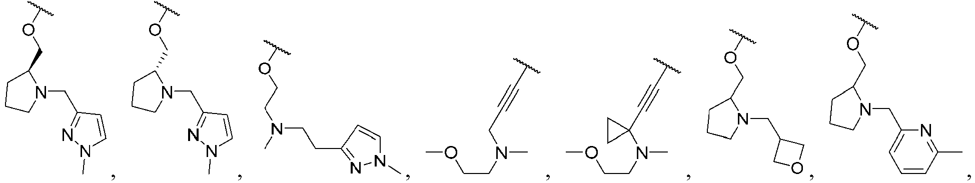 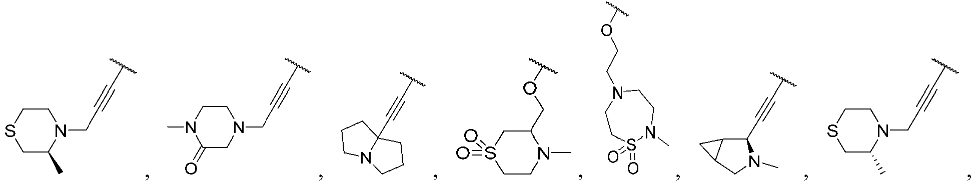 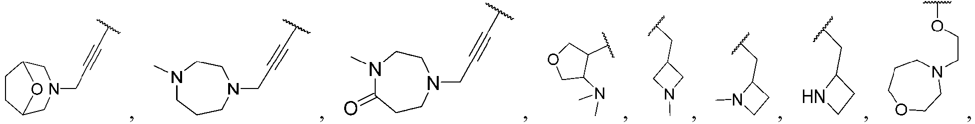 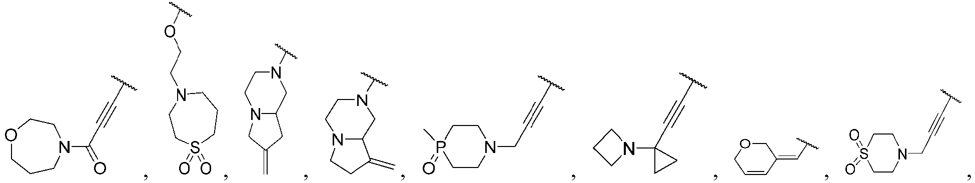 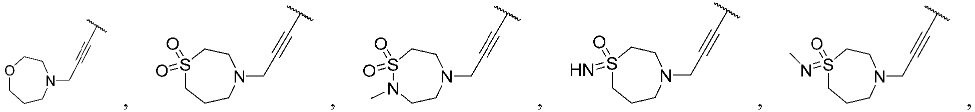 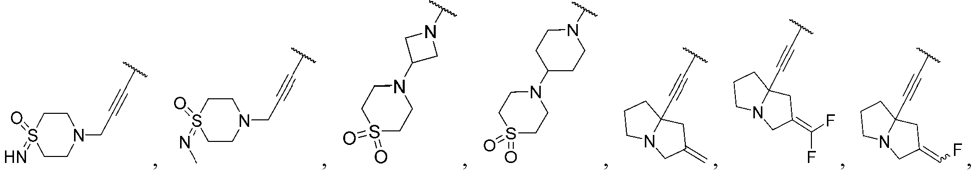 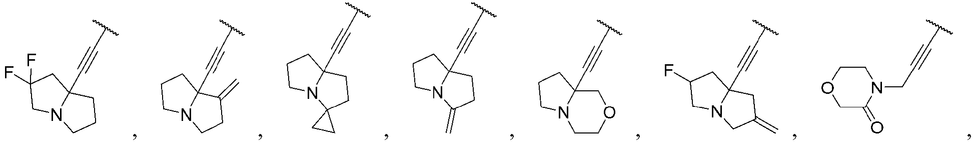 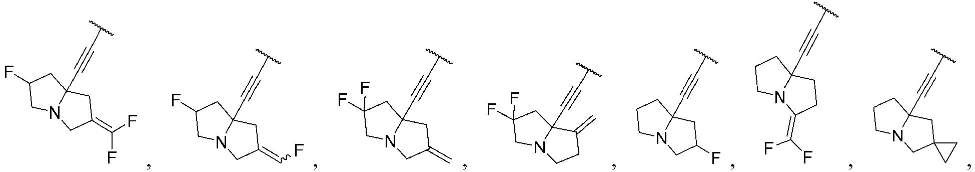 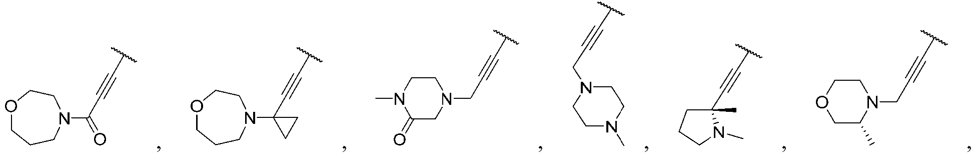 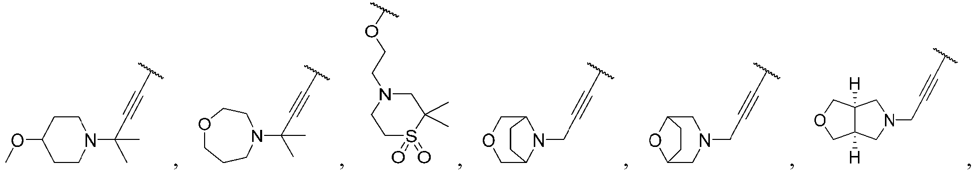 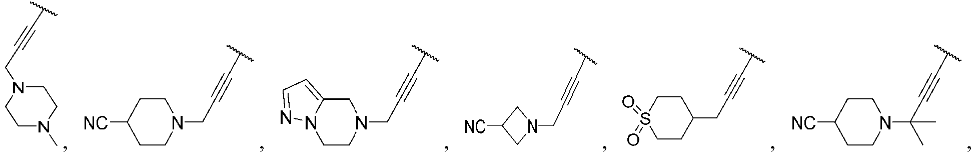 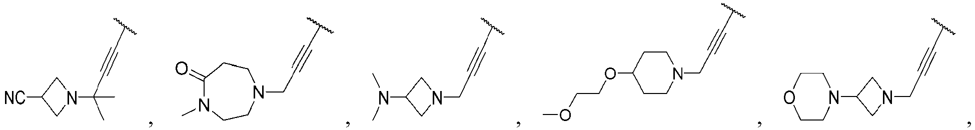 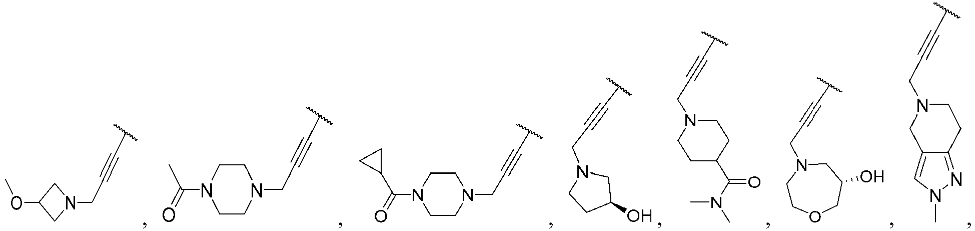 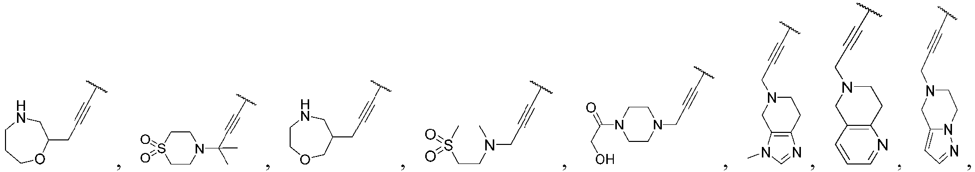 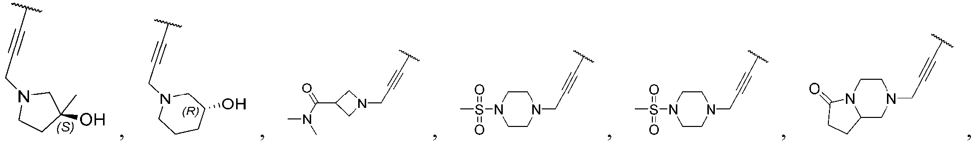 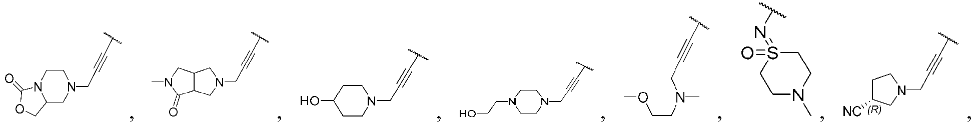 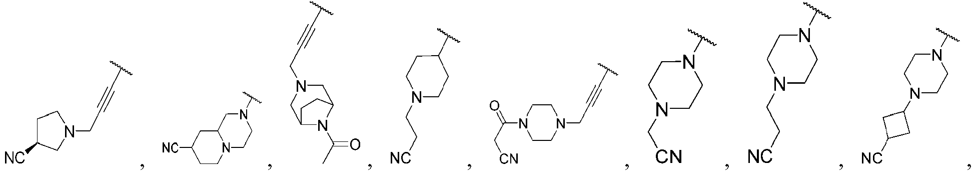 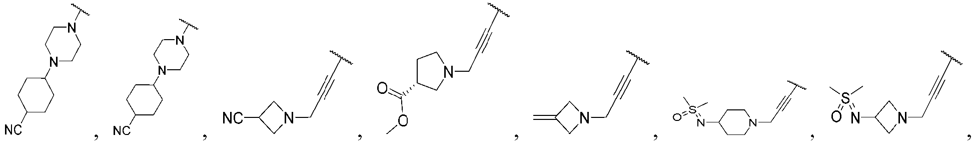 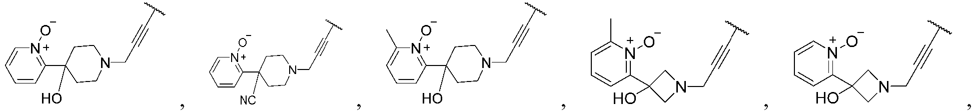 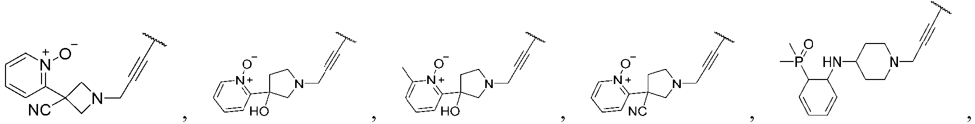 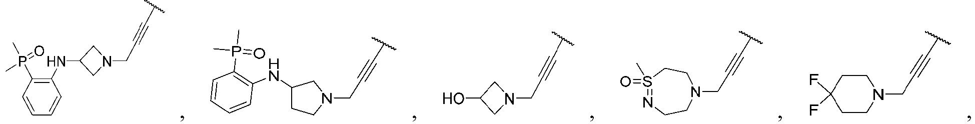 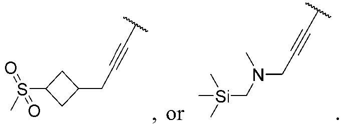 Preferably, R₅ is

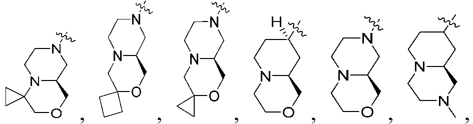

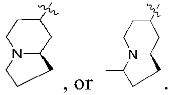

In one embodiment, R₁₁ is 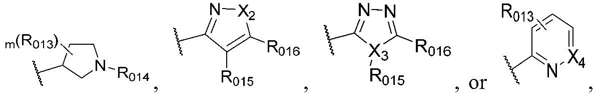 wherein X₂ is NH, O, or CR₀₁₃; X₃ and X₄ are each independently N or CR₀₁₃; R₀₁₃ and R₀₁₄ are each independently hydrogen, halogen, oxo, C₁₋₆ alkyl, or C₁₋₆ alkoxy; m is 1, 2, or 3; R₀₁₅ and R₀₁₆ are each independently hydrogen, C₁₋₆ alkyl, C₃₋₆ cycloalkyl, or -C₁₋₆ alkyl-5- or 6-membered monocyclic heteroaryl; or R₀₁₅ and R₀₁₆, together with the carbon atom to which they are attached, form substituted or unsubstituted 3- to 8-membered monocyclic heterocyclyl; the substitutions are as defined herein.

In one embodiment, R₁₁ is pyrrolidinyl, isoxazolyl, triazolyl, pyridazinyl, or triazolopyridinyl, wherein the pyrrolidinyl, isoxazolyl, triazolyl, pyridazinyl, or triazolopyridinyl is optionally substituted with a substituent selected from oxo, halogen, C₁₋₆ alkyl, C₁₋₆ alkoxy, C₃₋₆ cycloalkyl, -C₁₋₄ alkylene-5- or 6-membered heteroaryl.

In one embodiment, R₁₁ is selected from the group consisting of:

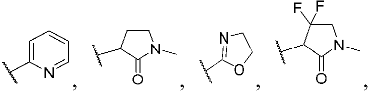

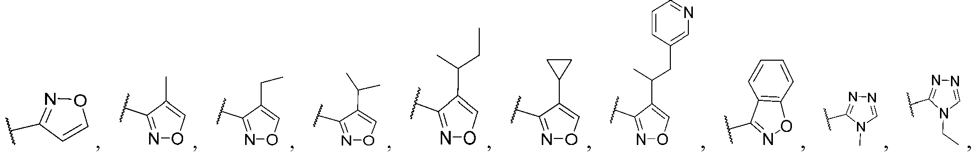

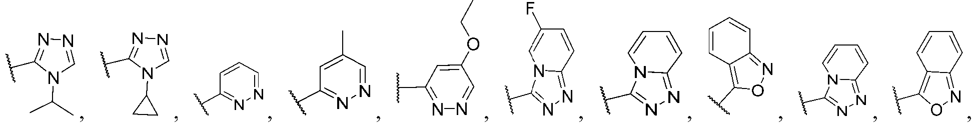

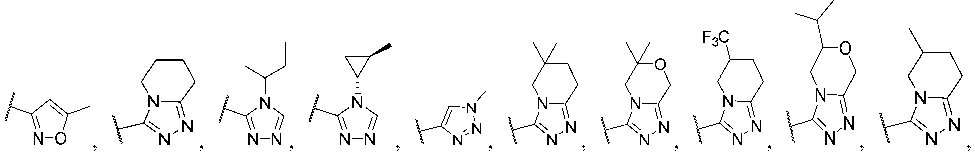

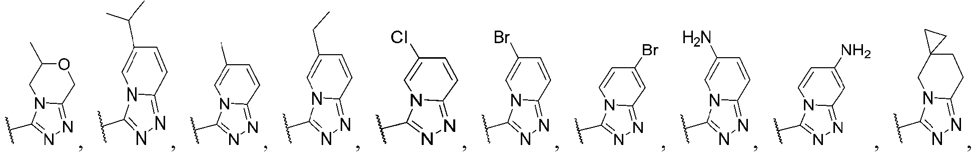

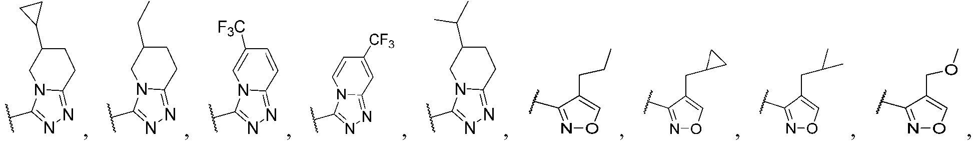

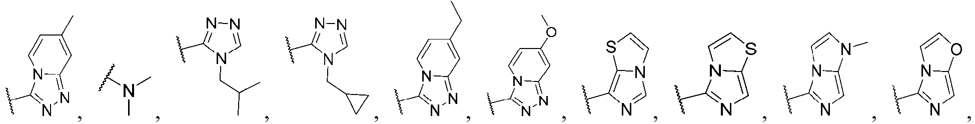

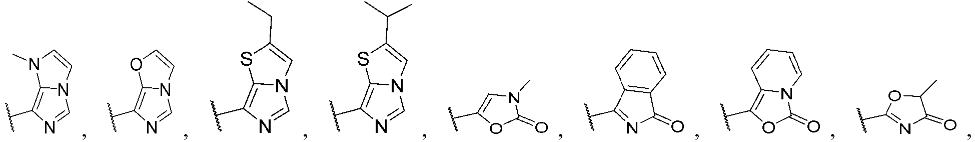

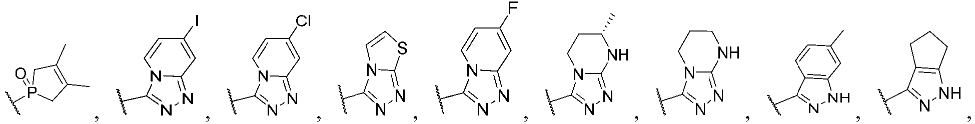

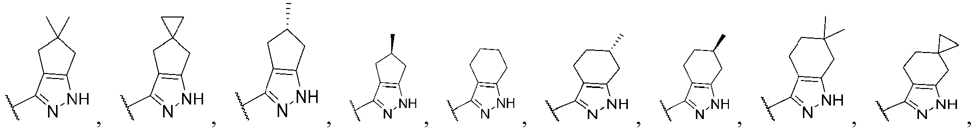

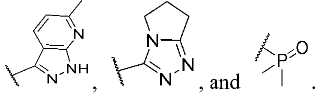

In one embodiment, R₁₁ is pyrrolidinyl, oxazolyl, isoxazolyl, dihydrooxazolyl, triazolyl, pyridazinyl, triazolopyridinyl, tetrahydrotriazolopyridinyl, or triazolomorpholinyl; the pyrrolidinyl, oxazolyl, isoxazolyl, dihydrooxazolyl, triazolyl, pyridazinyl, triazolopyridinyl, tetrahydrotriazolopyridinyl, or triazolomorpholinyl is optionally substituted with a substituent selected from oxo, halogen, C₁₋₆ alkyl, C₁₋₆ alkoxy, C₃₋₆ cycloalkyl, -C₁₋₄ alkylene-5- or 6-membered heteroaryl.

In one embodiment, 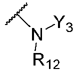 is selected from the group consisting of:

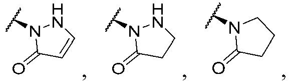

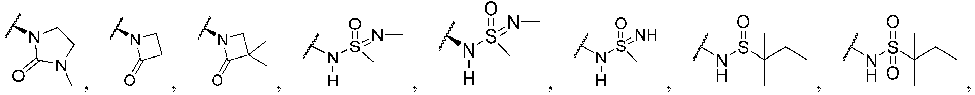

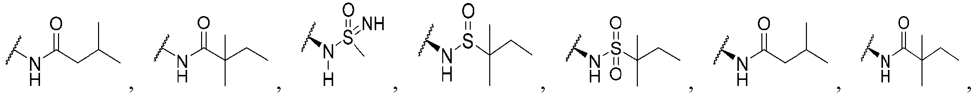

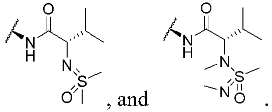

In one embodiment, R₆ is halogen-substituted or unsubstituted C₁₋₆ alkyl.

In one embodiment, R₆ is substituted or unsubstituted C₁₋₆ alkyl, or substituted or unsubstituted C₃₋₆ cycloalkyl. In one embodiment, R₆ is C₁₋₆ alkyl substituted with halogen, preferably C₁₋₆ alkyl substituted with F.

In one embodiment, R₆ is cyclopropyl substituted with C₁₋₆ alkyl, preferably cyclopropyl substituted with one or two methyl groups.

In one embodiment, R₆ is

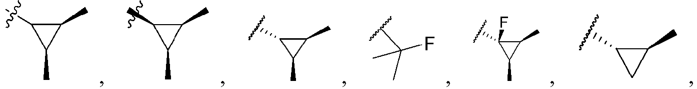

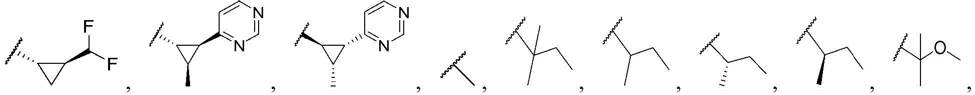

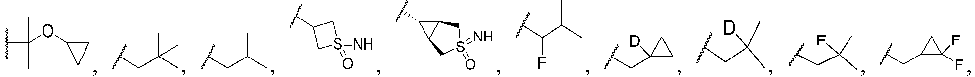

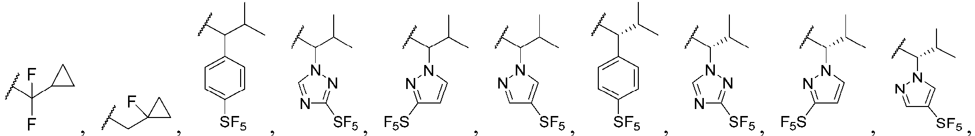

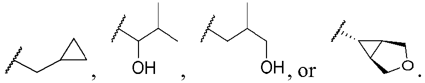

### Linker-drug

In a second aspect, the present disclosure provides a compound represented by formula (VII),

L₀-D (VII)

wherein D is as defined in any one of the embodiments of the first aspect of the present disclosure;
L₀ comprises H₁, L₁ₐ, L_{1b}, L_{1c}, and L_{1d}; L₁ₐ, L_{1b}, L_{1c}, and L_{1d} are as defined in any one of the embodiments of the first aspect of the present disclosure;
H₁ is an antibody-linking unit.

In one embodiment, H₁ is selected from the group consisting of: 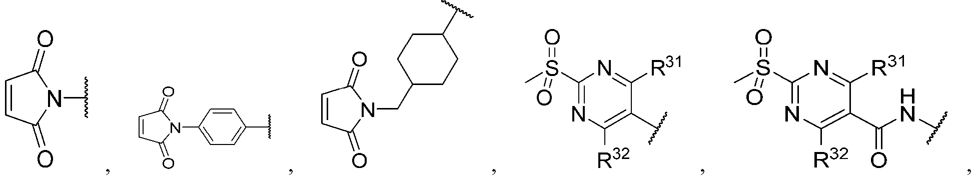 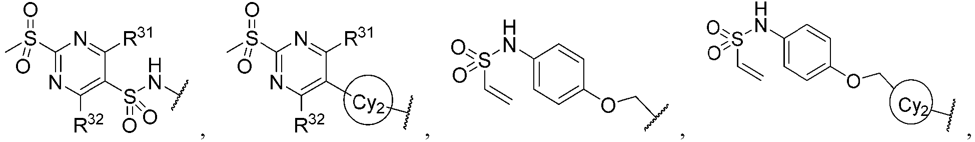 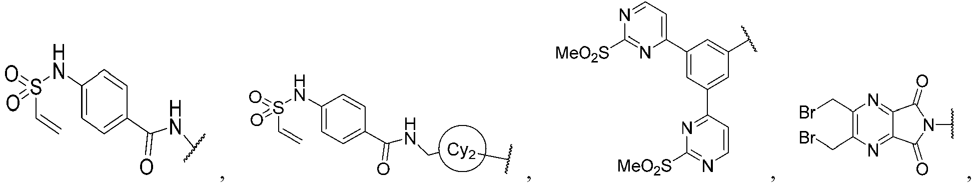 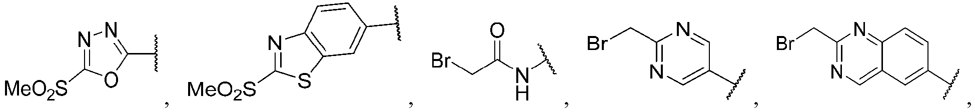 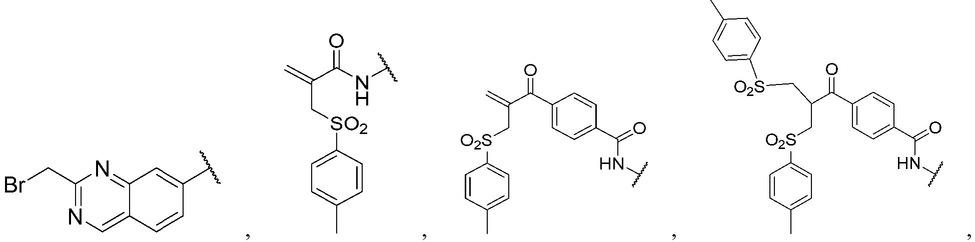 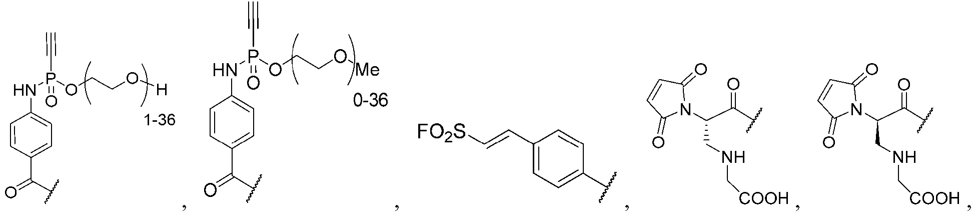 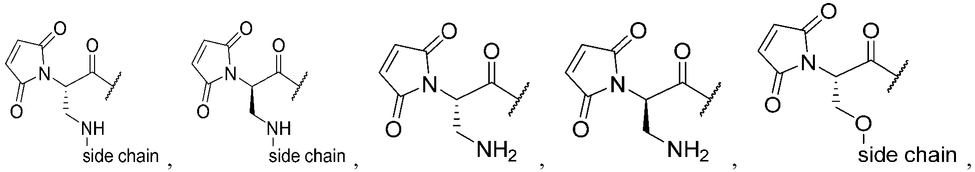 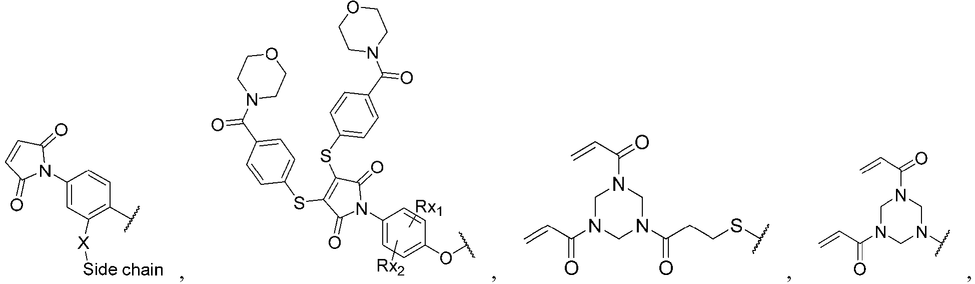 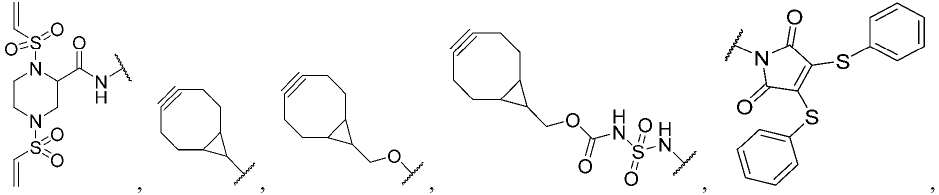 and 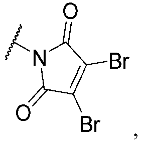 wherein:
X is -O-(CH₂)₀₋₆-, -N(CH₃)-, -O-, -CONH-, or -CON(CH₃)-;
Rₓ₁ and Rₓ₂ are each independently hydrogen, C₁₋₆ alkyl, halogen, deuterium, C₁₋₆ alkoxy, C₁₋₆ alkylene-NH-, or (C₁₋₆ alkyl)₂-N-;
R³¹ is hydrogen, deuterium, halogen, cyano, or C₁₋₄ alkyl;
R³² is hydrogen, deuterium, halogen, cyano, or C₁₋₄ alkyl;
Cy2 is 5- to 10-membered heteroaryl, C₆₋₁₀ aryl, 3- to 10-membered heterocyclyl, or C₃₋₁₀ cycloalkyl;
the side chain is a hydrophilic group, wherein the hydrophilic group is as defined in any one of the embodiments.

In one embodiment, Rₓ₁ and Rₓ₂ are each independently hydrogen, C₁₋₄ alkyl, halogen, deuterium, C₁₋₄ alkoxy, C₁₋₄ alkylene-NH-, or (C₁₋₄ alkyl)₂-N-.

In one embodiment, Rₓ₁ and Rₓ₂ are each independently hydrogen, methyl, halogen, deuterium, methoxy, methyl-NH-, or (methyl)₂-N-.

In one embodiment, R³¹ is hydrogen, deuterium, halogen, cyano, or methyl.

In one embodiment, R³¹ is hydrogen.

In one embodiment, R³² is hydrogen, deuterium, halogen, cyano, or methyl.

In one embodiment, R³² is hydrogen.

In one embodiment, Cy2 is 5- to 10-membered heteroaryl, C₆₋₁₀ aryl, 4- to 8-membered heterocyclyl, or C₃₋₆ cycloalkyl.

In one embodiment, Cy2 is pyrrolyl, imidazolyl, pyridinyl, pyrimidinyl, phenyl, pyrrolidinyl, piperidinyl, piperazinyl, cyclopropyl, cyclobutyl, or cyclopentyl.

In one embodiment, H₁ is selected from the group consisting of: 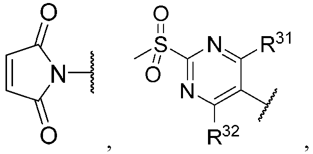 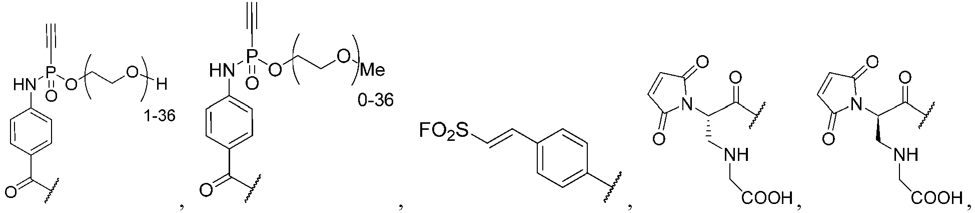 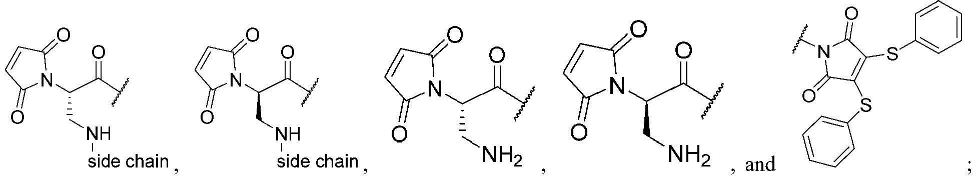 the side chain is a hydrophilic group, wherein the hydrophilic group is as defined in any one of the embodiments, and R³¹ and R³² are as defined in any one of the embodiments.

In one embodiment, H₁ is selected from the group consisting of:

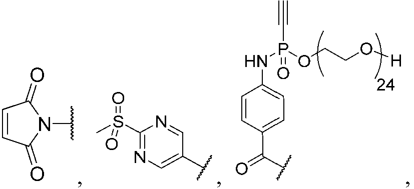

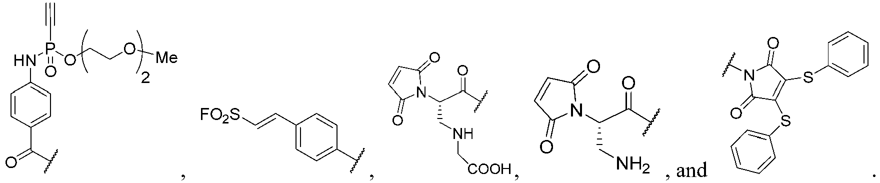

In one embodiment, H₁ is selected from the group consisting of: 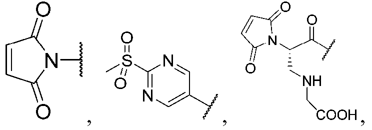 , and

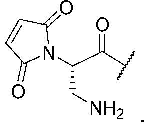

In one embodiment, H₁ is selected from the group consisting of:

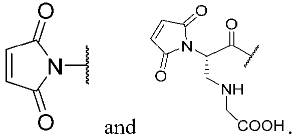

In one embodiment, H₁ is

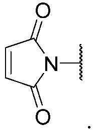

In one embodiment, L₀-D is selected from a structure represented by formula (VII-A) or formula (VII-B):

H₁-L₁ₐ-L_{1b}-L_{1c}-L_{1d}-D (VII-A)

H₁-L₁ₐ-L_{1b}- L_{1d}(-L_{1c}-C(=O)-CH₃)-D (VII-B)

L₁ₐ, L_{1b}, L_{1c}, L_{1d}, and D are as defined in any one of the embodiments; H₁ is as defined in any one of the embodiments.

In one embodiment, L₀-D is derived from a structure represented by formula (III-1A):

H₁-L₁ₐ-L_{1b}-L_{1c}-L_{1d}-D (III-1A)

wherein:
-D is as defined in any one of the embodiments;
H₁- is selected from the group consisting of: 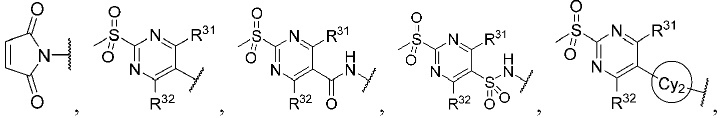 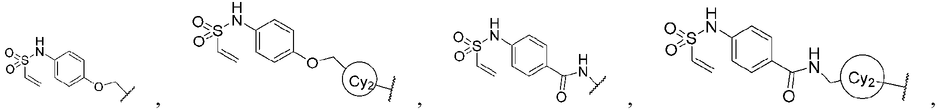 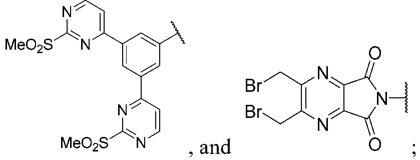 wherein:
   R³¹ is hydrogen, deuterium, halogen, cyano, or C₁₋₄ alkyl;
   R³² is hydrogen, deuterium, halogen, cyano, or C₁₋₄ alkyl;
   Cy2 is 5- to 10-membered heteroaryl, C₆₋₁₀ aryl, 3- to 10-membered heterocyclyl, or C₃₋₁₀ cycloalkyl;
   L₁ₐ is a bond or a fragment linking Y to L_{1d}, wherein the fragment is selected from C₁₋₆ alkyl, C₁₋₆ heteroalkyl, C₂₋₆ alkenyl, C₂₋₆ alkynyl, C₃₋₈ cycloalkyl, 4- to 10-membered heterocyclyl, C₆₋₁₀ aryl, 5- to 10-membered heteroaryl, C₁₋₄ alkyl-C₃₋₈ cycloalkyl, C₁₋₄ alkyl-4- to 10-membered heterocyclyl, C₁₋₄ alkyl-C₆₋₁₀ aryl, and C₁₋₄ alkyl-5- to 10-membered heteroaryl;
   L_{1b} is a bond or a fragment linking L₁ₐ to L_{1c}, wherein the fragment is selected from: 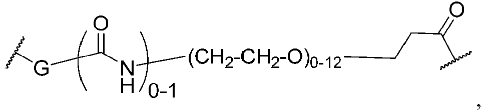 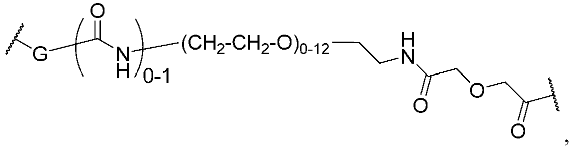 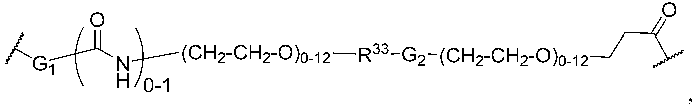 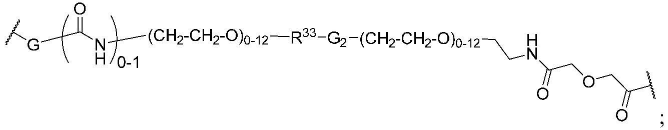
   wherein the left side of the fragment described above is attached to L₁ₐ, and the right side is attached to L_{1c};
   G is C₀₋₈ hydrocarbyl or C₀₋₈ heteroalkyl;
   G₁ and G₂ are each independently C₀₋₈ hydrocarbyl or C₀₋₈ heteroalkyl;
   R³³ is a 3- to 12-membered heterocyclic ring, a 5- to 10-membered heteroaromatic ring, NR³⁴, -CONR³⁴-, or - NR³⁴CO-;
   R³⁴ is hydrogen, -(CH₂CH₂O)₀₋₁₂-H, -(CH₂CH₂O)₀₋₁₂-CH₃, -CO-(CH₂CH₂O)₀₋₁₂-H, -CO-(CH₂CH₂O)₀₋₁₂-CH₃, or CH₂-CO-(N(Me)-CH₂-CO)₀₋₂₄-OH;
   L_{1c} is a bond or a fragment linking L_{1b} to L_{1d}, wherein the fragment is an amino acid residue or a short peptide consisting of 2-10 amino acid residues;
   L_{1d} is a bond or a fragment linking L_{1c} to D, and L_{1d} is selected from the group consisting of: 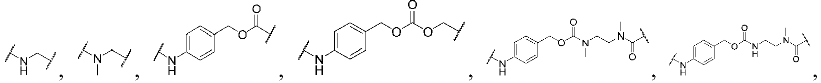 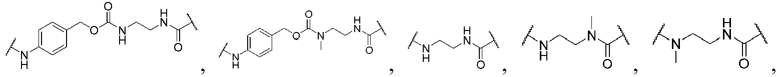 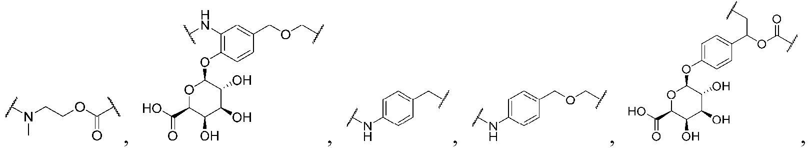 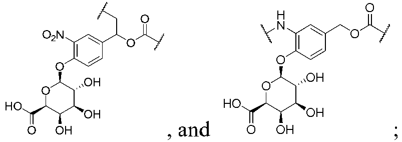
   the left side of the fragment described above is attached to L_{1c}, and the right side is attached to D.

In one embodiment, L₀-D is selected from structures shown in Table 12.

**Table 12**

| Number | Structure |
|---|---|
| Z6-L1 | 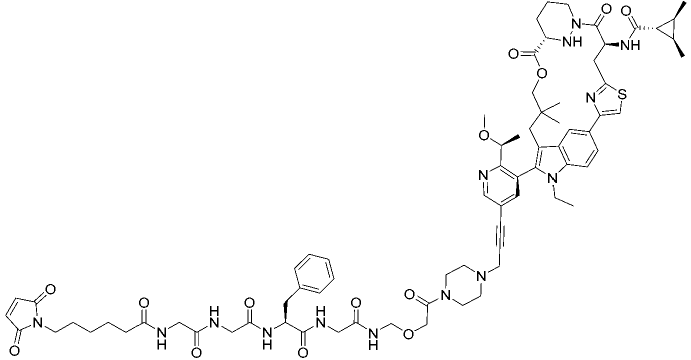 |
| Z6-L2 | 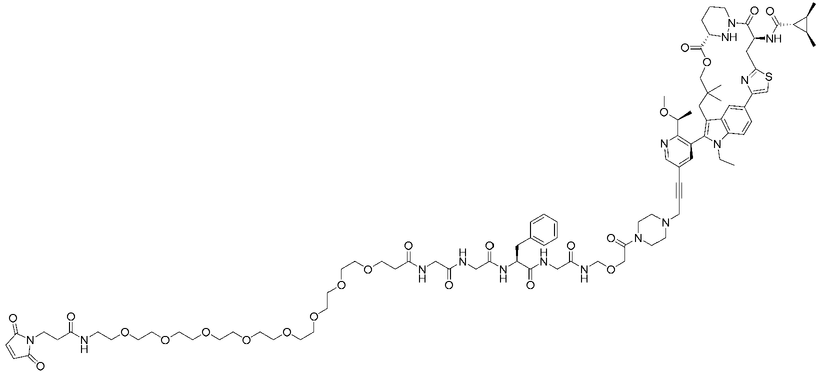 |
| Z34-L1 | 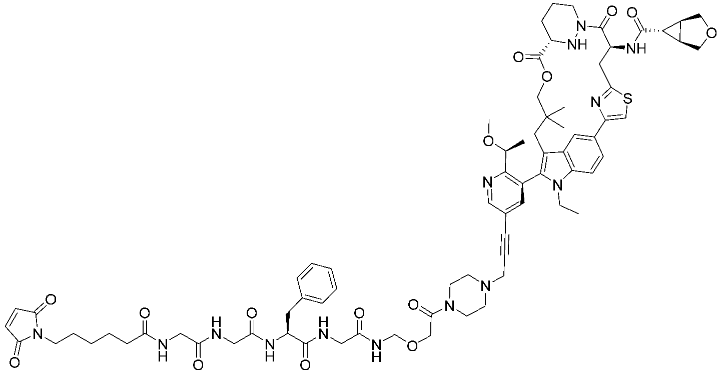 |
| Z34-L2 | 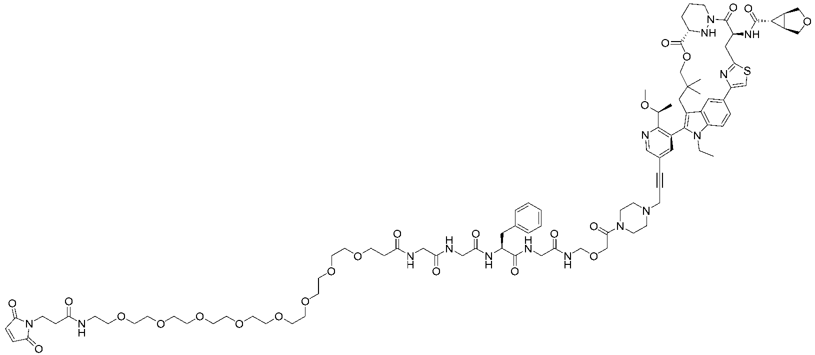 |
| Z34-L3 | 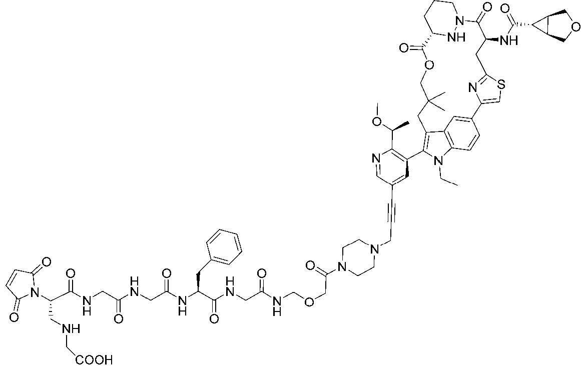 |
| Z34-L4 | 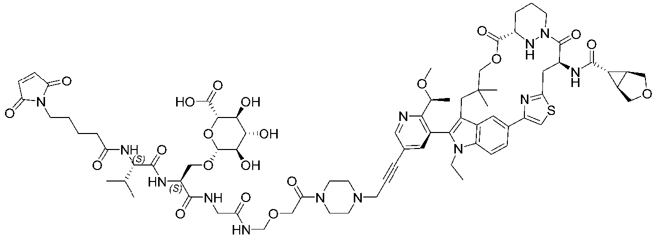 |
| Z63-L1 | 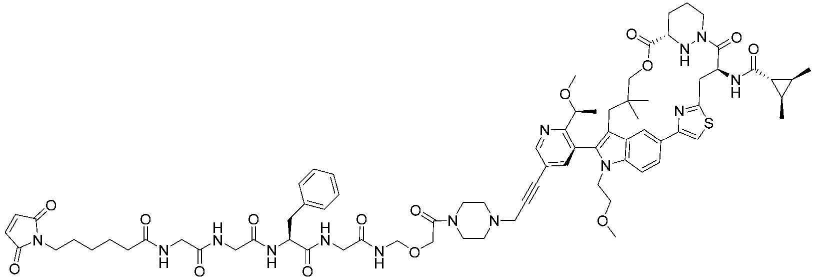 |
| Z48-L1 | 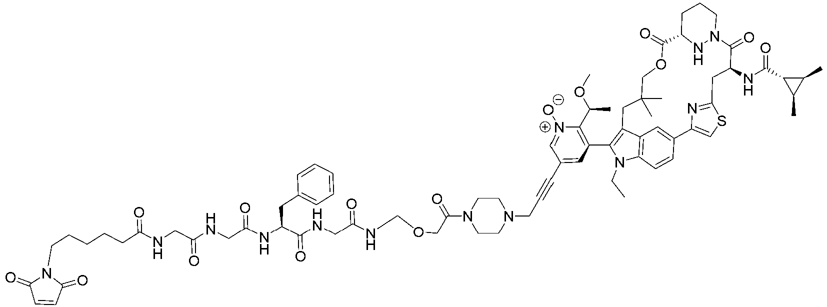 |
| Z54-L1 | 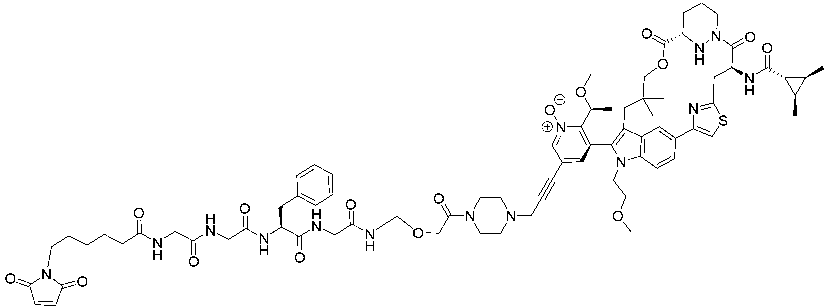 |
| Z54-L2 | 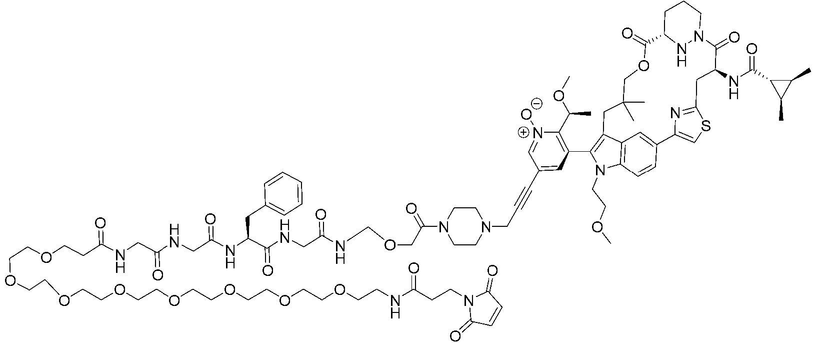 |
| Z31-L1 | 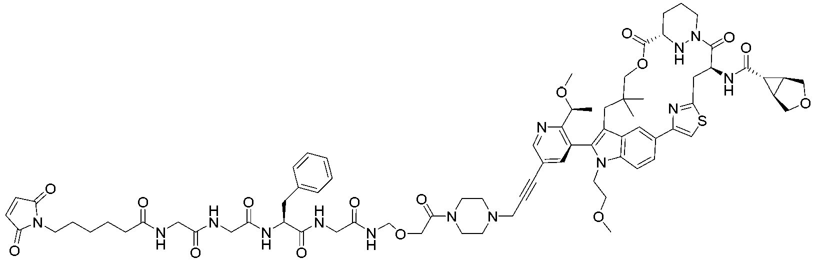 |
| Z35-L1-A | 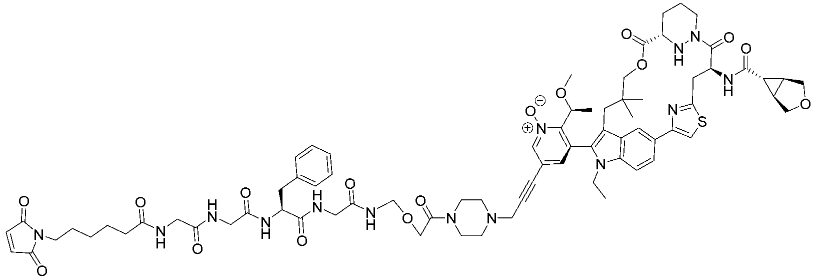 |
| Z38-L1-A | 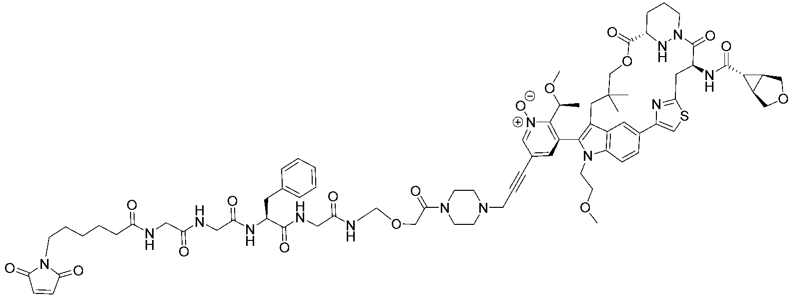 |
| Z38-L2-A | 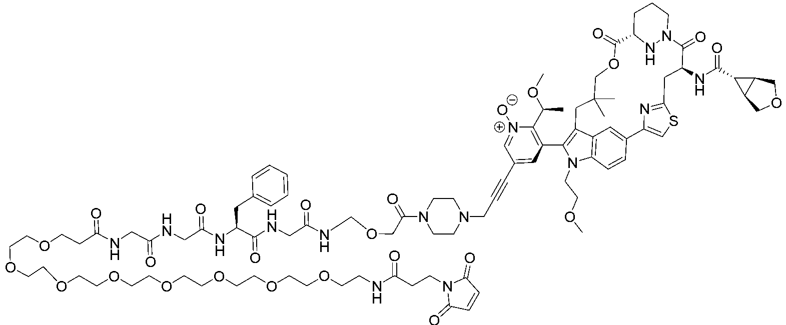 |
| Z47-L5 | 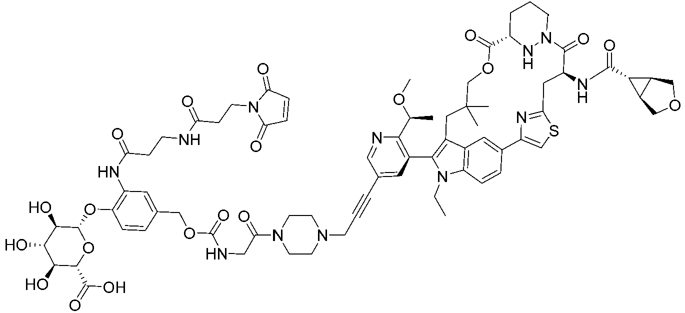 |
| Z47-L6 | 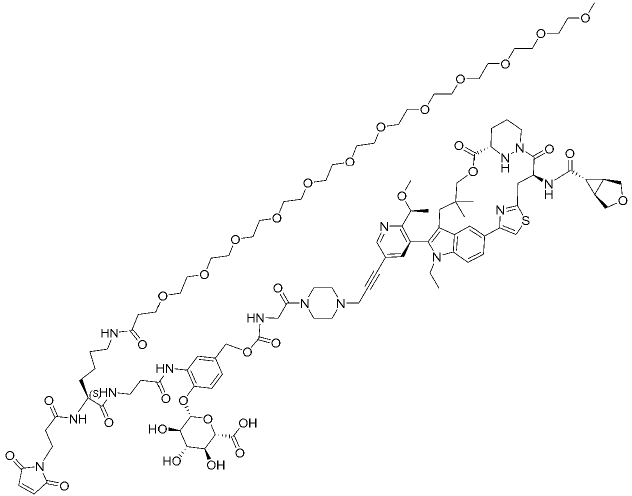 |
| Z52-L7 | 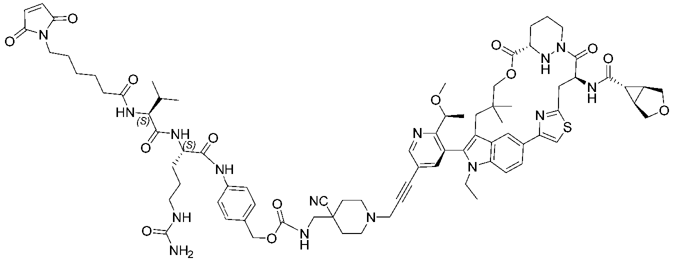 |
| Z52-L8 | 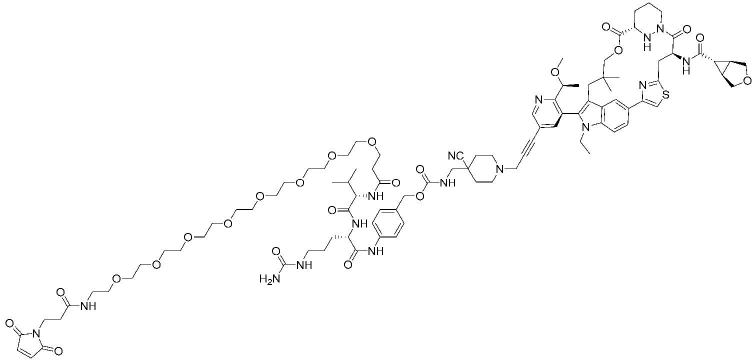 |
| Z52-L9 | 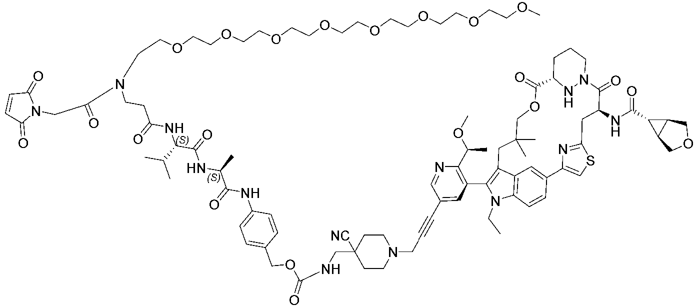 |
| Z52-L10 | 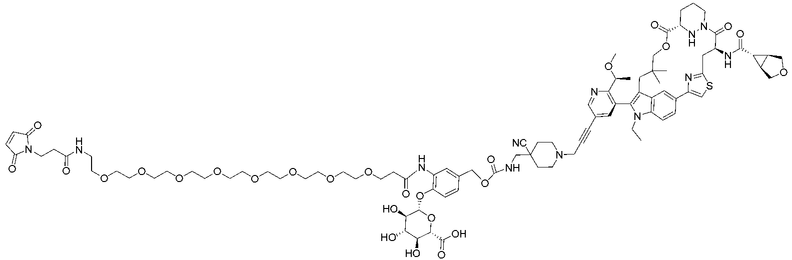 |
| Z52-L12 | 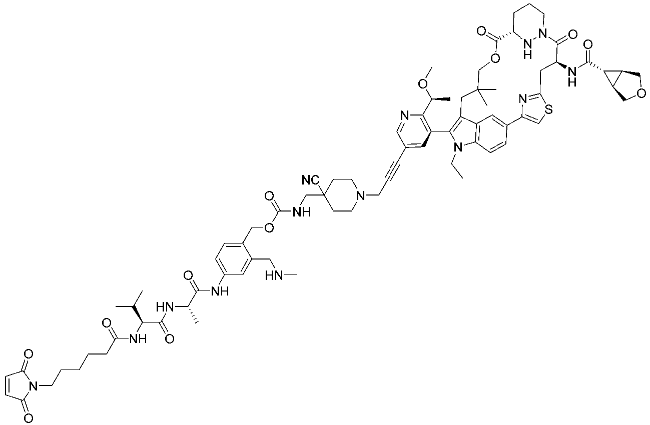 |
| Z52-L13 | 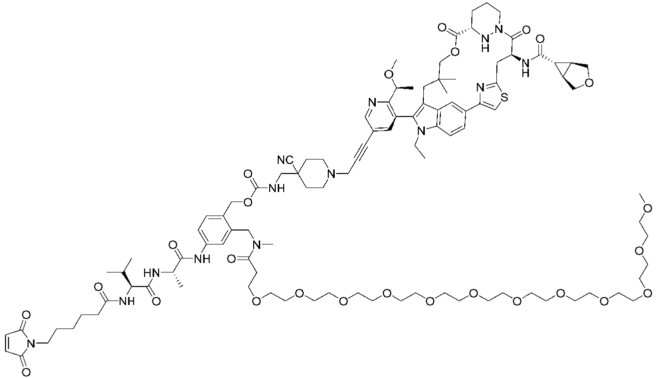 |
| Z52-L14 | 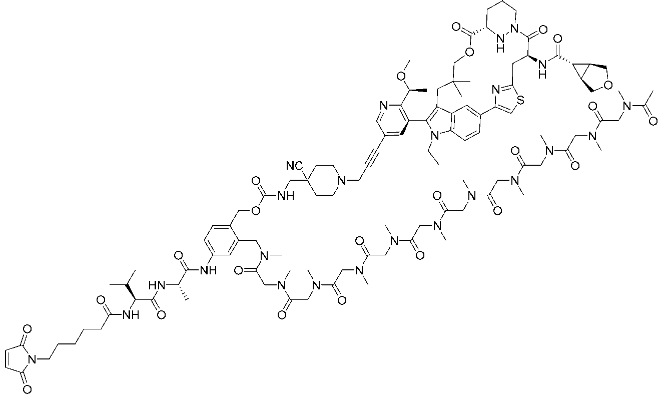 |
| Z166-L14 | 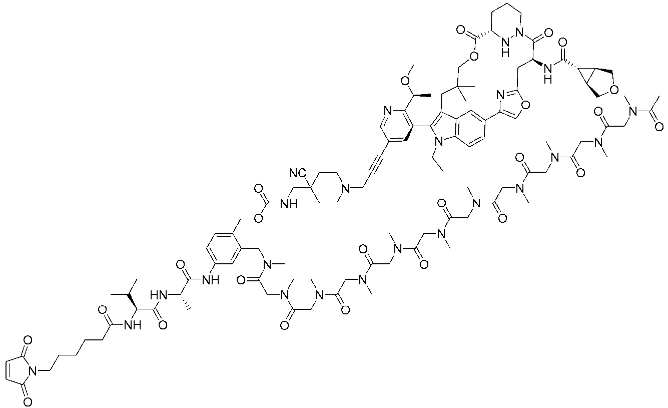 |
| Z52-L15 | 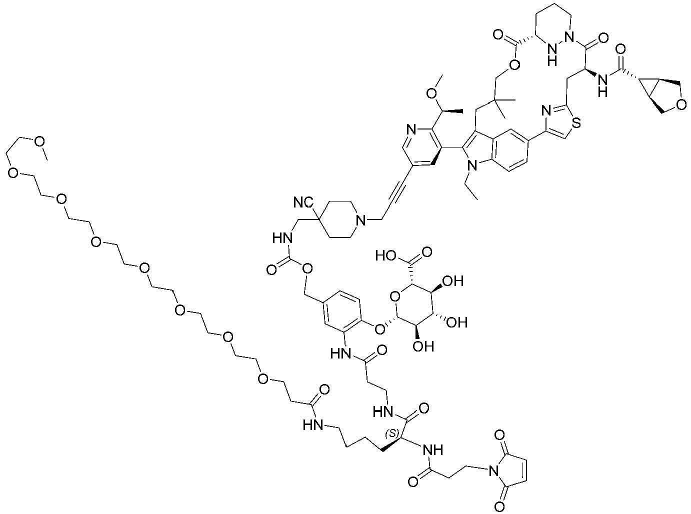 |
| Z52-L16 | 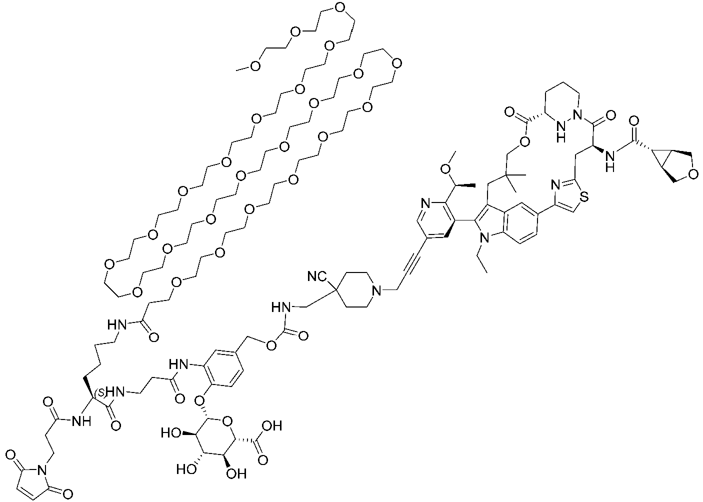 |
| Z57-L7-A | 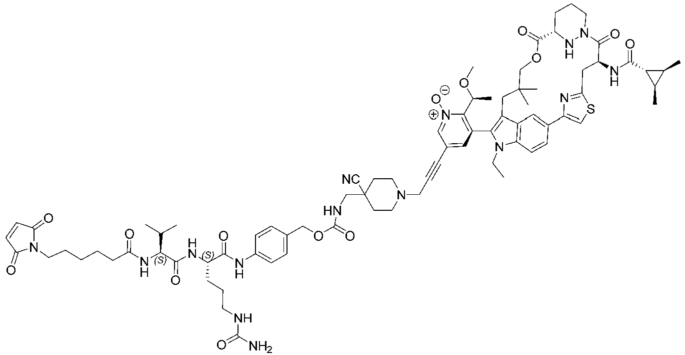 |
| Z64-L7-A | 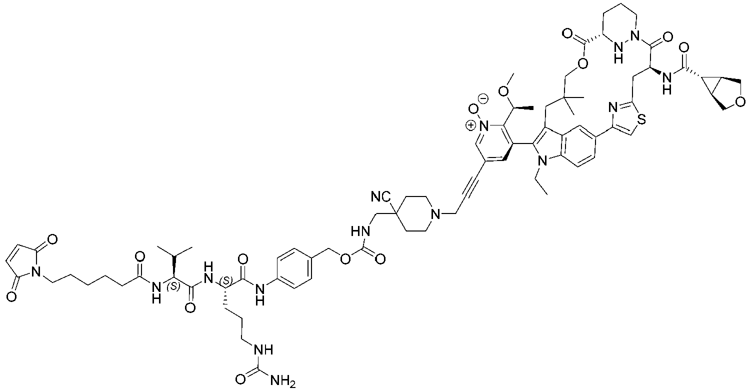 |
| Z64-1-L16 | 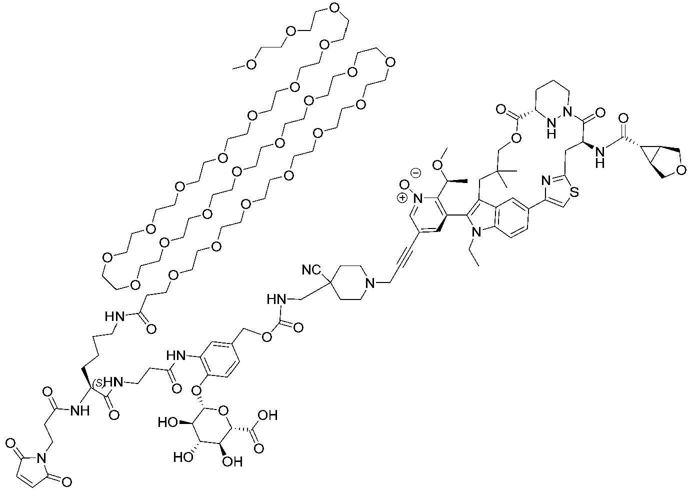 |
| Z61-L7 | 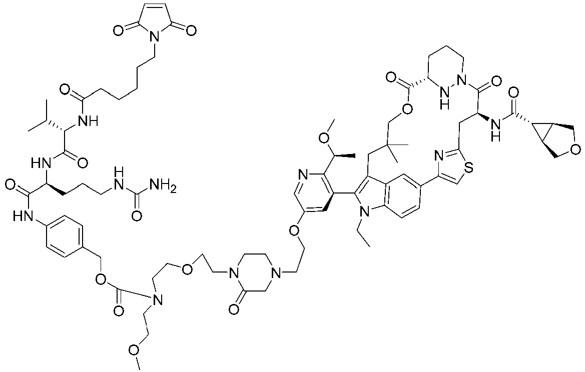 |
| Z45-L2-A | 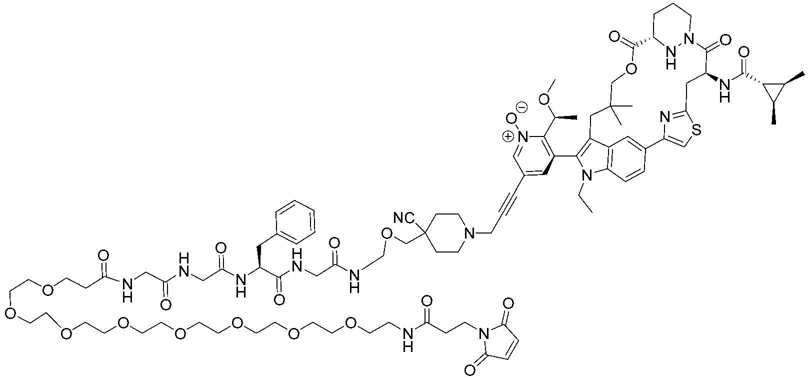 |
| Z11-L2 | 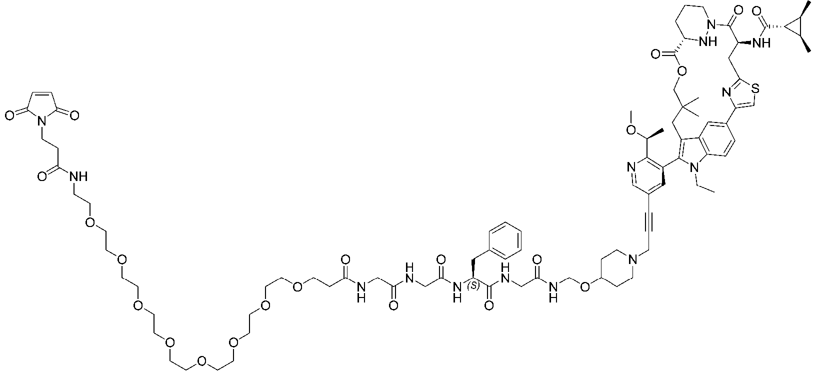 |
| Z170-L17 | 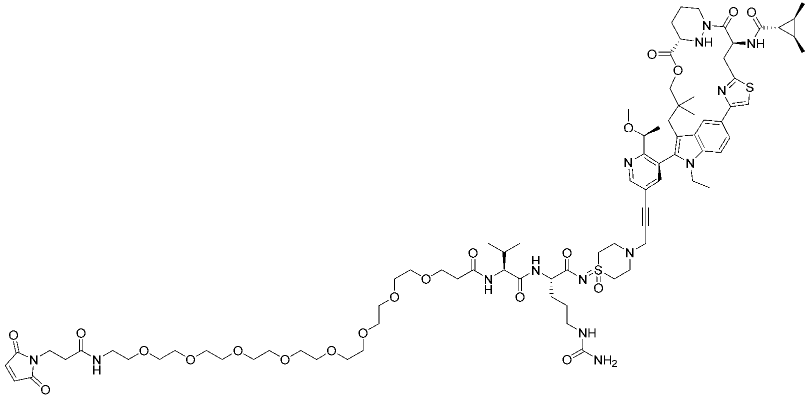 |
| Z56-L19 | 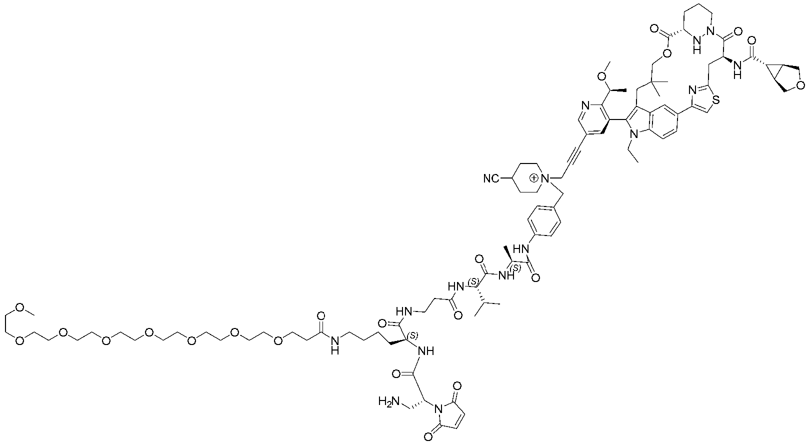 |
| Z56-L23 | 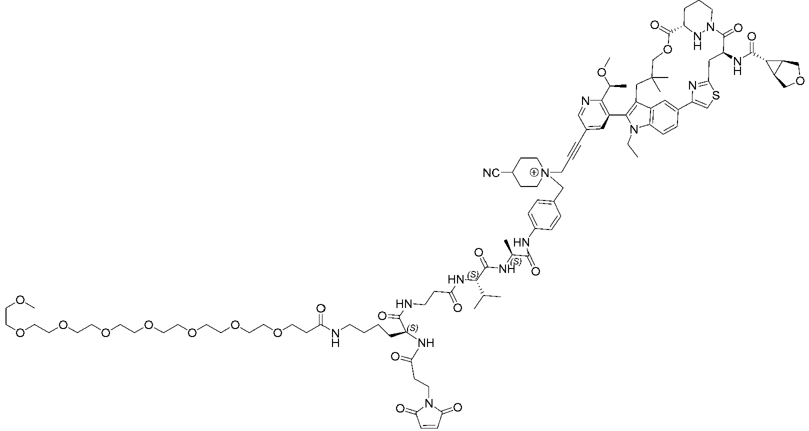 |
| Z10-L1 | 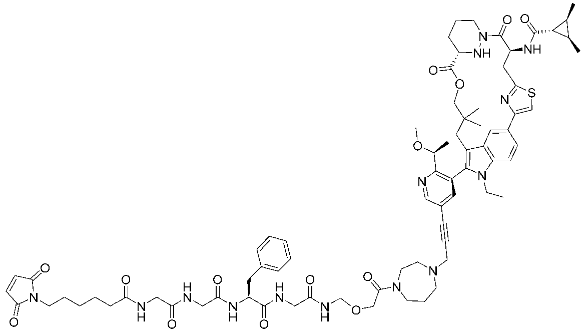 |
| Z52-L11 | 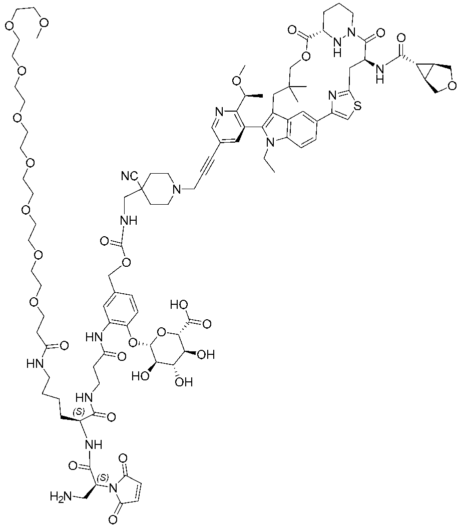 |
| Z56-L26 | 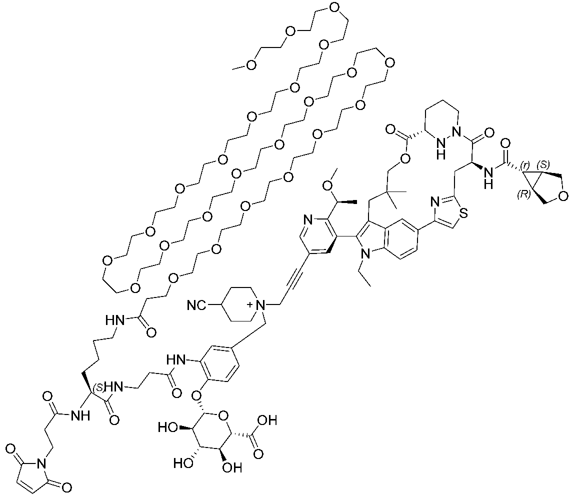 |
| Z168-L22 | 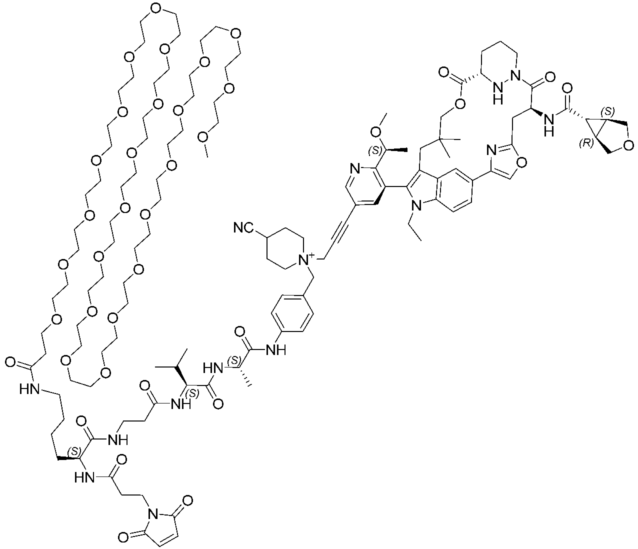 |
| Z168-L24 | 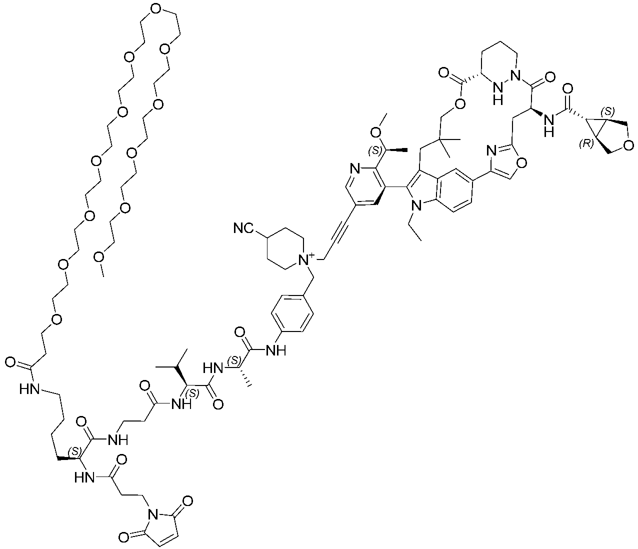 |
| Z168-L23 | 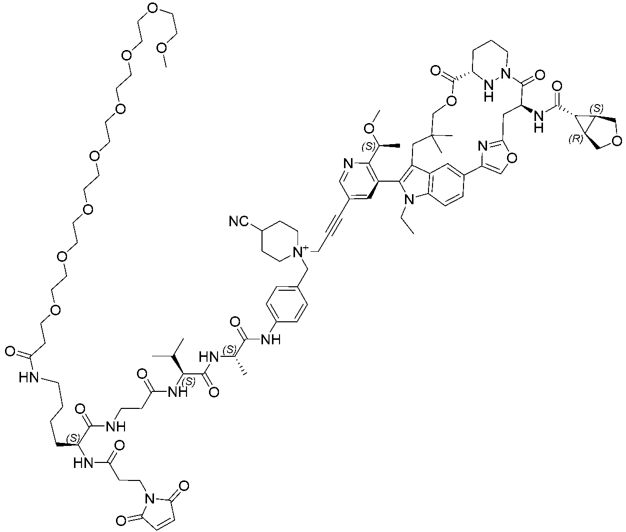 |
| Z4-L7 | 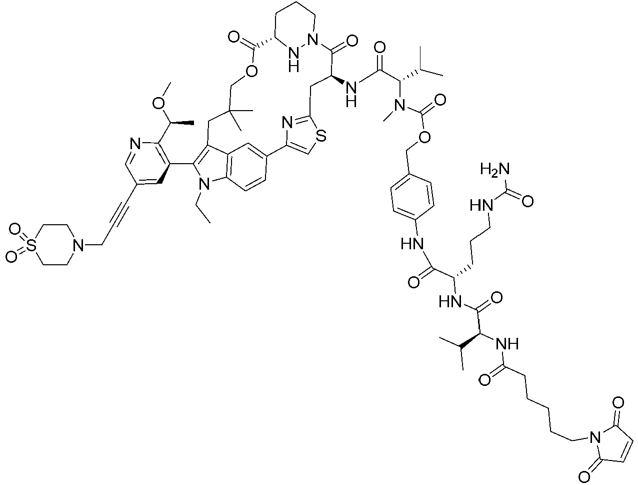 |
| Z4-L8 | 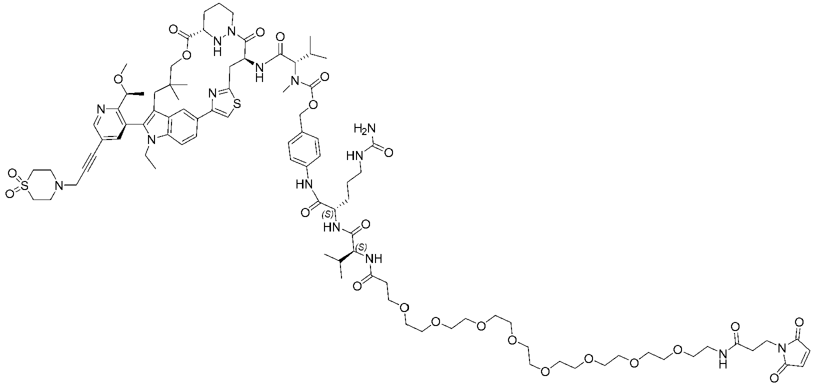 |
| Z22-L17 | 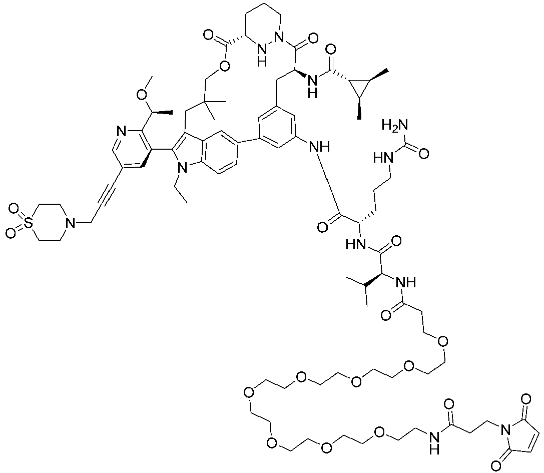 |
| Z22-L25 | 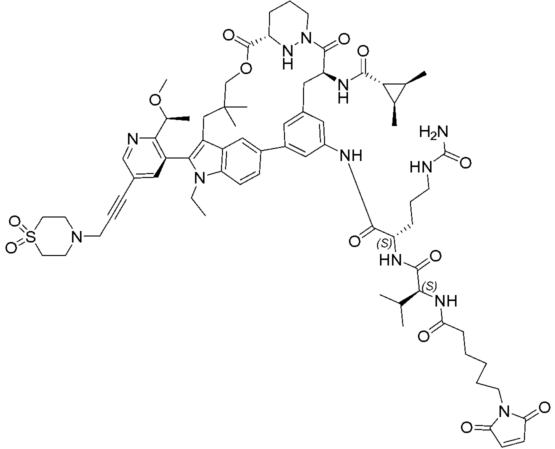 |
| Z3-L1 | 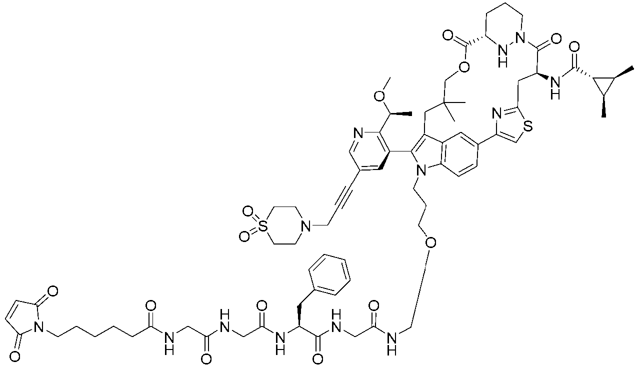 |
| Z169-1-L16 | 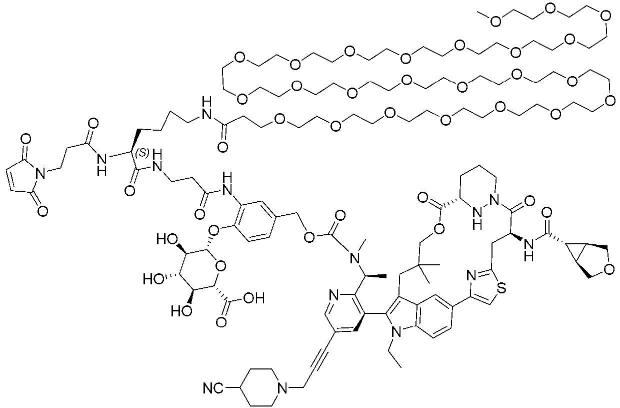 |
| Z283-L65 | 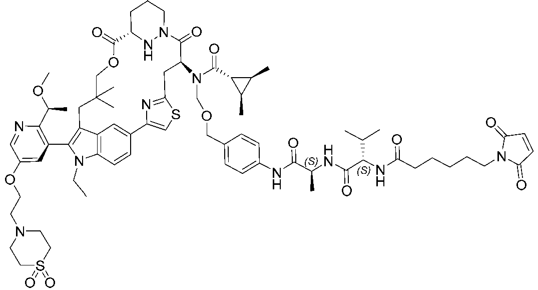 |
| Z284-L66 | 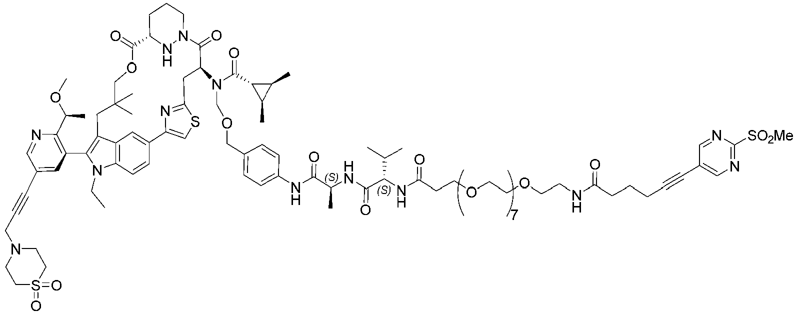 |
| Z285-L67 | 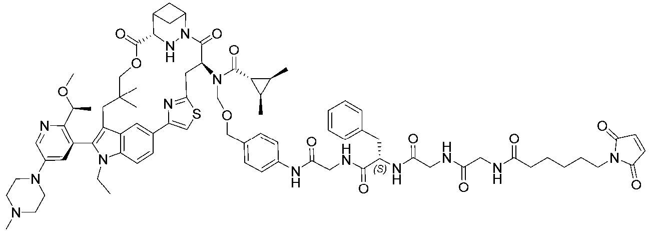 |
| Z284-L68 | 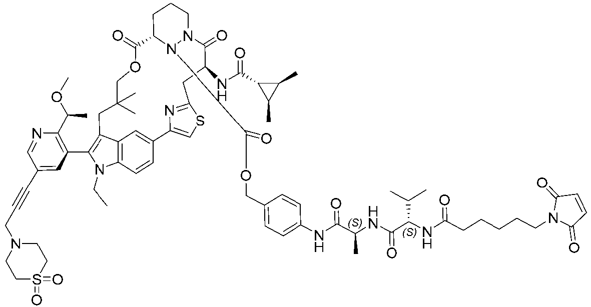 |
| Z286-L68 | 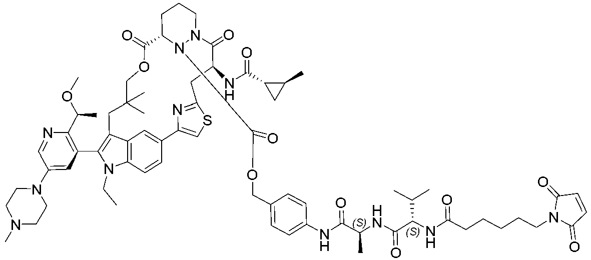 |
| Z285-L68 | 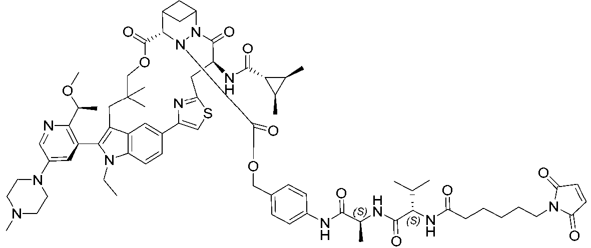 |
| Z287-L57 | 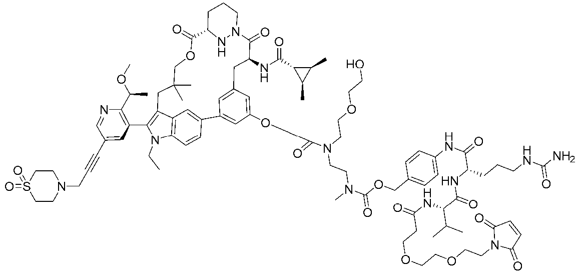 |
| Z283-L69 | 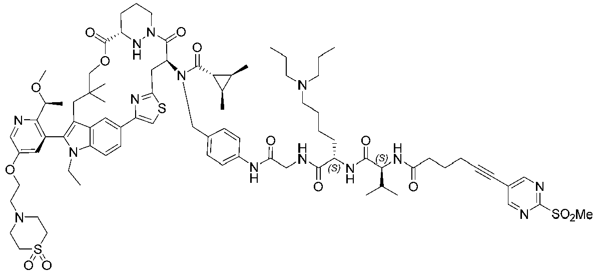 |
| Z284-A-L53 | 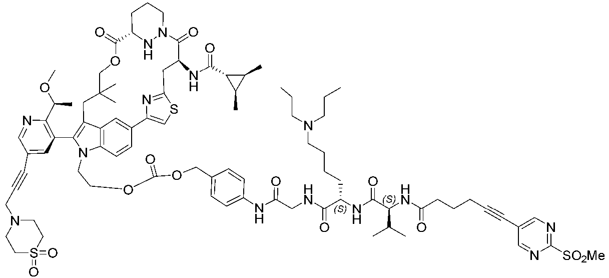 |
| Z286-A-L1 | 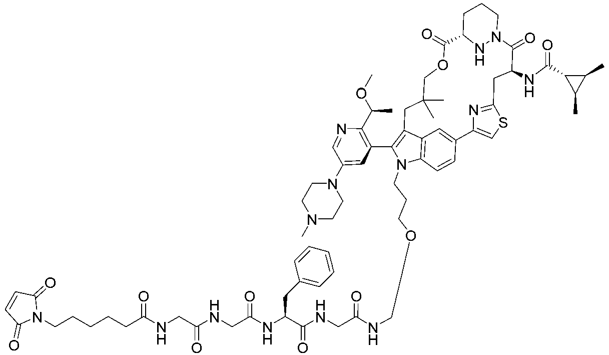 |
| Z285-A-L1 | 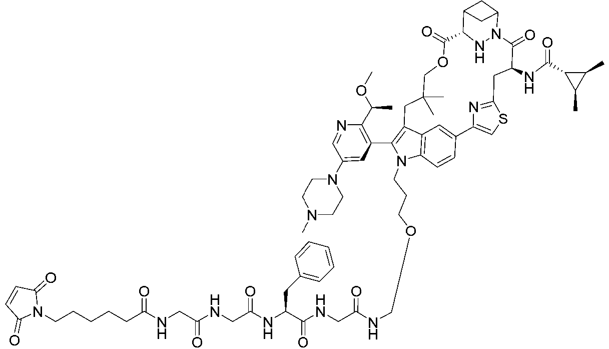 |
| Z285-A-L63 | 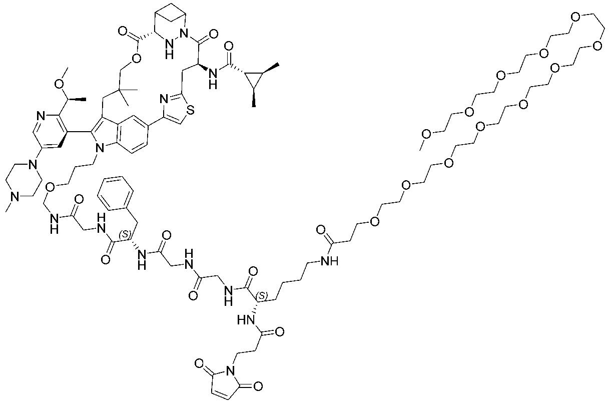 |
| Z289-L53 | 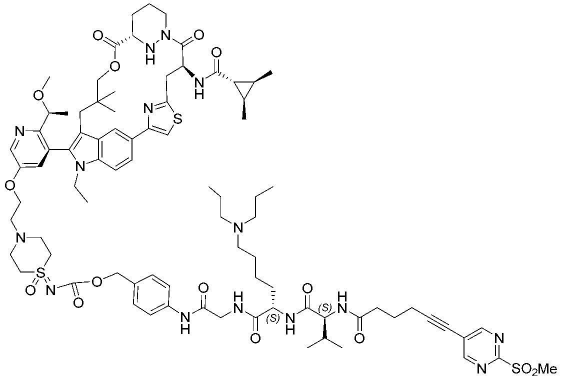 |
| Z286-B-L70 | 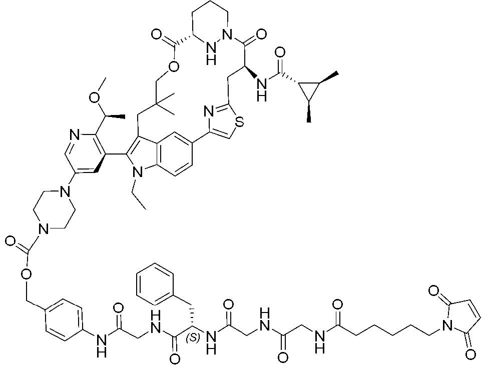 |
| Z290-L2 | 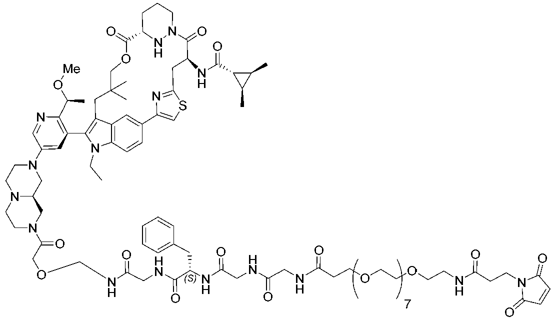 |
| Z285-B-L70 | 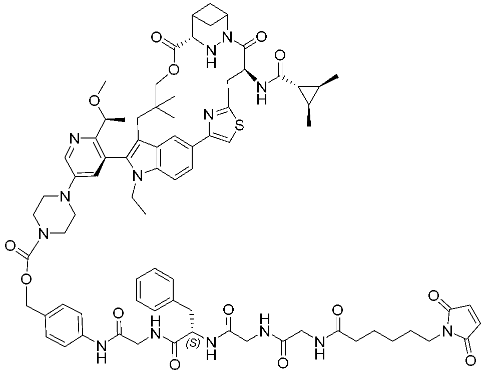 |
| Z286-B-L7 | 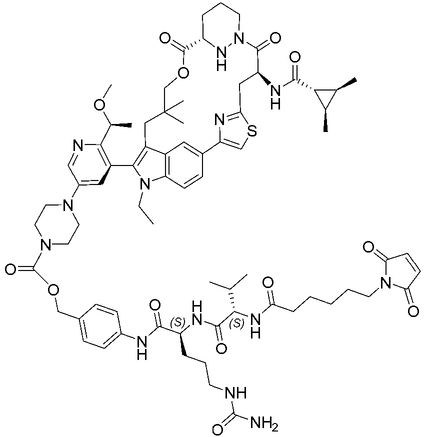 |
| Z288-L7 | 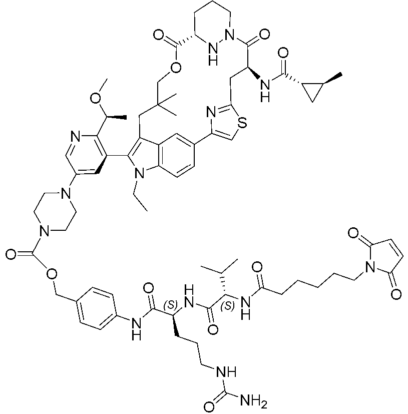 |
| Z285-B-L7 | 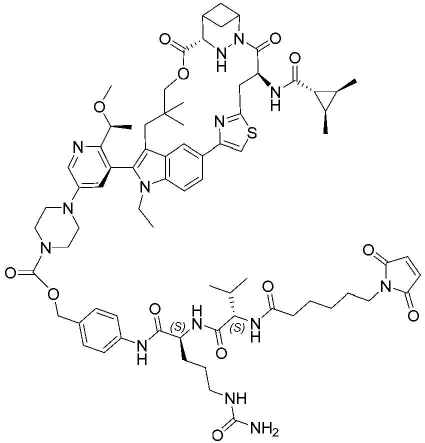 |
| Z288-L7 | 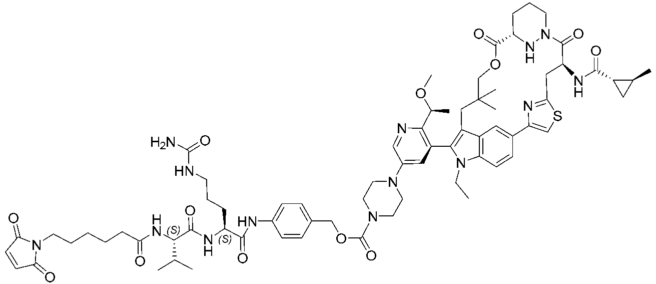 |
| 7286-172 | 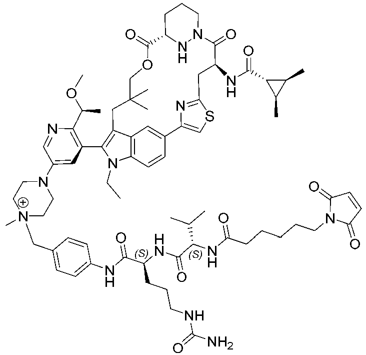 |
| Z288-A-L72 | 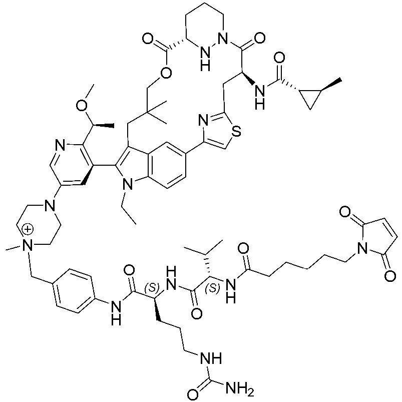 |
| Z285-L72 | 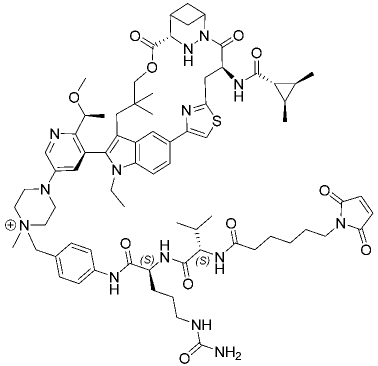 |
| Z285-B-L16 | 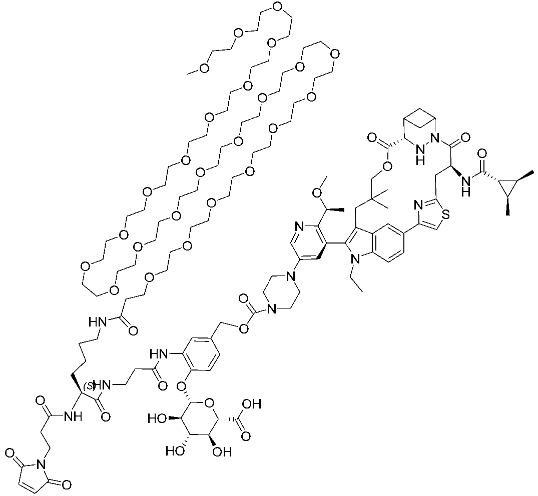 |
| Z286-B-L16 | 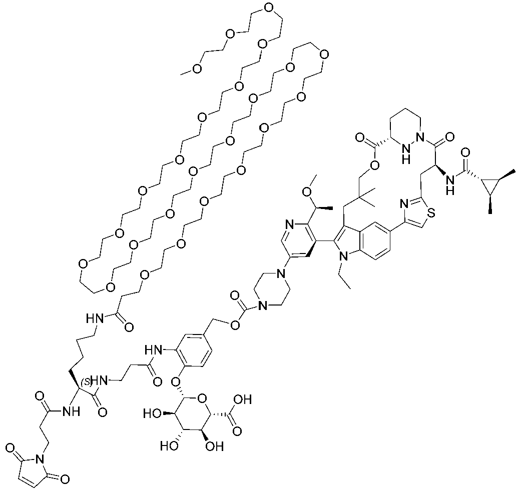 |
| Z288-L16 | 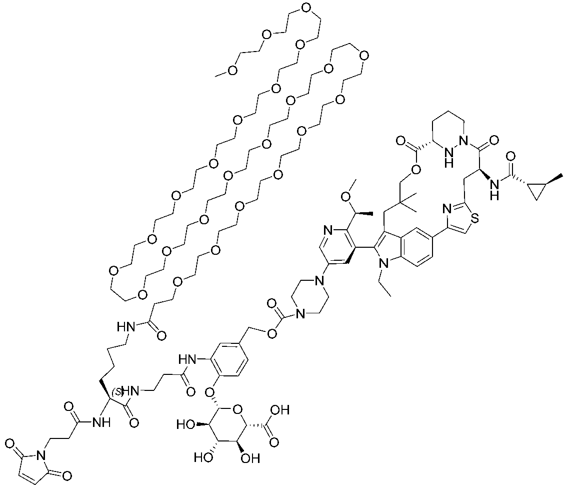 |
| Z285-L26 | 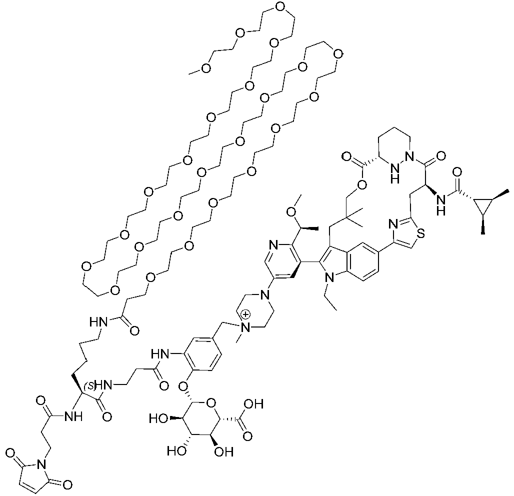 |
| Z286-L26 | 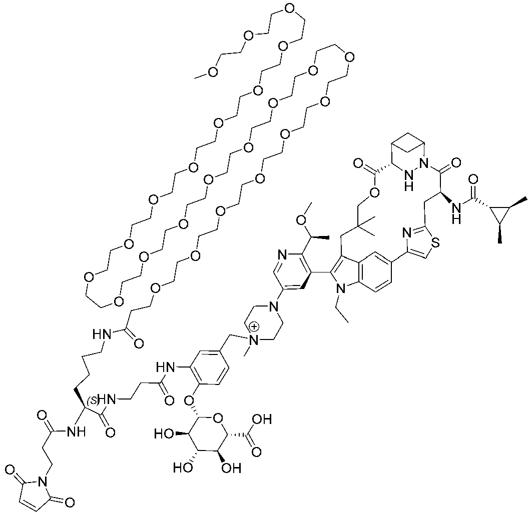 |
| Z288-A-L26 | 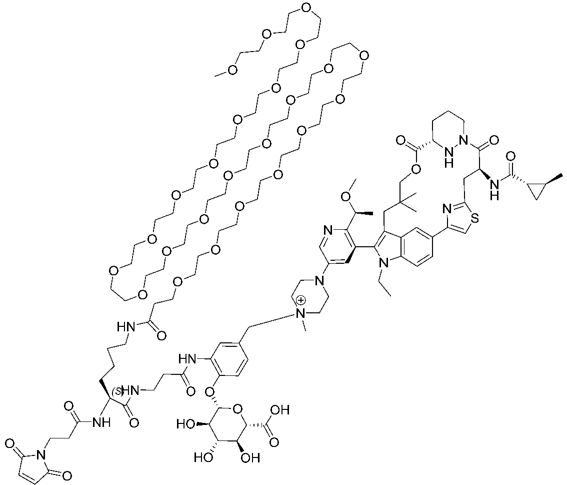 |
| Z285-B-L34 | 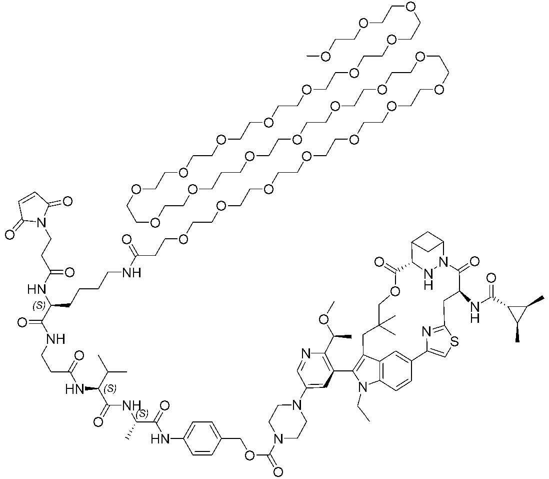 |
| Z286-B-L34 | 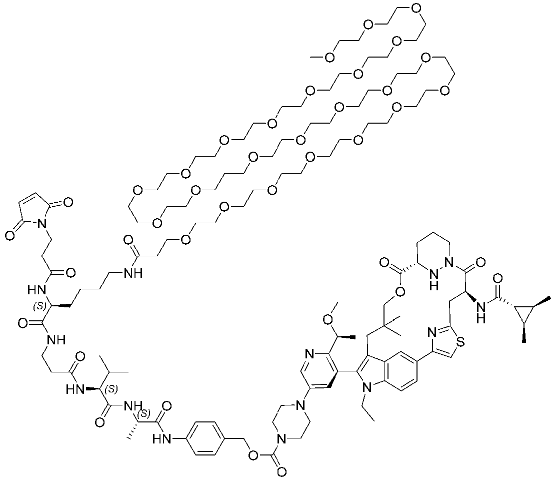 |
| Z285-L22 | 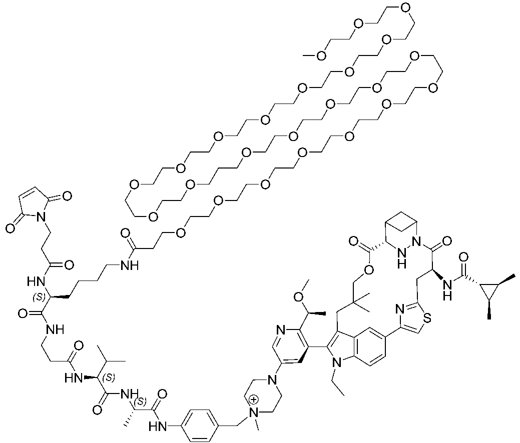 |
| Z286-L22 | 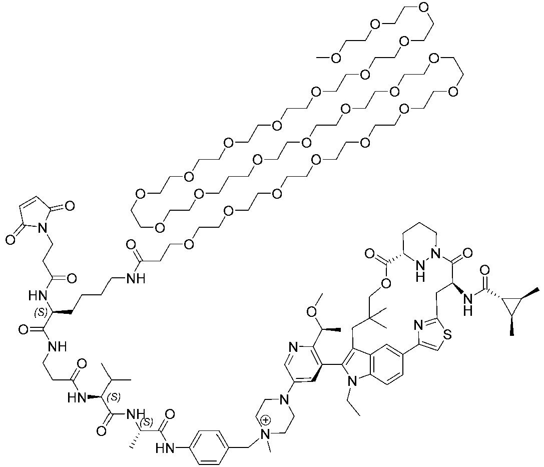 |
| Z288-A-L22 | 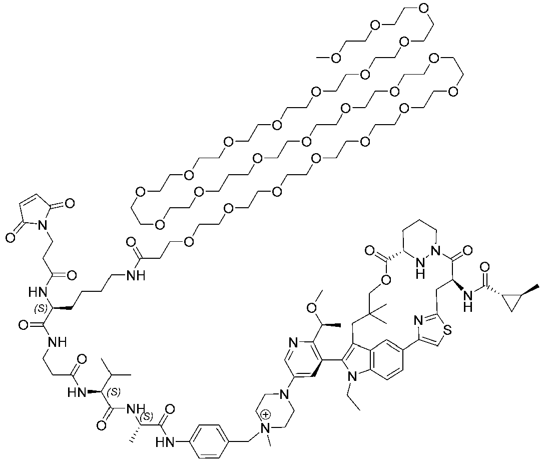 |
| Z7-2-L1 | 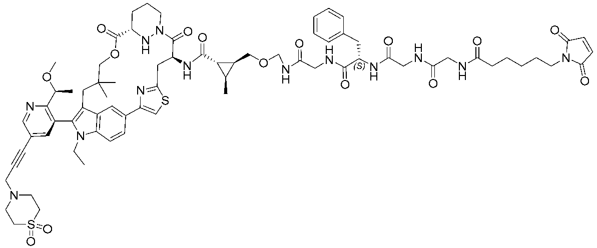 |
| Z291-L73 | 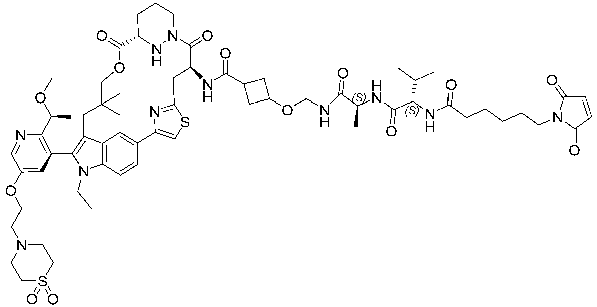 |
| Z310-L2 | 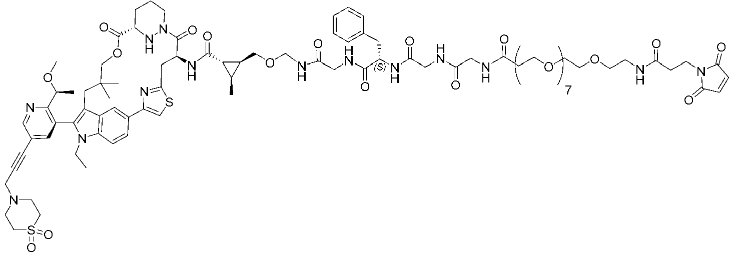 |
| Z292-L74 | 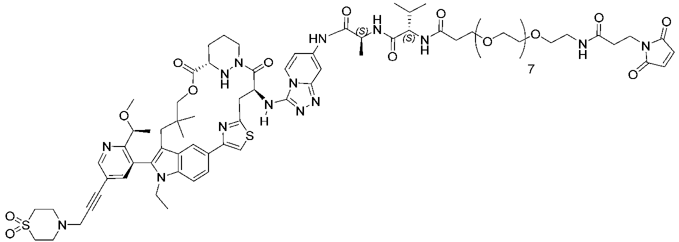 |
| Z7-2-L64 | 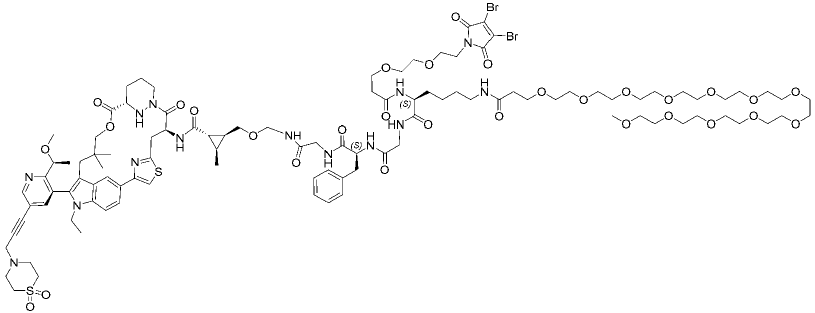 |
| Z7-2-L63 | 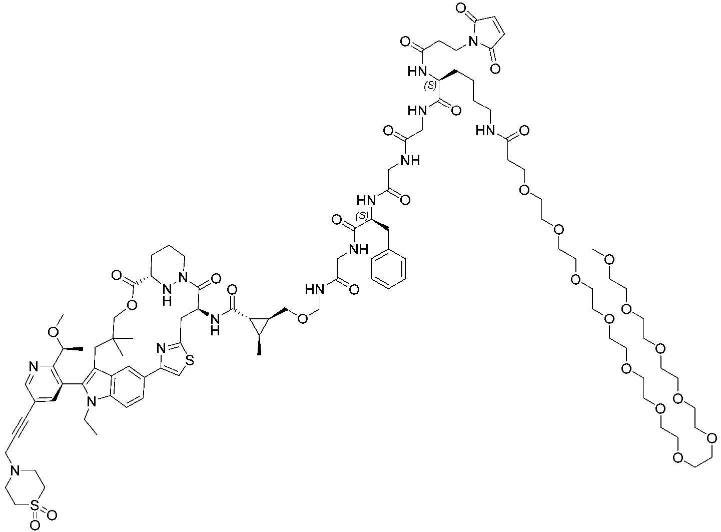 |
| Z293-L57 | 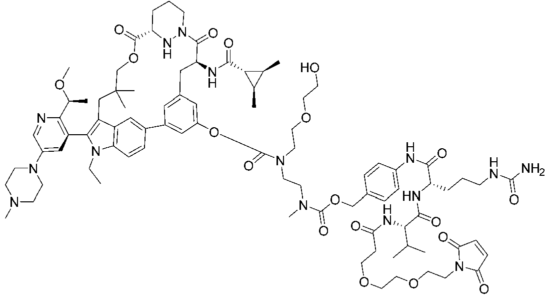 |
| Z294-L57 | 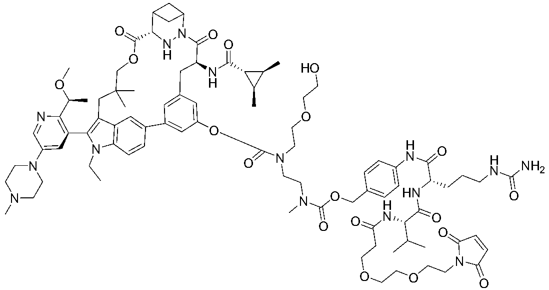 |
| Z88-L7 | 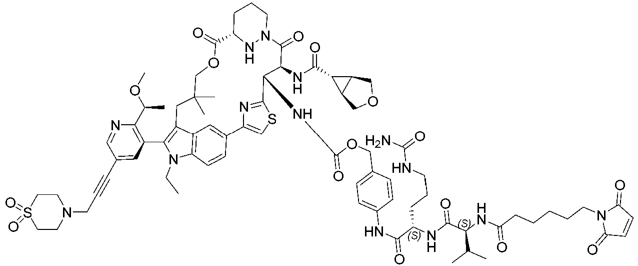 |
| Z295-L7 | 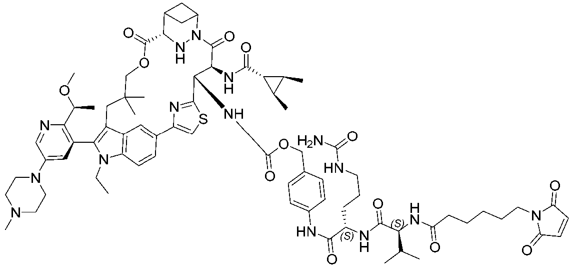 |
| Z296-L7 | 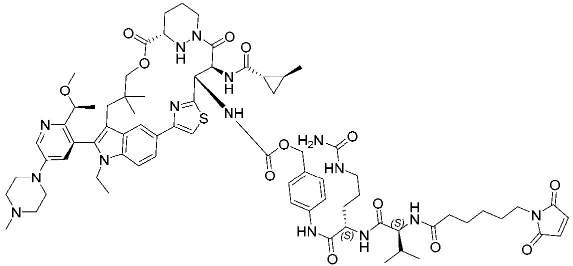 |
| Z297-L7 | 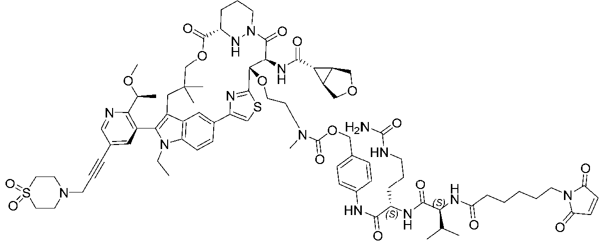 |
| Z297-A-L72 | 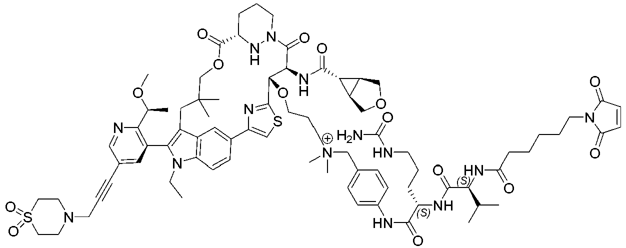 |
| Z284-L72 | 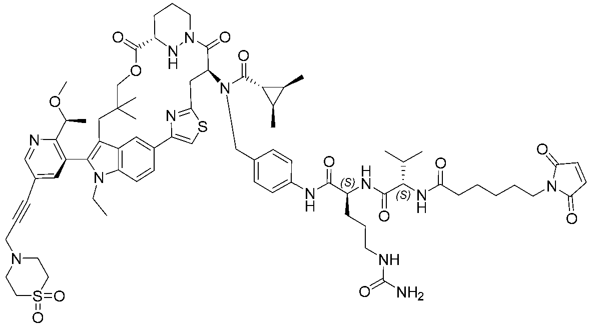 |
| Z192-C-L72 | 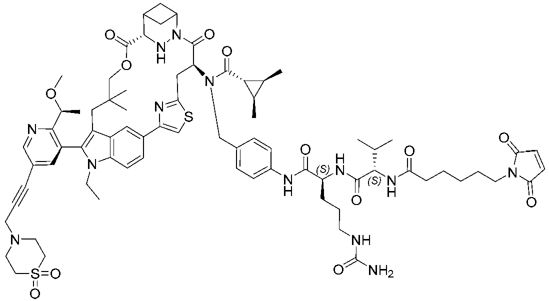 |
| Z283-L7 | 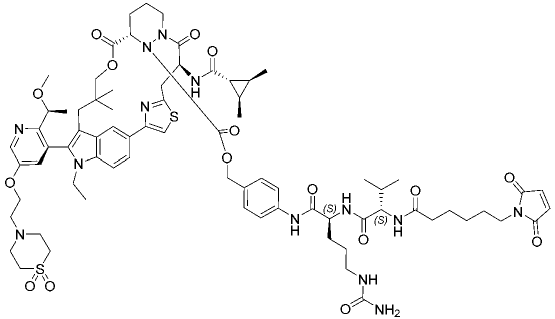 |
| Z298-L7 | 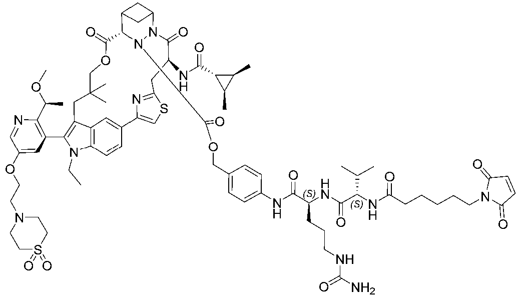 |
| Z299-L52 | 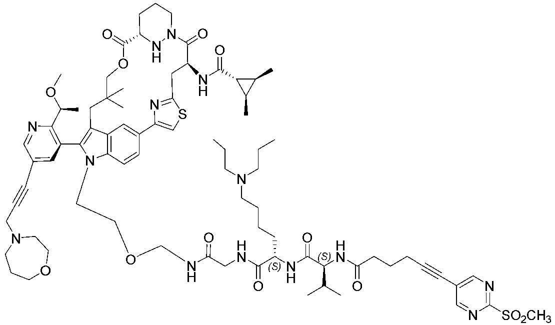 |
| Z187-A-L52 | 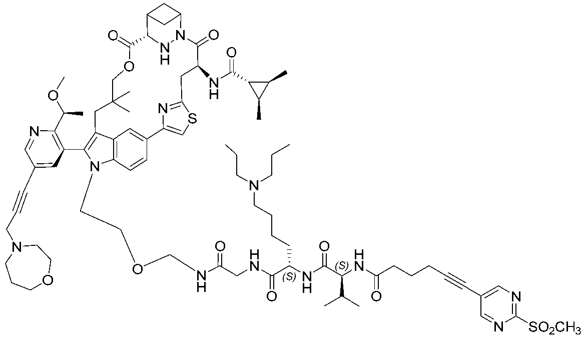 |
| Z300-L7 | 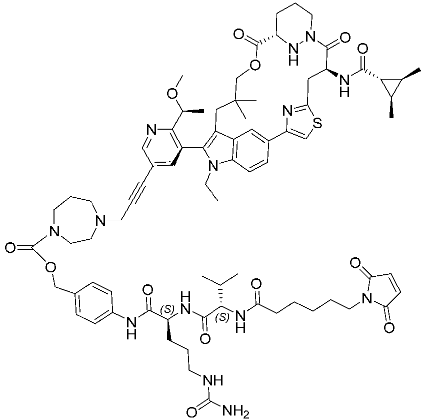 |
| Z301-L7 | 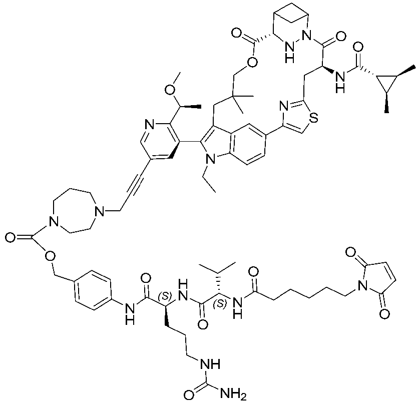 |
| Z299-A-L72 | 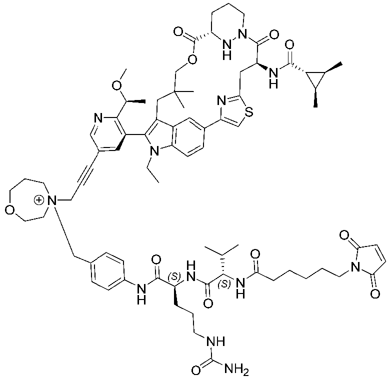 |
| Z187-L72 | 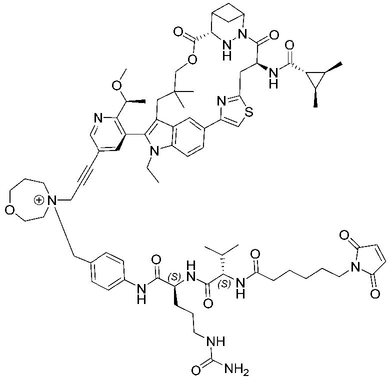 |
| Z302-L7 | 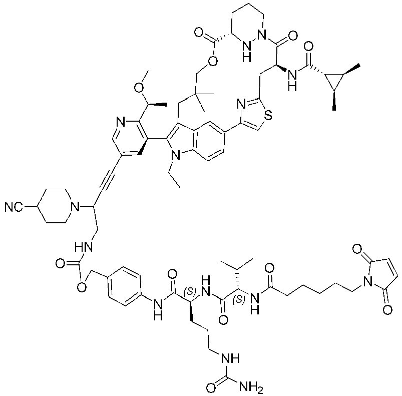 |
| Z303-L7 | 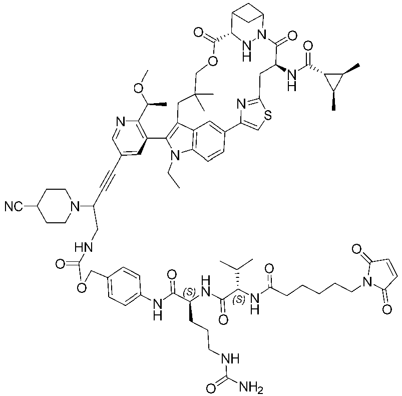 |
| Z7-2-L2 | 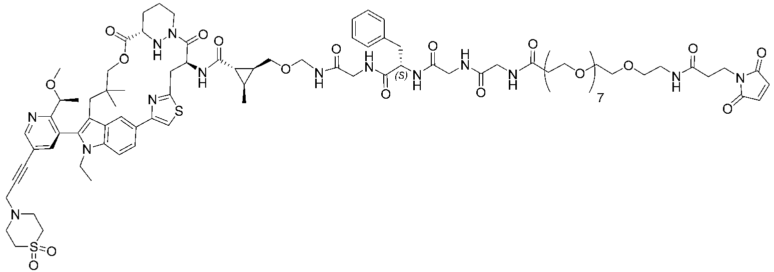 |
| Z192-A-L2 | 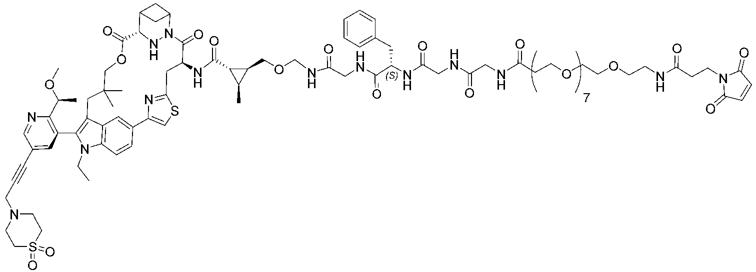 |
| Z304-L54-2 | 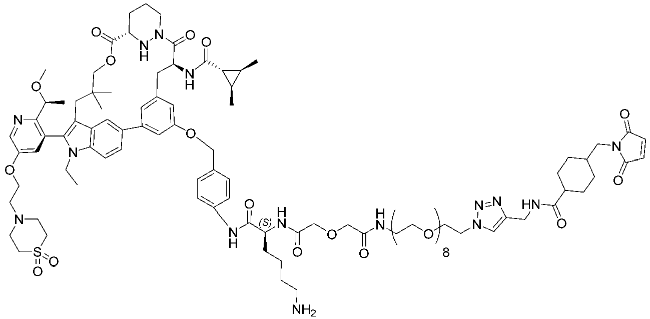 |
| Z311-L74 | 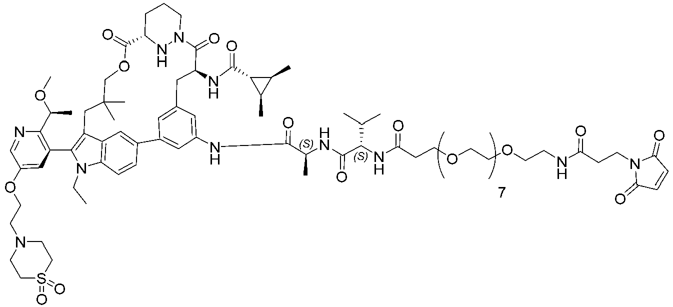 |
| Z305-L54-2 | 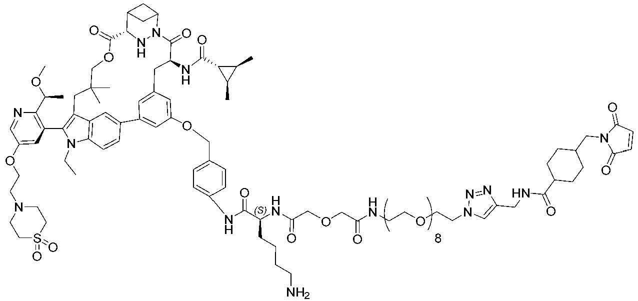 |
| Z305-A-L74 | 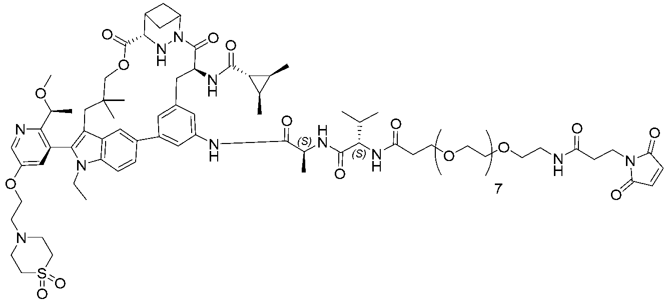 |
| Z306-L7 | 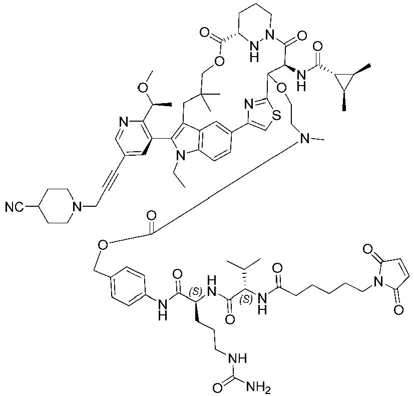 |
| Z307-L7 | 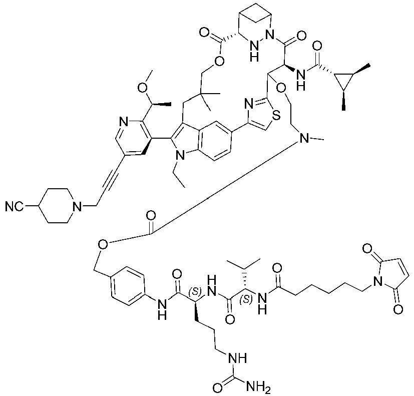 |
| Z282-A-L7 | 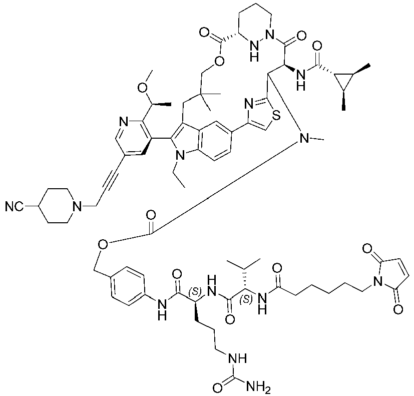 |
| Z308-L7 | 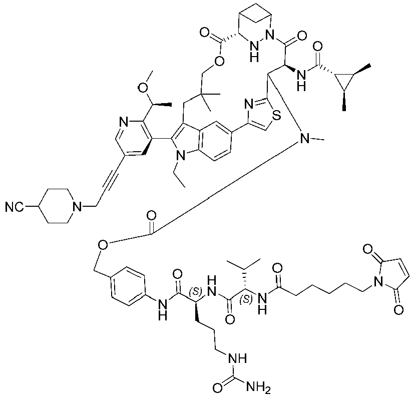 |
| Z67-A-L7 | 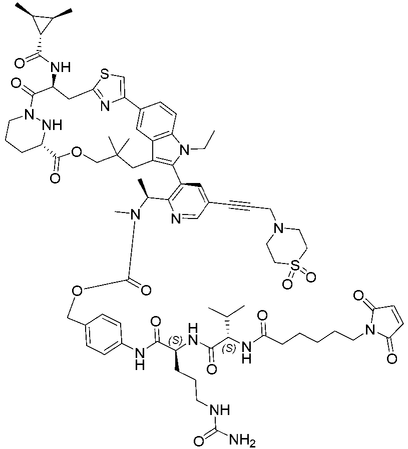 |
| Z309-L7 | 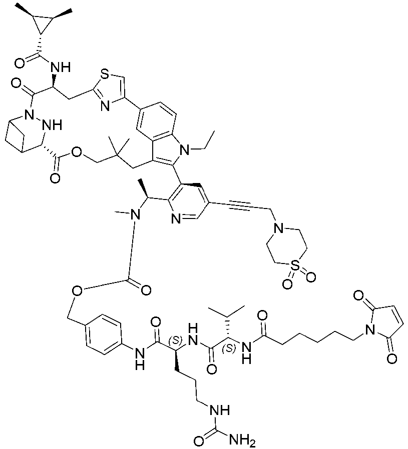 |
| Z52-L58 | 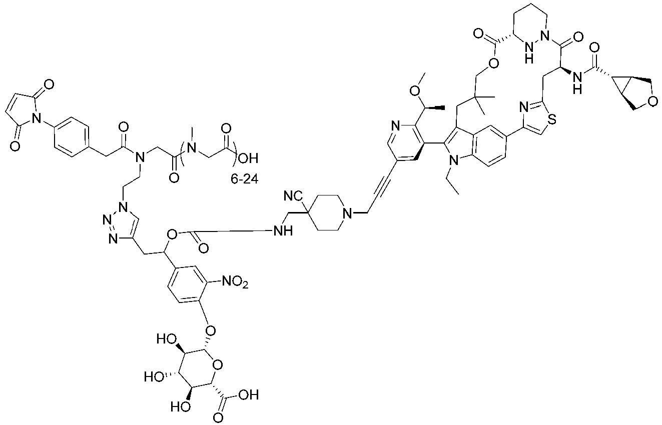 |
| Z52-L59 | 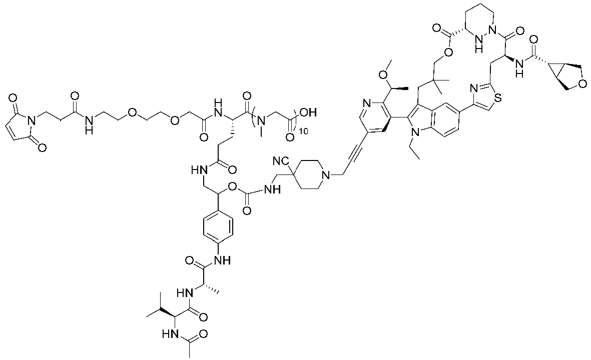 |
| Z52-L60 | 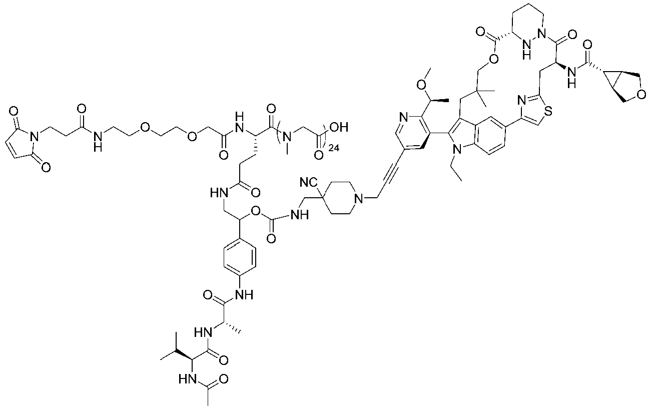 |

### Compound

In a third aspect, the present disclosure provides a compound represented by formula (Z-III), or a stable deuterated derivative thereof, a stereoisomer thereof, a pharmaceutically acceptable salt thereof, a solvate thereof, or a prodrug thereof: 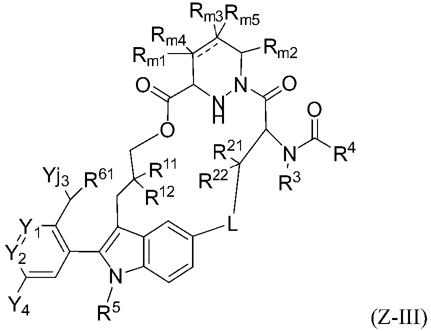 wherein in the formula,
Rₘ₁ is hydrogen; Rₘ₂ is hydrogen; Rₘ₃ is hydrogen; Rₘ₄ is hydrogen, halogen, or C₁₋₆ alkyl; Rₘ₅ is hydrogen, halogen, or C₁₋₆ alkyl; and 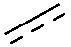 is a single bond; or
Rₘ₁ and Rₘ₃ are both absent; Rₘ₂ is hydrogen; Rₘ₄ is hydrogen, halogen, or C₁₋₆ alkyl; Rₘ₅ is hydrogen, halogen, or C₁₋₆ alkyl; and 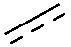 is a double bond; or
Rₘ₁ is attached to Rₘ₂ to jointly form -CRₘ₇Rₘ₆- or -CRₘ₇Rₘ₆-CRₘ₉Rₘ₈-; Rₘ₃ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 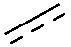 is a single bond; or
Rₘ₁ is attached to Rₘ₃ to jointly form -CRₘ₇Rₘ₆- or -CRₘ₇Rₘ₆-CRₘ₉Rₘ₈-; Rₘ₂ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 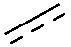 is a single bond; or
Rₘ₁ is attached to Rₘ₄ to jointly form -CRₘ₇Rₘ₆- or -CRₘ₇Rₘ₆-CRₘ₉Rₘ₈-; Rₘ₂ is hydrogen; Rₘ₃ is hydrogen; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 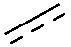 is a single bond;
R¹¹ and R¹² are each independently hydrogen, deuterated C₁₋₆ alkyl, or C₁₋₆ alkyl;
Y₁ is N or CH, wherein N may be oxidized (N⁺-O⁻);
Y₂ is N or CH, wherein N may be oxidized (N⁺-O⁻);
R³ is hydrogen or C₁₋₆ alkyl;
Yj₃ is NH-R⁶, O-R⁶, -C₁₋₄ alkylene-NH-R⁶, or -C₁₋₄ alkylene-O-R⁶;
R⁶ is hydrogen or C₁₋₆ alkyl;
R⁶¹ is C₁₋₆alkyl;
R²¹, R²², R⁵, L, R⁴, and Y₄ are as described in cases (1), (2), (3), (4), or (5):
   Case (1)
      R²¹ and R²² are each independently hydrogen, -NR^{f}R^{g}, -O-C₁₋₄ alkylene-NR^{f}R^{g}, or C₁₋₆ alkyl;
      L is C₆₋₁₀ aryl or 5- or 6-membered monocyclic heteroaryl, wherein the C₆₋₁₀ aryl is optionally substituted with hydroxy or amino;
      R⁵ is hydrogen, ethyl, -C₁₋₄ alkylene-OH, -C₁₋₄ alkylene-NHR^{e}, or -C₁₋₄ alkylene-C₁₋₆ alkoxy;
      R⁴ is C₆₋₁₀ aryl, heteroaryl, C₃₋₆ cycloalkyl, or -CR^{a}R^{b}-NR^{c}R^{d}, wherein the C₆₋₁₀ aryl, heteroaryl, or C₃₋₆ cycloalkyl is optionally substituted with one or more substituents selected from R⁴¹; R⁴¹ is SF₅, P(=O)(C₁₋₆ alkyl)₂, methyl, -C₁₋₄ alkylene-OH, or -C(O)-NR^{c}R^{d}; Y₄ is 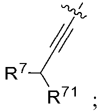 wherein:
         R⁷ is 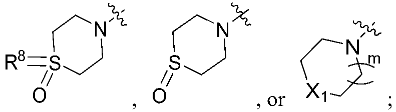
         R⁸ is O or NH; X₁ is -CR⁹¹R⁹²- or -NR¹⁰-;
         R⁷¹ is hydrogen, C₁₋₆ alkyl, -C₁₋₄ alkylene-OH, or -C₁₋₄ alkylene-NH-R⁶;
         R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen, and -C₁₋₄ alkylene- is optionally substituted with methyl;
         R¹⁰ is hydrogen, -C(O)-C₁₋₄ alkylene-NR^{f}R^{g}, or -C(O)-C₁₋₄ alkylene-OH;
         m is 1 or 2;
         R^{a} and R^{b} are each independently hydrogen, C₁₋₆ alkyl, or C₃₋₆ cycloalkyl;
         R^{c} and R^{d} are each independently hydrogen, -C(O)H, C₁₋₆ alkyl, C₁₋₆ haloalkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
         R⁴² is methyl, -NR^{f}R^{g}, -C(O)R^{e}, or -C(O)OR^{e};
         R^{e} is C₁₋₆ alkyl;
         when the following conditions are satisfied, R⁷ is not 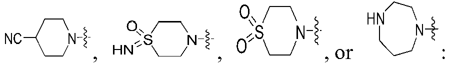 Rₘ₁ is hydrogen; Rₘ₂ is hydrogen; Rₘ₃ is hydrogen; Rₘ₄ is hydrogen; Rₘ₅ is hydrogen; 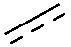 is a single bond; R⁴ is 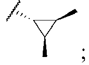 R³, R²¹, and R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is ethyl;
         when the following conditions are satisfied, R⁷ is not 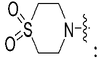 Rₘ₁ is hydrogen; Rₘ₂ is hydrogen; Rₘ₃ is hydrogen; Rₘ₄ is hydrogen; Rₘ₅ is hydrogen; 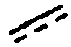 is a single bond; R⁴ is 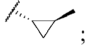 R³, R²¹, and R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is ethyl;
         when the following conditions are satisfied, R⁷ is not 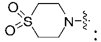 Rₘ₁ is hydrogen; Rₘ₂ is hydrogen; Rₘ₃ is hydrogen; Rₘ₄ is hydrogen; Rₘ₅ is hydrogen; 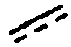 is a single bond; R⁴ is 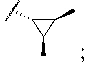 R³, R²¹, and R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is -CH₂CH₂OH or -CH₂CH₂NH(CH₃);
            or
   Case (2)
      R²¹ and R²² are each independently hydrogen, -NR^{f}R^{g}, -O-C₁₋₄ alkylene-NR^{f}R^{g}, or C₁₋₆ alkyl;
      L is C₆₋₁₀ aryl or 5- or 6-membered monocyclic heteroaryl, wherein the C₆₋₁₀ aryl is optionally substituted with hydroxy or amino;
      R⁵ is hydrogen, ethyl, -C₁₋₄ alkylene-OH, -C₁₋₄ alkylene-NHR^{e}, or -C₁₋₄ alkylene-C₁₋₆ alkoxy;
      R⁴ is C₆₋₁₀ aryl, heteroaryl, C₃₋₆ cycloalkyl, or 5- to 8-membered heterocyclyl, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d}; Y₄ is 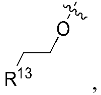
      wherein R¹³ is as described in case (21) or (22):
         case (21): R¹³ is 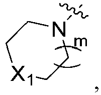 wherein X₁ is -CR⁹¹R⁹²-; R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen; m is 1 or 2; and Y₁ is N⁺-O⁻; Y₂ is CH; or Y₁ is CH; Y₂ is N⁺-O⁻;
            or
         case (22): R¹³ is oxo-5-membered heterocyclyl-L₁-L₂-R^{I4}, oxo-6-membered heterocyclyl-L₁-L₂-R^{I4}, or oxo-7-membered heterocyclyl-L₁-L₂-R¹⁴; L₁ is -C₁₋₄ alkylene-O- or -C₁₋₄ alkylene-NH-; L₂ is -C₁₋₄ alkylene-O- or -C₁₋₄ alkylene-NH-; R¹⁴ is C₁₋₆ heteroalkyl;
         R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
         R⁴² is methyl, -NR^{f}R^{g}, -C(O)R^{e}, or -C(O)OR^{e};
         R^{e} is C₁₋₆ alkyl;
   Case (3)
      R²¹ and R²² are each independently hydrogen, -NR^{f}R^{g}, -O-C₁₋₄ alkylene-NR^{f}R^{g}, or C₁₋₆ alkyl;
      L is C₆₋₁₀ aryl or 5- or 6-membered monocyclic heteroaryl, wherein the C₆₋₁₀ aryl is optionally substituted with hydroxy or amino;
      R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
      R⁴ is 5- to 8-membered heterocyclyl;
      Y₄ is 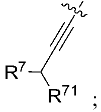
      wherein:
         R⁷ is 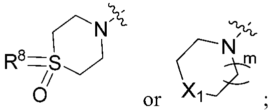
         R⁸ is O or NH;
         X₁ is -CR⁹¹R⁹²- or -NR¹⁰-;
         R⁷¹ is hydrogen, C₁₋₆ alkyl, -C₁₋₄ alkylene-OH, or -C₁₋₄ alkylene-NH-R⁶;
         R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen;
         R¹⁰ is hydrogen, C₁₋₆ alkyl, -C(O)-C₁₋₄ alkylene-NR^{f}R^{g}, or -C(O)-C₁₋₄ alkylene-OH;
         m is 1 or 2;
         R^{a} and R^{b} are each independently hydrogen, C₁₋₆ alkyl, or C₃₋₆ cycloalkyl;
         R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
         R⁴² is methyl, -NR^{f}R^{g}, -C(O)R^{e}, or -C(O)OR^{e};
         R^{e} is C₁₋₆ alkyl;
   Case (4)
      R²¹ and R²² are each independently hydrogen, -NR^{f}R^{g}, -O-C₁₋₄ alkylene-NR^{f}R^{g}, or C₁₋₆ alkyl; and R²¹ and R²² are not both hydrogen;
      L is C₆₋₁₀ aryl or 5- or 6-membered monocyclic heteroaryl, wherein the C₆₋₁₀ aryl is optionally substituted with hydroxy or amino;
      R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
      R⁴ is C₆₋₁₀ aryl, heteroaryl, C₃₋₆ cycloalkyl, or -CR^{a}R^{b}-NR^{c}R^{d}, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d}; Y₄ is 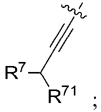
      wherein:
         R⁷ is 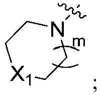
         X₁ is -NR¹⁰-;
         R⁷¹ is hydrogen, C₁₋₆ alkyl, -C₁₋₄ alkylene-OH, or -C₁₋₄ alkylene-NH-R⁶;
         R¹⁰ is C₁₋₆ alkyl;
         m is 1 or 2;
         R^{a} and R^{b} are each independently hydrogen, C₁₋₆ alkyl, or C₃₋₆ cycloalkyl;
         R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
         R⁴² is methyl, -NR^{f}R^{g}, -C(O)R^{e}, or -C(O)OR^{e};
         R^{e} is C₁₋₆ alkyl;
         Case (5)
         R²¹ and R²² are each independently hydrogen, -NR^{f}R^{g}, -O-C₁₋₄ alkylene-NR^{f}R^{g}, or C₁₋₆ alkyl;
         L is oxazolyl;
         R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
         R⁴ is C₆₋₁₀ aryl, heteroaryl, C₃₋₆ cycloalkyl, 5- to 8-membered heterocyclyl, or -CR^{a}R^{b}-NR^{c}R^{d}, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d}; Y₄ is 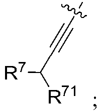
         wherein:
            R⁷ is 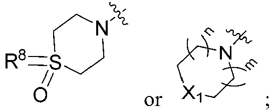 R⁷ may optionally be substituted with -C₁₋₄ alkylene-OH or -C₁₋₄ alkylene-NH-R⁶;
            R⁸ is O or NH; X₁ is -O-, -CR⁹¹R⁹²-, or -NR¹⁰-;
            R⁷¹ is hydrogen, C₁₋₆ alkyl, -C₁₋₄ alkylene-OH, or -C₁₋₄ alkylene-NH-R⁶;
            R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}, and -C₁₋₄ alkylene- may be substituted with methyl;
            R¹⁰ is hydrogen, C₁₋₆ alkyl, -C(O)-C₁₋₄ alkylene-NR^{f}R^{g}, or -C(O)-C₁₋₄ alkylene-OH;
            m is 0, 1, or 2;
            n is 0, 1, or 2;
            R^{a} and R^{b} are each independently hydrogen, C₁₋₆ alkyl, or C₃₋₆ cycloalkyl;
            R^{c} and R^{d} are each independently hydrogen, -C(O)H, C₁₋₆ alkyl, phenyl, pyridinyl, -C₁₋₄ alkylene-OH, -C(O)-5-to 8-membered heterocyclyl, or -C(O)-C₁₋₄ alkylene-OH, wherein the 5- to 8-membered heterocyclyl is optionally substituted with one or more substituents selected from R⁴²;
            R⁴² is methyl, -NR^{f}R^{g}, -C(O)R^{e}, or -C(O)OR^{e};
            R^{e} is C₁₋₆ alkyl;
            R^{f} is hydrogen or C₁₋₆ alkyl;
            R^{g} is hydrogen or C₁₋₆ alkyl.

In the third aspect, the present disclosure further provides a compound represented by formula (Z-IIIA), or a stable deuterated derivative thereof, a stereoisomer thereof, a pharmaceutically acceptable salt thereof, a solvate thereof, or a prodrug thereof: 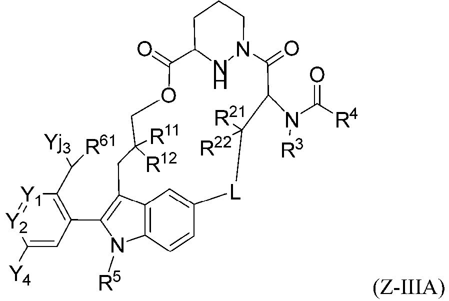 wherein in the formula,
R¹¹ and R¹² are each independently hydrogen, deuterated C₁₋₆ alkyl, or C₁₋₆ alkyl;
R²¹ and R²² are each independently hydrogen, -NR^{f}R^{g}, -O-C₁₋₄ alkylene-NR^{f}R^{g}, or C₁₋₆ alkyl;
Y₁ is N or CH, wherein N may be oxidized (N⁺-O⁻);
Y₂ is N or CH, wherein N may be oxidized (N⁺-O⁻);
L is C₆₋₁₀ aryl or 5- or 6-membered monocyclic heteroaryl, wherein the C₆₋₁₀ aryl is optionally substituted with hydroxy or amino;
R³ is hydrogen or C₁₋₆ alkyl;
R⁵ is hydrogen, ethyl, -C₁₋₄ alkylene-OH, -C₁₋₄ alkylene-NHR^{e}, or -C₁₋₄ alkylene-C₁₋₆ alkoxy;
Yj₃ is NH-R⁶, O-R⁶, -C₁₋₄ alkylene-NH-R⁶, or -C₁₋₄ alkylene-O-R⁶;
R⁶ is hydrogen or C₁₋₆ alkyl;
R⁶¹ is C₁₋₆alkyl;
R⁴ and Y₄ are as described in case (1) or (2):
   case (1): R⁴ is C₃₋₆ cycloalkyl, 5- to 8-membered heterocyclyl, or -CR^{a}R^{b}-NR^{c}R^{d}, wherein the C₃₋₆ cycloalkyl is optionally substituted with one or more substituents selected from R⁴¹; R⁴¹ is methyl, -C₁₋₄ alkylene-OH, or - C(O)-NR^{c}R^{d}; Y₄ is 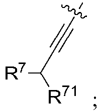 wherein:
      R⁷ is 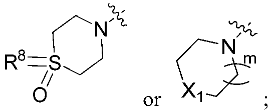
      R⁸ is O or NH; X₁ is -CR⁹¹R⁹²- or -NR¹⁰-;
      R⁷¹ is hydrogen, C₁₋₆ alkyl, -C₁₋₄ alkylene-OH, or -C₁₋₄ alkylene-NH-R⁶;
      R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen;
      R¹⁰ is hydrogen, -C(O)-C₁₋₄ alkylene-NR^{f}R^{g}, or -C(O)-C₁₋₄ alkylene-OH;
      m is 1 or 2;
      R^{a} and R^{b} are each independently hydrogen, C₁₋₆ alkyl, or C₃₋₆ cycloalkyl;
      R^{c} and R^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
      R⁴² is methyl, -NR^{f}R^{g}, -C(O)R^{e}, or -C(O)OR^{e};
      R^{e} is C₁₋₆ alkyl;
      R^{f} is hydrogen or C₁₋₆ alkyl;
      R^{g} is hydrogen or C₁₋₆ alkyl;
      when the following conditions are satisfied, R⁷ is not 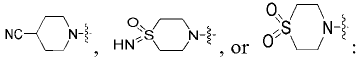 R⁴ is 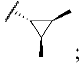 R³, R²¹, and R²² are hydrogen; Yj₃ is O-R⁶; Y₁ is N; Y₂ is CH; R¹¹, R¹², R⁶, and R⁶¹ are methyl; R⁷¹ is hydrogen; and R⁵ is ethyl;
      when the following conditions are satisfied, R⁷ is not 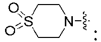 R⁴ is 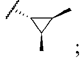 R³, R²¹, and R²² are hydrogen; Yj₃ is O-R⁶; Y₁ is N; Y₂ is CH; R¹¹, R¹², R⁶, and R⁶¹ are methyl; R⁷¹ is hydrogen; and R⁵ is -CH₂CH₂OH or - CH₂CH₂NH(CH₃);
         or
   case (2): when the following conditions are satisfied, Y₄ is 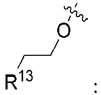 R⁴ is C₃₋₆ cycloalkyl or 5- to 8-membered heterocyclyl, wherein the C₃₋₆ cycloalkyl is optionally substituted with one or more substituents selected from R⁴¹; and R⁴¹ is methyl, -C₁₋₄ alkylene-OH, or -C(O)-NR^{c}R^{d};
      R¹³ is as described in case (21) or (22):
      case (21): R¹³ is 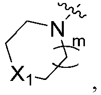 wherein X₁ is -CR⁹¹R⁹²-; R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen; m is 1 or 2; and Y₁ is N⁺-O⁻; Y₂ is CH; or Y₁ is CH; Y₂ is N⁺-O⁻;
         or
      case (22): R¹³ is oxo-5-membered heterocyclyl-L₁-L₂-R¹⁴, oxo-6-membered heterocyclyl-L₁-L₂-R¹⁴, or oxo-7-membered heterocyclyl-L₁-L₂-R¹⁴; L₁ is -C₁₋₄ alkylene-O- or -C₁₋₄ alkylene-NH-; L₂ is -C₁₋₄ alkylene-O- or -C₁₋₄ alkylene-NH-; R¹⁴ is C₁₋₆ heteroalkyl;
      R^{c} and R^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
      R⁴² is methyl, -NR^{f}R^{g}, -C(O)R^{e}, or -C(O)OR^{e};
      R^{e} is C₁₋₆ alkyl;
      R^{f} is hydrogen or C₁₋₆ alkyl;
      R^{g} is hydrogen or C₁₋₆ alkyl.

In the third aspect, the present disclosure further provides a compound represented by formula (Z-IB), or a stable deuterated derivative thereof, a stereoisomer thereof, a pharmaceutically acceptable salt thereof, a solvate thereof, or a prodrug thereof: 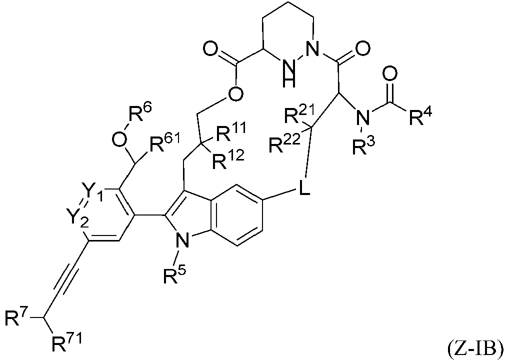 wherein in the formula,
R¹¹ and R¹² are each independently hydrogen, deuterated C₁₋₆ alkyl, or C₁₋₆ alkyl;
R²¹ and R²² are each independently hydrogen or C₁₋₆ alkyl;
Y₁ is N or CH, wherein N may be oxidized (N⁺-O⁻);
Y₂ is N or CH, wherein N may be oxidized (N⁺-O⁻);
L is C₆₋₁₀ aryl or 5- or 6-membered monocyclic heteroaryl, wherein the C₆₋₁₀ aryl is optionally substituted with hydroxy or amino;
R³ is hydrogen or C₁₋₆ alkyl;
R⁴ is C₃₋₆ cycloalkyl, 5- to 8-membered heterocyclyl, or -CR^{a}R^{b}-NR^{c}R^{d}, wherein the C₃₋₆ cycloalkyl is optionally substituted with one or more substituents selected from R⁴¹;
R⁴¹ is methyl, -C₁₋₄ alkylene-OH, or -C(O)-NR^{c}R^{d};
R⁵ is hydrogen, ethyl, -C₁₋₄ alkylene-OH, -C₁₋₄ alkylene-NHR^{e}, or -C₁₋₄ alkylene-C₁₋₆ alkoxy;
R⁶ is C₁₋₆ alkyl; R⁶¹ is C₁₋₆ alkyl;
R⁷ is 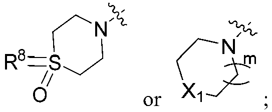
wherein, R⁸ is O or NH; X₁ is -CR⁹¹R⁹²- or -NR¹⁰-;
R⁷¹ is hydrogen, C₁₋₆ alkyl, or -C₁₋₄ alkylene-OH;
R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen;
R¹⁰ is hydrogen, -C(O)-C₁₋₄ alkylene-NH₂, or -C(O)-C₁₋₄ alkylene-OH;
m is 1 or 2;
R^{a} and R^{d} are each independently hydrogen, C₁₋₆ alkyl, or C₃₋₆ cycloalkyl;
R^{c} and R^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -C(O)R^{e}, or -C(O)OR^{e};
R^{e} is C₁₋₆ alkyl;
when the following conditions are satisfied, R⁷ is not 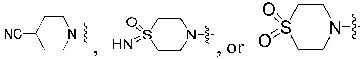 : R⁴ is 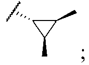 R³, R²¹, and R²² are hydrogen; R¹¹, R¹², R⁶, and R⁶¹ are methyl; and R⁵ is ethyl; when the following conditions are satisfied, R⁷ is not 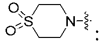 R⁴ is 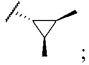 R³, R²¹, and R²² are hydrogen; R¹¹, R¹², R⁶, and R⁶¹ are methyl; and R⁵ is -CH₂CH₂OH or -CH₂CH₂NH(CH₃).

In the third aspect, the present disclosure further provides a compound represented by formula (Z-IA), or a stable deuterated derivative thereof, a stereoisomer thereof, a pharmaceutically acceptable salt thereof, a solvate thereof, or a prodrug thereof: 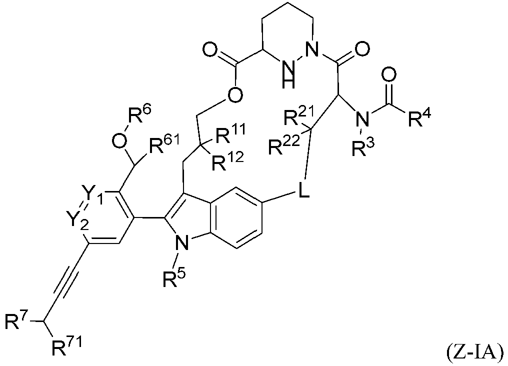 wherein in the formula,
R¹¹ and R¹² are each independently hydrogen, deuterated C₁₋₆ alkyl, or C₁₋₆ alkyl;
R²¹ and R²² are each independently hydrogen or C₁₋₆ alkyl;
Y₁ is N or CH, wherein N may be oxidized (N⁺-O⁻);
Y₂ is N or CH, wherein N may be oxidized (N⁺-O⁻);
L is C₆₋₁₀ aryl or 5- or 6-membered monocyclic heteroaryl, wherein the C₆₋₁₀ aryl is optionally substituted with hydroxy or amino;
R³ is hydrogen or C₁₋₆ alkyl;
R⁴ is C₃₋₆ cycloalkyl, 5- to 8-membered heterocyclyl, or -CR^{a}R^{b}-NR^{c}R^{d}, wherein the C₃₋₆ cycloalkyl is optionally substituted with one or more substituents selected from R⁴¹;
R⁴¹ is methyl, -C₁₋₄ alkylene-OH, or -C(O)-NR^{c}R^{d};
R⁵ is hydrogen, ethyl, -C₁₋₄ alkylene-OH, -C₁₋₄ alkylene-NHR^{e}, or -C₁₋₄ alkylene-C₁₋₆ alkoxy;
R⁶ is C₁₋₆ alkyl; R⁶¹ is C₁₋₆ alkyl;
R⁷ is 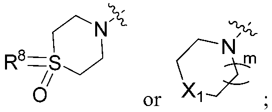
wherein, R⁸ is O or NH; X₁ is -CR⁹¹R⁹²- or -NR¹⁰-;
R⁷¹ is hydrogen, C₁₋₆ alkyl, or -C₁₋₄ alkylene-OH;
R⁹¹ and R⁹² are each independently hydrogen, hydroxy, cyano, -C₁₋₄ alkylene-OH, or -C(O)-NR^{c}R^{d}; R⁹¹ and R⁹² are not both hydrogen;
R¹⁰ is hydrogen, -C(O)-C₁₋₄ alkylene-NH₂, or -C(O)-C₁₋₄ alkylene-OH;
m is 1 or 2;
R^{a} and R^{b} are each independently hydrogen, C₁₋₆ alkyl, or C₃₋₆ cycloalkyl;
R^{c} and R^{d} are each independently hydrogen, C₁₋₆ alkyl, -C₁₋₄ alkylene-OH, or -C(O)-C₁₋₄ alkylene-OH;
R^{e} is C₁₋₆ alkyl;
when the following conditions are satisfied, R⁷ is not 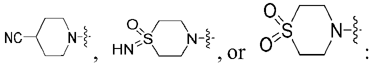 R⁴ is 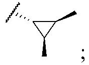 R³, R²¹, and R²² are hydrogen; R¹¹, R¹², R⁶, and R⁶¹ are methyl; and R⁵ is ethyl; when the following conditions are satisfied, R⁷ is not 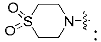 R⁴ is 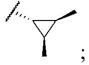 R³, R²¹, and R²² are hydrogen; R¹¹, R¹², R⁶, and R⁶¹ are methyl; and R⁵ is -CH₂CH₂OH or -CH₂CH₂NH(CH₃).

In the third aspect, the present disclosure further provides a compound represented by formula (Z-I), or a stable deuterated derivative thereof, a stereoisomer thereof, a pharmaceutically acceptable salt thereof, a solvate thereof, or a prodrug thereof: 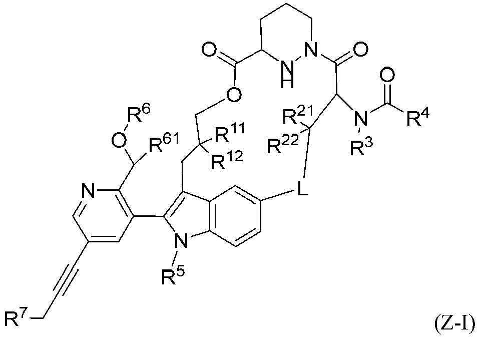 wherein in the formula,
R¹¹ and R¹² are each independently hydrogen, deuterated C₁₋₆ alkyl, or C₁₋₆ alkyl;
R²¹ and R²² are each independently hydrogen or C₁₋₆ alkyl;
Y₁ is N or CH, wherein N may be oxidized (N⁺-O⁻);
Y₂ is N or CH, wherein N may be oxidized (N⁺-O⁻);
L is C₆₋₁₀ aryl or 5- or 6-membered monocyclic heteroaryl, wherein the C₆₋₁₀ aryl is optionally substituted with hydroxy or amino;
R³ is hydrogen or C₁₋₆ alkyl;
R⁴ is C₃₋₆ cycloalkyl, 5- to 8-membered heterocyclyl, or -CR^{a}R^{b}-NR^{c}R^{d}, wherein the C₃₋₆ cycloalkyl is optionally substituted with one or more substituents selected from R⁴¹;
R⁴¹ is methyl, -C₁₋₄ alkylene-OH, or -C(O)-NR^{c}R^{d};
R⁵ is hydrogen, ethyl, -C₁₋₄ alkylene-OH, -C₁₋₄ alkylene-NHR^{e}, or -C₁₋₄ alkylene-C₁₋₆ alkoxy;
R⁶ is C₁₋₆ alkyl; R⁶¹ is C₁₋₆ alkyl;
R⁷ is 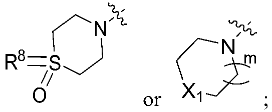
wherein, R⁸ is O or NH; X₁ is -CR⁹¹R⁹²- or -NR¹⁰-;
R⁹¹ and R⁹² are each independently hydrogen, hydroxy, cyano, -C₁₋₄ alkylene-OH, or -C(O)-NR^{c}R^{d}; R⁹¹ and R⁹² are not both hydrogen;
R¹⁰ is hydrogen or -C(O)-C₁₋₄ alkylene-OH;
m is 1 or 2;
R^{a} and R^{b} are each independently hydrogen, C₁₋₆ alkyl, or C₃₋₆ cycloalkyl;
R^{c} and R^{d} are each independently hydrogen, C₁₋₆ alkyl, -C₁₋₄ alkylene-OH, or -C(O)-C₁₋₄ alkylene-OH;
R^{e} is C₁₋₆ alkyl;
when the following conditions are satisfied, R⁷ is not 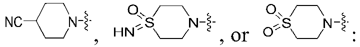 R⁴ is 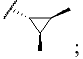 R³, R²¹, and R²² are hydrogen; R¹¹, R¹², R⁶, and R⁶¹ are methyl; and R⁵ is ethyl; when the following conditions are satisfied, R⁷ is not 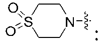 R⁴ is 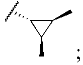 R³, R²¹, and R²² are hydrogen; R¹¹, R¹², R⁶, and R⁶¹ are methyl; and R⁵ is -CH₂CH₂OH or -CH₂CH₂NH(CH₃).

In one embodiment, in case (1) of formula (Z-III), when R⁴ is 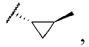 R⁷ is not 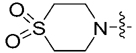 or 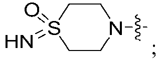 when the following conditions are satisfied, R⁷ is not 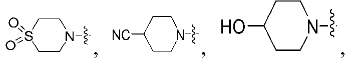 or 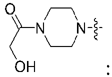 Rₘ₁ is hydrogen; Rₘ₂ is hydrogen; Rₘ₃ is hydrogen; Rₘ₄ is hydrogen; Rₘ₅ is hydrogen; 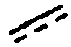 is a single bond; and R⁴ is heteroaryl, wherein the heteroaryl is optionally substituted with one or more substituents selected from R⁴¹, and R⁴¹ is SF₅, P(=O)(C₁₋₆ alkyl)₂, methyl, -C₁₋₄ alkylene-OH, or -C(O)-NR^{c}R^{d}.

In one embodiment, Rₘ₁ is hydrogen; Rₘ₂ is hydrogen; Rₘ₃ is hydrogen; Rₘ₄ is hydrogen; Rₘ₅ is hydrogen; and 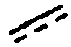 is a single bond.

In one embodiment, Rₘ₁ is attached to Rₘ₂ to jointly form -CRₘ₇Rₘ₆- or -CRₘ₇Rₘ₆-CRₘ₉Rₘ₈-; Rₘ₃ is hydrogen; Rₘ₄ is hydrogen, halogen (e.g., fluorine, chlorine, or bromine), or C₁₋₄ alkyl (e.g., methyl, ethyl, n-propyl, or isopropyl); Rₘ₅ is hydrogen, halogen (e.g., fluorine, chlorine, or bromine), or C₁₋₄ alkyl (e.g., methyl, ethyl, n-propyl, or isopropyl); Rₘ₆, Rₘ₇, Rₘ₈, and Rₘ₉ are each independently hydrogen, halogen (e.g., fluorine, chlorine, or bromine), or C₁₋₄ alkyl (e.g., methyl, ethyl, n-propyl, or isopropyl); and 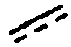 is a single bond.

In one embodiment, in case (1) of formula (Z-IIIA), when the following conditions are satisfied, R⁷ is not 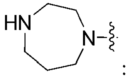 R⁴ is 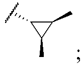 R³, R²¹, and R²² are hydrogen; Yj₃ is O-R⁶; Y₁ is N; Y₂ is CH; R¹¹, R¹², R⁶, and R⁶¹ are methyl; R⁷¹ is hydrogen, and R⁵ is ethyl; when R⁴ is 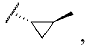 R⁷ is not 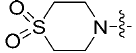 or

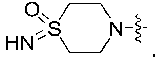

In one embodiment, R^{a} and R^{b} are each independently hydrogen, C₁₋₄ alkyl, or C₃₋₅ cycloalkyl. In one embodiment, R^{a} and R^{b} are each independently hydrogen, methyl, ethyl, n-propyl, isopropyl, cyclopropyl, cyclobutyl, or cyclopentyl. In one embodiment, R^{a} is hydrogen, and R^{b} is isopropyl, cyclobutyl, or cyclopentyl.

In one embodiment, R^{c} and R^{d} are each independently hydrogen, -C(O)H, C₁₋₄ alkyl, C₁₋₄ haloalkyl, -C₁₋₄ alkylene-OH, -C(O)-5- to 6-membered monocyclic heterocyclyl, or -C(O)-C₁₋₄ alkylene-OH, wherein the 5- to 6-membered monocyclic heterocyclyl is optionally substituted with one or more R⁴², and R⁴² is as defined in any one of the embodiments. In one embodiment, R^{c} and R^{d} are each independently hydrogen, methyl, ethyl, fluoromethyl, fluoroethyl, -C(O)H, -C(=O)CH₂OH, -C(=O)CH₂CH₂OH, -C(=O)-azetidinyl, or -C(=O)-pyrrolidinyl, wherein the azetidinyl or pyrrolidinyl is optionally substituted with 1, 2, or 3 R⁴², and R⁴² is as defined in any one of the embodiments. In one embodiment, R^{c} and R^{d} are each independently hydrogen, methyl, -C(=O)CH₂OH, or -C(=O)-pyrrolidinyl(methyl)-C(=O)OCH₃.

In one embodiment, R^{e} is C₁₋₄ alkyl. In one embodiment, R^{e} is methyl or ethyl. In one embodiment, R^{e} is methyl. In one embodiment, R^{f} is hydrogen or C₁₋₄ alkyl. In one embodiment, R^{f} is hydrogen, methyl, or ethyl. In one embodiment, R^{f} is hydrogen.

In one embodiment, R^{g} is hydrogen or C₁₋₄ alkyl. In one embodiment, R^{g} is hydrogen, methyl, or ethyl. In one embodiment, R^{g} is hydrogen.

In one embodiment, R⁴² is methyl, -NHC₁₋₄ alkyl, -N(C₁₋₄ alkyl)₂, -C(O)C₁₋₄ alkyl, or -C(O)OC₁₋₄ alkyl. In one embodiment, R⁴² is methyl, -NHCH₃, -N(CH₃)₂, -C(O)CH₃, or -C(O)OCH₃. In one embodiment, R⁴² is methyl, -NHCH₃, or -C(O)OCH₃.

In one embodiment, Y₁ is N or N⁺-O⁻; Y₂ is CH.

In one embodiment, Y₁ is N; Y₂ is CH.

In one embodiment, R¹¹ is methyl or deuterated methyl; R¹² is methyl or deuterated methyl.

In one embodiment, R¹¹ is methyl; R¹² is methyl.

In one embodiment, R²¹ and R²² are each independently hydrogen, -NR^{f}R^{g}, or -O-C₁₋₄ alkylene-NR^{f}R^{g}, wherein R^{f} and R^{g} are as defined in any one of the embodiments. In one embodiment, R^{f} is hydrogen or C₁₋₆ alkyl; R^{g} is hydrogen or C₁₋₆ alkyl.

In one embodiment, R²¹ and R²² are each independently hydrogen, -O-C₁₋₄ alkylene-NR^{f}R^{g}, wherein R^{f} is C₁₋₆ alkyl; R^{g} is C₁₋₆ alkyl.

In one embodiment, R²¹ is hydrogen; R²² is hydrogen, -NH₂, -OCH₂CH₂N(CH₃)₂, or -OCH₂CH₂NH(CH₃).

In one embodiment, R²¹ is hydrogen; R²² is hydrogen, -OCH₂CH₂N(CH₃)₂, or -OCH₂CH₂NH(CH₃).

In one embodiment, R²¹ is hydrogen; R²² is hydrogen or -OCH₂CH₂N(CH₃)₂.

In one embodiment, R²¹ is hydrogen; R²² is hydrogen.

In one embodiment, L is phenyl, thiazolyl, oxazolyl, 1,3,4-oxadiazolyl, or 1,2,4-oxadiazolyl, wherein the phenyl is optionally substituted with hydroxy or amino.

In one embodiment, L is phenyl, oxazolyl, or thiazolyl, wherein the phenyl is optionally substituted with hydroxy or amino.

In one embodiment, L is oxazolyl or thiazolyl.

In one embodiment, L is thienyl; further, L is

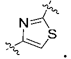

In one embodiment, L is oxazolyl; further, L is

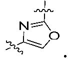

In one embodiment, the compound is represented by formula (Z-IA-1), formula (Z-IA-2), formula (Z-IA-3), or formula (Z-IA-4): 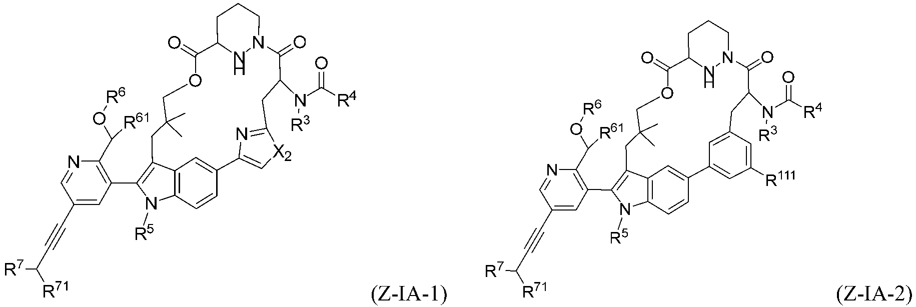 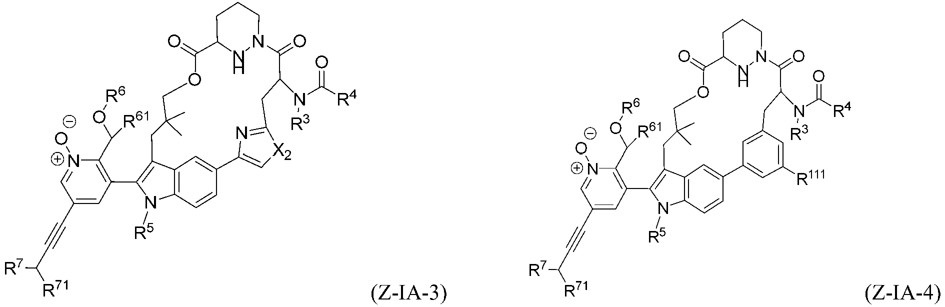
wherein in the formulas, R³, R⁴, R⁵, R⁶, R⁶¹, R⁷, and R⁷¹ are each as defined above;
X₂ is S or O; R¹¹¹ is amino or hydroxy.

In one embodiment, the compound of formula (Z-IA-1) is represented by formula (Z-IA-1a) or formula (Z-IA-1b): 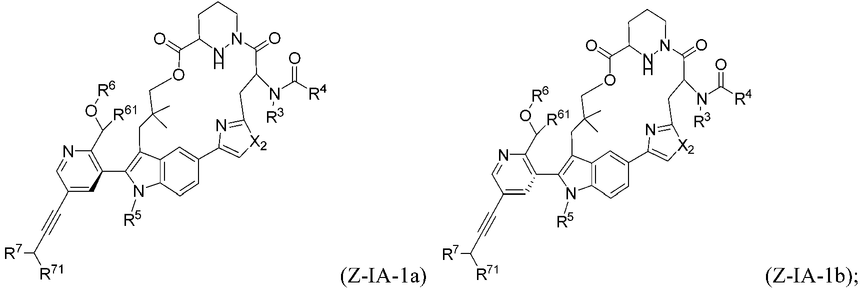 wherein in the formulas, R³, R⁴, R⁵, R⁶, R⁶¹, R⁷, and R⁷¹ are each as defined above; X₂ is S or O.

In one embodiment, the compound of formula (Z-IA-1) is represented by formula (Z-IA-1a1) or formula (Z-IA-1b1): 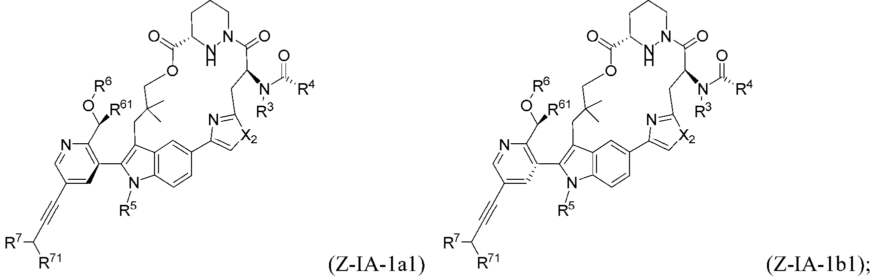 wherein in the formulas, R³, R⁴, R⁵, R⁶, R⁶¹, R⁷, and R⁷¹ are each as defined above; X₂ is S or O.

In one embodiment, the compound of formula (Z-IA-2) is represented by formula (Z-IA-2a) or formula (Z-IA-2b): 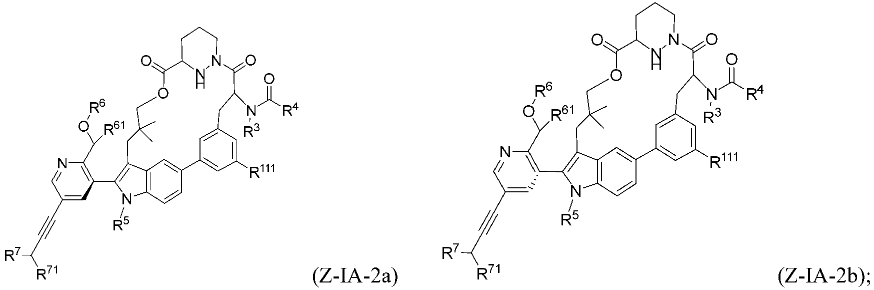 wherein in the formulas, R³, R⁴, R⁵, R⁶, R⁶¹, R⁷, and R⁷¹ are each as defined above; R¹¹¹ is amino or hydroxy.

In one embodiment, the compound of formula (Z-IA-2) is represented by formula (Z-IA-2a1) or formula (Z-IA-2b1): 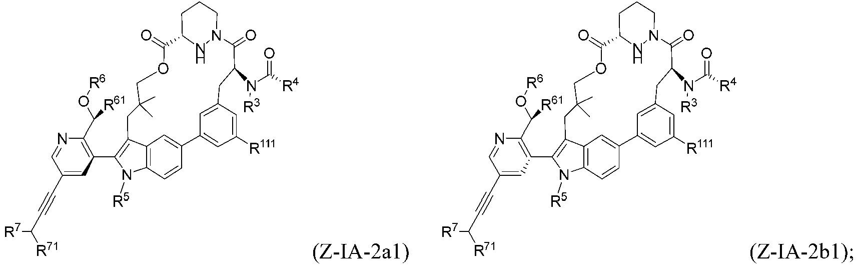 wherein in the formulas, R³, R⁴, R⁵, R⁶, R⁶¹, R⁷, and R⁷¹ are each as defined above; R¹¹¹ is amino or hydroxy.

In one embodiment, the compound of formula (Z-IA-3) is represented by formula (Z-IA-3a) or formula (Z-IA-3b): 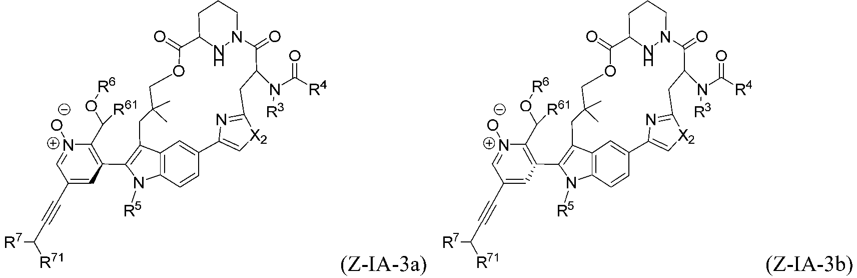 wherein in the formulas, R³, R⁴, R⁵, R⁶, R⁶¹, R⁷, and R⁷¹ are each as defined above; X₂ is S or O.

In one embodiment, the compound of formula (Z-IA-3) is represented by formula (Z-IA-3a1) or formula (Z-IA-3b1): 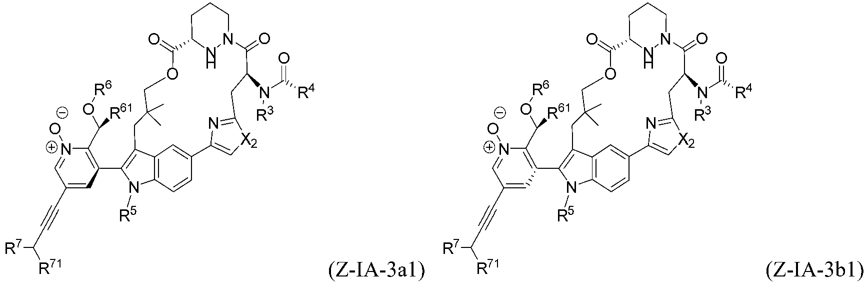 wherein in the formulas, R³, R⁴, R⁵, R⁶, R⁶¹, R⁷, and R⁷¹ are each as defined above; X₂ is S or O.

In one embodiment, the compound of formula (Z-IA-4) is represented by formula (Z-IA-4a) or formula (Z-IA-4b): 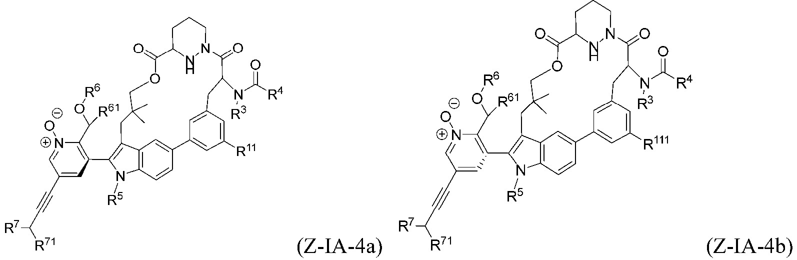 wherein in the formulas, R³, R⁴, R⁵, R⁶, R⁶¹, R⁷, and R⁷¹ are each as defined above; R¹¹¹ is amino or hydroxy.

In one embodiment, the compound of formula (Z-IA-4) is represented by formula (Z-IA-4a1) or formula (Z-IA-4b1): 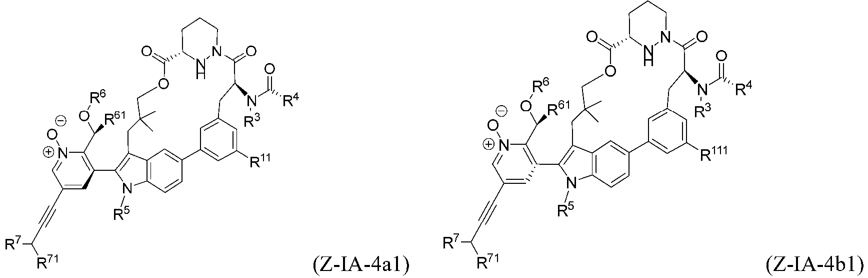 wherein in the formulas, R³, R⁴, R⁵, R⁶, R⁶¹, R⁷, and R⁷¹ are each as defined above; R¹¹¹ is amino or hydroxy.

In one embodiment, the compound is represented by formula (Z-I-1), formula (Z-I-2), formula (Z-I-3), or formula (Z-I-4): 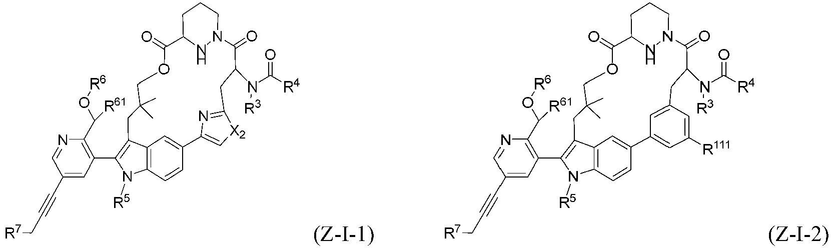 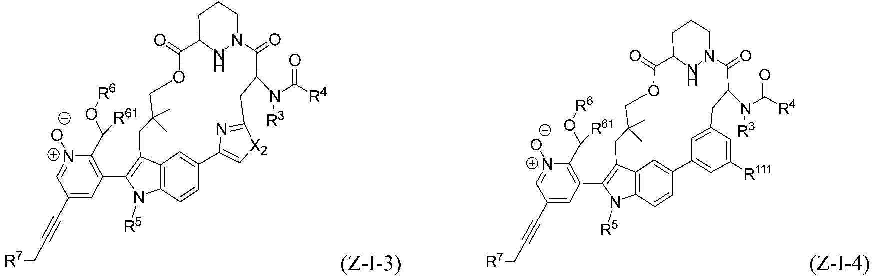
wherein in the formulas, R³, R⁴, R⁵, R⁶, R⁶¹, and R⁷ are each as defined above;
X₂ is S or O; R¹¹¹ is amino or hydroxy.

In one embodiment, R³ is hydrogen or methyl, further hydrogen.

In one embodiment, R⁴ is C₃₋₆ cycloalkyl, wherein the C₃₋₆ cycloalkyl is optionally substituted with one or more substituents selected from R⁴¹; R⁴¹ is selected from methyl, -C₁₋₄ alkylene-OH, and -C(O)-NH(C₁₋₄ alkylene-OH); further, R⁴¹ is selected from methyl, -CH₂OH, and -C(O)-NH(CH₂CH₂OH).

In one embodiment, R⁴ is 5- to 8-membered oxacyclyl, further 5- to 8-membered oxa-fused heterocyclyl.

In one embodiment, R⁴ is

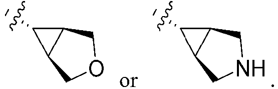

In one embodiment, R⁴ is -CR^{a}R^{b}-NR^{c}R^{d}, wherein R^{a} and R^{b} are each independently hydrogen, C₁₋₆ alkyl, or C₃₋₆ cycloalkyl; R^{c} and R^{d} are each independently hydrogen, C₁₋₆ alkyl, or -C(O)-C₁₋₄ alkylene-OH.

In one embodiment, R⁴ is -CR^{a}R^{b}-NR^{c}R^{d}, wherein R^{a} and R^{d} are each independently hydrogen or C₁₋₆ alkyl; R^{c} and R^{d} are each independently hydrogen, C₁₋₆ alkyl, or -C(O)-pyrrolidinyl, wherein the pyrrolidinyl is optionally substituted with one or more substituents selected from R⁴²; R⁴² is methyl or -C(O)OCH₃.

In one embodiment, R⁴ is selected from the group consisting of:

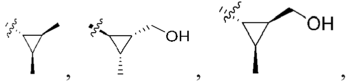

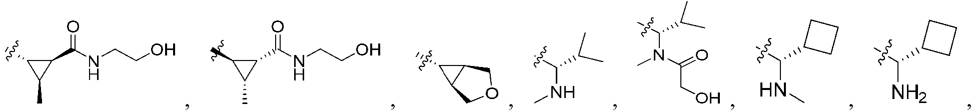

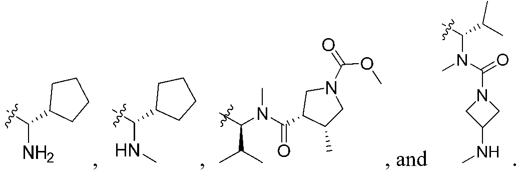

In one embodiment, 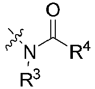 is selected from the group consisting of: 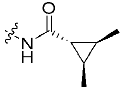 (preferably

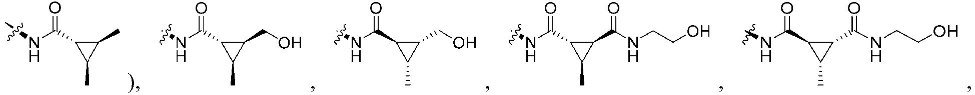

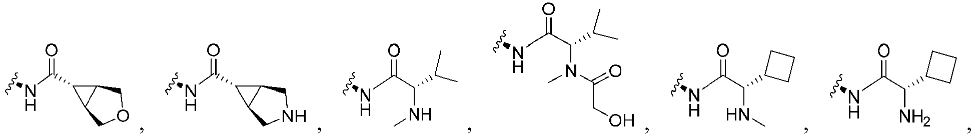

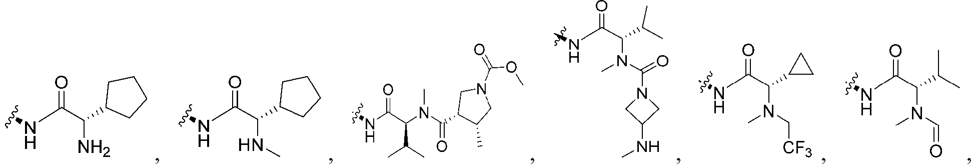

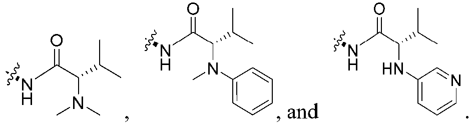

In one embodiment, 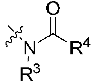 is selected from the group consisting of: 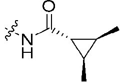 (preferably

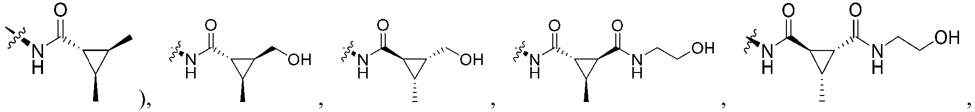

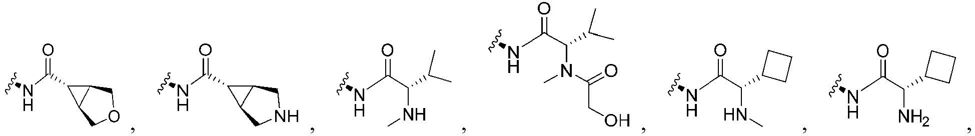

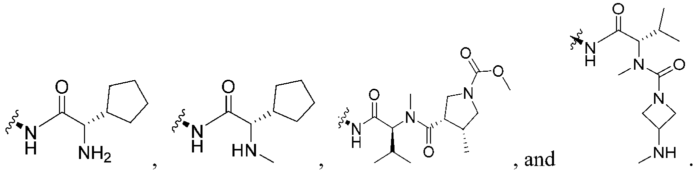

In one embodiment, 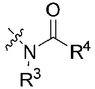 is selected from the group consisting of: 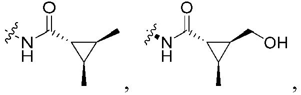 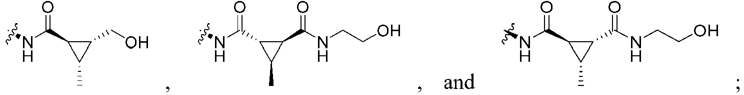 further, 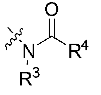 is

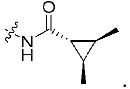

In one embodiment, 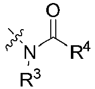 is selected from the group consisting of:

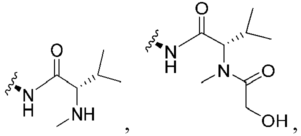

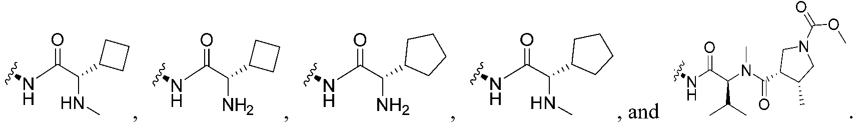

In one embodiment, 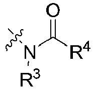 is 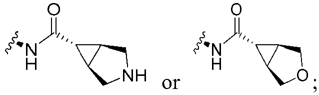 further, 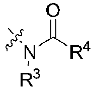 is

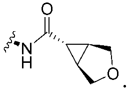

In one embodiment, R⁵ is hydrogen, C₁₋₄ alkyl, C₃₋₆ cycloalkyl, -C₁₋₄ alkylene-OH, -C₁₋₄ alkylene-NHC₁₋₄ alkyl, or -C₁₋₄ alkylene-C₁₋₄ alkoxy.

In one embodiment, R⁵ is ethyl, cyclopropyl, -(CH₂)₂OH, -(CH₂)₃OH, -(CH₂)₂-NH(CH₃), or -(CH₂)₂-OCH₃.

In one embodiment, R⁵ is hydrogen, ethyl, -C₁₋₄ alkylene-OH, -C₁₋₄ alkylene-NHC₁₋₄ alkyl, or -C₁₋₄ alkylene-C₁₋₄ alkoxy.

In one embodiment, R⁵ is ethyl, -(CH₂)₂OH, -(CH₂)₃OH, -(CH₂)₂-NH(CH₃), or -(CH₂)₂-OCH₃.

In one embodiment, R⁵ is ethyl, -(CH₂)₂OH, -(CH₂)₃OH, or -(CH₂)₂-OCH₃.

In one embodiment, R⁵ is ethyl, -(CH₂)₃OH, or -(CH₂)₂-OCH₃.

In one embodiment, R⁵ is ethyl.

In one embodiment, R⁵ is ethyl, isopropyl, -(CH₂)₃OH, or -(CH₂)₂-OCH₃.

In one embodiment, R⁶ is hydrogen or C₁₋₄ alkyl.

In one embodiment, R⁶ is hydrogen.

In one embodiment, R⁶ is hydrogen, methyl, ethyl, n-propyl, or isopropyl.

In one embodiment, R⁶¹ is C₁₋₄ alkyl.

In one embodiment, R⁶¹ is methyl, ethyl, n-propyl, or isopropyl.

In one embodiment, R⁶ is methyl; R⁶¹ is methyl.

In one embodiment, R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}, wherein R^{c} and R^{d} are each independently hydrogen or -C₁₋₄ alkylene-OH; R⁹¹ and R⁹² are not both hydrogen. In one embodiment, R⁹¹ and R⁹² are each independently hydrogen, hydroxy, cyano, amino, -C₁₋₄ alkylene-OH, -C₁₋₄ alkylene-NH₂, or -C(O)-NHC₁₋₄ alkyl-OH, wherein R⁹¹ and R⁹² are not both hydrogen. In one embodiment, R⁹¹ and R⁹² are each independently hydrogen, hydroxy, cyano, amino, -CH₂OH, -CH₂NH₂, or -C(O)NHCH₂CH₂OH, wherein R⁹¹ and R⁹² are not both hydrogen. In one embodiment, R⁹¹ is cyano.

In one embodiment, R¹⁰ is hydrogen, -C(O)-C₁₋₄ alkylene-NH₂, -C(O)-C₁₋₄ alkylene-NHC₁₋₄ alkyl, -C(O)-C₁₋₄ alkylene-N(C₁₋₄ alkyl)₂, or -C(O)-C₁₋₄ alkylene-OH. In one embodiment, R¹⁰ is hydrogen, -C(O)CH₂NH₂, - C(O)CH₂CH₂NH₂, -C(O)CH₂NHCH₃, -C(O)CH₂N(CH₃)₂, -C(O)CH₂CH₂NHCH₃, -C(O)CH₂OH, or - C(O)CH₂CH₂OH. In one embodiment, R¹⁰ is -C(O)CH₂OH, -C(O)CH₂NH₂, or -C(O)CH₂NHCH₃-

In one embodiment, R¹⁰ is C₁₋₄ alkyl. In one embodiment, R¹⁰ is methyl, ethyl, or isopropyl. In one embodiment, R¹⁰ is methyl.

**In** one embodiment, R⁷ is 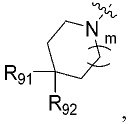 wherein R⁹¹ and R⁹² are each independently hydrogen, hydroxy, cyano, -NH₂, -CH₂NH₂, -CH₂NH(CH₃), -CH₂OH, or -C(O)-NH(CH₂CH₂OH); R⁹¹ and R⁹² are not both hydrogen; m is 1 or 2.

In one embodiment, R⁷¹ is hydrogen; R⁷ is selected from 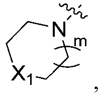 wherein m is 1; X₁ is -CR⁹¹R⁹²-, wherein R⁹¹ is cyano; R⁹² is as defined above. In one embodiment, R⁹² is hydrogen, -NR^{c}R^{d}, -C₁₋₄ alkylene-NR^{c}R^{d}, or - C₁₋₄ alkylene-OH, wherein R^{c} and R^{d} are each independently hydrogen or C₁₋₆ alkyl. In one embodiment, R⁹² is hydrogen, -C₁₋₄ alkylene-NR^{c}R^{d}, or -C₁₋₄ alkylene-OH, wherein R^{c} and R^{d} are each independently hydrogen. In one embodiment, R⁹² is hydrogen, -NH₂, -CH₂NH₂, -CH₂NH(CH₃), -CH₂OH, or -C(O)-NH(CH₂CH₂OH).

In one embodiment, R⁷ is 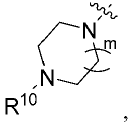 wherein R¹⁰ is hydrogen or -C(O)CH₂OH, and m is 1 or 2.

In one embodiment, R⁷ is selected from the group consisting of:

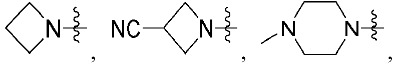

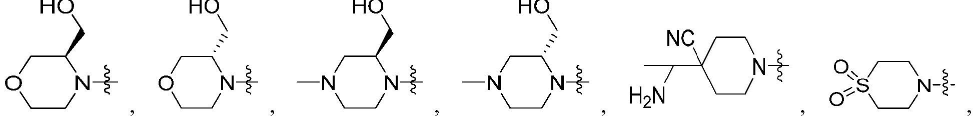

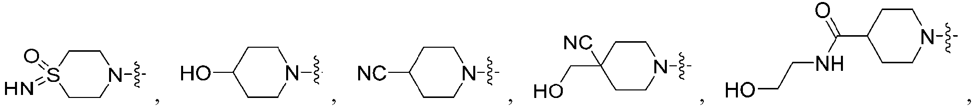

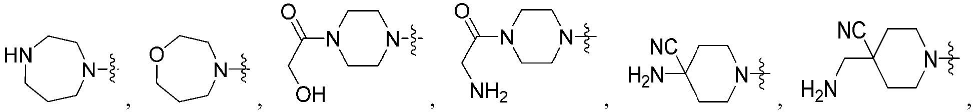

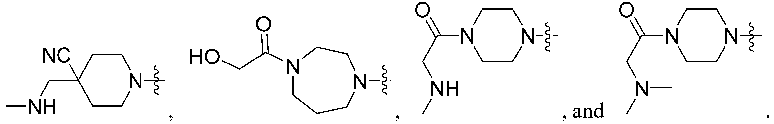

In one embodiment, R⁷ is selected from the group consisting of:

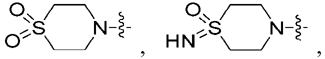

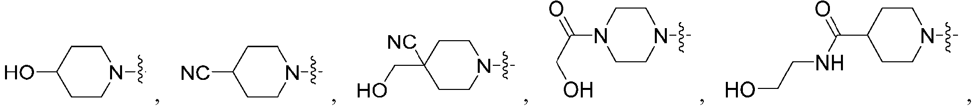

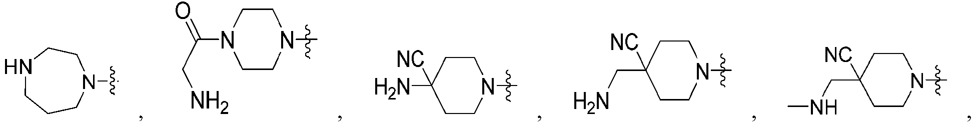

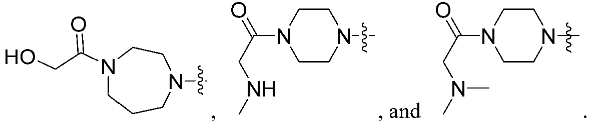

In one embodiment, R⁷¹ is hydrogen, -C₁₋₄ alkylene-OH, or -C₁₋₄ alkylene-NH-R⁶, wherein R⁶ is hydrogen.

In one embodiment, R⁷¹ is hydrogen, -CH₂OH, or -CH₂NH₂.

In one embodiment, R⁷¹ is hydrogen or -C₁₋₄ alkylene-OH.

In one embodiment, R⁷¹ is hydrogen or -CH₂OH.

In one embodiment, R⁷¹ is -C₁₋₄ alkylene-OH, further -(CH₂)₃OH, -(CH₂)₂OH, or -CH₂OH.

In one embodiment, R⁷¹ is hydrogen.

In one embodiment, R⁷ is selected from the group consisting of: 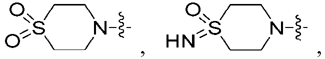 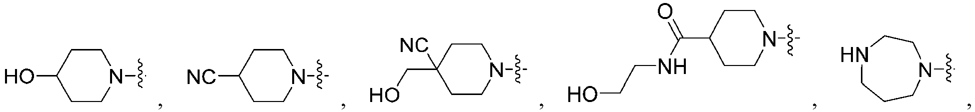 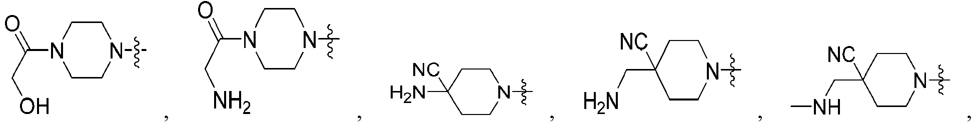 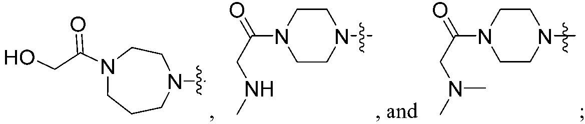 R⁷¹ is hydrogen or -CH₂OH.

In one embodiment, R⁷ is selected from the group consisting of: 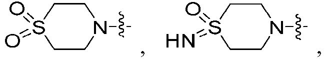 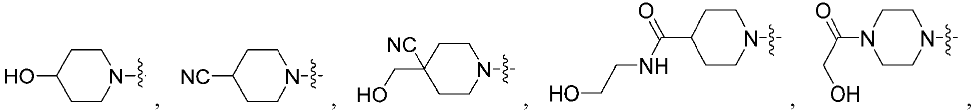 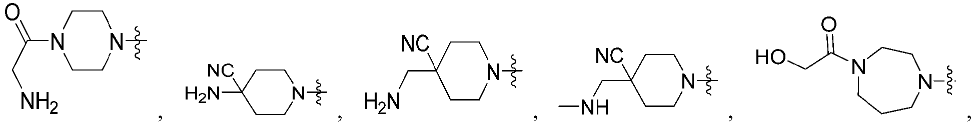 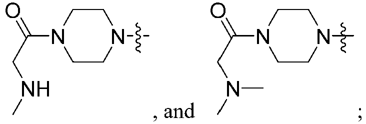 R⁷¹ is hydrogen or -CH₂OH.

In one embodiment, R⁷ is selected from the group consisting of: 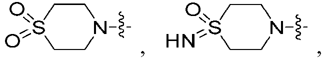 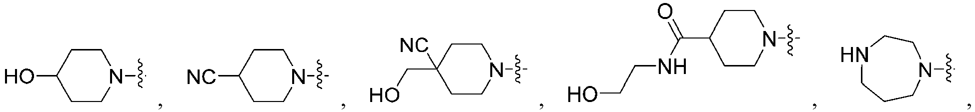 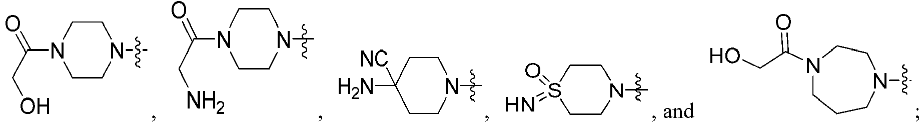 R⁷¹ is hydrogen or -CH₂OH.

In one embodiment, R⁷¹ is hydrogen, and R⁷ is selected from the group consisting of:

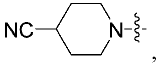

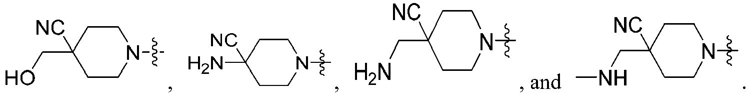

In one embodiment, R⁷¹ is hydrogen, and R⁷ is selected from the group consisting of:

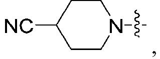

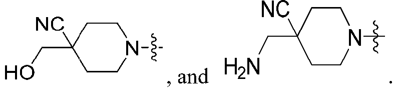

In one embodiment, the compound is selected from Table A-1.

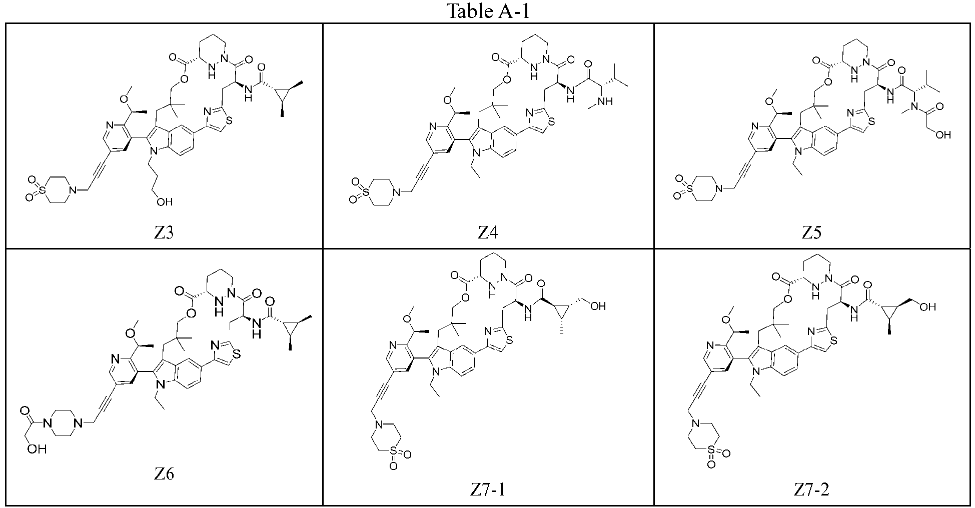

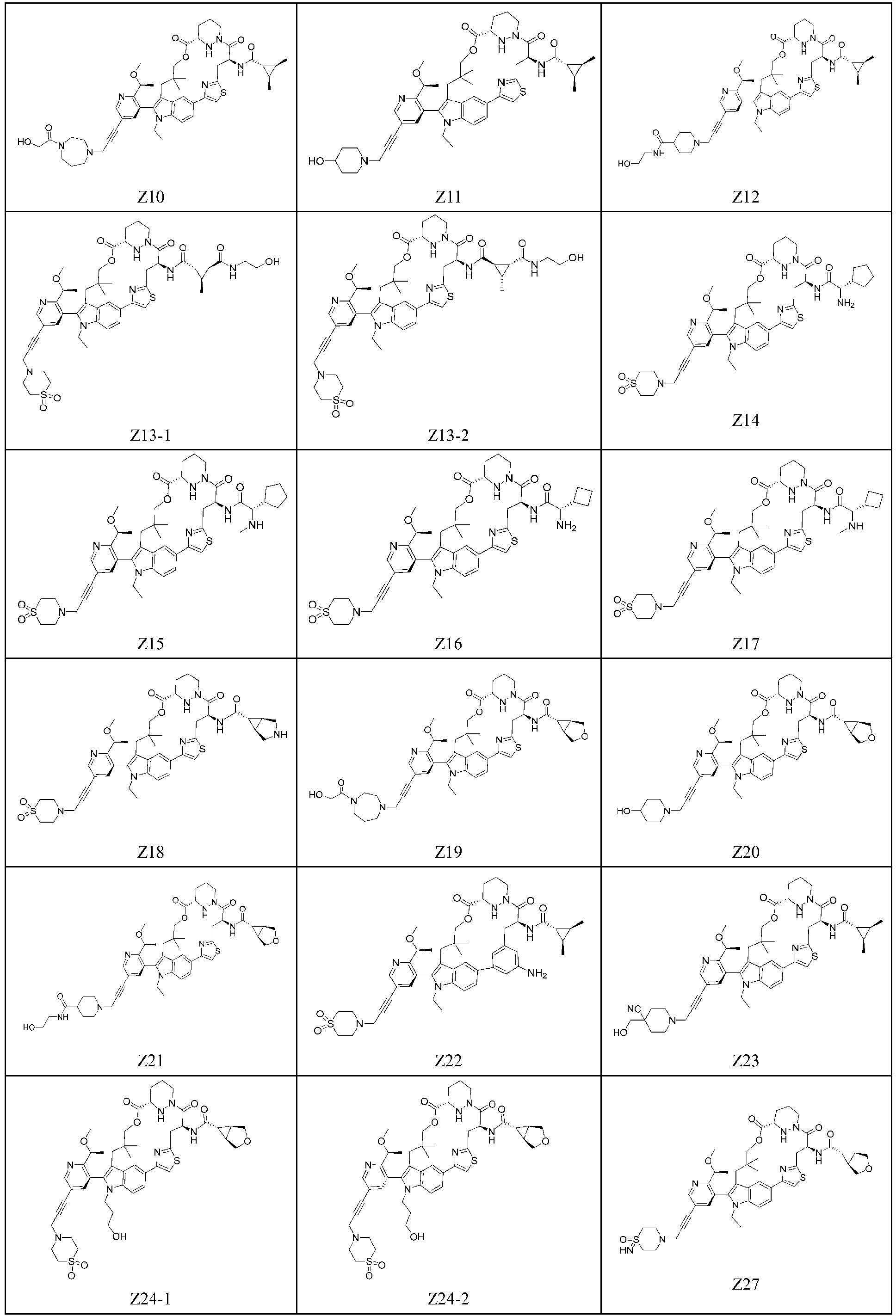

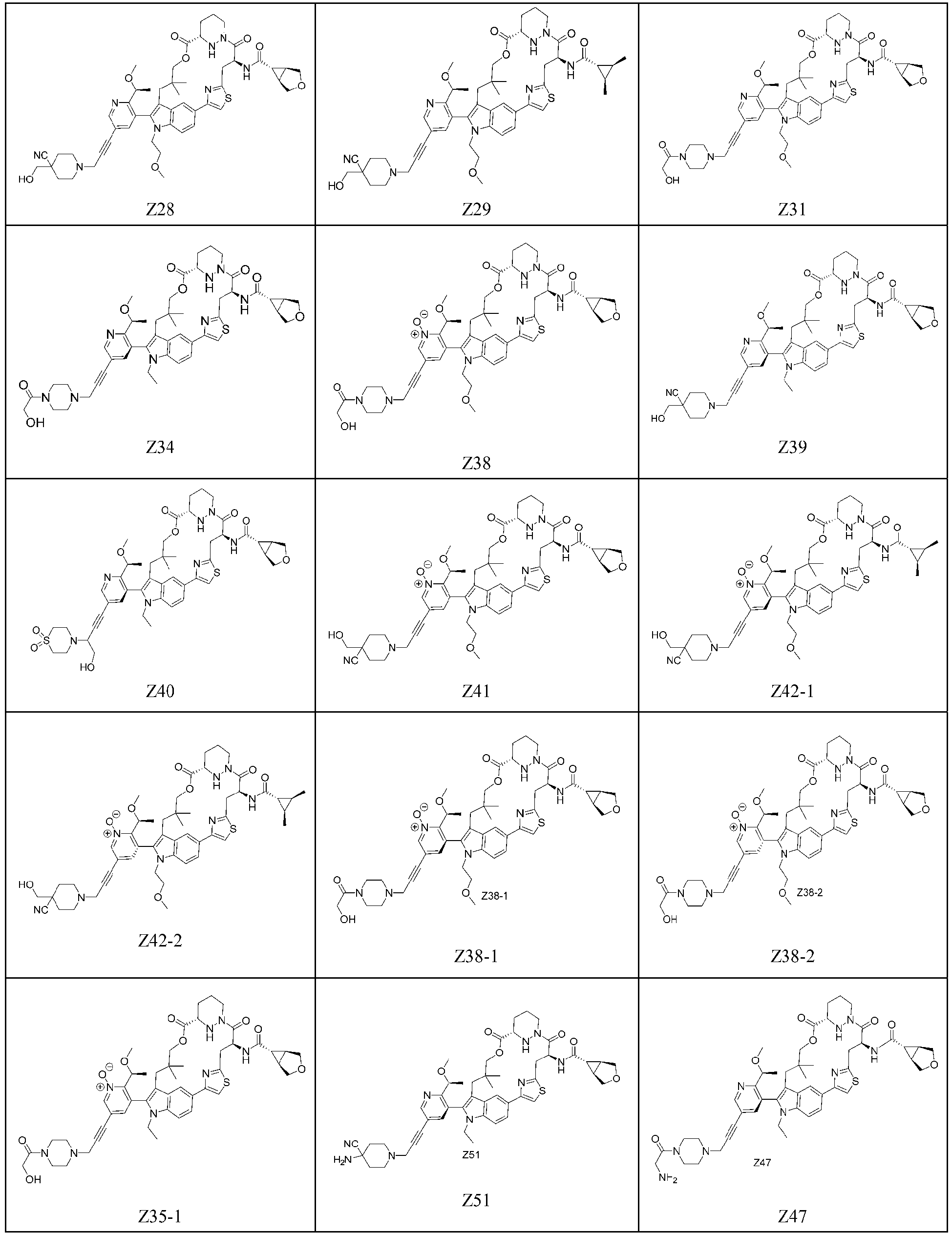

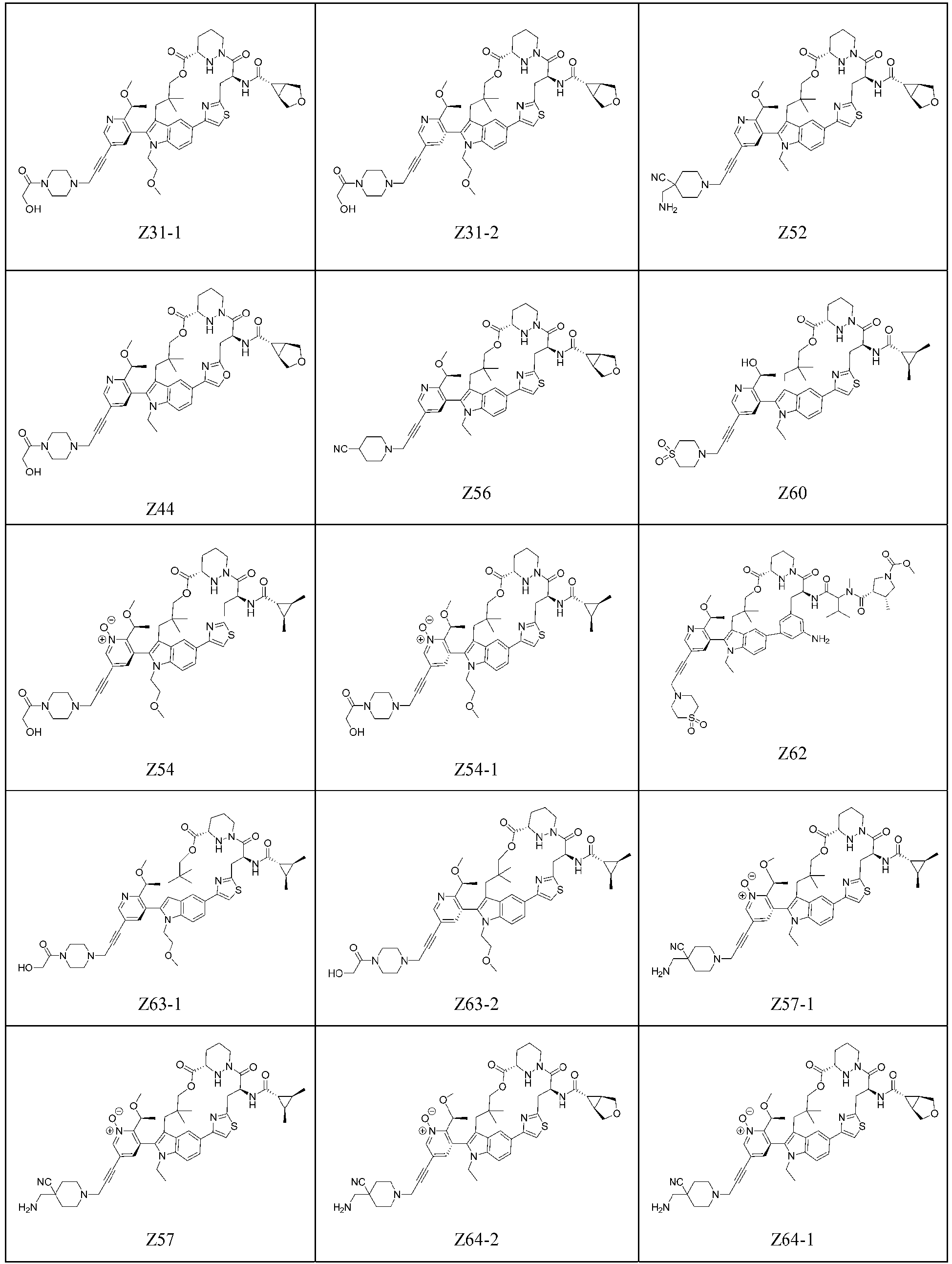

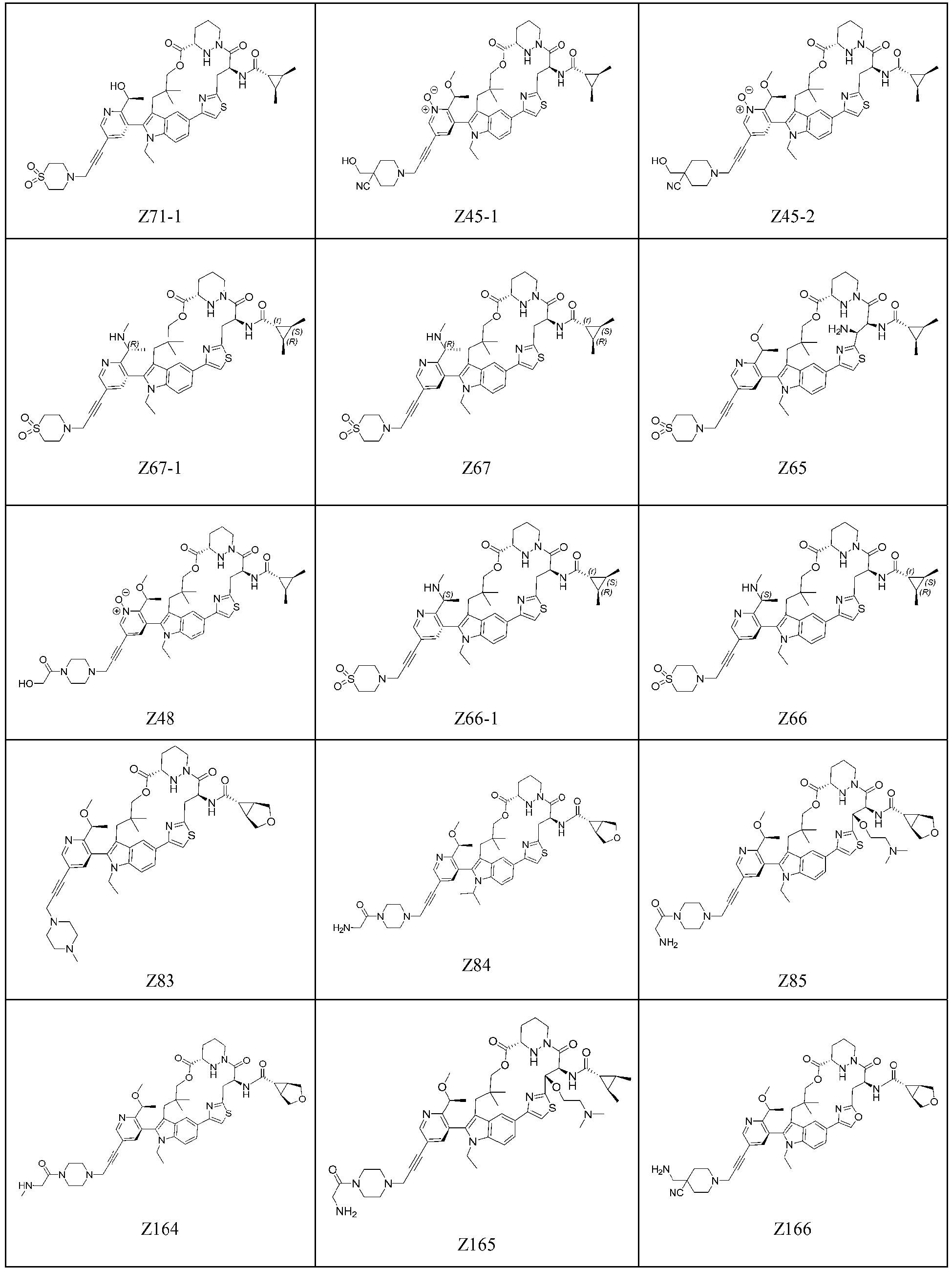

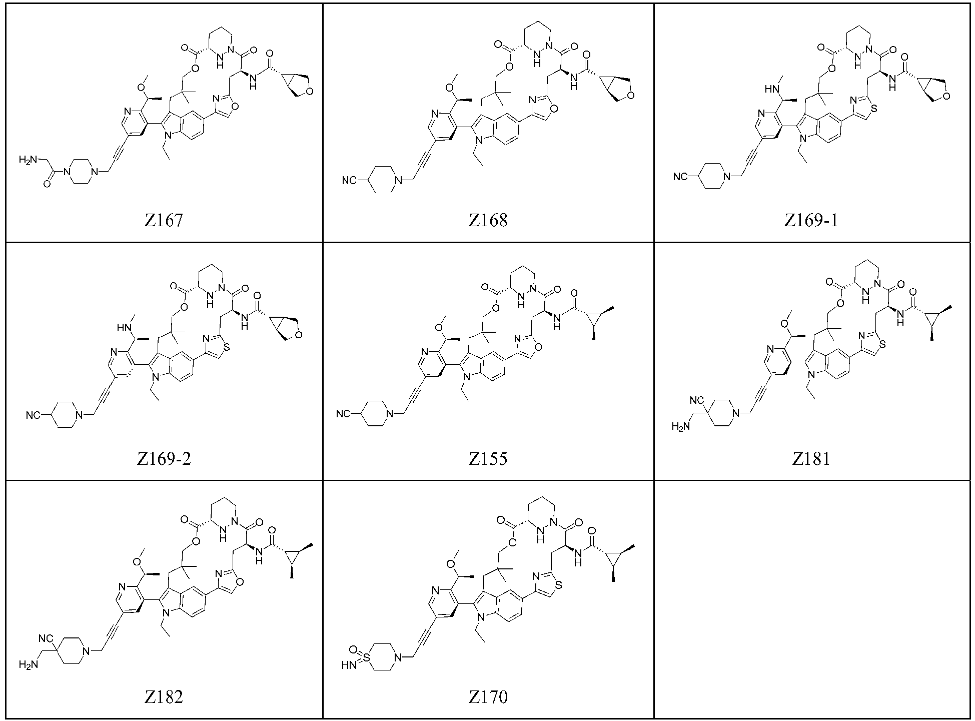

In one embodiment, the compound is selected from Table A-2.

In one embodiment, the compound is represented by formula (Z-IIA-1) or formula (Z-IIA-2): 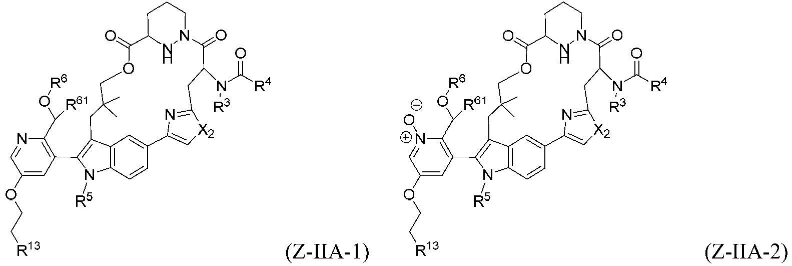
wherein in the formulas, R³, R⁴, R⁵, R⁶, R⁶¹, and R¹³ are each as defined above;
X₂ is S or O.

In one embodiment, the compound of formula (Z-IIA-1) is represented by formula (Z-IIA-1a) or formula (Z-IIA-1b):

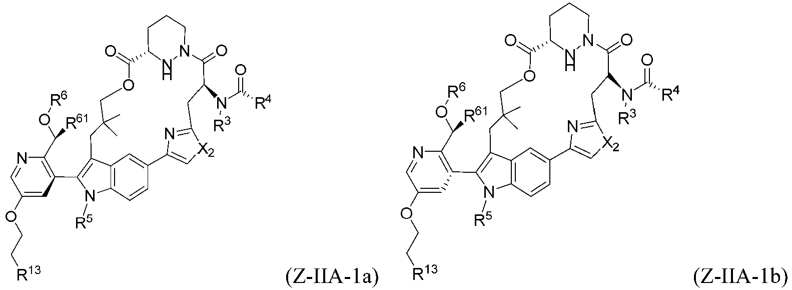

In one embodiment, the compound of formula (Z-IIA-2) is represented by formula (Z-IIA-2a) or formula (Z-IIA-2b): 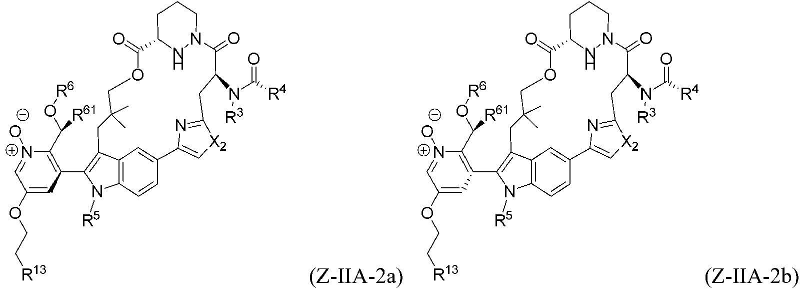
wherein in the formulas, R³, R⁴, R⁵, R⁶, R⁶¹, and R¹³ are each as defined above;
X₂ is S or O.

In one embodiment, 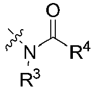 is selected from the group consisting of: 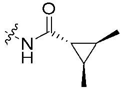 (preferably

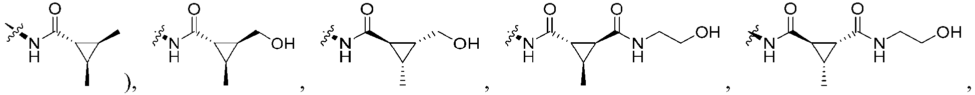

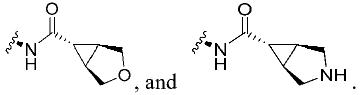

In one embodiment, 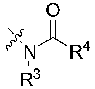 is selected from the group consisting of: 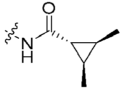 (preferably

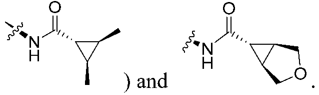

In one embodiment, R¹³ is 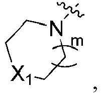 wherein m is 1; X₁ is -CR⁹¹R⁹²-, wherein R⁹¹ and R⁹² are each independently cyano or -C₁₋₄ alkylene-NR^{c}R^{d}; R^{c} and R^{d} are each independently hydrogen or C₁₋₆ alkyl; R⁹¹ and R⁹² are not both hydrogen. In one embodiment, R⁹¹ is cyano; R⁹² is -C₁₋₄ alkylene-NH₂ or -C₁₋₄ alkylene-NH-C₁₋₄ alkyl. In one embodiment, R⁹¹ is cyano; R⁹² is -CH₂NH₂ or -CH₂NHCH₃.

In one embodiment, R¹³ is oxo-6-membered heterocyclyl-L₁-L₂-R¹⁴, wherein L₁ is -C₁₋₄ alkylene-O-; L₂ is -C₁₋₄ alkylene-NH-; R¹⁴ is C₁₋₄ heteroalkyl. In one embodiment, the 6-membered heterocyclyl is piperidinyl or piperazinyl. In one embodiment, L₁ is -CH₂O-, -CH₂CH₂O-, or -CH₂CH(CH₃)O-. In one embodiment, L₂ is - CH₂NH-, -CH₂CH₂NH-, or -CH₂CH(CH₃)NH-. In one embodiment, R¹⁴ is C₁₋₄ oxa-alkyl; preferably, R¹⁴ is - CH₂OCH₃, -CH₂CH₂OCH₃, or -CH₂CH(CH₃)OCH₃.

In one embodiment, R¹³ is selected from the group consisting of: 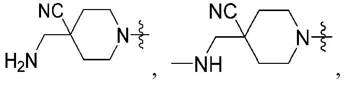 , and

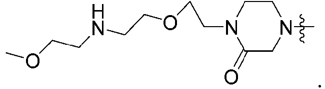

In one embodiment, the compound is selected from Table B-1.

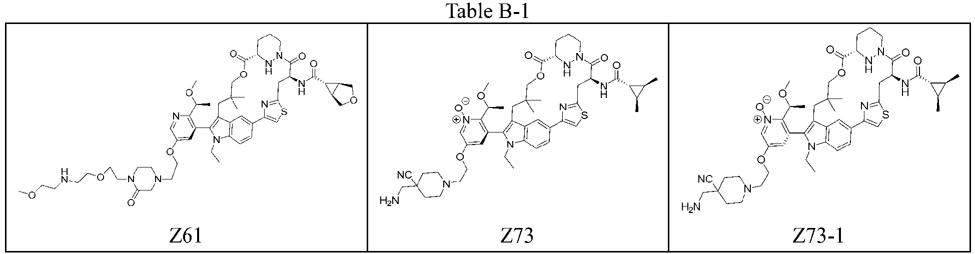

In one embodiment, the compound is selected from Table B-2.

### Composition

In a fourth aspect, the present disclosure provides a pharmaceutical composition comprising the antibody-drug conjugate according to the first aspect of the present disclosure, and a pharmaceutically acceptable carrier.

In a fifth aspect, the present disclosure provides a pharmaceutical composition comprising the compound, or the stable deuterated derivative thereof, the stereoisomer thereof, the pharmaceutically acceptable salt thereof, the solvate thereof, or the prodrug thereof according to the third aspect described above, and a pharmaceutically acceptable carrier.

### Use

In a sixth aspect, the present disclosure provides the aforementioned antibody-drug conjugate as a medicament. In a seventh aspect, the present disclosure provides the aforementioned compound as a medicament.

In an eighth aspect, the present disclosure provides use of the antibody-drug conjugate according to the first aspect of the present disclosure or the pharmaceutical composition according to the fourth aspect of the present disclosure in the preparation of an RAS protein activity inhibitor. In another aspect, the present disclosure provides a method for inhibiting RAS protein activity in a cell, and the method comprises a step of: contacting the cell with the antibody-drug conjugate according to the first aspect of the present disclosure; or contacting the cell with the pharmaceutical composition according to the fourth aspect of the present disclosure. The cell may be *in vivo* or *in vitro.*

In a ninth aspect, the present disclosure provides use of the antibody-drug conjugate according to the first aspect of the present disclosure or the pharmaceutical composition according to the fourth aspect of the present disclosure in the preparation of a medicament for preventing and/or treating a disease or disorder associated with RAS protein activity. In another aspect, the present disclosure provides the antibody-drug conjugate according to the first aspect of the present disclosure or the pharmaceutical composition according to the fourth aspect of the present disclosure for use in preventing and/or treating a disease or disorder associated with RAS protein activity. In another aspect, the present disclosure provides a method for preventing and/or treating a disease or disorder associated with RAS protein activity, and the method comprises a step of: administering to a subject in need a therapeutically effective amount of the antibody-drug conjugate according to the first aspect of the present disclosure; or administering to a subject in need a therapeutically effective amount of the pharmaceutical composition according to the fourth aspect of the present disclosure.

In a tenth aspect, the present disclosure provides use of the compound, or the stable deuterated derivative thereof, the stereoisomer thereof, the pharmaceutically acceptable salt thereof, the solvate thereof, or the prodrug thereof according to the third aspect of the present disclosure, or the pharmaceutical composition according to the fifth aspect described above in the preparation of an RAS protein activity inhibitor. In another aspect, the present disclosure provides a method for inhibiting RAS protein activity in a cell, and the method comprises a step of: contacting the cell with the compound, or the stable deuterated derivative thereof, the stereoisomer thereof, the pharmaceutically acceptable salt thereof, the solvate thereof, or the prodrug thereof according to the third aspect of the present disclosure; or contacting the cell with the pharmaceutical composition according to the fifth aspect of the present disclosure. The cell may be *in vivo* or *in vitro.*

In an eleventh aspect, the present disclosure further provides use of the compound, or the stable deuterated derivative thereof, the stereoisomer thereof, the pharmaceutically acceptable salt thereof, the solvate thereof, or the prodrug thereof according to the third aspect of the present disclosure, or the pharmaceutical composition according to the fifth aspect of the present disclosure in the preparation of a medicament for preventing and/or treating a disease or disorder associated with RAS protein activity. In another aspect, the present disclosure provides the compound, or the stable deuterated derivative thereof, the stereoisomer thereof, the pharmaceutically acceptable salt thereof, the solvate thereof, or the prodrug thereof according to the third aspect of the present disclosure, or the pharmaceutical composition according to the fifth aspect of the present disclosure for use in preventing and/or treating a disease or disorder associated with RAS protein activity. In another aspect, the present disclosure provides a method for preventing and/or treating a disease or disorder associated with RAS protein activity, and the method comprises a step of: administering to a subject in need a therapeutically effective amount of the compound, or the stable deuterated derivative thereof, the stereoisomer thereof, the pharmaceutically acceptable salt thereof, the solvate thereof, or the prodrug thereof according to the third aspect of the present disclosure; or administering to a subject in need a therapeutically effective amount of the pharmaceutical composition according to the fifth aspect of the present disclosure.

In another aspect, the present disclosure provides a method for treating a cancer in a subject in need thereof, and the method comprises:
(a) determining that the cancer is associated with RAS protein activity; and
(b) administering to the subject a therapeutically effective amount of the antibody-drug conjugate according to the first aspect of the present disclosure; or administering to the subject a therapeutically effective amount of the pharmaceutical composition according to the fourth aspect of the present disclosure.

In another aspect, the present disclosure provides a method for treating a cancer in a subject in need thereof, and the method comprises:
(a) determining that the cancer is associated with RAS protein activity; and
(b) administering to the subject a therapeutically effective amount of the compound, or the stable deuterated derivative thereof, the stereoisomer thereof, the pharmaceutically acceptable salt thereof, the solvate thereof, or the prodrug thereof according to the third aspect of the present disclosure; or administering to the subject a therapeutically effective amount of the pharmaceutical composition according to the fifth aspect of the present disclosure.

In one embodiment, the disease or disorder associated with RAS protein activity is a cancer.

In one embodiment, the RAS includes HRAS, KRAS, and NRAS.

In one embodiment, the cancer comprises wild-type RAS and an RAS mutation.

In one embodiment, the RAS mutation is KRAS G12C, KRAS G12D, KRAS G12V, KRAS G12S, KRAS G13C, KRAS G13D, KRAS Q61L, KRAS Q61H, NRAS G12C, NRAS Q61K, NRAS Q61R, or other RAS mutations. In one embodiment, the cancer is pancreatic cancer, colorectal cancer, lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.

In one embodiment, the lung cancer is non-small cell lung cancer.

In the embodiments of the present disclosure, the pharmaceutical composition may be administered in any of the following ways: oral administration, spray inhalation, rectal administration, nasal administration, buccal administration, topical administration, parenteral administration (e.g., subcutaneous, intravenous, intramuscular, intraperitoneal, intrathecal, intraventricular, intrasternal, and intracranial injection or infusion), or administration by means of an implantable reservoir. Oral, intraperitoneal, or intravenous administration is preferred. When being administered orally, the compounds of the present disclosure may be prepared into any orally acceptable formulation forms, including but not limited to tablets, capsules, aqueous solutions, or aqueous suspensions. Carriers used for tablets generally include lactose and corn starch, and additionally, a lubricant such as magnesium stearate may be added. Diluents used for capsule formulations generally include lactose and dried corn starch. Aqueous suspension formulations are generally used by mixing the active ingredient with suitable emulsifying and suspending agents. If needed, a certain amount of sweetening, flavoring, or coloring agents may be also added to the above oral formulation forms. When being administered topically, especially for treating affected areas or organs that are easily accessible for topical external application, such as eyes, skin, or lower intestinal neurological diseases, the compounds of the present disclosure may be formulated into different formulation forms for topical administration according to different affected areas or organs. When being administered topically to the eye, the compounds of the present disclosure may be formulated into a formulation form of a micronized suspension or solution, and the carrier used is isotonic sterile saline with a certain pH, with or without the addition of a preservative such as chlorinated benzyl alkanolate. For ophthalmic use, the compound may also be formulated into ointments, such as vaseline. When being administered topically to the skin, the compounds of the present disclosure may be formulated into a formulation form of a suitable ointment, lotion, or cream, in which the active ingredient is suspended or dissolved in one or more carriers. Carriers that can be used in the ointment formulations include, but are not limited to: mineral oil, liquid vaseline, white vaseline, propylene glycol, polyethylene oxide, polypropylene oxide, emulsifying wax, and water; carriers that can be used in the lotions or creams include, but are not limited to: mineral oil, sorbitan monostearate, Tween 60, cetyl ester wax, hexadecene aryl alcohol, 2-octyldodecanol, benzyl alcohol, and water. The compounds of the present disclosure may also be administered in the form of a sterile injectable formulation, including a sterile injectable aqueous or oil suspension or a sterile injectable solution. The carriers and solvents that may be used include water, Ringer's solution, and isotonic sodium chloride solution. In addition, sterilized non-volatile oils may also be used as solvents or suspending media, such as monoglycerides or diglycerides.

In one embodiment, the administration is accomplished by a route selected from parenteral, intraperitoneal, intradermal, intracardiac, intraventricular, intracranial, intracerebrospinal, intracavitary, intrasynovial, intrathecal, intramuscular injection, intravitreal injection, intravenous injection, intraarterial injection, oral, buccal, sublingual, transdermal, topical, intratracheal, rectal, subcutaneous, and topical administrations.

Herein, the use or method further comprises administering an additional anti-cancer therapy. In one embodiment, the additional anti-cancer therapy is an HER2 inhibitor, an EGFR inhibitor, a second RAS inhibitor, an SHP2 inhibitor, an SOS1 inhibitor, an RAF inhibitor, an MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, or a combination thereof.

In a twelfth aspect, the present disclosure provides use of the compound according to the second aspect of the present disclosure in the preparation of the antibody-drug conjugate according to the first aspect of the present disclosure.

### Definitions of Terms

In the present disclosure, unless otherwise defined, the scientific and technical terms used herein have the meanings generally understood by those skilled in the art. In addition, the laboratory operational procedures involved herein are all conventional procedures widely used in the corresponding fields. Meanwhile, in order to better understand the present disclosure, the definitions and explanations of the related terms are provided below. When the name of a compound used herein is not consistent with the chemical structural formula, the chemical structural formula shall prevail.

As used herein, when the variable is a bond, it means that the groups on both sides of the variable are directly linked, e.g., A-L-B; when L is a bond, A-L-B is A-B.

As used herein, "optionally substituted with..." or "optionally substituted" means that the group may be unsubstituted or may be substituted with a substituent. For example, "C₁₋₆ alkyl is optionally substituted with halogen" means that the C₁₋₆ alkyl may be unsubstituted or may be substituted with halogen to give haloalkyl. "Substituted or unsubstituted" means that the group may be unsubstituted or may be substituted with a substituent. It should be understood that the expression "R is selected from C₁₋₆ alkyl, -O-C₁₋₆ alkyl, and -NH-C₁₋₆ alkyl, wherein the C₁₋₆ alkyl is optionally substituted with halogen" means that the C₁₋₆ alkyl in the C₁₋₆ alkyl, -O-C₁₋₆ alkyl, and -NH-C₁₋₆ alkyl is optionally substituted with halogen. It should be understood that -N-(C₁₋₆ alkyl)₂ means that two C₁₋₆ alkyl groups are separately attached to the nitrogen atom, wherein the two alkyl groups may be identical or different.

As used herein, the term "pharmaceutically acceptable carrier" refers to any formulation or carrier medium capable of delivering an effective amount of the active substance of the present disclosure without interfering with the biological activity of the active substance and having no toxic and side effects on the host or subject. Representative carriers include water, oils, minerals, matrices, and the like. The matrices include suspending agents, viscosity increasing agents, pH adjusting agents, surfactants, diluents, stabilizers, and the like.

As used herein, a wavy bond ( 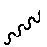 ) represents any one of the absolute configurations of the stereogenic center or a mixture thereof, that is, the wavy bond ( 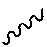 ) represents a wedged solid bond ( 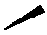 ), a wedged dashed bond ( 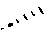 ), or a mixture; for example, 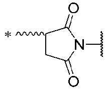 represents 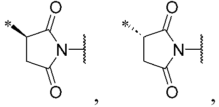 or a mixture of the two configurations; a crossed double bond represents the Z configuration, the E configuration, or a mixture thereof; for example, 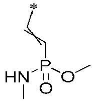 represents 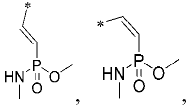 or a mixture of the two configurations.

As used herein, the term "subject" refers to an animal, particularly a mammal. A human is preferred.

As used herein, the term "effective amount" or "therapeutically effective amount" refers to an amount of a medicament or agent that is sufficient to provide the desired effect and is non-toxic. In the embodiments of the present disclosure, when treating a patient according to the present disclosure, the amount of a given medicament depends on a number of factors, such as the specific dosing regimen, the type of disease or disorder and its severity, and the individuality of the subject or host in need of treatment (e.g., body weight). However, the administration dose can be routinely determined by methods known in the art according to the specific circumstances, including, for example, the specific medicament that has been used, the route of administration, the disorder being treated, and the subject or host being treated. In general, for doses used for the treatment of adults, the administration doses are typically in the range of 0.02-5000 mg/day, e.g., about 1-1500 mg/day. The required dose may conveniently be represented as a single dose, or as divided doses administered simultaneously (or within a short period of time) or at appropriate intervals, e.g., two, three, four, or more doses per day. It will be understood by those skilled in the art that, although the dose ranges described above are given, the specific effective amount may be adjusted as appropriate to the patient's condition in combination with the physician's diagnosis.

As used herein, the term "stereoisomer" includes conformational isomers and configurational isomers, wherein the configurational isomers mainly include cis-trans isomers and optical isomers. The antibody-drug conjugates described in the present disclosure may be present in the form of stereoisomers and thus encompass all possible stereoisomeric forms, including but not limited to cis-trans isomers, tautomers, enantiomers, diastereomers, atropisomers, and the like. The antibody-drug conjugates described in the present disclosure may also be present in the form of any combination or any mixture of the aforementioned stereoisomers, such as equimolar mixtures of mesomers, racemates, and atropisomers. For example, a single enantiomer, a single diastereomer, or a mixture of the foregoing, or a single atropisomer or a mixture thereof. When the antibody-drug conjugates described in the present disclosure contain an olefinic double bond, they include cis isomers and trans isomers, as well as any combination thereof, unless otherwise specified. The atropisomers of the present disclosure are stereoisomers with axial or planar chirality that are produced on the basis of restricted rotation within the molecule. As medicaments, stereoisomers with excellent activity are preferred. The antibody-drug conjugates of the present disclosure have optical isomers derived from asymmetric carbons and the like. If necessary, single isomers can be obtained by resolution through methods known in the art, such as crystallization or chiral chromatography.

As used herein, the substitution with =CR^{e}R^{f} means that an atom of the molecule is attached to the carbon atom (C) in CR^{e}R^{f} via a double bond. That is, an atom of the molecule is substituted with 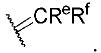 As used herein, the substitution with =NR^{e} means that an atom of the molecule is attached to the nitrogen atom (N) in NR^{e} via a double bond. That is, an atom of the molecule is substituted with 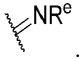 As used herein, the oxo means that an atom of the molecule is attached to an oxygen atom (O) via a double bond. That is, an atom of the molecule is substituted with 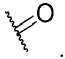 As used herein, the thio means that an atom of the molecule is attached to a sulfur atom (S) via a double bond. That is, an atom of the molecule is substituted with 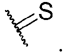 As used herein, when a group such as alkyl is located in the middle of a structural formula, the group is a -ylene group. For example, alkyl in this case is alkylene, etc.

As used herein, the term "alkyl" refers to a linear or branched saturated aliphatic hydrocarbyl group. The term "C₁₋₂₀ alkyl" refers to linear or branched alkyl having 1 to 20 carbon atoms. C₁₋₁₀ alkyl is preferred. C₁₋₆ alkyl (i.e., linear or branched alkyl having 1, 2, 3, 4, 5, or 6 carbon atoms) is more preferred. C₁₋₄ alkyl is more preferred. C₁₋₃ alkyl is more preferred. Specific examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. In the present disclosure, the various alkyl groups described above may be substituted or unsubstituted, and when each of them is substituted, the substituent is preferably one or more substituent groups described in the present application. As used herein, the term "alkyl" includes "alkylene".

As used herein, the term "heteroalkyl" refers to alkyl having at least one carbon atom replaced by a heteroatom (e.g., an O, N, or S atom), wherein the alkyl is as defined above. Heteroatoms may be present in the middle or at the end of a group. In the present disclosure, the various heteroalkyl groups described above may be substituted or unsubstituted, and when each of them is substituted, the substituent is preferably one or more substituent groups described in the present application. As used herein, the term "heteroalkyl" includes "heteroalkylene".

As used herein, the term "alkoxy" refers to a group having a structure of -O-alkyl, wherein the alkyl is as defined above. The term "C₁₋₁₀ alkoxy" refers to alkoxy having 1 to 10 carbon atoms. C₁₋₆ alkoxy is preferred. C₁₋₄ alkoxy is more preferred. C₁₋₃ alkoxy is more preferred. Specific examples include, but are not limited to, methoxy, ethoxy, n-propoxy, isopropoxy, n-butoxy, tert-butoxy, isobutoxy, n-pentoxy, and the like. In the present disclosure, the various alkoxy groups described above may be substituted or unsubstituted, and when each of them is substituted, the substituent is preferably one or more substituent groups described in the present application. As used herein, the term "alkoxy" includes "oxyalkylene".

As used herein, the term "alkenyl" refers to a linear or branched aliphatic hydrocarbyl group having one or more carbon-carbon double bonds at any site on the chain; the term "C₂₋₈ alkenyl" refers to alkenyl having 2 to 8 carbon atoms and at least one (e.g., 1 to 2) carbon-carbon double bond. C₂₋₆ alkenyl (i.e., alkenyl having 2 to 6 carbon atoms and 1 to 2 carbon-carbon double bonds) is preferred. C₂₋₄ alkenyl (i.e., alkenyl having 2 to 4 carbon atoms and 1 to 2 carbon-carbon double bonds) is more preferred. Specific examples include, but are not limited to, ethenyl, 1-propenyl, 2-propenyl, 1-, 2-, or 3-butenyl, pentenyl, hexenyl, butadienyl, and the like. In the present disclosure, the various alkenyl groups described above may be substituted or unsubstituted, and when each of them is substituted, the substituent is preferably one or more substituent groups described in the present application. As used herein, the term "alkenyl" includes "alkenylene".

As used herein, the term "alkynyl" refers to a linear or branched saturated aliphatic hydrocarbyl group having one or more carbon-carbon triple bonds at any site on the chain; the term "C₂₋₈ alkynyl" refers to alkynyl having 2 to 8 carbon atoms and at least one (e.g., 1 to 2) carbon-carbon triple bond. C₂₋₆ alkynyl (i.e., alkynyl having 2 to 6 carbon atoms and 1 to 2 carbon-carbon triple bonds) is preferred. C₂₋₄ alkynyl (i.e., alkynyl having 2 to 4 carbon atoms and 1 to 2 carbon-carbon triple bonds) is more preferred. Specific examples include, but are not limited to, ethynyl, 1-propynyl, 2-propynyl, 1-, 2-, or 3-butynyl, and the like. In the present disclosure, the various alkynyl groups described above may be substituted or unsubstituted, and when each of them is substituted, the substituent is preferably one or more substituent groups described in the present application. As used herein, the term "alkynyl" includes "alkynylene".

As used herein, the term "halogen" refers to fluorine, chlorine, bromine, or iodine.

As used herein, the term "halo" refers to fluoro, chloro, bromo, or iodo.

As used herein, the term "haloalkyl" refers to alkyl, in which one or more (e.g., 1, 2, 3, 4, or 5) hydrogen atoms are substituted with halogen, wherein the alkyl is as defined above. The term "C₁₋₁₀ haloalkyl" refers to haloalkyl having 1 to 10 carbon atoms. C₁₋₆ haloalkyl is preferred. C₁₋₄ haloalkyl is more preferred. C₁₋₃ haloalkyl is more preferred. Specific examples include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, and the like.

As used herein, the term "haloalkoxy" refers to alkoxy, in which one or more (e.g., 1, 2, 3, 4, or 5) hydrogen atoms are substituted with halogen, wherein the alkoxy is as defined above. The term "C₁₋₁₀ haloalkoxy" refers to haloalkoxy having 1 to 10 carbon atoms. C₁₋₆ haloalkoxy is preferred. C₁₋₄ haloalkoxy is more preferred. C₁₋₃ haloalkoxy is more preferred. Specific examples include, but are not limited to, trifluoromethoxy, trifluoroethoxy, monofluoromethoxy, monofluoroethoxy, difluoromethoxy, difluoroethoxy, and the like.

As used herein, the term "deuterated" refers to a substitution of one or more hydrogen atoms in a group with a deuterium atom.

As used herein, the term "deuterated alkyl" refers to alkyl, in which one or more (e.g., 1, 2, 3, 4, or 5) hydrogen atoms are substituted with a deuterium atom, wherein the alkyl is as defined above. The term "deuterated C₁₋₁₀ alkyl" refers to deuterated alkyl having 1 to 10 carbon atoms. Deuterated C₁₋₆ alkyl is preferred. Deuterated C₁₋₄ alkyl is more preferred. Deuterated C₁₋₃ alkyl is more preferred. Specific examples include, but are not limited to, monodeuteriomethyl, dideuteriomethyl, trideuteriomethyl, monodeuterioethyl, 1,2-dideuterioethyl, trideuterioethyl, and the like.

As used herein, the term "deuterated alkoxy" refers to alkoxy, in which one or more (e.g., 1, 2, 3, 4, or 5) hydrogen atoms are substituted with a deuterium atom, wherein the alkoxy is as defined above. The term "deuterated C₁₋₁₀ alkoxy" refers to deuterated alkoxy having 1 to 10 carbon atoms. Deuterated C₁₋₆ alkoxy is preferred. Deuterated C₁₋₄ alkoxy is more preferred. Deuterated C₁₋₃ alkoxy is more preferred. Specific examples include, but are not limited to, trideuteriomethoxy, trideuterioethoxy, monodeuteriomethoxy, monodeuterioethoxy, dideuteriomethoxy, dideuterioethoxy, and the like.

As used herein, the terms "cycloalkyl" and "cycloalkyl ring" are used interchangeably and refer to saturated monocyclic or polycyclic cyclic hydrocarbyl, for example, including monocyclic cycloalkyl, spirocycloalkyl, fused cycloalkyl, and bridged cycloalkyl. The ring carbon atoms of the cycloalkyl described in the present disclosure may be optionally substituted with 1, 2, or 3 oxo groups to form a cyclic ketone structure. The term "3- to 20-membered cycloalkyl" or "C₃₋₂₀ cycloalkyl" refers to cycloalkyl having 3 to 20 ring carbon atoms, including monocyclic cycloalkyl, spirocycloalkyl, fused cycloalkyl, and bridged cycloalkyl. C₃₋₁₂ cycloalkyl, C₅₋₂₀ spirocycloalkyl, C₅₋₂₀ fused cycloalkyl, or C₅₋₂₀ bridged cycloalkyl is preferred. C₃₋₈ monocyclic cycloalkyl is more preferred.

The terms "C₃₋₈ monocyclic cycloalkyl" and "3- to 8-membered monocyclic cycloalkyl" refer to saturated monocyclic cyclic hydrocarbyl having 3 to 8 ring carbon atoms. C₃₋₆ monocyclic cycloalkyl (i.e., 3- to 6-membered monocyclic cycloalkyl) or C₄₋₆ monocyclic cycloalkyl (i.e., 4- to 6-membered monocyclic cycloalkyl) is preferred. C₃, C₄, C₅, or C₆ monocyclic cycloalkyl is more preferred. Specific examples of monocyclic cycloalkyl include, but are not limited to, cyclopropyl, cyclobutyl, cyclopentyl, cyclohexyl, cycloheptyl, cyclooctyl, and the like.

As used herein, the terms "spirocycloalkyl" and "spirocycloalkyl ring" refer to polycyclic cyclic hydrocarbyl formed by sharing one carbon atom (referred to as a spiro atom) between two or more monocyclic rings. According to the number of spiro atoms shared between rings, the spirocycloalkyl may be classified as monospirocycloalkyl, bispirocycloalkyl, and polyspirocycloalkyl. The term "5- to 20-membered spirocycloalkyl" or "C₅₋₂₀ spirocycloalkyl" refers to polycyclic cyclic hydrocarbyl having 5 to 20 ring carbon atoms, wherein the monocyclic rings sharing a spiro atom are 3- to 8-membered monocyclic cycloalkyl rings. 6- to 14-membered (i.e., C₆₋₁₄) spirocycloalkyl is preferred. 6- to 14-membered monospirocycloalkyl is more preferred. 7- to 11-membered (i.e., C₇₋₁₁) spirocycloalkyl is more preferred. 7- to 11-membered monospirocycloalkyl is more preferred. 7-membered (4-membered monocyclic cycloalkyl ring/4-membered monocyclic cycloalkyl ring), 8-membered (4-membered monocyclic cycloalkyl ring/5-membered monocyclic cycloalkyl ring), 9-membered (4-membered monocyclic cycloalkyl ring/6-membered monocyclic cycloalkyl ring, 5-membered monocyclic cycloalkyl ring/5-membered monocyclic cycloalkyl ring), 10-membered (5-membered monocyclic cycloalkyl ring/6-membered monocyclic cycloalkyl ring), or 11-membered (6-membered monocyclic cycloalkyl ring/6-membered monocyclic cycloalkyl ring) monospirocycloalkyl is the most preferred. Specific examples of spirocycloalkyl include, but are not limited to:

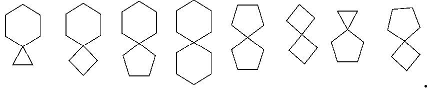

These spirocycloalkyl groups can be attached to the rest of the molecule through any one of the ring atoms.

As used herein, the terms "fused cycloalkyl" and "fused cycloalkyl ring" refer to polycyclic cyclic hydrocarbyl formed by sharing a pair of adjacent carbon atoms between two or more monocyclic rings. According to the number of the formed rings, the fused cycloalkyl may be classified as bicyclic, tricyclic, tetracyclic, or polycyclic fused cycloalkyl. The term "5- to 20-membered fused cycloalkyl" or "C₅₋₂₀ fused cycloalkyl" refers to polycyclic cyclic hydrocarbyl having 5 to 20 ring carbon atoms, wherein the monocyclic rings sharing a pair of adjacent carbon atoms are 3- to 8-membered monocyclic cycloalkyl rings. 6- to 14-membered (i.e., C₆₋₁₄) fused cycloalkyl is preferred. 6- to 14-membered bicyclic fused cycloalkyl is more preferred. 7- to 10-membered (i.e., C₇₋₁₀) fused cycloalkyl is more preferred. 7- to 10-membered bicyclic fused cycloalkyl is more preferred. 8-membered (5-membered monocyclic cycloalkyl ring fused to 5-membered monocyclic cycloalkyl ring), 9-membered (5-membered monocyclic cycloalkyl ring fused to 6-membered monocyclic cycloalkyl ring), or 10-membered (6-membered monocyclic cycloalkyl ring fused to 6-membered monocyclic cycloalkyl ring) bicyclic fused cycloalkyl is the most preferred. Specific examples of fused cycloalkyl include, but are not limited to:

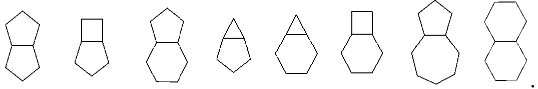

These fused cycloalkyl groups can be attached to the rest of the molecule through any one of the ring atoms.

As used herein, the terms "bridged cycloalkyl" and "bridged cycloalkyl ring" refer to polycyclic cyclic hydrocarbyl formed by sharing two carbon atoms that are not directly connected between two or more monocyclic rings. According to the number of the formed rings, the bridged cycloalkyl may be classified as bicyclic, tricyclic, tetracyclic, or polycyclic bridged cycloalkyl. The terms "5- to 20-membered bridged cycloalkyl" and "C₅₋₂₀ bridged cycloalkyl" refer to polycyclic cyclic hydrocarbyl having 5 to 20 ring carbon atoms, wherein any two rings share two carbon atoms that are not directly connected. 6- to 14-membered (i.e., C₆₋₁₄) bridged cycloalkyl is preferred. 7- to 10-membered (i.e., C₇₋₁₀) bridged cycloalkyl is more preferred. Specific examples of bridged cycloalkyl include, but are not limited to:

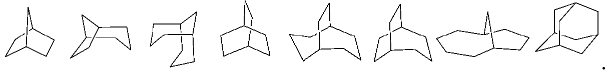

These bridged cycloalkyl groups can be attached to the rest of the molecule through any one of the ring atoms. As used herein, the term "halocycloalkyl" refers to cycloalkyl, in which one or more (e.g., 1, 2, 3, 4, or 5) hydrogen atoms are substituted with halogen, wherein the cycloalkyl is as defined above.

As used herein, the term "C₃₋₈ monocyclic halocycloalkyl" refers to monocyclic halocycloalkyl having 3 to 8 ring carbon atoms. C₃₋₆ monocyclic halocycloalkyl is preferred. C₃, C₄, C₅, or C₆ monocyclic halocycloalkyl is more preferred. Specific examples include, but are not limited to, trifluorocyclopropyl, monofluorocyclopropyl, monofluorocyclohexyl, difluorocyclopropyl, difluorocyclohexyl, and the like.

In the present disclosure, the various cycloalkyl groups described above may be substituted or unsubstituted, and when each of them is substituted, the substituent is preferably one or more substituent groups described in the present application.

As used herein, the terms "heterocyclyl" and "heterocyclyl ring" are used interchangeably and refer to saturated or partially unsaturated monocyclic or polycyclic cyclic hydrocarbyl, for example, including monocyclic heterocyclyl, spiroheterocyclyl, fused heterocyclyl, and bridged heterocyclyl. The ring carbon atoms of the heterocyclyl described in the present disclosure may be optionally substituted with 1, 2, or 3 oxo groups to form a cyclic ketone, cyclic lactone, or cyclic lactam structure. The term "3- to 20-membered heterocyclyl" refers to saturated or partially unsaturated monocyclic or polycyclic cyclic hydrocarbyl having 3 to 20 ring atoms, of which one or more (preferably 1, 2, 3, or 4) ring atoms are heteroatoms selected from nitrogen, oxygen, and S(=O)ₘ' (where m' is an integer of 0 to 2), excluding a cyclic moiety of -O-O-, -O-S-, or -S-S-, and the remaining ring atoms are carbon. When the ring atom is a nitrogen atom, the nitrogen atom may be substituted or unsubstituted (i.e., N or NR, R being hydrogen or other substituents already defined herein). The 3- to 20-membered heterocyclyl described in the present disclosure includes monocyclic heterocyclyl (e.g., 3- to 8-membered monocyclic heterocyclyl), 5- to 20-membered spiroheterocyclyl, 5- to 20-membered fused heterocyclyl, and 5- to 20-membered bridged heterocyclyl.

As used herein, the terms "3- to 8-membered monocyclic heterocyclyl" and "3- to 8-membered monocyclic heterocyclyl ring" refer to saturated or partially unsaturated monocyclic cyclic hydrocarbyl having 3 to 8 ring atoms, of which 1, 2, or 3 ring atoms are heteroatoms selected from nitrogen, oxygen, and S(=O)ₘ' (where m' is an integer of 0 to 2). 3- to 6-membered monocyclic heterocyclyl having 3 to 6 ring atoms, of which 1 or 2 ring atoms are heteroatoms, is preferred. 4- to 6-membered monocyclic heterocyclyl having 4 to 6 ring atoms, of which 1 or 2 ring atoms are heteroatoms, is more preferred. 5- or 6-membered monocyclic heterocyclyl having 5 or 6 ring atoms, of which 1 or 2 ring atoms are heteroatoms, is more preferred. When the heteroatom is a nitrogen atom, the nitrogen atom may be substituted or unsubstituted (i.e., N or NR, R being hydrogen or other substituents already defined herein). When the heteroatom is a sulfur atom, the sulfur atom may be optionally oxidized (i.e., S(=O)ₘ', where m' is an integer of 0 to 2). The ring carbon atoms of the monocyclic heterocyclyl may be optionally substituted with 1, 2, or 3 oxo groups to form a cyclic ketone, cyclic lactone, or cyclic lactam structure. Specific examples of monocyclic heterocyclyl include, but are not limited to, aziridine, oxirane, azetidine, azetidin-2-one, oxetane, oxetan-2-one, oxazolidine, pyrrolidin-2-one, pyrrolidine-2,5-dione, 1,3-dioxolane, dihydrofuran-2(3H)-one, dihydrofuran-2,5-dione, piperidin-2-one, piperidine-2,6-dione, tetrahydro-2H-pyran-2-one, imidazolidine, tetrahydrofuran, tetrahydrothiophene, tetrahydropyrrole, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazete, 1,2-dihydrooxete, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6-dihydropyridin-2(1H)-one, pyridine-2,6(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine, and the like.

As used herein, the term "3- to 6-membered nitrogen-containing heterocyclyl" refers to saturated or partially unsaturated monocyclic cyclic hydrocarbyl having 3 to 6 ring atoms, of which 1 ring atom is a nitrogen atom, and 1 or 2 of the remaining ring atoms are heteroatoms selected from nitrogen, oxygen, and S(=O)ₘ' (where m' is an integer of 0 to 2). Specific examples include, but are not limited to, aziridinyl, azetidinyl, azacyclopentyl (i.e., tetrahydropyrrole), azacyclohexyl (i.e., piperidine), morpholinyl, piperazinyl, and oxazolidine.

As used herein, the term "3- to 8-membered monocyclic heterocycloalkyl" refers to saturated monocyclic cyclic hydrocarbyl having 3 to 8 ring atoms, of which 1 or 2 ring atoms are heteroatoms. 3- to 6-membered monocyclic heterocycloalkyl, i.e., saturated monocyclic cyclic hydrocarbyl having 3 to 6 ring atoms, of which 1 or 2 ring atoms are heteroatoms, is preferred. Specific examples of heterocycloalkyl include, but are not limited to, aziridinyl, oxiranyl, azetidinyl, oxetanyl, oxazolidinyl, 1,3-dioxolanyl, dioxanyl, imidazolidinyl, tetrahydrofuranyl, tetrahydrothienyl, tetrahydropyrrolyl, piperidinyl, piperazinyl, morpholinyl, thiomorpholinyl, thiomorpholine-1,1-dioxide group, tetrahydropyranyl, 1,4-oxazepanyl, 1,3-oxazepanyl, 1,3-oxazinanyl, hexahydropyrimidinyl, and 1,4-dioxanyl.

The 2 connected ring atoms on the monocyclic heterocyclyl ring described above, including C-C and N-C, may both optionally be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heterocyclyl that form a fused ring with another ring are preferably C-C.

As used herein, the terms "spiroheterocyclyl" and "spiroheterocyclyl ring" refer to polycyclic heterocyclyl formed by sharing one carbon atom (referred to as a spiro atom) between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the remaining ring atoms are carbon. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. According to the number of spiro atoms shared between the rings, the spiroheterocyclyl may be classified as monospiroheterocyclyl, dispiroheterocyclyl, or polyspiroheterocyclyl. The term "5- to 20-membered spiroheterocyclyl" refers to spiroheterocyclyl having 5 to 20 ring atoms, wherein one of the monocyclic rings that share a spiro atom is a 3- to 8-membered monocyclic heterocyclyl ring, and the other monocyclic ring is a 3- to 8-membered monocyclic heterocyclyl ring or a 3- to 8-membered monocyclic cycloalkyl ring. 6- to 14-membered spiroheterocyclyl having 6 to 14 ring atoms, of which 1 or 2 ring atoms are heteroatoms, is preferred. 7- to 11-membered spiroheterocyclyl having 7 to 11 ring atoms, of which 1 or 2 ring atoms are heteroatoms, is more preferred. 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring), or 11-membered (6-membered monocyclic heterocyclyl ring/6-membered monocyclic heterocyclyl ring) monospiroheterocyclyl is the most preferred. Specific examples of spiroheterocyclyl include, but are not limited to:

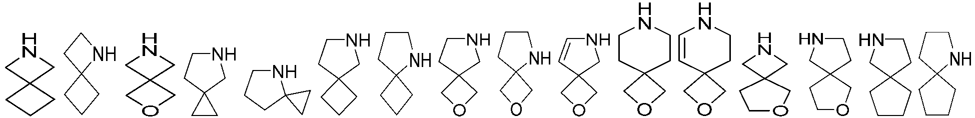

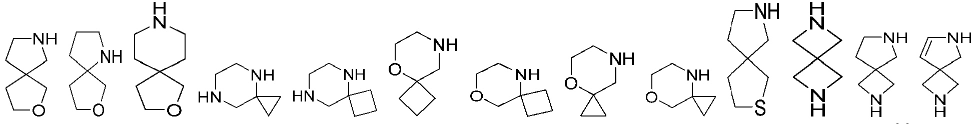

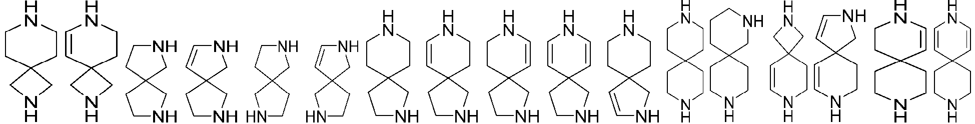

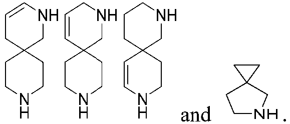

These spiroheterocyclyl groups can be attached to the rest of the molecule through any one of the suitable ring atoms.

As used herein, the terms "fused heterocyclyl" and "fused heterocyclyl ring" refer to polycyclic heterocyclyl formed by sharing a pair of adjacent ring atoms between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the remaining ring atoms are carbon. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. The shared pair of adjacent ring atoms may be C-C or N-C. According to the number of constituent rings, the fused heterocyclyl may be classified as bicyclic, tricyclic, tetracyclic, or polycyclic fused heterocyclyl. The term "5- to 20-membered fused heterocyclyl" refers to fused heterocyclyl having 5 to 20 ring atoms, wherein the monocyclic rings sharing a pair of adjacent ring atoms are 3- to 8-membered monocyclic heterocyclyl rings. 6- to 14-membered fused heterocyclyl having 6 to 14 ring atoms, of which 1 or 2 ring atoms are heteroatoms, is preferred. 6- to 10-membered fused heterocyclyl having 6 to 10 ring atoms, of which 1 or 2 ring atoms are heteroatoms, is more preferred. 8- to 10-membered fused heterocyclyl having 8 to 10 ring atoms, of which 1 or 2 ring atoms are heteroatoms, is more preferred. 8-membered (5-membered monocyclic heterocyclyl ring fused to 5-membered monocyclic heterocyclyl ring), 9-membered (5-membered monocyclic heterocyclyl ring fused to 6-membered monocyclic heterocyclyl ring), or 10-membered (6-membered monocyclic heterocyclyl ring fused to 6-membered monocyclic heterocyclyl ring) bicyclic fused heterocyclyl is the most preferred. Specific examples of fused heterocyclyl include, but are not limited to:

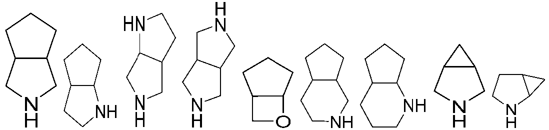

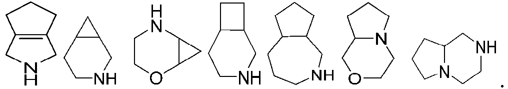

These fused heterocyclyl groups can be attached to the rest of the molecule through any one of the suitable ring atoms.

As used herein, the terms "bridged heterocyclyl" and "bridged heterocyclyl ring" refer to polycyclic heterocyclyl formed by sharing two ring atoms that are not directly connected between two or more saturated or partially unsaturated monocyclic rings, wherein one or more (e.g., 1, 2, or 3) ring atoms are heteroatoms selected from nitrogen, oxygen, and S(=O)ₘ' (where m' is an integer of 0 to 2), and the remaining ring atoms are carbon. According to the number of the formed rings, the bridged cycloalkyl may be classified as bicyclic, tricyclic, tetracyclic, or polycyclic bridged cycloalkyl. The term "5- to 20-membered bridged heterocyclyl" refers to saturated or partially unsaturated polycyclic heterocyclyl having 5 to 20 ring atoms, wherein any two rings share two ring atoms that are not directly connected, and each monocyclic ring may contain one or more double bonds, but none of the rings has a fully conjugated π-electron system. 6- to 14-membered bridged heterocyclyl is preferred. 7- to 10-membered bridged heterocyclyl is more preferred. Specific examples of bridged heterocyclyl include, but are not limited to:

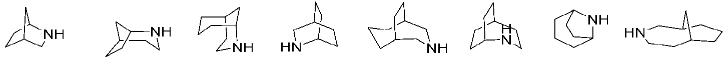

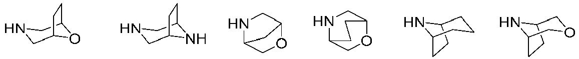

These bridged heterocyclyl groups can be attached to the rest of the molecule through any one of the suitable ring atoms.

The term "5- to 15-membered tricyclic heterocyclyl" refers to tricyclic heterocyclyl having 5 to 15 ring atoms, wherein three rings may be connected in one or more ways selected from spiro connection, fused connection, and bridged connection. Specific examples may include, but are not limited to, a first monocyclic ring being a 6-membered monocyclic heterocyclic ring, a second monocyclic ring being a 6-membered monocyclic heterocyclic ring, and a third monocyclic ring being a 3- to 6-membered monocyclic heterocyclic ring; and the first monocyclic ring is fused to the second monocyclic ring, and the third monocyclic ring is spiro-connected to the second monocyclic ring.

The term "7- to 12-membered bicyclic heterocyclyl" refers to bicyclic heterocyclyl having 7 to 12 ring atoms, wherein two rings may be connected in one way selected from spiro connection, fused connection, and bridged connection. Specific examples may be selected from the specific examples of the spiroheterocyclyl, the specific examples of the fused heterocyclyl, and the specific examples of the bridged heterocyclyl described above.

The term "9- to 11-membered bicyclic heterocyclyl" refers to bicyclic heterocyclyl having 9 to 11 ring atoms, wherein two rings may be connected in one way selected from spiro connection, fused connection, and bridged connection. Specific examples may be selected from the specific examples of the spiroheterocyclyl, the specific examples of the fused heterocyclyl, and the specific examples of the bridged heterocyclyl described above.

In the present disclosure, the various heterocyclyl groups described above may be optionally substituted, and when each of them is substituted, the substituent is preferably one or more substituent groups described in the present application.

As used herein, the terms "aryl", "aryl ring", and "aromatic ring" are used interchangeably and refer to an all-carbon monocyclic, an all-carbon non-fused polycyclic (rings are connected by covalent bonds and not fused), or an all-carbon fused polycyclic (i.e., rings that share a pair of adjacent carbon atoms) group. At least one ring in the group is aromatic, i.e., has a conjugated π-electron system. The term "C₆₋₁₄ aryl" refers to aryl having 6 to 14 ring atoms. C₆₋₁₀ aryl is preferred. In the present disclosure, C₆₋₁₄ aryl includes monocyclic aryl, non-fused polycyclic aryl, and aromatic fused polycyclic ring, wherein examples of monocyclic aryl include phenyl, and examples of non-fused polycyclic aryl include biphenyl and the like.

In the present disclosure, when C₆₋₁₄ aryl is an aromatic fused polycyclic ring, the aromatic fused polycyclic ring may be a polycyclic group formed by fusing a monoaryl ring to one or more monoaryl rings; non-limiting examples include naphthyl, anthryl, and the like.

In some embodiments of the present disclosure, when C₆₋₁₄ aryl is an aromatic fused polycyclic ring, the aromatic fused polycyclic ring may also be a polycyclic group formed by fusing a monoaryl ring (e.g., phenyl) to one or more non-aromatic rings, wherein the ring attached to the parent structure is an aromatic ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure) and a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure). The polycyclic group formed by fusing the monoaryl ring to one or more non-aromatic rings described above may be attached to another group or the parent structure via a nitrogen atom or carbon atom, and the ring attached to the parent structure is a monoaryl ring or non-aromatic ring.

In the present disclosure, the various aryl groups described above may be substituted or unsubstituted, and when each of them is substituted, the substituent is preferably one or more substituent groups described in the present application.

As used herein, the terms "heteroaryl", "heteroaryl ring", and "heteroaromatic ring" are used interchangeably and refer to a monocyclic or fused polycyclic (i.e., sharing a pair of adjacent ring atoms, which may be C-C or N-C) group, in which the ring atoms are substituted with at least one heteroatom independently selected from nitrogen, oxygen, and sulfur, wherein the nitrogen and sulfur atoms may optionally be oxidized, and the nitrogen atom may optionally be quaternized. The heteroaryl has 6, 10, or 14 π electrons shared, and at least one ring in the group is aromatic. The term "5- to 14-membered heteroaryl" refers to heteroaryl having 5 to 14 ring atoms, of which 1, 2, 3, 4, or 5 ring atoms are heteroatoms selected from nitrogen, oxygen, and S(=O)ₘ' (where m' is an integer of 0 to 2). 5- to 10-membered heteroaryl having 5 to 10 ring atoms, of which 1, 2, 3, or 4 ring atoms are heteroatoms, is preferred. In the present disclosure, the 5- to 14-membered heteroaryl may be monocyclic heteroaryl, fused bicyclic heteroaryl, or fused tricyclic heteroaryl.

As used herein, the term "5- or 6-membered monocyclic heteroaryl" refers to monocyclic heteroaryl having 5 or 6 ring atoms, of which 1, 2, or 3 ring atoms are heteroatoms selected from nitrogen, oxygen, and S(=O)ₘ' (where m' is an integer of 0 to 2). Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

As used herein, the term "8- to 10-membered bicyclic heteroaryl" refers to fused bicyclic heteroaryl having 8 to 10 ring atoms, of which 1, 2, 3, 4, or 5 ring atoms are heteroatoms selected from nitrogen, oxygen, and S(=O)ₘ' (where m' is an integer of 0 to 2). The fused bicyclic heteroaryl may be a bicyclic group (preferably a 9- or 10-membered bicyclic heteroaryl ring) formed by fusing a monoaryl ring (e.g., phenyl) to a monocyclic heteroaryl ring (preferably a 5- or 6-membered monocyclic heteroaryl ring), or a bicyclic group formed by fusing a monocyclic heteroaryl ring (preferably a 5- or 6-membered monocyclic heteroaryl ring) to a monocyclic heteroaryl ring (preferably a 5- or 6-membered monocyclic heteroaryl ring).

Any 2 connected ring atoms on the monocyclic heteroaryl ring described above, including C-C, N-C, and N-N, may all be fused to the cycloalkyl, heterocyclyl, aryl, or heteroaryl defined in the present disclosure, such as a monocyclic cycloalkyl ring, a monocyclic heterocyclyl ring, a monoaryl ring, or a 5- or 6-membered monocyclic heteroaryl ring, to form a fused polycyclic ring. The 2 connected ring atoms on the monocyclic heteroaryl ring that form a fused ring with another ring are preferably C-C, including but not limited to the following forms:

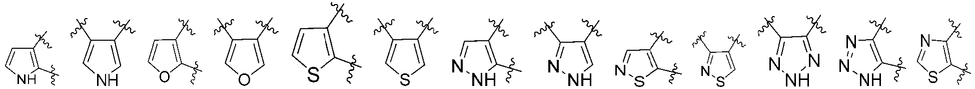

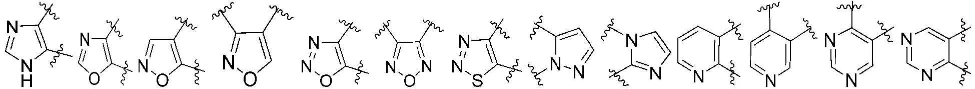

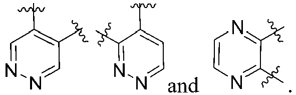

The ring atoms marked by " 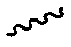 " in the groups described above are attached to other moieties of the molecule. Non-limiting examples of 8- to 10-membered bicyclic heteroaryl include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, and the like.

Specific examples of bicyclic heteroaryl include, but are not limited to: 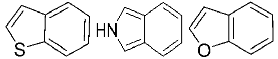 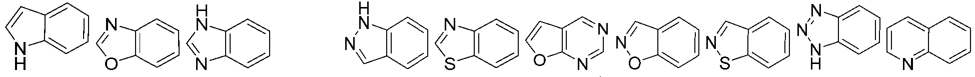 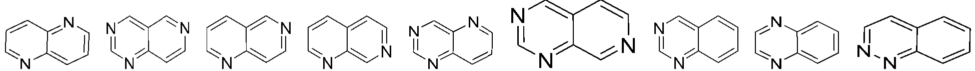 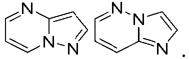 These groups can be attached to the rest of the molecule through any one of the suitable ring atoms. The ring attached to the parent structure may be a monocyclic heteroaryl ring or a benzene ring.

In some embodiments of the present disclosure, the fused bicyclic heteroaryl or fused tricyclic heteroaryl may be a polycyclic group formed by fusing a monocyclic heteroaryl ring (preferably a 5- or 6-membered monocyclic heteroaryl ring) to one or more non-aromatic rings, wherein the ring attached to the parent structure is a monocyclic heteroaryl ring or a non-aromatic ring. The non-aromatic ring includes, but is not limited to, a 3- to 6-membered monocyclic heterocyclyl ring (preferably a 5- or 6-membered monocyclic heterocyclyl ring, wherein ring carbon atoms of the monocyclic heterocyclyl ring may be substituted with 1 to 2 oxo groups to form a cyclic lactam or cyclic lactone structure), a 3- to 6-membered monocyclic cycloalkyl ring (preferably a 5- or 6-membered monocyclic cycloalkyl ring, wherein ring carbon atoms of the monocyclic cycloalkyl ring may be substituted with 1 or 2 oxo groups to form a cyclic ketone structure), and the like. The polycyclic group formed by fusing the monocyclic heteroaryl ring to one or more non-aromatic rings described above may be attached to another group or the parent structure via a nitrogen atom or carbon atom, and the ring attached to the parent structure is a monocyclic heteroaryl ring or non-aromatic ring.

The 6- to 12-membered heteroaryl-fused cycloalkyl is formed by fusing monocyclic heteroaryl to cycloalkyl, and the ring attached to the parent structure may be monocyclic heteroaryl or cycloalkyl. For example, the 6- to 12-membered heteroaryl-fused cycloalkyl is formed by fusing 5-membered monocyclic heteroaryl to 3-, 4-, 5-, 6-, or 7-membered cycloalkyl, or by fusing 6-membered monocyclic heteroaryl to 3-, 4-, 5-, or 6-membered cycloalkyl.

The 6- to 12-membered heteroaryl-fused heterocyclyl is formed by fusing monocyclic heteroaryl to heterocyclyl, and the ring attached to the parent structure may be monocyclic heteroaryl or heterocyclyl. For example, the 6-to 12-membered heteroaryl-fused heterocyclyl is formed by fusing 5-membered monocyclic heteroaryl to 3-, 4-, 5-, 6-, or 7-membered heterocyclyl, or by fusing 6-membered monocyclic heteroaryl to 3-, 4-, 5-, or 6-membered heterocyclyl.

In the present disclosure, the various heteroaryl groups described above may be substituted or unsubstituted, and when each of them is substituted, the substituent is preferably one or more substituent groups described in the present application.

As used herein, a subscript numerical value of a group indicates the number of the group, and when the numerical value is a range, the group may be present in any number within the numerical value range. For example, - (CH₂CH₂O)₀₋₁₂- indicates 0 to 12 (e.g., 0, 1, 2, 3, 4, 5, 6, 7, 8, 9, 10, 11, or 12) CH₂CH₂O; -(CH₂)₁₋₄- indicates 1 to 4 (e.g., 1, 2, 3, or 4) CH₂.

As used herein, the term "hydroxy" refers to -OH.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "carboxyl" refers to -C(=O)OH.

As used herein, the term "acetyl" refers to -COCH₃.

As used herein, the term "substituted" or "substitution" means that any one or more hydrogen atoms on a specific atom are substituted with a substituent that may include deuterium and hydrogen variants, as long as the valence of the specific atom is normal and the compound after substitution is stable. When the substituent is oxo (i.e., =O), it means that two hydrogen atoms are substituted. The term "optionally substituted" means that the substitution may or may not take place. Unless otherwise specified, the type and number of the substituent may be arbitrary as long as being chemically achievable.

When any variable (e.g., R) occurs once or more in the constitution or structure of a compound, the definition of the variable in each case is independent. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

In any embodiment, any or all hydrogens present in the compound, or hydrogens in a particular group or moiety within the compound, may be replaced by deuterium or tritium. One to the maximum number of hydrogens present in the compound may be replaced by deuterium. One to the maximum number of hydrogens present in any group in the general formula compound or specific compound may be deuterated. For example, when a certain group is described as ethyl, the ethyl may be C₂H₅ or C₂H₅ with x (1 to 5) hydrogens replaced by deuterium, e.g., C₂DₓH₅₋ₓ. When a certain group is described as deuterated ethyl, the deuterated ethyl may be C₂H₅ with x (1 to 5) hydrogens replaced by deuterium, e.g., C₂DₓH₅₋ₓ. The stable deuterated derivatives described in the present disclosure are preferably stable deuterated isotopic derivatives obtained by substituting any hydrogen atom that can be deuterated in each general formula with 1 to the maximum number (e.g., 1 to 2, 1 to 3, 1 to 4, 1 to 5, 1 to 6, 1 to 7, etc.) of deuterium atoms.

Unless otherwise specified, structures depicted herein are also intended to include compounds that differ only by the presence of one or more isotopically enriched atoms. Exemplary isotopes that can be incorporated into the compounds of the present disclosure include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I. Isotopically labeled compounds (e.g., compounds labeled by ³H and ¹⁴C) can be used in compound or substrate tissue distribution assays. Tritium (i.e., ³H) and carbon-14 (i.e., ¹⁴C) isotopes are available for use for ease of preparation and detectability. Additionally, substitution with heavier isotopes such as deuterium (i.e., ²H) may afford certain therapeutic benefits resulting from increased metabolic stability (e.g., increased *in vivo* half-life or reduced dose requirements). In some embodiments, one or more hydrogen atoms are replaced by ²H or ³H, or one or more carbon atoms are replaced by ¹³C- or ¹⁴C-enriched carbon. Positron emitting isotopes, such as ¹⁵O, ¹³N, ¹¹C, and ¹⁸F, can be used in positron emission tomography (PET) studies to determine receptor occupancy by substrates. The preparation of isotopically labeled compounds is known to those skilled in the art. For example, isotopically labeled compounds can generally be prepared following procedures analogous to those disclosed for the compounds of the present disclosure described herein by replacing a non-isotopically labeled reagent with an isotopically labeled reagent.

The term "antibody-drug conjugate (ADC)" means that an antibody is linked to a biologically active cytotoxic drug via a stable linking unit. In the present application, the ADC may mean that a monoclonal antibody or antibody fragment is linked to a biologically active cytotoxic drug via a stable linking unit.

The term "antibody" refers to any form of antibody having a desired biological activity. An antibody specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "monoclonal antibody" is an antibody or a fragment of an antibody that is derived from a population of highly homologous antibody molecules, that is, the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "full-length antibody" refers to an immunoglobulin molecule comprising four peptide chains when present naturally, including two heavy (H) chains and two light (L) chains linked to each other via disulfide bonds. Each heavy chain consists of a heavy chain variable region (abbreviated herein as VH) and a heavy chain constant region (abbreviated herein as CH). The heavy chain constant region consists of 3 domains, CH1, CH2, and CH3. Each light chain consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains comprise binding domains that interact with antigens. The constant regions of an antibody can mediate the binding of immunoglobulins to host tissues or factors, including the binding of various cells of the immune system (e.g., effector cells) to the first component (Clq) of a classical complement system.

The term "CDR" refers to a complementarity-determining region within an antibody variable sequence. There are 3 CDRs in each of the heavy chain variable regions and light chain variable regions, which are named HCDR1, HCDR2 and HCDR3 for the heavy chain variable region, or LCDR1, LCDR2 and LCDR3 for the light chain variable region. The precise amino acid sequence boundaries of the variable region CDRs of the antibodies of the present disclosure can be determined using any of many well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures. The boundaries of the CDRs of the antibodies of the present disclosure can be determined by those skilled in the art according to any scheme (e.g., different assignment systems or combinations) in the art.

It should be noted that boundaries of CDRs of variable regions of the same antibody obtained based on different assignment systems may differ. That is, CDR sequences of the variable regions of the same antibody defined by different assignment systems are different. Thus, when it comes to defining an antibody with a specific CDR sequence defined in the present disclosure, the scope of the antibody also encompasses antibodies whose variable region sequences comprise the specific CDR sequence but whose claimed CDR boundaries differ from the specific CDR boundaries defined in the present disclosure due to the application of different schemes (e.g., different assignment systems or combinations).

The term "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of binding fragments of an antibody include, but are not limited to, Fab, Fab', F(ab')2, and Fv fragments; a diabody; a linear antibody; a single-chain antibody molecule, such as scFv; and a nanobody and a multispecific antibody formed by antibody fragments. A binding fragment or a derivative generally retains at least 10% of its antigen-binding activity when the antigen-binding activity is expressed on a molar concentration basis. Preferably, the binding fragment or the derivative retains at least 20%, 50%, 70%, 80%, 90%, 95%, or 100% or more of the antigen-binding affinity of the parent antibody. It is also contemplated that an antigen-binding fragment of an antibody may include conservative or non-conservative amino acid substitutions that do not significantly alter its bioactivity (referred to as "conservative variants" or "function-conservative variants" of the antibody).

The term "chimeric antibody" is an antibody having the variable domains of a first antibody and the constant domains of a second antibody, wherein the first and second antibodies are from different species. Typically, the variable domains are derived from an antibody of a rodent and the like ("parent antibody"), and the constant domain sequences are derived from a human antibody, such that the resulting chimeric antibody is less likely to induce an adverse immune response in a human subject as compared to the parent rodent antibody.

The term "humanized antibody" refers to an antibody form containing sequences from both human and non-human (such as mouse and rat) antibodies. In general, a humanized antibody comprises substantially all of at least one, and typically two, variable domains, in which all or substantially all of the hypervariable loops correspond to those of a non-human immunoglobulin and all or substantially all of the framework regions (FRs) are those of a human immunoglobulin sequence. The humanized antibody may optionally comprise at least a portion of a human immunoglobulin constant region (Fc).

The term "fully human antibody" refers to an antibody that comprises only human immunoglobulin sequences. A fully human antibody may contain mouse glycochains if produced in mice, mouse cells, or hybridomas derived from mouse cells. Likewise, a "mouse antibody" refers to an antibody that comprises only mouse immunoglobulin sequences. Alternatively, a fully human antibody may contain rat glycochains if produced in rats, rat cells, or hybridomas derived from rat cells. Likewise, a "rat antibody" refers to an antibody that comprises only rat immunoglobulin sequences.

The term "isotype" of an antibody refers to types of antibodies (e.g., IgM, IgE, and IgG (such as IgG1, IgG2, or IgG4)) provided by heavy chain constant region genes. Isotypes also include modified forms of one of these types in which modifications have been made to alter Fc function, for example, to enhance or attenuate effector function or binding to Fc receptors.

The term "Fc region" is used to define the C-terminal region of an immunoglobulin heavy chain comprising at least a part of constant regions. The term includes Fc regions of native sequences and variant Fc regions. In one embodiment, the Fc region of a human IgG heavy chain extends from Cys226 or Pro230 to the carboxyl-terminus of the heavy chain. However, the C-terminal lysine (Lys447) of the Fc region may or may not exist (numbering in this paragraph is according to the EU numbering system, also known as the EU index, as described in Rabat et al., Sequences of Proteins of Immunological Interest, 5th Ed. Public Health Service, National Institutes of Health, Bethesda, MD, 1991).

The term "cytotoxic drug" generally refers to a toxic drug, and the cytotoxic drug may have a chemical molecule that can strongly disrupt the normal growth of tumor cells. Cytotoxic drugs can kill tumor cells at a sufficiently high concentration. The "cytotoxic drug" may include toxins, such as small molecule toxins or enzymatically active toxins of bacterial, fungal, plant, or animal origin, radioisotopes (e.g., At211, I131, I125, Y90, Re186, Re188, Sm153, Bi212, P32, or radioactive isotopes of Lu), toxic drugs, chemotherapy drugs, antibiotics, and nucleolytic enzymes; for example, the cytotoxic drug may be toxic drugs, including but not limited to camptothecin derivatives.

The term "linker unit" or "linker structure" generally refers to a chemical structural fragment or bond that is linked to an antibody at one end and to a cytotoxic drug at the other end, or that is linked to other linkers before being linked to the cytotoxic drug. The direct or indirect linking to an antibody may mean that the group is directly linked to the antibody via a covalent bond, and may also be linked to the antibody via a linker structure. The term a structure being "optionally linked/attached to other molecular moieties" generally means that the structure is not linked to any other chemical structure, or that the structure is linked (e.g., via a chemical bond or a linker structure) to one or more other chemical structures (e.g., antibodies described in the present application) different from the structure.

The term "human epidermal growth factor receptor 3 (Her3)", also known as receptor tyrosine-protein kinase ERBB-3 (ERBB3), refers to a member of the EGFR/erBB family. Unlike other erBB family members Her2 and EGFR, Her3 *per se* does not have kinase activity. Thus, Her3 must bind to its kinase active member EGFR or Her2 to trigger its downstream activity as a heterodimer. Upon binding to its natural ligand NRG1, Her3 undergoes conformational changes, heterodimerization, and phosphorylation, and then activates MAPK, PI3K/Akt, and PLCγ via signal transduction.

It should be noted that in the following groups, No. 1 and No. 2 have the same meaning; for example, R₁ has the same range as Rj²¹, and when Rj²¹ is defined, R₁ is concurrently defined.

| Group | No. 1 | No. 2 | Group | No. 1 | No. 2 |
|---|---|---|---|---|---|
| 1 | R₁ | Rj²¹ | 6 | R₃ | Rj⁵ |
| 2 | R₂ | Rj²² | 7 | R₆ | Rj⁴ |
| 3 | L₁ | Lj | 8 | R₁₂ | Rj³ |
| 4 | R₄₂ | Rj¹¹ | 9 | R₅ | Yj₄ |
| 5 | R₄₃ | Rj¹² | | | |

### BRIEF DESCRIPTION OF THE DRAWINGS

FIG. 1 shows an ellipsoid plot of the three-dimensional molecular structure of intermediate 11-e-1.
FIG. 2 shows an ellipsoid plot of the three-dimensional molecular structure of compound 14-1A.

### Sequence information

Part of the sequence information is shown in the preparation of ADCs, and part of the sequence information is shown in the table below.

| SEQ ID NO: | | Sequence information | SEQ ID NO: | | Sequence information |
|---|---|---|---|---|---|
| 39 | Short peptide | GGFG | 42 | Short peptide | KAAD |
| 40 | Short peptide | GGVA | 43 | Short peptide | VKGG |
| 41 | Short peptide | KAAN | 44 | Linker | GGGGS |

### DETAILED DESCRIPTION

The antibody-drug conjugates and compounds of the present disclosure may be prepared by a variety of synthetic methods well known to those skilled in the art, including the specific embodiments listed below, embodiments formed by combinations thereof with other chemical synthetic methods, and equivalents thereof well known to those skilled in the art. Preferred embodiments include, but are not limited to, the examples of the present disclosure.

The present disclosure will be described in detail below with reference to examples, but those skilled in the art will appreciate that the following examples are only for illustrating the present disclosure and should not be construed as limitations to the scope of the present disclosure. Although the present disclosure has been described in detail herein and specific examples have also been disclosed, it will be apparent to those skilled in the art that various changes and modifications can be made to the specific examples without departing from the spirit and scope of the present disclosure. Experimental procedures without specified conditions in the examples are conducted according to conventional conditions or conditions recommended by the manufacturers. Reagents or instruments without specified manufacturers used herein are all commercially available conventional products. The structures of the compounds were determined by methods such as nuclear magnetic resonance (NMR) and mass spectrometry (MS).

The NMR determination was performed on a Bruker Avance NEO 400 or Bruker Avance NEO 500 nuclear magnetic resonance instrument. The chemical shift (δ) was in parts per million (ppm). The solvents for determination were as shown in each of the examples. The internal standard was tetramethylsilane (TMS).

The MS determination was performed on an Agilent 1100 liquid chromatograph.

The high performance liquid chromatography (HPLC) analysis was performed on a Waters 2695 high performance liquid chromatograph.

The preparative high performance liquid chromatography was performed on a Waters 2767 high performance liquid chromatograph.

The chiral HPLC analysis was performed on a Waters 2695 high performance liquid chromatograph or a Waters Investigator SFC system.

The preparative chiral chromatography was performed on a gilson gx-281 chromatograph or a waters SFC-80 chromatograph.

Qingdao GF254 or Yantai Huanghai HSGF254 silica gel plates were used as thin-layer chromatography silica gel plates. 0.15 mm-0.2 mm silica gel plates were used for thin-layer chromatography (TLC). 0.4 mm-0.5 mm silica gel plates were used for thin-layer chromatography product separation and purification.

The ISCO CombiFlash NextGen 300 column chromatography system was generally used for column chromatography, and the Agela Flash Column Silica-Cs series silica gel column was used.

The prep-HPLC used in the following examples, unless otherwise specified, may be performed under the following conditions:
Prep-HPLC (ammonium bicarbonate method 1): chromatographic column: Waters XBridge C18, 190 × 250 mm, 5 µm; mobile phase: A: 0.1% aqueous ammonium bicarbonate solution; B: preparative grade acetonitrile; flow rate: 15 mL/min; B% = 20%-100%; column temperature: room temperature.
Prep-HPLC (ammonium bicarbonate method 2): chromatographic column: Welch Xtimate, 21.2 × 150 mm, 5 µm; mobile phase A: 0.1% aqueous ammonium bicarbonate solution, mobile phase B: ACN; flow rate: 15 mL/min; column temperature: room temperature.
Prep-HPLC (formic acid method): column type: Waters XBridge C18, 19 × 250 mm, 5 µm; mobile phase system: A: 0.1% aqueous formic acid solution; B: preparative grade acetonitrile; flow rate: 15 mL/min; B% = 20%-100%; column temperature: room temperature.
Prep-HPLC (trifluoroacetic acid method): chromatographic column: Welch Xtimate C18, 21.2 × 150 mm, 5 µm; mobile phase A: 0.1% trifluoroacetic acid-water, mobile phase B: acetonitrile; flow rate: 15 mL/min; column temperature: room temperature.
Retention time measurement (3 min LCMS) conditions: A: water (0.05% TFA) B: ACN (0.05% TFA); elution gradient: 0-3 min, 5%-95% B; flow rate: 1.7 mL/min; column type: SunFire C18, 4.6 × 50 mm, 3.5 µm; column temperature: 40 °C.

### Example 1

### I. Preparation of Linker-Payload A1 (Such as Compound AI-3, Compound AII-3, Compound AIII-3, Compound AIV-3, Compound AV-3, and Compound AVI-3)

1. Compound AI-1 can react with functional group Fg₂ in Fg₁-Fg₂ to form compound AI-2; compound AI-2 can react with functional group Fg₃ in H-L₁ₐ-L_{1b}-Fg₃ via functional group Fg₁ to form compound AI-3. 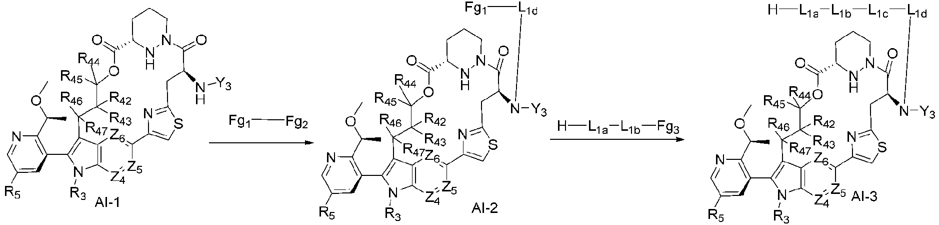
2. Compound AI-1 can react with functional group Fg₅ in Fg₄-Fg₅ to form compound AII-2; compound AII-2 can react with functional group Fg₆ in H-L₁ₐ-L_{1b}-Fg₆ via functional group Fg₄ to form compound AII-3. 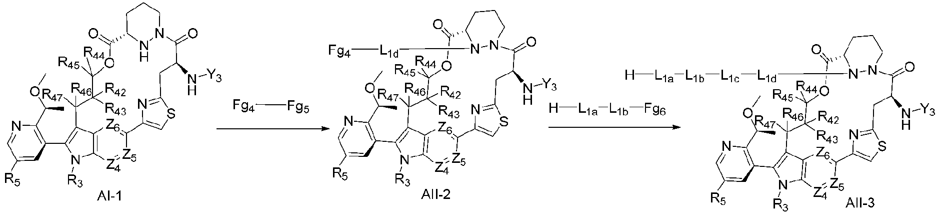
3. Compound AIII-1 can react with functional group Fg₈ in Fg₇-Fg₈ to form compound AIII-2; compound AIII-2 can react with functional group Fg₉ in H-L₁ₐ-L_{1b}-Fg₉ via functional group Fg₇ to form compound AIII-3. 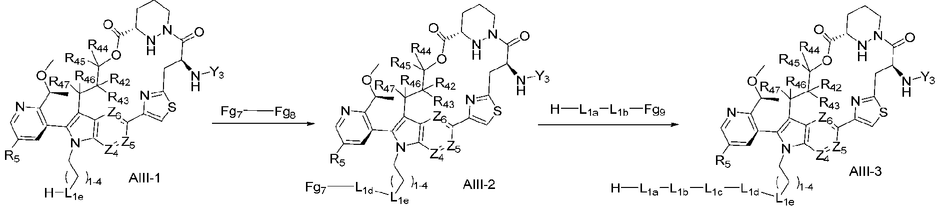
4. Compound AIV-1 can react with functional group Fg₁₂ in Fg₁₁-Fg₁₂ via functional group Fg₁₀ to form compound AIV-2; compound AIV-2 can react with functional group Fg₁₃ in H-L₁ₐ-L_{1b}-Fg₁₃ via functional group Fg₁₁ to form compound AIV-3, wherein R₅'-Fg₁₀ in formula AIV-1 = R₅. 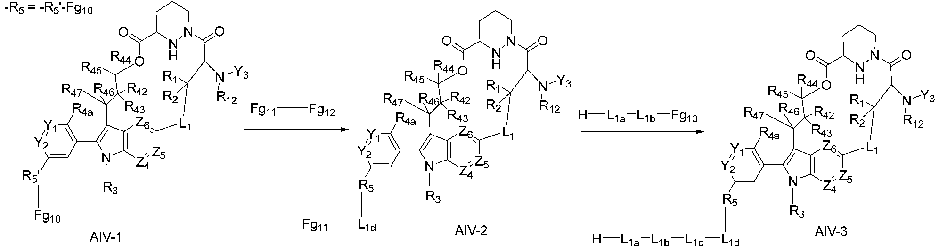
5. CompoundAV-1 can react with functional group Fg₁₆ in Fg₁₅-Fg₁₆ via functional group Fg₁₄ to form compound AV-2; compound AV-2 can react with functional group Fg₁₇ in H-L₁ₐ-L_{1b}-Fg₁₇ via functional group Fg₁₅ to form compound AV-3, wherein Y₃'-Fg₁₄ in formula AV-1 = Y₃. 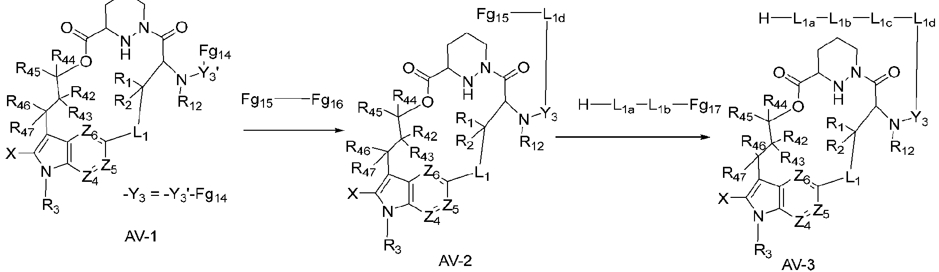
6. Compound AVI-1 can react with functional group Fg₂₀ in Fg₁₉-Fg₂₀ via functional group Fg₁₈ to form compound AVI-2; compound AVI-2 can react with functional group Fg₂₁ in H-L₁ₐ-L_{1b}-Fg₂₁ via functional group Fg₁₉ to form compound AVI-3, wherein Y_{10'}-Fg₁₈ in formula AVI-1 = Y₁₀. 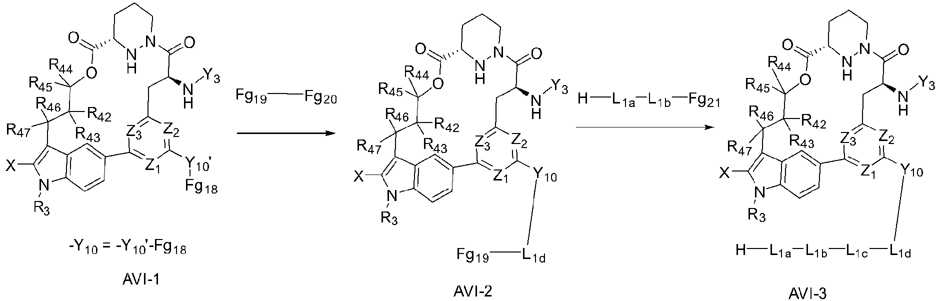 wherein H is as defined for H₁, and H₁, L₁ₐ, L_{1b}, L_{1c}, L_{1d}, L₁ₑ, R₁, R₂, R₁₂, R₃, R₅, R₄ₐ, R₄₂, R₄₃, R₄₄, R₄₅, R₄₆, R₄₇, X, L₁, Z₁, Z₂, Z₃, Z₄, Z₅, Z₆, Y₁, Y₂, Y₃, and Y₁₀ are as defined herein.

### Preparation Example 1. Synthesis of Intermediate 1

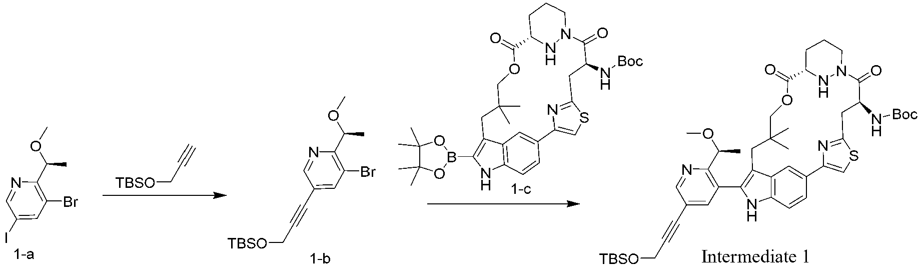

Step 1: tert-Butyldimethyl(2-propynyloxy)silane (2.09 g, 12.282 mmol) and compound 1-a (4 g, 11.697 mmol) were dissolved in acetonitrile (20 mL), and DIPEA (3.02 g, 23.394 mmol), PdCl₂(PPh₃)₂ (0.41 g, 0.585 mmol), and copper(I) iodide (0.22 g, 1.170 mmol) were sequentially added. The mixture was reacted under an argon atmosphere. The reaction mixture was stirred at room temperature overnight, diluted with ethyl acetate, and filtered. The filtrate was concentrated by evaporation to remove the solvent. The residue was purified by a flash silica gel column (0-10% ethyl acetate/heptane) to give compound 1-b (4.3 g, 11.187 mmol, yield: 95.64%) as a pale yellow liquid. ES-API: [M+H]⁺= 384.1/386.1.

Step 2: Compound 1-c (100 mg, 0.144 mmol, intermediate 3 in WO202260836A1) and compound 1-b (110.83 mg, 0.288 mmol) were dissolved in 1,4-dioxane (1.5 mL), toluene (0.5 mL), and water (0.5 mL), and then potassium phosphate (91.70 mg, 0.432 mmol) and Pd(dtbpf)Cl₂ (18.88 mg, 0.029 mmol) were added. The mixture was stirred at 65 °C overnight under an argon atmosphere. After the reaction was completed, the mixture was diluted with ethyl acetate, washed with brine, and concentrated to give a residue. The residue was purified by a flash silica gel column (0-70% ethyl acetate/petroleum ether) to give intermediate 1 (110 mg, 0.126 mmol, yield: 87.59%) as a yellow oil. ES-API [M+H]⁺= 871.3.

### Preparation Example 2. Synthesis of Intermediate 2

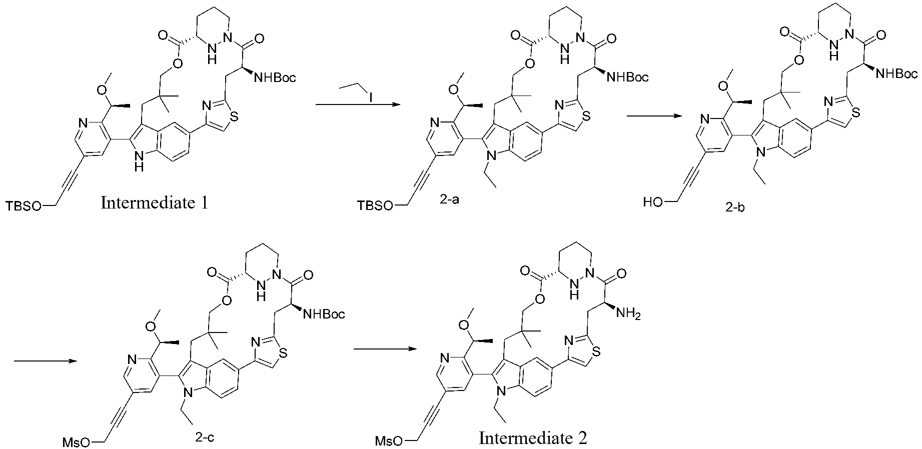

Step 1: Cesium carbonate (3572 mg, 10.962 mmol) and iodoethane (0.88 mL, 10.962 mmol) were sequentially added to a solution of intermediate 1 (955 mg, 1.096 mmol) in N,N-dimethylformamide (10 mL). The mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to give crude compound 2-a (1136 mg, yield: 99%). ES-API: [M+H]⁺= 899.3.

Step 2: A tetrabutylammonium fluoride solution (3.670 mL, 3.670 mmol) was added to a solution of compound 2-a (1100 mg, 1.233 mmol) in tetrahydrofuran (30 mL) under an ice-water bath. The reaction mixture was stirred at 0 °C for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to give crude compound 2-b (960 mg, yield: 99%). ES-API: [M+H]⁺= 785.3.

Step 3: N,N-Diisopropylethylamine (1.0 mL, 5.733 mmol) and methanesulfonic anhydride (300 mg, 1.720 mmol) were sequentially added to a solution of compound 2-b (900 mg, 1.147 mmol) in dichloromethane (10 mL) under an ice-water bath. The mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give compound 2-c (970 mg, yield: 98%). ES-API: [M+H]⁺= 863.2.

Step 4: Trifluoroacetic acid (5 mL) was added to a solution (5 mL) of compound 2-c (970 mg, 1.124 mmol) in dichloromethane, and the reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined and dried over anhydrous sodium sulfate. The mixture was concentrated, and the crude product was purified by automated flash chromatography on silica gel (methanol/dichloromethane = 0-6%) to give intermediate 2 (591 mg, yield: 69%). ES-API: [M+H]⁺= 763.1.

### Preparation Example 3. Synthesis of Intermediate 3

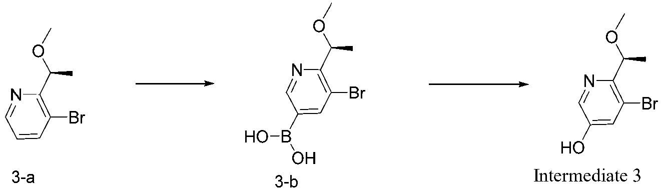

Step 1: Compound 3-a (10 g, 46.279 mmol) was weighed out and dissolved in tetrahydrofuran (100 mL), and bis(pinacolato)diboron (17.63 g, 69.419 mmol), chloro(1,5-cyclooctadiene)iridium(I) dimer (0.93 g, 1.388 mmol), and 4,4'-di-tert-butyl-2,2'-dipyridyl (1.86 g, 6.942 mmol) were added. The mixture was reacted at 80 °C for 15 h under a nitrogen atmosphere, filtered through celite, and concentrated under reduced pressure to give crude compound 3-b, which was directly used in the next step. ES-API[M+H]⁺=260.0.

Step 2: The crude product obtained in the previous step was dissolved in acetonitrile (100 mL), and hydrogen peroxide (1308.25 mg, 11.543 mmol) was added dropwise under an ice-water bath. The mixture was reacted at room temperature for 3 h, and the reaction was quenched with a saturated sodium sulfite solution (20 mL). The mixture was concentrated under reduced pressure and extracted with dichloromethane (25 mL × 3). The organic phase was washed with water (20 mL) and saturated brine (20 mL), dried over anhydrous sodium sulfate, and concentrated under reduced pressure to give a crude product. The crude product was purified by a flash silica gel column (petroleum ether/ethyl acetate = 1/1) to give intermediate 3 (7000 mg, 30.162 mmol, yield: 64.8%) as a yellow solid, ES-API [M+H]⁺ = 232.1, 234.1.

### Preparation Example 4. Synthesis of Intermediate 7 and Intermediate 7A

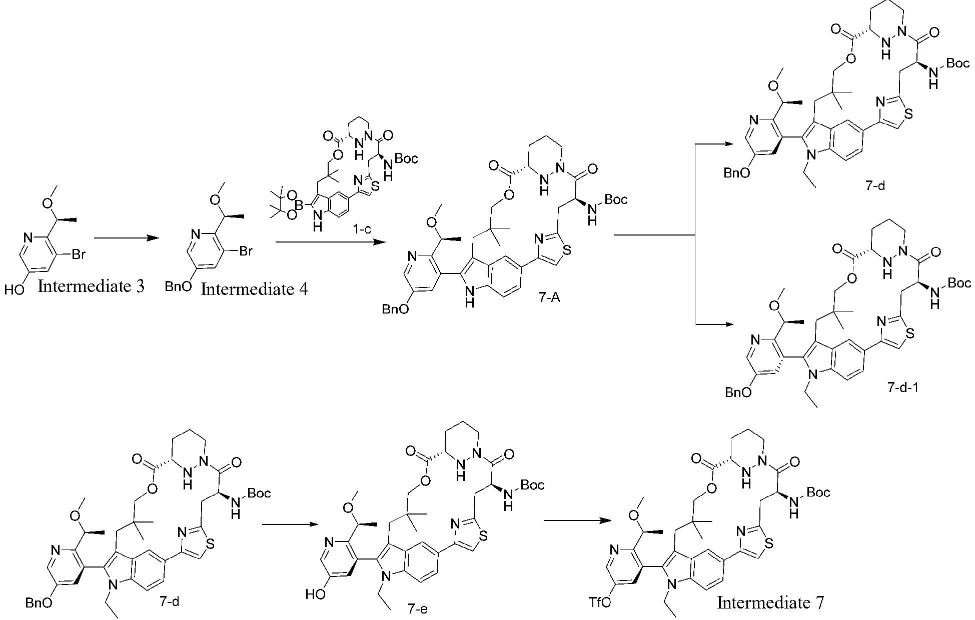

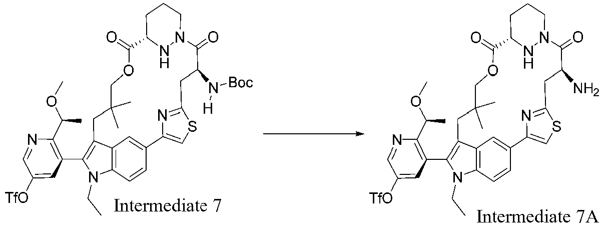

Step 1: Intermediate 3 (500 mg, 2.154 mmol) was dissolved in N,N-dimethylformamide (10 mL), and benzyl bromide (0.512 mL, 4.308 mmol) and potassium carbonate (893 mg, 6.462 mmol) were added to the solution described above. After the addition was completed, the reaction system was stirred at 80 °C for 17 h. Water (50 mL) was added to the reaction mixture, and the mixture was extracted with ethyl acetate (50 mL × 2). The organic phase was washed with saturated brine (50 mL × 3), dried over anhydrous sodium sulfate, filtered, and concentrated to give a crude product. The crude product was purified by a flash silica gel column (methanol/dichloromethane: 0%-2%) to give intermediate 4 (600 mg, 1.862 mmol, yield: 86.44%) as a white solid. ES-API: [M+H]⁺=322.1, 324.1.

Step 2: Intermediate 4 (279 mg, 0.865 mmol) was dissolved in dioxane (1 mL), toluene (3 mL), and water (1 mL). Compound 1-c (300 mg, 0.432 mmol), [1,1'-bis(di-tert-butylphosphino)ferrocene]dichloropalladium(II) (28 mg, 0.043 mmol), and potassium phosphate (275.40 mg, 1.297 mmol) were added to the solution described above at room temperature. After the addition was completed, the reaction system was stirred at 70 °C for 2 h under an argon atmosphere. After the reaction was completed, water (30 mL) was added to the reaction mixture, and the mixture was extracted with ethyl acetate (30 mL × 2). The organic phase was washed with saturated brine (30 mL × 3), dried over anhydrous sodium sulfate, filtered, and concentrated to give a crude product. The crude product was purified by a flash silica gel column (methanol/dichloromethane: 0%-3%) to give compound 7-A (300 mg, 0.371 mmol, yield: 85.74%) as a light yellow solid. ES-API: [M +H]⁺=809.1.

Step 3: Compound 7-A (600 mg, 0.742 mmol) was dissolved in N,N-dimethylformamide (10 mL). Iodoethane (580 mg, 3.710 mmol) and cesium carbonate (725 mg, 2.226 mmol) were added to the solution described above. The reaction system was stirred at room temperature for 2 h. After the reaction was completed, water (50 mL) was added to the solution described above, and the mixture was extracted with ethyl acetate (30 mL × 2). The organic phase was washed with saturated brine (50 mL × 3), dried over anhydrous sodium sulfate, filtered, and concentrated to give a crude product. The crude product was purified by a flash silica gel column (ethyl acetate/petroleum ether: 0%-50%) to give 2 isomeric compounds. The structure of one of the isomeric compounds was compound 7-d (200 mg, 0.239 mmol, yield: 32.22%, ethyl acetate:petroleum ether = 1:1, Rf = 0.35) as a white solid. ES-API: [M +H]⁺=837.1.

The structure of the other isomeric compound was compound 7-d-1 (350 mg, 0.418 mmol, yield: 56.35%, ethyl acetate:petroleum ether = 1:1, Rf = 0.40) as a white solid. ES-API: [M +H]⁺=837.1.

Step 4: Compound 7-d (200 mg, 0.239 mmol) was dissolved in methanol (5 mL), and wet palladium on carbon (10 wt%, 20 mg) and wet palladium hydroxide/carbon (10 wt%, 20 mg) were added to the solution described above. After being purged three times with hydrogen, the reaction system was stirred at room temperature for 17 h under a hydrogen atmosphere (15 psi). The reaction mixture was filtered through celite, and the filter cake was washed with methanol (20 mL). The filtrate was concentrated to give compound 7-e (150 mg, 0.201 mmol, yield: 84%) as a white solid. ES-API: [M +H]⁺=747.1.

Step 5: Compound 7-e (40 mg, 0.054 mmol) was dissolved in dichloromethane (2 mL), and triethylamine (16 mg, 0.16 mmol) and 1,1,1-trifluoro-N-phenyl-N-[(trifluoromethyl)sulfonyl]methanesulfonamide (28.70 mg, 0.080 mmol) were added to the solution described above. After the addition was completed, the reaction system was stirred at room temperature for 2 h. Water (10 mL) was added to the reaction mixture, and the mixture was extracted with dichloromethane (15 mL × 2). The organic phase was washed with saturated brine (20 mL × 2), dried over anhydrous sodium sulfate, filtered, and concentrated to give a crude product. The crude product was purified by a flash silica gel column (methanol/dichloromethane: 0%-2%) to give intermediate 7 (30 mg, 0.034 mmol, yield: 63.73%) as a white solid. ES-API: [M +H]⁺=879.2. ¹H NMR (400 MHz, DMSO-*d₆*) *δ* 9.00 (d, *J =* 2.8 Hz, 1H), 8.52 (d, *J =* 1.2 Hz, 1H), 8.25 (d, *J =* 2.8 Hz, 1H), 7.85 (s, 1H), 7.82 - 7.76 (m, 1H), 7.62 (d, *J =* 8.8 Hz, 1H), 7.31 (d, *J =* 9.2 Hz, 1H), 5.23 (t, *J=* 8.8 Hz, 1H), 5.07 (d, *J =* 12.4 Hz, 1H), 4.44 - 4.31 (m, 2H), 4.27 - 4.17 (m, 2H), 4.15 - 4.08 (m, 1H), 3.60 - 3.52 (m, 2H), 3.30 (s, 3H), 3.25 - 3.14 (m, 2H), 3.03 - 2.95 (m, 1H), 2.80 - 2.70 (m, 1H), 2.47 - 2.41 (m, 1H), 2.14 - 2.06 (m, 1H), 1.85 - 1.75 (m, 2H), 1.54 - 1.46 (m, 1H), 1.40 (s, 9H), 1.36 (d, *J =* 6.0 Hz, 3H), 0.92 (s, 3H), 0.87 (t, *J =* 7.2 Hz, 3H), 0.28 (s, 3H).

Step 6: Intermediate 7 (600 mg, 0.683 mmol) was dissolved in methanol (2 mL) under an ice-water bath, and hydrochloric acid (5 mL, a 4 M solution in dioxane) was slowly added. The mixture was reacted for 1 h and concentrated, and then dichloromethane (20 mL) was added for dilution. The resulting mixture was washed sequentially with a saturated aqueous sodium bicarbonate solution (10 mL) and saturated brine (10 mL), dried over anhydrous sodium sulfate, filtered, and concentrated to give crude intermediate 7A (530 mg, 0.680 mmol, yield: 99%), which was directly used in the next step. ES-API: [M+H]⁺=779.2.

### Preparation Example 5. Synthesis of Intermediate 5

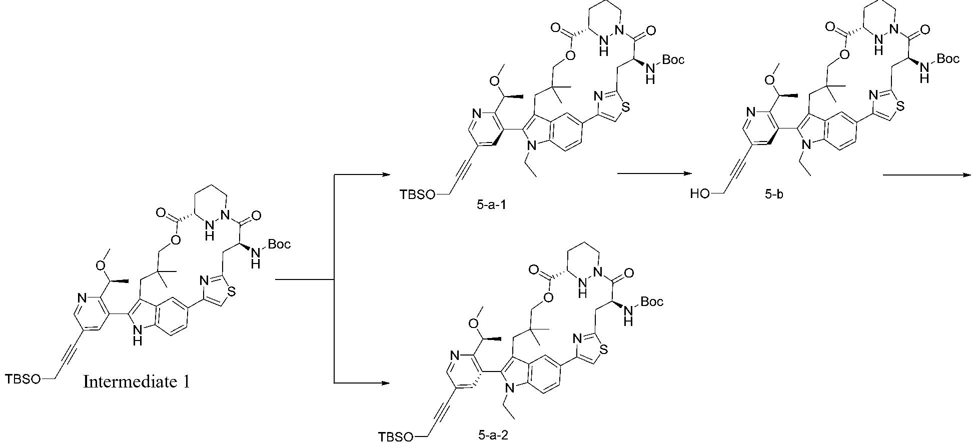

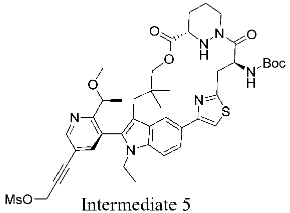

Step 1: Cesium carbonate (1481.03 mg, 4.546 mmol) and iodoethane (0.606 mL, 7.576 mmol) were sequentially added to a solution of intermediate 1 (1320 mg, 1.515 mmol) in N,N-dimethylformamide (10 mL). The mixture was stirred at room temperature for 1 h. When the reaction was completed as detected by LCMS, the reaction mixture was diluted with ethyl acetate (20 mL), and then the mixture was washed with water (10 mL) and saturated brine (15 mL × 3). The organic phase was dried over anhydrous sodium sulfate, filtered, and concentrated to give a crude product. The crude product was then purified by silica gel column chromatography (petroleum ether/ethyl acetate = 1/1) to give two isomeric compounds. The structure of one of the isomeric compounds was compound 5-a-1 (430 mg, yield: 31.56%, retention time = 3.491 min). ES-API: [M+H]⁺= 899.3. The structure of the other isomeric compound was compound 5-a-2 (800 mg, yield: 58.72%, retention time = 3.257 min). ES-API: [M+H]⁺= 899.3.

Step 2: A tetrabutylammonium fluoride solution (4 mL, a 1.0 M solution in tetrahydrofuran) was added to compound 5-a-1 (430 mg, 0.478 mmol) under an ice-water bath. The mixture was stirred at room temperature for 1 h. When the reaction was completed as detected by LCMS, the reaction mixture was diluted with ethyl acetate (20 mL), and then the mixture was washed with saturated brine (15 mL × 3). The organic phase was dried over anhydrous sodium sulfate, filtered, and concentrated to give crude compound 5-b (350 mg, 0.446 mmol, yield: 93.24%). ES-API: [M+H]⁺= 785.3.

Step 3: N,N-Diisopropylethylamine (172.89 mg, 1.338 mmol) and methanesulfonic anhydride (116.50 mg, 0.669 mmol) were sequentially added to a solution of compound 5-b (350 mg, 0.446 mmol) in dichloromethane (10 mL) under an ice-water bath. The mixture was stirred at room temperature for 1 h. When the reaction was completed as detected by LCMS, the reaction mixture was diluted with dichloromethane (20 mL), and then the mixture was washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give intermediate 5 (340 mg, 0.394 mmol, yield: 88.35%). ES-API: [M+H]⁺= 863.2.

### Preparation Example 6. Synthesis of Intermediate 6

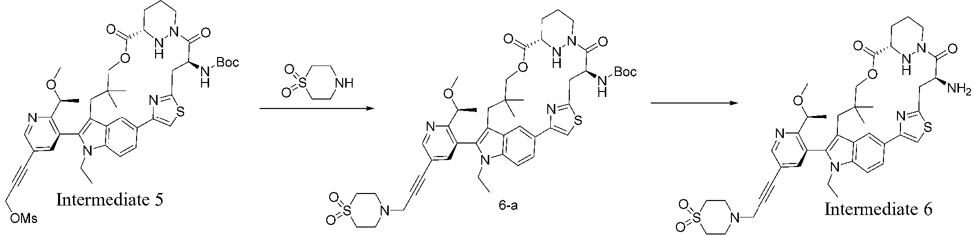

Step 1: N,N-Diisopropylethylamine (0.08 mL, 0.465 mmol) and 1,1-dioxidothiomorpholine (25 mg, 0.186 mmol) were sequentially added to a solution of intermediate 5 (80 mg, 0.093 mmol) in dichloromethane (6 mL). The mixture was stirred at room temperature overnight. When the reaction was completed as detected by LCMS, the mixture was concentrated under reduced pressure to give crude compound 6-a (83 mg, yield: 99%). ES-API: [M+H]⁺= 902.1.

Step 2: Compound 6-a (83 mg, 0.092 mmol) was dissolved in a solution (3 mL) of hydrochloric acid in dioxane and a solution (0.5 mL) of hydrochloric acid in methanol. The reaction mixture was stirred at room temperature for 1 h. When the reaction was completed as detected by LCMS, the reaction mixture was concentrated under reduced pressure to give crude intermediate 6 (73 mg, yield: 99%). ES-API: [M+H]⁺= 802.1.

### Preparation Example 7. Synthesis of Intermediate 8

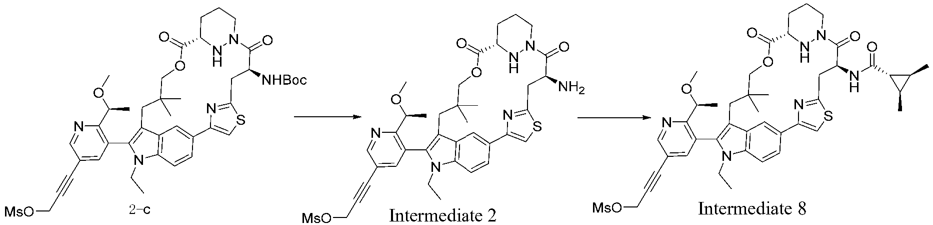

Step 1: Trifluoroacetic acid (5 mL) was added to a solution (5 mL) of intermediate 2-c (970 mg, 1.124 mmol) in dichloromethane, and the reaction mixture was stirred at room temperature for 1.5 h. When the reaction was completed as detected by LCMS, the mixture was concentrated under reduced pressure to remove most of the solvent, and a saturated sodium bicarbonate solution (10 mL) was added to the residue under an ice-water bath. The mixture was extracted with dichloromethane (15 mL × 3). The organic phases were combined and dried over anhydrous sodium sulfate. The mixture was concentrated, and the crude product was purified by automated flash chromatography on silica gel (methanol/dichloromethane = 0-6%) to give compound intermediate 2 (591 mg, yield: 69%). ES-API: [M+H]⁺= 763.1.

Step 2: (1r,2R,3S)-2,3-Dimethylcyclopropane-1-carboxylic acid (101 mg, 0.885 mmol), N,N-diisopropylethylamine (0.52 mL, 2.949 mmol), and O-(7-azabenzotriazol-1-yl)-N,N,N',N'-tetramethyluronium hexafluorophosphate (113 mg, 0.297 mmol) were sequentially added to a solution of compound intermediate 2 (450 mg, 0.590 mmol) in N,N-dimethylformamide (10 mL) under an ice-water bath. The reaction mixture was warmed to room temperature and stirred for 10 min. When the reaction was completed as detected by LCMS, the reaction mixture was diluted with ethyl acetate (20 mL), and then the mixture was washed with water (10 mL) and saturated brine (10 mL × 3). The organic phase was dried over anhydrous sodium sulfate, filtered, and concentrated to dryness. The residue was purified by automated flash chromatography on silica gel (tetrahydrofuran/petroleum ether = 0-60%) to give intermediate 8 (400 mg, yield: 79%). ES-API: [M+H]⁺= 859.2.

### Preparation Example 8. Synthesis of Intermediate 9

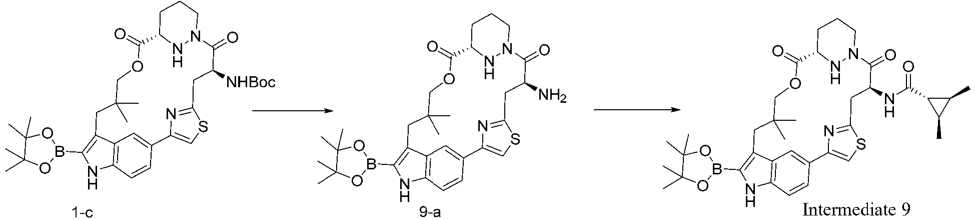

Step 1: Compound 1-c (120 mg, 0.173 mmol) was dissolved in methanol (0.5 mL), and a solution (3 mL, 0.173 mmol) of hydrochloric acid in dioxane was added. The mixture was reacted at room temperature for 1 h. The reaction mixture was concentrated under reduced pressure to give compound 9-a (100 mg, 0.168 mmol, yield: 97.39%). ES-API: [M+H]⁺=594.3.

Step 2: Compound 9-a (80 mg, 0.099 mmol) was dissolved in N,N-dimethylformamide (2 mL), and (1r,2R,3S)-2,3-dimethylcyclopropane-1-carboxylic acid (17.31 mg, 0.152 mmol), O-(7-azabenzotriazol-1-yl)-N,N,N',N'-tetramethyluronium hexafluorophosphate (86.48 mg, 0.227 mmol), and N,N-diisopropylethylamine (58.79 mg, 0.455 mmol) were added. The mixture was reacted at room temperature for 1 h. Water (10 mL) was added to the reaction mixture, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give intermediate 9 (90 mg, 0.130 mmol, yield: 86.06%). ES-API: [M+H]⁺=690.3.

### Preparation Example 9. Synthesis of Intermediate 10

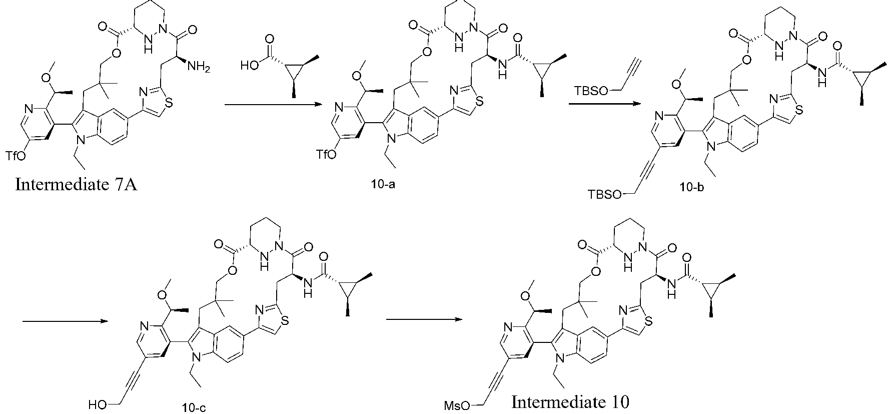

Step 1: Intermediate 7A(5700 mg, 7.318 mmol) was dissolved in N,N-dimethylformamide (10 mL). (1R,2R,3S)-2,3-Dimethylcyclopropane-1-carboxylic acid (1085.92 mg, 9.514 mmol), O-(7-azabenzotriazol-1-yl)-N,N,N',N'-tetramethyluronium hexafluorophosphate (1416.11 mg, 10.978 mmol), and N,N'-diisopropylethylamine (945.90 mg, 7.318 mmol) were added to the solution described above.After the addition was completed, the reaction system was stirred at room temperature for 2 h. After the reaction was completed, water (50 mL) was added, and the mixture was extracted with ethyl acetate (35 mL × 2). The organic phase was washed with saturated brine (20 mL × 3), dried, and concentrated to give a crude product. The crude product was purified by a flash silica gel column (methanol/dichloromethane: 0%-10%) to give compound 10-a (5650 mg, 6.457 mmol, yield: 88.23%) as a white solid, ES-API: [M+H]⁺ = 875.2.

Step 2: Compound 10-a (5650 mg, 6.457 mmol) and tert-butyldimethyl(2-propynyloxy)silane (2199.72 mg, 12.914 mmol) were dissolved in acetonitrile (50 mL), and bis(triphenylphosphine)palladium(II) dichloride (1810.60 mg, 2.583 mmol), copper(I) iodide (743.87 mg, 3.874 mmol), N,N'-diisopropylethylamine (3331.92 mg, 25.829 mmol), and anhydrous lithium chloride (1084.81 mg, 25.829 mmol) were added. The mixture was reacted at 80 °C for 5 h under a nitrogen atmosphere. After the reaction was completed, the mixture was filtered under vacuum and concentrated under reduced pressure to give a crude product. The crude product was purified by a flash silica gel column (40 g, methanol/dichloromethane: 0%-10%) to give compound 10-b (4550 mg, 5.082 mmol, 78.71%) as a brown solid, ES-API: [M+H]⁺ = 895.3.

Step 3: Compound 10-b (4550 mg, 5.082 mmol) was dissolved in tetrahydrofuran (50 mL), and a 1 M tetrabutylammonium fluoride solution (12 mL) was added. The mixture was reacted at room temperature for 2 h under a nitrogen atmosphere. When the reaction was completed as monitored by LC-MS, water (50 mL) was added, and the mixture was extracted with ethyl acetate (35 mL × 2). The organic phase was washed with saturated brine (20 mL × 3), dried, and concentrated to give a crude product. The crude product was purified by a flash silica gel column (methanol/dichloromethane: 0%-10%) to give compound 10-c (3900 mg, 4.994 mmol, 98.25%, purity: 85%) as a brown solid, ES-API: [M+H]⁺ = 781.3.

Step 4: Compound 10-c (3900 mg, 4.994 mmol, purity: 85%) was dissolved in dichloromethane (50 mL), and methanesulfonic anhydride (1042.68 mg, 5.992 mmol) was added. N,N'-Diisopropylethylamine (2581.72 mg, 19.975 mmol) was then added under an ice-water bath. The mixture was reacted at room temperature for 1 h under a nitrogen atmosphere. After the starting materials were consumed as monitored by LC-MS, the mixture was concentrated under reduced pressure to give intermediate 10 (3600 mg, 4.191 mmol, yield: 98.73%), ES-API: [M+H]⁺ = 859.3.

### Preparation Example 10. Synthesis of Intermediate 11

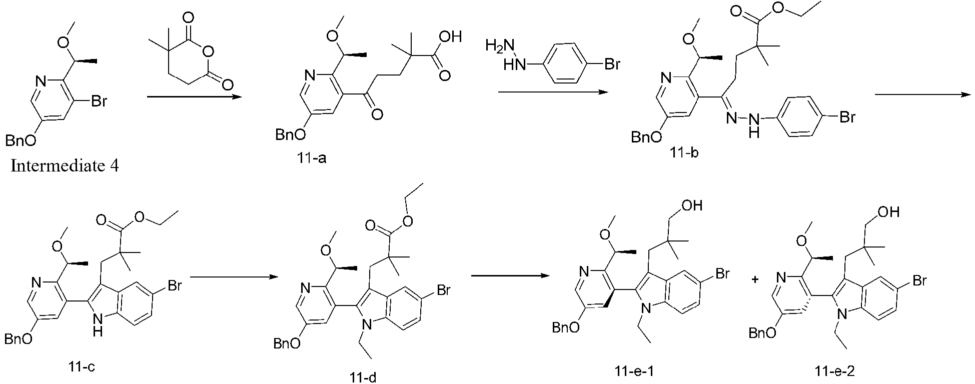

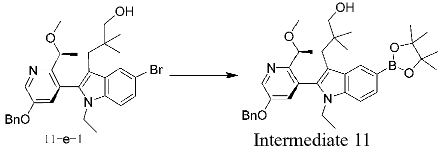

Step 1: Isopropylmagnesium chloride-lithium chloride complex (10.027 mL, 13.035 mmol, a 1.3 M solution in tetrahydrofuran) was slowly added dropwise to a solution of intermediate 4 (3 g, 9.311 mmol) in tetrahydrofuran (20 mL) at 0 °C under a nitrogen atmosphere, and the mixture was stirred at 0 °C for 0.5 h. The solution described above was then added dropwise to a solution of 3,3-dimethyltetrahydropyran-2,6-dione (1.59 g, 11.173 mmol) in tetrahydrofuran (20 mL) at -10 °C, and the mixture was stirred at 0 °C for 2 h. The pH of the solution was adjusted to 4-5 by dropwise addition of 4 M hydrochloric acid/dioxane (4 mL) to quench the reaction. The reaction mixture was extracted with ethyl acetate (20 mL × 3), dried over anhydrous sodium sulfate, filtered, and concentrated, and the residue was then purified by a silica gel column (petroleum ether/ethyl acetate = 1/1) to give compound 11-a (2.25 g, 5.837 mmol, yield: 62.69%). ES-API: [M+H]⁺=386.2.

Step 2: (4-Bromophenyl)hydrazine (970.36 mg, 5.188 mmol) and a 4 M solution (3.891 mL) of hydrochloric acid in 1,4-dioxane were added to a solution of compound 11-a (1000 mg, 2.594 mmol) in ethanol (20 mL), and the reaction mixture was stirred at 90 °C for 36 h. The mixture was concentrated, and the pH was adjusted to neutrality by adding an aqueous sodium bicarbonate solution. The mixture was diluted with water, extracted with ethyl acetate (20 mL × 3), dried over anhydrous sodium sulfate, filtered, and concentrated, and the residue was purified by a silica gel column (petroleum ether/ethyl acetate = 1/2) to give compound 11-b (1.15 g, 1.974 mmol, yield: 76.09%). ES-API: [M+H]⁺=582.2, 584.2.

Step 3: Compound 11-b (1.15 g, 1.974 mmol) was dissolved in trifluoroacetic acid (10 mL). The mixture was heated to 65 °C, stirred for 2 h, concentrated under reduced pressure, and diluted with ethyl acetate. The residue was adjusted to pH 7-8 with saturated ammonium bicarbonate and then extracted with ethyl acetate (20 mL × 3). The organic layers were combined, dried over anhydrous sodium sulfate, and filtered, and the filtrate was concentrated under reduced pressure and then purified by silica gel column chromatography (petroleum ether/ethyl acetate = 1/2) to give compound 11-c (780 mg, 1.379 mmol, yield: 69.87%). ES-API: [M+H]⁺=565.2, 567.2.

Step 4: Compound 11-c (780 mg, 1.379 mmol) was dissolved in dry N,N-dimethylformamide (5 mL), and cesium carbonate (1347.83 mg, 4.137 mmol) and iodoethane (1075.41 mg, 6.895 mmol) were sequentially added. The mixture was reacted with stirring at room temperature for 2 h under a nitrogen atmosphere. The mixture was extracted with ethyl acetate (20 mL × 3). The organic layers were combined, washed sequentially with water (60 mL) and saturated brine (60 mL), dried over anhydrous sodium sulfate, and filtered. The filtrate was concentrated under reduced pressure and then purified by silica gel column chromatography (petroleum ether/ethyl acetate = 1/2) to give compound 11-d (750 mg, 1.264 mmol, yield: 91.61%). ES-API: [M+H]⁺= 593.2, 595.2.

Step 5: Compound 11-d (750 mg, 1.264 mmol) was dissolved in dry tetrahydrofuran (10 mL). The mixture was cooled to 0 °C under an ice-water bath, and lithium aluminum hydride (3.159 mL, 3.159 mmol, a 1 M solution in tetrahydrofuran) was slowly added dropwise under a nitrogen atmosphere. After the dropwise addition was completed, the mixture was stirred at 0 °C for another 1 h. When the reaction was completed as detected by LCMS, water (0.12 mL), an aqueous sodium hydroxide solution (0.12 mL, 15%), and water (0.36 mL) were slowly added sequentially to quench the reaction. The suspension was stirred at room temperature for 30 min, filtered through celite, and washed with ethyl acetate (100 mL). The mother liquor was dried over anhydrous sodium sulfate, filtered, and concentrated, and the residue was then purified by a flash silica gel column (ethyl acetate/petroleum ether = 20%-60%) to give 2 intermediate compounds. The structure of one of the intermediate compounds was: 3-((Rₐ)-2-(5-(benzyloxy)-2-((1S)-1-methoxyethyl)pyridin-3-yl)-5-bromo-1-ethylindol-3-yl)-2,2-dimethylpropan-1-ol (11-e-1, 300 mg, 0.544 mmol, yield: 43.05%). 11-e-1: Rt=1.847 min(3 min LCMS);¹H NMR (400 MHz, DMSO-*d₆*) δ 8.48 (d, *J =* 2.8 Hz, 1H), 7.84 (d, *J =* 2.0 Hz, 1H), 7.45 - 7.30 (m, 7H), 7.25 - 7.20 (m, 1H), 5.28 - 5.17 (m, 2H), 4.46 (t, *J =* 5.2 Hz, 1H), 4.00 - 3.90 (m, 2H), 3.82 - 3.72 (m, 1H), 3.02 - 2.90 (m, 2H), 2.79 (s, 3H), 2.55 (d, *J =* 14.0 Hz, 1H), 2.09 (d, *J =* 14.0 Hz, 1H), 1.32 (d, *J =* 6.0 Hz, 3H), 1.00 (t, *J =* 7.2 Hz, 3H), 0.55 - 0.48 (m, 6H). ES-API: [M+H]⁺= 551.2, 553.2.

The structure of the other intermediate compound was: 3-((Sₐ)-2-(5-(benzyloxy)-2-((1S)-1-methoxyethyl)pyridin-3-yl)-5-bromo-1-ethylindol-3-yl)-2,2-dimethylpropan-1-ol (11-e-2, 210 mg, 0.381 mmol, yield: 30.13%). ES-API: [M+H]⁺= 551.2, 553.2. 11-e-2: Rt=1.758 min (3 min LCMS).

### Single-crystal culture of intermediate 11-e-1

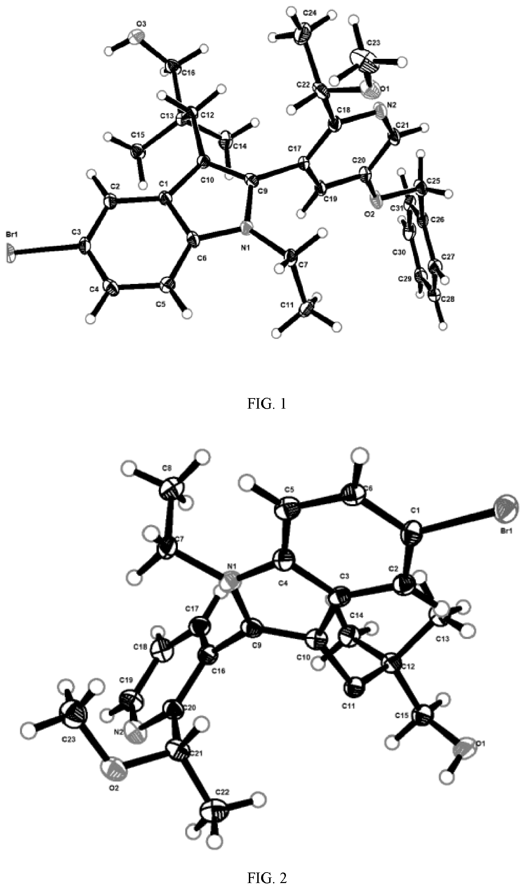

Single-crystal culture: About 10 mg of a powder sample of intermediate 11-e-1 was weighed out, and 1 mL of acetonitrile was added. The mixture was completely dissolved by ultrasonication and slowly volatilized in a fume hood. After a period of time, a yellowish-brown needle-like crystal, i.e., a single crystal of intermediate 11-e-1, was precipitated. The X-ray single-crystal diffraction test was performed using a Bruker D8 Venture instrument. The results are shown in Table 6 below and FIG. 1. FIG. 1 shows an ellipsoid plot of the molecular three-dimensional structure.

### Instrument parameters:

| | |
|---|---|
| Light source: Cu target | X-ray: Cu-Ka (= 1.54178 Å) |
| Detector: CMOS plane detector | Resolution: 0.80 Å |
| Current and voltage: 50 kV, 1.2 mA | Exposure time: 3 s |
| Area detector-to-sample distance: 40 mm | Test temperature: 170(2) K |

### Structure analysis and refinement procedures:

The diffraction data were integrated and reduced using the SAINT program, followed by empirical absorption correction using the SADABS program. The single crystal structure was solved by the direct method using SHELXT2014 and refined by the least square method. The hydrogen atom refinement process adopted isotropic calculation. The hydrogen atoms on N and O were located from residual electron density. The hydrogen atoms on C-H were geometrically added and refined using a riding model. The Flack constant was 0.035 (14). In FIG. 1, C22 is in S configuration, and the axial chirality at positions C9-C17 is in Rₐ configuration.

### Crystal data:

**Table 6**

| | |
|---|---|
| Molecular formula | C₃₀H₃₅BrN₂O₃ |
| Molecular weight | *Mᵣ* = 551.51 |
| Shape | Block, colorless |
| Crystal system | Orthorhombic |
| Space group | *P*2₁2₁2₁ |
| Unit cell parameters | *a* = 7.3026 (2) Å; *b* = 17.4557 (4) Å; *c* = 20.6871 (5) Å |
| Unit cell volume | *V* = 2637.03 (11) Å³ |
| Molecule number in unit cell | *Z* = 4 |
| Unit cell dimensions | 0.19 × 0.08 × 0.05 mm |
| Electron number in unit cell | *F*(000) = 1152 |
| Calculated density | *Dₓ* = 1.389 Mg m⁻³ |
| Radiation source and wavelength | Cu *K*a radiation, 1 = 1.54178 Å |
| Determination of the number of diffraction points of unit cell | Cell parameters from 9899 reflections |
| Range for data collection | q = 2.5-63.8° |
| Absorption coefficient | m = 2.39 mm⁻¹ |
| Temperature | *T* = 170 K |

Step 6: Compound 11-e-1 (18 g, 32.6 mmol), bis(pinacolato)diboron (16.6 g, 65.2 mmol), potassium acetate (6.41 g, 65.27 mmol), and Pd(dppf)Cl₂ (2.39 g, 3.26 mmol) were mixed in anhydrous toluene (200 mL) under a nitrogen atmosphere, and the mixture was stirred at 90 °C for 4 h. After the reaction was completed, the mixture was cooled and concentrated to remove toluene, and 300 mL of ethyl acetate and 500 mL of water were poured into the mixture. The resulting mixture was extracted 3 times with ethyl acetate and washed sequentially with 500 mL of water and 500 mL of saturated brine. The organic phase was dried over anhydrous sodium sulfate, filtered, and concentrated, and the residue was purified by a flash silica gel column (0-50% petroleum ether/ethyl acetate) to give intermediate 11 (20.8 g, yield: 85%). ES-API[M+H]⁺= 599.3.

### Preparation Example 11: Preparation of Intermediate 12

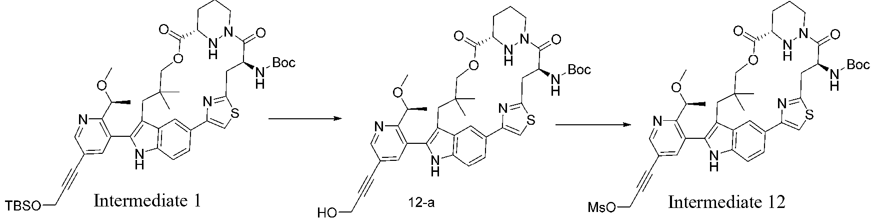

Step 1: A tetrabutylammonium fluoride solution (3.5 mL, 3.444 mmol) was added to a solution of intermediate 1 (1000 mg, 1.148 mmol) in tetrahydrofuran (15 mL) under an ice-water bath. The reaction mixture was stirred at 0 °C for 1 h. When the reaction was completed as detected by LCMS, the reaction mixture was diluted with 50 mL of ethyl acetate and then washed with saturated brine (15 mL × 3). The organic phase was dried over anhydrous sodium sulfate, filtered, and concentrated to give crude compound 12-a (868 mg, yield: 99%). ES-API: [M+H]⁺= 757.3.

Step 2: N,N-Diisopropylethylamine (1.0 mL, 5.734 mmol) and methanesulfonic anhydride (300 mg, 1.720 mmol) were sequentially added to a solution of compound 12-a (868 mg, 1.147 mmol) in dichloromethane (15 mL) under an ice-water bath. The mixture was stirred at room temperature for 1 h. When the reaction was completed as detected by LCMS, the reaction mixture was diluted with 20 mL of dichloromethane and then washed with water (10 mL) and saturated brine (15 mL × 3). The organic phase was dried over anhydrous sodium sulfate, filtered, and concentrated to dryness. The residue was purified by automated flash chromatography on silica gel (tetrahydrofuran/petroleum ether = 0-45%) to give intermediate 12 (957 mg, yield: 99%). ES-API: [M+H]⁺= 835.2.

### Preparation Example 12: Preparation of Intermediate 13

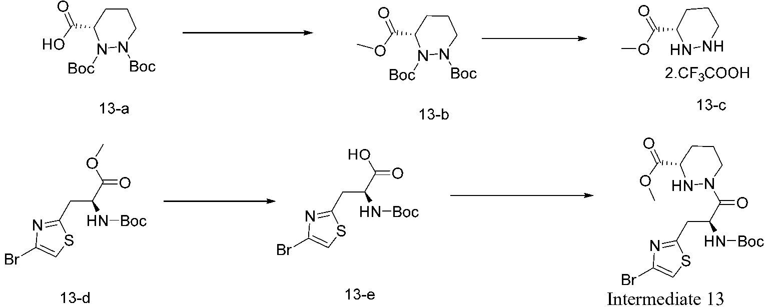

Step 1: Compound 13-a (60 g, 181.609 mmol) was dissolved in DMF (200 mL). Sodium bicarbonate (45.77 g, 544.827 mmol) was added, and iodomethane (33.918 mL, 544.827 mmol) was added dropwise under an ice-water bath and a nitrogen atmosphere. The mixture was reacted overnight at room temperature. Water (200 mL) was added, and the mixture was extracted with ethyl acetate (200 mL × 3), washed with water (200 mL) and saturated brine (200 mL × 3), dried over anhydrous sodium sulfate, and concentrated under reduced pressure to give compound 13-b (65 g, 188.729 mmol, yield: 103.92%) as an oily liquid. ES-API [M-156+H]⁺=189.1.

Step 2: Compound 13-b (65 g, 188.729 mmol) was dissolved in dichloromethane (200 mL), and trifluoroacetic acid (200 mL) was added dropwise under an ice-water bath and a nitrogen atmosphere. The mixture was reacted overnight at room temperature and concentrated under reduced pressure to give crude compound 13-c (ditrifluoroacetate) as a colorless transparent oily liquid. The product was directly used in the next step. ES-API[M+H]⁺=145.1.

Step 3: Compound 13-d (50 g, 136.896 mmol) was dissolved in tetrahydrofuran (300 mL) and water (75 mL), and lithium hydroxide monohydrate (9.38 g, 223.415 mmol) was added. The mixture was reacted at room temperature for 4 h. The reaction mixture was adjusted to pH 3 with 1 N HCl, extracted with ethyl acetate (200 mL × 3), washed with water (200 mL × 3) and saturated brine (200 mL), dried over anhydrous sodium sulfate, and concentrated under reduced pressure to give compound 13-e (48 g, 136.670 mmol, yield: 99.83%) as a colorless transparent oily liquid. ES-API [M-56+H]⁺=295.0.

Step 4: Compound 13-c was dissolved in anhydrous dichloromethane (200 mL). 4-Methylmorpholine (100 g, 996 mmol) was added dropwise under an ice-water bath and a nitrogen atmosphere, and compound 13-e (35 g, 99.655 mmol), EDCI (38.07 g, 199.311 mmol), and HOBt (4.04 g, 29.897 mmol) were sequentially added. The mixture was reacted at room temperature for 4 h, washed sequentially with water (200 mL), 1 N HCl (80 mL), and saturated brine (200 mL), dried over anhydrous sodium sulfate, concentrated under reduced pressure, and triturated (petroleum ether/ethyl acetate = 8/1) to give intermediate 13 (33 g, 69.129 mmol). The mother liquor was then purified by a flash silica gel column to give intermediate 13 (4 g, 8.34 mmol). Intermediate 13 (in a total amount of 37 g, 77.5 mmol, yield: 78.7%) was obtained as a white solid. ES-API [M+H]⁺=477.1.

### Preparation Example 13: Preparation of Intermediate 14

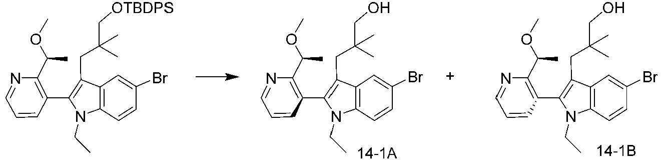

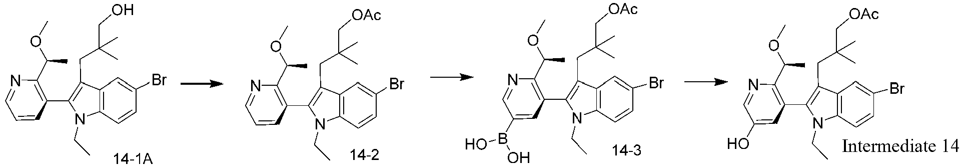

Step 1: (S)-5-Bromo-3-(3-((tert-butyldiphenylsilyl)oxy)-2,2-dimethylpropyl)-1-ethyl-2-(2-(1-methoxyethyl)pyridin-3-yl)-1H-indole (8800 mg, 12.907 mmol, see step 8 of intermediate 1 in WO2022060836A1 for the preparation method) was dissolved in a solution (1 M, 60 mL) of tetrabutylammonium fluoride in tetrahydrofuran. The reaction system was stirred at 50 °C for 17 h under a nitrogen atmosphere. After the reaction was completed, water (200 mL) was added to the solution described above, and the mixture was extracted with ethyl acetate (100 mL × 2). The organic phase was washed with saturated brine (200 mL × 5), dried over anhydrous sodium sulfate, filtered, and concentrated to give a crude product. The crude product was purified by a flash silica gel column (ethyl acetate/petroleum ether: 0%-70%) to give (S)-3-(5-bromo-1-ethyl-(Rₐ)-2-(2-(1-methoxyethyl)pyridin-3-yl)-1H-indol-3-yl)-2,2-dimethylpropan-1-ol (compound 14-1A, 2600 mg, 5.864 mmol, yield: 45.43%, Rf = 0.43) as a yellow oily liquid. ES-API: [M +H]⁺=445.2. (S)-3-(5-Bromo-1-ethyl-(Sₐ)-2-(2-(1-methoxyethyl)pyridin-3-yl)-1H-indol-3-yl)-2,2-dimethylpropan-1-ol (compound 14-1B, 2600 mg, 5.864 mmol, yield: 45.43%, Rf = 0.40) as a yellow oily liquid. ES-API: [M +H]⁺=445.2.

Step 2: Compound 14-1A (2500 mg, 5.639 mmol), triethylamine (1.567 mL, 11.277 mmol), and 4-dimethylaminopyridine (68.89 mg, 0.564 mmol) were dissolved in dichloromethane (40 mL). Acetic anhydride (6.355 mL, 67.662 mmol) was slowly added to the solution described above under an ice bath. After the addition was completed, the reaction system was warmed to room temperature and stirred for another 2 h. After the reaction was completed, the reaction mixture was concentrated. Then water (100 mL) was added to the residue, and the mixture was extracted with dichloromethane (100 mL × 2). The organic phase was washed sequentially with a saturated aqueous sodium bicarbonate solution (100 mL × 2) and a saturated sodium chloride solution (200 mL), dried over anhydrous sodium sulfate, filtered, and concentrated to give a crude product. The crude product was purified by a flash silica gel column (ethyl acetate/petroleum ether: 0%-40%) to give compound 14-2 (2600 mg, 5.356 mmol, yield: 94.99%) as a yellow solid. ES-API: [M +H]⁺=487.1.

Step 3: Compound 14-2 (2500 mg, 5.150 mmol), bis(pinacolato)diboron (2223.32 mg, 8.755 mmol), chloro(1,5-cyclooctadiene)iridium(I) dimer (221.17 mg, 0.824 mmol), and 4,4'-di-tert-butyl-2,2'-bipyridine (50.32 mg, 0.187 mmol) were dissolved in n-hexane (45 mL). The reaction system was stirred at 80 °C for 17 h under a nitrogen atmosphere. After the reaction was completed, the reaction system was concentrated to remove n-hexane to give crude compound 14-3 (5000 mg) as a black oily liquid. ES-API: [M +H]⁺=531.5.

Step 4: Compound 14-3 (5000 mg) was dissolved in acetonitrile (30 mL), and hydrogen peroxide (1750.62 mg, 30 wt%, 15.447 mmol) was added to the solution described above under an ice bath. After the addition was completed, the reaction system was warmed to room temperature and stirred for 3 h. After the reaction was completed, the reaction was quenched with saturated sodium sulfite (30 mL). The mixture was extracted with ethyl acetate (50 mL × 2). The organic phase was washed with saturated brine (50 mL × 2), dried over anhydrous sodium sulfate, filtered, and concentrated to give a crude product. The crude product was purified by a flash silica gel column (methanol/dichloromethane: 0%-4%) to give intermediate 14 (1700 mg, 3.390 mmol, two-step yield: 65.8%) as a yellow solid. ES-API: [M +H]⁺= 503.2.

### Single-crystal culture of compound 14-1A

Single-crystal culture: 30 mg of intermediate 14-1A was dissolved in 0.5 mL of chloroform, and 1.5 mL of n-hexane was added. The mixture was shaken until it was clear. The solution was filtered and sealed. Several small holes were made in the sealed part by using a needle. After slow evaporation for 24 h, a single crystal was grown and obtained. The X-ray single-crystal diffraction test was performed using a Bruker D8 Venture instrument. The results are shown in Table 7 below and FIG. 2. FIG. 2 shows an ellipsoid plot of the molecular three-dimensional structure.

### Instrument parameters:

| | |
|---|---|
| Light source: Cu target | X-ray: Cu-Kα (λ = 1.54178 Å) |
| Detector: CMOS area detector | Resolution: 0.80 Å |
| Current and voltage: 50 kV, 1.2 mA | Exposure time: 10 s |
| Area detector-to-sample distance: 40 mm | Test temperature: 100(2) K |

### Structure analysis and refinement procedures:

The diffraction data were integrated and reduced using the SAINT program, followed by empirical absorption correction using the SADABS program. The single crystal structure was solved by the direct method using SHELXT2014 and refined by the least square method. The hydrogen atom refinement process adopted isotropic calculation. The hydrogen atoms on C-H were geometrically added and refined using a riding model. The Flack constant was 0.043 (10). In FIG. 2, C21 is in S configuration, and the axial chirality at positions C9-C16 is in Rₐ configuration.

### Crystal data:

**Table 7**

| | |
|---|---|
| Molecular formula | 0.09(C₂₃H₂₇BrN₂O₂) |
| Molecular weight | *Mᵣ* = 41.24 |
| Shape | Block, colorless |
| Crystal system | Orthorhombic |
| Space group | *P*2₁2₁2₁ |
| Unit cell parameters | *a* = 7.7650 (2) Å;b = 10.7737 (3) Å; c = 25.6701 (6) Å |
| Unit cell volume | *V* = 2147.50 (10) Å³ |
| Molecule number in unit cell | *Z* = 43 |
| Unit cell dimensions | *0.15* × *0.08* × *0.05 mm* |
| Electron number in unit cell | *F*(000) = 920 |
| Calculated density | *D*ₓ = 1.371 Mg m⁻³ |
| Radiation source and wavelength | Cu *K*a radiation, 1 = 1.54178 Å |
| Determination of the number of diffraction points of unit cell | Cell parameters from 9839 reflections |
| Range for data collection | q = 4.1-72.3° |
| Absorption coefficient | m = 2.76 mm⁻¹ |
| Temperature | *T= 100 K* |

### Preparation Example 14: Preparation of Intermediate 38-INT

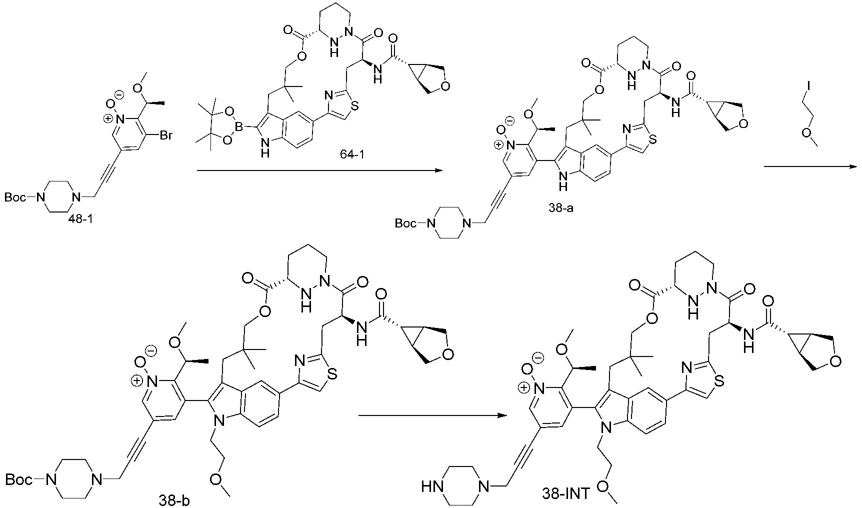

Step 1: Compound 64-1 (300 mg, 0.432 mmol) and compound 48-1 (265 mg, 0.648 mmol) were dissolved in dioxane (2 mL), toluene (6 mL), and water (2 mL) under a nitrogen atmosphere, and [1,1'-bis(di-tert-butylphosphino)ferrocene]dichloropalladium(II) (63 mg, 0.097 mmol) and potassium phosphate (205 mg, 0.97 mmol) were added. The reaction system was stirred at 75 °C for 3 h. Ethyl acetate (50 mL) was added to the reaction mixture, and the mixture was washed with saturated brine (15 mL). The organic phase was dried over anhydrous sodium sulfate and filtered, and the filtrate was concentrated under reduced pressure. The crude product was purified by a flash silica gel column (methanol/dichloromethane: 0-8%) to give compound 38-a (280 mg, yield: 61.0%) as a pale brown solid. ES-API:[M+H]⁺= 951.3.

Step 2: Compound 38-a (280 mg, 0.294 mmol) was dissolved in N,N-dimethylformamide (10 mL), and cesium carbonate (288 mg, 0.883 mmol) and 1-iodo-2-methoxyethane (164 mg, 0.883 mmol) were added at room temperature. The reaction system was stirred at room temperature for another 48 h. Water (15 mL) was added to the reaction mixture, and the mixture was extracted with ethyl acetate (50 mL). The organic phase was washed with saturated brine (20 mL × 3), dried over anhydrous sodium sulfate, and filtered, and the filtrate was concentrated under reduced pressure. The crude product was purified by a flash silica gel column (methanol/dichloromethane: 0-10%) to give compound 38-b (210 mg, yield: 70.7%) as a pale yellow solid. ES-API:[M+H]⁺= 1009.3.

Step 3: Compound 38-b (35 mg, 0.035 mmol) was dissolved in methanol (0.2 mL), and a 4.0 M hydrogen chloride/dioxane solution (1.5 mL) was added at 0 °C. The reaction system was stirred at room temperature for 1 h. The reaction mixture was concentrated to give compound 38-INT (31 mg, yield: 98.3%) as a yellow solid. ES-API: [M+H]⁺= 909.3.

### Preparation Example 15: Preparation of Intermediate 54-INT

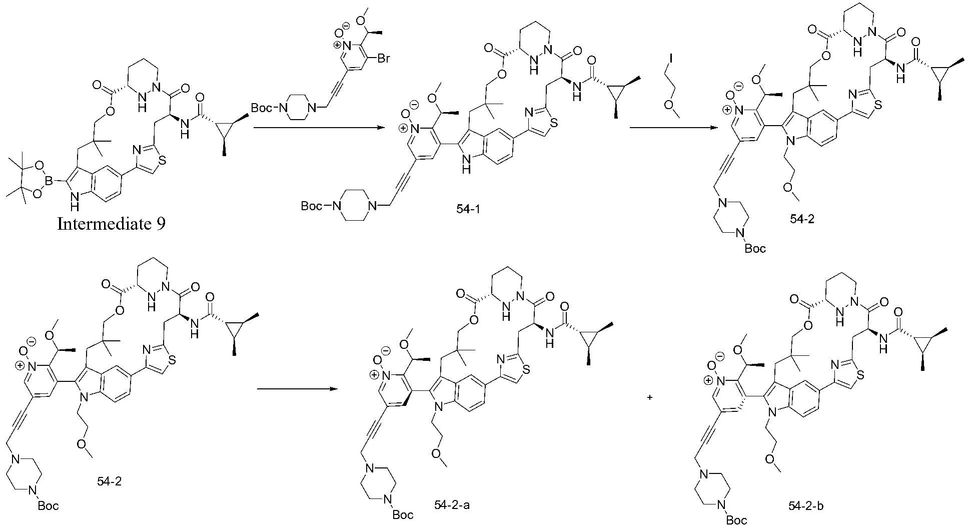

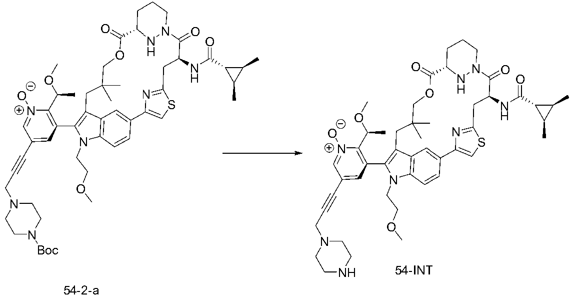

Step 1: Intermediate 9 (850 mg, 1.23 mmol), (S)-3-bromo-5-(3-(4-(tert-butoxycarbonyl)piperazin-1-yl)prop-1-yn-1-yl)-2-(1-methoxyethyl)pyridine-1-oxide (48-1, 840 mg, 1.85 mmol), [1,1'-bis(di-tert-butylphosphino)ferrocene]dichloropalladium(II) (80 mg, 0.12 mmol), potassium phosphate (784 mg, 0.69 mmol), tetrahydrofuran (9 mL), toluene (6 mL), and water (3 mL) were added to a flask. The reaction mixture was purged 3 times with nitrogen and stirred at 65 °C for 3 h. Water was added to the reaction mixture, and the mixture was extracted with ethyl acetate (50 mL × 3). The organic phase was washed with saturated brine, dried over anhydrous sodium sulfate, and filtered. The filtrate was concentrated, and the crude product was purified by a flash silica gel column (methanol/dichloromethane: 0-6%) to give compound 54-1 (800 mg, yield: 69.3%) as a yellow solid. ES-API: [M+H]⁺=937.3.

Step 2: Compound 54-1 (800 mg, 0.85 mmol), 1-iodo-2-methoxyethane (476.3 mg, 2.56 mmol), cesium carbonate (556.26 mg, 1.71 mmol), acetonitrile (3 mL), and N,N-dimethylformamide (3 mL) were added to a flask. The reaction system was stirred at 25 °C for 24 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-3%) to give compound 54-2 (750 mg, yield: 88.3%) as a yellow solid. ES-API: [M+H]⁺=995.3.

Step 3: Compound 54-2 (500 mg, 0.50 mmol) was purified by preparative chiral chromatography (separation column: Daicel CHIRALPAK^{®} IH 250 × 4.6 mm × 5 µm; mobile phase: A acetonitrile + 0.2% diethylamine, B (methanol:isopropanol = 1:1) + 0.2% diethylamine; A:B = 80:20; flow rate: 1 mL/min; column temperature: 30 °C) to give 2 isomeric compounds. The structure of one of the isomeric compounds was 5-(3-(4-(tert-butoxycarbonyl)piperazin-1-yl)prop-1-yn-1-yl)-3-((6³S,4S,Z)-4-((1r,2R,3S))-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-1¹H-8-oxo-2(4,2)-thiazola-1(5,3)-indola-6(1,3)-pyridazinacycloundecaphane-1²-yl)-2-((S)-1-methoxyethyl)pyridine-1-oxide (54-2-a, 163 mg, yield: 32.8%, retention time: 3.72 min) as a white solid. ES-API: [M+H]⁺= 995.3. The structure of the other isomeric compound was 5-(3-(4-(tert-butoxycarbonyl)piperazin-1-yl)prop-1-yn-1-yl)-3-((6³S,4S,Z)-4-((1r,2R,3S))-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-1¹H-8-oxo-2(4,2)-thiazola-1(5,3)-indola-6(1,3)-pyridazinacycloundecaphane-1²-yl)-2-((S)-1-methoxyethyl)pyridine-1-oxide (54-2-b, 168 mg, yield: 33.6%, retention time: 5.106 min) as a white solid. ES-API: [M+H]⁺= 995.3.

Step 4: Compound 54-2-a (163 mg, 0.16 mmol), methanol (1 mL), and dichloromethane (1 mL) were added to a flask. A 4 M hydrogen chloride/dioxane solution (3 mL, 12.00 mmol) was added dropwise thereto. The reaction system was stirred at room temperature for 1 h. The reaction mixture was concentrated to give crude compound 54-INT (152 mg). ES-API: [M+H]⁺= 895.4.

### Preparation Example 16: Preparation of Intermediate 63-INT

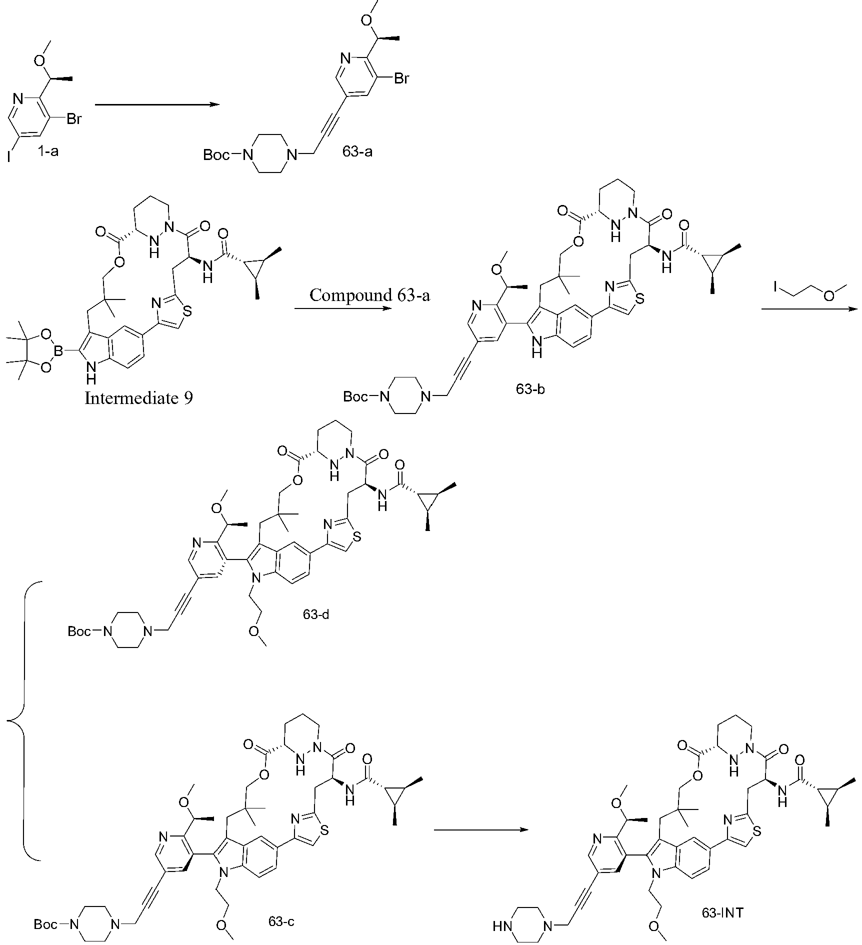

Step 1: 2-Methylpropan-2-yl 4-(prop-2-ynyl)piperazine-1-carboxylate (1.80 g, 8.025 mmol) and N,N-dimethylethylamine (4.205 mL, 24.075 mmol) were added to a solution of compound 1-a (3.0 g, 8.773 mmol), bis(triphenylphosphine)palladium(II) dichloride (0.28 g, 0.401 mmol), and copper(I) iodide (0.23 g, 1.204 mmol) in anhydrous acetonitrile (15 mL) under an ice-water bath, and the reaction mixture was stirred at room temperature for 12 h. The mixture was diluted with ethyl acetate (100 mL), washed with saturated brine (30 mL), dried over anhydrous sodium sulfate, filtered, and concentrated. The residue was purified by a silica gel column (washed with 0-50% tetrahydrofuran/petroleum ether) to give compound 63-a (3.0 g, 6.844 mmol, yield: 85.28%) as a yellow solid.

Step 2: Intermediate 9 (300.0 mg, 0.435 mmol) and compound 63-a (300.0 mg, 0.684 mmol) were dissolved in toluene (12.0 mL), 1,4-dioxane (4.0 mL), and water (4.0 mL), and potassium phosphate (128.0 mg, 0.605 mmol) and [1,1'-bis(di-tert-butylphosphino)ferrocene]dichloropalladium(II) (27.0 mg, 0.040 mmol) were added. The mixture was reacted at 70 °C for 5 h under a nitrogen atmosphere. After the reaction was completed, the mixture was concentrated under reduced pressure, and the residue was purified by silica gel column chromatography (dichloromethane:methanol = 10:1) to give compound 63-b (330.0 mg, yield: 82.31%). ES-API: [M+H]⁺=921.4. Step 3: Compound 63-b (330.0 mg, 0.358 mmol) was dissolved in N,N-dimethylformamide (10.0 mL), and cesium carbonate (0.33 g, 1.010 mmol) and 1-iodo-2-methoxyethane (200.0 mg, 1.075 mmol) were added. The mixture was reacted at room temperature for 2 h. After the reaction was completed, water (20 mL) was added, and the mixture was extracted with ethyl acetate (50 mL × 3). The organic phase was washed with saturated brine (20 mL), dried over anhydrous sodium sulfate, concentrated under reduced pressure, and resolved by an SFC chiral column [CHIRALPAK^{®} IB 250 × 30 mm, 10 µm; mobile phase A: HEX + 0.2% DEA, mobile phase B: EtOH + 0.2% DEA; detection wavelength: 214 nm/254 nm; flow rate: 25 mL/min; injection volume: 10 µL; column temperature: 30 °C; run time: 15 min; isocratic elution program: mobile phase A:mobile phase B = 70:30 (V/V)] to give compound 63-c (59 mg, yield: 20%, retention time: 12.5 min) (ES-API: [M+H]⁺ = 979.3) and compound 63-d (251.0 mg, yield: 80%, retention time: 9.9 min). ES-API: [M+H]⁺=979.3.

Step 4: A hydrochloric acid/methanol solution (1.0 mL, 4 M, 4.0 mmol) was added to a solution of compound 63-c (59 mg, 0.060 mmol) in methanol (1.0 mL). The mixture was stirred at room temperature for 1 h and then concentrated to dryness by rotary evaporation under reduced pressure to remove the solvent to give (2R,3S)-N-((6³S,4S,Z)-1¹-(2-methoxyethyl)-1²-(2-((S)-1-methoxyethyl ester)-5-(3-(piperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (63-INT, 62.0 mg). ES-API: [M+H]⁺=879.4.

### Preparation Example 17: Preparation of Intermediate 31-INT

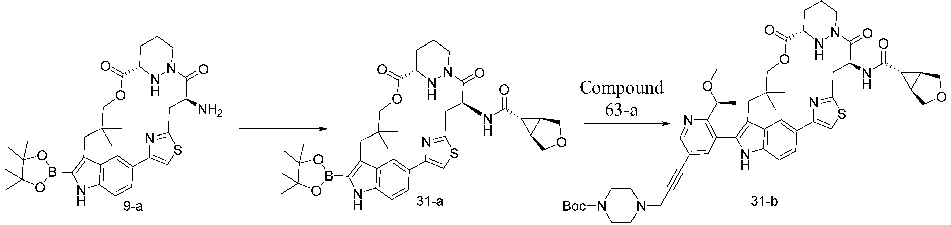

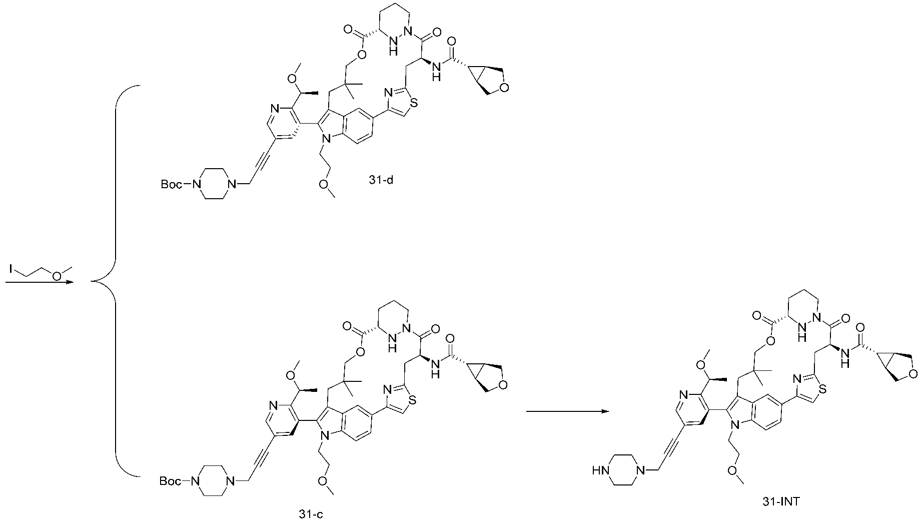

Step 1: Compound 9-a (350.0 mg, 0.590 mmol) was dissolved in N,N-dimethylformamide (5.0 mL), and (1R,5S)-3-oxabicyclo[3.1.0]hexane-6-carboxylic acid (113.0 mg, 0.885 mmol), O-(7-azabenzotriazol-1-yl)-N,N,N',N'-tetramethyluronium hexafluorophosphate (673.0 mg, 1.769 mmol), and *N,N*'-diisopropylethylamine (0.50 mL, 3.0 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried over anhydrous sodium sulfate, filtered, and concentrated to give a crude product. The crude product was purified by a flash silica gel column [dichloromethane/methanol = 100:0 to 90:10 (V/V)] to give compound 31-a (450.0 mg, crude product) as a white solid. ES-API: [M +H]⁺=704.3.

Step 2: Compound 31-a (450.0 mg, crude product) and compound 63-a (450.0 mg, 1.027 mmol) were dissolved in toluene (12.0 mL), 1,4-dioxane (4.0 mL), and water (4.0 mL), and potassium phosphate (128.0 mg, 0.605 mmol) and [1,1'-bis(di-tert-butylphosphino)ferrocene]dichloropalladium(II) (27.0 mg, 0.040 mmol) were added. The mixture was reacted at 70 °C for 5 h under a nitrogen atmosphere. After the reaction was completed, the mixture was concentrated under reduced pressure, and the resulting crude product was purified by silica gel column chromatography (dichloromethane:methanol = 10:1) to give compound 31-b (290.0 mg, yield: 52.56%). ES-API: [M+H]⁺=935.3.

Step 3: Compound 31-b (290.0 mg, 0.310 mmol) was dissolved in N,N-dimethylformamide (10.0 mL), and cesium carbonate (0.33 g, 1.010 mmol) and 1-iodo-2-methoxyethane (200.0 mg, 1.075 mmol) were added. The mixture was reacted at room temperature for 2 h. After the reaction was completed, water (20 mL) was added, and the mixture was extracted with ethyl acetate (50 mL × 3). The organic phase was washed with saturated brine (20 mL), dried over anhydrous sodium sulfate, concentrated under reduced pressure, and resolved by an SFC chiral column [CHIRALPAK^{®} IB 250 × 30 mm, 10 µm; mobile phase A: HEX + 0.2% DEA, mobile phase B: ETOH + 0.2% DEA; detection wavelength: 214 nm/254 nm; flow rate: 25 mL/min; injection volume: 10 µL; column temperature: 30 °C; run time: 15 min; isocratic elution program: mobile phase A:mobile phase B = 70:30 (V/V)] to give compound 31-c (54 mg, yield: 20.4%, retention time: 11.5 min) (ES-API: [M+H]⁺ = 979.3) and compound 31-d (201 mg, yield: 77.0%, retention time: 4.5 min) (ES-API: [M+H]⁺ = 993.3).

Step 4: A hydrochloric acid/methanol solution (1.0 mL, 4 M, 4.0 mmol) was added to a solution of compound 31-c (54.0 mg, 0.054 mmol) in methanol (1.0 mL). The mixture was stirred at room temperature for 1 h and then concentrated to dryness by rotary evaporation under reduced pressure to remove the solvent to give (1R,5S)-N-((6³S,4S,Z)-1¹-(2-methoxyethyl)-1²-(2-((S)-1-methoxyethyl ester)-5-(3-(piperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (31-INT, 58.0 mg). ES-API: [M+H]⁺=893.3.

### Preparation Example 18: Preparation of Intermediate 35-INT

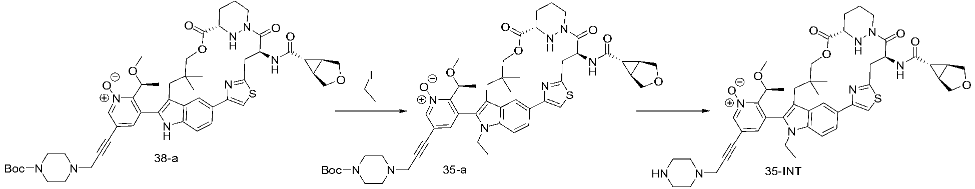

Step 1: Compound 38-a (150 mg, 0.158 mmol) was dissolved in N,N-dimethylformamide (5 mL), and cesium carbonate (206 mg, 0.631 mmol) and iodoethane (98 mg, 0.631 mmol) were added at room temperature. The reaction system was stirred at room temperature for another 18 h. Water (15 mL) was added to the reaction mixture, and the mixture was extracted with ethyl acetate (50 mL). The organic phase was washed with saturated brine (20 mL × 3), dried over anhydrous sodium sulfate, and filtered, and the filtrate was concentrated under reduced pressure. The crude product was purified by a flash silica gel column (methanol/dichloromethane: 0-5%) to give compound 35-a (110 mg, yield: 71.2%) as a pale yellow solid. ES-API:[M+H]⁺= 979.3.

Step 2: Compound 35-a (110 mg, 0.112 mmol) was dissolved in methanol (0.4 mL), and a 4.0 M hydrogen chloride/dioxane solution (3 mL) was added at 0 °C. The reaction system was stirred at room temperature for 1 h. The reaction mixture was concentrated to give compound 35-INT (98 mg, yield: 99.2%) as a yellow solid. ES-API: [M+H]⁺= 879.3.

### Preparation Example 19: Preparation of Intermediate 15

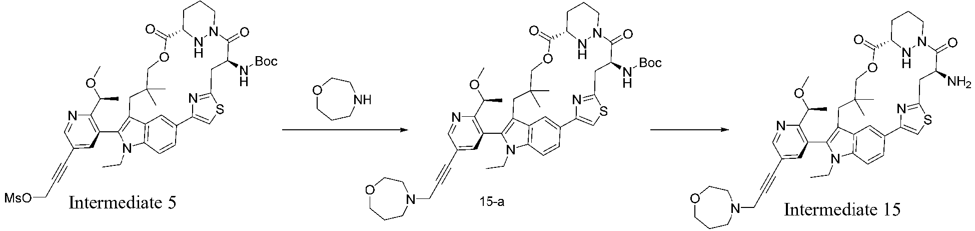

Step 1: 1,4-Oxazepane (1.68 g, 16.650 mmol) was added to a solution (50 mL) of intermediate 5 (4.79 g, 5.550 mmol) in dichloromethane. The reaction mixture was stirred at room temperature for 2 h. When the reaction was completed as detected by LCMS, the mixture was concentrated, and the crude product was purified by automated flash chromatography on silica gel (methanol/dichloromethane = 0-6%) to give compound 15-a (4.29 g, yield: 89.04%). ES-API: [M+H]⁺= 868.4.

Step 2: Hydrochloric acid (20 mL, a 4 M solution in dioxane) was added to a solution of compound 15-a (4.29 g, 4.942 mmol) in methanol (5 mL) under an ice-water bath. The mixture was reacted at room temperature for 1 h and concentrated to give a hydrochloride of intermediate compound 15 (3.80 g). ES-API: [M+H]⁺= 768.4. Preparation Example 20: Preparation of Intermediate 38-1a

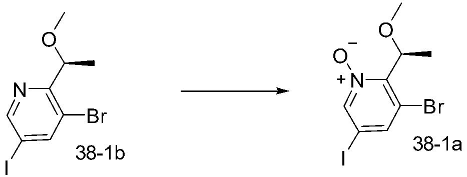

Step 1: Compound 38-1b (1.0 g, 2.92 mmol) was dissolved in dichloromethane (20 mL), and m-chloroperoxybenzoic acid (1.19 g, 5.85 mmol) was added under an ice bath. The reaction system was stirred at room temperature for 18 h. Dichloromethane (50 mL) was added to the reaction mixture, and the mixture was washed sequentially with saturated sodium thiosulfate (20 mL), saturated sodium bicarbonate (30 mL), and saturated brine (30 mL). The organic phase was dried over anhydrous sodium sulfate and filtered, and the filtrate was concentrated under reduced pressure. The crude product was purified by a flash silica gel column (methanol/dichloromethane: 0-2%) to give compound 38-1a (600 mg, yield: 57.3%) as a pale yellow liquid. ES-API:[M+H]⁺= 358.0, 359.9.

### Preparation Example 21. Synthesis of Intermediate 7

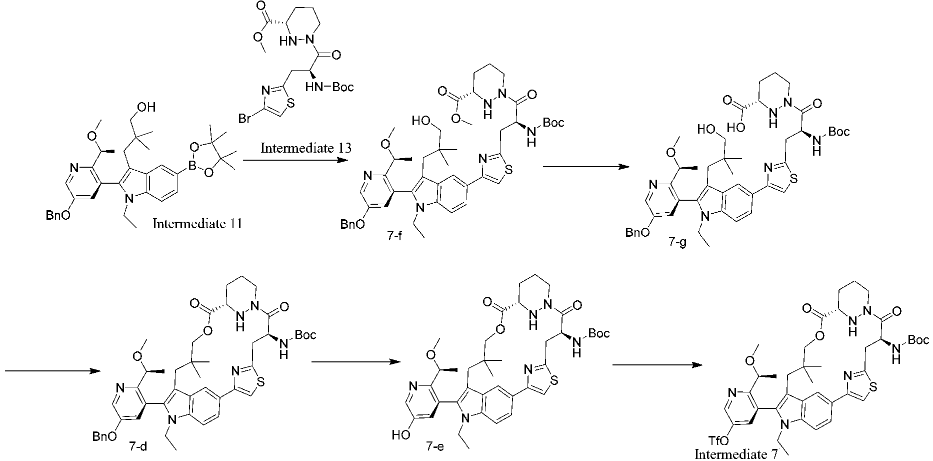

Step 1: Intermediate 11 (24 g, 40.09 mmol), intermediate 13 (22.97 g, 48.11 mmol), K₃PO₄ (17.05 g, 80.19 mmol), and Pd(dtbpf)Cl₂ (2.07 g, 3.21 mmol) were mixed in a mixed solution of toluene (50 mL), dioxane (150 mL), and water (50 mL) under a nitrogen atmosphere, and the mixture was stirred at 70 °C for 18 h. After the reaction was completed, 300 mL of ethyl acetate and 300 mL of water were poured into the mixture. The resulting mixture was extracted 3 times with ethyl acetate and washed sequentially with 300 mL of water and 300 mL of saturated brine. The organic phase was dried over anhydrous sodium sulfate, filtered, and concentrated, and the residue was purified by a flash silica gel column (0-50% petroleum ether/ethyl acetate) to give compound 7-f (28 g, yield: 80%). ES-API[M+H]⁺= 869.3.

Step 2: Water (50 mL) and LiOH·H₂O (2.71 g, 64.435 mmol) were added to a solution of compound 7-f (28 g, 32.218 mmol) in tetrahydrofuran (200 mL) at 0 °C. The mixture was stirred at 0 °C for 1 h. After the reaction was completed, the reaction mixture was adjusted to pH 3-4 with 1 M hydrochloric acid, and 200 mL of ethyl acetate and 200 mL of water were poured into the reaction mixture. The mixture was extracted 3 times with ethyl acetate and washed sequentially with 200 mL of water and 200 mL of saturated brine. The organic phase was dried over anhydrous sodium sulfate, filtered, and concentrated to give compound 7-g (28 g, crude product). ES-API[M+H]⁺= 855.3.

Step 3: HOBt (39.51 g, 292.377 mmol) and DIPEA (178.254 mL, 1023.320 mmol) were added to a solution of compound 7-g (25 g, 29.3 mmol) in dichloromethane (1000 mL), and EDCI (168.15 g, 877.131 mmol) was slowly added at 0 °C. The mixture was stirred at room temperature for 16 h. After the reaction was completed, the reaction mixture was adjusted to pH 5-6 with 1 M hydrochloric acid, and 200 mL of ethyl acetate and 200 mL of water were poured into the mixture. The resulting mixture was extracted 3 times with ethyl acetate and washed sequentially with 200 mL of water and 200 mL of saturated brine. The organic phase was dried over anhydrous sodium sulfate, filtered, and concentrated, and the residue was purified by a flash silica gel column (0-80% petroleum ether/ethyl acetate) to give compound 7-d (17.1 g, purity: 86%, yield: 60%). ES-API[M+H]⁺= 837.3.

Step 4: Pd/C 10% (7.12 g, 6.690 mmol) was added to a solution of compound 7-d (14 g, 16.725 mmol) in methanol (350 mL), and the mixture was reacted at room temperature for 40 h under a hydrogen atmosphere. The reaction mixture was filtered and concentrated to give compound 7-e (12 g, purity: 90%, yield: 87%). ES-API[M+H]⁺= 747.3.

Step 5: Triethylamine (2.791 mL, 20.082 mmol) and N-phenyl-bis(trifluoromethanesulfonimide) (3.59 g, 10.041 mmol) were added to a solution of compound 7-e (5 g, 6.694 mmol) in dichloromethane (60 mL), and the mixture was stirred at room temperature for 1 h. After the reaction was completed, 50 mL of ethyl acetate and 50 mL of water were poured into the mixture. The resulting mixture was extracted 3 times with ethyl acetate and washed sequentially with 50 mL of water and 50 mL of saturated brine. The organic phase was dried over anhydrous sodium sulfate, filtered, and concentrated, and the residue was purified by a flash silica gel column (0-60% petroleum ether/ethyl acetate) to give intermediate 7 (5 g, purity: 91%, yield: 77%). ES-API[M+H]⁺= 879.3.

### Example 1.1. Synthesis of Compound Z3

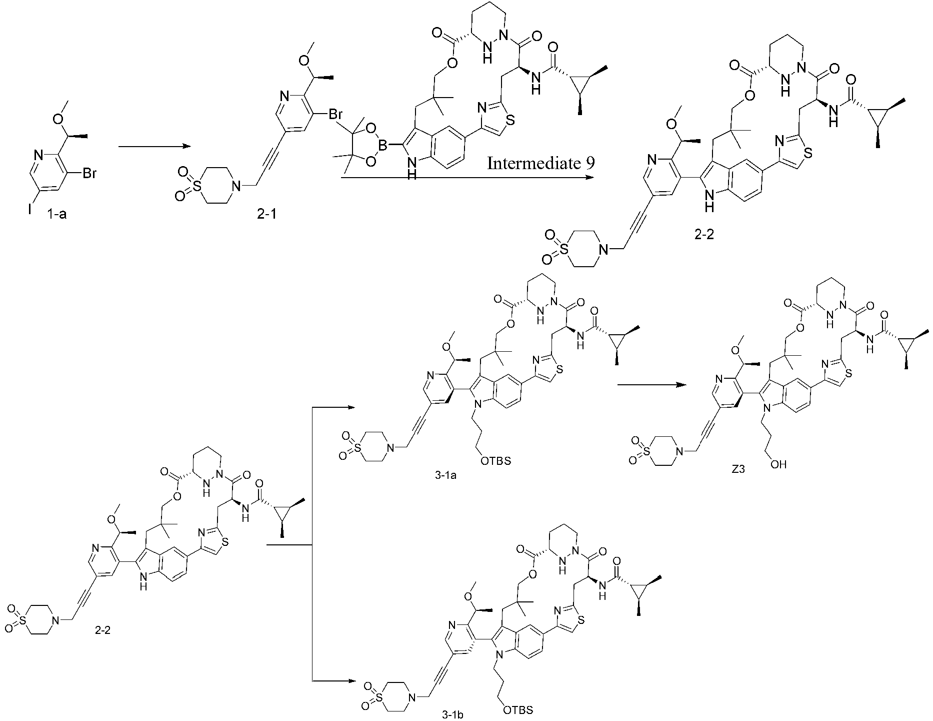

Step 1: Compound 1-a (340 mg, 1 mmol) and 4-(prop-2-ynyl)-1^{λ}6-1,4-thiazine-1,1-dione (345 mg, 2 mmol, CAS: 10442-03-2) were dissolved in acetonitrile (5 mL), and bis(triphenylphosphine)palladium(II) dichloride (140 mg, 0.2 mmol), copper(I) iodide (75 mg, 0.394 mmol), N,N-diisopropylethylamine (0.5 mL, 2.98 mmol), and anhydrous lithium chloride (170 mg, 4.010 mmol) were added. The mixture was stirred at 80 °C for 2 h under a nitrogen atmosphere, then filtered under vacuum, and concentrated to dryness under reduced pressure. The crude product was purified by a flash silica gel column (ethyl acetate/petroleum ether: 0%-50%) to give compound 2-1 (250 mg, 0.646 mmol, yield: 64.93%) as a yellow solid. ES-API: [M +H]⁺=387.0/389.0.

Step 2: Intermediate 9 (230 mg, 0.333 mmol), compound 2-1 (200 mg, 0.516 mmol), [1,1'-bis(di-tert-butylphosphino)ferrocene]dichloropalladium(II) (25 mg, 0.038 mmol), and potassium phosphate (220 mg, 1.036 mmol) were dissolved in toluene (3 mL), 1,4-dioxane (1 mL), and water (1 mL). The reaction system was stirred at 70 °C for 1 h under a nitrogen atmosphere. After the reaction was completed, water (30 mL) was added to the solution described above, and the mixture was extracted with ethyl acetate (20 mL × 2). The organic phase was washed with saturated brine (50 mL), dried, and concentrated to give a crude product. The crude product was purified by a flash silica gel column (methanol/dichloromethane: 0%-3%) to give compound 2-2 (200 mg, 0.230 mmol, yield: 68.93%) as a yellow solid. ES-API: [M +H]⁺=870.3.

Step 3: Compound 2-2 (100 mg, 0.115 mmol) was dissolved in N,N-dimethylformamide (3 mL), and anhydrous cesium carbonate (112.34 mg, 0.345 mmol) was added. The mixture was stirred at room temperature for 1 h, and then tert-butyl(3-iodopropoxy)dimethylsilane (98.69 mg, 0.345 mmol) was added. The reaction system was stirred at 50 °C for 4 h under a nitrogen atmosphere. After the reaction was completed, water (10 mL) was added to the reaction mixture, and the mixture was extracted with ethyl acetate (10 mL × 2). The organic phase was washed with saturated brine (10 mL × 2), dried, and concentrated to give a crude product. The crude product was purified by thin-layer chromatography (methanol/dichloromethane = 1:15) to give 2 isomeric compounds. The structure of one of the isomeric compounds was 3-1a (50 mg, 0.048 mmol, yield: 41.71%, Rf = 0.45) as a white solid. ES-API: [M +H]⁺=1042.3. The structure of the other isomeric compound was 3-1b (60 mg, 0.06 mmol, yield: 50.7%, Rf = 0.4) as a yellow solid. ES-API: [M +H]⁺=1042.3.

Step 4: Compound 3-1a (50 mg, 0.048 mmol) was dissolved in tetrahydrofuran (3 mL), and a 4 M solution (1 mL, 0.018 mmol) of hydrochloric acid in dioxane was added under an ice-water bath. The mixture was stirred at room temperature for 1 h under a nitrogen atmosphere. After the reaction was completed, the mixture was concentrated under reduced pressure, and the residue was redissolved in dichloromethane, adjusted to pH 8 with a 7 M solution of ammonia water/methanol, and concentrated under reduced pressure. The resulting crude product was purified by a flash silica gel column (methanol/dichloromethane: 0%-3%) to give (1r,2R,3S)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-(2-hydroxypropyl)-10,10-dimethyl-5,7-dioxo-6,6',6',6⁴6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z3, 30 mg, 0.032 mmol, yield: 67.39%) as a white solid, ES-API: [M+H]⁺ = 928.3.

### Example 1.2. Synthesis of compounds Z4 and Z5

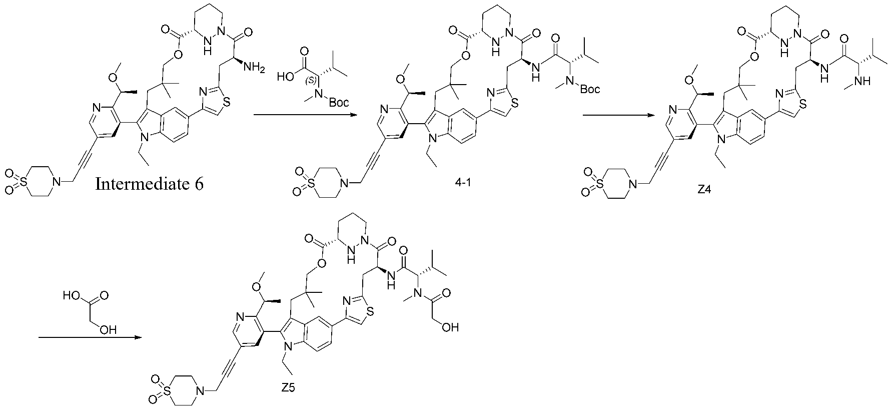

Step 1: N-(tert-Butoxycarbonyl)-N-methyl-L-valine (56.23 mg, 0.243 mmol), N,N-diisopropylethylamine (96.49 mg, 0.748 mmol), and O-(7-azabenzotriazol-1-yl)-N,N,N',N'-tetramethyluronium hexafluorophosphate (142.12 mg, 0.374 mmol) were sequentially added to a solution (5 mL) of intermediate 6 (150 mg, 0.187 mmol) in dichloromethane under an ice-water bath. The mixture was warmed to room temperature and stirred for 2 h. When the reaction was completed as detected by LCMS, the reaction mixture was diluted with 20 mL of dichloromethane and then washed with water (10 mL) and saturated brine (10 mL × 3). The organic phase was dried over anhydrous sodium sulfate, filtered, and concentrated to dryness. The resulting crude product was purified by silica gel column chromatography (methanol/dichloromethane = 1/20) to give compound 4-1 (110 mg, yield: 57.93%), ES-API: [M+H]⁺ = 1015.3.

Step 2: Trifluoroacetic acid (3 mL) was added to a solution of compound 4-1 (110 mg, 0.108 mmol) in dichloromethane (5 mL), and the mixture was reacted at room temperature for 1 h. The mixture was concentrated, then diluted with dichloromethane, washed sequentially with a saturated aqueous sodium bicarbonate solution (20 mL) and saturated brine (20 mL), dried over anhydrous sodium sulfate, filtered, and concentrated to give a crude product. The crude product was then purified by prep-HPLC (ammonium bicarbonate method 1) to give (2S)-N-((6³S,4S,Z)-1²-(5-(3-(1,1-dioxidothiomorpholin-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵, 6⁶-hexahydro-1¹H-8-oxa-2(4,2)-thiazola-1(5,3)-indola-6(1,3)-pyridazinacycloundecaphane-4-yl)-3-methyl-2-(methylamino)butanamide (Z4, 60 mg, 0.0656 mmol, yield: 60.8%), ES-API: [M+H]⁺ = 915.3. ¹H NMR (400 MHz, DMSO-*d*₆) δ 8.81 (d, *J =* 2.1 Hz, 1H), 8.50 (s, 1H), 8.28 (d, *J =* 9.3 Hz, 1H), 7.86 (d, *J =* 2.1 Hz, 1H), 7.83 (s, 1H), 7.78 - 7.73 (m, 1H), 7.58 (d, *J =* 8.7 Hz, 1H), 5.58 (t, *J* = 9.1 Hz, 1H), 5.12 (d, *J* = 12.2 Hz, 1H), 4.37 - 4.23 (m, 4H), 4.13 - 4.04 (m, 1H), 3.76 (s, 2H), 3.62 - 3.53 (m, 2H), 3.29 - 2.24 (m, 5H), 3.16-3.13 (s, 4H), 3.05 - 2.97 (m, 5H), 2.77 - 2.72 (m, 1H), 2.67 - 2.66 (m, 1H), 2.39 - 2.32 (m, 2H), 2.28 (s, 3H), 2.11 (d, *J* = 12.1 Hz, 1H), 1.79-1.75 (m, 3H), 1.56-1.50 (m, 1H), 1.36 (d, *J* = 6.0 Hz, 3H), 0.99 - 0.90 (m, 9H), 0.87 (t, *J =* 7.0 Hz, 3H), 0.34 (s, 3H).

Step 3: 2-Hydroxyacetic acid (6.2 mg, 0.0819 mmol), N,N-diisopropylethylenediamine (21.13 mg, 0.1638 mmol) and O-(7-azabenzotriazol-1-yl)-N,N,N',N'-tetramethyluronium hexafluorophosphate (41.5 mg, 0.1092 mmol) were sequentially added to a solution of compound Z4 (50 mg, 0.0546 mmol) in N,N-dimethylformamide (2 mL), and the mixture was reacted at room temperature for 1 h. The mixture was extracted with ethyl acetate (30 mL), washed with saturated brine, dried over anhydrous sodium sulfate, filtered, and concentrated. The residue was purified by prep-HPLC (ammonium bicarbonate method 1) to give (2S)-N-((6³S,4S,Z)-1²-(5-(3-(1,1-dioxidothiomorpholin-4-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-(2-hydroxy-N-methylacetamide)-3-methylbutanamide (Z5, 7 mg, yield: 13.1%), ES-API: [M+H]⁺= 973.3.

### Example 1.3. Synthesis of Compound Z6

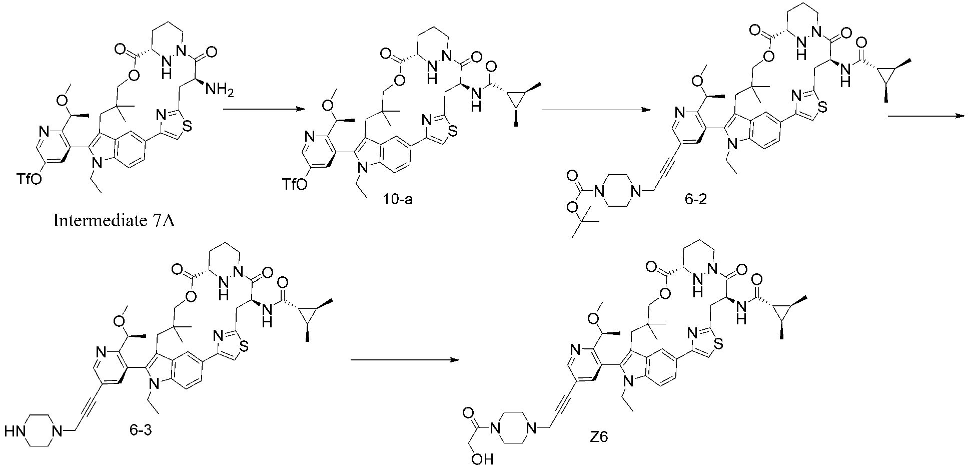

Step 1: (1r,2R,3S)-2,3-Dimethylcyclopropane-1-carboxylic acid (77 mg, 0.68 mmol) and triethylamine (343 mg, 3.4 mmol) were added to a solution of intermediate 7A(440 mg, 0.56 mmol) in N,N-dimethylformamide (5 mL), and O-(7-azabenzotriazol-1-yl)-N,N,N',N'-tetramethyluronium hexafluorophosphate (644 mg, 1.69 mmol) was added under an ice bath. The mixture was stirred at room temperature for 1 h. After the reaction was completed, the mixture was extracted with ethyl acetate (20 mL × 3). The combined extract was washed with 30 mL of brine, filtered, and concentrated, and the residue was purified by a flash silica gel column (0-80% petroleum ether/ethyl acetate) to give compound 10-a (490 mg, yield: 99%). ES-API:[M+H]⁺=875.2.

Step 2: Compound 10-a (150 mg, 0.17 mmol), 1-(tert-butoxycarbonyl)-4-(2-propynyl)piperazine (77 mg, 0.34 mmol), Pd(PPh₃)₂Cl₂ (24 mg, 0.03 mmol), copper(I) iodide (20 mg, 0.1 mmol), and N,N-diisopropylethylamine (88 mg, 0.68 mmol) were mixed in N,N-dimethylformamide (10 mL) under a nitrogen atmosphere, and the mixture was stirred at 80 °C for 10 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 6-2 (150 mg, yield: 92%). ES-API[M+H]⁺=949.3.

Step 3: Trifluoroacetic acid (3 mL) was added to a solution of compound 6-2 (60 mg, 0.06 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted three times with 20 mL of ethyl acetate, and concentrated to give compound 6-3 (50 mg, yield: 93%). ES-API[M+H]⁺=849.3.

Step 4: Hydroxyacetic acid (9 mg, 0.12 mmol) and N,N-diisopropylethylenediamine (46 mg, 0.36 mmol) were added to a solution of compound 6-3 (50 mg, 0.06 mmol) in N,N-dimethylformamide (5 mL), and O-(7-azabenzotriazol-1-yl)-N,N,N',N'-tetramethyluronium hexafluorophosphate (68 mg, 0.18 mmol) was added under an ice bath. The mixture was stirred at room temperature for 1 h. After the reaction was completed, the mixture was extracted with ethyl acetate (20 mL × 3), and the combined extract was washed with 30 mL of brine. The organic phase was dried and concentrated, and then the residue was purified by preparative thin-layer chromatography (dichloromethane/methanol = 20/1) to give (1r,2R,3S)-N-((6³S,4S,Z)-1¹-ethyl-(Rₐ)-1²-(5-(3-(4-(2-hydroxyacetyl)piperazin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z6, 20 mg, yield: 36%, purity: 98%). ES-API[M+H]⁺=907.3. ¹HNMR (400 MHz, DMSO-*d*₆) 8.81 (1 H, d, *J* 2.0), 8.50 (1 H, s), 8.39 (1 H, d, *J* 8.9), 7.86 (1 H, d, *J* 2.0), 7.82 (1 H, s), 7.74 (1 H, s), 7.58 (1 H, d, *J* 8.7), 5.56 (1 H, t, *J* 9.1), 5.05 (1 H, s), 4.52 (1 *H*, d, *J* 5.5), 4.17 (7H, dd, *J* 76.4, 5.8), 3.62 (2 H, s), 3.57 (2 H, s), 3.51 (2 H, s), 3.37 (2 H, s), 3.30 (2 H, s), 3.25 (3 H, s), 3.22 - 3.07 (2 H, m), 2.95 (1 H, d, *J* 15.4), 2.76 (1 H, t, *J* 9.0), 2.53 (2 H, s), 2.38 (1 H, d, *J* 14.3), 2.07 (1 *H*, d, J 10.5), 1.78 (2 H, s), 1.58 - 1.46 (1 H, m), 1.35 (3 H, d, *J* 6.0), 1.16 (3 H, d, *J* 4.4), 1.09 - 1.05 (6H, m), 0.88 (6 H, dd, *J* 12.9, 5.6), 0.34 (3 H, s).

### Example 1.4. Synthesis of Compounds Z7-1 and Z7-2

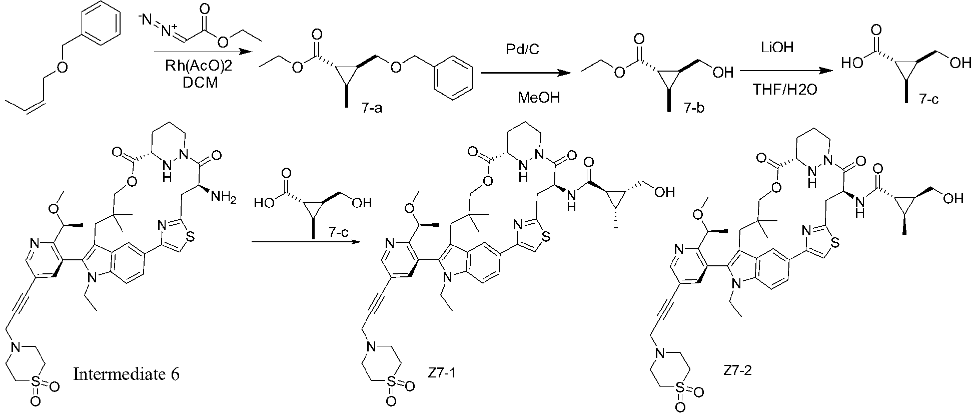

Step 1: (Z)-((But-2-en-1-oxy)methyl)benzene (Journal of Organic Chemistry, 2016, vol. 81, #20, p. 9957-9963) (0.5 g, 3.082 mmol) was dissolved in dichloromethane (15 mL). Rhodium acetate (0.07 g, 0.154 mmol) was added, and a solution of ethyl diazoacetate (0.80 g, 6.164 mmol) in dichloromethane was added dropwise. The mixture was reacted at room temperature for 24 h under a nitrogen atmosphere. After the reaction was completed, the mixture was filtered under vacuum and concentrated to dryness under reduced pressure, and the residue was purified by a flash silica gel column (ethyl acetate/petroleum ether: 0%-10%) to give compound 7-a (150 mg, 0.681 mmol, yield: 22.10%) as a colorless oily liquid, ES-API: [M+H]⁺ = 249.1.

Step 2: Compound 7-a (150 mg, 0.604 mmol) and Pd/C 10% (100 mg, 0.940 mmol) were dissolved in methanol (5 mL). The mixture was purged three times with hydrogen and reacted at room temperature for 18 h under a hydrogen atmosphere. After the reaction was completed, the mixture was filtered under vacuum and concentrated to dryness under reduced pressure, and the residue was purified by a flash silica gel column (ethyl acetate/petroleum ether: 0%-50%) to give compound 7-b (80 mg, 0.506 mmol, yield: 83.72%) as a colorless oily liquid, ES-API: [M+H]⁺ = 176.2.

Step 3: Compound 7-b (25 mg, 0.158 mmol) was dissolved in tetrahydrofuran (1 mL) and water (0.2 mL), and lithium hydroxide (13.26 mg, 0.316 mmol) was added. The mixture was reacted at room temperature for 18 h under a nitrogen atmosphere. After the reaction was completed, the mixture was filtered under vacuum and concentrated under reduced pressure to give compound 7-c (20 mg, 0.154 mmol, yield: 97.25%). ES-API: [M +H]⁺=148.1.

Step 4: Intermediate 6 (20 mg, 0.025 mmol) was dissolved in N,N-dimethylformamide (2 mL), and compound 7-c (13.01 mg, 0.100 mmol), O-(7-azabenzotriazol-1-yl)-N,N,N',N'-tetramethyluronium hexafluorophosphate (14.22 mg, 0.037 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by prep-HPLC (formic acid method) to give 2 isomeric compounds. The structure of one of the isomeric compounds was (1R,2R,3S)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-(hydroxymethyl)-3-methylcyclopropane-1-carboxamide (Z7-1, 3.6 mg, 0.004 mmol, yield: 15.75%, retention time = 1.89 min) as a white solid, ES-API: [M+H]⁺= 914.1. ¹H NMR (400 MHz, DMSO-*d*₆) δ 8.81 (d, *J* = 2.0 Hz, 1H), 8.50 (s, 1H), 8.39 (d, *J* = 8.8 Hz, 1H), 7.86 (d, *J =* 2.0 Hz, 1H), 7.81 (s, 1H), 7.75 (d, *J =* 8.4 Hz, 1H), 7.58 (d, *J =* 8.8Hz, 1H), 5.56 (t, *J =* 9.0 Hz, 1H), 5.06 (d, *J =* 12.0 Hz, 1H), 4.53 (t, *J =* 5.2 Hz, 1H), 4.41-4.10 (m, 5H), 3.75 (s, 2H), 3.66 - 3.54 (m, 3H), 3.46 - 3.36 (m, 1H), 3.29 - 3.28 (m, 1H), 3.25 (s, 3H), 3.19 - 3.14 (m, 4H), 3.06-3.04 (m, 4H), 2.95 *(d, J =* 14.0 Hz, 1H), 2.79-2.76 (m, 1H), 2.39 (d, *J =* 14.0 Hz, 1H), 2.09-2.07(m, 1H),1.81-1.78(m, 2H), 1.53-1.50 (m, 1H), 1.38-1.34 (m, 5H), 1.25-1.21(m, 2H), 1.10 (d, *J =* 6.4 Hz, 3H), 0.97 - 0.81 (m, 6H), 0.35 (s, 3H). The structure of the other isomeric compound was (1S,2S,3R)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-(hydroxymethyl)-3-methylcyclopropane-1-carboxamide (Z7-2, 3.6 mg, 0.004 mmol, yield: 15.75%, retention time = 1.93 min) as a white solid, ES-API: [M+H]⁺ = 914.1. ¹H NMR (400 MHz, DMSO-*d*₆) δ 8.81 (s, 1H), 8.50 (s, 1H), 8.40 (d, *J* = 8.8 Hz, 1H), 7.87 (s, 1H), 7.81 (s, 1H), 7.75 (d, *J* = 8.0 Hz, 1H), 7.58 (d, *J =* 8.4 Hz, 1H), 5.58 (t, *J* = 9.0 Hz, 1H), 5.06 (d, *J* = 12.0 Hz, 1H), 4.39 - 4.05 (m, 5H), 3.60-3.58(m, 3H), 3.44-3.40 (m, 1H), 3.25 (s, 3H), 3.20-3.16 (m, 4H), 3.08-3.04 (m, 4H), 2.97-2.94 (m, 1H), 2.79-2.76(m, 1H), 2.43 - 2.32 (m, 2H), 2.12 - 1.98 (m, 2H), 1.79-1.78 (m, 2H), 1.56-1.52 (m, 1H), 1.43-1.36 (m, 5H), 1.26-1.24 (m, 4H), 1.15-1.12(m, 3H),0.92-0.89(m, 6H), 0.35 (s, 3H).

### Example 1.5. Synthesis of Compound Z10

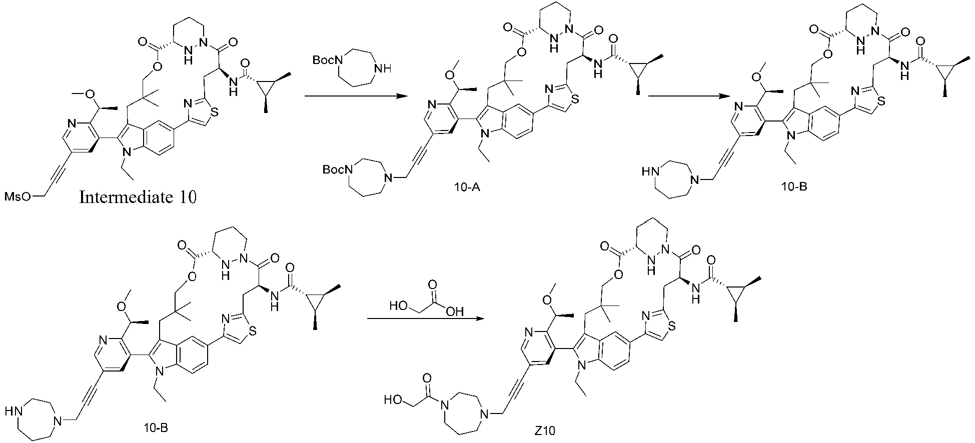

Step 1: Intermediate 10 (0.15 g, 0.175 mmol) and N,N'-diisopropylethylamine (0.11 g, 0.875 mmol) were dissolved in dichloromethane (20 mL). 2-Methylpropan-2-yl 1,4-diazepine-1-carboxylate (0.19 g, 0.931 mmol, CAS: 112275-50-0) was added. The reaction system was stirred at room temperature for 5 h. The mixture was concentrated under reduced pressure, and the crude product was purified by a flash silica gel column (methanol/dichloromethane: 0-5%) to give compound 10-A (150 mg, 0.156 mmol, yield: 89.18%). ES-API: [M+H]⁺=963.2.

Step 2: Compound 10-A (120 mg, 0.125 mmol) was dissolved in methanol (0.5 mL), and a solution (4 M, 1 mL) of hydrogen chloride in dioxane was added to the solution described above. The mixture was stirred at room temperature for 2 h. After the reaction was completed, the reaction system was concentrated to give a crude product. The crude product was purified by prep-HPLC (formic acid method) to give (1r,2R,3S)-N-((6³S,4S,Z)-(Rₐ)-1²-(5-(3-(1,4-oxazepan-4-yl)propyn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (compound 10-B, 60 mg, 0.063 mmol, formate, yield: 50.42%) as a white solid, ES-API: [M+H]⁺ = 863.4.

Step 3: Compound 10-B (20.0 mg, 0.0209 mmol, formate) was added to a mixed solution of dichloromethane/saturated sodium bicarbonate (20.0 mL/10.0 mL). The mixture was stirred at room temperature for 30 min, and then the dichloromethane phase was concentrated to dryness by rotary evaporation under reduced pressure to give a crude product (21.0 mg). ES-API: [M+H]⁺= 863.4.

Glycolic acid (2.60 mg, 0.035 mmol), N,N-diisopropylethylamine (0.21 mL, 1.225 mmol), and O-(7-azabenzotriazol-1-yl)-N,N,N',N'-tetramethyluronium hexafluorophosphate (27.0 mg, 0.070 mmol) were sequentially added to a solution of the crude product (21.0 mg) in N,N-dimethylformamide (10 mL) under an ice-water bath. The mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The crude product was purified by prep-HPLC (ammonium bicarbonate method 2) to give the compound (1r,2R,3S)-N-((6³S,4S,Z)-1¹-ethyl-(Rₐ)-1²-(5-(3-(4-(2-hydroxyacetyl)-1,4-diazepin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z10, 7.86 mg, 0.009 mmol, yield: 36.82). ES-API: [M+H]⁺= 921.1.

### Example 1.6. Synthesis of Compound Z11

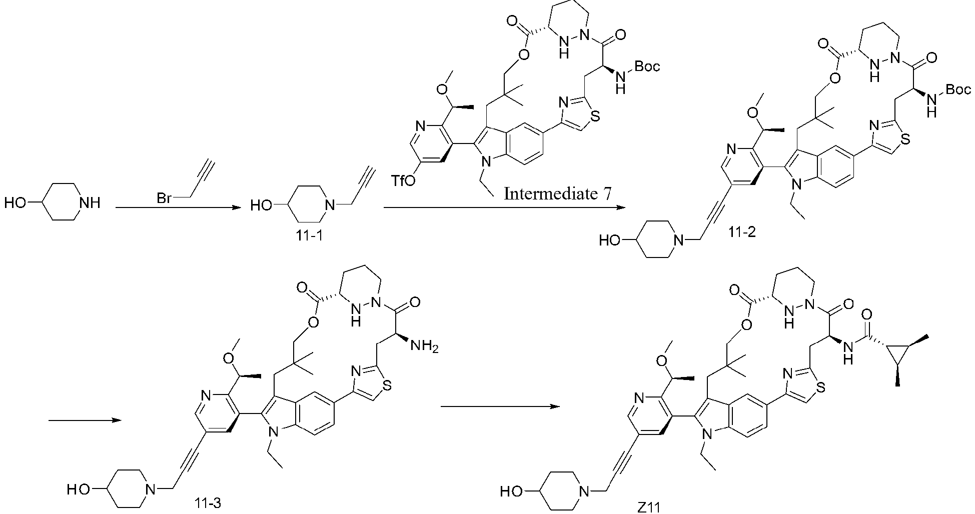

Step 1: 3-Bromoprop-1-yne (1.76 g, 14.829 mmol) and potassium carbonate (6.15 g, 44.48 mmol) were added to a solution of piperidin-4-ol (1.50 g, 14.829 mmol) in N,N-dimethylformamide (15.0 mL), and the mixture was stirred at 25 °C for 18 h. After the reaction was completed, the cooled solution was extracted with ethyl acetate (100 mL × 2) and saturated brine (100 mL). The ethyl acetate phase was dried over anhydrous sodium sulfate and filtered. The filtrate was concentrated, and then the residue was purified by automated flash chromatography on silica gel [dichloromethane:methanol = 100:0 to 90:10, (v/v)] to give the desired compound 11-1 (0.90 g, yield: 45.0%). ES-API: [M+ H]⁺= 140.2.

Step 2: Compound 11-1 (120.0 mg, 0.862 mmol), bis(triphenylphosphine)palladium(II) dichloride (59.89 mg, 0.085 mmol), copper(I) iodide (32.50 mg, 0.171 mmol), N,N-diisopropylethylamine (0.198 mL, 1.138 mmol), and lithium chloride (60.28 mg, 1.422 mmol) were added to a solution of intermediate 7 (250.0 mg, 0.284 mmol) in N,N-dimethylformamide (5.0 mL). After the addition, the mixture was stirred at 80 °C for 1.5 h under a nitrogen atmosphere. The reaction mixture was then diluted with water (80 mL) and extracted with ethyl acetate (30 mL × 3). The organic layers were combined, washed with brine, dried over anhydrous sodium sulfate, filtered, and concentrated. The residue was purified by automated flash chromatography on silica gel (a 0% to 1.0% solution of methanol in dichloromethane) to give compound 11-2 (260.0 mg, crude product).

Step 3: A solution (5.0 mL, 4 M, 20.0 mmol) of hydrochloric acid in methanol was slowly added dropwise to a solution of compound 11-2 (260.0 mg, crude product) in methanol (2 mL) under an ice-water bath. The reaction mixture was stirred at room temperature for 1.5 h. When the reaction was completed as detected by LCMS, the mixture was concentrated under reduced pressure at a low temperature (30 °C) to remove the solvent. 5 mL of a saturated sodium bicarbonate solution was added to the residue under an ice-water bath, and the mixture was extracted with dichloromethane (6 mL × 3). The organic phases were combined, washed with saturated brine (6 mL × 2), dried over anhydrous sodium sulfate, filtered, and concentrated to give crude compound 11-3 (290.0 mg, crude product). ES-API: [M+H]⁺= 768.3.

Step 4: (1r,2R,3S)-2,3-Dimethylcyclopropane-1-carboxylic acid (55.47 mg, 0.486 mmol), N,N-diisopropylethylamine (209.0 mg, 1.620 mmol), and O-(7-azabenzotriazol-1-yl)-N,N,N',N'-tetramethyluronium hexafluorophosphate (185.0 mg, 0.486 mmol) were sequentially added to a solution of compound 11-3 (290.0 mg, crude product) in N,N-dimethylformamide (10.0 mL) under an ice-water bath.The mixture was warmed to room temperature and stirred for 10 min. When the reaction was completed as detected by LCMS, the reaction mixture was diluted with 20 mL of ethyl acetate and then washed with water (10 mL) and saturated brine (10 mL × 3). The organic phase was dried over anhydrous sodium sulfate, filtered, and concentrated to dryness. The residue was purified by prep-HPLC (ammonium bicarbonate method 2) to give the compound (1r,2R,3S)-N-((6³S,4S,Z)-1¹-ethyl-(Rₐ)-1²-(5-(3-(4-hydroxypiperidin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z11, 220 mg, 0.255 mmol, yield: 89.65%, retention time: 7.45 min). ES-API[M+H]⁺= 864.3.

### Example 1.7. Synthesis of Compound Z12

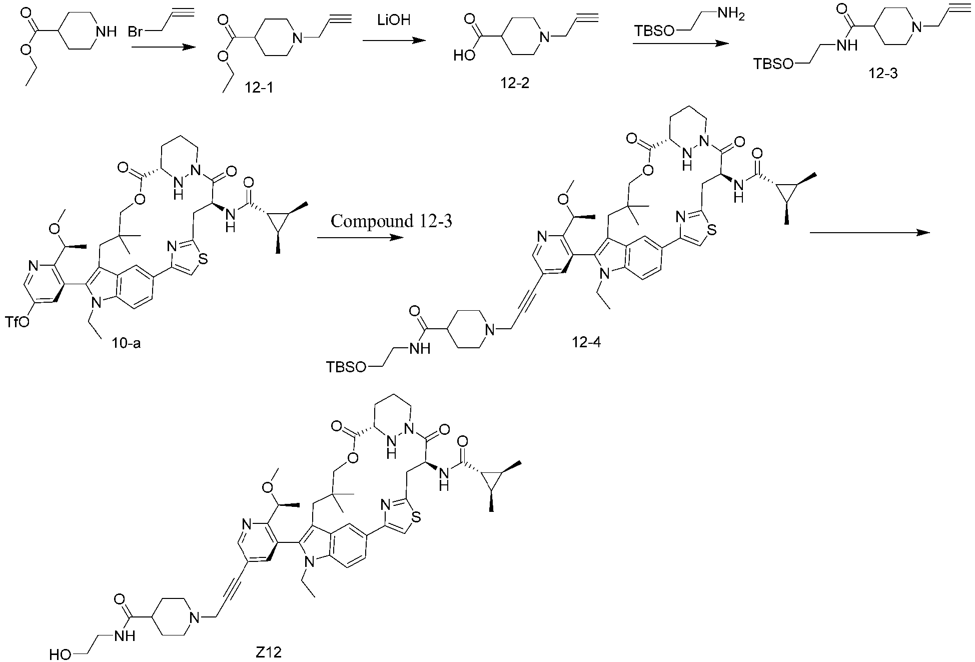

Step 1: Ethyl 4-piperidinecarboxylate (150 mg, 1.280 mmol) was dissolved in acetonitrile (5 mL), and potassium carbonate (527.45 mg, 3.817 mmol) and 3-bromopropyne (227.01 mg, 1.908 mmol) were added. The mixture was reacted at room temperature for 4 h under a nitrogen atmosphere. After the reaction was completed, the mixture was filtered under vacuum and concentrated to dryness under reduced pressure, and the residue was purified by a flash silica gel column (ethyl acetate/petroleum ether: 0%-50%) to give compound 12-1 (120 mg, 0.773 mmol, yield: 60.39%) as a colorless oily liquid. ES-API: [M +H]⁺=196.2.

Step 2: Compound 12-1 (100 mg, 0.5 mmol) was dissolved in tetrahydrofuran (2 mL), methanol (0.5 mL), and water (0.5 mL), and lithium hydroxide (42 mg, 1 mmol) was added. The mixture was reacted at room temperature for 5 h under a nitrogen atmosphere. The mixture was concentrated under reduced pressure to give compound 12-2 (90 mg, 0.5 mmol, yield: 94.59%). ES-API: [M +H]⁺=168.3.

Step 3: Compound 12-2 (100 mg, 0.6 mmol) and 2-{[dimethyl(2-methylpropan-2-yl)silyl]oxy}ethan-1-amine (105 mg, 0.6 mmol) were dissolved in N,N-dimethylformamide (2 mL), and O-(7-azabenzotriazol-1-yl)-N,N,N',N'-tetramethyluronium hexafluorophosphate (300 mg, 0.9 mmol) and N,N'-diisopropylethylamine (230 mg, 1.8 mmol) were added. The mixture was reacted at room temperature for 2 h under a nitrogen atmosphere. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (ethyl acetate/petroleum ether: 0%-50%) to give compound 12-3 (120 mg, 0.34 mmol, yield: 60.11%). ES-API: [M +H]⁺=325.3.

Step 4: Compound 10-a (50 mg, 0.057 mmol) and compound 12-3 (37.09 mg, 0.114 mmol) were dissolved in acetonitrile (3 mL), and bis(triphenylphosphine)palladium(II) dichloride (16.02 mg, 0.023 mmol), copper(I) iodide (6.58 mg, 0.034 mmol), N,N-diisopropylethylamine (29.49 mg, 0.229 mmol), and anhydrous lithium chloride (9.60 mg, 0.229 mmol) were added. The mixture was reacted at 80 °C for 5 h under a nitrogen atmosphere. After the reaction was completed, the mixture was filtered under vacuum, dried, and concentrated to give a crude product. The crude product was purified by a flash silica gel column (methanol/dichloromethane: 0%-10%) to give compound 12-4 (30 mg, 0.029 mmol, yield: 50.03%) as a pale yellow solid. ES-API: [M +H]⁺=1049.4.

Step 5: Compound 12-4 (30 mg, 0.029 mmol) was dissolved in tetrahydrofuran (2 mL), and a 4 M solution (0.2 mL, 0.8 mmol) of hydrochloric acid in dioxane was added. The mixture was reacted at room temperature for 1 h under a nitrogen atmosphere. After the reaction was completed, the mixture was concentrated to give a crude product. The crude product was then purified by a flash silica gel column (methanol/dichloromethane: 0%-10%) to give 1-(3-(5-((6³*S*,4*S*,*Z*)-4-(((*1R,2R,3S*)-2,3-dimethylcyclopropane-1-carboxamido)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-1²-yl-(*Rₐ*)-6-((*S*)-1-methoxyethyl)pyridin-3-yl)prop-2-yn-1-yl)-N-(2-hydroxyethyl)piperidine-4-carboxamide (Z12, 15 mg, 0.016 mmol, yield: 56.12%) as a white solid. ES-API: [M +H]⁺=935.3.

### Example 1.8. Synthesis of Compounds Z13-1 and Z13-2

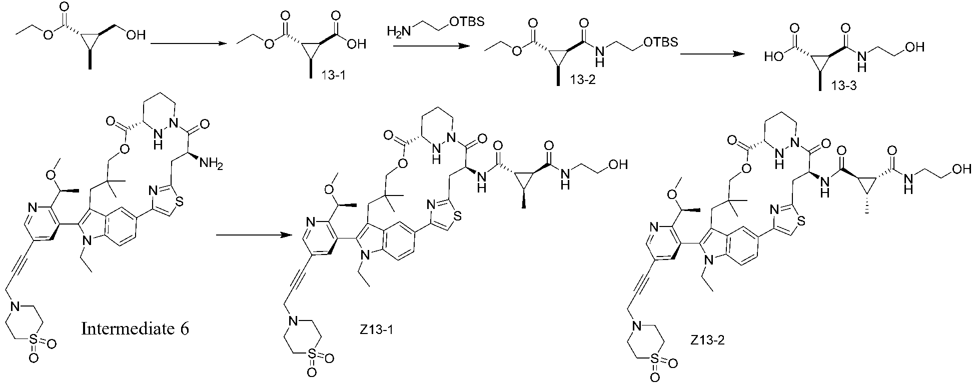

Step 1: Ethyl 2-(hydroxymethyl)-3-methylcyclopropane-1-carboxylate (7-b, 60 mg, 0.228 mmol, purity: 60%) was dissolved in acetonitrile (3 mL), and 2,2,6,6-tetramethylpiperidine oxide (7.10 mg, 0.046 mmol), disodium hydrogen phosphate (40 mg, 0.341 mmol), and sodium dihydrogen phosphate (48.81 mg, 0.341 mmol) were added. A sodium hypochlorite solution (14%, 0.1 mL) and an aqueous solution (1.5 mL) of sodium chlorite (41.16 mg, 0.455 mmol) were added. The mixture was reacted at room temperature for 16 h. After the reaction was completed, water (10 mL) was added, and the mixture was extracted with ethyl acetate (15 mL × 2). The organic phase was washed with saturated brine (10 mL × 3), dried, and concentrated to give compound 13-1 (30 mg, 0.174 mmol, yield: 76.57%) as a colorless oil. ES-API: [M +H]⁺=190.1.

Step 2: Compound 13-1 (33 mg, 0.192 mmol) and 2-((tert-butyldimethylsilyl)oxy)ethan-1-amine (36.97 mg, 0.211 mmol) were dissolved in N,N-dimethylformamide (1 mL), and O-(7-azabenzotriazol-1-yl)-N,N,N',N'-tetramethyluronium hexafluorophosphate (94.74 mg, 0.249 mmol) and N,N'-diisopropylethylamine (74.32 mg, 0.575 mmol) were added. The mixture was reacted at room temperature for 1 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (ethyl acetate/petroleum ether: 0%-50%) to give compound 13-2 (45 mg, 0.137 mmol, yield: 71.25%) as a white solid, ES-API: [M+H]⁺ = 330.3.

Step 3: Compound 13-2 (40 mg, 0.121 mmol) was dissolved in tetrahydrofuran (1.5 mL) and water (0.3 mL), and lithium hydroxide (10.19 mg, 0.243 mmol) was added. The mixture was reacted at room temperature for 16 h. The reaction mixture was concentrated under reduced pressure to give compound 13-3 (22 mg, 0.118 mmol, yield: 97.13%) as an oily liquid.

Step 4: Intermediate 6 (20 mg, 0.025 mmol) was dissolved in N,N-dimethylformamide (2 mL), and compound 13-3 (13.01 mg, 0.100 mmol), O-(7-azabenzotriazol-1-yl)-N,N,N',N'-tetramethyluronium hexafluorophosphate (14.22 mg, 0.037 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give 2 isomeric compounds. The structure of one of the isomeric compounds was (1R,2R,3S)-N¹-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-N²-(2-hydroxyethyl)-3-methylcyclopropane-1,2-dicarboxamide (Z13-1, 3 mg, 0.003 mmol, yield: 12.36%, retention time: 6.36 min) as a white solid, ES-API: [M+H]⁺ = 971.3. The structure of the other isomeric compound was (1S,2S,3R)-N¹-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-N²-(2-hydroxyethyl)-3-methylcyclopropane-1,2-dicarboxamide (Z13-2, 3 mg, 0.003 mmol, yield: 12.36%, retention time: 6.51 min) as a white solid, ES-API: [M+H]⁺ = 971.3.

### Example 1.9. Synthesis of compounds Z14 and Z15

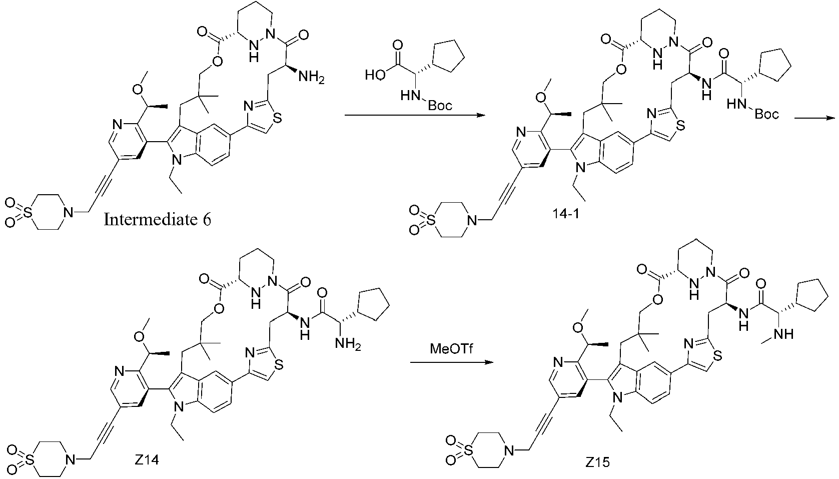

Step 1: N,N-Diisopropylethylamine (97 mg, 0.75 mmol) and O-(7-azabenzotriazol-1-yl)-N,N,N',N'-tetramethyluronium hexafluorophosphate (85 mg, 0.22 mmol) were sequentially added to a solution of (S)-2-((tert-butoxycarbonyl)amino)-2-cyclopentylacetic acid (44 mg, 0.18 mmol) and intermediate 6 (120 mg, 0.15 mmol) in dichloromethane (2 mL). The mixture was stirred at room temperature for 1 h. After the reaction was completed, the mixture was washed with 2 mL of water and concentrated, and the residue was purified by a preparative chromatographic column (0-60% tetrahydrofuran/petroleum ether) to give compound 14-1 (140 mg, yield: 91%). ES-API:[M+H]⁺=1027.2.

Step 2: Compound 14-1 (140 mg, 0.14 mmol) was dissolved in methanol (1 mL), and a solution (5 mL, 20 mmol) of hydrochloric acid in dioxane was added. The mixture was stirred at room temperature for 2 h. The reaction mixture was neutralized with a saturated sodium bicarbonate solution, extracted with ethyl acetate, and concentrated, and the residue was purified by a flash silica gel column (0-10% methanol (ammonia)/dichloromethane) to give (2S)-2-amino-2-cyclopentyl-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)acetamide (Z14, 80 mg, yield: 63%, purity: 98%). ES-API:[M+H]⁺=927.2. ¹H NMR (400 MHz, DMSO-*d*₆) δ 8.82 (d, *J =* 2.0 Hz, 1H), 8.51 (s, 1H), 8.32 (d, *J =* 9.2 Hz, 1H), 7.87 (d, *J =* 2.0 Hz, 1H), 7.84 (s, 1H), 7.77 (dd, *J* = 8.6, 1.2 Hz, 1H), 7.59 (d, *J* = 8.8 Hz, 1H), 5.59 (t, *J* = 9.2 Hz, 1H), 5.13 (d, *J* = 12.4 Hz, 1H), 4.40 - 4.16 (m, 4H), 4.08 (q, *J =* 7.6 Hz, 1H), 3.76 (s, 2H), 3.59 (s, 2H), 3.26 (s, 3H), 3.25 - 3.20 (m, 1H), 3.19 - 3.14 (m, 6H), 3.14 - 3.10 (m, 1H), 3.08 - 3.02 (m, 4H), 2.97 (d, *J* = 13.6 Hz, 1H), 2.85 - 2.64 (m, 2H), 2.39 (d, *J =* 14.4 Hz, 1H), 2.15 - 2.04 (m, 2H), 1.85 - 1.76 (m, 2H), 1.71 - 1.40 (m, 8H), 1.36 (d, *J* = 6.0 Hz, 3H), 1.24 (s, 1H), 0.92 (s, 3H), 0.88 (t, *J =* 7.2 Hz, 3H), 0.35 (s, 3H).

Step 3: Compound Z14 (67 mg, 0.07 mmol) was dissolved in 1,1,1,3,3,3-hexafluoroisopropanol (0.5 mL), and then methyl trifluoromethanesulfonate (18 mg, 0.11 mmol) was added. The mixture was stirred at room temperature for 1 h. The reaction mixture was concentrated, and the residue was purified by prep-HPLC (ammonium bicarbonate method 1) to give (2S)-2-methylamino-2-cyclopentyl-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)acetamide (Z15, 5.32 mg, yield: 8%, purity: 98%). ES-API:[M+H]⁺=941.2. ¹H NMR (400 MHz, DMSO-*d*₆) δ 8.81 (d, *J =* 2.0 Hz, 1H), 8.50 (s, 1H), 8.26 (d, *J =* 9.2 Hz, 1H), 7.86 (d, *J =* 2.0 Hz, 1H), 7.84 (s, 1H), 7.75 (dd, *J* = 8.8, 1.2 Hz, 1H), 7.58 (d, *J =* 8.8 Hz, 1H), 5.57 (t*, J* = 9.2 Hz, 1H), 5.11 (d, *J* = 12.0 Hz, 1H), 4.47 - 4.18 (m, 4H), 4.08 (q, *J =* 7.2 Hz, 1H), 3.75 (s, 3H), 3.57 (d, *J* = 2.8 Hz, 2H), 3.27 (s, 3H), 3.25 - 3.19 (m, 1H), 3.19 - 3.13 (m, 4H), 3.09 - 3.02 (m, 4H), 3.02 - 2.94 (m, 1H), 2.85 - 2.64 (m, 3H), 2.35 (d, *J=* 14.0 Hz, 1H), 2.27 (s, 3H), 2.16 - 2.08 (m, 1H), 2.03 - 1.90 (m, 2H), 1.86 - 1.70 (m, 3H), 1.62 - 1.52 (m, 4H), 1.52 - 1.43 (m, 3H), 1.36 (d, *J =* 6.0 Hz, 3H), 1.23 (s, 1H), 0.93 (s, 3H), 0.87 (t, *J =* 7.2 Hz, 3H), 0.33 (s, 3H).

### Example 1.10. Synthesis of compounds Z16 and Z17

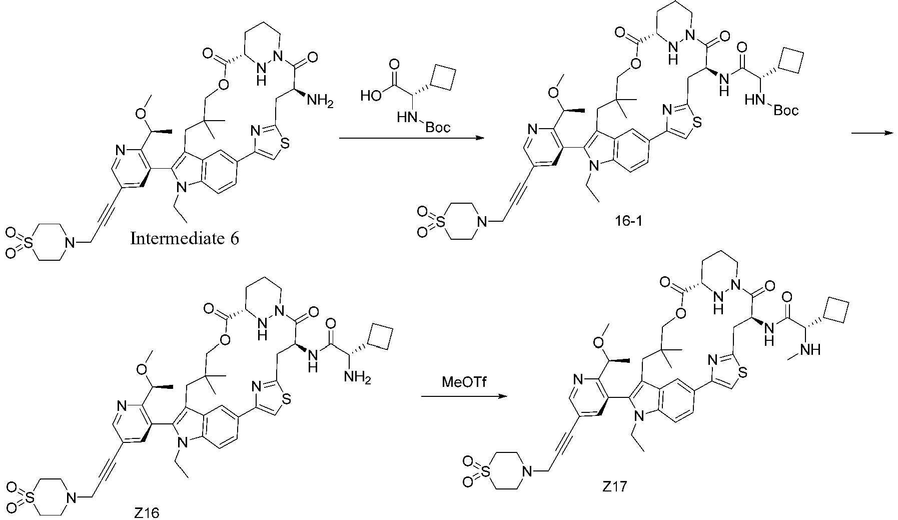

Step 1: N,N-Diisopropylethylamine (97 mg, 0.75 mmol) and O-(7-azabenzotriazol-1-yl)-N,N,N',N'-tetramethyluronium hexafluorophosphate (85 mg, 0.22 mmol) were sequentially added to a solution of (S)-2-((tert-butoxycarbonyl)amino)-2-cyclobutylacetic acid (44 mg, 0.18 mmol) and intermediate 6 (120 mg, 0.15 mmol) in dichloromethane (2 mL). The mixture was stirred at room temperature for 1 h. After the reaction was completed, the mixture was washed with 2 mL of water and concentrated, and the residue was purified by a preparative chromatographic column (0-60% tetrahydrofuran/petroleum ether) to give compound 16-1 (140 mg, yield: 91%). ES-API:[M+H]⁺=1027.2.

Step 2: Compound 16-1 (140 mg, 0.14 mmol) was dissolved in methanol (1 mL), and a solution (5 mL, 20 mmol) of hydrochloric acid in dioxane was added. The mixture was stirred at room temperature for 2 h. The reaction mixture was neutralized with a saturated sodium bicarbonate solution, extracted with ethyl acetate, and concentrated, and the residue was purified by a flash silica gel column (0-10% methanol (ammonia)/dichloromethane) to give (2S)-2-amino-2-cyclobutyl-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)acetamide (Z16, 80 mg, yield: 63%, purity: 98%). ES-API:[M+H]⁺=913.2. Step 3: Compound Z16 (67 mg, 0.07 mmol) was dissolved in 1,1,1,3,3,3-hexafluoroisopropanol (0.5 mL), and then methyl trifluoromethanesulfonate (18 mg, 0.11 mmol) was added. The mixture was stirred at room temperature for 1 h. The reaction mixture was concentrated, and the residue was purified by prep-HPLC (ammonium bicarbonate method 1) to give (2S)-2-methylamino-2-cyclobutyl-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)acetamide (Z17, 6.66 mg, yield: 10%, purity: 98%). ES-API:[M+H]⁺=927.2. ¹H NMR (400 MHz, DMSO-*d*₆) δ 8.81 (d, *J =* 2.0 Hz, 1H), 8.51 (s, 1H), 8.20 (d, *J =* 9.6 Hz, 1H), 7.86 (d, *J =* 2.0 Hz, 1H), 7.83 (s, 1H), 7.76 (dd, *J* = 8.8, 1.2 Hz, 1H), 7.58 (d, *J* = 8.8 Hz, 1H), 5.56 (t, *J* = 9.2 Hz, 1H), 5.11 (d, *J =* 12.0 Hz, 1H), 4.37 - 4.28 (m, 2H), 4.27 - 4.20 (m, 2H), 4.14 - 4.04 (m, 1H), 3.75 (s, 2H), 3.58 (s, 2H), 3.27 (s, 3H), 3.24 - 3.20 (m, 1H), 3.18 - 3.14 (m, 4H), 3.07 - 3.02 (m, 4H), 3.01 - 2.96 (m, 1H), 2.88 (d, *J =* 8.4 Hz, 1H), 2.83 - 2.64 (m, 2H), 2.43 - 2.35 (m, 2H), 2.27 (s, 3H), 2.14 - 2.08 (m, 1H), 2.02 - 1.94 (m, 2H), 1.86 - 1.76 (m, 6H), 1.57 - 1.48 (m, 1H), 1.35 (d, *J =* 6.0 Hz, 3H), 1.24 (s, 1H), 0.93 (s, 3H), 0.87 (t, *J =* 7.0 Hz, 3H), 0.33 (s, 3H).

### Example 1.11. Synthesis of Compound Z18

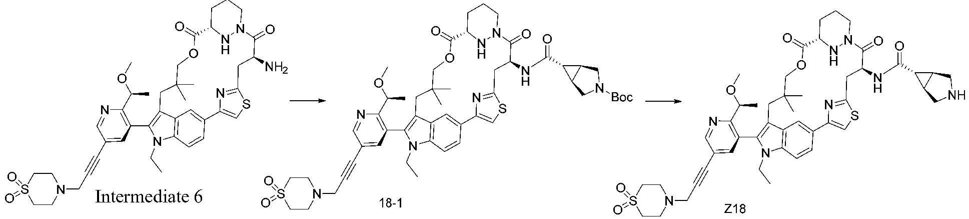

Step 1: N,N-Diisopropylethylamine (40 mg, 0.31 mmol) and O-(7-azabenzotriazol-1-yl)-N,N,N',N'-tetramethyluronium hexafluorophosphate (36 mg, 0.09 mmol) were sequentially added to a solution of (1R,5S,6r)-3-[(tert-butoxy)carbonyl]-3-azabicyclo[3.1.0]hexane-6-carboxylic acid (17 mg, 0.07 mmol) and intermediate 6 (50 mg, 0.06 mmol) in dichloromethane (2 mL). The mixture was stirred at room temperature for 1 h. After the reaction was completed, the mixture was washed with 2 mL of water and concentrated, and the residue was purified by a preparative chromatographic column (0-60% tetrahydrofuran/petroleum ether) to give compound 18-1 (50 mg, yield: 80%). ES-API:[M+H]⁺=1011.2.

Step 2: Compound 18-1 (50 mg, 0.14 mmol) was dissolved in methanol (1 mL), and a solution (5 mL, 20 mmol) of hydrochloric acid in dioxane was added. The mixture was stirred at room temperature for 2 h. The reaction mixture was neutralized with a saturated sodium bicarbonate solution, extracted with ethyl acetate, and concentrated, and the residue was purified by a flash silica gel column (0-10% methanol (ammonia)/dichloromethane) to give (1R,5S,6r)-N-((6³S,4S,Z)-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-azabicyclo[3.1.0]hexane-6-carboxamide (Z18, 9.88 mg, yield: 22%, purity: 98%). ES-API:[M+H]⁺=911.2. ¹H NMR (400 MHz, DMSO-*d*₆) δ 8.81 (d, *J =* 2.0 Hz, 1H), 8.50 (s, 1H), 8.41 (d, *J =* 8.8 Hz, 1H), 7.86 (d, *J =* 2.0 Hz, 1H), 7.81 (s, 1H), 7.76 (dd, *J =* 8.8, 1.2 Hz, 1H), 7.58 (d, *J =* 8.8 Hz, 1H), 5.53 (t, *J* = 9.2 Hz, 1H), 5.05 (d, *J* = 12.0 Hz, 1H), 4.51 - 4.14 (m, 4H), 4.08 (q, *J =* 7.6 Hz, 1H), 3.75 (s, 2H), 3.57 (s, 2H), 3.34 - 3.26 (m, 1H), 3.26 (s, 3H), 3.23 - 3.19 (m, 1H), 3.18 - 3.13 (m, 4H), 3.08 - 3.01 (m, 4H), 3.02 - 2.92 (m, 3H), 2.87 - 2.78 (m, 2H), 2.78 - 2.65 (m, 1H), 2.39 (d, *J* = 14.0 Hz, 1H), 2.08 (d, *J =* 10.4 Hz, 1H), 1.85 - 1.72 (m, 3H), 1.72 - 1.62 (m, 2H), 1.54 - 1.46 (m, 1H), 1.35 (d, *J =* 6.0 Hz, 3H), 1.23 (s, 1H), 0.91 (s, 3H), 0.88 (t, *J =* 7.2 Hz, 3H), 0.34 (s, 3H).

### Example 1.12. Synthesis of Compound Z19

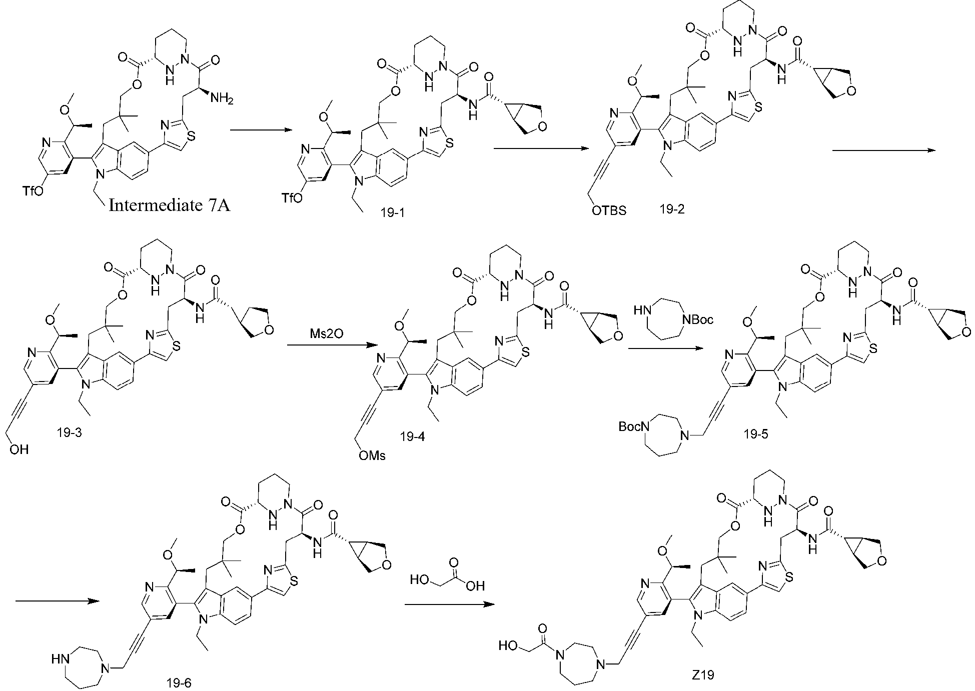

Step 1: Intermediate 7A (530 mg, 0.680 mmol) and (1R,5S,6r)-3-oxabicyclo[3.1.0]hexane-6-carboxylic acid (113.35 mg, 0.885 mmol) were dissolved in N,N-dimethylformamide (3 mL). O-(7-Azabenzotriazol-1-yl)-N,N,N',N'-tetramethyluronium hexafluorophosphate (388.12 mg, 1.021 mmol) and N,N'-diisopropylethylamine (263.86 mg, 2.041 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give compound 19-1 (560 mg, 0.630 mmol, yield: 92.57%) as a white solid, ES-API: [M+H]⁺ = 889.4.

Step 2: Compound 19-1 (480 mg, 0.540 mmol), tert-butyldimethyl(2-propynyloxy)silane (183.94 mg, 1.080 mmol), bis(triphenylphosphine)palladium(II) dichloride (151.42 mg, 0.216 mmol), copper(I) iodide (61.70 mg, 0.324 mmol), N,N'-diisopropylethylamine (279.15 mg, 2.160 mmol), and anhydrous lithium chloride (91.55 mg, 2.160 mmol) were dissolved in acetonitrile (10 mL). The mixture was reacted at 80 °C for 5 h under a nitrogen atmosphere. After the reaction was completed, the mixture was filtered under vacuum and concentrated under reduced pressure to give a crude product. The crude product was purified by a flash silica gel column (methanol/dichloromethane: 0%-10%) to give compound 19-2 (392 mg, 0.431 mmol, yield: 79.85%) as a pale yellow solid, ES-API: [M+H]⁺ = 909.3.

Step 3: Compound 19-2 (500 mg, 0.550 mmol) was dissolved in tetrahydrofuran (3 mL), and a solution (1 M, 0.825 mL, 0.825 mmol) of tetrabutylammonium fluoride in tetrahydrofuran was added. The mixture was reacted at room temperature for 1 h under a nitrogen atmosphere. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give compound 19-3 (400 mg, 0.503 mmol, yield: 91.50%) as a pale yellow solid, ES-API: [M+H]⁺ = 795.3.

Step 4: Compound 19-3 (300 mg, 0.377 mmol) was dissolved in dichloromethane (5 mL), and methanesulfonic anhydride (72.23 mg, 0.415 mmol) and N,N'-diisopropylethylamine (146.33 mg, 1.132 mmol) were added. The mixture was reacted at room temperature for 1 h under a nitrogen atmosphere and concentrated to dryness under reduced pressure. The crude product was purified by a flash silica gel column (methanol/dichloromethane: 0%-10%) to give compound 19-4 (300 mg, 0.344 mmol, yield: 91.06%) as a pale yellow solid, ES-API: [M+H]⁺ *=* 873.3.

Step 5: Compound 19-4 (50 mg, 0.057 mmol) was dissolved in dichloromethane (5 mL), and N,N'-diisopropylethylamine (7.40 mg, 0.057 mmol), and 2-methylpropan-2-yl 1,4-diazepine-1-carboxylate (34.41 mg, 0.172 mmol, CAS: 112275-50-0) were added. The mixture was reacted at room temperature for 1 h under a nitrogen atmosphere and concentrated to dryness under reduced pressure. The crude product was purified by a flash silica gel column (methanol/dichloromethane: 0%-10%) to give compound 19-5 (40 mg, 0.041 mmol, yield: 71.47%) as a pale yellow solid, ES-API: [M+H]⁺ = 977.3.

Step 6: Compound 19-5 (35 mg, 0.036 mmol) was dissolved in methanol (0.5 mL), and a 4 M solution (2 mL) of hydrochloric acid in dioxane was added. The mixture was reacted at room temperature for 1 h under a nitrogen atmosphere and concentrated under reduced pressure to give compound 19-6 (31 mg, 0.035 mmol, yield: 98.68%) as a yellow solid, ES-API: [M+H]⁺ = 877.3.

Step 7: Hydroxyacetic acid (2.25 mg, 0.030 mmol) was dissolved in N,N-dimethylformamide (2.5 mL), and compound 19-6 (20 mg, 0.023 mmol), O-(7-azabenzotriazol-1-yl)-N,N,N',N'-tetramethyluronium hexafluorophosphate (13.01 mg, 0.034 mmol), and N,N'-diisopropylethylamine (11.79 mg, 0.091 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give (1*R,*5*S,*6*r*)-N-((6³*S*,4*S*,*Z*)-1¹-ethyl-(*Rₐ*)-1²-(5-(3-(4-(2-hydroxyacetyl)-1,4-diazepan-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-ylbicyclo[3.1.0]hexane-6-carboxamide (Z19, 6 mg, 0.006 mmol, yield: 28.14%) as a white solid, ES-API: [M+H]⁺ = 935.3. ¹H NMR (400 MHz, DMSO-*d*₆) δ 8.80 (dd, *J* = 4.0, 2.0 Hz, 1H), 8.56 (d, *J =* 8.8 Hz, 1H), 8.50 (s, 1H), 7.83 (dd, *J =* 8.0, 5.6 Hz, 2H), 7.78 - 7.72 (m, 1H), 7.58 *(d, J =* 8.8 Hz, 1H), 5.56 (t, *J* = 8.8 Hz, 1H), 5.07 *(d, J =* 12.2 Hz, 1H), 4.46 - 4.04 (m, 8H), 3.83 (t*, J* = 8.0 Hz, 2H), 3.71-3.65 (m, 4H), 3.58 (s, 3H), 3.55-3.52 (m, 1H), 3.46 - 3.38 (m, 3H), 3.25 (s, 3H), 3.16-3.13 (m, 1H), 2.95 (d, *J* = 13.8 Hz, 1H), 2.85 - 2.64 (m, 5H), 2.39 (d, *J* = 14.4 Hz, 1H), 2.08 *(d, J =* 10.0 Hz, 1H), 1.99 - 1.72 (m, 6H), 1.71-1.65 (m, 1H), 1.54-1.52 (m, 1H), 1.35 (d, *J* = 6.0 Hz, 3H), 0.99 - 0.82 (m, 6H), 0.35 (s, 3H).

### Example 1.13. Synthesis of Compound Z20

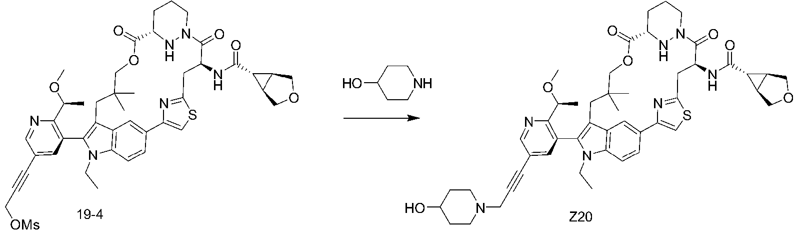

Step 1: Compound 19-4 (10 mg, 0.011 mmol) was dissolved in anhydrous dichloromethane (5 mL), and N,N-diisopropylethylamine (5.92 mg, 0.046 mmol) and 4-hydroxypiperidine (4.63 mg, 0.046 mmol) were added. The mixture was reacted at room temperature for 2 h under a nitrogen atmosphere. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give *(1R,5S,6r)-N-*((6³*S*,4*S,Z*)-1¹-ethyl-(*Rₐ*)-1²-(5-(3-(4-hydroxypiperidin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-ylbicyclo[3.1.0]hexane-6-carboxamide (Z20, 6 mg, 0.007 mmol, yield: 59.65%) as a white solid, ES-API: [M+H]⁺ = 878.3. ¹H NMR (400 MHz, DMSO-*d*₆) δ 8.80 (d, *J =* 2.0 Hz, 1H), 8.55 (d, *J* = 9.0 Hz, 1H), 8.50 (s, 1H), 7.86 - 7.79 (m, 2H), 7.75 (d, *J =* 8.8 Hz, 1H), 7.58 (d, *J* = 8.8 Hz, 1H), 5.56 (t, *J =* 9.0 Hz, 1H), 5.07 (d, *J =* 12.0 Hz, 1H), 4.54 (d, *J =* 4.0 Hz, 1H), 4.39 - 4.00 (m, 5H), 3.86-3.83 (m, 2H), 3.70 - 3.61 (m, 2H), 3.58 (s, 2H), 3.52 (s, 2H), 3.46-3.45 (m, 1H), 3.35-3.33 (m, 1H), 3.25 (s, 3H), 3.17-3.13 (m, 1H), 2.95 (d, *J* = 14.0 Hz, 1H), 2.78-2.75 (m, 3H), 2.39 (d, *J* = 14.0 Hz, 1H), 2.28-2.26 (m, 2H), 2.09-2.06 (m, 1H), 1.94-1.88 (m, 2H), 1.79-1.73 (m, 4H),1.66 - 1.65 (m, 1H), 1.56 - 1.34 (m, 6H), 0.99 - 0.80 (m, 6H), 0.35 (s, 3H).

### Example 1.14. Synthesis of Compound Z21

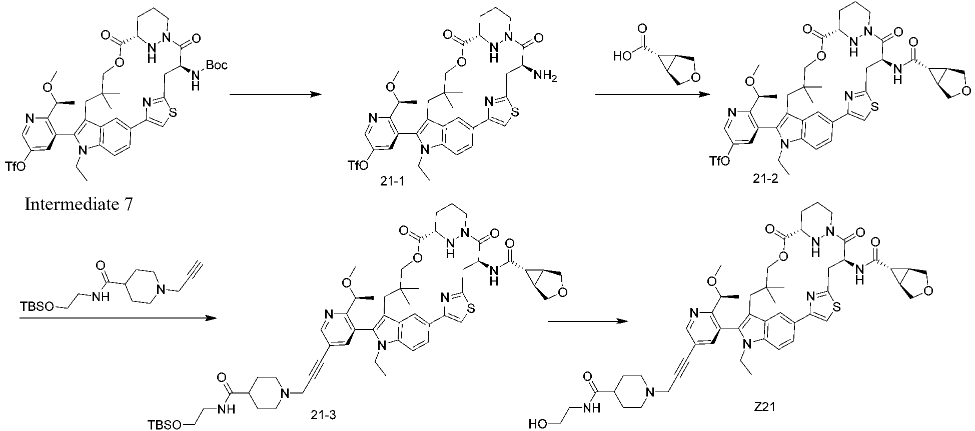

Step 1: Intermediate 7 (1000 mg, 1.138 mmol) was dissolved in methanol (2 mL), and a solution (10 mL) of hydrogen chloride in dioxane was added to the solution described above. After the addition was completed, the reaction system was stirred at room temperature for 1 h. After the reaction was completed, the reaction mixture was concentrated to give a hydrochloride. A saturated aqueous sodium bicarbonate solution (20 mL) was added to the hydrochloride described above, and the mixture was extracted with ethyl acetate (20 mL × 2). The organic phase was separated, washed with saturated brine (20 mL × 2), dried, and concentrated to give compound 21-1 (866 mg, 1.112 mmol, yield: 97.73%) as a light yellow solid. ES-API: [M+H]⁺=779.2.

Step 2: Compound 21-1 (100 mg, 0.128 mmol) was dissolved in dichloromethane (5 mL), and (1R,5S,6r)-3-oxabicyclo[3.1.0]hexane-6-carboxylic acid (24.60 mg, 0.192 mmol), O-(7-azabenzotriazol-1-yl)-*N,N,N',N*'-tetramethyluronium hexafluorophosphate (97.64 mg, 0.257 mmol), and *N,N*'-diisopropylethylamine (49.78 mg, 0.385 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-2%) to give compound 21-2 (100 mg, 0.112 mmol, yield: 87.88%) as a white solid. ES-API: [M +H]⁺=889.3. Step 3: Compound 21-2 (100 mg, 0.112 mmol) was dissolved in acetonitrile (10 mL), and N-(2-((tert-butyldimethylsilyl)oxy)ethyl)-1-(prop-2-yn-1-yl)piperidine-4-carboxamide (73.01 mg, 0.225 mmol), bis(triphenylphosphine)palladium(II) dichloride (15.79 mg, 0.022 mmol), copper(I) iodide (8.57 mg, 0.045 mmol), N,N'-diisopropylethylamine (43.62 mg, 0.337 mmol), and lithium chloride (19.07 mg, 0.450 mmol) were added to the solution described above at room temperature. After the addition was completed, the reaction system was stirred at 80 °C for 4 h under an argon atmosphere. After the reaction was completed, water (20 mL) was added to the reaction mixture, and the mixture was extracted with ethyl acetate (20 mL × 2). The organic phase was washed with saturated brine (30 mL × 2), dried, and concentrated to give a crude product. The crude product was purified by a flash silica gel column (methanol/dichloromethane: 0%-5%) to give compound 21-3 (100 mg, 0.094 mmol, yield: 83.59%) as a light yellow solid. ES-API: [M +H]⁺=1063.3.

Step 4: Compound 21-3 (100 mg, 0.094 mmol) was dissolved in tetrahydrofuran (2 mL), and a solution (4 M, 2 mL) of hydrogen chloride in dioxane was added to the solution described above. After the addition was completed, the reaction system was stirred at room temperature for 2 h. After the reaction was completed, the reaction mixture was concentrated to remove the solvent. A saturated aqueous sodium bicarbonate solution (20 mL) was added to the residue, and the mixture was extracted with ethyl acetate (20 mL × 2). The organic phase was separated, washed with saturated brine (20 mL × 2), dried, and concentrated to give a crude product. The crude product was purified by prep-HPLC (ammonium bicarbonate method 1) to give 1-(3-(5-((6³S,4S,Z)-4-((1R,5S,6r)-3-oxabicyclo[3.1.0]hexane-6-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-6-(Rₐ)-((S)-1-methoxyethyl)pyridin-3-yl)prop-2-yn-1-yl)-N-(2-hydroxyethyl)piperidine-4-carboxamide (Z21, 60 mg, 0.063 mmol, yield: 67.22%) as a white solid. ES-API: [M +H]⁺=949.3. ¹H NMR (300 MHz, CD₃OD) δ 8.81 (d, *J =* 2.1 Hz, 1H), 8.61 (s, 1H), 7.91 (d, *J =* 2.1 Hz, 1H), 7.76 (dd, *J =* 8.7, 1.2 Hz, 1H), 7.60 (s, 1H), 7.53 (d, *J =* 8.7 Hz, 1H), 5.79 (d, *J* = 7.5 Hz, 1H), 4.87 - 4.15 (m, 5H), 3.97 (t, *J* = 8.7 Hz, 2H), 3.81 - 3.76 (m, 4H), 3.67 - 3.56 (m, 4H), 3.49 - 3.44 (m, 1H), 3.39 - 3.35 (m, 5H), 3.15 - 3.08 (m, 3H), 2.87 - 2.75 (m, 1H), 2.65 - 2.55 (m, 1H), 2.45 - 2.30 (m, 2H), 2.34 - 2.09 (m, 4H), 2.02 - 1.56 (m, 9H), 1.48 (d, *J* = 6.0 Hz, 3H), 1.02 - 0.93 (m, 6H), 0.51 (s, 3H).

### Example 1.15. Synthesis of Compound Z22

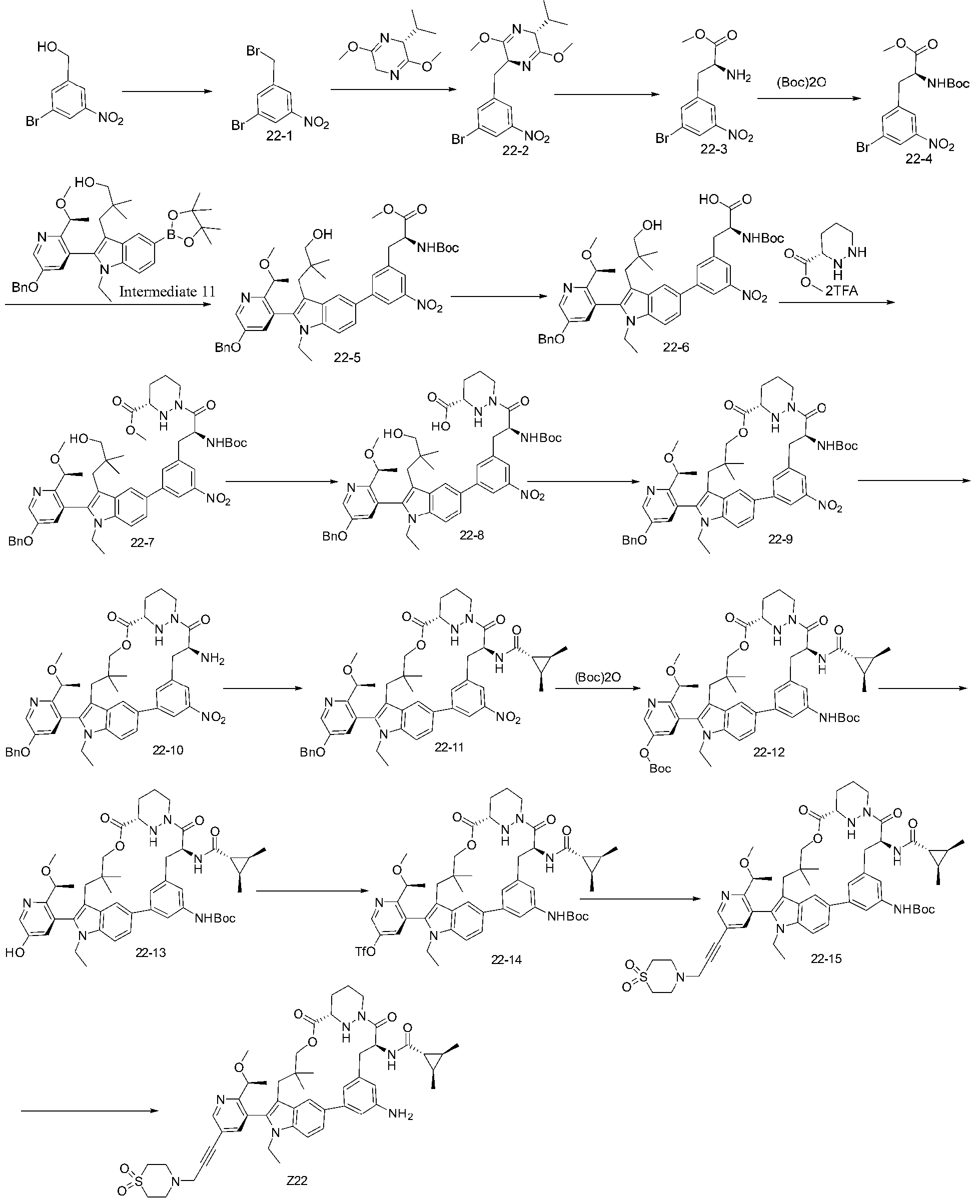

Step 1: (5-Bromo-3-nitrophenyl)methanol (5.0 g, 21.55 mmol) and triphenylphosphine (8.48 g, 32.32 mmol) were dissolved in dichloromethane (70 mL), and carbon tetrabromomethane (7.67 g, 43.10 mmol) was added in portions at 0 °C. The reaction system was stirred at room temperature for 18 h. The reaction mixture was concentrated, and the crude product was purified by a flash silica gel column (ethyl acetate/petroleum ether: 0-10%) to give compound 22-1 (6.2 g, yield: 97.6%) as a white solid. ¹H NMR (400 MHz, CDCl₃) δ 8.31 (t, *J =* 1.6 Hz, 1H), 8.20 (t, *J* = 1.6 Hz, 1H), 7.87 (t, *J =* 1.6 Hz, 1H), 4.48 (s, 2H).

Step 2: (2R)-3,6-Dimethoxy-2-(propan-2-yl)-2,5-dihydropyrazine (6.25 g, 33.91 mmol) was dissolved in dry tetrahydrofuran (50 mL), and 2.5 M n-butyllithium (13.56 mL, 33.91 mmol) was added dropwise at -78 °C. The reaction system was stirred at this temperature for 30 min. In another reaction flask, copper(I) cyanide (1.52 g, 16.95 mmol) was suspended in tetrahydrofuran (20 mL). The mixture was stirred at room temperature for 10 min, then cooled to 0 °C, and stirred for 20 min. The n-butyllithium solution described above was added via a syringe. The mixed reaction mixture was stirred at 0 °C for 15 min, then cooled to -78 °C, and stirred at this temperature for 15 min. A solution of compound 22-1 (5 g, 16.95 mmol) in tetrahydrofuran (10 mL) was added dropwise, and the reaction system was stirred at this temperature for 30 min, then quenched with ammonium chloride/ammonia water (9/1) (50 mL), and extracted with ethyl acetate (100 mL × 2). The organic phases were mixed, washed with saturated brine (100 mL), dried over anhydrous sodium sulfate, and filtered, and the filtrate was concentrated under reduced pressure. The crude product was purified by a flash silica gel column (ethyl acetate/petroleum ether: 0-15%) to give compound 22-2 (3.7 g, yield: 54.8%) as a pale yellow liquid. ES-API: [M+H]⁺= 398.1, 400.1.

Step 3: Compound 22-2 (400 mg, 1.0 mmol) was dissolved in acetonitrile (6 mL), and 0.25 M diluted hydrochloric acid (12.0 mL, 3.0 mmol) was added at room temperature. The reaction system was stirred at room temperature for 3 h. The mixture was concentrated to dryness by rotary evaporation to remove the solvent, adjusted to pH 8 with a saturated sodium bicarbonate solution, and extracted with ethyl acetate (40 mL × 2). The organic phase was dried over anhydrous sodium sulfate and concentrated to give compound 22-3 (304 mg, yield: 100%) as a pale yellow liquid. ES-API: [M+H]⁺= 303.0, 305.0.

Step 4: Compound 22-3 (304 mg, 1.0 mmol) was dissolved in tetrahydrofuran (5 mL) and water (5 mL), and sodium bicarbonate (253 mg, 3.0 mmol) and di-tert-butyl dicarbonate (0.35 mL, 1.50 mmol) were added under an ice bath. The reaction system was stirred at room temperature for 1 h. The reaction mixture was extracted with ethyl acetate (60 mL). The organic phase was washed with saturated brine (30 mL), dried over anhydrous sodium sulfate, and filtered, and the filtrate was concentrated under reduced pressure. The crude product was purified by a flash silica gel column (ethyl acetate/petroleum ether: 0-25%) to give compound 22-4 (300 mg, yield: 74.2%) as a white solid. ¹H NMR (400 MHz, CDCl₃) δ 8.26 (t, *J =* 2.0 Hz, 1H), 7.95 (t, *J =* 2.0 Hz, 1H), 7.62 (t, *J* = 2.0 Hz, 1H), 5.10 (d, *J* = 6.4 Hz, H), 4.59 (dd, *J =* 13.6, 6.4 Hz, 1H), 3.77 (s, 3H), 3.30 (dd, *J =* 13.6, 5.2 Hz, 1H), 3.09 (dd, *J =* 13.6, 6.0 Hz, 1H), 1.43 (s, 9H).ES-API: [M+Na]⁺= 469.9,471.0.

Step 5: Intermediate 11 (50 mg, 0.084 mmol) and compound 22-4 (44 mg, 0.11 mmol) were dissolved in toluene (1.5 mL), dioxane (0.5 mL), and water (0.5 mL), 2-dicyclohexylphosphino-2',4',6'-triisopropylbiphenyl (6 mg, 0.013 mmol), methanesulfonato(2-dicyclohexylphosphino-2',4',6'-triisopropyl-1,1'-biphenyl)(2'-amino-1,1'-biphenyl-2-yl)palladium(II) (7 mg, 0.008 mmol), and potassium phosphate (44 mg, 0.21 mmol) were added, and the reaction system was purged 3 times with nitrogen and stirred at 70 °C for 5 h. Ethyl acetate (50 mL) and water (5 mL) were added to the reaction mixture. The separated organic phase was washed with saturated brine (15 mL), dried over anhydrous sodium sulfate, and filtered, and the filtrate was concentrated under reduced pressure. The crude product was purified by a flash silica gel column (ethyl acetate/petroleum ether: 0-70%) to give compound 22-5 (50 mg, yield: 74.9%) as a pale yellow solid. ES-API: [M+H]⁺= 795.3.

Step 6: Compound 22-5 (50 mg, 0.063 mmol) was dissolved in tetrahydrofuran (2 mL) and water (0.6 mL), and lithium hydroxide monohydrate (5 mg, 0.126 mmol) was added at 0 °C. The reaction system was stirred at room temperature for 2 h. The reaction mixture was adjusted to pH 6 with 1 M diluted hydrochloric acid and extracted with ethyl acetate (15 mL × 2). The organic phase was dried over anhydrous sodium sulfate and concentrated to give compound 22-6 (49 mg, yield: 100%) as a pale yellow solid. ES-API: [M+H]⁺= 781.3.

Step 7: Compound 22-6 (49 mg, 0.063 mmol) was dissolved in dichloromethane (5 mL), and methyl (S)-hexahydropyridazine-3-carboxylate ditrifluoroacetate (47 mg, 0.125 mmol), O-(7-azabenzotriazol-1-yl)-N,N,N',N'-tetramethyluronium hexafluorophosphate (36 mg, 0.094 mmol), and N,N'-diisopropylethylamine (49 mg, 0.376 mmol) were added at 0 °C. The reaction system was stirred for 1 h under an ice bath. Water (15 mL) was added to the reaction mixture, and the mixture was extracted with dichloromethane (25 mL). The organic phase was washed with saturated brine (10 mL), dried over anhydrous sodium sulfate, and filtered, and the filtrate was concentrated under reduced pressure. The crude product was purified by a flash silica gel column (tetrahydrofuran/petroleum ether: 0-50%) to give compound 22-7 (50 mg, yield: 87.8%) as a yellow solid. ES-API: [M+H]⁺= 907.3.

Step 8: Compound 22-7 (50 mg, 0.055 mmol) was dissolved in tetrahydrofuran (2 mL) and water (0.6 mL), and lithium hydroxide monohydrate (4 mg, 0.095 mmol) was added at 0 °C. The reaction system was stirred at room temperature for 1 h. The reaction mixture was adjusted to pH 6 with 1 M diluted hydrochloric acid and extracted with ethyl acetate (15 mL × 2). The organic phase was dried over anhydrous sodium sulfate and concentrated to give compound 22-8 (49 mg, yield: 100%) as a pale yellow solid. ES-API: [M+H]⁺= 893.3.

Step 9: Compound 22-8 (1.7 g, 1.90 mmol) was dissolved in dichloromethane (200 mL), and 1-(3-dimethylaminopropyl)-3-ethylcarbodiimide hydrochloride (10.22 g, 53.30 mmol), 1-hydroxybenzotriazole (1.29 g, 9.52 mmol), and diisopropylethylamine (7.38 g, 57.11 mmol) were added under an ice bath. The reaction system was stirred at room temperature for 18 h. The reaction mixture was washed with water (80 mL × 2) and saturated brine (80 mL), dried over anhydrous sodium sulfate, and filtered, and the filtrate was concentrated under reduced pressure. The crude product was purified by a flash silica gel column (ethyl acetate/petroleum ether: 0-70%) to give compound 22-9 (1.3 g, yield: 78.0%) as a yellow solid. ES-API: [M+H]⁺=875.3.

Step 10: Compound 22-9 (500 mg, 0.57 mmol) was dissolved in absolute methanol (2 mL), and a 4.0 M hydrogen chloride/dioxane solution (8 mL) was added at 0 °C. The reaction system was stirred at room temperature for 1 h. The reaction mixture was concentrated to give compound 22-10 (442 mg, yield: 100%) as a white solid. ES-API: [M+H]⁺= 775.3.

Step 11: Compound 22-10 (425 mg, 0.55 mmol) was dissolved in dichloromethane (20 mL), and (1r,2R,3S)-2,3-dimethylcyclopropane-1-carboxylic acid (94 mg, 0.82 mmol), O-(7-azabenzotriazol-1-yl)-N,N,N',N'-tetramethyluronium hexafluorophosphate (417 mg, 1.10 mmol), and N,N'-diisopropylethylamine (425 mg, 3.29 mmol) were added. The reaction system was stirred at room temperature for 1 h. Dichloromethane (25 mL) was added to the reaction mixture. The organic phase was washed sequentially with water (10 mL) and saturated brine (10 mL), dried over anhydrous sodium sulfate, and filtered, and the filtrate was concentrated under reduced pressure. The crude product was purified by a flash silica gel column (ethyl acetate/petroleum ether: 0-100%) to give compound 22-11 (450 mg, yield: 94.2%) as a yellow liquid. ES-API: [M+H]⁺= 871.3.

Step 12: Compound 22-11 (100 mg, 0.115 mmol) and di-tert-butyl dicarbonate (62 mg, 0.28 mmol) were dissolved in absolute methanol (10 mL), and 10% palladium hydroxide on carbon (100 mg) was added at room temperature. The reaction system was stirred at 30 °C for 18 h under a hydrogen atmosphere. The reaction mixture was filtered through celite and washed with methanol, and the filtrate was concentrated to give compound 22-12 (105 mg, yield: 95.5%) as a white solid. ES-API: [M+H]⁺=951.3.

Step 13: Compound 22-12 (75 mg, 0.079 mmol) was dissolved in methanol (8 mL), and potassium carbonate (22 mg, 0.158 mmol) was added at 0 °C. The reaction system was stirred at 0 °C for 3 h. The reaction mixture was poured into ice water, and the pH was adjusted to 7 with 1 M diluted hydrochloric acid. The mixture was concentrated by rotary evaporation to remove the organic solvent, and the mixture was extracted with ethyl acetate (30 mL). The organic phase was washed with saturated brine (10 mL), dried over anhydrous sodium sulfate, and filtered, and the filtrate was concentrated under reduced pressure. The crude product was purified by a flash silica gel column (tetrahydrofuran/petroleum ether: 0-80%) to give compound 22-13 (50 mg, yield: 74.5%) as a white solid. ES-API:[M+H]⁺= 851.3.

Step 14: Compound 22-13 (50 mg, 0.059 mmol) was dissolved in dichloromethane (5 mL), and diisopropylethylamine (23 mg, 0.176 mmol) and 1,1,1-trifluoro-N-phenyl-N-[(trifluoromethyl)sulfonyl]methanesulfonamide (31 mg, 0.088 mmol) were added. The reaction system was stirred at room temperature for 2 h. The reaction mixture was concentrated. The crude product was purified by a flash silica gel column (tetrahydrofuran/petroleum ether: 0-50%) to give compound 22-14 (50 mg, yield: 86.6%) as a white solid. ES-API: [M+H]⁺=983.3.

Step 15: Compound 22-14 (50 mg, 0.051 mmol) was dissolved in N,N-dimethylformamide (2.5 mL), and 4-prop-2-ynyl-thiomorpholine-1,1-dioxide (35 mg, 0.203 mmol), bis(triphenylphosphine)palladium(II) dichloride (11 mg, 0.015 mmol), copper(I) iodide (6 mg, 0.031 mmol), N,N'-diisopropylethylamine (39 mg, 0.305 mmol), and lithium chloride (9 mg, 0.203 mmol) were added. The reaction system was stirred at 85 °C for 3 h under a nitrogen atmosphere. Water (20 mL) was added to the reaction mixture, and the mixture was extracted with ethyl acetate (30 mL × 2). The organic phase was washed with saturated brine (20 mL × 2), dried, and concentrated to give a crude product. The crude product was purified by a flash silica gel column (tetrahydrofuran/petroleum ether: 0-70%) to give compound 22-15 (40 mg, yield: 78.2%) as a pale brown solid. ES-API: [M+H]⁺=1006.3. Step 16: Compound 22-15 (75 mg, 0.075 mmol) was dissolved in dichloromethane (4 mL), and trifluoroacetic acid (1.5 mL) was added at 0 °C. The reaction system was stirred at room temperature for 3 h. The reaction mixture was concentrated, and a saturated sodium bicarbonate solution (5 mL) was added. The mixture was extracted with dichloromethane (15 mL × 2). The organic phase was dried over anhydrous sodium sulfate and filtered, and the filtrate was concentrated under reduced pressure. The crude product was subjected to prep-HPLC (ammonium bicarbonate method 1) to give the desired product (1r,2R,3S)-N-((6³S,4S)-2⁵-amino-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-1(5,3)-indola-6(1,3)-pyridazina-2(1,3)-benzenacycloundecaphane-4-yl)-2,3-dimethylcyclopropane-1-carboxamide (Z22, 23 mg, yield: 34.0%) as a white solid. ES-API: [M+H]⁺= 906.3.

### Example 1.16. Synthesis of Compound Z23

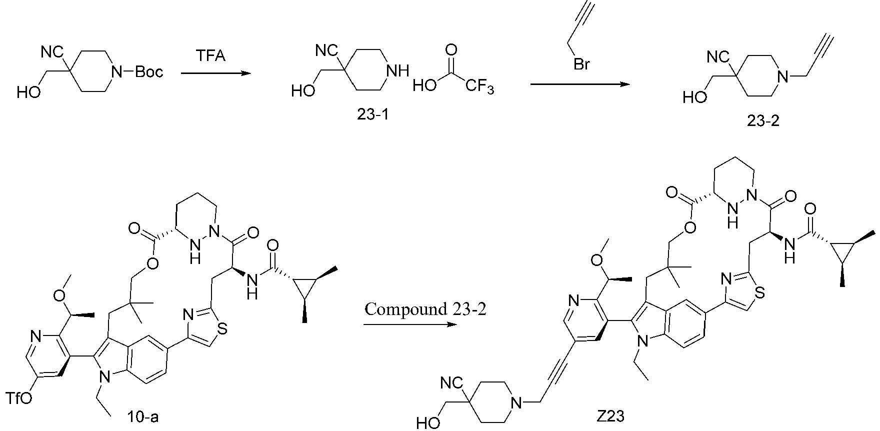

Step 1: tert-Butyl 4-cyano-4-(hydroxymethyl)-1-piperidinecarboxylate (500 mg, 2.301 mmol) was dissolved in dichloromethane (2 mL), and trifluoroacetic acid (2 mL, 2.301 mmol) was added. The mixture was reacted for 1 h under a nitrogen atmosphere and concentrated under reduced pressure to give compound 23-1 (500 mg, 1.967 mmol, yield: 94.53%) as an oily liquid, ES-API: [M+H]⁺ = 141.3.

Step 2: Compound 23-1 (500 mg, 1.967 mmol) was dissolved in anhydrous acetonitrile (15 mL), and anhydrous potassium carbonate (815.47 mg, 5.901 mmol) and 3-bromopropyne (233.98 mg, 1.967 mmol) were added. The mixture was reacted for 3 h under a nitrogen atmosphere, filtered under vacuum, and concentrated under reduced pressure. The crude product was purified by a flash silica gel column (ethyl acetate/petroleum ether: 0%-50%) to give compound 23-2 (150 mg, 0.842 mmol, yield: 42.79%) as a white solid, ES-API: [M+H]⁺ = 179.2.

Step 3: Compound 10-a (40.0 mg, 0.046 mmol) was dissolved in acetonitrile (3 mL), and 23-2 (16.3 mg, 0.091 mmol), bis(triphenylphosphine)palladium(II) dichloride (12.82 mg, 0.018 mmol), copper(I) iodide (5.27 mg, 0.027 mmol), N,N'-diisopropylethylamine (23.59 mg, 0.183 mmol), and anhydrous lithium chloride (7.68 mg, 0.183 mmol) were added. The mixture was reacted at 80 °C for 5 h under a nitrogen atmosphere. After the reaction was completed, the mixture was filtered under vacuum and concentrated under reduced pressure to give a crude product. The crude product was purified by a flash silica gel column (methanol/dichloromethane: 0%-10%) to give (1*r*,2*R*,3*S*)-N-((6³*S*,4*S*,*Z*)-(*Rₐ*)-1²-(5-(3-(4-cyano-4-(hydroxymethyl)piperidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z23, 22 mg, 0.024 mmol, yield: 53.28%) as a white solid, ES-API: [M+H]⁺ = 903.3. ¹H NMR (400 MHz, DMSO-*d*₆) δ 8.80 (s, 1H), 8.50 (s, 1H), 8.40 (d, *J =* 8.8 Hz, 1H), 7.83 (d, *J =* 8.0 Hz, 2H), 7.75 (d, *J* = 8.8 Hz, 1H), 7.58 (d, *J =* 8.8 Hz, 1H), 5.56 (t, *J =* 9.0 Hz, 1H), 5.43 (t, *J* = 5.6 Hz, 1H), 5.07 (d, *J =* 12.0 Hz, 1H), 4.41 - 4.05 (m, 5H), 3.62 (s, 2H), 3.57 (s, 2H), 3.46 (d, *J =* 5.6 Hz, 2H), 3.38-3.36 (m, 1H),3.32-3.30 (m, 1H), 3.25 (s, 3H), 3.15 -3.13(m, 1H), 2.92-2.81 (m, 3H), 2.80-2.75 (m, 1H), 2.46-2.42 (m, 2H), 2.11 - 1.94 (m, 2H), 1.86-1.84 (m, 2H), 1.80-1.78 (m, 2H), 1.58-1.53 (m, 3H), 1.40 - 1.33 (m, 5H), 1.10-1.26 (m, 6H), 0.93 - 0.83 (m, 6H), 0.34 (s, 3H).

### Example 1.17. Synthesis of Compounds Z24-1 and Z24-2

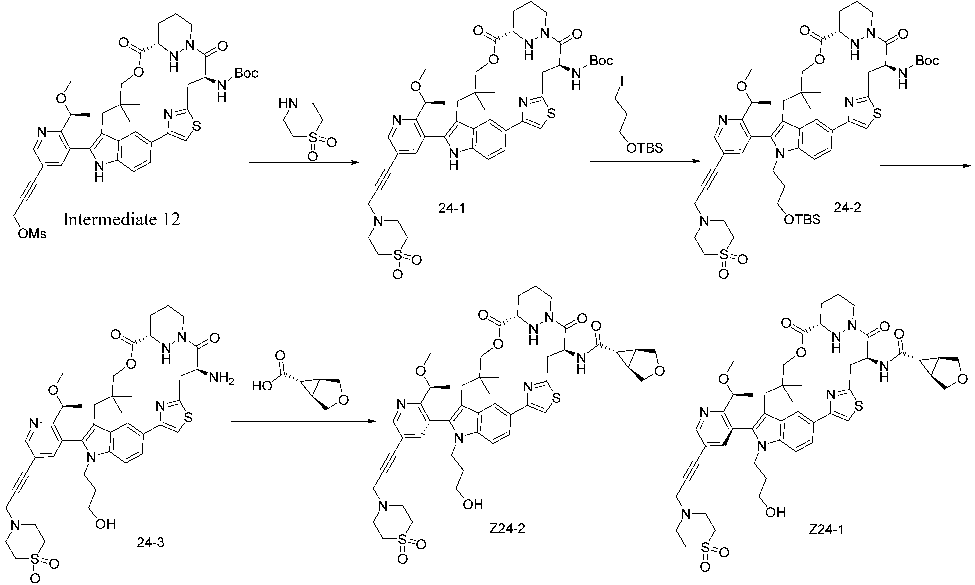

Step 1: Intermediate 12 (1900 mg, 2.275 mmol) was dissolved in dichloromethane (10 mL), and thiomorpholine-1,1-dioxide (1384.18 mg, 10.240 mmol) was added. The mixture was heated to 40 °C and reacted for 3 h. The mixture was diluted with ethyl acetate (100 mL) and water (50 mL), washed with saturated brine (50 mL × 3), dried over anhydrous sodium sulfate, filtered, and concentrated. The crude product was purified by automated flash chromatography on silica gel (methanol/dichloromethane = 0-6%) to give compound 24-1 (1.8 g, 2.059 mmol, yield: 90.5%). ES-API: [M+H]⁺=874.3.

Step 2: Cesium carbonate (4.03 g, 12.356 mmol) and tert-butyl(3-iodopropoxy)dimethylsilane (1.24 g, 4.119 mmol) were sequentially added to a solution (15 mL) of compound 24-1 (1.8 g, 2.059 mmol) in N,N-dimethylformamide, and the reaction mixture was stirred at 40 °C for 3 h. The mixture was diluted with ethyl acetate (100 mL) and water (50 mL), washed with saturated brine (50 mL × 3), dried over anhydrous sodium sulfate, filtered, and concentrated. The crude product was purified by automated flash chromatography on silica gel (methanol/dichloromethane = 0-6%) to give compound 24-2 (1.5 g, yield: 69.6%). ES-API: [M+H]⁺=1046.4. Step 3: Compound 24-2 (200 mg, 0.191 mmol) was dissolved in methanol (2 mL), and a 4 M solution (5 mL) of hydrochloric acid in dioxane was added at room temperature. The mixture was reacted for 1 h and then concentrated to give compound 24-3 (130 mg, crude product). ES-API: [M+H]⁺=832.2.

Step 4: Compound 24-3 (130 mg, crude product) was dissolved in dichloromethane (10 mL), and (1R,5S,6r)-3-oxabicyclo[3.1.0]hexane-6-carboxylic acid (30 mg, 0.235 mmol), DIPEA (100.9 mg, 0.782 mmol), and O-(7-azabenzotriazol-1-yl)-N,N,N',N'-tetramethyluronium hexafluorophosphate (89.3 mg, 0.235 mmol) were sequentially added. The mixture was reacted at room temperature for 1 h, diluted with ethyl acetate (20 mL), washed sequentially with water (20 mL × 3) and saturated brine (20 mL), dried over anhydrous sodium sulfate, filtered, and concentrated, and the residue was purified and separated by prep-HPLC (ammonium bicarbonate method 1) to give 2 isomers. The structure of one of the isomeric compounds was: (1R,5S,6r)-N-((6³S,4S,Z)-1²-(5-(3-(1,1-dioxidothiomorpholino)prop-1-yn-1-yl)-(Sₐ)-2-((S)-1-methoxyethyl)-pyridin-3-yl)-1'-(3-hydroxypropyl)-10,10-dimethyl-5,7-dioxo-6¹,6²,6³,6⁴,6⁵,6⁶-hexahydro-1¹ H-8-oxa-2(4,2)-thiazola-1(5,3)-indola-6(1,3)-pyridazinacycloundecaphane-4-yl)-3-oxabicyclo[3.1.0]hexane-6-carboxamide (Z24-2, 24 mg, two-step yield: 13.3%, retention time: 9.08 min), ES-API: [M+H]⁺ = 942.4. ¹H NMR (400 MHz, DMSO-*d*₆) δ 8.82 (d, J = 2.1 Hz, 1H), 8.58 (d, J = 8.9 Hz, 1H), 8.52 (d, J = 1.7 Hz, 1H), 7.99 (d, J = 2.2 Hz, 1H), 7.81 (s, 1H), 7.74 (dd, J = 8.7, 1.6 Hz, 1H), 7.54 (d, J = 8.7 Hz, 1H), 5.54 (t, J = 9.1 Hz, 1H), 5.05 (d, J = 12.1 Hz, 1H), 4.50 (t, J = 4.9 Hz, 1H), 4.22 (td, J = 12.2, 3.3 Hz, 2H), 4.03-3.96 (m, 1H), 3.93-3.88 (m, 1H), 3.86-3.81 (m, 3H), 3.76 (s, 2H), 3.68-3.62 (m, 3H), 3.55 (d, J = 11.0 Hz, 1H), 3.35-3.32 (m, 3H), 3.17 (t, J = 5.3 Hz, 5H), 3.09 - 3.02 (m, 8H), 2.80-2.73 (m, 1H), 2.31 (d, J = 14.2 Hz, 1H), 2.12 (d, J = 12.0 Hz, 1H), 1.98-1.95 (m, 1H), 1.92-1.89 (m, 1H), 1.84-1.76 (m, 2H), 1.70-1.65(m, 2H), 1.62 - 1.49 (m, 2H), 1.23 (d, J = 6.2 Hz, 3H), 0.93 (s, 3H), 0.51 (s, 3H). The structure of the other isomeric compound was (1R,5S,6r)-N-((6³S,4S,Z)-1²-(5-(3-(1,1-dioxidothiomorpholino)prop-1-yn-1-yl)-(Rₐ)-2-((S)-1-methoxyethyl)-pyridin-3-yl)-1¹-(3-hydroxypropyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z24-1, 26 mg, two-step yield: 14.4%, retention time: 9.21 min), ES-API: [M+H]⁺ = 942.4.

### Example 1.18. Synthesis of Compound Z30

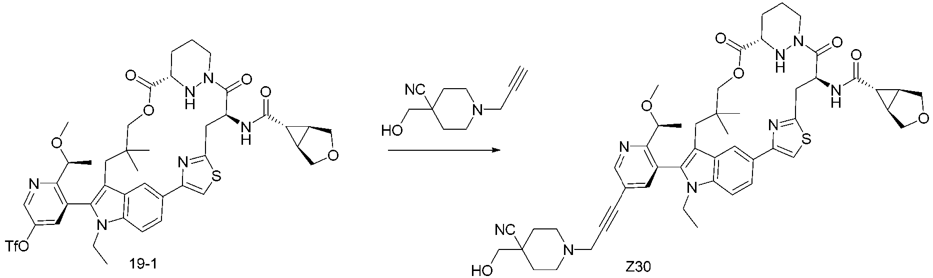

Step 1: Compound 19-1 (50 mg, 0.056 mmol) was dissolved in acetonitrile (4 mL). 4-(Hydroxymethyl)-1-(prop-2-yn-1-yl)piperidine-4-carbonitrile (compound 23-2, 30.07 mg, 0.169 mmol), bis(triphenylphosphine)palladium(II) dichloride (7.90 mg, 0.011 mmol), copper(I) iodide (4.28 mg, 0.022 mmol), *N,N'*-diisopropylethylamine (21.81 mg, 0.169 mmol), and lithium chloride (9.54 mg, 0.225 mmol) were added to the solution described above at room temperature. After the addition was completed, the reaction system was stirred at 80 °C for 4 h under a nitrogen atmosphere. After the reaction was completed, water (10 mL) was added to the reaction mixture, and the mixture was extracted with ethyl acetate (10 mL × 2). The organic phase was washed with saturated brine (10 mL × 2), dried over anhydrous sodium sulfate, filtered, and concentrated to give a crude product. The crude product was purified by a flash silica gel column (methanol/dichloromethane: 0%-5%) to give (1*R*,5*S*,6*r*)-N-((6³*S*,4*S*,*Z*)-(*Rₐ*)-1²-(5-(3-(4-cyano-4-(hydroxymethyl)piperidin-1-yl)prop-1-yn-1-yl)-2-((S)-1-methoxyethyl)pyridin-3-yl)-11-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (Z30, 24 mg, 0.026 mmol, yield: 46.53%) as a white solid. ES-API: [M +H]⁺=917.3.

### Example 1.19. Synthesis of Compound Z27

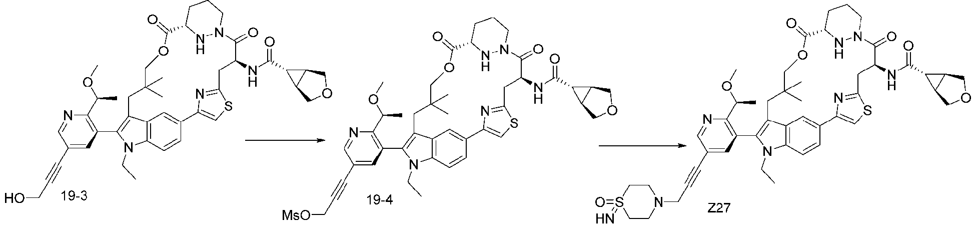

Step 1: N,N-Diisopropylethylamine (29.27 mg, 0.226 mmol) and methanesulfonic anhydride (14.45 mg, 0.083 mmol) were added to a solution of compound 19-3 (60.0 mg, 0.075 mmol) in dichloromethane (5 mL) at room temperature, and the mixture was stirred at room temperature for 0.5 h. The mixture was concentrated to dryness by rotary evaporation under reduced pressure to remove the solvent to give compound 19-4 (70.0 mg, crude product), which was directly used in the next step. ES-API: [M+H]⁺= 873.3.

Step 2: N,N-Diisopropylethylamine (38.7 mg, 0.300 mmol), sodium iodide (11.3 mg, 0.075 mmol), and 1-iminothiomorpholine-1-oxide hydrochloride (25.5 mg, 0.170 mmol) were sequentially added to a solution of compound 19-4 (70.0 mg, crude product) in acetonitrile (10.0 mL). The mixture was stirred at 70 °C for 3 h. When the reaction was completed as detected by LCMS, the mixture was concentrated under reduced pressure to remove the solvent. The residue was purified by prep-HPLC (ammonium bicarbonate method 1) to give (1R,5S,6r)-N-((6³S,4S,Z)-1¹-ethyl-(*Rₐ*)-1²-(5-(3-(1-imino-1-oxido-1λ⁶-thiomorpholino)prop-1-yn-1-yl-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z27, 18.0 mg, yield: 26.34%). ES-API: [M+H]⁺= 911.3.

### Example 1.20. Synthesis of Compound Z29

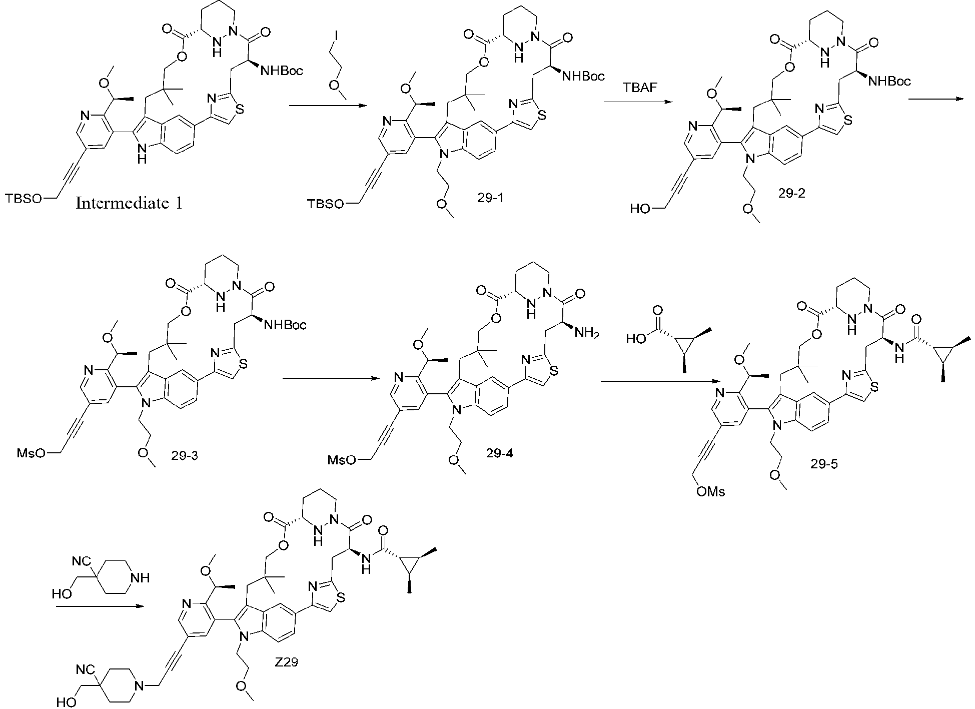

Step 1: Intermediate 1 (700 mg, 0.804 mmol) was dissolved in N,N-dimethylformamide (5 mL), cesium carbonate (785.88 mg, 2.412 mmol) and 1-iodo-2-methoxyethane (448.61 mg, 2.412 mmol) were added, and the mixture was reacted at room temperature for 15 h. After the reaction was completed, water (15 mL) was added, and the mixture was extracted with ethyl acetate (20 mL × 3). The organic phase was washed with saturated brine (15 mL), dried over anhydrous sodium sulfate, and concentrated. The crude product was purified by a flash silica gel column (tetrahydrofuran/petroleum ether: 0%-30%) to give compound 29-1 (700 mg, 0.753 mmol, yield: 93.75%), ES-API: [M+H]⁺ = 929.3.

Step 2: Compound 29-1 (500 mg, 0.550 mmol) was dissolved in tetrahydrofuran (3 mL), and a solution (1 M, 0.825 mL, 0.825 mmol) of tetrabutylammonium fluoride in tetrahydrofuran was added. The mixture was reacted at room temperature for 1 h under a nitrogen atmosphere. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give compound 29-2 (540 mg, 0.663 mmol, yield: 90.54%) as a pale yellow solid, ES-API: [M+H]⁺ = 815.3.

Step 3: Compound 29-2 (400 mg, 0.491 mmol) was dissolved in dichloromethane (5 mL), and methanesulfonic anhydride (102.48 mg, 0.589 mmol) and N,N'-diisopropylethylamine (190.31 mg, 1.472 mmol) were added. The mixture was reacted at room temperature for 1 h under a nitrogen atmosphere and concentrated to dryness under reduced pressure. The crude product was purified by a flash silica gel column (methanol/dichloromethane: 0%-10%) to give compound 29-3 (350 mg, 0.392 mmol, yield: 79.85%) as a pale yellow solid, ES-API: [M+H]⁺ *=* 893.3.

Step 4: Compound 29-3 (60 mg, 0.067 mmol) was dissolved in methanol (0.5 mL), and a solution (4 *M,* 1 mL) of hydrogen chloride in dioxane was added to the solution described above. The mixture was stirred at room temperature for 2 h. After the reaction was completed, the reaction system was concentrated to give crude compound 29-4 (55 mg, 0.066 mmol, yield: 98.97%) as a yellow solid. ES-API: [M +H]⁺=793.2.

Step 5: Crude compound 29-4 (50 mg, 0.063 mmol) was dissolved in dichloromethane (2 mL). (1r,2R,3S)-2,3-Dimethylcyclopropane-1-carboxylic acid (9.19 mg, 0.067 mmol), O-(7-azabenzotriazol-1-yl)-*N,N,N',N*'-tetramethyluronium hexafluorophosphate (35.93 mg, 0.094 mmol), and *N,N*'-diisopropylethylamine (0.045 mL, 0.252 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0-5%) to give compound 29-5 (49 mg, 0.055 mmol, yield: 87.41%) as a yellow solid. ES-API: [M +H]⁺=889.3. Step 6: Compound 29-5 (35 mg, 0.039 mmol) was dissolved in dichloromethane (5 mL), and N,N'-diisopropylethylamine (15 mg, 0.117 mmol) and 4-(hydroxymethyl)piperidine-4-carbonitrile (16.40 mg, 0.117 mmol) were added. The mixture was reacted at room temperature for 3 h under a nitrogen atmosphere and concentrated to dryness under reduced pressure. The crude product was purified by a flash silica gel column (methanol/dichloromethane: 0%-10%) to give (1*r*,2*R*,3*S*)-N-((6³*S*,4*S*,*Z*)-1²-(5-(3-(4-cyano-4-(hydroxymethyl)piperidin-1-yl)prop-1-yn-1-yl)-2-((S)-1-methoxyethyl)pyridin-3-yl)-1¹-(methoxyethyl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2,3-dimethylcyclopropane-1-carboxamide (Z29, 13 mg, 0.014 mmol, yield: 35.39%) as a white solid, ES-API: [M+H]⁺ = 933.3.

### Example 1.21. Synthesis of Compound Z34

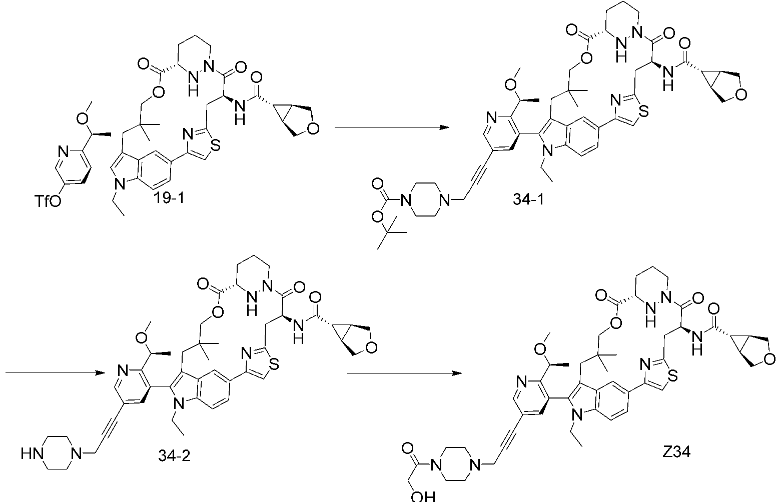

Step 1: Compound 19-1 (150 mg, 0.17 mmol), 2-methylprop-2-yl-4-(prop-2-ynyl)piperazine-1-carboxylate (77 mg, 0.34 mmol, CAS: 199538-99-3), Pd(PPh₃)₂Cl₂ (24 mg, 0.03 mmol), CuI (20 mg, 0.1 mmol), and DIPEA (88 mg, 0.68 mmol) were mixed in DMF (10 mL) under a nitrogen atmosphere, and the mixture was stirred at 80 °C for 10 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 34-1 (120 mg, yield: 74%). ES-API[M+H]⁺=963.3.

Step 2: Trifluoroacetic acid (3 mL) was added to a solution of compound 34-1 (260 mg, 0.27 mmol) in dichloromethane (5 mL), and the mixture was stirred at room temperature for 2 h. After the reaction was completed, the mixture was concentrated, and the residue was basified with a saturated sodium bicarbonate solution to pH = 8, extracted three times with 20 mL of ethyl acetate, and concentrated to give compound 34-2 (220 mg, yield: 94%). ES-API[M+H]⁺=863.3.

Step 3: Hydroxyacetic acid (5 mg, 0.07 mmol) and DIPEA (46 mg, 0.36 mmol) were added to a solution of compound 34-2 (30 mg, 0.04 mmol) in dichloromethane (5 mL), and O-(7-azabenzotriazol-1-yl)-N,N,N',N'-tetramethyluronium hexafluorophosphate (68 mg, 0.18 mmol) was added under an ice bath. The mixture was stirred at room temperature for 1 h. After the reaction was completed, the mixture was extracted with ethyl acetate (20 mL × 3), and the combined extract was washed with 30 mL of brine. The organic phase was dried and concentrated, and then the residue was purified by preparative thin-layer chromatography (dichloromethane/methanol *=* 20/1) to give (1R,5S,6r)-N-((6³S,4S,Z)-1¹-ethyl-1²-(5-(3-(4-(2-hydroxyacetyl)piperazin-1-yl)prop-1-yn-1-yl)-(Rₐ)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z34, 5 mg, yield: 16%, purity: 98%). ES-API[M+H]⁺=921.3. ¹HNMR (400 MHz, DMSO-*d*₆) 8.81 (1 H, d, *J* 2.0)*,* 8.57 (1 H, d, *J* 8.9), 8.50 (1 H, s), 7.86 (1 H, d, *J* 2.0), 7.81 (1 H, s), 7.75 (1 H, d, *J* 8.6), 7.58 (1 H, d, *J* 8.7)*,* 5.56 (1 H, t, *J* 9.1), 5.08 (1 H, *d, J12.2), 4.54* (1 H, s), 4.39 - 3.98 (7 H, m), 3.83 (2 H, t, *J* 8.2), 3.69 - 3.48 (8 H, m), 3.32 - 3.28 (3 H, m), 3.25 (3 H, s), 3.13 (2 H, dd, *J* 14.7, 9.1), 2.95 (1 H, d, *J* 14.1), 2.75 (1 H, s), 2.52 (3 H, s), 2.38 (1 H, d, *J* 14.5), 2.08 (1 H, d, *J* 10.2), 1.98 - 1.86 (2 H, m), 1.78 (2 H, s), 1.65 (1 H, t, *J* 3.0), 1.52 (1 H, d, *J* 10.4), 1.35 (3 H, d, *J* 6.0), 0.88 (6 H, dd, *J* 13.2, 6.0), 0.34 (3 H, s).

### Example 1.22. Synthesis of Compounds Z38, Z38-1, and Z38-2

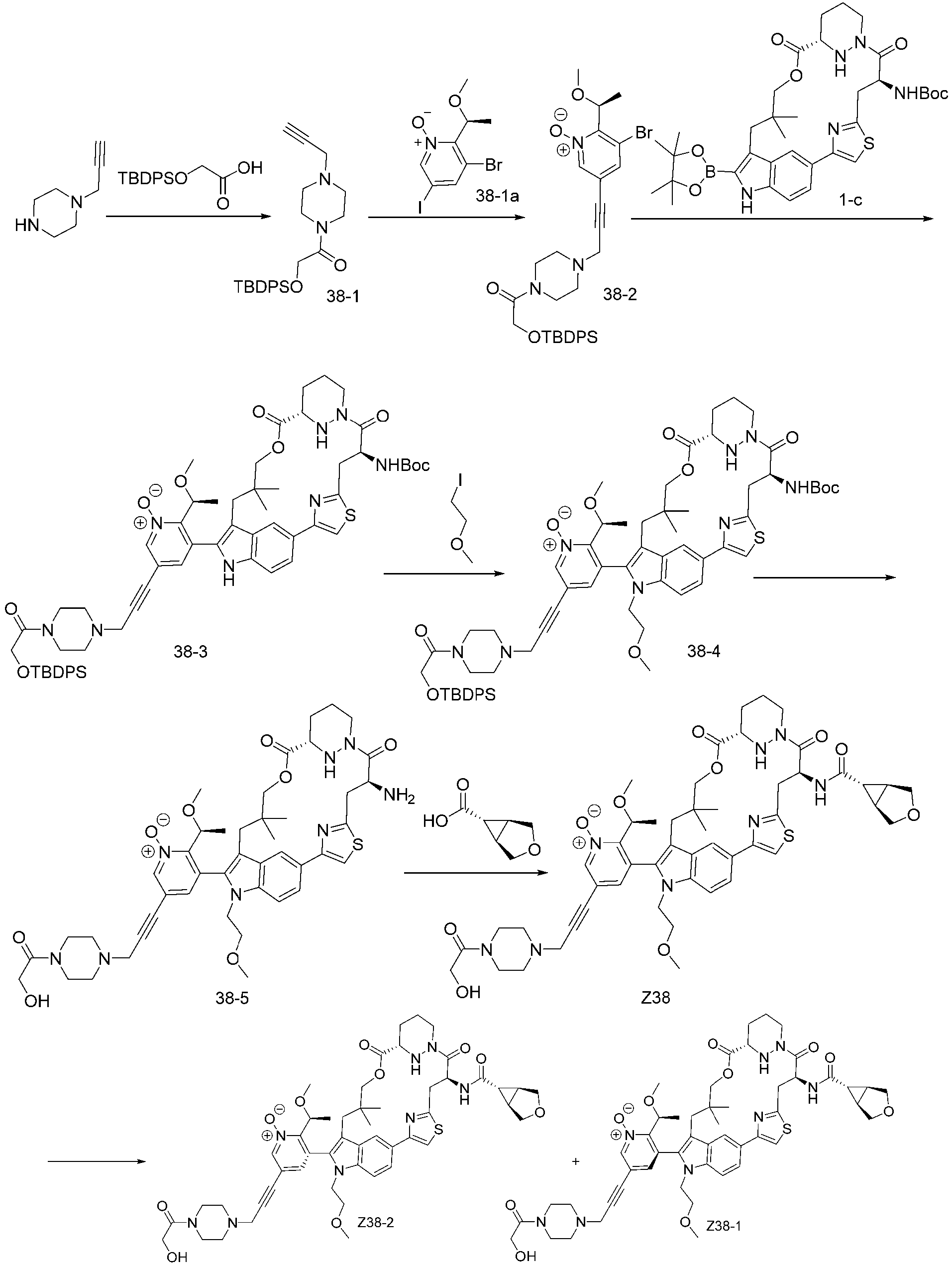

Step 1: 1-(2-Propynyl)piperazine (400 mg, 3.22 mmol) and 2-((tert-butyldiphenylsilyl)oxy)acetic acid (1.01 g, 3.22 mmol) were dissolved in dichloromethane (30 mL). Triethylamine (1.79 mL, 12.88 mmol) and a 50% solution (4.10 g, 6.44 mmol) of 1-propylphosphoric cyclic anhydride in ethyl acetate were added at room temperature. The reaction system was stirred at room temperature for 2 h. Dichloromethane (50 mL) was added to the reaction mixture. The resulting mixture was washed sequentially with water (30 mL) and saturated brine (30 mL), dried over anhydrous sodium sulfate, and filtered. The filtrate was concentrated under reduced pressure. The crude product was purified by a flash silica gel column (tetrahydrofuran/petroleum ether: 0-40%) to give compound 38-1 (780 mg, yield: 57.6%) as a colorless liquid. ES-API: [M+H]⁺=421.1.

Step 2: Intermediate 38-1a (500 mg, 1.40 mmol), bis(triphenylphosphine)palladium(II) dichloride (49 mg, 0.07 mmol), and copper(I) iodide (27 mg, 0.14 mmol) were dissolved in acetonitrile (10 mL) under a nitrogen atmosphere. A solution of compound 38-1 (646 mg, 1.54 mmol) and N,N-diisopropylethylamine (361 mg, 2.79 mmol) in acetonitrile (5 mL) was added dropwise under an ice bath. The reaction system was stirred at room temperature for 1 h. Ethyl acetate (100 mL) was added to the reaction mixture, and the mixture was washed with saturated brine (30 mL). The organic phase was dried over anhydrous sodium sulfate and filtered, and the filtrate was concentrated under reduced pressure. The crude product was purified by a flash silica gel column (methanol/dichloromethane: 0-6%) to give compound 38-2 (700 mg, yield: 77.0%) as a yellow liquid. ES-API:[M+H]⁺= 650.2, 652.2.

Step 3: Compound 1-c (300 mg, 0.43 mmol) and compound 38-2 (422 mg, 0.65 mmol) were dissolved in dioxane (2 mL), toluene (6 mL), and water (2 mL) under a nitrogen atmosphere, and [1,1'-bis(di-tert-butylphosphino)ferrocene]dichloropalladium(II) (56 mg, 0.086 mmol) and potassium phosphate (183 mg, 0.87 mmol) were added. The reaction system was stirred at 75 °C for 3 h. Ethyl acetate (50 mL) was added to the reaction mixture, and the mixture was washed with saturated brine (15 mL). The organic phase was dried over anhydrous sodium sulfate and filtered, and the filtrate was concentrated under reduced pressure. The crude product was purified by a flash silica gel column (methanol/dichloromethane: 0-8%) to give compound 38-3 (350 mg, yield: 71.2%) as a pale brown solid. ES-API:[M+H]⁺= 1137.4.

Step 4: Compound 38-3 (300 mg, 0.26 mmol) was dissolved in N,N-dimethylformamide (15 mL), and cesium carbonate (258 mg, 0.79 mmol) and 1-iodo-2-methoxyethane (147 mg, 0.79 mmol) were added at room temperature. The reaction system was stirred at room temperature for another 48 h. Ethyl acetate (50 mL) was added to the reaction mixture, and the mixture was washed with saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-5%) to give compound 38-4 (250 mg, yield: 79.3%) as a pale brown solid. ES-API:[M+H]⁺= 1195.4.

Step 5: Compound 38-4 (200 mg, 0.107 mmol) was dissolved in dichloromethane (5 mL), and trifluoroacetic acid (2 mL) was added at 0 °C. The reaction system was stirred at room temperature for 3 h. The reaction mixture was concentrated, and a saturated sodium bicarbonate solution (5 mL) was added. The mixture was extracted with dichloromethane (15 mL × 2). The organic phases were mixed, dried over anhydrous sodium sulfate, and filtered. The filtrate was concentrated under reduced pressure to give compound 38-5 (143 mg, yield: 100%) as a pale brown solid. ES-API: [M+H]⁺= 857.3.

Step 6: Compound 38-5 (143 mg, 0.17 mmol) was dissolved in dichloromethane (15 mL), and (1R,5S,6r)-3-oxabicyclo[3.1.0]hexane-6-carboxylic acid (28 mg, 0.22 mmol), O-(7-azabenzotriazol-1-yl)-N,N,N',N'-tetramethyluronium hexafluorophosphate (95 mg, 0.25 mmol), and N,N'-diisopropylethylamine (86 mg, 0.27 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure. The crude product was purified by a flash silica gel column (methanol/dichloromethane: 0-6%) to give the desired product 3-((6³S,4S,Z)-4-((1R,5S,6r)-3-oxabicyclo[3.1.0]hexane-6-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(2-hydroxyacetyl)piperazin-1-yl)prop-1-yn-1-yl)-2-((S)-1-methoxyethyl)pyridine-1-oxide (Z38, 105 mg, yield: 65.1%) as a pale yellow solid. ES-API: [M+H]⁺= 967.3.

Step 7: Compound Z38 (100 mg, 0.103 mmol) was subjected to preparative chiral resolution (separation column: IG, 250 mm × 4.6 mm × 5 µm, mobile phase: acetonitrile:isopropanol:diethylamine = 60:40:2, flow rate: 1 mL/min, column temperature: 30 °C) to give 2 isomeric compounds. The structure of one of the isomeric compounds was (Sₐ)-3-((6³S,4S,Z)-4-((1R,5S,6r)-3-oxabicyclo[3.1.0]hexane-6-carboxamido)-1¹-(2-methoxyethyl)-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-1²-yl)-5-(3-(4-(2-hydroxyacetyl)piperazin-1-yl)prop-1-yn-1-yl)-2-((S)-1-methoxyethyl)pyridine-1-oxide (Z38-2, 45 mg, retention time: 7.968 min, yield: 45.0%) as a white solid. ES-API: [M+H]⁺=967.3. ¹H NMR (400 MHz, DMSO-*d*₆) δ 8.58 (d, *J =* 9.2 Hz, 1H), 8.52 (d, *J =* 1.2 Hz, 1H), 8.47 (s, 1H), 7.80 (s, 1H), 7.72 (dd, *J* = 8.8, 1.2 Hz, 1H), 7.53 (d, *J* = 8.8 Hz, 1H), 7.45 (d, *J* = 1.2 Hz, 1H), 5.53 (t, *J* = 9.2 Hz, 1H), 5.05 (d, *J* = 12.0 Hz, 1H), 4.68 - 4.57 (m, 1H), 4.51 (t, *J* = 5.2 Hz, 1H), 4.29 - 4.14 (m, 3H), 4.07 (d, *J =* 5.2 Hz, 2H), 4.03 - 3.91 (m, 1H), 3.89 - 3.77 (m, 2H), 3.73 - 3.43 (m, 10H), 3.40 - 3.33 (m, 3H), 3.19 - 3.08 (m, 4H), 3.05 - 2.91 (m, 4H), 2.83 - 2.70 (m, 1H), 2.56 - 2.50 (m, 4H), 2.24 - 2.08 (m, 2H), 2.00 - 1.94 (m, 1H), 1.93 - 1.87 (m, 1H), 1.86 - 1.71 (m, 2H), 1.67 (t, *J* = 2.8 Hz, 1H), 1.59 - 1.45 (m, 1H), 1.27 (d, *J* = 6.8 Hz, 3H), 0.97 (s, 3H), 0.54 (s, 3H). The structure of the other isomeric compound was (Rₐ)-3-((6³S,4S,Z)-4-((1R,5S,6r)-3-oxabicyclo[3.1.0]hexane-6-carboxamido)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(2-hydroxyacetyl)piperazin-1-yl)prop-1-yn-1-yl)-2-((S)-1-methoxyethyl)pyridine-1-oxide (Z38-1, 17 mg, retention time: 11.092 min, yield: 17.0%) as a white solid. ES-API: [M+H]⁺=967.3. ¹H NMR (400 MHz, DMSO-*d*₆) δ 8.54 (d, *J =* 8.8 Hz, 1H), 8.51 - 8.45 (m, 2H), 7.81 (s, 1H), 7.74 (d, *J =* 8.8 Hz, 1H), 7.59 (d, *J =* 8.8 Hz, 1H), 7.24 (d, *J =* 1.2 Hz, 1H), 5.59 (t, *J* = 9.0 Hz, 1H), 5.10 (d, *J =* 12.0 Hz, 1H), 4.59 - 4.30 (m, 3H), 4.30 - 4.10 (m, 3H), 4.06 (d, *J* = 5.2 Hz, 2H), 3.90 - 3.76 (m, 2H), 3.70 - 3.55 (m, 6H), 3.53 - 3.44 (m, 2H), 3.39 - 3.25 (m, 5H), 3.20 - 3.09 (m, 4H), 3.01 - 2.90 (m, 4H), 2.84 - 2.70 (m, 1H), 2.55 - 2.51 (m, 5H), 2.07 (d, *J =* 10.4 Hz, 1H), 1.97 - 1.86 (m, 2H), 1.84 - 1.69 (m, 2H), 1.64 (t, *J =* 2.8 Hz, 1H), 1.59 - 1.43 (m, 4H), 0.94 (s, 3H), 0.52 (s, 3H).

### Example 1.23. Synthesis of Compounds Z31, Z31-1, and Z31-2

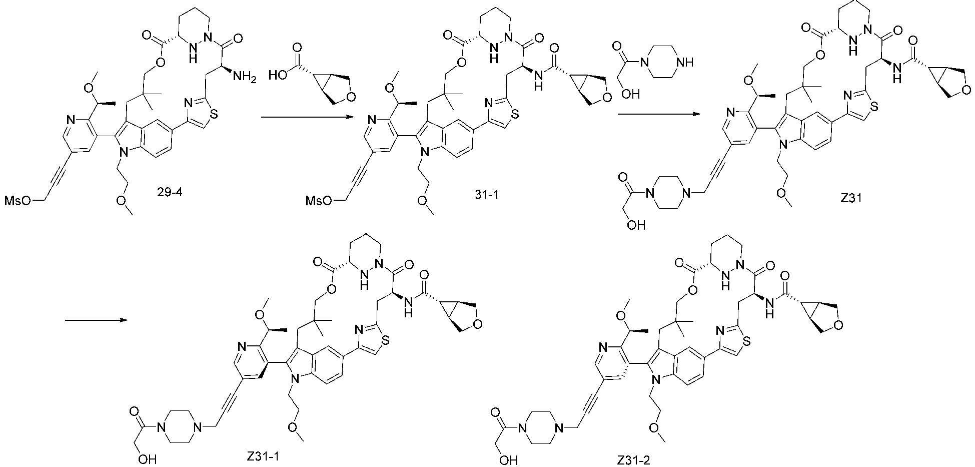

Step 1: Compound 29-4 (50 mg, 0.063 mmol) was dissolved in dichloromethane (2 mL). (1R,5S,6r)-3-Oxabicyclo[3.1.0]hexane-6-carboxylic acid (9.19 mg, 0.067 mmol), O-(7-azabenzotriazol-1-yl)-*N,N,N',N*'-tetramethyluronium hexafluorophosphate (35.93 mg, 0.094 mmol), and *N,N*'-diisopropylethylamine (0.045 mL, 0.252 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0-5%) to give compound 31-1 (49 mg, 0.055 mmol, yield: 87.41%) as a yellow solid. ES-API: [M +H]⁺=903.3. Step 2: Compound 31-1 (40 mg, 0.044 mmol) was dissolved in dichloromethane (5 mL), and N,N'-diisopropylethylamine (15 mg, 0.117 mmol) and 4-2-hydroxy-1-(piperazin-1-yl)ethan-1-one (16.40 mg, 0.117 mmol) were added. The mixture was reacted at room temperature for 3 h under a nitrogen atmosphere and concentrated to dryness under reduced pressure. The crude product was purified by a flash silica gel column (methanol/dichloromethane: 0%-10%) to give (1*R,*5*S,*6*r*)-N-((6³*S,*4*S*,*Z*)-1²-(5-(3-(4-(2-hydroxyacetyl)piperazin-1-yl)prop-1-yn-1-yl)-2-((S)-1-methoxyethyl)pyridin-3-yl)-1¹-(methoxyethyl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (Z31, 15 mg, 0.016 mmol, yield: 35.60%) as a white solid, ES-API: [M+H]⁺ = 951.3.

Step 3: Compound Z31 (15 mg, 0.016 mmol) was resolved (column type: IE 250 mm, 10 mm, 5 µm; mobile phase system: (A: n-hexane; B: ethanol); flow rate: 1 mL/min; B% = 0-30%; column temperature: 30 °C) to give 2 isomeric compounds. The structure of one of the isomeric compounds was (1*R*,5*S*,6*r*)-N-((6³*S*,4*S*,*Z*)-(*Rₐ*)-1²-(5-(3-(4-(2-hydroxyacetyl)piperazin-1-yl)prop-1-yn-1-yl)-2-((S)-1-methoxyethyl)pyridin-3-yl)-1¹-(methoxyethyl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (Z31-1, 3 mg, 0.0032 mmol, yield: 20%, retention time: 8.07 min) as a white solid, ES-API: [M+H]⁺ = 951.3. The structure of the other isomeric compound was (1*R*,5*S*,6*r*)-N-((6³*S*,4*S*,*Z*)-(*Sₐ*)-1²-(5-(3-(4-(2-hydroxyacetyl)piperazin-1-yl)prop-1-yn-1-yl)-2-((S)-1-methoxyethyl)pyridin-3-yl)-1¹-(methoxyethyl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (8 mg, 0.0085 mmol, yield: 53%, retention time: 9.8 min) as a white solid, ES-API: [M+H]⁺ = 951.3.

### Example 1.24. Synthesis of Compound Z28

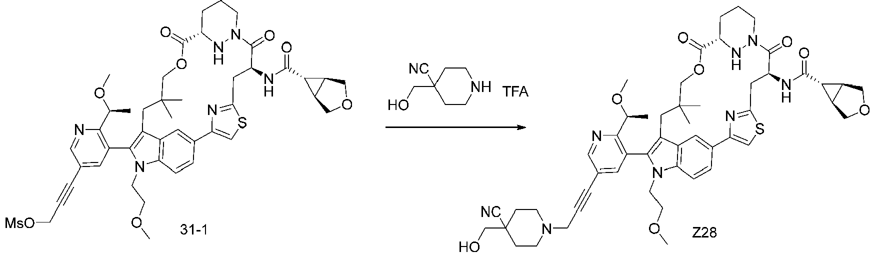

Step 1: Compound 31-1 (30 mg, 0.033 mmol) was dissolved in dichloromethane (5 mL), and N,N'-diisopropylethylamine (15 mg, 0.117 mmol) and 4-(hydroxymethyl)piperidine-4-carbonitrile (compound 23-1, 25.54 mg, 0.177 mmol) were added. The mixture was reacted at room temperature for 3 h under a nitrogen atmosphere and concentrated to dryness under reduced pressure. The crude product was purified by a flash silica gel column (methanol/dichloromethane: 0%-10%) to give (1*R*,5*S,*6*r*)*-*N-((6³*S,*4*S*,*Z*)-1²-(5-(3-(4-cyano-4-hydroxymethylpiperidin-1-yl)prop-1-yn-1-yl)-2-((S)-1-methoxyethyl)pyridin-3-yl)-1¹-(methoxyethyl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (Z28, 15 mg, 0.016 mmol, yield: 47.67%) as a white solid, ES-API: [M+H]⁺ = 947.3.

### Example 1.25. Synthesis of Compound Z40

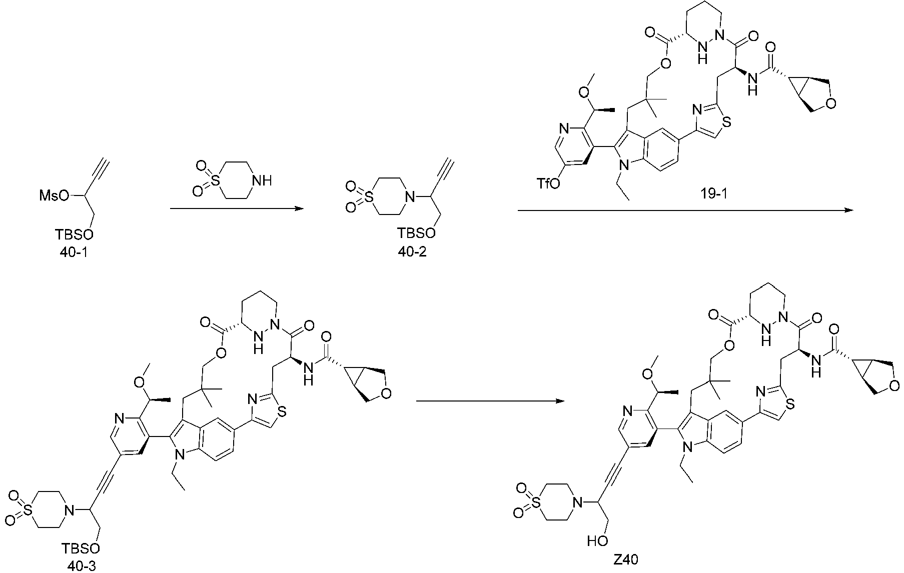

Step 1: Thiomorpholine-1,1-dioxide (970.98 mg, 7.183 mmol) was added to a solution of compound 40-1 (500 mg, 1.796 mmol, synthesized by referring to the synthetic method in Tetrahedron, 2011, vol. 67, # 29, p. 5133-5141) in dichloromethane (10 mL), and the reaction mixture was stirred at 40 °C for 2 h. The mixture was diluted with dichloromethane (50 mL) and water (50 mL). The organic layer was separated, further washed with a saturated sodium chloride solution (50 mL × 3), dried over anhydrous sodium sulfate, filtered, and concentrated. The residue was purified by silica gel column chromatography (tetrahydrofuran/petroleum ether = 1/1) to give compound 40-2 (285 mg, 0.898 mmol, yield: 50%). ES-API: [M+H]⁺=318.1.

Step 2: Bis(triphenylphosphine)palladium(II) dichloride (23.69 mg, 0.034 mmol), copper(I) iodide (12.85 mg, 0.067 mmol), DIPEA (58.04 mg, 0.450 mmol), and lithium chloride (23.84 mg, 0.562 mmol) were sequentially added to a solution of compound 19-1 (100 mg, 0.112 mmol) and compound 40-2 (107 mg, 0.332 mmol) in N,N-dimethylformamide (3 mL). The mixture was heated to 85 °C and reacted for 1.5 h under a nitrogen atmosphere. After the reaction was completed, water (20 mL) and ethyl acetate (30 mL) were added. The organic layer was separated, further washed with a saturated sodium chloride solution (50 mL × 3), dried over anhydrous sodium sulfate, filtered, and concentrated. The residue was purified by silica gel column chromatography (tetrahydrofuran/petroleum ether = 2/1) to give compound 40-3 (80 mg, 0.076 mmol, yield: 67.32%). ES-API: [M+H]⁺=1056.5.

Step 3: Compound 40-3 (80 mg, 0.076 mmol) was dissolved in dichloromethane (2 mL), and a 4 M solution (2 mL) of hydrochloric acid in dioxane was added. The mixture was reacted for 1 h and concentrated, and the concentrate was dissolved in 5 mL of dichloromethane. A 7 M solution of ammonia-methanol (0.3 mL) was added dropwise, and the mixture was concentrated again. The residue was purified by prep-HPLC (ammonium bicarbonate method 1) to give (1R,5S,6r)-N-((6³S,4S,Z)-(Rₐ)-1²-(5-(3-(1,1-dioxidothiomorpholino)-4-hydroxybut-1-yn-1-yl)-(Rₐ)-2-((S)-1-methoxyethyl)-pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z40, 30 mg, yield: 42%), ES-API: [M+H]⁺ = 942.4. ¹H NMR (400 MHz, DMSO-*d*₆) δ 8.80 (d, J = 2.1 Hz, 1H), 8.56 (d, J = 8.9 Hz, 1H), 8.50 (d, J = 1.6 Hz, 1H), 7.85 (dd, J = 2.1, 1.1 Hz, 1H), 7.81 (s, 1H), 7.76 (dd, J = 8.6, 1.6 Hz, 1H), 7.58 (d, J = 8.7 Hz, 1H), 5.56 (t, J = 9.1 Hz, 1H), 5.07 (d, J = 12.2 Hz, 1H), 4.98 - 4.90 (m, 1H), 4.37 - 4.15 (m, 4H), 4.13 - 4.02 (m, 1H), 3.92 (t, J = 6.5 Hz, 1H), 3.83 (t, J = 8.2 Hz, 2H), 3.68 - 3.60 (m, 4H), 3.58 (s, 2H), 3.35 (s, 1H), 3.25 (s, 3H), 3.16-3.10 (m, 7H), 3.04-2.95 (m, 3H), 2.79-2.72 (m, 1H), 2.39 (d, J = 14.4 Hz, 1H), 2.08 (d, J = 12.0 Hz, 1H), 1.97 - 1.88 (m, 2H), 1.80 (d, J = 9.3 Hz, 2H), 1.65 (t, J = 3.2 Hz, 1H), 1.57-1.46 (m, 1H), 1.36 (d, J = 6.0 Hz, 3H), 0.96 - 0.84 (m, 6H), 0.36 (s, 3H).

### Example 1.26. Synthesis of Compounds Z42-1 and Z42-2

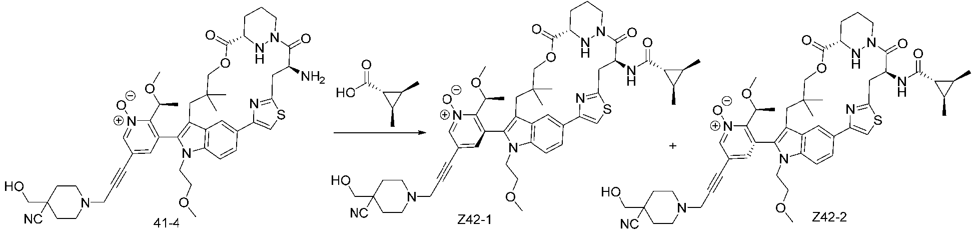

Step 1: Compound 41-4 (50 mg, 0.059 mmol) was dissolved in dichloromethane (6 mL), and (1r,2R,3S)-2,3-dimethylcyclopropane-1-carboxylic acid (9 mg, 0.076 mmol), O-(7-azabenzotriazol-1-yl)-N,N,N',N'-tetramethyluronium hexafluorophosphate (33 mg, 0.088 mmol), and N,N'-diisopropylethylamine (31 mg, 0.234 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure. The crude product was purified by prep-HPLC (ammonium bicarbonate method 1) to give 2 isomeric compounds. The structure of one of the isomeric compounds was 5-(3-(4-cyano-4-(hydroxymethyl)piperidin-1-yl)prop-1-yn-1-yl)-(Rₐ)-3-((6³S,4S,Z)-4-((1r,2R,3S)-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((S)-1-methoxyethyl)pyridine 1-oxide (Z42-1, 4 mg, yield: 7.2%, retention time: 5.321 min) as a white solid. ES-API: [M+H]⁺= 949.3. The structure of the other isomeric compound was 5-(3-(4-cyano-4-(hydroxymethyl)piperidin-1-yl)prop-1-yn-1-yl)-(Sₐ)-3-((6³S,4S,Z)-4-((1r,2R,3S)-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-1²-yl)-2-((S)-1-methoxyethyl)pyridine 1-oxide (Z42-2, 6 mg, yield: 10.7%, retention time: 5.435 min) as a white solid. ES-API: [M+H]⁺= 949.3. ¹H NMR (400 MHz, DMSO-*d*₆) δ 8.51 - 8.43 (m, 2H), 8.40 (d, *J* = 9.2 Hz, 1H), 7.80 (s, 1H), 7.71 (d, *J* = 7.6 Hz, 1H), 7.53 (d, *J* = 8.8 Hz, 1H), 7.46 (s, 1H), 5.53 (t, *J* = 9.2 Hz, 1H), 5.40 (t, *J =* 5.6 Hz, 1H), 5.03 (d, *J =* 12.0 Hz, 1H), 4.67 - 4.58 (m, 1H), 4.27 - 4.14 (m, 3H), 4.02 - 3.92 (m, 1H), 3.71 - 3.51 (m, 6H), 3.45 (d, *J* = 5.6 Hz, 2H), 3.29 - 3.23 (m, 1H), 3.22 - 3.11 (m, 4H), 3.04 - 2.95 (m, 4H), 2.90 - 2.82 (m, 2H), 2.81 - 2.67 (m, 1H), 2.46 - 2.31 (m, 2H), 2.23 - 2.07 (m, 2H), 1.92 - 1.70 (m, 4H), 1.61 - 1.42 (m, 3H), 1.27 (d, *J* = 6.4 Hz, 3H), 1.23 - 1.15 (m, 3H), 1.12 - 1.03 (m, 6H), 0.96 (s, 3H), 0.56 (s, 3H).

### Example 1.27. Synthesis of Compound Z41

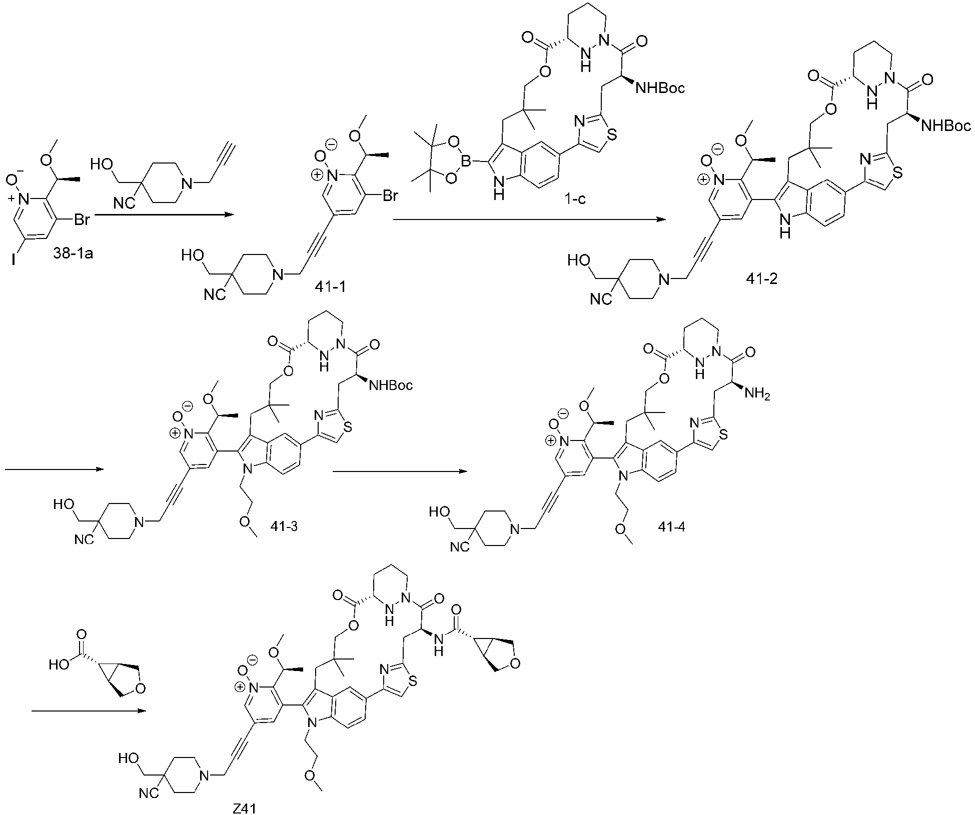

Step 1: Compound 38-1a (300 mg, 0.84 mmol), bis(triphenylphosphine)palladium(II) dichloride (29 mg, 0.042 mmol), and copper(I) iodide (16 mg, 0.084 mmol) were dissolved in acetonitrile (10 mL) under a nitrogen atmosphere. A solution of 4-(hydroxymethyl)-1-(prop-2-ynyl)piperidine-4-carbonitrile (compound 23-2, 164 mg, 0.92 mmol) and N,N-diisopropylethylamine (217 mg, 1.68 mmol) in acetonitrile (5 mL) was added dropwise under an ice bath. The reaction system was stirred at room temperature for 1 h. Ethyl acetate (100 mL) was added to the reaction mixture, and the mixture was washed with saturated brine (30 mL). The organic phase was dried over anhydrous sodium sulfate and filtered, and the filtrate was concentrated under reduced pressure. The crude product was purified by a flash silica gel column (methanol/dichloromethane: 0-6%) to give compound 41-1 (270 mg, yield: 78.9%) as a yellow solid. ES-API:[M+H]⁺= 408.0, 410.0.

Step 2: Compound 1-c (300 mg, 0.43 mmol) and compound 41-1 (265 mg, 0.65 mmol) were dissolved in dioxane (2 mL), toluene (6 mL), and water (2 mL) under a nitrogen atmosphere, and [1,1'-bis(di-tert-butylphosphino)ferrocene]dichloropalladium(II) (56 mg, 0.086 mmol) and potassium phosphate (183 mg, 0.87 mmol) were added. The reaction system was stirred at 75 °C for 3 h. Ethyl acetate (50 mL) was added to the reaction mixture, and the mixture was washed with saturated brine (15 mL). The organic phase was dried over anhydrous sodium sulfate and filtered, and the filtrate was concentrated under reduced pressure. The crude product was purified by a flash silica gel column (methanol/dichloromethane: 0-10%) to give compound 41-2 (230 mg, yield: 59.5%) as a pale brown solid. ES-API:[M+H]⁺= 895.3.

Step 3: Compound 41-2 (230 mg, 0.26 mmol) was dissolved in N,N-dimethylformamide (10 mL), and cesium carbonate (251 mg, 0.77 mmol) and 1-iodo-2-methoxyethane (143 mg, 0.77 mmol) were added at room temperature. The reaction system was stirred at room temperature for another 48 h. Ethyl acetate (50 mL) was added to the reaction mixture, and the mixture was washed with saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-5%) to give compound 41-3 (180 mg, yield: 73.5%) as a pale yellow solid. ES-API:[M+H]⁺= 953.3.

Step 4: Compound 41-3 (180 mg, 0.19 mmol) was dissolved in dichloromethane (6 mL), and trifluoroacetic acid (3 mL) was added at 0 °C. The reaction system was stirred at room temperature for 3 h. The reaction mixture was concentrated, and a saturated sodium bicarbonate solution (5 mL) was added. The mixture was extracted with dichloromethane (15 mL × 2). The organic phases were mixed, dried over anhydrous sodium sulfate, and filtered. The filtrate was concentrated under reduced pressure to give compound 41-4 (145 mg, yield: 90.0%) as a pale brown solid. ES-API: [M+H]⁺= 853.3.

Step 5: Compound 41-4 (50 mg, 0.059 mmol) was dissolved in dichloromethane (6 mL), and trans-3-oxabicyclo[3.1.0]hexane-6-carboxylic acid (10 mg, 0.077 mmol), O-(7-azabenzotriazol-1-yl)-N,N,N',N'-tetramethyluronium hexafluorophosphate (32 mg, 0.083 mmol), and N,N'-diisopropylethylamine (31 mg, 0.234 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure. The crude product was purified by prep-HPLC (ammonium bicarbonate method 1) to give the desired product 3-((6³S,4S,Z)-4-((1R,5S,6r)-3-oxabicyclo[3.1.0]hexane-6-carboxamido)-1¹-(methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-cyano-4-(hydroxymethyl)piperidin-1-yl)prop-1-yn-1-yl)-2-((S)-1-methoxyethyl)pyridine-1-oxide (Z41, 18 mg, yield: 31.7%) as a white solid. ES-API: [M+H]⁺= 963.3.

### Example 1.28. Synthesis of Compound Z35-1

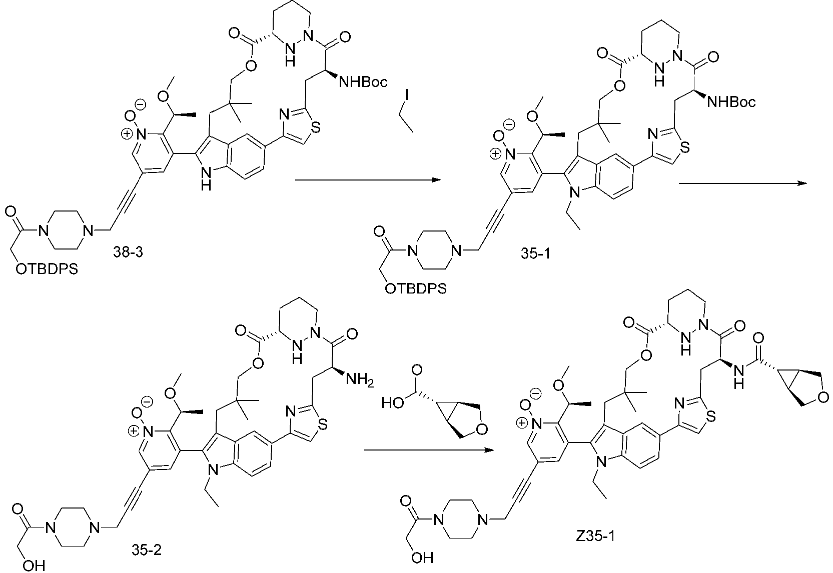

Step 1: Compound 38-3 (100 mg, 0.088 mmol) was dissolved in N,N-dimethylformamide (5 mL), and cesium carbonate (86 mg, 0.264 mmol) and iodoethane (41 mg, 0.264 mmol) were added at room temperature. The reaction system was stirred at room temperature for another 48 h. Ethyl acetate (50 mL) was added to the reaction mixture, and the mixture was washed with saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-5%) to give compound 35-1 (55 mg, yield: 53.7%) as a pale brown solid. ES-API:[M+H]⁺= 1165.3.

Step 2: Compound 35-1 (55 mg, 0.047 mmol) was dissolved in dichloromethane (3 mL), and trifluoroacetic acid (1.5 mL) was added at 0 °C. The reaction system was stirred at room temperature for 3 h. The reaction mixture was concentrated, and a saturated sodium bicarbonate solution (5 mL) was added. The mixture was extracted with dichloromethane (15 mL × 2). The organic phases were mixed, dried over anhydrous sodium sulfate, and filtered. The filtrate was concentrated under reduced pressure to give compound 35-2 (39 mg, yield: 100%) as a pale brown solid. ES-API: [M+H]⁺= 827.3.

Step 3: Compound 35-2 (39 mg, 0.047 mmol) was dissolved in dichloromethane (5 mL), and (1R,5S,6r)-3-oxabicyclo[3.1.0]hexane-6-carboxylic acid (8 mg, 0.061 mmol), O-(7-azabenzotriazol-1-yl)-N,N,N',N'-tetramethyluronium hexafluorophosphate (23 mg, 0.061 mmol), and N,N'-diisopropylethylamine (24 mg, 0.189 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure. The crude product was subjected to prep-HPLC (ammonium bicarbonate method 1) to give the desired product 3-((6³S,4S,Z)-4-((1R,5S,6r)-3-oxabicyclo[3.1.0]hexane-6-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(2-hydroxyacetyl)piperazin-1-yl)prop-1-yn-1-yl)-2-((S)-1-methoxyethyl)pyridine-1-oxide (Z35-1, 10 mg, yield: 22.6%) as a white solid. ES-API: [M+H]⁺= 937.3.

### Example 1.29. Synthesis of Compound Z51

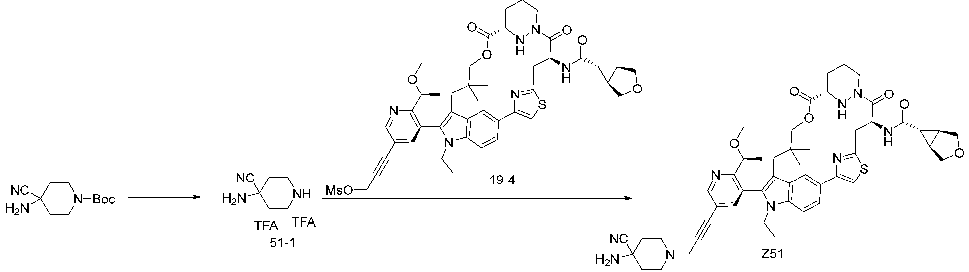

Step 1: Trifluoroacetic acid (10 mL) was added to a solution of tert-butyl 4-amino-4-cyanopiperidine-1-carboxylate (1000 mg, 4.439 mmol) in dichloromethane (10 mL). The reaction mixture was stirred at room temperature for 2 h and then concentrated to give compound 51-1 (1008.60 mg, 4.217 mmol, crude product, trifluoroacetate). ES-API: [M+H]⁺=126.1.

Step 2: Potassium carbonate (158.30 mg, 1.145 mmol) was added to a solution of compound 51-1 (220.68 mg, 0.687 mmol) in acetonitrile (10 mL), and the mixture was stirred at room temperature for 10 min. Then compound 19-4 (200 mg, 0.229 mmol) was added, and the mixture was heated to 50 °C and reacted for 2 h. After the reaction was completed, water (20 mL) and ethyl acetate (30 mL) were added. The organic layer was separated, further washed with a saturated sodium chloride solution (50 mL × 3), dried over anhydrous sodium sulfate, filtered, and concentrated. The residue was purified by silica gel column chromatography (tetrahydrofuran/petroleum ether *=* 2/1) to give (1R,5S,6r)-N-((6³S,4S,Z)-(Rₐ)-1²-(5-(3-(4-amino-4-cyanopiperidin-1-yl)prop-1-yn-1-yl)-2-((S)-1-methoxyethyl)-pyridin-3-yl)-1¹-ethyl-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-4-yl)-3-oxabicyclo[3.1.0]hexane-6-carboxamide (Z51, 80 mg, 0.089 mmol, yield: 38.71%), ES-API: [M+H]⁺= 902.3. ¹H NMR (400 MHz, DMSO-*d*₆) δ 8.80 (d, *J =* 2.0 Hz, 1H), 8.56 (d, *J =* 8.9 Hz, 1H), 8.50 (d, *J =* 1.6 Hz, 1H), 7.83 (d, *J* = 2.2 Hz, 1H), 7.81 (s, 1H), 7.75 (dd, *J =* 8.8, 1.6 Hz, 1H), 7.58 (d, *J =* 8.7 Hz, 1H), 5.57 (t, *J* = 9.1 Hz, 1H), 5.08 (d, *J* = 12.2 Hz, 1H), 4.36 - 4.16 (m, 4H), 4.12 - 4.03 (m, 1H), 3.83 (t, *J* = 8.1 Hz, 2H), 3.68 - 3.56 (m, 6H), 3.35 (s, 2H), 3.25 (s, 3H), 3.16-3.10 (m, 1H), 2.95 (d, *J =* 13.9 Hz, 1H), 2.89 - 2.71 (m, 4H), 2.47 - 2.36 (m, 3H), 2.08 (d, *J =* 11.9 Hz, 1H), 2.00 - 1.88 (m, 4H), 1.79 (s, 2H), 1.70 - 1.56 (m, 3H), 1.54-1.49 (m, 1H), 1.36 (d, *J =* 6.0 Hz, 3H), 0.92 - 0.84 (m, 6H), 0.35 (s, 3H).

### Example 1.30. Synthesis of Compound Z47

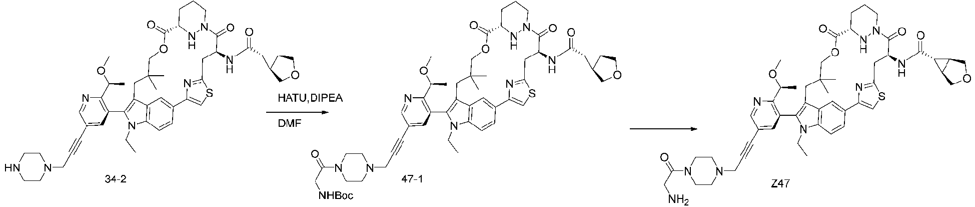

Step 1: Compound 34-2 (20 mg, 0.023 mmol) was dissolved in N,N-dimethylformamide (2 mL). N-{[(2-Methylpropan-2-yl)oxy]carbonyl}glycine (4.87 mg, 0.028 mmol), O-(7-azabenzotriazol-1-yl)-N,N,N',N'-tetramethyluronium hexafluorophosphate (12.34 mg, 0.032 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3, dried, and concentrated to give a crude product. The crude product was purified by a flash silica gel column (methanol/dichloromethane: 0%-10%) to give compound 47-1 (22 mg, 0.022 mmol, yield: 93.06%) as a yellow solid, ES-API: [M+H]⁺ = 1020.2.

Step 2: Compound 47-1 (22 mg, 0.022 mmol) was dissolved in methanol (0.5 mL), and a solution (2 mL) of hydrochloric acid in dioxane was added. The reaction system was stirred at room temperature for 2 h and concentrated to dryness under reduced pressure. The crude product was purified by prep-HPLC (formic acid method) to give (1*R*,5*S*,6*r*)-N-((6³*S*,4*S*,*Z*)-1¹-ethyl-(*Rₐ*)-1²-(5-(3-(4-glycylpiperazin-1-yl)prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-3-oxabicyclo[3.1.0]hexane-6-carboxamide (Z47, 6 mg, 0.006 mmol, yield: 31.68%, formate) as a white solid. ES-API: [M+H]⁺ = 920.3.

### Example 1.31. Synthesis of Compound Z52

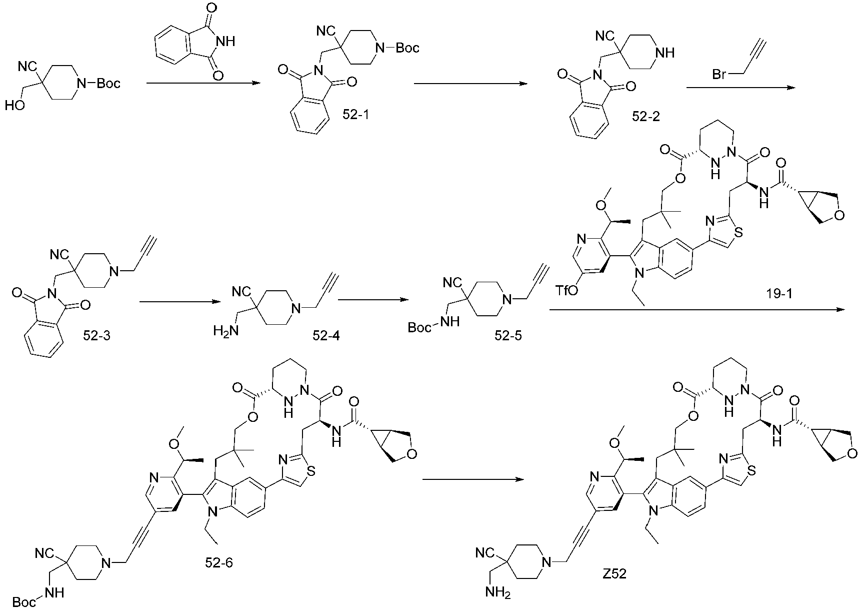

Step 1: 2H-Isoindole-1,3-dione (2.02 g, 13.733 mmol), PPh₃ (5.87 g, 22.472 mmol), and DIAD (4.54 g, 22.472 mmol) were added to a solution of tert-butyl 4-cyano-4-(hydroxymethyl)piperidine-1-carboxylate (3.00 g, 12.4484 mmol) in tetrahydrofuran (150 mL), and the reaction mixture was stirred at 40 °C for 3 h. After the reaction was completed, water (100 mL) and ethyl acetate (100 mL) were added. The organic layer was separated, further washed with a saturated sodium chloride solution (100 mL × 3), dried over anhydrous sodium sulfate, filtered, and concentrated. The residue was purified by silica gel column chromatography (ethyl acetate/petroleum ether = 1/2) to give compound 52-1 (2500 mg, 6.767 mmol, yield: 54.21%). ES-API: [M+H]⁺=370.1.

Step 2: tert-Butyl 4-cyano-4-((1,3-dioxoisoindolin-2-yl)methyl)piperidine-1-carboxylate (compound 52-1, 2 g, 5.414 mmol) was dissolved in dichloromethane (6 mL), and trifluoroacetic acid (6.219 mL, 81.208 mmol) was added. The mixture was reacted at room temperature for 1 h, and the reaction mixture was concentrated under vacuum and dried to give compound 52-2 (1.45 g, crude product). ES-API: [M+H]⁺=270.1.

Step 3: Compound 52-2 (1.45 g, crude product) was dissolved in acetonitrile (15 mL), potassium carbonate (2.31 g, 16.710 mmol) and 3-bromoprop-1-yne (0.53 g, 4.456 mmol) were added, and the mixture was reacted at room temperature for 3 h. After the reaction was completed, water (50 mL) and ethyl acetate (50 mL) were added. The organic layer was separated, further washed with a saturated sodium chloride solution (50 mL × 3), dried over anhydrous sodium sulfate, filtered, and concentrated. The residue was purified by silica gel column chromatography to give compound 52-3 (800 mg, 2.603 mmol, yield: 46.73%). ES-API: [M+H]⁺=308.1.

Step 4: Compound 52-3 (800 mg, 2.603 mmol) was dissolved in ethanol (15 mL), and a 30% methylamine/methanol solution (4 g, 39.045 mmol) was added. The mixture was heated to 80 °C and reacted for 6 h. After the reaction was completed, the mixture was concentrated to remove excess methylamine to give compound 52-4 (980 mg, crude product). ES-API: [M+H]⁺=178.1.

Step 5: Compound 52-4 (980 mg, crude product) was dissolved in tetrahydrofuran (15 mL), and a saturated aqueous sodium bicarbonate solution (5 mL) and di-tert-butyl dicarbonate (1.12 mL, 5.2 mmol) were added. The mixture was reacted for 1 h. After the reaction was completed, water (50 mL) and ethyl acetate (50 mL) were added. The organic layer was separated, further washed with a saturated sodium chloride solution (50 mL × 3), dried over anhydrous sodium sulfate, filtered, and concentrated. The residue was purified by silica gel column chromatography to give compound 52-5 (500 mg, 1.80 mmol, two-step yield: 69.3%). ES-API: [M+H]⁺=278.1. Step 6: Pd(PPh₃)₂Cl₂ (49.74 mg, 0.071 mmol), CuI (26.99 mg, 0.142 mmol), DIPEA (152.37 mg, 1.181 mmol), and lithium chloride (60.08 mg, 1.417 mmol) were sequentially added to a solution of compound 19-1 (210 mg, 0.236 mmol) and compound 52-5 (220 mg, 0.793 mmol) in DMF (3 mL). The mixture was heated to 85 °C and reacted for 1.5 h under a nitrogen atmosphere. After the reaction was completed, water (20 mL) and ethyl acetate (30 mL) were added. The organic layer was separated, further washed with a saturated sodium chloride solution (50 mL × 3), dried over anhydrous sodium sulfate, filtered, and concentrated. The residue was purified by silica gel column chromatography (tetrahydrofuran/petroleum ether = 2/1) to give compound 52-6 (220 mg, 0.216 mmol, yield: 91.64%). ES-API: [M+H]⁺=1016.5.

Step 7: Compound 52-6 (220 mg, 0.216 mmol) was dissolved in dichloromethane (5 mL), and trifluoroacetic acid (5 mL) was added. The mixture was reacted for 1 h and concentrated, the residue was purified by prep-HPLC (ammonium bicarbonate method 1) to give (1R,5S,6r)-N-((6³S,4S,Z)-(Rₐ)-1²-(5-(3-(4-(aminomethyl)-4-cyanopiperidin-1-yl)prop-1-yn-1-yl)-2-((S)-1-methoxyethyl)-pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z52, 60 mg, 0.065 mmol, yield: 30.25%), ES-API: [M+H]⁺ = 916.4. ¹H NMR (400 MHz, DMSO-*d*₆) δ 8.80 (d, *J =* 2.1 Hz, 1H), 8.55 (d, *J =* 8.9 Hz, 1H), 8.50 (d, *J =* 1.6 Hz, 1H), 7.83 (d, *J* = 2.2 Hz, 1H), 7.81 (s, 1H), 7.75 (dd, *J* = 8.7, 1.6 Hz, 1H), 7.58 (d, *J =* 8.7 Hz, 1H), 5.57 (t, *J =* 9.0 Hz, 1H), 5.08 (d, *J =* 12.1 Hz, 1H), 4.26 (m, 4H), 4.12 - 4.03 (m, 1H), 3.83 (t, *J =* 8.1 Hz, 2H), 3.66 - 3.56 (m, 6H), 3.36-3.32 (m, 2H), 3.25 (s, 3H), 3.15-3.10 (m, 1H), 2.97 - 2.86 (m, 3H), 2.79-2.74 (m, 1H), 2.70 (s, 2H), 2.47 - 2.36 (m, 3H), 2.10-2.06 (m, 1H), 1.97 - 1.85 (m, 4H), 1.79-1.75 (m, 2H), 1.65 (t, *J =* 3.2 Hz, 1H), 1.53-1.48 (m, 3H), 1.36 (d, *J =* 5.9 Hz, 3H), 1.24 (d, *J =* 5.6 Hz, 1H), 0.94 - 0.85 (m, 6H), 0.35 (s, 3H).

### Example 1.32. Synthesis of Compound Z44

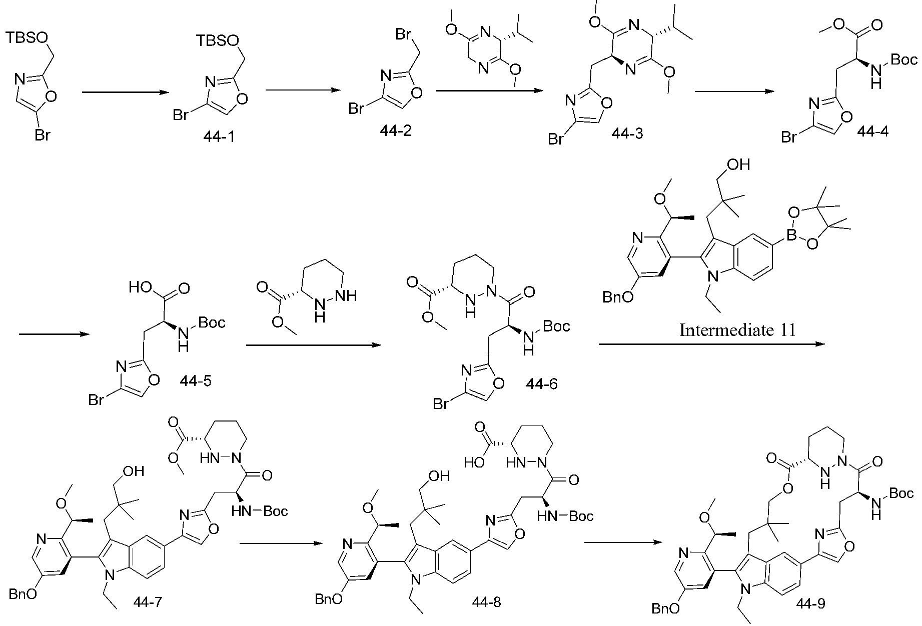

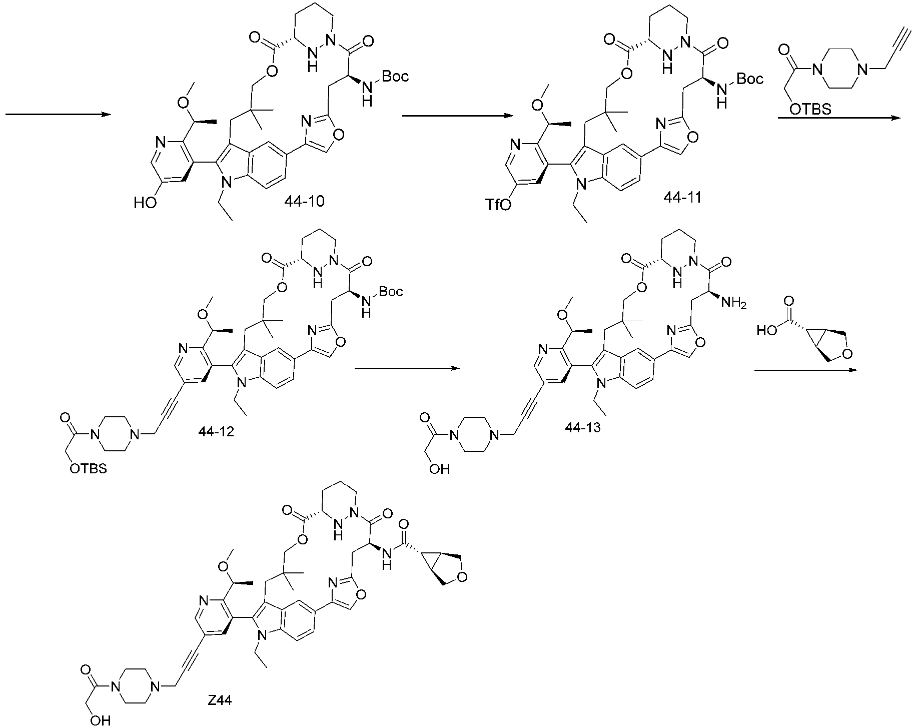

Step 1: A solution of lithium diisopropylamide (12.318 mL, 24.636 mmol) in tetrahydrofuran (2 M) was added dropwise to a solution of 5-bromo-2-(3,3,4,4-tetramethyl-2-oxa-3-silapentan-1-yl)-1,3-oxazole (4.8 g, 16.424 mmol) in tetrahydrofuran (100 mL) under a dry ice bath at -78 °C. After 30 min, wet tetrahydrofuran was added, and the mixture was warmed to room temperature. The mixture was diluted with ethyl acetate (50 mL), washed with a saturated aqueous sodium bicarbonate solution (20 mL × 1) and a saturated aqueous sodium chloride solution (20 mL × 1), dried over anhydrous sodium sulfate, filtered, and concentrated. The residue was purified by silica gel column chromatography (0-5% ethyl acetate/petroleum ether) to give compound 44-1 (2 g, 6.843 mmol, yield: 41.67%). ES-API: [M+H]⁺=292.0,294.0.

Step 2: Phosphorus tribromide (1.042 mL, 15.398 mmol) was added dropwise to a solution of compound 44-1 (1500 mg, 5.133 mmol) in dichloromethane (5 mL) under an ice-water bath. After the mixture was reacted at room temperature for 4 h under a nitrogen atmosphere, an aqueous sodium bicarbonate solution (50 mL) was slowly added, and the mixture was extracted with ethyl acetate (40 mL × 3), washed with a saturated aqueous sodium chloride solution (100 mL × 1), dried over anhydrous sodium sulfate, filtered, and concentrated. The residue was purified by silica gel column chromatography (0-5% ethyl acetate/petroleum ether) to give compound 44-2 (433 mg, 1.8 mmol, yield: 35%). ES-API: [M+H]⁺=241.9.

Step 3: n-Butyllithium (0.651 mL, a 2.5 M solution in n-hexane) was added dropwise to a solution of (2R)-3,6-dimethyl-2-(propan-2-yl)-2,5-dihydropyrazine (300 mg, 1.628 mmol) in tetrahydrofuran (5 mL) under a dry ice bath at -78 °C. After the mixture was reacted at this temperature for 30 min, a solution of compound 44-2 (392.15 mg, 1.628 mmol) in tetrahydrofuran (2 mL) was slowly added dropwise using a syringe. The mixture was reacted at this temperature for another 30 min, and the reaction was quenched with an aqueous ammonium chloride solution (10 mL). The mixture was extracted with ethyl acetate (40 mL × 3), washed with a saturated aqueous sodium chloride solution (100 mL × 1), dried over anhydrous sodium sulfate, filtered, and concentrated. The residue was purified by silica gel column chromatography (30% ethyl acetate/petroleum ether) to give compound 44-3 (360 mg, 1.05 mmol, yield: 64.4%). ES-API: [M+H]⁺=344.1, 346.1.

Step 4: 0.5 M hydrochloric acid (8.4 mL) was added to a solution of compound 44-3 (360 mg, 1.05 mmol) in acetonitrile (8 mL), and the mixture was reacted at room temperature for 3 h. The mixture was cooled to 0 °C, and an aqueous sodium bicarbonate solution (8 mL) and di-tert-butyl dicarbonate (0.719 mL, 3.132 mmol) were added. The mixture was reacted at room temperature for 1 h. The mixture was extracted with ethyl acetate (20 mL × 3), washed with a saturated aqueous sodium chloride solution (100 mL × 1), dried over anhydrous sodium sulfate, filtered, and concentrated. The residue was purified by silica gel column chromatography (30% ethyl acetate/petroleum ether) to give compound 44-4 (260.52 mg, 1.046 mmol, yield: 100%). ES-API: [M+H]⁺=349.0, 351.0.

Step 5: Lithium hydroxide (90.13 mg, 2.148 mmol) was added to a solution of compound 44-4 (260.52 mg, 1.046 mmol) in tetrahydrofuran (8 mL), and the mixture was reacted at 0 °C for 3 h. A 2 M aqueous hydrochloric acid solution (1 mL) was added, and the mixture was concentrated to give compound 44-5 (400 mg, crude product), which was directly used in the next step. ES-API: [M+H]⁺= 335.0, 337.0.

Step 6: Compound 44-5 (400 mg, crude product) was dissolved in dichloromethane (10 mL), and 4-methylmorpholine (434.38 mg, 4.297 mmol), HOBt (29.03 mg, 0.215 mmol), methyl (S)-hexahydropyridazine-3-carboxylate hydrochloride (270.94 mg, 1.500 mmol), and EDCI (411.83 mg, 2.148 mmol) were sequentially added. The mixture was reacted at room temperature for 1 h. The mixture was extracted with dichloromethane (20 mL × 3), washed with a saturated aqueous sodium chloride solution (100 mL × 1), dried over anhydrous sodium sulfate, filtered, and concentrated. The residue was purified by silica gel column chromatography (50% ethyl acetate/petroleum ether) to give compound 44-6 (280 mg, 0.607 mmol, two-step yield: 58%). ES-API: [M+H]⁺= 461.1, 463.1.

Step 7: Intermediate 11 (350 mg, 0.585 mmol), Pd(dtbpf)Cl₂ (56.66 mg, 0.088 mmol), and potassium phosphate (248.62 mg, 1.169 mmol) were sequentially added to a mixed solution of compound 44-6 (280 mg, 0.607 mmol) in 1,4-dioxane (6 mL), toluene (2 mL), and water (2 mL). The reaction mixture was heated to 70 °C and reacted for 5 h under a nitrogen atmosphere. The mixture was extracted with ethyl acetate (20 mL × 3), washed with a saturated aqueous sodium chloride solution (100 mL × 1), dried over anhydrous sodium sulfate, filtered, and concentrated. The residue was purified by silica gel column chromatography (70% ethyl acetate/petroleum ether) to give compound 44-7 (250 mg, 0.293 mmol, yield: 50.00%). ES-API: [M+H]⁺= 853.4.

Step 8: An aqueous solution (3 mL) of lithium hydroxide (14.76, 0.352 mmol) was added to a solution of compound 44-7 (250 mg, 0.293 mmol) in tetrahydrofuran (5 mL), and the mixture was reacted at room temperature for 1 h. A 0.5 M aqueous hydrochloric acid solution (0.6 mL) was added dropwise, and the mixture was concentrated to give compound 44-8 (300 mg, crude product), which was directly used in the next step. ES-API: [M+H]⁺=839.4.

Step 9: Compound 44-8 (300 mg, crude product) was dissolved in dry dichloromethane (30 mL), and HOBt (402.65 mg, 2.980 mmol), DIPEA (2.110 mL, 11.919 mmol), and EDCI (1713.65 mg, 8.939 mmol) were sequentially added. The mixture was reacted at room temperature overnight, water (10 mL) was added, and the mixture was washed sequentially with 1 M hydrochloric acid (20 mL) and saturated brine (20 mL). The mixture was dried over anhydrous sodium sulfate, filtered, and concentrated. The residue was purified by silica gel column chromatography (70% ethyl acetate/petroleum ether) to give compound 44-9 (170 mg, 0.207 mmol, yield: 69.49%), ES-API: [M+H]⁺ = 821.4.

Step 10: Palladium on carbon (10%, 100 mg, 0.940 mmol) was added to a solution (10 mL) of compound 44-9 (170 mg, 0.207 mmol) in methanol. The mixture was purged with hydrogen and stirred at 25 °C for 1 h. The mixture was filtered and concentrated to give compound 44-10 (80 mg, 0.109 mmol). ES-API: [M+H]⁺=731.4. Step 11: DIPEA (42.36 mg, 0.328 mmol) and N-phenyl-bis(trifluoromethanesulfonimide) (58.66 mg, 0.164 mmol) were added to a solution (5 mL) of compound 44-10 (80 mg, 0.109 mmol) in dichloromethane. The mixture was reacted at room temperature for 1 h, extracted with dichloromethane (20 mL × 3), washed with a saturated aqueous sodium chloride solution (100 mL × 1), dried over anhydrous sodium sulfate, filtered, and concentrated. The residue was purified by silica gel column chromatography (80% ethyl acetate/petroleum ether) to give compound 44-11 (75 mg, 0.087 mmol, yield: 79.40%). ES-API: [M+H]⁺=863.3.

Step 12: Pd(PPh₃)₂Cl₂ (18.30 mg, 0.026 mmol), copper(I) iodide (9.93 mg, 0.052 mmol), DIPEA (56.06 mg, 0.435 mmol), and lithium chloride (36.84 mg, 0.869 mmol) were sequentially added to a solution of compound 44-11 (75 mg, 0.087 mmol) and 2-((tert-butyldimethylsilyl)oxy)-1-(4-(prop-2-yn-1-yl)piperazin-1-yl)ethan-1-one (109.68 mg, 0.261 mmol) in N,N-dimethylformamide. The mixture was heated to 85 °C and reacted for 3 h. The mixture was extracted with ethyl acetate (20 mL × 3), washed with a saturated aqueous sodium chloride solution (30 mL × 1), dried over anhydrous sodium sulfate, filtered, and concentrated. The residue was purified by silica gel column chromatography (90% ethyl acetate/petroleum ether) to give compound 44-12 (50 mg, 0.044 mmol, 50.75%). ES-API: [M+H]⁺=1009.6.

Step 13: Compound 44-12 (50 mg, 0.044 mmol) was dissolved in methanol (1 mL), and 4 M hydrochloric acid/dioxane (2 mL) was added. The mixture was reacted at room temperature for 2 h. The reaction mixture was concentrated to give compound 44-13 (45 mg, crude product), which was directly used in the next step. ES-API: [M+H]⁺=795.4.

Step 14: (1R,5S,6r)-3-Oxabicyclo[3.1.0]hexane-6-carboxylic acid (4.87 mg, 0.038 mmol), DIPEA (4.91 mg, 0.038 mmol), and HATU (14.45 mg, 0.038 mmol) were sequentially added to a solution of compound 44-13 (45 mg, crude product) in dichloromethane (5 mL), and the mixture was reacted at room temperature for 2 h. The mixture was extracted with dichloromethane (20 mL × 3), washed with a saturated aqueous sodium chloride solution (30 mL × 1), dried over anhydrous sodium sulfate, filtered, and concentrated. The residue was purified by prep-HPLC (ammonium bicarbonate method 1) to give (1*R*,5*S*,6*r*)*-N-*((6³*S,*4*S*,*Z*)*-*(*Rₐ*)-1²-(5-(3-(4-(2-hydroxyacetyl)piperazin-1-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphane-4-yl)-3-oxabicyclo[3.1.0]hexane-6-carboxamide (Z44, 8 mg, 0.009 mmol, yield: 20.4%). ES-API: [M+H]⁺=905.4.

### Example 1.33. Synthesis of Compound Z56

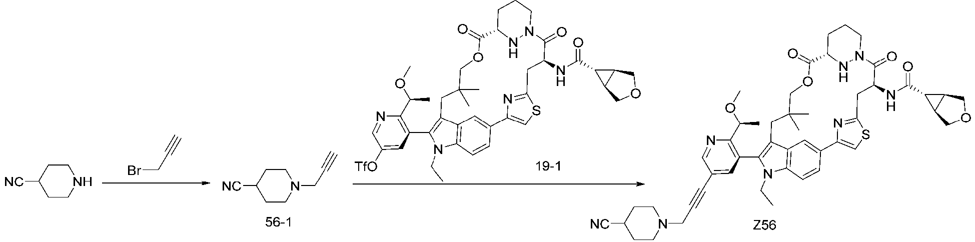

Step 1: 4-Piperidinecarbonitrile (1000 mg, 9.078 mmol) was dissolved in acetonitrile (15 mL), potassium carbonate (2509.16 mg, 18.156 mmol) and 3-bromoprop-1-yne (1079.88 mg, 9.078 mmol) were added, and the mixture was reacted at room temperature for 3 h. After the reaction was completed, water (50 mL) and ethyl acetate (50 mL) were added. The organic layer was separated, further washed with a saturated sodium chloride solution (50 mL × 3), dried over anhydrous sodium sulfate, filtered, and concentrated. The residue was purified by silica gel column chromatography to give compound 56-1 (1000 mg, 6.747 mmol, yield: 74.33%). ES-API: [M+H]⁺=149.1.

Step 2: Pd(PPh₃)₂Cl₂ (70.97 mg, 0.101 mmol), copper(I) iodide (38.56 mg, 0.202 mmol), DIPEA (217.67 mg, 1.687 mmol), and lithium chloride (85.04 mg, 2.025 mmol) were sequentially added to a solution of compound 19-1 (300 mg, 0.337 mmol) and compound 56-1 (150.05 mg, 1.012 mmol) in N,N-dimethylformamide (3 mL). The mixture was heated to 85 °C and reacted for 1.5 h under a nitrogen atmosphere. After the reaction was completed, water (20 mL) and ethyl acetate (30 mL) were added. The organic layer was separated, further washed with a saturated sodium chloride solution (50 mL × 3), dried over anhydrous sodium sulfate, filtered, and concentrated. The residue was purified and separated by prep-HPLC (ammonium bicarbonate method 1) to give (1R,5S,6r)-N-((6³S,4S,Z)-(Ra)-1²-(5-(3-(4-cyanopiperidin-1-yl)prop-1-yn-1-yl)-2-((S)-1-methoxyethyl)-pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z56, 240 mg, 0.271 mmol, yield: 80.17%), ES-API: [M+H]⁺ = 887.4. ¹H NMR (400 MHz, DMSO-*d*₆) δ 8.80 (d, *J =* 2.1 Hz, 1H), 8.55 (d, *J* = 8.9 Hz, 1H), 8.50 (d, *J* = 1.6 Hz, 1H), 7.84 (d, *J* = 2.2 Hz, 1H), 7.81 (s, 1H), 7.75 (dd, *J* = 8.7, 1.6 Hz, 1H), 7.58 (d, *J =* 8.7 Hz, 1H), 5.56 (t, *J =* 9.0 Hz, 1H), 5.07 (d, *J =* 12.2 Hz, 1H), 4.37 - 4.15 (m, 4H), 4.11 - 4.02 (m, 1H), 3.83 (t, *J =* 8.2 Hz, 2H), 3.69 - 3.60 (m, 2H), 3.58 (s, 3H), 3.35-3.29 (m, 3H), 3.25 (s, 3H), 3.16-3.10 (m, 1H), 2.97 - 2.86 (m, 2H), 2.78-2.68 (m, 3H), 2.44 - 2.32 (m, 2H), 2.08 (d, *J* = 12.1 Hz, 1H), 1.97 -1.85 (m, 4H), 1.81 - 1.69 (m, 4H), 1.65 (t, *J* = 3.3 Hz, 1H), 1.53-1.49 (m, 1H), 1.35 (d, *J* = 6.1 Hz, 3H), 0.94 - 0.83 (m, 6H), 0.35 (s, 3H).

### Example 1.34. Synthesis of Compounds Z60 and Z71-1

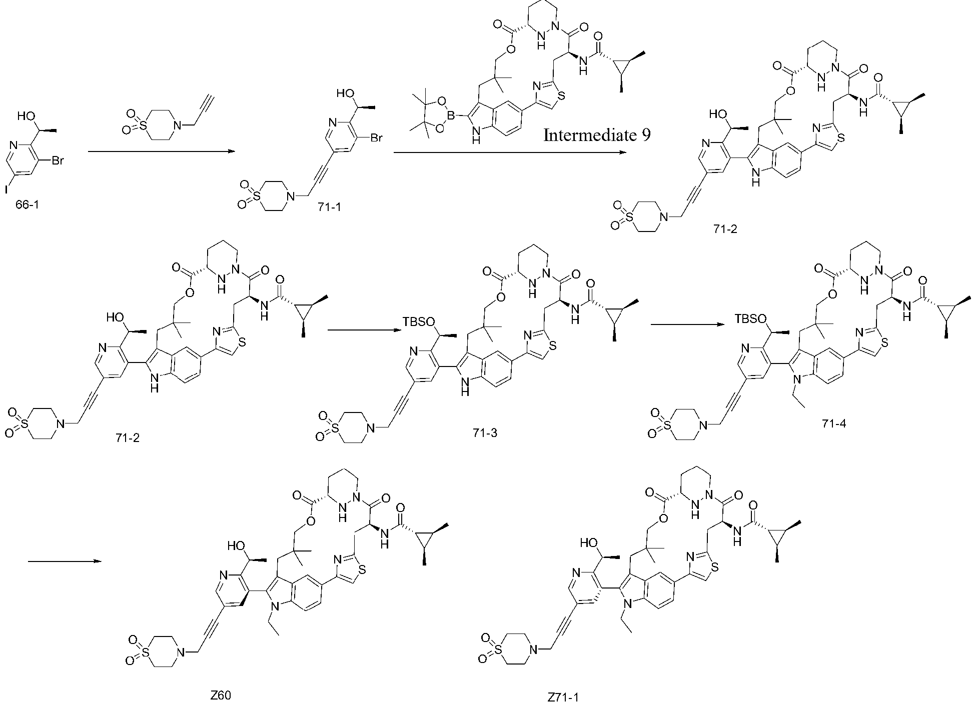

Step 1: Bis(triphenylphosphine)palladium(II) dichloride (53.51 mg, 0.076 mmol), copper(I) iodide (43.55 mg, 0.23 mmol), and N,N-diisopropylethylamine (0.80 mL, 4.58 mmol) were sequentially added to a solution of compound 66-1 (500 mg, 1.53 mmol) and 4-(prop-2-yn-1-yl)thiomorpholine 1,1-dioxide (343 mg, 1.98 mmol) in acetonitrile (10 mL) at 0 °C. The reaction mixture was purged 3 times with nitrogen and stirred at room temperature for 2 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-4%) to give compound 71-1 (550 mg, yield: 96.6%) as a yellow solid. ES-API: [M+H]⁺=373.0.

Step 2: Intermediate 9 (100 mg, 0.14 mmol), compound 71-1 (70.36 mg, 0.19 mmol), [1,1'-bis(di-tert-butylphosphino)ferrocene]dichloropalladium(II) (9.44 mg, 0.014 mmol), potassium phosphate (92.33 mg, 0.435 mmol), 1,4-dioxane (2 mL), toluene (2 mL), and water (0.8 mL) were added to a flask. The reaction mixture was purged 3 times with nitrogen and stirred at 65 °C for 5 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-3%) to give compound 71-2 (100 mg, yield: 80.5%) as a yellow solid. ES-API: [M+H]+=856.2.

Step 3: Compound 71-2 (280 mg, 0.33 mmol), imidazole (22.27 mg, 0.33 mmol), and triethylamine (0.1 mL, 0.72 mmol) were sequentially added to N,N-dimethylformamide (5 mL). tert-Butyldimethylsilyl chloride (73.95 mg, 0.49 mmol) was added thereto. The reaction mixture was stirred at 50 °C for 16 h under an oil bath. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-5%) to give compound 71-3 (250 mg, yield: 78.7%) as a yellow solid. ES-API: [M+H]⁺=970.3.

Step 4: Compound 71-3 (250 mg, 0.26 mmol), iodoethane (120.55 mg, 0.77 mmol), cesium carbonate (168 mg, 0.52 mmol), acetonitrile (5 mL), and N,N-dimethylformamide (3 mL) were added to a flask. The reaction system was stirred at 25 °C for 16 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-5%) to give compound 71-4 (250 mg, yield: 97.2%). ES-API: [M+H]⁺= 998.3. Step 5: Compound 71-4 (250 mg, 0.25 mmol), tetrahydrofuran (6 mL), and a solution (0.75 mL, 0.75 mmol) of tetrabutylammonium fluoride in tetrahydrofuran were added to a flask. The reaction system was stirred at room temperature for 3 h. Water was added to the reaction mixture, and the mixture was extracted with ethyl acetate (50 mL × 3). The organic phase was washed with saturated brine, dried over sodium sulfate, and filtered. The filtrate was concentrated to give a crude product, and the crude product was purified by a flash silica gel column (tetrahydrofuran/petroleum ether: 0-80%) to give a racemic product (150 mg). The racemic product was purified by preparative chiral HPLC (separation column: Daicel CHIRALPAK^{®} IC 250 × 4.6 mm × 5 µm; mobile phase: A n-hexane + 0.2% diethylamine, B ethanol + 0.2% diethylamine; A:B = 40:60; flow rate: 1 mL/min; column temperature: 30 °C) to give 2 isomeric compounds. The structure of one of the isomeric compounds was (1r,2R,3S)-N-((6³S,4S,Z)-(Rₐ)-1²-(5-(3-(1,1-dioxidothiomorpholino)prop-1-yn-1-yl)-2-((S)-1-hydroxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z60, 22 mg, yield: 9.9%, retention time: 18.735 min) as a white solid. ES-API: [M+H]⁺= 884.2. ¹HNMR (400MHz, DMSO-*d₆*): 8.82 (d, *J*=1.6Hz, 1H), 8.50 (s, 1H), 8.37 (d, *J*=8.8Hz, 1H), 7.81-7.73 (m, 3H), 7.56 (d, *J*=8.4Hz, 1H), 5.54 (t, *J*=9.2Hz, 1H), 5.26 (d, *J*=7.2Hz, 1H), 5.05 (d, *J*=12.0Hz, 1H), 4.61-4.56 (m, 1H), 4.25-4.16 (m, 4H), 3.75 (s, 2H), 3.61-3.52 (m, 2H), 3.30-3.29 (m, 1H), 3.16-3.12 (m, 5H), 3.06-2.95 (m, 5H), 2.80-2.70 (m, 1H), 2.39-2.32(m, 1H), 2.09-2.07(m, 1H), 1.85-1.70 (m, 2H), 1.60-1.45(m, 1H), 1.35 (d, *J*=6.0Hz, 3H), 1.20-1.05 (m, 9H), 0.92-0.83 (m, 6H), 0.34 (s, 3H). The structure of the other isomeric compound was (1r,2R,3S)-N-((6³S,4S,Z)-(Sₐ)-1²-(5-(3-(1,1-dioxidothiomorpholino)prop-1-yn-1-yl)-2-((S)-1-hydroxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z71-1, 68 mg, yield: 30.7%, retention time: 22.949 min) as a white solid. ES-API: [M+H]⁺= 884.2. ¹HNMR (400MHz, DMSO-*d₆*): 8.82 (d, *J*=1.6Hz, 1H), 8.52 (s, 1H), 8.39 (d, *J*=8.8Hz, 1H), 7.95 (d, *J*=2.0Hz, 1H), 7.80 (s, 1H), 7.74-7.72 (m, 1H), 7.54(d, *J*=8.8Hz, 1H), 5.54 (t, *J*=9.2Hz, 1H), 5.03-5.01 (m, 2H), 4.48-4.41 (m, 1H), 4.25-4.16 (m, 2H), 3.98-3.85(m, 2H), 3.75 (s, 2H), 3.64-3.52 (m, 2H), 3.32-3.29 (m, 1H), 3.22-3.15 (m, 5H), 3.06-2.95 (m, 5H), 2.80-2.70 (m, 1H), 2.45-2.42(m, 1H), 2.13-2.07(m, 1H), 1.85-1.70 (m, 2H), 1.61-1.46(m, 1H), 1.20-1.05 (m, 15H), 0.92 (s, 3H), 0.34 (s, 3H).

### Example 1.35. Synthesis of Compounds Z54 and Z54-1

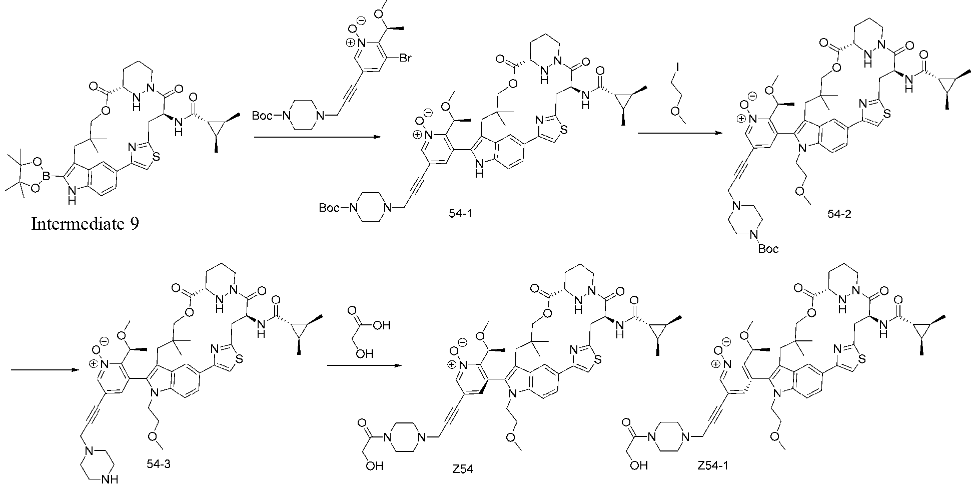

Step 1: Intermediate 9 (850 mg, 1.23 mmol), (S)-3-bromo-5-(3-(4-(tert-butoxycarbonyl)piperazin-1-yl)prop-1-yn-1-yl)-2-(1-methoxyethyl)pyridine-1-oxide (compound 48-1, 840 mg, 1.85 mmol), [1,1'-bis(di-tert-butylphosphino)ferrocene]dichloropalladium(II) (80 mg, 0.12 mmol), potassium phosphate (784 mg, 0.69 mmol), tetrahydrofuran (9 mL), toluene (6 mL), and water (3 mL) were added to a flask. The reaction mixture was purged 3 times with nitrogen and stirred at 65 °C for 3 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-6%) to give compound 54-1 (800 mg, yield: 69.3%) as a yellow solid. ES-API: [M+H]⁺=937.3.

Step 2: Compound 54-1 (800 mg, 0.85 mmol), 1-iodo-2-methoxyethane (476.3 mg, 2.56 mmol), cesium carbonate (556.26 mg, 1.71 mmol), acetonitrile (3 mL), and N,N-dimethylformamide (3 mL) were added to a flask. The reaction system was stirred at 25 °C for 24 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-3%) to give compound 54-2 (750 mg, yield: 88.3%) as a yellow solid. ES-API: [M+H]⁺=995.3.

Step 3: Compound 54-2 (105 mg, 0.11 mmol), methanol (1 mL), and dichloromethane (1 mL) were added to a flask. A 4 M solution (3 mL) of hydrogen chloride in dioxane was added to the solution described above. The reaction system was stirred at room temperature for 1 h. The reaction mixture was concentrated to give crude compound 54-3 (94 mg, yield: 99.5%) as a yellow solid. ES-API: [M+H]⁺=895.5.

Step 4: Compound 54-3 (94 mg, 0.105 mmol), hydroxyacetic acid (8 mg, 0.105 mmol), N,N-diisopropylethylamine (0.04 mL, 0.229 mmol), *O*-(7-azabenzotriazol-1-yl)-N,N,N',N'-tetramethyluronium hexafluorophosphate (52 mg, 0.137 mmol), and dichloromethane (3 mL) were added to a flask. The reaction system was stirred at room temperature for 1 h. Water was added to the reaction mixture, and the mixture was extracted with dichloromethane (50 mL × 3). The organic phase was washed with saturated brine, dried over sodium sulfate, and filtered. The filtrate was concentrated, and the crude product was purified by prep-HPLC (ammonium bicarbonate method 2) to give 2 isomeric compounds. The structure of one of the isomeric compounds was (Rₐ)-3-((6³S,4S,Z)-4-((1r,2R,3S)-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacyclodecaphane-1²-yl)-5-(3-(4-(2-hydroxyacetyl)piperazin-1-yl)prop-1-yn-1-yl)-2-((S)-1-methoxyethyl)pyridine 1-oxide (Z54, 26 mg, yield: 26%, retention time: 8.23 min) as a white solid. ES-API: [M+H]⁺=953.3. ¹HNMR (400MHz, DMSO-*d₆*): 8.50-8.48 (m, 2H), 8.36 (d, *J*=8.8Hz, 1H), 7.81 (s, 1H),7.74-7.72 (m, 1H), 7.58 (d, *J*=8.8Hz, 1H), 7.24 (d,J=1.2Hz, 1H), 5.58 (t, *J*=9.2Hz, 1H),5.07 (d, *J*=12.4Hz, 1H), 4.51-4.36 (m, 3H), 4.26-4.11 (m, 3H), 4.06 (d, *J*=5.2Hz, 2H), 3.61-3.45 (m, 6H), 3.35-3.28 (m, 6H), 3.18-3.11 (m, 4H), 3.00-2.94 (m, 4H), 2.78-2.66 (m, 1H), 2.55-2.53(m, 2H), 2.40-2.37 (m, 1H), 2.10-2.01 (m, 1H), 1.85-1.70 (m, 2H), 1.61-1.45 (m, 4H), 1.20-1.05(m, 9H), 0.94(s, 3H), 0.52(s, 3H). The structure of the other isomeric compound was (Sₐ)-3-((6³S,4S,2)-4-((1r,2R,3S)-2,3-dimethylcyclopropane-1-carboxamido)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacyclodecaphane-1²-yl)-5-(3-(4-(2-hydroxyacetyl)piperazin-1-yl)prop-1-yn-1-yl)-2-((S)-1-methoxyethyl)pyridine 1-oxide (Z54-1, 38 mg, yield: 38%, retention time: 8.69 min) as a white solid. ES-API: [M+H]⁺=953.3. ¹HNMR (400MHz, DMSO-*d₆*): 8.52-8.45 (m, 2H), 8.39 (d, *J*=8.8Hz, 1H), 7.80 (s, 1H),7.74-7.70 (m, 1H), 7.53 (d, *J*=8.8Hz, 1H), 7.45 (d, *J*=1.2Hz, 1H), 5.53 (t, *J*=8.8Hz, 1H),5.04(d, *J*=12.0Hz, 1H), 4.64-4.49 (m, 2H), 4.26-4.17 (m, 3H), 4.06 (d, *J*=5.2Hz, 2H), 4.01-3.90 (m, 1H), 3.78-3.45 (m, 9H), 3.36-3.28 (m, 3H), 3.15-3.11 (m, 4H), 3.00-2.95 (m, 4H), 2.80-2.70 (m, 1H), 2.55-2.53(m, 2H), 2.20-2.11 (m, 2H), 1.85-1.75 (m, 2H), 1.54-1.50 (m, 1H), 1.30-1.26 (m, 3H),1.20-1.05(m, 9H), 0.96(s, 3H), 0.54(s, 3H).

### Example 1.36. Synthesis of Compound Z61

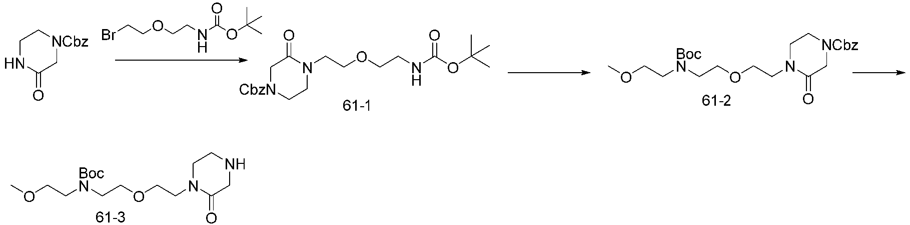

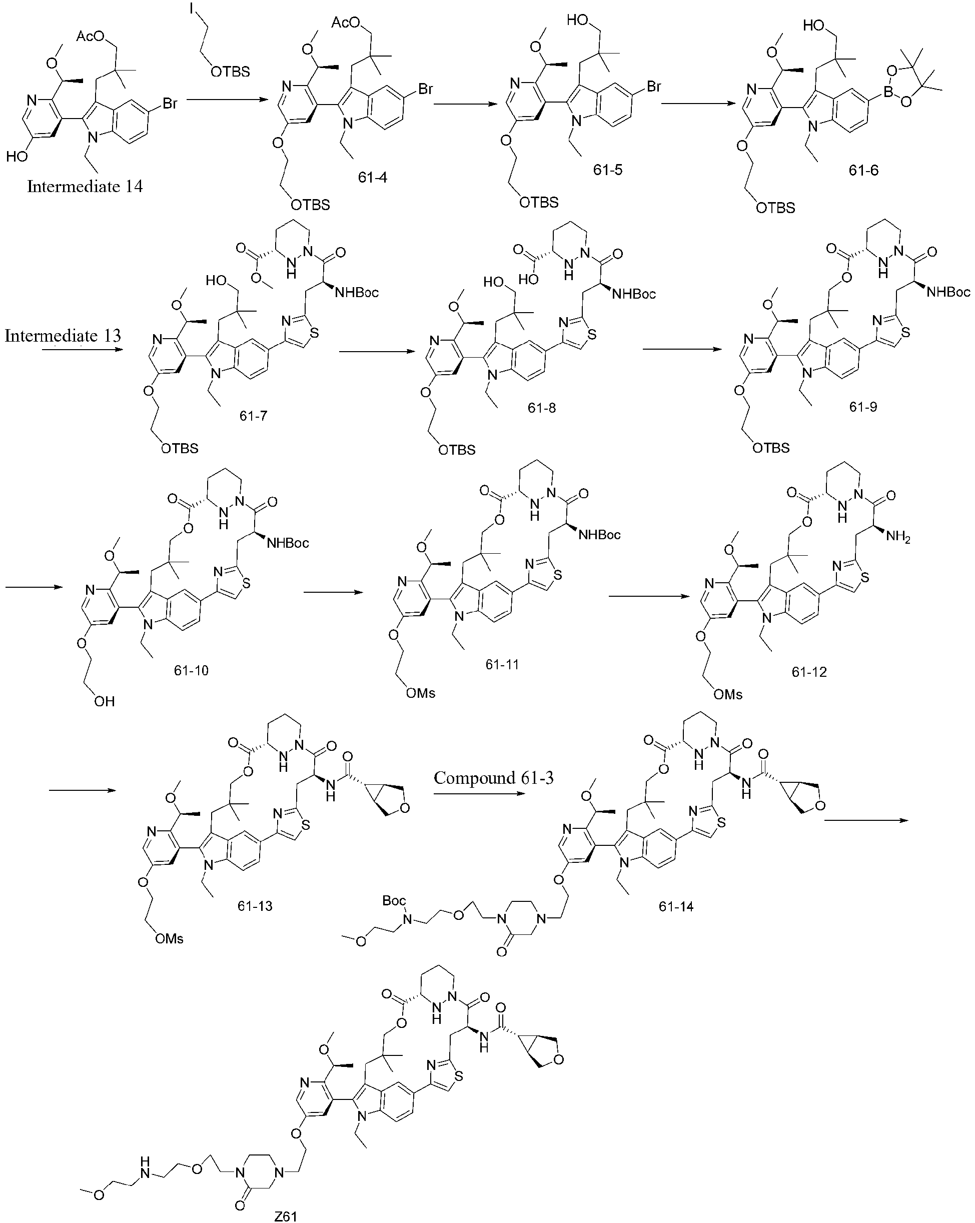

Step 1: Benzyl 3-oxopiperazine-1-carboxylate (2300 mg, 9.829 mmol) was dissolved in N,N-dimethylformamide (15 mL), and sodium hydride (179.48 mg, 4.487 mmol) was added at 0 °C. The mixture was reacted at room temperature for 1 h. Then 2-methylpropan-2-yl ({2-[(2-bromoethyl)oxy]ethyl}amino)formate (CAS: 164332-88-1, 6589.16 mg, 24.573 mmol) was added, and the mixture was reacted at room temperature for 5 h under a nitrogen atmosphere. After the reaction was completed, water (30 mL) was added to the reaction mixture, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 61-1 (1800 mg, 6.713 mmol, yield: 68.29%) as a colorless oily liquid, ES-API: [M-Boc+H]⁺ *=* 322.1.

Step 2: Compound 61-1 (2300 mg, 9.829 mmol) was dissolved in N,N-dimethylformamide (15 mL), and sodium hydride (400.00 mg, 10.000 mmol) was added at 0 °C. The mixture was reacted at room temperature for 1 h, and then 1-iodo-2-methoxyethane (1859.90 mg, 10.000 mmol) was added. The mixture was reacted at room temperature for 5 h under a nitrogen atmosphere. After the reaction was completed, water (30 mL) was added to the reaction mixture, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (ethyl acetate/petroleum ether: 0%-70%) to give compound 61-2 (800 mg, 1.251 mmol, yield: 37.54%) as a colorless oily liquid, ES-API: [M-Boc+H]⁺ = 380.2.

Step 3: Compound 61-2 (300 mg, 0.625 mmol) was dissolved in methanol (15 mL), and palladium on carbon (100 mg, 0.940 mmol) was added. The mixture was reacted at room temperature for 16 h. After the reaction was completed, the mixture was filtered under vacuum and concentrated under reduced pressure to give compound 61-3 (200 mg, 0.579 mmol, yield: 92.64%) as a colorless oily liquid, ES-API: [M+H]⁺ = 346.2.

Step 4: Intermediate 14 (10 g, 19.863 mmol) was dissolved in N,N-dimethylformamide (50 mL), and potassium carbonate (5.49 g, 39.727 mmol) and 1-iodo-4,4,5,5-tetramethyl-3-oxa-4-silahexane (8.53 g, 29.795 mmol) were added. The mixture was reacted at room temperature for 16 h. After the reaction was completed, water (100 mL) was added to the reaction mixture, and the mixture was extracted with ethyl acetate (50 mL × 2). The organic phase was washed with saturated brine (100 mL × 3), dried, and concentrated to give a crude product. The crude product was purified by a flash silica gel column (ethyl acetate/petroleum ether: 0%-70%) to give compound 61-4 (9000 mg, 13.600 mmol, yield: 68.47%) as a yellow solid, ES-API: [M+H]⁺ = 661.2.

Step 5: Compound 61-4 (9 g, 13.600 mmol) was dissolved in tetrahydrofuran (30 mL), methanol (10 mL), and water (10 mL). Lithium hydroxide monohydrate (1.43 g, 34.001 mmol) was added to the solution described above under an ice bath. After the addition was completed, the reaction system was stirred at room temperature for 17 h. The pH of the reaction mixture was adjusted to about 6 with diluted hydrochloric acid (1 M). The mixture was then extracted with ethyl acetate (50 mL × 2). The organic phase was washed with saturated brine (20 mL), dried, and concentrated to give compound 61-5 (7000 mg, 11.295 mmol, yield: 83.05%) as a yellow oily liquid. ES-API: [M +H]⁺=619.2.

Step 6: Compound 61-5 (5 g, 8.068 mmol) was dissolved in anhydrous dioxane (25 mL), and bis(pinacolato)diboron (4.10 g, 16.136 mmol), [1,1'-bis(diphenylphosphino)ferrocene]palladium(II) dichloride (1.18 g, 1.614 mmol), and potassium acetate (1.98 g, 20.170 mmol) were added to the solution described above at room temperature. After the addition was completed, the reaction system was stirred at 90 °C for 3 h under a nitrogen atmosphere. After the reaction was completed, the reaction mixture was filtered using a sand core funnel and concentrated. The residue was purified by a silica gel column (ethyl acetate/petroleum ether: 0-100%) to give compound 61-6 (4500 mg, 6.749 mmol, yield: 83.65%) as a yellow oily liquid. ES-API: [M +H]⁺=667.4.

Step 7: Compound 61-6 (5 g, 6.749 mmol) and intermediate 13 (3.87 g, 8.099 mmol) were dissolved in dioxane (10 mL), toluene (30 mL), and water (10 mL). [1,1'-Bis(di-tert-butylphosphino)ferrocene]dichloropalladium(II) (444 mg, 0.675 mmol) and potassium phosphate (2.87 g, 13.5 mmol) were added to the solution described above at room temperature. After the addition was completed, the reaction system was stirred at 70 °C for 5 h under an argon atmosphere. After the reaction was completed, water (30 mL) was added to the solution described above, and the mixture was extracted with ethyl acetate (60 mL × 2). The organic phase was washed with saturated brine (50 mL), dried, and concentrated to give a crude product. The crude product was purified by a flash silica gel column (methanol/dichloromethane: 0%-3%) to give compound 61-7 (5 g, 5.335 mmol, yield: 79.04%) as a yellow solid. ES-API: [M +H]⁺=937.3.

Step 8: Compound 61-7 (4.2 g, 4.481 mmol) was dissolved in tetrahydrofuran (30 mL) and water (10 mL). Lithium hydroxide monohydrate (0.389 g, 8.9 mmol) was added to the solution described above under an ice bath. After the addition was completed, the reaction system was stirred at room temperature for 7 h. The pH of the reaction mixture was adjusted to about 6 with diluted hydrochloric acid (1 M). The mixture was then extracted with ethyl acetate (50 mL × 2). The organic phase was washed with saturated brine (20 mL), dried, and concentrated to give compound 61-8 (3.8 g, 4.116 mmol, yield: 91.85%) as a yellow oily liquid. ES-API: [M +H]⁺=923.3.

Step 9: Compound 61-8 (3.5 g, 3.734 mmol), 1-hydroxybenzotriazole (100 mg, 0.76 mmol), and N,N-diisopropylethylamine (13 mL, 75.8 mmol) were dissolved in dichloromethane (100 mL). 1-(3-Dimethylaminopropyl)-3-ethylcarbodiimide hydrochloride (14.5 g, 75.8 mmol) was added to the solution described above under an ice bath. After the addition was completed, the reaction system was stirred at room temperature for 17 h. After the reaction was completed, the reaction mixture was washed with saturated brine (50 mL × 2), dried, and concentrated to give a crude product. The crude product was purified by a flash silica gel column (methanol/dichloromethane: 0%-5%) to give compound 61-9 (2 g, 2.166 mmol, yield: 57.97%) as a light yellow solid. ES-API: [M +H]⁺=905.3.

Step 10: Compound 61-9 (400 mg, 0.442 mmol) was dissolved in tetrahydrofuran (3 mL), and a solution (1 M, 0.825 mL, 0.825 mmol) of tetrabutylammonium fluoride in tetrahydrofuran was added. The mixture was reacted at room temperature for 1 h under a nitrogen atmosphere. After the reaction was completed, water (10 mL) was added, and the mixture was extracted with ethyl acetate (15 mL × 2). The organic phase was washed with saturated brine (10 mL × 3, dried, and concentrated to give a crude product. The crude product was purified by a flash silica gel column (methanol/dichloromethane: 0%-10%) to give compound 61-10 (320 mg, 0.405 mmol, yield: 91.56%) as a pale yellow solid, ES-API: [M+H]⁺ = 791.3.

Step 11: Compound 61-10 (310 mg, 0.392 mmol) was dissolved in dichloromethane (5 mL), and methanesulfonic anhydride (88 mg, 0.509 mmol) and N,N'-diisopropylethylamine (76 mg, 0.588 mmol) were added. The mixture was reacted at room temperature for 1 h under a nitrogen atmosphere and concentrated to dryness under reduced pressure. The crude product was purified by a flash silica gel column (methanol/dichloromethane: 0%-10%) to give compound 61-11 (270 mg, 0.311 mmol, yield: 79.27%) as a pale yellow solid, ES-API: [M+H]⁺ = 869.3.

Step 12: Compound 61-11 (270 mg, 0.311 mmol) was dissolved in methanol (1 mL), and a solution (5 mL, 2 mmol) of hydrochloric acid in dioxane was added. The mixture was reacted at room temperature for 2 h and concentrated under reduced pressure to give compound 61-12 (230 mg, 0.299 mmol, yield: 96.28%) as a pale yellow solid, ES-API: [M+H]⁺ = 769.3.

Step 13: Compound 61-12 (200 mg, 0.260 mmol) and (1R,5S,6r)-3-oxabicyclo[3.1.0]hexane-6-carboxylic acid (39.99 mg, 0.312 mmol) were dissolved in N,N-dimethylformamide (3 mL). O-(7-Azabenzotriazol-1-yl)-N,N,N',N'-tetramethyluronium hexafluorophosphate (148.12 mg, 0.39 mmol) and N,N'-diisopropylethylamine (263.86 mg, 2.041 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3, dried, and concentrated to give a crude product. The crude product was purified by a flash silica gel column (methanol/dichloromethane: 0%-10%) to give compound 61-13 (200 mg, 0.228 mmol, yield: 87.47%) as a white solid, ES-API: [M+H]⁺ = 879.2.

Step 14: Compound 61-13 (126.53 mg, 0.366 mmol), compound 61-3 (126.53 mg, 0.366 mmol), and sodium iodide (78.44 mg, 0.523 mmol) were dissolved in acetonitrile (15 mL), and N,N'-diisopropylethylamine (170 mg, 1.3 mmol) was added. The mixture was reacted at room temperature for 18 h under a nitrogen atmosphere and concentrated to dryness under reduced pressure. The crude product was purified by a flash silica gel column (methanol/dichloromethane: 0%-10%) to give compound 61-14 (130 mg, 0.115 mmol, yield: 44.03%) as a pale yellow solid, ES-API: [M+H]⁺ = 1128.3.

Step 15: Compound 61-14 (30 mg, 0.027 mmol) was dissolved in methanol (1 mL), and a solution (5 mL, 2 mmol) of hydrochloric acid in dioxane was added. The mixture was reacted at room temperature for 2 h and concentrated under reduced pressure to give a crude product. The crude product was purified by prep-HPLC (trifluoroacetic acid method) to give (1*R,*5*S,*6*r*)*-*N-((6³*S,*4*S,Z*)-1¹-ethyl-(*Rₐ*)-1²-(2-((S)-1-methoxyethyl)-5-(2-(4-(2-(2-((2-methoxyethyl)(amino)ethoxy)ethyl)-3-oxopiperazin-1-yl)ethoxy)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-ylbicyclo[3.1.0]hexane-6-carboxamide (Z61, 10 mg, 0.009 mmol, yield: 32.93%, trifluoroacetate) as a white solid, ES-API: [M+H]⁺ = 1028.3.

### Example 1.37. Synthesis of Compound Z62

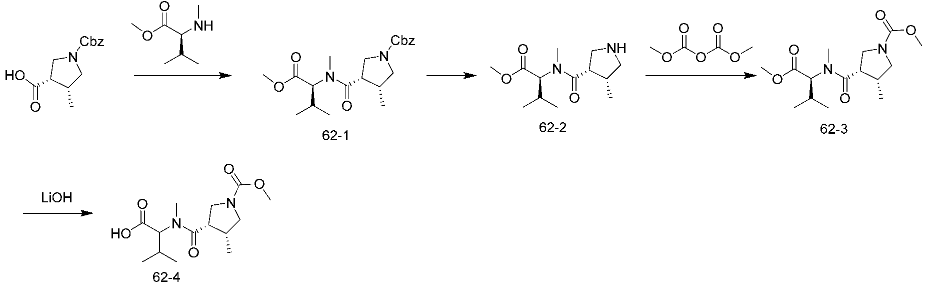

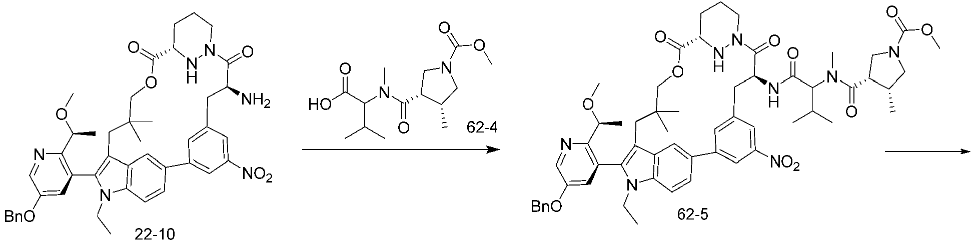

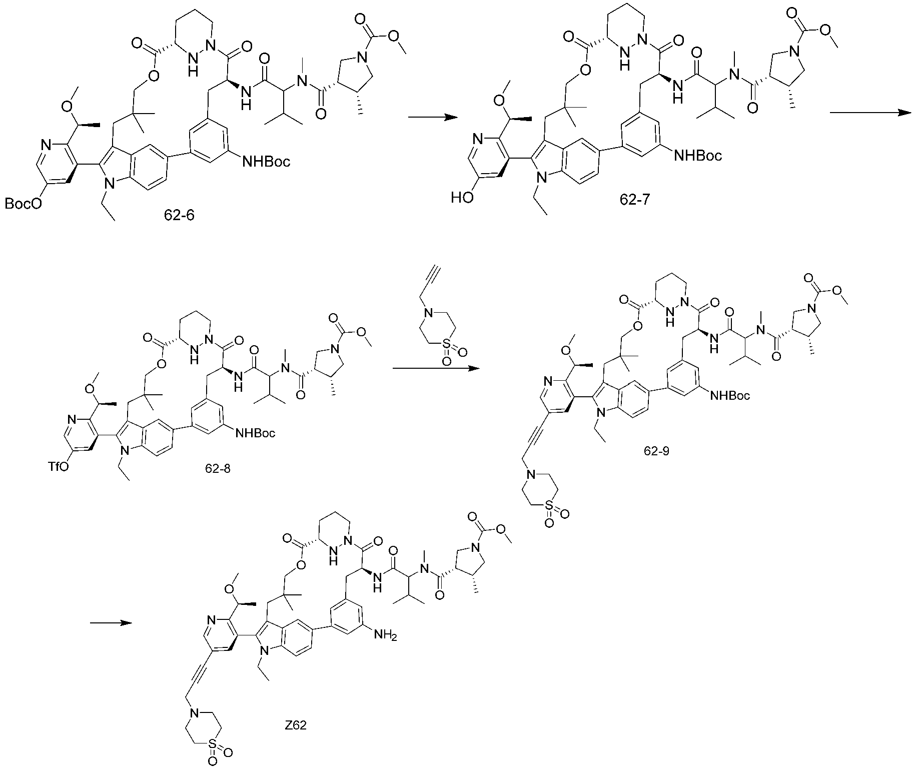

Step 1: (3S,4R)-1-((Benzyloxy)carbonyl)-4-methylpyrrolidine-3-carboxylic acid (250 mg, 0.950 mmol) was dissolved in dichloromethane (10 mL). Methyl methyl-L-valinate (224.24 mg, 1.234 mmol), O-(7-azabenzotriazol-1-yl)*-N,N,N',N'-t*etramethyluronium hexafluorophosphate (722.09 mg, 1.899 mmol), and *N,N'-*diisopropylethylamine (368.18 mg, 2.849 mmol) were added to the solution described above. After the addition was completed, the reaction system was stirred at room temperature for 24 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (tetrahydrofuran/petroleum ether: 0%-40%) to give compound 62-1 (300 mg, 0.768 mmol, yield: 80.91%) as a colorless transparent oily liquid. ES-API: [M +H]⁺=391.1.

Step 2: Compound 62-1 (250 mg, 0.640 mmol) was dissolved in methanol (8 mL), and wet palladium on carbon (10 wt%, 68.13 mg, 0.064 mmol) was added to the solution described above. After the addition was completed, the system was purged with hydrogen three times by a hydrogen balloon to replace the air. The reaction system was stirred at room temperature for 17 h under a hydrogen atmosphere. After the reaction was completed, the reaction mixture was filtered to remove palladium on carbon, and the mother liquor was concentrated to give compound 62-2 (165 mg, crude product) as a colorless transparent oily liquid. ES-API: [M +H]⁺=257.2.

Step 3: Compound 62-2 (165 mg, crude product) was dissolved in dichloromethane (10 mL), and triethylamine (0.268 mL, 1.931 mmol) and dimethyl dicarbonate (172.61 mg, 1.287 mmol) were added to the solution described above. After the addition was completed, the reaction system was stirred at room temperature for 2 h. After the reaction was completed, water (20 mL) was added to the reaction mixture, and the mixture was extracted with ethyl acetate (20 mL × 2). The organic phase was washed with saturated brine (20 mL × 2), dried, and concentrated to give a crude product. The crude product was purified by a flash silica gel column (tetrahydrofuran/petroleum ether: 5%-40%) to give compound 62-3 (200 mg, 0.636 mmol, two-step yield: 98.84%) as a white solid. ES-API: [M +H]⁺=315.2.

Step 4: Compound 62-3 (150 mg, 0.477 mmol) was dissolved in tetrahydrofuran (2 mL) and water (2 mL), and lithium hydroxide (20 mg, 0.834 mmol) was added to the solution described above. After the addition was completed, the reaction system was stirred at room temperature for 2 h. After the reaction was completed, the reaction mixture was adjusted to weak acidity (pH = 6) with diluted hydrochloric acid (1 M) and then concentrated under reduced pressure to give crude compound 62-4 (200 mg) as a colorless transparent liquid. ES-API: [M +H]⁺=301.1.

Step 5: Compound 22-10 (170 mg, 0.219 mmol) was dissolved in dichloromethane (5 mL). Compound 62-4 (65.89 mg, crude product), O-(7-azabenzotriazol-1-yl)-*N,N,N',N'*-tetramethyluronium hexafluorophosphate (166.83 mg, 0.439 mmol), and N,N'-diisopropylethylamine (85.06 mg, 0.658 mmol) were added to the solution described above. After the addition was completed, the reaction system was stirred at room temperature for 24 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-3%) to give compound 62-5 (200 mg, 0.189 mmol, yield: 86.23%) as a light yellow solid. ES-API: [M +H]⁺= 1057.3.

Step 6: Compound 62-5 (200 mg, 0.189 mmol) and di-tert-butyl dicarbonate (0.217 mL, 0.946 mmol) were dissolved in methanol (10 mL). Wet palladium on carbon (10%, 100 mg, 0.094 mmol) was added to the solution described above. After the addition was completed, the reaction system was stirred at 30 °C for 17 h under a hydrogen atmosphere. After the reaction was completed, the reaction mixture was filtered and concentrated to give compound 62-6 (400 mg, crude product) as a colorless transparent liquid. ES-API: [M-Boc +H]⁺= 1037.3. Step 7: Compound 62-6 (400 mg, crude product) was dissolved in methanol (10 mL), and potassium carbonate (24.30 mg, 0.176 mmol) was added. After the addition was completed, the reaction system was stirred at room temperature for 2 h. After the reaction was completed, water (20 mL) was added to the reaction mixture, and the mixture was extracted with ethyl acetate (20 mL × 2). The organic phase was washed with saturated brine (30 mL × 2), dried, and concentrated to give a crude product. The crude product was purified by a flash silica gel column (methanol/dichloromethane: 0%-5%) to give compound 62-7 (150 mg, 0.145 mmol, two-step yield: 76.5%) as a white solid. ES-API: [M+H]⁺= 1037.3.

Step 8: Compound 62-7 (100 mg, 0.096 mmol) was dissolved in dichloromethane (3 mL). Triethylamine (0.040 mL, 0.289 mmol) and N-phenyl-bis(trifluoromethanesulfonimide) (51.66 mg, 0.145 mmol) were added to the solution described above at room temperature. After the addition was completed, the reaction system was stirred at room temperature for 1 h. After the reaction was completed, the reaction mixture was concentrated to give a crude product. The crude product was purified by a flash silica gel column (methanol/dichloromethane: 0%-3%) to give compound 62-8 (80 mg, 0.068 mmol, yield: 70.97%) as a white solid. ES-API: [M +H]⁺= 1169.3.

Step 9: Compound 62-8 (80 mg, 0.068 mmol) and 4-(prop-2-yn-1-yl)thiomorpholine 1,1-dioxide (35.55 mg, 0.205 mmol) were dissolved in acetonitrile (3 mL). Bis(triphenylphosphine)palladium(II) dichloride (9.60 mg, 0.014 mmol), copper(I) iodide (5.21 mg, 0.027 mmol), N,N'-diisopropylethylamine (26.53 mg, 0.205 mmol), and lithium chloride (11.60 mg, 0.274 mmol) were added to the solution described above. After the addition was completed, the reaction system was stirred at 80 °C for 4 h. After the reaction was completed, water (20 mL) was added to the reaction mixture, and the mixture was extracted with ethyl acetate (20 mL × 2). The organic phase was washed with saturated brine (30 mL × 2), dried, and concentrated to give a crude product. The crude product was purified by a flash silica gel column (methanol/dichloromethane: 0%-3%) to give compound 62-9 (65 mg, 0.055 mmol, yield: 79.68%) as a light yellow solid. ES-API: [M +H]⁺= 1192.3.

Step 10: Compound 62-9 (65 mg, 0.055 mmol) was dissolved in dichloromethane (3 mL), and trifluoroacetic acid (1 mL) was added to the solution described above. After the addition was completed, the reaction system was stirred at room temperature for 2 h. After the reaction was completed, the reaction mixture was concentrated to remove the solvent. A saturated aqueous sodium bicarbonate solution (10 mL) was added to the residue, and the mixture was extracted with ethyl acetate (10 mL × 2). The organic phase was separated, washed with saturated brine (10 mL × 2), dried, and concentrated to give a crude product. The crude product was purified by prep-HPLC (ammonium bicarbonate method 1) to give methyl (3S,4R)-3-((1-(((6³S,4S)-2⁵-amino-(*Rₐ*)-1²-(5-(3-(1,1-dioxidothiomorpholino))prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-1(5,3)-indola-6(1,3)-pyridazina-2(1,3)-benzenacycloundecaphane-4-yl)amino)-3-methyl-1-oxobutan-2-yl)(methyl)carbamoyl)-4-methylpyrrolidine-1-carboxylate (Z62, 8 mg, 0.007 mmol, yield: 17.47%) as a white solid. ES-API: [M +H]⁺=1092.3.

### Example 1.38. Synthesis of Compounds Z63-1 and Z63-2

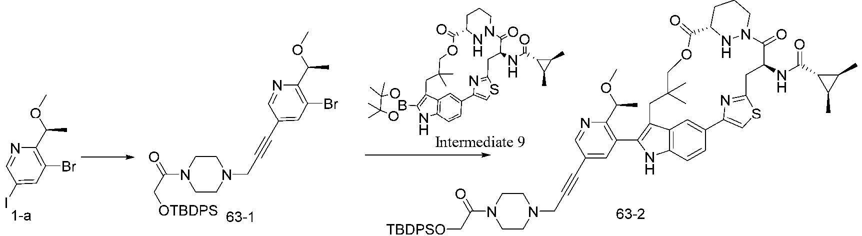

Step 1: Copper(I) iodide (33.42 mg, 0.175 mmol), 2-((tert-butyldiphenylsilyl)oxy)-1-(4-(prop-2-yn-1-yl)piperazin-1-yl)ethan-1-one (compound 38-1, 492.1 mg, 1.170 mmol), and N,N-diisopropylethylamine (0.98 mL, 5.611 mmol) were added to a solution of compound 1-a (400.0 mg, 1.170 mmol) and bis(triphenylphosphine)palladium(II) dichloride (41.05 mg, 0.058 mmol) in acetonitrile (15 mL) at 0 °C, and the mixture was stirred at room temperature for 12 h. After the reaction was completed, the mixture was diluted with ethyl acetate (100 mL), washed with saturated brine (30 mL), dried over anhydrous sodium sulfate, filtered, and concentrated. The residue was purified by automated flash chromatography on silica gel (20 g, elution by a 0-50% solution of tetrahydrofuran in petroleum ether) to give compound 63-1 (520.0 mg, 0.819 mmol, yield: 70.04%) as a yellow solid. ES-API[M+H]⁺= 634.2.

Step 2: Intermediate 9 (100.0 mg, 0.145 mmol), compound 63-1 (227.31 mg, 0.609 mmol), [1,1'-bis(di-tert-butylphosphino)ferrocene]dichloropalladium(II) (9.44 mg, 0.014 mmol), and potassium phosphate (92.33 mg, 0.435 mmol) were added to a mixed solution of dioxane (6 mL), toluene (6 mL), and water (2 mL). The mixture was stirred at 90 °C for 2 h under a N₂ atmosphere. After the reaction was completed, the reaction was quenched with water, and the mixture was extracted with ethyl acetate (100 mL). The organic layer was washed with saturated brine (100 mL), dried over anhydrous sodium sulfate, filtered, and concentrated. The residue was purified by automated flash chromatography on silica gel (a 0-70% solution of tetrahydrofuran in petroleum ether) to give compound 63-2 (100.0 mg, 0.089 mmol, yield: 61.72%) as a yellow solid. ES-API[M+H]⁺= 1117.3. Step 3: 1-Iodo-2-methoxyethane (49.93 mg, 0.268 mmol) was added to a mixture of compound 63-2 (100.0 mg, 0.089 mmol) and cesium carbonate (87.47 mg, 0.268 mmol) in N,N-dimethylformamide (3 mL)/acetonitrile (3 mL). The mixture was stirred at 25 °C for 24 h. After the reaction was completed, the reaction was quenched with water, and the mixture was extracted with ethyl acetate (100 mL). The organic layer was washed with saturated brine (100 mL), dried over anhydrous sodium sulfate, filtered, and concentrated. The residue was purified by automated flash chromatography on silica gel (a 0-3% solution of methanol in dichloromethane) to give the desired compound 63-3 (105.0 mg, yield: 99.81%). ES-API[M+H]⁺= 1175.3.

Step 4: Tetrabutylammonium fluoride (1.0 mL, 1.0 mmol) was added to a solution of compound 63-3 (100.0 mg, 0.085 mmol) in tetrahydrofuran (5.0 mL) at 0 °C, and then the mixture was stirred at room temperature for 0.5 h. After the reaction was completed, the solution was extracted with ethyl acetate (30 mL) and saturated brine (3 × 20 mL). The organic phase was dried over anhydrous sodium sulfate and filtered, and the filtrate was concentrated to give a racemate. The racemate was purified by resolution (Acq. Method Set: C3_AB_40_60_1ML_30C; Injection #: 1 Label: IB-HEX-IPA-DEA-40-60-02; Injection Volume: 10.00 µL Channel Name: 2998 Ch2 254 nm@1.2 nm: Run Time: 25.0 Minutes Proc. Chnl. Descr.: 2998 Ch2 254 nm@1.2 nm) to give 2 isomeric compounds. The structure of one of the isomeric compounds was (2R,3S)-N-((6³S,4S,Z)-1²-(5-(3-(4-(2-hydroxyacetyl)piperazin-1-yl)prop-1-yn-1-yl)-(Rₐ)-2-((S)-1-methoxyethyl)pyridin-3-yl)-1'-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z63-1, 5.06 mg, retention time: 20.026 min), ES-API: [M+H]⁺ = 937.3. The structure of the other isomeric compound was (2R,3S)-N-((6³S,4S,Z)-1²-(5-(3-(4-(2-hydroxyacetyl)piperazin-1-yl)prop-1-yn-1-yl)-(Sₐ)-2-((S)-1-methoxyethyl)pyridin-3-yl)-1¹-(2-methoxyethyl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z63-2, retention time: 14.90 min, 11.13 mg), ES-API: [M+H]⁺ = 937.3.

### Example 1.39. Synthesis of Compounds Z57 and Z57-1

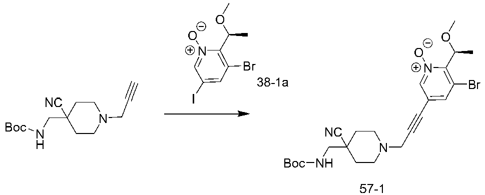

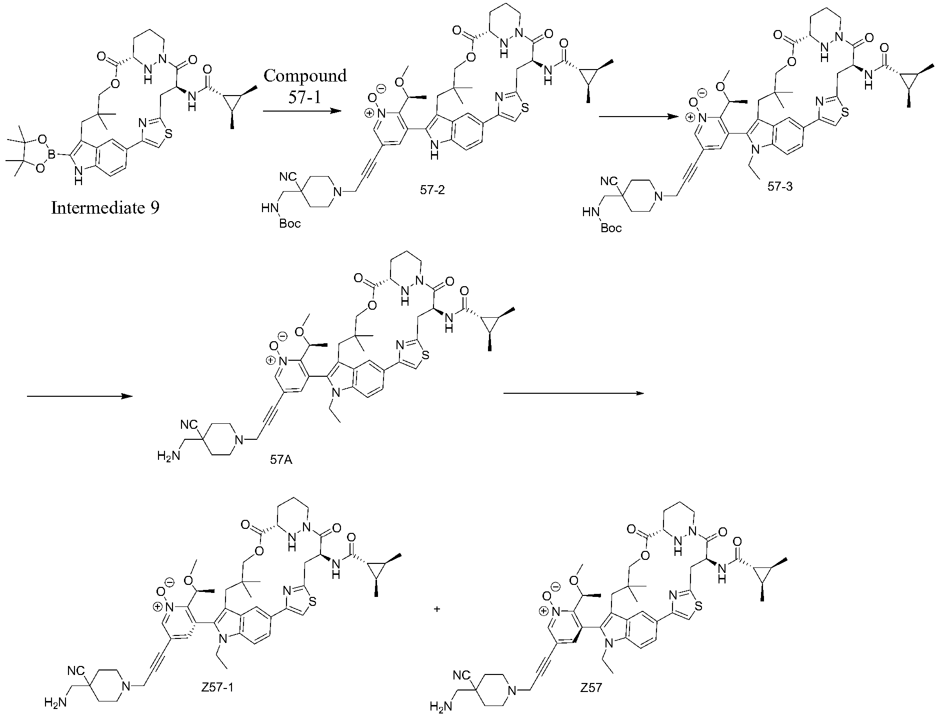

Step 1: tert-Butyl ((4-cyano-1-(prop-2-yn-1-yl)piperidin-4-yl)methyl)carbamate (500 mg, 1.397 mmol) and compound 38-1a (503.73 mg, 1.816 mmol) were dissolved in acetonitrile (15 mL). Bis(triphenylphosphine)palladium(II) dichloride (49.02 mg, 0.070 mmol), copper(I) iodide (39.90 mg, 0.210 mmol), and N,N'-diisopropylethylamine (0.98 mL, 5.611 mmol) were added to the solution described above. After the addition was completed, the reaction system was stirred at room temperature for 4 h under a nitrogen atmosphere. After the reaction was completed, water (30 mL) was added to the reaction mixture, and the mixture was extracted with ethyl acetate (30 mL × 2). The organic phase was washed with saturated brine (30 mL × 2), dried, and concentrated to give a crude product. The crude product was purified by a flash silica gel column (methanol/dichloromethane: 0%-3%) to give compound 57-1 (500 mg, 0.985 mmol, yield: 70.53%) as a light yellow solid. ES-API: [M+H]⁺= 507.1, 509.1.

Step 2: Intermediate 9 (200 mg, 0.290 mmol) was dissolved in dioxane (2 mL), toluene (3 mL), and water (1 mL). Compound 57-1 (191.29 mg, 0.377 mmol), [1,1'-bis(di-tert-butylphosphino)ferrocene]dichloropalladium(II) (18.88 mg, 0.029 mmol), and potassium phosphate (184.66 mg, 0.870 mmol) were added to the solution described above at room temperature. After the addition was completed, the reaction system was stirred at 70 °C for 3 h under an argon atmosphere. After the reaction was completed, water (30 mL) was added to the reaction mixture, and the mixture was extracted with ethyl acetate (30 mL × 2). The organic phase was washed with saturated brine (30 mL × 3), dried, and concentrated to give a crude product. The crude product was purified by a flash silica gel column (methanol/dichloromethane: 0%-4%) to give compound 57-2 (200 mg, 0.202 mmol, yield: 69.65%) as a light yellow solid. ES-API: [M+H]⁺=990.7.

Step 3: Compound 57-2 (200 mg, 0.202 mmol) was dissolved in N,N-dimethylformamide (5 mL). Iodoethane (0.065 mL, 0.808 mmol) and cesium carbonate (263.23 mg, 0.808 mmol) were added to the solution described above. The reaction system was stirred at room temperature for 48 h. After the reaction was completed, water (15 mL) was added to the solution described above, and the mixture was extracted with ethyl acetate (20 mL × 2). The organic phase was washed with saturated brine (20 mL × 3), dried, and concentrated to give a crude product. The crude product was purified by a flash silica gel column (methanol/dichloromethane: 0%-5%) to give compound 57-3 (170 mg, 0.167 mmol, yield: 82.66%) as a light yellow solid. ES-API: [M+H]⁺=1018.5. Step 4: Compound 57-3 (20 mg, 0.020 mmol) was dissolved in dichloromethane (1 mL), and trifluoroacetic acid (0.5 mL) was added to the solution described above. After the addition was completed, the mixture was stirred at room temperature for 1 h. After the reaction was completed, the reaction mixture was concentrated to remove trifluoroacetic acid. The resulting residue was neutralized with a solution (7 *M*) of ammonia in methanol to weak alkalinity (pH = 8). After the neutralization was completed, the solution was concentrated to give crude compound 57A. Crude compound 57A was then subjected to chiral resolution (column type: IG 250 mm, 10 mm, 5 µm; mobile phase system: (A: acetonitrile; B: isopropanol; C: diethylamine); flow rate: 1 mL/min; A/B/C = 70/30/2; column temperature: 30 °C) to give 2 isomeric compounds. The structure of one of the isomeric compounds was 5-(3-(4-(aminomethyl)-4-cyanopiperidin-1-yl)prop-1-yn-1-yl)-(*Sₐ*)-3-((6³*S*,4*S*,*Z*)-4-((1*r*,2*R*,3*S*)-2,3-dimethylcyclopropane-1-carboxamido)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-1²-yl)-2-((*S*)-1-methoxyethyl)pyridine 1-oxide (Z57-1, 1.79 mg, 0.002 mmol, yield: 13.31%, retention time: 6.523 min) as a white solid. The structure of the other isomeric compound was 5-(3-(4-(aminomethyl)-4-cyanopiperidin-1-yl)prop-1-yn-1-yl)-(*Rₐ*)-3-((6³*S*,4*S*,*Z*)-4-((1*r*,2*R*,3*S*)-2,3-dimethylcyclopropane-1-carboxamido)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-1²-yl)-2-((*S*)-1-methoxyethyl)pyridine 1-oxide (Z57, 2.40 mg, 0.003 mmol, yield: 13.31%, retention time: 8.789 min) as a white solid. ES-API: [M+H]⁺=918.3.

### Example 1.40. Synthesis of Compounds Z64-1 and Z64-2

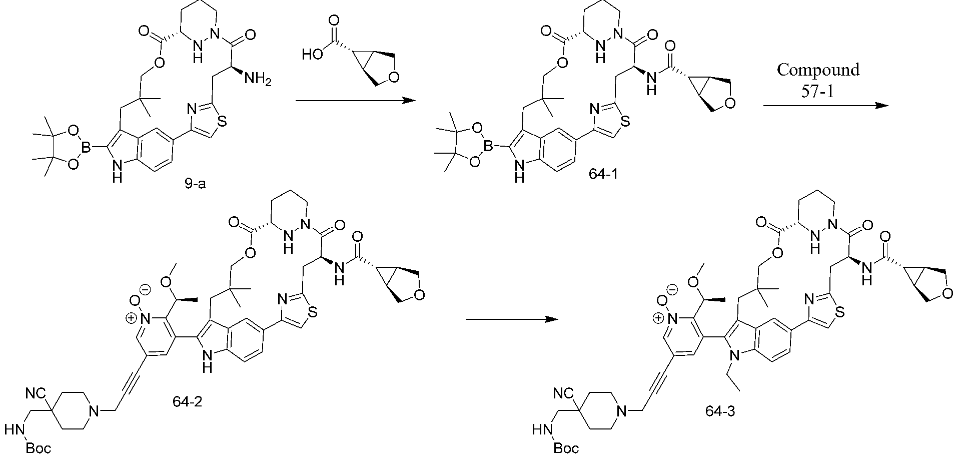

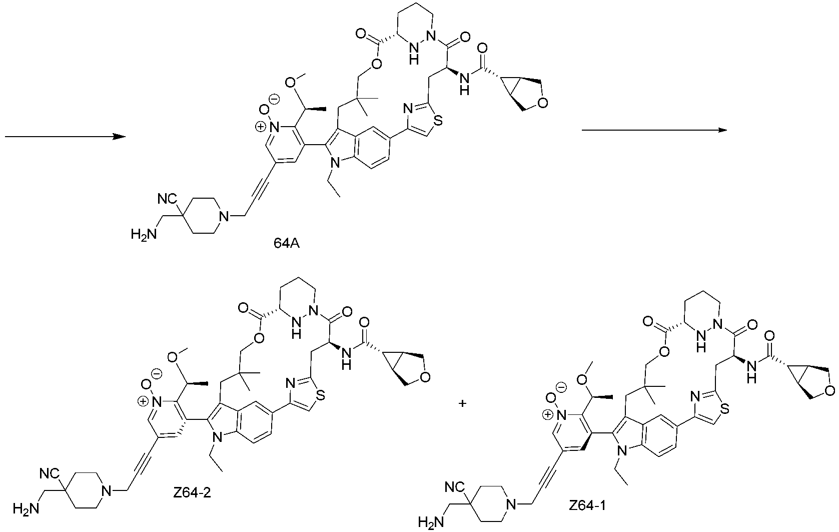

Step 1: Compound 9-a (1500 mg, 2.527 mmol) was dissolved in dichloromethane (50 mL). (1R,5S,6r)-3-Oxabicyclo[3.1.0]hexane-6-carboxylic acid (485.71 mg, 3.791 mmol), O-(7-azabenzotriazol-1-yl)-*N,N,N',N*'-tetramethyluronium hexafluorophosphate (1249.21 mg, 3.285 mmol), and N,N'-diisopropylethylamine (979.91 mg, 7.582 mmol) were added to the solution described above. After the addition was completed, the mixture was stirred at room temperature for 24 h. After the reaction was completed, the reaction mixture was concentrated to remove dichloromethane. Water (200 mL) was added to the residue, and the mixture was extracted with ethyl acetate (250 mL × 2). The organic phase was washed with saturated brine (200 mL × 2), dried, and concentrated to give a crude product. The crude product was purified by a flash silica gel column (ethyl acetate/petroleum ether: 0%-70%) to give compound 64-1 (1600 mg, 2.274 mmol, yield: 89.98%) as a light yellow solid. ES-API: [M +H]⁺=704.2.

Step 2: Compound 64-1 (200 mg, 0.284 mmol) was dissolved in dioxane (2 mL), toluene (3 mL), and water (1 mL). Compound 57-1 (187.49 mg, 0.369 mmol), [1,1'-bis(di-tert-butylphosphino)ferrocene]dichloropalladium(II) (18.50 mg, 0.028 mmol), and potassium phosphate (180.99 mg, 0.853 mmol) were added to the solution described above at room temperature. After the addition was completed, the reaction system was stirred at 70 °C for 3 h under an argon atmosphere. After the reaction was completed, water (30 mL) was added to the reaction mixture, and the mixture was extracted with ethyl acetate (30 mL × 2). The organic phase was washed with saturated brine (30 mL × 3), dried, and concentrated to give a crude product. The crude product was purified by a flash silica gel column (methanol/dichloromethane: 0%-4%) to give compound 64-2 (200 mg, 0.199 mmol, yield: 70.07%) as a light yellow solid. ES-API: [M+H]⁺=1004.7.

Step 3: Compound 64-2 (200 mg, 0.199 mmol) was dissolved in N,N-dimethylformamide (5 mL). Iodoethane (124.25 mg, 0.797 mmol) and cesium carbonate (259.56 mg, 0.797 mmol) were added to the solution described above. The reaction system was stirred at room temperature for 48 h. After the reaction was completed, water (15 mL) was added to the solution described above, and the mixture was extracted with ethyl acetate (20 mL × 2). The organic phase was washed with saturated brine (20 mL × 3), dried, and concentrated to give a crude product. The crude product was purified by a flash silica gel column (methanol/dichloromethane: 0%-5%) to give compound 64-3 (150 mg, 0.145 mmol, yield: 72.96%) as a light yellow solid. ES-API: [M+H]⁺=1032.3.

Step 4: Compound 64-3 (20 mg, 0.019 mmol) was dissolved in dichloromethane (1 mL), and trifluoroacetic acid (0.5 mL) was added to the solution described above. After the addition was completed, the reaction system was stirred at room temperature for 1 h. After the reaction was completed, the reaction mixture was concentrated to remove trifluoroacetic acid. The resulting residue was neutralized with a solution (7 *M*) of ammonia in methanol to weak alkalinity (pH = 8). After the neutralization was completed, the solution was concentrated to give crude compound 64A. Crude compound 64A was then subjected to chiral resolution (column type: IG 250 mm, 10 mm, 5 µm; mobile phase system: (A: acetonitrile; B: isopropanol; C: diethylamine); flow rate: 1 mL/min; A/B/C = 70/30/2; column temperature: 30 °C) to give 2 isomeric compounds. The structure of one of the isomeric compounds was (*Sₐ*)-3-((6³S,4S,Z)-4-((1R,5S,6r)-3-oxabicyclo[3.1.0]hexane-6-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(aminomethyl)-4-cyanopiperidin-1-yl)prop-1-yn-1-yl)-2-((S)-1-methoxyethyl)pyridine 1-oxide (Z64-2, 0.93 mg, 0.001 mmol, yield: 5.15%, retention time: 7.854 min). The structure of the other isomeric compound was (*Rₐ*)-3-((6³S,4S,Z)-4-((1R,5S,6r)-3-oxabicyclo[3.1.0]hexane-6-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(aminomethyl)-4-cyanopiperidin-1-yl)prop-1-yn-1-yl)-2-((S)-1-methoxyethyl)pyridine 1-oxide (Z64-1, 1.50 mg, 0.002 mmol, yield: 8.47%, retention time: 11.035). Both are white solids. ES-API: [M+H]⁺=932.3.

### Example 1.41. Synthesis of Compounds Z45-1 and Z45-2

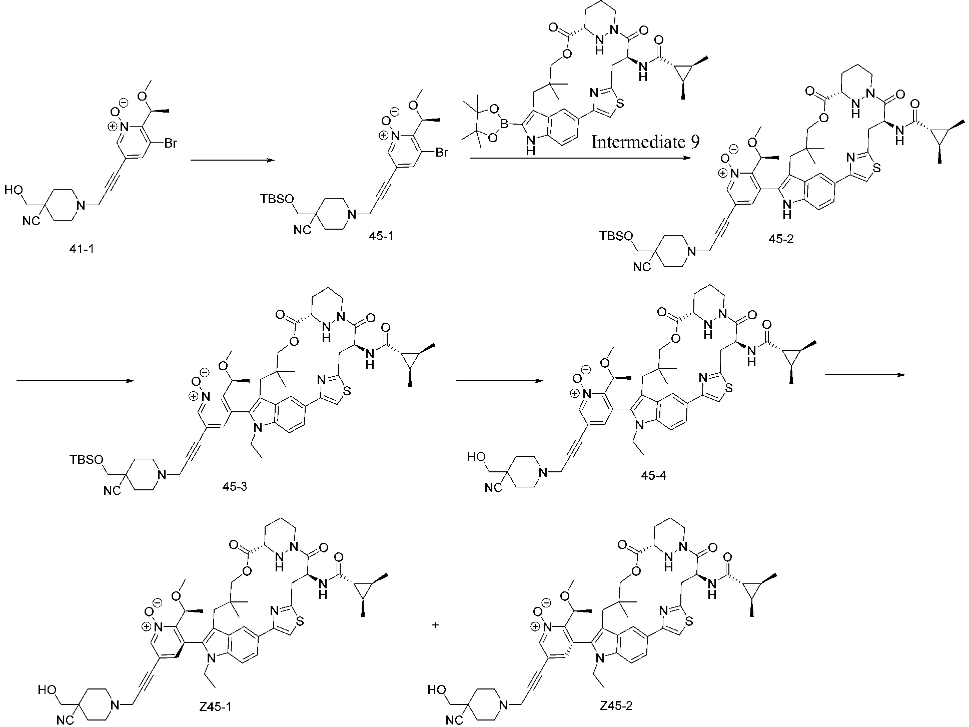

Step 1: Compound 41-1 (450 mg, 1.102 mmol) and N,N-diisopropylethylamine (0.5 mL, 2.863 mmol) were sequentially added to a solution of N,N-dimethylformamide (3 mL) and dichloromethane (5 mL). tertButyldimethylsilyl chloride (332 mg, 2.203 mmol) was added thereto. The reaction mixture was stirred at 40 °C for 16 h under an oil bath. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (methanol/dichloromethane: 0-5%) to give compound 45-1 (500 mg, yield: 86.8%) as a yellow solid. ES-API: [M+H]⁺=522.1.

Step 2: Intermediate 9 (400 mg, 0.58 mmol), compound 45-1 (400 mg, 0.76 mmol), [1,1'-bis(di-tert-butylphosphino)ferrocene]dichloropalladium(II) (37.76 mg, 0.058 mmol), potassium phosphate (369 mg, 1.74 mmol), 1,4-dioxane (6 mL), toluene (6 mL), and water (2 mL) were added to a flask. The reaction mixture was purged 3 times with nitrogen and stirred at 70 °C for 5 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (tetrahydrofuran/petroleum ether: 0-80%) to give compound 45-2 (400 mg, yield: 68.6%) as a yellow solid. ES-API: [M+H]⁺=1005.3.

Step 3: Compound 45-2 (400 mg, 0.40 mmol), iodoethane (186.17 mg, 1.19 mmol), cesium carbonate (259.27 mg, 0.80 mmol), acetonitrile (6 mL), and N,N-dimethylformamide (3 mL) were added to a flask. The reaction system was stirred at 25 °C for 24 h. Water was added to the reaction mixture, and the mixture was extracted with ethyl acetate (50 mL × 3). The organic phase was washed with saturated brine, dried over sodium sulfate, and filtered. The filtrate was concentrated to give compound 45-3 (400 mg, yield: 97.3%), and the crude product was directly used in the next step. ES-API: [M+H]⁺=1033.3.

Step 4: Compound 45-3 (400 mg, 0.387 mmol), tetrahydrofuran (3 mL), and a solution (1.2 mL, 1.200 mmol) of tetrabutylammonium fluoride in tetrahydrofuran were added to a flask. The reaction system was stirred at room temperature for 3 h. Water was added to the reaction mixture, and the mixture was extracted with ethyl acetate (50 mL × 3). The organic phase was washed with saturated brine, dried over sodium sulfate, and filtered. The filtrate was concentrated, and the crude product was purified by a flash silica gel column (tetrahydrofuran/petroleum ether: 0-80%) to give compound 45-4 (300 mg, yield: 84.3%), ES-API: [M+H]⁺ = 919.3.

Step 5: Compound 45-4 (30 mg, 0.033 mmol) was subjected to chiral resolution (separation column: Daicel CHIRALPAK^{®} IC 250 × 4.6 mm × 5 µm; mobile phase: A n-hexane + 0.2% diethylamine, B (ethanol:methanol = 1:1) + 0.2% diethylamine; A:B = 30:70; flow rate: 1 mL/min; column temperature: 30 °C) to give 2 isomeric compounds. The structure of one of the isomeric compounds was 5-(3-(4-cyano-4-(hydroxymethyl)piperidin-1-yl)prop-1-yn-1-yl)-(Rₐ)-3-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((*S*)-1-methoxyethyl)pyridine 1-oxide (Z45-1, 7.1 mg, retention time = 13.846 min). ES-API: [M+H]⁺=919.3. ¹HNMR (400MHz, DMSO-*d₆*): 8.50-8.46 (m, 2H), 8.36 (d, *J*=8.8Hz, 1H), 7.80 (s, 1H),7.76-7.72 (m, 1H), 7.55 (d, J=8.4Hz, 1H), 7.33 (d, J=1.2Hz, 1H), 5.58 (t, J=9.2Hz, 1H),5.40 (t, *J=5.6Hz,* 1H), 5.09(d, J=12.0Hz, 1H), 4.42-4.01 (m, 5H), 3.65-3.55 (m, 4H), 3.45 (t, J=5.6Hz, 2H), 3.30-3.29 (m, 1H), 3.18 (s, 3H), 3.15-3.11 (m, 1H), 2.95-2.86 (m, 3H), 2.78-2.66 (m, 1H), 2.45-2.32 (m, 3H), 2.10-2.04 (m, 1H), 1.90-1.70 (m, 4H), 1.60-1.48 (m, 6H), 1.25-1.12 (m, 3H), 1.08-1.05 (m, 6H), 0.99-0.90 (m, 6H), 0.52(s, 3H). The structure of the other isomeric compound was 5-(3-(4-cyano-4-(hydroxymethyl)piperidin-1-yl)prop-1-yn-1-yl)-(Sₐ)-3-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((*S*)-1-methoxyethyl)pyridine 1-oxide (Z45-2, 6.9 mg, retention time = 17.721 min). ES-API: [M+H]⁺=919.3. ¹HNMR (400MHz, DMSO-*d₆*): 8.50-8.38 (m, 3H), 7.79 (s, 1H), 7.72-7.70 (m, 1H), 7.46 (s, 1H), 7.33 (d, *J*=1.2Hz, 1H), 5.53 (t, *J*=9.2Hz, 1H), 5.40 (t, *J*=5.2Hz, 1H), 5.04(d, J=12.0Hz, 1H), 4.60-4.54(m, 1H), 4.30-4.01(m, 3H), 3.90-3.81(m, 1H), 3.70-3.54 (m, 4H), 3.45(d, J=5.2Hz, 2H), 3.31-3.30 (m, 1H), 3.18-3.12(m, 1H), 3.00-2.97(m, 4H), 2.88-2.84(m, 2H), 2.76-2.70(m, 1H), 2.49-2.40 (m, 3H), 2.22-2.10(m, 1H), 1.86-1.80(m, 4H), 1.54-1.49(m, 3H), 1.29(d, *J*=6.8Hz, 3H), 1.25-1.22(m, 3H), 1.17-1.45 (m, 3H), 1.10-1.05 (m, 6H), 0.96(s, 3H), 0.56(s, 3H).

### Example 1.42. Synthesis of Compounds Z67 and Z67-1

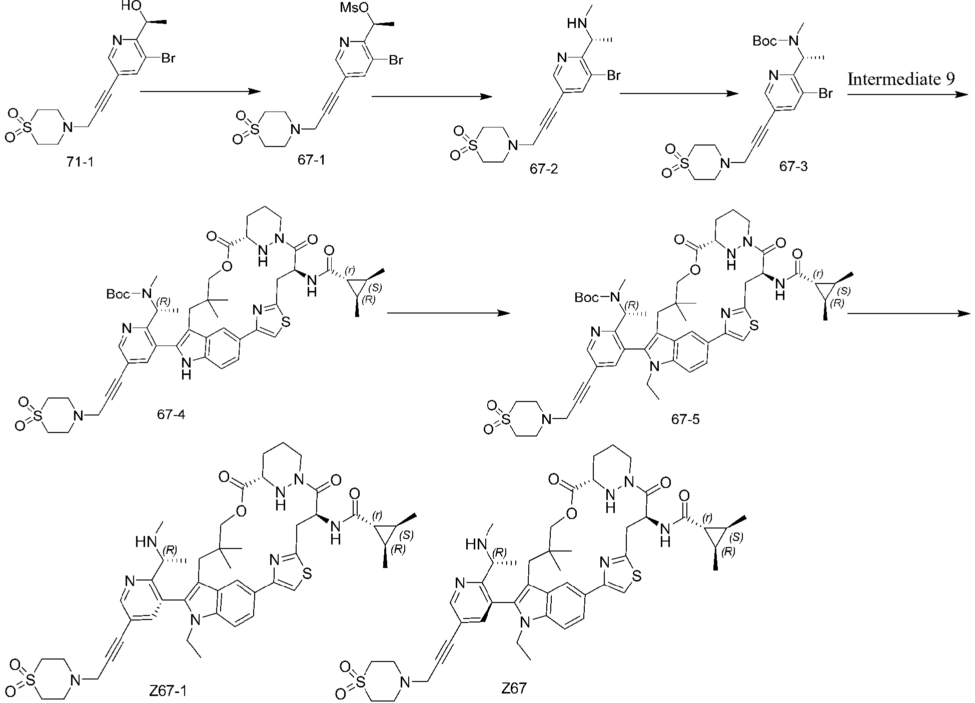

Step 1: Methanesulfonic anhydride (10.13 mg, 0.088 mmol) was slowly added to a solution of compound 71-1 (99 mg, 0.265 mmol) and triethylamine in dichloromethane, and the solution was stirred at room temperature for 70 min. The reaction mixture was diluted with dichloromethane (20 mL) and washed with a saturated aqueous sodium bicarbonate solution (2 × 15 mL) and brine (1 × 15 mL). The aqueous phases were combined and extracted with dichloromethane (1 × 15 mL). The organic phases were combined, dried, filtered, and concentrated under reduced pressure to give compound 67-1 (118 mg, yield: 99%), ES-API: [M+H]⁺ = 451.1. Step 2: Compound 67-1 (118 mg, 0.26 mmol) and methylamine (a 2.0 M solution in methanol, 20 mmol) were stirred at 50 °C overnight. The mixture was concentrated to remove the solvent to give crude compound 67-2 as an oil.

Step 3: Crude compound 67-2, di-tert-butyl dicarbonate (0.207 mL, 0.900 mmol), and triethylamine (0.125 mL, 0.900 mmol) were mixed in tetrahydrofuran (10 mL), and the mixture was stirred at 20 °C for 3 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 67-3 (100 mg, two-step yield: 77%), ES-API: [M+H]⁺ = 486.0.

Step 4: Intermediate 9 (90 mg, 0.130 mmol), compound 67-3 (63.48 mg, 0.130 mmol), Pd(dtbpf)Cl₂ (8.50 mg, 0.013 mmol), toluene (1 mL), water (1 mL), and potassium phosphate (83.10 mg, 0.391 mmol) were mixed in dioxane (3 mL) under a nitrogen atmosphere, and the mixture was stirred at 70 °C for 4 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-100% petroleum ether/ethyl acetate) to give compound 67-4 (94.96 mg, yield: 75%), ES-API: [M+H]⁺ = 969.3.

Step 5: Compound 67-4 (120 mg, 0.124 mmol), iodoethane (96.45 mg, 0.618 mmol), and cesium carbonate (201.60 mg, 0.618 mmol) were mixed in N,N-dimethylformamide (5 mL), and the mixture was stirred at 60 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-10% dichloromethane/methanol) to give compound 67-5 (120 mg, yield: 97.19%), ES-API: [M+H]⁺ = 997.3.

Step 6: Compound 67-5 (120 mg, 0.120 mmol) and trifluoroacetic acid (3 mL) were mixed in dichloromethane (6 mL), and the mixture was stirred at 20 °C for 1 h. After the reaction was completed, 30 mL of ethyl acetate was poured into the mixture, and the resulting mixture was washed sequentially with 15 mL of a sodium bicarbonate solution and 15 mL of saturated brine. The organic phase was dried over anhydrous sodium sulfate, filtered, and concentrated, and the residue was purified by prep-HPLC (ammonium bicarbonate method 1) to give 2 isomeric compounds. The structure of one of the isomeric compounds was (1r,2R,3S)-N-((6³S,4S,Z)-(S*ₐ*)-1*²*-(5-(3-(1,1-dioxidothiomorpholino)prop-1-yn-1-yl)-2-((R)-1-(methylamino)ethyl)pyridin-3-yl)-1¹-ethyl-10,10-dimethyl-5,7-dioxo-6*¹*,6*²*,6*³*,6*⁴*,6*⁵*,6*⁶*-hexahydro-1*¹*H-8-oxa-2(4,2)-thiazola-1(5,3)-indola-6(1,3)-pyridazinacycloundecaphane-4-yl)-2,3-dimethylcyclopropane-1-carboxamide (Z67-1, 14 mg, yield: 12.9%, purity: 98%, retention time: 5.428 min). ES-API[M+H]⁺=897.3. ¹HNMR(400 MHz, DMSO-*d₆*) 8.84 (1 H, d, *J* 1.9), 8.51 (1 H, s), 8.41 (1 H, d,19.1), 7.95 (1 H, *d, J1.9),* 7.82 (1 H, s), 7.74 (1 H, d, J7.5), 7.54 (1 H, d, *J* 8.7)*,* 5.54 (1 H, s), 5.05 (1 H, d, *J* 12.2), 4.29-4.15 (2 H, m), 4.13-4.01 (1 H, m), 3.76 (2 H, s), 3.74 - 3.64 (2 H, m), 3.61-3.53 (1 H, m), 3.28 (1 H, s), 3.21 - 3.13 (6 H, m), 3.09-2.98 (5 H, m), 2.77 (1 H, t, *J* 10.3), 2.28-2.06 (2 H, m), 2.02 - 1.92 (4 H, m), 1.80 (2 H, s), 1.6-1.45 (1 H, m), 1.32-1.15 (9 H, m), 1.12-1.01 (6 H, m), 0.94 (3 H, s), 0.55 (3 H, s). The structure of the other isomeric compound was: (1r,2R,3S)-N-((6³S,4S,Z)-(R*ₐ*)-1²-(5-(3-(1,1-dioxidothiomorpholino)prop-1-yn-1-yl)-2-((R)-1-(methylamino)ethyl)pyridin-3-yl)-1-ethyl-10,10-dimethyl-5,7-dioxo-6¹,6²,6³,6⁴,6⁵,6⁶-hexahydro-1*¹*H-8-oxa-2(4,2)-thiazola-1(5,3)-indola-6(1,3)-pyridazinacycloundecaphane-4-yl)-2,3-dimethylcyclopropane-1-carboxamide (Z67, 9 mg, yield: 8.3%, purity: 98%, retention time: 5.596 min). ES-API[M+H]⁺=897.3. ¹HNMR (400 MHz, DMSO-*d₆*) 8.80 (1 H, d, *J* 1.9), 8.52 (1 H, s), 8.30 (1 H, d, *J* 8.7), 7.86 (1 H, d, J 1.8), 7.78 (1 H, s), 7.72 (1 H, d, *J* 8.7), 7.54 (1 H, d, *J* 8.6), 5.80 (1 H, d, *J* 6.0), 5.21 (1 H, d, *J* 12.1), 4.34 - 4.11 (2 H, m), 3.95 (1 H, s), 3.75 (2 H, s), 3.66 (3 H, *d, J* 14.4), 3.51 (1 H, d, *J* 6.3), 3.30-3.11 (7 H, m), 3.09-2.98 (4 H, m), 2.82 - 2.65 (3 H, m), 2.19 (3 H, s), 1.95-1.85 (1 H, m), 1.80-1.71 (1 H, m), 1.61 - 1.42 (2 H, m), 1.23 (3 H, d, *J* 6.2), 1.14 - 0.93 (12 H, m), 0.71 (6 H, d*, J* 7.7)*.*

### Example 1.43. Synthesis of Compound Z73

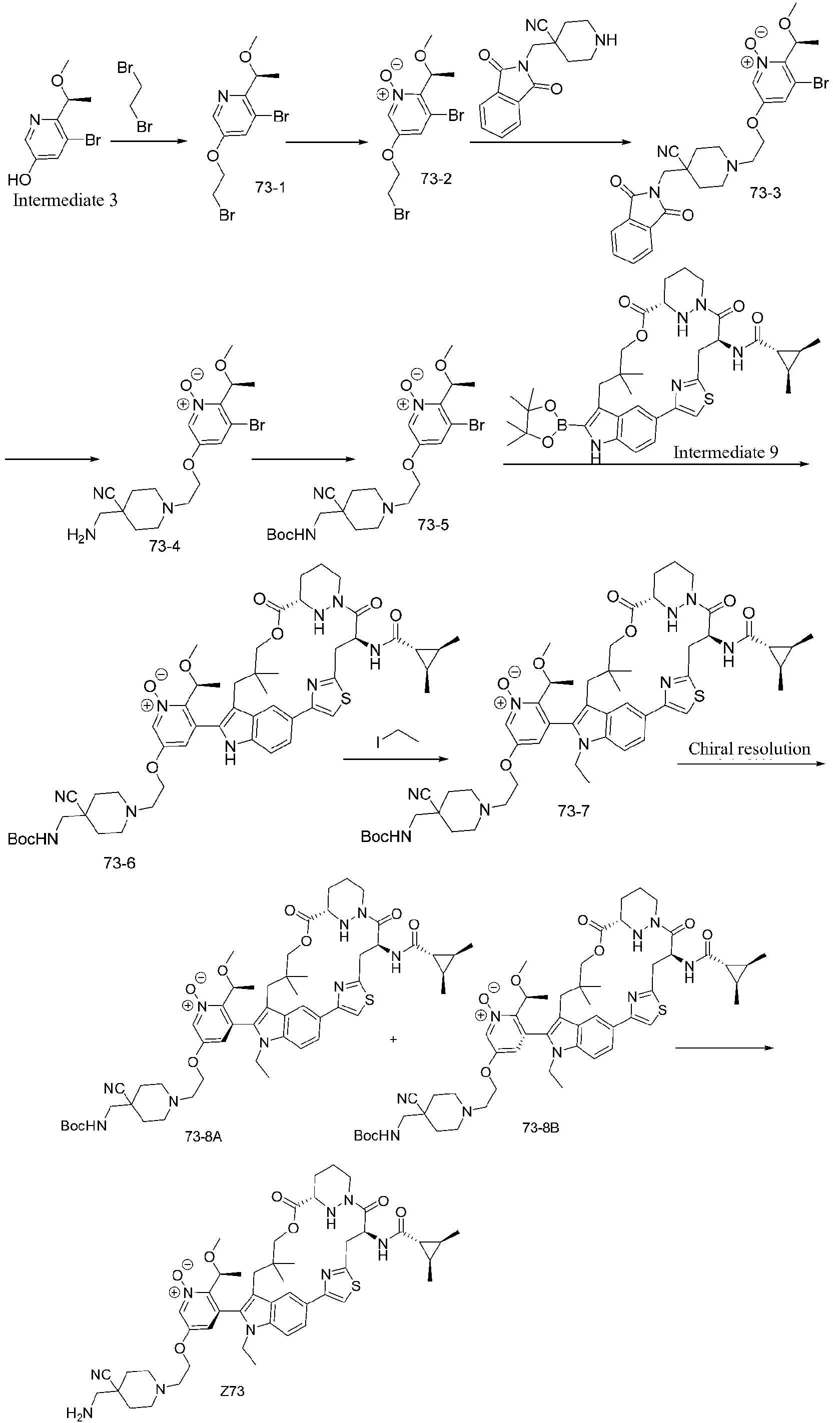

Step 1: Intermediate 3 (2 g, 8.62 mmol) and potassium carbonate (3.57 g, 25.85 mmol) were dissolved in acetonitrile (40 mL), and 1,2-dibromoethane (9.71 g, 51.71 mmol) was added at room temperature. The reaction system was stirred at 80 °C for 8 h. The reaction mixture was cooled to room temperature and filtered, and the filtrate was concentrated under reduced pressure. The crude product was purified by a flash silica gel column (tetrahydrofuran/petroleum ether: 0-40%) to give desired product compound 73-1 (2.1 g, yield: 71.9%) as a colorless liquid. ES-API: [M+H]⁺=340.0.

Step 2: Compound 73-1 (2.1 g, 6.19 mmol) was dissolved in dichloromethane (50 mL), and 85% m-chloroperoxybenzoic acid (1.89 g, 9.291 mmol) was added under an ice bath. The reaction system was stirred at room temperature for 18 h. Dichloromethane (50 mL) was added to the reaction mixture. The mixture was washed sequentially with a saturated sodium bicarbonate solution (50 mL × 2) and saturated brine (50 mL). The organic phase was dried over anhydrous sodium sulfate and filtered, and the filtrate was concentrated under reduced pressure. The crude product was purified by a flash silica gel column (tetrahydrofuran/petroleum ether: 0-50%) to give compound 73-2 (2.1 g, yield: 95.5%) as a pale yellow solid. ¹H NMR (400 MHz, CDCl3) δ 8.04 (d, J = 2.0 Hz, 1H), 7.18 (d, J = 2.0 Hz, 1H), 5.52 (q, J = 6.8 Hz, 1H), 4.30 (t, J = 6.0 Hz, 2H), 3.63 (t, J = 6.0 Hz, 2H), 3.25 (s, 3H), 1.57 (d, J = 6.8 Hz, 3H).ES-API:[M+H]⁺= 355.9.

Step 3: Compound 73-2 (550 mg, 1.55 mmol), N,N-diisopropylethylamine (601 mg, 4.65 mmol), and sodium iodide (186 mg, 1.24 mmol) were dissolved in acetonitrile (25 mL), and 4-[(1,3-dioxo-2,3-dihydro-1H-isoindol-2-yl)methyl]piperidine-4-carbonitrile (compound 52-2, 459 mg, 1.70 mmol) was added at room temperature. The reaction system was stirred at 80 °C for 24 h. The reaction mixture was cooled to room temperature. Ethyl acetate (80 mL) was added, and the mixture was washed sequentially with water (30 mL) and saturated brine (30 mL). The organic phase was dried over anhydrous sodium sulfate and filtered, and the filtrate was concentrated under reduced pressure. The crude product was purified by a flash silica gel column (methanol/dichloromethane: 0-3%) to give compound 73-3 (650 mg, yield: 77.2%) as a white solid. ES-API:[M+H]⁺= 543.1,545.1.

Step 4: Compound 73-3 (600 mg, 1.10 mmol) was added to a 30-33 wt.% methylamine/methanol solution (20 mL) at room temperature. The reaction system was stirred at 70 °C for 2 h. The reaction mixture was cooled to room temperature and concentrated under reduced pressure to give compound 73-4 (456 mg, yield: 99.9%) as a white solid. ES-API: [M+H]⁺= 413.1,415.1.

Step 5: Compound 73-4 (456 mg, 1.103 mmol) was dissolved in dichloromethane (20 mL), and triethylamine (335 mg, 3.31 mmol) and di-tert-butyl dicarbonate (482 mg, 2.21 mmol) were added at room temperature. The reaction system was stirred at room temperature for 17 h. Dichloromethane (30 mL) was added to the reaction mixture, and the mixture was washed sequentially with water (15 mL × 2) and saturated brine (15 mL). The organic phase was dried over anhydrous sodium sulfate and filtered, and the filtrate was concentrated under reduced pressure. The crude product was purified by a flash silica gel column (methanol/dichloromethane: 0-4%) to give compound 73-5 (500 mg, yield: 88.3%) as an off-white solid. ES-API:[M+H]⁺= 513.2, 515.2.

Step 6: Intermediate 9 (200 mg, 0.29 mmol) and compound 73-5 (194 mg, 0.38 mmol) were dissolved in dioxane (2 mL), toluene (6 mL), and water (2 mL) under a nitrogen atmosphere, and [1,1'-bis(di-tert-butylphosphino)ferrocene]dichloropalladium(II) (38 mg, 0.058 mmol) and potassium phosphate (123 mg, 0.58 mmol) were added. The reaction system was stirred at 75 °C for 3 h. Ethyl acetate (100 mL) was added to the reaction mixture, and the mixture was washed with saturated brine (30 mL). The organic phase was dried over anhydrous sodium sulfate and filtered, and the filtrate was concentrated under reduced pressure. The crude product was purified by a flash silica gel column (methanol/dichloromethane: 0-10%) to give compound 73-6 (250 mg, yield: 86.5%) as a yellow solid. ES-API:[M+H]⁺= 996.3.

Step 7: Compound 73-6 (250 mg, 0.25 mmol) was dissolved in N,N-dimethylformamide (8 mL), and cesium carbonate (327 mg, 1.00 mmol) and iodoethane (157 mg, 1.00 mmol) were added at room temperature. The reaction system was stirred at room temperature for another 18 h. Ethyl acetate (50 mL) was added to the reaction mixture, and the mixture was washed with saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-8%) to give compound 73-7 (240 mg, yield: 93.4%) as a pale yellow solid. ES-API:[M+H]⁺= 1024.4.

Step 8: Compound 73-7 (240 mg, 0.23 mmol) was subjected to preparative chiral resolution (separation column: IB, 250 mm × 4.6 mm × 5 µm, mobile phase: acetonitrile:isopropanol:diethylamine = 30:70:2, flow rate: 1 mL/min, column temperature: 30 °C) to give 2 isomeric compounds. The structure of one of the isomeric compounds was compound 73-8A (125 mg, retention time: 4.862 min, yield: 52.1%) as a white solid. ES-API: [M+H]⁺=1024.4. The structure of the other isomeric compound was compound 73-8B (70 mg, retention time: 10.913 min, yield: 29.2%) as a white solid. ES-API: [M+H]⁺=1024.4.

Step 9: Compound 73-8A (10 mg, 0.010 mmol) was dissolved in dichloromethane (1 mL), and trifluoroacetic acid (0.3 mL) was added at 0 °C. The reaction system was stirred at room temperature for 1 h. The reaction mixture was concentrated, and a saturated sodium bicarbonate solution (2 mL) was added. The mixture was extracted with dichloromethane (10 mL × 2). The organic phases were mixed, dried over anhydrous sodium sulfate, and filtered, and the filtrate was concentrated under reduced pressure. The crude product was subjected to prep-HPLC (ammonium bicarbonate method 1) to give the desired product 5-(2-(4-(aminomethyl)-4-cyanopiperidin-1-yl)ethoxy)-(Rₐ)-3-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((*S*)-1-methoxyethyl)pyridine 1-oxide (Z73, 8 mg, yield: 88.7%) as a white solid. ES-API: [M+H]⁺= 924.3. ¹H NMR (400 MHz, DMSO-*d₆*) δ 8.49 (s, 1H), 8.38 (d, *J* = 9.2 Hz, 1H), 8.23 (d, *J* = 2.4 Hz, 1H), 7.81 (s, 1H), 7.75 (dd, *J =* 8.8, 1.1 Hz, 1H), 7.57 (d, *J =* 8.8 Hz, 1H), 7.01 (d, *J* = 2.0 Hz, 1H), 5.57 (t, *J =* 9.2 Hz, 1H), 5.08 *(d, J=* 12.0 Hz, 1H), 4.37 - 4.02 (m, 7H), 3.64 - 3.53 (m, 2H), 3.31 - 3.25 (m, 3H), 3.20 - 3.09 (m, 5H), 3.00 - 2.87 (m, 3H), 2.80 - 2.69 (m, 3H), 2.66 (s, 2H), 2.57 (d, *J* = 14.0 Hz, 1H), 2.20 (t, *J =* 12.0 Hz, 2H), 2.07 (d, *J* = 10.4 Hz, 1H), 1.85 - 1.70 (m, 4H), 1.56 - 1.41 (m, 6H), 1.25 - 1.13 (m, 3H), 1.09 - 1.02 (m, 6H), 0.99 - 0.89 (m, 6H), 0.52 (s, 3H).

### Example 1.44. Synthesis of Compound Z73-1

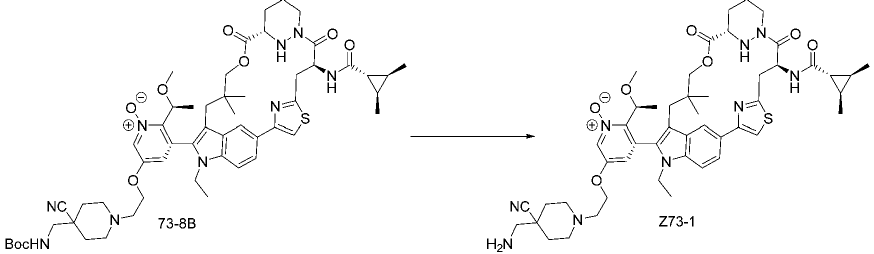

Step 1: Compound 73-8B (25 mg, 0.024 mmol) was dissolved in dichloromethane (1.5 mL), and trifluoroacetic acid (0.5 mL) was added at 0 °C. The reaction system was stirred at room temperature for 1 h. The reaction mixture was concentrated, and a saturated sodium bicarbonate solution (5 mL) was added. The mixture was extracted with dichloromethane (10 mL × 2). The organic phases were mixed, dried over anhydrous sodium sulfate, and filtered, and the filtrate was concentrated under reduced pressure. The crude product was purified by prep-HPLC (ammonium bicarbonate method 1) to give the desired product 5-(2-(4-(aminomethyl)-4-cyanopiperidin-1-yl)ethoxy)-(Sₐ)-3-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-2-((*S*)-1-methoxyethyl)pyridine 1-oxide (Z73-1, 18 mg, yield: 79.8%) as a white solid. ES-API: [M+H]⁺= 924.3. ¹H NMR (400 MHz, DMSO-*d₆*) δ 8.50 (s, 1H), 8.41 (d, *J =* 9.2 Hz, 1H), 8.26 (d*, J =* 2.0 Hz, 1H), 7.80 (s, 1H), 7.72 (dd*, J =* 8.8, 1.2 Hz, 1H), 7.52 (d*, J =* 8.8 Hz, 1H), 7.17 (d*, J = 2.0* Hz, 1H), 5.54 (t, *J =* 9.2 Hz, 1H), 5.04 (d, *J* = 12.0 Hz, 1H), 4.53 - 4.41 (m, 1H), 4.30 - 4.12 (m, 4H), 4.12 - 3.98 (m, 1H), 3.95 - 3.81 (m, 1H), 3.71 (d, *J* = 10.8 Hz, 1H), 3.57 (d, *J* = 10.8 Hz, 1H), 3.35 - 3.11 (m, 4H), 3.05 - 2.96 (m, 4H), 2.93 (d, *J =* 11.6 Hz, 2H), 2.82 - 2.64 (m, 5H), 2.30 - 2.09 (m, 4H), 1.88 - 1.71 (m, 4H), 1.60 - 1.40 (m, 3H), 1.30 *(d, J=* 6.4 Hz, 3H), 1.27 - 1.11 (m, 8H), 1.11 - 1.00 (m, 6H), 0.97 (s, 3H), 0.59 (s, 3H).

### Example 1.45. Synthesis of Compound Z65

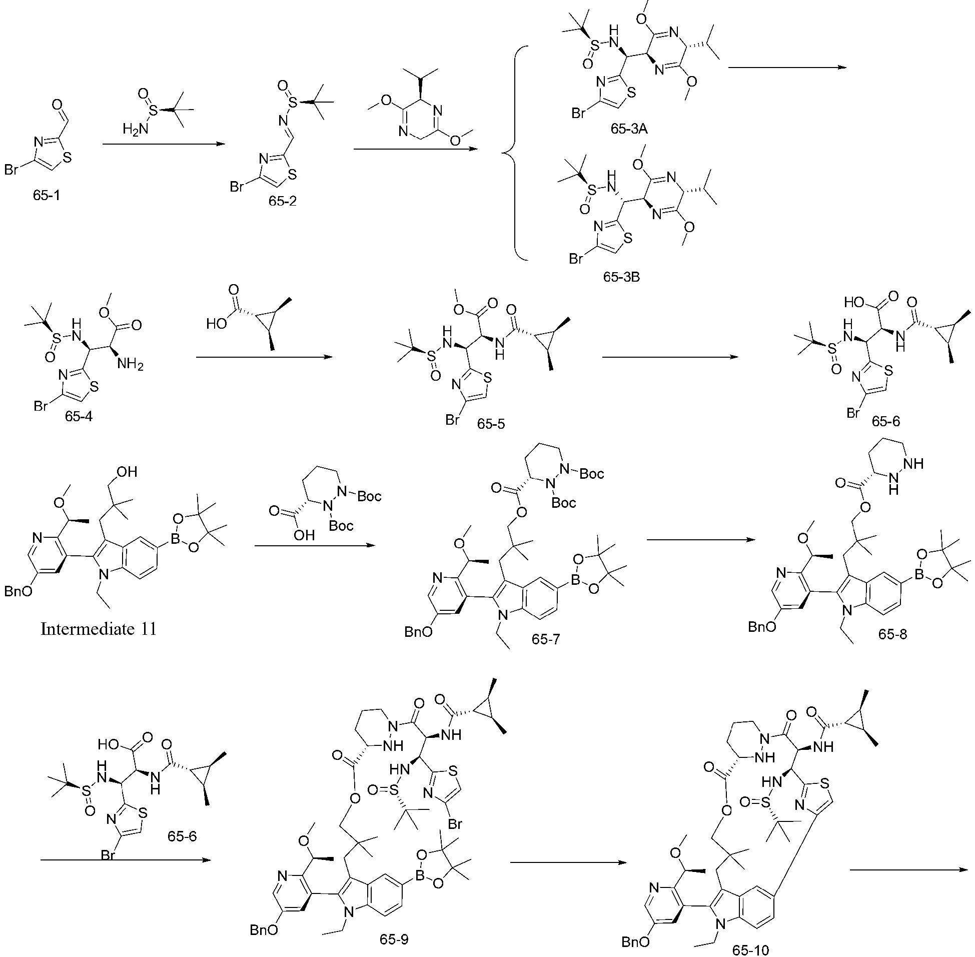

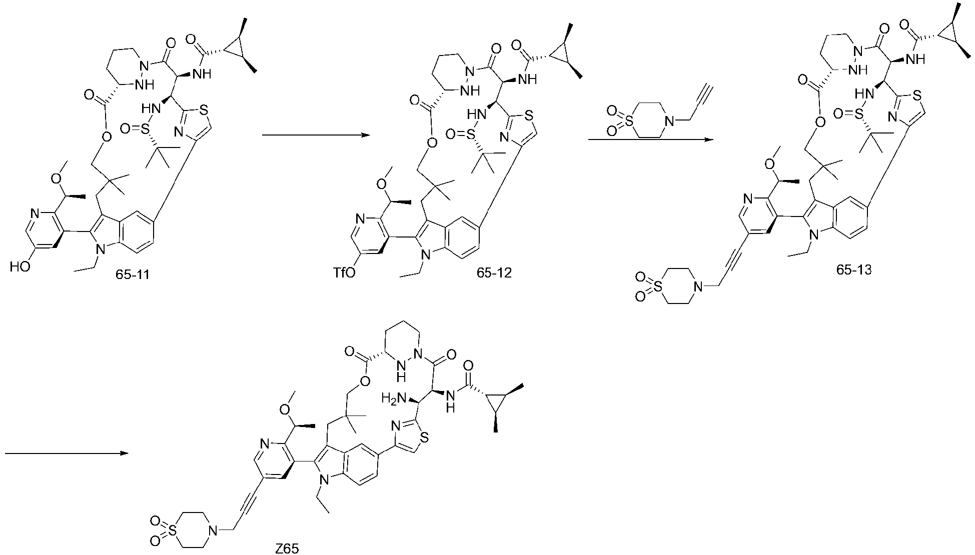

Step 1: Dichloromethane (150 mL) and cesium carbonate (33.93 g, 104.150 mmol) were added to a mixture of compound 65-1 (10 g, 52.075 mmol) and (S)-2-methylpropane-2-sulfinamide (6.31 g, 52.075 mmol). The mixture was stirred at room temperature for 2 h and then filtered, and the filter cake was washed with dichloromethane (20 mL × 3). The filtrate was concentrated under reduced pressure, and the residue was purified by silica gel column chromatography (ethyl acetate/petroleum ether = 1/1) to give compound 65-2 (14 g, 47.424 mmol, yield: 91.07%), ES-API: [M+H]⁺ = 294.9.

Step 2: (2R)-3,6-Dimethoxy-2-(propan-2-yl)-2,5-dihydropyrazine (9.44 g, 51.235 mmol) was dissolved in dry tetrahydrofuran (100 mL). The mixture was purged three times with nitrogen, and n-butyllithium (20.494 mL, 51.235 mmol, a 2.5 M solution in n-hexane) was slowly added dropwise under a dry ice bath at -78 °C. The mixture was stirred at -78 °C for 30 min, and then a solution (50 mL) of compound 65-2 (13.75 g, 46.577 mmol) in tetrahydrofuran was added dropwise. The mixture was stirred at -78 °C for 2 h, then warmed to 0 °C and quenched with a saturated ammonium chloride solution (50 mL). The aqueous layer was extracted with ethyl acetate (200 mL × 3), and the organic layers were combined and concentrated under reduced pressure. The residue was purified by silica gel column chromatography (petroleum ether/ethyl acetate = 2/1) to give 2 isomeric compounds. The structure of one of the isomeric compounds was compound 65-3A (10.4 g, 19.522 mmol, yield: 41.91%, acid-method HPLC 15 min, retention time = 8.908 min); the structure of the other isomeric compound was compound 65-3B (4.0 g, 8.343 mmol, yield: 17.91%, acid-method HPLC 15 min, retention time = 9.069 min). ES-API: [M+H]⁺=479.1.

Step 3: Compound 65-3A (10 g, 20.857 mmol) was dissolved in tetrahydrofuran (340 mL) and acetonitrile (200 mL), and 0.2 M hydrochloric acid (260 mL) was added under an ice-water bath. The mixture was stirred at room temperature for 18 h. A saturated aqueous sodium bicarbonate solution was slowly added until the pH was 8-9, and the mixture was extracted with ethyl acetate (500 mL × 3), washed with saturated brine (500 mL × 1), dried over anhydrous sodium sulfate, and concentrated to give product compound 65-4 (6 g, crude product). ES-API: [M+H]⁺=384.0.

Step 4: Compound 65-4 (2 g, crude product) and (1r,2R,3S)-2,3-dimethylcyclopropane-1-carboxylic acid (0.59 g, 5.204 mmol) were dissolved in dichloromethane (10 mL), and DIPEA (2.69 g, 20.817 mmol) and HATU (3.96 g, 10.408 mmol) were sequentially added. The mixture was reacted at room temperature for 1 h. The mixture was extracted with ethyl acetate (30 mL × 2), washed with saturated brine (50 mL × 1), dried over anhydrous sodium sulfate, and concentrated, and the residue was purified by a silica gel column (petroleum ether/ethyl acetate = 1/1) to give compound 65-5 (1.1 g, 2.290 mmol, two-step yield: 33%). ES-API: [M+H]⁺=480.1.

Step 5: A solution of compound 65-5 (1.1 g, 2.290 mmol) in tetrahydrofuran (10 mL) was cooled to 0 °C, and an aqueous solution (5 mL) of lithium hydroxide (57.7 mg, 2.404 mmol) was slowly added. The mixture was reacted for 5 min while maintaining the temperature at 0 °C. After the reaction was completed, 1 M diluted hydrochloric acid was added until the pH was 5-6, and the mixture was extracted with ethyl acetate (20 mL × 3), washed with saturated brine (50 mL × 1), dried over anhydrous sodium sulfate, and concentrated to give compound 65-6 (1 g, 2.15 mmol, yield: 93.9%). ES-API: [M+H]⁺=466.0.

Step 6: Intermediate 11 (1.2 g, 2.005 mmol) and (S)-1,2-bis(tert-butoxycarbonyl)hexahydropyridazine-3-carboxylic acid (0.73 g, 2.205 mmol) were dissolved in dichloromethane (10 mL), and DMAP (0.49 g, 4.010 mmol) and EDCI (0.77 g, 4.009 mmol) were sequentially added. The reaction system was stirred at 10 °C for 2 h. The mixture was extracted with ethyl acetate (20 mL × 3), washed with saturated brine (50 mL × 1), dried over anhydrous sodium sulfate, and concentrated, and the residue was purified by column chromatography (petroleum ether/ethyl acetate = 1/1) to give compound 65-7 (1.10 g, 1.203 mmol, yield: 60%). ES-API: [M+H]⁺=911.5.

Step 7: 4 M hydrochloric acid/dioxane (10 mL, 40.000 mmol) was added to compound 65-7 (300 mg, 0.329 mmol). The mixture was stirred at room temperature for 3 h. After the reaction was completed, the mixture was concentrated to give compound 65-8 (250 mg, crude product), ES-API: [M+H]⁺ = 711.4.

Step 8: Compound 65-8 (250 mg, crude product) and compound 65-6 (223.19 mg, 0.479 mmol) were dissolved in dichloromethane (10 mL), and HOBt (19 mg, 0.143 mmol), 4-methylmorpholine (97 mg, 0.958 mmol), and EDCI (0.77 g, 4.009 mmol) were sequentially added. The mixture was stirred at room temperature for 2 h. After the reaction was completed, the mixture was extracted with ethyl acetate (20 mL × 3), washed with saturated brine (50 mL × 1), dried over anhydrous sodium sulfate, and concentrated, and the residue was purified by column chromatography (petroleum ether/ethyl acetate = 1/5) to give compound 65-9 (250 mg, 0.216 mmol, yield: 65.6%). ES-API: [M+H]⁺=1158.5.

Step 9: Pd(dtbpf)Cl₂ (12.59 mg, 0.019 mmol) and potassium phosphate (24.83 mg, 0.117 mmol) were added to a mixed solution of compound 65-9 (200 mg, 0.173 mmol) in 1,4-dioxane (6 mL), toluene (2 mL), and water (2 mL), and the mixture was stirred at 80 °C for 1 h. The mixture was extracted with ethyl acetate (20 mL × 3), washed with saturated brine (50 mL × 1), dried over anhydrous sodium sulfate, and concentrated, and the residue was purified by column chromatography (petroleum ether/ethyl acetate = 1/100) to give compound 65-10 (60 mg, 0.063 mmol, yield: 36.52%). ES-API: [M+H]⁺=952.4.

Step 10: Compound 65-10 (130 mg, 0.137 mmol) was dissolved in methanol (10 mL), and 10% Pd/C (100 mg) was added. The mixture was reacted for 18 h under a hydrogen atmosphere. After the reaction was completed, the mixture was filtered and concentrated to give compound 65-11 (80 mg, 0.093 mmol, yield: 67.97%), ES-API: [M+H]⁺ = 862.4.

Step 11: N-Phenyl-bis(trifluoromethanesulfonimide) (39.87 mg, 0.112 mmol) and DIPEA (36.06 mg, 0.279 mmol) were added to a solution of compound 65-11 (80 mg, 0.093 mmol) in dichloromethane (10 mL). The mixture was reacted at room temperature for 3 h, extracted with dichloromethane (10 mL × 3), washed with saturated brine (20 mL × 1), dried over anhydrous sodium sulfate, and concentrated, and the residue was purified by column chromatography (petroleum ether/ethyl acetate = 1/100) to give compound 65-12 (83 mg, 0.084 mmol, yield: 90%). ES-API: [M+H]⁺=994.3.

Step 12: Pd(PPh₃),Cl, (12.63 mg, 0.018 mmol), CuI (20.00 mg, 0.105 mmol), and DIPEA (18.06 mg, 0.140 mmol) were sequentially added to a solution of compound 65-12 (35 mg, 0.035 mmol) and 4-(prop-2-yn-1-yl)thiomorpholine-1,1-dioxide (30.32 mg, 0.175 mmol) in N,N-dimethylformamide (1.5 mL), and the mixture was heated to 85 °C and reacted for 2 h under a nitrogen atmosphere. After the reaction was completed, water (20 mL) and ethyl acetate (30 mL) were added. The organic layer was separated, further washed with a saturated sodium chloride solution (20 mL × 3), dried over anhydrous sodium sulfate, filtered, and concentrated. The residue was purified by a silica gel column (MeOH/DCM = 1/20) to give compound 65-13 (23 mg, 0.023 mmol, yield: 64.21%), ES-API: [M+H]⁺ = 1017.4.

Step 13: Compound 65-13 (23 mg, 0.023 mmol) was dissolved in methanol (3 mL), and 4 M hydrochloric acid/dioxane (3 mL) was added. The mixture was stirred at room temperature for 3 h. After the reaction was completed, the mixture was concentrated. The residue was purified and separated by prep-HPLC (ammonium bicarbonate method 1) to give (1r,2R,3S)-N-((6³S,3S,4S,Z)-3-amino-(Rₐ)-1²-(5-(3-(1,1-dioxidothiomorpholino)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-11H-8-oxa-2(4,2)-thiazola-1(5,3)-indola-6(1,3)-pyridazinacycloundecaphane-4-yl)-2,3-dimethylcyclopropane-1-carboxamide (Z65, 12.9 mg, 0.012 mmol, yield: 51.87%), ES-API: [M+H]⁺= 913.4.

### Example 1.46. Synthesis of Compound Z48

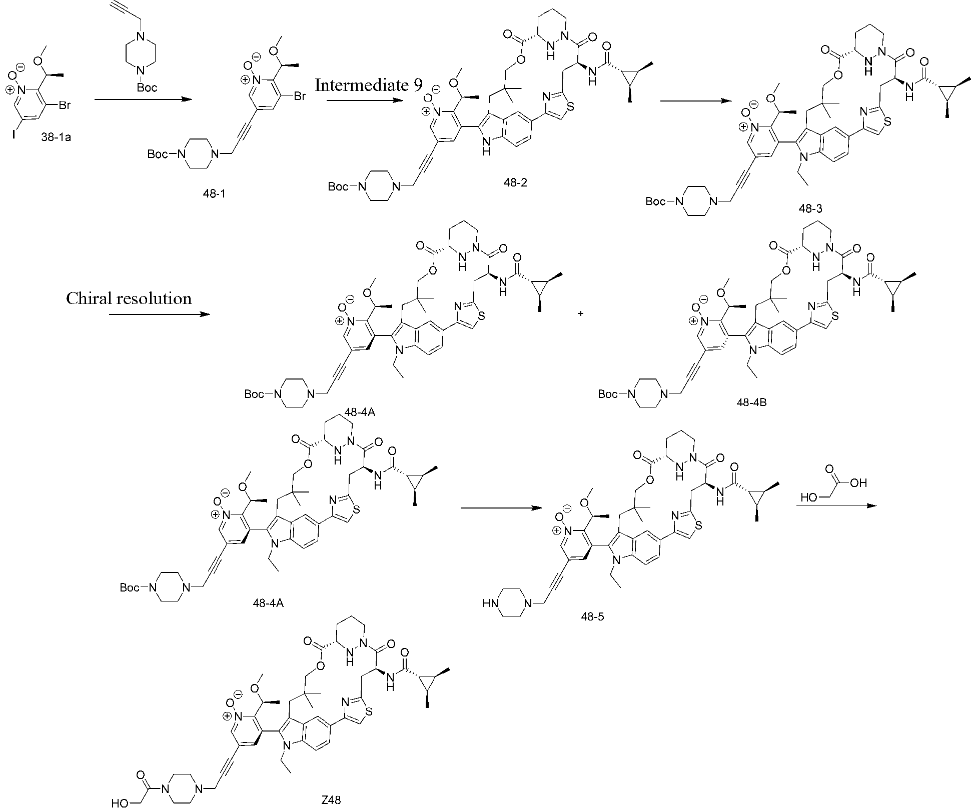

Step 1: Compound 38-1a (500 mg, 1.397 mmol) and tert-butyl 4-(prop-2-yn-1-yl)piperazine-1-carboxylate (380 mg, 2.132 mmol) were dissolved in acetonitrile (15 mL). Bis(triphenylphosphine)palladium(II) dichloride (49.02 mg, 0.070 mmol), copper(I) iodide (39.90 mg, 0.210 mmol), and N,N'-diisopropylethylamine (0.98 mL, 5.611 mmol) were added to the solution described above. After the addition was completed, the reaction system was stirred at room temperature for 4 h under a nitrogen atmosphere. After the reaction was completed, water (30 mL) was added to the reaction mixture, and the mixture was extracted with ethyl acetate (30 mL × 2). The organic phase was washed with saturated brine (30 mL × 2), dried, and concentrated to give a crude product. The crude product was purified by a flash silica gel column (methanol/dichloromethane: 0%-3%) to give compound 48-1 (500 mg, 1.100 mmol, yield: 78.77%) as a light yellow solid. ES-API: [M+H]⁺= 454.1, 456.1.

Step 2: Intermediate 9 (120 mg, 0.174 mmol) was dissolved in dioxane (2 mL), toluene (3 mL), and water (1 mL). Compound 48-1 (118.59 mg, 0.261 mmol), [1,1'-bis(di-tert-butylphosphino)ferrocene]dichloropalladium(II) (11.33 mg, 0.017 mmol), and potassium phosphate (110.80 mg, 0.522 mmol) were added to the solution described above at room temperature. After the addition was completed, the reaction system was stirred at 70 °C for 3 h under an argon atmosphere. After the reaction was completed, water (30 mL) was added to the reaction mixture, and the mixture was extracted with ethyl acetate (30 mL × 2). The organic phase was washed with saturated brine (30 mL × 3), dried, and concentrated to give a crude product. The crude product was purified by a flash silica gel column (methanol/dichloromethane: 0%-5%) to give compound 48-2 (140 mg, 0.149 mmol, yield: 85.86%) as a light yellow solid. ES-API: [M+H]⁺=937.2.

Step 3: Compound 48-2 (110 mg, 0.117 mmol) was dissolved in N,N-dimethylformamide (5 mL). Iodoethane (0.038 mL, 0.469 mmol) and cesium carbonate (153 mg, 0.469 mmol) were added to the solution described above. The reaction system was stirred at room temperature for 48 h. After the reaction was completed, water (15 mL) was added to the solution described above, and the mixture was extracted with ethyl acetate (20 mL × 2). The organic phase was washed with saturated brine (20 mL × 3), dried, and concentrated to give a crude product. The crude product was purified by a flash silica gel column (methanol/dichloromethane: 0%-3%) to give compound 48-3 (80 mg, 0.084 mmol, yield: 71.80%) as a light yellow solid. ES-API: [M+H]⁺=965.3. Step 4: Compound 48-3 (80 mg, 0.084 mmol) was subjected to chiral resolution (column type: IG 250 mm, 10 mm, 5 µm; mobile phase system: [A: acetonitrile; B: (isopropanol:methanol = 1:1); C: diethylamine]; flow rate: 1 mL/min; A/B/C = 70/30/2; column temperature: 30 °C) to give 2 isomeric compounds. The structure of one of the isomeric compounds was compound 48-4B (35 mg, 0.036 mmol, yield: 43.75%, retention time: 5.637 min) as a white solid, ES-API: [M+H]+ = 965.3. The structure of the other isomeric compound was compound 48-4A (20 mg, 0.023 mmol, yield: 27.5%, retention time: 7.635) as a white solid, ES-API: [M+H]⁺ = 965.3.

Step 5: Compound 48-4A (20 mg, 0.023 mmol) was dissolved in methanol (0.5 mL), and a solution (4 M, 0.5 mL) of hydrogen chloride in dioxane was added to the solution described above. After the addition was completed, the reaction system was stirred at room temperature for 1 h. The reaction mixture was concentrated to give compound 48-5 (crude product, 20 mg) as a light yellow solid, ES-API: [M+H]⁺ = 865.3.

Step 6: Compound 48-5 (crude product, 20 mg) was dissolved in dichloromethane (2 mL). 2-Hydroxyacetic acid (1.76 mg, 0.023 mmol), O-(7-azabenzotriazol-1-yl)-N,N,N',N'-tetramethyluronium hexafluorophosphate (17.58 mg, 0.046 mmol), and N,N'-diisopropylethylamine (8.96 mg, 0.069 mmol) were added to the solution described above. After the addition was completed, the reaction system was stirred at room temperature for 24 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give (Rₐ)-3-((6³S,4S,Z)-4-((1r,2R,3S)-2,3-dimethylcyclopropane-1-carboxamido)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-1²-yl)-5-(3-(4-(2-hydroxyacetyl)piperazin-1-yl)prop-1-yn-1-yl)-2-((S)-1-methoxyethyl)pyridine 1-oxide (Z48, 9 mg, 0.010 mmol, two-step yield: 43.5%) as a white solid. ES-API: [M +H]⁺=923.3.

### Example 1.47. Synthesis of Compounds Z66 and Z66-1

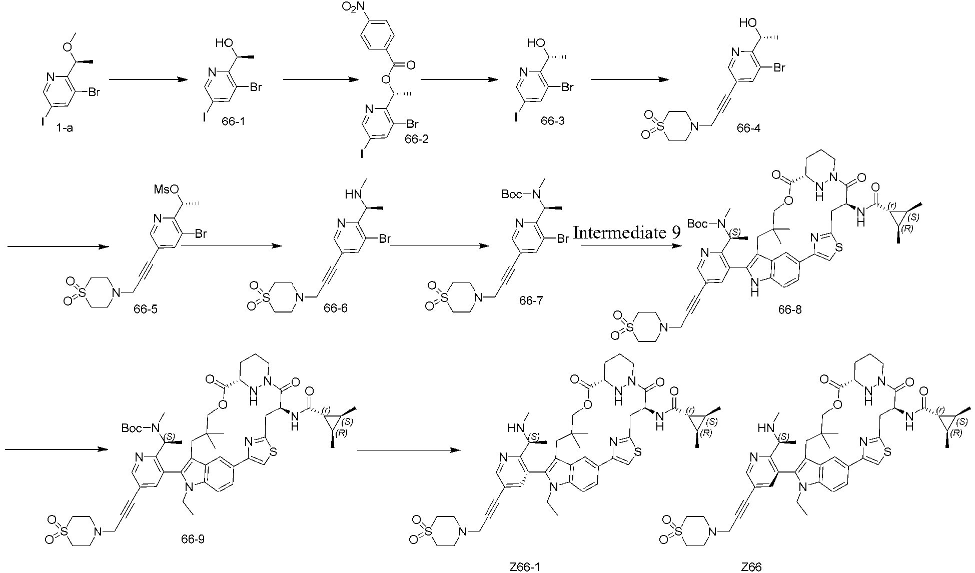

Step 1: Compound 1-a (1.70 g, 4.971 mmol) was dissolved in dichloromethane (20 mL), and boron tribromide (7.457 mL, 14.914 mmol) was slowly added at 0 °C. The mixture was stirred at room temperature for 4 h. After the reaction was completed, the mixture was poured into an ice-cold sodium bicarbonate solution. 50 mL of dichloromethane was poured into the mixture, and the resulting mixture was washed sequentially with 50 mL of water and 50 mL of saturated brine. The organic phase was dried over anhydrous sodium sulfate, filtered, and concentrated, and the residue was purified by a flash silica gel column (0-50% petroleum ether/ethyl acetate) to give compound 66-1 (1.3 g, yield: 79.74%), ES-API: [M+H]⁺ = 328.1.

Step 2: Compound 66-1 (100 mg, 0.305 mmol), p-nitrobenzoic acid (61.15 mg, 0.366 mmol), and triphenylphosphine (199.95 mg, 0.762 mmol) were dissolved in tetrahydrofuran (5 mL) under a nitrogen atmosphere, and DIAD (123.32 mg, 0.610 mmol) was slowly added dropwise at 0 °C. The mixture was stirred at 20 °C for 18 h. After the reaction was completed, the mixture was diluted with an appropriate amount of tetrahydrofuran, cooled to 0 °C, washed with ethyl acetate and water, concentrated, and dried to give a crude product. The crude product was purified by a flash silica gel column (0-50% petroleum ether/ethyl acetate) to give compound 66-2 (71 mg, yield: 49%), ES-API: [M+H]⁺ = 476.9.

Step 3: Compound 66-2 (1431.15 mg, 3 mmol), water (10 mL), and lithium hydroxide (629.40 mg, 15.000 mmol) were mixed in tetrahydrofuran (10 mL), and the mixture was stirred at 20 °C for 3 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-40% petroleum ether/ethyl acetate) to give compound 66-3 (870 mg, yield: 88.43%), ES-API: [M+H]⁺ = 328.1.

Step 4: Compound 66-3 (435 mg, 1.326 mmol), 4-prop-2-ynyl-thiomorpholine-1,1-dioxide (275.73 mg, 1.592 mmol), PdCl₂(PPh₃)₂ (46.55 mg, 0.066 mmol), DIPEA (0.80 mL, 4.580 mmol), and CuI (25.26 mg, 0.133 mmol) were mixed in acetonitrile (10 mL) under a nitrogen atmosphere, and the mixture was stirred at 20 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 66-4 (380 mg, yield: 76.75%), ES-API: [M+H]⁺ = 373.0.

Step 5: Compound 66-4 (200 mg, 0.536 mmol), methanesulfonic anhydride (112.00 mg, 0.643 mmol), and triethylamine (0.112 mL, 0.804 mmol) were mixed in dichloromethane (10 mL) under a nitrogen atmosphere, and the mixture was stirred at 20 °C for 2 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated to give compound 66-5 (240 mg, crude product, yield: 99%), ES-API: [M+H]⁺ = 451.0.

Step 6: The reaction mixture of compound 66-5 (241 mg, crude product, 0.532 mmol) and methylamine (a 2.0 M solution in methanol, 20 mmol) was stirred at 50 °C overnight. The mixture was concentrated to remove the solvent to give desired crude compound 66-6 as an oily product.

Step 7: Compound 66-6 (crude product), di-tert-butyl dicarbonate (0.345 mL, 1.500 mmol), and triethylamine (0.208 mL, 1.500 mmol) were mixed in tetrahydrofuran (10 mL), and the mixture was stirred at 20 °C for 3 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 66-7 (150 mg, two-step yield: 58.0%), ES-API: [M+H]⁺ = 486.0.

Step 8: Intermediate 9 (92.2 mg, 0.134 mmol), compound 66-7 (64 mg, 0.130 mmol), Pd(dtbpf)Cl₂ (6.69 mg, 0.010 mmol), toluene (1 mL), water (1 mL), and potassium phosphate (65.45 mg, 0.308 mmol) were mixed in dioxane (3 mL) under a nitrogen atmosphere, and the mixture was stirred at 70 °C for 4 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-100% petroleum ether/ethyl acetate) to give compound 66-8 (99.7 mg, yield: 79%), ES-API: [M+H]⁺ = 969.3.

Step 9: Compound 66-8 (60 mg, 0.06 mmol), iodoethane (48.23 mg, 0.309 mmol), and cesium carbonate (100.80 mg, 0.309 mmol) were mixed in DMF (5 mL), and the mixture was stirred at 60 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-10% dichloromethane/methanol) to give compound 66-9 (60 mg, yield: 97%), ES-API: [M+H]⁺ = 997.3.

Step 10: Compound 66-9 (60 mg, 0.060 mmol) and trifluoroacetic acid (3 mL, 0.120 mmol) were mixed in dichloromethane (6 mL), and the mixture was stirred at 20 °C for 1 h. After the reaction was completed, 30 mL of ethyl acetate was poured into the mixture, and the resulting mixture was washed sequentially with 15 mL of a sodium bicarbonate solution and 15 mL of saturated brine. The organic phase was dried over anhydrous sodium sulfate, filtered, and concentrated, and the residue was purified by prep-HPLC (ammonium bicarbonate method 1) to give 2 isomeric compounds. The structure of one of the isomeric compounds was (1r,2R,3S)-N-((6³S,4S,Z)-(S*ₐ*)-1²-(5-(3-(1,1-dioxidothiomorpholino)prop-1-yn-1-yl)-2-((S)-1-(methylamino)ethyl)pyridin-3-yl)-11-ethyl-10,10-dimethyl-5,7-dioxo-6*¹*,6*²*,6*³*,6*⁴*,6*⁵*,6*⁶*-hexahydro-1*¹*H-8-oxa-2(4,2)-thiazola-1(5,3)-indola-6(1,3)-pyridazinacycloundecaphane-4-yl)-2,3-dimethylcyclopropane-1-carboxamide (Z66-1, 12 mg, yield: 22%, purity: 98%, retention time: 5.46 min). ES-API[M+H]⁺=897.3. ¹HNMR (400 MHz, DMSO-*d₆*) 8.81 (1 H, d, *J* 1.7)*,* 8.52 (1 H, s), 8.41 (1 H, d, *J* 9.1), 7.95 (1 H, s), 7.82 (1 H, s), 7.74 (1 H, d, *J* 8.6)*,* 7.54 (1 H, d*, J* 8.6)*,* 5.54 (1 H, t, *J* 9.2), 5.04 (1 H, d, *J* 12.0), 4.21 (2 H, t, *J* 12.4), 4.04 (1 H, dd, *J* 14.6, 7.2), 3.74 (3 H,s), 3.71-3.51 (3 H, m), 3.42 - 3.35 (2 H, m), 3.22-3.12 (5 H, m), 3.09-2.98 (5 H, m), 2.80-2.71 (1 H, m), 2.38-2.28 (1 H, m), 2.19 (3 H, s), 2.12 (1 H, d, *J* 10.2), 1.85-1.74 (2 H, m), 1.59 - 1.45 (1 H, m), 1.30-1.15 (6 H, m), 1.14-1.02 (6 H, m), 0.99 (3 H, d, *J* 6.4), 0.92 (3 H, s), 0.48 (3 H, s). The structure of the other isomeric compound was: (1r,2R,3S)-N-((6³S,4S,Z)-(R*ₐ*)-1²-(5-(3-(1,1-dioxidothiomorpholino)prop-1-yn-1-yl)-2-((S)-1-(methylamino)ethyl)pyridin-3-yl)-1*¹*-ethyl-10,10-dimethyl-5,7-dioxo-6¹,6²,6³,6⁴,6⁵,6⁶-hexahydro-1*¹*H-8-oxa-2(4,2)-thiazola-1(5,3)-indola-6(1,3)-pyridazinacycloundecaphane-4-yl)-2,3-dimethylcyclopropane-1-carboxamide (Z66, 8 mg, yield: 14.8%, purity: 98%, retention time: 5.50 min). ES-API[M+H]⁺=897.3. ¹HNMR (400 MHz, DMSO-*d₆*) 8.78 (1 H, s), 8.50 (1 H, s), 8.37 (1 H, d,*J* 8.8), 7.81 (2 H, s), 7.74 (1 H, d, *J* 7.5), 7.56 (1 H, d, *J* 8.7), 5.60 (1 H, s), 5.09 (1 H, d*,J* 12.2)*,* 4.45-4.1 (3H, m), 3.75 (2 H, s), 3.67 - 3.51 (3 H, m), 3.29 - 3.24 (2 H, m), 3.18 - 3.01 (10 H, m), 2.95-2.85 (1 H, m), 2.80-2.71 (1 H, m), 2.38-2.31 (4 H, m), 2.08-2.01 (1 H, m), 1.82-1.71 (2 H, m), 1.55-1.42 (1 H,m), 1.26 (3 H, d, *J* 6.0)*,* 1.15 (3 H, s), 1.08 - 1.02 (6 H, m), 0.95-0.81 (6 H, m), 0.44 (3 H, s).

### Example 1.48. Synthesis of Compound Z83

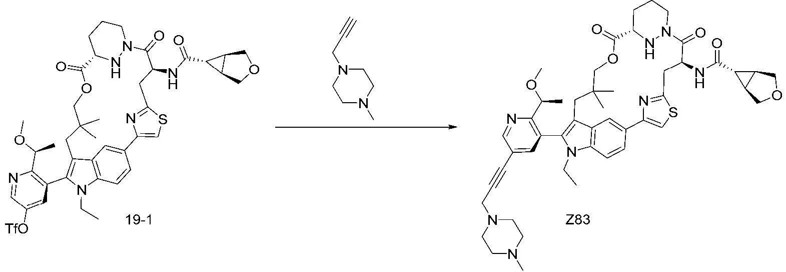

Step 1: Compound 19-1 (140.0 mg, 0.158 mmol) and 1-methyl-4-(prop-2-yn-1-yl)piperazine (110.0 mg, 0.789 mmol) were added to N,N-dimethylformamide (5.0 mL), and then bis(triphenylphosphine)palladium(II) dichloride (59.89 mg, 0.085 mmol), copper(I) iodide (32.50 mg, 0.171 mmol), N,N-diisopropylethylamine (0.198 mL, 1.138 mmol), and lithium chloride (60.28 mg, 1.422 mmol) were added. After the addition, the reaction mixture was stirred at 80 °C for 1.5 h under a nitrogen atmosphere. The reaction mixture was then diluted with water (80 mL) and extracted with ethyl acetate (30 mL × 3). The organic layers were combined, washed with brine, dried over anhydrous sodium sulfate, filtered, and concentrated. The residue was purified by an automated flash chromatographic column (elution by a 0% to 1.0% solution of methanol in dichloromethane) to give (1R,5S,6r)-N-((6³S,4S,Z)-1¹-ethyl-(Rₐ)-1²-(2-((S)-1-methoxyethyl)-5-(3-(4-methylpiperazin-1-yl)prop-1-yn-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z83, 90.0 mg, yield: 65.16%, retention time: 5.23 min). ES-API[M+H]⁺= 877.3. ¹H NMR (400 MHz, DMSO-*d₆*) δ 8.82 - 8.80 (m, 1H), 8.57 - 8.55 (m, 1H), 8.50 (s, 1H), 7.86 - 7.84 (m, 1H), 7.81 (s, 1H), 7.77 - 7.74 (m, 1H), 7.59 - 7.55 (m, 1H), 5.59 - 5.54 (m, 1H), 5.09 - 5.06 (m, 1H), 4.39 - 3.98 (m, 5H), 3.85 - 3.81 (m, 2H), 3.68 - 3.55 (m, 6H), 3.35 - 3.31 (m, 8H), 3.16 - 3.10 (m, 1H), 2.98 - 2.94 (m, 1H), 2.78 - 2.55 (m, 8H), 2.42 - 2.31 (m, 4H), 2.09 - 2.06 (m, 1H), 1.99 - 1.87 (m, 2H), 1.79 - 1.77 (m, 2H), 1.66 (t, *J =* 3.0 Hz, 1H), 1.54 - 1.49(m, 1H), 1.36 - 1.33 (m, 3H), 0.93 - 0.84 (m, 6H).

### Example 1.49. Synthesis of Compound Z84

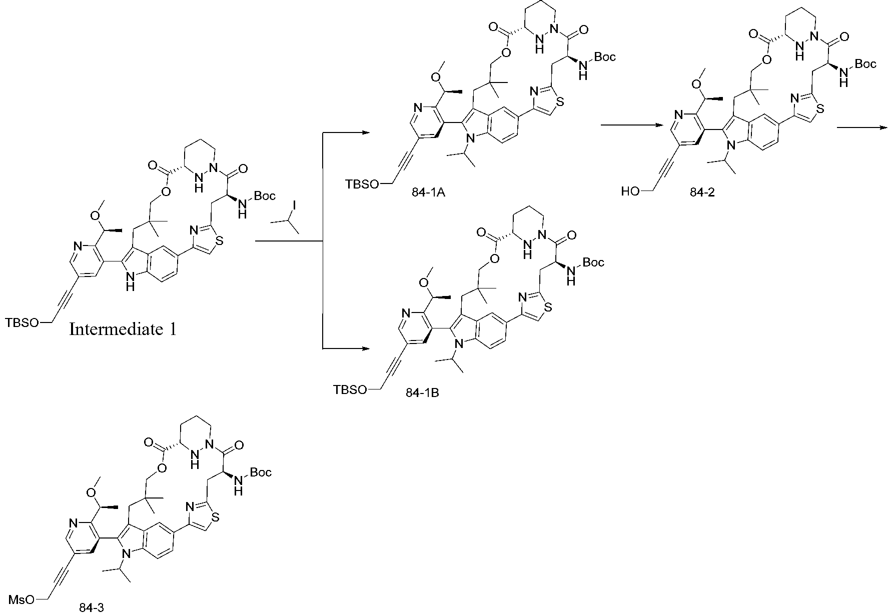

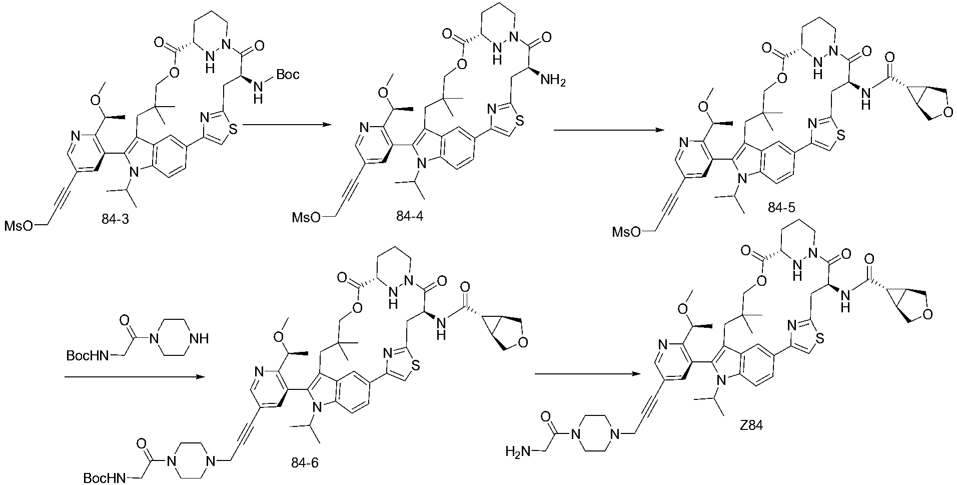

Step 1: Intermediate 1 (1000 mg, 1.148 mmol) was dissolved in N,N-diisopropylethylenediamine (10 mL), and cesium carbonate (3731.00 mg, 11.480 mmol) and 2-iodopropane (1.148 mL, 11.479 mmol) were added. The mixture was stirred at 50 °C for 48 h under a nitrogen atmosphere. The mixture was cooled to room temperature, extracted with ethyl acetate (30 mL × 3), washed sequentially with water (50 mL) and saturated brine (50 mL), dried over anhydrous sodium sulfate, filtered, and concentrated, and the residue was purified by silica gel column chromatography (ethyl acetate/dichloromethane = 2/3) to give 2 isomeric compounds. The structure of one of the isomeric compounds was compound 84-1A (262 mg, yield: 25%, retention time: 3.554 min). ES-API[M+H] ⁺=913.4. The structure of the other isomeric compound was compound 84-1B (510 mg, yield: 48.6%, retention time: 3.342 min). ES-API[M+H] ⁺=913.4.

Step 2: Compound 84-1A (262 mg, 0.287 mmol) was dissolved in tetrabutylammonium fluoride (5 mL, a 1 M solution in tetrahydrofuran), and the mixture was stirred at room temperature for 1 h. The mixture was diluted with water (20 mL), extracted with ethyl acetate (30 mL × 3), washed sequentially with water (50 mL) and saturated brine (50 mL), dried over anhydrous sodium sulfate, filtered, and concentrated to give compound 84-2 (206 mg, 0.258 mmol, yield: 90%). ES-API[M+H] ⁺= 799.3.

Step 3: N,N-Diisopropylethylamine (166.4 mg, 1.29 mmol) and methanesulfonic anhydride (67.3 mg, 0.387 mmol) were added to a solution of compound 84-2 (206 mg, 0.258 mmol) in dichloromethane (15 mL). The mixture was stirred at 25 °C for 1 h. When the reaction was completed as detected by LCMS, the reaction mixture was diluted with dichloromethane (20 mL) and then washed with saturated brine (15 mL × 3). The organic phase was dried over anhydrous sodium sulfate, filtered, and concentrated, and the residue was purified by silica gel column chromatography (ethyl acetate/dichloromethane = 1/1) to give compound 84-3 (181 mg, 0.206 mmol, yield: 85%). ES-API: [M+H]⁺= 877.3.

Step 4: A solution (2 mL, 4 M, 8.0 mmol) of hydrochloric acid in dioxane was added to a solution of compound 84-3 (150.0 mg, 0.171 mmol) in methanol (2 mL), and the reaction mixture was stirred at room temperature for 1.0 h. When the reaction was completed as monitored by LC-MS, the reaction system was concentrated to give crude compound 84-4 (168.0 mg, crude product). ES-API: [M+H]⁺= 777.2.

Step 5: N,N-Diisopropylethylamine (0.18 mL, 1.026 mmol) was added to a solution of compound 84-4 (132.86 mg, 0.171 mmol) and (1R,5S,6r)-3-oxabicyclo[3.1.0]hexane-6-carboxylic acid (32.8 mg, 0.257 mmol) in dry N,N-dimethylformamide (2.0 mL) at 0 °C, and then O-(7-azabenzotriazol-1-yl)*-*N,N,N',N'-tetramethyluronium hexafluorophosphate (97.5 mg, 0.257 mol) was added. The reaction system was warmed to room temperature and reacted for 10 min. When the reaction was completed as monitored by LC-MS, the reaction mixture was diluted with 100 mL of ethyl acetate and washed with saturated brine (30 mL × 5). The organic phase was concentrated, and the crude product was purified by a flash silica gel [methanol/dichloromethane = 0-2%] to give compound 84-5 (190.0 mg, crude product). ES-API: [M+H]⁺= 887.2.

Step 6: Potassium carbonate (48 mg, 0.349 mmol), sodium iodide (17 mg, 0.116 mmol), and tert-butyl (2-oxo-2-(piperazin-1-yl)ethyl)carbamate (125 mg, 0.513 mmol) were sequentially added to a solution of compound 84-5 (150 mg, 0.171 mmol) in acetonitrile (6 mL). The reaction mixture was heated to 70 °C and stirred for 3 h. When the reaction was completed as detected by LC-MS, the reaction mixture was diluted with ethyl acetate (15 mL) and washed with water (15 mL) and saturated brine (15 mL × 2). The organic phase was dried over anhydrous sodium sulfate and filtered, and the filtrate was concentrated to dryness. The crude product was purified by a flash silica gel column [dichloromethane/methanol = 100:0 to 90:10 (v/v)] to give compound 84-6 (110.0 mg, yield: 62.19%) as a white solid. ES-API: [M+H]⁺= 1034.3.

Step 7: Trifluoroacetic acid (1.0 mL, 123.0 mmol) was added to a solution of compound 84-6 (110.0 mg, 0.106 mmol) in dichloromethane (5.0 mL) at room temperature. After the mixture was reacted for 1 h, the mixture was concentrated to dryness by rotary evaporation under reduced pressure to remove the solvent to give a crude product. The crude product was subjected to prep-HPLC (ammonium bicarbonate method 1) to give (1R,5S,6r)-N-((6³S,4S,Z)-(Rₐ)-1²-(5-(3-(4-glycylpiperazin-1-yl)prop-1-yn-1-yl)-2-((S)-1-methoxyethyl)pyridin-3-yl)-1¹-isopropyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z84, 30.0 mg, yield: 30.20%). ES-API: [M+H]⁺= 934.3. ¹H NMR (400 MHz, DMSO-*d₆*) δ 8.82 - 8.80(m, 1H), 8.54 - 8.52 (m, 1H), 8.48 (s, 1H), 7.81 - 7.64 (m, 4H), 5.62 - 5.60 (m, 1H), 5.12 - 5.09 (m, 1H), 4.51 - 4.49 (m, 1H), 4.32 - 4.19 (m, 2H), 4.12 - 4.10 (m, 1H), 3.83 - 3.80 (m, 2H), 3.66 - 3.55 (m, 6H), 3.50 (s, 2H), 3.34 (m, 13H), 3.15 - 3.09(m, 2H), 2.87 - 2.73 (m, 2H), 2.39 - 2.35 (m, 1H), 2.04 - 2.01 (m, 1H), 1.92 - 1.88 (m, 2H), 1.75 - 1.73 (m, 5H), 1.65 - 1.63 (m, 1H), 1.53 - 1.50 (m, 1H), 1.41 - 1.38 (m, 3H), 1.22 - 1.18(m, 3H), 0.84 (s, 3H), 0.45 (s, 3H).

### Example 1.50. Synthesis of Compound Z85

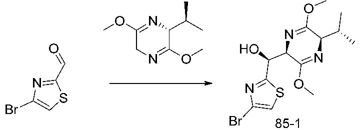

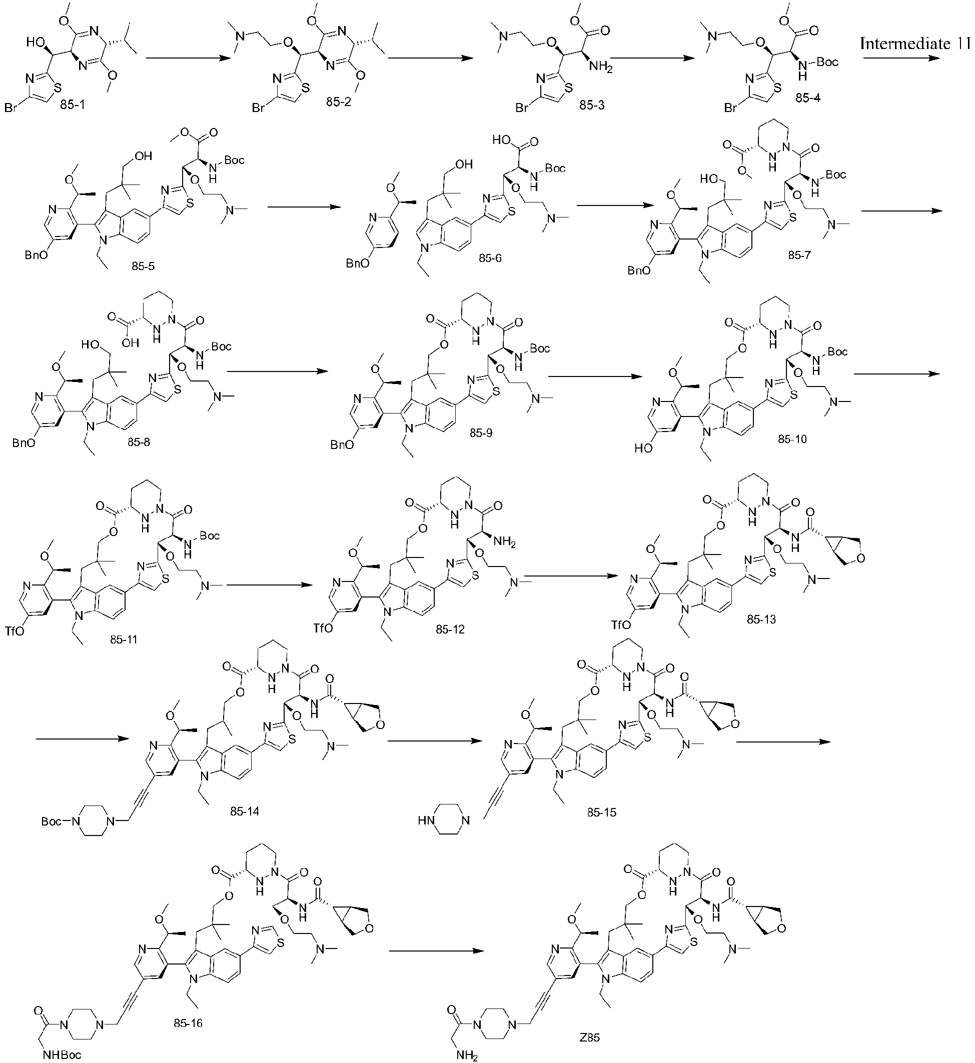

Step 1: (2R)-3,6-Dimethoxy-2-(propan-2-yl)-2,5-dihydropyrazine (45 g, 244.247 mmol) was dissolved in tetrahydrofuran (400 mL). n-Butyllithium (2.5 M in hexane, 100 mL) was added, and the mixture was stirred at -78 °C for 1 h under a nitrogen atmosphere. A solution (65 mL) of tin tetrachloride (55.57 g, 293.096 mmol) in tetrahydrofuran was added, and the mixture was stirred at -78 °C for 1 h under a nitrogen atmosphere. A solution (50 mL) of 2-bromo-5-formylthiazole (46.90 g, 244.247 mmol) in tetrahydrofuran was then added, and the mixture was stirred at -78 °C for 2 h under a nitrogen atmosphere. After the reaction was completed, water (200 mL) was added, and the mixture was extracted with ethyl acetate (150 mL × 2). The organic phase was washed with saturated brine (100 mL × 3), dried over anhydrous sodium sulfate, and concentrated to give a crude product. The crude product was purified by a flash silica gel column (ethyl acetate/petroleum ether: 0%-20%) to give compound 85-1 (26000 mg, 69.099 mmol, yield: 28.29%) as a colorless oily liquid, ES-API: [M+H]⁺ = 376.0, 378.0.

Step 2: Compound 85-1 (550 mg, 1.462 mmol), dimethylaminoethyl chloride hydrochloride (2947.64 mg, 20.464 mmol), sodium hydroxide (2455.68 mg, 61.392 mmol), and benzyltriethylammonium chloride (233.06 mg, 1.023 mmol) were mixed in water (5 mL) and toluene (5 mL) under a nitrogen atmosphere. The mixture was stirred at room temperature for 30 min and then stirred at 100 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 85-2 (380 mg, yield: 58.11%), ES-API: [M+H]⁺ = 447.1.

Step 3: Compound 85-2 (380 mg, 0.849 mmol) was dissolved in acetonitrile (5 mL), and hydrochloric acid (1 M, 10 mL) was added at 20 °C. The mixture was stirred for 3 h. After the reaction was completed, the mixture was concentrated to dryness by rotary evaporation to give crude compound 85-3, which was directly used in the next step.

Step 4: Crude compound 85-3, di-tert-butyl dicarbonate (1.608 mL, 7.000 mmol), and DIPEA (904.75 mg, 7.000 mmol) were mixed in water (5 mL) and tetrahydrofuran (5 mL), and the mixture was stirred at 20 °C for 3 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-20% dichloromethane/methanol) to give compound 85-4 (360 mg, two-step yield: 93%), ES-API: [M+H]⁺ = 452.0.

Step 5: Intermediate 11 (575.45 mg, 0.961 mmol), compound 85-4 (300 mg, 0.663 mmol), Pd(dtbpf)Cl₂ (38.54 mg, 0.060 mmol), toluene (5 mL), water (5 mL), and potassium phosphate (281.99 mg, 1.326 mmol) were mixed in dioxane (15 mL) under a nitrogen atmosphere, and the mixture was stirred at 70 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-20% dichloromethane/methanol) to give compound 85-5 (400 mg, yield: 71.43%), ES-API: [M+H]⁺ = 844.2.

Step 6: Compound 85-5 (400 mg, 0.474 mmol), lithium hydroxide (59.71 mg, 1.422 mmol), and water (3 mL) were mixed in tetrahydrofuran (12 mL), and the mixture was stirred at 20 °C for 2 h. After the reaction was completed, 30 mL of ethyl acetate was poured into the mixture, and the resulting mixture was adjusted to a weakly acidic pH with diluted hydrochloric acid, washed sequentially with 15 mL of water and 15 mL of saturated brine. The organic phase was dried over anhydrous sodium sulfate, filtered, and concentrated, and the residue was purified by a flash silica gel column to give compound 85-6 (350 mg, yield: 90%), ES-API: [M+H]⁺ = 830.2.

Step 7: Compound 85-6 (400 mg, 0.482 mmol), (S)-hexahydropyridazine-3-carboxylate trifluoroacetate (538.12 mg, 1.446 mmol), HOBt (97.58 mg, 0.723 mmol), 4-methylmorpholine (486.72 mg, 4.819 mmol), and EDCI (460.21 mg, 2.409 mmol) were mixed in dichloromethane (10 mL), and the mixture was stirred at 20 °C for 1 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 85-7 (400 mg, yield: 86.96%), ES-API: [M+H]⁺ = 956.3.

Step 8: Compound 85-7 (400 mg, 0.418 mmol), lithium hydroxide (20.08 mg, 0.837 mmol), and water (4 mL) were mixed in tetrahydrofuran (16 mL), and the mixture was stirred at 20 °C for 1 h. After the reaction was completed, the mixture was adjusted to weak acidity with diluted hydrochloric acid. 30 mL of ethyl acetate was poured into the mixture, and the resulting mixture was washed sequentially with 15 mL of water and 15 mL of saturated brine. The organic phase was dried over anhydrous sodium sulfate, filtered, and concentrated to give crude compound 85-8.

Step 9: Crude compound 85-8, HOBt (215.13 mg, 1.592 mmol), DIPEA (0.832 mL, 4.776 mmol), and EDCI (915.58 mg, 4.776 mmol) were mixed in dichloromethane (20 mL) under a nitrogen atmosphere, and the mixture was stirred at 20 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column to give compound 85-9 (150 mg, two-step yield: 39%), ES-API: [M+H]⁺ = 924.3.

Step 10: Compound 85-9 (150 mg, 0.162 mmol), Pd/C 10% (80 mg, 0.162 mmol), and tetrahydrofuran (5 mL) were mixed in methanol (5 mL) under a hydrogen atmosphere, and the mixture was stirred at 40 °C for 18 h. After the reaction was completed, the mixture was filtered and concentrated to give crude compound 85-10, which was directly used in the next step.

Step 11: Crude compound 85-10, DIPEA (60.32 mg, 0.468 mmol), and N-phenyl-bis(trifluoromethanesulfonimide) (111.36 mg, 0.312 mmol) were mixed in dichloromethane (10 mL), and the mixture was stirred at 20 °C for 2 h. After the reaction was completed, the mixture was concentrated, and the residue was purified by a flash silica gel column to give compound 85-11 (80 mg, two-step yield: 51%). ES-API[M+1]⁺=966.3.

Step 12: Compound 85-11 (35 mg, 0.036 mmol) and a solution (4 M, 3 mL) of hydrochloric acid/dioxane were mixed in methanol (1 mL), and the mixture was stirred at 20 °C for 1 h. After the reaction was completed, the mixture was concentrated to give crude compound 85-12 (crude product).

Step 13: Compound 85-12 (crude product), (1R,5S,6r)-3-oxabicyclo[3.1.0]hexane-6-carboxylic acid (13.84 mg, 0.108 mmol), and DIPEA (27.92 mg, 0.216 mmol) were mixed in DMF (3 mL), and HATU (27.38 mg, 0.072 mmol) was added at 0 °C. The mixture was stirred for 1 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (dichloromethane/methanol = 100:6) to give compound 85-13 (30 mg, two-step yield: 86%), ES-API: [M+H]⁺ = 976.3.

Step 14: Compound 85-13 (30 mg, 0.031 mmol), tert-butyl 4-(prop-2-ynyl)piperazine-1-carboxylate (13.79 mg, 0.061 mmol), Pd(PPh₃)₂Cl₂ (4.31 mg, 0.006 mmol), CuI (1.17 mg, 0.006 mmol), DIPEA (15.86 mg, 0.123 mmol), and lithium chloride (5.16 mg, 0.123 mmol) were mixed in N,N-dimethylformamide (3 mL) under a nitrogen atmosphere, and the mixture was stirred at 80 °C for 18 h. After the reaction was completed, 20 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (dichloromethane/methanol = 100:3) to give compound 85-14 (25 mg, yield: 77.45%), ES-API: [M+H]⁺ = 1050.3.

Step 15: Compound 85-14 (25 mg, 0.024 mmol) and trifluoroacetic acid (2 mL, 0.029 mmol) were mixed in dichloromethane (4 mL), and the mixture was stirred at 20 °C for 1 h. After the reaction was completed, 30 mL of ethyl acetate was poured into the mixture, and the resulting mixture was washed sequentially with 15 mL of a saturated sodium bicarbonate solution and 15 mL of saturated brine. The organic phase was dried over anhydrous sodium sulfate to give crude compound 85-15.

Step 16: Crude compound 85-15, N-BOC-glycine (8.41 mg, 0.048 mmol), DIPEA (18.61 mg, 0.144 mmol), and HATU (18.25 mg, 0.048 mmol) were mixed in dichloromethane (5 mL), and the mixture was stirred at 20 °C for 2 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated to give crude compound 85-16, which was directly used in the next step.

Step 17: Crude compound 85-16 and trifluoroacetic acid (1 mL, 0.024 mmol) were mixed in dichloromethane (2 mL), and the mixture was stirred at 20 °C for 0.5 h. After the reaction was completed, the mixture was concentrated, and the residue was purified by prep-HPLC (formic acid method 1) to give (1S,5S,6r)-N-((6³S,3S,4S,Z)-3-(2-(dimethylamino)ethoxy)-1¹-ethyl-(Rₐ)-1²-(5-(3-(4-glycylpiperazin-1-yl))prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z85, 8 mg, three-step yield: 26%, purity: 98%). ES-API[M+H]⁺=1007.3. ¹HNMR (400 MHz, DMSO-*d₆*) 8.81 (1 H, d, *J* 2.0), 8.51 (1 H, s), 8.30 (1 H, s), 8.15 (1 H, d, *J* 9.7), 7.91 (1H, s), 7.87 (1 H, d, *J* 2.0), 7.76 (1 H, d, J 8.7), 7.58 (1 H, d, *J* 8.7), 5.85 (1 H, d, *J* 9.6), 5.18 (1 H, *d, J* 11.9), 5.10 (1 H, s), 4.36 - 3.24 (24 H, m), 3.17 (3 H, s), 2.91-2.72 (2 H, m), 2.62-2.35 (5 H, m), 2.23 (6 H, s), 2.05 (1 **H,** d, *J* 12.6), 1.95-1.72 (5 **H,** m), 1.58-1.42 (1 H, m), 1.36 (3 H, d, *J* 6.1), 0.91 (6 H, dd, J22.1, 15.1), 0.46 (3 H, s).

### Example 1.51. Synthesis of Compound Z164

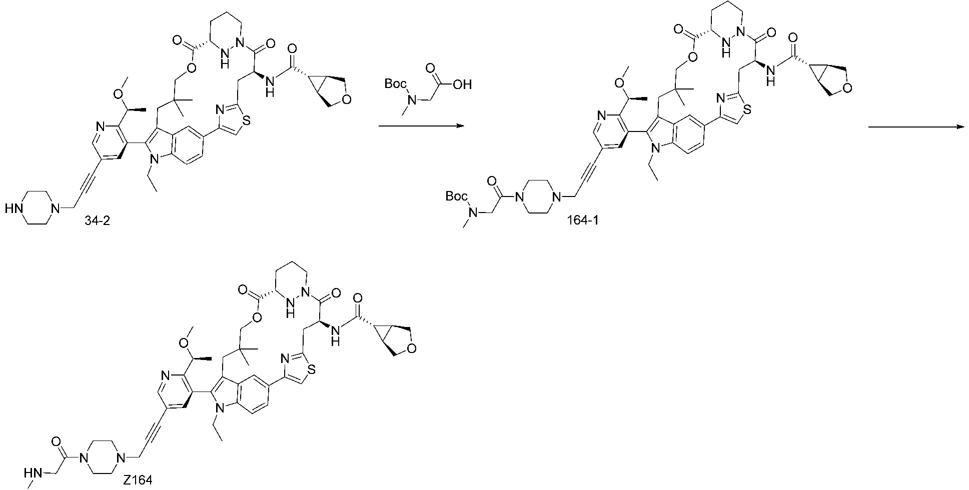

Step 1: Compound 34-2 (50 mg, 0.058 mmol) and tert-butoxycarbonyl-sarcosine (16.46 mg, 0.087 mmol) were dissolved in N,N-dimethylformamide (3 mL), and O-(7-azabenzotriazol-1-yl)-N,N,N',N'-tetramethyluronium hexafluorophosphate (33 mg, 0.09 mmol) and N,N'-diisopropylethylamine (22.46 mg, 0.174 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried over anhydrous sodium sulfate, filtered, and concentrated to give a crude product. The crude product was purified by a flash silica gel column (methanol/dichloromethane: 0%-10%) to give compound 164-1 (50 mg, 0.048 mmol, yield: 83.44%) as a white solid, ES-API: [M+H]⁺ = 1034.3.

Step 2: Compound 164-1 (40 mg, 0.039 mmol) was dissolved in methanol (1 mL), and a 4 M solution (3 mL) of hydrochloric acid in dioxane was added. The reaction system was stirred at room temperature for 1 h under a nitrogen atmosphere and concentrated to dryness under reduced pressure. The crude product was purified by prep-HPLC (ammonium bicarbonate method 1) to give (1*R*,5*S*,6*r*)-N-(6³*S*,4*S*,*Z*)-(*Rₐ*)-1¹-ethyl-1²-(2-((S)-1-methoxyethyl)-5-(3-(4-(methylglycyl)piperazin-1-yl)prop-1-yn-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-ylbicyclo[3.1.0]hexane-6-carboxamide (Z164, 17 mg, 0.018 mmol, yield: 43.5%) as a white solid, ES-API: [M+H]⁺ = 934.3. ¹H NMR (400 MHz, DMSO-*d₆*) δ 8.80 (d, *J* = 2.0 Hz, 1H), 8.55 (d, *J* = 8.8 Hz, 1H), 8.50 (s, 1H), 7.85 (d, *J* = 2.0 Hz, 1H), 7.81 (s, 1H), 7.75 (d, *J* = 7.6 Hz, 1H), 7.58 (d, *J* = 8.8 Hz, 1H), 5.56 (t, *J* = 8.8 Hz, 1H), 5.08 (d, *J =* 12.0 Hz, 1H), 4.40 - 4.00 (m, 5H), 3.83 (t, *J* = 8.0 Hz, 2H), 3.68 - 3.54 (m, 6H), 3.50 (s, 2H), 3.43-3.41 (m, 3H), 3.33-3.31 (m, 5H), 3.25 (s, 3H), 3.13-3.10 (m, 1H), 2.97-2.93 (m, 1H), 2.84-2.75 (m, 1H), 2.54-2.52 (m, 1H), 2.40-2.36 (m, 1H), 2.27 (s, 3H),2.09-2.06 (m, 1H), 1.98 - 1.87 (m, 2H), 1.79-1.77 (m, 2H), 1.71 - 1.70 (m, 1H), 1.66-1.65 (m, 1H), 1.53-1.49 (m, 1H), 1.35 *(d, J=* 6.0 Hz, 3H), 0.90-0.86 (m, 5H), 0.34 (s, 3H).

### Example 1.52. Synthesis of Compound Z165

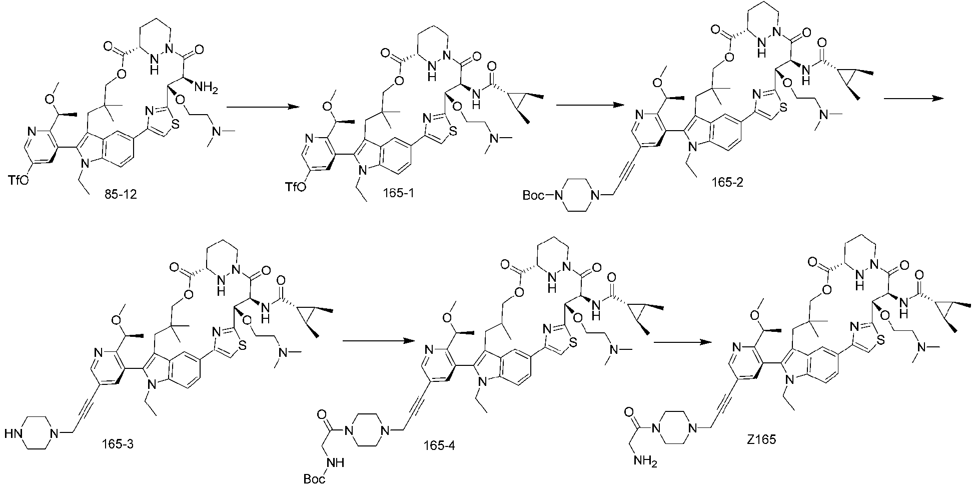

Step 1: Compound 85-12 (40 mg, 0.046 mmol), (1r,2R,3S)-2,3-dimethylcyclopropane-1-carboxylic acid (10.50 mg, 0.092 mmol), and DIPEA (59.70 mg, 0.462 mmol) were mixed in N,N-dimethylformamide (3 mL), and HATU (35.13 mg, 0.092 mmol) was added at 0 °C. The mixture was stirred for 1 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (dichloromethane/methanol = 100:6) to give compound 165-1 (40 mg, 0.042 mmol, yield: 90.38%), ES-API: [M+H]⁺ = 962.3.

Step 2: Compound 165-1 (40 mg, 0.042 mmol), tert-butyl 4-(prop-2-ynyl)piperazine-1-carboxylate (18.65 mg, 0.083 mmol), Pd(PPh₃)₂Cl₂ (5.84 mg, 0.008 mmol), copper(I) iodide (1.58 mg, 0.008 mmol), DIPEA (21.45 mg, 0.166 mmol), and lithium chloride (6.98 mg, 0.166 mmol) were mixed in N,N-dimethylformamide (3 mL) under a nitrogen atmosphere, and the mixture was stirred at 80 °C for 18 h. After the reaction was completed, 20 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (dichloromethane/methanol = 100:3) to give compound 165-2 (30 mg, 0.029 mmol, yield: 69.63%), ES-API: [M+H]⁺ = 1036.3.

Step 3: Compound 165-2 (30 mg, 0.029 mmol) and triethylamine (2 mL, 0.029 mmol) were mixed in dichloromethane (4 mL), and the mixture was stirred at 20 °C for 1 h. After the reaction was completed, 30 mL of ethyl acetate was poured into the mixture, and the resulting mixture was washed sequentially with 15 mL of a saturated sodium bicarbonate solution and 15 mL of saturated brine. The organic phase was dried over anhydrous sodium sulfate to give crude compound 165-3.

Step 4: Crude compound 165-3, N-{[(2-methylpropan-2-yl)oxy]carbonyl}glycine (10.16 mg, 0.058 mmol), DIPEA (22.49 mg, 0.174 mmol), and HATU (22.05 mg, 0.058 mmol) were mixed in dichloromethane (5 mL), and the mixture was stirred at 20 °C for 2 h. After the reaction was completed, 30 mL of a mixed solution (dichloromethane/isopropanol = 3/1) was poured into the mixture, and the resulting mixture was washed sequentially with 15 mL of water and 15 mL of saturated brine. The organic phase was dried over anhydrous sodium sulfate, filtered, and concentrated to give crude compound 165-4, which was directly used in the next step.

Step 5: Crude compound 165-4 obtained in the previous step and triethylamine (1 mL, 0.024 mmol) were mixed in dichloromethane (2 mL), and the mixture was stirred at 20 °C for 0.5 h. After the reaction was completed, the mixture was concentrated, and the residue was purified by prep-HPLC (formic acid method 1) to give (1r,2R,3S)-N-((6³S,3S,4S,Z)-3-(2-(dimethylamino)ethoxy)-1¹-ethyl-(Ra)-1²-(5-(3-(4-glycylpiperazin-1-yl))prop-1-yn-1-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide (Z165, 8 mg, three-step yield: 28%, purity: 98%, formate). ES-API[M+H]⁺=993.3. ¹HNMR (400 MHz, DMSO-*d₆*) 8.81 (1 H, d, *J* 2.0), 8.50 (1 H, s), 8.30 (1 H, s), 7.92 (1 H, s), 7.87 (1 H, d, J2.0), 7.84 (1 H, d, J9.7), 7.76 (1 H, d, *J* 8.7), 7.58 (1 H, d, *J* 8.7), 5.84 (1 H, d, J9.7), 5.17 (1 H, d, *J* 12.0), 5.09 (1 H, s), 4.37 - 3.33 (20 H, m), 3.17 (3 H, s), 2.80 (2 H, d, *J* 11.7), 2.61 - 2.41 (5 H, m), 2.23 (6 H, s), 2.04 (1 H, d, *J* 11.0), 1.82 (2 H, s), 1.51 (1 H, d*, J* 7.0)*,* 1.36 (4 H, d, *J* 5.9), 1.14 (2 H, s), 1.06 (6 H, t, *J* 6.2), 0.93 (3 H, t, *J* 7.0), 0.85 (3 H, s), 0.45 (3 H, s).

### Example 1.53. Synthesis of Compound Z166

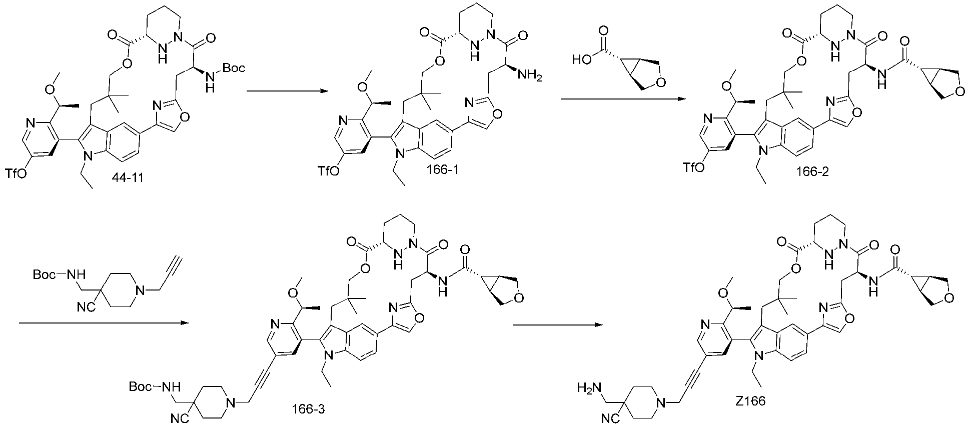

Step 1: Compound 44-11 (530 mg, 0.614 mmol) was dissolved in methanol (1 mL), and 4 M hydrochloric acid/dioxane (8 mL) was added. The mixture was reacted at room temperature for 1 h. The reaction mixture was concentrated to give compound 166-1 (580 mg, crude product), which was directly used in the next step. ES-API: [M+H]⁺=763.3.

Step 2: (1R,5S,6r)-3-Oxabicyclo[3.1.0]hexane-6-carboxylic acid (103 mg, 0.8 mmol), 4-methylmorpholine (248.42 mg, 2.456 mmol), and HATU (350.20 mg, 0.921 mmol) were sequentially added to a solution of compound 166-1 (580 mg, crude product) in dichloromethane (10 mL), and the mixture was reacted at room temperature for 2 h. The mixture was extracted with dichloromethane (20 mL × 3), washed with an aqueous sodium chloride solution (30 mL × 1), dried over anhydrous sodium sulfate, filtered, and concentrated. The resulting crude product was purified by silica gel column chromatography (ethyl acetate/petroleum ether = 2/1) to give compound 166-2 (380 mg, 0.435 mmol, two-step yield: 70.90%). ES-API: [M+H]⁺=873.3.

Step 3: Pd(PPh₃)₂Cl₂ (24.15 mg, 0.034 mmol), copper(I) iodide (13.10 mg, 0.069 mmol), DIPEA (88.75 mg, 0.688 mmol), and lithium chloride (43.75 mg, 1.032 mmol) were sequentially added to a solution of compound 166-2 (75 mg, 0.087 mmol) and tert-butyl ((4-cyano-1-(prop-2-yn-1-yl)piperidin-4-yl)methyl)carbamate (143.12 mg, 0.516 mmol) in N,N-dimethylformamide (3 mL). The mixture was heated to 85 °C and reacted for 3 h. The mixture was extracted with ethyl acetate (20 mL × 3), washed with an aqueous sodium chloride solution (30 mL × 1), dried over anhydrous sodium sulfate, filtered, and concentrated. The resulting crude product was purified by silica gel column chromatography (90% ethyl acetate/petroleum ether) to give compound 166-3 (75 mg, 0.075 mmol, yield: 43.64%). ES-API: [M+H]⁺=1000.3.

Step 4: Compound 166-3 (15 mg, 0.015 mmol) was dissolved in dichloromethane (1 mL), and trifluoroacetic acid (1 mL) was added. The mixture was reacted at room temperature for 1 h. The mixture was concentrated to give a crude product. The crude product was subjected to prep-HPLC (ammonium bicarbonate method 1) to give (1*R*,5*S*,6r)-*N*-((6³*S*,4*S*,*Z*)-(*R*ₐ)-1²-(5-(3-(4-(aminomethyl)-4-cyanopiperidin-1-yl)prop-1-yn-1-yl)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphane-4-yl)-3-oxabicyclo[3.1.0]hexane-6-carboxamide (Z166, 7.2 mg, 0.008 mmol, yield: 50.73%). ES-API: [M+H]⁺=900.5. ¹H NMR (400 MHz, DMSO-*d₆*) δ 8.80 (d, J = 2.1 Hz, 1H), 8.56 (d, J = 8.6 Hz, 1H), 8.36 (s, 1H), 8.27 (s, 1H), 7.84 (d, J = 2.1 Hz, 1H), 7.58 - 7.49 (m, 2H), 5.75 - 5.66 (m, 1H), 5.07 (d, J = 11.9 Hz, 1H), 4.39 - 4.17 (m, 4H), 4.08 - 3.99 (m, 1H), 3.83-3.78 (m, 2H), 3.66 - 3.48 (m, 6H), 3.20 (s, 3H), 3.16-3.10 (m, 2H), 2.98 - 2.83 (m, 5H), 2.69 (s, 2H), 2.47 - 2.39 (m, 3H), 2.06 - 1.83 (m, 6H), 1.77-1.74 (m, 1H), 1.62 (t, J = 3.2 Hz, 2H), 1.53-1.49 (m,3H), 1.35 (d, J = 6.1 Hz, 3H), 0.91 (t, J = 7.0 Hz, 3H), 0.85 (s, 3H), 0.36 (s, 3H).

### Example 1.54. Synthesis of Compound Z167

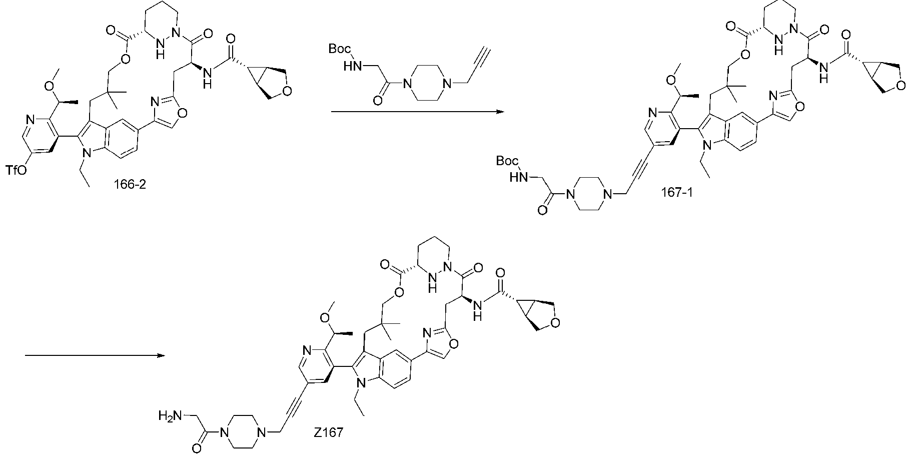

Step 1: Pd(PPh₃)₂Cl₂ (8.07 mg, 0.011 mmol), copper(I) iodide (4.37 mg, 0.023 mmol), DIPEA (11.87 mg, 0.092 mmol), and lithium chloride (5.85 mg, 0.138 mmol) were sequentially added to a solution of compound 166-2 (20 mg, 0.023 mmol) and tert-butyl (2-oxo-2-(4-(prop-2-yn-1-yl)piperazin-1-yl)ethyl)carbamate (19.34 mg, 0.069 mmol) in acetonitrile (5 mL). The mixture was heated to 85 °C and reacted for 1 h. The mixture was extracted with ethyl acetate (20 mL × 3), washed with an aqueous sodium chloride solution (30 mL × 1), dried over anhydrous sodium sulfate, filtered, and concentrated. The resulting crude product was purified by silica gel column chromatography (90% ethyl acetate/petroleum ether) to give compound 167-1 (18 mg, 0.018 mmol, yield: 78.23%). ES-API: [M+H]⁺=1004.3.

Step 2: Compound 167-1 (18 mg, 0.018 mmol) was dissolved in methanol (1 mL), and a solution (2 mL, 4 M) of hydrochloric acid/dioxane was added. The mixture was reacted at room temperature for 1 h. The mixture was concentrated to give a crude product. The crude product was subjected to prep-HPLC (formic acid method) to give (1*R*,5*S*,6r)-*N*-((6³*S*,4*S*,*Z*)-1²-(5-(3-(4-glycylpiperazin-1-yl)prop-1-yn-1-yl)-(*R*a)-2-((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphane-4-yl)-3-oxabicyclo[3.1.0]hexane-6-carboxamide (Z167, 5.85 mg, 0.006 mmol, yield: 33.52%, formate). ES-API: [M+H]⁺=904.5.

### Example 1.55. Synthesis of Compound Z168

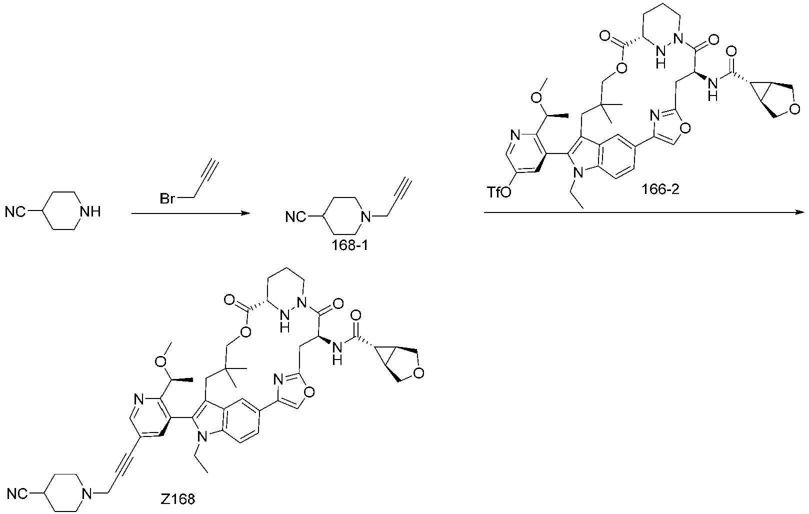

Step 1: 4-Piperidinecarbonitrile (1000 mg, 9.078 mmol) was dissolved in acetonitrile (15 mL), potassium carbonate (2509.16 mg, 18.156 mmol) and 3-bromoprop-1-yne (1079.88 mg, 9.078 mmol) were added, and the mixture was reacted at room temperature for 3 h. After the reaction was completed, water (50 mL) and ethyl acetate (50 mL) were added. The organic layer was separated, further washed with a saturated sodium chloride solution (50 mL × 3), dried over anhydrous sodium sulfate, filtered, and concentrated. The resulting crude product was purified by silica gel column chromatography to give compound 168-1 (1000 mg, 6.747 mmol, yield: 74.33%). ES-API: [M+H]⁺=149.1.

Step 2: Pd(PPh₃)₂Cl₂ (62.32 mg, 0.089 mmol), copper(I) iodide (33.82 mg, 0.178 mmol), DIPEA (114.53 mg, 0.888 mmol), and lithium chloride (37.64 mg, 0.888 mmol) were sequentially added to a solution of compound 166-2 (155 mg, 0.178 mmol) and compound 168-1 (78.95 mg, 0.533 mmol) in acetonitrile (5 mL). The mixture was heated to 85 °C and reacted for 1 h under a nitrogen atmosphere. After the reaction was completed, water (20 mL) and ethyl acetate (30 mL) were added. The organic layer was separated, further washed with a saturated sodium chloride solution (50 mL × 3), dried over anhydrous sodium sulfate, filtered, and concentrated. The residue was purified by prep-HPLC (ammonium bicarbonate method 1) to give (1R,5S,6r)-N-((6³S,4S,Z)-(Rₐ)-1²-(5-(3-(4-cyanopiperidin-1-yl)prop-1-yn-1-yl)-2-((S)-1-methoxyethyl)-pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphane-4-yl)-3-oxabicyclo[3.1.0]hexane-6-carboxamide (Z168, 135.7 mg, 0.156 mmol, yield: 87.6%), ES-API: [M+H]⁺ = 871.3. ¹H NMR (400 MHz, DMSO-*d₆*) δ 8.81 (1 H, d, *J=* 2.1Hz), 8.58 (1 H, d, *J=* 8.5 Hz), 8.37 (1 H, s), 8.27 (1 H, s), 7.85 (1 H, d, *J=* 2.1 Hz), 7.60 - 7.45 (2 H, m), 5.77 - 5.67 (1 H, m), 5.09 (1 H, d*, J =* 11.9 Hz), 4.41 - 4.18 (4 H, m), 4.09 - 3.76 (4 H, m), 3.68 - 3.55 (6 H, m), 3.20 (3 H, s), 3.14 (1 H, dd, *J=* 15.4, 4.3 Hz), 2.98 - 2.83 (3 H, m), 2.76 - 2.63 (3 H, m), 2.45 - 2.38 (2 H, m), 2.08 - 2.01 (1 H, m), 1.98-1.94 (1 H, m), 1.93 - 1.84 (3 H, m), 1.80-1.68 (3 H, m), 1.66-1.56 (2 H, m), 1.54 - 1.45 (1 H, m), 1.35 (3 H, d*, J=* 6.1 Hz), 0.98-0.81 (6 H, m), 0.35 (3 H, s).

### Example 1.56. Synthesis of Compounds Z169-1 and Z169-2

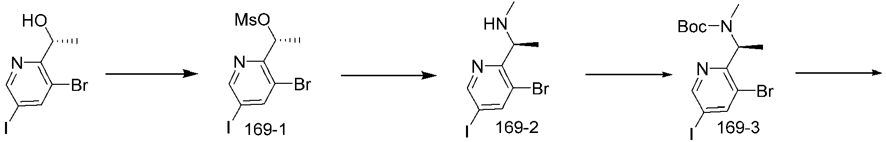

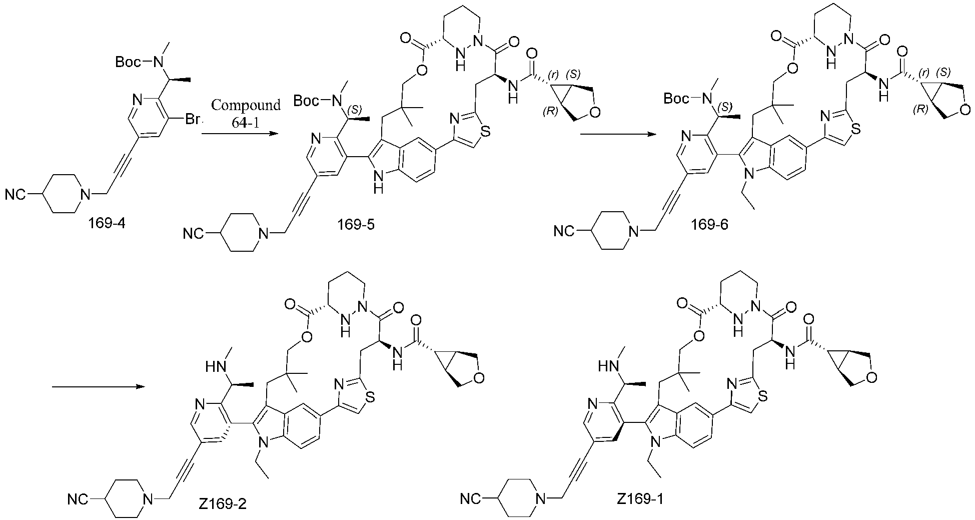

Step 1: (1R)-1-(3-Bromo-5-iodopyridin-2-yl)-1-ethanol (3 g, 9.148 mmol), methanesulfonic anhydride (1.91 g, 10.977 mmol), and triethylamine (1.39 g, 13.722 mmol) were mixed in dichloromethane (30 mL) under a nitrogen atmosphere, and the mixture was stirred at 20 °C for 2 h. After the reaction was completed, 30 mL of dichloromethane was poured into the mixture, and the resulting mixture was washed sequentially with 15 mL of water and 15 mL of saturated brine. The organic phase was dried over anhydrous sodium sulfate, filtered, and concentrated to give crude compound 169-1, ES-API: [M+1]⁺ = 405.9/407.9.

Step 2: The reaction mixture of crude compound 169-1 and a methylamine/methanol solution (5.0 M, 25 mL) was stirred at 45 °C overnight. The mixture was concentrated to remove the solvent to give desired crude compound 169-2 as an oily product, ES-API: [M+1]⁺ = 340.9/342.9.

Step 3: Crude compound 169-2, di-tert-butyl dicarbonate (6.272 mL, 27.300 mmol), and triethylamine (2.76 g, 27.300 mmol) were mixed in tetrahydrofuran (30 mL), and the mixture was stirred at 20 °C for 3 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 169-3 (4 g, 9.068 mmol, three-step yield: 99%), ES-API: [M+1]⁺ = 440.9/442.9.

Step 4: Compound 169-3 (753 mg, 1.707 mmol), 1-(prop-2-yn-1-yl)piperidine-4-carbonitrile (225 mg, 1.518 mmol), Pd(PPh₃)₂Cl₂ (106.42 mg, 0.152 mmol), copper(I) iodide (28.84 mg, 0.152 mmol), and DIPEA (979.18 mg, 7.591 mmol) were mixed in acetonitrile (10 mL) under a nitrogen atmosphere, and the mixture was stirred at 20 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 169-4 (600 mg, 1.300 mmol, yield: 85.66%), ES-API: [M+1]⁺= 461.1.

Step 5: Compound 64-1 (198.26 mg, 0.282 mmol), compound 169-4 (100 mg, 0.217 mmol), Pd(dtbpf)Cl₂ (14.11 mg, 0.022 mmol), potassium phosphate (137.84 mg, 0.650 mmol), water (1 mL), and toluene (1 mL) were mixed in dioxane (3 mL) under a nitrogen atmosphere, and the mixture was stirred at 65 °C for 18 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 169-5 (80 mg, 0.083 mmol, yield: 38.52%), ES-API: [M+1]⁺ = 958.3.

Step 6: Compound 169-5 (80 mg, 0.083 mmol), iodoethane (325.55 mg, 2.087 mmol), and cesium carbonate (680.45 mg, 2.087 mmol) were mixed in N,N-dimethylformamide (5 mL) under a nitrogen atmosphere, and the mixture was stirred at 20 °C for 6 h. After the reaction was completed, 30 mL of ethyl acetate was poured into the mixture, and the resulting mixture was washed sequentially with 15 mL of water and 15 mL of saturated brine. The organic phase was dried over anhydrous sodium sulfate, filtered, and concentrated, and the residue was purified by a flash silica gel column (0-80% petroleum ether/ethyl acetate) to give compound 169-6 (66 mg, 0.067 mmol, yield: 80.15%), ES-API: [M+H]⁺ = 986.5.

Step 7: Compound 169-6 (40 mg, 0.041 mmol) and trifluoroacetic acid (3 mL, 0.041 mmol) were mixed in dichloromethane (9 mL), and the mixture was stirred at 20 °C for 1 h. After the reaction was completed, the mixture was concentrated to dryness by rotary evaporation, and the residue was extracted with a mixed solution (dichloromethane/isopropanol = 5/1) and water. The organic phase was washed sequentially with 15 mL of water and 15 mL of saturated brine, dried over anhydrous sodium sulfate, filtered, and concentrated. The crude product was purified by prep-HPLC (trifluoroacetic acid method) to give 2 isomeric compounds. The structure of one of the isomeric compounds was (1R,5S,6r)-N-((6³S,4S,Z)-(Sₐ)-1²-(5-(3-(4-cyanopiperidin-1-yl)prop-1-yn-1-yl)-2-((S)-1-(methylamino)ethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z169-2, 2 mg, yield: 5.6%, purity: 98%, retention time = 4.389 min, trifluoroacetate). ES-API[M+H]+=886.4. The structure of the other isomeric compound was: (1R,5S,6r)-N-((6³S,4S,Z)-(Rₐ)-1²-(5-(3-(4-cyanopiperidin-1-yl)prop-1-yn-1-yl)-2-((S)-1-(methylamino)ethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-3-oxabicyclo[3.1.0]hexane-6-carboxamide (Z169-1, 2 mg, yield: 5.6%, purity: 98%, retention time = 4.477 min, trifluoroacetate). ES-API[M+H]⁺=886.4.

### Example 1.57. Synthesis of Compound Z170

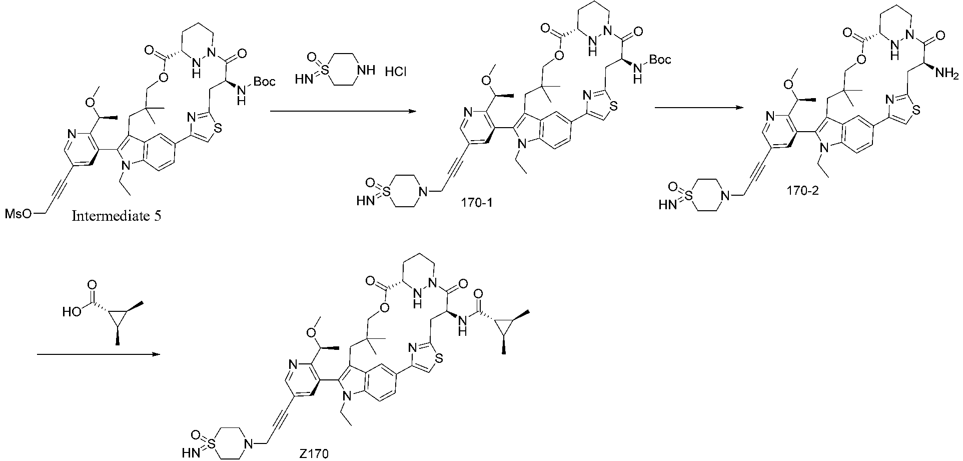

Step 1: Intermediate 5 (45 mg, 0.052 mmol) was dissolved in acetonitrile (10 mL). 1-Imino-1λ6-1,4-thiazin-1-one (CAS: 1633667-60-3, 13.99 mg, 0.104 mmol), N,N'-diisopropylethylamine (26.96 mg, 0.209 mmol), and sodium iodide (7.82 mg, 0.052 mmol) were added. The mixture was reacted at 60 °C for 2 h under a nitrogen atmosphere. After the reaction was completed, the mixture was filtered under vacuum and concentrated to dryness under reduced pressure, and the residue was purified by a flash silica gel column (methanol/dichloromethane: 0%-10%) to give compound 170-1 (38 mg, 0.042 mmol, yield: 80.87%) as a white solid, ES-API: [M+H]⁺ = 901.3.

Step 2: Compound 170-1 (30 mg, 0.033 mmol) was dissolved in methanol (0.5 mL), and 4 M hydrochloric acid/dioxane (1.5 mL) was added. The mixture was reacted at room temperature for 2 h under a nitrogen atmosphere. After the reaction was completed, the mixture was concentrated under reduced pressure to give compound 170-2 (26 mg, 0.032 mmol, yield: 97.49%) as a white solid, ES-API: [M+H]⁺ = 801.3.

Step 3: Compound 170-2 (0.25 g, 0.421 mmol) was dissolved in dichloromethane (2 mL), and (1r,2R,3S)-2,3-dimethylcyclopropane-1-carboxylic acid (3.42 mg, 0.030 mmol), O-(7-azabenzotriazol-1-yl)-N,N,N',N'-tetramethyluronium hexafluorophosphate (14.22 mg, 0.037 mmol), and N,N'-diisopropylethylamine (92.75 mg, 0.718 mmol) were added to the solution described above. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by prep-HPLC (ammonium bicarbonate method 1) to give *(1R,2R,3S)-N-*((6³*S,*4*S,Z*)-1¹-ethyl-(*Rₐ*)-1²-(5-(3-(1-imino-1-oxido-1λ6-thiomorpholino)prop-1-yn-1-yl)-2-((*S*)-1-methoxyethyl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-(hydroxymethyl)-3-methylcyclopropane-1-carboxamide (Z170, 2.4 mg, 0.003 mmol, yield: 10.71%) as a white solid, ES-API: [M+H]⁺ = 897.3.

### Example 1.58. Synthesis of Compound Z171

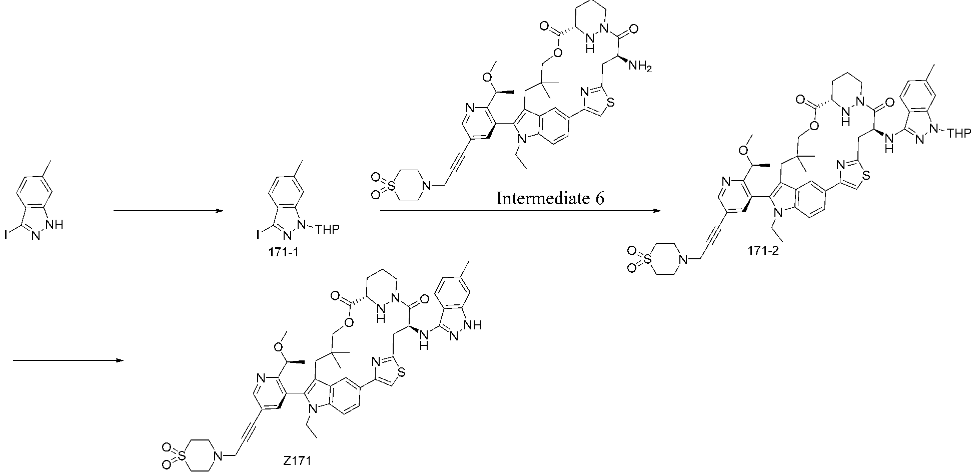

Step 1: DHP (0.424 mL, 4.650 mmol) and TsOH (26.69 mg, 0.155 mmol) were added to a solution of 3-iodo-6-methyl-1H-indazole (400 mg, 1.550 mmol) in dichloromethane (10 mL). The solution was stirred for 1 h, and HPLC analysis showed complete conversion. The solution was further diluted with dichloromethane, washed with a saturated sodium bicarbonate solution, and evaporated. The residue was purified by flash chromatography using 10% ethyl acetate/hexane for elution to give compound 171-1 (520 mg, 1.520 mmol, yield: 98.04%) as a white solid. ES-API: [M+H]⁺= 343.0.

Step 2: Potassium carbonate (120 mg, 0.873 mmol), copper(I) iodide (12 mg, 0.062 mmol), and pyrrole-2-carboxylic acid (6.93 mg, 0.062 mmol) were sequentially added to a solution of intermediate 6 (100 mg, 0.13 mmol) and compound 171-1 (85.33 mg, 0.391 mmol) in dimethyl sulfoxide (2 mL). The reaction mixture was heated to 100 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried over anhydrous sodium sulfate, filtered, and concentrated. The crude product was purified by prep-HPLC (formic acid method) to give compound 171-2 (70 mg, yield: 55.24%) as a white solid. ES-API: [M+H]⁺= 1016.3.

Step 3: Trifluoroacetic acid (2 mL, 26.118 mmol) was added to a solution of compound 171-2 (70 mg, 0.069 mmol) in dichloromethane (2 mL), and the reaction mixture was stirred at room temperature for 1 h. The reaction mixture was concentrated under vacuum, and the residue was purified by prep-HPLC (formic acid method) to give (6³S,4S,Z)-(Rₐ)-1²-(5-(3-(1,1-dioxidothiomorpholino)prop-1-yn-1-yl)-2-((*S*)-1-methoxyethyl)pyridin-3-yl)-1¹-ethyl-10,10-dimethyl-4-((6-methyl-1H-indazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z171, 10 mg, 0.011 mmol, yield: 15.57%) as a white solid. ES-API: [M+H]⁺= 932.4.

### Example 1.59. Synthesis of Compounds Z172-1 and Z172-2

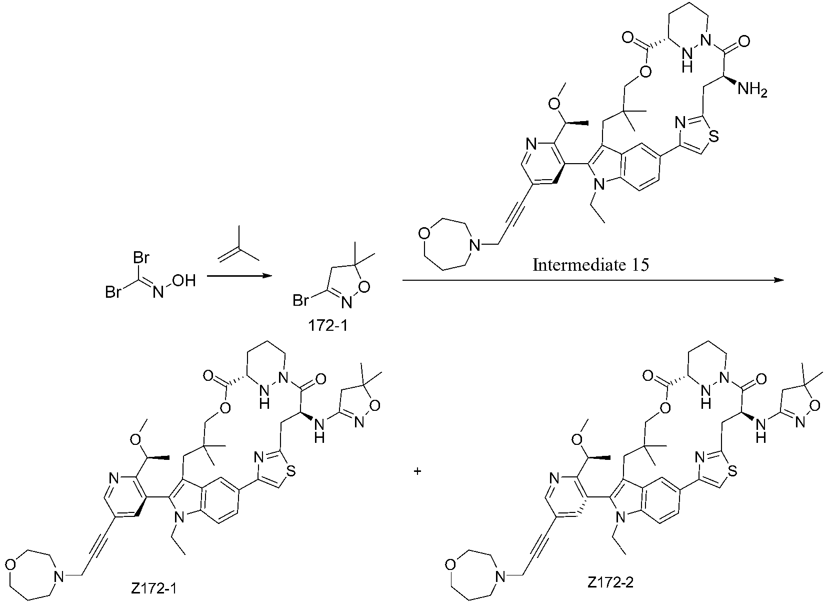

Step 1: A solution of 1,1-dibromoformaldoxime (10 g, 49.3 mmol) in ethyl acetate (20 mL) was slowly added dropwise to a mixed solution of sodium bicarbonate (12.43 g, 147.91 mmol) dissolved in 20 mL of water and 2-methylprop-1-ene (8.3 g, 59.163 mmol) dissolved in ethyl acetate (80 mL) under an ice-water bath. The reaction mixture was slowly warmed to room temperature and stirred overnight. The organic phase was separated, dried over anhydrous sodium sulfate, filtered, and concentrated. The crude product was distilled under reduced pressure to give compound 172-1 (6 g, yield: 68.36%). ES-API: [M+H]⁺= 178.1/180.1.

Step 2: Potassium carbonate (54 mg, 0.391 mmol), copper(I) iodide (24.80 mg, 0.13 mmol), and pyrrole-2-carboxylic acid (14.47 mg, 0.13 mmol) were sequentially added to a solution of intermediate 15 (100 mg, 0.13 mmol) and compound 172-1 (70 mg, 0.391 mmol) in dimethyl sulfoxide (2 mL). The reaction mixture was heated to 90 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried over anhydrous sodium sulfate, filtered, and concentrated. The crude product was purified by prep-HPLC (formic acid method) to give 2 isomeric compounds. The structure of one of the isomeric compounds was (6³S,4S,Z)-(Rₐ)-1²-(5-(3-(1,4-oxazepan-4-yl)prop-1-yn-1-yl)-2-((5)-1-methoxyethyl)pyridin-3-yl)-1¹-ethyl-10,10-dimethyl-4-((5,5-dimethyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z172-1, 2.2 mg, yield: 5.67%, retention time: 5.425 min) as a white solid, ES-API: [M+H]⁺ = 865.4. The structure of the other isomeric compound was (6³S,4S,Z)-(Sₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5,5-dimethyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z172-2, 1.6 mg, yield: 1.42%, retention time: 5.467 min) as a white solid. ES-API: [M+H]⁺= 865.4.

### Example 1.60. Synthesis of Compounds Z173, Z173-1, and Z173-2

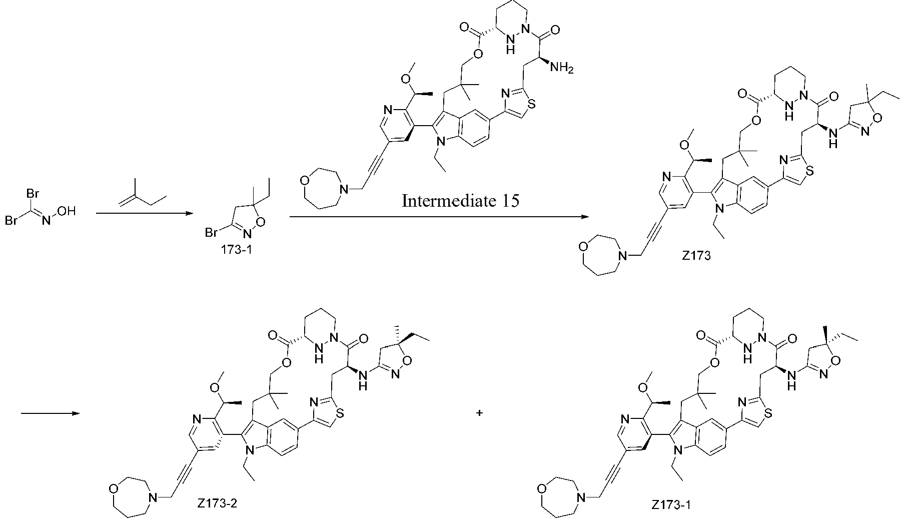

Step 1: A solution of 1,1-dibromoformaldoxime (9.4 g, 46.344 mmol) in ethyl acetate (20 mL) was slowly added dropwise to a mixed solution of sodium bicarbonate (11.68 g, 139 mmol) dissolved in 20 mL of water and 2,3-dimethylbut-1-ene (10 mL, 92.672 mmol) dissolved in ethyl acetate (80 mL) under an ice-water bath. The reaction mixture was slowly warmed to room temperature and stirred overnight. The organic phase was separated, dried over anhydrous sodium sulfate, filtered, and concentrated. The crude product was distilled under reduced pressure to give compound 173-1 (5 g, yield: 56.17%). ES-API: [M+H]⁺= 192.1/194.1.

Step 2: Potassium carbonate (72 mg, 0.52 mmol), copper(I) iodide (74.28 mg, 0.39 mmol), and pyrrole-2-carboxylic acid (43 mg, 0.39 mmol) were sequentially added to a solution of intermediate 15 (100 mg, 0.13 mmol) and compound 173-1 (125 mg, 0.650 mmol) in dimethyl sulfoxide (2 mL). The reaction mixture was heated to 90 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried and concentrated. The crude product was purified by prep-HPLC (formic acid method) to give (6³S,4S,Z)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5-ethyl-5-methyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z173, 4.36 mg, yield: 3.81%) as a white solid. ES-API: [M+H]⁺= 879.4.

Step 3: Compound Z173 (3 mg) was purified by chiral HPLC (IB column, 250 mm × 4.6 mm × 5 µm, mobile phase: n-hexane:EtOH:DEA = 40:60:2; flow rate: 1 mL/min, T = 30 °C) to give 2 isomeric compounds. The structure of one of the isomeric compounds was (6³S,4S,Z)-(Sₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5-ethyl-5-methyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z173-2, 0.5 mg, yield: 16.7%, retention time: 5.568 min) as a white solid. The structure of the other isomeric compound was (6³S,4S,Z)-(Rₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5-ethyl-5-methyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z173-1, 1 mg, yield: 33.3%, retention time: 9.376 min) as a white solid.

### Example 1.61. Synthesis of Compound Z174

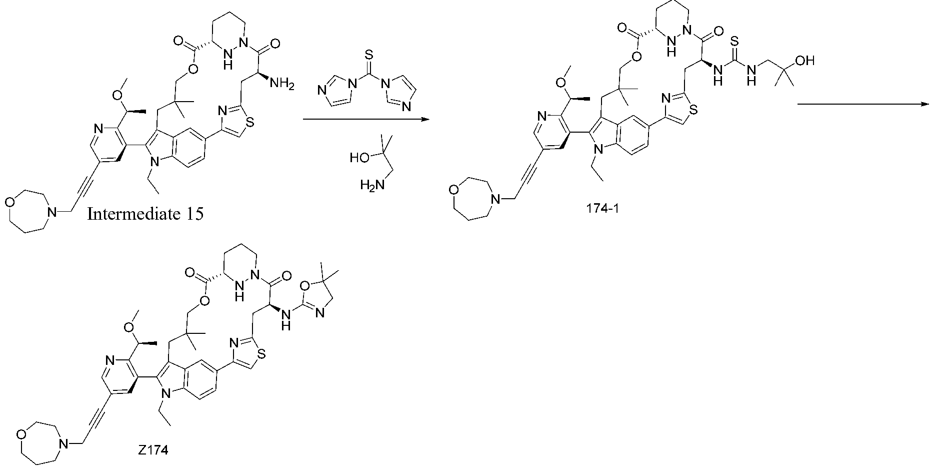

Step 1: Intermediate 15 (100 mg, 0.130 mmol), N,N'-thiocarbonyldiimidazole (34.81 mg, 0.195 mmol), and acetonitrile (4 mL) were added to a flask at 0 °C. The reaction system was stirred at room temperature for 30 min. A solution of 1-amino-2-methylpropan-2-ol (34.82 mg, 0.391 mmol) in acetonitrile (1 mL) was added to the reaction mixture. The reaction system was stirred at 50 °C for 60 min. Water (50 mL) was added to the reaction mixture, and the mixture was extracted with ethyl acetate (50 mL × 3). The organic phase was dried and concentrated. The crude product was purified by a flash silica gel column (6% methanol/dichloromethane) to give compound 174-1 (90 mg, yield: 77%). ES-API: [M+H]⁺=899.2.

Step 2: Compound 174-1 (80 mg, 0.089 mmol), N,N'-diisopropylcarbodiimide (33.68 mg, 0.267 mmol), and N,N-dimethylformamide (3 mL) were added to a flask. The reaction system was stirred under an oil bath at 105 °C for 16 h under a nitrogen atmosphere. Water (50 mL) was added to the reaction mixture, and the mixture was extracted with ethyl acetate (50 mL). The organic phase was dried over anhydrous sodium sulfate, filtered, and concentrated. The crude product was purified by prep-HPLC (formic acid method) to give (6³S,4S,Z)-1²-(5-(3-(1,4-oxazepan-4-yl)prop-1-yn-1-yl)-2-((S)-1-methoxyethyl)pyridin-3-yl)-4-((5,5-dimethyl-4,5-dihydrooxazol-2-yl)amino)-1¹-ethyl-10,10-dimethyl-6¹,6²,6³,6⁴,6⁵,6⁶-hexahydro-1¹H-8-oxa-2(4,2)-thiazole-1(5,3)-indole-6(1,3)-pyridazinacycloundecaphane-5,7-dione (Z174, 5.12 mg, yield: 6.3%). ES-API: [M+H]⁺=865.3.

### Example 1.62. Synthesis of Compound Z175

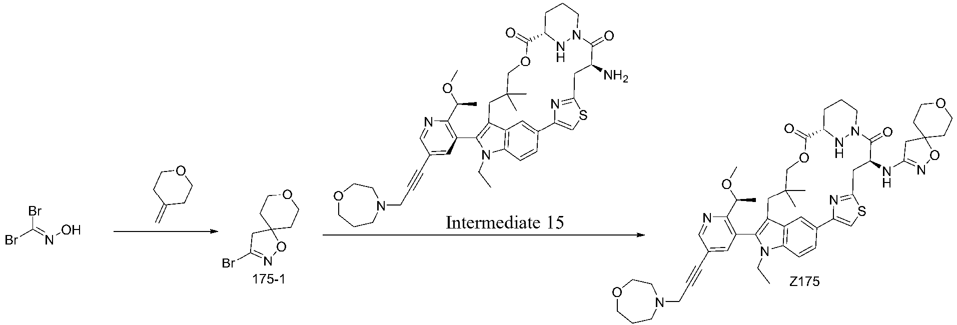

Step 1: A solution of 1,1-dibromoformaldoxime (827 mg, 4.076 mmol) in ethyl acetate (5 mL) was slowly added dropwise to a solution of 4-methylenetetrahydro-2H-pyran (800 mg, 8.152 mmol) and sodium bicarbonate (1027 mg, 12.228 mmol) in ethyl acetate (30 mL) and water (5 mL) under an ice-water bath. The reaction mixture was slowly warmed to room temperature and stirred overnight. The organic phase was separated, dried over anhydrous sodium sulfate, filtered, and concentrated to give crude compound 175-1 (850 mg, yield: 95%). ES-API: [M+H]⁺= 220.1.

Step 2: Potassium carbonate (72 mg, 0.521 mmol), copper(I) iodide (5.0 mg, 0.026 mmol), and pyrrole-2-carboxylic acid (3.0 mg, 0.026 mmol) were sequentially added to a solution of intermediate 15 (100 mg, 0.130 mmol) and compound 175-1 (86 mg, 0.391 mmol) in dimethyl sulfoxide (2 mL). The reaction mixture was heated to 90 °C and stirred overnight. A product was generated as detected by LCMS. Insoluble substances were removed by filtration, and the filtrate was purified by HPLC (trifluoroacetic acid method) to give (6³S,4S,Z)-1²-(5-(3-(1,4-oxazepan-4-yl)prop-1-yn-1-yl)-2-((S)-1-methoxyethyl)pyridin-3-yl)-4-((1,8-dioxa-2-azaspiro[4.5]dec-2-en-3-yl)amino)-1¹-ethyl-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z175, 1.49 mg, yield: 1.26%) as a white solid. ES-API: [M+H]⁺= 907.2.

### Example 1.63. Synthesis of Compound Z176

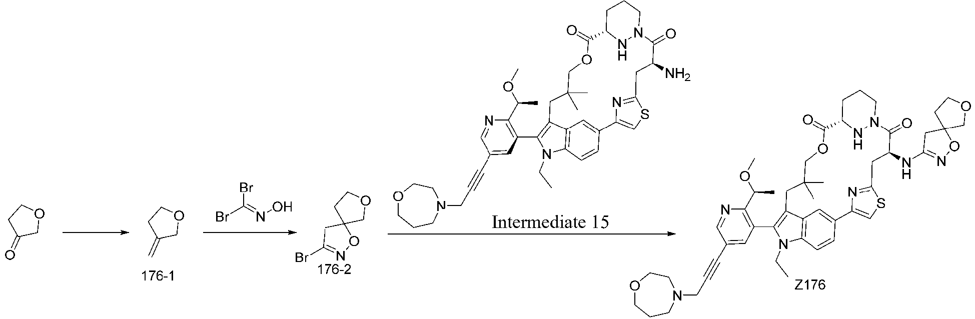

Step 1: Dihydro-3(2H)-furanone (5 g, 58.079 mmol) was added to a solution of methyltriphenylphosphonium bromide (41.49 g, 116.158 mmol) and potassium tert-butoxide (13.03 g, 116.158 mmol) in tetrahydrofuran (500 mL) at room temperature. The mixture was stirred overnight at room temperature. The reaction mixture was distilled at 80 °C under normal pressure to give compound 176-1 (3.1 g, yield: 63%). ¹H NMR (400 MHz, CDCl3) : 4.94-4.87 (m, 2 H), 4.18 (s, 2 H), 3.83 (t, J= 6.8 Hz, 2 H), 2.48 (t, J= 6.4 Hz, 2 H).

Step 2: A solution of 1,1-dibromoformaldoxime (800 mg, 3.944 mmol) in ethyl acetate (5 mL) was slowly added dropwise to a solution of compound 176-1 (664 mg, 7.888 mmol) and sodium bicarbonate (994 mg, 11.833 mmol) in ethyl acetate (30 mL) and water (5 mL) under an ice-water bath. The reaction mixture was slowly warmed to room temperature and stirred overnight. The organic phase was separated, dried over anhydrous sodium sulfate, filtered, and concentrated. The residue was purified by automated flash chromatography on silica gel (ethyl acetate:petroleum ether = 0-20%) to give crude compound 176-2 (60 mg, yield: 7%). ES-API: [M+H]⁺= 206.0.

Step 3: Potassium carbonate (50 mg, 0.365 mmol), copper(I) iodide (17 mg, 0.091 mmol), and pyrrole-2-carboxylic acid (2 mg, 0.018 mmol) were sequentially added to a solution of intermediate 15 (70 mg, 0.091 mmol) and compound 176-2 (56 mg, 0.273 mmol) in dimethyl sulfoxide (2 mL). The reaction mixture was heated to 90 °C and stirred overnight. A product was generated as detected by LCMS. Insoluble substances were removed by filtration, and the filtrate was purified by HPLC (trifluoroacetic acid method) to give (6³S,4S,Z)-1²-(5-(3-(1,4-oxazepan-4-yl)prop-1-yn-1-yl)-2-((S)-1-methoxyethyl)pyridin-3-yl)-4-((1,7-dioxa-2-azaspiro[4.4]non-2-en-3-yl)amino)-1¹-ethyl-10,10-dimethyl-6¹,6²,6³,6^{4,}6⁵,6⁶-hexahydro-1¹H-8-oxa-2(4,2)-thiazola-1(5,3)-indola-6(1,3)-pyridazinacycloundecaphane-5,7-dione (Z176, 1.27 mg, yield: 1.56%) as a white solid. ES-API: [M+H]⁺= 893.2.

### Example 1.64. Synthesis of Compound Z279

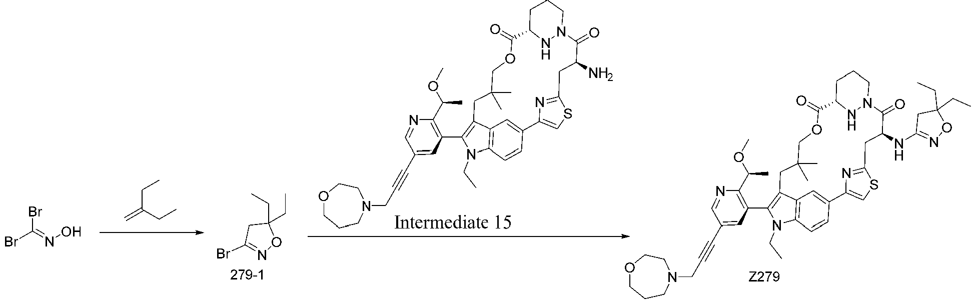

Step 1: A solution of 1,1-dibromoformaldoxime (6 g, 29.581 mmol) in ethyl acetate (20 mL) was slowly added dropwise to a mixed solution of sodium bicarbonate (7.46 g, 88.744 mmol) dissolved in 20 mL of water and 2,3-dimethylbut-1-ene (7.227 mL, 59.163 mmol) dissolved in ethyl acetate (80 mL) under an ice-water bath. The reaction mixture was slowly warmed to room temperature and stirred overnight. The organic phase was separated, dried over anhydrous sodium sulfate, filtered, and concentrated. The crude product was distilled under reduced pressure to give compound 279-1 (3.5 g, yield: 57.41%). ES-API: [M+H]⁺= 206.1/208.1.

Step 2: Potassium carbonate (86.38 mg, 0.625 mmol), copper(I) iodide (89.27, 0.469 mmol), and pyrrole-2-carboxylic acid (52.08 mg, 0.469 mmol) were sequentially added to a solution of intermediate 15 (120 mg, 0.156 mmol) and compound 279-1 (161 mg, 0.780 mmol) in dimethyl sulfoxide (2 mL). The reaction mixture was heated to 80 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried over anhydrous sodium sulfate, filtered, and concentrated. The crude product was purified by prep-HPLC (formic acid method) to give (6³S,4S,Z)-(Rₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5,5-diethyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z279, 1.7 mg, yield: 1.22%) as a white solid. ES-API: [M+H]⁺= 893.3.

### Example 1.65. Synthesis of Compound Z280

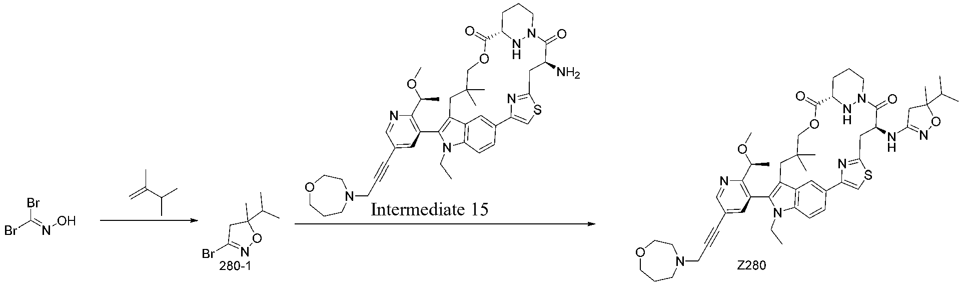

Step 1: A solution of 1,1-dibromoformaldoxime (8 g, 39.44 mmol) in ethyl acetate (20 mL) was slowly added dropwise to a mixed solution of sodium bicarbonate (6.63 g, 78.884 mmol) dissolved in 20 mL of water and 2,3-dimethylbut-1-ene (9.763 mL, 78.884 mmol) dissolved in ethyl acetate (80 mL) under an ice-water bath. The reaction mixture was slowly warmed to room temperature and stirred overnight. The organic phase was separated, dried over anhydrous sodium sulfate, filtered, and concentrated. The crude product was distilled under reduced pressure to give compound 280-1 (6.3 g, yield: 77.51%). ES-API: [M+H]⁺= 206.1/208.1.

Step 2: Potassium carbonate (180 mg, 1.3 mmol), copper(I) iodide (248 mg, 1.3 mmol), and pyrrole-2-carboxylic acid (145 mg, 1.3 mmol) were sequentially added to a solution of intermediate 15 (200 mg, 0.260 mmol) and compound 280-1 (268 mg, 1.3 mmol) in dimethyl sulfoxide (2 mL). The reaction mixture was heated to 80 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried and concentrated. The crude product was purified by prep-HPLC (formic acid method) to give (6³S,4S,Z)-(Rₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5-isopropyl-5-methyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z280, 2.96 mg, yield: 1.27%) as a white solid. ES-API: [M+H]⁺= 893.3.

### Example 1.66. Synthesis of Compound Z281

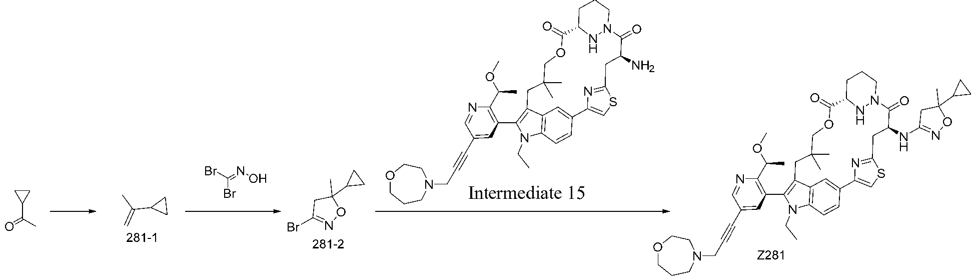

Step 1: Methyl(triphenyl)phosphonium bromide (25.5 g, 71.33 mmol) was dissolved in tetrahydrofuran (80 mL). The mixture was cooled to -78 °C, and n-BuLi (2.5 M, 28.5 mL, 71.33 mmol) was added dropwise under a nitrogen atmosphere. After the addition, the mixture was stirred at 0 °C for 0.5 h under a nitrogen atmosphere. The mixture was cooled to -78 °C again, and a solution of 1-cyclopropylethan-1-one (5 g, 59.44 mmol) dissolved in tetrahydrofuran (20 mL) was slowly added. The mixture was stirred at room temperature for 16 h under a nitrogen atmosphere. The reaction mixture was diluted with water (100 mL) and extracted with ethyl acetate (100 mL × 3). The combined organic layers were dried over sodium sulfate and filtered, and the filtrate was evaporated to give a mixture of compound 281-1 (400 mL), which was used in the next step without further purification.

Step 2: Under a nitrogen atmosphere, sodium bicarbonate (10 g, 121.75 mmol) was added to the mixture of compound 281-1 (150 mL) obtained in the previous step, and then 1,1-dibromoformaldoxime (5 g, 24.35 mmol) was slowly added dropwise under an ice-water bath. The resulting mixture was stirred at room temperature for 16 h, and then the reaction was quenched by the addition of water (200 mL). The resulting mixture was extracted with ethyl acetate (200 mL × 3). The combined organic layers were washed with brine (200 mL), dried over anhydrous sodium sulfate, filtered, and concentrated. The residue was purified by flash chromatography (petroleum ether = 100%) to give compound 281-2 (1.1 g, 5.42 mmol, yield: 22%) as a colorless liquid. LCMS: [M+H]⁺=204.1/206.1. ¹H NMR (400 MHz, DMSO-*d₆*) δ 3.14 - 3.02 (m, 2H), 1.33 (s, 3H), 1.12 (tt, *J =* 8.4, 5.4 Hz, 1H), 0.46 - 0.20 (m, 4H).

Step 3: Potassium carbonate (90 mg, 0.65 mmol), copper(I) iodide (74 mg, 0.39 mmol), and pyrrole-2-carboxylic acid (43 mg, 0.39 mmol) were sequentially added to a solution of intermediate 15 (100 mg, 0.130 mmol) and compound 281-2 (106 mg, 0.521 mmol) in dimethyl sulfoxide (2 mL). The reaction mixture was heated to 80 °C and stirred overnight under a nitrogen atmosphere. The reaction mixture was cooled to room temperature, and water (10 mL) was added. The resulting mixture was filtered, and the filtrate was extracted with ethyl acetate (10 mL × 3). The organic phase was dried over anhydrous sodium sulfate, filtered, and concentrated. The crude product was purified by prep-HPLC (formic acid method) to give (6³S,4S,Z)-(Rₐ)-1²-(5-(3-(1,4-oxazepan-4-yl)prop-1-yn-1-yl)-2-((*S*)-1-methoxyethyl)pyridin-3-yl)-1¹-ethyl-10,10-dimethyl-4-((5-cyclopropyl-5-methyl-4,5-dihydroisoxazol-3-yl)amino)-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (Z281, 1.45 mg, yield: 1.25%) as a white solid. ES-API: [M+H]⁺= 891.3

### Example 1.67. Synthesis of Compound Z155

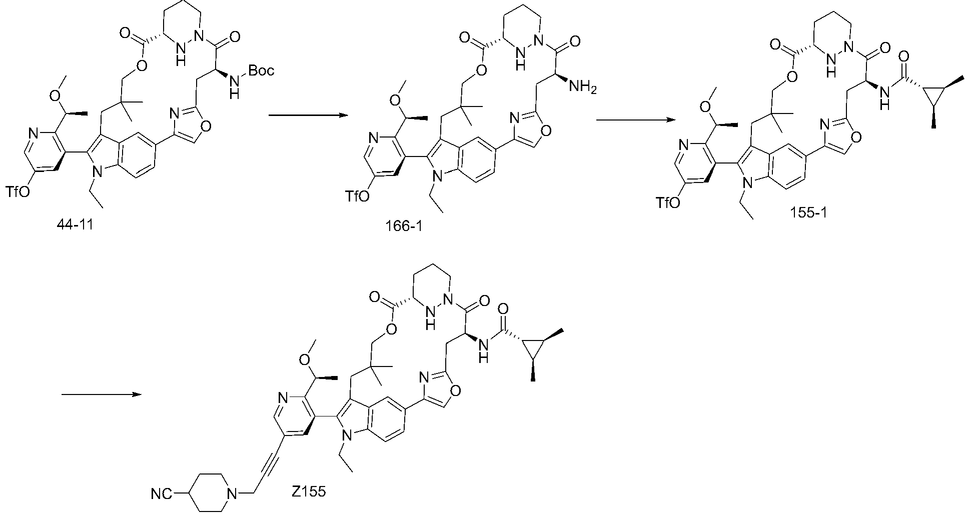

Step 1: Compound 44-11 (200 mg, 0.232 mmol) was dissolved in methanol (1 mL), and a solution of hydrochloric acid in dioxane (3 mL, 4 M) was added dropwise under an ice-water bath. The mixture was reacted at room temperature for 30 min. The reaction mixture was concentrated to give crude compound 166-1 (176.80 mg, 0.232 mmol, yield: 100%). ES-API: [M+1]⁺= 763.2.

Step 2: Compound 166-1 (176 mg, 0.231 mmol) was dissolved in dichloromethane (5 mL), and (1r,2R,3S)-2,3-dimethylcyclopropane-1-carboxylic acid (52.67 mg, 0.461 mmol), O-(7-azabenzotriazol-1-yl)-*N,N,N',N'-*tetramethyluronium hexafluorophosphate (175.46 mg, 0.461 mmol), and N,N'-diisopropylethylamine (59.64 mg, 0.461 mmol) were added. The reaction system was stirred at room temperature for 2 h. After the reaction was completed, the reaction mixture was concentrated. Water (5.0 mL) was added to the residue, and the mixture was extracted with dichloromethane (10.0 mL × 2). The organic phase was washed with saturated brine (5.0 mL × 3), dried, and concentrated. The crude product was purified by a flash silica gel column (methanol/dichloromethane: 0-10%) to give compound 155-1 (190 mg, 0.221 mmol, yield: 95.87%). ES-API: [M+1]⁺=859.2.

Step 3: Compound 155-1 (90 mg, 0.105 mmol), 1-(prop-2-yn-1-yl)piperidine-4-carbonitrile (46.59 mg, 0.314 mmol), copper(I) iodide (7.98 mg, 0.042 mmol), lithium chloride (44.42 mg, 1.048 mmol), *N,N'-*diisopropylethylamine (0.055 mL, 0.314 mmol), and bis(triphenylphosphine)palladium(II) dichloride (14.71 mg, 0.021 mmol) were dissolved in acetonitrile (5 mL). The mixture was reacted at 80 °C for 2 h under a nitrogen atmosphere. The reaction mixture was filtered to give a filtrate, and the filtrate was concentrated to give a crude product. The crude product was purified by prep-HPLC (ammonium bicarbonate method 2) to give compound (1r,2R,3S)-N-((6³S,4S,Z)-*(Rₐ)*²-(5-(3-(4-cyanopiperidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphan-4-yl)-2,3-dimethylcyclopropanecarboxamide (Z155, 33 mg, 0.039 mmol, yield: 36.74%). ES-API: [M+1]⁺=857.3. ¹H NMR (400 MHz, DMSO-*d₆*) δ 8.81 (d, J = 4.0 Hz, 1H), 8.40 (d, J = 8.0 Hz, 1H), 8.37 (s, 1H), 8.27 (s, 1H), 7.87 (s, 1H), 7.60-7.52 (m, 2H), 5.75 - 5.65 (m, 1H), 5.15-5.07 (m, 1H), 4.37-4.28 (m, 4H), 4.11-4.01 (m, 1H), 3.62-3.52 (m, 3H), 3.20 (s, 3H), , 3.06 - 2.94 (m, 2H), 2.93-2.87 (m, 2H), 2.75 - 2.64 (m, 3H), 2.45-2.33 (m, 2H), 1.97-1.88 (m, 2H), 1.85-1.75 (m, 4H), 1.66 - 1.45 (m, 2H), 1.39-1.33 (m, 3H), 1.27 - 1.11 (m, 4H), 1.07-1.00 (m, 8H), 0.91 (t, J = 8.0 Hz, 3H), 0.86 (s, 2H), 0.36 (s, 3H).

### Example 1.68. Synthesis of Compound Z181

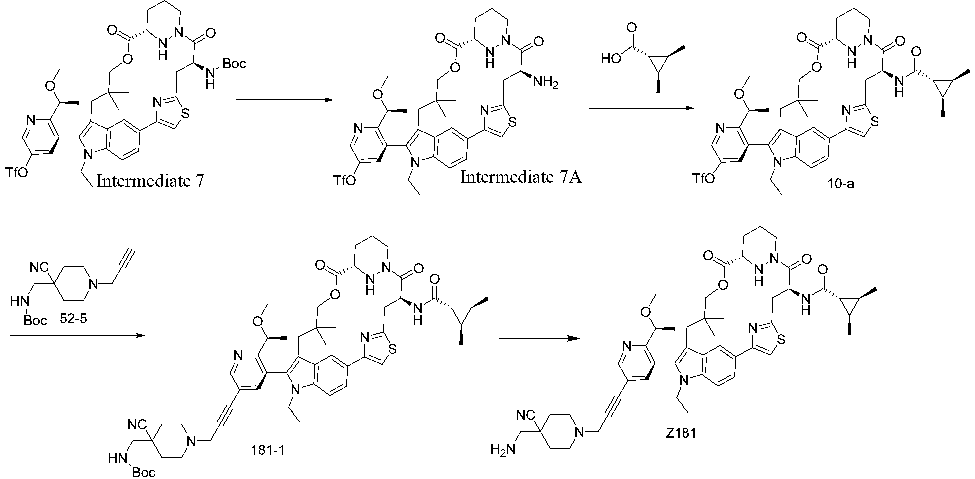

Step 1: Intermediate 7 (1000 mg, 1.138 mmol) was dissolved in methanol (2 mL). A solution of hydrogen chloride in dioxane (10 mL) was added to the solution described above. After the addition was completed, the reaction system was stirred at room temperature for 1 h. After the reaction was completed, the reaction mixture was concentrated to give a hydrochloride. A saturated aqueous sodium bicarbonate solution (20 mL) was added to the hydrochloride described above, and the mixture was extracted with ethyl acetate (20 mL × 2). The organic phase was separated, washed with saturated brine (20 mL × 2), dried, and concentrated to give intermediate 7A (866 mg, 1.112 mmol, yield: 97.73%) as a light yellow solid. ES-API: [M+H]⁺=779.2.

Step 2: Intermediate 7A (354.44 mg, 0.455 mmol) was dissolved in dichloromethane (10 mL). (1r,2R,3S)-2,3-Dimethylcyclopropane-1-carboxylic acid (67.52 mg, 0.592 mmol), O-(7-azabenzotriazol-1-yl)-*N,N,N',N'-*tetramethyluronium hexafluorophosphate (224.95 mg, 0.592 mmol), and N,N'-diisopropylethylamine (0.476 mL, 2.730 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-2%) to give compound 10-a (360 mg, 0.411 mmol, yield: 90.41%). as a light yellow solid. ES-API: [M +H]⁺=875.2.

Step 3: Compound 10-a (360 mg, 0.411 mmol) was dissolved in acetonitrile (20 mL). Compound 52-5 (342.36, 1.234 mmol), bis(triphenylphosphine)palladium(II) dichloride (57.76 mg, 0.082 mmol), copper(I) iodide (31.34 mg, 0.165 mmol), N,N'-diisopropylethylamine (0.287 mL, 1.646 mmol), and lithium chloride (104.64 mg, 2.469 mmol) were added to the solution described above at room temperature. After the addition was completed, the reaction system was stirred at 80 °C for 1.5 h under an argon atmosphere. After the reaction was completed, water (30 mL) was added to the reaction mixture, and the mixture was extracted with ethyl acetate (30 mL × 2). The organic phase was washed with saturated brine (30 mL × 2), dried, and concentrated to give a crude product. The crude product was purified by a flash silica gel column (methanol/dichloromethane: 0%-3%) to give compound 181-1 (390 mg, 0.389 mmol, yield: 94.57%) as a light yellow solid. ES-API: [M +H]⁺=1002.3.

Step 4: Compound 181-1 (390 mg, 0.389 mmol) was dissolved in dichloromethane (4 mL), and trifluoroacetic acid (4 mL) was added to the solution described above. After the addition was completed, the reaction system was stirred at room temperature for 2 h. After the reaction was completed, the reaction mixture was concentrated to remove the solvent. A saturated aqueous sodium bicarbonate solution (20 mL) was added to the residue, and the mixture was extracted with ethyl acetate (20 mL × 2). The organic phase was separated, washed with saturated brine (20 mL × 2), dried, and concentrated to give a crude product. The crude product was purified by prep-HPLC (ammonium bicarbonate method 2) to give (1*r,*2*R,*3*S*)-*N-*((6³*S,*4*S,Z*)-1²-(*R*ₐ)-(5-(3-(4-(aminomethyl)-4-cyanopiperidin-1-yl)prop-1-yn-1-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphan-4-yl)-2,3-dimethylcyclopropane-1-carboxamide (Z181, 148 mg, 0.149 mmol, yield: 42.17%) as a white solid. ES-API: [M +H]⁺=902.3. ¹H NMR (400 MHz, DMSO-*d₆*) δ 8.80 (d, *J=* 2.0 Hz, 1H), 8.50 (s, 1H), 8.41 (d, *J* = 8.8 Hz, 1H), 7.88 - 7.80 (m, 2H), 7.75 (dd, *J* = 8.8, 1.2 Hz, 1H), 7.58 (d, *J* = 8.8 Hz, 1H), 5.56 (t, *J =* 9.2 Hz, 1H), 5.08 (d, *J =* 12.0 Hz, 1H), 4.39 - 4.01 (m, 5H), 3.65 - 3.54 (m, 4H), 3.45 - 3.27 (m, 5H), 3.20 - 3.08 (m, 2H), 3.00 - 2.85 (m, 3H), 2.82 - 2.72 (m, 1H), 2.68 (s, 2H), 2.49 - 2.36 (m, 3H), 2.15 - 2.00 (m, 1H), 1.92 - 1.84 (m, 2H), 1.84 - 1.71 (m, 2H), 1.56 - 1.44 (m, 3H), 1.35 *(d, J=* 6.0 Hz, 3H), 1.21 - 1.13 (m, 3H), 1.11 - 1.03 (m, 6H), 0.94 - 0.81 (m, 6H), 0.34 (s, 3H).

### Example 1.69. Synthesis of Compound Z182

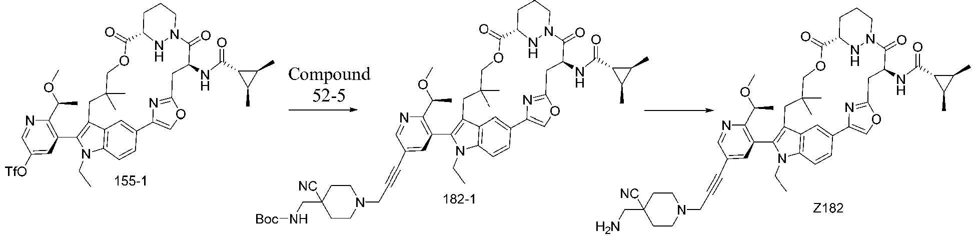

Step 1: Compound 155-1 (100 mg, 0.116 mmol), compound 52-5 (96.88 mg, 0.349 mmol), copper(I) iodide (8.87 mg, 0.047 mmol), lithium chloride (49.35 mg, 1.164 mmol), N,N'-diisopropylethylamine (0.061 mL, 0.349 mmol), and bis(triphenylphosphine)palladium(II) dichloride (16.34 mg, 0.023 mmol) were dissolved in acetonitrile (5 mL). The mixture was reacted at 80 °C for 2 h under a nitrogen atmosphere. The reaction mixture was filtered to give a filtrate, and the filtrate was concentrated to give a crude product. The crude product was purified by a flash silica gel column (methanol/dichloromethane: 0-10%) to give compound 182-1 (83 mg, 0.084 mmol, yield: 72.29%). ES-API: [M+1]⁺= 986.3.

Step 2: Compound 182-1 (83 mg, 0.084 mmol) was dissolved in dichloromethane (1 mL), and trifluoroacetic acid (2 mL) was added under an ice-water bath. The reaction mixture was reacted at room temperature for 30 min and concentrated to give a crude product. The crude product was purified by prep-HPLC (ammonium bicarbonate method 2) to give compound (1r,2R,3S)-N-((6³S,4S,Z)-*(Rₐ)*²-(5-(3-(4-(aminomethyl)-4-cyanopiperidin-1-yl)prop-1-yn-1-yl)-2-(((*S*)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-oxazola-1(5,3)-indola-6(1,3)-pyridazinacycloundecaphan-4-yl)-2,3-dimethylcyclopropane-1-carboxamide (Z182, 43 mg, 0.049 mmol, yield: 57.66%). ES-API: [M+1]⁺= 886.3. ¹H NMR (400 MHz, DMSO-*d₆*) δ 8.81 (d, J = 4.0 Hz, 1H), 8.40 (d, J = 8.0 Hz, 1H), 8.37 (s, 1H), 8.27 (s, 1H), 7.85 (d, J = 8.0 Hz, 1H), 7.60-7.52 (m, 2H), 5.73 - 5.68 (m, 1H), 5.14-5.07 (m, 1H), 4.38-4.23 (m, 4H), 4.12-4.00 (m, 1H), 3.60-3.63 (m, 2H), 3.56 (d, J = 8.0 Hz, 3H), 3.21 (s, 3H), 3.06 - 2.94 (m, 2H), 2.93-2.87 (m, 2H), 2.75 - 2.64 (m, 2H), 2.45-2.33 (m, 2H), 1.97-1.88 (m, 2H), 1.85-1.75 (m, 4H), 1.66 - 1.45 (m, 4H), 1.39-1.33 (m, 3H), 1.27 - 1.11 (m, 4H), 1.07-1.00 (m, 8H), 0.91 (t, J = 8.0 Hz, 3H), 0.86 (s, 2H), 0.36 (s, 3H).

### Example 1.70. Synthesis of Compound Z282

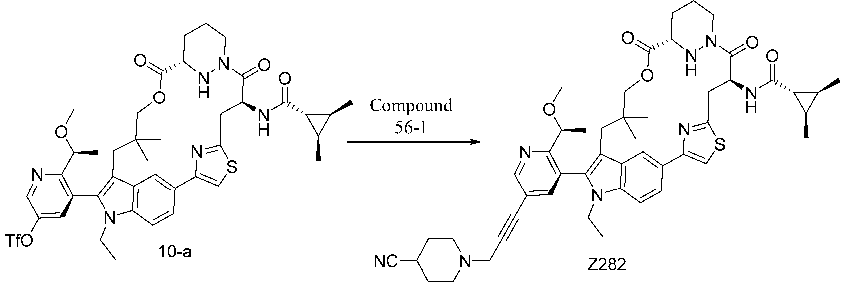

Step 1: Under a nitrogen atmosphere, compound 10-a (300 mg, 0.343 mmol), compound 56-1 (76.22 mg, 0.514 mmol), Pd(PPh₃)₂Cl₂ (48.14 mg, 0.069 mmol), copper(I) iodide (6.51 mg, 0.034 mmol), DIPEA (176.92 mg, 1.371 mmol), and LiCl (57.60 mg, 1.371 mmol) were mixed in anhydrous acetonitrile (10 mL), and the reaction system was stirred at 80 °C for 3 h. After the reaction was completed, the mixture was extracted with ethyl acetate and water. The organic phase was dried over sodium sulfate, concentrated, and purified by prep-HPLC (formic acid method) to give (1r,2R,3S)-N-((6³S,4S,Z)-1²-(5-(3-(4-cyanopiperidin-1-yl)prop-1-yn-1-yl)-(Rₐ)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphan-4-yl)-2,3-dimethylcyclopropane-1-carboxamide (Z282, 100 mg, yield: 33.40%, purity: 98%). ES-API[M+H]⁺=873.3. δ H (400 MHz, DMSO-*d₆*) 8.82 (1 H, s), 8.50 (1 H, s), 8.40 (1 H, d, J 9.0), 7.84 (2 H, d, J 14.0), 7.76 (1 H, d, J 7.4), 7.59 (1 H, d, J 8.7), 5.55 (1 H, t, J 9.1), 5.08 (1 H, d, J 12.2), 4.42 - 4.01 (5 H, m), 3.75-3.5 (4 H, m), 3.30 (1 H, s), 3.25 (3 H, s), 3.18-3.11(1 H, m), 2.96 (2 H, d, J 14.4), 2.80 - 2.65 (3 H, m), 2.54 (1 H, s), 2.46 - 2.32 (2 H, m), 2.07 (1 H, d, J 11.3), 1.95-1.81 (6 H, m), 1.61-1.45 (1 H, m), 1.35 (3 H, d, J 6.0), 1.22-1.11 (3 H,m), 1.07 (6 H, dd, J 9.2, 5.5), 0.88 (6 H, dd, J 13.9, 6.8), 0.34 (3 H, s).

According to the methods described in the above examples, the compounds in Table A-2, Table B-2, or Table C were synthesized by modifying some of the starting materials.

**Table A-2**

| Compound structure Compound No. ([M+H]⁺) | Compound structure Compound No. ([M+H]⁺) | Compound structure Compound No. ([M+H]⁺) |
|---|---|---|
| 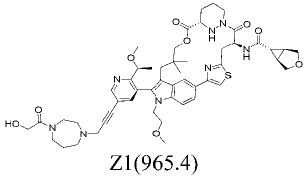 | 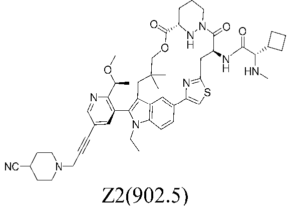 | 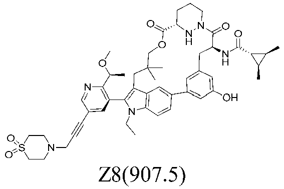 |
| 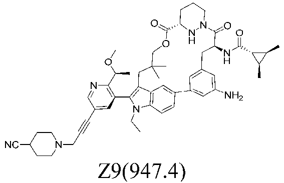 | 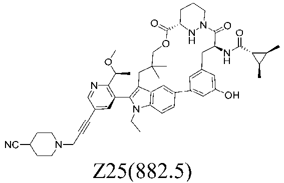 | 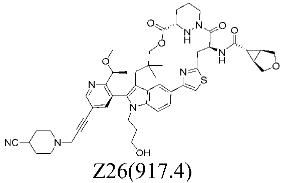 |
| 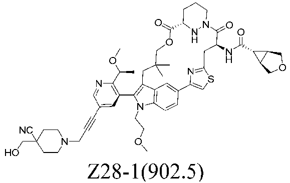 | 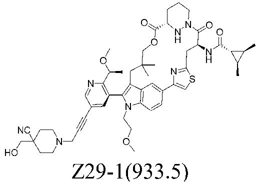 | |
| 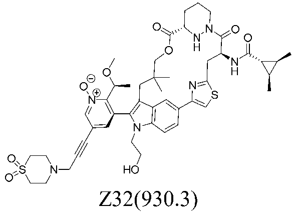 | 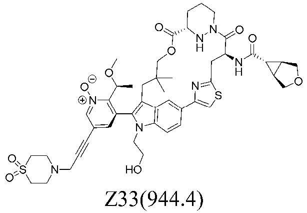 | 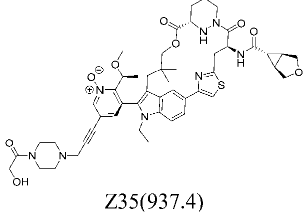 |
| 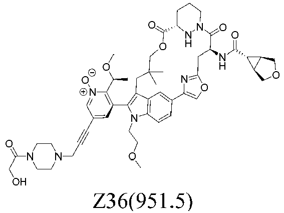 | 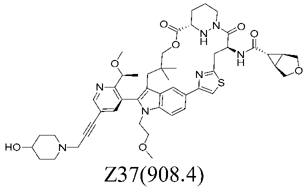 | 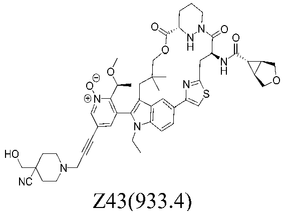 |
| 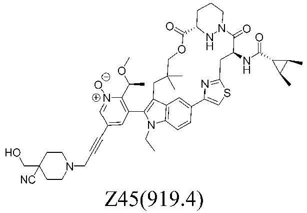 | 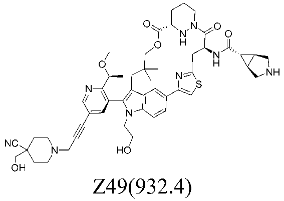 | 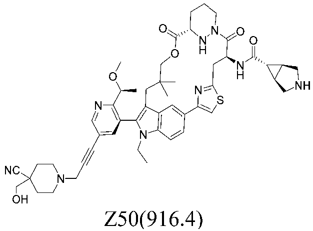 |
| 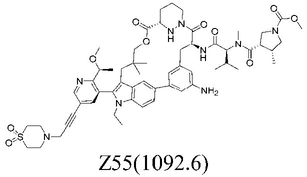 | 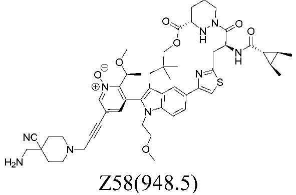 | 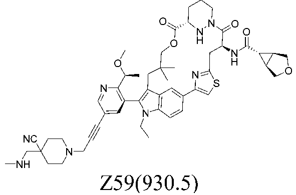 |
| 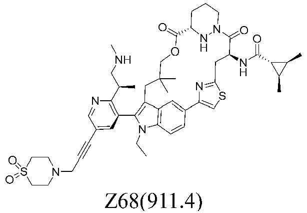 | 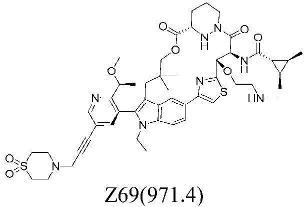 | 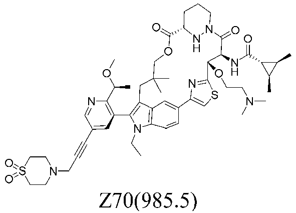 |
| 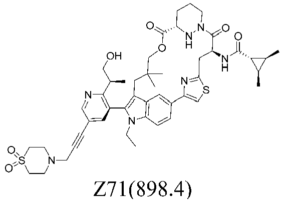 | 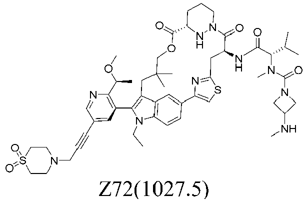 | 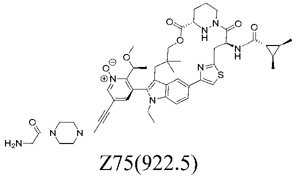 |
| 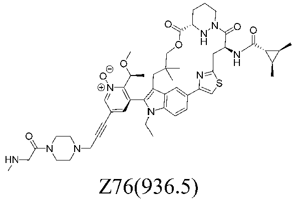 | 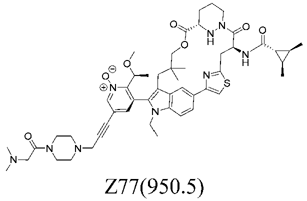 | 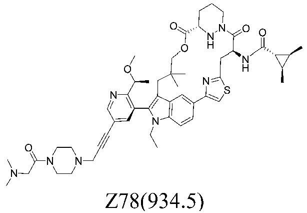 |
| 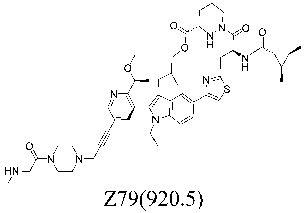 | 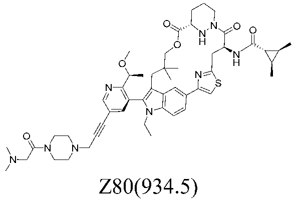 | 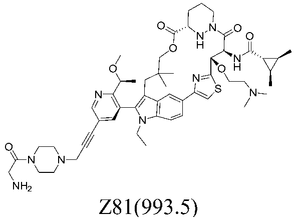 |
| 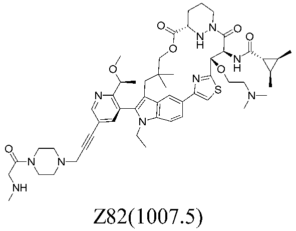 | 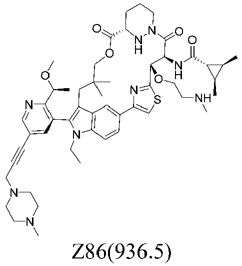 | 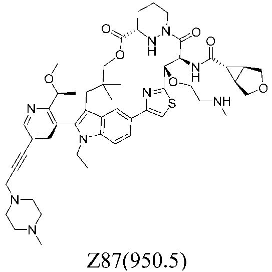 |
| 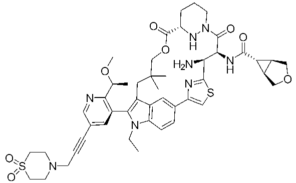 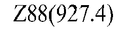 | 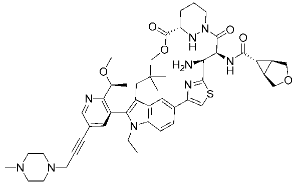 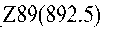 | 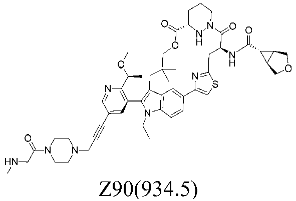 |
| 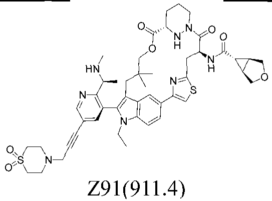 | 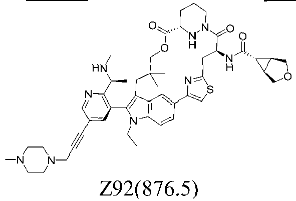 | 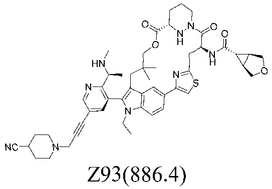 |
| 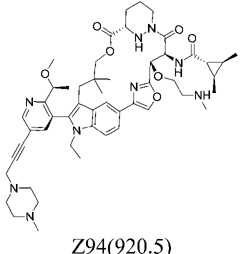 | 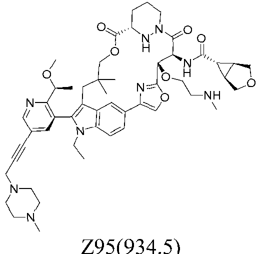 | 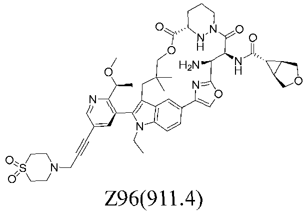 |
| 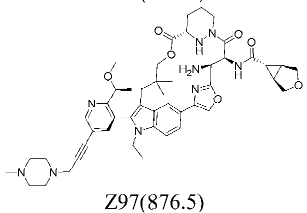 | 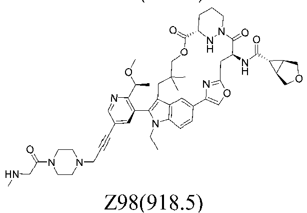 | 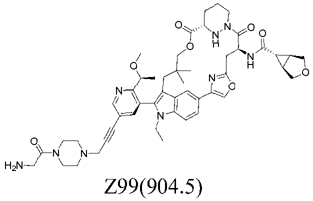 |
| 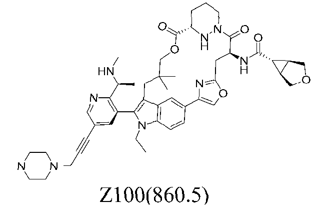 | 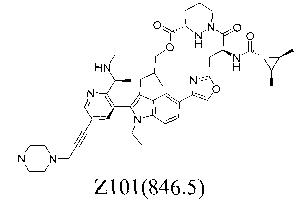 | 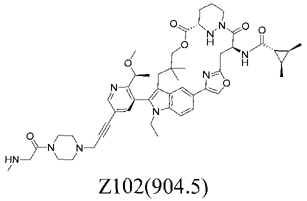 |
| 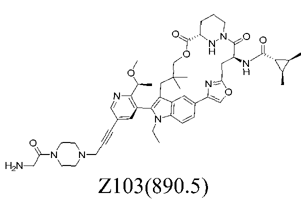 | 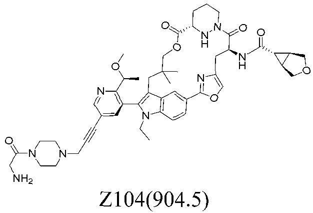 | 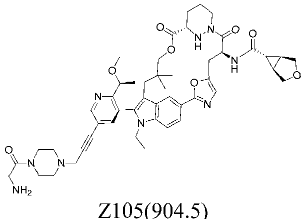 |
| 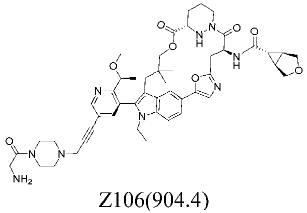 | 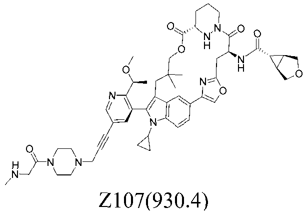 | 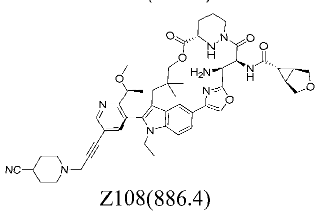 |
| 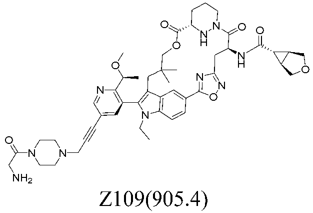 | 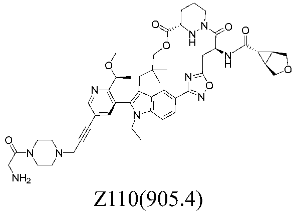 | 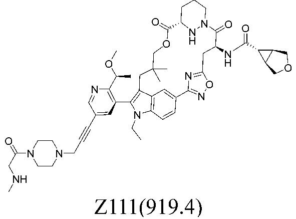 |
| 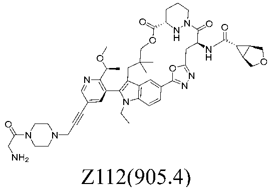 | 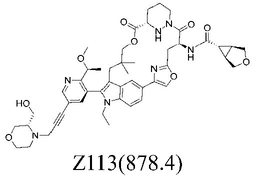 | 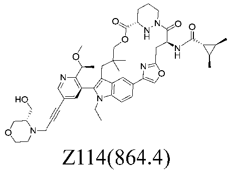 |
| 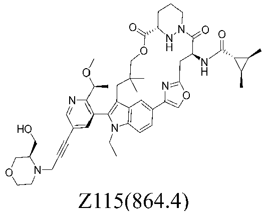 | 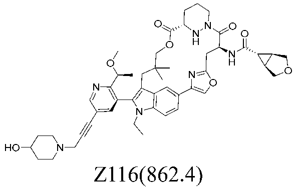 | 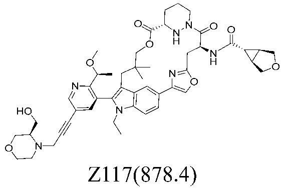 |
| 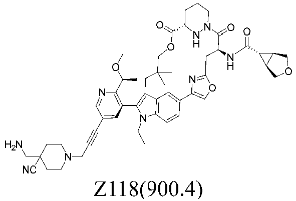 | 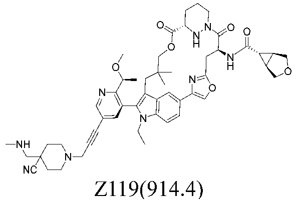 | 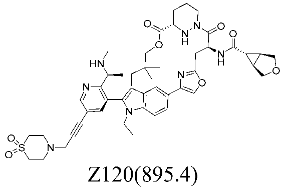 |
| 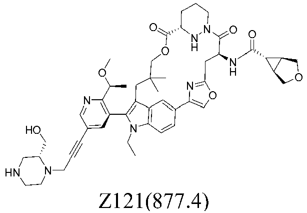 | 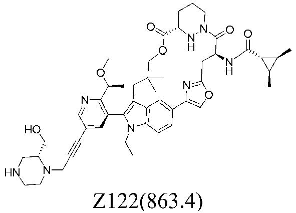 | 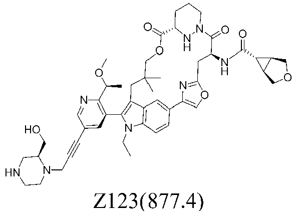 |
| 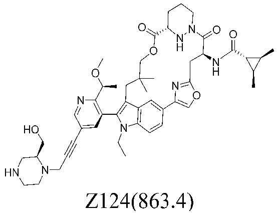 | 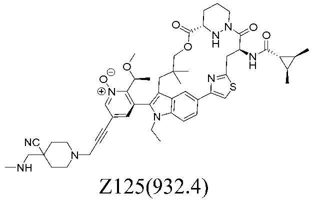 | 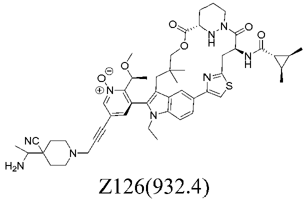 |
| 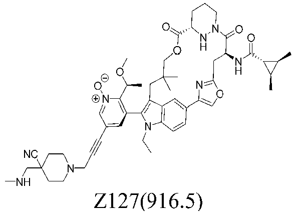 | 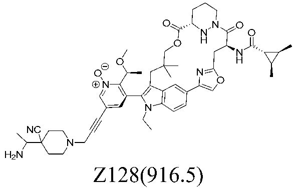 | 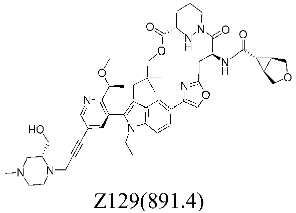 |
| 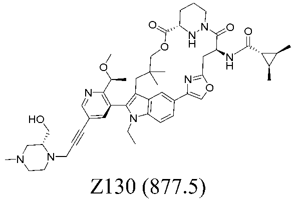 | 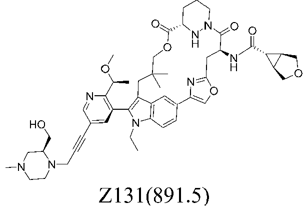 | 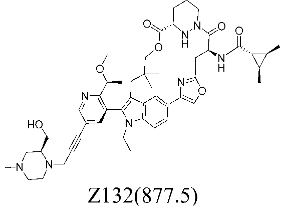 |
| 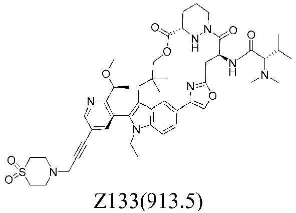 | 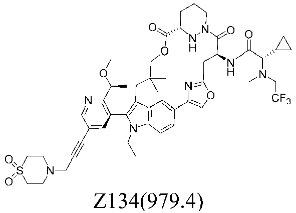 | 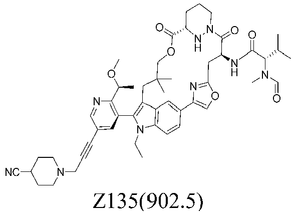 |
| 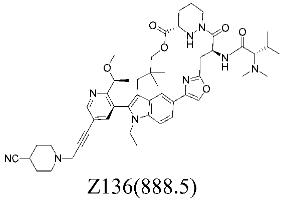 | 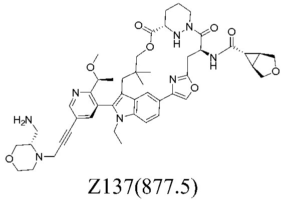 | 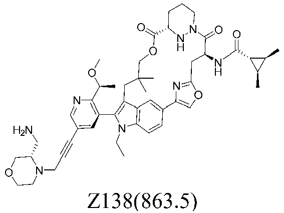 |
| 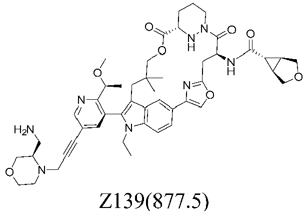 | 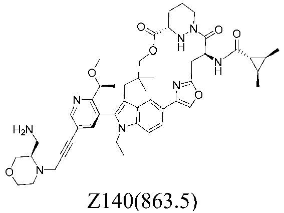 | 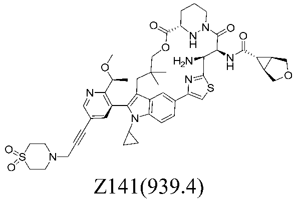 |
| 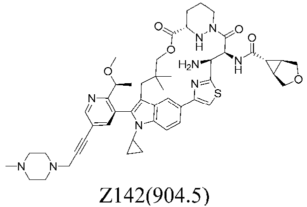 | 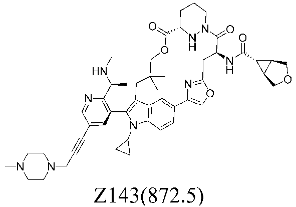 | 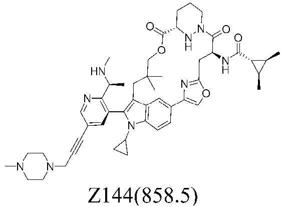 |
| 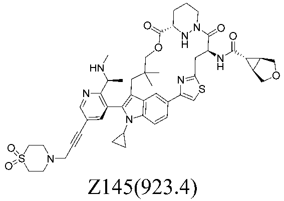 | 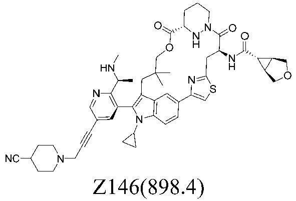 | 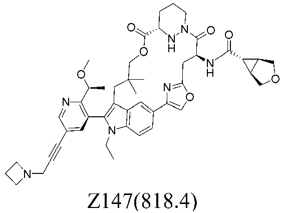 |
| 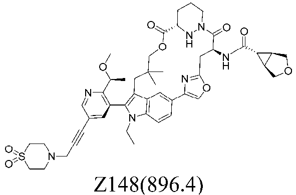 | 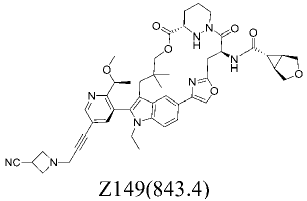 | 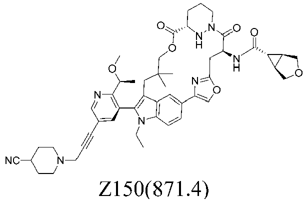 |
| 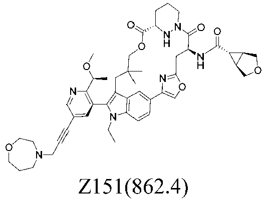 | 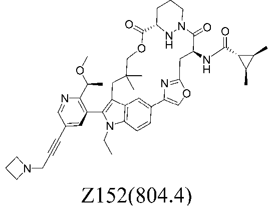 | 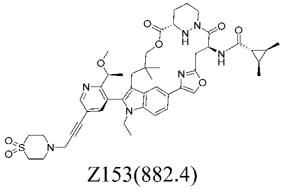 |
| 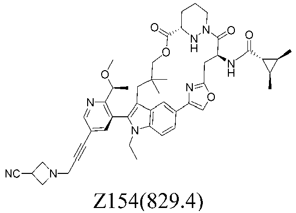 | 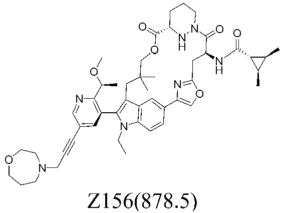 | 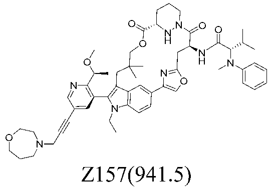 |
| 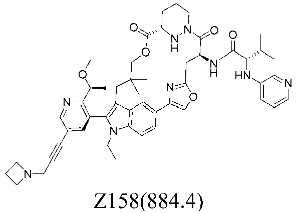 | 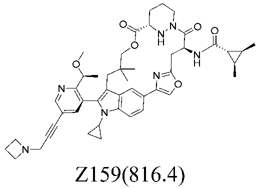 | 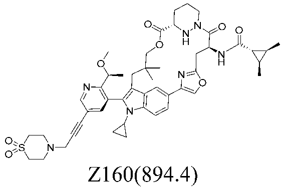 |
| 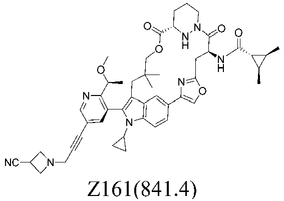 | 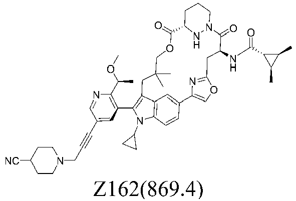 | 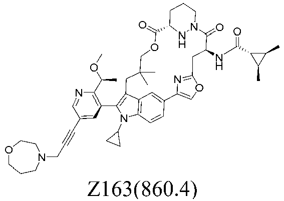 |
| 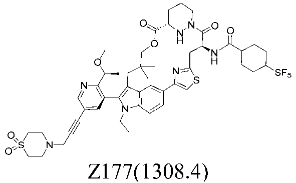 | 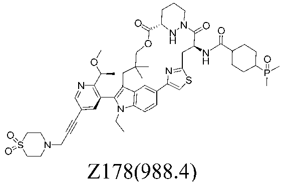 | 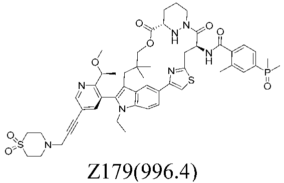 |
| 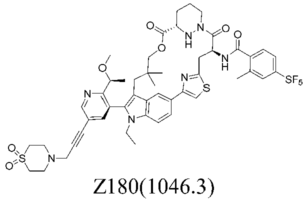 | 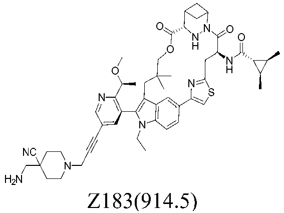 | 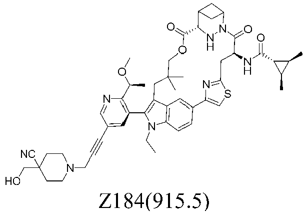 |
| 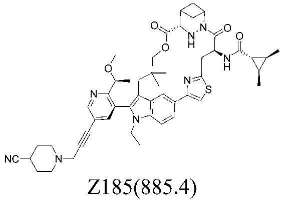 | 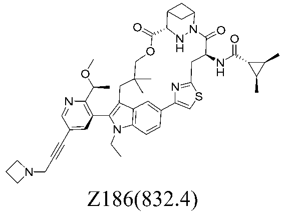 | 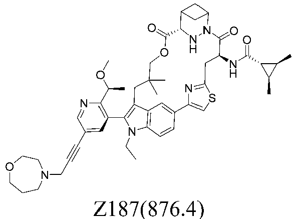 |
| 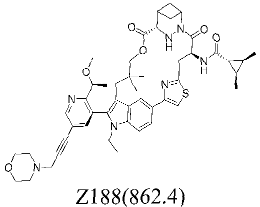 | 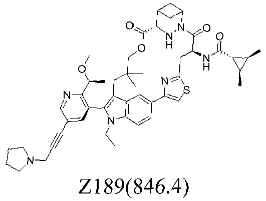 | 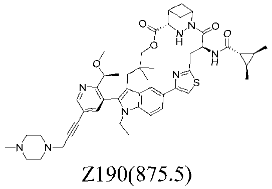 |
| 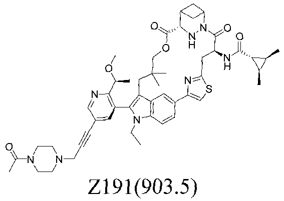 | 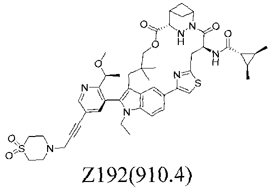 | 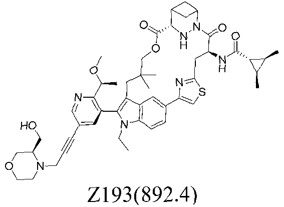 |
| 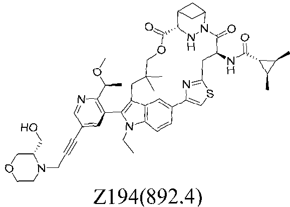 | 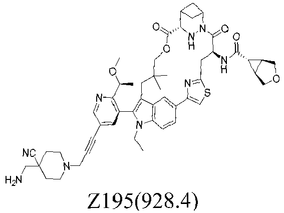 | 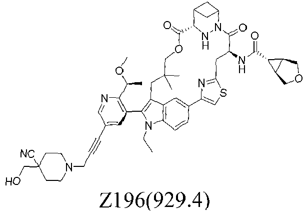 |
| 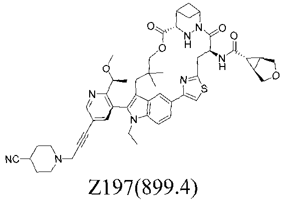 | 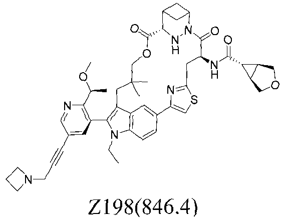 | 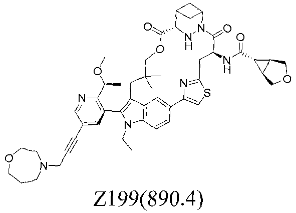 |
| 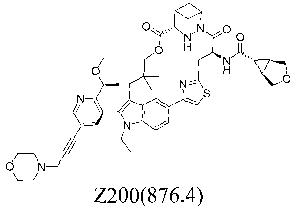 | 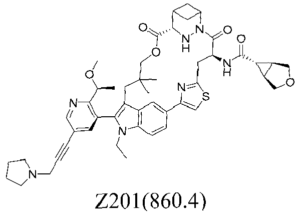 | 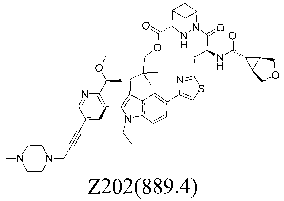 |
| 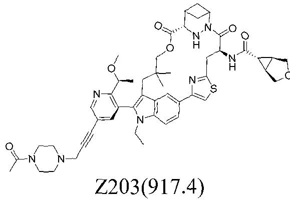 | 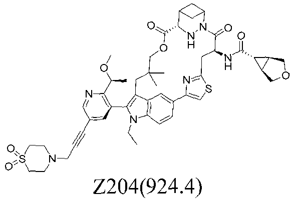 | 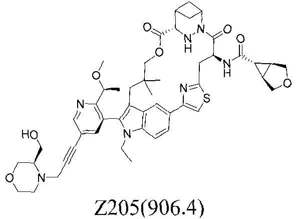 |
| 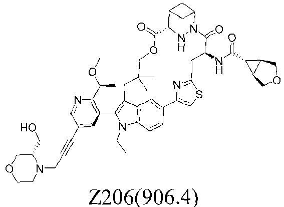 | 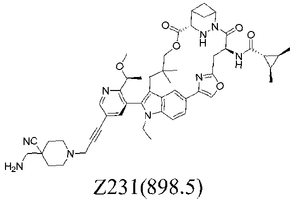 | 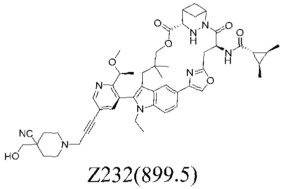 |
| 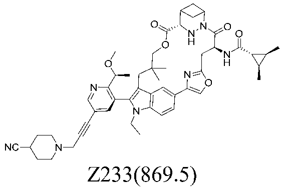 | 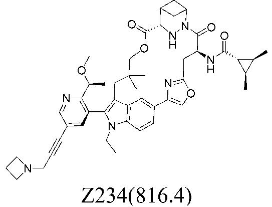 | 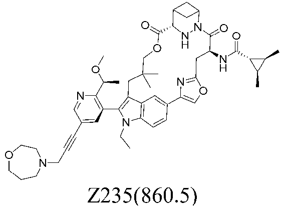 |
| 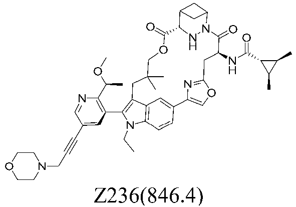 | 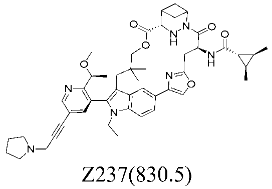 | 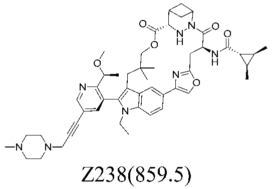 |
| 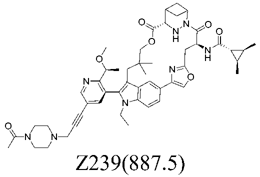 | 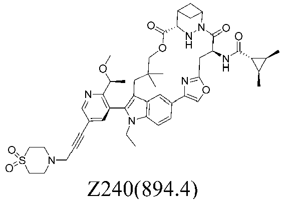 | 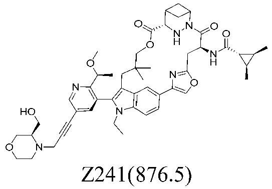 |
| 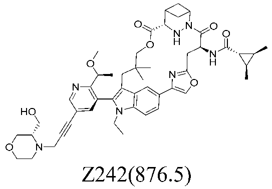 | 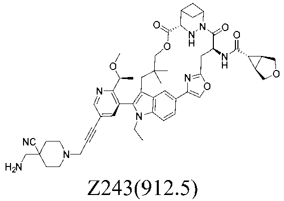 | 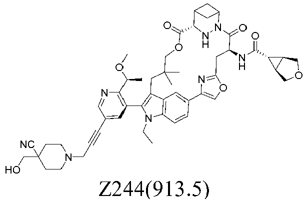 |
| 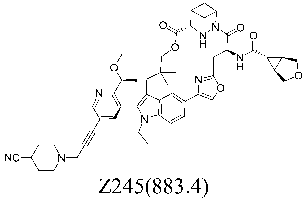 | 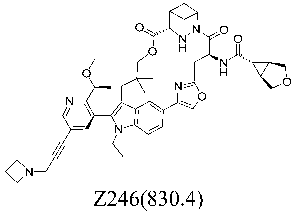 | 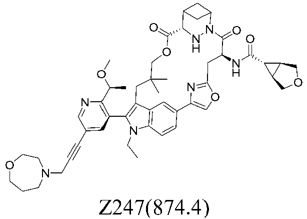 |
| 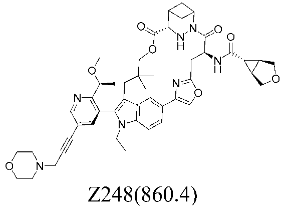 | 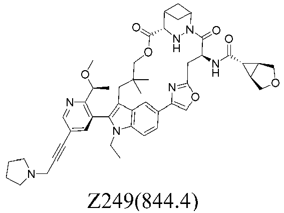 | 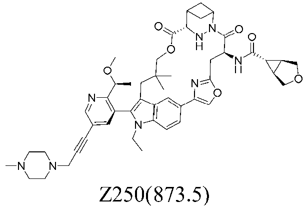 |
| 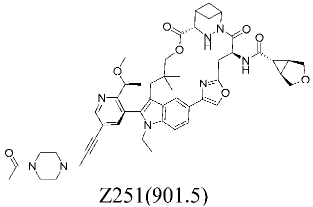 | 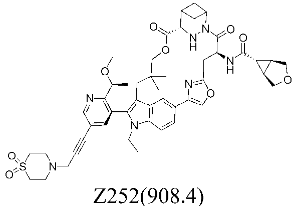 | 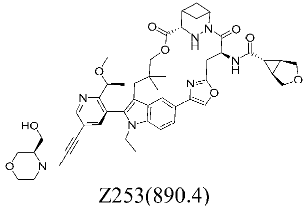 |
| 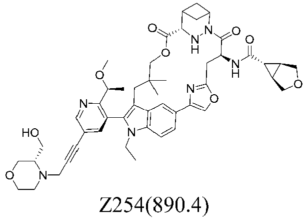 | 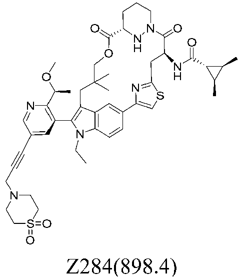 | 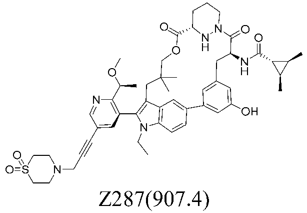 |
| 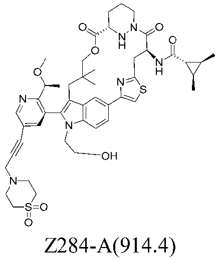 | 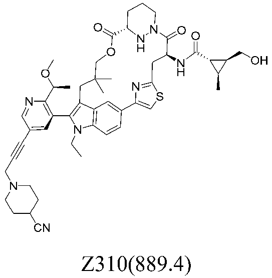 | 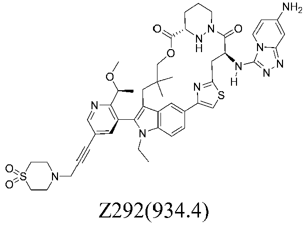 |
| 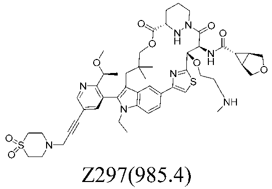 | 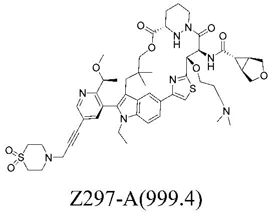 | 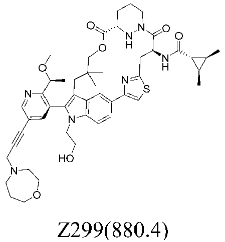 |
| 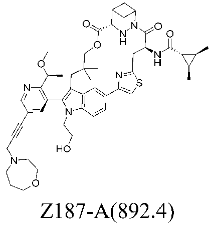 | 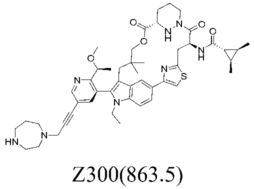 | 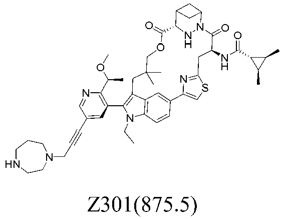 |
| 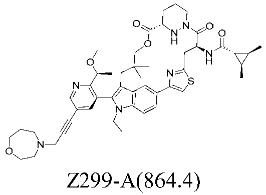 | 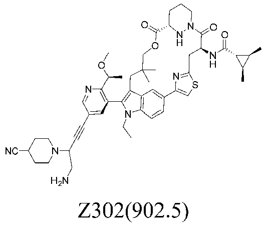 | 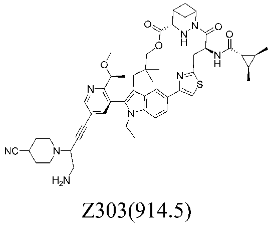 |
| 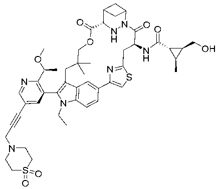 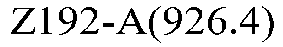 | 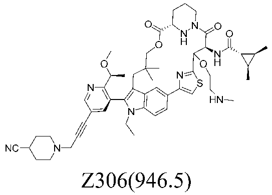 | 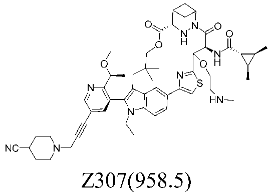 |
| 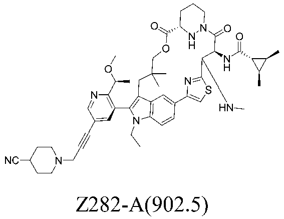 | 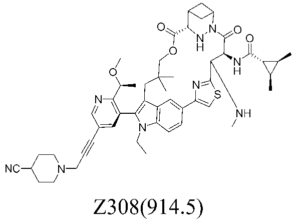 | 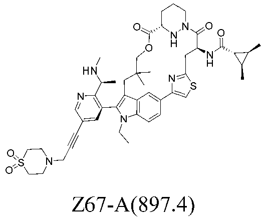 |
| 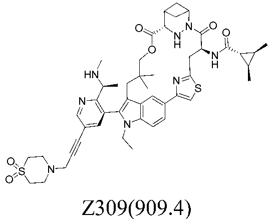 | | |

**Table B-2**

| Compound structure | Compound structure | Compound structure |
|---|---|---|
| Compound No. ([M+H]⁺) | Compound No. ([M+H]⁺) | Compound No. ([M+H]⁺) |
| 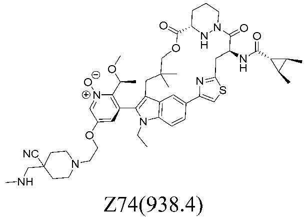 | 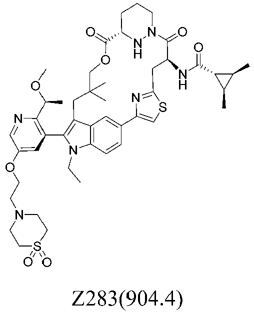 | 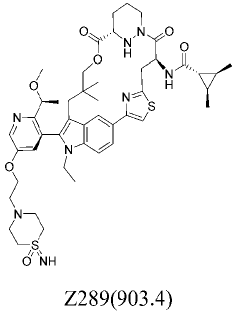 |
| 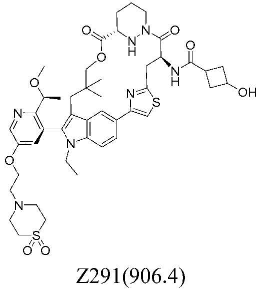 | 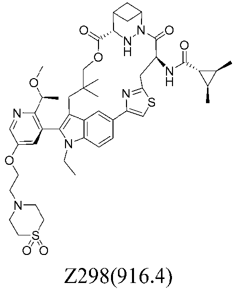 | 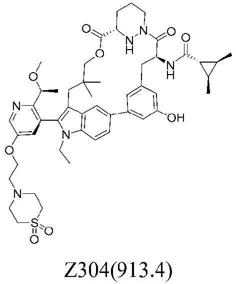 |
| 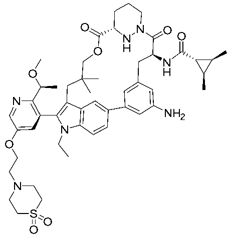 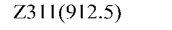 | 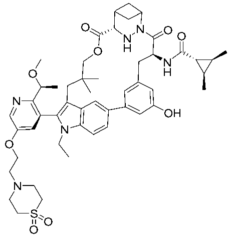 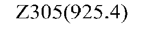 | 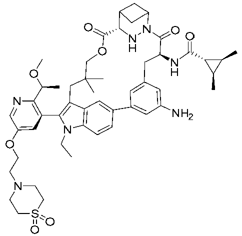 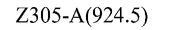 |

**Table C**

| Compound structure | Compound structure | Compound structure |
|---|---|---|
| Compound No. ([M+H]⁺) | Compound No. ([M+H]⁺) | Compound No. ([M+H]⁺) |
| 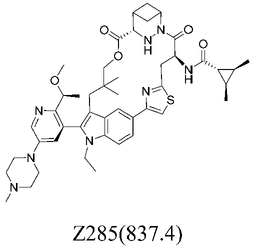 | 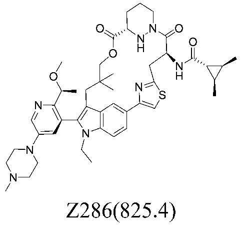 | 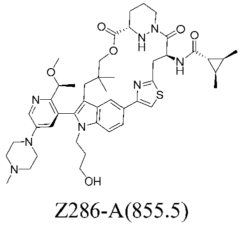 |
| 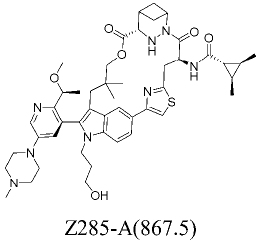 | 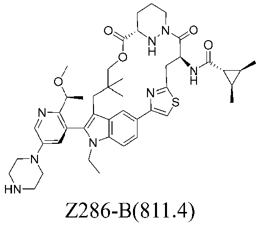 | 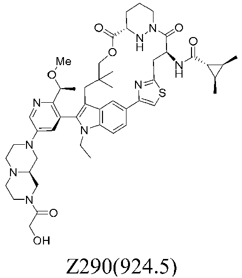 |
| 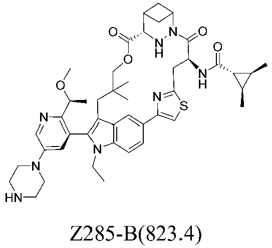 | 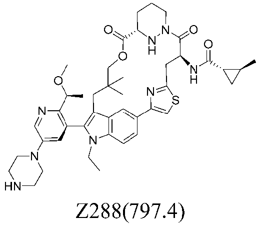 | 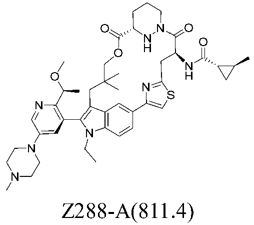 |
| 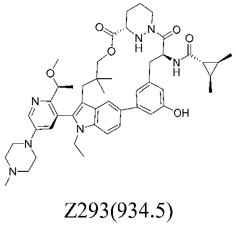 | 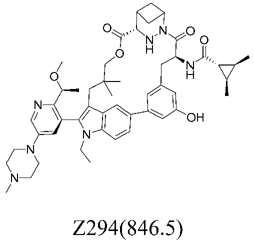 | 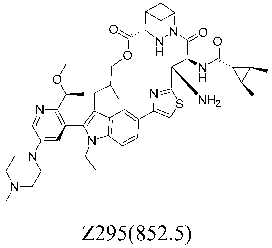 |
| 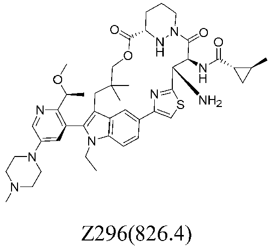 | | |

### Example A-1. Synthesis of Compound Z6-L1

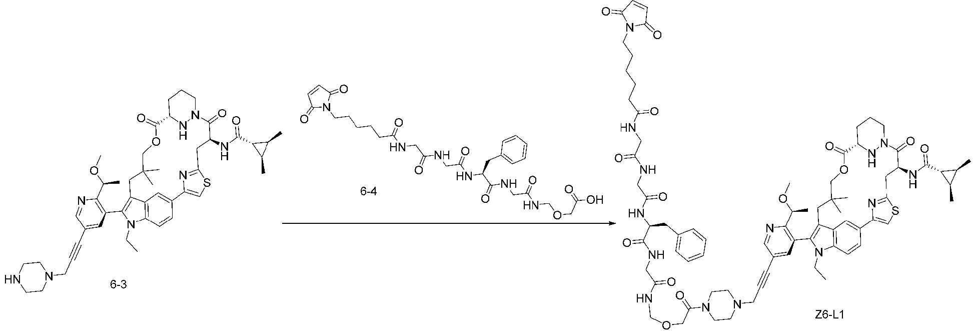

Step 1: Compound 6-3 (53 mg, 0.062 mmol) was dissolved in N,N-dimethylformamide (2.5 mL), and compound 6-4 (58 mg, 0.094 mmol, prepared by referring to the method in WO2019195665A1), O-(7-azabenzotriazol-1-yl)-N,N,N',N'-tetramethyluronium hexafluorophosphate (47 mg, 0.125 mmol), and N,N'-diisopropylethylamine (48 mg, 0.375 mmol) were added. The reaction system was stirred at room temperature for 1 h. The reaction mixture was purified by prep-HPLC (ammonium bicarbonate method 1) to give the desired product Z6-L1 (33 mg, yield: 65.1%) as a white solid. ES-API: [M+H]⁺= 1447.4. ¹H NMR (400 MHz, DMSO-*d₆*) δ 8.80 (d, *J* = 2.0 Hz, 1H), 8.58 - 8.47 (m, 2H), 8.38 (d, *J* = 8.8 Hz, 1H), 8.29 (t, *J* = 5.6 Hz, 1H), 8.12 (d, *J* = 8.0 Hz, 1H), 8.06 (t, *J* = 5.6 Hz, 1H), 8.00 (t, *J =* 5.6 Hz, 1H), 7.84 (d, *J =* 2.0 Hz, 1H), 7.81 (s, 1H), 7.75 (d, *J =* 8.8 Hz, 1H), 7.57 (d, *J* = 8.8 Hz, 1H), 7.27 - 7.13 (m, 5H), 6.98 (s, 2H), 5.56 (t, *J* = 9.2 Hz, 1H), 5.05 (d, *J* = 12.0 Hz, 1H), 4.58 (d, *J* = 6.4 Hz, 2H), 4.53 - 4.45 (m, 1H), 4.38 - 3.99 (m, 7H), 3.77 - 3.57 (m, 10H), 3.52 - 3.41 (m, 4H), 3.33 - 3.29 (m, 3H), 3.24 - 3.02 (m, 5H), 2.95 (d, *J =* 14.8 Hz, 1H), 2.85 - 2.71 (m, 2H), 2.60 - 2.51 (m, 4H), 2.38 (d, *J* = 14.0 Hz, 1H), 2.14 - 2.03 (m, 3H), 1.83 - 1.68 (m, 2H), 1.56 - 1.30 (m, 8H), 1.24 - 1.13 (m, 5H), 1.11 - 1.03 (m, 6H), 0.94 - 0.79 (m, 6H), 0.34 (s, 3H).

### Example A-2. Synthesis of Compound Z10-L1

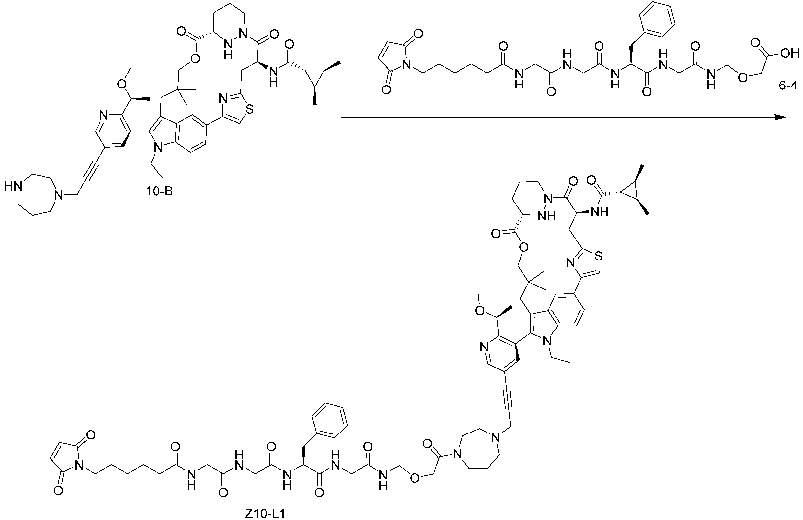

Step 1: Compound 6-4 (17.15 mg, 0.028 mmol) and compound 10-B (20 mg, 0.023 mmol) were dissolved in N,N-dimethylformamide (2 mL). O-(7-Azabenzotriazol-1-yl)-N,N,N',N'-tetramethyluronium hexafluorophosphate (10.57 mg, 0.028 mmol) and N,N'-diisopropylethylamine (20.15 mg, 0.156 mmol) were added to the solution described above. After the addition was completed, the reaction system was stirred at room temperature for 2 h. After the reaction was completed, the reaction mixture was purified by prep-HPLC (ammonium bicarbonate method 1) to give the desired product Z10-L1 (15 mg, 0.010 mmol, yield: 44.29%) as a white solid, ES-API: [M+H]⁺ = 1461.4. ¹H NMR (400 MHz, DMSO-*d₆*) δ 8.82-8.80 (m, 1H), 8.53-8.50 (m, 2H), 8.37 (d, J = 8.8 Hz, 1H), 8.28 (t, J = 5.6 Hz, 1H), 8.12 (d, J = 8.0 Hz, 1H), 8.05 (t, J = 5.6 Hz, 1H), 7.99 (t, J = 5.6 Hz, 1H), 7.86 - 7.79 (m, 2H), 7.75 (d, J = 7.6 Hz, 1H), 7.57 (d, J = 8.8 Hz, 1H), 7.29 - 7.13 (m, 5H), 6.98 (s, 2H), 5.56 (t, J = 9.0 Hz, 1H), 5.05 (d, J = 12.0 Hz, 1H), 4.59 (d, J = 6.4 Hz, 2H), 4.52-4.49 (m, 1H), 4.37 - 4.00 (m, 7H), 3.77 - 3.51 (m, 11H), 3.37 (d, J = 7.2 Hz, 4H), 3.29 - 3.28 (m, 1H), 3.24 (s, 3H), 3.21-3.15 (m, 1H), 3.06 (dd, J = 13.8, 4.4 Hz, 1H), 2.95 (d, J = 14.4 Hz, 1H), 2.86 - 2.59 (m, 6H), 2.39 (d, J = 14.0 Hz, 1H), 2.11-2.09 (m, 3H), 1.81-1.78 (m, 4H), 1.52-1.47 (m, 5H), 1.35 (d, J = 6.0 Hz, 3H), 1.27 - 1.13 (m, 6H), 1.07 (m, 6H), 0.95 - 0.82 (m, 6H), 0.34 (s, 3H).

### Example A-3. Synthesis of Compound Z3-L1

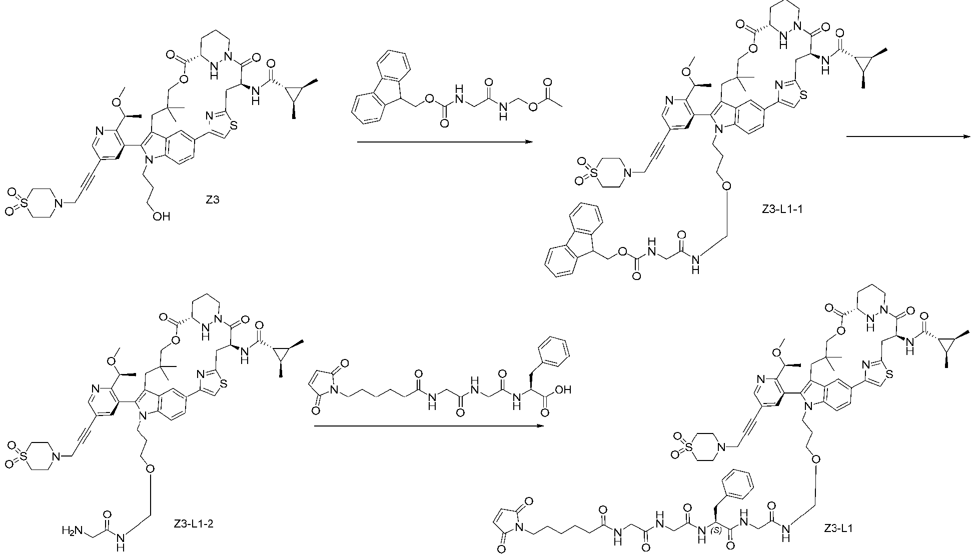

Step 1: Compound Z3 (150 mg, 0.162 mmol) and 9H-fluoren-9-ylmethyl [(2,6-dioxo-5-aza-3-oxahept-7-yl)amino]formate (149.20 mg, 0.405 mmol, prepared by referring to the method in WO2019195665A1) were dissolved in dry dichloromethane (5 mL). Under an ice-water bath, p-toluenesulfonic acid monohydrate (0.20 mg, 0.001 mmol) was added. The mixture was reacted at room temperature for 5 h. After the reaction was completed, the reaction mixture was washed with a saturated aqueous sodium bicarbonate solution (10 mL × 1), dried over anhydrous sodium sulfate, filtered, and concentrated. The resulting crude product was purified by silica gel column chromatography (methanol/dichloromethane = 1/20) to give compound Z3-L1-1 (60 mg, yield: 30%). ES-API: [M+H]⁺= 1236.3.

Step 2: Compound Z3-L1-1 (60 mg, 0.049 mmol) was dissolved in N,N-dimethylformamide (2 mL). The mixture was cooled under ice-brine (< 0 °C), and DBU (14.92 mg, 0.098 mmol) was slowly added. The reaction system was stirred for 5 min while maintaining the ice-water bath. After the reaction was completed as detected, 1 M diluted hydrochloric acid was added for neutralization to give a reaction mixture of compound Z3-L1-2, which was directly used in the next step. ES-API: [M+H]⁺=1014.4.

Step 3: N-[12-(2,5-dioxo-2,5-dihydro-1H-pyrrol-1-yl)-1,4,7-trioxo-3,6-diazadodecyl]-L-phenylalanine (23.29 mg, 0.049 mmol, prepared by referring to the method in WO2021198965A1), N,N-diisopropylethylamine (12.77 mg, 0.099 mmol), and PyBOP (51.31 mg, 0.099 mmol) were directly and sequentially added to the reaction mixture of compound Z3-L1-2 described above. The mixture was reacted at room temperature for 10 min. Ethyl acetate (20 mL) was added, and the mixture was sequentially washed with water (20 mL × 3) and saturated brine (20 mL × 3). The organic layer was separated, dried, and concentrated, and the residue was purified by prep-HPLC (ammonium bicarbonate method 1) to give the desired product Z3-L1 (3.5 mg, yield: 4.08%). ES-API: [M+H]⁺=1468.4.

### Example A-4. Synthesis of Compound Z11-L2

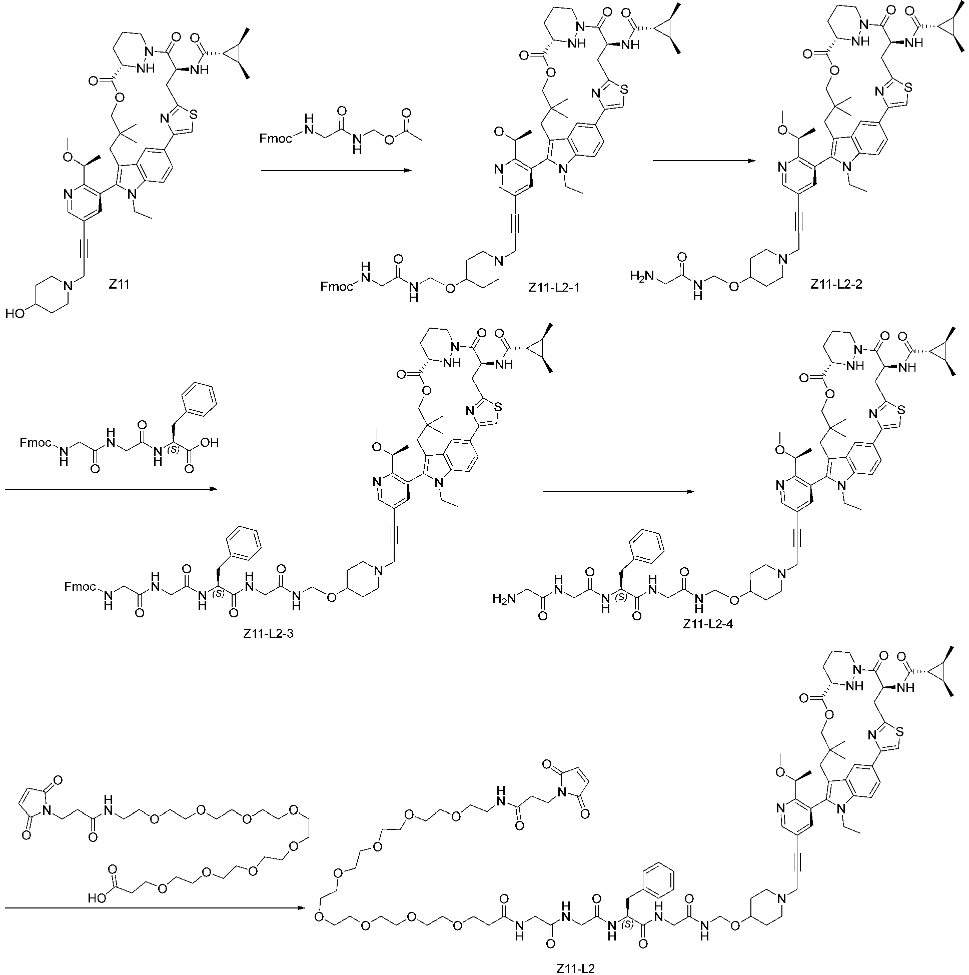

Step 1: Compound Z11 (800 mg, 0.926 mmol) and 9H-fluoren-9-ylmethyl [(2,6-dioxo-5-aza-3-oxahept-7-yl)amino]formate (682.11 mg, 1.852 mmol, prepared with reference to the method in WO2019195665A1) were dissolved in dry dichloromethane (10 mL). Under an ice-water bath, trifluoroacetic acid (2 mL) was added. The mixture was reacted at room temperature for 2 h. After the reaction was completed, the reaction mixture was washed with a saturated aqueous sodium bicarbonate solution (10 mL × 1), dried over anhydrous sodium sulfate, filtered, and concentrated. The residue was purified by prep-HPLC (trifluoroacetic acid method) to give compound Z11-L2-1 (370 mg, 0.316 mmol, yield: 34.09%). ES-API: [M+H]⁺= 1172.5.

Step 2: Compound Z11-L2-1 (80 mg, 0.068 mmol) was dissolved in N,N-dimethylformamide (2 mL). The mixture was cooled under ice-brine (< 0 °C), and DBU (20.78 mg, 0.136 mmol) was slowly added. The reaction system was stirred for 5 min while maintaining the ice-water bath. After the reaction was completed as detected, 1 M diluted hydrochloric acid was added for neutralization to give a reaction mixture of compound Z11-L2-2, which was directly used in the next step. ES-API: [M+H]⁺=950.3.

Step 3: (((9H-Fluoren-9-yl)methoxy)carbonyl)glycylglycyl-L-phenylalanine (51.46 mg, 0.103 mmol, prepared by referring to the method in WO2022246576A1), N,N-diisopropylethylamine (26.52 mg, 0.205 mmol), and PyBOP (53.40 mg, 0.103 mmol) were added directly and sequentially to the reaction mixture of compound Z11-L2-2 described above. The mixture was reacted at room temperature for 10 min. Ethyl acetate (20 mL) was added, and the mixture was sequentially washed with water (20 mL × 3) and saturated brine (20 mL × 3). The organic layer was separated, dried, and concentrated, and the residue was purified by prep-TLC (methanol/dichloromethane = 7/100) to give compound Z11-L2-3 (60 mg, 0.042 mmol, yield: 61.18%). ES-API: [M+H]⁺=1433.5.

Step 4: Compound Z11-L2-3 (60 mg, 0.042 mmol) was dissolved in N,N-dimethylformamide (2 mL). The mixture was cooled under ice-brine (< 0 °C), and DBU (12.72 mg, 0.084 mmol) was slowly added. The reaction system was stirred for 5 min while maintaining the ice-water bath. After the reaction was completed as detected, 1 M diluted hydrochloric acid was added for neutralization to give a reaction mixture of compound Z11-L2-4, which was directly used in the next step. ES-API: [M+H]⁺=1211.6.

Step 5: 1-(2,5-Dioxo-2,5-dihydro-1H-pyrrol-1-yl)-3-oxo-7,10,13,16,19,22,25,28-octaoxa-4-azahentriacontanoic acid (CAS: 1334177-86-4, 25 mg, 0.042 mmol), N,N-diisopropylethylamine (16.36 mg, 0.127 mmol), and PyBOP (32.93 mg, 0.063 mmol) were directly and sequentially added to the reaction mixture of compound Z11-L2-4 described above. The mixture was reacted at room temperature for 10 min and purified by prep-HPLC (trifluoroacetic acid method) to give the desired product Z11-L2 (26 mg, yield: 34.51%). ES-API: [M+H]⁺=1786.4.

### Example A-5. Synthesis of Compound Z6-L2

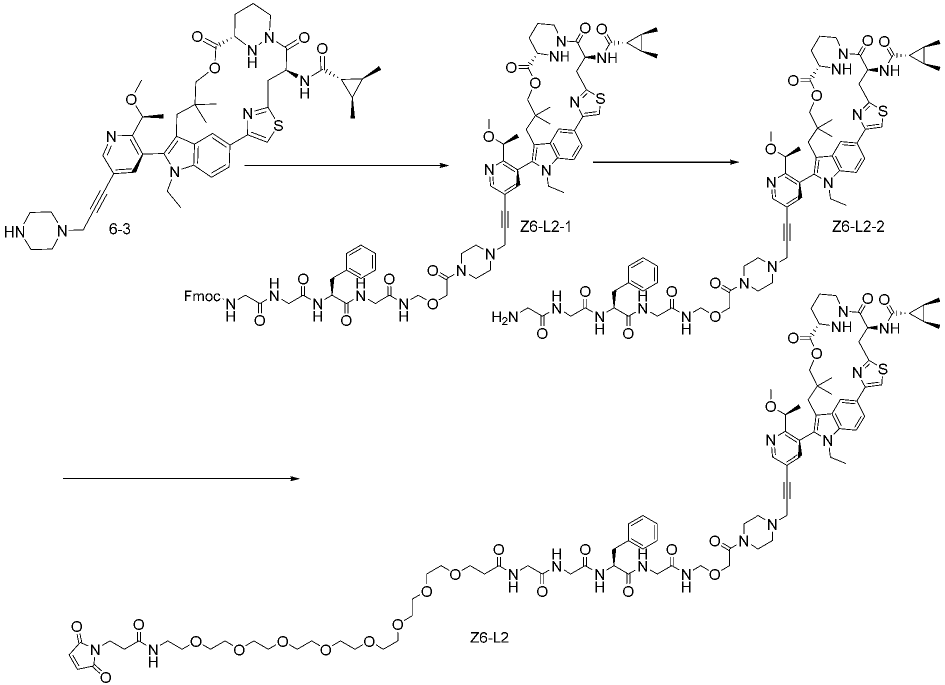

Step 1: N,N-Diisopropylethylamine (110.11 mg, 0.852 mmol) and O-(7-azabenzotriazol-1-yl)-N,N,N',N'-tetramethyluronium hexafluorophosphate (161.88 mg, 0.426 mmol) were added to a solution of compound 6-3 (120.57 mg, 0.142 mmol) and {[(11S)-11-benzyl-1-(9H-fluoren-9-yl)-3,6,9,15-tetraoxo-2-oxo-4,7,10,13,16-pentaazaheptadecan-17-yl]oxy}acetic acid (100.0 mg, 0.152 mmol) in N,N-dimethylformamide (5.0 mL), and the reaction mixture was stirred at room temperature for 20 min and extracted with ethyl acetate (80 mL) and water (80 mL). The organic phase was concentrated to dryness by rotary evaporation under reduced pressure to give a crude product. The crude product was purified by flash silica gel column chromatography [dichloromethane/methanol = 100:0 to 90:10 (v/v)], concentrated under vacuum, and dried to give compound Z6-L2-1 (110 mg, 0.074 mmol, yield: 52.46%). ES-API[M+H⁺]=1476.5.

Step 2: Compound Z6-L2-1 (110.0 mg, 0.074 mmol) was added to N,N-dimethylformamide (2.0 mL). The mixture was well stirred and cooled to 0 °C, and then 1,8-diazacyclo[5,4,0]undecene-7 (22.64 mg, 0.149 mmol) was slowly added under a nitrogen atmosphere. After the addition was completed, the mixture was reacted at 0 °C for 30 min. After the starting materials were consumed completely as monitored by TLC, the reaction mixture was quenched with HCl (1 M) to the pH of about 6-7 to give a reaction mixture of compound Z6-L2-2, which was directly used in the next step without post-treatment.

Step 3: 1-(2,5-Dioxo-2,5-dihydro-1H-pyrrol-1-yl)-3-oxo-7,10,13,16,19,22,25,28-octaoxa-4-azahentriacontanoic acid (CAS: 1334177-86-4, 43.0 mg, 0.073 mmol), N,N-diisopropylethylamine (30.0 mg, 0.220 mmol), and (benzotriazol-1-yloxy)tripyrrolidinophosphonium hexafluorophosphate (57.20 mg, 0.110 mmol) were added to the reaction mixture of compound Z6-L2-2 obtained in the previous step. The reaction mixture was stirred at room temperature for 30 min. The reaction mixture was extracted with ethyl acetate (100 mL) and water (100 mL). The organic layer was separated and washed with saturated brine (60 mL), and the organic phase was concentrated under vacuum. The reaction mixture was filtered, and the filtrate was purified by prep-HPLC (ammonium bicarbonate method 1) to give the desired product Z6-L2 (26 mg, 0.015 mmol, yield: 34.51%). ES-API[M+H⁺]=1829.7. ¹H NMR (400 MHz, DMSO-d₆) δ 8.81 (d, *J* = 2.0 Hz, 1H), 8.55 - 8050 (m, 2H), 8.45 - 8.38 (m, 1H), 8.35 - 8.25 (m, 1H), 8.22 - 8.09 (m, 2H), 8.05 - 8.02 (m, 2H), 7.88 - 7.80 (m, 2H), 7.78 - 7.73 (m, 1H), 7.60 - 7.56(m, 1H), 7.28 - 7.15 (m, 5H), 7.00 (s, 2H), 5.60 - 5.50 (m, 1H), 5.10 - 5.07 (m, 1H), 4.65 - 4.55 (m, 2H), 4.53 - 4.44 (m, 1H), 4.40 - 4.00 (m, 7H), 3.80 - 3.65(m, 5H), 3.65 - 3.55(m, 9H), 3.53 - 3.43 (m, 32H), 3.40 - 3.35 (m, 5H), 3.25 (s, 3H), 3.18 - 3.10 (m, 3H), 3.08 - 2.90(m, 3H), 2.85 - 2.70 (m, 2H), 2.57 - 2.53(m, 2H), 2.45 - 2.25 (m, 6H), 2.10 - 2.00(m, 1H), 1.85 - 1.65 (m, 2H), 1.52 - 1.45 (m, 1H), 1.40 - 1.30 (m, 3H), 1.25 - 1.15(m, 4H), 1.10 - 1.00 (m, 6H), 0.94 - 0.84 (m, 6H).

### Example A-6. Synthesis of Compound Z34-L1

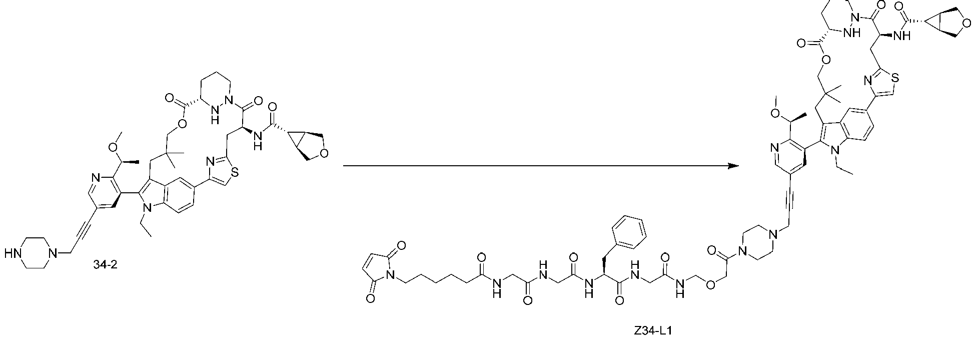

Step 1: Compound 34-2 (30 mg, 0.035 mmol), (S)-10-benzyl-23-(2,5-dioxo-2,5-dihydro-1H-pyrrol-1-yl)-6,9,12,15,18-pentaoxo-3-oxa-5,8,11,14,17-pentaazatricosanoic acid (32.3 mg, 0.05 mmol, prepared by referring to the method in WO2019195665A1), DIPEA (27 mg, 0.2 mmol), and HATU (39.65 mg, 0.104 mmol) were mixed in N,N-dimethylformamide (5 mL). The mixture was stirred at room temperature for 20 min. After the reaction was completed, 30 mL of ethyl acetate was poured into the mixture, and the resulting mixture was sequentially washed with water (15 mL) and saturated brine (15 mL). The organic phase was dried over anhydrous sodium sulfate, filtered, and concentrated, and the residue was purified by a flash silica gel column (0-10% dichloromethane/methanol) to give the desired product Z34-L1 (10 mg, yield: 19.5%), ES-API [M+1]⁺ = 1461.5.

### Example A-7. Synthesis of Compound Z34-L2

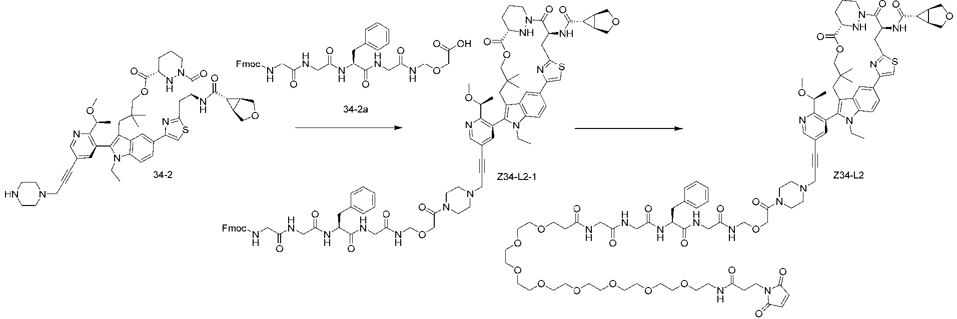

Step 1: Compound 34-2 (60 mg, 0.070 mmol), compound 34-2a (CAS: 2264011-98-3, 44 mg, 0.07 mmol, prepared by referring to the method in WO2019195665 A1), DIPEA (54 mg, 0.41 mmol), and HATU (79.30 mg, 0.209 mmol) were dissolved in DMF (6 mL). The mixture was stirred at room temperature for 1 h. After the reaction was completed, 30 mL of ethyl acetate was poured into the mixture, and the resulting mixture was sequentially washed with 15 mL of water and 15 mL of saturated brine. The organic phase was dried over anhydrous sodium sulfate, filtered, and concentrated, and the residue was purified by a flash silica gel column (0-10% dichloromethane/methanol) to give compound Z34-L2-1 (60 mg, yield: 58%), ES-API [M+1]⁺= 1490.4. Step 2: Compound Z34-L2-1 (60 mg, 0.040 mmol) was dissolved in N,N-dimethylformamide (2 mL), and DBU (12.24 mg, 0.080 mmol) was added at 0 °C. The mixture was stirred for 10 min under an ice bath. With the temperature maintained at 0 °C, 1 M hydrochloric acid was added to adjust the reaction mixture to weak acidity. 1-Maleimido-3-oxo-7,10,13,16,19,22,25,28-octaoxa-4-aza-hentriacontan-31-oic acid (23.71 mg, 0.040 mmol) and DIPEA (15.48 mg, 0.120 mmol) were added to the mixed solution, and PyBOP (31.20 mg, 0.060 mmol) was added at 0 °C. The reaction mixture was stirred at room temperature for 1 h and directly purified by prep-HPLC (formic acid method 1) to give the desired product Z34-L2 (8 mg, yield: 10.8%, formate). ES-API[M+1]⁺=1842.6.

### Example A-8. Synthesis of Compound Z170-L17

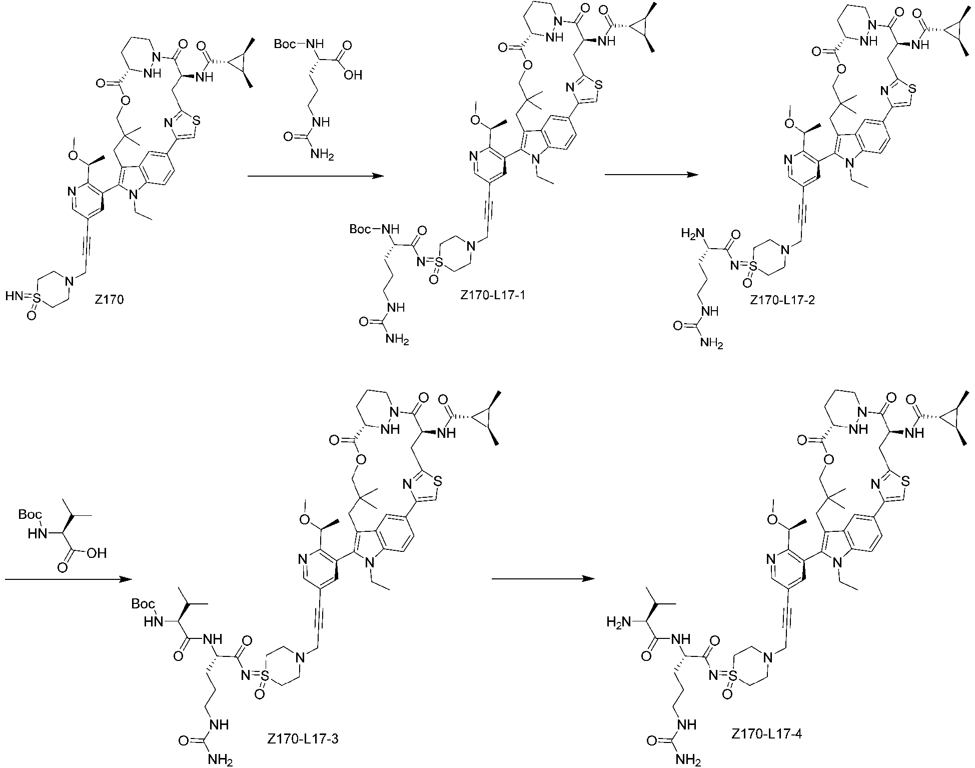

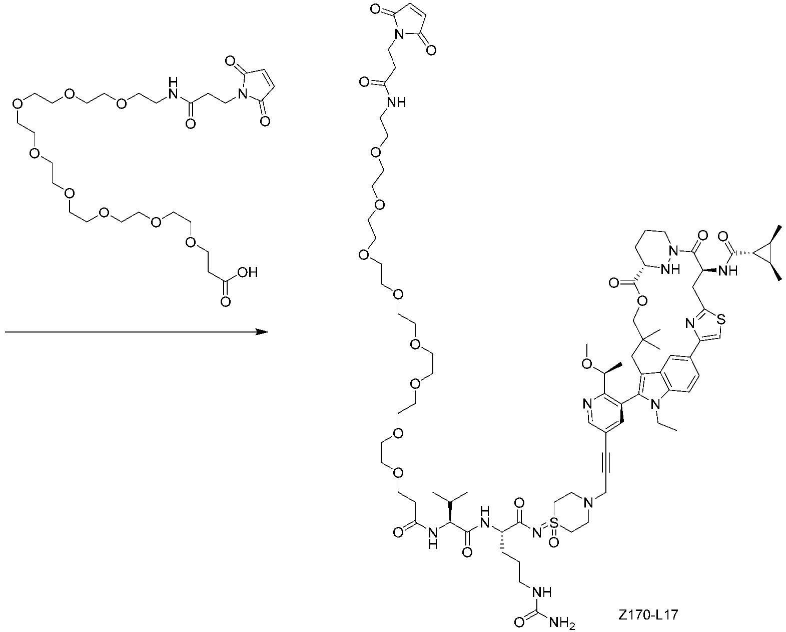

Step 1: Compound Z170 (180 mg, 0.201 mmol) was dissolved in N,N-dimethylformamide (5 mL), and (S)-2-((tert-butoxycarbonyl)amino)-5-ureidopentanoic acid (165.71 mg, 0.602 mmol, prepared by referring to the method in WO2020165600A1), O-(7-azabenzotriazol-1-yl)-*N,N,N',N'*-tetramethyluronium hexafluorophosphate (152.58 mg, 0.401 mmol), and N,N'-diisopropylethylamine (77.79 mg, 0.602 mmol) were added to the solution described above. After the addition was completed, the reaction system was stirred at room temperature for 24 h. After the reaction was completed, the reaction mixture was concentrated to remove dichloromethane. Water (20 mL) was added to the residue, and the mixture was extracted with ethyl acetate (15 mL × 2). The organic phase was washed with saturated brine (20 mL × 3), dried, and concentrated to give a crude product. The crude product was purified by a flash silica gel column (methanol/dichloromethane: 0%-5%) to give compound Z170-L17-1 (180 mg, 0.156 mmol, yield: 77.71%) as a light yellow solid, [M+H]⁺ = 1154.3. Step 2: Compound Z170-L17-1 (180 mg, 0.156 mmol) was dissolved in methanol (1 mL), and a solution (4 M, 2 mL) of hydrogen chloride in dioxane was added to the solution described above. After the addition was completed, the reaction system was stirred at room temperature for 1 h. After the reaction was completed, the reaction mixture was concentrated to give compound Z170-L17-2 (160 mg, crude product) as a white solid, [M+H]⁺ = 1054.3.

Step 3: Compound Z170-L17-2 (160 mg, crude product) was dissolved in dichloromethane (6 mL), and (tert-butoxycarbonyl)-L-valine (65.94 mg, 0.304 mmol), O-(7-azabenzotriazol-1-yl)-*N,N,N',N'*-tetramethyluronium hexafluorophosphate (86.55 mg, 0.228 mmol), and *N,N'*-diisopropylethylamine (58.84 mg, 0.455 mmol) were added to the solution described above. After the addition was completed, the reaction system was stirred at room temperature for 24 h. After the reaction was completed, the reaction mixture was concentrated to remove dichloromethane. Water (20 mL) was added to the residue, and the mixture was extracted with ethyl acetate (15 mL × 2). The organic phase was washed with saturated brine (20 mL × 3), dried, and concentrated to give a crude product. The crude product was purified by a flash silica gel column (methanol/dichloromethane: 0%-4%) to give compound Z170-L17-3 (100 mg, 0.080 mmol, two-step yield: 52.57%) as a light yellow solid, [M+H]⁺ = 1253.3.

Step 4: Compound Z170-L17-3 (150 mg, 0.120 mmol) was dissolved in dichloromethane (1 mL). Trifluoroacetic acid (1 mL) was added to the solution described above. After the addition was completed, the mixture was stirred at room temperature for 2 h. After the reaction was completed, the reaction mixture was concentrated to give compound Z170-L17-4 (140 mg, crude product) [M+H]⁺= 1153.1.

Step 5: Compound Z170-L17-4 (140 mg, crude product) was dissolved in dichloromethane (6 mL), and 1-maleimido-3-oxo-7,10,13,16,19,22,25,28-octaoxa-4-aza-hentriacontan-31-oic acid (92.17 mg, 0.156 mmol), O-(7-azabenzotriazol-1-yl)-*N,N,N',N'*-tetramethyluronium hexafluorophosphate (91 mg, 0.239 mmol), and *N,N'-*diisopropylethylamine (46.39 mg, 0.359 mmol) were added to the solution described above. After the addition was completed, the reaction system was stirred at room temperature for 2 h. After the reaction was completed, the reaction mixture was concentrated to remove dichloromethane. Water (20 mL) was added to the residue, and the mixture was extracted with ethyl acetate (15 mL × 2). The organic phase was washed with saturated brine (20 mL × 3), dried, and concentrated to give a crude product. The crude product was purified by prep-HPLC (ammonium bicarbonate method 1) to give the desired product Z170-L17 (36 mg, 0.021 mmol, two-step yield: 17.5%) as a white solid, [M+H]⁺ = 1727.5. ¹H NMR (400 MHz, DMSO-*d₆*) δ 8.83 (d, *J =* 2.0 Hz, 1H), 8.50 (s, 1H), 8.39 (d, *J =* 8.8 Hz, 1H), 8.01 (t, *J =* 5.6 Hz, 1H), 7.95 (d, *J =* 7.6 Hz, 1H), 7.88 (d, *J =* 2.0 Hz, 1H), 7.83 - 7.73 (m, 3H), 7.58 (d, *J =* 8.8 Hz, 1H), 7.00 (s, 2H), 5.89 (t, *J =* 5.6 Hz, 1H), 5.56 (t, *J =* 9.2 Hz, 1H), 5.35 (s, 2H), 5.07 (d, *J* = 12.0 Hz, 1H), 4.41 - 4.21 (m, 4H), 4.18 - 4.03 (m, 3H), 3.73 (s, 2H), 3.62 - 3.56 (m, 7H), 3.49 (dd, *J* = 7.3, 2.6 Hz, 32H), 3.36 (t, *J =* 6.0 Hz, 2H), 3.25 (s, 3H), 3.20 - 3.11 (m, 5H), 3.00 - 2.88 (m, 5H), 2.80 - 2.68 (m, 1H), 2.47 - 2.42 (m, 1H), 2.41 - 2.28 (m, 4H), 2.12 - 2.03 (m, 1H), 2.01 - 1.91 (m, 1H), 1.85 - 1.68 (m, 3H), 1.61 - 1.47 (m, 2H), 1.43 - 1.30 (m, 5H), 1.22 - 1.13 (m, 3H), 1.10 - 1.04 (m, 6H), 0.93 - 0.84 (m, 9H), 0.81 (d, *J* = 6.8 Hz, 3H), 0.34 (s, 3H).

### Example A-9. Synthesis of Compound Z22-L17

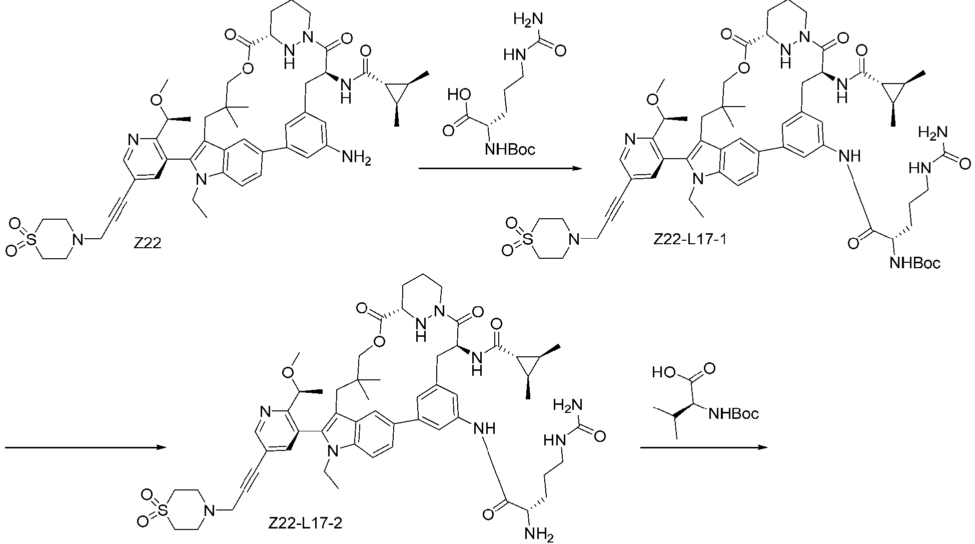

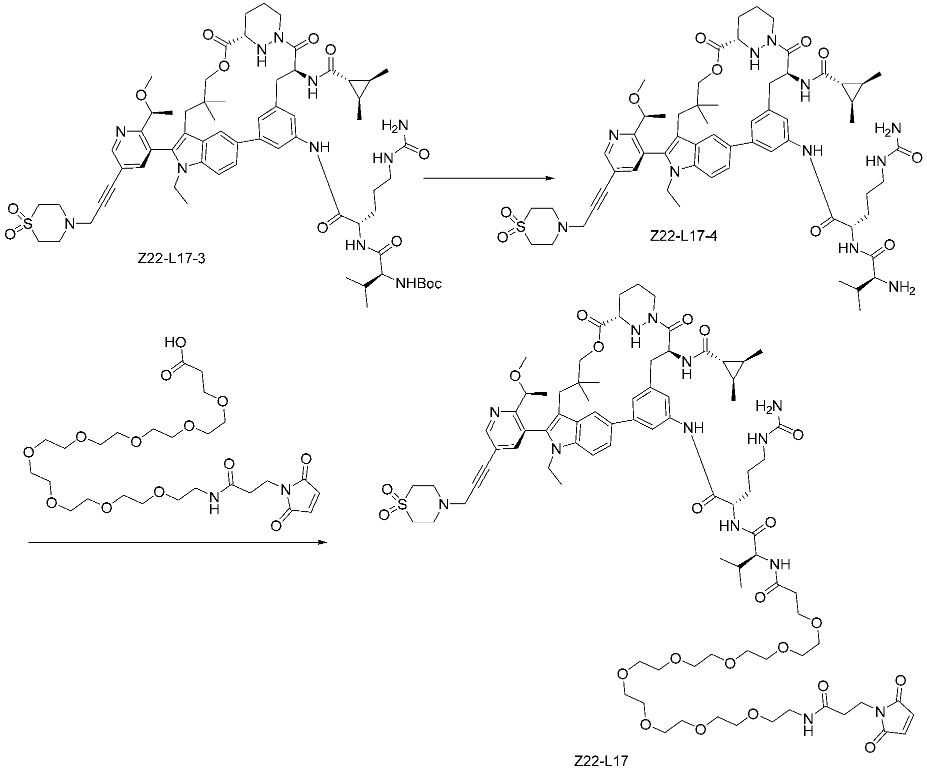

Step 1: tert-Butoxycarbonyl-L-citrulline (36 mg, 0.13 mmol) was dissolved in dichloromethane (8 mL), and O-(7-azabenzotriazol-1-yl)-N,N,N',N'-tetramethyluronium hexafluorophosphate (38 mg, 0.10 mmol) and N,N'-diisopropylethylamine (26 mg, 0.20 mmol) were added at room temperature. The reaction system was stirred at room temperature for 30 min. Then, compound Z22 (60 mg, 0.066 mmol) was added, and the reaction system was stirred at room temperature for another 1 h. Dichloromethane (20 mL) was added to the reaction mixture, and the mixture was sequentially washed with water (10 mL) and saturated brine (10 mL). The organic phase was dried over anhydrous sodium sulfate and filtered, and the filtrate was concentrated under reduced pressure. The crude product was purified by a flash silica gel column (methanol/dichloromethane: 0-8%) to give compound Z22-L17-1 (60 mg, yield: 77.9%) as a pale yellow solid. ES-API: [M+H]⁺=1163.4.

Step 2: Compound Z22-L17-1 (50 mg, 0.043 mmol) was dissolved in methanol (0.2 mL), and a 4.0 M hydrogen chloride/dioxane solution (2 mL) was added at 0 °C. The reaction system was stirred at room temperature for 1 h. The reaction mixture was concentrated to give crude compound Z22-L17-2 (45 mg, yield: 98.5%) as a pale yellow solid. ES-API: [M+H]⁺= 1063.3.

Step 3: Compound Z22-L17-2 (45 mg, 0.042 mmol) and tert-butoxycarbonyl-L-valine (23 mg, 0.106 mmol) were dissolved in dichloromethane (5 mL), and O-(7-azabenzotriazol-1-yl)-N,N,N',N'-tetramethyluronium hexafluorophosphate (24 mg, 0.063 mmol) and N,N'-diisopropylethylamine (27 mg, 0.212 mmol) were added at room temperature. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure. The crude product was purified by a flash silica gel column (methanol/dichloromethane: 0-8%) to give compound Z22-L17-3 (50 mg, yield: 93.6%) as a white solid.

ES-API: [M+H]⁺=1262.3.

Step 4: Compound Z22-L17-3 (40 mg, 0.032 mmol) was dissolved in methanol (0.2 mL), and a 4.0 M hydrogen chloride/dioxane solution (2.5 mL) was added at 0 °C. The reaction system was stirred at room temperature for 1 h. The reaction mixture was concentrated to give crude compound Z22-L17-4 (36 mg, yield: 97.7%) as a pale yellow solid. ES-API: [M+H]⁺= 1162.4.

Step 5: Compound Z22-L17-4 (36 mg, 0.031 mmol) and 1-maleimido-3-oxo-7,10,13,16,19,22,25,28-octaoxa-4-aza-hentriacontan-31-oic acid (55 mg, 0.095 mmol) were dissolved in dichloromethane (5 mL), and O-(7-azabenzotriazol-1-yl)-N,N,N',N'-tetramethyluronium hexafluorophosphate (18 mg, 0.046 mmol) and N,N'-diisopropylethylamine (20 mg, 0.155 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure to give a crude product. The crude product was purified by prep-HPLC (ammonium bicarbonate method 1) to give the desired product Z22-L17 (40 mg, yield: 74.4%) as a white solid. ES-API: [M+H]⁺= 1736.8. 1H NMR (400 MHz, DMSO-*d₆*) δ 9.96 (s, 1H), 8.82 (d, J = 2.0 Hz, 1H), 8.19 (d, J = 8.4 Hz, 1H), 8.09 (d, J = 7.6 Hz, 1H), 8.03 - 7.95 (m, 2H), 7.93 - 7.84 (m, 3H), 7.68 - 7.49 (m, 3H), 7.34 (s, 1H), 6.99 (s, 2H), 5.99 (t, J = 5.6 Hz, 1H), 5.52 - 5.26 (m, 4H), 4.52 - 4.40 (m, 1H), 4.36 - 4.11 (m, 4H), 4.07 - 3.88 (m, 1H), 3.76 (s, 2H), 3.70 - 3.43 (m, 34H), 3.36 (t, J = 6.0 Hz, 2H), 3.30 - 3.26 (m, 1H), 3.20 - 3.11 (m, 6H), 3.10 - 2.92 (m, 9H), 2.91 - 2.66 (m, 4H), 2.59 - 2.44 (m, 2H), 2.42 - 2.26 (m, 3H), 2.07 - 1.90 (m, 2H), 1.84 - 1.69 (m, 2H), 1.67 - 1.34 (m, 8H), 1.15 - 0.91 (m, 12H), 0.87 (m, 6H), 0.73 (s, 3H), 0.62 (s, 3H).

### Example A-10. Synthesis of Compounds Z38-L1-A and Z38-L1-B

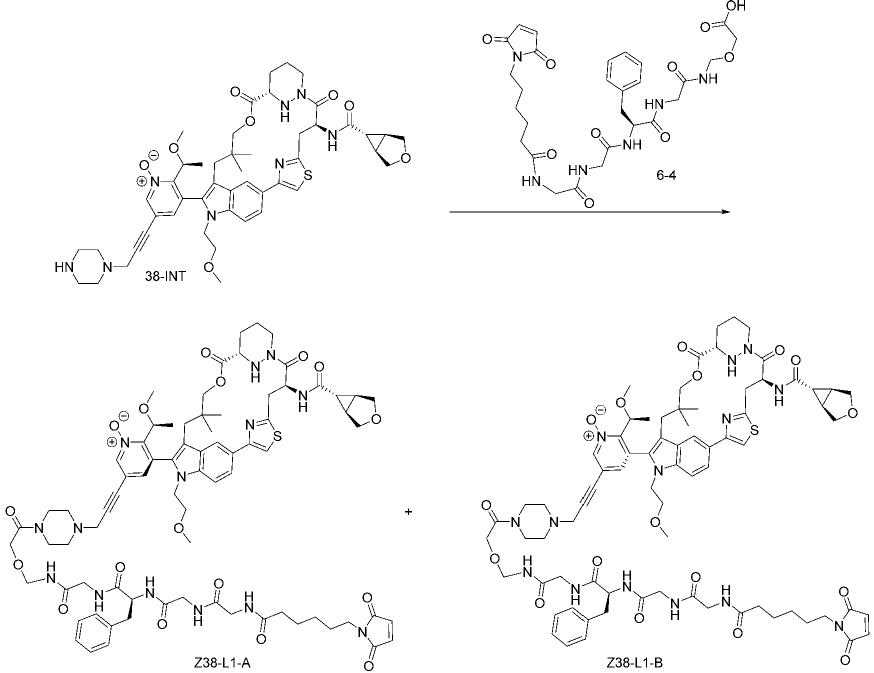

Step 1: Compound 38-INT (53 mg, 0.062 mmol) was dissolved in dichloromethane (3 mL), and compound 6-4 (27 mg, 0.044 mmol), O-(7-azabenzotriazol-1-yl)-N,N,N',N'-tetramethyluronium hexafluorophosphate (18 mg, 0.048 mmol), and N,N'-diisopropylethylamine (22 mg, 0.170 mmol) were added. The reaction system was stirred at room temperature for 1 h. Dichloromethane (20 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure to give a crude product. The crude product was purified by prep-HPLC (ammonium bicarbonate method 1) to give 2 isomeric compounds. The structure of one of the isomeric compounds was Z38-L1-A (5 mg, retention time: 5.03 min, yield: 9.8%) as a white solid. ES-API: [M+H]⁺= 1507.2. **1H** NMR (400 MHz, DMSO-*d₆*) δ 8.59 - 8.44 (m, 4H), 8.30 (t, J = 5.6 Hz, 1H), 8.13 (d, J = 8.0 Hz, 1H), 8.06 (t, J = 5.6 Hz, 1H), 8.00 (t, J = 5.6 Hz, 1H), 7.81 (s, 1H), 7.74 (d, J = 8.8 Hz, 1H), 7.59 (d, J = 8.8 Hz, 1H), 7.27 - 7.20 (m, 5H), 7.19 - 7.13 (m, 1H), 6.99 (s, 2H), 5.58 (t, J = 8.8 Hz, 1H), 5.10 (d, J = 12.0 Hz, 1H), 4.57 (d, J = 6.8 Hz, 2H), 4.54 - 4.41 (m, 2H), 4.41 - 4.33 (m, 1H), 4.29 - 4.07 (m, 5H), 3.87 - 3.78 (m, 2H), 3.78 - 3.54 (m, 12H), 3.50 - 3.41 (m, 2H), 3.39 - 3.24 (m, 7H), 3.19 - 3.03 (m, 5H), 3.00 - 2.89 (m, 4H), 2.84 - 2.71 (m, 2H), 2.56 - 2.51 (m, 5H), 2.15 - 2.01 (m, 3H), 1.98 - 1.86 (m, 2H), 1.83 - 1.69 (m, 2H), 1.64 (t, J = 3.2 Hz, 1H), 1.57 - 1.40 (m, 8H), 1.24 - 1.13 (m, 2H), 0.94 (s, 3H), 0.52 (s, 3H). The structure of the other isomeric compound was Z38-L1-B (10 mg, retention time: 5.11 min, yield: 19.5%) as a white solid. ES-API: [M+H]⁺= 1507.2. ¹H NMR (400 MHz, DMSO-*d₆*) δ 8.62 - 8.49 (m, 3H), 8.47 (s, 1H), 8.30 (t, *J* = 5.6 Hz, 1H), 8.12 (d, *J* = 8.0 Hz, 1H), 8.05 (t, *J =* 5.6 Hz, 1H), 8.00 (t, *J* = 5.6 Hz, 1H), 7.80 (s, 1H), 7.71 (dd, *J =* 8.8, 1.2 Hz, 1H), 7.53 (d, *J =* 8.8 Hz, 1H), 7.45 (d, *J =* 1.2 Hz, 1H), 7.28 - 7.20 (m, 4H), 7.19 - 7.13 (m, 1H), 6.98 (s, 2H), 5.53 (t, *J =* 9.2 Hz, 1H), 5.04 (d, *J =* 12.0 Hz, 1H), 4.67 - 4.53 (m, 3H), 4.53 - 4.45 (m, 1H), 4.31 - 4.06 (m, 5H), 4.03 - 3.91 (m, 1H), 3.88 - 3.80 (m, 2H), 3.77 - 3.53 (m, 14H), 3.51 - 3.41 (m, 2H), 3.40 - 3.30 (m, 4H), 3.16 - 2.93 (m, 9H), 2.86 - 2.71 (m, 2H), 2.55 - 2.48 (m, 4H), 2.23 - 2.06 (m, 4H), 1.99 - 1.87 (m, 2H), 1.84 - 1.70 (m, 2H), 1.66 (t, *J* = 3.2 Hz, 1H), 1.58 - 1.38 (m, 5H), 1.34 - 1.13 (m, 6H), 0.96 (s, 3H), 0.53 (s, 3H).

### Example A-11. Synthesis of Compounds Z38-L2-A and Z38-L2-B

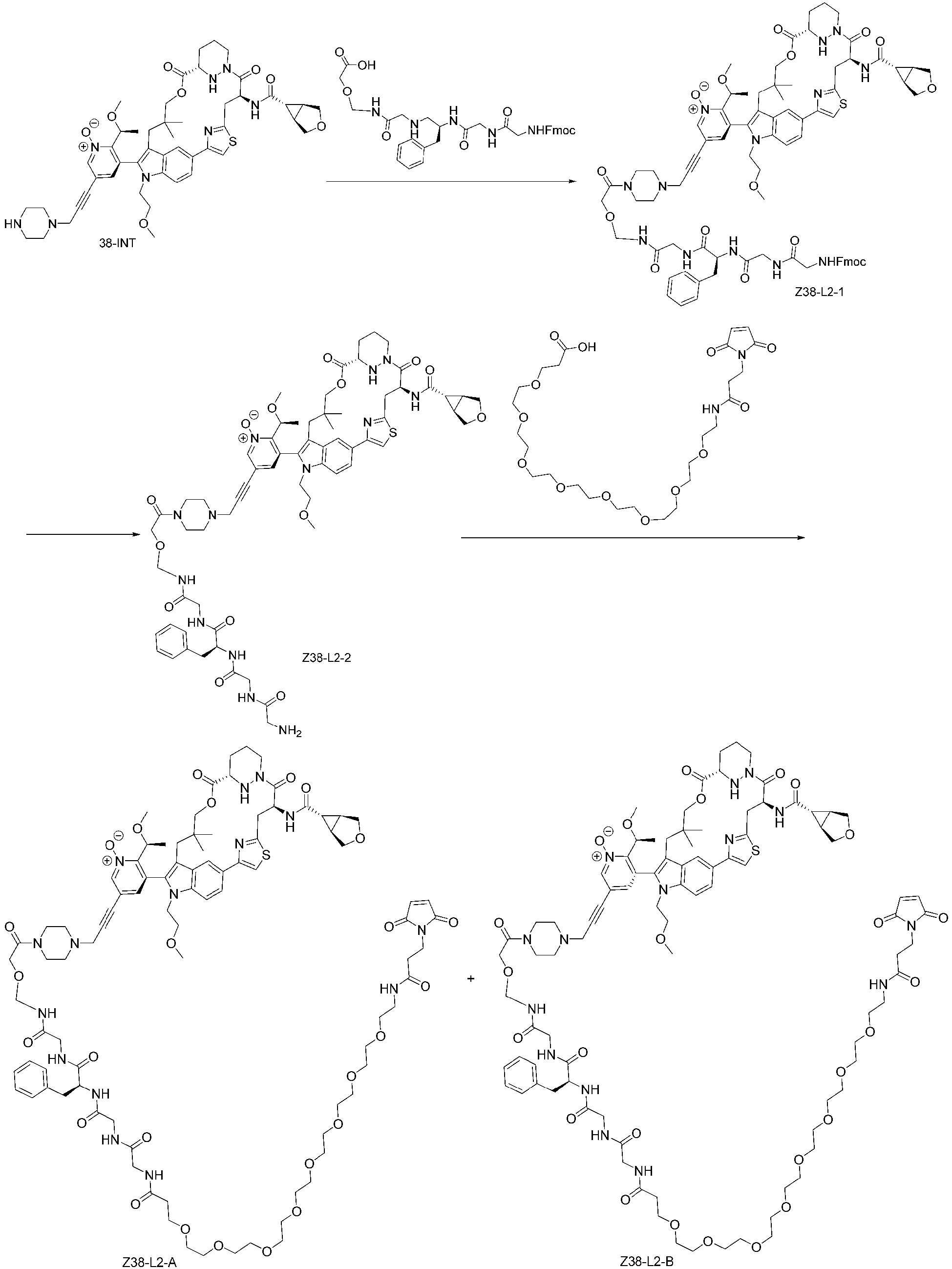

Step 1: Compound 38-INT (90 mg, 0.099 mmol) was dissolved in dichloromethane (10 mL), and (S)-11-benzyl-1-(9H-fluoren-9-yl)-3,6,9,12,15-pentaoxo-2,18-dioxa-4,7,10,13,16-pentaazaeicosan-20-oic acid (81 mg, 0.129 mmol), O-(7-azabenzotriazol-1-yl)-N,N,N',N'-tetramethyluronium hexafluorophosphate (53 mg, 0.139 mmol), and N,N'-diisopropylethylamine (64 mg, 0.495 mmol) were added at room temperature. The reaction system was stirred under an ice bath for 18 h. The reaction mixture was concentrated under reduced pressure. The crude product was purified by a flash silica gel column (methanol/dichloromethane: 0-20%) to give compound Z38-L2-1 (80 mg, yield: 52.6%) as a pale yellow solid. ES-API: [M+H]⁺= 1536.4.

Step 2: Compound Z38-L2-1 (70 mg, 0.046 mmol) was dissolved in N,N-dimethylformamide (3 mL), and 1,8-diazacyclo[5,4,0]undecene-7 (14 mg, 0.091 mmol) was added at 0 °C. The reaction system was stirred at 0 °C for 30 min. The reaction mixture was adjusted to pH 7 with 3 M diluted hydrochloric acid to give a reaction mixture of compound Z38-L2-2, which was directly used in the next step. ES-API:[M+H]⁺= 1314.3.

Step 3: 1-Maleimido-3-oxo-7,10,13,16,19,22,25,28-octaoxa-4-aza-hentriacontan-31-oic acid (34.58 mg, 0.058 mmol), O-(7-azabenzotriazol-1-yl)-N,N,N',N'-tetramethyluronium hexafluorophosphate (22 mg, 0.058 mmol), and N,N'-diisopropylethylamine (29 mg, 0.224 mmol) were added to the reaction mixture of compound Z38-L2-2 described above. The reaction system was stirred at room temperature for 1 h. The reaction mixture was purified by prep-HPLC (ammonium bicarbonate method 1) to give 2 isomeric compounds. The structure of one of the isomeric compounds was Z38-L2-A (15 mg, retention time: 4.88 min, two-step yield: 17.7%) as a white solid. ES-API: [M+H]⁺= 1888.9. The structure of the other isomeric compound was Z38-L2-B (22 mg, retention time: 4.95 min, yield: 25.9%) as a white solid. ES-API: [M+H]⁺= 1888.9.

### Example A-12. Synthesis of Compound Z52-L7

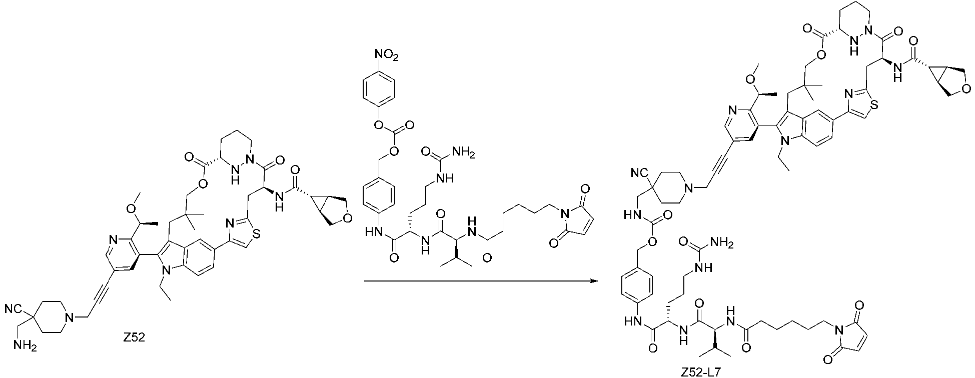

Step 1: Compound Z52 (25 mg, 0.027 mmol) and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl)carbonate (24.16 mg, 0.033 mmol, prepared by referring to the method in WO2019108797 A1) were dissolved in dry N,N-dimethylformamide (2 mL), and DIPEA (17.63 mg, 0.136 mmol) was added. The mixture was reacted at room temperature for 18 h. After the reaction was completed, the mixture was purified by prep-HPLC (ammonium bicarbonate method 1) to give the desired product Z52-L7 (28 mg, yield: 66.38%). ES-API: [M+H]⁺=1514.4. ¹H NMR (400 MHz, DMSO-d₆) δ 9.96 (s, 1H), 8.80 (d, J = 2.1 Hz, 1H), 8.55 (d, J = 8.9 Hz, 1H), 8.50 (s, 1H), 8.06 (d, J = 7.5 Hz, 1H), 7.84 (d, J = 2.2 Hz, 1H), 7.82 - 7.67 (m, 4H), 7.58 (d, J = 8.2 Hz, 3H), 7.28 (d, J = 8.2 Hz, 2H), 6.99 (s, 2H), 5.96 (t, J = 5.7 Hz, 1H), 5.56 (t, J = 9.0 Hz, 1H), 5.39 (s, 2H), 5.07 (d, J = 12.1 Hz, 1H), 4.96 (s, 2H), 4.38 - 4.17(m, 6H), 4.10 - 4.04 (m, 1H), 3.83 (t, J = 8.2 Hz, 2H), 3.65 - 3.59 (m, 4H), 3.57 (s, 2H), 3.36 (t, J = 7.0 Hz, 2H), 3.30 - 3.25 (m, 5H), 3.17 - 3.11 (m, 1H), 3.04-2.87(m, 5H), 2.75 - 2.73 (m, 1H), 2.48 - 2.31 (m, 5H), 2.20 - 2.06 (m, 4H), 1.98 - 1.76 (m, 7H), 1.67-1.64 (m, 1H), 1.60 - 1.45 (m, 8H), 1.35 (d, J = 6.1 Hz, 3H), 1.24-1.16 (m, 3H), 0.90 - 0.81 (m, 12H), 0.34 (s, 3H).

### Example A-13. Synthesis of Compound Z47-L5

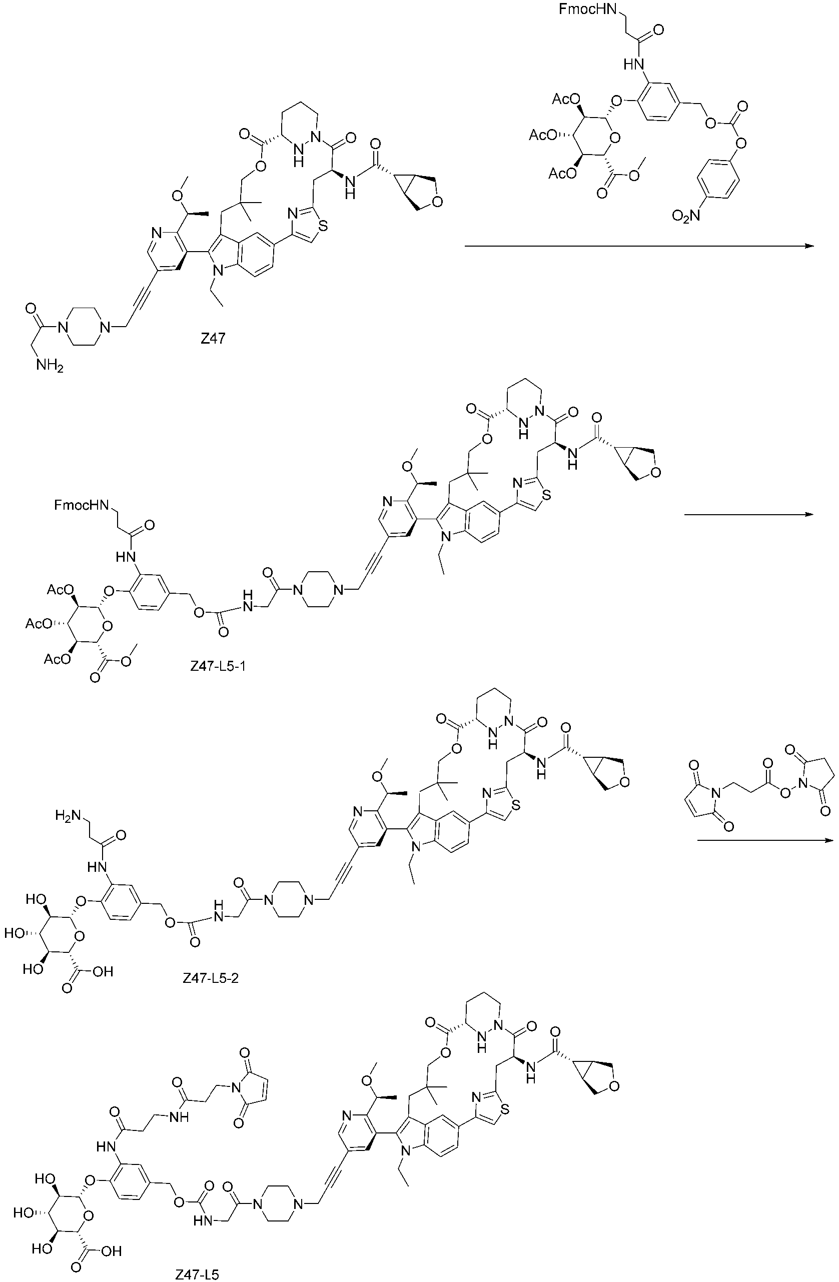

Step 1: Compound Z47 (100 mg, 0.109 mmol) and methyl (2-{[1-(9H-fluoren-9-yl)-3,7-dioxo-4-aza-2-oxahept-7-yl]amino}-1-{[(2S,3R,4S,5S,6S)-3,4,5-triacetoxy-6-(methoxycarbonyl)-3,4,6-tetrahydro-2H-pyran-2-yl]oxy}phen-4-yl)methyl[(4-nitrophenyl)oxy]formate (99.61 mg, 0.109 mmol) (for synthesis steps, refer to WO2021050917) were dissolved in N,N-dimethylformamide (5 mL) and pyridine (1 mL), and N,N'-diisopropylethylamine (0.036 mL, 0.217 mmol) and 1-hydroxybenzotriazole (14.69 mg, 0.109 mmol) were added. After the addition was completed, the reaction system was stirred at room temperature for 12 h under a nitrogen atmosphere. After the reaction was completed, water (15 mL) was added, and the mixture was extracted with ethyl acetate (20 mL × 3). The organic phase was washed with saturated brine (15 mL), dried over anhydrous sodium sulfate, and concentrated. The crude product was purified by a flash silica gel column (tetrahydrofuran/petroleum ether: 0%-60%) to give compound Z47-L5-1 (110 mg, 0.065 mmol, yield: 59.72%) as a colorless oily liquid, ES-API: [M/2+H]⁺ = 847.7.

Step 2: Compound Z47-L5-1 (100 mg, 0.059 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z47-L5-2 (70 mg, 0.053 mmol, yield: 89.04%) as a colorless oily liquid, ES-API: [M/2+H]⁺ = 667.0.

Step 3: Compound Z47-L5-2 (70 mg, 0.037 mmol) was dissolved in N,N-dimethylformamide (1 mL), and N,N'-diisopropylethylamine (9.51 mg, 0.074 mmol) and N-succinimidyl 3-maleimidopropionate (11.75 mg, 0.044 mmol) were added. The reaction system was stirred at room temperature for 3 h under a nitrogen atmosphere and purified by prep-HPLC (formic acid method 1) to give the desired product Z47-L5 (25 mg, 0.017 mmol, yield: 45.82%, formate) as a white solid, ES-API: [M/2+H]⁺ = 742.4. ¹H NMR (400 MHz, DMSO-d₆) δ 9.10 (s, 1H), 8.82 (d, *J =* 2.0 Hz, 1H), 8.58 (d, *J =* 8.8 Hz, 1H), 8.50 (s, 1H), 8.16 (s, 1H), 8.11 (t, *J =* 5.6 Hz, 1H), 7.86 (d, *J* = 2.0 Hz, 1H), 7.82 (s, 1H), 7.76 (d, *J* = 8.4 Hz, 1H), 7.58 (d, *J* = 8.4 Hz, 1H), 7.20 (t, *J* = 5.6 Hz, 1H), 7.08 (d, *J* = 8.4 Hz, 1H), 7.02 (d, *J =* 7.6 Hz, 1H), 6.98 (s, 2H), 5.82 (s, 1H), 5.56 (t, *J* = 9.2 Hz, 1H), 5.27 (s, 1H), 5.09 (d, *J* = 12.0 Hz, 1H), 4.93 (s, 2H), 4.81 (d, *J =* 7.2 Hz, 1H), 4.36 - 4.04 (m, 5H), 3.89 - 3.80 (m, 5H), 3.66 - 3.56 (m, 8H), 3.51 - 3.40 (m, 8H), 3.34-3.29 (m, 6H), 3.25 (s, 3H), 3.16 - 3.10 (m, 1H), 2.97-2.95 (m, 1H), 2.78-2.73 (m, 1H), 2.56-2.53 (m, 3H), 2.43 - 2.26 (m, 4H), 2.08 (d, *J =* 10.6 Hz, 1H), 1.95-1.90 (m, 2H), 1.81-1.75 (m, 2H), 1.65 (t, *J* = 2.8 Hz, 1H), 1.56-1.46 (m, 1H), 1.35 (d, *J* = 6.0 Hz, 3H), 0.95-0.85 (m, 6H), 0.35 (s, 3H).

### Example A-14. Synthesis of Compound Z4-L7

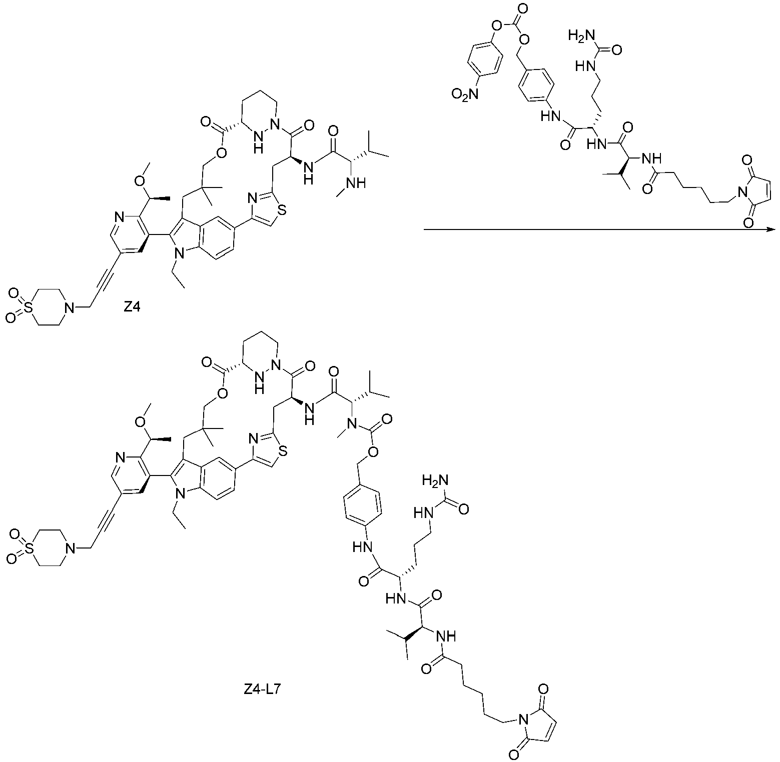

Step 1: Compound Z4 (30 mg, 0.033 mmol) and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl)carbonate (44 mg, 0.059 mmol, prepared by referring to the method in WO2019108797 A1) were dissolved in N,N-dimethylformamide (2.5 mL), and 1-hydroxybenzotriazole (5 mg, 0.033 mmol) was added at room temperature, followed by the addition of a solution of 2,6-lutidine (11 mg, 0.098 mmol) in N,N-dimethylformamide (0.2 mL). The reaction system was stirred at room temperature for 18 h. Ethyl acetate (50 mL) was added to the reaction mixture, and the mixture was sequentially washed with water (15 mL) and saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-8%) to give a crude product. The crude product was purified by prep-HPLC (ammonium bicarbonate 1) to give the desired product Z4-L7 (26 mg, yield: 52.4%) as a white solid. ES-API: [M+H]⁺=1513.2. ¹H NMR (400 MHz, DMSO-*d₆*) δ 10.12 - 9.91 (m, 1H), 8.81 (d, *J* = 2.0 Hz, 1H), 8.64 - 8.53 (m, 1H), 8.49 (s, 1H), 8.09 (d, *J* = 7.2 Hz, 1H), 7.87 (s, 1H), 7.84 - 7.72 (m, 3H), 7.68 - 7.55 (m, 3H), 7.43 - 7.29 (m, 2H), 6.99 (s, 2H), 5.95 (t, *J =* 6.0 Hz, 1H), 5.53 - 5.34 (m, 3H), 5.25 - 5.01 (m, 3H), 4.45 - 4.16 (m, 7H), 4.14 - 4.02 (m, 1H), 3.76 (s, 2H), 3.63 - 3.48 (m, 2H), 3.39 - 3.33 (m, 3H), 3.31 - 3.22 (m, 5H), 3.20 - 3.13 (m, 4H), 3.07 - 2.90 (m, 7H), 2.88 - 2.71 (m, 4H), 2.37 (d, *J* = 14.0 Hz, 1H), 2.23 - 2.04 (m, 4H), 2.01 - 1.91 (m, 1H), 1.86 - 1.67 (m, 3H), 1.64 - 1.32 (m, 11H), 1.23 - 1.10 (m, 2H), 0.96 - 0.75 (m, 18H), 0.33 (s, 3H).

### Example A-15. Synthesis of Compound Z4-L8

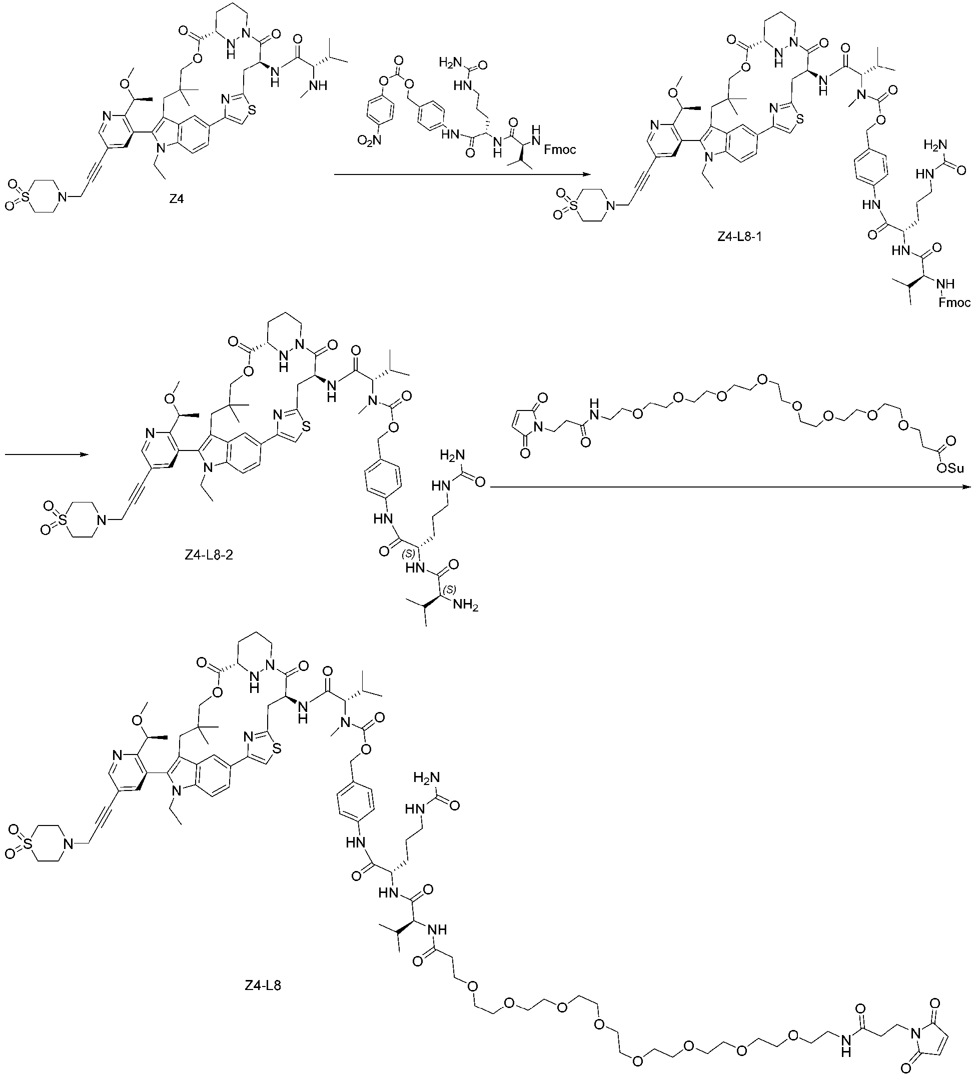

Step 1: Compound Z4 (60 mg, 0.066 mmol) and (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (75 mg, 0.098 mmol, prepared by referring to the method in WO201544003 A1) were dissolved in N,N-dimethylformamide (3 mL), and 1-hydroxybenzotriazole (9 mg, 0.066 mmol) was added at room temperature, followed by the addition of a solution of 2,6-lutidine (21 mg, 0.197 mmol) in N,N-dimethylformamide (0.3 mL). The reaction system was stirred at room temperature for 18 h. Ethyl acetate (50 mL) was added to the reaction mixture, and the mixture was sequentially washed with water (15 mL) and saturated brine (20 mL × 3). The organic phase was dried over anhydrous sodium sulfate and filtered, and the filtrate was concentrated under reduced pressure. The crude product was purified by a flash silica gel column (methanol/dichloromethane: 0-8%) to give compound Z4-L8-1 (80 mg, yield: 79.1%) as a pale yellow solid. ES-API: [M+H]⁺=1542.2.

Step 2: Compound Z4-L8-1 (70 mg, 0.045 mmol) was dissolved in a 2.0 M solution of dimethylamine in tetrahydrofuran (6 mL). The mixture was stirred at room temperature for 1 h. The reaction mixture was concentrated under reduced pressure. The crude product was purified by a flash silica gel column (7.0 M ammonia-methanol/dichloromethane: 0-15%) to give compound Z4-L8-2 (45 mg, yield: 75.1%) as a pale yellow solid. ES-API: [M+H]⁺=1320.2.

Step 3: Compound Z4-L8-2 (45 mg, 0.034 mmol) was dissolved in N,N-dimethylformamide (3 mL), and 2,5-dioxopyrrolidin-1-yl 1-(2,5-dioxo-2,5-dihydro-1H-pyrrol-1-yl)-3-oxo-7,10,13,16,19,22,25,28-octaoxa-4-azahentriacontan-31-oate (42 mg, 0.061 mmol) and N,N'-diisopropylethylamine (13 mg, 0.102 mmol) were added at room temperature. The reaction system was stirred at room temperature for 2 h. The reaction mixture was purified by prep-HPLC (ammonium bicarbonate method 1) to give the desired product Z4-L8 (30 mg, yield: 46.5%) as a white solid. ES-API: [M+H]⁺= 1895.3. ¹H NMR (400 MHz, DMSO-*d₆*) δ 10.09 - 9.96 (m, 1H), 8.81 (d, *J* = 2.0 Hz, 1H), 8.67 - 8.52 (m, 1H), 8.49 (s, 1H), 8.12 (d, *J =* 7.2 Hz, 1H), 8.01 (t, *J =* 5.2 Hz, 1H), 7.92 - 7.71 (m, 4H), 7.69 - 7.54 (m, 3H), 7.43 - 7.27 (m, 2H), 7.00 (s, 2H), 5.97 (t, *J* = 5.6 Hz, 1H), 5.53 - 5.33 (m, 3H), 5.25 - 5.02 (m, 3H), 4.46 - 4.15 (m, 7H), 4.14 - 4.02 (m, 1H), 3.76 (s, 2H), 3.63 - 3.44 (m, 34H), 3.39 - 3.34 (m, 3H), 3.29 - 3.24 (m, 4H), 3.19 - 3.10 (m, 6H), 3.07 - 2.91 (m, 7H), 2.88 - 2.72 (m, 4H), 2.48 - 2.43 (m, 1H), 2.41 - 2.28 (m, 4H), 2.14 - 1.92 (m, 3H), 1.87 - 1.68 (m, 3H), 1.64 - 1.32 (m, 7H), 0.97 - 0.76 (m, 18H), 0.33 (s, 3H).

### Example A-16. Synthesis of Compound Z52-L8

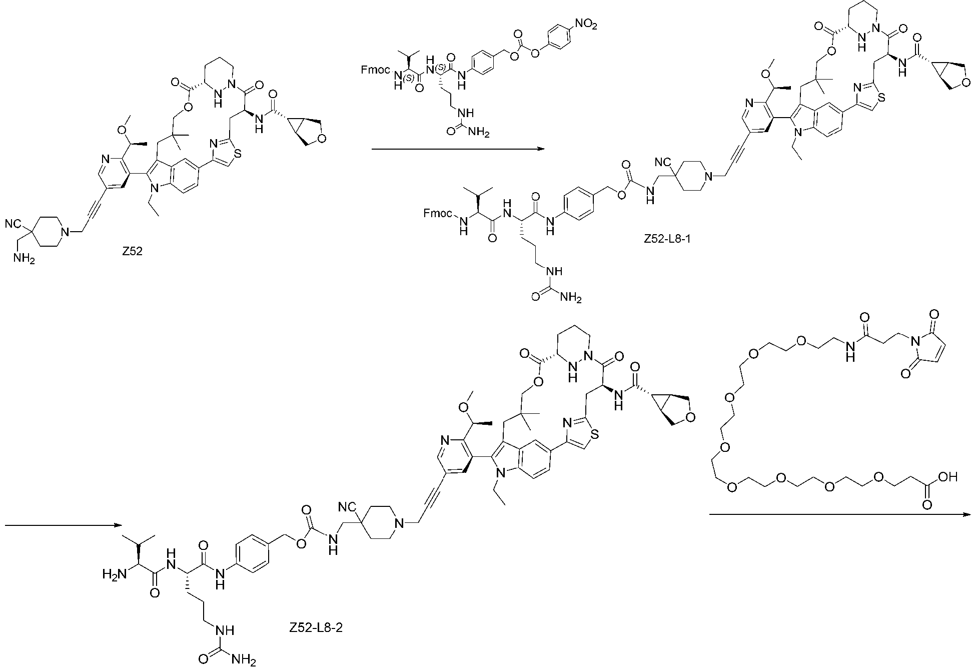

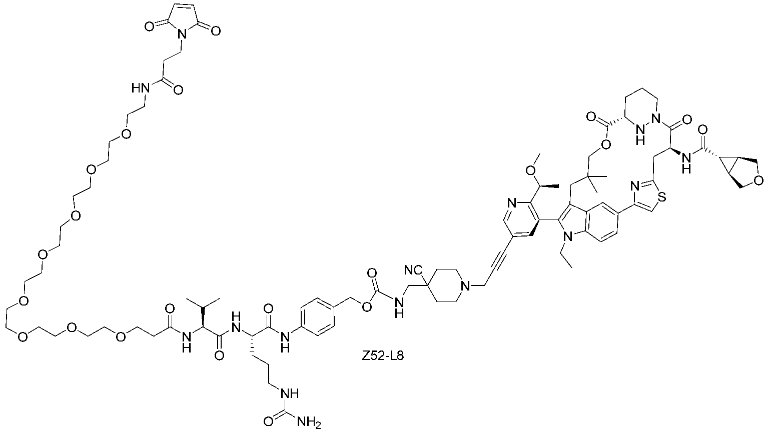

Step 1: Compound Z52 (20 mg, 0.022 mmol) and (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (20.09 mg, 0.026 mmol, prepared by referring to the method in WO201544003 A1) were dissolved in dry N,N-dimethylformamide (0.5 mL), and DIPEA (14.11 mg, 0.109 mmol) was added. The mixture was reacted at room temperature for 18 h. After the reaction was completed, the reaction mixture was purified by prep-HPLC (trifluoroacetic acid method) to give compound Z52-L8-1 (20 mg, 0.013 mmol, yield: 59%). ES-API: [M+H]⁺= 1543.5.

Step 2: Compound Z52-L8-1 (20 mg, 0.013 mmol) was dissolved in N,N-dimethylformamide (1 mL). The mixture was cooled under ice-brine (< 0 °C), and DBU (3.94 mg, 0.026 mmol) was slowly added. The reaction system was stirred for 5 min while maintaining the ice-water bath. After the reaction was completed as detected, 1 M diluted hydrochloric acid was added for neutralization to give a reaction mixture of compound Z52-L8-2, which was directly used in the next step. ES-API: [M+H]⁺=1321.4.

Step 3: 1-Maleimido-3-oxo-7,10,13,16,19,22,25,28-octaoxa-4-aza-hentriacontan-31-oic acid (13.45 mg, 0.023 mmol), N,N-diisopropylethylamine (5.86 mg, 0.045 mmol), and PyBOP (15.75 mg, 0.030 mmol) were directly and sequentially added to the reaction mixture of compound Z52-L8-2 described above. The mixture was reacted at room temperature for 10 min and directly purified by prep-HPLC (trifluoroacetic acid method) to give product Z52-L8 (1.9 mg, yield: 7.7%, trifluoroacetate). ES-API: [M+H]⁺=1896.9.

### Example A-17. Synthesis of Compound Z61-L7

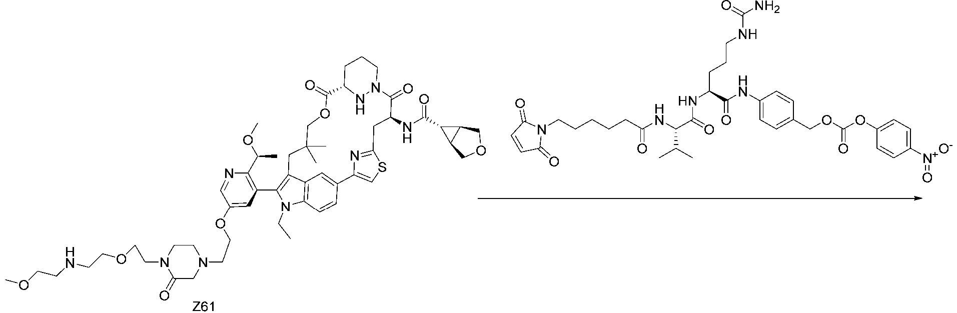

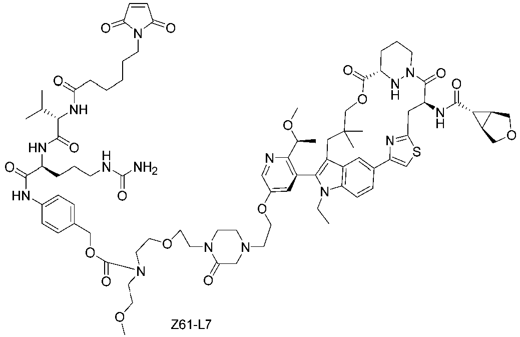

Step 1: Compound Z61 (70 mg, 0.068 mmol) was dissolved in N,N-dimethylformamide (1 mL), N,N'-diisopropylethylamine (17.6 mg, 0.136 mmol) was added, and maleimidocaproyl-L-valine-L-citrulline-p-aminobenzyl alcohol p-nitrophenyl carbonate (CAS: 159857-81-5, 75.34 mg, 0.102 mmol, prepared by referring to the method in WO2017134547 A1). The reaction system was stirred at room temperature for 3 h under a nitrogen atmosphere and purified by high performance liquid chromatography (trifluoroacetic acid method) to give the desired product Z61-L7 (45 mg, 0.028 mmol, yield: 40.63%, trifluoroacetate) as a white solid, ES-API: [M/2+H]⁺ = 814.0. ¹H NMR (400 MHz, DMSO-*d₆*) δ 10.06 (s, 1H), 8.58 (d, *J* = 8.8 Hz, 1H), 8.49 (s, 2H), 8.10 (d, *J* = 7.6 Hz, 1H), 7.89 - 7.79 (m, 2H), 7.74 (d, *J* = 8.4 Hz, 1H), 7.62-7.59 (m, 3H), 7.44-7.41 (m, 2H), 7.34 - 7.23 (m, 3H), 7.18 (s, 1H), 6.99 (s, 2H), 6.14 (s, 1H), 5.61-5.55 (m, 3H), 5.14 - 4.93 (m, 4H), 4.43 - 4.12 (m, 11H), 3.84 (d, *J* = 8.0 Hz, 3H), 3.63-3.60 (m, 10H), 3.48-3.41 (m, 5H), 3.23 - 3.17 (m, 7H), 3.12-2.95 (m, 4H), 2.78-2.76 (m, 1H), 2.22 - 1.87 (m, 7H), 1.81-1.69 (m, 5H), 1.52-1.41 (m, 11H), 1.23-1.18(m, 5H), 0.95 - 0.79 (m, 12H), 0.35 (s, 3H).

### Example A-18. Synthesis of Compound Z22-L25

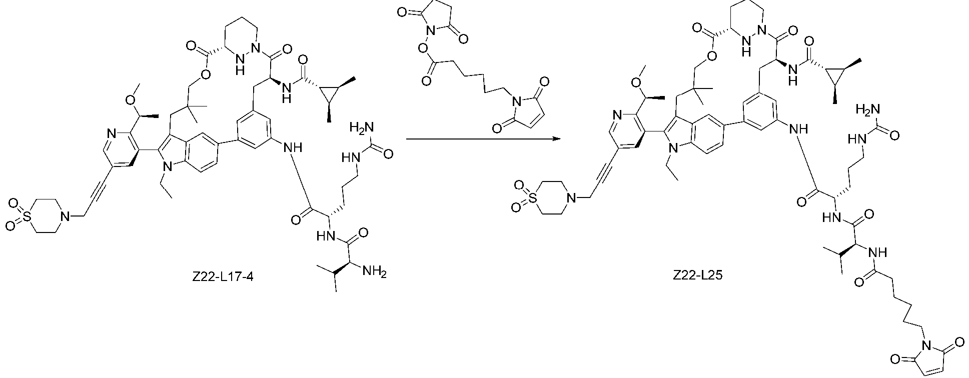

Step 1: Compound Z22-L17-4 (55 mg, 0.047 mmol) was dissolved in N,N-dimethylformamide (3 mL), and N-succinimidyl 6-maleimidohexanoate (22 mg, 0.071 mmol) and N,N'-diisopropylethylamine (18 mg, 0.142 mmol) were added at room temperature. The reaction system was stirred at room temperature for 2 h. The reaction mixture was purified by prep-HPLC (formic acid method 1) to give the desired product Z22-L25 (32 mg, yield: 49.9%, formate) as a white solid. ES-API: [M+H]⁺= 1355.3. ¹H NMR (400 MHz, DMSO-*d₆*) δ 9.96 (s, 1H), 8.82 (d, *J* = 2.0 Hz, 1H), 8.20 (d, *J =* 8.4 Hz, 1H), 8.07 (d, *J =* 7.8 Hz, 1H), 8.00 - 7.88 (m, 3H), 7.83 (d, *J =* 8.8 Hz, 1H), 7.66 - 7.49 (m, 3H), 7.34 (s, 1H), 6.99 (s, 2H), 5.99 (t, *J =* 5.6 Hz, 1H), 5.56 - 5.22 (m, 4H), 4.52 - 4.40 (m, 1H), 4.36 - 4.15 (m, 4H), 4.05 - 3.87 (m, 1H), 3.76 (s, 2H), 3.70 - 3.59 (m, 2H), 3.53 (d, *J* = 10.8 Hz, 1H), 3.36 (t, *J* = 7.2 Hz, 2H), 3.31 - 3.28 (m, 1H), 3.21 - 3.12 (m, 4H), 3.11 - 2.68 (m, 13H), 2.59 - 2.52 (m, 1H), 2.49 - 2.45 (m, 1H), 2.26 - 2.08 (m, 2H), 2.05 - 1.87 (m, 2H), 1.83 - 1.69 (m, 2H), 1.67 - 1.33 (m, 12H), 1.24 - 1.15 (m, 2H), 1.14 - 0.92 (m, 12H), 0.91 - 0.81 (m, 6H), 0.80 - 0.50 (m, 6H).

### Example A-19. Synthesis of Compound Z54-L1

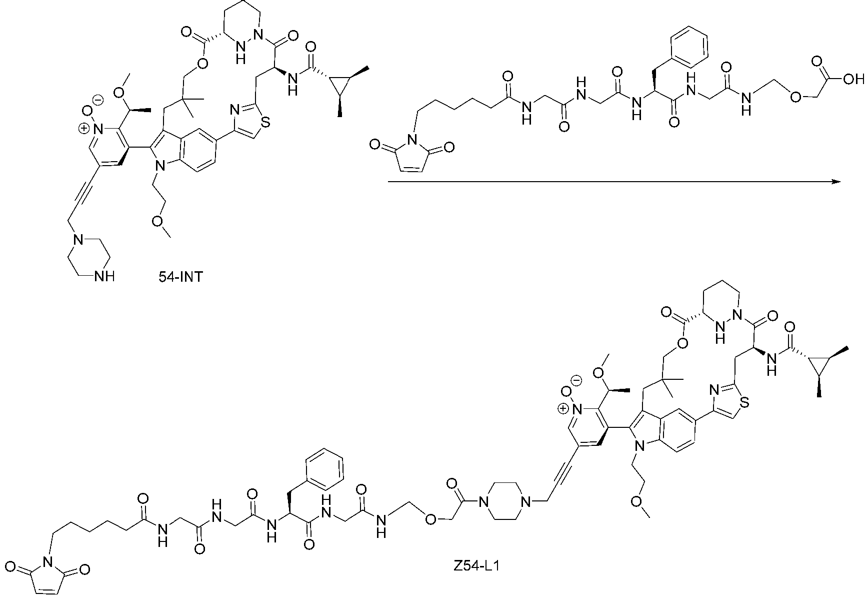

Step 1: Crude compound 54-INT (40 mg, 0.045 mmol), (S)-10-benzyl-23-(2,5-dioxo-2,5-dihydro-1H-pyrrol-1-yl)-6,9,12,15,18-pentaoxo-3-oxa-5,8,11,14,17-pentaazatricosanoic acid (36 mg, 0.058 mmol, prepared by referring to the method in WO2019195665A1), N,N-diisopropylethylamine (0.023 mL, 0.13 mmol), dichloromethane (3 mL), and O-(7-azabenzotriazol-1-yl)-N,N,N',N'-tetramethyluronium hexafluorophosphate (22.09 mg, 0.058 mmol) were sequentially added to a flask. The reaction system was stirred at room temperature for 1 h. The reaction mixture was concentrated, and the crude product was purified by prep-HPLC (formic acid method 1) to give the desired product Z54-L1 (25 mg, yield: 37.4%, formate) as a white solid. ES-API: [M+H]⁺= 1493.3. ¹HNMR (400MHz, DMSO-*d₆*):8.53-8.48 (m, 3H), 8.37-8.27(m, 2H), 8.12-7.98(m, 3H), 7.80 (s, 1H), 7.75-7.72 (m, 1H), 7.58 (d, *J*=8.4Hz, 1H), 7.30-7.15 (m, 6H), 7.00-6.97 (m, 2H), 5.58 (t, *J*=8.8Hz, 1H), 5.07(d, J=12.0Hz, 1H), 4.56(d, J=6.8Hz, 2H), 4.51-4.36 (m, 3H), 4.25-4.11 (m, 5H), 3.76-3.55 (m, 11H), 3.50-3.25 (m, 9H), 3.20-2.90 (m, 10H), 2.83-2.73 (m, 2H), 2.60-2.55 (m, 2H), 2.12-2.05 (m, 3H), 1.85-1.65 (m, 2H), 1.51-1.42 (m, 8H), 1.23-1.15 (m, 6H), 1.11-1.05 (m, 6H), 0.92 (s, 3H), 0.52 (s, 3H).

### Example A-20. Synthesis of Compound Z52-L9

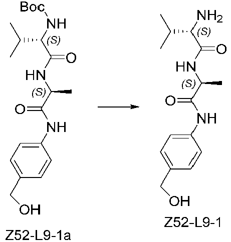

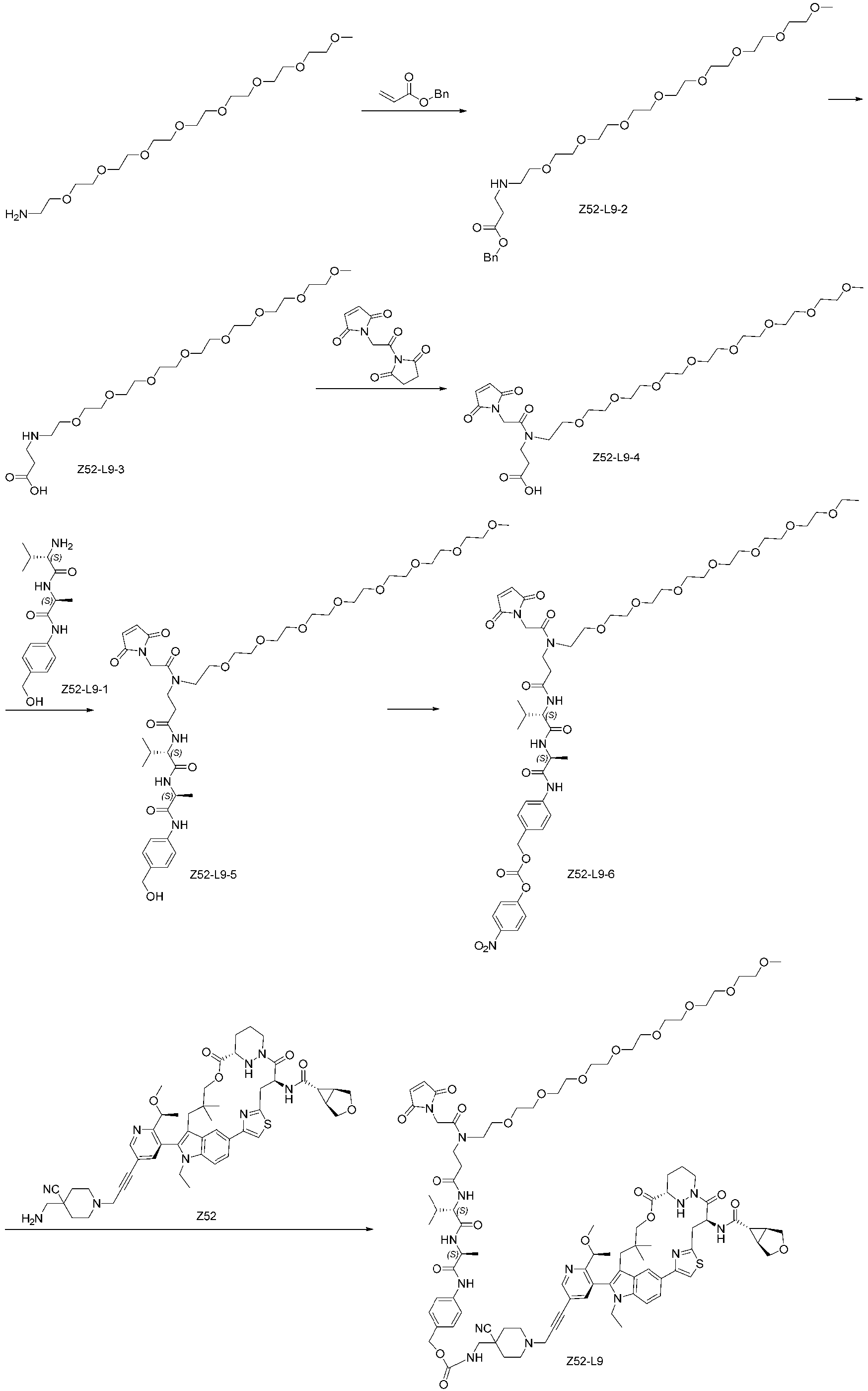

Step 1: 4 M hydrogen chloride-dioxane (2 mL, 8 mmol) was added to a solution of compound Z52-L9-1a (350 mg, 0.89 mmol, prepared by referring to the method in Journal of Medicinal Chemistry, 2021, vol. 64, # 7, p. 4117-4129) in methanol (2 mL). The mixture was stirred at room temperature for 1 h. After the reaction was completed, the reaction mixture was concentrated to give compound Z52-L9-1 (260 mg). ES-API[M+H] ⁺=294.2. Step 2: A mixture of 2-(2,5,8,11,14,17,20-heptyloxydocosyloxy)ethan-1-amine (450 mg, 1.173 mmol) and benzyl acrylate (190 mg, 1.17 mmol) in N,N-dimethylformamide (3 mL) and (2 mL) was stirred at room temperature overnight. The reaction mixture was dissolved in 30 mL of ethyl acetate, and the resulting mixture was washed three times with 10 mL of saturated brine. The organic phase was dried over anhydrous sodium sulfate, filtered, and purified by a flash silica gel column (0-20% methanol (1% ammonia water)/dichloromethane) to give compound Z52-L9-2 (100 mg, yield: 70.28%). ES-API[M+H] ⁺=546.3.

Step 3: A solution of compound Z52-L9-2 (550 mg, 1.008 mmol) and 10% palladium on carbon (150 mg, 0.14 mmol) in tetrahydrofuran (10 mL) was stirred at room temperature for 2 h under a hydrogen atmosphere. The reaction mixture was filtered through celite, washed with tetrahydrofuran, and concentrated to give compound Z52-L9-3 (400 mg, yield: 87%). ES-API[M+H] ⁺=456.3.

Step 4: N,N-Diisopropylethylamine (226 mg, 1.75 mmol) was added to a solution of 1-{2-[(2,5-dioxotetrahydro-1H-pyrrol-1-yl)oxy]-2-oxoethyl}pyrrole-2,5-dione (147 mg, 0.58 mmol) and compound Z52-L9-3 (266 mg, 0.58 mmol) in N,N-dimethylformamide (2 mL). The mixture was stirred at room temperature for 2 h. The reaction mixture was purified by prep-HPLC (trifluoroacetic acid method) to give compound Z52-L9-4 (200 mg, yield: 58%). ES-API[M+H] ⁺=593.2.

Step 5: Compound Z52-L9-1 (74 mg, 0.25 mmol), N,N-diisopropylethylamine (98 mg, 0.759 mmol), and O-(7-azabenzotriazol-1-yl)-N,N,N',N'-tetramethyluronium hexafluorophosphate (144 mg, 0.38 mmol) were added to a solution of compound Z52-L9-4 (150 mg, 0.25 mmol) in dichloromethane (2 mL). The mixture was stirred at room temperature for 1 h. After the reaction was completed, the reaction mixture was concentrated, and the residue was purified by a flash silica gel column (0-10% methanol/dichloromethane) to give compound Z52-L9-5 (140 mg, yield: 63.73%). ES-API[M+H] ⁺=868.2.

Step 6: Compound Z52-L9-5 (150 mg, 0.17 mmol) was dissolved in N,N-dimethylformamide (2 mL), and N,N-diisopropylethylamine (0.12 mL, 0.69 mmol) and bis(p-nitrophenyl) carbonate (63 mg, 0.21 mmol) were sequentially added. The mixture was stirred at room temperature for 2 h. The reaction mixture was dissolved in 10 mL of ethyl acetate, and the resulting mixture was sequentially washed with 5 mL of saturated brine and 5 mL of water. The organic phase was dried over anhydrous sodium sulfate, filtered, and concentrated, and the residue was purified by a flash silica gel column (0-50% tetrahydrofuran/petroleum ether) to give compound Z52-L9-6 (110 mg, yield: 62%). ES-API[M+H] ⁺=1033.3.

Step 7: Compound Z52-L9-6 (20 mg, 0.019 mmol) was dissolved in N,N-dimethylformamide (0.5 mL), and N,N-diisopropylethylamine (8 mg, 0.06 mmol) and compound Z52 (18 mg, 0.02 mmol) were sequentially added. The mixture was stirred at room temperature overnight. The reaction mixture was directly purified by prep-HPLC (ammonium bicarbonate method 1) to give the desired product Z52-L9 (10.6 mg, yield: 26%). ES-API[M/2+H⁺]⁺=905.5.

### Example A-21. Synthesis of Compound Z52-L10

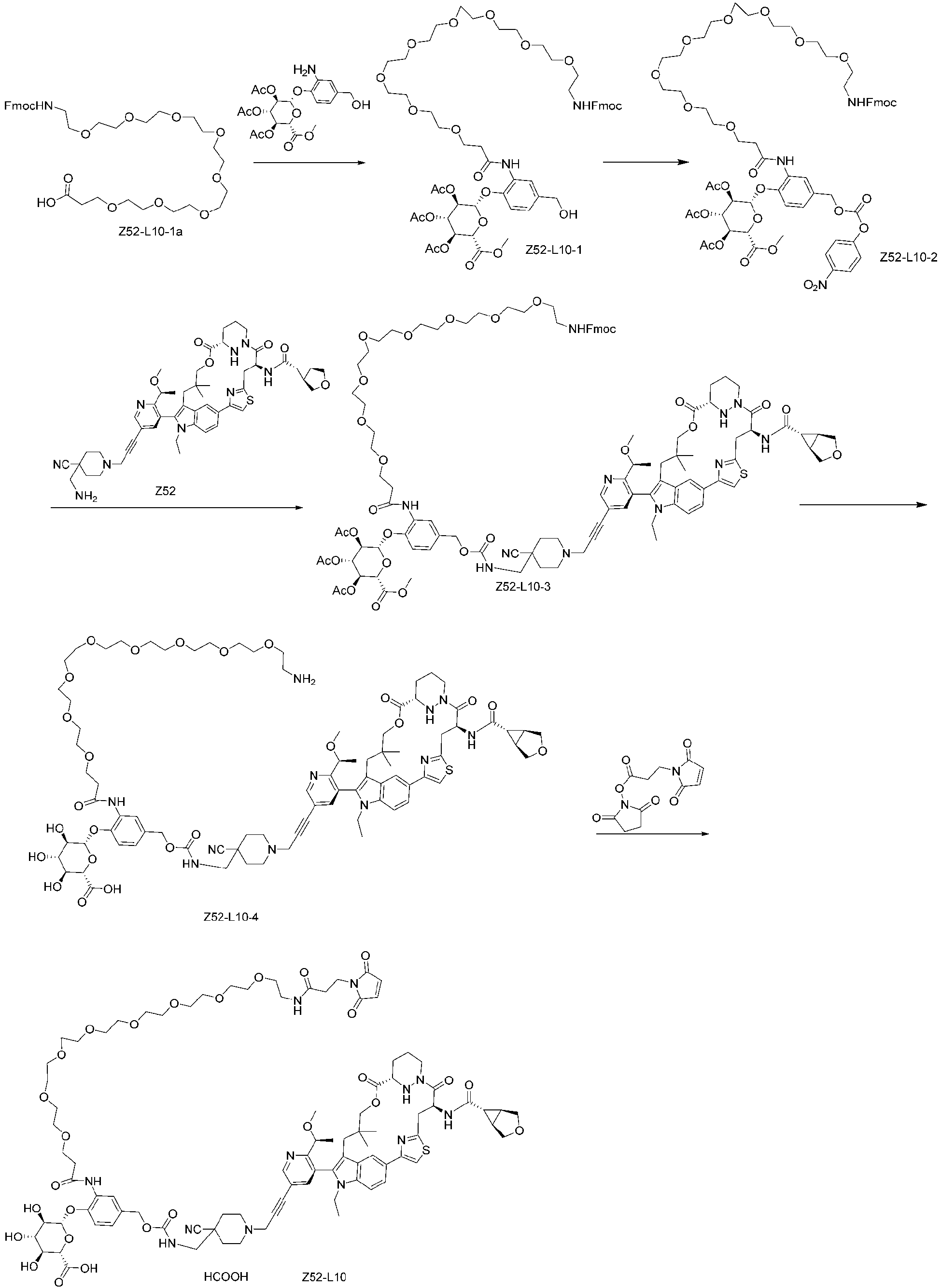

Step 1: Methyl (2S,3S,4S,5R,6S)-3,4,5-triacetoxy-6-{[2-amino-4-(hydroxymethyl)phenyl]oxy}tetrahydropyran-2-carboxylate (200 mg, 0.439 mmol) and compound Z52-L10-1a (360.56 mg, 1.159 mmol) were dissolved in dichloromethane (5 mL) and methanol (0.5 mL), and 2-ethoxy-1-ethoxycarbonyl-1,2-dihydroquinoline (217.20 mg, 0.878 mmol) was added. After the addition was completed, the reaction system was stirred at room temperature for 12 h under a nitrogen atmosphere and concentrated under reduced pressure. The crude product was purified by a flash silica gel column (tetrahydrofuran/petroleum ether: 0%-60%) to give compound Z52-L10-1 (350 mg, 0.318 mmol, yield: 72.38%) as a yellow solid, ES-API: [M+H]⁺ = 1101.3.

Step 2: Compound Z52-L10-1 (230 mg, 0.209 mmol) was dissolved in N,N-dimethylformamide (5 mL), and N,N'-diisopropylethylamine (54.03 mg, 0.418 mmol) and bis(p-nitrophenyl) carbonate (95.37 mg, 0.313 mmol) were added. After the addition was completed, the reaction system was stirred at room temperature for 12 h under a nitrogen atmosphere. After the reaction was completed, water (15 mL) was added, and the mixture was extracted with ethyl acetate (20 mL × 3). The organic phase was washed with saturated brine (15 mL), dried over anhydrous sodium sulfate, and concentrated. The crude product was purified by a flash silica gel column (tetrahydrofuran/petroleum ether: 0%-60%) to give compound Z52-L10-2 (200 mg, 0.158 mmol, yield: 75.62%) as a yellow solid, ES-API: [M+H]⁺ = 1266.3.

Step 3: Compound Z52 (80 mg, 0.087 mmol) and compound Z52-L10-2 (132.69 mg, 0.105 mmol) were dissolved in N,N-dimethylformamide (5 mL) and pyridine (1 mL), and N,N'-diisopropylethylamine (22.57 mg, 0.175 mmol) and 1-hydroxybenzotriazole (14.69 mg, 0.109 mmol) were added. After the addition was completed, the reaction system was stirred at room temperature for 12 h under a nitrogen atmosphere. After the reaction was completed, water (15 mL) was added, and the mixture was extracted with ethyl acetate (20 mL × 3). The organic phase was washed with saturated brine (15 mL), dried over anhydrous sodium sulfate, and concentrated. The crude product was purified by a flash silica gel column (tetrahydrofuran/petroleum ether: 0%-60%) to give compound Z52-L10-3 (110 mg, 0.054 mmol, yield: 61.65%) as a yellow solid, ES-API: [M/2+H]⁺ = 1021.9.

Step 4: Compound Z52-L10-3 (100 mg, 0.053 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z52-L10-4 (90 mg, 0.054 mmol, yield: 99.46%) as a colorless oily liquid, ES-API: [M/2+H]⁺ = 841.3.

Step 5: Compound Z52-L10-4 (70 mg, 0.042 mmol) was dissolved in N,N-dimethylformamide (1 mL), and N,N'-diisopropylethylamine (9.51 mg, 0.074 mmol) and N-succinimidyl 3-maleimidopropionate (22.17 mg, 0.083 mmol) were added. The reaction system was stirred at room temperature for 3 h under a nitrogen atmosphere and purified by prep-HPLC (formic acid method 1) to give the desired product Z52-L10 (14 mg, 0.008 mmol, yield: 19.86%, formate) as a white solid, ES-API: [M/2+H]⁺ = 916.3.

### Example A-22. Synthesis of Compound Z54-L2

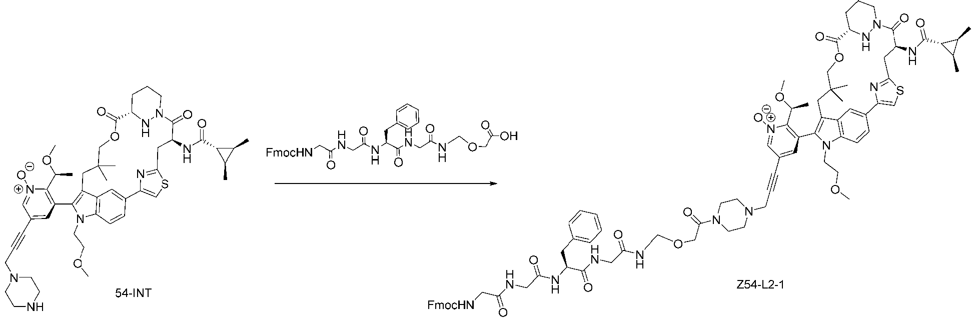

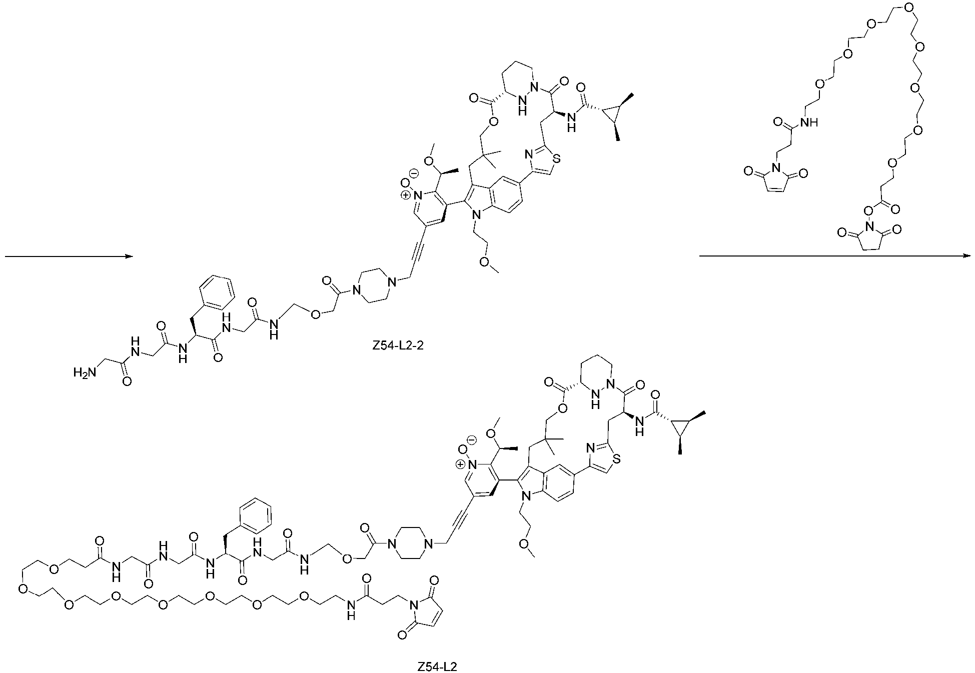

Step 1: Compound 54-INT (120 mg, 0.13 mmol), compound 34-2a (87 mg, 0.13 mmol), N,N-diisopropylethylamine (0.06 mL, 0.34 mmol), dichloromethane (5 mL), and O-(7-azabenzotriazol-1-yl)-N,N,N',N'-tetramethyluronium hexafluorophosphate (66.27 mg, 0.17 mmol) were sequentially added to a flask. The reaction system was stirred at room temperature for 1 h. The reaction mixture was quenched with saturated sodium bicarbonate, and the mixture was extracted with dichloromethane. The organic phase was washed with saturated brine, dried over anhydrous sodium sulfate, and filtered. The filtrate was concentrated to give crude compound Z54-L2-1 (170 mg, yield: 83.3%). ES-API: [M+H]⁺= 1522.6.

Step 2: Crude compound Z54-L2-1 (150 mg, 0.099 mmol) and N,N-dimethylformamide (4 mL) were sequentially added to a 25 mL flask. The reaction mixture was cooled to 0 °C, and 1,8-diazabicyclo[5.4.0]undec-7-ene (30 mg, 0.20 mmol) was added thereto. The reaction system was stirred at 0 °C for 30 min. The reaction mixture was adjusted to pH 7 with 2 M hydrochloric acid to give a reaction mixture of compound Z54-L2-2, which was directly used in the next step.

Step 3: The reaction mixture of compound Z54-L2-2 obtained in the previous step and 2,5-dioxopyrrolidin-1-yl 1-(2,5-dihydro-2,5-dihydro-1H-pyrrol-1-yl)-3-oxo-7,10,13,16,19,22,25,28-octaoxa-4-azahentriacontan-31-oate (88 mg, 0.13 mmol) were sequentially added to a flask. The reaction mixture was cooled to 0 °C, and N,N-diisopropylethylamine (25.44 mg, 0.20 mmol) was added thereto. The reaction system was stirred at room temperature for 1 h. The reaction mixture was cooled to 0 °C and adjusted to pH 7 with 1 M hydrochloric acid. Water was added thereto, and the mixture was extracted with dichloromethane. The organic phase was washed with saturated brine, dried over anhydrous sodium sulfate, and filtered. The filtrate was concentrated to give a crude product. The crude product was purified by prep-HPLC (formic acid method 1) to give the desired product Z54-L2 (45 mg, yield: 24.4%, formate) as a white solid. ES-API: [M+H]⁺= 1875.6. ¹HNMR (400MHz, DMSO-*d₆*): 8.53-8.45 (m, 3H), 8.37-8.27(m, 2H), 8.16-7.98(m, 2H), 8.01-7.98 (m, 2H), 7.86 (s, 1H), 7.75-7.67 (m, 1H), 7.58 (d, J=8.8Hz, 1H), 7.30-7.15 (m, 6H), 6.99 (s, 2H), 5.58 (t, J=8.8Hz, 1H), 5.07(d, J=12.0Hz, 1H), 4.57 (d, *J=6.4Hz,* 2H), 4.53-4.35 (m, 3H), 4.30-4.10(m, 5H), 3.80-3.55 (m, 15H), 3.52-3.45 (m, 32H), 3.40-3.29 (m, 7H), 3.20-2.90 (m, 11H), 2.83-2.65 (m, 2H), 2.60-2.55 (m, 2H), 2.40-2.31 (m, 4H), 2.10-2.05 (m, 1H), 1.85-1.65 (m, 2H), 1.60-1.42 (m, 4H), 1.30-1.10 (m, 9H), 0.94 (s, 3H), 0.52 (s, 3H).

### Example A-23. Synthesis of Compound Z63-L1

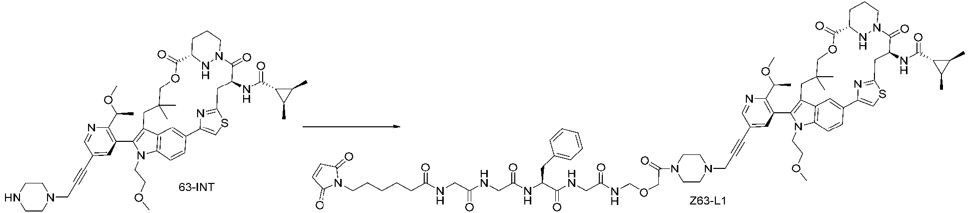

Step 1: Compound 63-INT (62.0 mg, crude product) was dissolved in dichloromethane (3.0 mL), and {[(7S)-7-benzyl-20-(2,5-dioxo-2,5-dihydro-1H-pyrrol-1-yl)-3,6,9, 12, 15-pentaoxo-2,5,8, 11, 14-pentaaza-1-yl]oxy} acetic acid (50.0 mg, 0.080 mmol, prepared by referring to the method in WO2019195665 A1), O-(7-azabenzotriazol-1-yl)-N,N,N',N'-tetramethyluronium hexafluorophosphate (40.0 mg, 0.105 mmol), and *N,N'-*diisopropylethylamine (37.0 mg, 0.286 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried, and concentrated to give a crude product, which was purified by prep-HPLC (ammonium bicarbonate method 1) to give the desired product Z63-L1 (50.08 mg, yield: 56.48%) as a white solid. ES-API: [M +H]⁺=1477.5. ¹H NMR (400 MHz, DMSO-*d₆*) δ 8.79 - 8.77 (m, 1H), 8.51 - 8.48 (m, 2H), 8.40 - 8.38 (m, 1H), 8.29 - 8.27 (m, 1H), 8.12 - 8.10 (m, 1H), 8.06 - 8.04 (m, 1H), 8.00 - 7.98 (m, 1H), 7.81 (s, 1H), 7.76 - 7.69 (m, 2H), 7.60 (s, 1H), 7.25 - 7.22 (m, 4H), 7.20 - 7.13 (m, 1H), 6.98 (s, 2H), 5.58 - 5.56(m, 1H), 5.07 - 5.04 (m, 1H), 4.58 - 4.55 (m, 2H), 4.52 - 4.37 (m, 2H), 4.31 - 4.05 (m, 6H), 3.67 - 3.58 (m, 10H), 3.47 (s, 2H), 3.38 - 3.34 (m, 4H), 3.26 - 3.22(m, 4H), 3.18 - 3.15 (m, 1H), 3.08 - 3.03(m, 1H), 2.96 - 2.94 (m, 1H), 2.88 (s, 3H), 2.84 - 2.71 (m, 2H), 2.42 - 2.33 (m, 1H), 2.10 - 2.08 (m, 3H), 1.78 (s, 2H), 1.50 - 1.43 (m, 5H), 1.36 - 1.33 (m, 3H), 1.27 - 1.12 (m, 6H), 1.09 - 1.05 (m, 7H), 0.90 (s, 3H), 0.34 (s, 3H).

### Example A-24. Synthesis of Compound Z31-L1

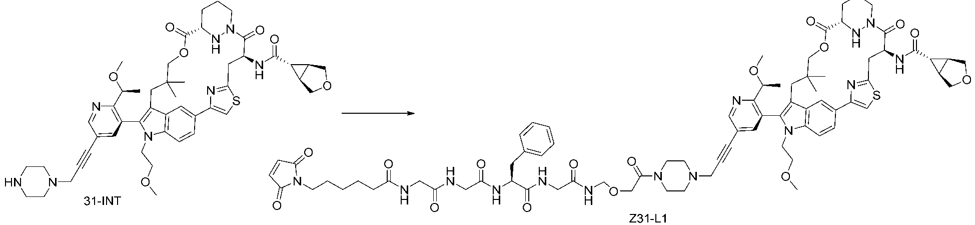

Step 1: Compound 31-INT (58.0 mg, crude product) was dissolved in dichloromethane (3.0 mL), and {[(7S)-7-benzyl-20-(2,5-dioxo-2,5-dihydro-1H-pyrrol-1-yl)-3,6,9, 12, 15-pentaoxo-2,5,8, 11, 14-pentaaza-1-yl]oxy} acetic acid (50.0 mg, 0.080 mmol, prepared by referring to the method in WO2019195665 A1), O-(7-azabenzotriazol-1-yl)-N,N,N',N'-tetramethyluronium hexafluorophosphate (40.0 mg, 0.105 mmol), and *N,N'-*diisopropylethylamine (37.0 mg, 0.286 mmol) were added. The reaction system was stirred for 2 h under an ice-water bath. After the reaction was completed, water (10 mL) was added to the solution, and the mixture was extracted with ethyl acetate (30 mL × 2). The organic phase was washed with saturated brine (40 mL × 3), dried, and concentrated to give a crude product, which was purified by prep-HPLC (ammonium bicarbonate method 1) to give the desired product Z31-L1 (35.17 mg, yield: 43.72%) as a white solid. ES-API: [M +H]⁺=1477.5. ¹H NMR (400 MHz, DMSO-*d₆*) δ 8.79 - 8.76 (m, 1H), 8.60 - 8.47 (m, 3H), 8.29 - 8.27 (m, 1H), 8.12 - 8.10 (m, 1H), 8.07 - 8.04 (m, 1H), 8.02 - 7.98 (m, 1H), 7.81 (s, 1H), 7.74 - 7.72 (m, 2H), 7.62 - 7.58 (m, 1H), 7.24 - 7.20 (m, 4H), 7.20 - 7.13 (m, 1H), 6.98 (s, 2H), 5.58 - 5.55(m, 1H), 5.10 - 5.05(m, 1H), 4.60 - 4.57 (m, 2H), 4.53 - 4.38 (m, 2H), 4.30 - 4.10 (m, 6H), 3.86 - 3.80 (m, 2H), 3.76 - 3.54 (m, 12H), 3.45 (m, 2H), 3.40 - 3.35 (m, 5H), 3.24 (s, 4H), 3.18 - 2.90 (m, 4H), 2.88 (s, 3H), 2.85 - 2.70 (m, 2H), 2.40 - 2.35 (m, 1H), 2.15 - 2.05 (m, 3H), 1.98 - 1.86 (m, 2H), 1.78 (s, 2H), 1.66 - 1.64(m, 1H), 1.55 - 1.45(m, 5H), 1.40 - 1.35(m, 3H), 1.25 - 1.20(m, 2H), 0.90 (s, 3H), 0.34 (s, 3H).

### Example A-25. Synthesis of Compounds Z35-L1-A and Z35-L1-B

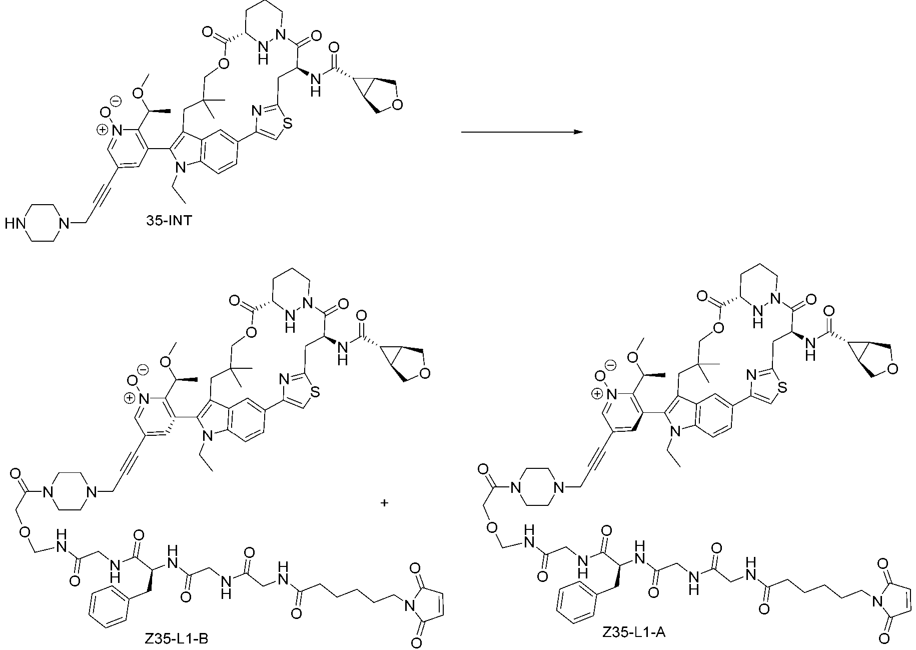

Step 1: Compound 35-INT (98 mg, 0.111 mmol) was dissolved in dichloromethane (8 mL), and compound 6-4 (103 mg, 0.167 mmol), O-(7-azabenzotriazol-1-yl)-N,N,N',N'-tetramethyluronium hexafluorophosphate (59 mg, 0.156 mmol), and N,N'-diisopropylethylamine (72 mg, 0.557 mmol) were added. The reaction system was stirred at room temperature for 1 h. Dichloromethane (30 mL) was added to the reaction mixture, and the mixture was washed sequentially with water (10 mL) and a saturated sodium bicarbonate solution (10 mL). The organic phase was dried over anhydrous sodium sulfate and filtered, and the filtrate was concentrated under reduced pressure to give a crude product. The crude product was purified by prep-HPLC (ammonium bicarbonate method 1) to give a crude compound (70 mg). The crude product was subjected to preparative chiral resolution (separation column: IB, 250 mm × 4.6 mm × 5 µm; mobile phase: (n-hexane + 0.2% diethylamine):[(ethanol:methanol = 50:50) + 0.2% diethylamine] = 30:70; flow rate: 1 mL/min; column temperature: 30 °C) to give 2 isomeric compounds. The structure of one of the isomeric compounds was Z35-L1-B (28 mg, retention time: 29.350 min, yield: 17.0%) as a white solid. ES-API: [M+H]⁺= 1507.2. ¹H NMR (400 MHz, DMSO-*d₆*) δ 8.68 - 8.51 (m, 3H), 8.49 (s, 1H), 8.33 (t, *J* = 5.6 Hz, 1H), 8.16 (d, *J* = 8.0 Hz, 1H), 8.12 - 7.98 (m, 2H), 7.80 (s, 1H), 7.73 (d, *J* = 8.0 Hz, 1H), 7.57 - 7.44 (m, 2H), 7.33 - 7.09 (m, 5H), 6.99 (s, 2H), 5.54 (t, *J =* 9.2 Hz, 1H), 5.06 (d, *J =* 12.0 Hz, 1H), 4.72 - 4.45 (m, 4H), 4.30 - 3.98 (m, 5H), 3.91 - 3.77 (m, 3H), 3.76 - 3.54 (m, 11H), 3.51 - 3.36 (m, 6H), 3.31 - 3.28 (m, 1H), 3.20 - 2.93 (m, 6H), 2.89 - 2.69 (m, 2H), 2.59 - 2.51 (m, 4H), 2.28 - 2.06 (m, 4H), 2.02 - 1.88 (m, 2H), 1.86 - 1.71 (m, 2H), 1.70 - 1.61 (m, 1H), 1.59 - 1.38 (m, 5H), 1.30 (d, *J =* 6.5 Hz, 3H), 1.25 - 1.14 (m, 5H), 0.96 (s, 3H), 0.55 (s, 3H). The structure of the other isomeric compound was Z35-L1-A (20 mg, retention time: 42.689 min, yield: 12.1%) as a white solid. ES-API: [M+H]⁺= 1507.2. ¹H NMR (400 MHz, DMSO-*d₆*) δ 8.61 - 8.44 (m, 4H), 8.31 (t, *J =* 5.7 Hz, 1H), 8.14 (d, *J =* 8.0 Hz, 1H), 8.11 - 7.96 (m, 2H), 7.81 (s, 1H), 7.75 (d, *J* = 8.8 Hz, 1H), 7.57 (d, *J* = 8.7 Hz, 1H), 7.35 (s, 1H), 7.27 - 7.13 (m, 5H), 6.99 (s, 2H), 5.58 (t, *J* = 9.3 Hz, 1H), 5.10 (d, *J* = 12.1 Hz, 1H), 4.58 (d, *J* = 6.5 Hz, 2H), 4.53 - 4.44 (m, 1H), 4.43 - 4.28 (m, 2H), 4.27 - 3.98 (m, 5H), 3.88 - 3.79 (m, 2H), 3.78 - 3.53 (m, 12H), 3.51 - 3.43 (m, 2H), 3.40 - 3.34 (m, 4H), 3.30 - 3.28 (m, 1H), 3.20 - 2.99 (m, 5H), 2.94 (d, *J* = 13.6 Hz, 1H), 2.85 - 2.71 (m, 2H), 2.59 - 2.51 (m, 5H), 2.16 - 2.03 (m, 3H), 1.96 - 1.85 (m, 2H), 1.85 - 1.70 (m, 2H), 1.67 - 1.61 (m, 1H), 1.53 - 1.41 (m, 7H), 1.25 - 1.14 (m, 3H), 1.00 - 0.83 (m, 6H), 0.52 (s, 3H).

### Example A-26. Synthesis of Compounds Z57-L7-A and Z57-L7-B

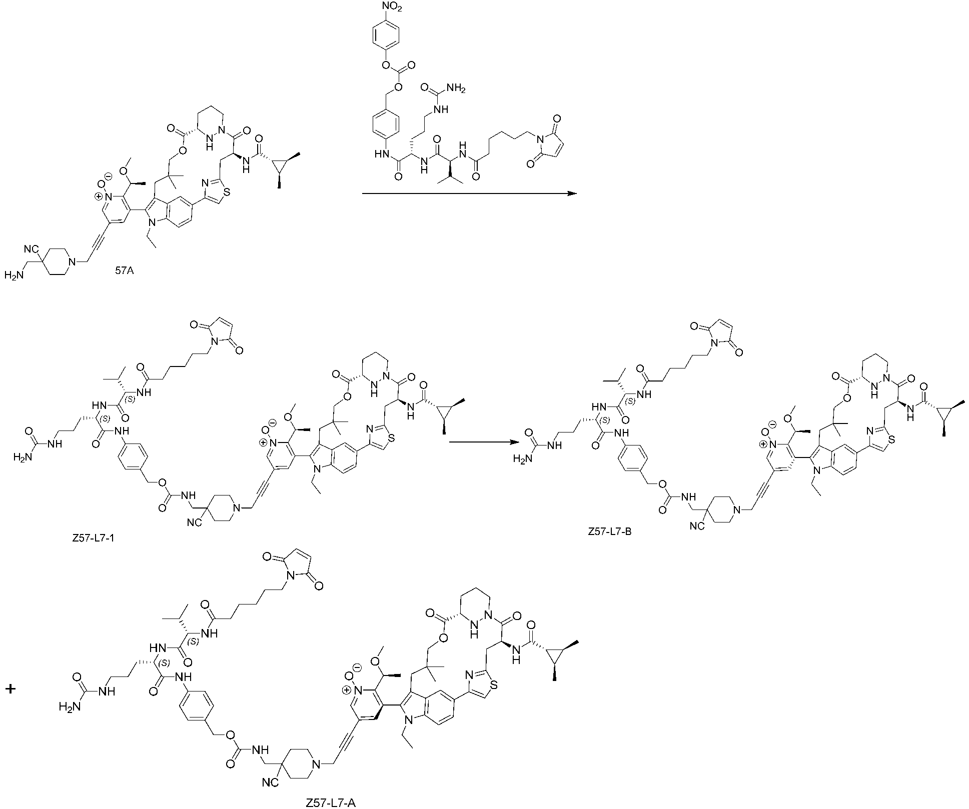

Step 1: Compound 57A (120 mg, crude product) was dissolved in N,N-dimethylformamide (3 mL), and N,N-diisopropylethylamine (70.95 mg, 0.549 mmol) and benzyl 4-((S)-2-((S)-2-(6-(2,5-dioxo-2,5-dihydro-1H-pyrrol-1-yl)hexanamido)-3-methylbutanamido)-5-ureidopentanamido)(4-nitrophenyl)carbonate (151.87 mg, 0.206 mmol) were added to the solution described above. After the addition was completed, the reaction system was stirred at room temperature for 2 h. After the reaction was completed, the reaction mixture was directly purified by prep-HPLC (ammonium bicarbonate method 1) to give compound Z57-L7-1 (100 mg, 0.066 mmol, two-step yield: 48.17%) as a white solid. [M+H]⁺=1516.5.

Step 2: Compound Z57-L7-1 (100 mg, 0.066 mmol) was subjected to chiral resolution (column type: IG 250 mm, 10 mm, 5 µm; mobile phase system: (A: acetonitrile; B: isopropanol; C: diethylamine); flow rate: 1 mL/min; A/B/C = 70/30/2; column temperature: 30 °C) to give 2 isomeric compounds. The structure of one of the isomeric compounds was Z57-L7-B (36.22 mg, 0.024 mmol, yield: 36.22%, retention time: 15.276 min) as a white solid, ES-API: [M+H]⁺ = 1516.5. ¹H NMR (400 MHz, DMSO-*d₆*) δ 10.01 (s, 1H), 8.50 (d, *J =* 7.6 Hz, 2H), 8.42 (d, *J* = 9.2 Hz, 1H), 8.09 (d, *J* = 7.6 Hz, 1H), 7.88 - 7.79 (m, 2H), 7.76 - 7.68 (m, 2H), 7.59 (d, *J =* 8.4 Hz, 2H), 7.54 - 7.46 (m, 2H), 7.29 (d, *J =* 8.4 Hz, 2H), 7.00 (s, 2H), 6.03-5.96 (m, 1H), 5.53 (t, *J =* 9.2 Hz, 1H), 5.42 (s, 2H), 5.04 (d, *J* = 12.0 Hz, 1H), 4.97 (s, 2H), 4.63 - 4.51 (m, 1H), 4.43 - 4.34 (m, 1H), 4.28 - 4.16 (m, 3H), 4.12 - 4.02 (m, 1H), 3.94 - 3.80 (m, 1H), 3.72 - 3.50 (m, 4H), 3.44 - 3.34 (m, 5H), 3.30 - 3.23 (m, 3H), 3.20 - 3.11 (m, 1H), 3.00 - 2.81 (m, 4H), 2.80 - 2.70 (m, 1H), 2.40 - 2.35 (m 2H), 2.25 - 2.06 (m, 4H), 2.03 - 1.92 (m, 1H), 1.90 - 1.67 (m, 5H), 1.63 - 1.42 (m, 9H), 1.34 - 1.15 (m, 13H), 1.13 - 1.04 (m, 6H), 0.96 (s, 3H), 0.88 - 0.79 (m, 6H), 0.56 (s, 3H). The structure of the other isomeric compound was Z57-L7-A(29.38 mg, 0.019 mmol, yield: 29.38%, retention time: 22.428 min) as a white solid, ES-API: [M+H]⁺ = 1516.5. 1H NMR (400 MHz, DMSO-*d₆*) δ 10.00 (s, 1H), 8.52 - 8.46 (m, 2H), 8.39 (d, J = 8.8 Hz, 1H), 8.09 (d, J = 7.6 Hz, 1H), 7.88 - 7.79 (m, 2H), 7.77 - 7.67 (m, 2H), 7.63 - 7.53 (m, 3H), 7.35 (s, 1H), 7.28 (d, J = 8.8 Hz, 2H), 7.03 - 6.98 (m, 2H), 6.00 (t, J = 5.6 Hz, 1H), 5.58 (t, J = 9.2 Hz, 1H), 5.42 (s, 2H), 5.09 (d, J = 12.0 Hz, 1H), 4.96 (s, 2H), 4.46 - 4.29 (m, 3H), 4.28 - 4.10 (m, 3H), 4.09 - 3.95 (m, 1H), 3.68 - 3.50 (m, 4H), 3.41 - 3.35 (m, 3H), 3.28 - 3.22 (m, 2H), 3.21 - 3.09 (m, 4H), 3.05 - 2.82 (m, 5H), 2.80 - 2.70 (m, 1H), 2.47 - 2.34 (m, 2H), 2.24 - 2.04 (m, 3H), 2.01 - 1.92 (m, 1H), 1.89 - 1.68 (m, 5H), 1.63 - 1.31 (m, 14H), 1.27 - 1.14 (m, 7H), 1.09 - 1.05 (m, 5H), 0.98 - 0.91 (m, 5H), 0.88 - 0.78 (m, 6H), 0.52 (s, 3H).

### Example A-27. Synthesis of Compounds Z64-L7-A and Z64-L7-B

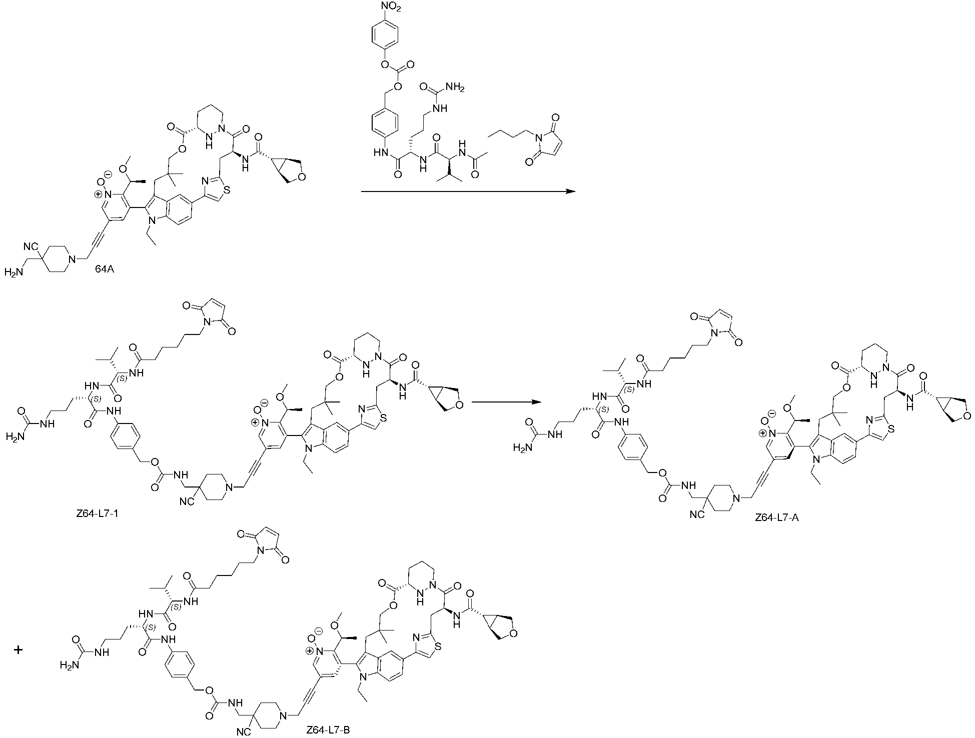

Step 1: Compound 64A (90 mg, crude product) was dissolved in N,N-dimethylformamide (3 mL), and N,N-diisopropylethylamine (49.92 mg, 0.386 mmol) and benzyl 4-((S)-2-((S)-2-(6-(2,5-dioxo-2,5-dihydro-1H-pyrrol-1-yl)hexanamido)-3-methylbutanamido)-5-ureidopentanamido)(4-nitrophenyl)carbonate (106.85 mg, 0.145 mmol) were added to the solution described above. After the addition was completed, the reaction system was stirred at room temperature for 2 h. After the reaction was completed, the reaction mixture was directly purified by prep-HPLC (ammonium bicarbonate method 1) to give compound Z64-L7-1 (100 mg, 0.067 mmol, two-step yield: 53.17%) as a white solid. [M+H]⁺=1531.3.

Step 2: Compound Z64-L7-1 (100 mg, 0.067 mmol) was subjected to chiral resolution (column type: IG 250 mm, 10 mm, 5 µm; mobile phase system: (A: acetonitrile; B: (isopropanol:methanol = 1:1); C: diethylamine); flow rate: 1 mL/min; A/B/C = 70/30/2; column temperature: 30 °C) to give 2 isomeric compounds. The structure of one of the isomeric compounds was Z64-L7-A (20.51 mg, 0.013 mmol, yield: 20.51%, retention time: 4.090 min) as a white solid, ES-API: [M+H]⁺ = 1531.3. 1H NMR (400 MHz, DMSO-d₆) δ 9.99 (s, 1H), 8.56 (d, J = 8.8 Hz, 1H), 8.49 (s, 1H), 8.47 (d, J = 1.2 Hz, 1H), 8.08 (d, J = 7.2 Hz, 1H), 7.85 - 7.76 (m, 2H), 7.78 - 7.68 (m, 2H), 7.62 - 7.54 (m, 3H), 7.35 (d, J = 1.2 Hz, 1H), 7.28 (d, J = 8.4 Hz, 2H), 7.00 (s, 2H), 5.99 (t, J = 5.6 Hz, 1H), 5.58 (t, J = 9.2 Hz, 1H), 5.41 (s, 2H), 5.11 (d, J = 12.4 Hz, 1H), 4.96 (s, 2H), 4.46 - 4.29 (m, 3H), 4.26 - 4.13 (m, 3H), 4.09 - 4.00 (m, 1H), 3.87 - 3.78 (m, 2H), 3.68 - 3.53 (m, 6H), 3.40 - 3.33 (m, 4H), 3.25 (d, J = 5.6 Hz, 2H), 3.21 - 3.09 (m, 4H), 3.05 - 2.84 (m, 5H), 2.80 - 2.70 (m, 1H), 2.40 (t, J = 11.2 Hz, 2H), 2.22 - 2.05 (m, 3H), 2.01 - 1.67 (m, 9H), 1.57 - 1.36 (m, 12H), 1.26 - 1.12 (m, 4H), 0.97 - 0.92 (m, 5H), 0.86 - 0.79 (m, 6H), 0.52 (s, 3H). The structure of the other isomeric compound was Z64-L7-B (26.94 mg, 0.013 mmol, yield: 26.94%, retention time: 4.838 min) as a white solid, ES-API: [M+H]⁺ = 1531.3. ¹H NMR (400 MHz, DMSO-d₆) δ 10.01 (s, 1H), 8.60 (d, *J* = 8.8 Hz, 1H), 8.52 - 8.46 (m, 2H), 8.09 (d, *J =* 6.8 Hz, 1H), 7.86 - 7.76 (m, 2H), 7.75 - 7.68 (m, 2H), 7.59 (d, *J =* 8.4 Hz, 2H), 7.54 - 7.46 (m, 2H), 7.28 (d, *J =* 8.4 Hz, 2H), 7.00 (s, 2H), 6.01 (s, 1H), 5.53 (t, *J* = 9.2 Hz, 1H), 5.42 (s, 2H), 5.06 (d, *J* = 12.0 Hz, 1H), 4.97 (s, 2H), 4.63 - 4.53 (m, 1H), 4.45 - 4.33 (m, 1H), 4.27 - 4.15 (m, 3H), 4.11 - 3.99 (m, 1H), 3.90 - 3.78 (m, 3H), 3.69 - 3.54 (m, 6H), 3.42 - 3.36 (m, 3H), 3.26 (d, *J* = 6.0 Hz, 2H), 3.20 - 3.10 (m, 1H), 3.03 - 2.74 (m, 9H), 2.42 (t, *J =* 11.6 Hz, 2H), 2.23 - 2.10 (m, 4H), 2.02 - 1.80 (m, 7H), 1.73 - 1.45 (m, 11H), 1.31 - 1.17 (m, 9H), 0.97 (s, 3H), 0.88 - 0.78 (m, 6H), 0.56 (s, 3H).

### Example A-28. Synthesis of Compounds Z45-L2-A and Z45-L2-B

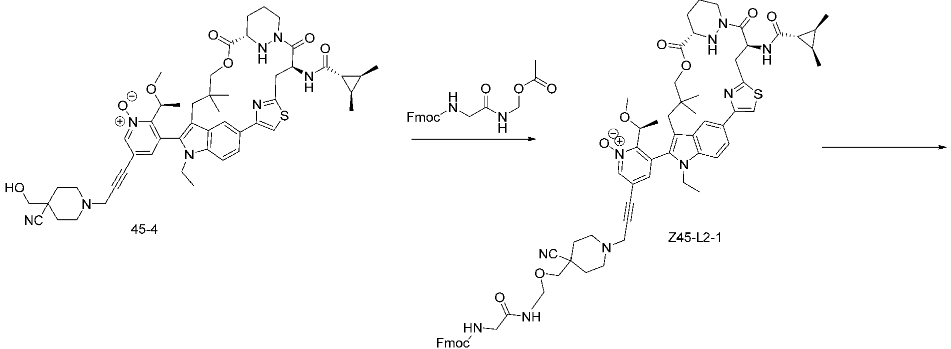

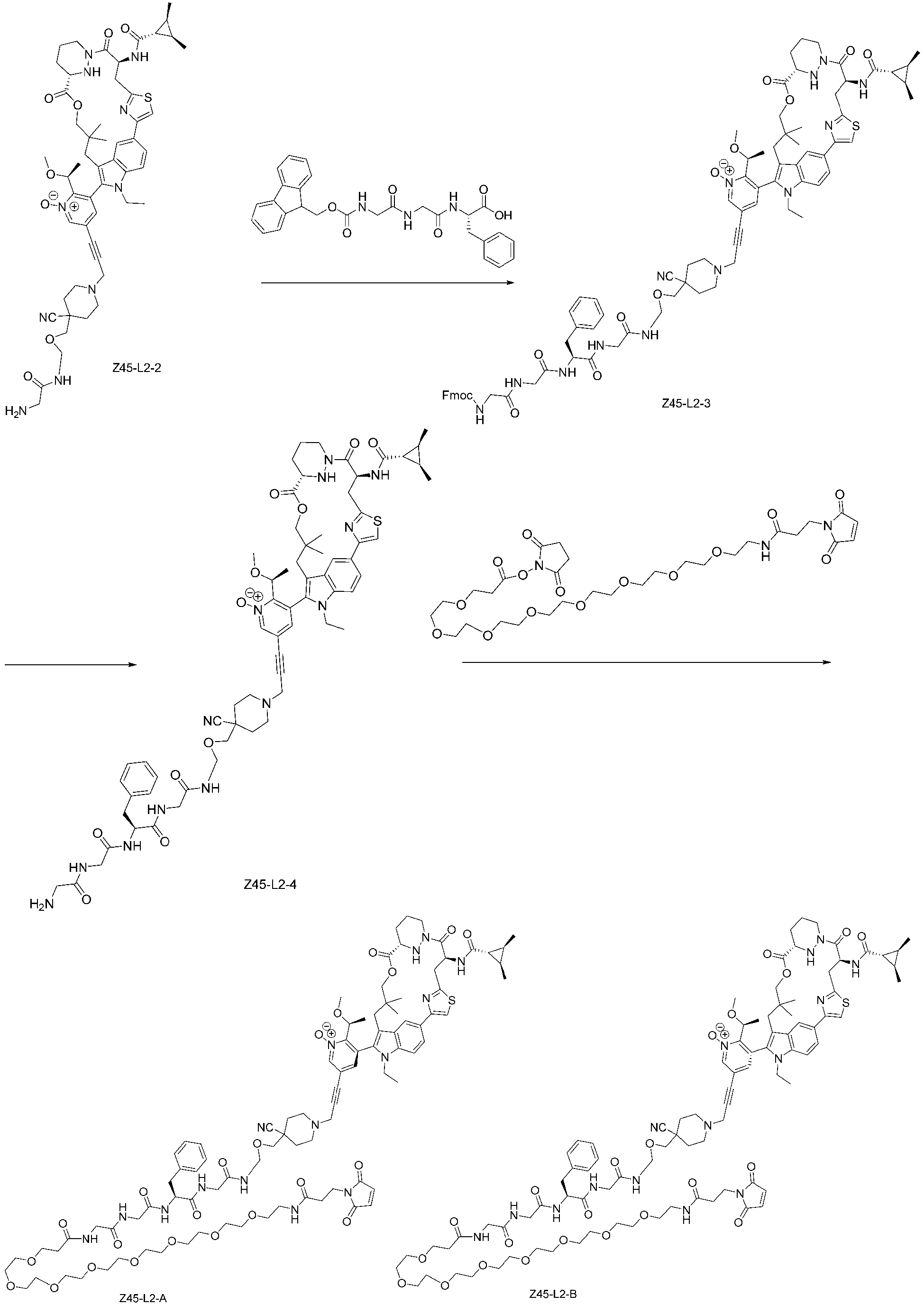

Step 1: Compound 45-4 (200 mg, 0.218 mmol), (2-((((9H-fluoren-9-yl)methoxy)carbonyl)amino)acetamido)methyl acetate (160.32 mg, 0.435 mmol), 4-methylbenzenesulfonic acid (56 mg, 0.325 mmol), and dichloromethane (6 mL) were added to a flask. The reaction system was placed under an oil bath at 50 °C and stirred for 2 h under a nitrogen atmosphere. Water was added to the reaction mixture, and the mixture was extracted with dichloromethane (50 mL × 3). The organic phase was washed with saturated brine, dried over sodium sulfate, and filtered. The filtrate was concentrated, and the crude product was purified by a flash silica gel column (tetrahydrofuran/petroleum ether: 0-100%) to give compound Z45-L2-1 (150 mg, crude product) as a yellow solid. ES-API: [M+H]⁺=1227.3.

Step 2: Compound Z45-L2-1 (150 mg, crude product), tetrahydrofuran (5 mL), and N,N-dimethylformamide (1 mL) were sequentially added to a flask. The reaction mixture was cooled to 0 °C, and 1,8-diazabicyclo[5.4.0]undec-7-ene (20 mg, 0.13 mmol) was added to thereto under a nitrogen atmosphere. The reaction system was stirred at 0 °C for 30 min. The reaction mixture was adjusted to pH 7 with 1 M hydrochloric acid to give a reaction mixture of compound Z45-L2-2, which was directly used in the next step.

Step 3: The reaction mixture of compound Z45-L2-2 obtained in the previous step was added to a flask, and (((9H-fluoren-9-yl)methoxy)carbonyl)glycylglycyl-L-phenylalanine (45 mg, 0.090 mmol), N,N-diisopropylethylamine (23.53 mg, 0.18 mmol), and (benzotriazol-1-yloxy)tripyrrolidinophosphonium hexafluorophosphate (45 mg, 0.086 mmol) were sequentially added to the above reaction mixture with stirring. The reaction system was stirred at room temperature for 30 min. The reaction was quenched with water, and the mixture was extracted with ethyl acetate. The organic phase was washed with saturated brine, dried over anhydrous sodium sulfate, and filtered. The filtrate was concentrated to give a crude product. The crude product was purified by a flash silica gel column (methanol/dichloromethane: 0-5%) to give compound Z45-L2-3 (60 mg, yield: 66.4%) as a yellow solid. ES-API: [M+H]⁺= 1488.3.

Step 4: Compound Z45-L2-3 (60 mg, 0.040 mmol), tetrahydrofuran (3 mL), and N,N-dimethylformamide (1 mL) were sequentially added to a flask. The reaction mixture was cooled to 0 °C, and 1,8-diazabicyclo[5.4.0]undec-7-ene (13 mg, 0.085 mmol) was added to thereto under a nitrogen atmosphere. The reaction system was stirred at 0 °C for 30 min. The reaction mixture was adjusted to pH 7 with 1 M hydrochloric acid to give a reaction mixture of compound Z45-L2-4, which was directly used in the next step.

Step 5: The reaction mixture of compound Z45-L2-4 obtained in the previous step and 2,5-dioxopyrrolidin-1-yl 1-(2,5-dihydro-2,5-dihydro-1H-pyrrol-1-yl)-3-oxo-7,10,13,16,19,22,25,28-octaoxa-4-azahentriacontan-31-oate (41 mg, 0.059 mmol) were sequentially added to a flask. The solution described above was cooled to 0 °C, and N,N-diisopropylethylamine (11 mg, 0.085 mmol) was added thereto. The reaction system was stirred at room temperature for 1 h. The solution described above was cooled to 0 °C and adjusted to pH 7 with 1 M hydrochloric acid. Water was added to the reaction mixture, and the resulting mixture was extracted with dichloromethane. The organic phase was washed with saturated brine, dried over anhydrous sodium sulfate, and filtered. The filtrate was concentrated to give a crude product. The crude product was purified by prep-HPLC (ammonium bicarbonate method 1) to give 2 isomeric compounds. The structure of one of the isomeric compounds was Z45-L2-A (5 mg, yield: 6.7%, retention time: 5.485 min). ES-API: [M+H]⁺= 1841.3. The structure of the other isomeric compound was Z45-L2-B (5 mg, yield: 6.7%, retention time: 5.573 min). ES-API: [M+Na]⁺ = 1862.6.

### Example A-29. Synthesis of Compound Z52-L12

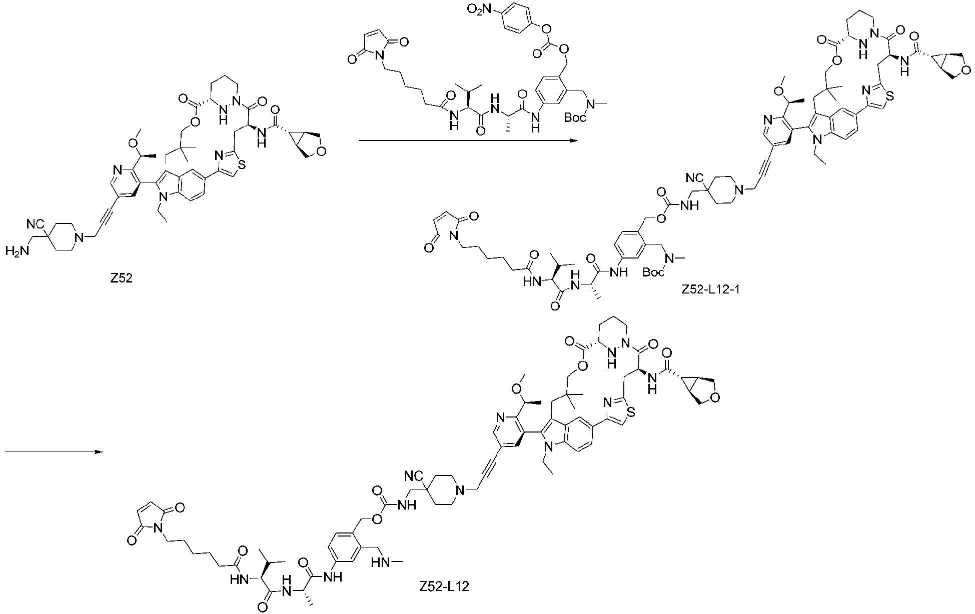

Step 1: Compound Z52 (50.0 mg, 0.055 mmol) and tert-butyl (5-((S)-2-(S)-(6-(2,5-dioxo-2,5-dihydro-1H-pyrrol-1-yl)hexanamido)-3-methylbutanamido)propanamido)-2-((((4-nitrophenoxy)carbonyl)oxy)methyl)benzyl)(methyl)carbamate (50.0 mg, 0.063 mmol) were added to anhydrous N,N-dimethylformamide (2.0 mL), and finally, dry N,N-diisopropylethylamine (36.0 mg, 0.275 mmol) was added. The mixture was stirred for reaction at room temperature overnight. After the reaction was completed, the cooled solution was extracted with ethyl acetate (80 mL × 3) and saturated brine (80 mL). The ethyl acetate phase was dried over anhydrous sodium sulfate and filtered. The filtrate was concentrated, and then the residue was purified by flash silica gel column chromatography [PE:EA = 100:0 to 30:70, (v/v)] to give compound Z52-L12-1 (105.0 mg, crude product). ES-API: [M +H]⁺=1572.3.

Step 2: Trifluoroacetic acid (2.0 mL) was added to a solution of compound Z52-L12-1 (105.0 mg, crude product) in dichloromethane (8.0 mL), and the mixture was stirred at room temperature for 1 h. After the reaction was completed, the reaction mixture was concentrated to dryness by rotary evaporation under reduced pressure to remove the solvent. The crude product was purified by prep-HPLC (trifluoroacetic acid method) to give the desired product Z52-L12 (49.0 mg, yield: 56.18%, trifluoroacetate). ES-API: [M +H]⁺=1472.5.

### Example A-30. Synthesis of Compound Z48-L1

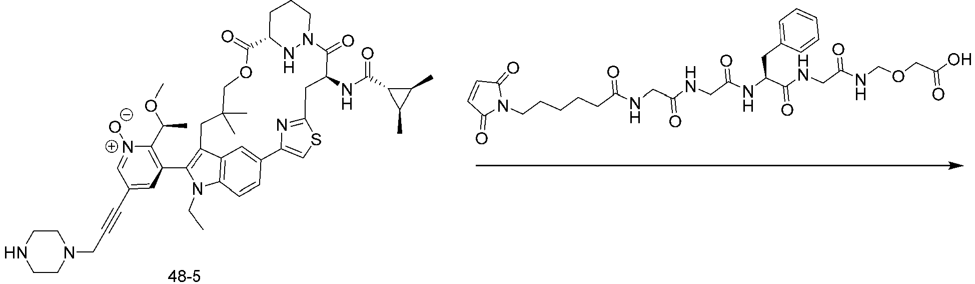

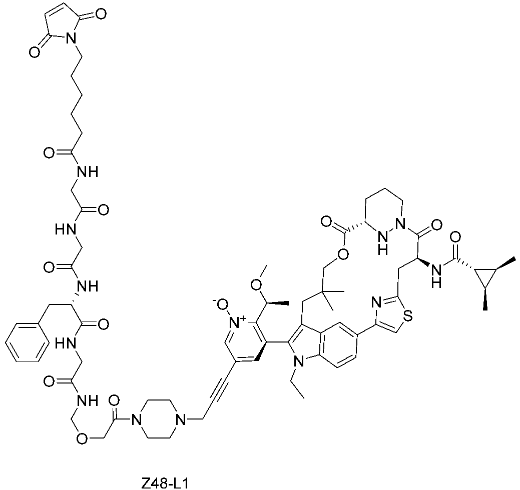

Step 1: Compound 48-5 (9 mg, 0.010 mmol) was dissolved in dichloromethane (1 mL), and (S)-10-benzyl-23-(2,5-dioxo-2,5-dihydro-1H-pyrrol-1-yl)-6,9,12,15,18-pentaoxo-3-oxa-5,8,11,14,17-pentaazatricosanoic acid (9.62 mg, 0.016 mmol), O-(7-azabenzotriazol-1-yl)-*N,N,N',N'*-tetramethyluroniumhexafluorophosphate (7.98 mg, 0.021 mmol), and *N,N'*-diisopropylethylamine (4.03 mg, 0.031 mmol) were added to the solution described above. After the addition was completed, the reaction system was stirred at room temperature for 2 h. After the reaction was completed, the reaction mixture was concentrated to remove dichloromethane. Water (10 mL) was added to the residue, and the mixture was extracted with dichloromethane (15 mL × 2). The organic phase was washed with saturated brine (10 mL × 3), dried, and concentrated to give a crude product. The crude product was purified by prep-HPLC (ammonium bicarbonate method 1) to give the desired product Z48-L1 (5.78 mg, 0.004 mmol, yield: 37.95%) as a white solid. ES-API: [M +H]⁺=1464.6.

### Example A-31. Synthesis of Compound Z52-L11

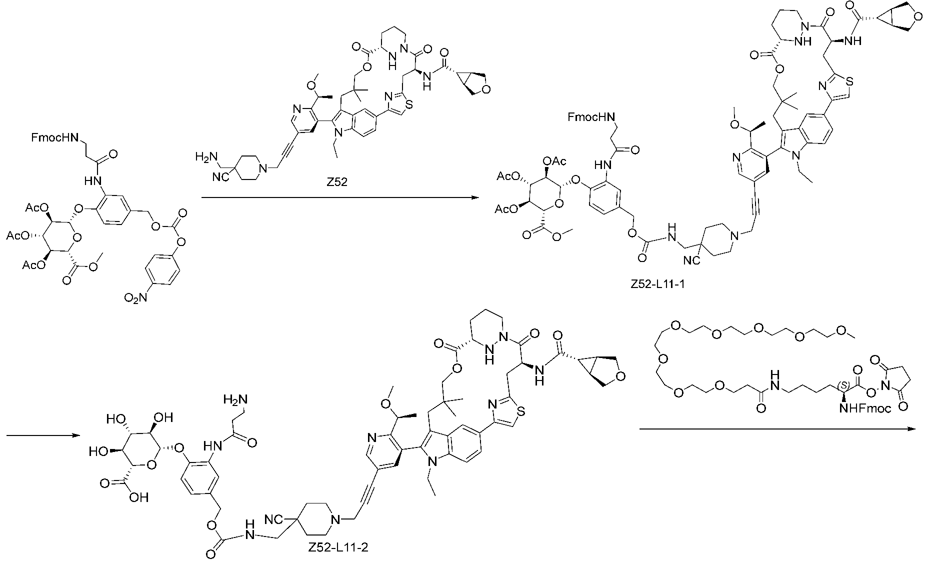

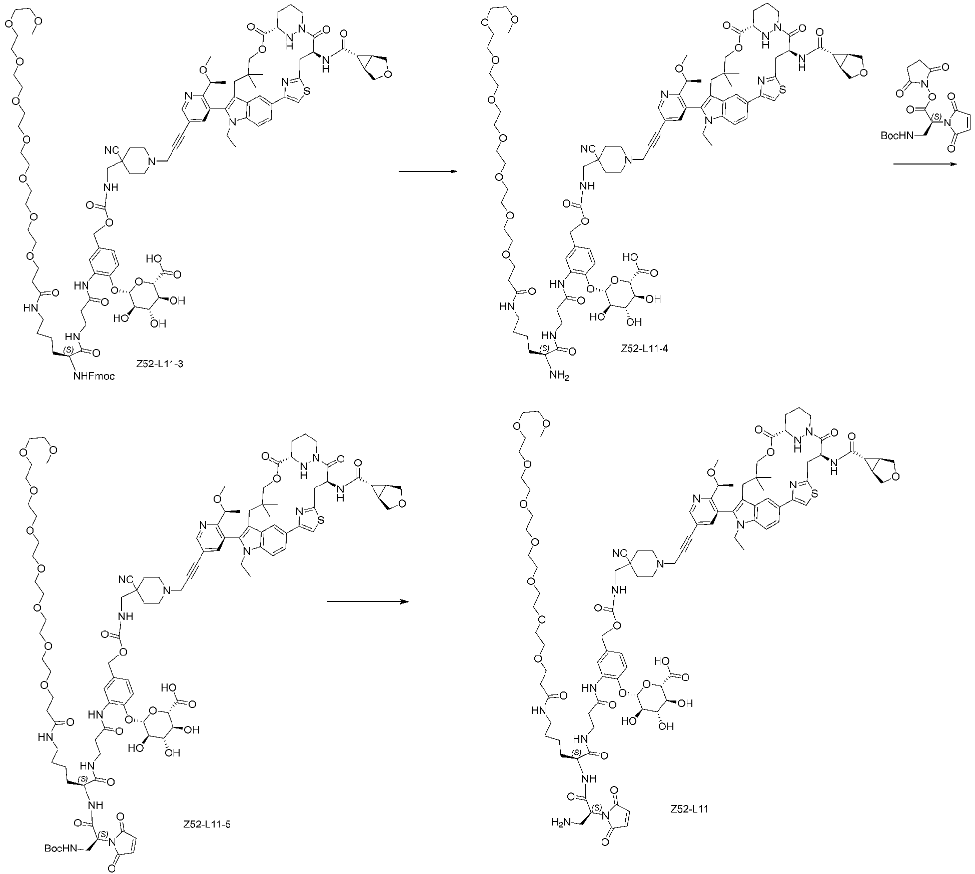

Step 1: Compound Z52 (80 mg, 0.087 mmol) and methyl (2-{[1-(9H-fluoren-9-yl)-3,7-dioxo-4-aza-2-oxahept-7-yl]amino}-1-{[(2S,3R,4S,5S,6S)-3,4,5-triacetoxy-6-(methoxycarbonyl)-3,4,6-tetrahydro-2H-pyran-2-yl]oxy}phen-4-yl)methyl[(4-nitrophenyl)oxy]formate (159.60 mg, 0.175 mmol) (for synthesis steps, refer to WO2021050917) were dissolved in N,N-dimethylformamide (5 mL) and pyridine (1 mL), and N,N'-diisopropylethylamine (11.29 mg, 0.087 mmol) and 1-hydroxybenzotriazole (11.80 mg, 0.087 mmol) were added. After the addition was completed, the reaction system was stirred at room temperature for 12 h under a nitrogen atmosphere. After the reaction was completed, water (15 mL) was added, and the mixture was extracted with ethyl acetate (20 mL × 3). The organic phase was washed with saturated brine (15 mL), dried over anhydrous sodium sulfate, and concentrated. The crude product was purified by a flash silica gel column (tetrahydrofuran/petroleum ether: 0%-60%) to give compound Z52-L11-1 (110 mg, 0.065 mmol, yield: 74.50%) as a colorless oily liquid, ES-API: [M/2+H]⁺ = 846.0.

Step 2: Compound Z52-L11-1 (80 mg, 0.047 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z52-L11-2 (60 mg, 0.045 mmol, yield: 95.46%) as a colorless oily liquid, ES-API: [M/2+H]⁺ = 664.9.

Step 3: Compound Z52-L11-2 (50 mg, 0.038 mmol) was dissolved in N,N-dimethylformamide (1 mL), and N,N'-diisopropylethylamine (9.51 mg, 0.074 mmol) and methyl 9H-fluoren-9-ylmethyl{[(32S)-33-[(2,5-dioxotetrahydro-1H-pyrrol-1-yl)oxy]-26,33-dioxo-27-aza-2,5,8,11,14,17,20,23-octaoxa-32-yl]amino}formate (38.84 mg, 0.045 mmol) (for synthesis steps, refer to [Bioorganic Chemistry, 2022, vol. 124, art. no. 105831]). The reaction system was stirred at room temperature for 3 h under a nitrogen atmosphere and purified by prep-HPLC (trifluoroacetic acid method) to give compound Z52-L11-3 (40 mg, 0.019 mmol, yield: 50.82%) as a white solid, ES-API: [M/2+H]⁺ = 1037.2.

Step 4: Compound Z52-L11-3 (25 mg, 0.012 mmol) was dissolved in acetonitrile (1 mL), and diethylamine (0.2 mL) was added. The reaction system was stirred at room temperature for 2 h under a nitrogen atmosphere. After the reaction was completed, the reaction mixture was concentrated under reduced pressure to give compound Z52-L11-4 (20 mg, 0.011 mmol, yield: 89.60%) as a colorless oily liquid, ES-API: [M+H]⁺ = 926.3.

Step 5: Methyl 2-methylpropan-2-yl {[(2S)-2-(2,5-dioxo-2,5-dihydro-1H-pyrrol-1-yl)-3-[(2,5-dioxotetrahydro-1H-pyrrol-1-yl)oxy]-3-oxopropyl]amino}formate (4.94 mg, 0.013 mmol) (for synthesis steps, refer to WO2015057699) and compound Z52-L11-4 (20 mg, 0.011 mmol) were dissolved in N,N-dimethylformamide (1 mL), and N,N'-diisopropylethylamine (9.51 mg, 0.074 mmol) was added. The reaction system was stirred at room temperature for 3 h under a nitrogen atmosphere and purified by prep-HPLC (trifluoroacetic acid method) to give compound Z52-L11-5 (15 mg, 0.007 mmol, yield: 65.57%) as a white solid, ES-API: [M/2+H]⁺ = 1058.7. Step 6: Compound Z52-L11-5 (13 mg, 0.006 mmol) was dissolved in dichloromethane (1 mL), and trifluoroacetic acid (0.3 mL) was added. The reaction system was stirred at room temperature for 3 h under a nitrogen atmosphere and purified by prep-HPLC (trifluoroacetic acid method) to give the desired product Z52-L11 (5 mg, 0.002 mmol, yield: 22.35%, trifluoroacetate) as a white solid, ES-API: [M/2+H]⁺ = 1009.2.

### Example A-32. Synthesis of Compound Z52-L13

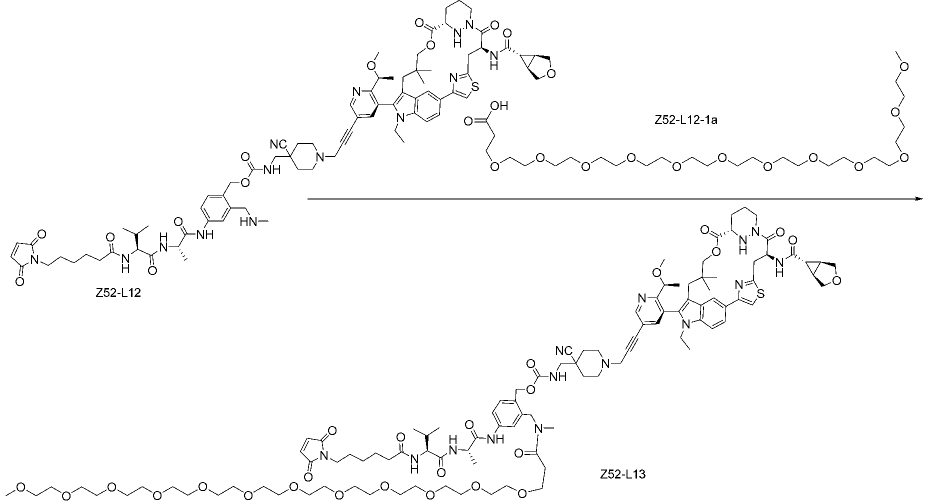

Step 1: Compound Z52-L12-1a (CAS: 2135793-73-4, 15.0 mg, 0.022 mmol), N,N-diisopropylethylamine (13.0 mg, 0.102 mmol), and O-(7-azabenzotriazol-1-yl)-N,N,N',N'-tetramethyluronium hexafluorophosphate (13.0 mg, 0.034 mmol) were added to a solution of compound Z52-L12 (15.0 mg, 0.010 mmol) in dry N,N-dimethylformamide (1.0 mL), and the reaction mixture was stirred for 2 h under a nitrogen atmosphere. The reaction mixture was purified by prep-HPLC (formic acid method 1) to give the desired product Z52-L13 (6.80 mg, yield: 32.66%, formate). ES-API: [M/2 +H]⁺ = 1021.7.

### Example A-33. Synthesis of Compound Z52-L14

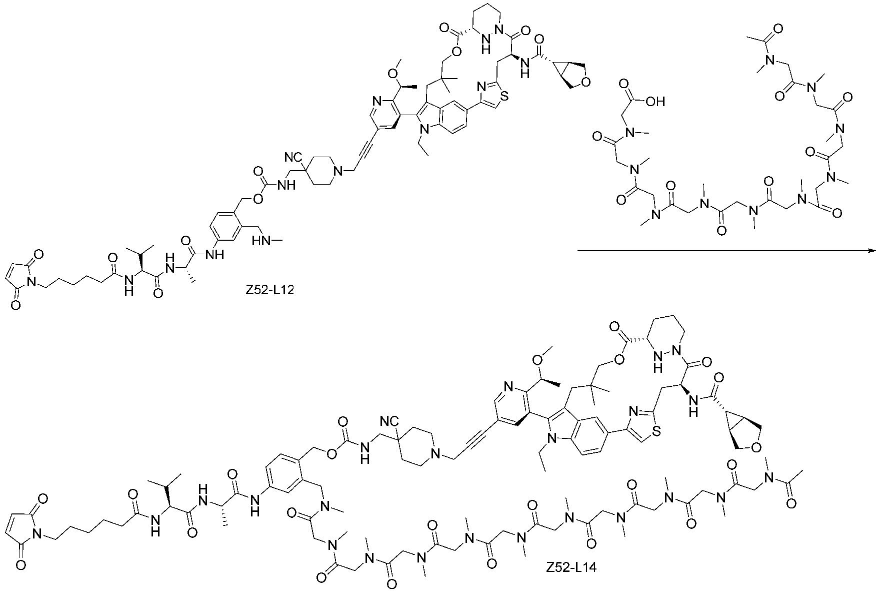

Step 1: 3,6,9,12,15,21,24,27,30-decamethyl-4,7,10,13,16,19,22,25,28,31,31-decaoxo-3,6,9,12,15,18,21,24,27,30-decaazadotriacontanoic acid (20.0 mg, 0.026 mol), N,N-diisopropylethylamine (13.0 mg, 0.102 mmol), and O-(7-azabenzotriazol-1-yl)-N,N,N',N'-tetramethyluronium hexafluorophosphate (13.0 mg, 0.034 mmol) were added to a solution of compound 52-L12 (15.0 mg, 0.010 mmol) in dry N,N-dimethylformamide (1.0 mL), and the reaction mixture was stirred for 2 h under a nitrogen atmosphere. The reaction mixture was purified by prep-HPLC (formic acid method 1) to give the desired product Z52-L14 (7.73 mg, yield: 34.73%, formate). ES-API: [M/2 +H]⁺=1112.8.

### Example A-34. Synthesis of Compound Z56-L23

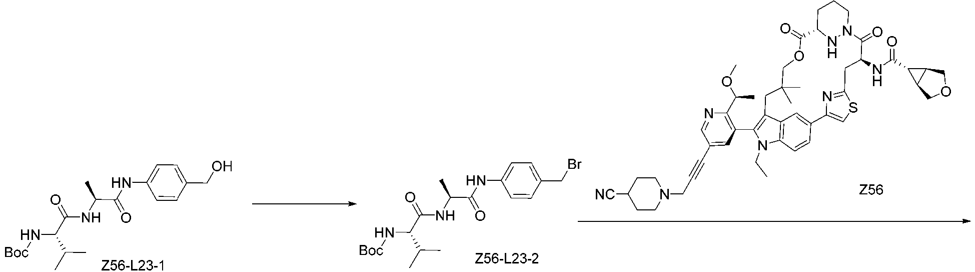

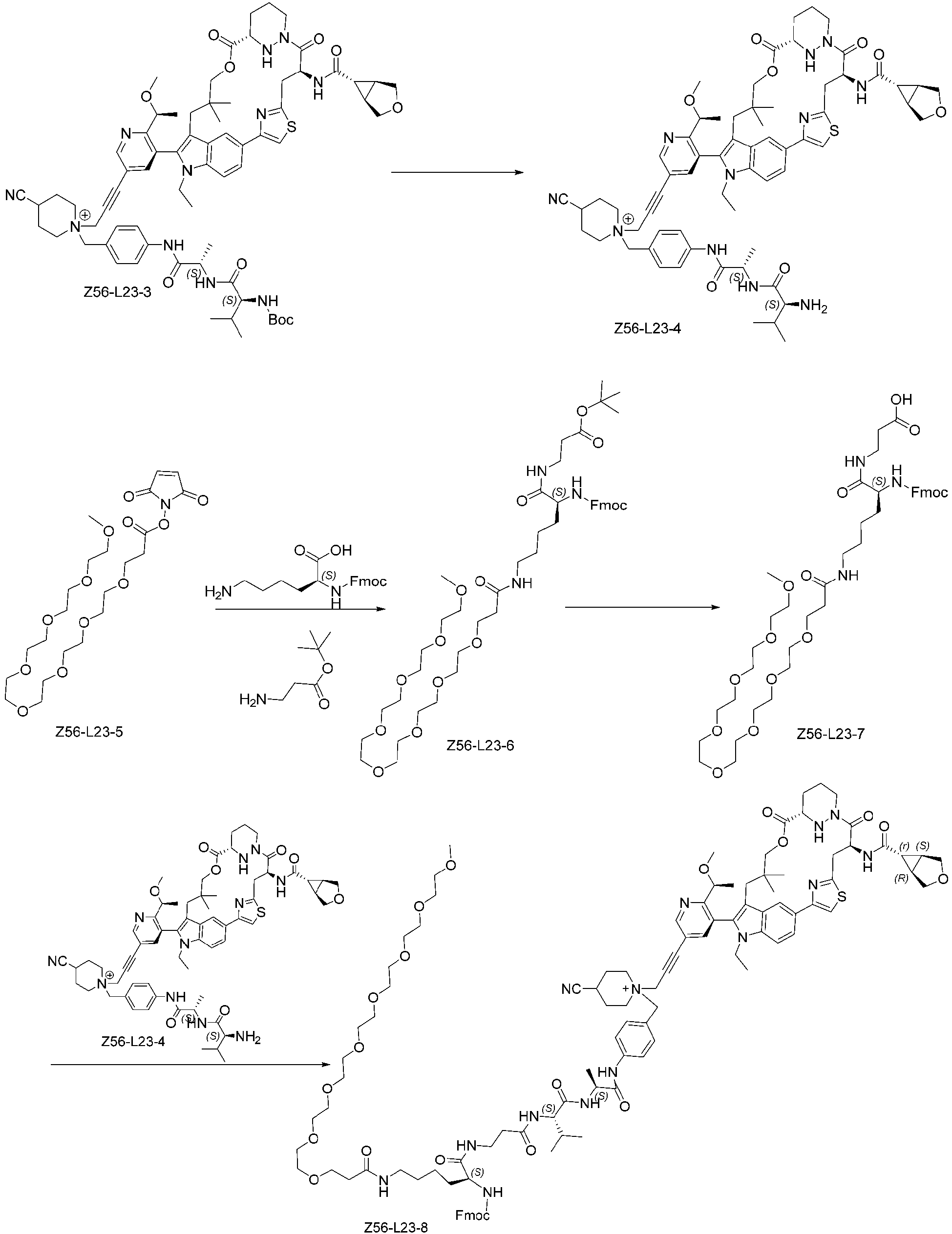

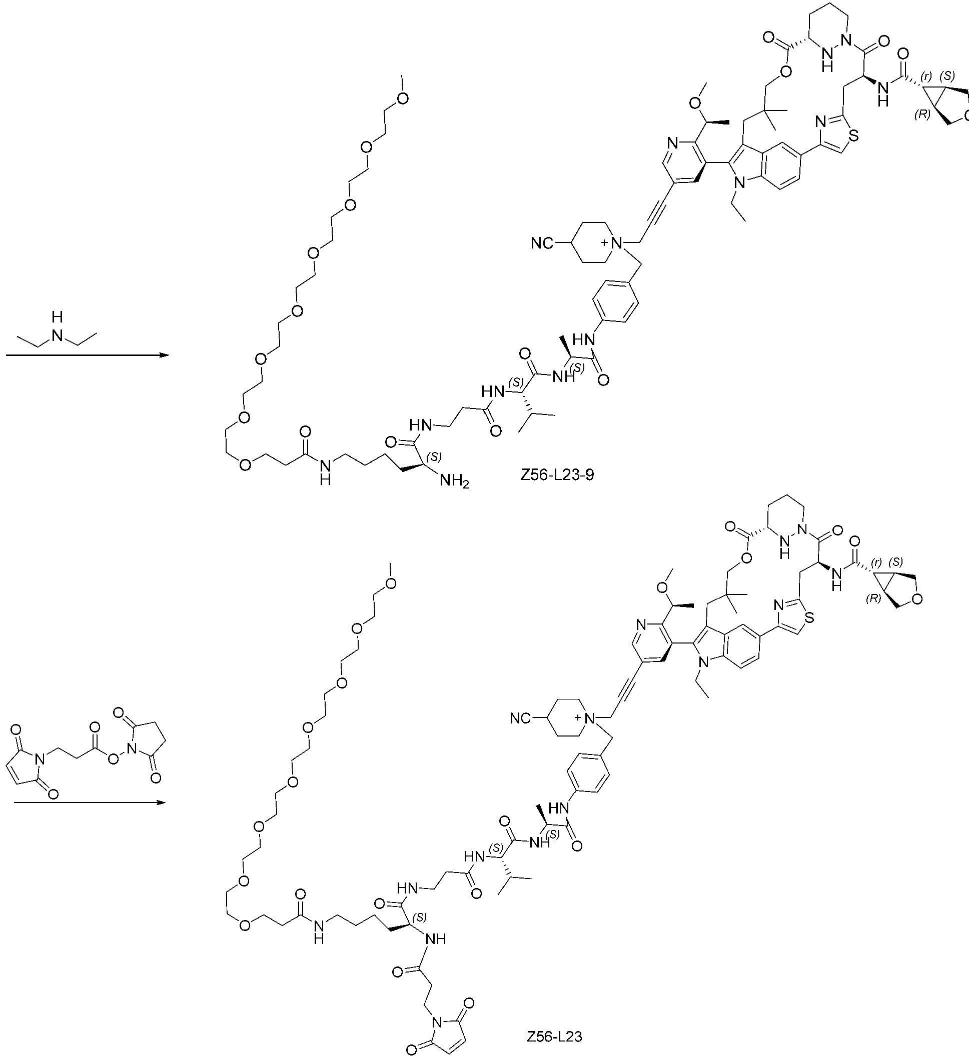

Step 1: Compound Z52-L9-1a (1000 mg, 2.541 mmol, prepared by referring to the method in Journal of Medicinal Chemistry, 2021, vol. 64, # 7, p. 4117-4129) was dissolved in dry dichloromethane (30 mL), and CBr₄ (1264.22 mg, 3.812 mmol) and PPh₃ (999.89 mg, 3.812 mmol) were sequentially added. The mixture was reacted at room temperature for 18 h. After the reaction was completed as detected, the mixture was sequentially washed with water (30 mL × 1) and saturated brine (30 mL × 1), dried over anhydrous sodium sulfate, filtered, and concentrated. The residue was purified by silica gel column chromatography (petroleum ether/ethyl acetate = 1/1) to give compound Z56-L23-2 (450 mg, 0.986 mmol, yield: 38.80%). ES-API: [M+H]⁺=456.1.

Step 2: Compound Z56 (500 mg, 0.564 mmol) and compound Z56-L23-2 (142.05 mg, 1.691 mmol) were dissolved in acetonitrile (10 mL), and sodium bicarbonate (142.05 mg, 1.691 mmol) was added. The mixture was heated to 75 °C and reacted for 5 h. Ethyl acetate (30 mL) was added. The mixture was sequentially washed with water (30 mL × 1) and saturated brine (30 mL × 1), dried over anhydrous sodium sulfate, filtered, and concentrated. The residue was purified by silica gel column chromatography (methanol/dichloromethane = 1/10) to give compound Z56-L23-3 (450 mg, 0.356 mmol, yield: 63.19%). ES-API: [M+H]⁺=1262.4.

Step 3: Compound Z56-L23-3 (450 mg, 0.356 mmol) was dissolved in dichloromethane (10 mL), and trifluoroacetic acid (5 mL) was added. The mixture was reacted at room temperature for 0.5 h. After the reaction was completed, the reaction mixture was concentrated to give compound Z56-L23-4 (460 mg, crude product), which was directly used in the next step. ES-API: [M+H]⁺=1162.4.

Step 4: Compound Z56-L23-5 (1.0 g, 1.963 mmol) was dissolved in N,N-dimethylformamide (15 mL), and DIPEA (759.49 mg, 5.888 mmol) and (((9H-fluoren-9-yl)methoxy)carbonyl)-L-lysine (650.74 mg, 1.766 mmol) were added. The mixture was reacted at room temperature for 1 h. After the reaction was completed, tert-butyl 3-aminopropanoate (0.33 g, 2.295 mmol), DIPEA (0.68 g, 5.297 mmol), and HATU (1.01 g, 2.648 mmol) were then added sequentially, and the mixture was reacted at room temperature for 1 h. Ethyl acetate (50 mL) was added. The mixture was sequentially washed with water (30 mL × 1) and saturated brine (30 mL × 1), dried over anhydrous sodium sulfate, filtered, and concentrated. The residue was purified by silica gel column chromatography (ethyl acetate/petroleum ether = 1/1) to give compound Z56-L23-6 (1.45 g, 1.629 mmol, yield: 82.9%). ES-API: [M+H]⁺=890.5.

Step 5: Trifluoroacetic acid (8 mL) was added to a solution of compound Z56-L23-6 (1.45 g, 1.629 mmol) in DCM (15 mL), and the mixture was stirred at room temperature for 2 h. The reaction mixture was concentrated to give compound Z56-L23-7 (1.36 g, 1.629 mmol, yield: 100%). ES-API: [M+H]⁺ = 834.4.

Step 6: Compound Z56-L23-7 (484.26 mg, 0.581 mmol), DIPEA (150.10 mg, 1.161 mmol), and HATU (220.65 mg, 0.581 mmol) were added to a solution of compound Z56-L23-4 (460 mg, crude product) in DMF (5 mL), and the mixture was reacted at room temperature for 10 min. After the reaction was completed, the reaction mixture was purified by prep-HPLC (trifluoroacetic acid method) to give compound Z56-L23-8 (500 mg, 0.253 mmol, yield: 71%). ES-API: [M+H]⁺=1978.0.

Step 7: Diethylamine (0.2 mL, 1.933 mmol) was added to a solution of compound Z56-L23-8 (100 mg, 0.051 mmol) in acetonitrile (3 mL), and the mixture was reacted at room temperature for 2 h. After the reaction was completed as detected by LC-MS, the reaction mixture was concentrated under reduced pressure to remove the solvent and diethylamine, so as to give compound Z56-L23-9 (110 mg, crude product), which was directly used in the next step. ES-API: [M+H]⁺=1756.4.

Step 8: 2,5-Dioxopyrrolidin-1-yl 3-(2,5-dioxo-2,5-dihydro-1H-pyrrol-1-yl)propanoate (30.30 mg, 0.114 mmol) and DIPEA (22.07 mg, 0.171 mmol) were sequentially added to a solution of compound Z56-L23-9 (110 mg, crude product) in acetonitrile (5 mL). The mixture was reacted at room temperature for 2 h and concentrated, and then the residue was purified by prep-HPLC (trifluoroacetic acid method) to give the desired product Z56-L23 (37 mg, 0.017 mmol, yield: 33.3%, trifluoroacetate). ES-API: [M+H]⁺=1906.7. ¹H NMR (400 MHz, DMSO-*d₆*) δ 10.16 (s, 1H), 9.01 (d, *J* = 2.0 Hz, 1H), 8.56 (d, *J* = 8.9 Hz, 1H), 8.50 (s, 1H), 8.23 (d, *J* = 6.6 Hz, 1H), 8.19 (d, *J =* 2.2 Hz, 1H), 8.04 (d, *J =* 7.9 Hz, 1H), 7.89 (d, *J* = 8.3 Hz, 2H), 7.82 (s, 1H), 7.80 - 7.72 (m, 4H), 7.62 - 7.51 (m, 3H), 7.00 (s, 2H), 5.56 (t, *J* = 9.0 Hz, 1H), 5.09 (d, *J* = 12.1 Hz, 1H), 4.83 (s, 1H), 4.66 (d, *J= 12.6* Hz, 2H), 4.40-4.30 (m, 6H), 4.27 - 4.04 (m, 11H), 3.83 (t, *J =* 7.7 Hz, 2H), 3.66 - 3.54 (m, 10H), 3.50 - 3.42 (m, 30H), 3.23 (s, 3H), 3.00 - 2.96 (m, 3H), 2.80 - 2.72 (m, 1H), 2.44 - 2.25 (m, 12H), 2.00 - 1.86 (m, 4H), 1.68-1.63 (m, 1H), 1.53-1.49 (m, 1H), 1.38-1.30 (m, 9H), 1.26 - 1.10 (m, 4H), 0.95 - 0.80 (m, 12H), 0.36 (s, 3H).

### Example A-35. Synthesis of Compound Z34-L4

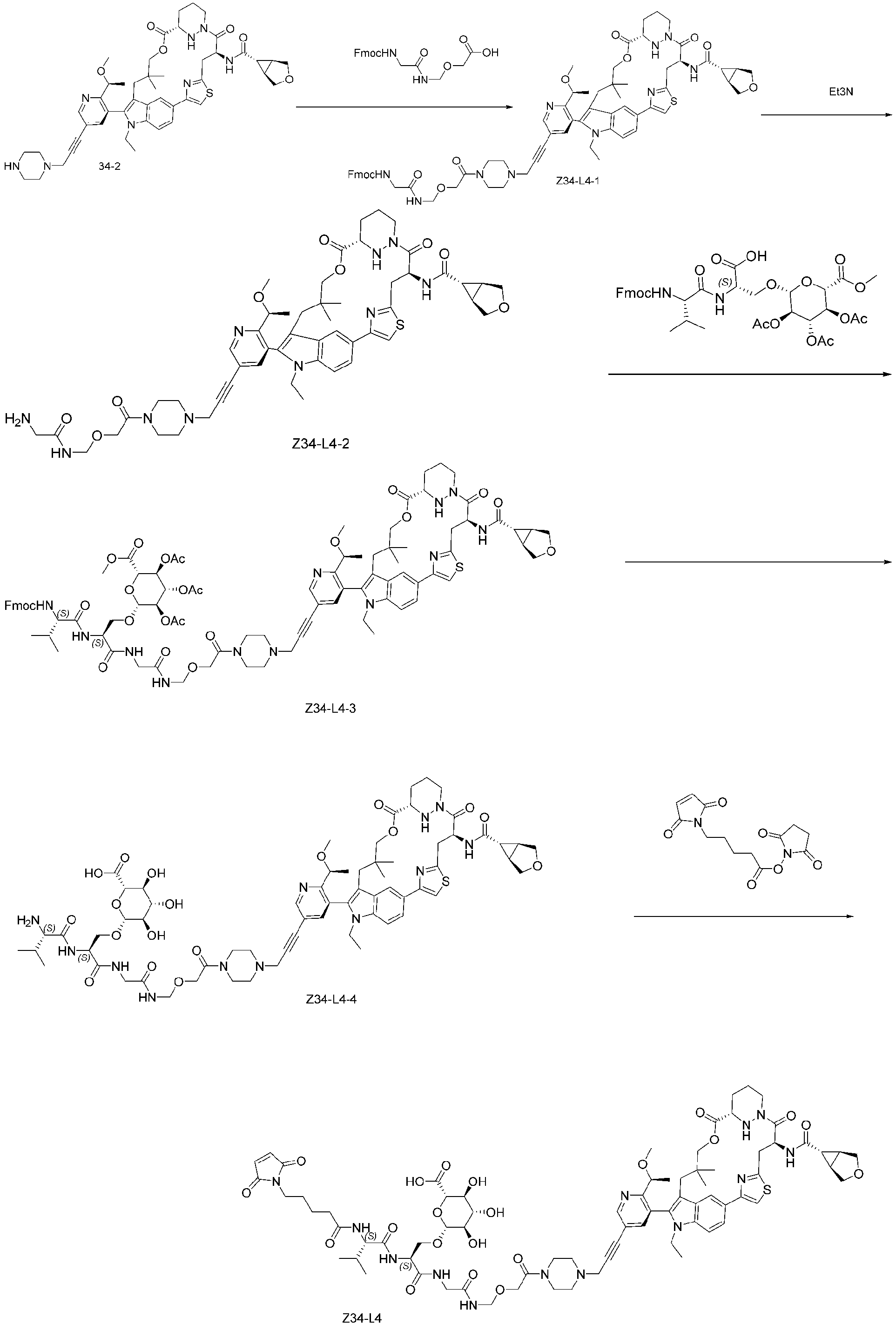

Step 1: Compound 34-2 (60 mg, 0.070 mmol) and {[1-(9H-fluoren-9-yl)-3,6-dioxo-4,7-diaza-2-octaoxa-8-yl]oxy}acetic acid (32.07 mg, 0.083 mmol) were dissolved in N,N-dimethylformamide (3 mL), and O-(7-azabenzotriazol-1-yl)-N,N,N',N'-tetramethyluronium hexafluorophosphate (39.65 mg, 0.104 mmol) and N,N'-diisopropylethylamine (22.46 mg, 0.174 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give compound Z34-L4-1 (220 mg, 0.179 mmol, yield: 77.22%) as a white solid, ES-API: [M+H]⁺ = 1229.3.

Step 2: Compound Z34-L4-1 (200 mg, 0.163 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z34-L4-2 (150 mg, 0.149 mmol, yield: 91.55%) as a colorless oily liquid, ES-API: [M+H]⁺ = 1007.3.

Step 3: Compound Z34-L4-2 (80 mg, 0.08 mmol) and (2S)-3-{[(2R,3R,4S,5S,6S)-3,4,5-triacetoxy-6-(methoxycarbonyl)-3,4,6-tetrahydro-2H-pyran-2-yl]oxy}-2-{[(5S)-1-(9H-fluoren-9-yl)-3,6-dioxo-5-(propan-2-yl)-4-aza-2-oxahex-6-yl]amino}propanoic acid (59 mg, 0.08 mmol) (for synthesis steps, refer to WO2023237050) were dissolved in N,N-dimethylformamide (3 mL), and O-(7-azabenzotriazol-1-yl)-N,N,N',N'-tetramethyluronium hexafluorophosphate (45 mg, 0.12 mmol) and N,N'-diisopropylethylamine (31 mg, 0.238 mmol) were added. After the addition was completed, the reaction system was stirred at room temperature for 2 h. After the reaction was completed, water (10 mL) was added, and the mixture was extracted with ethyl acetate (15 mL × 2). The organic phase was washed with saturated brine (10 mL × 3), dried, and concentrated to give a crude product. The crude product was purified by a flash silica gel column (methanol/dichloromethane: 0%-10%) to give compound Z34-L4-3 (95 mg, 0.114 mmol, yield: 83.35%) as a colorless oily liquid, ES-API: [M/2+H]⁺ = 866.3.

Step 4: Compound Z34-L4-3 (40 mg, 0.023 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z34-L4-4 (30 mg, 0.022 mmol, yield: 94.84%) as a colorless oily liquid, ES-API: [M/2+H]⁺ = 685.2.

Step 5: Compound Z34-L4-4 (20 mg, 0.015 mmol) was dissolved in N,N-dimethylformamide (1 mL), and N,N'-diisopropylethylamine (5.3 mg, 0.18 mmol) and N-succinimidyl 5-maleimidopentanoate (5.30 mg, 0.018 mmol) were added. The reaction system was stirred at room temperature for 3 h under a nitrogen atmosphere and purified by prep-HPLC (formic acid method 1) to give the desired product Z34-L4 (7 mg, 0.004 mmol, yield: 20.04%, formate) as a white solid, ES-API: [M/2+H]⁺ = 775.3.

### Example A-36. Synthesis of Compound Z52-L15

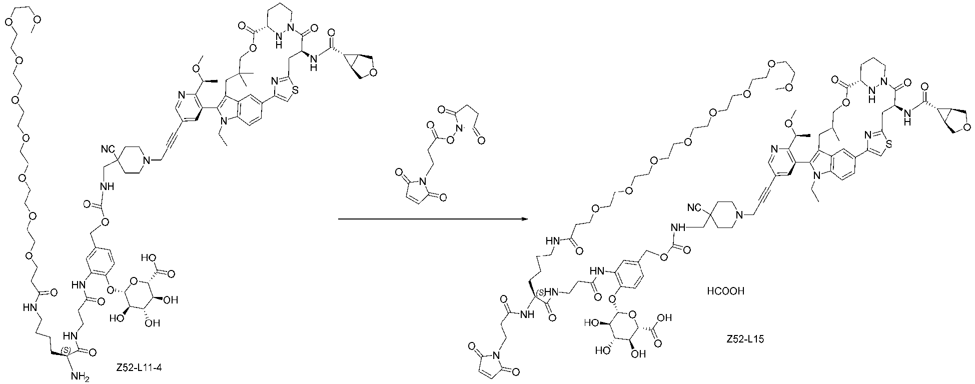

Step 1: N-Succinimidyl 3-maleimidopropionate (7.85 mg, 0.029 mmol) and compound Z52-L11-4-4 (60 mg, 0.023 mmol) were dissolved in N,N-dimethylformamide (1 mL), and N,N'-diisopropylethylamine (9.51 mg, 0.074 mmol) was added. The reaction system was stirred at room temperature for 3 h under a nitrogen atmosphere and purified by prep-HPLC (formic acid method 1) to give the desired product Z52-L15 (8 mg, 0.004 mmol, yield: 16.35%, formate) as a white solid, ES-API: [M/2+H]⁺ = 1001.5.

### Example A-37. Synthesis of Compound Z52-L16

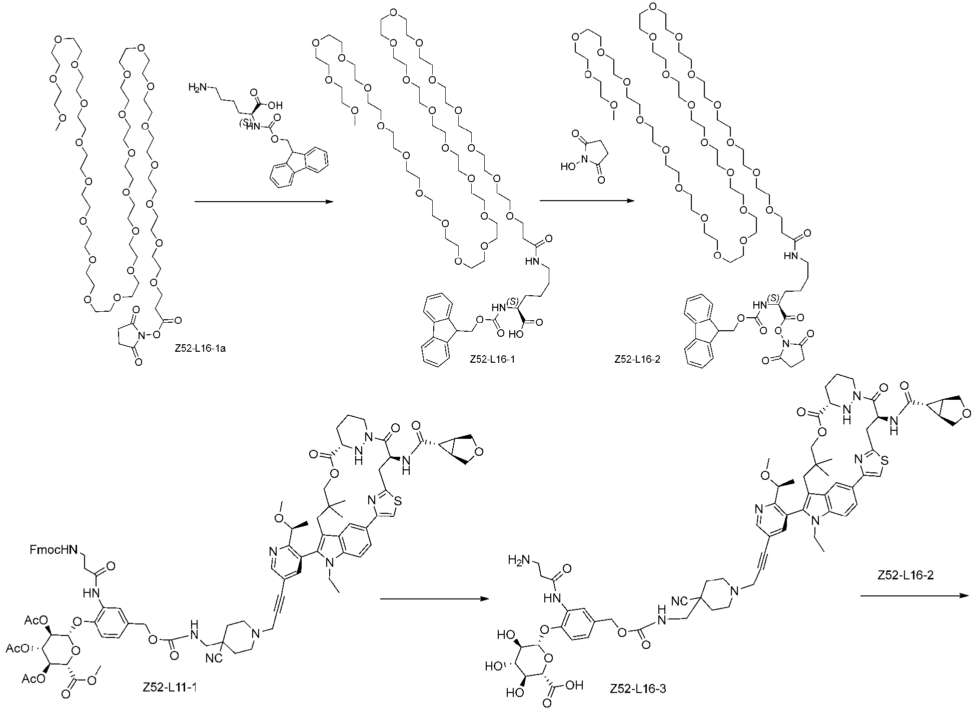

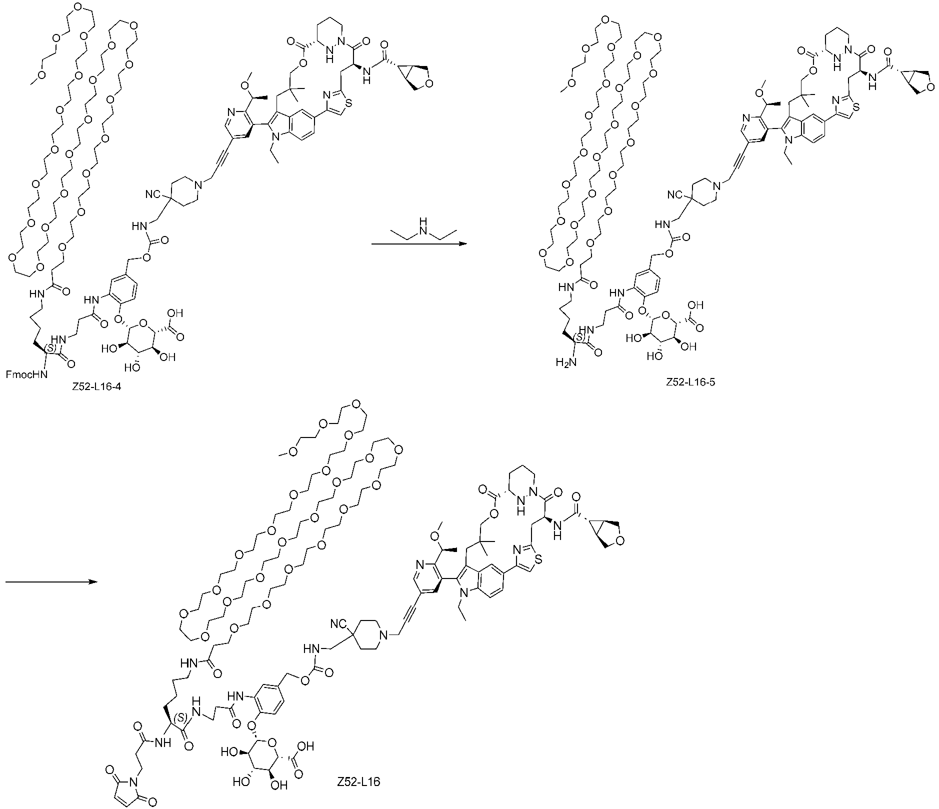

Step 1: N-{[(9H-Fluoren-9-ylmethyl)oxy]carbonyl}-L-lysine (151.69 mg, 0.412 mmol) and N,N-diisopropylethylamine (253.66 mg, 1.963 mmol) were added to a solution of compound Z52-L16-1a (500 mg, 0.981 mmol, see P247 of WO2022/40596 in detail) in anhydrous acetonitrile (15.0 mL). The reaction system was stirred for 20 min and then reacted at room temperature for 3 h under a nitrogen atmosphere. After the reaction was completed, the reaction mixture was concentrated by evaporation under reduced pressure to remove the solvent, so as to give compound Z52-L16-1 (605 mg, 0.412 mmol, yield: 100%), which was directly used in the next step.

Step 2: 1-Hydroxypyrrolidine-2,5-dione (56.90 mg, 0.494 mmol) and N,N'-diisopropylcarbodiimide (77.99 mg, 0.618 mmol) were added to a solution of compound Z52-L16-1 (640.7 mg, 0.412 mmol) in anhydrous tetrahydrofuran (15.0 mL), and the reaction mixture was stirred at room temperature for 2 h. After the reaction was completed as detected by LC-MS, the solution was purified by flash silica gel column chromatography [petroleum ether/tetrahydrofuran = 100:0 to 0:100 (v/v)] to give compound Z52-L16-2 (500.0 mg, yield: 77.55%). ES-API: [M/2 +H]⁺=783.1.

Step 3: Triethylamine (0.5 mL, 3.597 mmol) was added to a solution of compound Z52-L11-1 (280 mg, 0.166 mmol) in methanol/water (10 mL/3 mL), and the reaction mixture was stirred at 60 °C for 3 h under a nitrogen atmosphere. After the reaction was completed, the reaction mixture was concentrated to dryness by rotary evaporation under reduced pressure to remove the solvent, so as to give compound Z52-L16-3 (200 mg, 0.151 mmol, yield: 90.91%). ES-API: [M +H]⁺=1328.3.

Step 4: Compound Z52-L16-2 (240.0 mg, 0.153 mmol) and N,N-diisopropylethylamine (38.92 mg, 0.301 mmol) were added to a solution of compound Z52-L16-3 (200 mg, 0.151 mmol) in N,N-dimethylformamide (2 mL), and the mixture was stirred and reacted at room temperature for 1 h. After the reaction was completed, a reaction mixture of compound Z52-L16-4 was obtained, which was directly used in the next step. ES-API [M/2+H]⁺ = 1389.8.

Step 5: Diethylamine (0.2 mL, 1.505 mmol) was added to the reaction mixture of Z52-L16-4 in the previous step, and the reaction mixture was stirred at room temperature for 1 h. After the reaction was completed as monitored by LC-MS, a reaction mixture of compound Z52-L16-5 was obtained, which was directly used in the next step. ES-API: [M/2+H]⁺ = 1278.6.

Step 6: N-Succinimidyl 3-maleimidopropionate (48.09 mg, 0.181 mmol) and N,N-diisopropylethylamine (40.0 mg, 0.301 mmol) were added to the reaction mixture of compound Z52-L16-5 in the previous step. The mixture was stirred and reacted at room temperature for 1 h and purified by prep-HPLC (formic acid method 2) to give the desired product Z52-L16 (33.1 mg, yield: 8.12%). ES-API: [M/2+H] ⁺= 1354.1. ¹H NMR (400 MHz, DMSO-d₆) δ 9.09 (s, 1H), 8.92 - 8.87 (m, 1H), 8.60 - 8.55 (d, J= 8.8 Hz, 1H), 8.50 (s, 1H), 8.20 - 8.15 (s, 1H), 8.10 - 8.05 (m, 1H), 8.03 (s, 1H), 8.0 - 7.95 (s, 1H), 7.90 - 7.83 (m, 2H), 7.80 - 7.75 (m, 2H), 7.63 - 7.55 (m, 1H), 7.10 - 6.95 (m, 4H), 6.00 - 5.60 (m, 1H), 5.59 - 5.50 (m, 1H), 5.12 - 5.08 (m, 1H), 4.95 (s, 2H), 4.90 - 4.80 (m, 1H), 4.50 - 4.20 (m, 10H), 3.90 - 3.60 (m, 27H), 3.54 - 3.40 (m, 89H), 3.27 (s, 3H), 3.24 (s, 3H), 3.19 - 3.05 (m, 3H), 3.02 - 2.94 (m, 3H), 2.80 - 2.75 (m, 1H), 2.60 - 2.50(m, 2H), 2.45 - 2.25(m, 5H), 2.20 - 2.05(m, 3H), 1.95 - 1.85(m, 2H), 1.1.84 - 1.75 (m, 4H), 1.66 (s, 1H), 1.63 - 1.42 (m, 3H), 1.40 - 1.35 (m, 5H), 1.20 - 1.05 (m, 3H), 0.95 - 0.65 (m, 6H).

### Example A-38. Synthesis of Compound Z47-L6

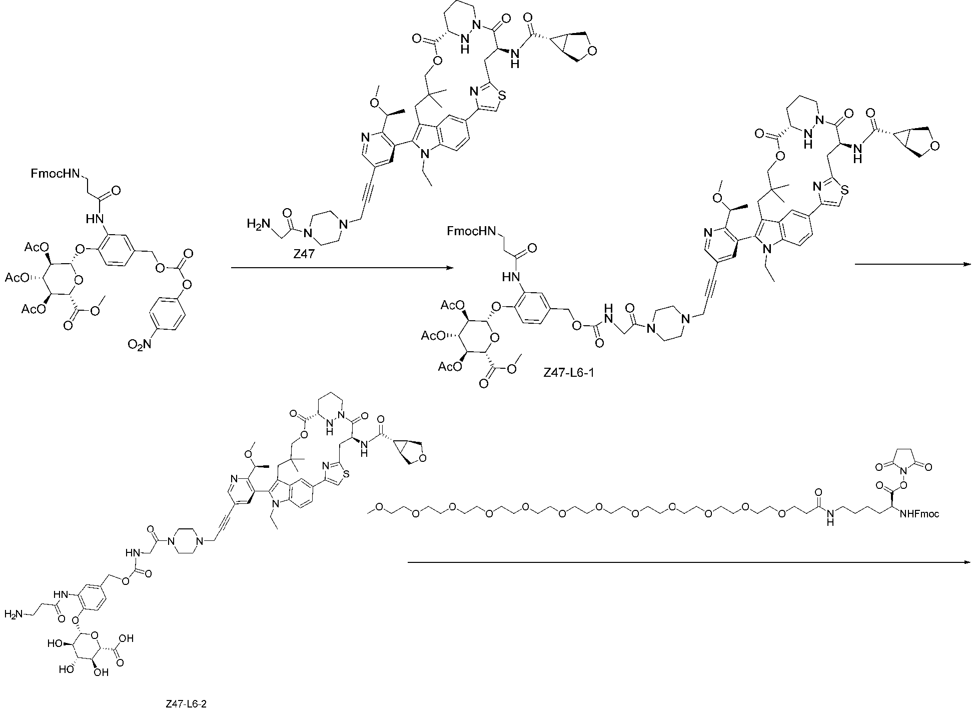

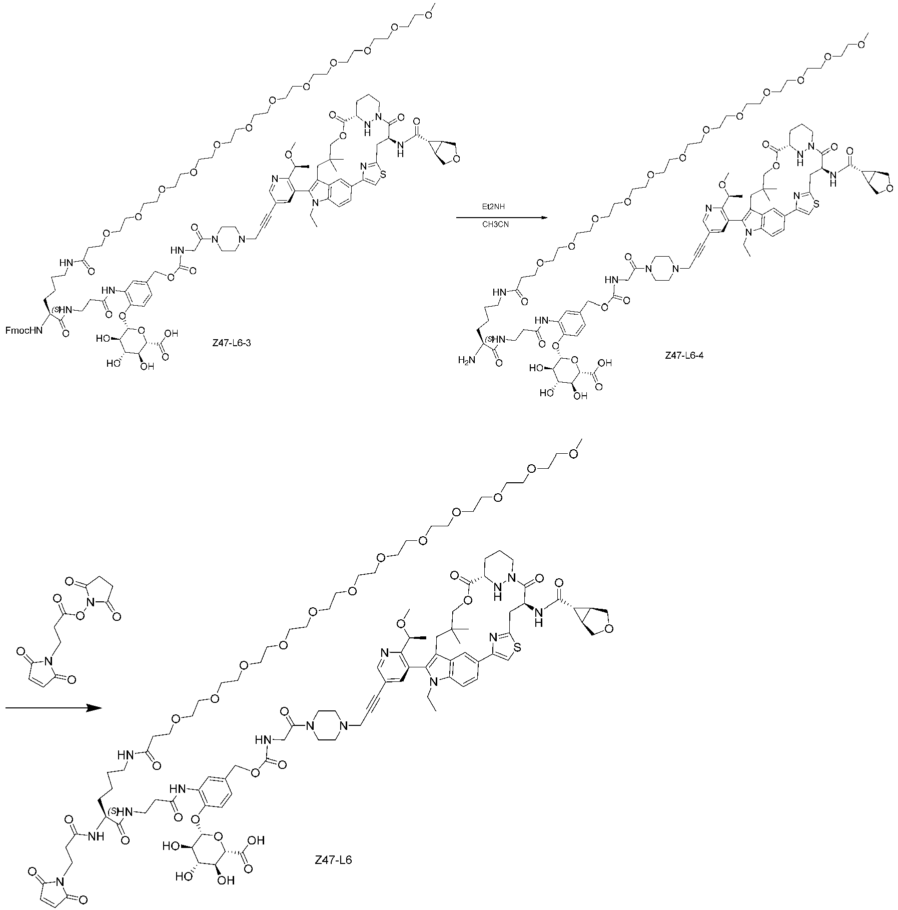

Step 1: Compound Z47 (200 mg, 0.217 mmol) and (2S,3R,4S,5S,6S)-2-(2-(3-((((9H-fluoren-9-yl)methoxy)carbonyl)amino)propanamido)-4-(((4-nitrophenoxy)carbonyloxy)methyl)phenoxy)-6-(methoxycarbonyl)tetrahydro-2H-pyran-3,4,5-triyl triacetate (198.63 mg, 0.217 mmol, CAS: 894095-98-8) were dissolved in N,N-dimethylformamide (5 mL) and pyridine (1 mL), and N,N'-diisopropylethylamine (0.072 mL, 0.435 mmol) and 1-hydroxybenzotriazole (29.37 mg, 0.217 mmol) were added. After the addition was completed, the reaction system was stirred at room temperature for 12 h under a nitrogen atmosphere. After the reaction was completed, water (15 mL) was added, and the mixture was extracted with ethyl acetate (20 mL × 3).The organic phase was washed with saturated brine (15 mL), dried over anhydrous sodium sulfate, and concentrated. The crude product was purified by a flash silica gel column (tetrahydrofuran/petroleum ether: 0%-60%) to give compound Z47-L6-1 (250 mg, 0.148 mmol, yield: 67.86%) as a colorless oily liquid, ES-API: [M/2+H]⁺ = 847.7.

Step 2: Compound Z47-L6-1 (200 mg, 0.163 mmol) was dissolved in methanol (3 mL) and water (1 mL), and triethylamine (0.15 mL) was added. The reaction system was stirred at 60 °C for 3 h under a nitrogen atmosphere and concentrated under reduced pressure to give compound Z47-L6-2 (150 mg, 0.149 mmol, yield: 91.55%) as a colorless oily liquid, ES-API: [M/2+H]⁺ = 666.7.

Step 3: Compound Z47-L6-2 (200 mg, 0.150 mmol) was dissolved in N,N-dimethylformamide (1 mL), and N,N'-diisopropylethylamine (38.0 mg, 0.3 mmol) and 9H-fluoren-9-ylmethyl {[(44S)-45-[(2,5-dioxotetrahydro-1H-pyrrol-1-yl)oxy]-38,45-dioxo-39-aza-2,5,8,11,14,17,20,23,26,29,32,35-dodecaoxapentatetracont-44-yl]amino} methanoate (171.08 mg, 0.165 mmol) (for synthesis steps, refer to WO2016149535) were added. The reaction system was stirred at room temperature for 3 h under a nitrogen atmosphere and purified by prep-HPLC (trifluoroacetic acid method) to give compound Z47-L6-3 (157 mg, 0.07 mmol, yield: 50.82%) as a colorless oily liquid, ES-API: [M/2+H]⁺ = 1127.5.

Step 4: Compound Z47-L6-3 (100 mg, 0.044 mmol) was dissolved in acetonitrile (1 mL), and diethylamine (0.2 mL) was added. The reaction system was stirred at room temperature for 2 h under a nitrogen atmosphere. After the reaction was completed, the reaction mixture was concentrated under reduced pressure to give compound Z47-L6-4 (85 mg, 0.042 mmol, yield: 94.30%) as a colorless oily liquid, ES-API: [M+H]⁺ = 1016.0.

Step 5: N-Succinimidyl 3-maleimidopropionate (10.48 mg, 0.039 mmol) and compound Z47-L6-4 (80 mg, 0.039 mmol) were dissolved in N,N-dimethylformamide (1 mL), and N,N'-diisopropylethylamine (9.51 mg, 0.074 mmol) was added. The reaction system was stirred at room temperature for 3 h under a nitrogen atmosphere and purified by prep-HPLC (formic acid method 1) to give the desired product Z47-L6 (30 mg, 0.013 mmol, yield: 32.79%, formate) as a white solid, ES-API: [M+H]⁺ = 1091.7.

### Example A-39. Synthesis of Compound Z64-1-L16

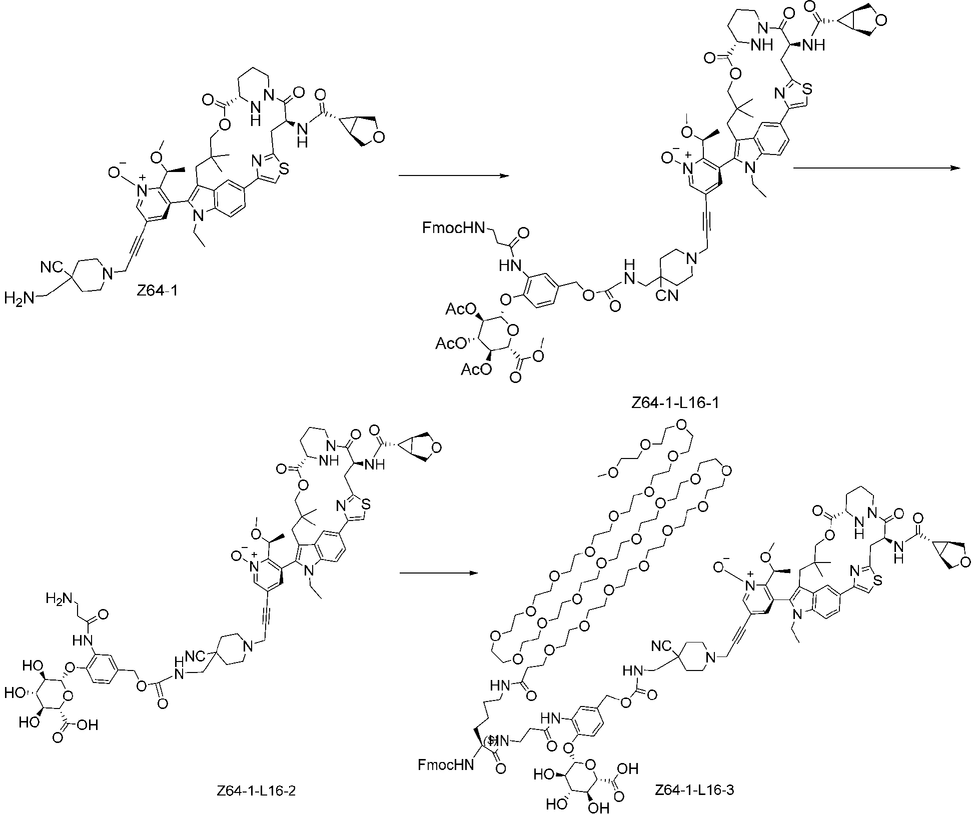

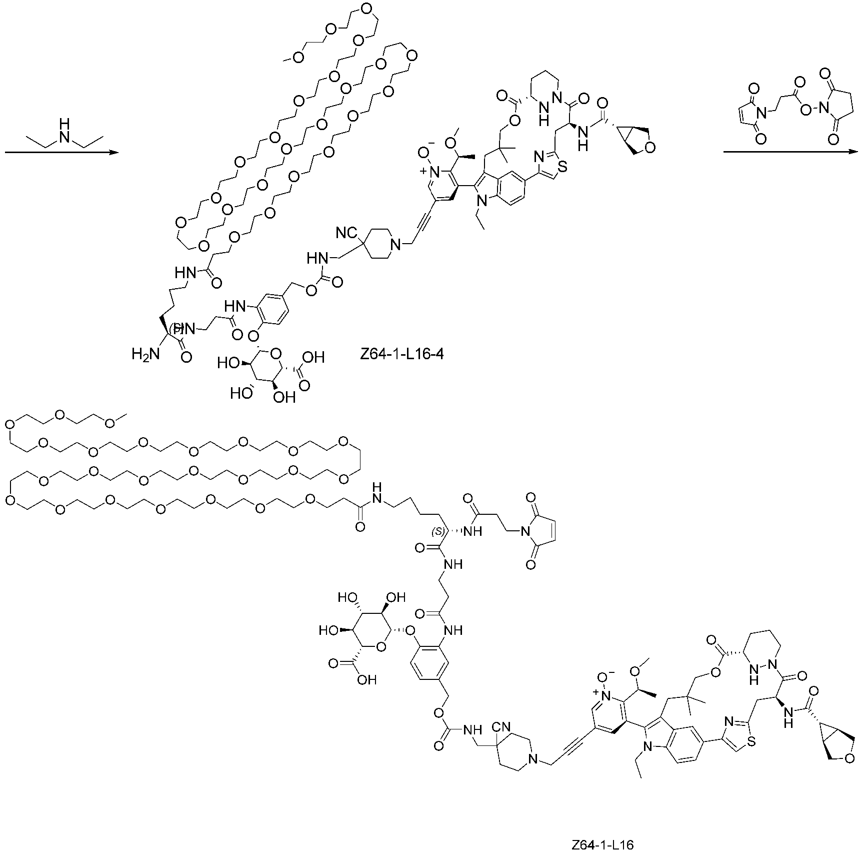

Step 1: (2S,3R,4S,5S,6S)-2-(2-(3-((((9H-Fluoren-9-yl)methoxy)carbonyl)amino)propanamido)-4-(((4-nitrophenoxy)carbonyloxy)methyl)phenoxy)-6-(methoxycarbonyl)tetrahydro-2H-pyran-3,4,5-triyl triacetate (220 mg, 0.241 mmol, CAS: 894095-98-8), pyridine (1.00 mL), 1-hydroxybenzotriazole (15.95 mg, 0.118 mmol), and N,N-diisopropylethylamine (30.50 mg, 0.236 mmol) were added to a solution of compound Z64-1 (110.0 mg, 0.118 mmol) in N,N-dimethylformamide (3 mL). The reaction mixture was stirred at room temperature for 12 h. After the reaction was completed as detected by LC-MS, The reaction mixture was diluted with water (15 mL), extracted with ethyl acetate (15 mL × 3), and washed with saturated brine (15 mL). The organic phase was concentrated under vacuum and purified by silica gel column chromatography [dichloromethane:methanol = 100:0 to 90:10 (v/v)] to give compound Z64-1-L16-1 (230.0 mg, crude product). ES-API [M+H]⁺ = 1707.3. Step 2: Triethylamine (0.5 mL, 4.833 mmol) was added to compound Z64-1-L16-1 (230.0 mg, crude product), and the reaction mixture was stirred at 60 °C for 3 h under a nitrogen atmosphere. After the reaction was completed as monitored by LC-MS, the mixture was concentrated to dryness by rotary evaporation under reduced pressure to remove the solvent, so as to give compound Z64-1-L16-2 (200 mg, crude product). ES-API[M/2+H] ⁺= 672.8.

Step 3: 2,5-Dioxopyrrolidin-1-yl (S)-80-((((9H-fluoren-9-yl)methoxy)carbonyl)amino)-74-oxo-2,5,8,11,14,17,20,23,26,29,32,35,38,41,44,47,50,53,56,59,62,65,68,71-tetracosaoxa-75-azahenoctacontan-81-oate (Z52-L16-2, 250.0 mg, 0.160 mmol) and N,N-diisopropylethylamine (34.90 mg, 0.270 mmol) were added to a solution of compound Z64-1-L16-2 (200 mg, crude product) in N,N-dimethylformamide (2 mL). The mixture was stirred and reacted at room temperature for 1 h. After the reaction was completed, the solution was purified by prep-HPLC (formic acid method 1) to give compound Z64-1-L16-3 (77.0 mg, yield: 20.41%). ES-API [M/2+H]⁺ = 1397.1.

Step 4: Diethylamine (0.2 mL, 1.505 mmol) was added to a solution of compound Z64-1-L16-3 (70.0 mg, 0.025 mmol) in N,N-dimethylformamide (2.0 mL), and the reaction mixture was stirred at room temperature for 1 h. After the reaction was completed as monitored by LC-MS, a reaction mixture of compound Z64-1-L16-4 was obtained, which was directly used in the next step. ES-API[M/2+H] ⁺= 1286.4.

Step 5: 1-{5-[(2,5-dioxotetrahydro-1H-pyrrol-1-yl)oxy]-5-oxopentyl}pyrrole-2,5-dione (15.0 mg, 0.056 mmol) and N,N-diisopropylethylamine (32.0 mg, 0.250 mmol) were added to the reaction mixture of compound Z64-1-L16-4 described above. The mixture was stirred and reacted at room temperature for 1 h and purified by prep-HPLC (formic acid method 2) to give the desired product Z64-1-L16 (18.0 mg, yield: 26.0%, formate). ES-API[M/2+H]⁺= 1361.8.

### Example A-40. Synthesis of Compound Z166-L14

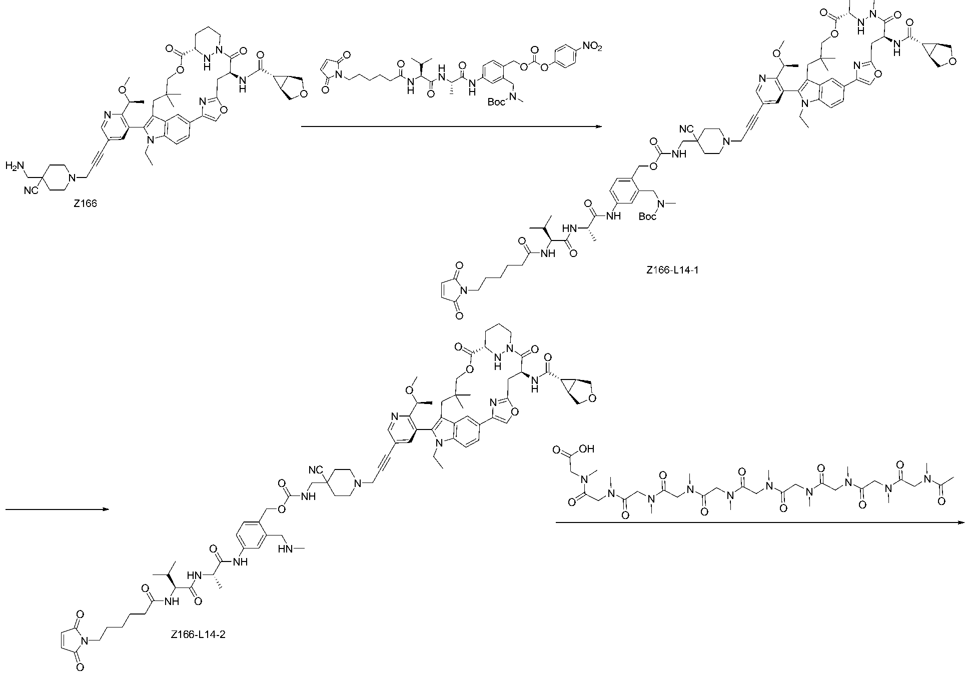

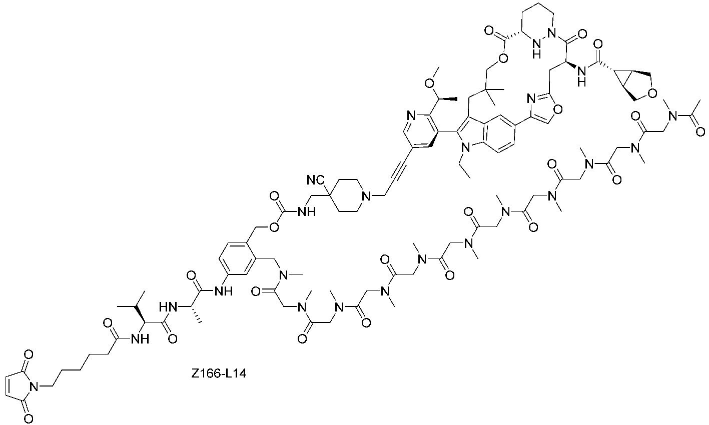

Step 1: Compound Z166 (52.99 mg, 0.067 mmol) and tert-butyl (5-((S)-2-((S)-2-(6-(2,5-dioxo-2,5-dihydro-1H-pyrrol-1-yl)hexanamido)-3-methylbutanamido))propanamido)-2-((((4-nitrophenoxy)carbonyl)oxy)methyl)benzyl)(methyl)carbamate (52.99 mg, 0.067 mmol, prepared by referring to the method for 11-3 in Example 1 of WO2024/83162) were dissolved in acetonitrile (2 mL), and DIPEA (36.19 mg, 0.280 mmol) was added. The mixture was reacted at room temperature for 18 h. The mixture was extracted with dichloromethane (20 mL × 2), washed with an aqueous sodium chloride solution (30 mL × 1), dried over anhydrous sodium sulfate, filtered, and concentrated. The resulting crude product was purified by silica gel column chromatography (methanol/dichloromethane = 1/15) to give compound Z166-L14-1 (60 mg, 0.039 mmol, yield: 69.42%), ES-API: [M+H]⁺ = 1555.8.

Step 2: Compound Z166-L14-1 (60 mg, 0.039 mmol) was dissolved in dichloromethane (2 mL), and trifluoroacetic acid (1 mL) was added. The mixture was reacted at room temperature for 1 h. The reaction mixture was concentrated to give compound Z166-L14-2 (53 mg, crude product). ES-API: [M+H]⁺=1455.5.

Step 3: 3,6,9,12,15,18,21,24,27,30-Decamethyl-4,7,10,13,16,19,22,25,28,31-decaoxo-3,6,9,12,15,18,21,24,27,30-decaazadotriacontanoic acid (28.06 mg, 0.036 mmol), DIPEA (14.12 mg, 0.109 mmol), and HATU (20.77 mg, 0.055 mmol) were sequentially added to a solution of compound Z166-L14-2 (53 mg, crude product) in dichloromethane (3 mL). The mixture was reacted at room temperature for 0.5 h and directly concentrated. The residue was purified by prep-HPLC (trifluoroacetic acid method) to give the desired product Z166-L14 (38 mg, 0.014 mmol, yield: 38.28%, trifluoroacetate). ES-API [M+2H⁺]/2 = 1105.3.

### Example A-41. Synthesis of Compound Z168-L23

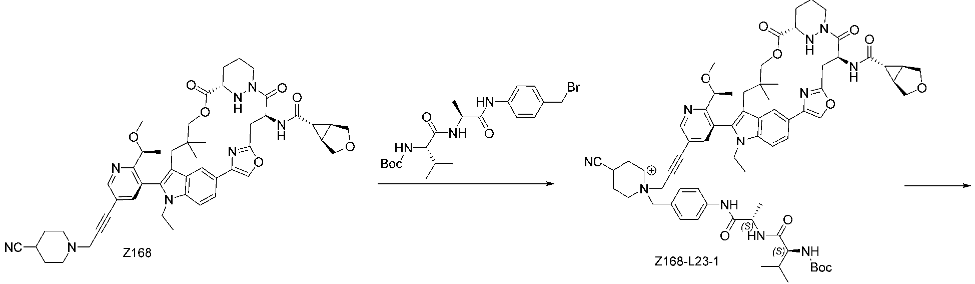

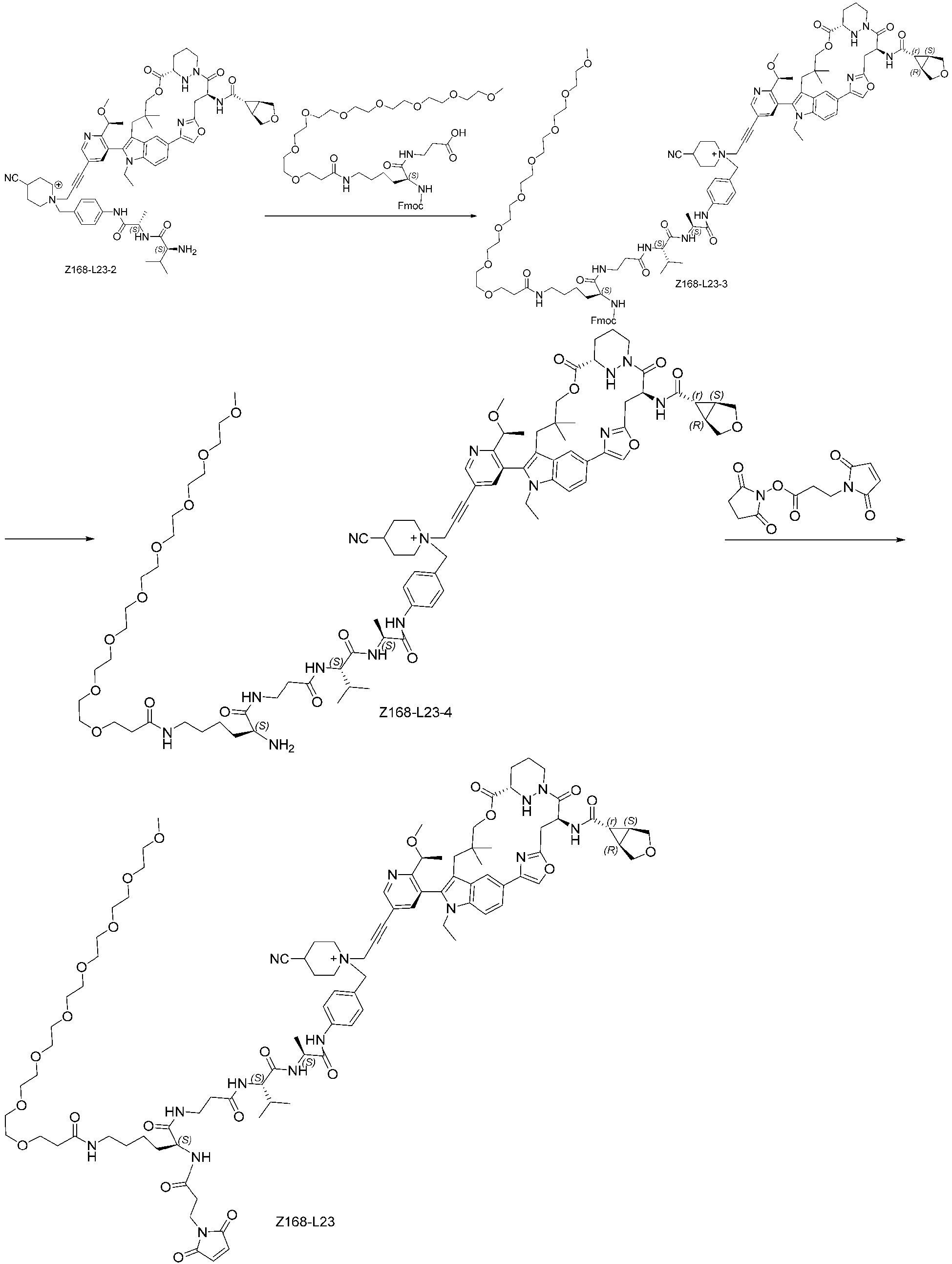

Step 1: Compound Z168 (100 mg, 0.115 mmol) and tert-butyl ((S)-1-(((S)-1-((4-(bromomethyl)phenyl)amino)-1-oxopropan-2-yl)amino)-3-methyl-1-oxobutan-2-yl)carbamate (104.71 mg, 0.230 mmol) were dissolved in acetonitrile (5 mL), and sodium bicarbonate (2.89 mg, 0.034 mmol) was added. The mixture was heated to 75 °C and reacted for 3 h. Dichloromethane (5 mL) was added. The mixture was filtered and concentrated, and the residue was purified by silica gel column chromatography (methanol/dichloromethane = 1/10) to give compound Z168-L23-1 (105 mg, 0.084 mmol, yield: 73.34%). ES-API: [M+H]⁺= 1246.8.

Step 2: Compound Z168-L23-1 (105 mg, 0.084 mmol) was dissolved in dichloromethane (3 mL), and trifluoroacetic acid (2 mL) was added. The mixture was reacted at room temperature for 0.5 h. After the reaction was completed, the reaction mixture was concentrated to give compound Z168-L23-2 (90 mg, crude product), which was directly used in the next step. ES-API: [M+H]⁺= 1147.4.

Step 3: (S)-32-((((9H-Fluoren-9-yl)methoxy)carbonyl)amino)-26,33-dioxo-2,5,8,11,14,17,20,23-octaoxa-27,34-diazaheptatriacontan-37-oic acid (85.07 mg, 0.102 mmol), DIPEA (30.43 mg, 0.235 mmol), and HATU (44.75 mg, 0.118 mmol) were added to a solution of compound Z168-L23-2 (90 mg, crude product) in dichloromethane (5 mL). The mixture was reacted at room temperature for 10 min. After the reaction was completed, the reaction mixture was purified by silica gel column chromatography (methanol/dichloromethane = 1/10) to give compound Z168-L23-3 (60 mg, 0.031 mmol, yield: 38.95%). ES-API: [M+H]⁺=1963.7.

Step 4: Diethylamine (0.128 mL, 1.240 mmol) was added to a solution of compound Z168-L23-3 (60 mg, 0.031 mmol) in CH₃CN (2 mL). The mixture was stirred at room temperature for another 2 h. After the reaction was completed as detected, the reaction mixture was concentrated to remove diethylamine, so as to give compound Z168-L23-4 (53.21 mg, crude product), which was directly used in the next step. ES-API: [M+H]⁺= 1739.7. Step 5: 2,5-Dioxopyrrolidin-1-yl 3-(2,5-dioxo-2,5-dihydro-1H-pyrrol-1-yl)propanoate (15.29 mg, 0.057 mmol) and DIPEA (18.56 mg, 0.144 mmol) were sequentially added to a solution of compound Z168-L23-4 (53.21 mg, crude product) in acetonitrile (3 mL). The mixture was reacted at room temperature for 2 h and purified by prep-HPLC (trifluoroacetic acid method) to give the desired product Z168-L23 (1.5 mg, yield: 2.50%, trifluoroacetate). ES-API: [M+H]⁺= 1892.8.

### Example A-42. Synthesis of Compound Z168-L24

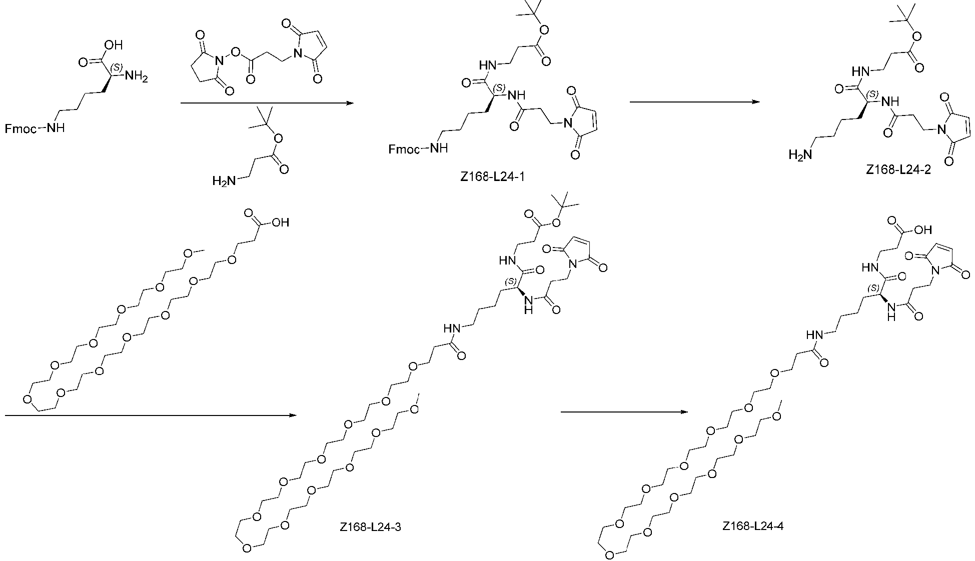

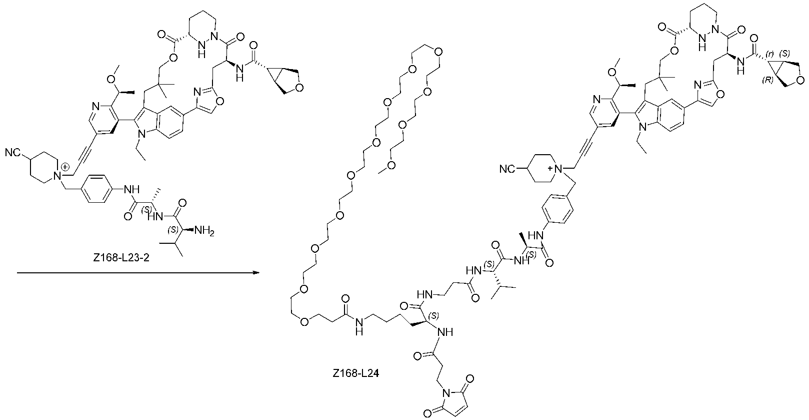

Step 1: 2,5-Dioxopyrrolidin-1-yl 3-(2,5-dioxo-2,5-dihydro-1H-pyrrol-1-yl)propanoate (867.06 mg, 3.257 mmol) and DIPEA (0.935 mL, 5.428 mmol) were sequentially added to a solution of N'-Fmoc-L-lysine (1000 mg, 2.714 mmol) in N,N-dimethylformamide (10 mL). The mixture was reacted at room temperature for 2 h, and then tert-butyl 3-aminopropanoate (0.51 g, 3.503 mmol) and HATU (2.05 g, 5.389 mmol) were sequentially added. The mixture was stirred at room temperature for another 0.5 h. The mixture was extracted with ethyl acetate (20 mL × 3), washed with an aqueous sodium chloride solution (30 mL ×), dried over anhydrous sodium sulfate, filtered, and concentrated. The resulting crude product was purified by silica gel column chromatography (50% ethyl acetate/petroleum ether) to give compound Z168-L24-1 (800 mg, 1.237 mmol, yield: 45.90%). ES-API: [M+H]⁺= 647.2.

Step 2: Compound Z168-L24-1 (200 mg, 0.309 mmol) was dissolved in N,N-dimethylformamide (2 mL), and DBU (94 mg, 0.618 mmol) was added under an ice-water bath. After 10 min, the reaction was completed. 1 M diluted hydrochloric acid was added for neutralization to give a reaction mixture of compound Z168-L24-2, which was directly used in the next step. ES-API [M+H]⁺ = 425.2.

Step 3: DIPEA (164.67 mg, 1.274 mmol), 2,5,8,11,14,17,20,23,26,29,32,35-dodecaoxaoctatriacontan-38-oic acid (250 mg, 0.425 mmol, CAS: 2135793-73-4), and HATU (322.95 mg, 0.849 mmol) were sequentially added to the reaction mixture of compound Z168-L24-2 described above. The mixture was reacted at room temperature for 10 min. After the reaction was completed, the reaction mixture was poured into water (20 mL) and extracted with isopropanol/dichloromethane (20 mL × 2, 1/3). The combined organic layers were washed with a saturated sodium bicarbonate solution (100 mL × 2), dried over anhydrous sodium sulfate, filtered, and concentrated. The resulting crude product was purified by silica gel column chromatography (methanol/dichloromethane = 1/10) to give compound Z168-L24-3 (150 mg, 0.151 mmol, yield: 48.8%). ES-API[M+H] ⁺= 995.4.

Step 4: Compound Z168-L24-3 (150 mg, 0.151 mmol) was dissolved in dichloromethane (3 mL), and trifluoroacetic acid (3 mL) was added. The mixture was reacted at room temperature for 1 h and then concentrated to give crude compound Z168-L24-4 (60 mg, crude product). ES-API[M+H] ⁺= 939.4.

Step 5: Compound Z168-L23-2 (46 mg, 0.040 mmol), DIPEA (30.80 mg, 0.238 mmol), and HATU (23.56 mg, 0.062 mmol) were sequentially added to a solution of compound Z168-L24-4 (60 mg, crude product) in N,N-dimethylformamide (2 mL). The mixture was reacted at room temperature for 10 min. After the reaction was completed, the reaction mixture was purified by prep-HPLC (trifluoroacetic acid method) to give the desired product Z168-L24 (13.67 mg, 0.006 mmol, yield: 15.6%, trifluoroacetate). ES-API [M/2+H]/⁺ = 1034.3.

### Example A-43. Synthesis of Compound Z169-1-L16

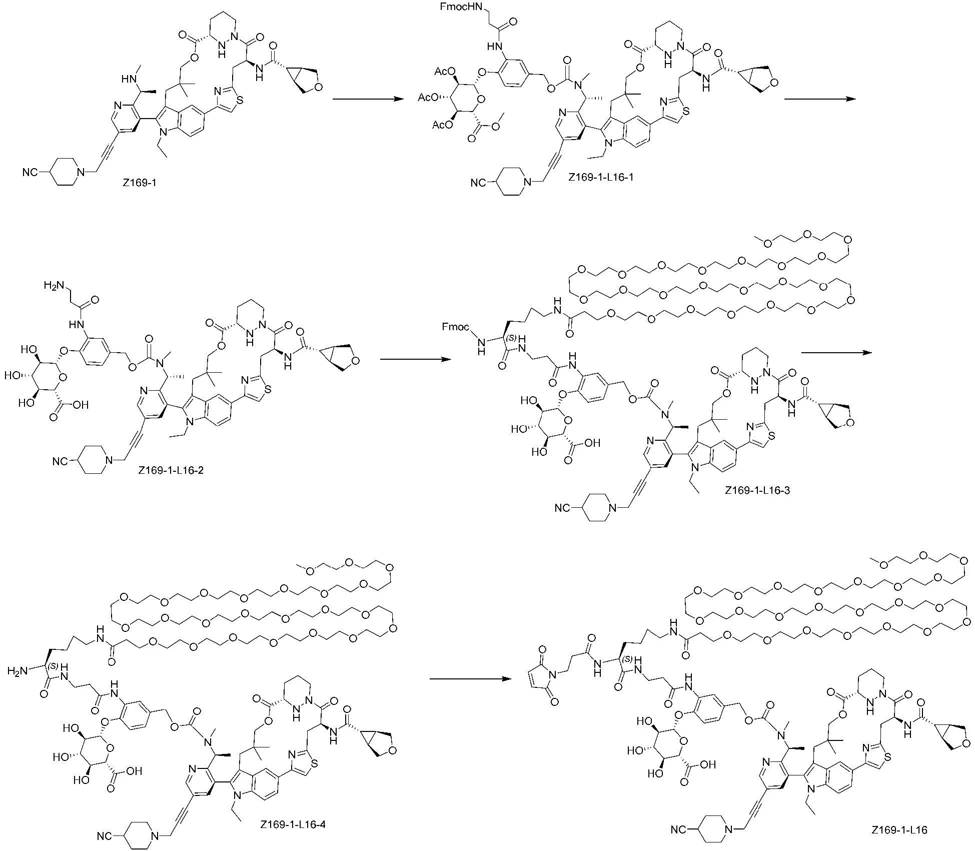

Step 1: Compound Z169-1 (44.31 mg, 0.05 mmol), (2S,3R,4S,5S,6S)-2-(2-(3-((((9H-fluoren-9-yl)methoxy)carbonyl)amino)propanamido)-4-(((4-nitrophenoxy)carbonyloxy)methyl)phenoxy)-6-(methoxycarbonyl)tetrahydro-2H-pyran-3,4,5-triyl triacetate (45.69 mg, 0.050 mmol, CAS: 894095-98-8), DIPEA (25.80 mg, 0.200 mmol), pyridine (1 mL), and HOBt (13.50 mg, 0.100 mmol) were mixed in DMF (5 mL). The mixture was stirred at 20 °C for 18 h. The mixture was extracted twice with ethyl acetate and water. The organic phase was washed with brine, dried, filtered, and concentrated, and then the residue was purified by column chromatography (DCM/MeOH = 100/4) to give compound Z169-1-L16-1 (66 mg, 0.040 mmol, yield: 79.48%), ES-API [M+1]⁺ = 1660.6.

Step 2: Z169-1-L16-1 (66 mg, 0.040 mmol), water (3 mg), and triethylamine (242.40 mg, 2.400 mmol) were mixed in methanol (10 mg). The mixture was stirred at 60 °C for 18 h. After the reaction was completed, the reaction mixture was concentrated to give crude compound Z169-1-L16-2, ES-API [M+1]⁺ = 1298.5.

Step 3: Crude compound Z169-1-L16-2, compound Z52-L16-2 (62.59 mg, 0.040 mmol), and DIPEA (20.68 mg, 0.160 mmol) were mixed in DMF (3 mL), and the mixture was stirred at 20 °C for 3 h to give a reaction mixture of Z169-1-L16-3, which was directly used in the next step. ES-API [M/3+1]⁺ = 916.3.

Step 4: Diethylamine (0.2 mL, 1.933 mmol) was added to the reaction mixture of Z169-1-L16-3 obtained in the previous step, and the resulting mixture was stirred at 20 °C for 1 h. After the reaction was completed, the reaction mixture was concentrated to give crude compound Z169-1-L16-4. ES-API[M/2+1]⁺=1263.1.

Step 5: Crude compound Z169-1-L16-4, 2,5-dioxopyrrolidin-1-yl 3-(2,5-dioxo-2,5-dihydro-1H-pyrrol-1-yl)propanoate (31.95 mg, 0.120 mmol), and DIPEA (51.70 mg, 0.400 mmol) were mixed in DMF (3 mL), and the mixture was stirred at 20 °C for 1 h. After the reaction was completed, the reaction mixture was concentrated, and the residue was purified by prep-HPLC (formic acid method) to give the desired product Z169-1-L16 (10 mg, four-step yield: 9.3%, purity: 98%). ES-API [M/2+H]⁺ = 1338.8.

### Example A-44. Synthesis of Compound Z56-L16

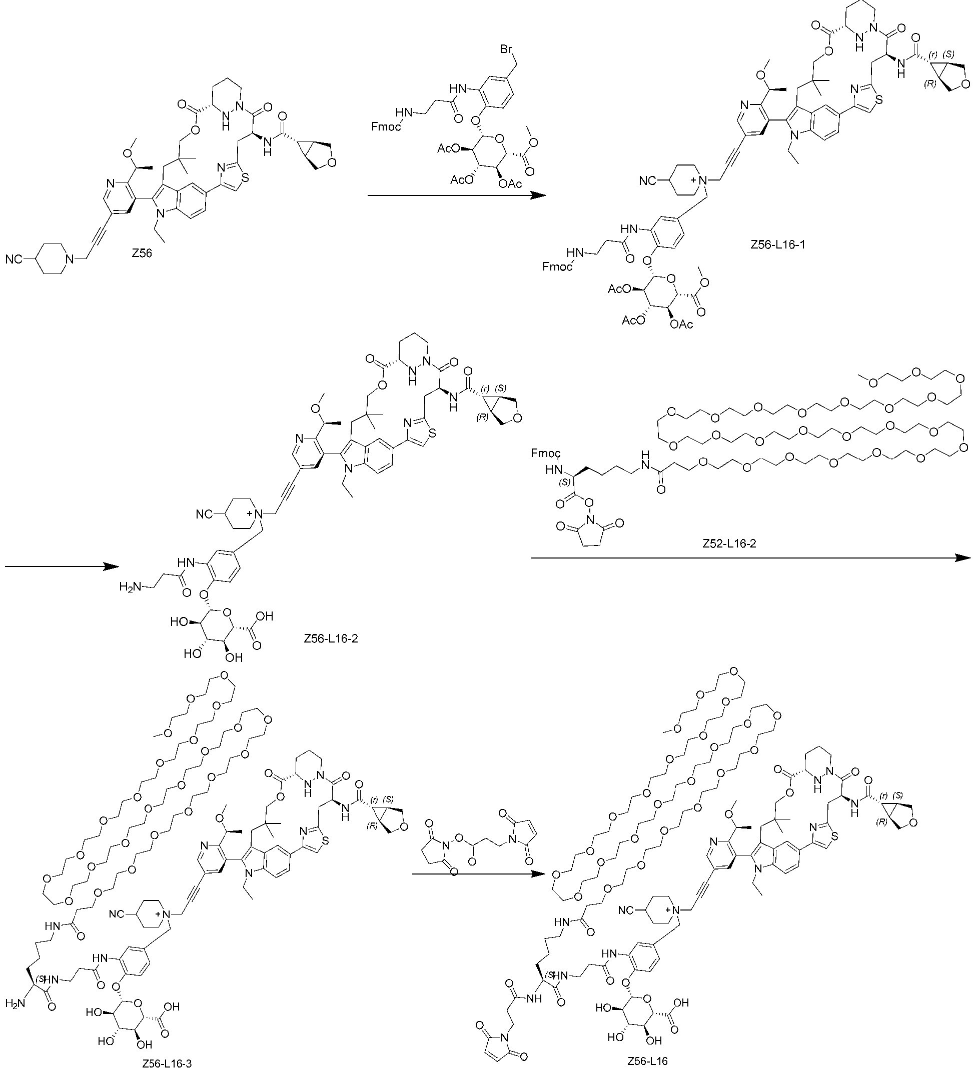

Step 1: Compound Z56 (430 mg, 0.494 mmol) and (2S,3R,4S,5S,6S)-2-(2-(3-((((9H-fluoren-9-yl)methoxy)carbonyl)amino)propanamido)-4-(bromomethyl)phenoxy)-6-(methoxycarbonyl)tetrahydro-2H-pyran-3,4,5-triyl triacetate (520.87 mg, 0.642 mmol, prepared by referring to the method for 2 in Example 1 of WO2016040684A1) were dissolved in acetonitrile (5 mL), and sodium bicarbonate (124.42 mg, 1.481 mmol) was added. The mixture was heated to 75 °C and reacted for 3 h. Dichloromethane (5 mL) was added. The mixture was filtered and concentrated, and the residue was purified by silica gel column chromatography (methanol/dichloromethane = 1/10) to give compound Z56-L16-1 (530 mg, 0.327 mmol, yield: 66.32%), ES-API: [M⁺] = 1618.4.

Step 2: Triethylamine (0.429 mL, 3.089 mmol) was added to a mixed solution of compound Z56-L16-1 (250 mg, 0.154 mmol) in methanol (3 mL) and water (1 mL), and the mixture was stirred at 65 °C for another 5 h. After the reaction was completed as detected, the reaction mixture was concentrated to give compound Z56-L16-2 (194 mg, crude product), which was directly used in the next step. ES-API: [M⁺] = 1255.4.

Step 3: Compound Z52-L16-2 (241.61 mg, 0.154 mmol) and DIPEA (39.84 mg, 0.309 mmol) were sequentially added to a solution of compound Z56-L16-2 (194 mg, crude product) in DMF (5 mL). The mixture was reacted at room temperature for 1 h. Diethylamine (0.064 mL, 0.621 mmol) was then added, and the mixture was stirred for another 30 min. The reaction mixture was purified by prep-HPLC (trifluoroacetic acid method) to give compound Z56-L16-3 (130 mg, 0.052 mmol, yield: 33.8%), ES-API: [M+3H⁺]/3 = 828.3.

Step 4: Compound Z56-L16-3 (130 mg, 0.052 mmol) was dissolved in DMF (2 mL), and DIPEA (20.16 mg, 0.156 mmol) and 2,5-dioxopyrrolidin-1-yl 3-(2,5-dioxo-2,5-dihydro-1H-pyrrol-1-yl)propanoate (13.84 mg, 0.052 mmol, CAS: 55750-62-4) were sequentially added. The mixture was reacted at room temperature for 2 h, and acetic acid (9.36 mg, 0.156 mmol) was added to quench the reaction. The reaction mixture was directly purified by prep-HPLC (formic acid method) to give the desired product Z56-L16 (20 mg, yield: 14.6%, formate). ES-API: [M+3H⁺]/3 = 878.7.

### Example A-45. Synthesis of Compound Z168-L16

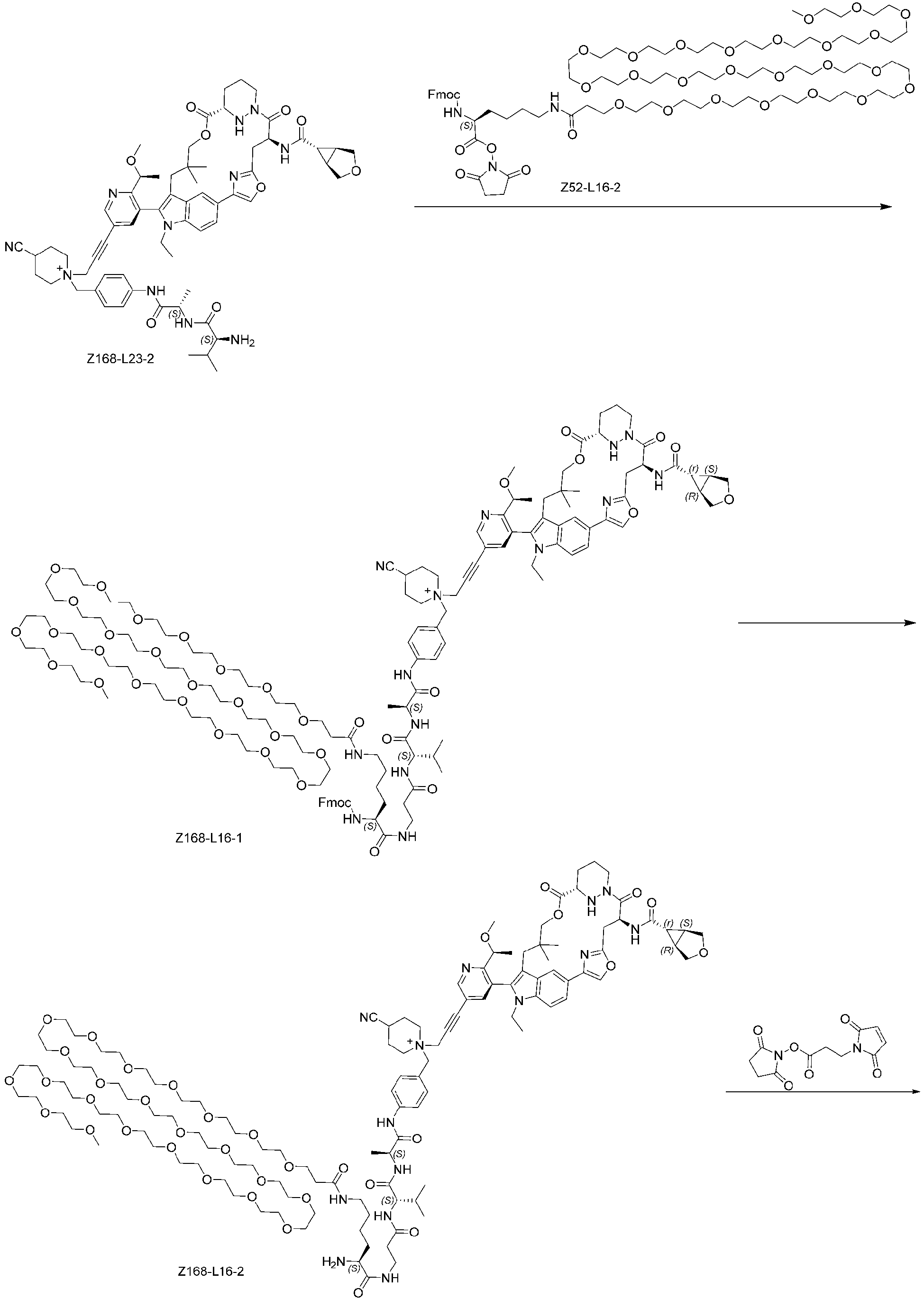

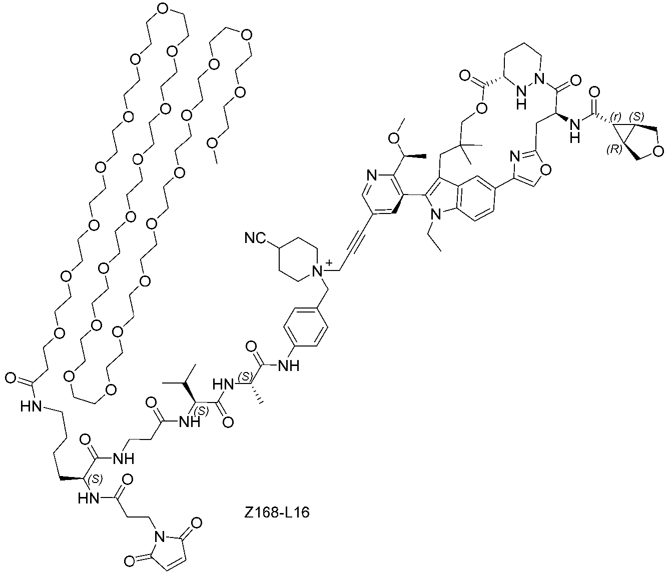

Step 1: Compound Z52-L16-2 (178.16 mg, 0.114 mmol) and DIPEA (36.60 mg, 0.283 mmol) were added to a solution of compound Z168-L23-2 (69 mg, 0.057 mmol) in DMF (2 mL). The mixture was reacted at room temperature overnight. After the reaction was completed, the reaction mixture was purified by silica gel column chromatography (methanol/dichloromethane = 1/10) to give compound Z168-L16-1 (30 mg, 0.011 mmol, yield: 19.86%). ES-API: [M+2H⁺]/2 = 1334.0.

Step 2: Diethylamine (0.011 mL, 0.110 mmol) was added to a solution of compound Z168-L16-1 (30 mg, 0.011 mmol) in acetonitrile (2 mL), and the mixture was stirred at room temperature for another 1 h. After the reaction was completed as detected, the reaction mixture was concentrated to remove diethylamine, so as to give compound Z168-L16-2 (27.50 mg, crude product), which was directly used in the next step. ES-API: [M+2H⁺]/2= 1223.5.

Step 3: 2,5-Dioxopyrrolidin-1-yl 3-(2,5-dioxo-2,5-dihydro-1H-pyrrol-1-yl)propanoate (5.86 mg, 0.022 mmol, CAS: 55750-62-4) and DIPEA (4.27 mg, 0.033 mmol) were sequentially added to a solution of compound Z168-L16-2 (27.50 mg, crude product) in acetonitrile (2 mL). The reaction mixture was reacted at room temperature for 18 h and purified by prep-HPLC (trifluoroacetic acid method) to give the desired product Z168-L16 (5.5 mg, yield: 18.29%, formate). ES-API: [M+3H⁺]/3= 866.1.

### Test Example 1: Cell p-ERK Assay

AsPC-1 cells were purchased from ATCC, Cat. No. CRL-1682; NCI-H441 cells were purchased from ATCC, Cat. No. HTB-174; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; trypsin (containing EDTA) was purchased from Gibco, Cat. No. 25200-072; DMSO was purchased from VWRAMRESCO, Cat. No. 0231-500ML; 96-well cell culture plates were purchased from Corning, Cat. No. 3599; white-bottomed, opaque 96-well plates were purchased from Cisbio, Cat. No. 66PL96025; Advance ERK phospho-T202/Y204 kit was purchased from Cisbio, Cat. No. 64AERPEH; cell counter was purchased from CHEMOMETEC, model: NC-200; microplate reader was purchased from PerkinElmer, model: EnVision.

AsPC-1, a human pancreatic cancer cell strain endogenously containing a KRAS^{G12D} mutation, was cultured in a complete medium containing RPMI-1640 + 10% FBS + 1% PS. NCI-H441, a human non-small cell lung cancer cell strain endogenously containing a KRAS^{G12V} mutation, was cultured in a complete medium containing RPMI-1640 + 10% FBS + 1% PS.

The cells were digested with trypsin (containing EDTA), collected in their corresponding complete media, and counted. The density of the cell suspensions was adjusted with their corresponding complete media, and 10000 AsPC-1 cells or 20000 NCI-H441 cells per well were seeded into a 96-well cell culture plate, with the volume of the medium in each well being 80 µL. The cell culture plate was incubated in an incubator at 37 °C with 5% CO₂ overnight. A series of 3.16-fold gradient dilutions of a 1000× compound stock solution were prepared with DMSO, and then diluted 200-fold with the complete medium described above to give 5× compound stock solutions. On the day after cell seeding, 20 µL of the 5× compound stock solution was added to each cell culture well. As a result, the final concentration of the compound in the cell culture well became 1×, and the DMSO content in the well was 0.1%. In addition to the compound-treated wells described above, cell-containing DMSO control wells were also set. A complete medium containing 0.5% DMSO was added at 20 µL/well. After the mixture was well mixed, the culture plate was incubated in the incubator at 37 °C with 5% CO₂ for another 3 h. The medium was then removed from the culture wells, and 50 µL of cell lysis buffer (from Advance ERK phospho-T202/Y204 kit) was added to each well. The mixture was well mixed and incubated for 30 min. Then, 16 µL of the mixture was transferred to a white-bottomed, opaque 96-well assay plate, and separately, 16 µL of cell lysis buffer was added to additional wells (serving as blank wells) in the assay plate. After the transfer was completed, 4 µL of p-ERK HTRF antibody mixture (from Advance ERK phospho-T202/Y204 kit) was added to each well of the assay plate, followed by a 4-hour incubation. The HTRF signal values (RLU) were then measured using a microplate reader, and the inhibition rate of the compound against p-ERK levels was calculated as follows: IR (%) = (RLU_{DMSO} - RLU_{compound}) / (RLU_{DMSO} - RLU_{blank}) × 100%, where RLU_{DMSO} is the average HTRF signal value from the DMSO control well samples, RLU_{blank} is the average HTRF signal value from the blank well samples, and RLU_{compound} is the HTRF signal value from the compound-treated well samples. Using an XLfit software, the IR (%) values were plotted against the logarithm of the compound concentrations, and the dose-response curves were fitted using the four-parameter method to calculate the IC₅₀ values.

The results show that the compounds of the present disclosure exhibited relatively high inhibitory activity against p-ERK in the cancer cells described above. The IC₅₀ values of some of the compounds were less than 1 µM, and the IC₅₀ values of some of the compounds were less than 500 nM or 100 nM, or even less than 1 nM or 0.5 nM. The results for some of the exemplary compounds are shown in Table 1-1 below.

**Table 1-1**

| Number | H441 p-ERK IC₅₀(nM) | AsPC-1 p-ERK IC₅₀(nM) | Number | H441 p-ERK IC₅₀(nM) | AsPC-1 p-ERK IC₅₀(nM) |
|---|---|---|---|---|---|
| Z3 | 0.72 | 0.59 | Z56 | 0.12 | 0.07 |
| Z4 | 0.53 | 0.52 | Z60 | 0.15 | 0.28 |
| Z6 | 0.21 | 0.15 | Z61 | 9.7 | - |
| Z7-1 | 0.5 | 0.51 | Z62 | 3.19 | - |
| Z7-2 | 0.45 | 0.42 | Z63-1 | 0.55 | 0.61 |
| Z10 | 0.18 | 0.12 | Z31-1 | 0.92 | 2.65 |
| Z11 | 0.21 | 0.11 | Z57 | 1.47 | 0.81 |
| Z12 | 0.15 | 0.36 | Z64-1 | 5.07 | 9.07 |
| Z14 | 0.41 | 0.45 | Z45-1 | 0.6 | 0.28 |
| Z15 | 0.24 | 0.42 | Z67 | 8.12 | 10.57 |
| Z16 | 1.14 | 1.67 | Z73 | 3.84 | 3.19 |
| Z17 | 0.22 | 0.26 | Z65 | 0.14 | 0.06 |
| Z19 | 0.13 | 0.24 | Z48 | 1.66 | 4.7 |
| Z20 | 0.09 | 0.16 | Z66 | 0.32 | 0.3 |
| Z21 | 0.51 | 1.68 | Z83 | 0.42 | 0.64 |
| Z22 | 1.09 | 1.54 | Z84 | 1.47 | 1.56 |
| Z23 | 0.1 | 0.08 | Z164 | 1.51 | 2.08 |
| Z24-1 | 3.76 | 2.64 | Z165 | 13.71 | 10.32 |
| Z27 | 0.64 | 0.51 | Z166 | 0.2 | 0.1 |
| Z28 | 0.48 | 0.42 | Z167 | 1.27 | 1.64 |
| Z29 | 0.38 | 0.07 | Z168 | 0.09 | 0.03 |
| Z31 | 2.39 | - | Z171 | 0.53 | 0.97 |
| Z30 | 0.15 | 0.07 | Z172-1 | 2.17 | 5.8 |
| Z34 | 0.27 | 0.28 | Z173 | 3.03 | - |
| Z40 | 0.45 | 0.38 | Z175 | 2.61 | 6.03 |
| Z42-1 | 1.8 | 1.32 | Z173-2 | 0.81 | 0.86 |
| Z51 | 0.18 | 0.27 | Z279 | 1.03 | 1.83 |
| Z47 | 0.58 | 2.33 | Z280 | 1.43 | - |
| Z52 | 0.13 | 0.06 | Z281 | 3.04 | - |
| Z44 | 0.42 | 1.73 | | | |

| | | | | | |
|---|---|---|---|---|---|
| Note: "-" denotes that no test was performed. | | | | | |

### Test Example 2: 3D Cell Proliferation Assay

MIA PaCa-2 cells were purchased from ATCC, Cat. No. CRL-1420; AsPC-1 cells were purchased from ATCC, Cat. No. CRL-1682; NCI-H441 cells were purchased from ATCC, Cat. No. HTB-174; NCI-H1975 cells were purchased from ATCC, Cat. No. CRL-5908; DMEM medium was purchased from Gibco, Cat. No. 11995-065; RPMI-1640 medium was purchased from Gibco, Cat. No. 11875-093; FBS was purchased from Gibco, Cat. No. 10099-141C; penicillin/streptomycin double-antibiotic solution (PS) was purchased from Gibco, Cat. No. 15140-122; Horse serum was purchased from Gibco, Cat. No. 16050130; trypsin (containing EDTA) was purchased from Gibco, Cat. No. 25200-072; DMSO was purchased from VWRAMRESCO, Cat. No. 0231-500ML; 384-well cell culture plates were purchased from Corning, Cat. No. 3830; white-bottomed, opaque 384-well assay plates were purchased from Corning, Cat. No. 3570; CellTiter Glo-3D kit was purchased from Promega, Cat. No. G9683; cell counter was purchased from CHEMOMETEC, model: NC-200; microplate reader was purchased from PerkinElmer, model: EnVision.

MIA PaCa-2, a human pancreatic cancer cell strain endogenously containing a KRAS^{G12C} mutation, was cultured in a complete medium containing DMEM + 10% FBS + 2.5% Horse serum + 1% PS. AsPC-1, a human pancreatic cancer cell strain endogenously containing a KRAS^{G12D} mutation, was cultured in a complete medium containing RPMI-1640 + 10% FBS + 1% PS. NCI-H441 cells, a human lung cancer cell strain endogenously containing a KRAS^{G12V} mutation, were cultured in a complete medium containing RPMI-1640 + 10% FBS + 1% PS. NCI-H1975, a KRAS wild-type human lung cancer cell strain whose cell proliferation depends on KRAS activity, was cultured in a complete medium containing RPMI-1640 + 10% FBS + 1% PS.

The cells were digested with trypsin (containing EDTA), collected in their respective low-serum complete media containing 2% FBS (for MIA Paca-2 cell culture: DMEM + 2% FBS + 0.5% Horse serum + 1% PS), and counted. After the cell density was adjusted as required, 200 MIA PaCa-2 cells, 800 AsPC-1 cells, 800 NCI-H441 cells, or 400 NCI-H1975 cells per well were seeded into a 384-well cell culture plate, with the volume of the medium in each well being 45 µL. The cell culture plate was incubated in an incubator at 37 °C with 5% CO₂ overnight. A series of 3.16-fold gradient dilutions of a 1000× compound stock solution were prepared with DMSO, and then diluted 100-fold with the complete medium containing 2% FBS described above to give 10× compound stock solutions. On the day after cell seeding, 5 µL of the 10× compound stock solution was added to each cell culture well. As a result, the final concentration of the compound in the cell culture well became 1×, and the DMSO content in the well was 0.1%. In addition to the compound-treated wells described above, cell-containing DMSO control wells (with 5 µL of the complete medium containing 1% DMSO added to each well) and cell-free medium control wells (containing 50 µL of the complete medium with 2% FBS) were also set. The culture plate was incubated in an incubator at 37 °C with 5% CO₂ for another 5 days, and then 30 µL of CellTiter-Glo working solution was added to each well. The mixture was well mixed, incubated for 30 min, left to stand at room temperature for 30 min, and then transferred to a white-bottomed, opaque 384-well assay plate at 40 µL/well. The chemiluminescence values (RLU) were measured using a microplate reader, and the cell proliferation inhibition rate for each well was calculated as follows: IR (%) = (RLU_{DMSO} - RLU_{compound}) / (RLU_{DMSO} - RLU_{blnk}) × 100%, where RLU_{DMSO} is the average RLU signal value from the DMSO control well samples, RLU_{blank} is the average RLU signal value from the medium control well samples, and RLU_{compound} is the RLU signal value from the compound-treated well samples. Using an XLfit software, the IR (%) values were plotted against the logarithm of the compound concentrations, and the dose-response curves were fitted using the four-parameter method to calculate the IC₅₀ values.

The results show that the compounds of the present disclosure exhibited relatively high inhibitory activity against the proliferation of KRAS mutant cancer cells, wherein the IC₅₀ values of some of the compounds were lower than 1000 nM, and the IC₅₀ values of some of the compounds were lower than 500 nM, or lower than 100 nM, or even lower than 1 nM or 0.5 nM. The results for some of the exemplary compounds are shown in Table 1-2 below.

**Table 1-2**

| Number | H441 AP IC₅₀(nM) | AsPC-1 AP IC₅₀(nM) | Number | H441 AP IC₅₀(nM) | AsPC-1 AP IC₅₀(nM) |
|---|---|---|---|---|---|
| Z3 | 0.28 | 0.18 | Z47 | 0.75 | 1.2 |
| Z6 | 0.28 | 0.31 | Z52 | 0.17 | 0.08 |
| Z7-1 | 0.32 | 0.52 | Z44 | 0.38 | 1.21 |
| Z7-2 | 0.35 | 0.56 | Z56 | 0.14 | 0.09 |
| Z10 | 0.13 | 0.16 | Z60 | 0.16 | 0.32 |
| Z11 | 0.1 | 0.15 | Z54 | 4.7 | 14 |
| Z12 | 0.21 | 0.44 | Z63-1 | 0.32 | 0.36 |
| Z13-1 | 3.36 | 3.7 | Z31-1 | 1.07 | 2.09 |
| Z15 | 0.35 | 0.31 | Z57 | 1.34 | 0.47 |
| Z17 | 0.35 | 0.46 | Z64-1 | 5.1 | 3.77 |
| Z19 | 0.44 | 0.41 | Z45-1 | 0.53 | 0.12 |
| Z20 | 0.19 | 0.25 | Z73 | 2.48 | 2.5 |
| Z21 | 0.41 | 1.11 | Z65 | 0.25 | 0.15 |
| Z22 | 2.31 | 8.37 | Z48 | 1.99 | 5.06 |
| Z23 | 0.06 | 0.03 | Z66 | 0.11 | 0.12 |
| Z24-1 | 1.77 | 1.73 | Z83 | 0.09 | 0.12 |
| Z27 | 0.44 | 0.78 | Z84 | 3.02 | 2.7 |
| Z29 | 0.21 | 0.09 | Z164 | 2.04 | 2.59 |
| Z34 | 0.47 | 0.77 | Z165 | 24.42 | 10.02 |
| Z30 | 0.11 | 0.07 | Z166 | 0.37 | 0.12 |
| Z28 | 0.4 | 0.37 | Z171 | 2.78 | 1.36 |
| Z40 | 0.4 | 0.43 | Z173-2 | 1.82 | 1.61 |
| Z42-1 | 0.65 | 0.33 | Z279 | 3.26 | 6.05 |
| Z51 | 0.28 | 0.42 | Z281 | 3 | 4.87 |

### Test Example 3: Assay for Binding of KRAS to cRAF

The recombinant RBD-c-RAF fragment protein with a GST tag (abbreviated as cRAF or RAF1) was purchased from BPS Bioscience, Cat. No. 100519; the recombinant KRAS G12V mutant protein with a His tag and pre-bound with GppNHp (GPPNP) was purchased from BPS Bioscience, Cat. No. 101359; the recombinant KRAS wild type (KRAS WT) protein with a His tag was purchased from Reaction Biology, Cat. No. CUST2019_01; Cyclophilin A protein was purchased from Biortus, Cat. No. PPIA-H00A; Tween-20 was purchased from Sigma, Cat. No. P9416; GPPNP was purchased from Sigma, Cat. No. G0635; the fluorescently labeled antibody MAb Anti GST-d2 was purchased from Cisbio, Cat. No. 61GSTDLA; the fluorescently labeled antibody MAb Anti-6HIS Tb cryptate Gold was purchased from Cisbio, Cat. No. 61HI2TLA; DMSO was purchased from VWRAMRESCO, Cat. No. 0231-500ML; MgCl₂ was purchased from Shyuanye, Cat. No. R21455; HEPES was purchased from invitrogen, Cat. No. 15630-080; DTT was purchased from Thermo Fisher, Cat. No. R0861; BSA was purchased from Sigma, Cat. No. B2064-100G; the ProxiPlate-384-well white assay plate was purchased from PerkinElmer, Cat. No. 6008289; the 96-well V-bottom storage plate was purchased from Corning, Cat. No. 3894; the thermostatic incubator was purchased from Shanghai Zhicheng Analytical Instruments, model: ZXMP-R1230; the microplate reader was purchased from PerkinElmer, model: EnVision.

An assay buffer was prepared, and the final concentrations of the components were as follows: 50 mM HEPES, 0.005% Tween-20, 0.1% BSA, and 1 mM DTT. A series of 3.16-fold gradient dilutions of a 300× compound stock solution were prepared with DMSO, and then diluted 40-fold with the assay buffer described above to give 7.5× compound working solutions, which were stored in V-bottom storage plates. 225 nM KRAS G12D or KRAS WT protein was mixed with 75 µM GPPNP, and the mixture was incubated in the thermostatic incubator at 20 °C for 60 min for later use. 2 µL of the 7.5× compound working solution and 4 µL of a 18.75 µM Cyclophilin A working solution were added to each well of an assay plate. The plate was centrifuged at 1000 rpm for 1 min and then incubated in the thermostatic incubator at 20 °C for 30 min. Then, 2 µL of a 225 nM GPPNP-KRAS G12V protein or GPPNP-KRAS WT mixed solution was added to each well. The plate was centrifuged at 1000 rpm for 1 min and then incubated in the thermostatic incubator at 20 °C for 60 min. A working solution containing 150 nM cRAF and 75 µM MgCl₂ was prepared with the assay buffer and added to the aforementioned assay plate at 2 µL/well. The plate was centrifuged at 1000 rpm for 1 min and then incubated in the thermostatic incubator at 20 °C for 15 min. A working solution containing 3 µg/mL MAb Anti GST-d2 and 0.15 µg/mL MAb Anti-6HIS Tb cryptate Gold was prepared with the assay buffer. The working solution was added to the aforementioned assay plate at 5 µL/well. The plate was centrifuged at 1000 rpm for 1 min and then incubated in the thermostatic incubator at 20 °C for 60 min. In addition to the compound-treated wells described above, DMSO control wells (2 µL of a buffer containing 0.75% DMSO was added to each well) and buffer control wells not containing the KRAS protein were also set. At the end of the assay (after the incubation with the fluorescently labeled antibody was completed), the luminescence values (RLU) of each well on the assay plate were measured using a microplate reader, and then the inhibition rate of the compound against the KRAS-cRAF interaction signal in each well was calculated as follows: IR (%) = (RLU_{DMSO} - RLU_{compound}) / (RLU_{DMSO} - RLU_{blank}) × 100%, where RLU_{DMSO} is the average RLU signal value from the DMSO control well samples, RLU_{blank} is the average RLU signal value from the buffer control wells not containing the KRAS protein, and RLU_{compound} is the RLU signal value from the compound-treated wells. Using an XLfit software, the IR (%) values were plotted against the logarithm of the compound concentrations, and the dose-response curves were fitted using the four-parameter method to calculate the IC₅₀ values, as shown in Tables 1-3 and 1-4.

The assay results show that the compounds of the present disclosure have superior binding activity.

**Table 1-3**

| | KRAS G12V-cRAF binding assay(nM) | KRAS WT-cRAF binding assay(nM) |
|---|---|---|
| Control compound 1 | 36 | 194 |
| Z11 | 12.3 | 27.7 |
| Z13-1 | 5.2 | 21.8 |
| Z6 | 10.7 | 19.2 |
| Z18 | 16.5 | 31 |
| Z34 | 17.1 | 25.3 |
| Z42-1 | 9.9 | 18.9 |
| Z47 | 19.3 | 34.4 |
| Z38-1 | 37.7 | 69.6 |
| Z52 | 13.3 | 16.5 |
| Z44 | 11.8 | 25.2 |
| Z64-1 | 4.1 | 31.2 |
| Z31-1 | 14.7 | 75 |
| Z57 | 6.3 | 38.8 |
| Z56 | 11.9 | 13.9 |
| Z54 | 12.1 | 74.8 |
| Z61 | 15.2 | 84.1 |
| Z60 | 15.2 | 15.3 |
| Z67 | 8.7 | 62 |
| Z65 | 5 | 22.9 |
| Z48 | 9.05 | 31.3 |
| Z66 | 8.54 | 19.5 |
| Z282 | 5.48 | 14.84 |
| Z13-2 | 5.1 | 22.6 |
| Z39 | 9.1 | 22.1 |
| Z20 | 7.5 | 25.3 |
| Z22 | 18.6 | 47.5 |
| Z29 | 18.5 | 37 |
| Z40 | 7.8 | 14.3 |

| | | |
|---|---|---|
| * Control compound 1 (RMC-6236, CAS No. 2765081-21-6) | | |

**Table 1-4**

| | KRAS G12V-cRAF binding assay(nM) | KRAS WT-cRAF binding assay(nM) |
|---|---|---|
| Control compound 2 | 10.91 | 48.65 |
| Z182 | 12.98 | 34.49 |
| Z169-1 | 17.52 | 58.25 |
| Z166 | 12.94 | 38.89 |
| Z167 | 13.88 | 40.94 |
| Z168 | 7.75 | 16.39 |
| Z155 | 8.29 | 18.67 |
| Z181 | 13.12 | 36.43 |

| | | |
|---|---|---|
| * Control compound 2 (CN 117720556 A, compound 41) 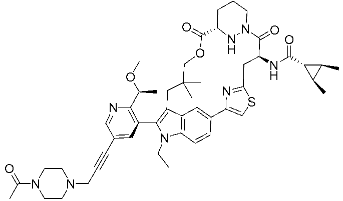 | | |

### Test Example 4: Human Liver microsomal Stability Experiment

This experiment was intended to investigate the metabolic stability of the test compounds in human liver microsomes. The materials used in the experiment included 10 mM solutions of the test compounds and the control sample, human liver microsomes, and reduced nicotinamide adenine dinucleotide phosphate (NADPH) powder. In the experiment, a 100 mM potassium phosphate buffer was used as the buffer system.

The liver microsome working solution was prepared by diluting the liver microsome stock solution to 0.75 mg/mL using the 100 mM potassium phosphate buffer. Then, 5 µL of the 10 mM stock solution of the test compound was taken, and 195 µL of methanol was added. The mixture was well mixed to prepare a working solution A with a concentration of 0.25 mM. Subsequently, the working solution A was diluted to 1.5 µM using the liver microsome working solution to prepare a compound-liver microsome mixed solution. Additionally, NADPH powder was dissolved in 100 mM potassium phosphate buffer to
prepare a 6 mM NADPH solution. The compound-liver microsome mixed solution was dispensed into a 96-well plate at 30 µL/well, with labeled time points of 0 min, 30 min, and 60 min. For 0-min samples, 180 µL of acetonitrile containing 100 ng/mL tolbutamide and 15 µL of the NADPH solution were added directly. The 96-well plate and the NADPH solution were then preheated under a 37 °C water bath for 5 min. After the preheating, 15 µL of the NADPH solution was added to each of the 30-min and 60-min sample wells. The plate was gently shaken for mixing and then incubated under the 37 °C water bath for the corresponding time. After the incubation was completed, 180 µL of acetonitrile containing 100 ng/mL tolbutamide was added to each sample well. The plate was sealed with a sealing film, shaken for 10 min (800 rpm/min), and then centrifuged for 15 min (4000 rpm). After the centrifugation, 50 µL of the supernatant was taken and added to a 96-well plate containing 150 µL of deionized water, and then the plate was sealed and shaken for 10 min (800 rpm) for uniform mixing. Subsequently, sample analysis was performed using liquid chromatography-tandem mass spectrometry (LC-MS/MS). The elimination rate constant and clearance rate of the test compound in liver microsomes were calculated using the following formulas: Remaining rate (%) = (peak area ratio of the compound to internal standard at any time point / peak area ratio of the compound to internal standard at 0 min) × 100%; T_{1/2} = 0.693/kₑ, where kₑ is the elimination rate constant, and T_{1/2} is the half-life; liver microsome intrinsic clearance rate (CL_{int(mic)}) = 0.693/(T_{1/2} × microsome protein concentration (mg/mL)); liver intrinsic clearance rate (CLᵢₙₜ₍ₗᵢᵥₑᵣ₎) = CL_{int(mic)} × microsome protein-to-liver weight ratio (mg/g) × liver-to-body weight ratio (g/kg), where CL_{int(mic)} is the liver microsome intrinsic clearance rate, CLᵢₙₜ₍ₗᵢᵥₑᵣ₎ is the liver intrinsic clearance rate, and CL₍ₗᵢᵥₑᵣ₎ is the liver clearance rate. For humans, the microsome protein-to-liver weight ratio was 45 mg/g, and the liver-to-body weight ratio was 20.7 g/kg. The stability results in human liver microsomes are shown in Table 1-5.

The experimental results show that the liver intrinsic clearance rates of the compounds of the present disclosure were generally lower than that of control compound 2, indicating that the compounds of the present disclosure have superior stability in human liver microsomes.

**Table 1-5. Assay results of stability in human liver microsomes**

| | T_{1/2} (min) | CLᵢₙₜ₍ₗᵢᵥₑᵣ₎ (mL/min/kg) |
|---|---|---|
| Control compound 2 | 9.8 | 164.1 |
| Z11 | 30.3 | 52.9 |
| Z182 | 16.4 | 97.9 |
| Z56 | 23.3 | 68.9 |
| Z52 | 47.3 | 33.9 |
| Z166 | 19.6 | 81.9 |
| Z282 | 18.1 | 88.5 |
| Z181 | 43.4 | 36.9 |

### Test Example 5: Solubility Experiment

This experiment was intended to determine the solubility of the compounds in media at different pH values. The materials used in the experiment included: a pH 1.2 medium, a pH 4.5 medium, a pH 7.4 medium, and a diluent (acetonitrile/water = 50/50).
1. The preparation methods for media are as follows:

| Medium | Preparation method |
|---|---|
| pH 1.2 medium | 0.765 mL of hydrochloric acid was measured out, water was added for dilution to bring the volume to 100 mL, and the mixture was well shaken to give the desired product |
| pH 4.5 medium | 1.79 g of anhydrous sodium acetate and 1.75 mL of acetic acid were taken, water was added for dilution to bring the volume to 1 L, and the mixture was well shaken to give the desired product. |
| pH 7.4 medium | 0.68 g of potassium dihydrogen phosphate was taken, 39.5 mL of 0.1 mol/L sodium hydroxide solution was added, water was added for dilution to bring the volume to 100 mL, and the mixture was well shaken to give the desired product |

2. Experimental procedures

One centrifuge tube (containing about 2 mg of the test sample) was taken, 1 mL of DMSO was added to completely dissolve the test sample, and the mixture was marked as stock-1. 0.2 mL of stock-1 was transferred into a 10 mL volumetric flask, and then the 50:50 (acetonitrile/water) solution was added to bring the volume to the mark, so as to give a control solution.

Additionally, centrifuge tubes each containing about 1 mg of the test sample were taken, 1 mL of the corresponding medium solution was then added, and the tubes were shaken on a 37 °C water bath shaker for 24 h. After centrifugation for 3 times (centrifugation time: 10 min, rotation speed: 12000 rpm), 0.2 mL of the supernatants were taken, 0.8 mL of the diluent (acetonitrile/water = 50/50) was added to each tube, and the mixtures were vortexed and mixed well to give solubility samples.

The solubility samples described above and the control solution were sequentially analyzed by high performance liquid chromatography. The chromatographic conditions are as follows:

| Chromatographic column: C18 column, 4.6 × 150 mm, 3.5 µm | | | | | | | |
|---|---|---|---|---|---|---|---|
| Mobile phase A: 0.05% TFA/H₂O; mobile phase B: ACN | | | | | | | |
| Column temperature: | 40°C | Flow rate: | 1.2ml/min | Wavelength: | 254nm | Injection volume: | 10ul |
| Gradient elution | Time | 0 | 1 | 5 | 8 | 8.01 | 10 |
| | A | 90 | 90 | 10 | 10 | 90 | 90 |
| | B | 10 | 10 | 90 | 90 | 10 | 10 |

### 3. Calculation method

The actual concentration of the compound in each solubility sample was calculated according to an external standard method. $Concentration \left(μg/mL\right) = \frac{{A}_{Sample} \times {C}_{Control} \times {D}_{Sample}}{{A}_{Control}} ∗ 1000 ;$ Aₛₐₘₚₗₑ: the peak area of the target peak in the sample solution; C_{control}: the concentration (mg/mL) of the control solution; Dₛₐₘₚₗₑ: the dilution factor of the sample solution; A_{control}: the peak area of the target peak in the control solution.

The experimental results (as shown in Table 1-6) show that the compounds of the present disclosure have superior solubility and hydrophilicity. Using a compound with relatively good hydrophilicity to be conjugated to an antibody via a linker can avoid the problems of protein precipitation and aggregation during the ADC conjugation, while improving the PK and stability of the ADC drugs.

**Table 1-6**

| | Solubility at pH 7.4 (µg/mL) | | Solubility at pH 7.4 (µg/mL) |
|---|---|---|---|
| Control compound 2 | 1.5 | Z61 | 1025.79 |
| Z6 | 7.8 | Z63-2 | 15.37 |
| Z16 | 14.96 | Z63-1 | 7.53 |
| Z18 | 22.4 | Z31-2 | 22.01 |
| Z20 | 10.86 | Z45-1 | 5.01 |
| Z21 | 9.71 | Z45-2 | 8.42 |
| Z24-1 | 12.31 | Z67 | 101.18 |
| Z27 | 20.81 | Z73 | 96.57 |
| Z34 | 16.22 | Z48 | 71.22 |
| Z42-1 | 15.57 | Z66-1 | 158.5 |
| Z47 | 109 | Z66 | 66.54 |
| Z38-2 | 176.02 | Z166 | 26.76 |
| Z38-1 | 97.04 | Z167 | 362.75 |
| Z44 | 55.75 | Z40 | 5.1 |
| Z54 | 36.22 | | |

### II. Preparation of ADCs:

A naked antibody was exchanged into a buffer, and a reducing agent was added. The mixture was stirred. The excess reducing agent was removed to form a reduced naked antibody. A linker-payload A1 (e.g., compound AI-3, compound AII-3, compound AIII-3, compound AIV-3, compound AV-3, or compound AVI-3) solution was added to the naked antibody, the mixture was stirred, and the excess A1 was removed to form an ADC.

### Example B-1: Preparation of Antibody-Drug Conjugates

Amino acid sequences of EGFR monoclonal antibody, HER3 monoclonal antibody, and EGFR×HER3 bispecific antibody
>EGFR antibody HC (SEQ ID NO: 1) 
QVQLQESGPGLVKPSETLSLTCTVSGFSLSNYDVHWVRQAPGKGLEWLGVIWSGGNTDYNTPFTSRL
TISVDTSKNQFSLKLSSVTAADTAVYYCARALDYYDYEFAYWGQGTLVTVSSASTKGPSVFPLAPSSK
 
STSGGTAALGCLVKDYFPEPVTVSWNSGALTSGVHTFPAVLQSSGLYSLSSVVTVPSSSLGTQTYICNV
NHKPSNTKVDKRVEPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVLDSDGSFF
LYSKLTVDKSRWQQGNVFSCSVMHEALHNHYTQKSLSLSPGK*
>EGFR antibody LC (SEQ ID NO: 2)

EIVLTQSPDFQSVTPKEKVTITCRASQSIGTNIHWYQQKPDQSPKLLIKYASESISGIPSRFSGSGSGTDFT
LTINSLEAEDAATYYCQQNNEWPTSFGQGTKLEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSF
NRGEC*

| SEQ ID NO: | EGFR antibody | Sequence information |
|---|---|---|
| 3 | VH | QVQLQESGPGLVKPSETLSLTCTVSGFSLSNYDVHWVRQAPGKGLEWLG VIWSGGNTDYNTPFTSRLTISVDTSKNQFSLKLSSVTAADTAVYYCARAL DYYDYEFAYWGQGTLVTVSS |
| 4 | CDR-H1 | NYDVH |
| 5 | CDR-H2 | VIWSGGNTDYNTPFTS |
| 6 | CDR-H3 | ALDYYDYEFAY |
| 7 | VL | EIVLTQSPDFQSVTPKEKVTITCRASQSIGTNIHWYQQKPDQSPKLLIKYAS ESISGIPSRFSGSGSGTDFTLTINSLEAEDAATYYCQQNNEWPTSFGQGTKL EIKRTV |
| 8 | CDR-L1 | RASQSIGTNIH |
| 9 | CDR-L2 | YASESIS |
| 10 | CDR-L3 | QQNNEWPTS |

>HER3 antibody HC (SEQ ID NO: 11) 
QVQLQQWGAGLLKPSETLSLTCAVYGGSFSGYYWSWIRQPPGKGLEWIGEINHSGSTNYNPSLKSRVT
ISVETSKNQFSLKLSSVTAADTAVYYCARDKWTWYFDLWGRGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAK
GQPREPQVYTLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPGK*
>HER3 antibody LC (SEQ ID NO: 12)

DIEMTQSPDSLAVSLGERATINCRSSQSVLYSSSNRNYLAWYQQNPGQPPKLLIYWASTRESGVPDRFS
GSGSGTDFTLTISSLQAEDVAVYYCQQYYSTPRTFGQGTKVEIKRTVAAPSVFIFPPSDEQLKSGTASVV
CLLNNFYPREAKVQWKVDNALQSGNSQESVTEQDSKDSTYSLSSTLTLSKADYEKHKVYACEVTHQ
GLSSPVTKSFNRGEC*

| SEQ ID NO: | HER3 antibody | Sequence information |
|---|---|---|
| 13 | VH | |
| 14 | CDR-H1 | GYYWS |
| 15 | CDR-H2 | EINHSGSTNYNPSLKS |
| 16 | CDR-H3 | DKWTWYFDL |
| 17 | VL | DIEMTQSPDSLAVSLGERATINCRSSQSVLYSSSNRNYLAWYQQNPGQ PPKLLIYWASTRESGVPDRFSGSGSGTDFTLTISSLQAEDVAVYYCQQY YSTPRTFGQGTKVEIKRTV |
| 18 | CDR-L1 | RSSQSVLYSSSNRNYLA |
| 19 | CDR-L2 | WASTRES |
| 20 | CDR-L3 | QQYYSTPRT |

>EGFR×HER3 bsAb HC (SEQ ID NO: 21) 
QVQLKQSGPGLVQPSQSLSITCTVSGFSLTNYGVHWVRQSPGKGLEWLGVIWSGGNTDYNTPFTSRLS
INKDNSKSQVFFKMNSLQSNDTAIYYCARALTYYDYEFAYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVN
HKPSNTKVDKRVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGGGGGSGGGGSQVQLQESGGGLVKPGGS
LRLSCAASGFTFSSYWMSWVRQAPGKGLEWVANINRDGSASYYVDSVKGRFTISRDDAKNSLYLQM
NSLRAEDTAVYYCARDRGVGYFDLWGRGTLVTVSSGGGGSGGGGSGGGGSQSALTQPASVSGSPGQS
ITISCTGTSSDVGGYNFVSWYQQHPGKAPKLMIYDVSDRPSGVSDRFSGSKSGNTASLIISGLQADDEA
DYYCSSYGSSSTHVIFGGGTKVTVL*
>EGFR×HER3 bsAb LC (SEQ ID NO: 22)

DILLTQSPVILSVSPGERVSFSCRASQSIGTNIHWYQQRTNGSPRLLIKYASESISGIPSRFSGSGSGTDFTL
SINSVESEDIADYYCQQNNNWPTTFGAGTKLELKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSF
NRGEC*

| SEQ ID NO: | Bispecific antibody | Sequence information |
|---|---|---|
| 23 | Anti-EGFR VH | QVQLKQSGPGLVQPSQSLSITCTVSGFSLTNYGVHWVRQSPGK GLEWLGVIWSGGNTDYNTPFTSRLSINKDNSKSQVFFKMNSLQ SNDTAIYYCARALTYYDYEFAYWGQGTLVTVSS |
| 24 | Anti-EGFR CDR-H1 | NYGVH |
| 25 | Anti-EGFR CDR-H2 | VIWSGGNTDYNTPFTS |
| 26 | Anti-EGFR CDR-H3 | ALTYYDYEFAY |
| 27 | Anti-EGFR VL | DILLTQSPVILSVSPGERVSFSCRASQSIGTNIHWYQQRTNGSPR LLIKYASESISGIPSRFSGSGSGTDFTLSINSVESEDIADYYCQQN NNWPTTFGAGTKLELKRTV |
| 28 | Anti-EGFR CDR-L1 | RASQSIGTNIH |
| 29 | Anti-EGFR CDR-L2 | YASESIS |
| 30 | Anti-EGFR CDR-L3 | QQNNNWPTT |
| 31 | Anti-HER3 VH | QVQLQESGGGLVKPGGSLRLSCAASGFTFSSYWMSWVRQAPG KGLEWVANINRDGSASYYVDSVKGRFTISRDDAKNSLYLQMN SLRAEDTAVYYCARDRGVGYFDLWGRGTLVTVSS |
| 32 | Anti-HER3 CDR-H1 | SYWMS |
| 33 | Anti-HER3 CDR-H2 | NINRDGSASYYVDSVKG |
| 34 | Anti-HER3 CDR-H3 | DRGVGYFDL |
| 35 | Anti-HER3 VL | QSALTQPASVSGSPGQSITISCTGTSSDVGGYNFVSWYQQHPGK APKLMIYDVSDRPSGVSDRFSGSKSGNTASLIISGLQADDEADY YCSSYGSSSTHVIFGGGTKVTVL |
| 36 | Anti-HER3 CDR-L1 | TGTSSDVGGYNFVS |
| 37 | Anti-HER3 CDR-L2 | DVSDRPS |
| 38 | Anti-HER3 CDR-L3 | SSYGSSSTHVI |

The sequences of the Fab fragment and the scFv fragment of the antibody described above were genetically synthesized, and then the DNA fragment was inserted into a pcDNA3.4-hIgG1 Fc expression plasmid and recombinantly constructed into a full-length antibody. The expression plasmid was amplified by *Escherichia coli,* extracted, and transiently transfected into Expi293 cells with a transfection reagent PEI for recombinant expression. After 5-7 days of culture, the cell culture medium was collected by centrifugation and added to a Protein A affinity chromatographic column for capture. The chromatographic column was then eluted with 1 M glycine (pH 3) to give a recombinant antibody, which was then dialyzed against a PBS buffer at pH 7.4.

EGFR, HER3, and EGFR×HER3 naked antibodies were exchanged into PBS/EDTA (PBS pH 6.0, 137 mM NaCl, and 5 mM EDTA) using ultrafiltration tubes (Amicon^{®} Ultra centrifugal ultrafiltration tube, 30 kDa MWCO) to prepare antibody solutions with a final concentration of 10 mg/mL. Each antibody solution (2 mL) was added to a 15 mL centrifuge tube, and a 1 M aqueous dipotassium phosphate solution (30 µL) was added. TCEP with a stock solution concentration of 10 mM was then added to the mixed solution in proportion (the molar ratio of the antibody to TCEP was 1:3, 1:6, or 1:9). The 15 mL centrifuge tube was placed in a shaker and shaken and mixed well (37 °C, 80 rpm) to reduce the interchain disulfide bond in the antibody.

The linker-payload A1 was prepared into a 10 mM stock solution with DMSO. The reduced antibody solution (2 mL) was taken, and a dimethyl sulfoxide solution (v/v, 5%) was added. Conjugation was performed by adding 10 mM linker-payload according to a molar ratio of 1:10. After conjugation in the shaker at 15 °C and 80 rpm for 30 min, a 100 mM aqueous NAC solution (the molar ratio of NAC to the antibody was 1:12.9) was added to terminate the reaction, so as to give an antibody-drug conjugate.

The reaction mixture was purified by ultrafiltration using a 30 kDa MWCO ultrafiltration centrifuge tube. PBS at pH 6.0 (137 mM NaCl) was used as a purification buffer and added to the reaction mixture. The mixed solution was concentrated to 1-2 mL, and the purification buffer was added again for circulation. The exchange factor was more than 1000. The final product solution was collected (about 2 mL) for later use. The concentration of the ADC was determined by calculation using UV full-wavelength scan analysis. The calculation formula was as follows:${C}_{ADC} = \left(ADCA280\times ε260drug-ADCA260\times ε280drug\right) / \left(ε280mab\times ε260drug-ε260mab\times ε280drug\right) ,$ where the A280 and A260 data for the ADC are the data for the product ADC, while ε280 mab, ε260 mab, ε280 drug, and ε260 drug are the data for the absorption peaks at a concentration of 1 mg/mL for the compound.

### Example B-2: Identification of Antibody-Drug Conjugates

The proportions of monomers, polymers, and fragments of the antibody-drug conjugates were analyzed by size exclusion chromatography SEC-HPLC. Method:

| SEC-HPLC method | | |
|---|---|---|
| | Instrument model | Agilent 1260 |
| | Chromatographic column | Waters |
| | | XBridge Protein BEH SEC |
| | | 7.8×300mm, 2.5µm |
| | Column temperature | 25°C |
| | Sample Temperature | 4°C |
| | Injection volume | 50 µg |
| | Mobile phase | 200 mM NaCl+ 100 mM PB, pH 6.8+10% IPA |
| | Flow rate | 1.0 mL/min |
| | Duration | 15 min |
| | Detection wavelength | 280 nm |
| | Gradient | Equivalence |

The DAR values of the antibody-drug conjugates were determined by hydrophobic interaction chromatography HIC as follows:

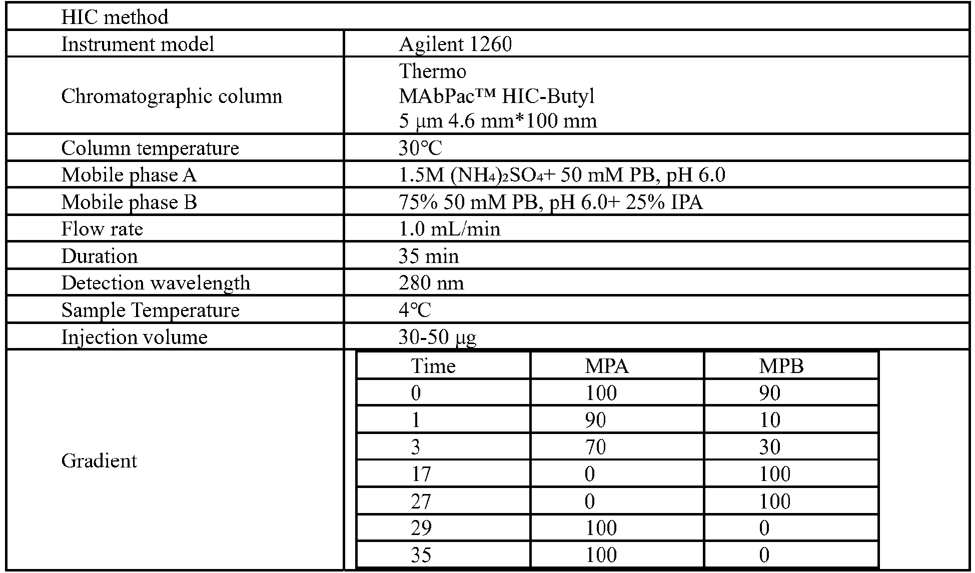

**Table 2-3. SEC and HIC chromatography results**

| ADC | antibody | Linker-Payload | Antibody: TCEP | HMW | SEC Monomer purity | LMW | DAR |
|---|---|---|---|---|---|---|---|
| Z38-L1-A-EGFR | EGFR | Z38-L1-A | 1:20 | 0.00% | 99.53% | 0.00% | 7.52 |
| Z47-L5-HER3 | HER3 | Z47-L5 | 1:09 | 2.71% | 97.29% | 0.00% | 7.67 |
| Z61-L7-HER3 | HER3 | Z61-L7 | 1:03 | 1.11% | 98.89% | 0.00% | 4.22 |
| Z52-L10-EGFR | EGFR | Z52-L10 | 1:03 | 1.97% | 98.03% | 0.00% | 4.03 |
| Z31-L1-HER3 | HER3 | Z31-L1 | 1:03 | 2.33% | 97.67% | 0.00% | 4.12 |
| Z35-L1-A-HER3 | HER3 | Z35-L1-A | 1:03 | 2.20% | 97.80% | 0.00% | 4.18 |
| Z31-L1-EGFR | EGFR | Z31-L1 | 1:5.1 | 2.15% | 97.85% | 0.00% | 3.76 |
| Z35-L1-A-EGFR | EGFR | Z35-L1-A | 1:5.3 | 1.85% | 98.15% | 0.00% | 3.88 |
| Z64-L7-A-HER3 | HER3 | Z64-L7-A | 1:03 | 1.75% | 98.25% | 0.00% | 4.1 |
| Z52-L12-HER3 | HER3 | Z52-L12 | 1:03 | 2.14% | 97.86% | 0.00% | 3.75 |
| Z52-L12-EGFR | EGFR | Z52-L12 | 1:05 | 1.03% | 98.97% | 0.00% | 4.42 |
| Z52-L13-HER3 | HER3 | Z52-L13 | 1:06 | 7.12% | 92.89% | 0.00% | 7.57 |
| Z52-L14-HER3 | HER3 | Z52-L14 | 1:06 | 6.40% | 93.60% | 0.00% | 7.25 |
| Z56-L23-HER3 | HER3 | Z56-L23 | 1:09 | 1.78% | 98.22% | 0.00% | 7.93 |
| Z34-L4-HER3 | HER3 | Z34-L4 | 1:06 | 0.57% | 99.06% | 0.38% | 7.24 |
| Z56-L23-EGFR | EGFR | Z56-L23 | 1:06 | 1.05% | 98.62% | 0.34% | 4.17 |
| Z34-L4-EGFR | EGFR | Z34-L4 | 1:09 | 2.49% | 97.45% | 0.06% | 3.76 |
| Z52-L15-HER3 | HER3 | Z52-L15 | 1:09 | 4.21% | 95.40% | 0.39% | 5.52 |
| Z52-L16-HER3 | HER3 | Z52-L16 | 1:09 | 7.62% | 92.38% | 0.00% | 7.25 |
| Z47-L6-HER3 | HER3 | Z47-L6 | 1:09 | 5.05% | 94.82% | 0.13% | 7.8 |
| Z64-1-L16-HER3 | HER3 | Z64-1-L16 | 1:09 | 9.79% | 90.21% | 0.00% | 7.38 |
| Z56-L23-EGFRxHER3 | EGFRxH ER3 | Z56-L23 | 1:09 | 2.30% | 97.70% | 0.00% | 5.9 |
| Z52-L16-EGFRxHER3 | EGFRxH ER3 | Z52-L16 | 1:09 | 5.52% | 94.48% | 0.00% | 7.46 |
| HER3-MC-GGFG-DXd | HER3 | MC-GGFG-DXd | 1:20 | 1.28% | 98.72% | 0.00% | 8 |
| Control ADC1 | | | | | | | |
| EGFR-MC-VC-PABC-MMAE | EGFR | MC-VC-PABC-MMAE | 1:2.45 | 2.08% | 97.92% | 0.00% | 4.02 |
| Control ADC2 | | | | | | | |

### Test Example B1: Cell p-ERK Assay

AsPC-1 cells were purchased from ATCC, Cat. No. CRL-1682; NCI-H441 cells were purchased from ATCC, Cat. No. HTB-174.

AsPC-1, a human pancreatic cancer cell strain endogenously containing a KRAS^{G12D} mutation, was cultured in a complete medium containing RPMI-1640 + 10% FBS + 1% PS. NCI-H441, a human non-small cell lung cancer cell strain endogenously containing a KRAS^{G12V} mutation, was cultured in a complete medium containing RPMI-1640 + 10% FBS + 1% PS.

The cells were digested with trypsin (containing EDTA), collected in their corresponding complete media, and counted. The density of the cell suspensions was adjusted with their corresponding complete media, and 10000 AsPC-1 cells or 20000 NCI-H441 cells per well were seeded into a 96-well cell culture plate, with the volume of the medium in each well being 80 µL. The cell culture plate was incubated in an incubator at 37 °C with 5% CO₂ overnight. A series of 3.16-fold gradient dilutions of a 1000× ADC stock solution were prepared with DMSO, and then diluted 200-fold with the complete medium described above to give 5× ADC stock solutions. On the day after cell seeding, 20 µL of the 5× ADC stock solution was added to each cell culture well. As a result, the final concentration of the ADC in the cell culture well became 1×, and the DMSO content in the well was 0.1%. In addition to the ADC-treated wells described above, cell-containing DMSO control wells were also set. A complete medium containing 0.5% DMSO was added at 20 µL/well. After the mixture was well mixed, the culture plate was incubated in the incubator at 37 °C with 5% CO₂ for another 3 h. The medium was then removed from the culture wells, and 50 µL of cell lysis buffer (from Advance ERK phospho-T202/Y204 kit) was added to each well. The mixture was well mixed and incubated for 30 min. Then, 16 µL of the mixture was transferred to a white-bottomed, opaque 96-well assay plate, and separately, 16 µL of cell lysis buffer was added to additional wells (serving as blank wells) in the assay plate. After the transfer was completed, 4 µL of p-ERK HTRF antibody mixture (from Advance ERK phospho-T202/Y204 kit) was added to each well of the assay plate, followed by a 4-hour incubation. The HTRF signal values (RLU) were then measured using a microplate reader, and the inhibition rate of the ADC against p-ERK levels was calculated as follows: IR (%) = (RLU_{DMSO} - RLU_{ADC}) / (RLU_{DMSO} - RLU_{blank}) × 100%, where RLU_{DMSO} is the average HTRF signal value from the DMSO control well samples, RLU_{blank} is the average HTRF signal value from the blank well samples, and RLU_{ADC} is the HTRF signal value from the ADC-treated well samples. Using an XLfit software, the IR (%) values were plotted against the logarithm of the ADC concentrations, and the dose-response curves were fitted using the four-parameter method to calculate the results. The results show that the ADCs of the present disclosure exhibited relatively high inhibitory activity against p-ERK in the cancer cells described above.

### Test Example B2: NCI-H441 and AsPC-1 Cell Proliferation Inhibition Assay

Lung adenocarcinoma cells NCI-H441 (purchased from ATCC) and pancreatic cancer cells AsPC-1 (purchased from ATCC) were cultured in an RPMI1640 + 10% FBS + 1% PS (brand: Gibco) complete medium. When the cells grew to about 90% confluence, the cells were digested with trypsin, counted, resuspended in an RPMI1640 + 2% FBS + 1% PS medium, and seeded in a 384-well plate (Corning 3830) at a density of 800 cells/45 µL/well. The cells were cultured overnight to adhere to the wall. The antibodies and the ADCs were separately diluted with an RPMI1640 + 2% FBS + 1% PS medium in a 3.162-fold gradient from an initial concentration of 1 µM, with a total of 9 concentration gradients, and 10× stock solutions were prepared. After 5 µL of each 10× stock solution was added to each well of the 384-well cell plate described above, the cell plate was incubated in an incubator at 37 °C with 5% CO₂ for 120 h. 30 µL of 3D-Cell Titer Glo (Promega G9683) was added to each well, and the plate was shaken in the dark for 30 min to fully lyse the cells. After the plate was left to stand in the dark at room temperature for 30 min, 40 µL of the liquid was transferred into a white-bottomed, opaque 384-well plate (Corning 3570). The chemiluminescence signals, i.e., relative luminescence units (RLU), of each well were measured using a microplate reader. Using an XLFit software, curve fitting was performed, and the IC₅₀ values were calculated.

The results for the inhibition of NCI-H441 and AsPC-1 cell proliferation by ADCs are shown in Tables 2-4-1 and 2-4-2.

**Table 2-4-1. Inhibition of NCI-H441 and AsPC-1 cell proliferation by ADCs**

| ADC | NCI-H441 IC₅₀: nM | NCI-H441 maximum inhibition rate | AsPC-1 IC₅₀: nM | AsPC-1 maximum inhibition rate |
|---|---|---|---|---|
| Z47-L5-HER3 | 2.47 | 84.06% | 31.08 | 62.98% |
| Z31-L1-HER3 | 33.15 | 62.36% | >100 | 32.59% |
| Z64-L7-A-HER3 | 7.23 | 70.51% | 0.88 | 74.12% |
| Z52-L12-HER3 | 1.11 | 74.82% | 0.25 | 98.72% |
| Z52-L12-EGFR | 3.49 | 79.33% | 0.08 | 90.65% |
| Z52-L13-HER3 | 0.76 | 79.65% | 0.11 | 88.75% |
| Z52-L14-HER3 | 1.54 | 70.68% | 0.17 | 80.54% |
| Z56-L23-HER3 | 1.60 | 83.42% | 0.20 | 93.13% |
| Z34-L4-HER3 | 16.09 | 62.67% | >100 | 47.95% |
| Z56-L23-EGFR | 0.86 | 79.67% | 0.03 | 84.44% |
| Z34-L4-EGFR | 3.55 | 70.88% | 0.11 | 69.91% |
| Z52-L16-HER3 | 1.44 | 82.88% | 0.30 | 94.16% |
| Z47-L6-HER3 | 4.53 | 69.84% | 76.35 | 55.96% |
| Z64-1-L16-HER3 | 2.13 | 79.78% | 1.18 | 140.66% |
| HER3-MC-GGFG-DXd | 2.11 | 93.13% | >100 | -2.67% |
| EGFR-MC-VC-PABC-MMAE | 0.03 | 73.28% | >100 | 42.63% |
| EGFR naked antibody | >100 | 14.56% | >100 | 27.84% |
| HER3 naked antibody | >100 | -1.54% | >100 | 3.40% |

**Table 2-4-2. Inhibition of AsPC-1 cell proliferation by ADCs**

| ADC | AsPC-1 IC₅₀: nM | AsPC-1 maximum inhibition rate |
|---|---|---|
| Z31-L1-EGFR | 2.12 | 51.76% |
| Z35-L1-A-EGFR | 0.83 | 53.72% |
| HER3-MC-GGFG-DXd | >100 | -2.67% |
| EGFR-MC-VC-PABC-MMAE | >100 | 42.63% |
| EGFR naked antibody | >100 | 27.84% |
| HER3 naked antibody | >100 | 3.40% |

### Test Example B3: In Vivo Efficacy of Antibody-Drug Conjugates in H441 and AsPC-1 Tumor-Bearing Mouse Models

NCI-H441 cells were cultured in an RPMI 1640 medium containing 10% FBS and 1% penicillin-streptomycin in an incubator at 37 °C with 5% CO₂. The cells were digested with 0.25% trypsin-EDTA for passaging as per conventional practice. When the cells were in the logarithmic growth phase and the confluence reached 80%-90%, the cells were collected and counted. The cells were collected, and 50% cell suspension and 50% Matrigel were well mixed. The final concentration of the cells was adjusted to 5.0 × 10⁷ cells/mL. Mice were grafted subcutaneously at the right dorsal region in a volume of 0.1 mL/5 × 10⁶ cells/mouse. When the tumors grew to an average size of about 150-200 mm³ (about D15 after grafting), tumor-bearing mice with a suitable tumor volume were selected, randomly grouped according to the tumor volume and the body weight of the animal, with 4 mice in each group, and treated by intravenous injection according to the doses in Table 5. The administration volume in mice was 10 mL/kg. The mice were weighed on an electronic balance twice weekly and the tumor volume was measured with a vernier caliper twice weekly.

AsPC-1 cells were cultured in an RPMI 1640 medium containing 10% FBS and 1% penicillin-streptomycin in an incubator at 37 °C with 5% CO₂. The cells were digested with 0.25% trypsin-EDTA for passaging as per conventional practice. When the cells were in the logarithmic growth phase and the confluence reached 80%-90%, the cells were collected and counted. The cells were collected, and Matrigel was added in a 1:1 ratio to adjust the final concentration of the cells to 5.0 × 10⁷ cells/mL. Mice were grafted subcutaneously at the right dorsal region in a volume of 0.1 mL/5 × 10⁶ cells/mouse. When the tumors grew to an average size of about 250 mm³ (D7 after grafting), tumor-bearing mice with a suitable tumor volume were selected, randomly grouped according to the tumor volume and the body weight of the animal, with 4 mice in each group, and treated by intravenous injection according to the doses in Table 5. The administration volume in mice was 10 mL/kg. The mice were weighed on an electronic balance twice weekly and the tumor volume was measured with a vernier caliper twice weekly.

The tumor growth inhibition rate TGI was calculated using the following formula: $TGI = \left[1-\left(avTi-0\right) / \left(avCi-0\right)\right]$ where avTi-0 denotes the average tumor volume of the treatment group on a specific day minus the average tumor volume of the treatment group at the baseline; avCi-0 denotes the average tumor volume of the control group on a specific day (Day14) minus the average tumor volume of the control group at the baseline.

The tumor growth inhibition rates and relative changes in body weight 14 days after administration of ADCs in H441 and AsPC-1 tumor-bearing mice are shown in Tables 2-5-1 and 2-5-2.

**Table 2-5-1. TGI and changes in mouse body weight 14 days after administration of ADCs in H441 and AsPC-1 tumor-bearing mice**

| ADC | Dose for H441 | NCI-H441 TGI | Changes in body weight of H441 mice | Dose for AsPC-1 | AsPC-1 TGI | Changes in body weight of AsPC-1 mice |
|---|---|---|---|---|---|---|
| Z52-L10-EGFR | 10 mg/kg | 83.38% | 4.86% | 10 mg/kg | 72.26% | 4.79% |
| | qw ×2 | | | qw ×2 | | |
| Z35-L1-A-HER3 | 10 mg/kg | 90.90% | 2.09% | 10 mg/kg | 61.80% | 8.95% |
| | qw ×2 | | | qw ×2 | | |
| Z35-L1-A-EGFR | 10 mg/kg | 73.90% | 1.70% | 10 mg/kg | 65.83% | 4.97% |
| | qw ×2 | | | qw ×2 | | |
| Z64-L7-A-HER3 | 10 mg/kg | 93.60% | 4.04% | 10 mg/kg | 83.40% | 1.74% |
| | qw ×2 | | | qw ×2 | | |
| Z52-L12-HER3 | 10 mg/kg | 123.20% | -2.07% | 10 mg/kg | 82.74% | 6.93% |
| | qw ×2 | | | qw ×2 | | |
| Z52-L12-EGFR | 10 mg/kg | 125.32% | 0.60% | 10 mg/kg | 71.83% | 5.83% |
| | qw ×2 | | | qw ×2 | | |
| Z52-L13-HER3 | 10 mg/kg | 105.99% | 4.21% | 10 mg/kg | 109.72% | -1.48% |
| | qw ×2 | | | qw ×2 | | |
| Z52-L14-HER3 | 10 mg/kg | 112.28% | 1.84% | 10 mg/kg | 123.54% | -5.93% |
| | qw ×2 | | | qw ×2 | | |
| Z56-L23-HER3 | 10 mg/kg | 99.15% | 2.37% | 10 mg/kg | 94.19% | -1.64% |
| | qw ×2 | | | qw ×2 | | |
| Z56-L23-EGFR | 10 mg/kg | 113.57% | 8.38% | 10 mg/kg | 113.19% | -0.45% |
| | qw ×2 | | | qw ×2 | | |
| Z34-L4-EGFR | 10 mg/kg | 106.49% | 8.13% | 10 mg/kg | 81.41% | 0.25% |
| | qw ×2 | | | qw ×2 | | |
| Z52-L15-HER3 | 10 mg/kg | 130.79% | -0.50% | 10 mg/kg | 77.53% | 2.81% |
| | qw ×2 | | | qw ×2 | | |
| Z52-L16-HER3 | 10 mg/kg | 132.93% | 0.00% | 10 mg/kg | 122.31% | 0.65% |
| | qw ×2 | | | qw ×2 | | |
| Z47-L6-HER3 | 10 mg/kg | 123.89% | 0.49% | 10 mg/kg | 50.92% | 4.00% |
| | qw ×2 | | | qw ×2 | | |
| Z64-1-L16-HER3 | 10 mg/kg | 130.27% | 2.76% | 10 mg/kg | 115.30% | 2.70% |
| | qw ×2 | | | qw ×2 | | |
| Z56-L23-EGFRxHER3 | 5 mg/kg | 124.66% | 1.24% | 5 mg/kg | 105.26 | 2.18% |
| | qw ×2 | | | qw ×2 | | |
| Z52-L16-EGFRxHER3 | 5 mg/kg | 122.58% | -1.39% | 5 mg/kg | 135.12% | 1.92% |
| | qw ×2 | | | qw ×2 | | |
| EGFR-MC-VC-PABC-MMAE | 10 mg/kg | 86.57% | 6.76% | 10 mg/kg | 82.29% | 7.60% |
| | qw ×2 | | | qw ×2 | | |
| HER3-MC-GGFG-DXd | 10 mg/kg | 115.65% | 5.21% | 10 mg/kg | 52.37% | 2.58% |
| | qw ×2 | | | qw ×2 | | |
| EGFR naked antibody | 10 mg/kg | 52.87% | 7.74% | 10 mg/kg | 5.67% | 3.87% |
| | qw ×2 | | | qw ×2 | | |
| HER3 naked antibody | 10 mg/kg | 66.39% | 4.82% | 10 mg/kg | 24.11% | 4.22% |
| | qw ×2 | | | qw ×2 | | |

**Table 2-5-2. TGI and mouse body weight changes 14 days after administration of ADCs in H441 tumor-bearing mice**

| ADC | Dose for H441 | NCI-H441 TGI | Changes in body weight of H441 mice |
|---|---|---|---|
| Z38-L1-A-EGFR | 10 mg/kg, qw ×2 | 82.41% | 4.10% |
| Z47-L5-HER3 | 10 mg/kg, qw ×2 | 80.71% | 2.87% |
| Z61-L7-HER3 | 10 mg/kg, qw ×2 | 82.10% | 2.26% |
| Z31-L1-HER3 | 10 mg/kg, qw ×2 | 96.60% | 3.12% |
| Z31-L1-EGFR | 10 mg/kg, qw ×2 | 83.80% | -0.32% |
| Z34-L4-HER3 | 10 mg/kg, qw ×2 | 82.56% | 3.80% |
| EGFR-MC-VC-PABC-MMAE | 10 mg/kg, qw ×2 | 86.57% | 6.76% |
| HER3-MC-GGFG-DXd | 10 mg/kg, qw ×2 | 115.65% | 5.21% |
| EGFR naked antibody | 10 mg/kg, qw ×2 | 52.87% | 7.74% |
| HER3 naked antibody | 10 mg/kg, qw ×2 | 66.39% | 4.82% |

Although specific embodiments of the present disclosure have been described in detail, various modifications and substitutions can be made to the details of the technical solutions of the present disclosure by those skilled in the art according to all the teachings that have been disclosed, and these changes are all encompassed within the claimed scope of the present disclosure. The entire scope of the present disclosure is given by the appended claims and any equivalents thereof.

## Claims

1. An antibody-drug conjugate represented by formula (I): 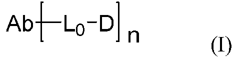
wherein Ab is an antibody or a fragment thereof that binds to a target;
n is 1-20 and may be an integer or non-integer;
D is an RAS protein-targeting inhibitor fragment;
L₀ is a linker linking Ab and D.

2. The antibody-drug conjugate according to claim 1, wherein 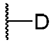 is a structure represented by formula (VI-1), formula (VI-2), formula (VI-3), formula (VI-4), formula (VI-5), or formula (VI-6), or a stable deuterated derivative thereof, or a stereoisomer thereof, 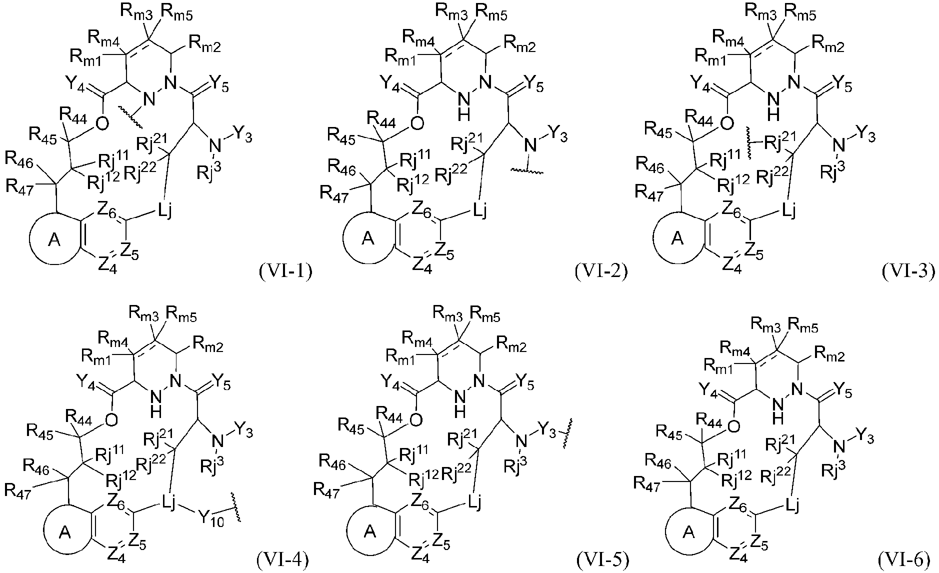 wherein:
in formula (VI-1), formula (VI-2), formula (VI-3), formula (VI-4), and formula (VI-5), the structural unit 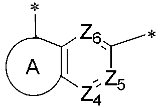 is 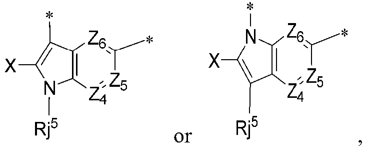 and X is 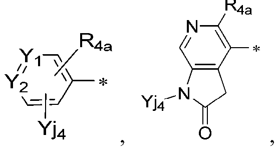 or 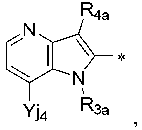
in formula (VI-6), the structural unit 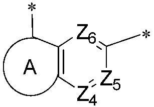 is 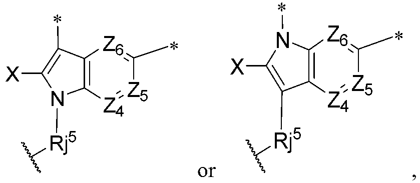 and
X is 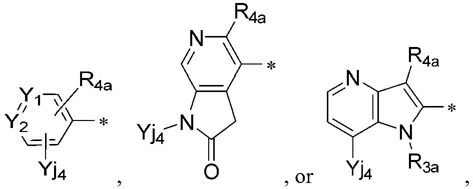 or
the structural unit 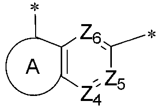 is 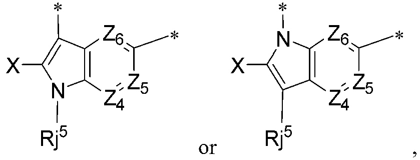 and X is 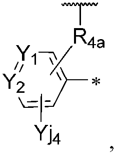 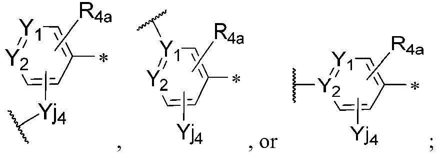
the 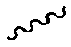 end is attached to L₀, and the * end is attached to another fragment in D;
when L₀ is attached to D, the hydrogen atom on D is replaced by L₀, or the N atom on D is quaternized;
Y₁ and Y₂ are each independently N, CH, CR₄ₐ, CR_{4b}, or CYj₄, wherein N may be oxidized (N⁺-O⁻);
Z₄, Z₅, and Z₆ are each independently N, CR₁₃, CR₁₄, CR₄₈, CR₄₉, or CR₅₀;
R₁₃ and R₁₄ are each independently hydrogen, halogen, C₁₋₆ alkyl optionally substituted with one or more S1, or -P(=O)-(C₁₋₆ alkyl)₂, or
R₁₃ and R₁₄, together with the carbon atom to which they are attached, form C₃₋₆ cycloalkyl optionally substituted with one or more S1 or 3- to 8-membered heterocyclyl optionally substituted with one or more S1;
R₄₈, R₄₉, and R₅₀ are each independently hydrogen, deuterium, halogen, C₁₋₄ alkyl, C₁₋₄ haloalkyl, or C₁₋₄ alkoxy;
R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, 8- to 10-membered bicyclic heteroaryl, or - P(=O)-(C₁₋₆ alkyl)₂, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more S1;
R₄ₐ is 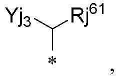 C₁₋₆ alkyl, C₃₋₆ cycloalkyl, or 3- to 8-membered heterocyclyl, wherein the C₁₋₆ alkyl, C₃₋₆ cycloalkyl, or 3- to 8-membered heterocyclyl is optionally substituted with one or more S1;
Yj₃ is NH-Rj⁶, O-Rj⁶, NRj⁶²Rj⁶, -C₁₋₄ alkylene-NH-Rj⁶, -C₁₋₄ alkylene-NRj⁶²Rj⁶, or -C₁₋₄ alkylene-O-Rj⁶;
Rj⁶ is hydrogen or C₁₋₆ alkyl;
Rj⁶² is hydrogen or C₁₋₆ alkyl;
Rj⁶¹ is C₁₋₆ alkyl;
R_{4b} is deuterium, halogen, C₁₋₆ alkyl optionally substituted with one or more S1, or C₁₋₆ heteroalkyl optionally substituted with one or more S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₃)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, - C₂₋₄ alkenylene-NR₀₁R₀₂, -C₂₋₄ alkenylene-C(R₀₅)R₀₃R₀₄, -C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), -S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 3- to 8-membered monocyclic heterocyclyl, 7- to 12-membered bicyclic heterocyclyl, C₆₋₁₀ aryl, 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, C₆₋₁₀ aryl, 5- or 6-membered monocyclic heteroaryl, or 8- to 10-membered bicyclic heteroaryl is optionally substituted with one or more S1;
R₀₁ and R₀₂ are each independently hydrogen, C₁₋₆ alkyl, C₁₋₆ alkoxy, 3- to 6-membered cycloalkyl, 3- to 8-membered monocyclic heteroalkyl, C₂₋₆ alkenyl, C₂₋₆ alkynyl, or (C₁₋₆ alkyl)₃-Si-, wherein the C₁₋₆ alkyl, C₁₋₆ alkoxy, 3- to 6-membered cycloalkyl, 3- to 8-membered monocyclic heteroalkyl, C₂₋₆ alkenyl, or C₂₋₆ alkynyl is optionally substituted with one or more S1, or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with one or more S1, or
R₀₁ and R₀₂ together form 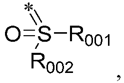 wherein R₀₀₁ and R₀₀₂, together with the sulfur atom to which they are attached, form 3- to 8-membered monocyclic heterocyclyl optionally substituted with one or more S1;
R₀₃ and R₀₄ are each independently hydrogen, C₁₋₆ alkyl, C₁₋₆ alkoxy, C₂₋₆ alkenyl, or C₂₋₆ alkynyl, wherein the C₁₋₆ alkyl, C₁₋₆ alkoxy, C₂₋₆ alkenyl, or C₂₋₆ alkynyl is optionally substituted with one or more S1, wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6, or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with one or more S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₄ is O or S;
Y₅ is O or S;
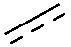 is a single bond or a double bond;
Rₘ₁ and Rₘ₃ are absent or are hydrogen, Rₘ₂ is hydrogen, and Rₘ₄ and Rₘ₅ are each independently hydrogen, halogen, or C₁₋₆ alkyl; or
Rₘ₁ is attached to Rₘ₂ to form -CRₘ₇Rₘ₆- or -CRₘ₇Rₘ₆-CRₘ₉Rₘ₈-; or
Rₘ₁ is attached to Rₘ₃ to form -CRₘ₇Rₘ₆- or -CRₘ₇Rₘ₆-CRₘ₉Rₘ₈-; or
Rₘ₁ is attached to Rₘ₄ to form -CRₘ₇Rₘ₆- or -CRₘ₇Rₘ₆-CRₘ₉Rₘ₈-;
Rₘ₆, Rₘ₇, Rₘ₈, and Rₘ₉ are each independently hydrogen, halogen, or C₁₋₆ alkyl;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with one or more S1;
Y₁₀ is a bond, O, NH, or N(C₁₋₄ alkyl);
R₀₀ is hydrogen or C₁₋₆ alkyl;
Rj²¹ is hydrogen, deuterium, halogen, hydroxy, amino, C₁₋₋₆ alkyl, C₁₋₆ heteroalkyl optionally substituted with one or more S1, -NRj^{f}Rj^{g}, or -O-C₁₋₄ alkylene-NRj^{f}Rj^{g};
Rj²² is hydrogen, deuterium, halogen, C₁₋₆ alkyl, C₁₋₆ heteroalkyl optionally substituted with one or more S1, - NRj^{f}Rj^{g}, or -O-C₁₋₄ alkylene-NRj^{f}Rj^{g};
Rj^{f} is hydrogen or C₁₋₆ alkyl;
Rj^{g} is hydrogen or C₁₋₆ alkyl;
Rj¹¹ and Rj¹² are each independently hydrogen, halogen, or C₁₋₆ alkyl optionally substituted with one or more S1; or
Rj¹¹ and Rj¹², together with the carbon atom to which they are attached, form C₃₋₆ cycloalkyl optionally substituted with one or more S1 or 3- to 8-membered heterocyclyl optionally substituted with one or more S1;
R₄₄, R₄₅, R₄₆, and R₄₇ are each independently hydrogen, deuterium, halogen, C₁₋₄ alkyl, C₁₋₄ haloalkyl, or C₁₋₄ alkoxy;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁;
Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with one or more S1; in the nitrogen-containing 3- to 8-membered heterocyclyl, one ring atom is a nitrogen atom, and optionally one or more ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, or heteroaryl, wherein the C₁₋₆ alkyl, C₁₋₆ heteroalkyl, C₃₋₆ cycloalkyl, 3- to 8-membered heterocyclyl, C₆₋₁₀ aryl, or heteroaryl is optionally substituted with one or more S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with one or more S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
or R₀₇ and R₀₈, together with the atom(s) to which they are attached, form 3- to 8-membered heterocyclyl optionally substituted with one or more S1;
in the groups described above, each S1 is independently selected from the group consisting of: deuterium, oxo (=O), thio (=S), =CR^{e}R^{f}, =NR^{e}, SF₅, halogen, cyano, hydroxy, carboxyl, nitro, C₁₋₆ alkyl, C₂₋₆ alkenyl, C₂₋₆ alkynyl, C₁₋₆ alkoxy, C₁₋₆ heteroalkyl, C₃₋₂₀ cycloalkyl, 3- to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C=C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C_{1A} alkylene-C₃₋₂₀ cycloalkyl, -C≡C-C₁_₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, -C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ heteroalkylene-C₁₋₄ heteroalkylene-C₁₋₆ heteroalkyl, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, - C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, - C₁₋₄ alkylene-O-5- or 6-membered monocyclic heteroaryl, -C₁₋₄ alkylene-8- to 10-membered bicyclic heteroaryl, -C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, - N=S(=O)(R^{g1})(R^{h1}), and -P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with one or more groups selected from halogen, deuterium, cyano, hydroxy, and amino; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with one or more groups selected from group S2; the nitrogen atom(s) in the 5- or 6-membered monocyclic heteroaryl may optionally be oxidized (N⁺-O⁻);
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(O)H, -C(O)-C₁₋₄ alkylene-OH, -C(=O)-C₃₋₆ monocyclic cycloalkyl, -C(=O)-3- to 6-membered monocyclic heterocyclyl, or -C(=O)-5- to 8-membered heterocyclyl, wherein the C₃₋₆ monocyclic cycloalkyl, 3-to 6-membered monocyclic heterocyclyl, 5- to 8-membered heterocyclyl, phenyl, 5- or 6-membered monocyclic heteroaryl, or 8- to 10-membered bicyclic heteroaryl is optionally substituted with one or more groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, -C(O)C₁₋₆ alkyl, -C(O)OC₁₋₆ alkyl, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3-to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, R^{g1} and R^{h1} are each independently H, C₁₋₆ alkyl, or deuterated C₁₋₆ alkyl; or R^{g1} and R^{h1}, together with the sulfur atom to which they are attached, form substituted or unsubstituted 3- to 8-membered monocyclic heterocyclyl;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, cyano, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -(C=O)OC₁₋₆alkyl, -C₁₋₄ alkylene-P(=O)-(C₁₋₆ alkyl)₂, - P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, C₃₋₆ cycloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more atoms of the C₁₋₆ heteroalkyl are heteroatoms selected from nitrogen, oxygen, sulfur, and phosphorus; one or more ring atoms of the 7- to 12-membered bicyclic heterocyclyl are heteroatoms selected from nitrogen, oxygen, sulfur, and phosphorus; one or more ring atoms of the 3- to 20-membered heterocyclyl are heteroatoms selected from nitrogen, oxygen, sulfur, and phosphorus; one or more ring atoms of the 5- to 20-membered heteroaryl are heteroatoms selected from nitrogen, oxygen, sulfur, and phosphorus; one or more ring atoms of the 5- or 6-membered monocyclic heteroaryl are heteroatoms selected from nitrogen, oxygen, sulfur, and phosphorus; one or more ring atoms of the 8- to 10-membered bicyclic heteroaryl are heteroatoms selected from nitrogen, oxygen, sulfur, and phosphorus; one or more ring atoms of the 3- to 6-membered monocyclic heterocyclyl are heteroatoms selected from nitrogen, oxygen, sulfur, and phosphorus; one or more ring atoms of the 5- to 15-membered tricyclic heterocyclyl are heteroatoms selected from nitrogen, oxygen, sulfur, and phosphorus; one or more ring atoms of the 6- to 12-membered heteroaryl-fused cycloalkyl are heteroatoms selected from nitrogen, oxygen, sulfur, and phosphorus; one or more ring atoms of the 6- to 12-membered heteroaryl-fused heterocyclyl are heteroatoms selected from nitrogen, oxygen, sulfur, and phosphorus; wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

3. The antibody-drug conjugate according to claim 1 or 2, wherein 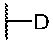 is a structure represented by formula (II-5), or a stable deuterated derivative thereof, or a stereoisomer thereof: 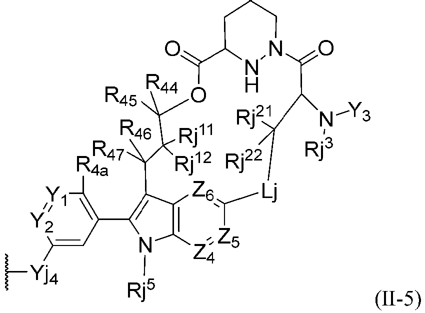 wherein in the formula,
Y₁ is N or CH, wherein N may be oxidized (N⁺-O⁻);
Y₂ is N or CH, wherein N may be oxidized (N⁺-O⁻);
R₄₄, R₄₅, R₄₆, and R₄₇ are each independently H, deuterium, halogen, C₁₋₄ alkyl, C₁₋₄ haloalkyl, or C₁₋₄ alkoxy;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
Lⱼ is -N(R₀₀)C(=O)-, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₀₀ is hydrogen or C₁₋₆ alkyl;
Rj²¹ is hydrogen, deuterium, halogen, hydroxy, C₁₋₆ alkyl, or C₁₋₆ heteroalkyl optionally substituted with 1, 2, 3, 4, 5, or 6 S1;
Rj²² is hydrogen, deuterium, halogen, C₁₋₆ alkyl, or C₁₋₆ heteroalkyl optionally substituted with 1, 2, 3, 4, 5, or 6 S1;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₃₋₆ cycloalkyl, or 3-to 8-membered heterocyclyl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5- or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁; Rj³ is hydrogen, C₁₋₆ alkyl, or deuterated C₁₋₆ alkyl;
or Y₃ and Rj³, together with the nitrogen atom to which they are attached, form nitrogen-containing 3- to 8-membered heterocyclyl optionally substituted with 1, 2, 3, 4, 5, or 6 S1; in the nitrogen-containing 3- to 8-membered heterocyclyl, one ring atom is a nitrogen atom, and optionally one or more ring atoms are heteroatoms selected from nitrogen, oxygen, sulfur, and phosphorus;
Rj⁴ is C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with 1, 2, 3, 4, 5, or 6 S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C=C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁. 4 alkylene-R^{c1}, -NR^{d1}-C(=O)-NR^{a1}R^{b1}, -NR^{d1}-S(=O)₂-R^{c1}, -S(=O)₂-NR^{a1}R^{b1}, -NR^{d1}-S(=O)₂-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3-to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more atoms of the C₁₋₆ heteroalkyl are heteroatoms selected from nitrogen, oxygen, sulfur, and phosphorus; one or more ring atoms of the 7- to 12-membered bicyclic heterocyclyl are heteroatoms selected from nitrogen, oxygen, sulfur, and phosphorus; one or more ring atoms of the 3- to 20-membered heterocyclyl are heteroatoms selected from nitrogen, oxygen, sulfur, and phosphorus; one or more ring atoms of the 5- to 20-membered heteroaryl are heteroatoms selected from nitrogen, oxygen, sulfur, and phosphorus; one or more ring atoms of the 5- or 6-membered monocyclic heteroaryl are heteroatoms selected from nitrogen, oxygen, sulfur, and phosphorus; one or more ring atoms of the 8- to 10-membered bicyclic heteroaryl are heteroatoms selected from nitrogen, oxygen, sulfur, and phosphorus; one or more ring atoms of the 3- to 6-membered monocyclic heterocyclyl are heteroatoms selected from nitrogen, oxygen, sulfur, and phosphorus; one or more ring atoms of the 5- to 15-membered tricyclic heterocyclyl are heteroatoms selected from nitrogen, oxygen, sulfur, and phosphorus; one or more ring atoms of the 6- to 12-membered heteroaryl-fused cycloalkyl are heteroatoms selected from nitrogen, oxygen, sulfur, and phosphorus; one or more ring atoms of the 6- to 12-membered heteroaryl-fused heterocyclyl are heteroatoms selected from nitrogen, oxygen, sulfur, and phosphorus; wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

4. The antibody-drug conjugate according to claim 1 or 2, wherein 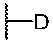 is a structure represented by formula (II-6a), or a stable deuterated derivative thereof, or a stereoisomer thereof: 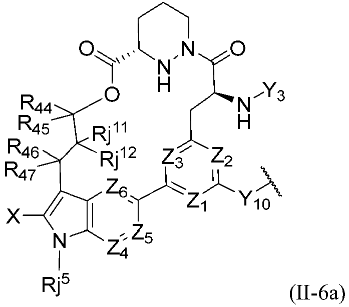 wherein in the formula,
Z₁ is C(H), C(deuterium), or N;
Z₂ is C(H), C(deuterium), or N;
Z₃ is C(H), C(deuterium), or N;
Z₄, Z₅, and Z₆ are each independently N, CR₄₈, CR₄₉, or CR₅₀; R₄₈, R₄₉, and R₅₀ are each independently H, deuterium, halogen, C₁₋₄ alkyl, C₁₋₄ haloalkyl, or C₁₋₄ alkoxy;
Y₁₀ is a bond, O, NH, or N(C₁₋₄ alkyl);
X is 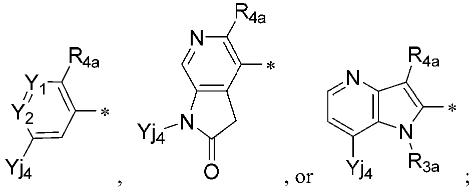
the 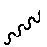 end is attached to L₀, and the * end is attached to another fragment in D;
Y₁ is N or CH, wherein N may be oxidized (N⁺-O⁻);
Y₂ is N or CH, wherein N may be oxidized (N⁺-O⁻);
Rj¹¹ is hydrogen, halogen, or C₁₋₆ alkyl optionally substituted with 1, 2, 3, 4, 5, or 6 S1; Rj¹² is hydrogen, halogen, or C₁₋₆ alkyl optionally substituted with 1, 2, 3, 4, 5, or 6 S1; or
Rj¹¹ and Rj¹², together with the carbon atom to which they are attached, form C₃₋₆ cycloalkyl optionally substituted with 1, 2, 3, 4, 5, or 6 S1 or 3- to 8-membered heterocyclyl optionally substituted with 1, 2, 3, 4, 5, or 6 S1;
R₄₄, R₄₅, R₄₆, and R₄₇ are each independently H, deuterium, halogen, C₁₋₄ alkyl, C₁₋₄ haloalkyl, or C₁₋₄ alkoxy;
R₃ₐ is C₁₋₆ alkyl or deuterated C₁₋₆ alkyl;
Rj⁵ is hydrogen, C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₂₋₆ alkenyl, C₂₋₆ alkynyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₄ₐ is C₁₋₆ alkyl, C₃₋₆ cycloalkyl, or 3- to 8-membered heterocyclyl, wherein the C₁₋₆ alkyl, C₃₋₆ cycloalkyl, or 3-to 8-membered heterocyclyl is optionally substituted with 1, 2, 3, 4, 5, or 6 S1;
Yj₄ is hydrogen, -NR₀₁R₀₂, -C(R₀₅)R₀₃R₀₄, -C₁₋₄ alkylene-NR₀₁R₀₂, -C₁₋₄ alkylene-C(R₀₅)R₀₃R₀₄, -C₁₋₄ alkylene-C(=O)-NR₀₁R₀₂, -C₁₋₄ alkylene-C(=O)-C(R₀₅)R₀₃R₀₄, -C₁₋₄ alkylene-C(=O)-C₁₋₄ alkylene-NR₀₁R₀₂, -C₁₋₄ alkylene-C(=O)-C₁₋₄ alkylene-C(R₀₅)R₀₃R₀₄, -C₁₋₄ alkylene-N(R₀₆)C(=O)-NR₀₁R₀₂, -C₁₋₄ alkylene-N(R₀₆)C(=O)-C(R₀₅)R₀₃R₀₄, -C₁₋₄ alkylene-N(R₀₆)C(=O)-C₁₋₄ alkylene-NR₀₁R₀₂, -C₁₋₄ alkylene-N(R₀₆)C(=O)-C₁₋₄ alkylene-C(R₀₅)R₀₃R₀₄, -O-C₁₋₄ alkylene-NR₀₁R₀₂, -O-C₁₋₄ alkylene-C(R₀₅)R₀₃R₀₄, -O-C₁₋₄ alkylene-N(R₀₆)C(=O)-R₀₃, -O-C(R₀₅)R₀₃R₀₄, -C₁₋₄ alkylene-NR₀₁R₀₂, -C₁₋₄ alkylene-C(R₀₅)R₀₃R₀₄, -N(R₀₆)C(=O)-NR₀₁R₀₂, -N(R₀₆)C(=O)-C(R₀₅)R₀₃R₀₄, -C₂₋₄ alkynylene-NR₀₁R₀₂, -C₂₋₄ alkynylene-C(=O)-NR₀₁R₀₂, -C₂₋₄ alkynylene-C(R₀₅)R₀₃R₀₄, -C₁₋₄ alkylene-C₂₋₄ alkynylene-NR₀₁R₀₂, -C₁₋₄ alkylene-C₂₋₄ alkynylene-C(R₀₅)R₀₃R₀₄, -C₂₋₄ alkynylene-C₁₋₄ alkylene-NR₀₁R₀₂, -C₂₋₄ alkynylene-C₁₋₄ alkylene-C(R₀₅)R₀₃R₀₄, -C₁₋₄ alkylene-C₂₋₄ alkynylene-C₁₋₄ alkylene-NR₀₁R₀₂, -CH=CR₀₁R₀₂, -C₂₋₄ alkenylene-NR₀₁R₀₂, -C₂₋₄ alkenylene-C(R₀₅)R₀₃R₀₄, - C₁₋₄ alkylene-C₂₋₄ alkenylene-NR₀₁R₀₂, -C₁₋₄ alkylene-C₂₋₄ alkenylene-C(R₀₅)R₀₃R₀₄, -C₂₋₄ alkenylene-C₁₋₄ alkylene-NR₀₁R₀₂, -C₂₋₄ alkenylene-C₁₋₄ alkylene-C(R₀₅)R₀₃R₀₄, -C₁₋₄ alkylene-C₂₋₄ alkenylene-C₁₋₄ alkylene-NR₀₁R₀₂, -C=CR₀₃R₀₄, -O-C₁₋₄ alkylene-3- to 8-membered monocyclic heterocyclyl, -O-C₁₋₄ alkylene-5- to 15-membered tricyclic heterocyclyl, -O-C₁₋₄ alkylene-7- to 12-membered bicyclic heterocyclyl, -O-3- to 8-membered monocyclic heterocyclyl, -O-5- to 15-membered tricyclic heterocyclyl, -O-7- to 12-membered bicyclic heterocyclyl, -N=S(=O)(R₀₆)(-C₁₋₄ alkylene-NR₀₁R₀₂), -N(R₀₆)-S(=O)(-C₁₋₄ alkylene-NR₀₁R₀₂), - S(=O)(R₀₆)(=N-R₀₆), -S(=O)(-C₁₋₄ alkylene-NR₀₁R₀₂)(=N-R₀₆), -S(=O)₂(-C₁₋₄ alkylene-NR₀₁R₀₂), -S(=O)(-C₁₋₄ alkylene-NR₀₁R₀₂), -S(=O)₂(-C₁₋₄ alkylene-C(R₀₅)R₀₃R₀₄), -S(=O)(-C₁₋₄ alkylene-C(R₀₅)R₀₃R₀₄), 5- or 6-membered monocyclic heteroaryl, or 8- to 10-membered bicyclic heteroaryl, wherein the 3- to 8-membered monocyclic heterocyclyl, 5- to 15-membered tricyclic heterocyclyl, 7- to 12-membered bicyclic heterocyclyl, 5- or 6-membered monocyclic heteroaryl, or 8- to 10-membered bicyclic heteroaryl is optionally substituted with 1, 2, 3, 4, 5, or 6 S1;
R₀₁ and R₀₂ are each independently selected from hydrogen, C₁₋₆ alkyl, C₁₋₆ alkoxy, 3- to 6-membered cycloalkyl, C₂₋₆ alkenyl, and C₂₋₆ alkynyl, wherein the C₁₋₆ alkyl, C₁₋₆ alkoxy, 3- to 6-membered cycloalkyl, C₂₋₆ alkenyl, or C₂₋₆ alkynyl is optionally substituted with 1, 2, 3, 4, 5, or 6 S1; or
R₀₁ and R₀₂, together with the nitrogen atom to which they are attached, form 5- to 15-membered tricyclic heterocyclyl, 7- to 12-membered bicyclic heterocyclyl, or 3- to 8-membered monocyclic heterocyclyl, wherein the 5- to 15-membered tricyclic heterocyclyl, 7- to 12-membered bicyclic heterocyclyl, or 3- to 8-membered monocyclic heterocyclyl is optionally substituted with 1, 2, 3, 4, 5, or 6 S1;
R₀₃ and R₀₄ are each independently selected from hydrogen, C₁₋₆ alkyl, C₁₋₆ alkoxy, C₂₋₆ alkenyl, and C₂₋₆ alkynyl, wherein the C₁₋₆ alkyl, C₁₋₆ alkoxy, C₂₋₆ alkenyl, or C₂₋₆ alkynyl is optionally substituted with 1, 2, 3, 4, 5, or 6 S1; wherein optionally, 2 hydrogen atoms on the same carbon atom of the C₁₋₆ alkyl or C₁₋₆ alkoxy are both substituted with -(CH₂)ⱼ- to form cycloalkyl, wherein j is 2, 3, 4, 5, or 6; or
R₀₃ and R₀₄, together with the carbon atom to which they are attached, form 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl, wherein the 7- to 12-membered bicyclic heterocyclyl, 5- to 15-membered tricyclic heterocyclyl, 3- to 8-membered monocyclic heterocyclyl, 5- or 6-membered monocyclic heteroaryl, or 8- to 10-membered bicyclic heteroaryl is optionally substituted with 1, 2, 3, 4, 5, or 6 S1;
R₀₅ is hydrogen, deuterium, halogen, deuterated C₁₋₆ alkyl, or C₁₋₆ alkyl;
R₀₆ is hydrogen, deuterium, or C₁₋₆ alkyl;
Y₃ is -C(=O)Rj⁴, -C(=S)Rj⁴, -S(=O)(R₀₇)(=N-R₀₈), -S(=O)(R₀₇)(R₀₈), -S(=O)₂(R₀₇)(R₀₈), -P(=O)(R₀₇)(R₀₈), - C(=O)-C₁₋₄ alkylene-NRj⁴-S(=O)(R₀₇)(=N-R₀₈), -C(=O)-C₁₋₄ alkylene-N=S(=O)(R₀₇)(R₀₈), or R₁₁;
Rj⁴ is C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl, wherein the C₁₋₆ alkyl, C₁₋₆ heteroalkyl, C₃₋₆ cycloalkyl, 3- to 8-membered heterocyclyl, C₆₋₁₀ aryl, 5- or 6-membered monocyclic heteroaryl, or 8- to 10-membered bicyclic heteroaryl is optionally substituted with 1, 2, 3, 4, 5, or 6 S1;
R₁₁ is 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7-to 12-membered bicyclic heterocyclyl, or 5- to 15-membered tricyclic heterocyclyl, wherein the 3- to 10-membered heterocyclyl, 5- or 6-membered monocyclic heteroaryl, 8- to 10-membered bicyclic heteroaryl, 6- to 12-membered heteroaryl-fused cycloalkyl, 6- to 12-membered heteroaryl-fused heterocyclyl, 7- to 12-membered bicyclic heterocyclyl, or 5- to 15-membered tricyclic heterocyclyl is optionally substituted with 1, 2, 3, 4, 5, or 6 S1;
R₀₇ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
R₀₈ is hydrogen, deuterium, C₁₋₆ alkyl, or C₃₋₆ cycloalkyl, wherein the C₁₋₆ alkyl or C₃₋₆ cycloalkyl is optionally substituted with one or more groups selected from the group consisting of: deuterium, C₁₋₆ alkyl, C₁₋₆ heteroalkyl, and 5- or 6-membered monocyclic heteroaryl;
each S1 is independently selected from the group consisting of: deuterium, oxo (=O), thio (=S), =CR^{e}R^{f}, =NR^{e}, halogen, cyano, hydroxy, carboxyl, nitro, C₁₋₆ alkyl, C₂₋₆ alkenyl, C₂₋₆ alkynyl, C₁₋₆ alkoxy, C₃₋₂₀ cycloalkyl, 3-to 20-membered heterocyclyl, C₆₋₁₄ aryl, 5- or 6-membered monocyclic heteroaryl, 8- to 10-membered bicyclic heteroaryl, -O-C₃₋₂₀ cycloalkyl, -O-3- to 20-membered heterocyclyl, -O-C₆₋₁₄ aryl, -O-5- or 6-membered monocyclic heteroaryl, -O-8- to 10-membered bicyclic heteroaryl, -C≡C-C₃₋₂₀ cycloalkyl, -C≡C-3- to 20-membered heterocyclyl, -C≡C-C₆₋₁₄ aryl, -C≡C-5- or 6-membered monocyclic heteroaryl, -C≡C-8- to 10-membered bicyclic heteroaryl, -C≡C-C₁₋₄ alkylene-C₃₋₂₀ cycloalkyl, -C≡C-C₁₋₄ alkylene-3- to 20-membered heterocyclyl, -C≡C-C₁₋₄ alkylene-C₆₋₁₄ aryl, -C≡C-C₁₋₄ alkylene-5- or 6-membered monocyclic heteroaryl, - C≡C-C₁₋₄ alkylene-8- to 10-membered bicyclic heteroaryl, -C₁₋₄ alkylene-hydroxy, -C₁₋₄ alkylene-cyano, -C₁₋₄ alkylene-C₁₋₆ alkyl, -C₁₋₄ alkylene-C₁₋₆ alkoxy, -C₁₋₄ alkylene-C₃₋₂₀ cycloalkyl, -C₁₋₄ alkylene-O-C₃₋₂₀ cycloalkyl, -C₁₋₄ alkylene-3- to 20-membered heterocyclyl, -C₁₋₄ alkylene-O-3- to 20-membered heterocyclyl, -C₁₋₄ alkylene-C₆₋₁₄ aryl, -C₁₋₄ alkylene-O-C₆₋₁₄ aryl, -C₁₋₄ alkylene-5- or 6-membered monocyclic heteroaryl, -C₁₋₄ alkylene-O-5- or 6-membered monocyclic heteroaryl, -C₁₋₄ alkylene-8- to 10-membered bicyclic heteroaryl, - C₁₋₄ alkylene-O-8- to 10-membered bicyclic heteroaryl, -O-C₁₋₄ alkylene-hydroxy, -O-C₁₋₄ alkylene-cyano, -O-C₁₋₄ alkylene-C₁₋₆ alkyl, -O-C₁₋₄ alkylene-C₁₋₆ alkoxy, -O-C₁₋₄ alkylene-C₃₋₂₀ cycloalkyl, -O-C₁₋₄ alkylene-O-C₃₋₂₀ cycloalkyl, -O-C₁₋₄ alkylene-3- to 20-membered heterocyclyl, -O-C₁₋₄ alkylene-O-3- to 20-membered heterocyclyl, -O-C₁₋₄ alkylene-C₆₋₁₄ aryl, -O-C₁₋₄ alkylene-O-C₆₋₁₄ aryl, -O-C₁₋₄ alkylene-5- or 6-membered monocyclic heteroaryl, -O-C₁₋₄ alkylene-O-5- or 6-membered monocyclic heteroaryl, -O-C₁₋₄ alkylene-8- to 10-membered bicyclic heteroaryl, -O-C₁₋₄ alkylene-O-8- to 10-membered bicyclic heteroaryl, -S(=O)₂-C₁₋₆ alkyl, - S(=O)₂-C₃₋₂₀ cycloalkyl, -S(=O)₂-3- to 20-membered heterocyclyl, -C(=O)O-C₁₋₆ alkyl, -C(=O)O-C₃₋₂₀ cycloalkyl, -C(=O)-C₁₋₆ alkyl, -C(=O)-C₃₋₂₀ cycloalkyl, -C(=O)-C₆₋₁₄ aryl, -NR^{a1}R^{b1}, -C(=O)-NR^{a1}R^{b1}, -C(=O)-NR^{d1}-C₁₋₄ alkylene-R^{c1}, -OR^{c1}, -C₁₋₄ alkylene-S(=O)₂-C₁₋₆ alkyl, -C₁₋₄ alkylene-S(=O)₂-C₃₋₂₀ cycloalkyl, -C₁₋₄ alkylene-S(=O)₂-3- to 20-membered heterocyclyl, -C₁₋₄ alkylene-C(=O)O-C₁₋₆ alkyl, -C₁₋₄ alkylene-C(=O)O-C₃₋₂₀ cycloalkyl, -C₁₋₄ alkylene-C(=O)-C₁₋₆ alkyl, -C₁₋₄ alkylene-C(=O)-C₃₋₂₀ cycloalkyl, -C₁₋₄ alkylene-C(=O)-C₆₋₁₄ aryl, -C(=O)-5- or 6-membered monocyclic heteroaryl, -C(=O)-8- to 10-membered bicyclic heteroaryl, - C(=O)-C₁₋₆ alkylene-C₃₋₂₀ cycloalkyl, -C(=O)-C₁₋₆ alkylene-3- to 20-membered heterocyclyl, -C(=O)-C₁₋₆ alkylene-C₆₋₁₄ aryl, -C(=O)-C₁₋₆ alkylene-5- or 6-membered monocyclic heteroaryl, -C(=O)-C₁₋₆ alkylene-8- to 10-membered bicyclic heteroaryl, -C₁₋₄ alkylene-NR^{a1}R^{b1}, -C₁₋₄ alkylene-C(=O)-NR^{a1}R^{b1}, -C≡C-C(=O)-NR^{a1}R^{b1}, -C≡C-C₁₋₄ alkylene-C(=O)-NR^{a1}R^{b1}, -C₁₋₄ alkylene-OR^{c1}, -C₁₋₄ alkylene-P(=O)-(C₁₋₆ alkyl)₂, -P(=O)-(C₁₋₆ alkyl)₂, -C₁₋₄ alkylene-NR^{d1}-C(=O)-R^{c1}, -C₁₋₄ alkylene-NR^{d1}-C(=O)-NR^{a1}R^{b1}, -C₁₋₄ alkylene-NR^{d1}-S(=O)₂-R^{c1}, -C₁₋₄ alkylene-S(=O)₂-NR^{a1}R^{b1}, -C₁₋₄ alkylene-NR^{d1}-S(=O)₂-NR^{a1}R^{b1}, -NR^{d1}-C(=O)-R^{c1}, -NR^{d1}-C(=O)-C₁₋4 alkylene-R^{c1}, -NR^{d1}-C(=O)-NR^{a1}R^{b1}, -NR^{d1}-S(=O)₂-R^{c1}, -S(=O)2-NR^{a1}R^{b1}, -NR^{d1}-S(=O)₂-NR^{a1}R^{b1}, and - P(=O)-(C₁₋₆ alkyl)₂, wherein the C₁₋₆ alkyl, C₁₋₆ alkoxy, C₂₋₆ alkenyl, and C₂₋₆ alkynyl are each independently and optionally substituted with 1, 2, or 3 groups selected from halogen, deuterium, cyano, and hydroxy; the C₃₋₂₀ cycloalkyl, 3- to 20-membered heterocyclyl, C₆₋₁₄ aryl, 5- or 6-membered monocyclic heteroaryl, and 8- to 10-membered bicyclic heteroaryl are each independently and optionally substituted with 1, 2, 3, or 4 groups selected from group S2;
in the groups described above, R^{a1} and R^{b1} are each independently H, C₁₋₆ alkyl, C₁₋₆ haloalkyl, deuterated C₁₋₆ alkyl, -C₁₋₄ alkylene-hydroxy, -C₁₋₄ alkylene-cyano, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, -C₁₋₄ alkylene-8- to 10-membered bicyclic heteroaryl, -S(=O)₂-C₁₋₆ alkyl, -S(=O)₂-C₃₋₆ monocyclic cycloalkyl, -S(=O)₂-3- to 6-membered monocyclic heterocyclyl, -C₁₋₄ alkylene-S(=O)₂-C₁₋₆ alkyl, -C₁₋₄ alkyleneS(=O)₂-C₃₋₆ monocyclic cycloalkyl, -C₁₋₄ alkylene-S(=O)₂-3- to 6-membered monocyclic heterocyclyl, -C(=O)-C₁₋₆ alkyl, -C(=O)-C₃₋₆ monocyclic cycloalkyl, or -C(=O)-3- to 6-membered monocyclic heterocyclyl, wherein the C₃₋₆ monocyclic cycloalkyl, 3- to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂; or
each R^{a1} and R^{b1}, together with the nitrogen atom to which they are attached, form 3- to 20-membered heterocyclyl, wherein each 3- to 20-membered heterocyclyl is independently and optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, each R^{d1} is independently H, C₁₋₆ alkyl, or deuterated C₁₋₆ alkyl;
in the groups described above, each R^{c1} is independently H, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -C₁₋₄ alkylene-C₁₋₆ haloalkyl, -C₁₋₄ alkylene-deuterated C₁₋₆ alkyl, -C₁₋₄ alkylene-C₁₋₆ alkoxy, -C₁₋₄ alkylene-C₁₋₆ haloalkoxy, -C₁₋₄ alkylene-deuterated C₁₋₆ alkoxy, C₃₋₆ monocyclic cycloalkyl, -C₁₋₄ alkylene-C₃₋₆ monocyclic cycloalkyl, -C₁₋₄ alkylene-O-C₃₋₆ monocyclic cycloalkyl, 3- to 6-membered monocyclic heterocyclyl, -C₁₋₄ alkylene-3- to 6-membered monocyclic heterocyclyl, -C₁₋₄ alkylene-O-3- to 6-membered monocyclic heterocyclyl, phenyl, -C₁₋₄ alkylene-phenyl, 5- or 6-membered monocyclic heteroaryl, -C₁₋₄ alkylene-5- or 6-membered monocyclic heteroaryl, 8- to 10-membered bicyclic heteroaryl, or -C₁₋₄ alkylene-8- to 10-membered bicyclic heteroaryl, wherein the C₃₋₆ monocyclic cycloalkyl, 3-to 6-membered monocyclic heterocyclyl, phenyl, 5- or 6-membered monocyclic heteroaryl, or 8- to 10-membered bicyclic heteroaryl is optionally substituted with 1 or 2 groups selected from the group consisting of: halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -C₁₋₄ alkylene-P(=O)-(C₁₋₆ alkyl)₂, and -P(=O)-(C₁₋₆ alkyl)₂;
in the groups described above, the groups of group S2 are each independently selected from the group consisting of: deuterium, oxo (C=O), thio (=S), =CR^{e}R^{f}, =NR^{e}, halogen, hydroxy, carboxyl, nitro, C₁₋₆ alkyl, C₁₋₆ haloalkyl, deuterated C₁₋₆ alkyl, C₁₋₆ alkoxy, C₁₋₆ haloalkoxy, deuterated C₁₋₆ alkoxy, -NH₂, -NHC₁₋₆ alkyl, -N(C₁₋₆ alkyl)₂, -(C=O)-NHC₁₋₆ alkyl, -(C=O)-N(C₁₋₆ alkyl)₂, -C₁₋₄ alkylene-P(=O)-(C₁₋₆ alkyl)₂, -P(=O)-(C₁₋₆ alkyl)₂, -(C=O)C₁₋₆ alkyl, and SF₅;
in the groups described above, the -C₁₋₄ alkylene- is unsubstituted; or 1, 2, 3, or 4 hydrogen atoms on the -C₁₋₄ alkylene- are each independently substituted with a group selected from halogen, cyano, hydroxy, C₁₋₆ alkyl, C₁₋₆ haloalkyl, deuterated C₁₋₆ alkyl, -CH₂-hydroxy, -CH₂-cyano, and phenyl; or 2 hydrogen atoms on the same carbon atom of the -C₁₋₄ alkylene- are both substituted with -(CH₂)ⱼ- to form cycloalkyl, wherein j is 2, 3, 4, 5, or 6; or 2 hydrogen atoms on the same carbon atom of the -C₁₋₄ alkylene- are both substituted with =CR^{e}R^{f};
in the groups described above, each R^{e} is independently H, halogen, C₁₋₆ alkyl, C₁₋₆ haloalkyl, or deuterated C₁₋₆ alkyl;
in the groups described above, each R^{f} is independently H, halogen, C₁₋₆ alkyl, C₁₋₆ haloalkyl, or deuterated C₁₋₆ alkyl;
in the groups described above, one or more ring atoms of the 3- to 8-membered heterocyclyl, 3- to 10-membered heterocyclyl, or 3- to 8-membered monocyclic heterocyclyl are heteroatoms selected from nitrogen, oxygen, sulfur, and phosphorus; one or more atoms of the C₁₋₆ heteroalkyl are heteroatoms selected from nitrogen, oxygen, sulfur, and phosphorus; one or more ring atoms of the 7- to 12-membered bicyclic heterocyclyl are heteroatoms selected from nitrogen, oxygen, sulfur, and phosphorus; one or more ring atoms of the 3- to 20-membered heterocyclyl are heteroatoms selected from nitrogen, oxygen, sulfur, and phosphorus; one or more ring atoms of the 5- to 20-membered heteroaryl are heteroatoms selected from nitrogen, oxygen, sulfur, and phosphorus; one or more ring atoms of the 5- or 6-membered monocyclic heteroaryl are heteroatoms selected from nitrogen, oxygen, sulfur, and phosphorus; one or more ring atoms of the 8- to 10-membered bicyclic heteroaryl are heteroatoms selected from nitrogen, oxygen, sulfur, and phosphorus; one or more ring atoms of the 3- to 6-membered monocyclic heterocyclyl are heteroatoms selected from nitrogen, oxygen, sulfur, and phosphorus; one or more ring atoms of the 5- to 15-membered tricyclic heterocyclyl are heteroatoms selected from nitrogen, oxygen, sulfur, and phosphorus; one or more ring atoms of the 6- to 12-membered heteroaryl-fused cycloalkyl are heteroatoms selected from nitrogen, oxygen, sulfur, and phosphorus; one or more ring atoms of the 6- to 12-membered heteroaryl-fused heterocyclyl are heteroatoms selected from nitrogen, oxygen, sulfur, and phosphorus; wherein when the ring atom is a sulfur atom, the sulfur atom is optionally substituted with one or two groups selected from oxo and =NR^{e}; R^{e} is as defined above;
wherein when the ring atom is a phosphorus atom, the phosphorus atom is optionally substituted with one or two groups selected from oxo and =NR^{e}; R^{e} is as defined above.

5. The antibody-drug conjugate according to any one of claims 1-4, wherein,
Z₄, Z₅*,* and Z₆ are each independently CR₁₃, CR₁₄, CR₄₈, CR₄₉, or CR₅₀, wherein R₁₃, R₁₄, R₄₈, R₄₉, or R₅₀ is as defined in any one of claims 2-4.

6. The antibody-drug conjugate according to any one of claims 1-5, wherein Z₄, Z₅*,* and Z₆ are each independently CH.

7. The antibody-drug conjugate according to any one of claims 1-6, wherein,
Rj⁵ is hydrogen, C₁₋₆ alkyl, C₁₋₆ heteroalkyl, C₂₋₆ alkenyl, C₂₋₆ alkynyl, C₃₋₆ cycloalkyl, or 3- to 8-membered heterocyclyl, wherein the C₁₋₆ alkyl, C₁₋₆ heteroalkyl, C₂₋₆ alkenyl, C₂₋₆ alkynyl, C₃₋₆ cycloalkyl, or 3- to 8-membered heterocyclyl is optionally substituted with 1, 2, 3, 4, 5, or 6 S1, and S1 is as defined in any one of claims 2-6 or in this claim;
each S1 is independently hydrogen, hydroxy, cyano, amino, -C₁₋₄ alkyl, NH(C₁₋₄ alkyl), or N(C₁₋₄ alkyl)₂, wherein the C₁₋₄ alkyl is optionally substituted with 1, 2, or 3 substituents selected from OH and NH₂.

8. The antibody-drug conjugate according to any one of claims 1-7, wherein Rj⁵ is hydrogen, C₁₋₆ alkyl, C₃₋₆ cycloalkyl, -C₁₋₄ alkylene-OH, -C₁₋₄ alkylene-NHC₁₋₆ alkyl, or -C₁₋₄ alkylene-C₁₋₆ alkoxy;
preferably, Rj⁵ is ethyl, cyclopropyl, -(CH₂)₂OH, -(CH₂)₃OH, -(CH₂)₂-NH(CH₃), or -(CH₂)₂-OCH₃;
when L₀ is attached to Rj⁵, any hydrogen atom on Rj⁵ is replaced by L₀, wherein Rj⁵ is as defined in any one of claims 2-7 or in this claim;
when L₀ is attached to Rj⁵, preferably, Rj⁵ is 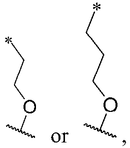
and further preferably, Rj⁵ is 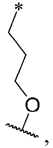
wherein the 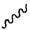 end is attached to L₀, and the * end is attached to another fragment in D.

9. The antibody-drug conjugate according to any one of claims 1-8, wherein,
Y₁ and Y₂ are each independently N, N⁺-O⁻, CH, or CR_{4b}, wherein R_{4b} is deuterium, halogen, C₁₋₆ alkyl, or C₁₋₆ heteroalkyl;
preferably, Y₁ is N or N⁺-O⁻;
preferably, Y₂ is CH;
when L₀ is attached to Y₁, any hydrogen atom on Y₁ is replaced by L₀, or any N atom on Y₁ is quaternized, wherein Y₁ is as defined in any one of claims 2-8 or in this claim;
when L₀ is attached to Y₁, preferably, Y₁ is N⁺.

10. The antibody-drug conjugate according to any one of claims 1-10, wherein,
R₄ₐ is 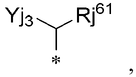 and Yj₃ is NH-Rj⁶, O-Rj⁶, NRj⁶²Rj⁶, -C₁₋₄ alkylene-NH-Rj⁶, -C₁₋₄ alkylene-NRj⁶²Rj⁶, or - C₁₋₄ alkylene-O-Rj⁶, wherein Rj⁶, Rj⁶², and Rj⁶¹ are as defined in claim 2, and the * end is attached to another fragment in D.

11. The antibody-drug conjugate according to any one of claims 1-10, wherein R₄ₐ is C₁₋₆ alkyl substituted with hydroxy or C₁₋₄ heteroalkyl (e.g., methoxy or methylamino);
preferably, R₄ₐ is -CH(CH₃)OCH₃ or -CH(CH₃)NHCH₃;
when L₀ is attached to R₄ₐ, any hydrogen atom on R₄ₐ is replaced by L₀, or any N atom on R₄ₐ is quaternized, wherein R₄ₐ is as defined in any one of claims 2-10 or in this claim;
when L₀ is attached to R₄ₐ, preferably, R₄ₐ is 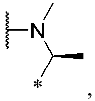 wherein the 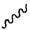 end is attached to L₀, and the * end is attached to another fragment in D.

12. The antibody-drug conjugate according to any one of claims 1-11, wherein,
Yj₄ is -C₂₋₄ alkynylene-C₁₋₄ alkylene-NR₀₁R₀₂, -C₂₋₄ alkynylene-C₁₋₄ alkylene-3- to 8-membered monocyclic heterocyclyl, -O-C₁₋₄ alkylene-3- to 8-membered monocyclic heterocyclyl, 3- to 8-membered monocyclic heterocyclyl, 7- to 12-membered bicyclic heterocyclyl, or C₆₋₁₀ aryl, wherein the 3- to 8-membered monocyclic heterocyclyl, 7- to 12-membered bicyclic heterocyclyl, or C₆₋₁₀ aryl is optionally substituted with 1, 2, 3, 4, 5, or 6 S1, and R₀₁ and R₀₂ are each independently hydrogen, C₁₋₆ alkyl, or 3- to 8-membered monocyclic heteroalkyl, wherein the C₁₋₆ alkyl or 3- to 8-membered monocyclic heteroalkyl is optionally substituted with 1, 2, 3, 4, 5, or 6 S1, and S1 is as defined in any one of claims 2-11;
preferably, Yj₄ is 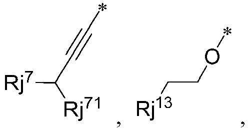 3- to 8-membered monocyclic heterocyclyl, 7- to 12-membered bicyclic heterocyclyl, or phenyl, wherein the 3- to 8-membered monocyclic heterocyclyl, 7- to 12-membered bicyclic heterocyclyl, or phenyl is optionally substituted with one or more substituents selected from C₁₋₆ alkyl, -C₁₋₄ alkylene-hydroxy, -C(=O)-C₁₋₄ alkylene-hydroxy, and -C₁₋₄ alkylene-3- to 8-membered monocyclic heterocyclyl;
each Rj⁷ or Rj¹³ is independently 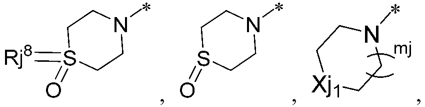 NR₀₁R₀₂, 4- to 5-membered monocyclic heterocyclyl, oxo-5-membered heterocyclyl-Lj₁-Lj₂-Rj¹⁴, oxo-6-membered heterocyclyl-Lj₁-Lj₂-Rj¹⁴, or oxo-7-membered heterocyclyl-Lj₁-Lj₂-Rj¹⁴, wherein the 4- to 5-membered monocyclic heterocyclyl is optionally substituted with hydroxy, cyano, or amino, and Rj⁷ is optionally substituted with -C₁₋₄ alkylene-OH or -C₁₋₄ alkylene-NH-Rj⁶;
Rj⁷¹ is hydrogen, C₁₋₆ alkyl, -C₁₋₄ alkylene-OH, or -C₁₋₄ alkylene-NH-Rj⁶;
Rj⁸ is O or NH;
Xj₁ is -O-, -CRj⁹¹Rj⁹²-, or -NRj¹⁰-;
mj is 1 or 2;
Lj₁ and Lj₂ are each independently -C₁₋₄ alkylene-O- or -C₁₋₄ alkylene-NH-;
Rj¹⁴ is C₁₋₆ heteroalkyl;
Rj⁶ is hydrogen or C₁₋₆ alkyl;
Rj⁹¹ and Rj⁹² are each independently hydrogen, hydroxy, cyano, -NRj^{c}Rj^{d}, -C₁₋₄ alkylene-NRj^{c}Rj^{d}, -C₁₋₄ alkylene-OH, -C(O)-NRj^{a}Rj^{b}, or -C(O)-NRj^{c}Rj^{d}, wherein the -C₁₋₄ alkylene- is optionally substituted with methyl;
Rj¹⁰ is hydrogen, C₁₋₆ alkyl, -C(O)-C₁₋₆ alkyl, -C(O)-C₁₋₄ alkylene-NRj^{f}Rj^{g}, or -C(O)-C₁₋₄ alkylene-OH;
Rj^{a} and Rj^{b} are each independently hydrogen, C₁₋₆ alkyl, or C₃₋₆ cycloalkyl;
Rj^{c} and Rj^{d} are each independently hydrogen, -C(O)H, C₁₋₆ alkyl, -C₁₋₄ alkylene-OH, 5- to 8-membered heterocyclyl, -C(O)-5- to 8-membered heterocyclyl, or -C(O)-C₁₋₄ alkylene-OH, wherein the 5- to 8-membered heterocyclyl is optionally substituted with one or more substituents selected from Rj⁴²;
Rj⁴² is methyl, -NRj^{f}Rj^{g}, -C(O)Rj^{e}, or -C(O)ORj^{e};
Rj^{e} is C₁₋₆ alkyl;
Rj^{f} and Rj^{g} are each independently hydrogen or C₁₋₆ alkyl;
preferably, Yj₄ is 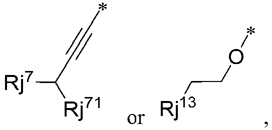 and Rj⁷, Rj⁷¹, and Rj¹³ are as defined in this claim;
preferably, Rj⁷¹ is hydrogen;
preferably, each Rj⁷ or Rj¹³ is independently 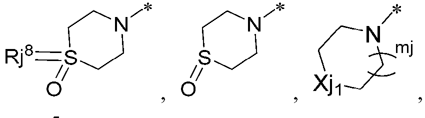 NR₀₁R₀₂, or oxo-6-membered heterocyclyl-Lj₁-Lj₂-Rj¹⁴, wherein Rj⁷ is optionally substituted with -C₁₋₄ alkylene-OH or -C₁₋₄ alkylene-NH-Rj⁶; Rj⁸, Xj₁, mj, R₀₁, R₀₂, Lj₁, Lj₂, and Rj¹⁴ are as defined in this claim; the * end is attached to another fragment in D;
more preferably, Rj⁷ is 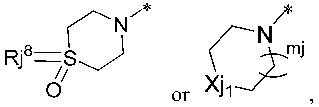 wherein Rj⁸, Xj₁, and mj are as defined in this claim, and preferably, Xj₁ is -CRj⁹¹Rj⁹²- or -NRj¹⁰-, wherein Rj⁹¹, Rj⁹², and Rj¹⁰ are as defined in this claim; the * end is attached to another fragment in D;
preferably, R₀₁ and R₀₂ are each independently hydrogen, C₁₋₄ alkyl, or 4- to 7-membered monocyclic heteroalkyl; preferably, R₀₁ and R₀₂ are each independently hydrogen, methyl, ethyl, n-propyl, isopropyl, oxetanyl, tetrahydrofuranyl, tetrahydropyranyl, azetidinyl, pyrrolidinyl, piperidinyl, piperazinyl, or morpholinyl;
preferably, R₀₁ and R₀₂ are each independently methyl, isopropyl, or tetrahydropyranyl;
preferably, Rj⁶ is hydrogen or C₁₋₄ alkyl;
preferably, Rj⁶ is hydrogen, methyl, ethyl, n-propyl, or isopropyl;
preferably, Rj⁶ is hydrogen;
preferably, Rj⁹¹ and Rj⁹² are each independently hydrogen, hydroxy, cyano, -NRj^{c}Rj^{d}, -C₁₋₄ alkylene-NRj^{c}Rj^{d}, - C₁₋₄ alkylene-OH, or -C(O)-NRj^{c}Rj^{d}, wherein Rj^{c} and Rj^{d} are as defined in this claim;
more preferably, Rj⁹¹ and Rj⁹² are each independently hydrogen, hydroxy, cyano, -CH₂OH, or -CH₂NH₂; preferably, Rj⁹¹ is hydrogen, hydroxy, or cyano, and preferably, Rj⁹¹ is cyano;
preferably, Rj⁹² is hydrogen, amino, -CH₂OH, -CH₂NH₂, or -C(O)NHCH₂CH₂OH;
preferably, Rj⁹¹ and Rj⁹² are not both hydrogen;
preferably, Rj¹⁰ is hydrogen, C₁₋₄ alkyl, -C(O)-C₁₋₄ alkyl, -C(O)-C₁₋₄ alkylene-NH₂, -C(O)-C₁₋₄ alkylene-NHC₁₋₄ alkyl, -C(O)-C₁₋₄ alkylene-N(C₁₋₄ alkyl)₂, or -C(O)-C₁₋₄ alkylene-OH;
preferably, Rj¹⁰ is hydrogen, methyl, ethyl, n-propyl, isopropyl, -C(O)CH₃, -C(O)CH₂CH₃, -C(O)CH(CH₃)₂, - C(O)CH₂NH₂, -C(O)CH₂CH₂NH₂, -C(O)CH₂NHCH₃, -C(O)CH₂N(CH₃)₂, -C(O)CH₂CH₂NHCH₃, - C(O)CH₂OH, or -C(O)CH₂CH₂OH;
preferably, Rj¹⁰ is hydrogen, methyl, -C(O)CH₃, -C(O)CH₂OH, or -C(O)CH₂NH₂;
more preferably, Rj¹⁰ is hydrogen, -C(O)-C₁₋₄ alkylene-NH₂, or -C(O)-C₁₋₄ alkylene-OH;
more preferably, Rj¹⁰ is hydrogen, -C(O)CH₂OH, or -C(O)CH₂NH₂;
further preferably, Rj¹⁰ is -C(O)CH₂OH or -C(O)CH₂NH₂;
preferably, Rj^{a} and Rj^{b} are each independently hydrogen or C₁₋₄ alkyl;
preferably, Rj^{a} and Rj^{b} are each independently hydrogen;
preferably, Rj^{c} and Rj^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from Rj⁴², and Rj⁴² is as defined in this claim;
preferably, Rj^{c} and Rj^{d} are each independently hydrogen, methyl, ethyl, n-propyl, isopropyl, -C(O)CH₃, - C(O)CH₂CH₃, -C(O)CH(CH₃)₂, -C(O)CH₂OH, or -C(O)CH₂CH₂OH;
preferably, Rj⁴² is methyl, -NRj^{f}Rj^{g}, -C(O)Rj^{e}, or -C(O)ORj^{e}, wherein Rj^{f}; Rj^{g}, and Rj^{c} are as defined in this claim;
preferably, Rj^{e} is C₁₋₄ alkyl;
preferably, Rj^{e} is methyl;
preferably, Rj^{f} and Rj^{g} are each independently hydrogen or C₁₋₄ alkyl;
preferably, Rj^{f} and Rj^{g} are each independently hydrogen or methyl;
preferably, Rj⁷ is 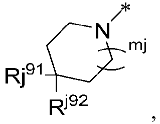 wherein Rj⁹¹ and Rj⁹² are each independently hydrogen, hydroxy, cyano, - NH₂, -CH₂NH₂, -CH₂NH(CH₃), -CH₂OH, or -C(O)-NH(CH₂CH₂OH), Rj⁹¹ and Rj⁹² are not both hydrogen, and mj is 1 or 2;
preferably, Rj⁷ is 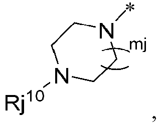 wherein Rj¹⁰ is hydrogen or -C(O)CH₂OH, and mj is 1 or 2;
preferably, Rj⁷ is 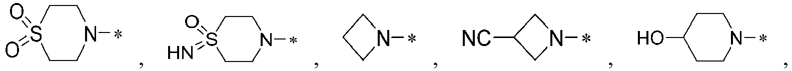 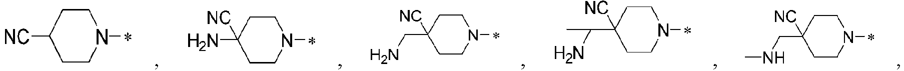 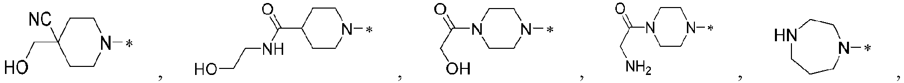 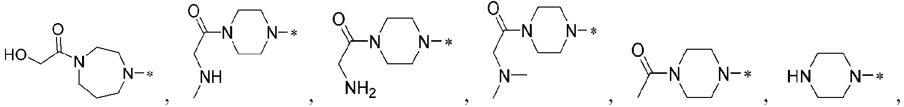 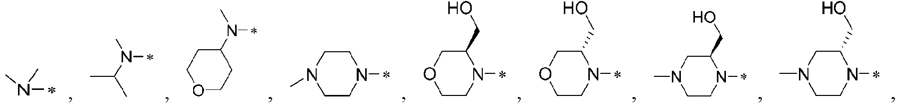 or 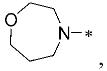
wherein the * end is attached to another fragment in D;
preferably, Rj¹³ is 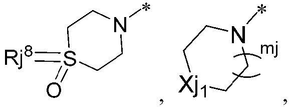 or oxo-6-membered heterocyclyl-Lj₁-Lj₂-Rj¹⁴ wherein Rj⁸,
Xj₁, mj, Lj₁, Lj₂, and Rj¹⁴ are as defined in this claim, and the * end is attached to another fragment in D; preferably, Xj₁ is -CRj⁹¹Rj⁹²-, wherein Rj⁹¹ and Rj⁹² are as defined in this claim;
preferably, Rj⁹¹ and Rj⁹² are each independently hydrogen, hydroxy, cyano, amino, -C₁₋₄ alkylene-NH₂, -C₁₋₄ alkylene-NHC₁₋₄ alkyl, or -C₁₋₄ alkylene-N(C₁₋₄ alkyl)₂, and Rj⁹¹ and Rj⁹² are not both hydrogen;
preferably, Rj⁹¹ is cyano, and Rj⁹² is -CH₂NH₂;
preferably, mj is 1;
preferably, Lj₁ is -CH₂CH₂-O-;
preferably, Lj₂ is -CH₂CH₂-NH-;
preferably, Rj¹⁴ is C₁₋₄ heteroalkyl;
preferably, Rj¹⁴ is -CH₂OCH₃ or -CH₂CH₂OCH₃;
preferably, Rj¹³ is 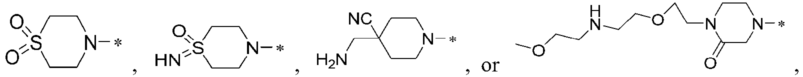 wherein the * end is attached to another fragment in D;
preferably, Yj₄ is 5- to 7-membered nitrogen-containing monocyclic heterocyclyl, 9- to 10-membered nitrogen-containing bicyclic heterocyclyl, or phenyl, wherein the 5- to 7-membered nitrogen-containing monocyclic heterocyclyl, 9- to 10-membered nitrogen-containing bicyclic heterocyclyl, or phenyl is optionally substituted with one or more substituents selected from C₁₋₄ alkyl, -methylene-hydroxy, -C(=O)C₁₋₄ alkylene-OH, and - methylene-5- to 7-membered nitrogen-containing monocyclic heterocyclyl;
preferably, Yj₄ is piperidinyl, piperazinyl, 9- to 10-membered nitrogen-containing bicyclic heterocyclyl, or phenyl, wherein the piperidinyl, piperazinyl, 9- to 10-membered nitrogen-containing bicyclic heterocyclyl, or phenyl is optionally substituted with one or more substituents selected from methyl, -methylene-hydroxy, - methylene-morpholinyl, -C(=O)CH₂OH, and -methylene-piperazinyl;
preferably, Yj₄ is 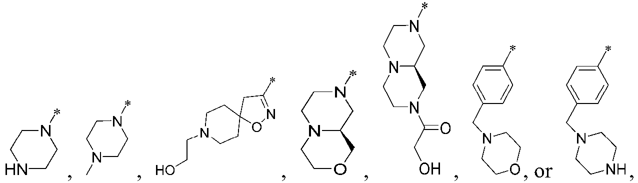 wherein the * end is attached to another fragment in D;
when L₀ is attached to Yj₄, any hydrogen atom on Yj₄ is replaced by L₀, or any N atom on Yj₄ is quaternized, wherein Yj₄ is as defined in any one of claims 2-11 or in this claim;
when L₀ is attached to Yj₄, preferably, Yj₄ is 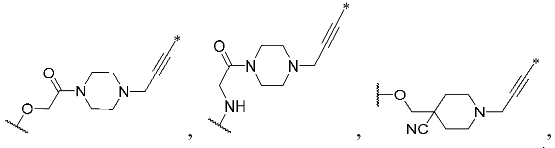 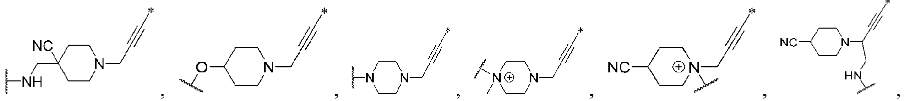 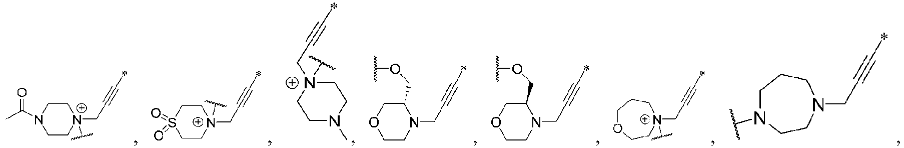 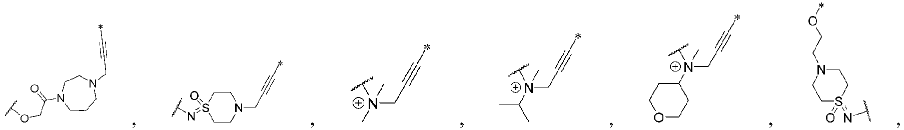 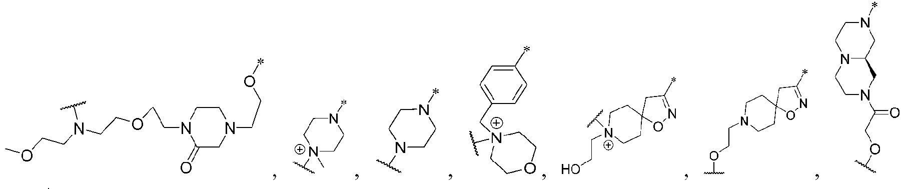 or 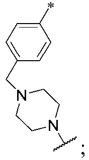
further preferably, Yj₄ is 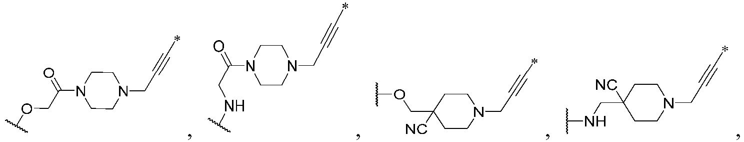 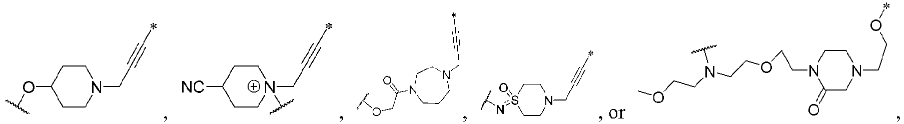
wherein the 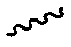 end is attached to L₀, and the * end is attached to another fragment in D.

13. The antibody-drug conjugate according to any one of claims 1-12, wherein,
the structural unit 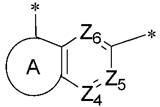 is 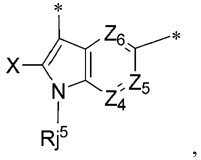 wherein X is 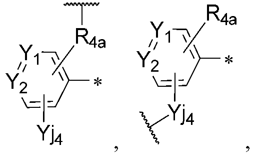 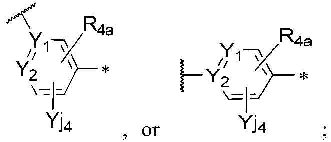 preferably, X is 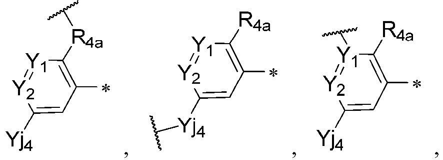 or 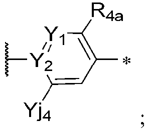 preferably, X is 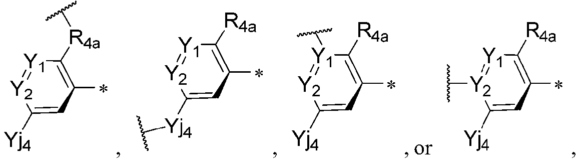 wherein Z₄, Z₅*,* Z₆, Y₁, Y₂, R₄ₐ, Yj₄, and Rj⁵ are as defined in any one of claims 2-12, the 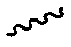 end is attached to L₀, and the * end is attached to another fragment in D;
preferably, the structural unit 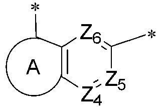 is 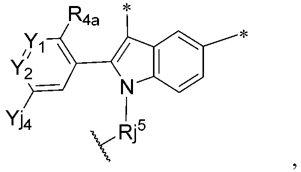 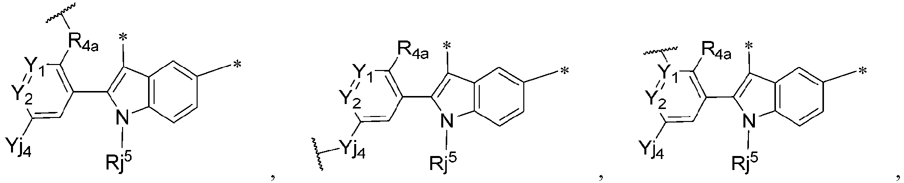 or 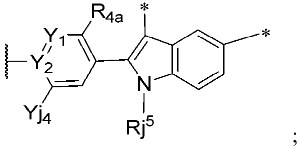 still further preferably, the structural unit 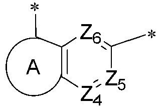 is 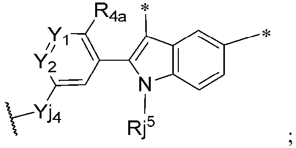 yet still further preferably, the structural unit 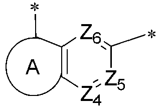 is 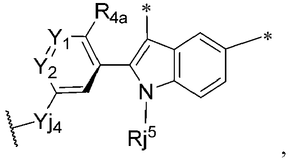 wherein Y₁, Y₂, R₄ₐ, Yj₄, and Rj⁵ are as defined in any one of claims 2-12, the 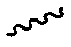 end is attached to L₀, and the * end is attached to another fragment in D.

14. The antibody-drug conjugate according to any one of claims 1-13, wherein,
Lj is -NHC(=O)-, -N(C₁₋₆ alkyl)C(=O)-, C₆₋₁₀ aryl, 5- or 6-membered monocyclic heteroaryl, or 8- to 10-membered bicyclic heteroaryl, wherein the C₆₋₁₀ aryl, 5- or 6-membered monocyclic heteroaryl, or 8- to 10-membered bicyclic heteroaryl is optionally substituted with 1, 2, 3, 4, 5, or 6 S1, and S1 is as defined in any one of claims 2-13;
preferably, each S1 is independently selected from deuterium, halogen, cyano, hydroxy, amino, and C₁₋₄ alkyl; preferably, Lj is phenyl, oxazolyl, or thiazolyl, wherein the phenyl is optionally substituted with hydroxy or amino;
preferably, Lj is phenyl, 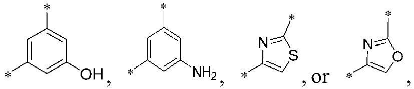 wherein the * end is attached to another fragment in D;
preferably, Lj is oxazolyl or thiazolyl;
preferably, Lj is 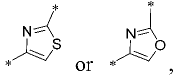 wherein the * end is attached to another fragment in D;
Y₁₀ is O, NH, NCH₃, NCH₂CH₃, or NCH(CH₃)₂;
preferably, Y₁₀ is O or NH;
when L₀ is attached to Lj, any hydrogen atom on Lj is replaced by L₀, or any N atom on Lj is quaternized, wherein Lj is as defined in any one of claims 2-13 or in this claim;
when L₀ is attached to Lj, preferably, Lj is 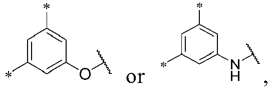 and preferably, Lj is 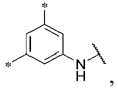 wherein the 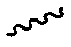 end is attached to L₀, and the * end is attached to another fragment in D.

15. The antibody-drug conjugate according to any one of claims 1-14, wherein,
Rj²¹ and Rj²² are each independently hydrogen, C₁₋₆ alkyl, -NRj^{f}Rj^{g}, or -O-C₁₋₄ alkylene-NRj^{f}Rj^{g}, wherein Rj^{f} and Rj^{g} are as defined in claim 2 or in this claim;
preferably, Rj^{f} and Rj^{g} are each independently hydrogen, methyl, ethyl, n-propyl, or isopropyl;
preferably, Rj²¹ and Rj²² are each independently hydrogen, amino, -NH(C₁₋₄ alkyl), -N(C₁₋₄ alkyl)₂, -O-C₁₋₄ alkylene-NH(C₁₋₄ alkyl), or -O-C₁₋₄ alkylene-N(C₁₋₄ alkyl)₂;
preferably, Rj²¹ is hydrogen, amino, -NH(CH₃), -OCH₂CH₂NHCH₃, or -OCH₂CH₂N(CH₃)₂;
preferably, Rj²² is hydrogen;
when L₀ is attached to Rj²¹, any hydrogen atom on Rj²¹ is replaced by L₀, or any N atom on Rj²¹ is quaternized, wherein Rj²¹ is as defined in any one of claims 2-14 or in this claim;
when L₀ is attached to Rj²¹, preferably, Rj²¹ is -NH-, -N(CH₃)-, 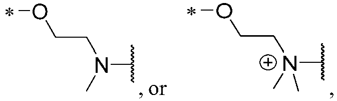 and more
preferably, Rj²¹ is -NH-, -N(CH₃)-, or 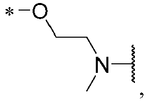 wherein the 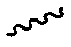 end is attached to L₀, and the * end is attached to another fragment in D.

16. The antibody-drug conjugate according to any one of claims 1-15, wherein,
Rj¹¹ and Rj¹² are each independently hydrogen, deuterated C₁₋₆ alkyl, or C₁₋₆ alkyl;
preferably, Rj¹¹ and Rj¹² are each independently methyl or deuterated methyl;
preferably, Rj¹¹ and Rj¹², together with the carbon atom to which they are attached, form cyclopropyl;
R₄₄, R₄₅, R₄₆, and R₄₇ are each independently hydrogen, deuterium, halogen, C₁₋₄ alkyl, or C₁₋₄ haloalkyl;
preferably, R₄₄, R₄₅, R₄₆, and R₄₇ are hydrogen.

17. The antibody-drug conjugate according to any one of claims 1-16, wherein,
Y₃ is -C(=O)Rj⁴ or R₁₁;
Rj⁴ is C₆₋₁₀ aryl, heteroaryl, C₃₋₆ cycloalkyl, 3- to 8-membered heterocyclyl, or -CRj^{a}Rj^{b}-NRj^{c}Rj^{d}, wherein the C₆₋₁₀ aryl, heteroaryl, C₃₋₆ cycloalkyl, or 3- to 8-membered heterocyclyl is optionally substituted with one or more Rj⁴¹;
R₁₁ is 5- or 6-membered monocyclic heteroaryl or 8- to 10-membered bicyclic heteroaryl, wherein the 5- or 6-membered monocyclic heteroaryl or 8- to 10-membered bicyclic heteroaryl is optionally substituted with one or more Rj⁴¹;
Rj⁴¹ is C₁₋₆ alkyl, hydroxy, SF₅, NRj^{c}Rj^{d}, -P(-O)(C₁₋₆ alkyl)₂, -C₁₋₄ alkylene-OH, or -C(O)-NRj^{c}Rj^{d};
Rj^{a} and Rj^{b} are each independently hydrogen, C₁₋₆ alkyl, or C₃₋₆ cycloalkyl;
Rj^{c} and Rj^{d} are each independently hydrogen, -C(O)H, C₁₋₆ alkyl, C₁₋₆ haloalkyl, phenyl, 5- or 6-membered monocyclic heteroaryl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more Rj⁴²;
Rj⁴² is C₁₋₆ alkyl, C₁₋₆ haloalkyl, -NH₂, -NHC₁₋₆ alkyl, -N(C₁₋₆ alkyl)₂, -C(O)C₁₋₆ alkyl, or -C(O)OC₁₋₆ alkyl;
preferably, Rj⁴ is C₆₋₁₀ aryl, 5- or 6-membered monocyclic heteroaryl, C₃₋₆ cycloalkyl, 3- to 8-membered heterocyclyl, or -CRj^{a}Rj^{b}-NRj^{c}Rj^{d}, wherein the C₆₋₁₀ aryl, 5- or 6-membered monocyclic heteroaryl, C₃₋₆ cycloalkyl, or 3- to 8-membered heterocyclyl is optionally substituted with one or more Rj⁴¹, wherein Rj^{a}, Rj^{b}, Rj^{c}, Rj^{a}, and Rj⁴¹ are as defined in this claim;
still further preferably, Rj⁴ is 5- to 8-membered oxacyclyl, preferably 5- to 8-membered oxa-fused heterocyclyl; or preferably, Rj⁴ is C₃₋₆ cycloalkyl, wherein the C₃₋₆ cycloalkyl is optionally substituted with 1, 2, 3, 4, or 5 Rj⁴¹, and Rj⁴¹ is as defined in this claim; preferably, Rj⁴¹ is hydroxy, methyl, -C₁₋₄ alkylene-OH, or -C(O)-NH(C₁₋₄ alkylene-OH); preferably, Rj⁴¹ is hydroxy, methyl, -CH₂OH, or -C(O)-NH(CH₂CH₂OH);
or preferably, Rj⁴ is -CRj^{a}Rj^{b}-NRj^{c}Rj^{d}, wherein Rj^{a}, Rj^{b}, Rj^{c}, and Rj^{d} are as defined in this claim; preferably, Rj^{a} and Rj^{b} are each independently hydrogen, C₁₋₆ alkyl, or C₃₋₆ cycloalkyl, and Rj^{c} and Rj^{d} are each independently hydrogen, C₁₋₆ alkyl, or -C(O)-C₁₋₄ alkylene-OH; or preferably, Rj⁴ is -CRj^{a}Rj^{b}-NRj^{c}Rj^{d}, wherein Rj^{a} and Rj^{b} are each independently hydrogen or C₁₋₆ alkyl, and Rj^{c} and Rj^{d} are each independently hydrogen, C₁₋₆ alkyl, or - C(O)-pyrrolidinyl, wherein the pyrrolidinyl is optionally substituted with 1, 2, 3, 4, or 5 Rj⁴², and Rj⁴² is methyl or -C(O)OCH₃;
preferably, Rj⁴¹ is C₁₋₄ alkyl, hydroxy, NRj^{c}Rj^{d}, -C₁₋₄ alkylene-OH, or -C(O)-NRj^{c}Rj^{d}, wherein Rj^{c} and Rj^{d} are as defined in this claim;
preferably, Rj⁴¹ is methyl, ethyl, hydroxy, amino, CH₂OH, CH₂CH₂OH, or -C(O)-NRj^{c}Rj^{d}, wherein Rj^{c} and Rj^{d} are as defined in this claim;
preferably, Rj^{a} and Rj^{b} are each independently hydrogen, isopropyl, cyclobutyl, or cyclopentyl;
preferably, Rj^{c} and Rj^{d} are each independently hydrogen, -C(O)H, C₁₋₄ alkyl, C₁₋₄ haloalkyl, phenyl, pyridinyl, - C₁₋₄ alkylene-OH, -C(O)-5- to 6-membered heterocyclyl, or -C(O)-C₁₋₄ alkylene-OH, wherein the 5- to 6-membered heterocyclyl is optionally substituted with one or more Rj⁴², and Rj⁴² is as defined in this claim;
preferably, Rj^{c} and Rj^{d} are each independently hydrogen, methyl, -C(=O)CH₂OH, -C(=O)CH₂CH₂OH, -C(=O)-azetidinyl-NHCH₃, or -C(=O)-pyrrolidinyl(methyl)-C(=O)OCH₃;
preferably, Rj⁴² is C₁₋₄ alkyl, C₁₋₄ haloalkyl, -NHC₁₋₄ alkyl, -N(C₁₋₄ alkyl)₂, -C(O)C₁₋₄ alkyl, or -C(O)OC₁₋₄ alkyl;
preferably, Rj⁴² is methyl, ethyl, halomethyl, haloethyl, -NHCH₃, -N(CH₃)₂, -C(O)CH₃, or -C(O)OCH₃;
preferably, Rj⁴ is 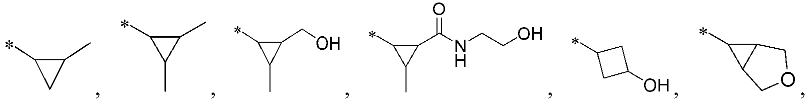 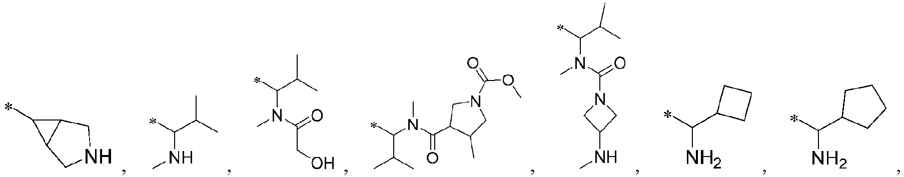 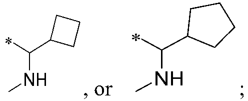
preferably, Rj⁴ is 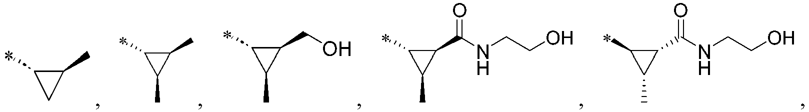 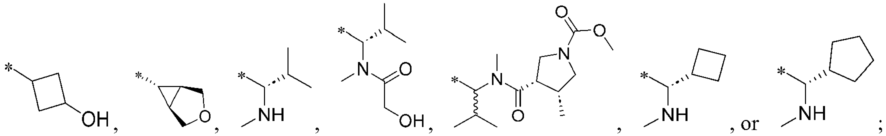
wherein the * end is attached to another fragment in D;
preferably, R₁₁ is indolyl, quinolyl, or triazolopyridinyl, wherein the indolyl, quinolyl, or triazolopyridinyl is optionally substituted with 1, 2, 3, 4, or 5 Rj⁴¹, and Rj⁴¹ is as defined in this claim;
preferably, R₁₁ is 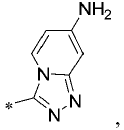 wherein the * end is attached to another fragment in D;
when L₀ is attached to Y₃, any hydrogen atom on Y₃ is replaced by L₀, or any N atom on Y₃ is quaternized, wherein Y₃ is as defined in any one of claims 2-16 or in this claim;
when L₀ is attached to Y₃, preferably, Y₃ is 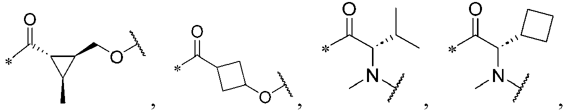 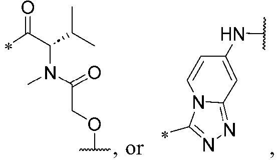 wherein the 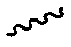 end is attached to L₀, and the * end is attached to another fragment in D;
Rj³ is hydrogen or C₁₋₆ alkyl;
preferably, Rj³ is hydrogen or C₁₋₄ alkyl;
preferably, Rj³ is hydrogen, methyl, or ethyl.

18. The antibody-drug conjugate according to any one of claims 1-17, wherein 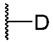 is a compound represented by any one of formula (AIII-1) to formula (AIII-10), or a stable deuterated derivative thereof, a stereoisomer thereof, or a pharmaceutically acceptable salt thereof: 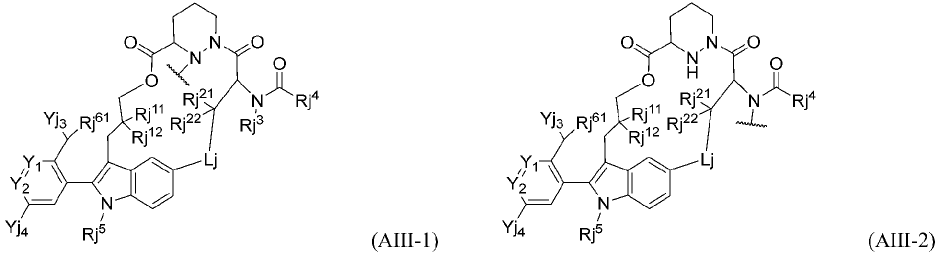 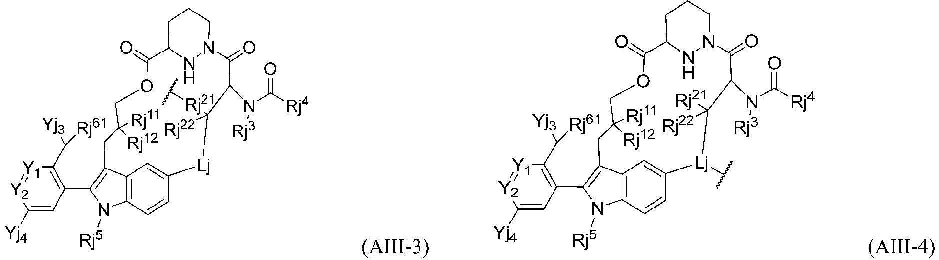 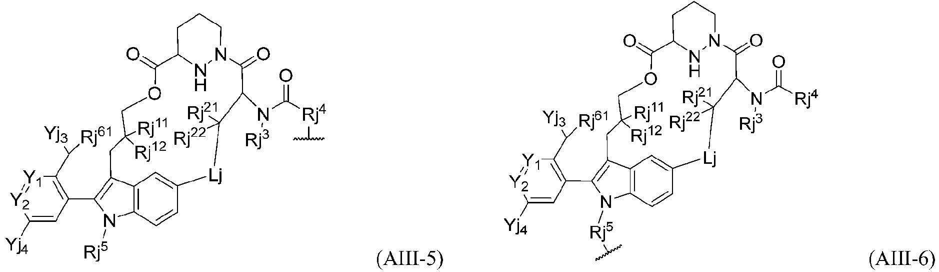 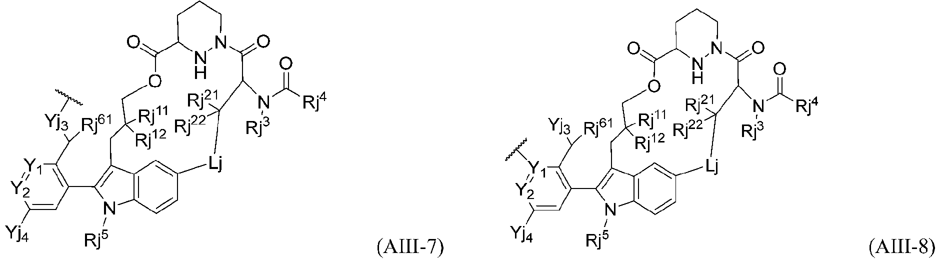 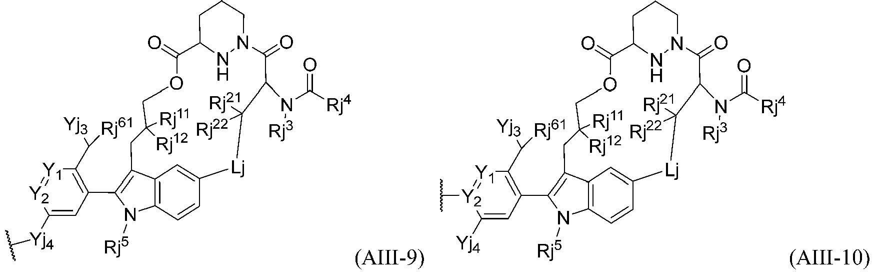 wherein in the formulas,
Rj¹¹, Rj¹², Rj²¹, Rj²², Rj³, Rj⁴, Rj⁵, Rj⁶¹, Y₁, Y₂, Yj₃, Yj₄, and Lj are as defined in any one of claims 1-17, and the 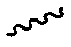 end is attached to L₀;
preferably, the structural unit 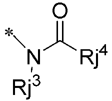 is selected from the group consisting of: 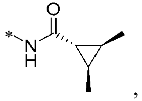 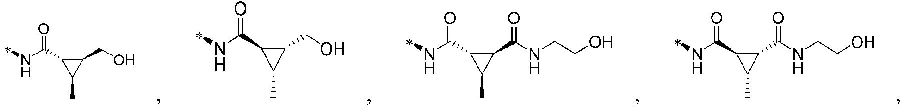 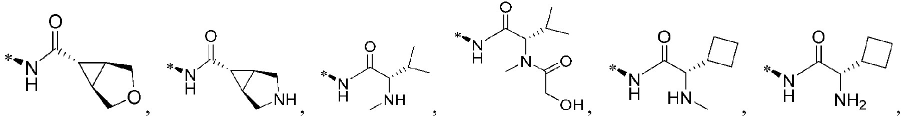 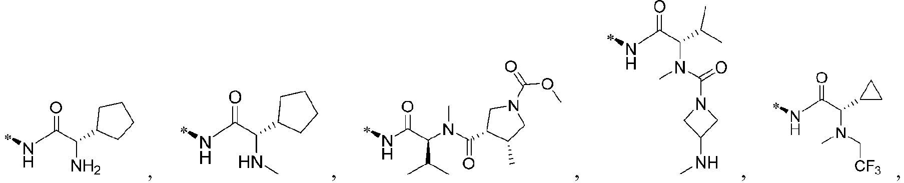 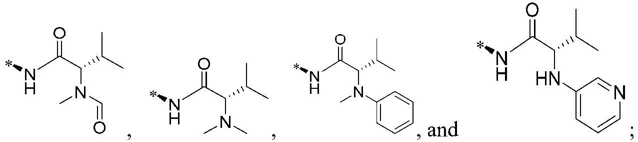
preferably,
**1)** Yj₄ is 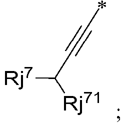
Rj⁷, Rj⁷¹, Rj¹¹, Rj¹², Rj²¹, Rj²², Rj³, Rj⁴, Rj⁵, Rj⁶¹, Y₁, Y₂, Yj₃, and Lj are as defined in any one of claims 1-17 or in this claim, and the * end is attached to another fragment in D;
preferably, Yj₄ is 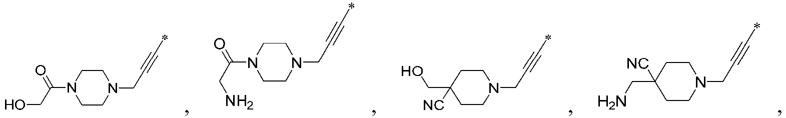 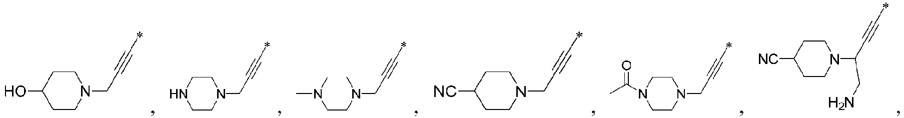 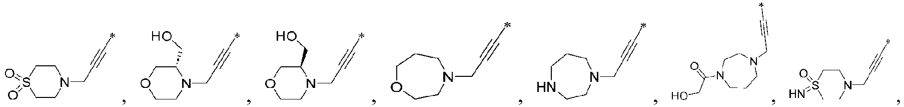 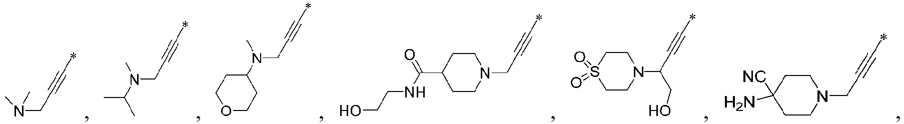 or 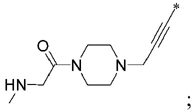
more preferably, Yj₄ is 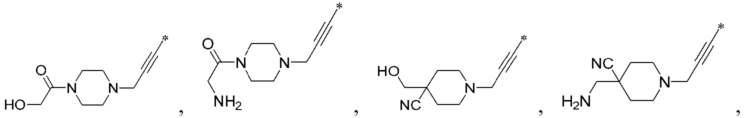 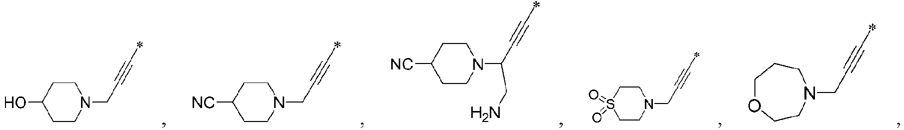 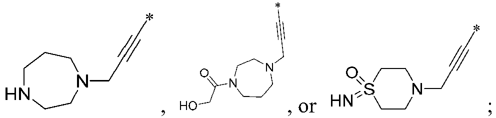
further preferably, Yj₄ is 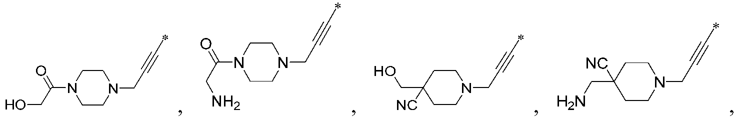 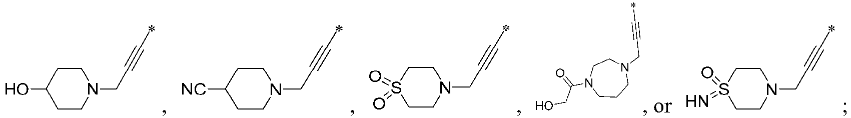
still further preferably, Yj₄ is 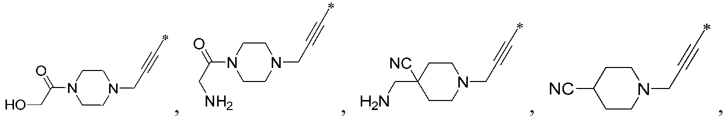 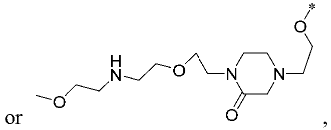
wherein the * end is attached to another fragment in D;
when L₀ is attached to Yj₄, preferably, Yj₄ is 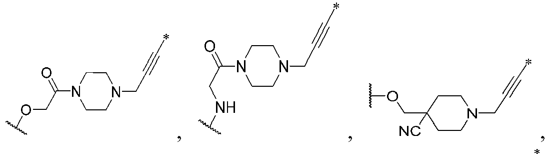 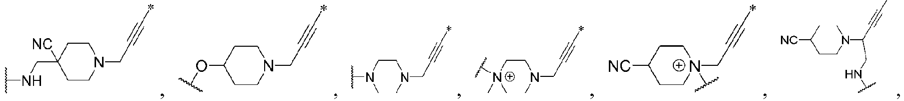 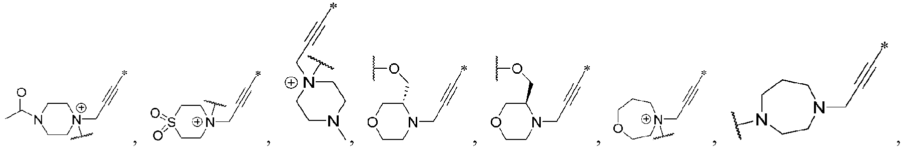 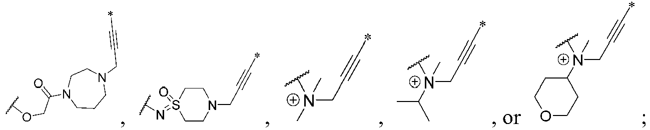
more preferably, Yj₄ is 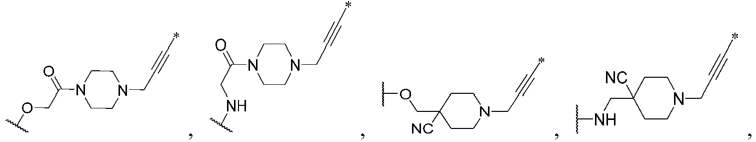 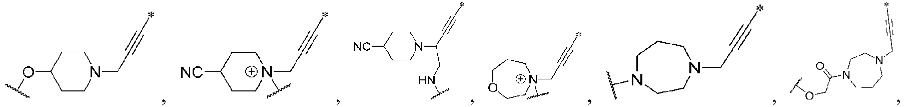 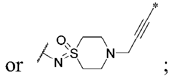
further preferably, Yj₄ is 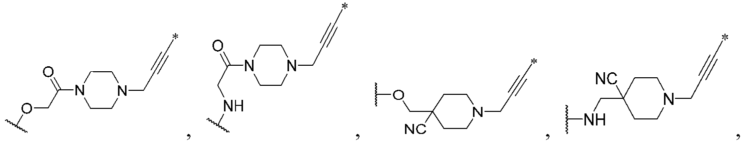 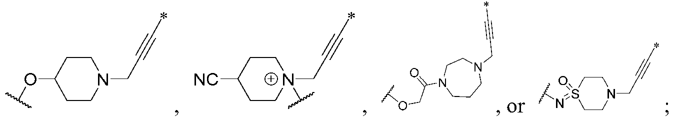
still further preferably, Yj₄ is 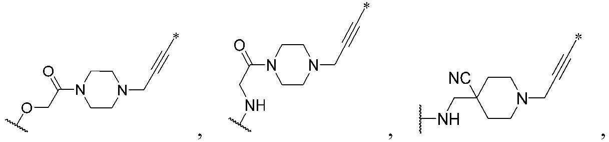 or 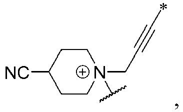
wherein the 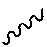 end is attached to L₀, and the * end is attached to another fragment in D;
or 2) Yj₄ is 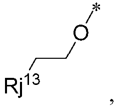
Rj¹³, Rj¹¹, Rj¹², Rj²¹, Rj²², Rj³, Rj⁴, Rj⁵, Rj⁶¹, Y₁, Y₂, Yj₃, and Lj are as defined in any one of claims 1-17 or in this claim, and the * end is attached to another fragment in D;
preferably, Yj₄ is 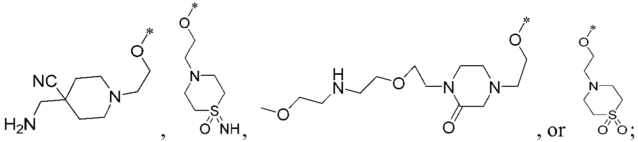
more preferably, Yj₄ is 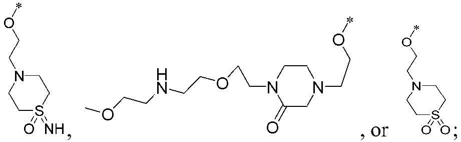
further preferably, Yj₄ is 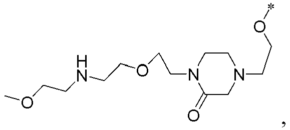
wherein the * end is attached to another fragment in D;
preferably, Rj⁴ is C₃₋₆ cycloalkyl or 3- to 8-membered heterocyclyl, wherein the C₃₋₆ cycloalkyl or 3- to 8-membered heterocyclyl is optionally substituted with one or more substituents selected from hydroxy, methyl, - C₁₋₄ alkylene-OH, and -C(O)-NH(C₁₋₄ alkylene-OH);
preferably, Rj⁴ is cyclopropyl, cyclobutyl, cyclopentyl, or 5- to 8-membered bicyclic heterocyclyl, wherein the cyclopropyl, cyclobutyl, cyclopentyl, or 5- to 8-membered bicyclic heterocyclyl is optionally substituted with 1, 2, or 3 substituents selected from hydroxy, methyl, -CH₂OH, and -C(O)-NH(CH₂CH₂OH);
preferably, Rj⁴ is 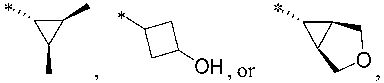
wherein the * end is attached to another fragment in D;
preferably, Lj is 5- or 6-membered heteroaryl;
preferably, Lj is oxazolyl;
preferably, Rj⁵ is C₁₋₄ alkyl;
preferably, Rj⁵ is ethyl;
when L₀ is attached to Yj₄ or Rj⁴, any hydrogen atom on Yj₄ or Rj⁴ is replaced by L₀, or any N atom on Yj₄ or Rj⁴ is quaternized, wherein Yj₄ and Rj⁴ are as defined in any one of claims 2-17 or in this claim;
when L₀ is attached to Yj₄, preferably, Yj₄ is 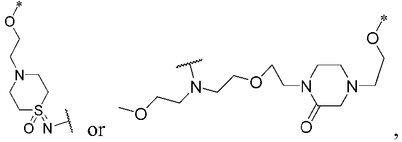 wherein the 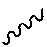 end is attached to L₀, and the * end is attached to another fragment in D;
when L₀ is attached to Rj⁴, preferably, Rj⁴ is 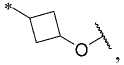 wherein the 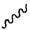 end is attached to L₀, and the * end is attached to another fragment in D;
or 3) Yj₄ is 3- to 8-membered monocyclic heterocyclyl, 7- to 12-membered bicyclic heterocyclyl, or phenyl, wherein the 3- to 8-membered monocyclic heterocyclyl, 7- to 12-membered bicyclic heterocyclyl, or phenyl is optionally substituted with one or more substituents selected from C₁₋₆ alkyl, -C₁₋₄ alkylene-hydroxy, and -C₁₋₄ alkylene-3- to 8-membered monocyclic heterocyclyl;
Rj¹¹, Rj¹², Rj²¹, Rj²², Rj³, Rj⁴, Rj⁵, Rj⁶¹, Y₁, Y₂, Yj₃, and Lj are as defined in any one of claims 1-17 or in this claim;
preferably, Yj₄ is as defined in claim 12;
preferably, Rj⁴ is C₃₋₆ cycloalkyl or 3- to 8-membered heterocyclyl, wherein the C₃₋₆ cycloalkyl or 3- to 8-membered heterocyclyl is optionally substituted with one or more substituents selected from hydroxy, methyl, - C₁₋₄ alkylene-OH, and -C(O)-NH(C₁₋₄ alkylene-OH);
preferably, Rj⁴ is cyclopropyl, cyclobutyl, or cyclopentyl, wherein the cyclopropyl, cyclobutyl, or cyclopentyl is optionally substituted with 1, 2, or 3 substituents selected from hydroxy, methyl, and -CH₂OH;
preferably, Rj⁴ is 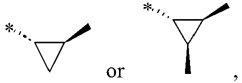 wherein the * end is attached to another fragment in D;
when L₀ is attached to Yj₄, Rj⁵, Lj, or Rj²¹, any hydrogen atom on Yj₄, Rj⁵, Lj, or Rj²¹ is replaced by L₀, or any N atom on Yj₄, Rj⁵, Lj, or Rj²¹ is quaternized, wherein Yj₄, Rj⁵, Lj, and Rj²¹ are as defined in any one of claims 2-17 or in this claim;
when L₀ is attached to Yj₄, preferably, Yj₄ is 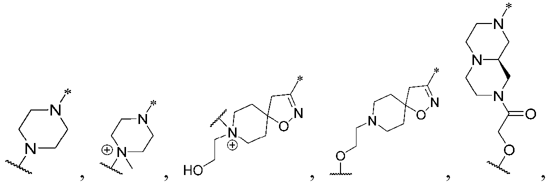 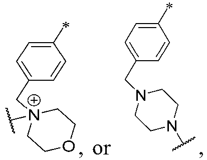 and more preferably, Yj₄ is 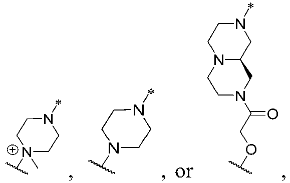 wherein the 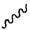 end is attached to L₀, and the * end is attached to another fragment in D;
when L₀ is attached to Rj⁵, preferably, Rj⁵ is 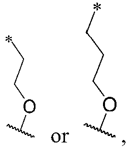 wherein the 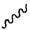 end is attached to L₀, and the * end is attached to another fragment in D;
when L₀ is attached to Lj, preferably, Lj is 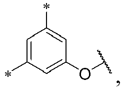 wherein the 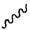 end is attached to L₀, and the * end is attached to another fragment in D;
when L₀ is attached to Rj²¹, preferably, Rj²¹ is 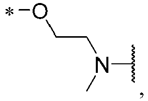 wherein the 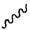 end is attached to L₀, and the * end is attached to another fragment in D.

19. The antibody-drug conjugate according to any one of claims 1-18, wherein 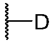 is a compound represented by any one of formula (AIA-1) to formula (AIA-8), or a stable deuterated derivative thereof, a stereoisomer thereof, or a pharmaceutically acceptable salt thereof: 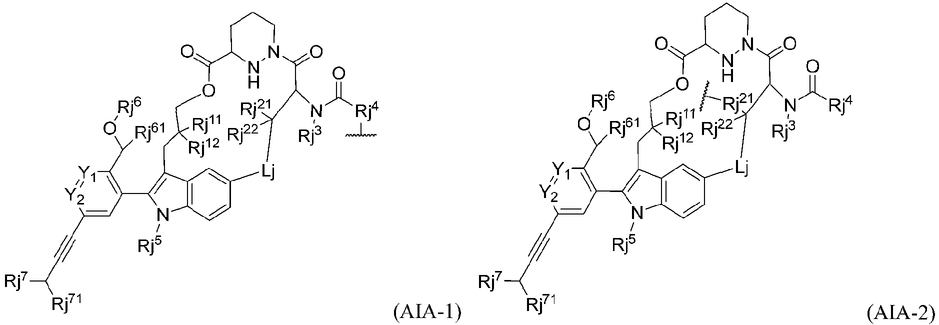 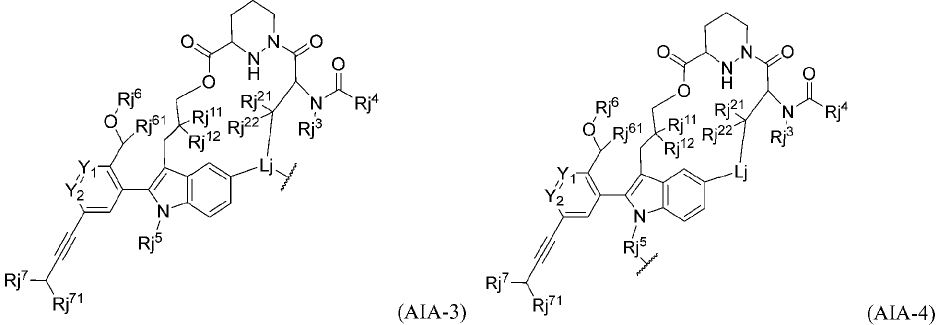 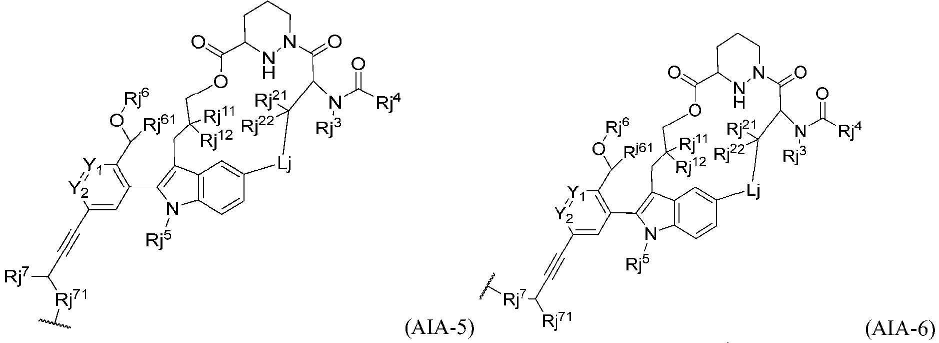 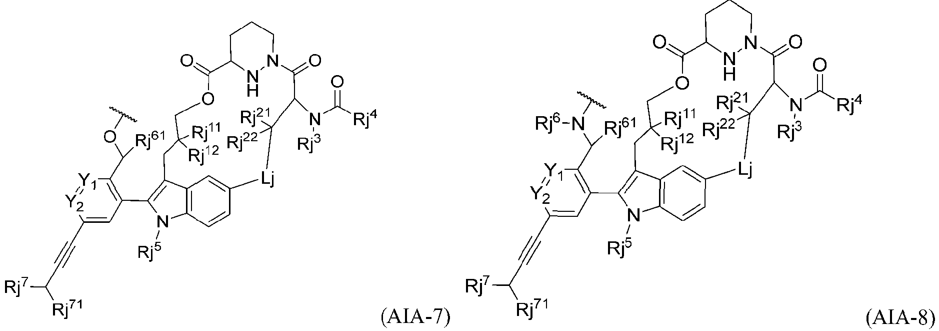 wherein in the formulas,
Rj¹¹, Rj¹², Rj²¹, Rj²², Y₁, Y₂, Rj⁵, Rj⁴, Rj³, Rj⁶, Rj⁶¹, Rj⁷, Rj⁷¹, and Lj are as defined in any one of claims 1-18, and the 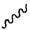 end is attached to L₀.

20. The antibody-drug conjugate according to any one of claims 1-19, wherein 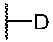 is a compound represented by any one of formula (AIA-1-1) to formula (AIA-1-7), formula (AIA-2-1) to formula (AIA-2-8), formula (AIA-3-1) to formula (AIA-3-5), and formula (AIA-4-1) to formula (AIA-4-6), or a stable deuterated derivative thereof, a stereoisomer thereof, or a pharmaceutically acceptable salt thereof: 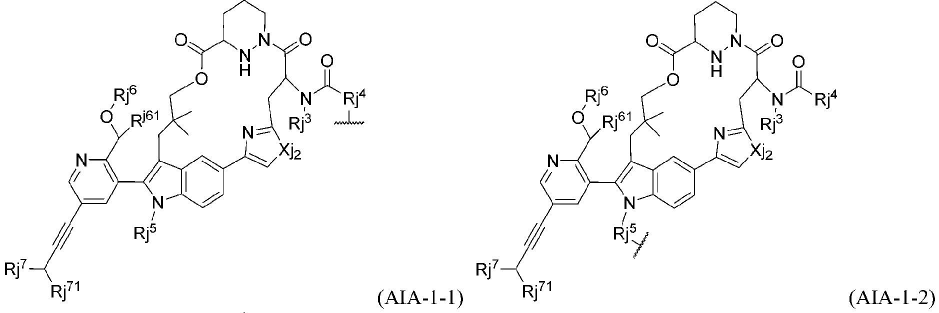 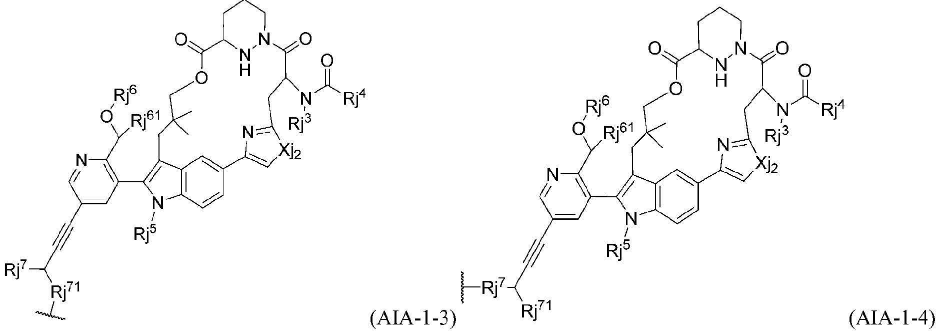 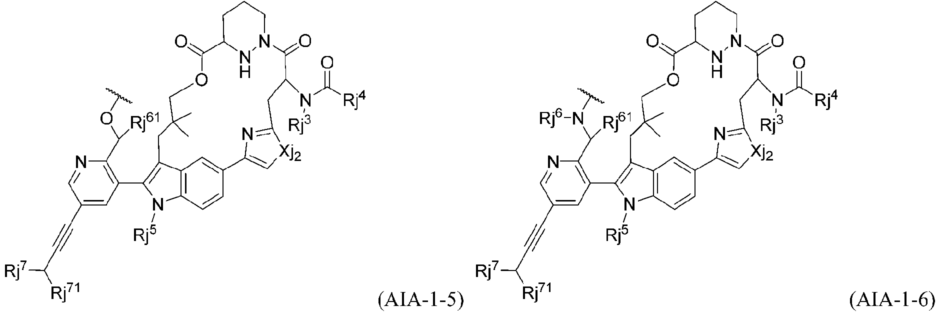 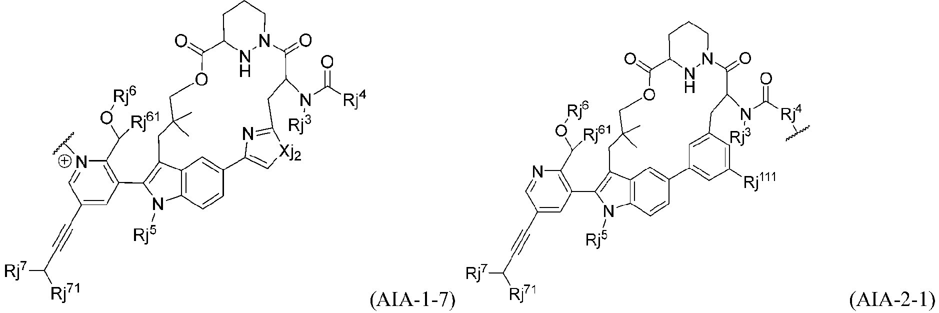 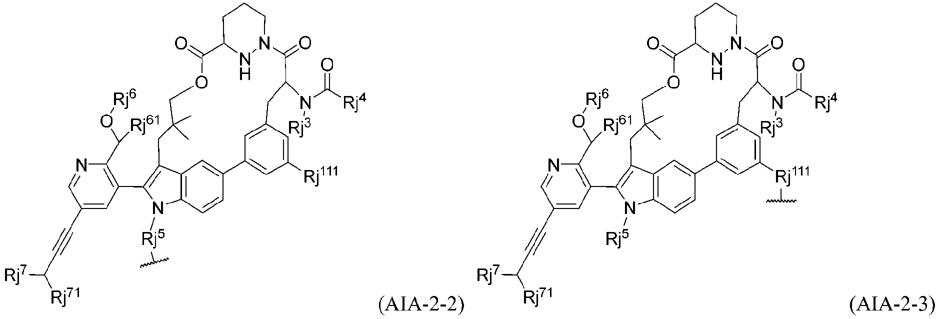 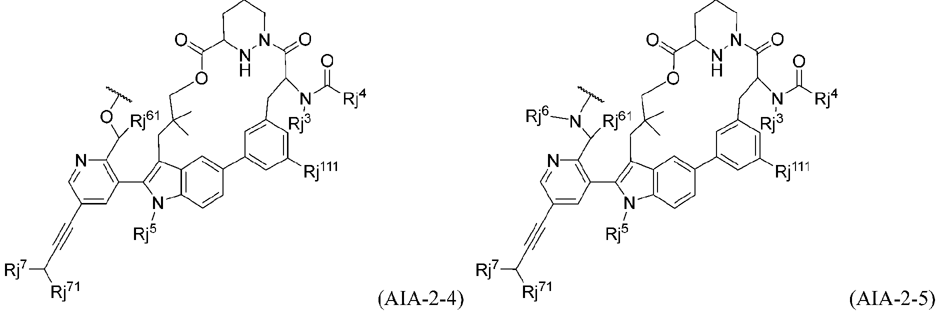 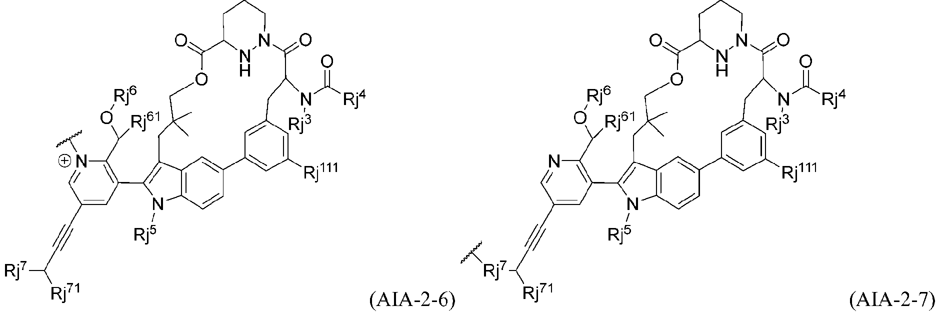 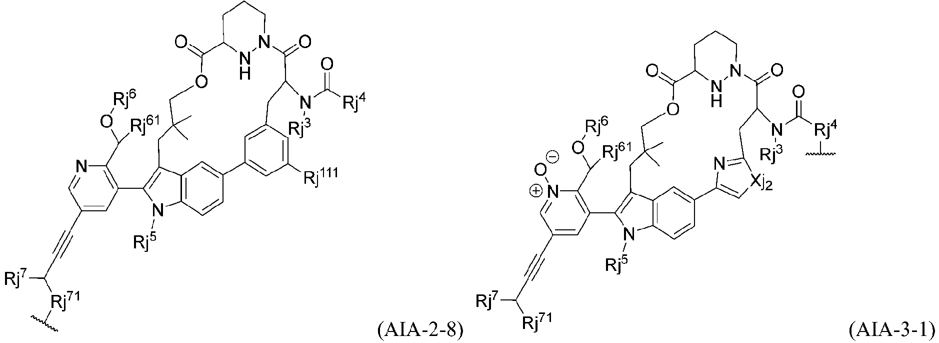 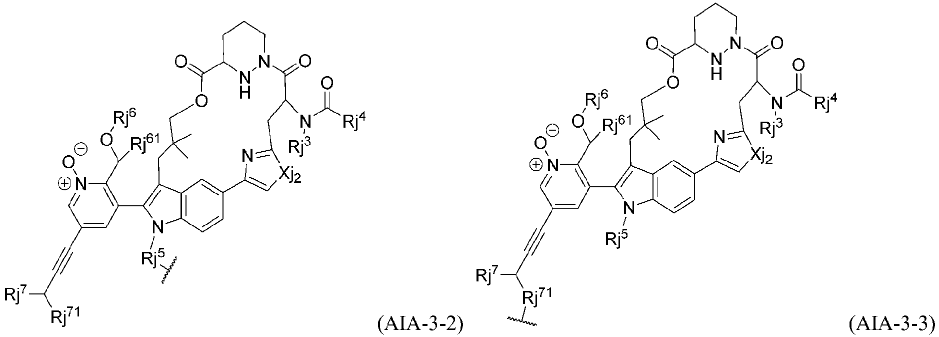 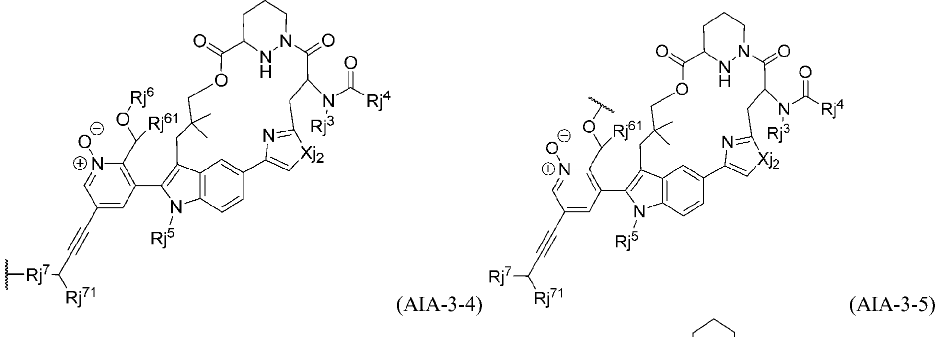 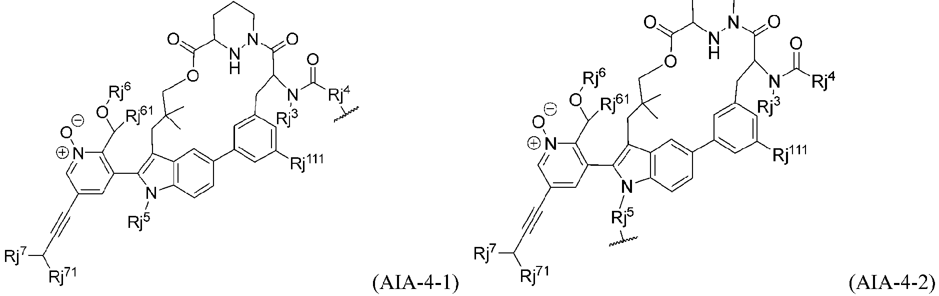 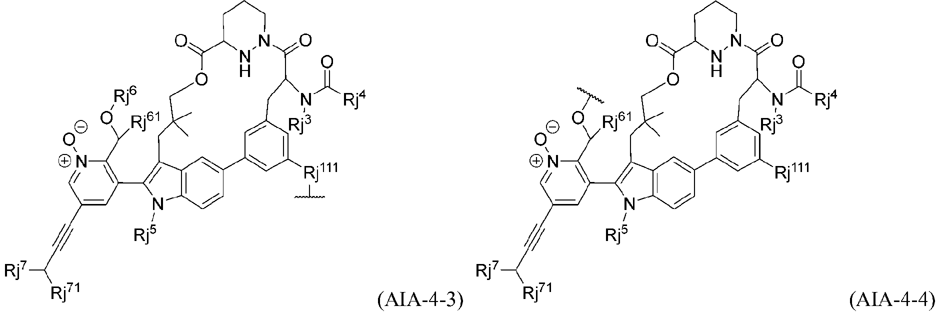 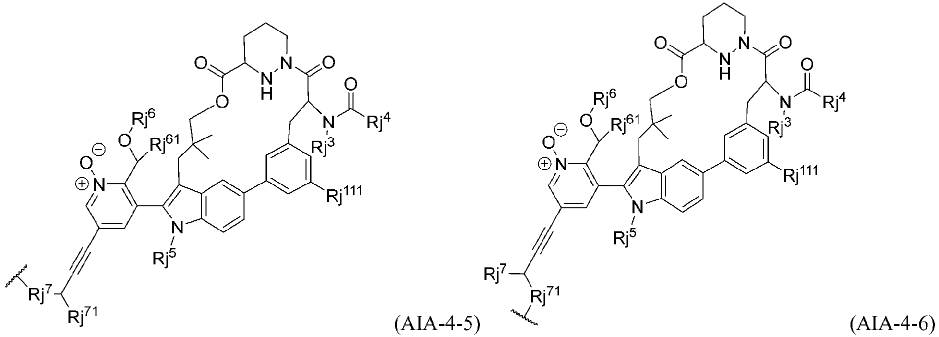
in the formulas, Rj³, Rj⁴, Rj⁵, Rj⁶, Rj⁶¹, Rj⁷, and Rj⁷¹ are as defined in any one of claims 1-19;
Xj₂ is S or O; Rj¹¹¹ is amino, hydroxy, or hydrogen;
the 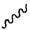 end is attached to L₀;
preferably,
1) 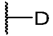 is a compound represented by formula (AIA-1a1-1) to formula (AIA-1a1-4) or formula (AIA-1b1-1) to formula (AIA-1b1-4), or a stable deuterated derivative thereof, a stereoisomer thereof, or a pharmaceutically acceptable salt thereof: 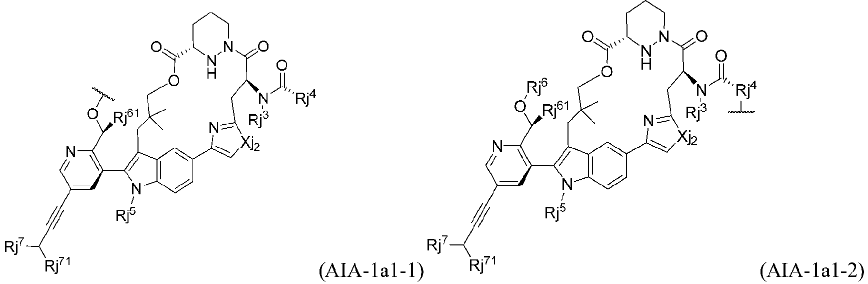 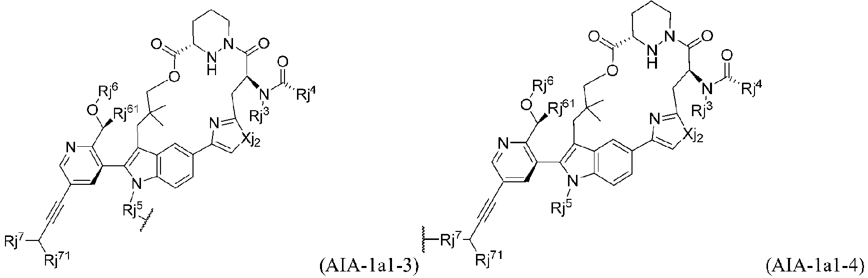 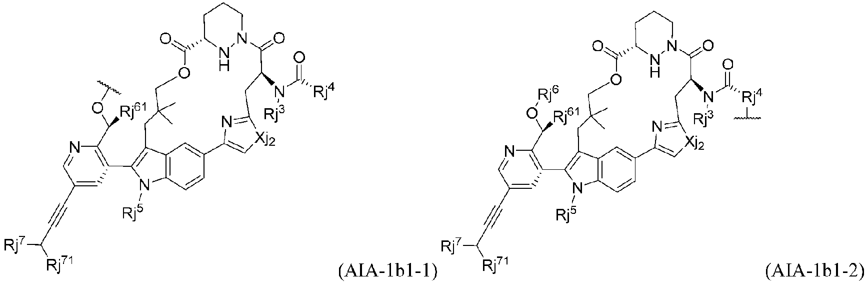 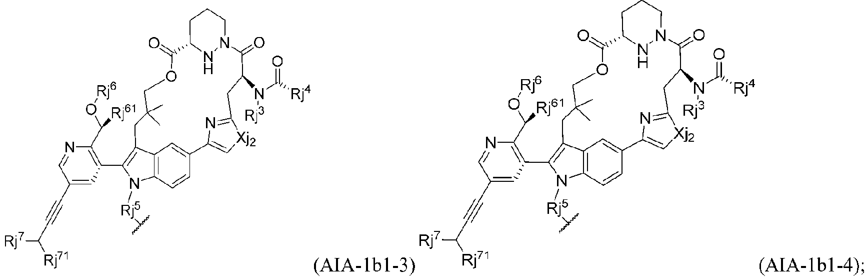
wherein in the formulas,
Rj³, Rj⁴, Rj⁵, Rj⁶, Rj⁶¹, Rj⁷, and Rj⁷¹ are as defined in any one of claims 1-19;
Xj₂ is S or O;
the 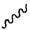 end is attached to L₀;
or 2) 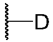 is a compound represented by any one of formula (AIA-2a1-1) to formula (AIA-2a1-5) and formula (AIA-2b1-1) to formula (AIA-2b1-3), or a stable deuterated derivative thereof, a stereoisomer thereof, or a pharmaceutically acceptable salt thereof: 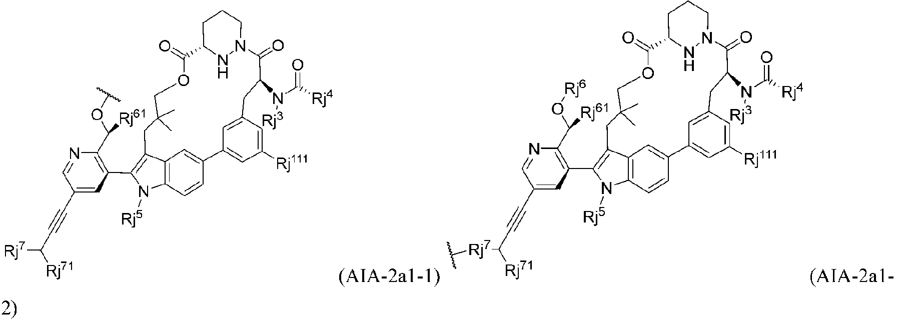 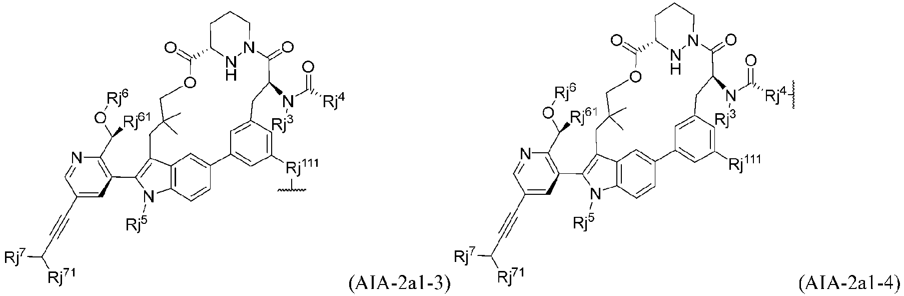 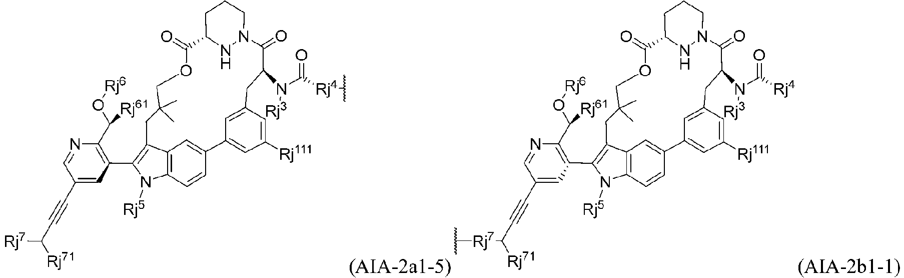 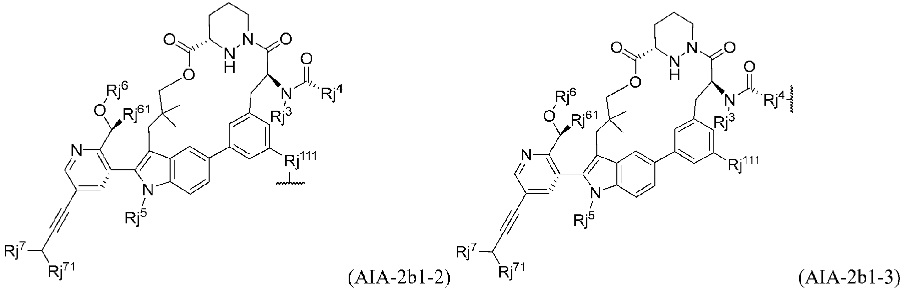
wherein in the formulas,
Rj³, Rj⁴, Rj⁵, Rj⁶, Rj⁶¹, Rj⁷, and Rj⁷¹ are as defined in any one of claims 1-19;
Rj¹¹¹ is amino or hydroxy;
the 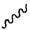 end is attached to L₀.

21. The antibody-drug conjugate according to any one of claims 1-18, wherein 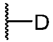 is a compound represented by any one of formula (AI-1) to formula (AI-6), or a stable deuterated derivative thereof, a stereoisomer thereof, or a pharmaceutically acceptable salt thereof: 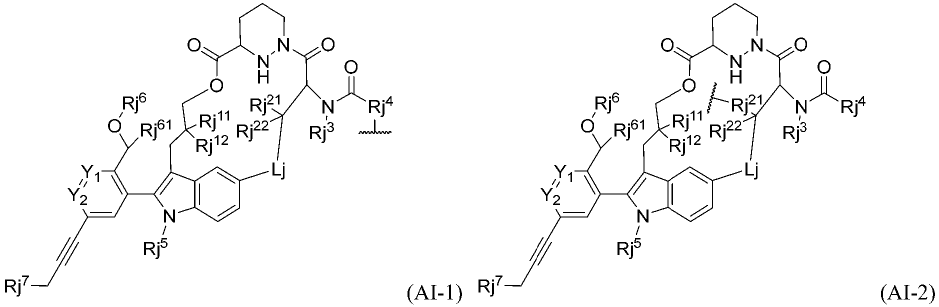 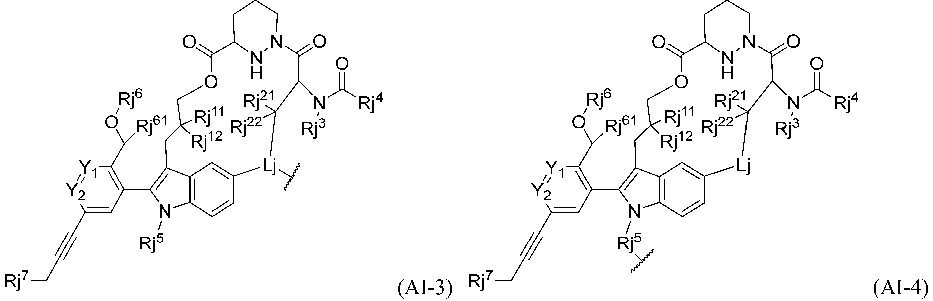 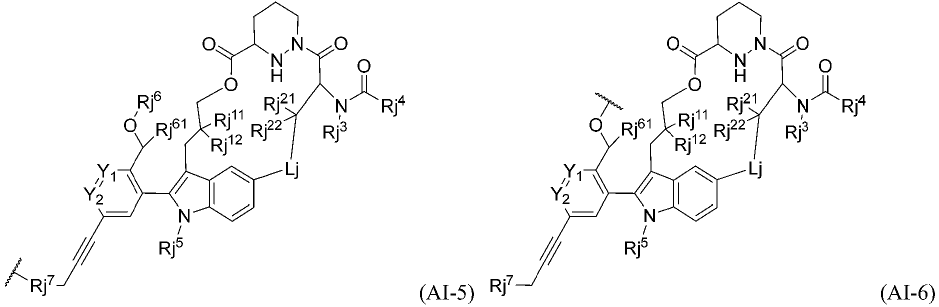 wherein in the formulas,
Rj¹¹, Rj¹², Rj²¹, Rj²², Rj³, Rj⁴, Rj⁵, Rj⁶, Rj⁶¹, Rj⁷, Y₁, Y₂, and Lj are as defined in any one of claims 1-18, and the 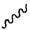 end is attached to L₀;
preferably, 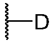 is a compound represented by any one of formula (AI-1-1) to formula (AI-1-4), formula (AI-2-1) to formula (AI-2-4), formula (Al-3-1) to formula (AI-3-4), and formula (AI-4-1) to formula (AI-4-4), or a stable deuterated derivative thereof, a stereoisomer thereof, or a pharmaceutically acceptable salt thereof: 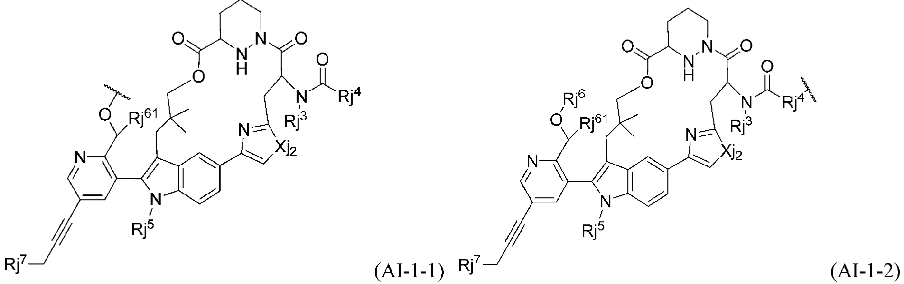 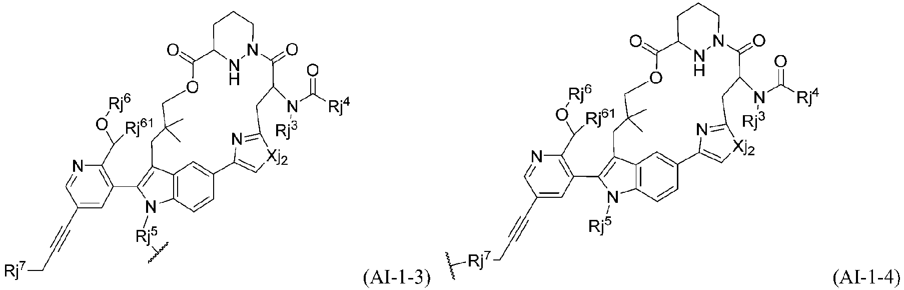 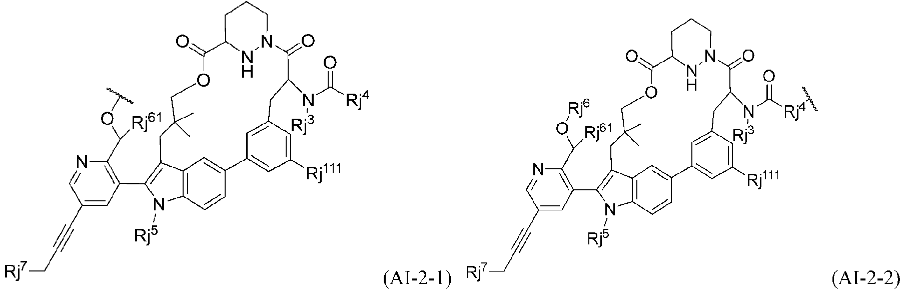 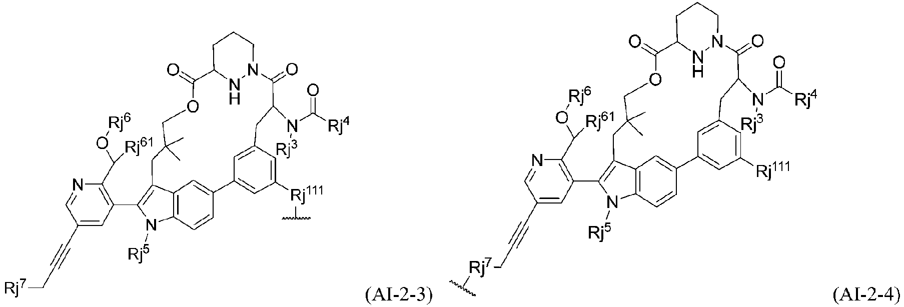 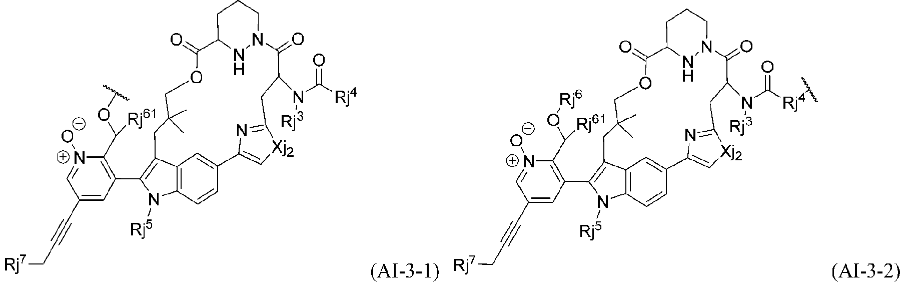 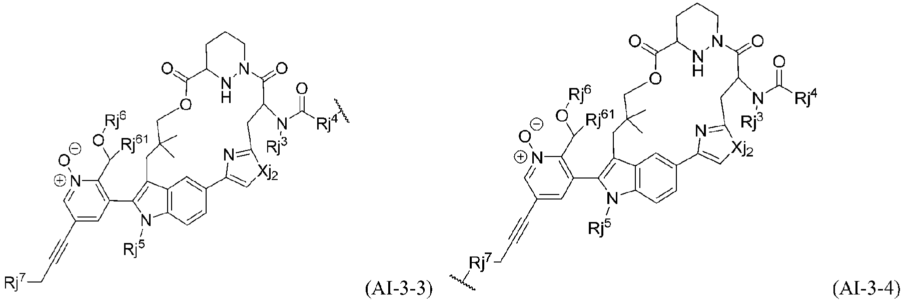 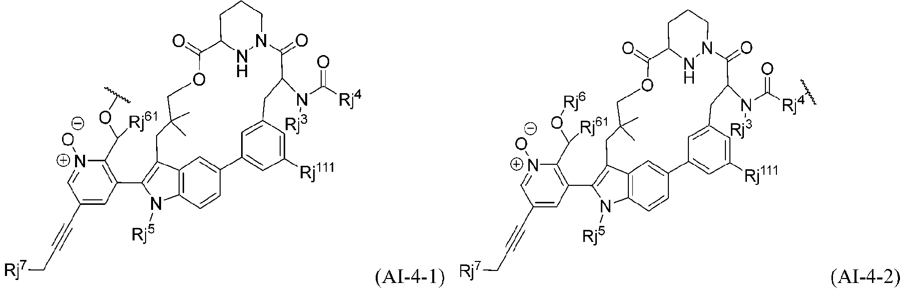 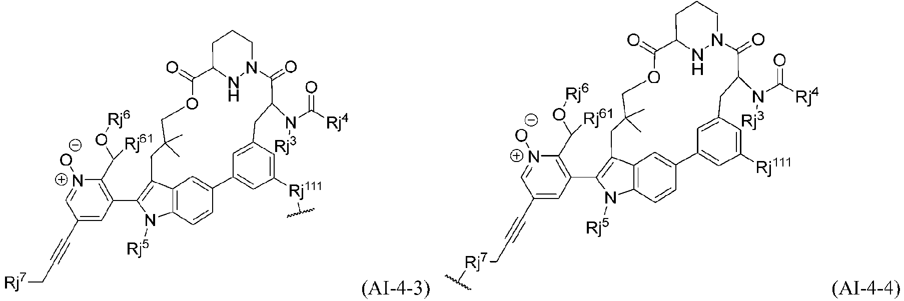
in the formulas, Rj³, Rj⁴, Rj⁵, Rj⁶, Rj⁶¹, and Rj⁷ are as defined in any one of claims 1-18;
Xj₂ is S or O; Rj¹¹¹ is amino or hydroxy;
the 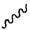 end is attached to L₀.

22. The antibody-drug conjugate according to any one of claims 1-18, wherein 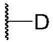 is a compound represented by any one of formula (AIIA-1-1) to formula (AIIA-1-4), or a stable deuterated derivative thereof, a stereoisomer thereof, or a pharmaceutically acceptable salt thereof: 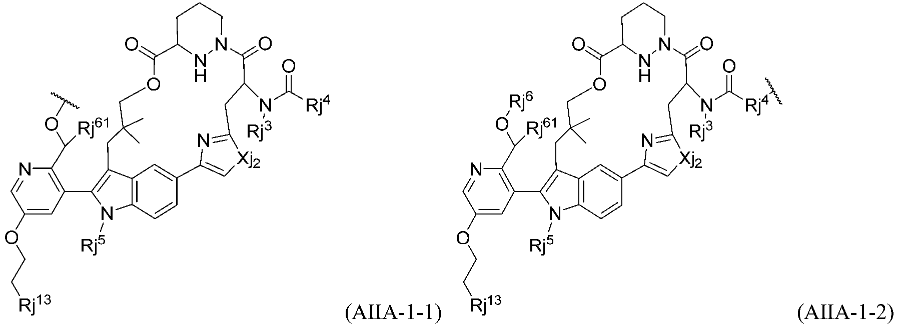 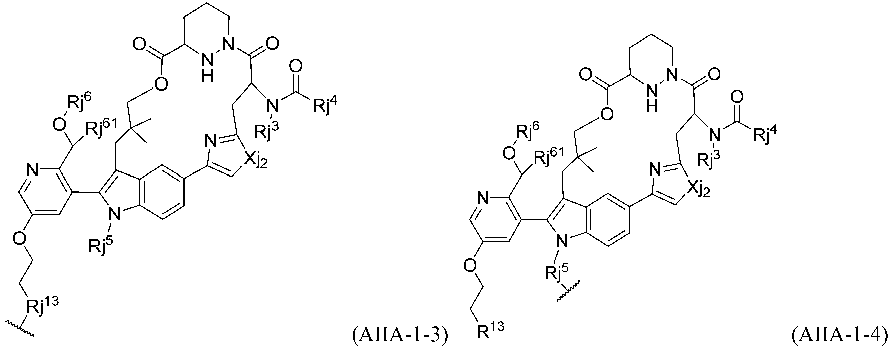
in the formulas, Rj³, Rj⁴, Rj⁵, Rj⁶, Rj⁶¹, and Rj¹³ are as defined in any one of claims 1-18;
Xj₂ is S or O;
the 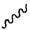 end is attached to L₀;
preferably, 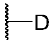 is a compound represented by any one of formula (AIIA-1a-1) to formula (AIIA-1a-4) and formula (AIIA-1b-1) to formula (AIIA-1b-4), or a stable deuterated derivative thereof, a stereoisomer thereof, or a pharmaceutically acceptable salt thereof: 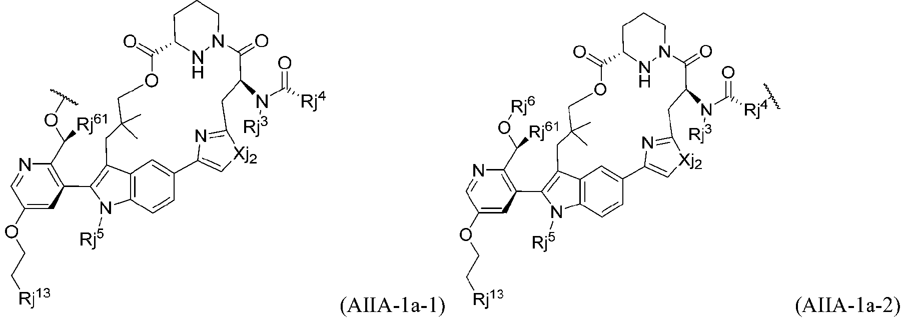 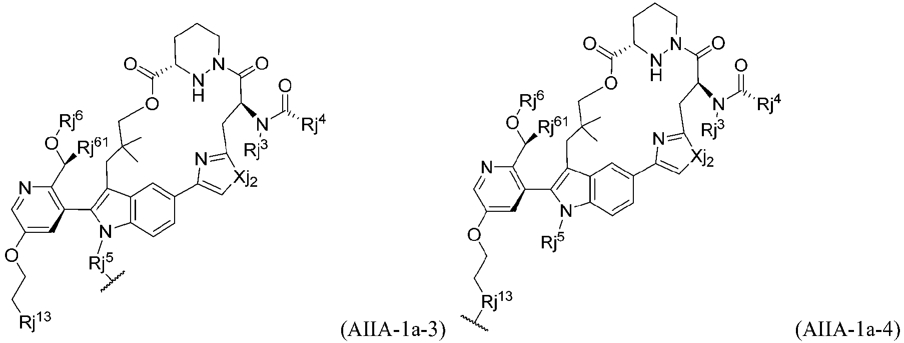 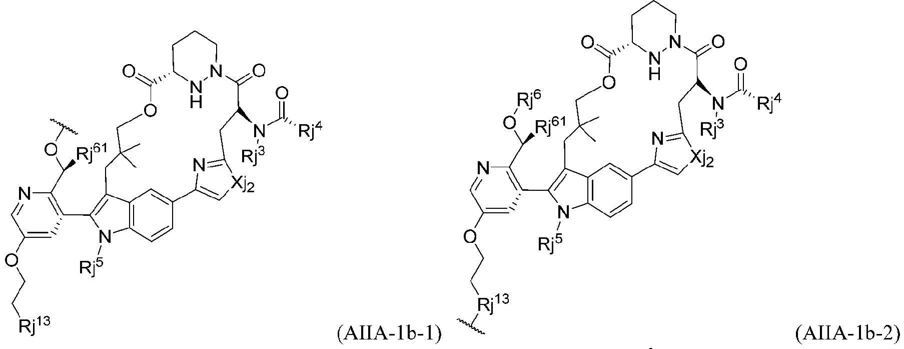 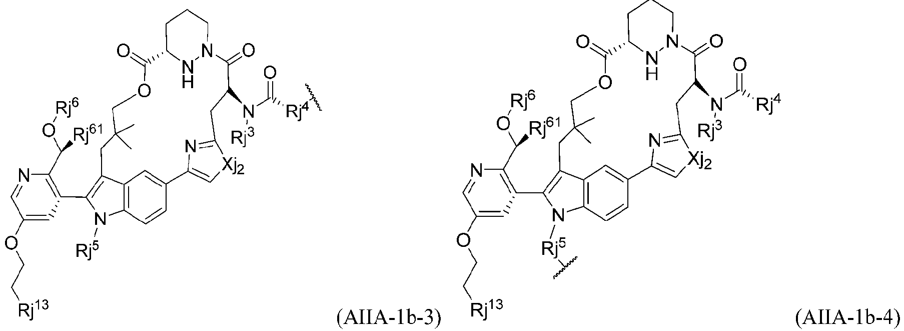
in the formulas, Rj³, Rj⁴, Rj⁵, Rj⁶, Rj⁶¹, and Rj¹³ are as defined in any one of claims 1-18;
Xj₂ is S or O;
the 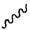 end is attached to L₀.

23. The antibody-drug conjugate according to any one of claims 1-22, wherein 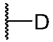 is selected from structures shown in Table 8;
more preferably, 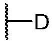 is selected from D10, D11, D166, D168, D169-1, D170, D187, D187-A, D192-A, D192-C, D22, D282-A, D283, D284, D284-A, D7-2, D285, D285-A, D285-B, D286, D286-A, D286-B, D287, D288, D288-A, D289, D290, D291, D292, D293, D294, D295, D296, D297, D298, D299, D299-A, D3, D300, D301, D302, D303, D304, D305, D305-A, D306, D307, D308, D309, D31, D310, D311, D34, D35-A, D38-1, D4, D45-1, D47, D48, D52, D54, D56, D57-A, D6, D61, D63, D64-1, D67-A, and D88;
further preferably, 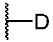 is selected from D10, D11, D166, D168, D169-1, D170, D22, D3, D31, D34, D35-A, D38-1, D4, D45-1, D47, D48, D52, D54, D56, D57-A, D6, D61, D63, and D64-1;
still further preferably, 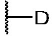 is selected from D31, D34, D35-A, D38-1, D47, D52, D56, D61, and D64-1.

24. The antibody-drug conjugate according to any one of claims 1-23, wherein L₀ is a hydrophilic linker or a non-hydrophilic linker;
preferably, 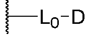 is selected from the structure represented by formula (III-1) or formula (III-2):
-H₂-L₁ₐ-L_{1b}-L_{1c}-L_{1d}-D (III-1)
-H₂-L₁ₐ-L_{1b}-L_{1d}(-L_{1c}-C(=O)-CH₃)-D (III-2) 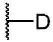
is as defined in any one of claims 1-23;
H₂ is an antibody-linking unit attached to Ab;
L₁ₐ is a bond or a derivative unit;
L_{1b} is a bond or a hydrophilic derivative unit;
L_{1c} is a bond or an amino acid unit;
L_{1d} is a bond or a drug-linking unit;
at least one of L₁ₐ, L_{1b}, L_{1c}, and L_{1d} is not a bond;
H₂, L₁ₐ, L_{1b}, L_{1c}, and L_{1d} are each independently and optionally substituted with a hydrophilic group;
preferably, 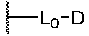 is selected from the structure represented by formula (III-1);
preferably, the hydrophilic group is -1- to 300-membered linear or branched heteroalkyl-R_{end}, wherein the 1- to 300-membered linear or branched heteroalkyl means that 1-100 carbon atoms at any position in the 1- to 300-membered linear or branched alkyl may be replaced by a heteroatom selected from N, O, and S, and the remaining carbon atoms may be optionally substituted with a group selected from deuterium, C₁₋₆ alkyl, oxo (=O), and thio (=S); R_{end} is H, deuterium, hydroxy, carboxyl, amino, an ester group, amido, alkoxy, or substituted amino;
preferably, the hydrophilic group consists of one or more groups selected from: N, NH, N(CH₃), O, C(O), NHC(O), N(CH₃)C(O), C₁₋₆ alkyl, polyethylene glycol, polysarcosine, glucose, glucuronic acid, 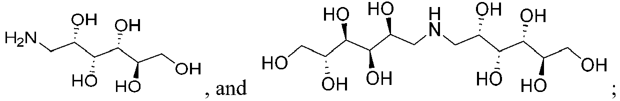
preferably, the hydrophilic group is selected from: 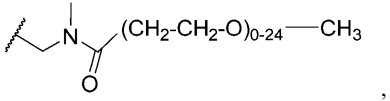 polysarcosine, 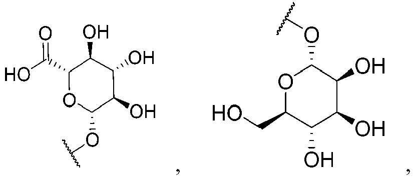 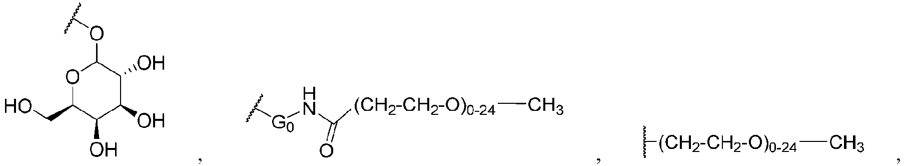 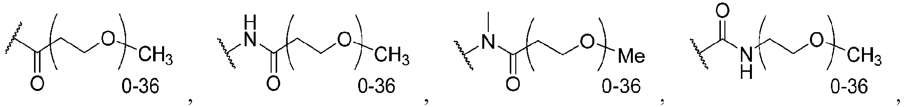 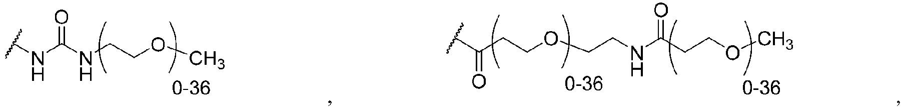 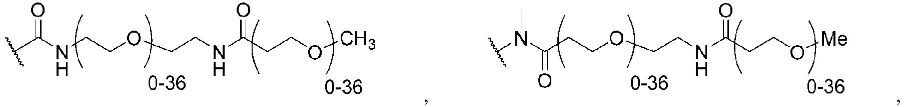 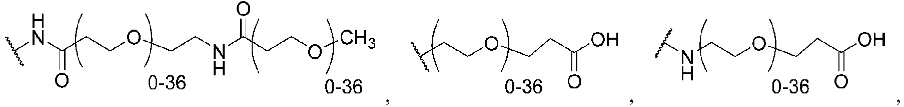 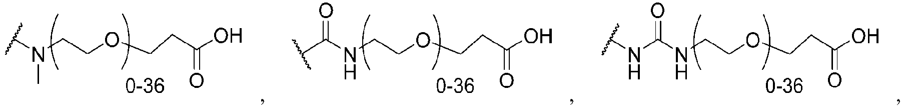 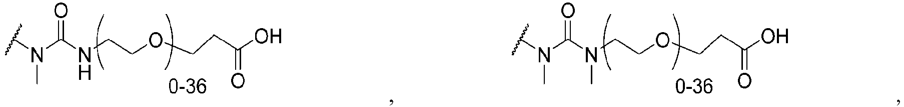 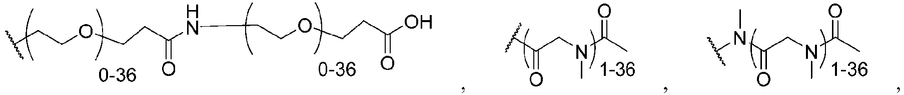 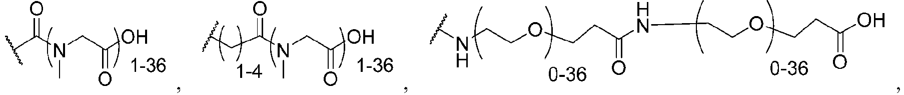 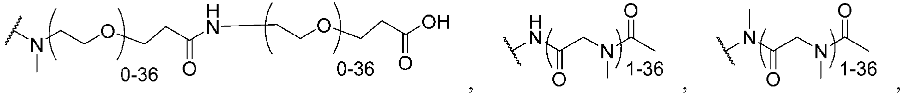 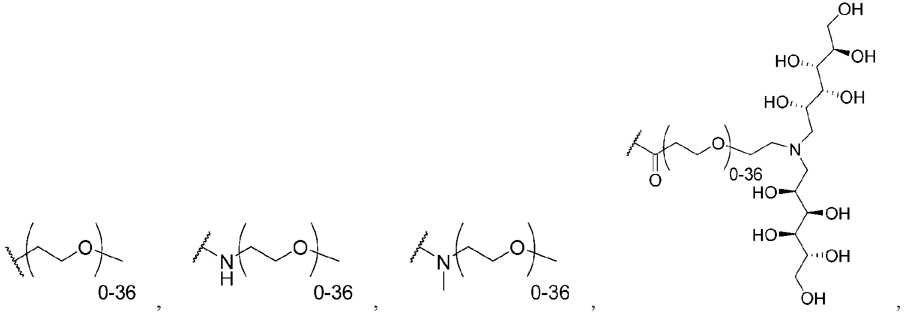 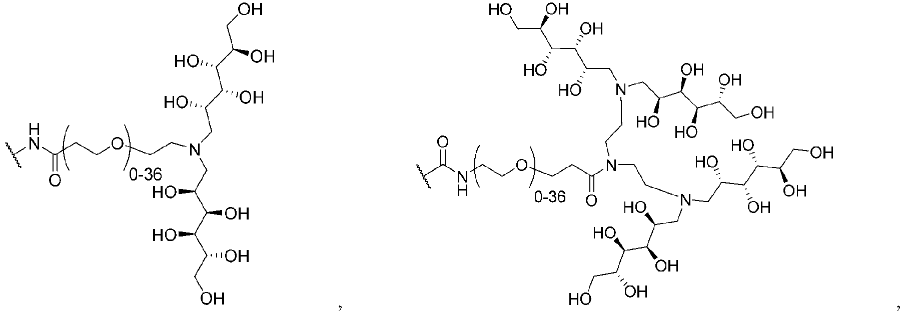 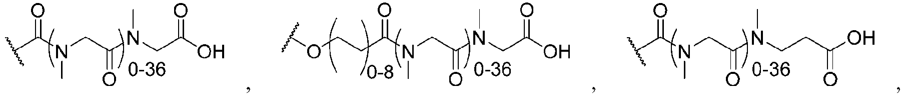 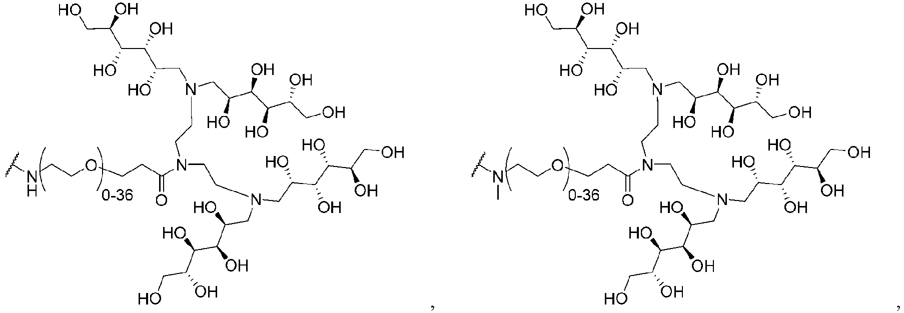 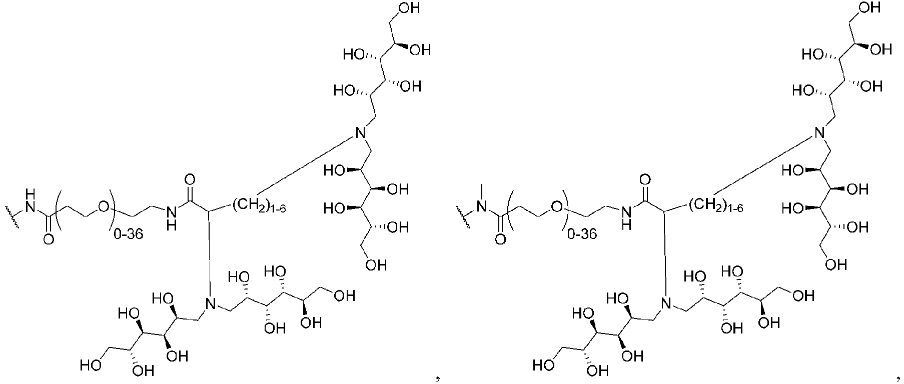 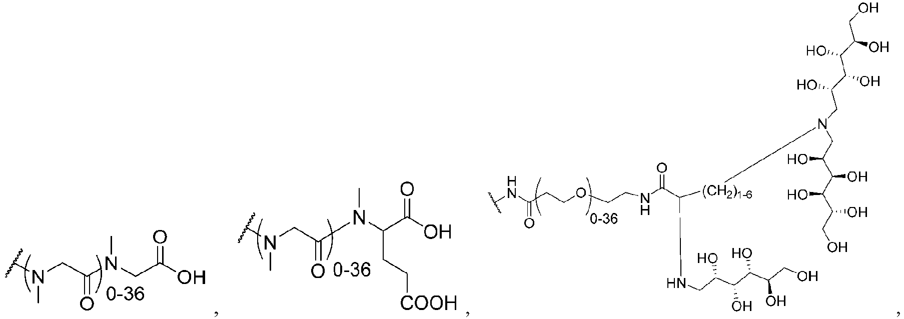 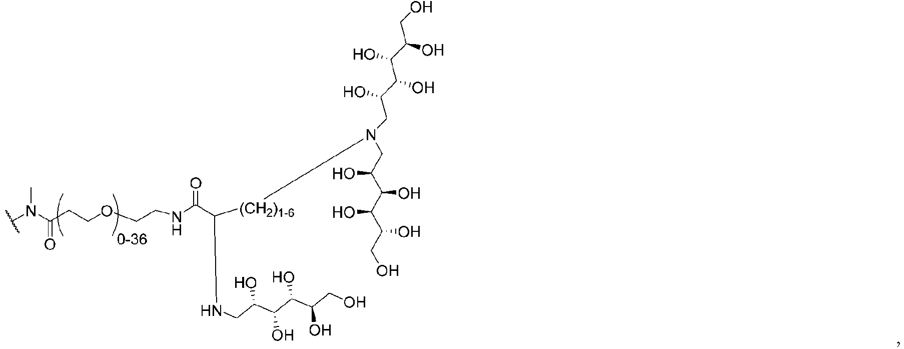 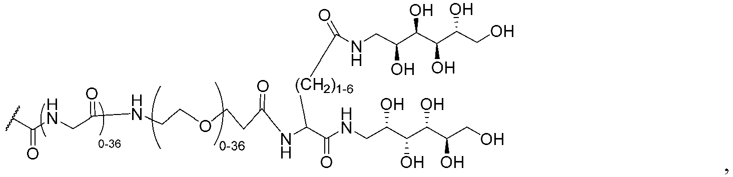 and 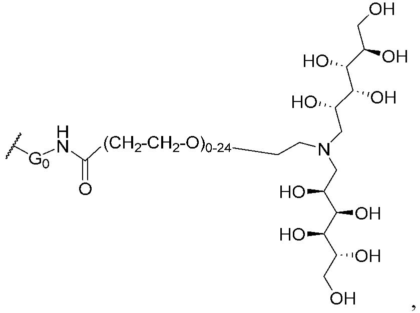
wherein G₀ is C₀₋₈ hydrocarbyl, C₀₋₈ heteroalkyl, -NH-, O, or S;
preferably, G₀ is C₀₋₈ hydrocarbyl or C₀₋₈ heteroalkyl;
preferably, G₀ is a bond, C₁₋₄ alkyl, or C₁₋₄ heteroalkyl;
preferably, G₀ is a bond or n-butyl;
preferably, the hydrophilic group is selected from:

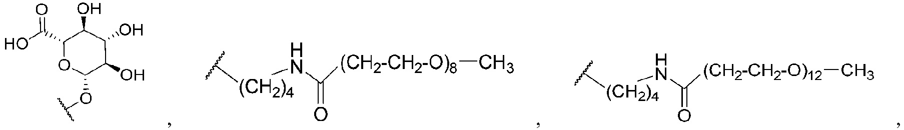

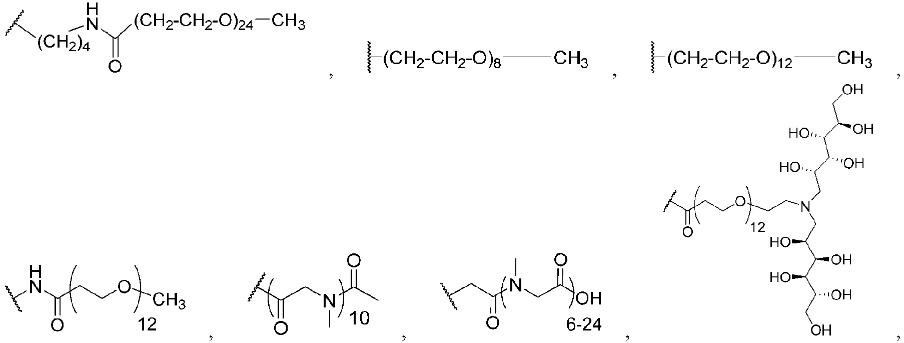

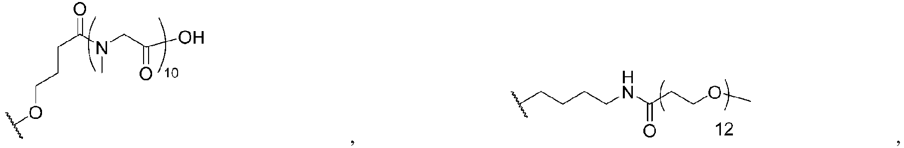

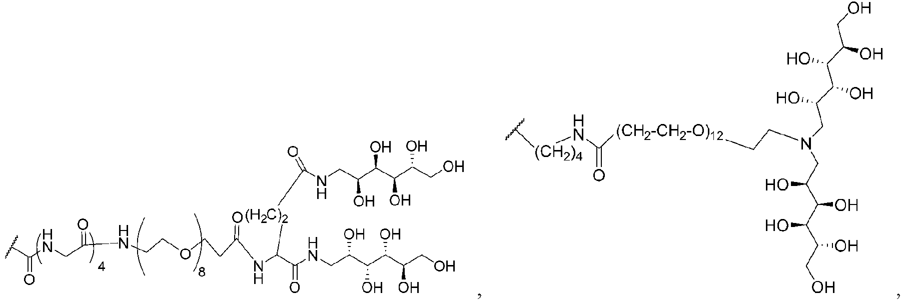

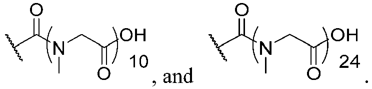

25. The antibody-drug conjugate according to any one of claims 1-24, wherein H₂ is selected from the group consisting of: 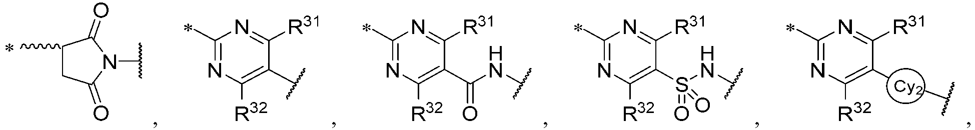 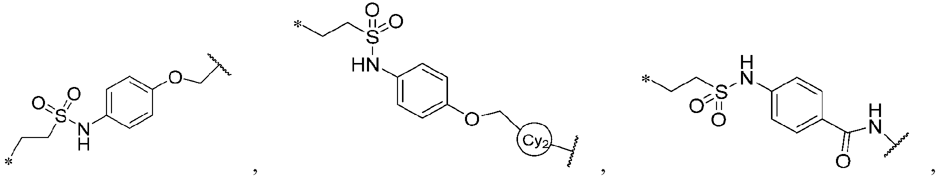 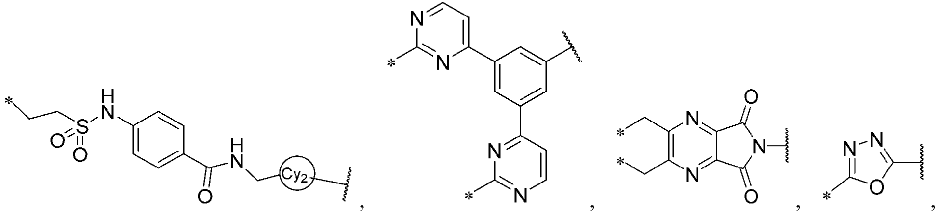 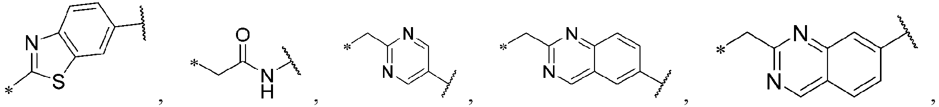 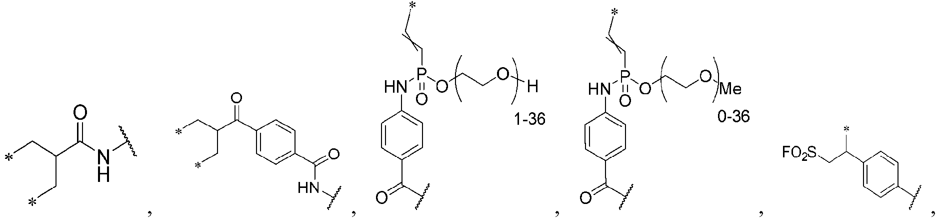 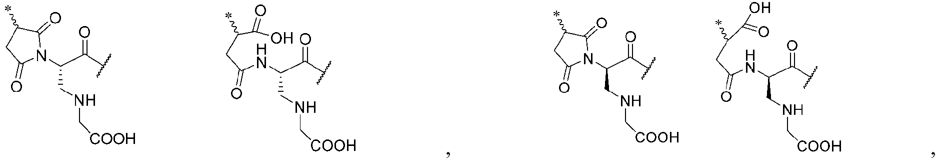 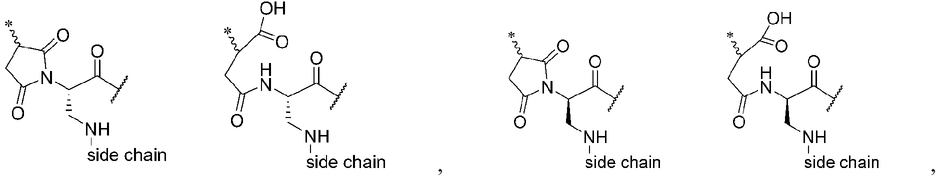 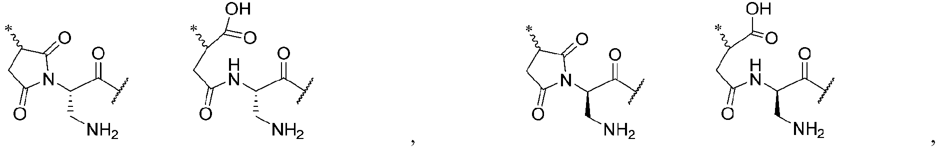 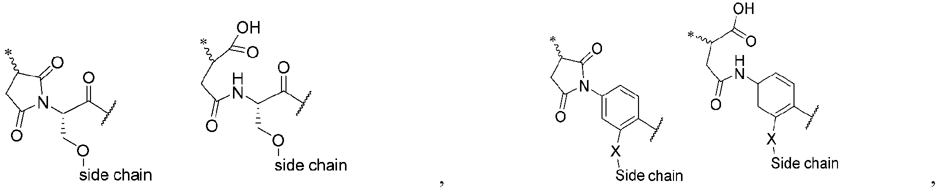 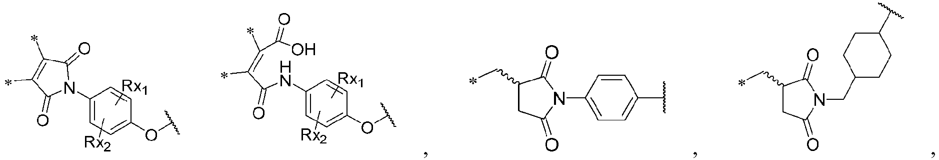 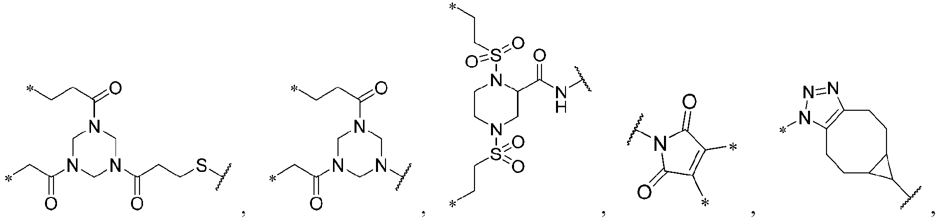 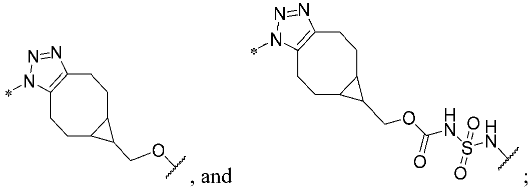 wherein:
the * end is attached to the antibody, and the 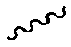 end is attached to L₁ₐ;
X is -O-(CH₂)₀₋₆-, -N(CH₃)-, -O-, -CONH-, or -CON(CH₃)-;
Rₓ₁ and Rₓ₂ are each independently hydrogen, C₁₋₆ alkyl, halogen, deuterium, C₁₋₆ alkoxy, C₁₋₆ alkylene-NH-, or (C₁₋₆ alkyl)₂-N-;
R³¹ is hydrogen, deuterium, halogen, cyano, or C₁₋₄ alkyl;
R³² is hydrogen, deuterium, halogen, cyano, or C₁₋₄ alkyl;
Cy2 is 5- to 10-membered heteroaryl, C₆₋₁₀ aryl, 3- to 10-membered heterocyclyl, or C₃₋₁₀ cycloalkyl;
the side chain is a hydrophilic group, wherein the hydrophilic group is as defined in claim 24;
preferably, Rₓ₁ and Rₓ₂ are each independently hydrogen, C₁₋₄ alkyl, halogen, deuterium, C₁₋₄ alkoxy, C₁₋₄ alkylene-NH-, or (C₁₋₄ alkyl)₂-N-;
preferably, Rₓ₁ and Rₓ₂ are each independently hydrogen, methyl, halogen, deuterium, methoxy, methyl-NH-, or (methyl)₂-N-;
preferably, R³¹ is hydrogen, deuterium, halogen, cyano, or methyl;
preferably, R³¹ is hydrogen;
preferably, R³² is hydrogen, deuterium, halogen, cyano, or methyl;
preferably, R³² is hydrogen;
preferably, Cy2 is 5- to 10-membered heteroaryl, C₆₋₁₀ aryl, 4- to 8-membered heterocyclyl, or C₃₋₆ cycloalkyl;
preferably, Cy2 is pyrrolyl, imidazolyl, pyridinyl, pyrimidinyl, phenyl, pyrrolidinyl, piperidinyl, piperazinyl, cyclopropyl, cyclobutyl, or cyclopentyl;
preferably, H₂ is selected from the group consisting of: 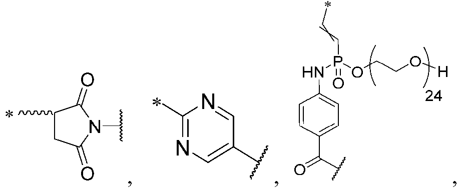 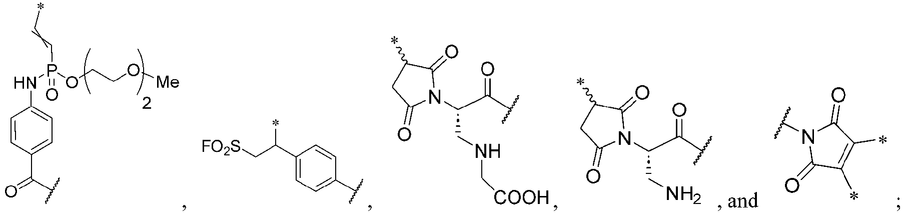
still further preferably, H₂ is 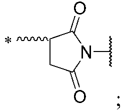
wherein the * end is attached to the antibody, and the 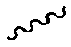 end is attached to L₁ₐ.

26. The antibody-drug conjugate according to any one of claims 1-25, wherein L₁ₐ is a bond or selected from C₁₋₆ alkyl, C₁₋₆ alkylene-C(=O), C₁₋₆ alkylene-C(=O)NH, C₁₋₆ heteroalkyl, C₂₋₆ alkenyl, C₂₋₆ alkynyl, C₃₋₈ cycloalkyl, 4- to 10-membered heterocyclyl, C₆₋₁₀ aryl, 5- to 10-membered heteroaryl, C₁₋₄ alkylene-C₃₋₈ cycloalkyl, C₁₋₄ alkylene-4- to 10-membered heterocyclyl, C₁₋₄ alkylene-C₆₋₁₀ aryl, C₁₋₄ alkylene-5- to 10-membered heteroaryl, C₁₋₆ heteroalkylene-C(=O), C₂₋₆ alkenylene-C(=O), C₂₋₆ alkynylene-C(=O), C₃₋₈ cycloalkyl-C(=O), 4- to 10-membered heterocyclyl-C(=O), C₆₋₁₀ aryl-C(=O), 5- to 10-membered heteroaryl-C(=O), C₁₋₄ alkylene-C₃₋₈ cycloalkyl-C(=O), C₁₋₄ alkylene-4- to 10-membered heterocyclyl-C(=O), C₁₋₄ alkylene-C₆₋₁₀ aryl-C(=O), C₁₋₄ alkylene-5- to 10-membered heteroaryl-C(=O), C₁₋₆ heteroalkylene-C(=O)NH, C₂₋₆ alkenylene-C(=O)NH, C₂₋₆ alkynylene-C(=O)NH, C₃₋₈ cycloalkyl-C(=O)NH, 4- to 10-membered heterocyclyl-C(=O)NH, C₆₋₁₀ aryl-C(=O)NH, 5- to 10-membered heteroaryl-C(=O)NH, C₁₋₄ alkylene-C₃₋₈ cycloalkyl-C(=O)NH, C₁₋₄ alkylene-4-to 10-membered heterocyclyl-C(=O)NH, C₁₋₄ alkylene-C₆₋₁₀ aryl-C(=O)NH, and C₁₋₄ alkylene-5- to 10-membered heteroaryl-C(=O)NH, wherein the C₁₋₆ alkyl, C₁₋₆ alkylene, or C₁₋₄ alkylene is optionally substituted with -C₁₋₄ alkylene-NH₂ or -C₁₋₄ alkylene-NH-C₁₋₄ alkylene-COOH; L₁ₐ is optionally substituted with a hydrophilic group; the hydrophilic group is as defined in claim 24;
preferably, L₁ₐ is a bond, phenyl, 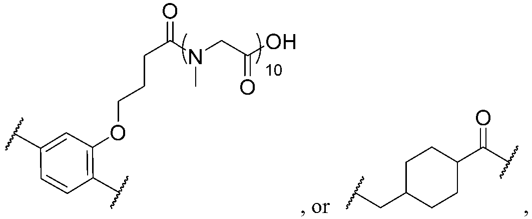 wherein the left
side of 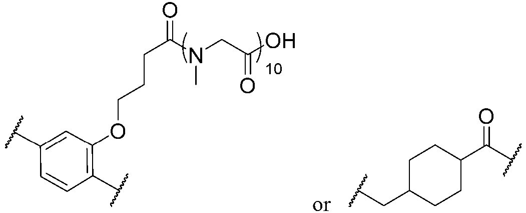 is attached to H₂, and the right side is attached to L_{1b};
further preferably, L₁ₐ is a bond.

27. The antibody-drug conjugate according to any one of claims 1-26, wherein L_{1b} is a bond or selected from: 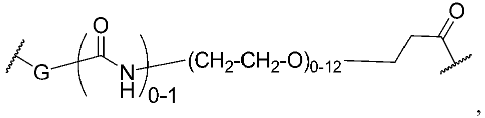 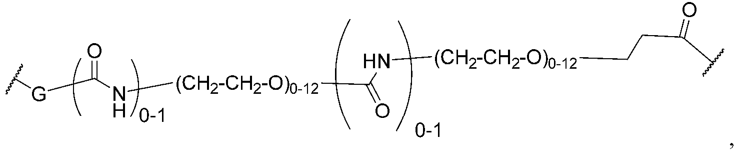 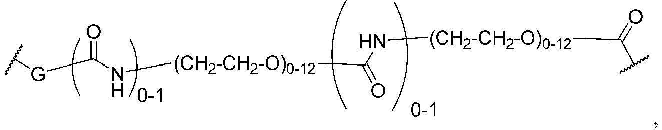 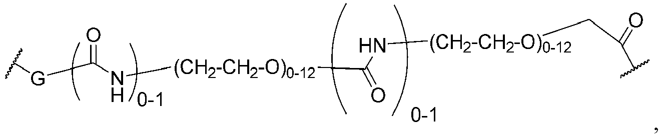 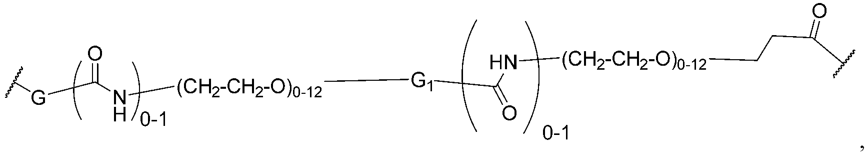 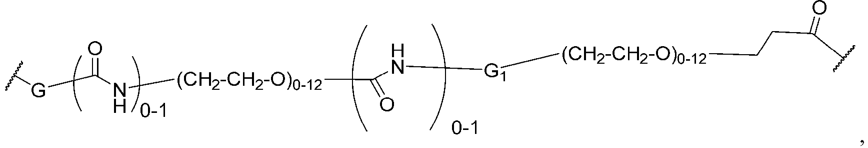 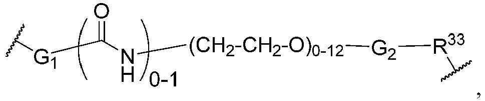 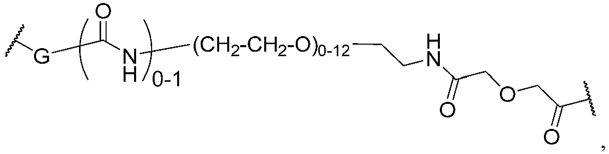 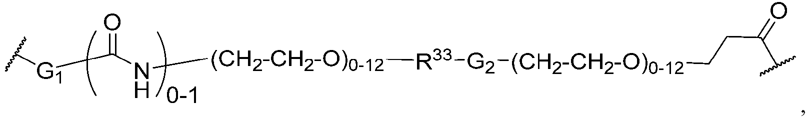 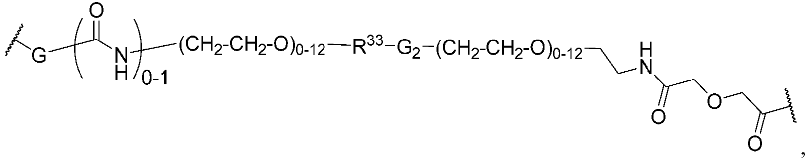 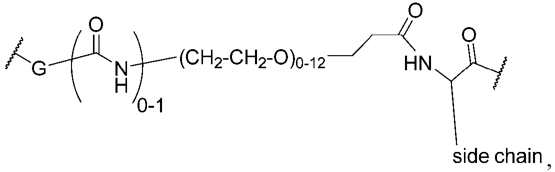 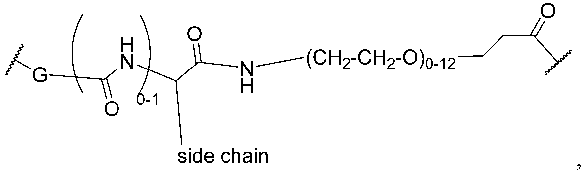 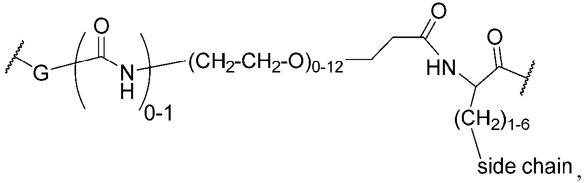 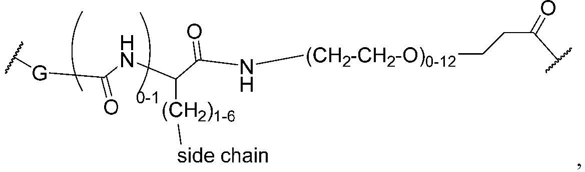 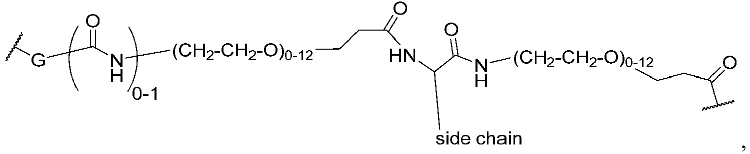 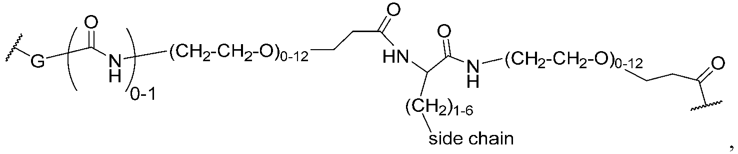 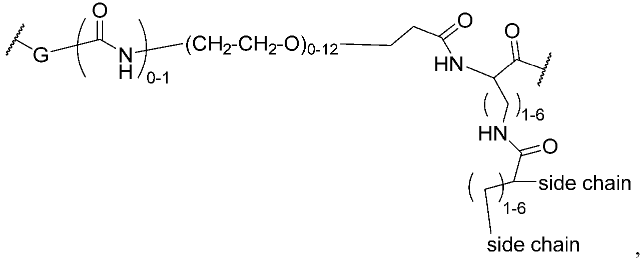 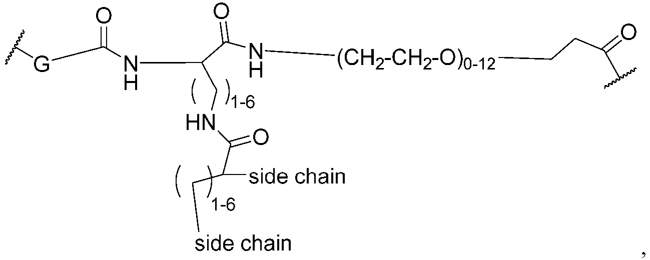 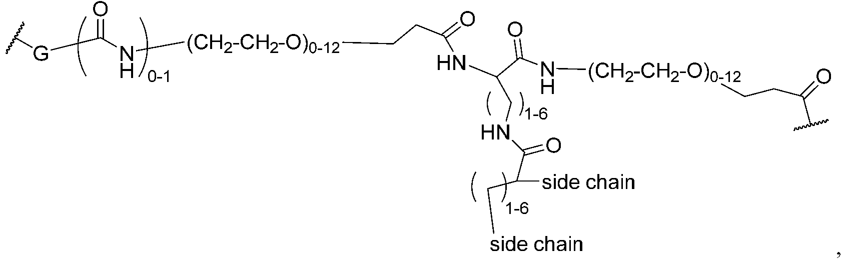 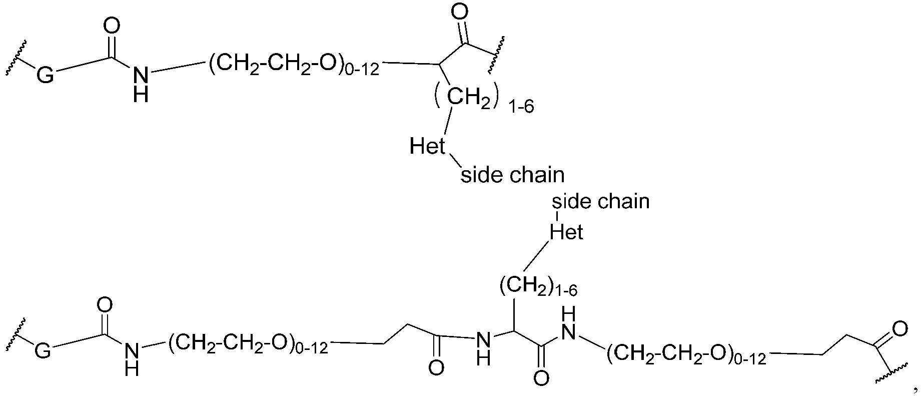 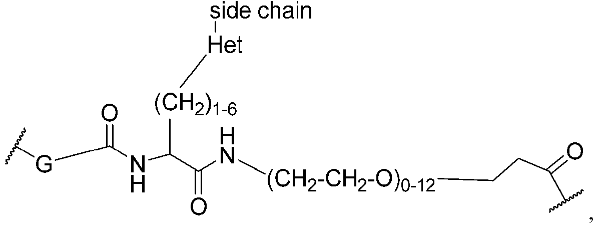 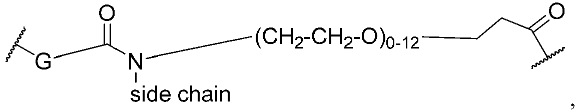 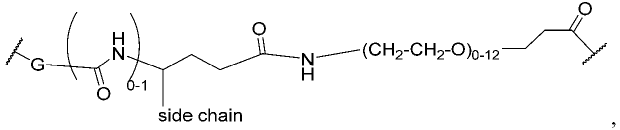 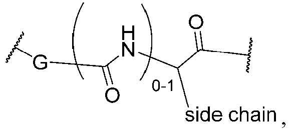 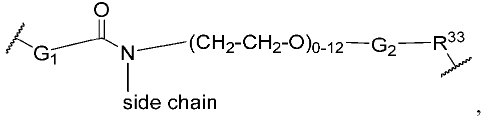 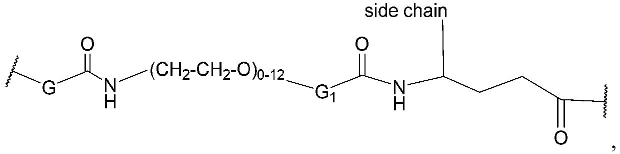
wherein the left side of the fragment described above is attached to L₁ₐ, and the right side is attached to L_{1c};
G is C₀₋₈ hydrocarbylene, C₀₋₈ heteroalkylene, -O-, -NH-, or a bond;
G₁ and G₂ are each independently C₀₋₈ hydrocarbylene, C₀₋₈ heteroalkylene, -O-, -NH-, or a bond;
R³³ is a 3- to 12-membered heterocyclic ring, a 5- to 10-membered heteroaromatic ring, NR³⁴, -CONR³⁴-, or - NR³⁴CO-;
R³⁴ is hydrogen, -(CH₂CH₂O)₀₋₁₂-H, -(CH₂CH₂O)₀₋₁₂-CH₃, -CO-(CH₂CH₂O)₀₋₁₂-H, -CO-(CH₂CH₂O)₀₋₁₂-CH₃, or CH₂-CO-(N(Me)-CH₂-CO)₀₋₂₄-OH;
Het is a 5- to 6-membered heteroaromatic ring;
the side chain is a hydrophilic group, wherein the hydrophilic group is as defined in claim 24;
preferably, G is C₁₋₆ alkylene, C₂₋₆ alkenylene, C₂₋₆ alkynylene, C₁₋₆ heteroalkylene, -O-, -NH-, or a bond;
preferably, G is methylene, ethylene, n-propylene, 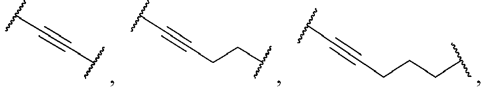 -N-CH₂-, -O-, -NH-, or a bond;
preferably, G₁ and G₂ are each independently C₁₋₄ alkylene, C₂₋₆ alkynylene, -NHC₁₋₄ alkylene, -O-, -NH-, or a bond;
preferably, G₁ and G₂ are each independently methylene, ethylene, n-propylene, -O-, -NH-, or a bond;
preferably, G₁ is methylene,
preferably, G₂ is ethylene;
preferably, R³³ is a 5- to 10-membered heteroaromatic ring;
preferably, R³³ is a 5- to 6-membered heteroaromatic ring;
preferably, R³³ is pyrrolyl, imidazolyl, pyrazolyl, triazolyl, pyridinyl, or pyrimidinyl;
preferably, R³³ is 1,2,3-triazolyl;
preferably, L_{1b} is a bond or selected from: 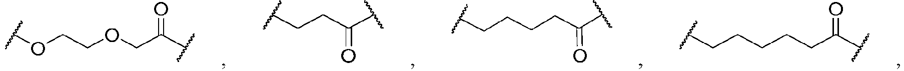 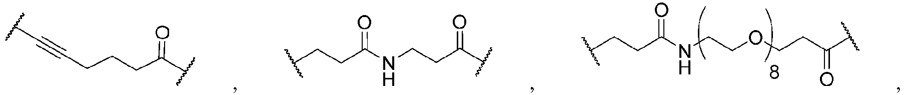 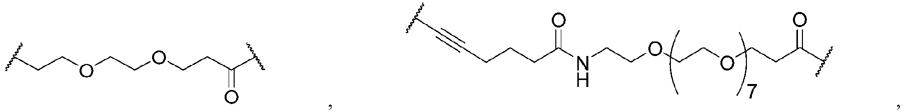 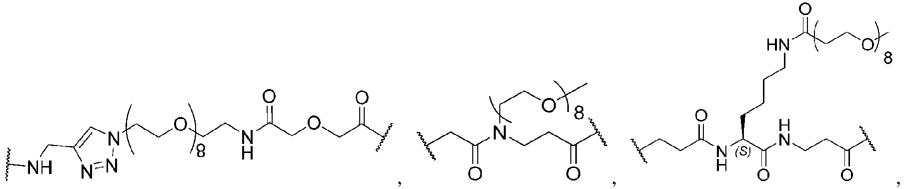 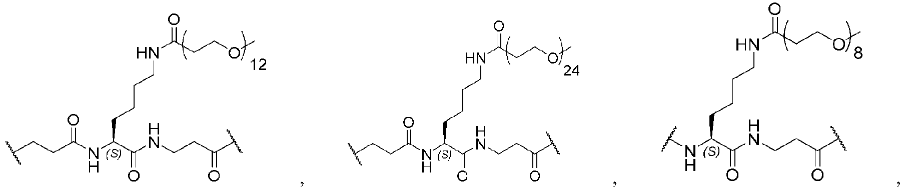 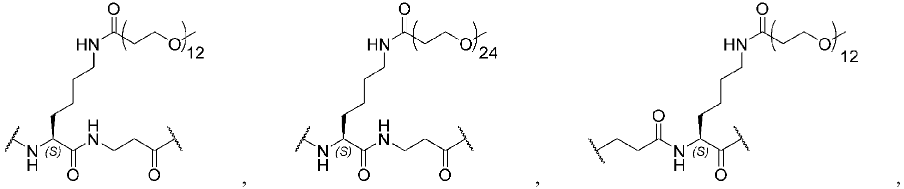 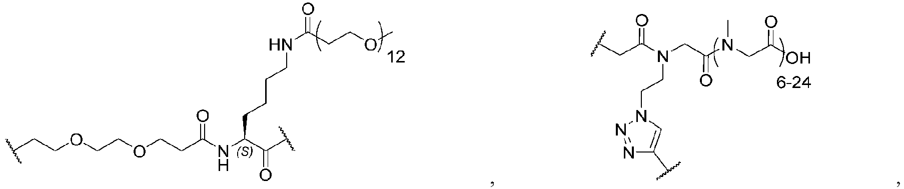 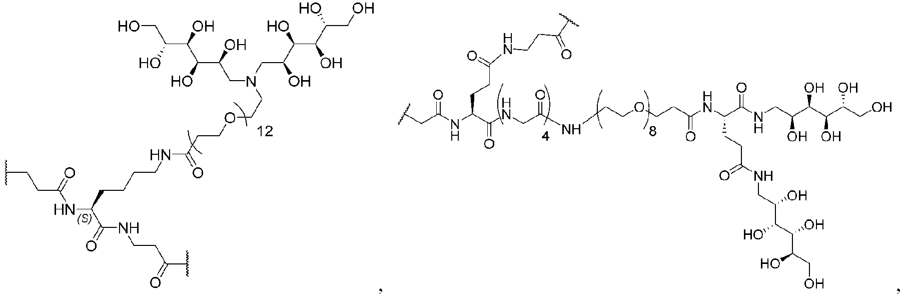 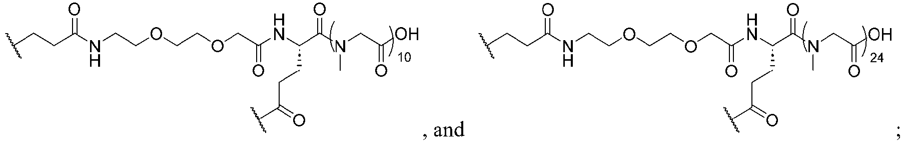
preferably, L_{1b} is 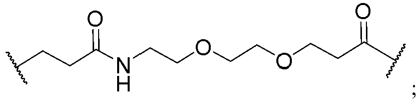
further preferably, L_{1b} is a bond or selected from: 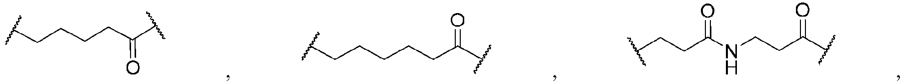 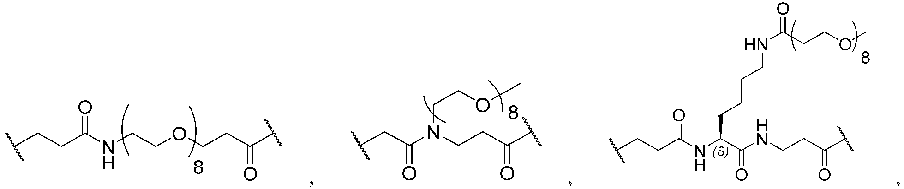 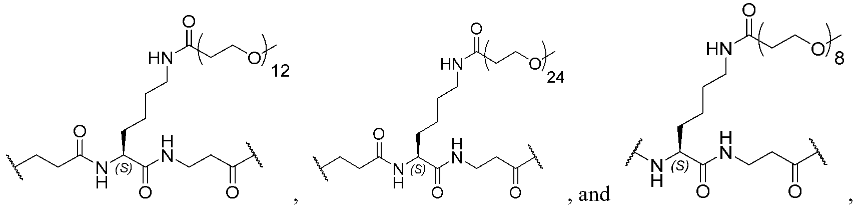
wherein the left side of the fragment described above is attached to L₁ₐ, and the right side is attached to L_{1c}.

28. The antibody-drug conjugate according to any one of claims 1-27, wherein,
L_{1c} is a bond or an amino acid unit, wherein the amino acid unit is an amino acid residue or a short peptide consisting of 2-10 amino acid residues; the amino acid residue is optionally substituted with a hydrophilic group or C₁₋₆ alkyl; the hydrophilic group is as defined in claim 24; the amino acid residue is selected from a natural amino acid residue, a non-natural amino acid residue, and an amino acid residue represented by formula (1a) or a stereoisomer thereof, 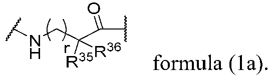
wherein:
R³⁵ and R³⁶ are each independently selected from: hydrogen, deuterium, 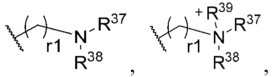 and 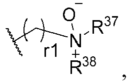 and R³⁵ and R³⁶ are not both H; or
R³⁵ and R³⁶, together with the carbon atom to which they are both attached, form 3- to 8-membered cycloalkyl or 4- to 10-membered heterocyclyl, wherein the 3- to 8-membered cycloalkyl or the 4- to 10-membered heterocyclyl is optionally substituted with one or more R⁴⁰;
r and r1 are each independently an integer from 0 to 20;
R³⁷ and R³⁸ are each independently H, C₁₋₆ alkyl, C₃₋₆ cycloalkyl, or -COO-C₁₋₆ alkyl; or
R³⁷ and R³⁸, together with the nitrogen atom to which they are attached, form 4- to 10-membered heterocyclyl, wherein the 4- to 10-membered heterocyclyl is optionally substituted with one or more R⁴⁰;
R³⁹ is C₁₋₆ alkyl;
R⁴⁰ is C₁₋₆ alkyl, C₁₋₆ haloalkyl, C₁₋₆ heteroalkyl, C₃₋₆ cycloalkyl, 4- to 10-membered heterocyclyl, C₁₋₆ alkylene-C₃₋₆ cycloalkyl, C₁₋₆ alkylene-4- to 10-membered heterocyclyl, -NR⁴⁰¹R⁴⁰², and 4- to 10-membered heterocyclyl optionally substituted with C₁₋₆ alkyl, wherein R⁴⁰¹ and R⁴⁰² are each independently H or C₁₋₆ alkyl;
preferably, r and r1 are each independently 0, 1, 2, 3, 4, or 5;
preferably, r is 0,
preferably, r1 is 4;
preferably, R³⁷ and R³⁸ are each independently H, C₁₋₄ alkyl, C₃₋₆ cycloalkyl, or -COO-C₁₋₄ alkyl;
preferably, R³⁷ and R³⁸ are each independently H or C₁₋₄ alkyl;
preferably, R³⁷ and R³⁸ are each independently ethyl;
preferably, R³⁹ is C₁₋₄ alkyl;
preferably, R³⁹ is methyl or ethyl;
preferably, R⁴⁰ is C₁₋₄ alkyl, C₁₋₄ haloalkyl, C₃₋₆ cycloalkyl, C₁₋₄ alkylene-C₃₋₆ cycloalkyl, or -NR⁴⁰¹R⁴⁰², wherein R⁴⁰¹ and R⁴⁰² are each independently H or C₁₋₄ alkyl;
preferably, R⁴⁰ is methyl, halomethyl, cyclopropyl, amino, NHCH₃, or N(CH₃)₂;
preferably, R³⁵ and R³⁶ are each independently selected from: hydrogen, deuterium, and 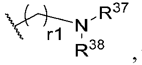 wherein r1, R³⁷, and R³⁸ are as defined in this claim;
preferably, R³⁵ is hydrogen,
preferably, R³⁶ is -(CH₂)₄N(CH₃)₂;
in formula (III-1),
preferably, L_{1c} is a bond or selected from: 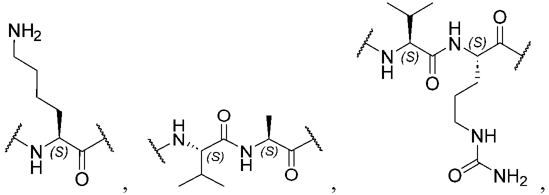 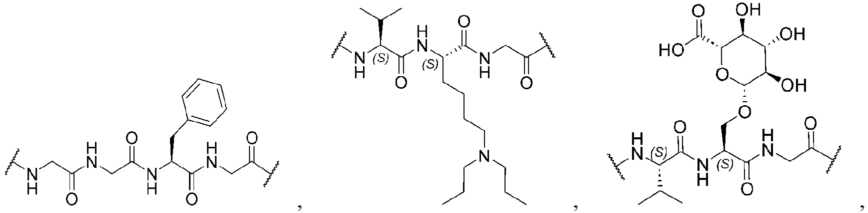 and 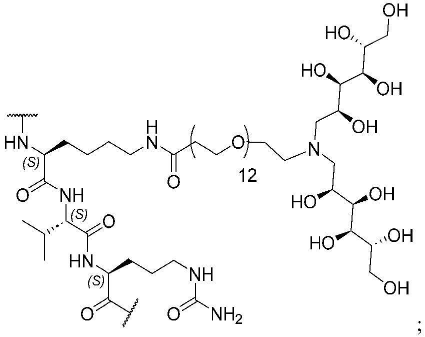
preferably, L_{1c} is selected from: 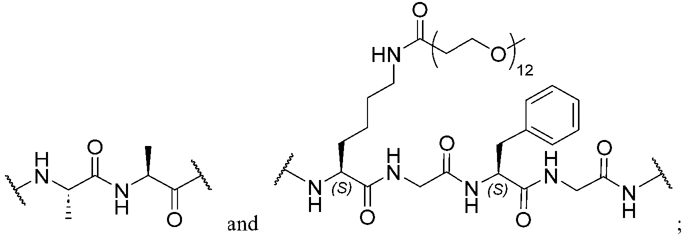
further preferably, L_{1c} is a bond or selected from: 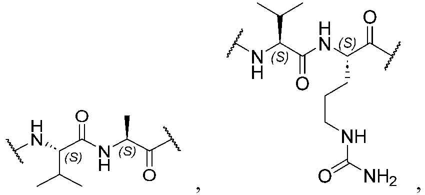 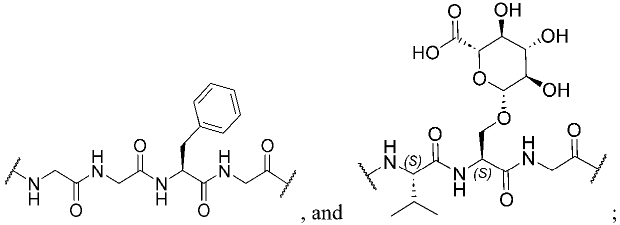
preferably, the left side of the fragment described above is attached to L_{1c}, and the right side is attached to L_{1d}; in formula (III-2),
preferably, L_{1c} is a bond or selected from: 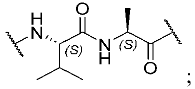
preferably, the left side of the fragment described above is attached to -C(=O)CH₃, and the right side is attached to L_{1d}.

29. The antibody-drug conjugate according to any one of claims 1-28, wherein,
in formula (III-1),
L_{1d} is a bond, or L_{1d} is selected from the group consisting of: 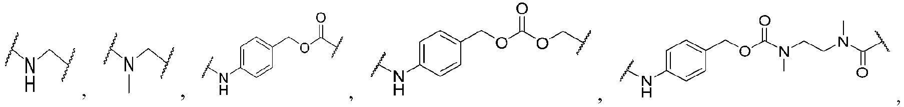 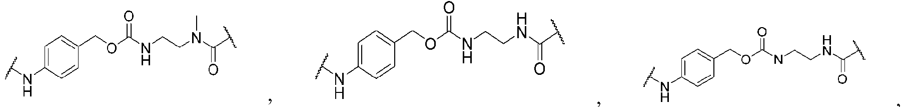 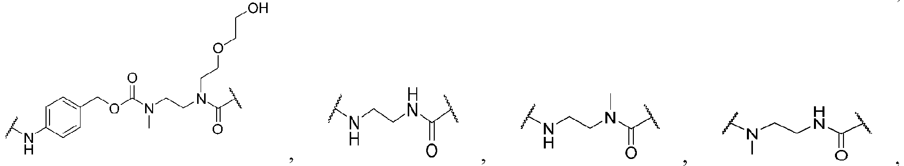 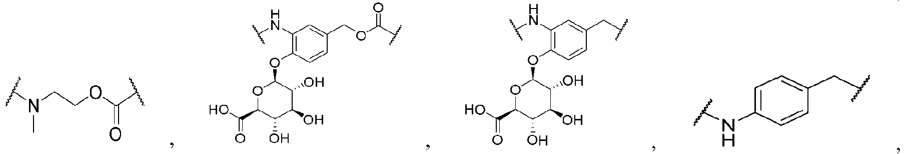 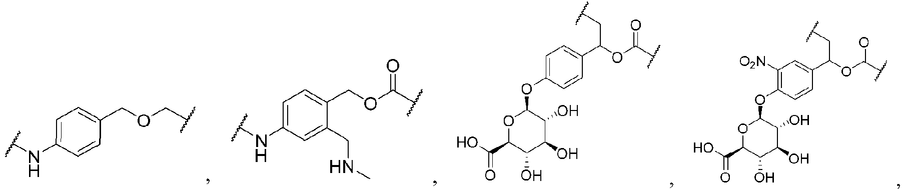 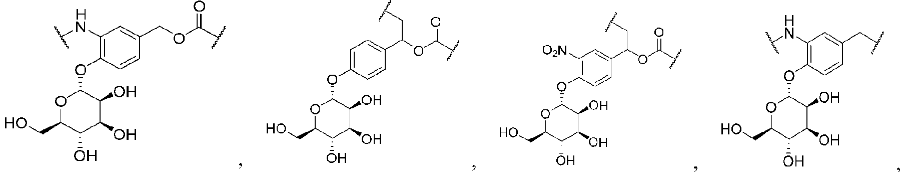 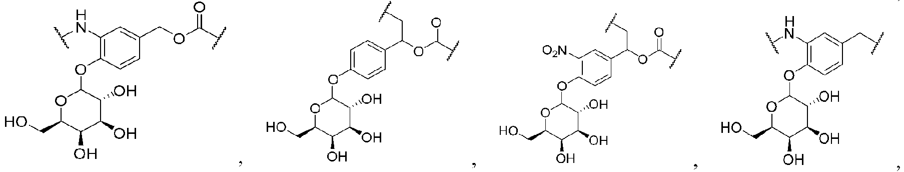 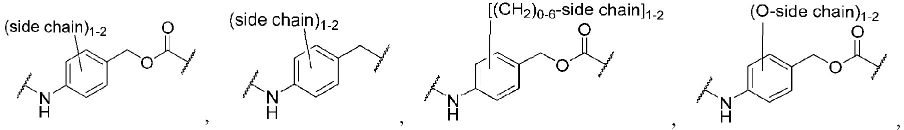 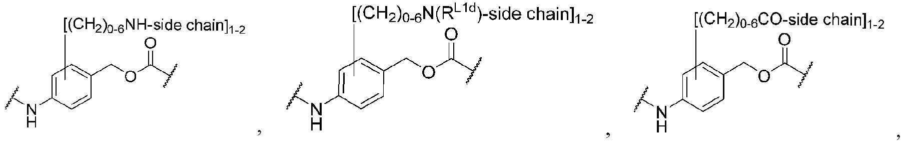 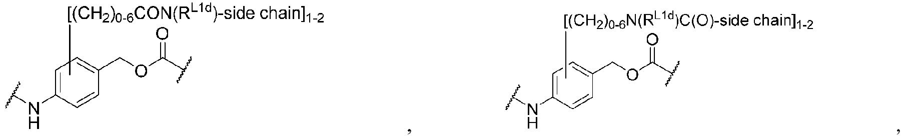 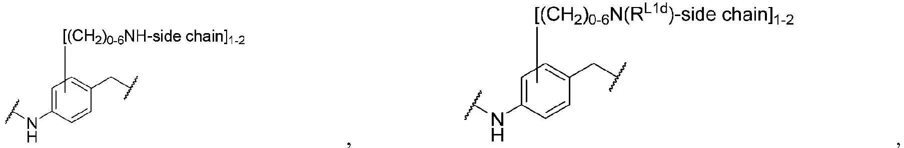 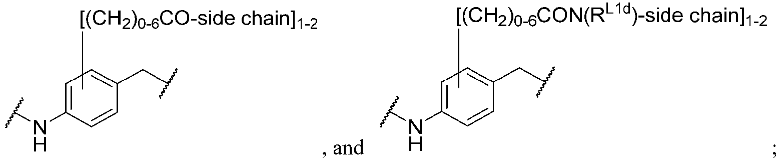
the benzene ring in the fragment described above is optionally substituted with one or more of the following groups: -C₁₋₄ alkylene-NH₂, -C₁₋₄ alkylene-NHC₁₋₄ alkyl, -C₁₋₄ alkylene-N(C₁₋₄ alkyl)₂, and -C₁₋₄ alkylene-NH-C₁₋₄ alkylene-COOH;
R^{L1d} in the fragment described above is hydrogen or C₁₋₄ alkyl;
the side chain in the fragment described above is a hydrophilic group, wherein the hydrophilic group is as defined in claim 24;
the left side of the fragment described above is attached to L_{1c}, and the right side is attached to D;
preferably, the benzene ring in the L_{1d} group is optionally substituted with one or more of the following groups: -C₁₋₄ alkylene-NHC₁₋₄ alkyl and -C₁₋₄ alkylene-N(C₁₋₄ alkyl)₂;
preferably, the benzene ring in the L_{1d} group is optionally substituted with 1, 2, or 3 of the following groups: - CH₂NHCH₃ and -CH₂NH(CH₃)₂;
preferably, R^{L1d} is hydrogen, methyl, ethyl, or isopropyl;
preferably, R^{L1d} is methyl;
further preferably, L_{1d} is a bond or selected from the group consisting of: 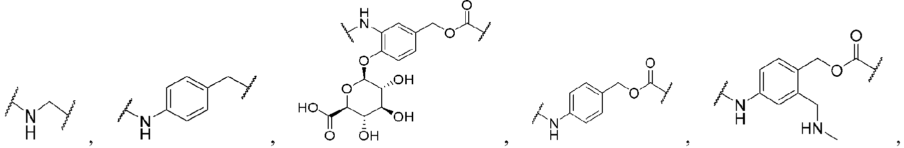 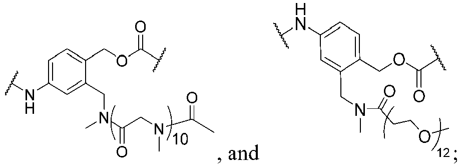
the left side of the fragment described above is attached to L_{1c}, and the right side is attached to D;
in formula (III-2),
L_{1d} is a bond, or L_{1d} is selected from the group consisting of: 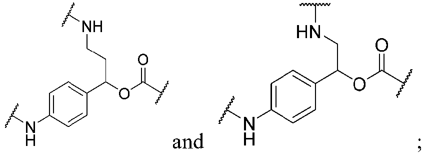
the benzene ring in the group described above is optionally substituted with one or more of the following groups: -C₁₋₄ alkylene-NH₂, -C₁₋₄ alkylene-NHC₁₋₄ alkyl, -C₁₋₄ alkylene-N(C₁₋₄ alkyl)₂, and -C₁₋₄ alkylene-NH-C₁₋₄ alkylene-COOH;
the upper side of the fragment described above is attached to L_{1b}, the left side is attached to L_{1c}, and the right side is attached to D;
preferably, L_{1d} is

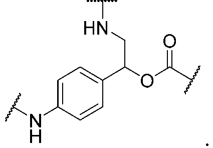

30. The antibody-drug conjugate according to any one of claims 1-29, wherein,
1) -H₂-L₁ₐ-L_{1d}-L_{1c}-L_{1d}- is selected from structures shown in Table 9;
more preferably, -H₂-L₁ₐ-L_{1d}-L_{1c}-L_{1d}- is selected from L1, L2, L3, L4, L5, L6, L7, L8, L9, L10, L11, L12, L13, L14, L15, L16, L17, L19, L22, L23, L24, L25, L26, L34, L52, L53, L54, L54-2, L57, L58, L63, L64, L65, L66, L67, L68, L69, L70, L72, L73, and L74;
further preferably, -H₂-L₁ₐ-L_{1d}-L_{1c}-L_{1d}- is selected from L1, L2, L4, L5, L6, L7, L8, L9, L10, L11, L12, L13, L14, L15, L16, L17, L23, L24, and L25;
still further preferably, -H₂-L₁ₐ₋L_{1b}-L_{1c-}L_{1d}- is selected from L1, L4, L5, L6, L7, L10, L12, L13, L14, L15, L16, and L23;
2) -H₂-L₁ₐ₋L_{1b}-L_{1d}(-L_{1c}-C(=O)-CH₃)- is selected from structures shown in Table 10.

31. The antibody-drug conjugate according to any one of claims 1-30, wherein the antibody-drug conjugate represented by formula (I) has a structure represented by formula (IV-1) or formula (IV-2): 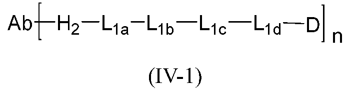 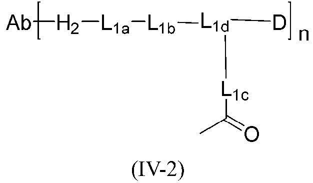 wherein:
Ab, n, H₂, L₁ₐ, L_{1b}, L_{1c}, L_{1d}, and D are as defined in any one of claims 1-30;
preferably, the antibody-drug conjugate represented by formula (I) has a structure represented by formula (IV-1).

32. The antibody-drug conjugate according to any one of claims 1-31, wherein Ab is an antibody or an antigen-binding fragment thereof that binds to one, two, or more targets selected from the group consisting of: HER2,
Trop2, EGFR, Claudin18.2, Nectin-4, FRα, TF, CD49B, Claudin7, HER3, Notch1, MUC5AC, IGF1R, cMET, PD-L1, B7-H3, B7-H4, CDCP1, MUC16, MUC1, MSLN, FAP, 5T4, CEACAM5, LIV, NaPi2b, AXL, LY6E, CDH6, CDH17, ITGB6, Claudin6, CEACAM6, LY6G6D, LGR5, PTK7, CD228, and SLC34A2;
preferably, Ab is selected from the group consisting of: an anti-EGFR antibody or an antigen-binding fragment thereof, an anti-HER3 antibody or an antigen-binding fragment thereof, and a bispecific antibody that specifically binds to EGFR and HER3 or an antigen-binding fragment thereof;
preferably, the anti-EGFR antibody or the antigen-binding fragment thereof is Panitumumab, Necitumumab, Nimotuzumab, EG-007, Laprituximab, Cetuximab, or JMT101;
preferably, the anti-HER3 antibody or the antigen-binding fragment thereof is Seribantumab, Elgemtumab, Lumretuzumab, AV203, Barecetamab, CDX-3379, GSK2849330, HMBD-001, REGN1400, or Patritumab;
preferably, the bispecific antibody that specifically binds to EGFR and HER3 or the antigen-binding fragment thereof is SI-B001 or Duligotuzumab;
preferably, Ab is attached to L₀ or H₂ via a sulfhydryl group thereof.

33. The antibody-drug conjugate according to any one of claims 1-32, wherein n is a number from 1 to 15;
preferably, n is a number from 2 to 10;
preferably, n is a number from 3 to 9, e.g., from 3 to 4.5, from 4.5 to 6, from 6 to 7, from 7 to 8, or from 8 to 9, e.g., about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, about 7, about 7.1, about 7.2, about 7.3, about 7.4, about 7.5, about 7.6, about 7.7, about 7.8, about 7.9, about 8, about 8.1, about 8.2, about 8.3, about 8.4, about 8.5, about 8.6, about 8.7, about 8.8, about 8.9, or about 9.

34. The antibody-drug conjugate according to any one of claims 1-33, wherein the antibody-drug conjugate is represented by formula (V-4) or formula (V-4-A) below: 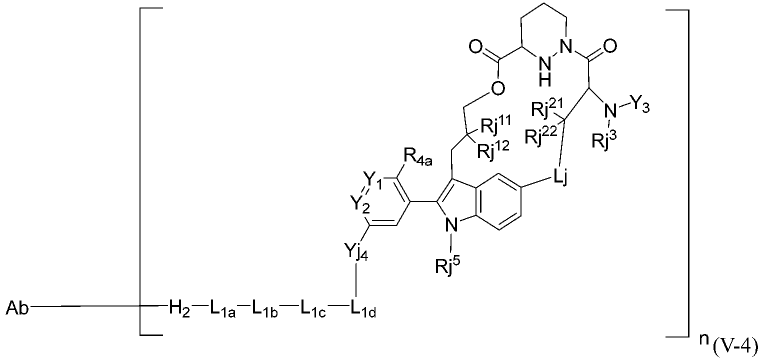 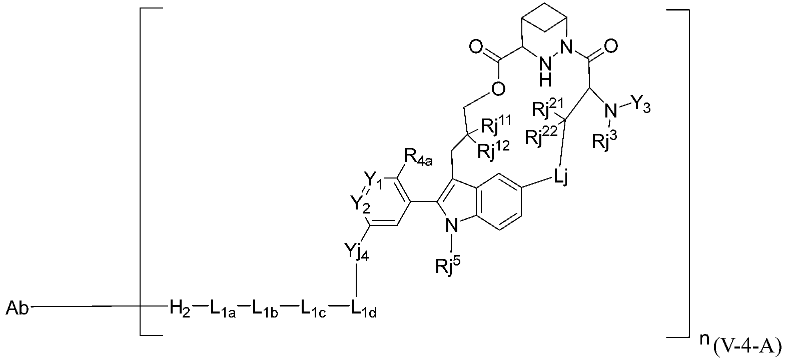 wherein:
H₂, L₁ₐ, L_{1b}, L_{1c}, L₁ₐ, Rj⁵, Rj¹¹, Rj¹², Rj²¹, Rj²², Rj³, R₄ₐ, Lj, Yj₄, Y₁, Y₂, Y₃, n, and Ab are as defined in any one of claims 1-33.

35. The antibody-drug conjugate according to any one of claims 1-34, wherein the antibody-drug conjugate is selected from structures shown in Table 11.

36. A pharmaceutical composition, comprising the antibody-drug conjugate according to any one of claims 1-35, and a pharmaceutically acceptable carrier.

37. Use of the antibody-drug conjugate according to any one of claims 1-35 or the pharmaceutical composition according to claim 36 in the preparation of a medicament for preventing and/or treating a disease or disorder associated with RAS protein activity, wherein
preferably, the disease or disorder associated with RAS protein activity is a cancer;
preferably, the cancer is pancreatic cancer, colorectal cancer, non-small cell lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer.

38. A compound represented by formula (VII),
L₀-D (VII)
wherein D is as defined in any one of claims 1-23;
L₀ comprises H₁, L₁ₐ, L_{1b}, L_{1c}, and L_{1d}, wherein L₁ₐ, L_{1b}, L_{1c}, and L_{1d} are as defined in any one of claims 24-31;
H₁ is an antibody-linking unit;
preferably, H₁ is selected from the group consisting of: 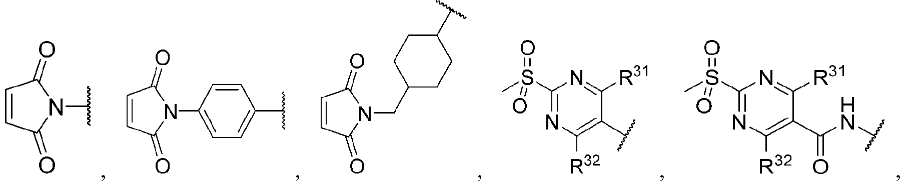 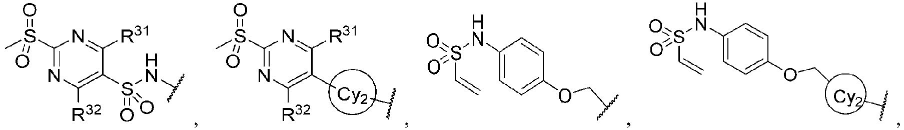 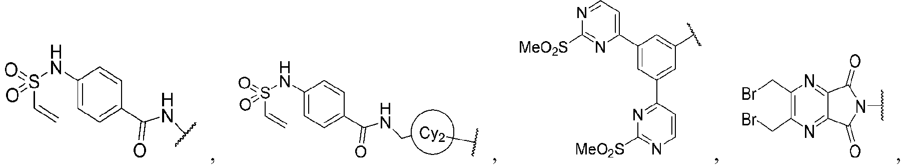 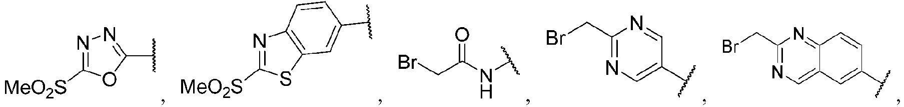 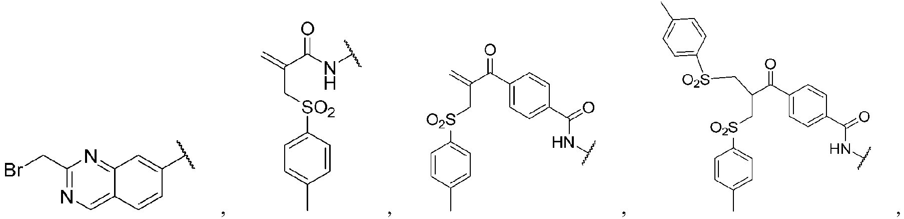 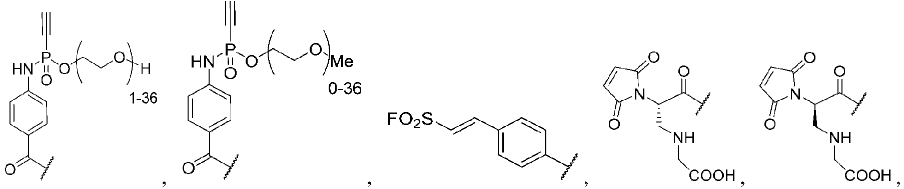 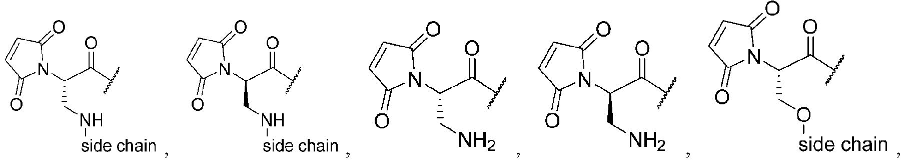 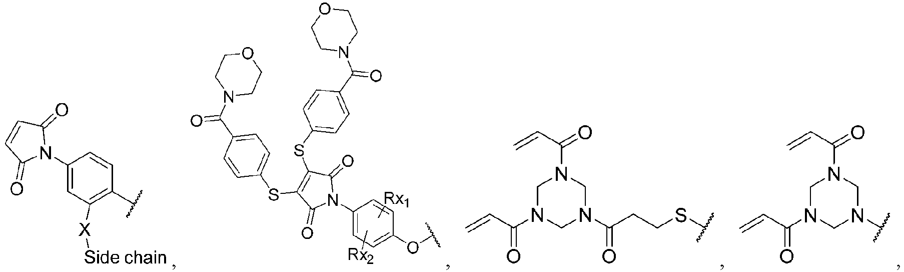 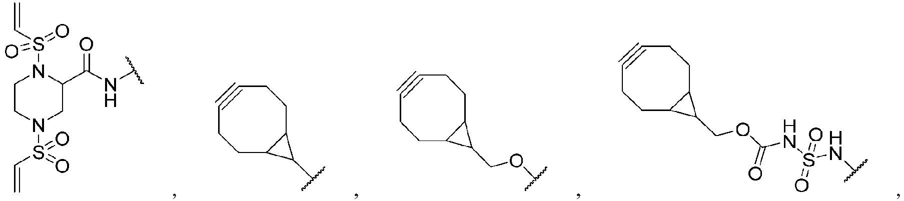 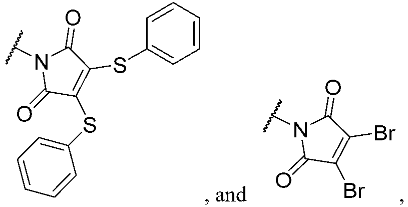 wherein:
X is -O-(CH₂)₀₋₆-, -N(CH₃)-, -O-, -CONH-, or -CON(CH₃)-;
Rₓ₁ and Rₓ₂ are each independently hydrogen, C₁₋₆ alkyl, halogen, deuterium, C₁₋₆ alkoxy, C₁₋₆ alkylene-NH-, or (C₁₋₆ alkyl)₂-N-;
R³¹ is hydrogen, deuterium, halogen, cyano, or C₁₋₄ alkyl;
R³² is hydrogen, deuterium, halogen, cyano, or C₁₋₄ alkyl;
Cy2 is 5- to 10-membered heteroaryl, C₆₋₁₀ aryl, 3- to 10-membered heterocyclyl, or C₃₋₁₀ cycloalkyl;
the side chain is a hydrophilic group, wherein the hydrophilic group is as defined in claim 24;
preferably, Rₓ₁ and Rₓ₂ are each independently hydrogen, C₁₋₄ alkyl, halogen, deuterium, C₁₋₄ alkoxy, C₁₋₄ alkylene-NH-, or (C₁₋₄ alkyl)₂-N-;
preferably, Rₓ₁ and Rₓ₂ are each independently hydrogen, methyl, halogen, deuterium, methoxy, methyl-NH-, or (methyl)₂-N-;
preferably, R³¹ is hydrogen, deuterium, halogen, cyano, or methyl;
preferably, R³¹ is hydrogen;
preferably, R³² is hydrogen, deuterium, halogen, cyano, or methyl;
preferably, R³² is hydrogen;
preferably, Cy2 is 5- to 10-membered heteroaryl, C₆₋₁₀ aryl, 4- to 8-membered heterocyclyl, or C₃₋₆ cycloalkyl;
preferably, Cy2 is pyrrolyl, imidazolyl, pyridinyl, pyrimidinyl, phenyl, pyrrolidinyl, piperidinyl, piperazinyl, cyclopropyl, cyclobutyl, or cyclopentyl;
preferably, H₁ is selected from the group consisting of: 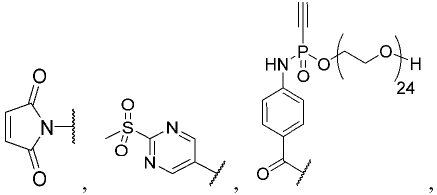 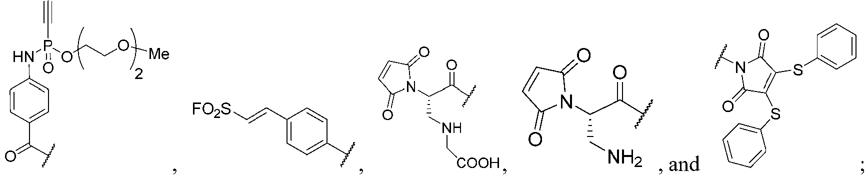
more preferably, H₁ is selected from the group consisting of: 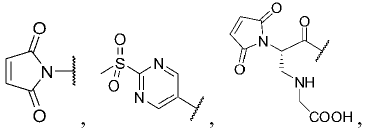 and 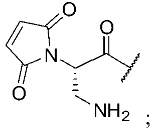
further preferably, H₁ is selected from the group consisting of: 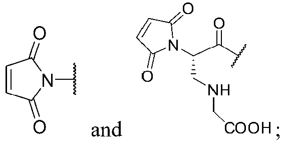
still further preferably, H₁ is 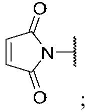
preferably, L₀-D is selected from the structure represented by formula (VII-A) or formula (VII-B):
H₁-L₁ₐ-L_{1d}-L_{1c}-L_{1d}-D (VII-A)
H₁-L₁ₐ-L_{1b}-L_{1d}(-L_{1c}-C(=O)-CH₃)-D (VII-B)
L₁ₐ, L_{1b}, L_{1c}, L_{1d}, and D are as defined in any one of claims 1-31, and H₁ is as defined in this claim.

39. The compound according to claim 38, wherein L₀-D is selected from structures shown in Table 12.

40. Use of the compound according to claim 38 or 39 in the preparation of the antibody-drug conjugate according to any one of claims 1-35.

41. A compound represented by formula (Z-III), or a stable deuterated derivative thereof, a stereoisomer thereof, a pharmaceutically acceptable salt thereof, a solvate thereof, or a prodrug thereof: 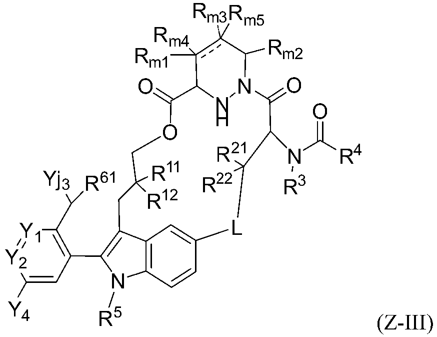 wherein in the formula,
Rₘ₁ is hydrogen; Rₘ₂ is hydrogen; Rₘ₃ is hydrogen; Rₘ₄ is hydrogen, halogen, or C₁₋₆ alkyl; Rₘ₅ is hydrogen, halogen, or C₁₋₆ alkyl; and 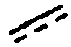 is a single bond; or
Rₘ₁ and Rₘ₃ are both absent; Rₘ₂ is hydrogen; Rₘ₄ is hydrogen, halogen, or C₁₋₆ alkyl; Rₘ₅ is hydrogen, halogen,
or C₁₋₆ alkyl; and 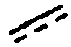 is a double bond; or
Rₘ₁ is attached to Rₘ₂ to jointly form -CRₘ₇Rₘ₆- or -CRₘ₇Rₘ₆-CRₘ₉Rₘ₈-; Rₘ₃ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 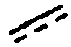 is a single bond; or
Rₘ₁ is attached to Rₘ₃ to jointly form -CRₘ₇Rₘ₆- or -CRₘ₇Rₘ₆-CRₘ₉Rₘ₈-; Rₘ₂ is hydrogen; Rₘ₄ is hydrogen, halogen, or C₁₋₆ alkyl; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 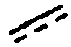 is a single bond; or
Rₘ₁ is attached to Rₘ₄ to jointly form -CRₘ₇Rₘ₆- or -CRₘ₇Rₘ₆-CRₘ₉Rₘ₈-; Rₘ₂ is hydrogen; Rₘ₃ is hydrogen; Rₘ₅ is hydrogen, halogen, or C₁₋₆ alkyl; Rₘ₆, Rₘ₇, Rₘ₈, and Rₘ₉ are each independently hydrogen, halogen, or C₁₋₆ alkyl; and 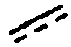 is a single bond;
R¹¹ and R¹² are each independently hydrogen, deuterated C₁₋₆ alkyl, or C₁₋₆ alkyl;
Y₁ is N or CH, wherein N may be oxidized (N⁺-O⁻);
Y₂ is N or CH, wherein N may be oxidized (N⁺-O⁻);
R³ is hydrogen or C₁₋₆ alkyl;
Yj₃ is NH-R⁶, O-R⁶, -C₁₋₄ alkylene-NH-R⁶, or -C₁₋₄ alkylene-O-R⁶;
R⁶ is hydrogen or C₁₋₆ alkyl;
R⁶¹ is C₁₋₆alkyl;
R²¹, R²², R⁵, L, R⁴, and Y₄ are as described in cases (1), (2), (3), (4), or (5):
Case (1)
R²¹ and R²² are each independently hydrogen, -NR^{f}R^{g}, -O-C₁₋₄ alkylene-NR^{f}R^{g}, or C₁₋₆ alkyl;
L is C₆₋₁₀ aryl or 5- or 6-membered monocyclic heteroaryl, wherein the C₆₋₁₀ aryl is optionally substituted with hydroxy or amino;
R⁵ is hydrogen, ethyl, -C₁₋₄ alkylene-OH, -C₁₋₄ alkylene-NHR^{e}, or -C₁₋₄ alkylene-C₁₋₆ alkoxy;
R⁴ is C₆₋₁₀ aryl, heteroaryl, C₃₋₆ cycloalkyl, or -CR^{a}R^{b}-NR^{e}R^{d}, wherein the C₆₋₁₀ aryl, heteroaryl, or C₃₋₆ cycloalkyl is optionally substituted with one or more substituents selected from R⁴¹; R⁴¹ is SF₅, P(=O)(C₁₋₆ alkyl)₂, methyl, -C₁₋₄ alkylene-OH, or -C(O)-NR^{c}R^{d};
Y₄ is 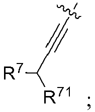
wherein:
R⁷ is 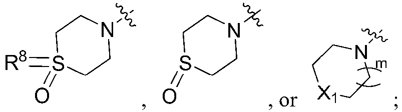
R⁸ is O or NH; X₁ is -CR⁹¹R⁹²- or -NR¹⁰-;
R⁷¹ is hydrogen, C₁₋₆ alkyl, -C₁₋₄ alkylene-OH, or -C₁₋₄ alkylene-NH-R⁶;
R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen, and -C₁₋₄ alkylene- is optionally substituted with methyl;
R¹⁰ is hydrogen, -C(O)-C₁₋₄ alkylene-NR^{f}R^{g}, or -C(O)-C₁₋₄ alkylene-OH;
m is 1 or 2;
R^{a} and R^{b} are each independently hydrogen, C₁₋₆ alkyl, or C₃₋₆ cycloalkyl;
R^{c} and R^{d} are each independently hydrogen, -C(O)H, C₁₋₆ alkyl, C₁₋₆ haloalkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
when the following conditions are satisfied, R⁷ is not 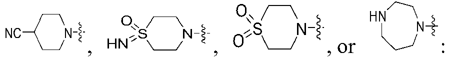 Rₘ₁ is hydrogen; Rₘ₂ is hydrogen; Rₘ₃ is hydrogen; Rₘ₄ is hydrogen; Rₘ₅ is hydrogen; 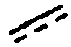 is a single bond; R⁴ is 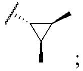 R³, R²¹, and R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is ethyl;
when the following conditions are satisfied, R⁷ is not 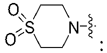 Rₘ₁ is hydrogen; Rₘ₂ is hydrogen; Rₘ₃ is hydrogen; Rₘ₄ is hydrogen; Rₘ₅ is hydrogen; 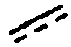 is a single bond; R⁴ is 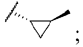 R³, R²¹, **and** R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is ethyl;
when the following conditions are satisfied, R⁷ is not 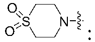 Rₘ₁ is hydrogen; Rₘ₂ is hydrogen; Rₘ₃ is
hydrogen; Rₘ₄ is hydrogen; Rₘ₅ is hydrogen; 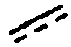 is a single bond; R⁴ is 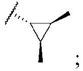 R³, R²¹, **and** R²² are hydrogen; L is thiazolyl; Yj₃ is O-R⁶; Y₁ is N; Y₂ is CH; R¹¹, R¹², R⁶, and R⁶¹ are methyl; R⁷¹ is hydrogen; and R⁵ is -CH₂CH₂OH or -CH₂CH₂NH(CH₃);
or
Case (2)
R²¹ and R²² are each independently hydrogen, -NR^{f}R^{g}, -O-C₁₋₄ alkylene-NR^{f}R^{g}, or C₁₋₆ alkyl;
L is C₆₋₁₀ aryl or 5- or 6-membered monocyclic heteroaryl, wherein the C₆₋₁₀ aryl is optionally substituted with hydroxy or amino;
R⁵ is hydrogen, ethyl, -C₁₋₄ alkylene-OH, -C₁₋₄ alkylene-NHR^{e}, or -C₁₋₄ alkylene-C₁₋₆ alkoxy;
R⁴ is C₆₋₁₀ aryl, heteroaryl, C₃₋₆ cycloalkyl, or 5- to 8-membered heterocyclyl, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d};
Y₄ is 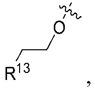
wherein R¹³ is as described in case (21) or (22):
case (21): R¹³ is 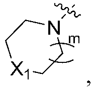 wherein X₁ is -CR⁹¹R⁹²-; R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen; m is 1 or 2; and Y₁ is N⁺-O⁻; Y₂ is CH; or Y₁ is CH; Y₂ is N⁺-O⁻;
or
case (22): R¹³ is oxo-5-membered heterocyclyl-L₁-L₂-R¹⁴, oxo-6-membered heterocyclyl-L₁₄-L₂-R¹⁴, or oxo-7-membered heterocyclyl-L₁-L₂-R¹⁴; L₁ is -C₁₋₄ alkylene-O- or -C₁₋₄ alkylene-NH-; L₂ is -C₁₋₄ alkylene-O- or -C₁₋₄ alkylene-NH-; R¹⁴ is C₁₋₆ heteroalkyl;
R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
Case (3)
R²¹ and R²² are each independently hydrogen, -NR^{f}R^{g}, -O-C₁₋₄ alkylene-NR^{f}R^{g}, or C₁₋₆ alkyl;
L is C₆₋₁₀ aryl or 5- or 6-membered monocyclic heteroaryl, wherein the C₆₋₁₀ aryl is optionally substituted with hydroxy or amino;
R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
R⁴ is 5- to 8-membered heterocyclyl;
Y₄ is 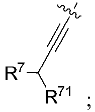
wherein:
R⁷ is 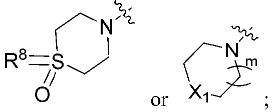
R⁸ is O or NH;
X₁ is -CR⁹¹R⁹²- or -NR¹⁰-;
R⁷¹ is hydrogen, C₁₋₆ alkyl, -C₁₋₄ alkylene-OH, or -C₁₋₄ alkylene-NH-R⁶;
R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen;
R¹⁰ is hydrogen, C₁₋₆ alkyl, -C(O)-C₁₋₄ alkylene-NR^{f}R^{g}, or -C(O)-C₁₋₄ alkylene-OH;
m is 1 or 2;
R^{a} and R^{d} are each independently hydrogen, C₁₋₆ alkyl, or C₃₋₆ cycloalkyl;
R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
Case (4)
R²¹ and R²² are each independently hydrogen, -NR^{f}R^{g}, -O-C₁₋₄ alkylene-NR^{f}R^{g}, or C₁₋₆ alkyl; and R²¹ and R²² are not both hydrogen;
L is C₆₋₁₀ aryl or 5- or 6-membered monocyclic heteroaryl, wherein the C₆₋₁₀ aryl is optionally substituted with hydroxy or amino;
R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
R⁴ is C₆₋₁₀ aryl, heteroaryl, C₃₋₆ cycloalkyl, or -CR^{a}R^{b}-NR^{e}R^{d}, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d};
Y₄ is 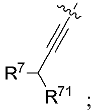
wherein:
R⁷ is 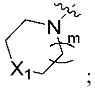
X₁ is -NR¹⁰-;
R⁷¹ is hydrogen, C₁₋₆ alkyl, -C₁₋₄ alkylene-OH, or -C₁₋₄ alkylene-NH-R⁶;
R¹⁰ is C₁₋₆ alkyl;
m is 1 or 2;
R^{a} and R^{d} are each independently hydrogen, C₁₋₆ alkyl, or C₃₋₆ cycloalkyl;
R^{c} and R^{d} are each independently hydrogen, -C(O)H, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
Case (5)
R²¹ and R²² are each independently hydrogen, -NR^{f}R^{g}, -O-C₁₋₄ alkylene-NR^{f}R^{g}, or C₁₋₆ alkyl;
L is oxazolyl;
R⁵ is hydrogen, C₁₋₆ alkyl (e.g., ethyl), C₃₋₆ cycloalkyl (e.g., cyclopropyl), -C₁₋₄ alkylene-OH, -C₁₋₄ alkylene-NHR^{e}, or -C₁₋₄ alkylene-C₁₋₆ alkoxy;
R⁴ is C₆₋₁₀ aryl, heteroaryl, C₃₋₆ cycloalkyl, 5- to 8-membered heterocyclyl, or -CR^{a}R^{b}-NR^{e}R^{d}, wherein the C₆₋₁₀ aryl, heteroaryl, or C₃₋₆ cycloalkyl is optionally substituted with one or more substituents selected from R⁴¹; R⁴¹ is methyl, -C₁₋₄ alkylene-OH, or -C(O)-NR^{c}R^{d};
Y₄ is 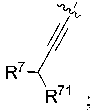
wherein:
R⁷ is 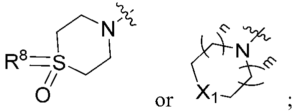
R⁷ may optionally be substituted with -C₁₋₄ alkylene-OH or -C₁₋₄ alkylene-NH-R⁶;
R⁸ is O or NH; X₁ is -O-, -CR⁹¹R⁹²-, or -NR¹⁰-;
R⁷¹ is hydrogen, C₁₋₆ alkyl, -C₁₋₄ alkylene-OH, or -C₁₋₄ alkylene-NH-R⁶;
R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}, and -C₁₋₄ alkylene- may be substituted with methyl;
R¹⁰ is hydrogen, C₁₋₆ alkyl, -C(O)-C₁₋₄ alkylene-NR^{f}R^{g}, or -C(O)-C₁₋₄ alkylene-OH;
m is 0, 1, or 2;
n is 0, 1, or 2;
R^{a} and R^{d} are each independently hydrogen, C₁₋₆ alkyl, or C₃₋₆ cycloalkyl;
R^{c} and R^{d} are each independently hydrogen, -C(O)H, C₁₋₆ alkyl, phenyl, pyridinyl, -C₁₋₄ alkylene-OH, -C(O)-5-to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
R^{f} is hydrogen or C₁₋₆ alkyl;
R^{g} is hydrogen or C₁₋₆ alkyl.

42. The compound, or the stable deuterated derivative thereof, the stereoisomer thereof, the pharmaceutically acceptable salt thereof, the solvate thereof, or the prodrug thereof according to claim 41, wherein in case (1),
when R⁴ is 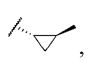 , R⁷ is not 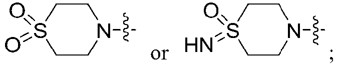
when the following conditions are satisfied, R⁷ is not 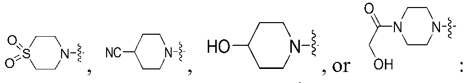 Rₘ₁ is hydrogen; Rₘ₂ is hydrogen; Rₘ₃ is hydrogen; Rₘ₄ is hydrogen; Rₘ₅ is hydrogen; 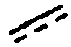 is a single bond; and R⁴ is heteroaryl, wherein the heteroaryl is optionally substituted with one or more substituents selected from R⁴¹, and R⁴¹ is SF₅, P(=O)(C₁₋₆ alkyl)₂, methyl, -C₁₋₄ alkylene-OH, or -C(O)-NR^{c}R^{d}.

43. The compound, or the stable deuterated derivative thereof, the stereoisomer thereof, the pharmaceutically acceptable salt thereof, the solvate thereof, or the prodrug thereof according to claim 41 or 42, wherein the compound is selected from a compound represented by formula (Z-IIIA): 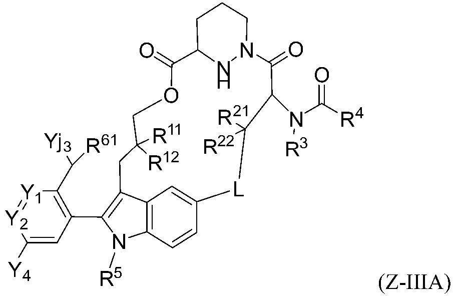 wherein in the formula,
R¹¹ and R¹² are each independently hydrogen, deuterated C₁₋₆ alkyl, or C₁₋₆ alkyl;
R²¹ and R²² are each independently hydrogen, -NR^{f}R^{g}, -O-C₁₋₄ alkylene-NR^{f}R^{g}, or C₁₋₆ alkyl;
Y₁ is N or CH, wherein N may be oxidized (N⁺-O⁻);
Y₂ is N or CH, wherein N may be oxidized (N⁺-O⁻);
L is C₆₋₁₀ aryl or 5- or 6-membered monocyclic heteroaryl, wherein the C₆₋₁₀ aryl is optionally substituted with hydroxy or amino;
R³ is hydrogen or C₁₋₆ alkyl;
R⁵ is hydrogen, ethyl, -C₁₋₄ alkylene-OH, -C₁₋₄ alkylene-NHR^{e}, or -C₁₋₄ alkylene-C₁₋₆ alkoxy;
Yj₃ is NH-R⁶, O-R⁶, -C₁₋₄ alkylene-NH-R⁶, or -C₁₋₄ alkylene-O-R⁶;
R⁶ is hydrogen or C₁₋₆ alkyl;
R⁶¹ is C₁₋₆ alkyl;
R⁴ and Y₄ are as described in case (1) or (2):
case (1): R⁴ is C₃₋₆ cycloalkyl, 5- to 8-membered heterocyclyl, or -CR^{a}R^{b}-NR^{c}R^{d}, wherein the C₃₋₆ cycloalkyl is optionally substituted with one or more substituents selected from R⁴¹; R⁴¹ is methyl, -C₁₋₄ alkylene-OH, or - C(O)-NR^{c}R^{d}; Y₄ is 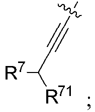 wherein:
R⁷ is 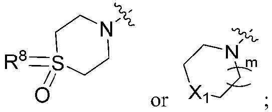
R⁸ is O or NH; X₁ is -CR⁹¹R⁹²- or -NR¹⁰-;
R⁷¹ is hydrogen, C₁₋₆ alkyl, -C₁₋₄ alkylene-OH, or -C₁₋₄ alkylene-NH-R⁶;
R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R ⁹¹ and R ⁹² are not both hydrogen;
R¹⁰ is hydrogen, -C(O)-C₁₋₄ alkylene-NR^{f}R^{g}, or -C(O)-C₁₋₄ alkylene-OH;
m is 1 or 2;
R^{a} and R^{b} are each independently hydrogen, C₁₋₆ alkyl, or C₃₋₆ cycloalkyl;
R^{c} and R^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
R^{f} is hydrogen or C₁₋₆ alkyl;
R^{g} is hydrogen or C₁₋₆ alkyl;
when the following conditions are satisfied, R⁷ is not 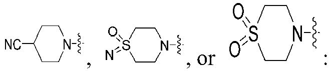 R⁴ is 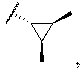 R³, R²¹, and R²² are hydrogen, Yj₃ is O-R⁶, Y₁ is N, Y₂ is CH, R¹¹, R¹², R⁶, and R⁶¹ are methyl, R⁷¹ is hydrogen, and R⁵ is ethyl;
when the following conditions are satisfied, R⁷ is not 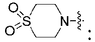 R⁴ is 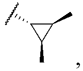 R³, R²¹, and R²² are hydrogen, Yj₃ is O-R⁶, Y₁ is N, Y₂ is CH, R¹¹, R¹², R⁶, and R⁶¹ are methyl, R⁷¹ is hydrogen, and R⁵ is -CH₂CH₂OH or - CH₂CH₂NH(CH₃);
or
case (2): R⁴ is C₃₋₆ cycloalkyl or 5- to 8-membered heterocyclyl, wherein the C₃₋₆ cycloalkyl is optionally substituted with one or more substituents selected from R⁴¹; R⁴¹ is methyl, -C₁₋₄ alkylene-OH, or -C(O)-NR^{c}R^{d}; Y₄ is 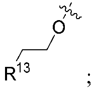
wherein R¹³ is as described in case (21) or (22):
case (21): R¹³ is 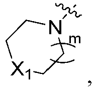 wherein X₁ is -CR⁹¹R⁹²-; R⁹¹ and R⁹² are each independently hydrogen, hydroxy, cyano, -NR^{c}R^{d}, -C₁₋₄ alkylene-NR^{c}R^{d}, -C₁₋₄ alkylene-OH, or -C(O)-NR^{c}R^{d}; R⁹¹ and R⁹² are not both hydrogen; m is 1 or 2; and Y₁ is N⁺-O⁻; Y₂ is CH; or Y₁ is CH; Y₂ is N⁺-O⁻;
or
case (22): R¹³ is oxo-5-membered heterocyclyl-L₁-L₂- R¹⁴, oxo-6-membered heterocyclyl-L₁-L₂-R¹⁴, or oxo-7-membered heterocyclyl-L₁-L₂-R¹⁴; L₁ is -C₁₋₄ alkylene-O- or -C₁₋₄ alkylene-NH-; L₂ is -C₁₋₄ alkylene-O- or -C₁₋₄ alkylene-NH-; R¹⁴ is C₁₋₆ heteroalkyl;
R^{c} and R^{d} are each independently hydrogen, C₁₋₆ alkyl, -C₁₋₄ alkylene-OH, -C(O)-5- to 8-membered heterocyclyl, or -C(O)-C₁₋₄ alkylene-OH, wherein the 5- to 8-membered heterocyclyl is optionally substituted with one or more substituents selected from R⁴²;
R⁴² is methyl, -NR^{f}R^{g}, -C(O)R^{e}, or -C(O)OR^{e};
R^{e} is C₁₋₆ alkyl;
R^{f} is hydrogen or C₁₋₆ alkyl;
R^{g} is hydrogen or C₁₋₆ alkyl.

44. The compound, or the stable deuterated derivative thereof, the stereoisomer thereof, the pharmaceutically acceptable salt thereof, the solvate thereof, or the prodrug thereof according to claim 43, wherein in case (1), when the following conditions are satisfied, R⁷ is not 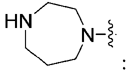 R⁴ is 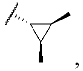 R³, R²¹, and R²² are hydrogen, Yj₃ is O-R⁶, Y₁ is N, Y₂ is CH, R¹¹, R¹², R⁶, and R⁶¹ are methyl, R⁷¹ is hydrogen, and R⁵ is ethyl; when R⁴ is 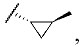 R⁷ is not 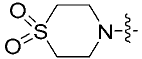 or

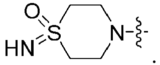

45. The compound, or the stable deuterated derivative thereof, the stereoisomer thereof, the pharmaceutically acceptable salt thereof, the solvate thereof, or the prodrug thereof according to any one of claims 41-44, wherein Y₁ is N or N⁺-O⁻; Y₂ is CH;
still further preferably, Y₁ is N; Y₂ is CH.

46. The compound, or the stable deuterated derivative thereof, the stereoisomer thereof, the pharmaceutically acceptable salt thereof, the solvate thereof, or the prodrug thereof according to any one of claims 41-45, wherein R¹¹ is methyl or deuterated methyl; R¹² is methyl or deuterated methyl;
further preferably, R¹¹ is methyl; R¹² is methyl.

47. The compound, or the stable deuterated derivative thereof, the stereoisomer thereof, the pharmaceutically acceptable salt thereof, the solvate thereof, or the prodrug thereof according to any one of claims 41-46, wherein R²¹ and R²² are each independently hydrogen or -O-C₁₋₄ alkylene-NR^{f}R^{g}; R^{f} is C₁₋₆ alkyl, and R^{g} is C₁₋₆ alkyl;
preferably, R²¹ is hydrogen; R²² is hydrogen, -NH₂, -OCH₂CH₂N(CH₃)₂, or -OCH₂CH₂NH(CH₃);
still further preferably, R²¹ is hydrogen; R²² is hydrogen.

48. The compound, or the stable deuterated derivative thereof, the stereoisomer thereof, the pharmaceutically acceptable salt thereof, the solvate thereof, or the prodrug thereof according to any one of claims 41-47, wherein L is phenyl, thiazolyl, oxazolyl, 1,3,4-oxadiazolyl, or 1,2,4-oxadiazolyl, and the phenyl is optionally substituted with hydroxy or amino;
preferably, L is phenyl, thienyl, or oxazolyl, and the phenyl is optionally substituted with hydroxy or amino;
still further preferably, L is thienyl or oxazolyl.

49. The compound, or the stable deuterated derivative thereof, the stereoisomer thereof, the pharmaceutically acceptable salt thereof, the solvate thereof, or the prodrug thereof according to any one of claims 41-48, wherein the compound is represented by formula (Z-IA-1), formula (Z-IA-2), formula (Z-IA-3), or formula (Z-IA-4): 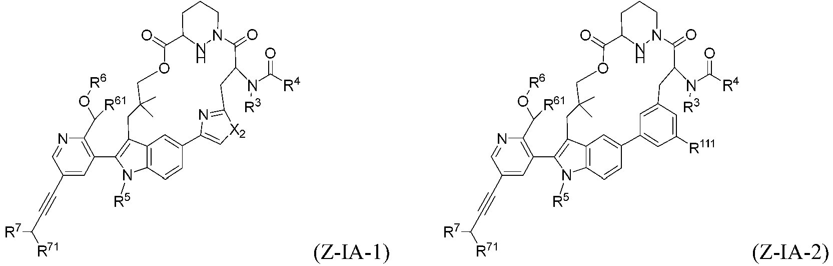 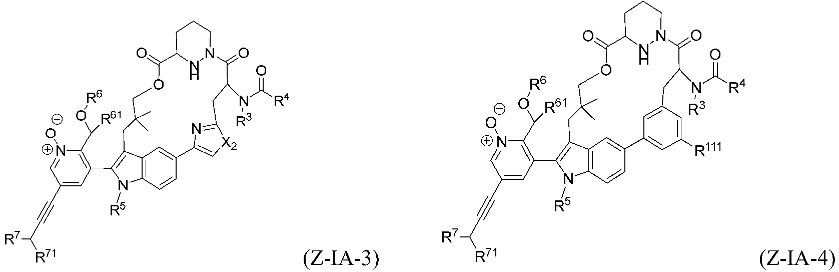
in the formulas, R³, R⁴, R⁵, R⁶, R⁶¹, R⁷, and R⁷¹ are each as defined in any one of claims 41-48;
X₂ is S or O; R¹¹¹ is amino, hydroxy, or hydrogen.

50. The compound, or the stable deuterated derivative thereof, the stereoisomer thereof, the pharmaceutically acceptable salt thereof, the solvate thereof, or the prodrug thereof according to any one of claims 41-49, wherein 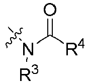 is selected from the group consisting of: 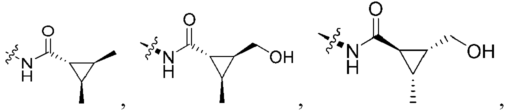 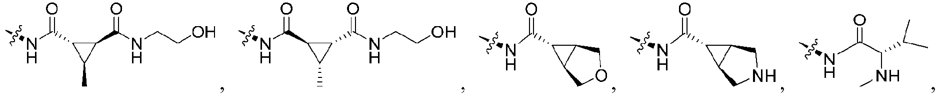 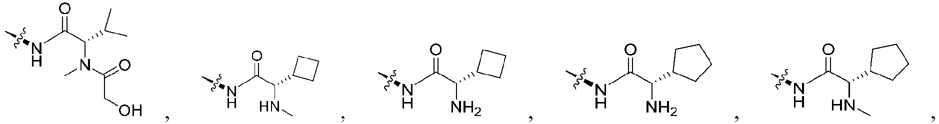 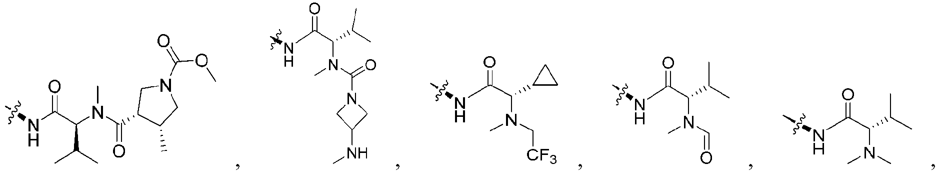 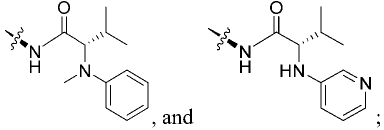 preferably, 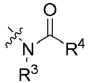 is selected from the group consisting of:

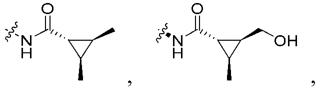

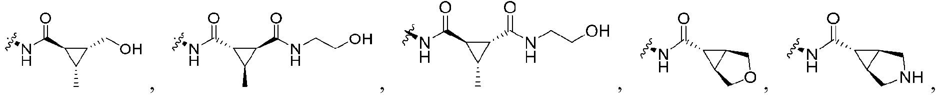

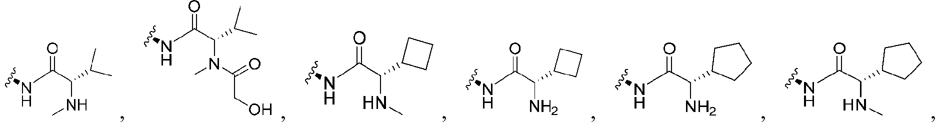

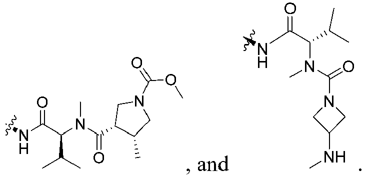

51. The compound, or the stable deuterated derivative thereof, the stereoisomer thereof, the pharmaceutically acceptable salt thereof, the solvate thereof, or the prodrug thereof according to any one of claims 41-50, wherein R⁵ is ethyl, cyclopropyl, -(CH₂)₂OH, -(CH₂)₃OH, -(CH₂)₂-NH(CH₃), or -(CH₂)₂-OCH₃;
preferably, R⁵ is ethyl, -(CH₂)₂OH, -(CH₂)₃OH, -(CH₂)₂-NH(CH₃), or -(CH₂)₂-OCH₃;
further preferably, R⁵ is ethyl, -(CH₂)₃OH, or -(CH₂)₂-OCH₃;
still further preferably, R⁵ is ethyl.

52. The compound, or the stable deuterated derivative thereof, the stereoisomer thereof, the pharmaceutically acceptable salt thereof, the solvate thereof, or the prodrug thereof according to any one of claims 41-51, wherein R⁶ is hydrogen; or
R⁶ is methyl, and R⁶¹ is methyl.

53. The compound, or the stable deuterated derivative thereof, the stereoisomer thereof, the pharmaceutically acceptable salt thereof, the solvate thereof, or the prodrug thereof according to any one of claims 41-52, wherein R⁷¹ is hydrogen or -C₁₋₄ alkylene-OH;
further preferably, R⁷¹ is hydrogen or -CH₂OH;
still further preferably, R⁷¹ is hydrogen;
R⁷ is selected from the group consisting of: 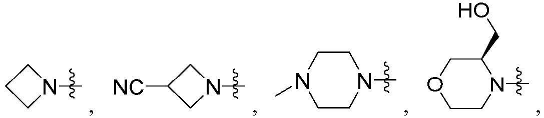 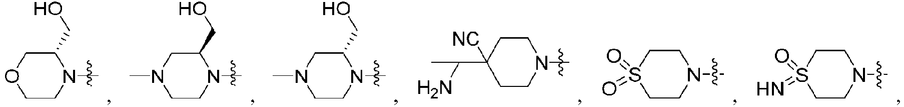 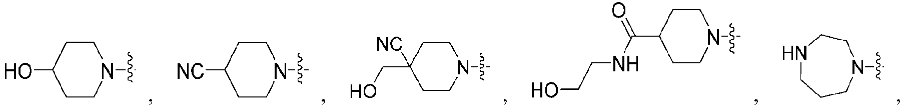 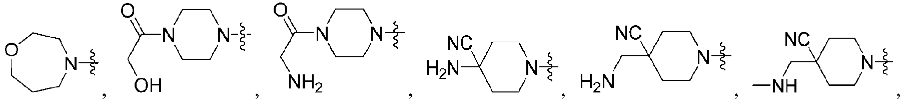 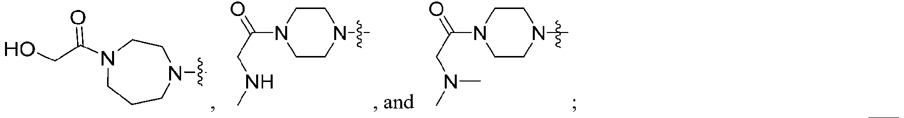
preferably, R⁷ is selected from the group consisting of: 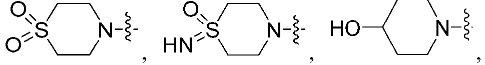 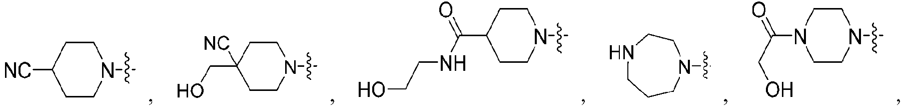 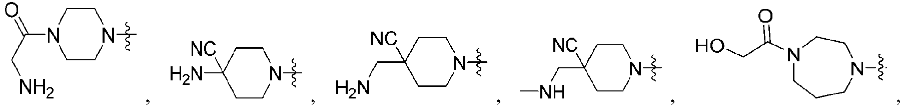 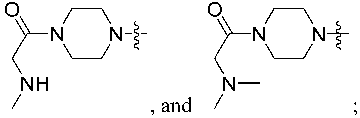 R⁷¹ is hydrogen or -CH₂OH;
preferably, R⁷ is selected from the group consisting of: 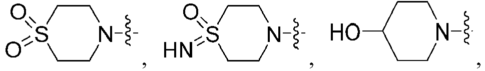 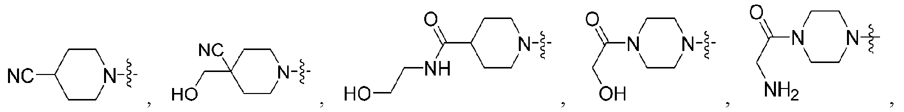 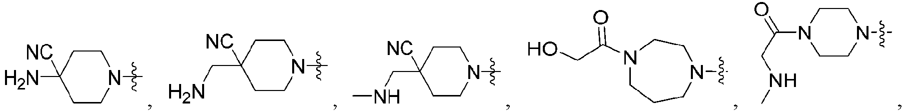 and 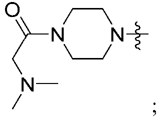 R⁷¹ is hydrogen or -CH₂OH;
still further preferably, R⁷¹ is hydrogen, and R⁷ is selected from 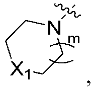 wherein m is 1; X₁ is -CR⁹¹R⁹²-; R⁹¹ is cyano; R⁹² is as defined in any one of claims 41-52; preferably, R⁹² is hydrogen, -NR^{c}R^{d}, -C₁₋₄ alkylene-NR^{c}R^{d}, or -C₁₋₄ alkylene-OH, and R^{c} and R ^{d} are each independently hydrogen or C₁₋₆ alkyl; preferably, R⁹² is hydrogen, -NH₂, -CH₂NH₂, -CH₂NH(CH₃), -CH₂OH, or -C(O)-NH(CH₂CH₂OH);
still further preferably, R⁷¹ is hydrogen, and R⁷ is selected from the group consisting of: 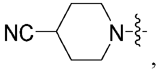 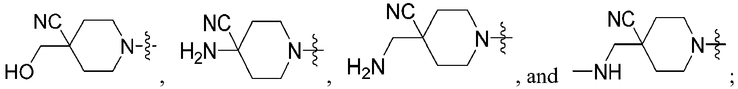
still further preferably, R⁷¹ is hydrogen, and R⁷ is selected from the group consisting of:

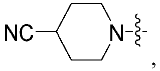

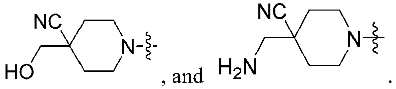

54. The compound, or the stable deuterated derivative thereof, the stereoisomer thereof, the pharmaceutically acceptable salt thereof, the solvate thereof, or the prodrug thereof according to claims 41-53, wherein the compound is selected from Table A-1 and Table A-2.

55. The compound, or the stable deuterated derivative thereof, the stereoisomer thereof, the pharmaceutically acceptable salt thereof, the solvate thereof, or the prodrug thereof according to any one of claims 41-48, wherein the compound is represented by formula (Z-IIA-1) or formula (Z-IIA-2): 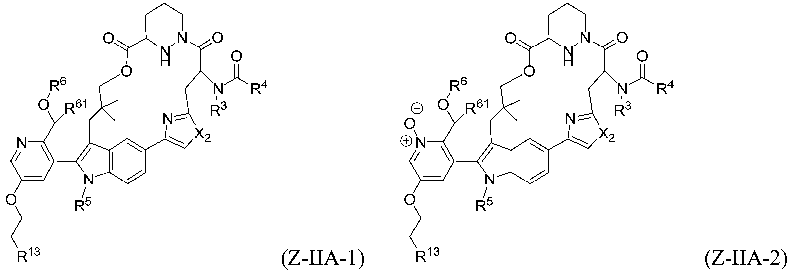
in the formulas, R³, R⁴, R⁵, R⁶, R⁶¹, and R¹³ are each as defined in any one of claims 41-48;
X₂ is S or O.

56. The compound, or the stable deuterated derivative thereof, the stereoisomer thereof, the pharmaceutically acceptable salt thereof, the solvate thereof, or the prodrug thereof according to claim 55, wherein 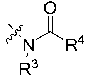 is selected from the group consisting of: 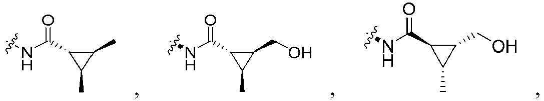 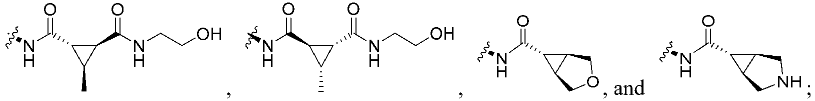 further preferably, 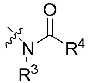 is selected from the group consisting of:

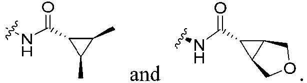

57. The compound, or the stable deuterated derivative thereof, the stereoisomer thereof, the pharmaceutically acceptable salt thereof, the solvate thereof, or the prodrug thereof according to claim 55 or 56, wherein
R¹³ is selected from the group consisting of:

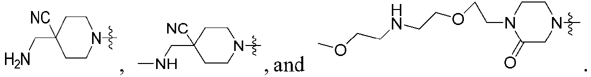

58. The compound, or the stable deuterated derivative thereof, the stereoisomer thereof, the pharmaceutically acceptable salt thereof, the solvate thereof, or the prodrug thereof according to any one of claims 55-57, wherein the compound is selected from Table B-1 and Table B-2.

59. A pharmaceutical composition, comprising the compound, or the stable deuterated derivative thereof, the stereoisomer thereof, the pharmaceutically acceptable salt thereof, the solvate thereof, or the prodrug thereof according to any one of claims 41-58, and a pharmaceutically acceptable carrier.

60. Use of the compound, or the stable deuterated derivative thereof, the stereoisomer thereof, the pharmaceutically acceptable salt thereof, the solvate thereof, or the prodrug thereof according to any one of claims 41-58 or the pharmaceutical composition according to claim 59 in the preparation of a medicament for preventing and/or treating a disease or disorder associated with RAS protein activity, wherein
preferably, the disease or disorder associated with RAS protein activity is a cancer;
preferably, the cancer is pancreatic cancer, colorectal cancer, lung cancer, acute myeloid leukemia, multiple myeloma, thyroid adenocarcinoma, myelodysplastic syndrome, squamous cell carcinoma of lung, esophageal cancer, ovarian cancer, uterine cancer, melanoma, bladder cancer, or head and neck cancer;
preferably, the lung cancer is non-small cell lung cancer.
